# EUROPEAN PATENT SPECIFICATION

(11) **EP 2 304 057 B1**
(45) Date of publication and mention of the grant of the patent: **17.09.2014**
(21) Application number: 09766208.4
(22) Date of filing: 17.06.2009
(51) Int. Cl.: C12Q 1/68

(54) **METHOD FOR THE DETECTION OF OVARIAN CANCER**
VERFAHREN ZUM NACHWEIS VON OVARIALKARZINOM
METHODE POUR DETECTER DU CANCER DE L'OVAIRE

(30) Priority: 17.06.2008 EP 08075561; 03.03.2009 EP 09075093
(43) Date of publication of application: 06.04.2011
(73) Proprietor: Signature Diagnostics AG, 14473 Potsdam (DE)
(72) Inventor: MODEL, Fabian, D-12683 Berlin (DE); RUJAN, Tamas, D- 79540 Lörrach (DE)
(74) Representative: Hoppe, Georg Johannes
(86) International application number: PCT/IB2009/006344
(87) International publication number: WO 2009/153667

(56) References cited:
- DATABASE Geneseq [Online] 1 November 2007 (2007-11-01), "Human genomic DNA containing a SNP SEQ ID NO:15728." XP002567635 retrieved from EBI accession no. GSN:AFI74072 Database accession no. AFI74072
- WILSON ET AL: "DNA hypomethylation and human diseases" BBA - REVIEWS ON CANCER, ELSEVIER SCIENCE BV, AMSTERDAM, NL, vol. 1775, no. 1, 13 December 2006 (2006-12-13), pages 138-162, XP005802354 ISSN: 0304-419X
- CZEKIERDOWSKI ARTUR ET AL: "The role of CpG islands hypomethylation and abnormal expression of neuronal protein synuclein-gamma (SNCG) in ovarian cancer." NEURO ENDOCRINOLOGY LETTERS JUN 2006, vol. 27, no. 3, June 2006 (2006-06), pages 381-386, XP9129245 ISSN: 0172-780X
- WEI SUSAN H ET AL: "Methylation microarray analysis of late-stage ovarian carcinomas distinguishes progression-free survival in patients and identifies candidate epigenetic markers" CLINICAL CANCER RESEARCH, THE AMERICAN ASSOCIATION FOR CANCER RESEARCH, US, vol. 8, no. 7, 1 July 2002 (2002-07-01), pages 2246-2252, XP002449289 ISSN: 1078-0432
- CANCER RESEARCH 1 JUL 2004, vol. 64, no. 13, 1 July 2004 (2004-07-01), pages 4472-4480, XP002567508 ISSN: 0008-5472
- GYNECOLOGIC ONCOLOGY APR 2008, vol. 109, no. 1, April 2008 (2008-04), pages 129-139, XP002567507 ISSN: 1095-6859
- MOLECULAR REPRODUCTION AND DEVELOPMENT NOV 2008, vol. 75, no. 11, 8 April 2008 (2008-04-08), pages 1591-1606, XP002567637 ISSN: 1098-2795
- DANIEL J WEISENBERGER ET AL: "Comprehensive DNA Methylation Analysis on the Illumina Infinium Assay Platform", INTERNET CITATION, 25 March 2008 (2008-03-25), pages 1-4, XP002592349, Retrieved from the Internet: URL:http://www.illumina.com/Documents/prod ucts/appnotes/appnote_infinium_methylation .pdf [retrieved on 2010-07-15]

### SEQUENCE LISTING

<110> occure GmbH
<120> METHODS FOR THE DETECTION OF OVARIAN CARCINOMA
<130> P_0004EP
<160> 650
<170> PatentIn version 3.5
<210> 1
   <211> 50
   <212> DNA
   <213> human
<400> 1
   ctatgccgct ctgtgtacag acctattctg atgaggtcct tgctggtccg 50
<210> 2
   <211> 50
   <212> DNA
   <213> human
<400> 2
   ttatgtcgtt ttgtgtatag atttattttg atgaggtttt tgttggttcg 50
<210> 3
   <211> 50
   <212> DNA
   <213> human
<400> 3
   ttatgttgtt ttgtgtatag atttattttg atgaggtttt tgttggtttg 50
<210> 4
   <211> 50
   <212> DNA
   <213> human
<400> 4
   cggattagta aggattttat tagaataggt ttgtatatag agcggtatag 50
<210> 5
   <211> 50
   <212> DNA
   <213> human
<400> 5
   tggattagta aggattttat tagaataggt ttgtatatag agtggtatag 50
<210> 6
   <211> 3001
   <212> DNA
   <213> human
<400> 6
   gagggcgtac aagtgactgc catgtggagg cccgaggaga ggacgaggcg gtcggggcga     60
   tgccgccgac gggcagcaga gcgagggaga gcctctgtcg gcggagagaa gggacggcca    120
   cccaccagca ggcaggaggg taccggtttc tgcccataga gagaaaggtg aagccctgcc    180
   cgcgggcggc agaggagggg cagagccccg tggtagccaa gagaggaagg aggcggccga    240
   ccgcccgtta gcgggaggag gagaggcccc gcgccggtgg gcggggcggg ggcccaggtt    300
   ctgacaggag ggggacgagc gcaacgcggc ggggaaccca ggacccgacg gccccgcggg    360
   gtcgaccgcg ccgggggtgc gaaggcgacg gtctcgcccg cggtgccccg gagggcgctg    420
   gatcccgcct gccggcggcc tctcaccagg agcgacacgg ctgcgcccag aaacgccgcc    480
   ttatgaagtc gttgaagtcg gaggccaggg tcgtgccatg gagacgccgc ggcggatgca    540
   gtctggctct ggcagcggct cctccccgct tccgcctcct tttccgacac ccggtcggca    600
   gctcgcccat tggccagttc aaaccccccc gggagccgct tgggtttcgc catcgaggcc    660
   cagcccctag cgtcacagag atgccccgcc ccgccagaag ggccgcccac ttccgggcca    720
   cgcgtcccgt caccaaggag acgtgctacg cgtccagcgg gcctggccgg gtctcgcccc    780
   ccattcaagg cctttcccgc tcggccctgt ttcccctgcc ttggatcctc ccggattccc    840
   ctcactcaaa ggagcgtacg gttccgacgc cctcccctcg gtacccacca ggaaactagg    900
   ctcacagagg acccatggaa ctagttattg ggagctgcac actgcctccg aaaatccagc    960
   cctgccccta gtccactatt aatgcggaga tggagtcggc ttctgcgcat tccccggcct   1020
   ctcccctgcc cacatcgcct ctgtcttggc cccaacagga gaaagggtcg gcattcccct   1080
   tgctgttctg ttcactacct tcctgcactt tttttttttt tttttttttt tttttttttt   1140
   ttttttttga gacagattct tgctctgttg cccaggctgg agtgcagtgg cgcgatctcg   1200
   gctcaccgca acctccgcct cccgggttca cgccattctc ctgcctgagc ctcccgagta   1260
   actgggacta ctggcgcccg ccacaacgcc cggctaattt ttttttgtat ttttagtaga   1320
   gacggggttt caccgtgttg ggcaggatag tctccatctc ctgacctcat gatccacccg   1380
   cctcggcctt ccaaagtgct tgtattacag gcttgagcct ccgcgcccgg tctactaccc   1440
   tcctgcactc tctatgccgc tctgtgtaca gacctattct gatgaggtcc ttgctggtcc   1500
   gccatcttct caattatgct tcactgtcag ccagtcaggg gaggtctgtc acgtggctca   1560
   taatgccgcc ctggaatctg ggagacctga accacctccg ggccaactgg gccacagtcc   1620
   cttgaccttt ctccagtgac cccgcagcac cttagccaca cagctccagg gtcacacaag   1680
   tgtttcacct gcaatcctaa agtcagactc gcaaacaaaa cctgcttttc cgcctcgctt   1740
   tcacagtata ctgcatactg aatttgttct gtaatgtcag ggagctcccc aatcccttga   1800
   tccattcatt gtctctctgt tgaataattg atagatttcc taatcctcat taaccattta   1860
   agactgcggt ggctcacgcc tgtagtccca gcactttggg aggctgaggc aggcggatca   1920
   cctaaggtca gaagttcaag accagactgc ccaacatagc gaaaccccgt ctctactaaa   1980
   aatacaaaaa ttaaccgggc atggtggtgt gtgcctgtaa tcccagctac tagggaggag   2040
   gaggcaggga gaattccttg aacctggaag gcggaggttg cagtgagcca aaatcaccac   2100
   tacacttcag cctgggtgac agagtgagac tcttgtctca aaaaaaaaaa aaaaaaaaaa   2160
   aaagtatata caggttcaag gaaaagagga caggaaaagg aggtcccggt cctcctgggg   2220
   caggcaggag atggttccca gaggtgatgg tattggaact gagccatgaa aggtgagtgg   2280
   aagttcgcca aacaagcagc aaagggaatt ctaaacagac tgcagtaggt acacaggtac   2340
   cacatgcagg tacaagagaa tactgcatat tctatgaagc atcatcactg cagtcccttg   2400
   aagtgttaca gggccagcga tgagagaaat cctgccaaag tcagcaggac cagactatgc   2460
   agcgtctgat aggccagcct aaggtgttgg acttcatccc tcagaagcct ggaaaaccat   2520
   gagagggttt tcagtatttc actctaggct gggcgctgtg gctcacacct gtaatcccag   2580
   cactttggga ggccgaggca ggttgatcac ttgaggtcaa gaatttgaga ccagcctggc   2640
   caacgtggtg aaaccccgtc tctactaaaa gtacaaaaat tagctgggcg tggcagccgg   2700
   cgactatagt cccagctact caggaggctg aggcaggaga atcacttaaa cccaggaggt   2760
   agaggttaca gtgagccaag attgcaccac tgcactccag cctgggcgac agagcgcgac   2820
   gccatctcaa aaaaaaaaaa aaaaaatagt ggccaagcgc aaagtggctc aagcctgtaa   2880
   taccagcact ttgagaggtt gaggcaggca gatcacctga ggtcaggagt tccagaccag   2940
   cctggcagac atgtggaaac ctcatctcta ctaaaaatac aaaaattatc cgggcgtggt   3000
   g                                                                   3001
<210> 7
   <211> 3001
   <212> DNA
   <213> human
<400> 7
   gagggcgtat aagtgattgt tatgtggagg ttcgaggaga ggacgaggcg gtcggggcga      60
   tgtcgtcgac gggtagtaga gcgagggaga gtttttgtcg gcggagagaa gggacggtta     120
   tttattagta ggtaggaggg tatcggtttt tgtttataga gagaaaggtg aagttttgtt     180
   cgcgggcggt agaggagggg tagagtttcg tggtagttaa gagaggaagg aggcggtcga     240
   tcgttcgtta gcgggaggag gagaggtttc gcgtcggtgg gcggggcggg ggtttaggtt     300
   ttgataggag ggggacgagc gtaacgcggc ggggaattta ggattcgacg gtttcgcggg      360
   gtcgatcgcg tcgggggtgc gaaggcgacg gtttcgttcg cggtgtttcg gagggcgttg     420
   gatttcgttt gtcggcggtt ttttattagg agcgatacgg ttgcgtttag aaacgtcgtt     480
   ttatgaagtc gttgaagtcg gaggttaggg tcgtgttatg gagacgtcgc ggcggatgta     540
   gtttggtttt ggtagcggtt ttttttcgtt ttcgtttttt ttttcgatat tcggtcggta     600
   gttcgtttat tggttagttt aaattttttc gggagtcgtt tgggtttcgt tatcgaggtt     660
   tagtttttag cgttatagag atgtttcgtt tcgttagaag ggtcgtttat tttcgggtta      720
   cgcgtttcgt tattaaggag acgtgttacg cgtttagcgg gtttggtcgg gtttcgtttt     780
   ttatttaagg tttttttcgt tcggttttgt tttttttgtt ttggattttt tcggattttt      840
   tttatttaaa ggagcgtacg gtttcgacgt ttttttttcg gtatttatta ggaaattagg    900
   tttatagagg atttatggaa ttagttattg ggagttgtat attgttttcg aaaatttagt     960
   tttgttttta gtttattatt aatgcggaga tggagtcggt ttttgcgtat ttttcggttt    1020
   tttttttgtt tatatcgttt ttgttttggt tttaatagga gaaagggtcg gtattttttt    1080
   tgttgttttg tttattattt ttttgtattt tttttttttt tttttttttt tttttttttt    1140
   ttttttttga gatagatttt tgttttgttg tttaggttgg agtgtagtgg cgcgatttcg    1200
   gtttatcgta attttcgttt ttcgggttta cgttattttt ttgtttgagt ttttcgagta    1260
   attgggatta ttggcgttcg ttataacgtt cggttaattt ttttttgtat ttttagtaga    1320
   gacggggttt tatcgtgttg ggtaggatag tttttatttt ttgattttat gatttattcg    1380
   tttcggtttt ttaaagtgtt tgtattatag gtttgagttt tcgcgttcgg tttattattt    1440
   ttttgtattt tttatgtcgt tttgtgtata gatttatttt gatgaggttt ttgttggttc    1500
   gttatttttt taattatgtt ttattgttag ttagttaggg gaggtttgtt acgtggttta    1560
   taatgtcgtt ttggaatttg ggagatttga attattttcg ggttaattgg gttatagttt    1620
   tttgattttt ttttagtgat ttcgtagtat tttagttata tagttttagg gttatataag    1680
   tgttttattt gtaattttaa agttagattc gtaaataaaa tttgtttttt cgtttcgttt    1740
   ttatagtata ttgtatattg aatttgtttt gtaatgttag ggagtttttt aattttttga   1800
   tttatttatt gtttttttgt tgaataattg atagattttt taatttttat taattattta     1860
   agattgcggt ggtttacgtt tgtagtttta gtattttggg aggttgaggt aggcggatta    1920
   tttaaggtta gaagtttaag attagattgt ttaatatagc gaaatttcgt ttttattaaa    1980
   aatataaaaa ttaatcgggt atggtggtgt gtgtttgtaa ttttagttat tagggaggag    2040
   gaggtaggga gaattttttg aatttggaag gcggaggttg tagtgagtta aaattattat   2100
   tatattttag tttgggtgat agagtgagat ttttgtttta aaaaaaaaaa aaaaaaaaaa    2160
   aaagtatata taggtttaag gaaaagagga taggaaaagg aggtttcggt ttttttgggg    2220
   taggtaggag atggttttta gaggtgatgg tattggaatt gagttatgaa aggtgagtgg    2280
   aagttcgtta aataagtagt aaagggaatt ttaaatagat tgtagtaggt atataggtat    2340
   tatatgtagg tataagagaa tattgtatat tttatgaagt attattattg tagttttttg  2400
   aagtgttata gggttagcga tgagagaaat tttgttaaag ttagtaggat tagattatgt    2460
   agcgtttgat aggttagttt aaggtgttgg attttatttt ttagaagttt ggaaaattat    2520
   gagagggttt ttagtatttt attttaggtt gggcgttgtg gtttatattt gtaattttag    2580
   tattttggga ggtcgaggta ggttgattat ttgaggttaa gaatttgaga ttagtttggt    2640
   taacgtggtg aaatttcgtt tttattaaaa gtataaaaat tagttgggcg tggtagtcgg    2700
   cgattatagt tttagttatt taggaggttg aggtaggaga attatttaaa tttaggaggt    2760
   agaggttata gtgagttaag attgtattat tgtattttag tttgggcgat agagcgcgac    2820
   gttattttaa aaaaaaaaaa aaaaaatagt ggttaagcgt aaagtggttt aagtttgtaa    2880
   tattagtatt ttgagaggtt gaggtaggta gattatttga ggttaggagt tttagattag    2940
   tttggtagat atgtggaaat tttattttta ttaaaaatat aaaaattatt cgggcgtggt    3000
   g                                                                    3001
<210> 8
   <211> 3001
   <212> DNA
   <213> human
<400> 8
<210> 9
   <211> 3001
   <212> DNA
   <213> human
<400> 9
   tattacgttc ggataatttt tgtattttta gtagagatga ggtttttata tgtttgttag      60
   gttggtttgg aatttttgat tttaggtgat ttgtttgttt taatttttta aagtgttggt      120
   attataggtt tgagttattt tgcgtttggt tattattttt tttttttttt tttgagatgg      180
   cgtcgcgttt tgtcgtttag gttggagtgt agtggtgtaa ttttggttta ttgtaatttt     240
   tattttttgg gtttaagtga tttttttgtt ttagtttttt gagtagttgg gattatagtc      300
   gtcggttgtt acgtttagtt aatttttgta tttttagtag agacggggtt ttattacgtt     360
   ggttaggttg gttttaaatt tttgatttta agtgattaat ttgtttcggt tttttaaagt     420
   gttgggatta taggtgtgag ttatagcgtt tagtttagag tgaaatattg aaaatttttt     480
   tatggttttt taggtttttg agggatgaag tttaatattt taggttggtt tattagacgt      540
   tgtatagttt ggttttgttg attttggtag gatttttttt atcgttggtt ttgtaatatt     600
   ttaagggatt gtagtgatga tgttttatag aatatgtagt atttttttgt atttgtatgt     660
   ggtatttgtg tatttattgt agtttgttta gaattttttt tgttgtttgt ttggcgaatt     720
   tttatttatt ttttatggtt tagttttaat attattattt ttgggaatta ttttttgttt     780
   gttttaggag gatcgggatt tttttttttt gttttttttt ttttgaattt gtatatattt       840
   tttttttttt tttttttttt ttgagataag agttttattt tgttatttag gttgaagtgt      900
   agtggtgatt ttggtttatt gtaattttcg ttttttaggt ttaaggaatt ttttttgttt      960
   tttttttttt agtagttggg attataggta tatattatta tgttcggtta atttttgtat     1020
   ttttagtaga gacggggttt cgttatgttg ggtagtttgg ttttgaattt ttgattttag     1080
   gtgattcgtt tgttttagtt ttttaaagtg ttgggattat aggcgtgagt tatcgtagtt     1140
   ttaaatggtt aatgaggatt aggaaattta ttaattattt aatagagaga taatgaatgg     1200
   attaagggat tggggagttt tttgatatta tagaataaat ttagtatgta gtatattgtg     1260
   aaagcgaggc ggaaaagtag gttttgtttg cgagtttgat tttaggattg taggtgaaat     1320
   atttgtgtga ttttggagtt gtgtggttaa ggtgttgcgg ggttattgga gaaaggttaa     1380
   gggattgtgg tttagttggt tcggaggtgg tttaggtttt ttagatttta gggcggtatt     1440
   atgagttacg tgatagattt tttttgattg gttgatagtg aagtataatt gagaagatgg     1500
   cggattagta aggattttat tagaataggt ttgtatatag agcggtatag agagtgtagg     1560
   agggtagtag atcgggcgcg gaggtttaag tttgtaatat aagtattttg gaaggtcgag     1620
   gcgggtggat tatgaggtta ggagatggag attattttgt ttaatacggt gaaatttcgt     1680
   ttttattaaa aatataaaaa aaaattagtc gggcgttgtg gcgggcgtta gtagttttag     1740
   ttattcggga ggtttaggta ggagaatggc gtgaattcgg gaggcggagg ttgcggtgag     1800
   tcgagatcgc gttattgtat tttagtttgg gtaatagagt aagaatttgt tttaaaaaaa     1860
   aaaaaaaaaa aaaaaaaaaa aaaaaaaaaa aaagtgtagg aaggtagtga atagaatagt     1920
   aaggggaatg tcgatttttt tttttgttgg ggttaagata gaggcgatgt gggtagggga     1980
   gaggtcgggg aatgcgtaga agtcgatttt attttcgtat taatagtgga ttaggggtag     2040
   ggttggattt tcggaggtag tgtgtagttt ttaataatta gttttatggg ttttttgtga     2100
   gtttagtttt ttggtgggta tcgaggggag ggcgtcggaa tcgtacgttt ttttgagtga     2160
   ggggaattcg ggaggattta aggtagggga aatagggtcg agcgggaaag gttttgaatg     2220
   gggggcgaga ttcggttagg ttcgttggac gcgtagtacg tttttttggt gacgggacgc     2280
   gtggttcgga agtgggcggt ttttttggcg gggcggggta tttttgtgac gttaggggtt     2340
   gggtttcgat ggcgaaattt aagcggtttt cgggggggtt tgaattggtt aatgggcgag    2400
   ttgtcgatcg ggtgtcggaa aaggaggcgg aagcggggag gagtcgttgt tagagttaga     2460
   ttgtattcgt cgcggcgttt ttatggtacg attttggttt tcgattttaa cgattttata     2520
   aggcggcgtt tttgggcgta gtcgtgtcgt ttttggtgag aggtcgtcgg taggcgggat     2580
   ttagcgtttt tcggggtatc gcgggcgaga tcgtcgtttt cgtattttcg gcgcggtcga     2640
   tttcgcgggg tcgtcgggtt ttgggttttt cgtcgcgttg cgttcgtttt ttttttgtta    2700
   gaatttgggt tttcgtttcg tttatcggcg cggggttttt ttttttttcg ttaacgggcg    2760
   gtcggtcgtt tttttttttt tttggttatt acggggtttt gttttttttt tgtcgttcgc      2820
   gggtagggtt ttattttttt ttttatgggt agaaatcggt atttttttgt ttgttggtgg     2880
   gtggtcgttt ttttttttcg tcgatagagg ttttttttcg ttttgttgtt cgtcggcggt     2940
   atcgtttcga tcgtttcgtt tttttttcgg gtttttatat ggtagttatt tgtacgtttt     3000
   t                                                                     3001
<210> 10
   <211> 3001
   <212> DNA
   <213> human
<400> 10
   tattatgttt ggataatttt tgtattttta gtagagatga ggtttttata tgtttgttag      60
   gttggtttgg aatttttgat tttaggtgat ttgtttgttt taatttttta aagtgttggt      120
   attataggtt tgagttattt tgtgtttggt tattattttt tttttttttt tttgagatgg      180
   tgttgtgttt tgttgtttag gttggagtgt agtggtgtaa ttttggttta ttgtaatttt      240
   tattttttgg gtttaagtga tttttttgtt ttagtttttt gagtagttgg gattatagtt      300
   gttggttgtt atgtttagtt aatttttgta tttttagtag agatggggtt ttattatgtt      360
   ggttaggttg gttttaaatt tttgatttta agtgattaat ttgttttggt tttttaaagt      420
   gttgggatta taggtgtgag ttatagtgtt tagtttagag tgaaatattg aaaatttttt      480
   tatggttttt taggtttttg agggatgaag tttaatattt taggttggtt tattagatgt      540
   tgtatagttt ggttttgttg attttggtag gatttttttt attgttggtt ttgtaatatt      600
   ttaagggatt gtagtgatga tgttttatag aatatgtagt atttttttgt atttgtatgt      660
   ggtatttgtg tatttattgt agtttgttta gaattttttt tgttgtttgt ttggtgaatt      720
   tttatttatt ttttatggtt tagttttaat attattattt ttgggaatta ttttttgttt      780
   gttttaggag gattgggatt tttttttttt gttttttttt ttttgaattt gtatatattt      840
   tttttttttt tttttttttt ttgagataag agttttattt tgttatttag gttgaagtgt      900
   agtggtgatt ttggtttatt gtaatttttg ttttttaggt ttaaggaatt ttttttgttt      960
   tttttttttt agtagttggg attataggta tatattatta tgtttggtta atttttgtat     1020
   ttttagtaga gatggggttt tgttatgttg ggtagtttgg ttttgaattt ttgattttag     1080
   gtgatttgtt tgttttagtt ttttaaagtg ttgggattat aggtgtgagt tattgtagtt     1140
   ttaaatggtt aatgaggatt aggaaattta ttaattattt aatagagaga taatgaatgg     1200
   attaagggat tggggagttt tttgatatta tagaataaat ttagtatgta gtatattgtg     1260
   aaagtgaggt ggaaaagtag gttttgtttg tgagtttgat tttaggattg taggtgaaat     1320
   atttgtgtga ttttggagtt gtgtggttaa ggtgttgtgg ggttattgga gaaaggttaa     1380
   gggattgtgg tttagttggt ttggaggtgg tttaggtttt ttagatttta gggtggtatt     1440
   atgagttatg tgatagattt tttttgattg gttgatagtg aagtataatt gagaagatgg     1500
   tggattagta aggattttat tagaataggt ttgtatatag agtggtatag agagtgtagg     1560
   agggtagtag attgggtgtg gaggtttaag tttgtaatat aagtattttg gaaggttgag     1620
   gtgggtggat tatgaggtta ggagatggag attattttgt ttaatatggt gaaattttgt    1680
   ttttattaaa aatataaaaa aaaattagtt gggtgttgtg gtgggtgtta gtagttttag     1740
   ttatttggga ggtttaggta ggagaatggt gtgaatttgg gaggtggagg ttgtggtgag     1800
   ttgagattgt gttattgtat tttagtttgg gtaatagagt aagaatttgt tttaaaaaaa     1860
   aaaaaaaaaa aaaaaaaaaa aaaaaaaaaa aaagtgtagg aaggtagtga atagaatagt     1920
   aaggggaatg ttgatttttt tttttgttgg ggttaagata gaggtgatgt gggtagggga     1980
   gaggttgggg aatgtgtaga agttgatttt atttttgtat taatagtgga ttaggggtag     2040
   ggttggattt ttggaggtag tgtgtagttt ttaataatta gttttatggg ttttttgtga   2100
   gtttagtttt ttggtgggta ttgaggggag ggtgttggaa ttgtatgttt ttttgagtga     2160
   ggggaatttg ggaggattta aggtagggga aatagggttg agtgggaaag gttttgaatg     2220
   gggggtgaga tttggttagg tttgttggat gtgtagtatg tttttttggt gatgggatgt     2280
   gtggtttgga agtgggtggt ttttttggtg gggtggggta tttttgtgat gttaggggtt     2340
   gggttttgat ggtgaaattt aagtggtttt tgggggggtt tgaattggtt aatgggtgag     2400
   ttgttgattg ggtgttggaa aaggaggtgg aagtggggag gagttgttgt tagagttaga     2460
   ttgtatttgt tgtggtgttt ttatggtatg attttggttt ttgattttaa tgattttata     2520
   aggtggtgtt tttgggtgta gttgtgttgt ttttggtgag aggttgttgg taggtgggat     2580
   ttagtgtttt ttggggtatt gtgggtgaga ttgttgtttt tgtatttttg gtgtggttga     2640
   ttttgtgggg ttgttgggtt ttgggttttt tgttgtgttg tgtttgtttt ttttttgtta    2700
   gaatttgggt ttttgttttg tttattggtg tggggttttt tttttttttg ttaatgggtg     2760
   gttggttgtt tttttttttt tttggttatt atggggtttt gttttttttt tgttgtttgt      2820
   gggtagggtt ttattttttt ttttatgggt agaaattggt atttttttgt ttgttggtgg     2880
   gtggttgttt tttttttttg ttgatagagg tttttttttg ttttgttgtt tgttggtggt     2940
   attgttttga ttgttttgtt ttttttttgg gtttttatat ggtagttatt tgtatgtttt     3000
   t                                                                     3001
<210> 11
   <211> 50
   <212> DNA
   <213> human
<400> 11
   ctactttaga cagacaggcc actggtgaaa ctatgctaaa caagtgctcg 50
<210> 12
   <211> 50
   <212> DNA
   <213> human
<400> 12
   ttattttaga tagataggtt attggtgaaa ttatgttaaa taagtgttcg 50
<210> 13
   <211> 50
   <212> DNA
   <213> human
<400> 13
   ttattttaga tagataggtt attggtgaaa ttatgttaaa taagtgtttg 50
<210> 14
   <211> 50
   <212> DNA
   <213> human
<400> 14
   cgagtatttg tttagtatag ttttattagt ggtttgtttg tttaaagtag 50
<210> 15
   <211> 50
   <212> DNA
   <213> human
<400> 15
   tgagtatttg tttagtatag ttttattagt ggtttgtttg tttaaagtag 50
<210> 16
   <211> 3001
   <212> DNA
   <213> human
<400> 16
   tgttttccta agcctcatta atgaacagca gctaattctg aagatttgat tttttactca 60
   gttctgcctc tctcagattc ctatggccac aggtaatggt tttgaacttc aaaattactt 120
   ctttctgagc ttctccagcg agaaaaataa aatcctcaaa tgtgaaccca ctcactcatc 180
   ttttgaatag tagtcagttc cctagagcaa ctcattttaa gggaggattt aaatccatgc 240
   ctgtcccccg tacattggga tgcactggaa agacattttc tccctcaaca ttttttcttt 300
   tacttggaaa tcattaagat aatgtttttt cctcttgcat gttaattggg taggttttgc 360
   atgccttgca ttttctttct caacgtttta gattattaac actccttaaa aaaagtctat 420
   cacactattc ttaatgctgt catcaagagt aaaaaattta tcttagaaaa aagggtggta 480
   ctgaaatgtt actcaatatg atatatgcag caatggaaga cgtgaatttc tggaggtctc 540
   agtagcagag atcacttaat tacccttgtg gaaatttatc cttgtgaaaa aaggaggaac 600
   tatttagtaa cttcatctgt ttaatgggat aacaagacct acctcataaa ttaactatga 660
   ggattaaatg agctaataat gtaaagactc aataaatagt ccatgttgcc tatttttggt 720
   atcattagcc ttagtaaaga tactgaatgt tgtacatggc agttgggact tagcgaaaat 780
   ttgggctggc ctggatcatg aaaagaagct gctatcttct tacctccaga gagtcatcag 840
   cattcaccat ccaacagtca gtccaggtgg ccacaatcaa aaacccagca gagaaaaagg 900
   caaagaagca agcgatgtat tgaagaagat ccctcattgt ggcaagcagc ccatcaggat 960
   ggcaacatgg gcagacacca gccctggccc cacttaagtg gcgggtgtca gtcttctgtg 1020
   tttctggtac ctggcaatgt gaaaatactg gtcttttact ttttctcacg ccctgctgca 1080
   ggtgtcttga gttgcagtaa gaataataca aacaggctgt aaccaacagt ggggtcctgg 1140
   aggtcattat ccgaagcaac cagcctccaa tcagaaggca ggtcagtgca agggctgagt 1200
   gtgtttcggg tgggggagag aggagttctg taacgttttc tgacccaact gtgggctagt 1260
   ggtggtcacc agaccaagtg ccagtgccaa gacgggggaa ggaggaaggg tcccacccca 1320
   tgctggaacc tgtaaactga tctttcttag gccctctagg gagagtgagc tattctcctc 1380
   cccactcgga agaaggctac acggttgact gagtgttatt ttattgttag gactatttta 1440
   atattcgccg actactttag acagacaggc cactggtgaa actatgctaa acaagtgctc 1500
   gctcaaggga gtctgcattc caaagtgttg cgtagaacag ccccatgaag gacttgtagc 1560
   acctgggcag tagggacagc caaggctcct aacctttcag catcgtagag tgggaacaag    1620
   tgtgcatttt aatgcagaca gctctgggtg gaaattcttg ctctgaattt atggtctgag    1680
   agcccttggg ctgtagtttc tgaaaatgca tgtattaata ttttctttat ataatgcttg    1740
   ttcttcatat gaggaataaa agatattgtg cctgtaagtc gcttagaata gcatctgtca    1800
   gtaatagcta aatggaaaat attaacatga ctaagaattt cctatattat tacttttccc    1860
   tcatggacca atgtttgaaa acgcgtctaa tgaagaaaaa aaaactgtta tattaggaag    1920
   aaagtgtgtt tcttttttgt aaaaaaaagt ttttaatatg taactggggc aactggaatt    1980
   ttatctgtat ctgaataggt atccttaatg ggtctacaaa ttccataacc aatactcttt    2040
   aatatgcttt aaaaagcaca tatatcaaca ttttctcttc atcaggtaga cttctatata    2100
   ctaaaataaa aattttgaat gagaacagta aaaacaaagt ggtgattctt aatgtaggag    2160
   agaatgtcaa aggggagaca gagtgagaga aagaaattgg gagggagaga aagaaaggag    2220
   cagagaaaag gccagaaaga ggtaattact ctggcatgaa tcagctacta agattcttat    2280
   tttaccaaca gcttataaaa ggattacttt tttccatttg aaaattattt gatcataata    2340
   tacagtataa aaggctaaaa tcagtattga taaaccattt ttcaaaatat attacaataa    2400
   taaacttttt gtggctaaac tttgtttttt ctaattttaa ttcattttat tcaagtatat    2460
   aaaacgtaaa tttaaatgta aaagctgcat ggaaaaccat aggtggaata taagcttaat    2520
   tatttctata catagggtct acagtaccac ccctgctatc cagaatttcc cttttgaggc    2580
   ttcagttacc agtggttaat catggtctgc aaaaattaca tgggaaattt catagataaa    2640
   caattcatca gtggtaaatt gaacactgtt ctgaatagcg tgatgaaatc tcaagccatc    2700
   ccacttcaca tgggatatga atcatccctt tggccagcat ttccacattg tatgcactac    2760
   ccacccctta gtcacttaat agctgcctcg gtgatctgac ctactgttgc gtatagtgtg    2820
   tgtaggatcc agtactgtcc ttcgtttctg acatccactg agggtctttg aacatattcc    2880
   tcaaggagaa ggggtgatta ctgtacacat tagcgatcgt tataactatc tgcgaggtac    2940
   attacagatg cttttatttt acttattttg tttaacagtt ggaaaaaaaa tacctgtttt    3000
   t                                                                    3001
<210> 17
   <211> 3001
   <212> DNA
   <213> human
<400> 17
   tgttttttta agttttatta atgaatagta gttaattttg aagatttgat tttttattta     60
   gttttgtttt ttttagattt ttatggttat aggtaatggt tttgaatttt aaaattattt    120
   ttttttgagt ttttttagcg agaaaaataa aatttttaaa tgtgaattta tttatttatt    180
   ttttgaatag tagttagttt tttagagtaa tttattttaa gggaggattt aaatttatgt    240
   ttgtttttcg tatattggga tgtattggaa agatattttt ttttttaata tttttttttt     300
   tatttggaaa ttattaagat aatgtttttt ttttttgtat gttaattggg taggttttgt     360
   atgttttgta tttttttttt taacgtttta gattattaat attttttaaa aaaagtttat    420
   tatattattt ttaatgttgt tattaagagt aaaaaattta ttttagaaaa aagggtggta    480
   ttgaaatgtt atttaatatg atatatgtag taatggaaga cgtgaatttt tggaggtttt    540
   agtagtagag attatttaat tatttttgtg gaaatttatt tttgtgaaaa aaggaggaat    600
   tatttagtaa ttttatttgt ttaatgggat aataagattt attttataaa ttaattatga    660
   ggattaaatg agttaataat gtaaagattt aataaatagt ttatgttgtt tatttttggt    720
   attattagtt ttagtaaaga tattgaatgt tgtatatggt agttgggatt tagcgaaaat    780
   ttgggttggt ttggattatg aaaagaagtt gttatttttt tatttttaga gagttattag    840
   tatttattat ttaatagtta gtttaggtgg ttataattaa aaatttagta gagaaaaagg    900
   taaagaagta agcgatgtat tgaagaagat tttttattgt ggtaagtagt ttattaggat    960
   ggtaatatgg gtagatatta gttttggttt tatttaagtg gcgggtgtta gttttttgtg   1020
   tttttggtat ttggtaatgt gaaaatattg gttttttatt tttttttacg ttttgttgta   1080
   ggtgttttga gttgtagtaa gaataatata aataggttgt aattaatagt ggggttttgg   1140
   aggttattat tcgaagtaat tagtttttaa ttagaaggta ggttagtgta agggttgagt   1200
   gtgtttcggg tgggggagag aggagttttg taacgttttt tgatttaatt gtgggttagt   1260
   ggtggttatt agattaagtg ttagtgttaa gacgggggaa ggaggaaggg ttttatttta   1320
   tgttggaatt tgtaaattga ttttttttag gttttttagg gagagtgagt tatttttttt   1380
   tttattcgga agaaggttat acggttgatt gagtgttatt ttattgttag gattatttta   1440
   atattcgtcg attattttag atagataggt tattggtgaa attatgttaa ataagtgttc   1500
   gtttaaggga gtttgtattt taaagtgttg cgtagaatag ttttatgaag gatttgtagt   1560
   atttgggtag tagggatagt taaggttttt aattttttag tatcgtagag tgggaataag   1620
   tgtgtatttt aatgtagata gttttgggtg gaaatttttg ttttgaattt atggtttgag   1680
   agtttttggg ttgtagtttt tgaaaatgta tgtattaata ttttttttat ataatgtttg   1740
   ttttttatat gaggaataaa agatattgtg tttgtaagtc gtttagaata gtatttgtta    1800
   gtaatagtta aatggaaaat attaatatga ttaagaattt tttatattat tatttttttt   1860
   ttatggatta atgtttgaaa acgcgtttaa tgaagaaaaa aaaattgtta tattaggaag   1920
   aaagtgtgtt ttttttttgt aaaaaaaagt ttttaatatg taattggggt aattggaatt   1980
   ttatttgtat ttgaataggt atttttaatg ggtttataaa ttttataatt aatatttttt   2040
   aatatgtttt aaaaagtata tatattaata tttttttttt attaggtaga tttttatata   2100
   ttaaaataaa aattttgaat gagaatagta aaaataaagt ggtgattttt aatgtaggag   2160
   agaatgttaa aggggagata gagtgagaga aagaaattgg gagggagaga aagaaaggag   2220
   tagagaaaag gttagaaaga ggtaattatt ttggtatgaa ttagttatta agatttttat   2280
   tttattaata gtttataaaa ggattatttt tttttatttg aaaattattt gattataata   2340
   tatagtataa aaggttaaaa ttagtattga taaattattt tttaaaatat attataataa   2400
   taaatttttt gtggttaaat tttgtttttt ttaattttaa tttattttat ttaagtatat   2460
   aaaacgtaaa tttaaatgta aaagttgtat ggaaaattat aggtggaata taagtttaat   2520
   tatttttata tatagggttt atagtattat ttttgttatt tagaattttt tttttgaggt   2580
   tttagttatt agtggttaat tatggtttgt aaaaattata tgggaaattt tatagataaa   2640
   taatttatta gtggtaaatt gaatattgtt ttgaatagcg tgatgaaatt ttaagttatt 2700
   ttattttata tgggatatga attatttttt tggttagttt ttatatattg tatgtattat 2760
   ttatttttta gttatttaat agttgtttcg gtgatttgat ttattgttgc gtatagtgtg   2820
   tgtaggattt agtattgttt ttcgtttttg atatttattg agggtttttg aatatatttt   2880
   ttaaggagaa ggggtgatta ttgtatatat tagcgatcgt tataattatt tgcgaggtat   2940
   attatagatg tttttatttt atttattttg tttaatagtt ggaaaaaaaa tatttgtttt   3000
   t                                                                   3001
<210> 18
   <211> 3001
   <212> DNA
   <213> human
<400> 18
   tgttttttta agttttatta atgaatagta gttaattttg aagatttgat tttttattta 60
   gttttgtttt ttttagattt ttatggttat aggtaatggt tttgaatttt aaaattattt 120
   ttttttgagt ttttttagtg agaaaaataa aatttttaaa tgtgaattta tttatttatt 180
   ttttgaatag tagttagttt tttagagtaa tttattttaa gggaggattt aaatttatgt 240
   ttgttttttg tatattggga tgtattggaa agatattttt ttttttaata tttttttttt 300
   tatttggaaa ttattaagat aatgtttttt ttttttgtat gttaattggg taggttttgt 360
   atgttttgta tttttttttt taatgtttta gattattaat atttttaaaa aaaagtttat 420
   tatattattt ttaatgttgt tattaagagt aaaaaattta ttttagaaaa aagggtggta 480
   ttgaaatgtt atttaatatg atatatgtag taatggaaga tgtgaatttt tggaggtttt 540
   agtagtagag attatttaat tatttttgtg gaaatttatt tttgtgaaaa aaggaggaat 600
   tatttagtaa ttttatttgt ttaatgggat aataagattt attttataaa ttaattatga 660
   ggattaaatg agttaataat gtaaagattt aataaatagt ttatgttgtt tatttttggt 720
   attattagtt ttagtaaaga tattgaatgt tgtatatggt agttgggatt tagtgaaaat 780
   ttgggttggt ttggattatg aaaagaagtt gttatttttt tatttttaga gagttattag 840
   tatttattat ttaatagtta gtttaggtgg ttataattaa aaatttagta gagaaaaagg 900
   taaagaagta agtgatgtat tgaagaagat tttttattgt ggtaagtagt ttattaggat 960
   ggtaatatgg gtagatatta gttttggttt tatttaagtg gtgggtgtta gttttttgtg 1020
   tttttggtat ttggtaatgt gaaaatattg gttttttatt tttttttatg ttttgttgta 1080
   ggtgttttga gttgtagtaa gaataatata aataggttgt aattaatagt ggggttttgg 1140
   aggttattat ttgaagtaat tagtttttaa ttagaaggta ggttagtgta agggttgagt 1200
   gtgttttggg tgggggagag aggagttttg taatgttttt tgatttaatt gtgggttagt 1260
   ggtggttatt agattaagtg ttagtgttaa gatgggggaa ggaggaaggg ttttatttta 1320
   tgttggaatt tgtaaattga ttttttttag gttttttagg gagagtgagt tatttttttt 1380
   tttatttgga agaaggttat atggttgatt gagtgttatt ttattgttag gattatttta 1440
   atatttgttg attattttag atagataggt tattggtgaa attatgttaa ataagtgttt 1500
   gtttaaggga gtttgtattt taaagtgttg tgtagaatag ttttatgaag gatttgtagt 1560
   atttgggtag tagggatagt taaggttttt aattttttag tattgtagag tgggaataag 1620
   tgtgtatttt aatgtagata gttttgggtg gaaatttttg ttttgaattt atggtttgag 1680
   agtttttggg ttgtagtttt tgaaaatgta tgtattaata ttttttttat ataatgtttg 1740
   ttttttatat gaggaataaa agatattgtg tttgtaagtt gtttagaata gtatttgtta 1800
   gtaatagtta aatggaaaat attaatatga ttaagaattt tttatattat tatttttttt 1860
   ttatggatta atgtttgaaa atgtgtttaa tgaagaaaaa aaaattgtta tattaggaag 1920
   aaagtgtgtt ttttttttgt aaaaaaaagt ttttaatatg taattggggt aattggaatt 1980
   ttatttgtat ttgaataggt atttttaatg ggtttataaa ttttataatt aatatttttt 2040
   aatatgtttt aaaaagtata tatattaata tttttttttt attaggtaga tttttatata 2100
   ttaaaataaa aattttgaat gagaatagta aaaataaagt ggtgattttt aatgtaggag 2160
   agaatgttaa aggggagata gagtgagaga aagaaattgg gagggagaga aagaaaggag 2220
   tagagaaaag gttagaaaga ggtaattatt ttggtatgaa ttagttatta agatttttat 2280
   tttattaata gtttataaaa ggattatttt tttttatttg aaaattattt gattataata 2340
   tatagtataa aaggttaaaa ttagtattga taaattattt tttaaaatat attataataa 2400
   taaatttttt gtggttaaat tttgtttttt ttaattttaa tttattttat ttaagtatat 2460
   aaaatgtaaa tttaaatgta aaagttgtat ggaaaattat aggtggaata taagtttaat 2520
   tatttttata tatagggttt atagtattat ttttgttatt tagaattttt tttttgaggt 2580
   tttagttatt agtggttaat tatggtttgt aaaaattata tgggaaattt tatagataaa 2640
   taatttatta gtggtaaatt gaatattgtt ttgaatagtg tgatgaaatt ttaagttatt 2700
   ttattttata tgggatatga attatttttt tggttagtat ttttatattg tatgtattat 2760
   ttatttttta gttatttaat agttgttttg gtgatttgat ttattgttgt gtatagtgtg 2820
   tgtaggattt agtattgttt tttgtttttg atatttattg agggtttttg aatatatttt 2880
   ttaaggagaa ggggtgatta ttgtatatat tagtgattgt tataattatt tgtgaggtat 2940
   attatagatg tttttatttt atttattttg tttaatagtt ggaaaaaaaa tatttgtttt 3000
   t 3001
<210> 19
   <211> 3001
   <212> DNA
   <213> human
<400> 19
   aaaaataggt attttttttt taattgttaa ataaaataag taaaataaaa gtatttgtaa      60
   tgtatttcgt agatagttat aacgatcgtt aatgtgtata gtaattattt tttttttttg     120
   aggaatatgt ttaaagattt ttagtggatg ttagaaacga aggatagtat tggattttat     180
   atatattata cgtaatagta ggttagatta tcgaggtagt tattaagtga ttaaggggtg     240
   ggtagtgtat ataatgtgga aatgttggtt aaagggatga tttatatttt atgtgaagtg     300
   ggatggtttg agattttatt acgttattta gaatagtgtt taatttatta ttgatgaatt     360
   gtttatttat gaaatttttt atgtaatttt tgtagattat gattaattat tggtaattga     420
   agttttaaaa gggaaatttt ggatagtagg ggtggtattg tagattttat gtatagaaat     480
   aattaagttt atattttatt tatggttttt tatgtagttt ttatatttaa atttacgttt     540
   tatatatttg aataaaatga attaaaatta gaaaaaataa agtttagtta taaaaagttt     600
   attattgtaa tatattttga aaaatggttt attaatattg attttagttt tttatattgt     660
   atattatgat taaataattt ttaaatggaa aaaagtaatt tttttataag ttgttggtaa     720
   aataagaatt ttagtagttg atttatgtta gagtaattat ttttttttgg tttttttttt     780
   gttttttttt tttttttttt tttaattttt tttttttatt ttgttttttt tttgatattt     840
   ttttttatat taagaattat tattttgttt ttattgtttt tatttaaaat ttttatttta     900
   gtatatagaa gtttatttga tgaagagaaa atgttgatat atgtgttttt taaagtatat     960
   taaagagtat tggttatgga atttgtagat ttattaagga tatttattta gatatagata    1020
   aaattttagt tgttttagtt atatattaaa aatttttttt tataaaaaag aaatatattt    1080
   tttttttaat ataatagttt tttttttttt attagacgcg tttttaaata ttggtttatg    1140
   agggaaaagt aataatatag gaaattttta gttatgttaa tattttttat ttagttatta    1200
   ttgatagatg ttattttaag cgatttatag gtataatatt ttttattttt tatatgaaga    1260
   ataagtatta tataaagaaa atattaatat atgtattttt agaaattata gtttaagggt    1320
   ttttagatta taaatttaga gtaagaattt ttatttagag ttgtttgtat taaaatgtat    1380
   atttgttttt attttacgat gttgaaaggt taggagtttt ggttgttttt attgtttagg    1440
   tgttataagt tttttatggg gttgttttac gtaatatttt ggaatgtaga tttttttgag    1500
   cgagtatttg tttagtatag ttttattagt ggtttgtttg tttaaagtag tcggcgaata    1560
   ttaaaatagt tttaataata aaataatatt tagttaatcg tgtagttttt tttcgagtgg    1620
   ggaggagaat agtttatttt ttttagaggg tttaagaaag attagtttat aggttttagt    1680
   atggggtggg attttttttt tttttttcgt tttggtattg gtatttggtt tggtgattat    1740
   tattagttta tagttgggtt agaaaacgtt atagaatttt tttttttttt attcgaaata    1800
   tatttagttt ttgtattgat ttgttttttg attggaggtt ggttgtttcg gataatgatt    1860
   tttaggattt tattgttggt tatagtttgt ttgtattatt tttattgtaa tttaagatat    1920
   ttgtagtagg gcgtgagaaa aagtaaaaga ttagtatttt tatattgtta ggtattagaa    1980
   atatagaaga ttgatattcg ttatttaagt ggggttaggg ttggtgtttg tttatgttgt    2040
   tattttgatg ggttgtttgt tataatgagg gatttttttt aatatatcgt ttgttttttt    2100
   gttttttttt ttgttgggtt tttgattgtg gttatttgga ttgattgttg gatggtgaat    2160
   gttgatgatt ttttggaggt aagaagatag tagttttttt ttatgattta ggttagttta   2220
   aattttcgtt aagttttaat tgttatgtat aatatttagt atttttatta aggttaatga    2280
   tattaaaaat aggtaatatg gattatttat tgagttttta tattattagt ttatttaatt    2340
   tttatagtta atttatgagg taggttttgt tattttatta aatagatgaa gttattaaat    2400
   agtttttttt ttttttataa ggataaattt ttataagggt aattaagtga tttttgttat    2460
   tgagattttt agaaatttac gttttttatt gttgtatata ttatattgag taatatttta   2520
   gtattatttt tttttttaag ataaattttt tatttttgat gatagtatta agaatagtgt    2580
   gatagatttt ttttaaggag tgttaataat ttaaaacgtt gagaaagaaa atgtaaggta    2640
   tgtaaaattt atttaattaa tatgtaagag gaaaaaatat tattttaatg atttttaagt    2700
   aaaagaaaaa atgttgaggg agaaaatgtt tttttagtgt attttaatgt acgggggata    2760
   ggtatggatt taaatttttt tttaaaatga gttgttttag ggaattgatt attatttaaa    2820
   agatgagtga gtgggtttat atttgaggat tttatttttt tcgttggaga agtttagaaa   2880
   gaagtaattt tgaagtttaa aattattatt tgtggttata ggaatttgag agaggtagaa    2940
   ttgagtaaaa aattaaattt ttagaattag ttgttgttta ttaatgaggt ttaggaaaat    3000
   a                                                                     3001
<210> 20
   <211> 3001
   <212> DNA
   <213> human
<400> 20
   aaaaataggt attttttttt taattgttaa ataaaataag taaaataaaa gtatttgtaa      60
   tgtattttgt agatagttat aatgattgtt aatgtgtata gtaattattt tttttttttg 120
   aggaatatgt ttaaagattt ttagtggatg ttagaaatga aggatagtat tggattttat 180
   atatattata tgtaatagta ggttagatta ttgaggtagt tattaagtga ttaaggggtg 240
   ggtagtgtat ataatgtgga aatgttggtt aaagggatga tttatatttt atgtgaagtg 300
   ggatggtttg agattttatt atgttattta gaatagtgtt taatttatta ttgatgaatt 360
   gtttatttat gaaatttttt atgtaatttt tgtagattat gattaattat tggtaattga 420
   agttttaaaa gggaaatttt ggatagtagg ggtggtattg tagattttat gtatagaaat 480
   aattaagttt atattttatt tatggttttt tatgtagttt ttatatttaa atttatgttt 540
   tatatatttg aataaaatga attaaaatta gaaaaaataa agtttagtta taaaaagttt 600
   attattgtaa tatattttga aaaatggttt attaatattg attttagttt tttatattgt 660
   atattatgat taaataattt ttaaatggaa aaaagtaatt tttttataag ttgttggtaa 720
   aataagaatt ttagtagttg atttatgtta gagtaattat ttttttttgg tttttttttt 780
   gttttttttt tttttttttt tttaattttt tttttttatt ttgttttttt tttgatattt 840
   ttttttatat taagaattat tattttgttt ttattgtttt tatttaaaat ttttatttta 900
   gtatatagaa gtttatttga tgaagagaaa atgttgatat atgtgttttt taaagtatat 960
   taaagagtat tggttatgga atttgtagat ttattaagga tatttattta gatatagata 1020
   aaattttagt tgttttagtt atatattaaa aatttttttt tataaaaaag aaatatattt 1080
   tttttttaat ataatagttt tttttttttt attagatgtg tttttaaata ttggtttatg 1140
   agggaaaagt aataatatag gaaattttta gttatgttaa tattttttat ttagttatta 1200
   ttgatagatg ttattttaag tgatttatag gtataatatt ttttattttt tatatgaaga 1260
   ataagtatta tataaagaaa atattaatat atgtattttt agaaattata gtttaagggt 1320
   ttttagatta taaatttaga gtaagaattt ttatttagag ttgtttgtat taaaatgtat 1380
   atttgttttt attttatgat gttgaaaggt taggagtttt ggttgttttt attgtttagg 1440
   tgttataagt tttttatggg gttgttttat gtaatatttt ggaatgtaga tttttttgag 1500
   tgagtatttg tttagtatag ttttattagt ggtttgtttg tttaaagtag ttggtgaata 1560
   ttaaaatagt tttaataata aaataatatt tagttaattg tgtagttttt ttttgagtgg 1620
   ggaggagaat agtttatttt ttttagaggg tttaagaaag attagtttat aggttttagt 1680
   atggggtggg attttttttt ttttttttgt tttggtattg gtatttggtt tggtgattat 1740
   tattagttta tagttgggtt agaaaatgtt atagaatttt tttttttttt atttgaaata 1800
   tatttagttt ttgtattgat ttgttttttg attggaggtt ggttgttttg gataatgatt 1860
   tttaggattt tattgttggt tatagtttgt ttgtattatt tttattgtaa tttaagatat 1920
   ttgtagtagg gtgtgagaaa aagtaaaaga ttagtatttt tatattgtta ggtattagaa 1980
   atatagaaga ttgatatttg ttatttaagt ggggttaggg ttggtgtttg tttatgttgt 2040
   tattttgatg ggttgtttgt tataatgagg gatttttttt aatatattgt ttgttttttt 2100
   gttttttttt ttgttgggtt tttgattgtg gttatttgga ttgattgttg gatggtgaat 2160
   gttgatgatt ttttggaggt aagaagatag tagttttttt ttatgattta ggttagttta 2220
   aatttttgtt aagttttaat tgttatgtat aatatttagt atttttatta aggttaatga 2280
   tattaaaaat aggtaatatg gattatttat tgagttttta tattattagt ttatttaatt 2340
   tttatagtta atttatgagg taggttttgt tattttatta aatagatgaa gttattaaat 2400
   agtttttttt ttttttataa ggataaattt ttataagggt aattaagtga tttttgttat 2460
   tgagattttt agaaatttat gttttttatt gttgtatata ttatattgag taatatttta 2520
   gtattatttt tttttttaag ataaattttt tatttttgat gatagtatta agaatagtgt 2580
   gatagatttt ttttaaggag tgttaataat ttaaaatgtt gagaaagaaa atgtaaggta 2640
   tgtaaaattt atttaattaa tatgtaagag gaaaaaatat tattttaatg atttttaagt 2700
   aaaagaaaaa atgttgaggg agaaaatgtt tttttagtgt attttaatgt atgggggata 2760
   ggtatggatt taaatttttt tttaaaatga gttgttttag ggaattgatt attatttaaa 2820
   agatgagtga gtgggtttat atttgaggat tttatttttt ttgttggaga agtttagaaa 2880
   gaagtaattt tgaagtttaa aattattatt tgtggttata ggaatttgag agaggtagaa 2940
   ttgagtaaaa aattaaattt ttagaattag ttgttgttta ttaatgaggt ttaggaaaat 3000
   a 3001
<210> 21
   <211> 50
   <212> DNA
   <213> human
<400> 21
   cgcctggcaa ggttccgcct gagcacactc tggcccagtg catatcctgg 50
<210> 22
   <211> 50
   <212> DNA
   <213> human
<400> 22
   cgtttggtaa ggtttcgttt gagtatattt tggtttagtg tatattttgg 50
<210> 23
   <211> 50
   <212> DNA
   <213> human
<400> 23
   tgtttggtaa ggttttgttt gagtatattt tggtttagtg tatattttgg 50
<210> 24
   <211> 50
   <212> DNA
   <213> human
<400> 24
   ttaggatatg tattgggtta gagtgtgttt aggcggaatt ttgttaggcg 50
<210> 25
   <211> 50
   <212> DNA
   <213> human
<400> 25
   ttaggatatg tattgggtta gagtgtgttt aggtggaatt ttgttaggtg 50
<210> 26
   <211> 3001
   <212> DNA
   <213> human
<400> 26
   attaccatca cctgcccata aagaaaaaca gcagcccagt tttagggagt gtattctatg     60
   agatgaactg gcctaccctt tgtctttctt tgattttttg tagatttggc cttttcagca    120
   tctgcttgac cagctgtagg ttgatcctct tgttcttcca catttgaatt tgaaactgtg    180
   agaacaattt cgtcacttct ttcatctcct gacattgttt ccacaaaaaa gagctgctcc    240
   tttgttcttt cagccaataa ctggttcttc tcctgctctt cctcgagcta ataaacaaca    300
   aatattcaaa tatgatacta ttgatttcag tataattaga cttttgcctg gatgaagcac    360
   agaatacaaa aagaaaagcc aaagtggaag attatgcatc acaatgttaa ttatttcgca    420
   tcagggttgt ggcagtgaaa cttaacactt ttttttaaaa cataatgcgc cttacactat    480
   tccaattagc accactttta aaactctttt ttaaaaaaga acctaagaac tctcaagatt    540
   ttcctgggaa gtatttgtac tgtctttaat ttgcaactgc aaaatacctt gtccagacat    600
   aatattgcat aaaagccgac ttgaattttt tctttttcat gtaaggggtt gtttattaaa    660
   accagctgta ccttgatcag tccagtagtt tcagccaggc tcaccgctaa cttactgtct    720
   tcactggcca ccatcacatt ctcctctaga gcgtggaact gcaacacctc catctcttgc    780
   ggggagtaca gctcttgctg gatactaatg gccacacact gctgcaggct ctgccggggc    840
   ccttcctcaa gccacagcaa cccagggcca ggctgttgca ccaccacctg caccccttct    900
   tgctgctgta tgctcagcaa gctcatatcc tgcaactcca cagcttcaga agtgaagtgc    960
   accgtctgca gggtcaggat gtacttctcg ctctcctccg agggggccag caccagctcc   1020
   acttcttcct ccaggacgct gtcctggaag gccccagagg tacgctcggc ctccaactca   1080
   ctagggctcc gatggtcttt ctctctgcac actccgtctt tttcctcctc cttcaggcct   1140
   ttttccggca tcaactcgag ttctttgatc ttggtgaatt gctcagaaag gacagagatc   1200
   tcagtggctg ccataatgac ttggcctgtt tgaaaaataa gcaagcggtt tccatagggg   1260
   ggagaagggg gtggtatgag gagggatgcg gggggtgctg gaacctagca ggctttggag   1320
   ttgaaacaag gtctggcctc aaaaggtttt ggacttagta gggtcagaac aatgctgatc   1380
   tggcttgggt ctaggagggg gtcaaggcta agcaggattt aggctaaagc aggattttgt   1440
   acaaaaccct agaacgaaca ggtttgggcc tcagcaggct tagggccagc acacatcctg   1500
   cgcctggcaa ggttccgcct gagcacactc tggcccagtg catatcctgg gcctagcaag   1560
   gttaggcctg agccggttca gtcagtgaac atcctgggcc tagtaaggtt tgggcccagc   1620
   aggctctcgg ccagtgcata tcctgggcct agcaagtttc aggtccaagc aggccccggg   1680
   ccagtgcaca cccggcgccc agcgaggttc gggcccgagc aggcattccc ctcgtgcacg   1740
   gttctagacc tagcaccctc agagccaggc aggctttggg cctagtggcc tcgagcccac   1800
   ccagacttgg ccaagcagcc ctcggccaga ccaagcacac tccctcggag gcctggcagg   1860
   gcccctgctt taccctgccc cccacgcccc gccccgaccc gaccctccca ggcagcccct   1920
   cagcgtctgc cgcccgccct tgggcctttc cggccagccc ctccctccgc ccacgcccag   1980
   aacagcccat gctcttggag gagagcaggt gggcttgacc gggactggcc cctcaccgcg   2040
   gaaggcgtgg ccggaatgct cggcccactc cgtctttggc ttgtgggctc tgcctcgtgc   2100
   accgcgtgct gcagcccaca gccggccgcc gccgggctgg cgcgagagtg gagggtggca   2160
   gtgcgcaggc gcggagcgga aaagaggggg cgtgaagggg gcggagccag gcagggttgg   2220
   gggagggggt gatgtactgt gggggcaggg gggaagggga gtagggaggg ggagggggag   2280
   cacggatgag gaggacccca gagggggcac tggtggacct gggggggtgg atgaggaggg   2340
   gaggagggtc ccatacaggg cactgggaga cctggggtgg gggtggataa ggagaggagg   2400
   agagcctgca gggcactgga agactgggta gtgggggagc gcctaaggag gggaggaggg   2460
   tcctgcaggg gcacggaaat accttggggt tgaagtggat gaggaaggga ggagggcccc   2520
   gcagggggcg ctgggagacc cgggggggaa gtaaggaggg cacgagggcc tgcagggggc   2580
   ctgggagatc tgagggggaa tggataagga ggggaggagg gcccacagga ggcactgaga   2640
   tactgggggg aggggtggaa tgaggggggg aggggtggaa tgaggcgggg aggatagccc   2700
   tgcagggggg ctgggagacc tgaggggggt ggtgggtaat ggataaggag gggagggggg   2760
   cctgcagggg gcctgtgaca cctaggggag gggaatggat aaggagggta ggagggccaa   2820
   caggaggcac tgagatacca gggggagggg tggaatgagg aggggagagc cctgcagggg   2880
   ggcggggaga cctgaggtga gggtaatgga taaggagggg aggagggtct gcaggggttc   2940
   ttgggagacc tgggggatga ataggagagg acaaggggag gagggtctgc agggggccct   3000
   g                                                                    3001

<210> 27
   <211> 3001
   <212> DNA
   <213> human
<400> 27
   attattatta tttgtttata aagaaaaata gtagtttagt tttagggagt gtattttatg      60
   agatgaattg gtttattttt tgtttttttt tgattttttg tagatttggt ttttttagta    120
   tttgtttgat tagttgtagg ttgatttttt tgttttttta tatttgaatt tgaaattgtg     180
   agaataattt cgttattttt tttatttttt gatattgttt ttataaaaaa gagttgtttt     240
   tttgtttttt tagttaataa ttggtttttt ttttgttttt tttcgagtta ataaataata     300
   aatatttaaa tatgatatta ttgattttag tataattaga tttttgtttg gatgaagtat     360
   agaatataaa aagaaaagtt aaagtggaag attatgtatt ataatgttaa ttatttcgta     420
   ttagggttgt ggtagtgaaa tttaatattt ttttttaaaa tataatgcgt tttatattat      480
   tttaattagt attattttta aaattttttt ttaaaaaaga atttaagaat ttttaagatt     540
   tttttgggaa gtatttgtat tgtttttaat ttgtaattgt aaaatatttt gtttagatat    600
   aatattgtat aaaagtcgat ttgaattttt ttttttttat gtaaggggtt gtttattaaa      660
   attagttgta ttttgattag tttagtagtt ttagttaggt ttatcgttaa tttattgttt     720
   ttattggtta ttattatatt tttttttaga gcgtggaatt gtaatatttt tattttttgc     780
   ggggagtata gtttttgttg gatattaatg gttatatatt gttgtaggtt ttgtcggggt     840
   ttttttttaa gttatagtaa tttagggtta ggttgttgta ttattatttg tatttttttt   900
   tgttgttgta tgtttagtaa gtttatattt tgtaatttta tagttttaga agtgaagtgt     960
   atcgtttgta gggttaggat gtatttttcg ttttttttcg agggggttag tattagtttt   1020
   attttttttt ttaggacgtt gttttggaag gttttagagg tacgttcggt ttttaattta    1080
   ttagggtttc gatggttttt ttttttgtat atttcgtttt tttttttttt ttttaggttt    1140
   tttttcggta ttaattcgag ttttttgatt ttggtgaatt gtttagaaag gatagagatt   1200
   ttagtggttg ttataatgat ttggtttgtt tgaaaaataa gtaagcggtt tttatagggg   1260
   ggagaagggg gtggtatgag gagggatgcg gggggtgttg gaatttagta ggttttggag    1320
   ttgaaataag gtttggtttt aaaaggtttt ggatttagta gggttagaat aatgttgatt    1380
   tggtttgggt ttaggagggg gttaaggtta agtaggattt aggttaaagt aggattttgt    1440
   ataaaatttt agaacgaata ggtttgggtt ttagtaggtt tagggttagt atatattttg    1500
   cgtttggtaa ggtttcgttt gagtatattt tggtttagtg tatattttgg gtttagtaag    1560
   gttaggtttg agtcggttta gttagtgaat attttgggtt tagtaaggtt tgggtttagt    1620
   aggttttcgg ttagtgtata ttttgggttt agtaagtttt aggtttaagt aggtttcggg    1680
   ttagtgtata ttcggcgttt agcgaggttc gggttcgagt aggtattttt ttcgtgtacg    1740
   gttttagatt tagtattttt agagttaggt aggttttggg tttagtggtt tcgagtttat    1800
   ttagatttgg ttaagtagtt ttcggttaga ttaagtatat tttttcggag gtttggtagg    1860
   gtttttgttt tattttgttt tttacgtttc gtttcgattc gattttttta ggtagttttt    1920
   tagcgtttgt cgttcgtttt tgggtttttt cggttagttt tttttttcgt ttacgtttag    1980
   aatagtttat gtttttggag gagagtaggt gggtttgatc gggattggtt ttttatcgcg    2040
   gaaggcgtgg tcggaatgtt cggtttattt cgtttttggt ttgtgggttt tgtttcgtgt    2100
   atcgcgtgtt gtagtttata gtcggtcgtc gtcgggttgg cgcgagagtg gagggtggta    2160
   gtgcgtaggc gcggagcgga aaagaggggg cgtgaagggg gcggagttag gtagggttgg    2220
   gggagggggt gatgtattgt gggggtaggg gggaagggga gtagggaggg ggagggggag    2280
   tacggatgag gaggatttta gagggggtat tggtggattt gggggggtgg atgaggaggg   2340
   gaggagggtt ttatataggg tattgggaga tttggggtgg gggtggataa ggagaggagg    2400
   agagtttgta gggtattgga agattgggta gtgggggagc gtttaaggag gggaggaggg    2460
   ttttgtaggg gtacggaaat attttggggt tgaagtggat gaggaaggga ggagggtttc    2520
   gtagggggcg ttgggagatt cgggggggaa gtaaggaggg tacgagggtt tgtagggggt   2580
   ttgggagatt tgagggggaa tggataagga ggggaggagg gtttatagga ggtattgaga    2640
   tattgggggg aggggtggaa tgaggggggg aggggtggaa tgaggcgggg aggatagttt    2700
   tgtagggggg ttgggagatt tgaggggggt ggtgggtaat ggataaggag gggagggggg    2760
   tttgtagggg gtttgtgata tttaggggag gggaatggat aaggagggta ggagggttaa    2820
   taggaggtat tgagatatta gggggagggg tggaatgagg aggggagagt tttgtagggg    2880
   ggcggggaga tttgaggtga gggtaatgga taaggagggg aggagggttt gtaggggttt    2940
   ttgggagatt tgggggatga ataggagagg ataaggggag gagggtttgt agggggtttt    3000
   g                                                                     3001
<210> 28
   <211> 3001
   <212> DNA
   <213> human
<400> 28
   attattatta tttgtttata aagaaaaata gtagtttagt tttagggagt gtattttatg     60
   agatgaattg gtttattttt tgtttttttt tgattttttg tagatttggt ttttttagta     120
   tttgtttgat tagttgtagg ttgatttttt tgttttttta tatttgaatt tgaaattgtg    180
   agaataattt tgttattttt tttatttttt gatattgttt ttataaaaaa gagttgtttt    240
   tttgtttttt tagttaataa ttggtttttt ttttgttttt ttttgagtta ataaataata    300
   aatatttaaa tatgatatta ttgattttag tataattaga tttttgtttg gatgaagtat    360
   agaatataaa aagaaaagtt aaagtggaag attatgtatt ataatgttaa ttattttgta    420
   ttagggttgt ggtagtgaaa tttaatattt ttttttaaaa tataatgtgt tttatattat     480
   tttaattagt attattttta aaattttttt ttaaaaaaga atttaagaat ttttaagatt    540
   tttttgggaa gtatttgtat tgtttttaat ttgtaattgt aaaatatttt gtttagatat    600
   aatattgtat aaaagttgat ttgaattttt ttttttttat gtaaggggtt gtttattaaa     660
   attagttgta ttttgattag tttagtagtt ttagttaggt ttattgttaa tttattgttt    720
   ttattggtta ttattatatt tttttttaga gtgtggaatt gtaatatttt tattttttgt     780
   ggggagtata gtttttgttg gatattaatg gttatatatt gttgtaggtt ttgttggggt    840
   ttttttttaa gttatagtaa tttagggtta ggttgttgta ttattatttg tatttttttt    900
   tgttgttgta tgtttagtaa gtttatattt tgtaatttta tagttttaga agtgaagtgt    960
   attgtttgta gggttaggat gtattttttg tttttttttg agggggttag tattagtttt   1020
   attttttttt ttaggatgtt gttttggaag gttttagagg tatgtttggt ttttaattta   1080
   ttagggtttt gatggttttt ttttttgtat attttgtttt tttttttttt ttttaggttt    1140
   ttttttggta ttaatttgag ttttttgatt ttggtgaatt gtttagaaag gatagagatt   1200
   ttagtggttg ttataatgat ttggtttgtt tgaaaaataa gtaagtggtt tttatagggg   1260
   ggagaagggg gtggtatgag gagggatgtg gggggtgttg gaatttagta ggttttggag   1320
   ttgaaataag gtttggtttt aaaaggtttt ggatttagta gggttagaat aatgttgatt   1380
   tggtttgggt ttaggagggg gttaaggtta agtaggattt aggttaaagt aggattttgt   1440
   ataaaatttt agaatgaata ggtttgggtt ttagtaggtt tagggttagt atatattttg   1500
   tgtttggtaa ggttttgttt gagtatattt tggtttagtg tatattttgg gtttagtaag   1560
   gttaggtttg agttggttta gttagtgaat attttgggtt tagtaaggtt tgggtttagt   1620
   aggtttttgg ttagtgtata ttttgggttt agtaagtttt aggtttaagt aggttttggg  1680
   ttagtgtata tttggtgttt agtgaggttt gggtttgagt aggtattttt tttgtgtatg   1740
   gttttagatt tagtattttt agagttaggt aggttttggg tttagtggtt ttgagtttat   1800
   ttagatttgg ttaagtagtt tttggttaga ttaagtatat ttttttggag gtttggtagg   1860
   gtttttgttt tattttgttt tttatgtttt gttttgattt gattttttta ggtagttttt   1920
   tagtgtttgt tgtttgtttt tgggtttttt tggttagttt ttttttttgt ttatgtttag   1980
   aatagtttat gtttttggag gagagtaggt gggtttgatt gggattggtt ttttattgtg   2040
   gaaggtgtgg ttggaatgtt tggtttattt tgtttttggt ttgtgggttt tgttttgtgt   2100
   attgtgtgtt gtagtttata gttggttgtt gttgggttgg tgtgagagtg gagggtggta   2160
   gtgtgtaggt gtggagtgga aaagaggggg tgtgaagggg gtggagttag gtagggttgg   2220
   gggagggggt gatgtattgt gggggtaggg gggaagggga gtagggaggg ggagggggag   2280
   tatggatgag gaggatttta gagggggtat tggtggattt gggggggtgg atgaggaggg   2340
   gaggagggtt ttatataggg tattgggaga tttggggtgg gggtggataa ggagaggagg   2400
   agagtttgta gggtattgga agattgggta gtgggggagt gtttaaggag gggaggaggg   2460
   ttttgtaggg gtatggaaat attttggggt tgaagtggat gaggaaggga ggagggtttt   2520
   gtagggggtg ttgggagatt tgggggggaa gtaaggaggg tatgagggtt tgtagggggt   2580
   ttgggagatt tgagggggaa tggataagga ggggaggagg gtttatagga ggtattgaga   2640
   tattgggggg aggggtggaa tgaggggggg aggggtggaa tgaggtgggg aggatagttt   2700
   tgtagggggg ttgggagatt tgaggggggt ggtgggtaat ggataaggag gggagggggg    2760
   tttgtagggg gtttgtgata tttaggggag gggaatggat aaggagggta ggagggttaa   2820
   taggaggtat tgagatatta gggggagggg tggaatgagg aggggagagt tttgtagggg   2880
   ggtggggaga tttgaggtga gggtaatgga taaggagggg aggagggttt gtaggggttt   2940
   ttgggagatt tgggggatga ataggagagg ataaggggag gagggtttgt agggggtttt   3000
   g                                                                   3001
<210> 29
   <211> 3001
   <212> DNA
   <213> human
<400> 29
   tagggttttt tgtagatttt tttttttttg ttttttttta tttatttttt aggtttttta     60
   agaatttttg tagatttttt tttttttttt atttattatt tttattttag gttttttcgt     120
   ttttttgtag ggtttttttt tttttatttt attttttttt ttggtatttt agtgtttttt    180
   gttggttttt ttattttttt tatttatttt ttttttttag gtgttatagg ttttttgtag     240
   gttttttttt tttttttatt tattatttat tatttttttt aggtttttta gtttttttgt    300
   agggttattt ttttcgtttt attttatttt tttttttttt attttatttt tttttttagt 360
   attttagtgt tttttgtggg tttttttttt ttttttattt attttttttt agatttttta 420
   ggttttttgt aggttttcgt gtttttttta tttttttttc gggtttttta gcgttttttg 480
   cggggttttt tttttttttt tatttatttt aattttaagg tattttcgtg tttttgtagg 540
   attttttttt tttttttagg cgttttttta ttatttagtt ttttagtgtt ttgtaggttt 600
   tttttttttt tttatttatt tttattttag gttttttagt gttttgtatg ggattttttt 660
   ttttttttta tttatttttt taggtttatt agtgtttttt ttggggtttt ttttattcgt 720
   gttttttttt ttttttttta tttttttttt tttttgtttt tatagtatat tatttttttt 780
   tttaattttg tttggtttcg ttttttttac gttttttttt tttcgtttcg cgtttgcgta 840
   ttgttatttt ttattttcgc gttagttcgg cggcggtcgg ttgtgggttg tagtacgcgg 900
   tgtacgaggt agagtttata agttaaagac ggagtgggtc gagtatttcg gttacgtttt 960
   tcgcggtgag gggttagttt cggttaagtt tatttgtttt tttttaagag tatgggttgt 1020
   tttgggcgtg ggcggaggga ggggttggtc ggaaaggttt aagggcgggc ggtagacgtt 1080
   gaggggttgt ttgggagggt cgggtcgggg cggggcgtgg ggggtagggt aaagtagggg 1140
   ttttgttagg ttttcgaggg agtgtgtttg gtttggtcga gggttgtttg gttaagtttg 1200
   ggtgggttcg aggttattag gtttaaagtt tgtttggttt tgagggtgtt aggtttagaa 1260
   tcgtgtacga ggggaatgtt tgttcgggtt cgaatttcgt tgggcgtcgg gtgtgtattg 1320
   gttcggggtt tgtttggatt tgaaatttgt taggtttagg atatgtattg gtcgagagtt 1380
   tgttgggttt aaattttatt aggtttagga tgtttattga ttgaatcggt ttaggtttaa 1440
   ttttgttagg tttaggatat gtattgggtt agagtgtgtt taggcggaat tttgttaggc 1500
   gtaggatgtg tgttggtttt aagtttgttg aggtttaaat ttgttcgttt tagggttttg 1560
   tataaaattt tgttttagtt taaattttgt ttagttttga ttttttttta gatttaagtt 1620
   agattagtat tgttttgatt ttattaagtt taaaattttt tgaggttaga ttttgtttta 1680
   attttaaagt ttgttaggtt ttagtatttt tcgtattttt ttttatatta tttttttttt 1740
   ttttttatgg aaatcgtttg tttatttttt aaataggtta agttattatg gtagttattg 1800
   agatttttgt tttttttgag taatttatta agattaaaga attcgagttg atgtcggaaa 1860
   aaggtttgaa ggaggaggaa aaagacggag tgtgtagaga gaaagattat cggagtttta 1920
   gtgagttgga ggtcgagcgt atttttgggg ttttttagga tagcgttttg gaggaagaag 1980
   tggagttggt gttggttttt tcggaggaga gcgagaagta tattttgatt ttgtagacgg 2040
   tgtattttat ttttgaagtt gtggagttgt aggatatgag tttgttgagt atatagtagt 2100
   aagaaggggt gtaggtggtg gtgtaatagt ttggttttgg gttgttgtgg tttgaggaag 2160
   ggtttcggta gagtttgtag tagtgtgtgg ttattagtat ttagtaagag ttgtattttt 2220
   cgtaagagat ggaggtgttg tagttttacg ttttagagga gaatgtgatg gtggttagtg 2280
   aagatagtaa gttagcggtg agtttggttg aaattattgg attgattaag gtatagttgg 2340
   ttttaataaa taatttttta tatgaaaaag aaaaaattta agtcggtttt tatgtaatat 2400
   tatgtttgga taaggtattt tgtagttgta aattaaagat agtataaata ttttttagga 2460
   aaattttgag agtttttagg ttttttttta aaaaagagtt ttaaaagtgg tgttaattgg 2520
   aatagtgtaa ggcgtattat gttttaaaaa aaagtgttaa gttttattgt tataattttg 2580
   atgcgaaata attaatattg tgatgtataa ttttttattt tggttttttt ttttgtattt 2640
   tgtgttttat ttaggtaaaa gtttaattat attgaaatta atagtattat atttgaatat 2700
   ttgttgttta ttagttcgag gaagagtagg agaagaatta gttattggtt gaaagaataa 2760
   aggagtagtt tttttttgtg gaaataatgt taggagatga aagaagtgac gaaattgttt 2820
   ttatagtttt aaatttaaat gtggaagaat aagaggatta atttatagtt ggttaagtag 2880
   atgttgaaaa ggttaaattt ataaaaaatt aaagaaagat aaagggtagg ttagtttatt 2940
   ttatagaata tattttttaa aattgggttg ttgttttttt ttatgggtag gtgatggtaa 3000
   t 3001
<210> 30
   <211> 3001
   <212> DNA
   <213> human
<400> 30
   tagggttttt tgtagatttt tttttttttg ttttttttta tttatttttt aggtttttta      60
   agaatttttg tagatttttt tttttttttt atttattatt tttattttag gtttttttgt       120
   ttttttgtag ggtttttttt tttttatttt attttttttt ttggtatttt agtgtttttt     180
   gttggttttt ttattttttt tatttatttt ttttttttag gtgttatagg ttttttgtag     240
   gttttttttt tttttttatt tattatttat tatttttttt aggtttttta gtttttttgt    300
   agggttattt tttttgtttt atttattttt tttttttttt atttattttt tttttttagt     360
   attttagtgt tttttgtggg tttttttttt ttttttattt attttttttt agatttttta      420
   ggttttttgt aggtttttgt gtttttttta tttttttttt gggtttttta gtgttttttg    480
   tggggttttt tttttttttt tatttatttt aattttaagg tatttttgtg tttttgtagg    540
   attttttttt tttttttagg tgttttttta ttatttagtt ttttagtgtt ttgtaggttt    600
   tttttttttt tttatttatt tttattttag gttttttagt gttttgtatg ggattttttt     660
   ttttttttta tttatttttt taggtttatt agtgtttttt ttggggtttt ttttatttgt    720
   gttttttttt ttttttttta tttttttttt tttttgtttt tatagtatat tatttttttt    780
   tttaattttg tttggttttg ttttttttat gttttttttt ttttgttttg tgtttgtgta     840
   ttgttatttt ttatttttgt gttagtttgg tggtggttgg ttgtgggttg tagtatgtgg    900
   tgtatgaggt agagtttata agttaaagat ggagtgggtt gagtattttg gttatgtttt   960
   ttgtggtgag gggttagttt tggttaagtt tatttgtttt tttttaagag tatgggttgt   1020
   tttgggtgtg ggtggaggga ggggttggtt ggaaaggttt aagggtgggt ggtagatgtt   1080
   gaggggttgt ttgggagggt tgggttgggg tggggtgtgg ggggtagggt aaagtagggg   1140
   ttttgttagg tttttgaggg agtgtgtttg gtttggttga gggttgtttg gttaagtttg   1200
   ggtgggtttg aggttattag gtttaaagtt tgtttggttt tgagggtgtt aggtttagaa   1260
   ttgtgtatga ggggaatgtt tgtttgggtt tgaattttgt tgggtgttgg gtgtgtattg    1320
   gtttggggtt tgtttggatt tgaaatttgt taggtttagg atatgtattg gttgagagtt   1380
   tgttgggttt aaattttatt aggtttagga tgtttattga ttgaattggt ttaggtttaa   1440
   ttttgttagg tttaggatat gtattgggtt agagtgtgtt taggtggaat tttgttaggt   1500
   gtaggatgtg tgttggtttt aagtttgttg aggtttaaat ttgtttgttt tagggttttg   1560
   tataaaattt tgttttagtt taaattttgt ttagttttga ttttttttta gatttaagtt   1620
   agattagtat tgttttgatt ttattaagtt taaaattttt tgaggttaga ttttgtttta   1680
   attttaaagt ttgttaggtt ttagtatttt ttgtattttt ttttatatta tttttttttt   1740
   ttttttatgg aaattgtttg tttatttttt aaataggtta agttattatg gtagttattg    1800
   agatttttgt tttttttgag taatttatta agattaaaga atttgagttg atgttggaaa    1860
   aaggtttgaa ggaggaggaa aaagatggag tgtgtagaga gaaagattat tggagtttta   1920
   gtgagttgga ggttgagtgt atttttgggg ttttttagga tagtgttttg gaggaagaag     1980
   tggagttggt gttggttttt ttggaggaga gtgagaagta tattttgatt ttgtagatgg   2040
   tgtattttat ttttgaagtt gtggagttgt aggatatgag tttgttgagt atatagtagt   2100
   aagaaggggt gtaggtggtg gtgtaatagt ttggttttgg gttgttgtgg tttgaggaag   2160
   ggttttggta gagtttgtag tagtgtgtgg ttattagtat ttagtaagag ttgtattttt   2220
   tgtaagagat ggaggtgttg tagttttatg ttttagagga gaatgtgatg gtggttagtg   2280
   aagatagtaa gttagtggtg agtttggttg aaattattgg attgattaag gtatagttgg   2340
   ttttaataaa taatttttta tatgaaaaag aaaaaattta agttggtttt tatgtaatat    2400
   tatgtttgga taaggtattt tgtagttgta aattaaagat agtataaata ttttttagga    2460
   aaattttgag agtttttagg ttttttttta aaaaagagtt ttaaaagtgg tgttaattgg    2520
   aatagtgtaa ggtgtattat gttttaaaaa aaagtgttaa gttttattgt tataattttg   2580
   atgtgaaata attaatattg tgatgtataa ttttttattt tggttttttt ttttgtattt    2640
   tgtgttttat ttaggtaaaa gtttaattat attgaaatta atagtattat atttgaatat   2700
   ttgttgttta ttagtttgag gaagagtagg agaagaatta gttattggtt gaaagaataa   2760
   aggagtagtt tttttttgtg gaaataatgt taggagatga aagaagtgat gaaattgttt   2820
   ttatagtttt aaatttaaat gtggaagaat aagaggatta atttatagtt ggttaagtag   2880
   atgttgaaaa ggttaaattt ataaaaaatt aaagaaagat aaagggtagg ttagtttatt   2940
   ttatagaata tattttttaa aattgggttg ttgttttttt ttatgggtag gtgatggtaa   3000
   t                                                                   3001
<210> 31
   <211> 50
   <212> DNA
   <213> human
<400> 31
   cgaccaagag taggaaccag atacagccag aggaaacaga cacccaagca 50
<210> 32
   <211> 50
   <212> DNA
   <213> human
<400> 32
   cgattaagag taggaattag atatagttag aggaaataga tatttaagta 50
<210> 33
   <211> 50
   <212> DNA
   <213> human
<400> 33
   tgattaagag taggaattag atatagttag aggaaataga tatttaagta 50
<210> 34
   <211> 50
   <212> DNA
   <213> human
<400> 34
   tgtttgggtg tttgtttttt ttggttgtat ttggttttta tttttggtcg 50
<210> 35
   <211> 50
   <212> DNA
   <213> human
<400> 35
   tgtttgggtg tttgtttttt ttggttgtat ttggttttta tttttggttg 50
<210> 36
   <211> 3001
   <212> DNA
   <213> human
<400> 36
   cgaggtagct aggaggggtt ttcgggtggt cggttgtgag agaccgctga cagtaaaatg 60
   taatgaactt ctcactttta aaaaaggggt gttattgccc aaatgagcgc tcagaacttt 120
   tttaatggcc ccaaagatcc cggagcccgg acgccgggct cttgagaacc cgcaagacga 180
   ccgccaaggc gcacgcacag ctcaccttcc cgcccggcac gtcagaagac ccgagattcc 240
   gtgagtgcca ccgcaccttc cggttcccgc cggaagtttt tcaaacgcca gaaggtccgc 300
   ctcgcttctt ccggaccaag ttagccacag acctccaagg cgcggcgcta ccgaacgggg 360
   cggagccagg ctggacggag gcggggctag agcgaggatc ccgagggagc gccggggtgg 420
   ggctggttcg tagaaatgaa aaatgacttt ttttttacgt gttatttacc aagaacacta 480
   aactcttaag cccacagata cagccaaata aattagatta taaaagtagg gaaataaggc 540
   tgctgttgtc tacattcgaa tttagcagat caaaacccca tggatttgtt ttagaaaatt 600
   ctaaaaagca gctttccact gctaatgact tatttccagg gactctgagt actgaagagc 660
   ctagcaagtt attgaaatga cctgtgaccg tgccttgttg cagatctacc ggaagtgagc 720
   atagatttta ctggataatt tttgtgattg ggggaaaaaa aaaaggattc tttctaaatt 780
   tagtaaatct aatcttctat atctgagatt gttggccagg aaagatcagt ttccgagatt 840
   gtttagggat ttttttttcc ttttaattag cacagtcgta tgttgccttt agtgatggtt 900
   ccagaaggcc tagaattgtc tactacaatc acagtctcct tcacaattta tggtcctgaa 960
   gaaatactag agaacacctt accgttcagg attgtgggaa gagaaaccag atcatagaaa 1020
   tcaacaacaa taatgtacta cttactattc acctactttt gatgttgcat ttgctactca 1080
   ggtttatggt aaggggttgt cttggtcaat tttctgctgc tgtaaccaaa taccatagac 1140
   tggtagattt ataaagaaag agatttgttt ggcttgtagt tctgaaggct gggaagtcca 1200
   agacagaggg cattattgtt gtgtcaaaat atgatggagg gcatcatact ggggtagggg 1260
   tgggggaggc atgtgaaccc aattgactcc cgaaataact aaccctcctg tgataatgga 1320
   attaactcat tcatgaccta atcatttcct aaaggtccca cctcttcata ctattacaat 1380
   gacaagtttc aacttgaatt tttgaagggg acattccaat cattgcagag gtagtaaggg 1440
   taaaggtgaa ctaatatact gcaaagtctt ttgacataag tcctgccctg gaggaattta 1500
   cgaccaagag taggaaccag atacagccag aggaaacaga cacccaagca aaacagtcta 1560
   gcttcaaggg ttgggtatgc aggtagtagc cacactacag tgaggaggag agtaagagtg 1620
   ggccagcaaa gcattgaagc ttgactttcc tctccccaca agggattttt gccttacagc 1680
   tggggaagaa aaaaaaaatc acaaaaataa atagcctgtt aagattttag acatcagaca 1740
   tctggggaaa ataaaatctg aagcaggttc tctaaaatta aagtatgtag gtagtggcct 1800
   actctaaagg ccaataagga ctgtccagag aatatttccc ttggaattct ataagagagt 1860
   cactcccact acacgctgtc aaggaaaatt tgtaatcact taagtataaa acaggccctc 1920
   ccacaaaaac tgccttttct gtctaggaca ttgcccaatc tattgtttac ttttggatct 1980
   caagtgctga attccaagaa attcatgggt ttagactgaa agcctggaat aatccggaca 2040
   cccacctgag actatgcaat agggtttggg agtaatgaaa acatgttttg gatttgatgg 2100
   aggttgtttt atccctccaa agtatatagc tcttcccatg atattcactg atacattatc 2160
   atttgtatgt tattatctgt tttcaagtcc ttcccctatg ggttctagct ccccagggca 2220
   aatgtaataa taatagtaat aacttacctt tattgattac ttacctgtgc caggcactaa 2280
   gttaatgctt ttagatgtat ttatgtatat aattcacata ttaccccaat gcaatattgc 2340
   tattaaacca tttttataaa tgagaatacc aggtataaag aagttaaata attcatctaa 2400
   ggtcacatca gctaataaat tgtagatctg agattcaaat ccaataatct gctttactcc 2460
   aaagcaagta ctctgaaagg tatccacata caatttgctg tctatccaca ggggattggt 2520
   ctgaggaccc cccacagata gcaaaattta cagatactca accaatcagt gatagaaaaa 2580
   gttggcccac cacatctgca ggttccatat ccatgaatgc agatacagat ggccagctgt 2640
   accacaaact caaagcaaag accttttctc gctccactat ggtggccagt gagactctca 2700
   atgtttaagg tgctccagaa ctgtcgaatc tctaaatact ctaaataaag tactgtctaa 2760
   ccacctgaaa cataacaaag ctattctctc tattggcctc aaactaagtt tcataagaac 2820
   aaaaattctt aatatattaa tagatgcaac tgtttcacct actaatagat atttggatct 2880
   aggaactaac tcttggtaat tttttatctc cccctctcca acttctccct ccacctacac 2940
   agtgcctaat aatgcttaac atataatagg tactcaataa tatagtagac taactcctgg 3000
   a                                                                  3001
<210> 37
   <211> 3001
   <212> DNA
   <213> human
<400> 37
   cgaggtagtt aggaggggtt ttcgggtggt cggttgtgag agatcgttga tagtaaaatg 60
   taatgaattt tttattttta aaaaaggggt gttattgttt aaatgagcgt ttagaatttt 120
   tttaatggtt ttaaagattt cggagttcgg acgtcgggtt tttgagaatt cgtaagacga 180
   tcgttaaggc gtacgtatag tttatttttt cgttcggtac gttagaagat tcgagatttc 240
   gtgagtgtta tcgtattttt cggttttcgt cggaagtttt ttaaacgtta gaaggttcgt 300
   ttcgtttttt tcggattaag ttagttatag atttttaagg cgcggcgtta tcgaacgggg 360
   cggagttagg ttggacggag gcggggttag agcgaggatt tcgagggagc gtcggggtgg 420
   ggttggttcg tagaaatgaa aaatgatttt ttttttacgt gttatttatt aagaatatta 480
   aatttttaag tttatagata tagttaaata aattagatta taaaagtagg gaaataaggt 540
   tgttgttgtt tatattcgaa tttagtagat taaaatttta tggatttgtt ttagaaaatt 600
   ttaaaaagta gttttttatt gttaatgatt tatttttagg gattttgagt attgaagagt 660
   ttagtaagtt attgaaatga tttgtgatcg tgttttgttg tagatttatc ggaagtgagt 720
   atagatttta ttggataatt tttgtgattg ggggaaaaaa aaaaggattt tttttaaatt 780
   tagtaaattt aattttttat atttgagatt gttggttagg aaagattagt tttcgagatt 840
   gtttagggat tttttttttt ttttaattag tatagtcgta tgttgttttt agtgatggtt 900
   ttagaaggtt tagaattgtt tattataatt atagtttttt ttataattta tggttttgaa 960
   gaaatattag agaatatttt atcgtttagg attgtgggaa gagaaattag attatagaaa 1020
   ttaataataa taatgtatta tttattattt atttattttt gatgttgtat ttgttattta 1080
   ggtttatggt aaggggttgt tttggttaat tttttgttgt tgtaattaaa tattatagat 1140
   tggtagattt ataaagaaag agatttgttt ggtttgtagt tttgaaggtt gggaagttta 1200
   agatagaggg tattattgtt gtgttaaaat atgatggagg gtattatatt ggggtagggg 1260
   tgggggaggt atgtgaattt aattgatttt cgaaataatt aatttttttg tgataatgga 1320
   attaatttat ttatgattta attatttttt aaaggtttta tttttttata ttattataat 1380
   gataagtttt aatttgaatt tttgaagggg atattttaat tattgtagag gtagtaaggg 1440
   taaaggtgaa ttaatatatt gtaaagtttt ttgatataag ttttgttttg gaggaattta 1500
   cgattaagag taggaattag atatagttag aggaaataga tatttaagta aaatagttta 1560
   gttttaaggg ttgggtatgt aggtagtagt tatattatag tgaggaggag agtaagagtg 1620
   ggttagtaaa gtattgaagt ttgatttttt tttttttata agggattttt gttttatagt 1680
   tggggaagaa aaaaaaaatt ataaaaataa atagtttgtt aagattttag atattagata 1740
   tttggggaaa ataaaatttg aagtaggttt tttaaaatta aagtatgtag gtagtggttt 1800
   attttaaagg ttaataagga ttgtttagag aatatttttt ttggaatttt ataagagagt 1860
   tatttttatt atacgttgtt aaggaaaatt tgtaattatt taagtataaa ataggttttt 1920
   ttataaaaat tgtttttttt gtttaggata ttgtttaatt tattgtttat ttttggattt 1980
   taagtgttga attttaagaa atttatgggt ttagattgaa agtttggaat aattcggata 2040
   tttatttgag attatgtaat agggtttggg agtaatgaaa atatgttttg gatttgatgg 2100
   aggttgtttt atttttttaa agtatatagt tttttttatg atatttattg atatattatt 2160
   atttgtatgt tattatttgt ttttaagttt tttttttatg ggttttagtt ttttagggta 2220
   aatgtaataa taatagtaat aatttatttt tattgattat ttatttgtgt taggtattaa 2280
   gttaatgttt ttagatgtat ttatgtatat aatttatata ttattttaat gtaatattgt 2340
   tattaaatta tttttataaa tgagaatatt aggtataaag aagttaaata atttatttaa 2400
   ggttatatta gttaataaat tgtagatttg agatttaaat ttaataattt gttttatttt 2460
   aaagtaagta ttttgaaagg tatttatata taatttgttg tttatttata ggggattggt 2520
   ttgaggattt tttatagata gtaaaattta tagatattta attaattagt gatagaaaaa 2580
   gttggtttat tatatttgta ggttttatat ttatgaatgt agatatagat ggttagttgt 2640
   attataaatt taaagtaaag attttttttc gttttattat ggtggttagt gagattttta 2700
   atgtttaagg tgttttagaa ttgtcgaatt tttaaatatt ttaaataaag tattgtttaa 2760
   ttatttgaaa tataataaag ttattttttt tattggtttt aaattaagtt ttataagaat 2820
   aaaaattttt aatatattaa tagatgtaat tgttttattt attaatagat atttggattt 2880
   aggaattaat ttttggtaat tttttatttt ttttttttta attttttttt ttatttatat 2940
   agtgtttaat aatgtttaat atataatagg tatttaataa tatagtagat taatttttgg 3000
   a 3001
<210> 38
   <211> 3001
   <212> DNA
   <213> human
<400> 38
   tgaggtagtt aggaggggtt tttgggtggt tggttgtgag agattgttga tagtaaaatg      60
   taatgaattt tttattttta aaaaaggggt gttattgttt aaatgagtgt ttagaatttt     120
   tttaatggtt ttaaagattt tggagtttgg atgttgggtt tttgagaatt tgtaagatga     180
   ttgttaaggt gtatgtatag tttatttttt tgtttggtat gttagaagat ttgagatttt     240
   gtgagtgtta ttgtattttt tggtttttgt tggaagtttt ttaaatgtta gaaggtttgt     300
   tttgtttttt ttggattaag ttagttatag atttttaagg tgtggtgtta ttgaatgggg     360
   tggagttagg ttggatggag gtggggttag agtgaggatt ttgagggagt gttggggtgg     420
   ggttggtttg tagaaatgaa aaatgatttt ttttttatgt gttatttatt aagaatatta      480
   aatttttaag tttatagata tagttaaata aattagatta taaaagtagg gaaataaggt     540
   tgttgttgtt tatatttgaa tttagtagat taaaatttta tggatttgtt ttagaaaatt     600
   ttaaaaagta gttttttatt gttaatgatt tatttttagg gattttgagt attgaagagt    660
   ttagtaagtt attgaaatga tttgtgattg tgttttgttg tagatttatt ggaagtgagt     720
   atagatttta ttggataatt tttgtgattg ggggaaaaaa aaaaggattt tttttaaatt     780
   tagtaaattt aattttttat atttgagatt gttggttagg aaagattagt ttttgagatt     840
   gtttagggat tttttttttt ttttaattag tatagttgta tgttgttttt agtgatggtt     900
   ttagaaggtt tagaattgtt tattataatt atagtttttt ttataattta tggttttgaa     960
   gaaatattag agaatatttt attgtttagg attgtgggaa gagaaattag attatagaaa    1020
   ttaataataa taatgtatta tttattattt atttattttt gatgttgtat ttgttattta    1080
   ggtttatggt aaggggttgt tttggttaat tttttgttgt tgtaattaaa tattatagat    1140
   tggtagattt ataaagaaag agatttgttt ggtttgtagt tttgaaggtt gggaagttta    1200
   agatagaggg tattattgtt gtgttaaaat atgatggagg gtattatatt ggggtagggg    1260
   tgggggaggt atgtgaattt aattgatttt tgaaataatt aatttttttg tgataatgga    1320
   attaatttat ttatgattta attatttttt aaaggtttta tttttttata ttattataat    1380
   gataagtttt aatttgaatt tttgaagggg atattttaat tattgtagag gtagtaaggg    1440
   taaaggtgaa ttaatatatt gtaaagtttt ttgatataag ttttgttttg gaggaattta    1500
   tgattaagag taggaattag atatagttag aggaaataga tatttaagta aaatagttta    1560
   gttttaaggg ttgggtatgt aggtagtagt tatattatag tgaggaggag agtaagagtg    1620
   ggttagtaaa gtattgaagt ttgatttttt tttttttata agggattttt gttttatagt    1680
   tggggaagaa aaaaaaaatt ataaaaataa atagtttgtt aagattttag atattagata    1740
   tttggggaaa ataaaatttg aagtaggttt tttaaaatta aagtatgtag gtagtggttt    1800
   attttaaagg ttaataagga ttgtttagag aatatttttt ttggaatttt ataagagagt    1860
   tatttttatt atatgttgtt aaggaaaatt tgtaattatt taagtataaa ataggttttt    1920
   ttataaaaat tgtttttttt gtttaggata ttgtttaatt tattgtttat ttttggattt    1980
   taagtgttga attttaagaa atttatgggt ttagattgaa agtttggaat aatttggata    2040
   tttatttgag attatgtaat agggtttggg agtaatgaaa atatgttttg gatttgatgg    2100
   aggttgtttt atttttttaa agtatatagt tttttttatg atatttattg atatattatt   2160
   atttgtatgt tattatttgt ttttaagttt tttttttatg ggttttagtt ttttagggta    2220
   aatgtaataa taatagtaat aatttatttt tattgattat ttatttgtgt taggtattaa    2280
   gttaatgttt ttagatgtat ttatgtatat aatttatata ttattttaat gtaatattgt    2340
   tattaaatta tttttataaa tgagaatatt aggtataaag aagttaaata atttatttaa    2400
   ggttatatta gttaataaat tgtagatttg agatttaaat ttaataattt gttttatttt    2460
   aaagtaagta ttttgaaagg tatttatata taatttgttg tttatttata ggggattggt    2520
   ttgaggattt tttatagata gtaaaattta tagatattta attaattagt gatagaaaaa    2580
   gttggtttat tatatttgta ggttttatat ttatgaatgt agatatagat ggttagttgt    2640
   attataaatt taaagtaaag attttttttt gttttattat ggtggttagt gagattttta     2700
   atgtttaagg tgttttagaa ttgttgaatt tttaaatatt ttaaataaag tattgtttaa    2760
   ttatttgaaa tataataaag ttattttttt tattggtttt aaattaagtt ttataagaat    2820
   aaaaattttt aatatattaa tagatgtaat tgttttattt attaatagat atttggattt    2880
   aggaattaat ttttggtaat tttttatttt ttttttttta attttttttt ttatttatat   2940
   agtgtttaat aatgtttaat atataatagg tatttaataa tatagtagat taatttttgg    3000
   a                                                                     3001
<210> 39
   <211> 3001
   <212> DNA
   <213> human
<400> 39
   tttaggagtt agtttattat attattgagt atttattata tgttaagtat tattaggtat 60
   tgtgtaggtg gagggagaag ttggagaggg ggagataaaa aattattaag agttagtttt 120
   tagatttaaa tatttattag taggtgaaat agttgtattt attaatatat taagaatttt 180
   tgtttttatg aaatttagtt tgaggttaat agagagaata gttttgttat gttttaggtg 240
   gttagatagt attttattta gagtatttag agattcgata gttttggagt attttaaata 300
   ttgagagttt tattggttat tatagtggag cgagaaaagg tttttgtttt gagtttgtgg 360
   tatagttggt tatttgtatt tgtatttatg gatatggaat ttgtagatgt ggtgggttaa 420
   ttttttttat tattgattgg ttgagtattt gtaaattttg ttatttgtgg ggggttttta 480
   gattaatttt ttgtggatag atagtaaatt gtatgtggat attttttaga gtatttgttt 540
   tggagtaaag tagattattg gatttgaatt ttagatttat aatttattag ttgatgtgat 600
   tttagatgaa ttatttaatt tttttatatt tggtattttt atttataaaa atggtttaat 660
   agtaatattg tattggggta atatgtgaat tatatatata aatatattta aaagtattaa 720
   tttagtgttt ggtataggta agtaattaat aaaggtaagt tattattatt attattatat 780
   ttgttttggg gagttagaat ttatagggga aggatttgaa aatagataat aatatataaa 840
   tgataatgta ttagtgaata ttatgggaag agttatatat tttggaggga taaaataatt 900
   tttattaaat ttaaaatatg tttttattat ttttaaattt tattgtatag ttttaggtgg 960
   gtgttcggat tattttaggt ttttagttta aatttatgaa ttttttggaa tttagtattt 1020
   gagatttaaa agtaaataat agattgggta atgttttaga tagaaaaggt agtttttgtg 1080
   ggagggtttg ttttatattt aagtgattat aaattttttt tgatagcgtg tagtgggagt 1140
   gattttttta tagaatttta agggaaatat tttttggata gtttttattg gtttttagag 1200
   taggttatta tttatatatt ttaattttag agaatttgtt ttagatttta ttttttttag 1260
   atgtttgatg tttaaaattt taataggtta tttatttttg tgattttttt tttttttttt 1320
   agttgtaagg taaaaatttt ttgtggggag aggaaagtta agttttaatg ttttgttggt 1380
   ttatttttat tttttttttt attgtagtgt ggttattatt tgtatattta atttttgaag 1440
   ttagattgtt ttgtttgggt gtttgttttt tttggttgta tttggttttt atttttggtc 1500
   gtaaattttt ttagggtagg atttatgtta aaagattttg tagtatatta gtttattttt 1560
   atttttatta tttttgtaat gattggaatg ttttttttaa aaatttaagt tgaaatttgt 1620
   tattgtaata gtatgaagag gtgggatttt taggaaatga ttaggttatg aatgagttaa 1680
   ttttattatt ataggagggt tagttatttc gggagttaat tgggtttata tgtttttttt 1740
   atttttattt tagtatgatg ttttttatta tattttgata taataataat gttttttgtt 1800
   ttggattttt tagtttttag aattataagt taaataaatt ttttttttta taaatttatt 1860
   agtttatggt atttggttat agtagtagaa aattgattaa gataattttt tattataaat 1920
   ttgagtagta aatgtaatat taaaagtagg tgaatagtaa gtagtatatt attgttgttg 1980
   atttttatga tttggttttt ttttttataa ttttgaacgg taaggtgttt tttagtattt 2040
   ttttaggatt ataaattgtg aaggagattg tgattgtagt agataatttt aggttttttg 2100
   gaattattat taaaggtaat atacgattgt gttaattaaa aggaaaaaaa aatttttaaa 2160
   taatttcgga aattgatttt ttttggttaa taattttaga tatagaagat tagatttatt 2220
   aaatttagaa agaatttttt tttttttttt ttaattataa aaattattta gtaaaattta 2280
   tgtttatttt cggtagattt gtaataaggt acggttatag gttattttaa taatttgtta 2340
   ggttttttag tatttagagt ttttggaaat aagttattag tagtggaaag ttgtttttta 2400
   gaatttttta aaataaattt atggggtttt gatttgttaa attcgaatgt agataatagt 2460
   agtttatttt ttttattttt ataatttaat ttatttggtt gtatttgtgg gtttaagagt 2520
   ttagtgtttt tggtaaataa tacgtaaaaa aaaagttatt ttttattttt acgaattagt 2580
   tttatttcgg cgttttttcg ggattttcgt tttagtttcg ttttcgttta gtttggtttc 2640
   gtttcgttcg gtagcgtcgc gttttggagg tttgtggtta atttggttcg gaagaagcga 2700
   ggcggatttt ttggcgtttg aaaaattttc ggcgggaatc ggaaggtgcg gtggtattta 2760
   cggaatttcg ggttttttga cgtgtcgggc gggaaggtga gttgtgcgtg cgttttggcg 2820
   gtcgttttgc gggtttttaa gagttcggcg ttcgggtttc gggatttttg gggttattaa 2880
   aaaagttttg agcgtttatt tgggtaataa tatttttttt ttaaaagtga gaagtttatt 2940
   atattttatt gttagcggtt ttttataatc gattattcga aaattttttt tagttatttc 3000
   g 3001
<210> 40
   <211> 3001
   <212> DNA
   <213> human
<400> 40
<210> 41
   <211> 50
   <212> DNA
   <213> human
<400> 41
   cgggggcagt cgtagcttca gcaccgcctc tgccatcacc ccgtctgtct 50
<210> 42
   <211> 50
   <212> DNA
   <213> human
<400> 42
   cgggggtagt cgtagtttta gtatcgtttt tgttattatt tcgtttgttt 50
<210> 43
   <211> 50
   <212> DNA
   <213> human
<400> 43
   tgggggtagt tgtagtttta gtattgtttt tgttattatt ttgtttgttt 50
<210> 44
   <211> 50
   <212> DNA
   <213> human
<400> 44
   agatagacgg ggtgatggta gaggcggtgt tgaagttacg attgttttcg 50
<210> 45
   <211> 50
   <212> DNA
   <213> human
<400> 45
   agatagatgg ggtgatggta gaggtggtgt tgaagttatg attgtttttg 50
<210> 46
   <211> 3001
   <212> DNA
   <213> human
<400> 46
   gtccagaacc ttgttctgct gctccaggaa ccgcacctgg aggggagcag ggtttgaaga 60
   tagagaaact tggatttcat gttctatgat caactcacta aggtgcctcc cacctctttc 120
   cgtccctcta aagcttttct gtgcactgtg cctggccctg ggccccagga ggggaggtgc    180
   agactgaggc acccaaactc acttttccag ggggagatag gacacctatc cagggcacag    240
   aaagcactag gagtgctgct tggagtgtgt cccggctcag gggtggctgc aaaggcactc    300
   aggctgctgc ccatgcactt gtctaccttc ctgtacagtc agccccttta tatatggcat    360
   gaaattcatc tctctgtcca acagttgtta agaggccctt gtgagctaac ctctgaatgg    420
   gaagaaataa ttctcccttc ccagctgcca gtctaattca gaacgtgtcc acaaatccag    480
   ggcacagaaa caaacggaaa cacagaactg tacaactata aaagtatttg cacaaagcca    540
   aaacatcttc cttctttctc tctctttggc atttatttca gacccacagt gattttttac     600
   aaaagatcgt agctcacctt gtcgatgaag gaggcaaact tattgttgag ggtcttgatc    660
   tgctcgcgct cctcggtcct caccctctgg atgctggggt cgatttgcag gttgagggga    720
   gtcaggagac tctggttgac agtgacctct tggatacctc caggagggca gacaggaaag    780
   ccagggccac cgaagccacc tccaaagcca gctccgccac cgagcccaaa gccaccacca    840
   gctccaccgc cgaaaccaaa tccactaccg gcaccacctc caaagccata gccgcctcca    900
   gcaccagcac caaaccggtt cctgaagctg ccaccactag tgctgatgga tatcctcttg    960
   gagcccccca ggttgtagag gctccggctg ccatagccac ccactccaca agcacccgca   1020
   aggctgaccc tgccgaagcc accaccaccg ccacccccgg accgggacac ggaggtgaag  1080
   ctggtgcggg agacagacgg ggtgatggca gaggcggtgc tgaagctacg actgcccccg   1140
   ctccggaagg acacacttga ctggcgagac atggtggctt gttcctggtg gagcaagaga   1200
   accaggcact agtgggttgg gaggtgctgg agagaacaga gctcagcagg acgcagaagg   1260
   tggctctgtt acccaggaac ggtgatgccc cctttttatc ccttccagaa ctgggctggg    1320
   cgagagagct gtcgagctgt gaatgattaa gtgggctggg catgcctggg gggcagctgt   1380
   gagctcaccg gtcacacctg cacagtggtg tggggtgcaa acgctttctt cctggcaggc   1440
   tctgctcagt tagaaataac cttgccttgc agcactgatc tcttggcagc aagttggtga   1500
   cgctgctagc aagtatgtct tcaccataga ggctgggggc tgtgagcagc ccaaaaagga   1560
   tgccaggaac tgagggggca ttggcatggc cagtgtcctg gggactctta caggaccagg   1620
   agtattgaca accatcccag tacggtacat gctgagcacc tgcccttcca gcttggggaa   1680
   gctgtccatc agcggggggc aaacgattcc ccagtgatct gctcttacac ttttccaggc   1740
   agcaggttgc tgcagggttt tcccctctgg gaaaggcaat ctgtagcctt atattcttgg   1800
   ttatgtgcta gagggaaaat agagtttcat ccttttctgc acaatttttt tcttagggaa    1860
   ggcacagcta tcccagagcc cactgggcag atctgcctgc aaggtttagg ccagagaatg   1920
   tgggaagccc tcagcgcccc ctataaacca ctttgacctc tttaaggcca ggtttatcct   1980
   tccatagctc ctcctaactc agcatttttt ctgccctttg accttttcct tgctctttgg   2040
   cctgggcagg actctgtggg gggtttagga aacccgggga tcccatgcaa tcccttaaaa   2100
   aaaaaccctg agactcatgg gctatagcgt ctctctttgg gggtagctac tctgccggtt   2160
   ccccagcccc ccaggtgtgt tccagcaagc tctattccac tagggaagaa atgagtcatc   2220
   gtatgtgttg atacaggctg aaggccgcca caaacacata cctgcaggag cttctaatct   2280
   catcaggaac atgttgagag tctgacggcc tgttataacg gcgaaccacc ctgttccaag   2340
   cagcgggact tgctctgctt tctctatgct gtagcccaca gcctccttca gggccaaggc   2400
   aatggaaaac gggattctaa gtctatggat tgtcctgcca gaaaggctag ccagagcagg   2460
   cctgtgggcc caggcagctg gcatgacaat ctgccagctg ctttagggag ggccctttga   2520
   gatcccctct gcaaaacttg tctggggggt tgtggcacca ggtggaggaa agggggtttc   2580
   tgatgaggtt ttaacacacg tctgttctaa accacaccca ctctttttgg cagcagatct   2640
   gagtggggcc acctctctgc cgtcccccaa atagaagcag ggctctcagg caggacagtg   2700
   cttgggctgt tcagagtttc gggggcgccc tgtggcccga ggctggaggt aggaatgcaa   2760
   tggggatggc ttcaaagcct cagggaggag gggctatatt ctcaagagta gaagaacagg   2820
   accagaccca ggctcaggaa tctctcacca gggcctgcgg ttggccacaa ggaagccaag   2880
   gcagtgggaa aatgactccc tggtgctgag gatcagctga aacagtgact tctcaccaga   2940
   gggctgcgat gagcaagcag ctgacaggtc aatctcggca gacagaccaa tggccggagc   3000
   c                                                                   3001
<210> 47
   <211> 3001
   <212> DNA
   <213> human
<400> 47
   gtttagaatt ttgttttgtt gttttaggaa tcgtatttgg aggggagtag ggtttgaaga      60
   tagagaaatt tggattttat gttttatgat taatttatta aggtgttttt tatttttttt     120
   cgttttttta aagttttttt gtgtattgtg tttggttttg ggttttagga ggggaggtgt     180
   agattgaggt atttaaattt atttttttag ggggagatag gatatttatt tagggtatag     240
   aaagtattag gagtgttgtt tggagtgtgt ttcggtttag gggtggttgt aaaggtattt     300
   aggttgttgt ttatgtattt gtttattttt ttgtatagtt agttttttta tatatggtat     360
   gaaatttatt tttttgttta atagttgtta agaggttttt gtgagttaat ttttgaatgg     420
   gaagaaataa tttttttttt ttagttgtta gtttaattta gaacgtgttt ataaatttag     480
   ggtatagaaa taaacggaaa tatagaattg tataattata aaagtatttg tataaagtta     540
   aaatattttt tttttttttt tttttttggt atttatttta gatttatagt gattttttat    600
   aaaagatcgt agtttatttt gtcgatgaag gaggtaaatt tattgttgag ggttttgatt     660
   tgttcgcgtt tttcggtttt tattttttgg atgttggggt cgatttgtag gttgagggga    720
   gttaggagat tttggttgat agtgattttt tggatatttt taggagggta gataggaaag     780
   ttagggttat cgaagttatt tttaaagtta gtttcgttat cgagtttaaa gttattatta     840
   gttttatcgt cgaaattaaa tttattatcg gtattatttt taaagttata gtcgttttta    900
   gtattagtat taaatcggtt tttgaagttg ttattattag tgttgatgga tatttttttg   960
   gagtttttta ggttgtagag gtttcggttg ttatagttat ttattttata agtattcgta    1020
   aggttgattt tgtcgaagtt attattatcg ttattttcgg atcgggatac ggaggtgaag    1080
   ttggtgcggg agatagacgg ggtgatggta gaggcggtgt tgaagttacg attgttttcg    1140
   tttcggaagg atatatttga ttggcgagat atggtggttt gtttttggtg gagtaagaga    1200
   attaggtatt agtgggttgg gaggtgttgg agagaataga gtttagtagg acgtagaagg    1260
   tggttttgtt atttaggaac ggtgatgttt tttttttatt ttttttagaa ttgggttggg     1320
   cgagagagtt gtcgagttgt gaatgattaa gtgggttggg tatgtttggg gggtagttgt    1380
   gagtttatcg gttatatttg tatagtggtg tggggtgtaa acgttttttt tttggtaggt    1440
   tttgtttagt tagaaataat tttgttttgt agtattgatt ttttggtagt aagttggtga    1500
   cgttgttagt aagtatgttt ttattataga ggttgggggt tgtgagtagt ttaaaaagga    1560
   tgttaggaat tgagggggta ttggtatggt tagtgttttg gggattttta taggattagg    1620
   agtattgata attattttag tacggtatat gttgagtatt tgttttttta gtttggggaa    1680
   gttgtttatt agcggggggt aaacgatttt ttagtgattt gtttttatat ttttttaggt    1740
   agtaggttgt tgtagggttt ttttttttgg gaaaggtaat ttgtagtttt atatttttgg   1800
   ttatgtgtta gagggaaaat agagttttat ttttttttgt ataatttttt ttttagggaa   1860
   ggtatagtta ttttagagtt tattgggtag atttgtttgt aaggtttagg ttagagaatg    1920
   tgggaagttt ttagcgtttt ttataaatta ttttgatttt tttaaggtta ggtttatttt    1980
   tttatagttt tttttaattt agtatttttt ttgttttttg attttttttt tgttttttgg 2040
   tttgggtagg atttgtgggg gggtttagga aattcgggga ttttatgtaa ttttttaaaa   2100
   aaaaattttg agatttatgg gttatagcgt ttttttttgg gggtagttat tttgtcggtt  2160
   ttttagtttt ttaggtgtgt tttagtaagt tttattttat tagggaagaa atgagttatc    2220
   gtatgtgttg atataggttg aaggtcgtta taaatatata tttgtaggag tttttaattt    2280
   tattaggaat atgttgagag tttgacggtt tgttataacg gcgaattatt ttgttttaag    2340
   tagcgggatt tgttttgttt tttttatgtt gtagtttata gtttttttta gggttaaggt   2400
   aatggaaaac gggattttaa gtttatggat tgttttgtta gaaaggttag ttagagtagg    2460
   tttgtgggtt taggtagttg gtatgataat ttgttagttg ttttagggag ggttttttga   2520
   gatttttttt gtaaaatttg tttggggggt tgtggtatta ggtggaggaa agggggtttt    2580
   tgatgaggtt ttaatatacg tttgttttaa attatattta ttttttttgg tagtagattt    2640
   gagtggggtt atttttttgt cgttttttaa atagaagtag ggtttttagg taggatagtg   2700
   tttgggttgt ttagagtttc gggggcgttt tgtggttcga ggttggaggt aggaatgtaa    2760
   tggggatggt tttaaagttt tagggaggag gggttatatt tttaagagta gaagaatagg    2820
   attagattta ggtttaggaa ttttttatta gggtttgcgg ttggttataa ggaagttaag    2880
   gtagtgggaa aatgattttt tggtgttgag gattagttga aatagtgatt ttttattaga    2940
   gggttgcgat gagtaagtag ttgataggtt aatttcggta gatagattaa tggtcggagt    3000
   t                                                                  3001

<210> 48
   <211> 3001
   <212> DNA
   <213> human
<400> 48
<210> 49
   <211> 3001
   <212> DNA
   <213> human
<400> 49
   ggtttcggtt attggtttgt ttgtcgagat tgatttgtta gttgtttgtt tatcgtagtt      60
   ttttggtgag aagttattgt tttagttgat ttttagtatt agggagttat ttttttattg     120
   ttttggtttt tttgtggtta atcgtaggtt ttggtgagag atttttgagt ttgggtttgg     180
   ttttgttttt ttatttttga gaatatagtt tttttttttt gaggttttga agttattttt     240
   attgtatttt tatttttagt ttcgggttat agggcgtttt cgaaattttg aatagtttaa     300
   gtattgtttt gtttgagagt tttgttttta tttgggggac ggtagagagg tggttttatt     360
   tagatttgtt gttaaaaaga gtgggtgtgg tttagaatag acgtgtgtta aaattttatt     420
   agaaattttt ttttttttat ttggtgttat aattttttag ataagttttg tagaggggat     480
   tttaaagggt tttttttaaa gtagttggta gattgttatg ttagttgttt gggtttatag     540
   gtttgttttg gttagttttt ttggtaggat aatttataga tttagaattt cgttttttat     600
   tgttttggtt ttgaaggagg ttgtgggtta tagtatagag aaagtagagt aagtttcgtt     660
   gtttggaata gggtggttcg tcgttataat aggtcgttag atttttaata tgtttttgat     720
   gagattagaa gtttttgtag gtatgtgttt gtggcggttt ttagtttgta ttaatatata     780
   cgatgattta tttttttttt agtggaatag agtttgttgg aatatatttg gggggttggg     840
   gaatcggtag agtagttatt tttaaagaga gacgttatag tttatgagtt ttagggtttt     900
   tttttaaggg attgtatggg attttcgggt tttttaaatt ttttatagag ttttgtttag     960
   gttaaagagt aaggaaaagg ttaaagggta gaaaaaatgt tgagttagga ggagttatgg    1020
   aaggataaat ttggttttaa agaggttaaa gtggtttata gggggcgttg agggtttttt    1080
   atattttttg gtttaaattt tgtaggtaga tttgtttagt gggttttggg atagttgtgt    1140
   tttttttaag aaaaaaattg tgtagaaaag gatgaaattt tatttttttt ttagtatata    1200
   attaagaata taaggttata gattgttttt tttagagggg aaaattttgt agtaatttgt    1260
   tgtttggaaa agtgtaagag tagattattg gggaatcgtt tgtttttcgt tgatggatag    1320
   tttttttaag ttggaagggt aggtgtttag tatgtatcgt attgggatgg ttgttaatat    1380
   ttttggtttt gtaagagttt ttaggatatt ggttatgtta atgttttttt agtttttggt    1440
   attttttttg ggttgtttat agtttttagt ttttatggtg aagatatatt tgttagtagc    1500
   gttattaatt tgttgttaag agattagtgt tgtaaggtaa ggttattttt aattgagtag    1560
   agtttgttag gaagaaagcg tttgtatttt atattattgt gtaggtgtga tcggtgagtt    1620
   tatagttgtt ttttaggtat gtttagttta tttaattatt tatagttcga tagttttttc     1680
   gtttagttta gttttggaag ggataaaaag ggggtattat cgtttttggg taatagagtt     1740
   attttttgcg ttttgttgag ttttgttttt tttagtattt tttaatttat tagtgtttgg     1800
   tttttttgtt ttattaggaa taagttatta tgtttcgtta gttaagtgtg ttttttcgga      1860
   gcgggggtag tcgtagtttt agtatcgttt ttgttattat ttcgtttgtt tttcgtatta    1920
   gttttatttt cgtgtttcgg ttcgggggtg gcggtggtgg tggtttcggt agggttagtt    1980
   ttgcgggtgt ttgtggagtg ggtggttatg gtagtcggag tttttataat ttggggggtt    2040
   ttaagaggat atttattagt attagtggtg gtagttttag gaatcggttt ggtgttggtg    2100
   ttggaggcgg ttatggtttt ggaggtggtg tcggtagtgg atttggtttc ggcggtggag    2160
   ttggtggtgg ttttgggttc ggtggcggag ttggttttgg aggtggtttc ggtggttttg    2220
   gtttttttgt ttgttttttt ggaggtattt aagaggttat tgttaattag agttttttga     2280
   ttttttttaa tttgtaaatc gattttagta tttagagggt gaggatcgag gagcgcgagt    2340
   agattaagat ttttaataat aagtttgttt tttttatcga taaggtgagt tacgattttt    2400
   tgtaaaaaat tattgtgggt ttgaaataaa tgttaaagag agagaaagaa ggaagatgtt    2460
   ttggttttgt gtaaatattt ttatagttgt atagttttgt gttttcgttt gtttttgtgt    2520
   tttggatttg tggatacgtt ttgaattaga ttggtagttg ggaagggaga attatttttt     2580
   tttatttaga ggttagttta taagggtttt ttaataattg ttggatagag agatgaattt    2640
   tatgttatat ataaaggggt tgattgtata ggaaggtaga taagtgtatg ggtagtagtt    2700
   tgagtgtttt tgtagttatt tttgagtcgg gatatatttt aagtagtatt tttagtgttt    2760
   tttgtgtttt ggataggtgt tttatttttt tttggaaaag tgagtttggg tgttttagtt     2820
   tgtatttttt tttttggggt ttagggttag gtatagtgta tagaaaagtt ttagagggac     2880
   ggaaagaggt gggaggtatt ttagtgagtt gattatagaa tatgaaattt aagttttttt     2940
   atttttaaat tttgtttttt tttaggtgcg gtttttggag tagtagaata aggttttgga    3000
   t                                                                    3001
<210> 50
   <211> 3001
   <212> DNA
   <213> human
<400> 50
   ggttttggtt attggtttgt ttgttgagat tgatttgtta gttgtttgtt tattgtagtt 60
   ttttggtgag aagttattgt tttagttgat ttttagtatt agggagttat ttttttattg 120
   ttttggtttt tttgtggtta attgtaggtt ttggtgagag atttttgagt ttgggtttgg 180
   ttttgttttt ttatttttga gaatatagtt tttttttttt gaggttttga agttattttt 240
   attgtatttt tatttttagt tttgggttat agggtgtttt tgaaattttg aatagtttaa 300
   gtattgtttt gtttgagagt tttgttttta tttgggggat ggtagagagg tggttttatt 360
   tagatttgtt gttaaaaaga gtgggtgtgg tttagaatag atgtgtgtta aaattttatt 420
   agaaattttt ttttttttat ttggtgttat aattttttag ataagttttg tagaggggat 480
   tttaaagggt tttttttaaa gtagttggta gattgttatg ttagttgttt gggtttatag 540
   gtttgttttg gttagttttt ttggtaggat aatttataga tttagaattt tgttttttat 600
   tgttttggtt ttgaaggagg ttgtgggtta tagtatagag aaagtagagt aagttttgtt 660
   gtttggaata gggtggtttg ttgttataat aggttgttag atttttaata tgtttttgat 720
   gagattagaa gtttttgtag gtatgtgttt gtggtggttt ttagtttgta ttaatatata 780
   tgatgattta tttttttttt agtggaatag agtttgttgg aatatatttg gggggttggg 840
   gaattggtag agtagttatt tttaaagaga gatgttatag tttatgagtt ttagggtttt 900
   tttttaaggg attgtatggg atttttgggt tttttaaatt ttttatagag ttttgtttag 960
   gttaaagagt aaggaaaagg ttaaagggta gaaaaaatgt tgagttagga ggagttatgg 1020
   aaggataaat ttggttttaa agaggttaaa gtggtttata gggggtgttg agggtttttt 1080
   atattttttg gtttaaattt tgtaggtaga tttgtttagt gggttttggg atagttgtgt 1140
   tttttttaag aaaaaaattg tgtagaaaag gatgaaattt tatttttttt ttagtatata 1200
   attaagaata taaggttata gattgttttt tttagagggg aaaattttgt agtaatttgt 1260
   tgtttggaaa agtgtaagag tagattattg gggaattgtt tgttttttgt tgatggatag 1320
   tttttttaag ttggaagggt aggtgtttag tatgtattgt attgggatgg ttgttaatat 1380
   ttttggtttt gtaagagttt ttaggatatt ggttatgtta atgttttttt agtttttggt 1440
   attttttttg ggttgtttat agtttttagt ttttatggtg aagatatatt tgttagtagt 1500
   gttattaatt tgttgttaag agattagtgt tgtaaggtaa ggttattttt aattgagtag 1560
   agtttgttag gaagaaagtg tttgtatttt atattattgt gtaggtgtga ttggtgagtt 1620
   tatagttgtt ttttaggtat gtttagttta tttaattatt tatagtttga tagttttttt 1680
   gtttagttta gttttggaag ggataaaaag gggtattatt tgtttttggg taatagagtt 1740
   attttttgtg ttttgttgag ttttgttttt tttagtattt tttaatttat tagtgtttgg 1800
   tttttttgtt ttattaggaa taagttatta tgttttgtta gttaagtgtg tttttttgga 1860
   gtgggggtag ttgtagtttt agtattgttt ttgttattat tttgtttgtt ttttgtatta 1920
   gttttatttt tgtgttttgg tttgggggtg gtggtggtgg tggttttggt agggttagtt 1980
   ttgtgggtgt ttgtggagtg ggtggttatg gtagttggag tttttataat ttggggggtt 2040
   ttaagaggat atttattagt attagtggtg gtagttttag gaattggttt ggtgttggtg 2100
   ttggaggtgg ttatggtttt ggaggtggtg ttggtagtgg atttggtttt ggtggtggag 2160
   ttggtggtgg ttttgggttt ggtggtggag ttggttttgg aggtggtttt ggtggttttg 2220
   gtttttttgt ttgttttttt ggaggtattt aagaggttat tgttaattag agttttttga 2280
   ttttttttaa tttgtaaatt gattttagta tttagagggt gaggattgag gagtgtgagt 2340
   agattaagat ttttaataat aagtttgttt tttttattga taaggtgagt tatgattttt 2400
   tgtaaaaaat tattgtgggt ttgaaataaa tgttaaagag agagaaagaa ggaagatgtt 2460
   ttggttttgt gtaaatattt ttatagttgt atagttttgt gtttttgttt gtttttgtgt 2520
   tttggatttg tggatatgtt ttgaattaga ttggtagttg ggaagggaga attatttttt 2580
   tttatttaga ggttagttta taagggtttt ttaataattg ttggatagag agatgaattt 2640
   tatgttatat ataaaggggt tgattgtata ggaaggtaga taagtgtatg ggtagtagtt 2700
   tgagtgtttt tgtagttatt tttgagttgg gatatatttt aagtagtatt tttagtgttt 2760
   tttgtgtttt ggataggtgt tttatttttt tttggaaaag tgagtttggg tgttttagtt 2820
   tgtatttttt tttttggggt ttagggttag gtatagtgta tagaaaagtt ttagagggat 2880
   ggaaagaggt gggaggtatt ttagtgagtt gattatagaa tatgaaattt aagttttttt 2940
   atttttaaat tttgtttttt tttaggtgtg gtttttggag tagtagaata aggttttgga 3000
   t 3001
<210> 51
   <211> 50
   <212> DNA
   <213> human
<400> 51
   ccctgaagct ggcaggccag ggactagccc agtggggcag ggtactcacg 50
<210> 52
   <211> 50
   <212> DNA
   <213> human
<400> 52
   ttttgaagtt ggtaggttag ggattagttt agtggggtag ggtatttacg 50
<210> 53
   <211> 50
   <212> DNA
   <213> human
<400> 53
   ttttgaagtt ggtaggttag ggattagttt agtggggtag ggtatttatg 50
<210> 54
   <211> 50
   <212> DNA
   <213> human
<400> 54
   cgtgagtatt ttgttttatt gggttagttt ttggtttgtt agttttaggg 50
<210> 55
   <211> 50
   <212> DNA
   <213> human
<400> 55
   tgtgagtatt ttgttttatt gggttagttt ttggtttgtt agttttaggg 50
<210> 56
   <211> 50
   <212> DNA
   <213> human
<400> 56
   cgggggaggg actgttgaag acaggtctcc acacacagct ccagcagcca 50
<210> 57
   <211> 50
   <212> DNA
   <213> human
<400> 57
   cgggggaggg attgttgaag ataggttttt atatatagtt ttagtagtta 50
<210> 58
   <211> 50
   <212> DNA
   <213> human
<400> 58
   tgggggaggg attgttgaag ataggttttt atatatagtt ttagtagtta 50
<210> 59
   <211> 50
   <212> DNA
   <213> human
<400> 59
   tggttgttgg agttgtgtgt ggagatttgt ttttaatagt ttttttttcg 50
<210> 60
   <211> 50
   <212> DNA
   <213> human
<400> 60
   tggttgttgg agttgtgtgt ggagatttgt ttttaatagt tttttttttg 50
<210> 61
   <211> 3001
   <212> DNA
   <213> human
<400> 61
   tctggtggcc cctctgtggc gtgtctgtct caagaatccc ttcttgtctc acccagactc 60
   acaggtcata aggttgtttg tctccaaagc ttgtttatgt tgggccctgg tctccggcca 120
   ctcgcatccg cccacacaca ctctaaccgc tcctcccagc agagaagcac agggtctggc 180
   aaatgtcccc tgtggtcaga gctggctccg tctgaacaga cccattttct ctgccccgct 240
   ccacccttac ctctcctcag cggcagcagg gcagagtgct gaacccagga ccccacagat 300
   cctccccgct cctgtctccc ggtgacaagg gtcctggaac ggggcgtctc tgactccctg 360
   ctccaggacg ggtttagtac aggcactcac aaccccctgg ggtgcggcgg gtggggctgg 420
   aagaggcatt ctcttttctg tccaccccgt cacacactcc tgcacgcgct gttgctcgat 480
   catccaaagc tccttcctga attcctgtct gagctggctc tgggggaggg acctgggagc 540
   ccaggaagaa aaaatgatct ccagcctccc tccagggtca gccatgagac tcacccggca 600
   aggagatggg gtagagtgag ctggagcctc agggctgagg tttataagca gcggggaagg 660
   aggagagagc tgcttccaag cccggaggtg tcaaatttcc gtggtcgatt ctgaagacca 720
   gagacagccc aatcaggccg cttgccctga gccctcaggt ctggacccgg gctccctgcc 780
   agccacacga gcgcccccac ttggtcaggg agaggagctg cctggacaga tctcgttcgg 840
   caggggtccc agggtcctag tcctctcaat actgggggct gctgccacca tacccatgtc 900
   taatcaggag tgtgtatgct gaggggggaa gagggaggca accaatcaag ggagctcaac 960
   actgagaggc ccaatctgag acacacacag aggccccttg gaatgagatc ccacaaagga 1020
   tgctacagac caagatccct cagagaaccc caggctgagg ccccacacac agagggggat 1080
   ccaggttgag acccccagag aagagtcctg gattgaaatc cctctgtggg ttctcagact 1140
   cccaagacta agtttctcac tgagggtcct ctcagtgagg ccccatggag gagccacaga 1200
   ctaagacccc ctcagcagaa ttcccccctt accgggctca accagcacag ccagccacag 1260
   gtactgcaag gcccagcttg gggtaagaca tccagtgtcc ctcccaggac ctccttccca 1320
   cctctttccc ctgcaccatc cactgctgcc tggagagaaa ggaacggggc aagcaaggct 1380
   gggaaagaga caagggccgc ctgttccagc ccaggagaca gagggcgcct ctgtctgctc 1440
   ctggcccctt gccccacagg gtgttcgtct gtgaaggggt ggagtcggtg ggggggtcag 1500
   cgggggaggg actgttgaag acaggtctcc acacacagct ccagcagcca catttgcaac 1560
   cttggccatc tgtccagaac ctgctcccac ctcaggccca ggccaaccgt gagtaccctg 1620
   ccccactggg ctagtccctg gcctgccagc ttcagggaga ggggtcttca gaagggctcc 1680
   aagaggctgg ggaccatagc actgtggagc actgaggatc tgggaggagt cagtcagggt 1740
   gagggcagtt tgggatttgg gggagacagg gtttggaagg tggtgatgag agacaaatga 1800
   actgaaggtc ggagagagag ctggcagctc agcaaaggag gaagccagtg gggaacccac 1860
   catgagcttc ctgaccgcct ggccctccca cagggagccc tcaagtccct gccaaggccc 1920
   cctctgtctc ccaggtgttg ggaagggtcc cagccctccc ctgcctcgcc tcctgtgggg 1980
   taagaagaga gcagatacaa cagctgtttc tgcccccacc tctcctgcca gccttagtct 2040
   ctgccacccc caccctgcct tgccaagagc tcaggtcccg gggagtctgg ggggtggcag 2100
   ggcaaagctc ccatgatatg gggaagcaga gatgtagggt gctgtgcccc ttcccaactc 2160
   gacctccaca ggacctcccc tttctcctcc tcccattttt ggcccagacc tcttgactcc 2220
   tctttccccc cattcttacc tctttgtttc cccatctccc ctgcccccaa gtcctctcag 2280
   aactgccacc acgtaaaatc ccaggctgct ttagggctcc aggtgacctc cagctgcccc 2340
   tctggcatgg agcaggtaat caagctacca gcacccctcc ctcaagcacc ccttatcccc 2400
   tcatcagcca cagcttgggt tccatctccc ccatgtctct gtgacaactg cttctcaggt 2460
   ccggagttca gatgacactc acaagggccc cattgaagaa ctgggatgtc attcgatcag 2520
   gggccattgt ccagccccct aggcctgggg aaggatgggg acatctgatc cgagacacct 2580
   gagctgcccc ctctggggtt gtggaaggcc agactgtccc agggccaagg gaaagaggcc 2640
   ccccggcttg gcagtcttct ctctcaacag accactcctt tctcctttct tctcctactc 2700
   tctcccagcc ccttgaactc aaaggaccgc atgctttcag ccctttctct cccccaacac 2760
   acagcagccc cattcccctt gctcccacta tccccgaatc aaccaggagt gagcagttgc   2820
   agggacaacg cctgcaggtc tcctctccca ctccctggga actctggctc caaggaaaag   2880
   gctcagacat tcctctctcc ccttctgcca cccaccagat ggaagggata attttgcaga   2940
   ggcaatggga gcatatccca aagaagccaa aatgacatgt tcaggagaga acagagttga   3000
   a                                                                   3001
<210> 62
   <211> 3001
   <212> DNA
   <213> human
<400> 62
   tttggtggtt tttttgtggc gtgtttgttt taagaatttt tttttgtttt atttagattt     60
   ataggttata aggttgtttg tttttaaagt ttgtttatgt tgggttttgg ttttcggtta    120
   ttcgtattcg tttatatata ttttaatcgt tttttttagt agagaagtat agggtttggt     180
   aaatgttttt tgtggttaga gttggtttcg tttgaataga tttatttttt ttgtttcgtt    240
   ttatttttat ttttttttag cggtagtagg gtagagtgtt gaatttagga ttttatagat     300
   ttttttcgtt tttgtttttc ggtgataagg gttttggaac ggggcgtttt tgattttttg       360
   ttttaggacg ggtttagtat aggtatttat aattttttgg ggtgcggcgg gtggggttgg     420
   aagaggtatt tttttttttg tttatttcgt tatatatttt tgtacgcgtt gttgttcgat     480
   tatttaaagt ttttttttga atttttgttt gagttggttt tgggggaggg atttgggagt    540
   ttaggaagaa aaaatgattt ttagtttttt tttagggtta gttatgagat ttattcggta    600
   aggagatggg gtagagtgag ttggagtttt agggttgagg tttataagta gcggggaagg    660
   aggagagagt tgtttttaag ttcggaggtg ttaaattttc gtggtcgatt ttgaagatta    720
   gagatagttt aattaggtcg tttgttttga gtttttaggt ttggattcgg gttttttgtt     780
   agttatacga gcgtttttat ttggttaggg agaggagttg tttggataga tttcgttcgg    840
   taggggtttt agggttttag tttttttaat attgggggtt gttgttatta tatttatgtt    900
   taattaggag tgtgtatgtt gaggggggaa gagggaggta attaattaag ggagtttaat     960
   attgagaggt ttaatttgag atatatatag aggttttttg gaatgagatt ttataaagga    1020
   tgttatagat taagattttt tagagaattt taggttgagg ttttatatat agagggggat   1080
   ttaggttgag atttttagag aagagttttg gattgaaatt tttttgtggg tttttagatt   1140
   tttaagatta agttttttat tgagggtttt tttagtgagg ttttatggag gagttataga    1200
   ttaagatttt tttagtagaa tttttttttt atcgggttta attagtatag ttagttatag   1260
   gtattgtaag gtttagtttg gggtaagata tttagtgttt tttttaggat ttttttttta    1320
   tttttttttt ttgtattatt tattgttgtt tggagagaaa ggaacggggt aagtaaggtt   1380
   gggaaagaga taagggtcgt ttgttttagt ttaggagata gagggcgttt ttgtttgttt   1440
   ttggtttttt gttttatagg gtgttcgttt gtgaaggggt ggagtcggtg ggggggttag  1500
   cgggggaggg attgttgaag ataggttttt atatatagtt ttagtagtta tatttgtaat   1560
   tttggttatt tgtttagaat ttgtttttat tttaggttta ggttaatcgt gagtattttg   1620
   ttttattggg ttagtttttg gtttgttagt tttagggaga ggggttttta gaagggtttt   1680
   aagaggttgg ggattatagt attgtggagt attgaggatt tgggaggagt tagttagggt   1740
   gagggtagtt tgggatttgg gggagatagg gtttggaagg tggtgatgag agataaatga   1800
   attgaaggtc ggagagagag ttggtagttt agtaaaggag gaagttagtg gggaatttat   1860
   tatgagtttt ttgatcgttt ggttttttta tagggagttt ttaagttttt gttaaggttt    1920
   tttttgtttt ttaggtgttg ggaagggttt tagttttttt ttgtttcgtt ttttgtgggg    1980
   taagaagaga gtagatataa tagttgtttt tgtttttatt ttttttgtta gttttagttt     2040
   ttgttatttt tattttgttt tgttaagagt ttaggtttcg ggggagtttg ggggtggtag   2100
   ggtaaagttt ttatgatatg gggaagtaga gatgtagggt gttgtgtttt tttttaattc   2160
   gatttttata ggattttttt tttttttttt ttttattttt ggtttagatt ttttgatttt   2220
   tttttttttt tatttttatt tttttgtttt tttatttttt ttgtttttaa gtttttttag   2280
   aattgttatt acgtaaaatt ttaggttgtt ttagggtttt aggtgatttt tagttgtttt   2340
   tttggtatgg agtaggtaat taagttatta gtattttttt tttaagtatt ttttattttt   2400
   ttattagtta tagtttgggt tttatttttt ttatgttttt gtgataattg ttttttaggt   2460
   tcggagttta gatgatattt ataagggttt tattgaagaa ttgggatgtt attcgattag   2520
   gggttattgt ttagtttttt aggtttgggg aaggatgggg atatttgatt cgagatattt   2580
   gagttgtttt ttttggggtt gtggaaggtt agattgtttt agggttaagg gaaagaggtt   2640
   tttcggtttg gtagtttttt tttttaatag attatttttt tttttttttt ttttttattt    2700
   ttttttagtt ttttgaattt aaaggatcgt atgtttttag tttttttttt tttttaatat   2760
   atagtagttt tatttttttt gtttttatta ttttcgaatt aattaggagt gagtagttgt   2820
   agggataacg tttgtaggtt ttttttttta ttttttggga attttggttt taaggaaaag    2880
   gtttagatat tttttttttt ttttttgtta tttattagat ggaagggata attttgtaga    2940
   ggtaatggga gtatatttta aagaagttaa aatgatatgt ttaggagaga atagagttga   3000
   a                                                                   3001
<210> 63
   <211> 3001
   <212> DNA
   <213> human
<400> 63
   tttggtggtt tttttgtggt gtgtttgttt taagaatttt tttttgtttt atttagattt     60
   ataggttata aggttgtttg tttttaaagt ttgtttatgt tgggttttgg tttttggtta    120
   tttgtatttg tttatatata ttttaattgt tttttttagt agagaagtat agggtttggt    180
   aaatgttttt tgtggttaga gttggttttg tttgaataga tttatttttt ttgttttgtt    240
   ttatttttat ttttttttag tggtagtagg gtagagtgtt gaatttagga ttttatagat    300
   tttttttgtt tttgtttttt ggtgataagg gttttggaat ggggtgtttt tgattttttg    360
   ttttaggatg ggtttagtat aggtatttat aattttttgg ggtgtggtgg gtggggttgg    420
   aagaggtatt tttttttttg tttattttgt tatatatttt tgtatgtgtt gttgtttgat    480
   tatttaaagt ttttttttga atttttgttt gagttggttt tgggggaggg atttgggagt    540
   ttaggaagaa aaaatgattt ttagtttttt tttagggtta gttatgagat ttatttggta    600
   aggagatggg gtagagtgag ttggagtttt agggttgagg tttataagta gtggggaagg    660
   aggagagagt tgtttttaag tttggaggtg ttaaattttt gtggttgatt ttgaagatta    720
   gagatagttt aattaggttg tttgttttga gtttttaggt ttggatttgg gttttttgtt    780
   agttatatga gtgtttttat ttggttaggg agaggagttg tttggataga ttttgtttgg    840
   taggggtttt agggttttag tttttttaat attgggggtt gttgttatta tatttatgtt    900
   taattaggag tgtgtatgtt gaggggggaa gagggaggta attaattaag ggagtttaat    960
   attgagaggt ttaatttgag atatatatag aggttttttg gaatgagatt ttataaagga   1020
   tgttatagat taagattttt tagagaattt taggttgagg ttttatatat agagggggat   1080
   ttaggttgag atttttagag aagagttttg gattgaaatt tttttgtggg tttttagatt   1140
   tttaagatta agttttttat tgagggtttt tttagtgagg ttttatggag gagttataga   1200
   ttaagatttt tttagtagaa tttttttttt attgggttta attagtatag ttagttatag   1260
   gtattgtaag gtttagtttg gggtaagata tttagtgttt tttttaggat ttttttttta   1320
   tttttttttt ttgtattatt tattgttgtt tggagagaaa ggaatggggt aagtaaggtt   1380
   gggaaagaga taagggttgt ttgttttagt ttaggagata gagggtgttt ttgtttgttt   1440
   ttggtttttt gttttatagg gtgtttgttt gtgaaggggt ggagttggtg ggggggttag   1500
   tgggggaggg attgttgaag ataggttttt atatatagtt ttagtagtta tatttgtaat   1560
   tttggttatt tgtttagaat ttgtttttat tttaggttta ggttaattgt gagtattttg   1620
   ttttattggg ttagtttttg gtttgttagt tttagggaga ggggttttta gaagggtttt   1680
   aagaggttgg ggattatagt attgtggagt attgaggatt tgggaggagt tagttagggt   1740
   gagggtagtt tgggatttgg gggagatagg gtttggaagg tggtgatgag agataaatga   1800
   attgaaggtt ggagagagag ttggtagttt agtaaaggag gaagttagtg gggaatttat   1860
   tatgagtttt ttgattgttt ggttttttta tagggagttt ttaagttttt gttaaggttt   1920
   tttttgtttt ttaggtgttg ggaagggttt tagttttttt ttgttttgtt ttttgtgggg   1980
   taagaagaga gtagatataa tagttgtttt tgtttttatt ttttttgtta gttttagttt   2040
   ttgttatttt tattttgttt tgttaagagt ttaggttttg ggggagtttg ggggtggtag   2100
   ggtaaagttt ttatgatatg gggaagtaga gatgtagggt gttgtgtttt tttttaattt   2160
   gatttttata ggattttttt tttttttttt ttttattttt ggtttagatt ttttgatttt   2220
   tttttttttt tatttttatt tttttgtttt tttatttttt ttgtttttaa gtttttttag   2280
   aattgttatt atgtaaaatt ttaggttgtt ttagggtttt aggtgatttt tagttgtttt   2340
   tttggtatgg agtaggtaat taagttatta gtattttttt tttaagtatt ttttattttt   2400
   ttattagtta tagtttgggt tttatttttt ttatgttttt gtgataattg ttttttaggt   2460
   ttggagttta gatgatattt ataagggttt tattgaagaa ttgggatgtt atttgattag   2520
   gggttattgt ttagtttttt aggtttgggg aaggatgggg atatttgatt tgagatattt   2580
   gagttgtttt ttttggggtt gtggaaggtt agattgtttt agggttaagg gaaagaggtt   2640
   ttttggtttg gtagtttttt tttttaatag attatttttt tttttttttt ttttttattt   2700
   ttttttagtt ttttgaattt aaaggattgt atgtttttag tttttttttt tttttaatat   2760
   atagtagttt tatttttttt gtttttatta tttttgaatt aattaggagt gagtagttgt   2820
   agggataatg tttgtaggtt ttttttttta ttttttggga attttggttt taaggaaaag   2880
   gtttagatat tttttttttt ttttttgtta tttattagat ggaagggata attttgtaga   2940
   ggtaatggga gtatatttta aagaagttaa aatgatatgt ttaggagaga atagagttga   3000
   a                                                                 3001
<210> 64
   <211> 3001
   <212> DNA
   <213> human
<400> 64
   tttaattttg tttttttttg aatatgttat tttggttttt ttgggatatg tttttattgt 60
   ttttgtaaaa ttattttttt tatttggtgg gtggtagaag gggagagagg aatgtttgag 120
   tttttttttt ggagttagag tttttaggga gtgggagagg agatttgtag gcgttgtttt 180
   tgtaattgtt tatttttggt tgattcgggg atagtgggag taaggggaat ggggttgttg 240
   tgtgttgggg gagagaaagg gttgaaagta tgcggttttt tgagtttaag gggttgggag 300
   agagtaggag aagaaaggag aaaggagtgg tttgttgaga gagaagattg ttaagtcggg 360
   gggttttttt tttttggttt tgggatagtt tggtttttta taattttaga gggggtagtt 420
   taggtgtttc ggattagatg tttttatttt tttttaggtt tagggggttg gataatggtt 480
   tttgatcgaa tgatatttta gttttttaat ggggtttttg tgagtgttat ttgaatttcg 540
   gatttgagaa gtagttgtta tagagatatg ggggagatgg aatttaagtt gtggttgatg 600
   aggggataag gggtgtttga gggaggggtg ttggtagttt gattatttgt tttatgttag 660
   aggggtagtt ggaggttatt tggagtttta aagtagtttg ggattttacg tggtggtagt 720
   tttgagagga tttgggggta ggggagatgg ggaaataaag aggtaagaat ggggggaaag 780
   aggagttaag aggtttgggt taaaaatggg aggaggagaa aggggaggtt ttgtggaggt 840
   cgagttggga aggggtatag tattttatat ttttgttttt ttatattatg ggagttttgt 900
   tttgttattt ttagattttt tcgggatttg agtttttggt aaggtagggt gggggtggta 960
   gagattaagg ttggtaggag aggtgggggt agaaatagtt gttgtatttg tttttttttt 1020
   attttatagg aggcgaggta ggggagggtt gggatttttt ttaatatttg ggagatagag 1080
   ggggttttgg tagggatttg agggtttttt gtgggagggt taggcggtta ggaagtttat 1140
   ggtgggtttt ttattggttt ttttttttgt tgagttgtta gttttttttt cgatttttag 1200
   tttatttgtt ttttattatt attttttaaa ttttgttttt tttaaatttt aaattgtttt 1260
   tattttgatt gatttttttt agatttttag tgttttatag tgttatggtt tttagttttt 1320
   tggagttttt ttgaagattt ttttttttga agttggtagg ttagggatta gtttagtggg 1380
   gtagggtatt tacggttggt ttgggtttga ggtgggagta ggttttggat agatggttaa 1440
   ggttgtaaat gtggttgttg gagttgtgtg tggagatttg tttttaatag tttttttttc 1500
   gttgattttt ttatcgattt tattttttta tagacgaata ttttgtgggg taaggggtta 1560
   ggagtagata gaggcgtttt ttgttttttg ggttggaata ggcggttttt gttttttttt 1620
   tagttttgtt tgtttcgttt tttttttttt aggtagtagt ggatggtgta ggggaaagag 1680
   gtgggaagga ggttttggga gggatattgg atgttttatt ttaagttggg ttttgtagta 1740
   tttgtggttg gttgtgttgg ttgagttcgg taagggggga attttgttga gggggtttta 1800
   gtttgtggtt tttttatggg gttttattga gaggattttt agtgagaaat ttagttttgg 1860
   gagtttgaga atttatagag ggattttaat ttaggatttt tttttggggg ttttaatttg 1920
   gatttttttt tgtgtgtggg gttttagttt ggggtttttt gagggatttt ggtttgtagt 1980
   attttttgtg ggattttatt ttaaggggtt tttgtgtgtg ttttagattg ggttttttag 2040
   tgttgagttt ttttgattgg ttgttttttt tttttttttt tagtatatat atttttgatt 2100
   agatatgggt atggtggtag tagtttttag tattgagagg attaggattt tgggattttt 2160
   gtcgaacgag atttgtttag gtagtttttt tttttgatta agtgggggcg ttcgtgtggt 2220
   tggtagggag ttcgggttta gatttgaggg tttagggtaa gcggtttgat tgggttgttt 2280
   ttggttttta gaatcgatta cggaaatttg atattttcgg gtttggaagt agtttttttt 2340
   ttttttttcg ttgtttataa attttagttt tgaggtttta gtttatttta ttttattttt 2400
   ttgtcgggtg agttttatgg ttgattttgg agggaggttg gagattattt tttttttttg 2460
   ggtttttagg tttttttttt agagttagtt tagataggaa tttaggaagg agttttggat 2520
   gatcgagtaa tagcgcgtgt aggagtgtgt gacggggtgg atagaaaaga gaatgttttt 2580
   tttagtttta ttcgtcgtat tttagggggt tgtgagtgtt tgtattaaat tcgttttgga 2640
   gtagggagtt agagacgttt cgttttagga tttttgttat cgggagatag gagcggggag 2700
   gatttgtggg gttttgggtt tagtattttg ttttgttgtc gttgaggaga ggtaagggtg 2760
   gagcggggta gagaaaatgg gtttgtttag acggagttag ttttgattat aggggatatt 2820
   tgttagattt tgtgtttttt tgttgggagg agcggttaga gtgtgtgtgg gcggatgcga 2880
   gtggtcggag attagggttt aatataaata agttttggag ataaataatt ttatgatttg 2940
   tgagtttggg tgagataaga agggattttt gagatagata cgttatagag gggttattag 3000
   a 3001
<210> 65
   <211> 3001
   <212> DNA
   <213> human
<400> 65
   tttaattttg tttttttttg aatatgttat tttggttttt ttgggatatg tttttattgt      60
   ttttgtaaaa ttattttttt tatttggtgg gtggtagaag gggagagagg aatgtttgag   120
   tttttttttt ggagttagag tttttaggga gtgggagagg agatttgtag gtgttgtttt   180
   tgtaattgtt tatttttggt tgatttgggg atagtgggag taaggggaat ggggttgttg   240
   tgtgttgggg gagagaaagg gttgaaagta tgtggttttt tgagtttaag gggttgggag   300
   agagtaggag aagaaaggag aaaggagtgg tttgttgaga gagaagattg ttaagttggg   360
   gggttttttt tttttggttt tgggatagtt tggtttttta taattttaga gggggtagtt   420
   taggtgtttt ggattagatg tttttatttt tttttaggtt tagggggttg gataatggtt   480
   tttgattgaa tgatatttta gttttttaat ggggtttttg tgagtgttat ttgaattttg   540
   gatttgagaa gtagttgtta tagagatatg ggggagatgg aatttaagtt gtggttgatg   600
   aggggataag gggtgtttga gggaggggtg ttggtagttt gattatttgt tttatgttag   660
   aggggtagtt ggaggttatt tggagtttta aagtagtttg ggattttatg tggtggtagt   720
   tttgagagga tttgggggta ggggagatgg ggaaataaag aggtaagaat ggggggaaag   780
   aggagttaag aggtttgggt taaaaatggg aggaggagaa aggggaggtt ttgtggaggt   840
   tgagttggga aggggtatag tattttatat ttttgttttt ttatattatg ggagttttgt   900
   tttgttattt ttagattttt ttgggatttg agtttttggt aaggtagggt gggggtggta   960
   gagattaagg ttggtaggag aggtgggggt agaaatagtt gttgtatttg tttttttttt  1020
   attttatagg aggtgaggta ggggagggtt gggatttttt ttaatatttg ggagatagag  1080
   ggggttttgg tagggatttg agggtttttt gtgggagggt taggtggtta ggaagtttat  1140
   ggtgggtttt ttattggttt ttttttttgt tgagttgtta gttttttttt tgatttttag  1200
   tttatttgtt ttttattatt attttttaaa ttttgttttt tttaaatttt aaattgtttt  1260
   tattttgatt gatttttttt agatttttag tgttttatag tgttatggtt tttagttttt  1320
   tggagttttt ttgaagattt ttttttttga agttggtagg ttagggatta gtttagtggg  1380
   gtagggtatt tatggttggt ttgggtttga ggtgggagta ggttttggat agatggttaa  1440
   ggttgtaaat gtggttgttg gagttgtgtg tggagatttg tttttaatag tttttttttt  1500
   gttgattttt ttattgattt tattttttta tagatgaata ttttgtgggg taaggggtta  1560
   ggagtagata gaggtgtttt ttgttttttg ggttggaata ggtggttttt gttttttttt  1620
   tagttttgtt tgttttgttt tttttttttt aggtagtagt ggatggtgta ggggaaagag  1680
   gtgggaagga ggttttggga gggatattgg atgttttatt ttaagttggg ttttgtagta  1740
   tttgtggttg gttgtgttgg ttgagtttgg taagggggga attttgttga gggggtttta  1800
   gtttgtggtt tttttatggg gttttattga gaggattttt agtgagaaat ttagttttgg  1860
   gagtttgaga atttatagag ggattttaat ttaggatttt tttttggggg ttttaatttg  1920
   gatttttttt tgtgtgtggg gttttagttt ggggtttttt gagggatttt ggtttgtagt  1980
   attttttgtg ggattttatt ttaaggggtt tttgtgtgtg ttttagattg ggttttttag  2040
   tgttgagttt ttttgattgg ttgttttttt tttttttttt tagtatatat atttttgatt  2100
   agatatgggt atggtggtag tagtttttag tattgagagg attaggattt tgggattttt  2160
   gttgaatgag atttgtttag gtagtttttt tttttgatta agtgggggtg tttgtgtggt  2220
   tggtagggag tttgggttta gatttgaggg tttagggtaa gtggtttgat tgggttgttt  2280
   ttggttttta gaattgatta tggaaatttg atatttttgg gtttggaagt agtttttttt  2340
   tttttttttg ttgtttataa attttagttt tgaggtttta gtttatttta ttttattttt  2400
   ttgttgggtg agttttatgg ttgattttgg agggaggttg gagattattt tttttttttg  2460
   ggtttttagg tttttttttt agagttagtt tagataggaa tttaggaagg agttttggat  2520
   gattgagtaa tagtgtgtgt aggagtgtgt gatggggtgg atagaaaaga gaatgttttt  2580
   tttagtttta tttgttgtat tttagggggt tgtgagtgtt tgtattaaat ttgttttgga  2640
   gtagggagtt agagatgttt tgttttagga tttttgttat tgggagatag gagtggggag  2700
   gatttgtggg gttttgggtt tagtattttg ttttgttgtt gttgaggaga ggtaagggtg  2760
   gagtggggta gagaaaatgg gtttgtttag atggagttag ttttgattat aggggatatt  2820
   tgttagattt tgtgtttttt tgttgggagg agtggttaga gtgtgtgtgg gtggatgtga  2880
   gtggttggag attagggttt aatataaata agttttggag ataaataatt ttatgatttg  2940
   tgagtttggg tgagataaga agggattttt gagatagata tgttatagag gggttattag  3000
   a                                                                  3001
<210> 66
   <211> 50
   <212> DNA
   <213> human
<400> 66
   gccaaatgtt ttattctctt acgaagatgc gtgtctttgc atgattcccg 50
<210> 67
   <211> 50
   <212> DNA
   <213> human
<400> 67
   gttaaatgtt ttattttttt acgaagatgc gtgtttttgt atgattttcg 50
<210> 68
   <211> 50
   <212> DNA
   <213> human
<400> 68
   gttaaatgtt ttattttttt atgaagatgt gtgtttttgt atgatttttg 50
<210> 69
   <211> 50
   <212> DNA
   <213> human
<400> 69
   cgggaattat gtaaagatac gtattttcgt aagagaataa aatatttggt 50
<210> 70
   <211> 50
   <212> DNA
   <213> human
<400> 70
   tgggaattat gtaaagatat gtatttttgt aagagaataa aatatttggt 50
<210> 71
   <211> 3001
   <212> DNA
   <213> human
<400> 71
   gacctgagga tgtggtcgca tcggtgacct gggcatgtaa ggagggcagt ccaggtcaga 60
   gggcgtgggg ctgctgagct gaggagagag ggttgatggg ctggagtctg gtggcctgct 120
   ctgcacaggg agatgagggg tgctggggac gggtggcaag acgcttcaat cttctcgcca 180
   gaccctctca gtcctgtacc gggactggca gagtaacaga tgtgaatgtg ccttcagtct 240
   gcacaacgct tttttgggac agttgagggc ccactctgat gccttgaaga ctcttcagag 300
   cctcacgatg ttgggactga acaaaggatg gaacctcttc ccggaaaagc tgaacaggac 360
   taaggcaagg agaccttcaa ccaggccagg gccaatgccc aggctccttc ctggccccac 420
   tgtggttgtg aggagggcac atcagcaagg ctgctggagc cgctcgcctc ctttgttgag 480
   gtcgtgggga cccattccca tgtcccagtc catctactct tatccttcca gctcagggta 540
   caagcagggc gacagatatg ggatagggga gagacagcag agatattaaa gtccttgggc 600
   tgagttttgt gttataaagg gcaagggaaa cacagctcct gggaaaacct gaaagtgcgt 660
   gagcgggcac gcaggtattt tcagagctcc tcttccctta gaaaaacaat ccttcccatt 720
   gacatttgaa aggtatttta catgcatgta aatcttgcac tcattgaaaa acgcacaatg 780
   gcatccctgt gatgatcttt gaaactttag cttttacttt ttactttttt ttttttttta 840
   aaagagacag ggtctcgttc tgctgcccag gctggagtgt attacttttt aaagaaatcg 900
   agcatttcct tccatcctgg cggaatttgg agcagcgacc taacgtgccg ttctcttttg 960
   caccactagg tggcccctgg tgctctcaga tgtcggccag gcttgcagac aggtgaatgc 1020
   atctcattgg ctccagggag gcatgatgcc accgggcagc tctttttaag agaaaagcca 1080
   gaaccggtgg agcagcgacc cctgagcagt gttctctgtg ctgagcggcg ggactgagct 1140
   gttgagttag agccaacatg agtgaggtga gtgatgcttc gacctggaga gggaaactta 1200
   ggagtgagaa gggacctcgg ctgaaggatt agtctcagga aatgtgaggg acttagcagt 1260
   gctgaggaac aaattaatcc tgtaattatt tgcttggaga actgacagga ttaatctctt 1320
   ggtttgaatg gtaagtgcag aaattggtta agtttgcaac ttctacaatt agcctgagga 1380
   ggagaaaggt ttgctttctg attgaatggt gactttgaaa agtgctttgg gttttagagc 1440
   tcagaagaga aagccaaatg ttttattctc ttacgaagat gcgtgtcttt gcatgattcc 1500
   cgcggtgata cctttcagaa tgtgagtggg caggtggtca tctggtgaat gcaagccatg 1560
   gaagggaagg ggacaaggtg gggaaagggc tggccgttgg gtggcatttc atggctcagt 1620
   tgtacaaact acctctgcag ttaatcagag caccagggaa agaaatgcaa aaagtagtag 1680
   gaactgggaa acatccgtgt gtatagatct gtggctctaa gaccggaggc ggccccggca 1740
   gaagaagagc tacaattttg caaatccaaa gtcacttctc taaacttgtt tatacagaat 1800
   gtgttttact ggggccaact gacagcacgt ttgtttttct aaaatcatct tctccttttt 1860
   tctcatctat gaagcagatt agcggtggta caacaccatt ctctaattca actcaataac 1920
   cgagccaaat gcatcttcat ttcctcttgg ttggcactta aataattact tttaaagatc 1980
   ttttatcttc tctgtctctt tttgacagat ggatgattct tttcttggaa gtctaggata 2040
   aaattaacaa cttcagaaat aaaatgcctc ctccctgctt ttatctctct gatgggtgat 2100
   ttttgatgta aacatgacaa gaagaagagc aaagcactat aaaacttaga aatggggttt 2160
   aggacaaagc caggggtttg aggcagggag agaccaactg tggggtggaa gatgggaatt 2220
   ctcccaccac cacaatcaat ggtacctgta acagcagcta acacttgcct aatggtctgt 2280
   gtgccgacag tgtcaggctg tctctgcatc tgtcaccttc tttgaacctc tcaacaactc 2340
   cgtgcagtgg gcaccattat tattcccaat tgacagatga ggacactgag gcacagagat 2400
   gggaactaag tggtttgagg tcaccagcta aagaacagta gagccaagac ttgggctgca 2460
   atgctgggtg gttgactgcc gggtgaatct tgatgtctaa atgtgtatgg aatttgctga 2520
   acagagggga tggcagaggt tggaggagga gaggggaggt gaagagaaag aaagaaagga 2580
   gctggggctc tgaaatagag atggaatgat gaggatgaga cctccatttc ccacatgcta 2640
   ggaggagggg ctttctgctg gactctacag atatttcaga agcttctgtg tccacatcag 2700
   ctcaccttgc ttgcaacaag aagggatggc tgcgtggagt ggtttttctt tatatacaac 2760
   ttatccaata agagtttcct atagaagaag gacttagaaa gcagcacagc actgaggggt 2820
   ggcctctcgg gtcaagtcag ctaccctggg attctggctc agtcatgccg tcacttgctg 2880
   aggcttctgg ccagcaaggt gctcatgagc tctccaggat gctcagctct gctgggctca 2940
   ctcaactgtg cgggtctcca gggagaaagc gcgtgttcca gagctcacct gtgattaggg 3000
   t 3001
<210> 72
   <211> 3001
   <212> DNA
   <213> human
<400> 72
   gatttgagga tgtggtcgta tcggtgattt gggtatgtaa ggagggtagt ttaggttaga 60
   gggcgtgggg ttgttgagtt gaggagagag ggttgatggg ttggagtttg gtggtttgtt 120
   ttgtataggg agatgagggg tgttggggac gggtggtaag acgttttaat tttttcgtta 180
   gattttttta gttttgtatc gggattggta gagtaataga tgtgaatgtg tttttagttt 240
   gtataacgtt tttttgggat agttgagggt ttattttgat gttttgaaga ttttttagag 300
   ttttacgatg ttgggattga ataaaggatg gaattttttt tcggaaaagt tgaataggat 360
   taaggtaagg agatttttaa ttaggttagg gttaatgttt aggttttttt ttggttttat 420
   tgtggttgtg aggagggtat attagtaagg ttgttggagt cgttcgtttt ttttgttgag 480
   gtcgtgggga tttattttta tgttttagtt tatttatttt tattttttta gtttagggta 540
   taagtagggc gatagatatg ggatagggga gagatagtag agatattaaa gtttttgggt 600
   tgagttttgt gttataaagg gtaagggaaa tatagttttt gggaaaattt gaaagtgcgt 660
   gagcgggtac gtaggtattt ttagagtttt ttttttttta gaaaaataat tttttttatt 720
   gatatttgaa aggtatttta tatgtatgta aattttgtat ttattgaaaa acgtataatg 780
   gtatttttgt gatgattttt gaaattttag tttttatttt ttattttttt ttttttttta 840
   aaagagatag ggtttcgttt tgttgtttag gttggagtgt attatttttt aaagaaatcg 900
   agtatttttt tttattttgg cggaatttgg agtagcgatt taacgtgtcg tttttttttg 960
   tattattagg tggtttttgg tgtttttaga tgtcggttag gtttgtagat aggtgaatgt 1020
   attttattgg ttttagggag gtatgatgtt atcgggtagt tttttttaag agaaaagtta 1080
   gaatcggtgg agtagcgatt tttgagtagt gttttttgtg ttgagcggcg ggattgagtt 1140
   gttgagttag agttaatatg agtgaggtga gtgatgtttc gatttggaga gggaaattta 1200
   ggagtgagaa gggatttcgg ttgaaggatt agttttagga aatgtgaggg atttagtagt 1260
   gttgaggaat aaattaattt tgtaattatt tgtttggaga attgatagga ttaatttttt 1320
   ggtttgaatg gtaagtgtag aaattggtta agtttgtaat ttttataatt agtttgagga 1380
   ggagaaaggt ttgttttttg attgaatggt gattttgaaa agtgttttgg gttttagagt 1440
   ttagaagaga aagttaaatg ttttattttt ttacgaagat gcgtgttttt gtatgatttt 1500
   cgcggtgata ttttttagaa tgtgagtggg taggtggtta tttggtgaat gtaagttatg 1560
   gaagggaagg ggataaggtg gggaaagggt tggtcgttgg gtggtatttt atggtttagt 1620
   tgtataaatt atttttgtag ttaattagag tattagggaa agaaatgtaa aaagtagtag 1680
   gaattgggaa atattcgtgt gtatagattt gtggttttaa gatcggaggc ggtttcggta 1740
   gaagaagagt tataattttg taaatttaaa gttatttttt taaatttgtt tatatagaat 1800
   gtgttttatt ggggttaatt gatagtacgt ttgttttttt aaaattattt tttttttttt 1860
   ttttatttat gaagtagatt agcggtggta taatattatt ttttaattta atttaataat 1920
   cgagttaaat gtatttttat ttttttttgg ttggtattta aataattatt tttaaagatt 1980
   ttttattttt tttgtttttt tttgatagat ggatgatttt ttttttggaa gtttaggata 2040
   aaattaataa ttttagaaat aaaatgtttt ttttttgttt ttattttttt gatgggtgat 2100
   ttttgatgta aatatgataa gaagaagagt aaagtattat aaaatttaga aatggggttt 2160
   aggataaagt taggggtttg aggtagggag agattaattg tggggtggaa gatgggaatt 2220
   tttttattat tataattaat ggtatttgta atagtagtta atatttgttt aatggtttgt 2280
   gtgtcgatag tgttaggttg tttttgtatt tgttattttt tttgaatttt ttaataattt 2340
   cgtgtagtgg gtattattat tatttttaat tgatagatga ggatattgag gtatagagat 2400
   gggaattaag tggtttgagg ttattagtta aagaatagta gagttaagat ttgggttgta 2460
   atgttgggtg gttgattgtc gggtgaattt tgatgtttaa atgtgtatgg aatttgttga 2520
   atagagggga tggtagaggt tggaggagga gaggggaggt gaagagaaag aaagaaagga 2580
   gttggggttt tgaaatagag atggaatgat gaggatgaga tttttatttt ttatatgtta 2640
   ggaggagggg ttttttgttg gattttatag atattttaga agtttttgtg tttatattag 2700
   tttattttgt ttgtaataag aagggatggt tgcgtggagt ggtttttttt tatatataat 2760
   ttatttaata agagtttttt atagaagaag gatttagaaa gtagtatagt attgaggggt 2820
   ggtttttcgg gttaagttag ttattttggg attttggttt agttatgtcg ttatttgttg 2880
   aggtttttgg ttagtaaggt gtttatgagt tttttaggat gtttagtttt gttgggttta 2940
   tttaattgtg cgggttttta gggagaaagc gcgtgtttta gagtttattt gtgattaggg 3000
   t 3001

<210> 73
   <211> 3001
   <212> DNA
   <213> human
<400> 73
   gatttgagga tgtggttgta ttggtgattt gggtatgtaa ggagggtagt ttaggttaga 60
   gggtgtgggg ttgttgagtt gaggagagag ggttgatggg ttggagtttg gtggtttgtt 120
   ttgtataggg agatgagggg tgttggggat gggtggtaag atgttttaat ttttttgtta 180
   gattttttta gttttgtatt gggattggta gagtaataga tgtgaatgtg tttttagttt 240
   gtataatgtt tttttgggat agttgagggt ttattttgat gttttgaaga ttttttagag 300
   ttttatgatg ttgggattga ataaaggatg gaattttttt ttggaaaagt tgaataggat 360
   taaggtaagg agatttttaa ttaggttagg gttaatgttt aggttttttt ttggttttat 420
   tgtggttgtg aggagggtat attagtaagg ttgttggagt tgtttgtttt ttttgttgag 480
   gttgtgggga tttattttta tgttttagtt tatttatttt tattttttta gtttagggta 540
   taagtagggt gatagatatg ggatagggga gagatagtag agatattaaa gtttttgggt 600
   tgagttttgt gttataaagg gtaagggaaa tatagttttt gggaaaattt gaaagtgtgt 660
   gagtgggtat gtaggtattt ttagagtttt ttttttttta gaaaaataat tttttttatt 720
   gatatttgaa aggtatttta tatgtatgta aattttgtat ttattgaaaa atgtataatg 780
   gtatttttgt gatgattttt gaaattttag tttttatttt ttattttttt ttttttttta 840
   aaagagatag ggttttgttt tgttgtttag gttggagtgt attatttttt aaagaaattg 900
   agtatttttt tttattttgg tggaatttgg agtagtgatt taatgtgttg tttttttttg 960
   tattattagg tggtttttgg tgtttttaga tgttggttag gtttgtagat aggtgaatgt 1020
   attttattgg ttttagggag gtatgatgtt attgggtagt tttttttaag agaaaagtta 1080
   gaattggtgg agtagtgatt tttgagtagt gttttttgtg ttgagtggtg ggattgagtt 1140
   gttgagttag agttaatatg agtgaggtga gtgatgtttt gatttggaga gggaaattta 1200
   ggagtgagaa gggattttgg ttgaaggatt agttttagga aatgtgaggg atttagtagt 1260
   gttgaggaat aaattaattt tgtaattatt tgtttggaga attgatagga ttaatttttt 1320
   ggtttgaatg gtaagtgtag aaattggtta agtttgtaat ttttataatt agtttgagga 1380
   ggagaaaggt ttgttttttg attgaatggt gattttgaaa agtgttttgg gttttagagt 1440
   ttagaagaga aagttaaatg ttttattttt ttatgaagat gtgtgttttt gtatgatttt 1500
   tgtggtgata ttttttagaa tgtgagtggg taggtggtta tttggtgaat gtaagttatg 1560
   gaagggaagg ggataaggtg gggaaagggt tggttgttgg gtggtatttt atggtttagt 1620
   tgtataaatt atttttgtag ttaattagag tattagggaa agaaatgtaa aaagtagtag 1680
   gaattgggaa atatttgtgt gtatagattt gtggttttaa gattggaggt ggttttggta 1740
   gaagaagagt tataattttg taaatttaaa gttatttttt taaatttgtt tatatagaat 1800
   gtgttttatt ggggttaatt gatagtatgt ttgttttttt aaaattattt tttttttttt 1860
   ttttatttat gaagtagatt agtggtggta taatattatt ttttaattta atttaataat 1920
   tgagttaaat gtatttttat ttttttttgg ttggtattta aataattatt tttaaagatt 1980
   ttttattttt tttgtttttt tttgatagat ggatgatttt ttttttggaa gtttaggata 2040
   aaattaataa ttttagaaat aaaatgtttt ttttttgttt ttattttttt gatgggtgat 2100
   ttttgatgta aatatgataa gaagaagagt aaagtattat aaaatttaga aatggggttt 2160
   aggataaagt taggggtttg aggtagggag agattaattg tggggtggaa gatgggaatt 2220
   tttttattat tataattaat ggtatttgta atagtagtta atatttgttt aatggtttgt 2280
   gtgttgatag tgttaggttg tttttgtatt tgttattttt tttgaatttt ttaataattt 2340
   tgtgtagtgg gtattattat tatttttaat tgatagatga ggatattgag gtatagagat 2400
   gggaattaag tggtttgagg ttattagtta aagaatagta gagttaagat ttgggttgta 2460
   atgttgggtg gttgattgtt gggtgaattt tgatgtttaa atgtgtatgg aatttgttga 2520
   atagagggga tggtagaggt tggaggagga gaggggaggt gaagagaaag aaagaaagga 2580
   gttggggttt tgaaatagag atggaatgat gaggatgaga tttttatttt ttatatgtta 2640
   ggaggagggg ttttttgttg gattttatag atattttaga agtttttgtg tttatattag 2700
   tttattttgt ttgtaataag aagggatggt tgtgtggagt ggtttttttt tatatataat 2760
   ttatttaata agagtttttt atagaagaag gatttagaaa gtagtatagt attgaggggt 2820
   ggttttttgg gttaagttag ttattttggg attttggttt agttatgttg ttatttgttg 2880
   aggtttttgg ttagtaaggt gtttatgagt tttttaggat gtttagtttt gttgggttta 2940
   tttaattgtg tgggttttta gggagaaagt gtgtgtttta gagtttattt gtgattaggg 3000
   t 3001
<210> 74
   <211> 3001
   <212> DNA
   <213> human
<400> 74
   attttaatta taggtgagtt ttggaatacg cgtttttttt ttggagattc gtatagttga     60
   gtgagtttag tagagttgag tattttggag agtttatgag tattttgttg gttagaagtt    120
   ttagtaagtg acggtatgat tgagttagaa ttttagggta gttgatttga ttcgagaggt    180
   tattttttag tgttgtgttg ttttttaagt ttttttttta taggaaattt ttattggata     240
   agttgtatat aaagaaaaat tattttacgt agttattttt ttttgttgta agtaaggtga    300
   gttgatgtgg atatagaagt ttttgaaata tttgtagagt ttagtagaaa gttttttttt    360
   ttagtatgtg ggaaatggag gttttatttt tattatttta tttttatttt agagttttag     420
   tttttttttt tttttttttt tatttttttt tttttttttt aatttttgtt attttttttg    480
   tttagtaaat tttatatata tttagatatt aagatttatt cggtagttaa ttatttagta    540
   ttgtagttta agttttggtt ttattgtttt ttagttggtg attttaaatt atttagtttt    600
   tatttttgtg ttttagtgtt tttatttgtt aattgggaat aataatggtg tttattgtac    660
   ggagttgttg agaggtttaa agaaggtgat agatgtagag atagtttgat attgtcggta    720
   tatagattat taggtaagtg ttagttgttg ttataggtat tattgattgt ggtggtggga    780
   gaatttttat tttttatttt atagttggtt tttttttgtt ttaaattttt ggttttgttt    840
   taaattttat ttttaagttt tatagtgttt tgtttttttt tttgttatgt ttatattaaa    900
   aattatttat tagagagata aaagtaggga ggaggtattt tatttttgaa gttgttaatt    960
   ttattttaga tttttaagaa aagaattatt tatttgttaa aaagagatag agaagataaa   1020
   agatttttaa aagtaattat ttaagtgtta attaagagga aatgaagatg tatttggttc   1080
   ggttattgag ttgaattaga gaatggtgtt gtattatcgt taatttgttt tatagatgag  1140
   aaaaaaggag aagatgattt tagaaaaata aacgtgttgt tagttggttt tagtaaaata   1200
   tattttgtat aaataagttt agagaagtga ttttggattt gtaaaattgt agtttttttt   1260
   ttgtcggggt cgttttcggt tttagagtta tagatttata tatacggatg ttttttagtt    1320
   tttattattt tttgtatttt tttttttggt gttttgatta attgtagagg tagtttgtat   1380
   aattgagtta tgaaatgtta tttaacggtt agtttttttt ttattttgtt tttttttttt  1440
   ttatggtttg tatttattag atgattattt gtttatttat attttgaaag gtattatcgc   1500
   gggaattatg taaagatacg tattttcgta agagaataaa atatttggtt tttttttttg   1560
   agttttaaaa tttaaagtat tttttaaagt tattatttaa ttagaaagta aatttttttt   1620
   ttttttaggt taattgtaga agttgtaaat ttaattaatt tttgtattta ttatttaaat   1680
   taagagatta attttgttag ttttttaagt aaataattat aggattaatt tgttttttag     1740
   tattgttaag ttttttatat tttttgagat taatttttta gtcgaggttt ttttttattt     1800
   ttaagttttt ttttttaggt cgaagtatta tttattttat ttatgttggt tttaatttaa   1860
   tagtttagtt tcgtcgttta gtatagagaa tattgtttag gggtcgttgt tttatcggtt   1920
   ttggtttttt ttttaaaaag agttgttcgg tggtattatg tttttttgga gttaatgaga  1980
   tgtatttatt tgtttgtaag tttggtcgat atttgagagt attaggggtt atttagtggt   2040
   gtaaaagaga acggtacgtt aggtcgttgt tttaaatttc gttaggatgg aaggaaatgt   2100
   tcgatttttt taaaaagtaa tatattttag tttgggtagt agaacgagat tttgtttttt   2160
   ttaaaaaaaa aaaaaaagta aaaagtaaaa gttaaagttt taaagattat tatagggatg   2220
   ttattgtgcg ttttttaatg agtgtaagat ttatatgtat gtaaaatatt ttttaaatgt   2280
   taatgggaag gattgttttt ttaagggaag aggagttttg aaaatatttg cgtgttcgtt   2340
   tacgtatttt taggtttttt taggagttgt gttttttttg ttttttataa tataaaattt   2400
   agtttaagga ttttaatatt tttgttgttt tttttttatt ttatatttgt cgttttgttt   2460
   gtattttgag ttggaaggat aagagtagat ggattgggat atgggaatgg gtttttacga   2520
   ttttaataaa ggaggcgagc ggttttagta gttttgttga tgtgtttttt ttataattat   2580
   agtggggtta ggaaggagtt tgggtattgg ttttggtttg gttgaaggtt tttttgtttt   2640
   agttttgttt agttttttcg ggaagaggtt ttattttttg tttagtttta atatcgtgag  2700
   gttttgaaga gtttttaagg tattagagtg ggtttttaat tgttttaaaa aagcgttgtg   2760
   tagattgaag gtatatttat atttgttatt ttgttagttt cggtatagga ttgagagggt   2820
   ttggcgagaa gattgaagcg ttttgttatt cgtttttagt attttttatt tttttgtgta  2880
   gagtaggtta ttagatttta gtttattaat tttttttttt tagtttagta gttttacgtt   2940
   ttttgatttg gattgttttt tttatatgtt taggttatcg atgcgattat atttttaggt   3000
   t                                                                   3001
<210> 75
   <211> 3001
   <212> DNA
   <213> human
<400> 75
   attttaatta taggtgagtt ttggaatatg tgtttttttt ttggagattt gtatagttga      60
   gtgagtttag tagagttgag tattttggag agtttatgag tattttgttg gttagaagtt     120
   ttagtaagtg atggtatgat tgagttagaa ttttagggta gttgatttga tttgagaggt     180
   tattttttag tgttgtgttg ttttttaagt ttttttttta taggaaattt ttattggata     240
   agttgtatat aaagaaaaat tattttatgt agttattttt ttttgttgta agtaaggtga     300
   gttgatgtgg atatagaagt ttttgaaata tttgtagagt ttagtagaaa gttttttttt     360
   ttagtatgtg ggaaatggag gttttatttt tattatttta tttttatttt agagttttag     420
   tttttttttt tttttttttt tatttttttt tttttttttt aatttttgtt attttttttg     480
   tttagtaaat tttatatata tttagatatt aagatttatt tggtagttaa ttatttagta     540
   ttgtagttta agttttggtt ttattgtttt ttagttggtg attttaaatt atttagtttt     600
   tatttttgtg ttttagtgtt tttatttgtt aattgggaat aataatggtg tttattgtat     660
   ggagttgttg agaggtttaa agaaggtgat agatgtagag atagtttgat attgttggta     720
   tatagattat taggtaagtg ttagttgttg ttataggtat tattgattgt ggtggtggga     780
   gaatttttat tttttatttt atagttggtt tttttttgtt ttaaattttt ggttttgttt     840
   taaattttat ttttaagttt tatagtgttt tgtttttttt tttgttatgt ttatattaaa     900
   aattatttat tagagagata aaagtaggga ggaggtattt tatttttgaa gttgttaatt     960
   ttattttaga tttttaagaa aagaattatt tatttgttaa aaagagatag agaagataaa    1020
   agatttttaa aagtaattat ttaagtgtta attaagagga aatgaagatg tatttggttt    1080
   ggttattgag ttgaattaga gaatggtgtt gtattattgt taatttgttt tatagatgag    1140
   aaaaaaggag aagatgattt tagaaaaata aatgtgttgt tagttggttt tagtaaaata    1200
   tattttgtat aaataagttt agagaagtga ttttggattt gtaaaattgt agtttttttt    1260
   ttgttggggt tgtttttggt tttagagtta tagatttata tatatggatg ttttttagtt    1320
   tttattattt tttgtatttt tttttttggt gttttgatta attgtagagg tagtttgtat    1380
   aattgagtta tgaaatgtta tttaatggtt agtttttttt ttattttgtt tttttttttt    1440
   ttatggtttg tatttattag atgattattt gtttatttat attttgaaag gtattattgt    1500
   gggaattatg taaagatatg tatttttgta agagaataaa atatttggtt tttttttttg    1560
   agttttaaaa tttaaagtat tttttaaagt tattatttaa ttagaaagta aatttttttt    1620
   ttttttaggt taattgtaga agttgtaaat ttaattaatt tttgtattta ttatttaaat    1680
   taagagatta attttgttag ttttttaagt aaataattat aggattaatt tgttttttag    1740
   tattgttaag ttttttatat tttttgagat taatttttta gttgaggttt ttttttattt    1800
   ttaagttttt ttttttaggt tgaagtatta tttattttat ttatgttggt tttaatttaa    1860
   tagtttagtt ttgttgttta gtatagagaa tattgtttag gggttgttgt tttattggtt    1920
   ttggtttttt ttttaaaaag agttgtttgg tggtattatg tttttttgga gttaatgaga    1980
   tgtatttatt tgtttgtaag tttggttgat atttgagagt attaggggtt atttagtggt    2040
   gtaaaagaga atggtatgtt aggttgttgt tttaaatttt gttaggatgg aaggaaatgt    2100
   ttgatttttt taaaaagtaa tatattttag tttgggtagt agaatgagat tttgtttttt    2160
   ttaaaaaaaa aaaaaaagta aaaagtaaaa gttaaagttt taaagattat tatagggatg    2220
   ttattgtgtg ttttttaatg agtgtaagat ttatatgtat gtaaaatatt ttttaaatgt    2280
   taatgggaag gattgttttt ttaagggaag aggagttttg aaaatatttg tgtgtttgtt    2340
   tatgtatttt taggtttttt taggagttgt gttttttttg ttttttataa tataaaattt    2400
   agtttaagga ttttaatatt tttgttgttt tttttttatt ttatatttgt tgttttgttt    2460
   gtattttgag ttggaaggat aagagtagat ggattgggat atgggaatgg gtttttatga    2520
   ttttaataaa ggaggtgagt ggttttagta gttttgttga tgtgtttttt ttataattat    2580
   agtggggtta ggaaggagtt tgggtattgg ttttggtttg gttgaaggtt tttttgtttt    2640
   agttttgttt agtttttttg ggaagaggtt ttattttttg tttagtttta atattgtgag    2700
   gttttgaaga gtttttaagg tattagagtg ggtttttaat tgttttaaaa aagtgttgtg    2760
   tagattgaag gtatatttat atttgttatt ttgttagttt tggtatagga ttgagagggt    2820
   ttggtgagaa gattgaagtg ttttgttatt tgtttttagt atttttattt tttttgtgta    2880
   gagtaggtta ttagatttta gtttattaat tttttttttt tagtttagta gttttatgtt    2940
   ttttgatttg gattgttttt tttatatgtt taggttattg atgtgattat atttttaggt    3000
   t                                                                    3001
<210> 76
   <211> 50
   <212> DNA
   <213> human
<400> 76
   cgtgccctgt gatgccttct ctccctcact ttgagcatgt ggttggggcg 50
<210> 77
   <211> 50
   <212> DNA
   <213> human
<400> 77
   cgtgttttgt gatgtttttt tttttttatt ttgagtatgt ggttggggcg 50
<210> 78
   <211> 50
   <212> DNA
   <213> human
<400> 78
   tgtgttttgt gatgtttttt tttttttatt ttgagtatgt ggttggggtg 50
<210> 79
   <211> 50
   <212> DNA
   <213> human
<400> 79
   cgttttaatt atatgtttaa agtgagggag agaaggtatt atagggtacg 50
<210> 80
   <211> 50
   <212> DNA
   <213> human
<400> 80
   tgttttaatt atatgtttaa agtgagggag agaaggtatt atagggtatg 50
<210> 81
   <211> 3001
   <212> DNA
   <213> human
<400> 81
   cttctagaaa aagaccctag aagagtacct acagagtgat cttcaggcag cctcagactt    60
   cagcccccag gcccttgggg caaggccggg cagttggccc tttgagtgga ggcctgtctt   120
   ctcctcacta tgaattcccc atctccaagc acattccctg tccgtaagtt agatgcacag   180
   taaatatgag taaataaatg aataaaagaa caaataagtg agtgtttgca ggaaactaaa   240
   gtagaccctg ataatctaca ggctcctgca ggcttctcca gcctcttctg ccactcccag   300
   cacaactcca tttcccactc ctagaattga ctcaagcctc ctccatccaa tccccgactc   360
   actgggccat ttcacacttt tgtgcctttg cacacgtggg tccctgtgct tggatcacca   420
   tgggaacctg tacgtgtaga aaaacacacc cctaccccca gaactctgag aaatatcctg 480
   tcctgtcctg taggatagga tgatcaggag ttcaagcatg tgtgtgcaca aaataaacac 540
   cagtgtgagc atgtgtgcac aggagacacc caacagttcc aagaaggcta aacttgggca 600
   gaaaattcca ggtgggagag aaaattttct gtcttatgga cagcccattt cccttttccc 660
   ttctaactag gataatggta atagttagta tttgttgaat gctgtgtgtc aggccctact 720
   ggaaagcact ttacctgtag gaacccatat ggtgctcctg ataacccttt gcactatcat 780
   tattcccact gtatagatca gggaacagac acaggtaggt tttggatgtg tggttacaca 840
   cccagaaagt caggaagtct ggctccagag ctgtgtactt aactgctgcc acactacagg 900
   aatgacagcc ctggggggat gaactaagag gtgctggatg agggtcctgg cctctaaagg 960
   cacagctgtt ctccaactct tgcaaggctg aaaccagaag atggcgggca ttgcagctgg 1020
   tggagggtct gaatacagct gtgaggatag tgatccctgg gctaggctct gcaaggaaac 1080
   tgagcagtgc agggccttac cagccccaac catctggggg ccaccctggc tggcaccagc 1140
   aggagggtgg gctggcttct cagaggtctg ggagactcag cctccttctg ccagggctgc 1200
   agtggccgac tcctcctccc ctctgtcccc accctgcacc gcctccagac cccagtcctg 1260
   actattgctt aatccccagg agcccagttc ctgtgggcag cgcctgacat gcctgatcct 1320
   ctcttttctg cagttcaagg gaaagacgag atcttgcaca aggcactctg cttctgccct 1380
   tggctgggga agggtggcat ggagcctctc cggctgctca tcttactctt tgtcacaggt 1440
   aggatcccct ccgtgccctg tgatgccttc tctccctcac tttgagcatg tggttggggc 1500
   gcgggtggtg ggccctgggg tgaattatga aggcggtggc agtcctcgca tgcctgagtg 1560
   ccctgttctg cactcctctc ctccccaccc tgcccatatt cttcacaccc tccttccctc 1620
   cccagtatca aaacctaccc tcagactctt tctgcatgtg gacatgtaag gggctggtgg 1680
   actctggttg cattggaagg gaaggagtgc taacagtggc atcccaggca ctagctggca 1740
   gttgggggaa gctttggggg gcactggcac tggtaatagc ctctgaaatt ataagccact 1800
   aattatgagc ccctacagtt ataaaggagg aaagaacctg aggatgttca tctgcatctt 1860
   tggggcactc tctcccctgc tctgaaggtc cccttgtcct cagctcttga tgggaggtga 1920
   ggagcgtaga gatttccgtt gctgagttga ggggaattga cacaccaaat ttttgtacac 1980
   aattgcttct tcccgctgag gaaagcgcac cttttgctcc cccagagcac tggacaaacc 2040
   tgggcaagag gagagacggt gccaaatgga gtcttgttcc tgcagcttct tagatggtgg 2100
   tcagggggaa ggcggggttc tgaggcatgg atgggaggtg gtcagatggg agagggccac 2160
   ggcccatctg gtctctctag ttccaaaagg caagccacca acttcccaat cctatctttc 2220
   aagcctctgt cacagtaatg gttgaagatg gcaggcaagg gaggaccaag agagaagtca 2280
   aagcaggggg ctctgggggc tgccccagca acaggctggt gctctaagcc catctccccc 2340
   gccctaagga gggttcccaa aatagcagcc tcatgtctcc cccaaaatat ctccgagacg 2400
   ggtccttcct gaaaggggaa caaagccaca gaaataggga agctggaagt taaaggtcag 2460
   gaaagtcggt accaatgtgg gcggctgcag agcaagcaag agtggcgggg cagggagagc 2520
   cagccccagg ccgagaggag aggtcccagt cccaaatgat ggcaaagaga tgtgcagaac 2580
   agaactggag ggggttttaa gaccaagtgc ctccagaata gacccagaga aggtcagtta 2640
   cttctccaag gaggcccagc aagagcaaca gtgagaaaac caaacccagg tcccaggttt 2700
   cctggttccc aatttcccac aaacacatgc tgtgccatcc gctcccaact tgtataagaa 2760
   cctaagctcc ttcagggcag gatttttgtc tgtttaggtc actgcacaga gcaagtgttc 2820
   aaagcgtatt tggtaaacaa aggagtgaat gaatgtctcc tccccagagc tgtccggagc 2880
   ccacaacacc acagtgttcc agggcgtggc gggccagtcc ctgcaggtgt cttgccccta 2940
   tgactccatg aagcactggg ggaggcgcaa ggcctggtgc cgccagctgg gagagaaggg 3000
   c                                                                 3001
<210> 82
   <211> 3001
   <212> DNA
   <213> human
<400> 82
   tttttagaaa aagattttag aagagtattt atagagtgat ttttaggtag ttttagattt      60
   tagtttttag gtttttgggg taaggtcggg tagttggttt tttgagtgga ggtttgtttt     120
   ttttttatta tgaatttttt atttttaagt atattttttg ttcgtaagtt agatgtatag      180
   taaatatgag taaataaatg aataaaagaa taaataagtg agtgtttgta ggaaattaaa     240
   gtagattttg ataatttata ggtttttgta ggttttttta gttttttttg ttatttttag     300
   tataatttta ttttttattt ttagaattga tttaagtttt ttttatttaa ttttcgattt     360
   attgggttat tttatatttt tgtgtttttg tatacgtggg tttttgtgtt tggattatta     420
   tgggaatttg tacgtgtaga aaaatatatt tttattttta gaattttgag aaatattttg     480
   ttttgttttg taggatagga tgattaggag tttaagtatg tgtgtgtata aaataaatat     540
   tagtgtgagt atgtgtgtat aggagatatt taatagtttt aagaaggtta aatttgggta     600
   gaaaatttta ggtgggagag aaaatttttt gttttatgga tagtttattt tttttttttt     660
   ttttaattag gataatggta atagttagta tttgttgaat gttgtgtgtt aggttttatt     720
   ggaaagtatt ttatttgtag gaatttatat ggtgtttttg ataatttttt gtattattat     780
   tatttttatt gtatagatta gggaatagat ataggtaggt tttggatgtg tggttatata     840
   tttagaaagt taggaagttt ggttttagag ttgtgtattt aattgttgtt atattatagg     900
   aatgatagtt ttggggggat gaattaagag gtgttggatg agggttttgg tttttaaagg     960
   tatagttgtt ttttaatttt tgtaaggttg aaattagaag atggcgggta ttgtagttgg    1020
   tggagggttt gaatatagtt gtgaggatag tgatttttgg gttaggtttt gtaaggaaat    1080
   tgagtagtgt agggttttat tagttttaat tatttggggg ttattttggt tggtattagt    1140
   aggagggtgg gttggttttt tagaggtttg ggagatttag tttttttttg ttagggttgt    1200
   agtggtcgat tttttttttt ttttgttttt attttgtatc gtttttagat tttagttttg     1260
   attattgttt aatttttagg agtttagttt ttgtgggtag cgtttgatat gtttgatttt    1320
   tttttttttg tagtttaagg gaaagacgag attttgtata aggtattttg tttttgtttt      1380
   tggttgggga agggtggtat ggagtttttt cggttgttta ttttattttt tgttataggt    1440
   aggatttttt tcgtgttttg tgatgttttt ttttttttat tttgagtatg tggttggggc     1500
   gcgggtggtg ggttttgggg tgaattatga aggcggtggt agttttcgta tgtttgagtg    1560
   ttttgttttg tatttttttt ttttttattt tgtttatatt ttttatattt tttttttttt     1620
   tttagtatta aaatttattt ttagattttt tttgtatgtg gatatgtaag gggttggtgg    1680
   attttggttg tattggaagg gaaggagtgt taatagtggt attttaggta ttagttggta    1740
   gttgggggaa gttttggggg gtattggtat tggtaatagt ttttgaaatt ataagttatt    1800
   aattatgagt ttttatagtt ataaaggagg aaagaatttg aggatgttta tttgtatttt    1860
   tggggtattt ttttttttgt tttgaaggtt tttttgtttt tagtttttga tgggaggtga     1920
   ggagcgtaga gattttcgtt gttgagttga ggggaattga tatattaaat ttttgtatat    1980
   aattgttttt tttcgttgag gaaagcgtat tttttgtttt tttagagtat tggataaatt    2040
   tgggtaagag gagagacggt gttaaatgga gttttgtttt tgtagttttt tagatggtgg    2100
   ttagggggaa ggcggggttt tgaggtatgg atgggaggtg gttagatggg agagggttac    2160
   ggtttatttg gtttttttag ttttaaaagg taagttatta attttttaat tttatttttt    2220
   aagtttttgt tatagtaatg gttgaagatg gtaggtaagg gaggattaag agagaagtta    2280
   aagtaggggg ttttgggggt tgttttagta ataggttggt gttttaagtt tatttttttc     2340
   gttttaagga gggtttttaa aatagtagtt ttatgttttt tttaaaatat tttcgagacg    2400
   ggtttttttt gaaaggggaa taaagttata gaaataggga agttggaagt taaaggttag    2460
   gaaagtcggt attaatgtgg gcggttgtag agtaagtaag agtggcgggg tagggagagt    2520
   tagttttagg tcgagaggag aggttttagt tttaaatgat ggtaaagaga tgtgtagaat    2580
   agaattggag ggggttttaa gattaagtgt ttttagaata gatttagaga aggttagtta    2640
   tttttttaag gaggtttagt aagagtaata gtgagaaaat taaatttagg ttttaggttt    2700
   tttggttttt aattttttat aaatatatgt tgtgttattc gtttttaatt tgtataagaa     2760
   tttaagtttt tttagggtag gatttttgtt tgtttaggtt attgtataga gtaagtgttt    2820
   aaagcgtatt tggtaaataa aggagtgaat gaatgttttt tttttagagt tgttcggagt    2880
   ttataatatt atagtgtttt agggcgtggc gggttagttt ttgtaggtgt tttgttttta    2940
   tgattttatg aagtattggg ggaggcgtaa ggtttggtgt cgttagttgg gagagaaggg    3000
   t                                                                    3001
<210> 83
   <211> 3001
   <212> DNA
   <213> human
<400> 83
   tttttagaaa aagattttag aagagtattt atagagtgat ttttaggtag ttttagattt        60
   tagtttttag gtttttgggg taaggttggg tagttggttt tttgagtgga ggtttgtttt       120
   ttttttatta tgaatttttt atttttaagt atattttttg tttgtaagtt agatgtatag       180
   taaatatgag taaataaatg aataaaagaa taaataagtg agtgtttgta ggaaattaaa       240
   gtagattttg ataatttata ggtttttgta ggttttttta gttttttttg ttatttttag       300
   tataatttta ttttttattt ttagaattga tttaagtttt ttttatttaa tttttgattt       360
   attgggttat tttatatttt tgtgtttttg tatatgtggg tttttgtgtt tggattatta       420
   tgggaatttg tatgtgtaga aaaatatatt tttattttta gaattttgag aaatattttg       480
   ttttgttttg taggatagga tgattaggag tttaagtatg tgtgtgtata aaataaatat       540
   tagtgtgagt atgtgtgtat aggagatatt taatagtttt aagaaggtta aatttgggta       600
   gaaaatttta ggtgggagag aaaatttttt gttttatgga tagtttattt tttttttttt       660
   ttttaattag gataatggta atagttagta tttgttgaat gttgtgtgtt aggttttatt       720
   ggaaagtatt ttatttgtag gaatttatat ggtgtttttg ataatttttt gtattattat       780
   tatttttatt gtatagatta gggaatagat ataggtaggt tttggatgtg tggttatata       840
   tttagaaagt taggaagttt ggttttagag ttgtgtattt aattgttgtt atattatagg       900
   aatgatagtt ttggggggat gaattaagag gtgttggatg agggttttgg tttttaaagg       960
   tatagttgtt ttttaatttt tgtaaggttg aaattagaag atggtgggta ttgtagttgg      1020
   tggagggttt gaatatagtt gtgaggatag tgatttttgg gttaggtttt gtaaggaaat      1080
   tgagtagtgt agggttttat tagttttaat tatttggggg ttattttggt tggtattagt      1140
   aggagggtgg gttggttttt tagaggtttg ggagatttag tttttttttg ttagggttgt      1200
   agtggttgat tttttttttt ttttgttttt attttgtatt gtttttagat tttagttttg       1260
   attattgttt aatttttagg agtttagttt ttgtgggtag tgtttgatat gtttgatttt      1320
   tttttttttg tagtttaagg gaaagatgag attttgtata aggtattttg tttttgtttt     1380
   tggttgggga agggtggtat ggagtttttt tggttgttta ttttattttt tgttataggt      1440
   aggatttttt ttgtgttttg tgatgttttt ttttttttat tttgagtatg tggttggggt      1500
   gtgggtggtg ggttttgggg tgaattatga aggtggtggt agtttttgta tgtttgagtg      1560
   ttttgttttg tatttttttt ttttttattt tgtttatatt ttttatattt tttttttttt     1620
   tttagtatta aaatttattt ttagattttt tttgtatgtg gatatgtaag gggttggtgg      1680
   attttggttg tattggaagg gaaggagtgt taatagtggt attttaggta ttagttggta      1740
   gttgggggaa gttttggggg gtattggtat tggtaatagt ttttgaaatt ataagttatt      1800
   aattatgagt ttttatagtt ataaaggagg aaagaatttg aggatgttta tttgtatttt      1860
   tggggtattt ttttttttgt tttgaaggtt tttttgtttt tagtttttga tgggaggtga      1920
   ggagtgtaga gatttttgtt gttgagttga ggggaattga tatattaaat ttttgtatat     1980
   aattgttttt ttttgttgag gaaagtgtat tttttgtttt tttagagtat tggataaatt      2040
   tgggtaagag gagagatggt gttaaatgga gttttgtttt tgtagttttt tagatggtgg      2100
   ttagggggaa ggtggggttt tgaggtatgg atgggaggtg gttagatggg agagggttat      2160
   ggtttatttg gtttttttag ttttaaaagg taagttatta attttttaat tttatttttt      2220
   aagtttttgt tatagtaatg gttgaagatg gtaggtaagg gaggattaag agagaagtta      2280
   aagtaggggg ttttgggggt tgttttagta ataggttggt gttttaagtt tatttttttt     2340
   gttttaagga gggtttttaa aatagtagtt ttatgttttt tttaaaatat ttttgagatg      2400
   ggtttttttt gaaaggggaa taaagttata gaaataggga agttggaagt taaaggttag      2460
   gaaagttggt attaatgtgg gtggttgtag agtaagtaag agtggtgggg tagggagagt      2520
   tagttttagg ttgagaggag aggttttagt tttaaatgat ggtaaagaga tgtgtagaat      2580
   agaattggag ggggttttaa gattaagtgt ttttagaata gatttagaga aggttagtta      2640
   tttttttaag gaggtttagt aagagtaata gtgagaaaat taaatttagg ttttaggttt     2700
   tttggttttt aattttttat aaatatatgt tgtgttattt gtttttaatt tgtataagaa      2760
   tttaagtttt tttagggtag gatttttgtt tgtttaggtt attgtataga gtaagtgttt      2820
   aaagtgtatt tggtaaataa aggagtgaat gaatgttttt tttttagagt tgtttggagt      2880
   ttataatatt atagtgtttt agggtgtggt gggttagttt ttgtaggtgt tttgttttta      2940
   tgattttatg aagtattggg ggaggtgtaa ggtttggtgt tgttagttgg gagagaaggg      3000
   t                                                                      3001
<210> 84
   <211> 3001
   <212> DNA
   <213> human
<400> 84
   gttttttttt tttagttggc ggtattaggt tttgcgtttt ttttagtgtt ttatggagtt 60
   ataggggtaa gatatttgta gggattggtt cgttacgttt tggaatattg tggtgttgtg 120
   ggtttcggat agttttgggg aggagatatt tatttatttt tttgtttatt aaatacgttt 180
   tgaatatttg ttttgtgtag tgatttaaat agataaaaat tttgttttga aggagtttag 240
   gtttttatat aagttgggag cggatggtat agtatgtgtt tgtgggaaat tgggaattag 300
   gaaatttggg atttgggttt ggttttttta ttgttgtttt tgttgggttt ttttggagaa 360
   gtaattgatt ttttttgggt ttattttgga ggtatttggt tttaaaattt tttttagttt 420
   tgttttgtat atttttttgt tattatttgg gattgggatt ttttttttcg gtttggggtt 480
   ggtttttttt gtttcgttat ttttgtttgt tttgtagtcg tttatattgg tatcgatttt 540
   tttgattttt aatttttagt ttttttattt ttgtggtttt gttttttttt taggaaggat 600
   tcgtttcgga gatattttgg gggagatatg aggttgttat tttgggaatt ttttttaggg 660
   cgggggagat gggtttagag tattagtttg ttgttggggt agtttttaga gttttttgtt 720
   ttgatttttt ttttggtttt tttttgtttg ttatttttaa ttattattgt gatagaggtt 780
   tgaaagatag gattgggaag ttggtggttt gttttttgga attagagaga ttagatgggt 840
   cgtggttttt ttttatttga ttatttttta tttatgtttt agaatttcgt tttttttttg 900
   attattattt aagaagttgt aggaataaga ttttatttgg tatcgttttt ttttttgttt 960
   aggtttgttt agtgttttgg gggagtaaaa ggtgcgtttt ttttagcggg aagaagtaat 1020
   tgtgtataaa aatttggtgt gttaattttt tttaatttag taacggaaat ttttacgttt 1080
   tttatttttt attaagagtt gaggataagg ggatttttag agtaggggag agagtgtttt 1140
   aaagatgtag atgaatattt ttaggttttt ttttttttta taattgtagg ggtttataat 1200
   tagtggttta taattttaga ggttattatt agtgttagtg ttttttaaag ttttttttaa 1260
   ttgttagtta gtgtttggga tgttattgtt agtatttttt tttttttaat gtaattagag 1320
   tttattagtt ttttatatgt ttatatgtag aaagagtttg agggtaggtt ttgatattgg 1380
   ggagggaagg agggtgtgaa gaatatgggt agggtgggga ggagaggagt gtagaatagg 1440
   gtatttaggt atgcgaggat tgttatcgtt tttataattt attttagggt ttattattcg 1500
   cgttttaatt atatgtttaa agtgagggag agaaggtatt atagggtacg gaggggattt 1560
   tatttgtgat aaagagtaag atgagtagtc ggagaggttt tatgttattt ttttttagtt 1620
   aagggtagaa gtagagtgtt ttgtgtaaga tttcgttttt tttttgaatt gtagaaaaga 1680
   gaggattagg tatgttaggc gttgtttata ggaattgggt ttttggggat taagtaatag 1740
   ttaggattgg ggtttggagg cggtgtaggg tggggataga ggggaggagg agtcggttat 1800
   tgtagttttg gtagaaggag gttgagtttt ttagattttt gagaagttag tttatttttt 1860
   tgttggtgtt agttagggtg gtttttagat ggttggggtt ggtaaggttt tgtattgttt 1920
   agtttttttg tagagtttag tttagggatt attattttta tagttgtatt tagatttttt 1980
   attagttgta atgttcgtta ttttttggtt ttagttttgt aagagttgga gaatagttgt 2040
   gtttttagag gttaggattt ttatttagta ttttttagtt tattttttta gggttgttat 2100
   ttttgtagtg tggtagtagt taagtatata gttttggagt tagatttttt gattttttgg 2160
   gtgtgtaatt atatatttaa aatttatttg tgtttgtttt ttgatttata tagtgggaat 2220
   aatgatagtg taaagggtta ttaggagtat tatatgggtt tttataggta aagtgttttt 2280
   tagtagggtt tgatatatag tatttaataa atattaatta ttattattat tttagttaga 2340
   agggaaaagg gaaatgggtt gtttataaga tagaaaattt ttttttttat ttggaatttt 2400
   ttgtttaagt ttagtttttt tggaattgtt gggtgttttt tgtgtatata tgtttatatt 2460
   ggtgtttatt ttgtgtatat atatgtttga atttttgatt attttatttt ataggatagg 2520
   ataggatatt ttttagagtt ttgggggtag gggtgtgttt ttttatacgt ataggttttt 2580
   atggtgattt aagtataggg atttacgtgt gtaaaggtat aaaagtgtga aatggtttag 2640
   tgagtcgggg attggatgga ggaggtttga gttaatttta ggagtgggaa atggagttgt 2700
   gttgggagtg gtagaagagg ttggagaagt ttgtaggagt ttgtagatta ttagggttta 2760
   ttttagtttt ttgtaaatat ttatttattt gtttttttat ttatttattt atttatattt 2820
   attgtgtatt taatttacgg atagggaatg tgtttggaga tggggaattt atagtgagga 2880
   gaagataggt ttttatttaa agggttaatt gttcggtttt gttttaaggg tttgggggtt 2940
   gaagtttgag gttgtttgaa gattattttg taggtatttt tttagggttt ttttttagaa 3000
   g 3001
<210> 85
   <211> 3001
   <212> DNA
   <213> human
<400> 85
   gttttttttt tttagttggt ggtattaggt tttgtgtttt ttttagtgtt ttatggagtt 60
   ataggggtaa gatatttgta gggattggtt tgttatgttt tggaatattg tggtgttgtg 120
   ggttttggat agttttgggg aggagatatt tatttatttt tttgtttatt aaatatgttt 180
   tgaatatttg ttttgtgtag tgatttaaat agataaaaat tttgttttga aggagtttag 240
   gtttttatat aagttgggag tggatggtat agtatgtgtt tgtgggaaat tgggaattag 300
   gaaatttggg atttgggttt ggttttttta ttgttgtttt tgttgggttt ttttggagaa 360
   gtaattgatt ttttttgggt ttattttgga ggtatttggt tttaaaattt tttttagttt 420
   tgttttgtat atttttttgt tattatttgg gattgggatt tttttttttg gtttggggtt 480
   ggtttttttt gttttgttat ttttgtttgt tttgtagttg tttatattgg tattgatttt 540
   tttgattttt aatttttagt ttttttattt ttgtggtttt gttttttttt taggaaggat 600
   ttgttttgga gatattttgg gggagatatg aggttgttat tttgggaatt ttttttaggg 660
   tgggggagat gggtttagag tattagtttg ttgttggggt agtttttaga gttttttgtt 720
   ttgatttttt ttttggtttt tttttgtttg ttatttttaa ttattattgt gatagaggtt 780
   tgaaagatag gattgggaag ttggtggttt gttttttgga attagagaga ttagatgggt 840
   tgtggttttt ttttatttga ttatttttta tttatgtttt agaattttgt tttttttttg 900
   attattattt aagaagttgt aggaataaga ttttatttgg tattgttttt ttttttgttt 960
   aggtttgttt agtgttttgg gggagtaaaa ggtgtgtttt ttttagtggg aagaagtaat 1020
   tgtgtataaa aatttggtgt gttaattttt tttaatttag taatggaaat ttttatgttt 1080
   tttatttttt attaagagtt gaggataagg ggatttttag agtaggggag agagtgtttt 1140
   aaagatgtag atgaatattt ttaggttttt ttttttttta taattgtagg ggtttataat 1200
   tagtggttta taattttaga ggttattatt agtgttagtg ttttttaaag ttttttttaa 1260
   ttgttagtta gtgtttggga tgttattgtt agtatttttt tttttttaat gtaattagag 1320
   tttattagtt ttttatatgt ttatatgtag aaagagtttg agggtaggtt ttgatattgg 1380
   ggagggaagg agggtgtgaa gaatatgggt agggtgggga ggagaggagt gtagaatagg 1440
   gtatttaggt atgtgaggat tgttattgtt tttataattt attttagggt ttattatttg 1500
   tgttttaatt atatgtttaa agtgagggag agaaggtatt atagggtatg gaggggattt 1560
   tatttgtgat aaagagtaag atgagtagtt ggagaggttt tatgttattt ttttttagtt 1620
   aagggtagaa gtagagtgtt ttgtgtaaga ttttgttttt tttttgaatt gtagaaaaga 1680
   gaggattagg tatgttaggt gttgtttata ggaattgggt ttttggggat taagtaatag 1740
   ttaggattgg ggtttggagg tggtgtaggg tggggataga ggggaggagg agttggttat 1800
   tgtagttttg gtagaaggag gttgagtttt ttagattttt gagaagttag tttatttttt 1860
   tgttggtgtt agttagggtg gtttttagat ggttggggtt ggtaaggttt tgtattgttt 1920
   agtttttttg tagagtttag tttagggatt attattttta tagttgtatt tagatttttt 1980
   attagttgta atgtttgtta ttttttggtt ttagttttgt aagagttgga gaatagttgt 2040
   gtttttagag gttaggattt ttatttagta ttttttagtt tattttttta gggttgttat 2100
   ttttgtagtg tggtagtagt taagtatata gttttggagt tagatttttt gattttttgg 2160
   gtgtgtaatt atatatttaa aatttatttg tgtttgtttt ttgatttata tagtgggaat 2220
   aatgatagtg taaagggtta ttaggagtat tatatgggtt tttataggta aagtgttttt 2280
   tagtagggtt tgatatatag tatttaataa atattaatta ttattattat tttagttaga 2340
   agggaaaagg gaaatgggtt gtttataaga tagaaaattt ttttttttat ttggaatttt 2400
   ttgtttaagt ttagtttttt tggaattgtt gggtgttttt tgtgtatata tgtttatatt 2460
   ggtgtttatt ttgtgtatat atatgtttga atttttgatt attttatttt ataggatagg 2520
   ataggatatt ttttagagtt ttgggggtag gggtgtgttt ttttatatgt ataggttttt 2580
   atggtgattt aagtataggg atttatgtgt gtaaaggtat aaaagtgtga aatggtttag 2640
   tgagttgggg attggatgga ggaggtttga gttaatttta ggagtgggaa atggagttgt 2700
   gttgggagtg gtagaagagg ttggagaagt ttgtaggagt ttgtagatta ttagggttta 2760
   ttttagtttt ttgtaaatat ttatttattt gtttttttat ttatttattt atttatattt 2820
   attgtgtatt taatttatgg atagggaatg tgtttggaga tggggaattt atagtgagga 2880
   gaagataggt ttttatttaa agggttaatt gtttggtttt gttttaaggg tttgggggtt 2940
   gaagtttgag gttgtttgaa gattattttg taggtatttt tttagggttt ttttttagaa 3000
   g 3001
<210> 86
   <211> 50
   <212> DNA
   <213> human
<400> 86
   gggaactaga atttaattgg gaatcttctg ctatttccct gcttcctccg 50
<210> 87
   <211> 50
   <212> DNA
   <213> human
<400> 87
   gggaattaga atttaattgg gaattttttg ttattttttt gtttttttcg 50
<210> 88
   <211> 50
   <212> DNA
   <213> human
<400> 88
   gggaattaga atttaattgg gaattttttg ttattttttt gttttttttg 50
<210> 89
   <211> 50
   <212> DNA
   <213> human
<400> 89
   cggaggaagt agggaaatag tagaagattt ttaattaaat tttagttttt 50
<210> 90
   <211> 50
   <212> DNA
   <213> human
<400> 90
   tggaggaagt agggaaatag tagaagattt ttaattaaat tttagttttt 50
<210> 91
   <211> 3001
   <212> DNA
   <213> human
<400> 91
   tttcacaaga ctcacttcac tggtcaccaa ggtctctact aaaacatcat caaggtccct     60
   gctgaaacaa cactctccct agaggccgtc ctgtaccact ctgccctcat ctctctcaga    120
   ccctccccgg cctaatctct tcctagcgct taacatctgg ggccccatgg gagcactttg    180
   tttttcttgc tggctgcact gtgtctagag tctggcccat ggcagatgct tgctaaatat    240
   ttgctgaata aatgcatgga tttgcgagtt gccatccaca atggtaaatt cagcccttca    300
   aagatgctca gcgctctggt ccccacagca gcggttaaac ccctttaagc agtgctgtgt    360
   gcagaagcct cttgggatgc agaggcctcc gaggaaggaa acatgacgtg tcttttgtgg    420
   agcgcaggct gtgatgagca tcttcaggct ccccatcttt tgtctgcagg agtagctgca    480
   gaattgaact tgggggctca gttacaggga gggacagaaa cccccaccag agagtatgtc    540
   tgccctcttg acggtcacat gctcagcact gggcagagcc taccccttct ttcaagtaaa    600
   aggagcggct gtgttccgat cactccctga tgcaaagctt ctgaaaaggt ttgctcacct    660
   tactgactaa ttaaagggaa gccacatttt tccctccctg gtctctgcag ccataccacc    720
   aagggctatt tgataggccc acggcagctt gggttacaca gatgcccgcg ataatgccat    780
   ttgctgcgat gattgagtcg acaaggctga gtgacttggg gaaaatattc cccgattgga    840
   ctaacctgtt ggcggcctgc ttaccatcac acttgttttt ataatgaaaa aggaaattat    900
   ttctgctttt aatgatggca gctccagaaa tggtgagtaa aatgggtgtg aggagagaag    960
   atgcttctca acacttctca gtgttgtaca gcttgccaac cacagcctgt ttgggagggg   1020
   atgttctcac ccaggttcca tctttcagaa gccatttgcc cacttattct ttttatatgg   1080
   ataaacgatg taatttgcta catgctgata ataaacagag gtatcagcaa gaatgaaagc   1140
   cagaaaatgg cccaacatga acttcttgct tttctaagtt actctttcaa gctcttcttg   1200
   cgatagaaat gaaatgaaca tttttggcac agaactcttg tttagatttt actatgatct   1260
   tgggaaacac gggttgcttc aaatggctta ctagatccct ttggaatttc aaacgttaca   1320
   acaggtagtc acaaagggaa tgaaagcgga tgatctgagt gtctgtgctg atgtgcctgc 1380
   tggagcccct gtggatttta gcccttggag gaagcaggtt cccagagcag ccatgcctga 1440
   tatctgggag agggaactag aatttaattg ggaatcttct gctatttccc tgcttcctcc 1500
   gccctctcag agttacaggg aatgacttag aaaggtctct ggtctccttg gcattgtgat 1560
   aggtggaatt ctttcgtagg aaacacagcc ccggggttat tttgatgcaa gccgttggtc 1620
   caaagccact ccccactttg ctatgtgggg gaaggaccgg gcaccggacg gctcgggtac 1680
   tttcgttctt aattaggtca tgcccgtgtg agccaggaaa gggctgtgtt tatgggaagc 1740
   cagtaacact gtggcctact atctcttccg tggtgccatc tacatttttg ggactcggga 1800
   attatgtgag taccgaaact acttatgctt atggtaggtg taccacacgc acagggaaag 1860
   aattgcgttt atgtgggaca ggtgaaaaca atcgcaaaaa agcaatggaa agggctttga 1920
   gagtaattta tcttctgaac aatagcaata tggcaacttc taaatggtga gagggagtcc 1980
   ctctaaagca atcgatttga agattggttg gacaaacaat gggaaagtca ttgtcttagc 2040
   agaattaagt tcatactttt tttttttttt tttttttgct aattctagaa gcttttctgt 2100
   tatctgctgt agctcagacg aaaatgcatt ctcaccagat gactgttttt ggttaatcga 2160
   tactgaatgc gctttgtgtg gactgtcgaa tttcaaagat ttaccgtatg accaagatgc 2220
   acctgatgct acaagtataa ataggggaac aaatgctttc tgttcttcct cggctaagga 2280
   ggtagaggtg gaggcggagc cggatgtcag aggtcctgaa atagtcacca tgggggaaaa 2340
   tgatccgcct gctgttgaag cccccttctc attccgatcg ctttttggcc ttgatgattt 2400
   gaaaataagt cctgttgcac caggtaagtg gacccaggtg aggactctgt gatttctgcc 2460
   cataccctca tgtaggtgac caatgtgact gactgtccct gtgggggaaa tatctcccca 2520
   gccattctga cacccacagg ctggacacct gcacttccct agatctgcag aatctcaggg 2580
   agaaggggca ttggagaggg gatcgtttcg ttaagccctt tgctctctcc ctggagaccg 2640
   gtgttttctt ctcttgttgg aggttttcag agactggggc tccacaattg tcctgtcaat 2700
   cctgaaggag gtcagatcct ggccaggaaa tctctgagtc ctccaggaag tcctgagaag 2760
   cagtggccac cagtccctcc gaaaactcag gataaactgg atgtcgccaa agatattaga 2820
   ggcctagatt aaaatccagc agcagatgac ggagtggctc ctaacaactg aaggaaaaaa 2880
   gttggtttcg taaataagtc acaaatgcct gataaaacag tggactctct agcaacctat 2940
   acggctagga tctttttgac cagagatagt caggactggt caaagggagc tgtggggttg 3000
   c 3001
<210> 92
   <211> 3001
   <212> DNA
   <213> human
<400> 92
   ttttataaga tttattttat tggttattaa ggtttttatt aaaatattat taaggttttt 60
   gttgaaataa tatttttttt agaggtcgtt ttgtattatt ttgtttttat tttttttaga 120
   tttttttcgg tttaattttt ttttagcgtt taatatttgg ggttttatgg gagtattttg 180
   ttttttttgt tggttgtatt gtgtttagag tttggtttat ggtagatgtt tgttaaatat 240
   ttgttgaata aatgtatgga tttgcgagtt gttatttata atggtaaatt tagtttttta 300
   aagatgttta gcgttttggt ttttatagta gcggttaaat ttttttaagt agtgttgtgt 360
   gtagaagttt tttgggatgt agaggttttc gaggaaggaa atatgacgtg ttttttgtgg 420
   agcgtaggtt gtgatgagta tttttaggtt ttttattttt tgtttgtagg agtagttgta 480
   gaattgaatt tggggtttta gttataggga gggatagaaa tttttattag agagtatgtt 540
   tgtttttttg acggttatat gtttagtatt gggtagagtt tatttttttt tttaagtaaa 600
   aggagcggtt gtgtttcgat tattttttga tgtaaagttt ttgaaaaggt ttgtttattt 660
   tattgattaa ttaaagggaa gttatatttt tttttttttg gtttttgtag ttatattatt 720
   aagggttatt tgataggttt acggtagttt gggttatata gatgttcgcg ataatgttat 780
   ttgttgcgat gattgagtcg ataaggttga gtgatttggg gaaaatattt ttcgattgga 840
   ttaatttgtt ggcggtttgt ttattattat atttgttttt ataatgaaaa aggaaattat 900
   ttttgttttt aatgatggta gttttagaaa tggtgagtaa aatgggtgtg aggagagaag 960
   atgtttttta atatttttta gtgttgtata gtttgttaat tatagtttgt ttgggagggg 1020
   atgtttttat ttaggtttta ttttttagaa gttatttgtt tatttatttt ttttatatgg 1080
   ataaacgatg taatttgtta tatgttgata ataaatagag gtattagtaa gaatgaaagt 1140
   tagaaaatgg tttaatatga attttttgtt tttttaagtt atttttttaa gttttttttg 1200
   cgatagaaat gaaatgaata tttttggtat agaatttttg tttagatttt attatgattt 1260
   tgggaaatac gggttgtttt aaatggttta ttagattttt ttggaatttt aaacgttata 1320
   ataggtagtt ataaagggaa tgaaagcgga tgatttgagt gtttgtgttg atgtgtttgt 1380
   tggagttttt gtggatttta gtttttggag gaagtaggtt tttagagtag ttatgtttga 1440
   tatttgggag agggaattag aatttaattg ggaatttttt gttatttttt tgtttttttc 1500
   gtttttttag agttataggg aatgatttag aaaggttttt ggtttttttg gtattgtgat 1560
   aggtggaatt ttttcgtagg aaatatagtt tcggggttat tttgatgtaa gtcgttggtt 1620
   taaagttatt ttttattttg ttatgtgggg gaaggatcgg gtatcggacg gttcgggtat 1680
   tttcgttttt aattaggtta tgttcgtgtg agttaggaaa gggttgtgtt tatgggaagt 1740
   tagtaatatt gtggtttatt atttttttcg tggtgttatt tatatttttg ggattcggga 1800
   attatgtgag tatcgaaatt atttatgttt atggtaggtg tattatacgt atagggaaag 1860
   aattgcgttt atgtgggata ggtgaaaata atcgtaaaaa agtaatggaa agggttttga 1920
   gagtaattta ttttttgaat aatagtaata tggtaatttt taaatggtga gagggagttt 1980
   ttttaaagta atcgatttga agattggttg gataaataat gggaaagtta ttgttttagt 2040
   agaattaagt ttatattttt tttttttttt tttttttgtt aattttagaa gtttttttgt 2100
   tatttgttgt agtttagacg aaaatgtatt tttattagat gattgttttt ggttaatcga 2160
   tattgaatgc gttttgtgtg gattgtcgaa ttttaaagat ttatcgtatg attaagatgt 2220
   atttgatgtt ataagtataa ataggggaat aaatgttttt tgtttttttt cggttaagga 2280
   ggtagaggtg gaggcggagt cggatgttag aggttttgaa atagttatta tgggggaaaa 2340
   tgattcgttt gttgttgaag tttttttttt atttcgatcg ttttttggtt ttgatgattt 2400
   gaaaataagt tttgttgtat taggtaagtg gatttaggtg aggattttgt gatttttgtt 2460
   tatattttta tgtaggtgat taatgtgatt gattgttttt gtgggggaaa tattttttta 2520
   gttattttga tatttatagg ttggatattt gtattttttt agatttgtag aattttaggg 2580
   agaaggggta ttggagaggg gatcgtttcg ttaagttttt tgtttttttt ttggagatcg 2640
   gtgttttttt tttttgttgg aggtttttag agattggggt tttataattg ttttgttaat 2700
   tttgaaggag gttagatttt ggttaggaaa tttttgagtt ttttaggaag ttttgagaag 2760
   tagtggttat tagttttttc gaaaatttag gataaattgg atgtcgttaa agatattaga 2820
   ggtttagatt aaaatttagt agtagatgac ggagtggttt ttaataattg aaggaaaaaa 2880
   gttggtttcg taaataagtt ataaatgttt gataaaatag tggatttttt agtaatttat 2940
   acggttagga tttttttgat tagagatagt taggattggt taaagggagt tgtggggttg 3000
   t 3001

<210> 93
   <211> 3001
   <212> DNA
   <213> human
<400> 93
   ttttataaga tttattttat tggttattaa ggtttttatt aaaatattat taaggttttt 60
   gttgaaataa tatttttttt agaggttgtt ttgtattatt ttgtttttat tttttttaga 120
   ttttttttgg tttaattttt ttttagtgtt taatatttgg ggttttatgg gagtattttg 180
   ttttttttgt tggttgtatt gtgtttagag tttggtttat ggtagatgtt tgttaaatat 240
   ttgttgaata aatgtatgga tttgtgagtt gttatttata atggtaaatt tagtttttta 300
   aagatgttta gtgttttggt ttttatagta gtggttaaat ttttttaagt agtgttgtgt 360
   gtagaagttt tttgggatgt agaggttttt gaggaaggaa atatgatgtg ttttttgtgg 420
   agtgtaggtt gtgatgagta tttttaggtt ttttattttt tgtttgtagg agtagttgta 480
   gaattgaatt tgggggttta gttataggga gggatagaaa tttttattag agagtatgtt 540
   tgtttttttg atggttatat gtttagtatt gggtagagtt tatttttttt tttaagtaaa 600
   aggagtggtt gtgttttgat tattttttga tgtaaagttt ttgaaaaggt ttgtttattt 660
   tattgattaa ttaaagggaa gttatatttt tttttttttg gtttttgtag ttatattatt 720
   aagggttatt tgataggttt atggtagttt gggttatata gatgtttgtg ataatgttat 780
   ttgttgtgat gattgagttg ataaggttga gtgatttggg gaaaatattt tttgattgga 840
   ttaatttgtt ggtggtttgt ttattattat atttgttttt ataatgaaaa aggaaattat 900
   ttttgttttt aatgatggta gttttagaaa tggtgagtaa aatgggtgtg aggagagaag 960
   atgtttttta atatttttta gtgttgtata gtttgttaat tatagtttgt ttgggagggg 1020
   atgtttttat ttaggtttta ttttttagaa gttatttgtt tatttatttt ttttatatgg 1080
   ataaatgatg taatttgtta tatgttgata ataaatagag gtattagtaa gaatgaaagt 1140
   tagaaaatgg tttaatatga attttttgtt tttttaagtt atttttttaa gttttttttg 1200
   tgatagaaat gaaatgaata tttttggtat agaatttttg tttagatttt attatgattt 1260
   tgggaaatat gggttgtttt aaatggttta ttagattttt ttggaatttt aaatgttata 1320
   ataggtagtt ataaagggaa tgaaagtgga tgatttgagt gtttgtgttg atgtgtttgt 1380
   tggagttttt gtggatttta gtttttggag gaagtaggtt tttagagtag ttatgtttga 1440
   tatttgggag agggaattag aatttaattg ggaatttttt gttatttttt tgtttttttt 1500
   gtttttttag agttataggg aatgatttag aaaggttttt ggtttttttg gtattgtgat 1560
   aggtggaatt tttttgtagg aaatatagtt ttggggttat tttgatgtaa gttgttggtt 1620
   taaagttatt ttttattttg ttatgtgggg gaaggattgg gtattggatg gtttgggtat 1680
   ttttgttttt aattaggtta tgtttgtgtg agttaggaaa gggttgtgtt tatgggaagt 1740
   tagtaatatt gtggtttatt attttttttg tggtgttatt tatatttttg ggatttggga 1800
   attatgtgag tattgaaatt atttatgttt atggtaggtg tattatatgt atagggaaag 1860
   aattgtgttt atgtgggata ggtgaaaata attgtaaaaa agtaatggaa agggttttga 1920
   gagtaattta ttttttgaat aatagtaata tggtaatttt taaatggtga gagggagttt 1980
   ttttaaagta attgatttga agattggttg gataaataat gggaaagtta ttgttttagt 2040
   agaattaagt ttatattttt tttttttttt tttttttgtt aattttagaa gtttttttgt 2100
   tatttgttgt agtttagatg aaaatgtatt tttattagat gattgttttt ggttaattga 2160
   tattgaatgt gttttgtgtg gattgttgaa ttttaaagat ttattgtatg attaagatgt 2220
   atttgatgtt ataagtataa ataggggaat aaatgttttt tgtttttttt tggttaagga 2280
   ggtagaggtg gaggtggagt tggatgttag aggttttgaa atagttatta tgggggaaaa 2340
   tgatttgttt gttgttgaag tttttttttt attttgattg ttttttggtt ttgatgattt 2400
   gaaaataagt tttgttgtat taggtaagtg gatttaggtg aggattttgt gatttttgtt 2460
   tatattttta tgtaggtgat taatgtgatt gattgttttt gtgggggaaa tattttttta 2520
   gttattttga tatttatagg ttggatattt gtattttttt agatttgtag aattttaggg 2580
   agaaggggta ttggagaggg gattgttttg ttaagttttt tgtttttttt ttggagattg 2640
   gtgttttttt tttttgttgg aggtttttag agattggggt tttataattg ttttgttaat 2700
   tttgaaggag gttagatttt ggttaggaaa tttttgagtt ttttaggaag ttttgagaag 2760
   tagtggttat tagttttttt gaaaatttag gataaattgg atgttgttaa agatattaga 2820
   ggtttagatt aaaatttagt agtagatgat ggagtggttt ttaataattg aaggaaaaaa 2880
   gttggttttg taaataagtt ataaatgttt gataaaatag tggatttttt agtaatttat 2940
   atggttagga tttttttgat tagagatagt taggattggt taaagggagt tgtggggttg 3000
   t                                                                 3001
<210> 94
   <211> 3001
   <212> DNA
   <213> human
<400> 94
   gtaattttat agtttttttt gattagtttt gattattttt ggttaaaaag attttagtcg 60
   tataggttgt tagagagttt attgttttat taggtatttg tgatttattt acgaaattaa 120
   tttttttttt ttagttgtta ggagttattt cgttatttgt tgttggattt taatttaggt 180
   ttttaatatt tttggcgata tttagtttat tttgagtttt cggagggatt ggtggttatt 240
   gttttttagg attttttgga ggatttagag attttttggt taggatttga ttttttttag 300
   gattgatagg ataattgtgg agttttagtt tttgaaaatt tttaataaga gaagaaaata 360
   tcggttttta gggagagagt aaagggttta acgaaacgat ttttttttta atgttttttt 420
   tttttgagat tttgtagatt tagggaagtg taggtgttta gtttgtgggt gttagaatgg 480
   ttggggagat atttttttta tagggatagt tagttatatt ggttatttat atgagggtat 540
   gggtagaaat tatagagttt ttatttgggt ttatttattt ggtgtaatag gatttatttt 600
   taaattatta aggttaaaaa gcgatcggaa tgagaagggg gttttaatag taggcggatt 660
   attttttttt atggtgatta ttttaggatt tttgatattc ggtttcgttt ttatttttat 720
   ttttttagtc gaggaagaat agaaagtatt tgttttttta tttatatttg tagtattagg 780
   tgtattttgg ttatacggta aatttttgaa attcgatagt ttatataaag cgtatttagt 840
   atcgattaat taaaaatagt tatttggtga gaatgtattt tcgtttgagt tatagtagat 900
   aatagaaaag tttttagaat tagtaaaaaa aaaaaaaaaa aaaaagtatg aatttaattt 960
   tgttaagata atgatttttt tattgtttgt ttaattaatt tttaaatcga ttgttttaga 1020
   gggatttttt tttattattt agaagttgtt atattgttat tgtttagaag ataaattatt 1080
   tttaaagttt tttttattgt ttttttgcga ttgtttttat ttgttttata taaacgtaat 1140
   tttttttttg tgcgtgtggt atatttatta taagtataag tagtttcggt atttatataa 1200
   ttttcgagtt ttaaaaatgt agatggtatt acggaagaga tagtaggtta tagtgttatt 1260
   ggttttttat aaatatagtt tttttttggt ttatacgggt atgatttaat taagaacgaa 1320
   agtattcgag tcgttcggtg ttcggttttt tttttatata gtaaagtggg gagtggtttt 1380
   ggattaacgg tttgtattaa aataatttcg gggttgtgtt ttttacgaaa gaattttatt 1440
   tattataatg ttaaggagat tagagatttt tttaagttat tttttgtaat tttgagaggg 1500
   cggaggaagt agggaaatag tagaagattt ttaattaaat tttagttttt ttttttagat 1560
   attaggtatg gttgttttgg gaatttgttt tttttaaggg ttaaaattta taggggtttt 1620
   agtaggtata ttagtataga tatttagatt attcgttttt attttttttg tgattatttg 1680
   ttgtaacgtt tgaaatttta aagggattta gtaagttatt tgaagtaatt cgtgtttttt 1740
   aagattatag taaaatttaa ataagagttt tgtgttaaaa atgtttattt tatttttatc 1800
   gtaagaagag tttgaaagag taatttagaa aagtaagaag tttatgttgg gttatttttt 1860
   ggtttttatt tttgttgata tttttgttta ttattagtat gtagtaaatt atatcgttta 1920
   tttatataaa aagaataagt gggtaaatgg tttttgaaag atggaatttg ggtgagaata 1980
   ttttttttta aataggttgt ggttggtaag ttgtataata ttgagaagtg ttgagaagta 2040
   tttttttttt ttatatttat tttatttatt atttttggag ttgttattat taaaagtaga 2100
   aataattttt ttttttatta taaaaataag tgtgatggta agtaggtcgt taataggtta 2160
   gtttaatcgg ggaatatttt ttttaagtta tttagttttg tcgatttaat tatcgtagta 2220
   aatggtatta tcgcgggtat ttgtgtaatt taagttgtcg tgggtttatt aaatagtttt 2280
   tggtggtatg gttgtagaga ttagggaggg aaaaatgtgg ttttttttta attagttagt 2340
   aaggtgagta aattttttta gaagttttgt attagggagt gatcggaata tagtcgtttt 2400
   ttttatttga aagaaggggt aggttttgtt tagtgttgag tatgtgatcg ttaagagggt 2460
   agatatattt tttggtgggg gtttttgttt ttttttgtaa ttgagttttt aagtttaatt 2520
   ttgtagttat ttttgtagat aaaagatggg gagtttgaag atgtttatta tagtttgcgt 2580
   tttataaaag atacgttatg tttttttttt cggaggtttt tgtattttaa gaggtttttg 2640
   tatatagtat tgtttaaagg ggtttaatcg ttgttgtggg gattagagcg ttgagtattt 2700
   ttgaagggtt gaatttatta ttgtggatgg taattcgtaa atttatgtat ttatttagta 2760
   aatatttagt aagtatttgt tatgggttag attttagata tagtgtagtt agtaagaaaa 2820
   ataaagtgtt tttatggggt tttagatgtt aagcgttagg aagagattag gtcggggagg 2880
   gtttgagaga gatgagggta gagtggtata ggacggtttt tagggagagt gttgttttag 2940
   tagggatttt gatgatgttt tagtagagat tttggtgatt agtgaagtga gttttgtgaa 3000
   a 3001
<210> 95
   <211> 3001
   <212> DNA
   <213> human
<400> 95
   gtaattttat agtttttttt gattagtttt gattattttt ggttaaaaag attttagttg 60
   tataggttgt tagagagttt attgttttat taggtatttg tgatttattt atgaaattaa 120
   tttttttttt ttagttgtta ggagttattt tgttatttgt tgttggattt taatttaggt 180
   ttttaatatt tttggtgata tttagtttat tttgagtttt tggagggatt ggtggttatt 240
   gttttttagg attttttgga ggatttagag attttttggt taggatttga ttttttttag 300
   gattgatagg ataattgtgg agttttagtt tttgaaaatt tttaataaga gaagaaaata 360
   ttggttttta gggagagagt aaagggttta atgaaatgat ttttttttta atgttttttt 420
   tttttgagat tttgtagatt tagggaagtg taggtgttta gtttgtgggt gttagaatgg 480
   ttggggagat atttttttta tagggatagt tagttatatt ggttatttat atgagggtat 540
   gggtagaaat tatagagttt ttatttgggt ttatttattt ggtgtaatag gatttatttt 600
   taaattatta aggttaaaaa gtgattggaa tgagaagggg gttttaatag taggtggatt 660
   attttttttt atggtgatta ttttaggatt tttgatattt ggttttgttt ttatttttat 720
   ttttttagtt gaggaagaat agaaagtatt tgttttttta tttatatttg tagtattagg 780
   tgtattttgg ttatatggta aatttttgaa atttgatagt ttatataaag tgtatttagt 840
   attgattaat taaaaatagt tatttggtga gaatgtattt ttgtttgagt tatagtagat 900
   aatagaaaag tttttagaat tagtaaaaaa aaaaaaaaaa aaaaagtatg aatttaattt 960
   tgttaagata atgatttttt tattgtttgt ttaattaatt tttaaattga ttgttttaga 1020
   gggatttttt tttattattt agaagttgtt atattgttat tgtttagaag ataaattatt 1080
   tttaaagttt tttttattgt ttttttgtga ttgtttttat ttgttttata taaatgtaat 1140
   tttttttttg tgtgtgtggt atatttatta taagtataag tagttttggt atttatataa 1200
   tttttgagtt ttaaaaatgt agatggtatt atggaagaga tagtaggtta tagtgttatt 1260
   ggttttttat aaatatagtt tttttttggt ttatatgggt atgatttaat taagaatgaa 1320
   agtatttgag ttgtttggtg tttggttttt tttttatata gtaaagtggg gagtggtttt 1380
   ggattaatgg tttgtattaa aataattttg gggttgtgtt ttttatgaaa gaattttatt 1440
   tattataatg ttaaggagat tagagatttt tttaagttat tttttgtaat tttgagaggg 1500
   tggaggaagt agggaaatag tagaagattt ttaattaaat tttagttttt ttttttagat 1560
   attaggtatg gttgttttgg gaatttgttt tttttaaggg ttaaaattta taggggtttt 1620
   agtaggtata ttagtataga tatttagatt atttgttttt attttttttg tgattatttg 1680
   ttgtaatgtt tgaaatttta aagggattta gtaagttatt tgaagtaatt tgtgtttttt 1740
   aagattatag taaaatttaa ataagagttt tgtgttaaaa atgtttattt tatttttatt 1800
   gtaagaagag tttgaaagag taatttagaa aagtaagaag tttatgttgg gttatttttt 1860
   ggtttttatt tttgttgata tttttgttta ttattagtat gtagtaaatt atattgttta 1920
   tttatataaa aagaataagt gggtaaatgg tttttgaaag atggaatttg ggtgagaata 1980
   ttttttttta aataggttgt ggttggtaag ttgtataata ttgagaagtg ttgagaagta 2040
   tttttttttt ttatatttat tttatttatt atttttggag ttgttattat taaaagtaga 2100
   aataattttt ttttttatta taaaaataag tgtgatggta agtaggttgt taataggtta 2160
   gtttaattgg ggaatatttt ttttaagtta tttagttttg ttgatttaat tattgtagta 2220
   aatggtatta ttgtgggtat ttgtgtaatt taagttgttg tgggtttatt aaatagtttt 2280
   tggtggtatg gttgtagaga ttagggaggg aaaaatgtgg ttttttttta attagttagt 2340
   aaggtgagta aattttttta gaagttttgt attagggagt gattggaata tagttgtttt 2400
   ttttatttga aagaaggggt aggttttgtt tagtgttgag tatgtgattg ttaagagggt 2460
   agatatattt tttggtgggg gtttttgttt ttttttgtaa ttgagttttt aagtttaatt 2520
   ttgtagttat ttttgtagat aaaagatggg gagtttgaag atgtttatta tagtttgtgt 2580
   tttataaaag atatgttatg tttttttttt tggaggtttt tgtattttaa gaggtttttg 2640
   tatatagtat tgtttaaagg ggtttaattg ttgttgtggg gattagagtg ttgagtattt 2700
   ttgaagggtt gaatttatta ttgtggatgg taatttgtaa atttatgtat ttatttagta 2760
   aatatttagt aagtatttgt tatgggttag attttagata tagtgtagtt agtaagaaaa 2820
   ataaagtgtt tttatggggt tttagatgtt aagtgttagg aagagattag gttggggagg 2880
   gtttgagaga gatgagggta gagtggtata ggatggtttt tagggagagt gttgttttag 2940
   tagggatttt gatgatgttt tagtagagat tttggtgatt agtgaagtga gttttgtgaa 3000
   a 3001
<210> 96
   <211> 50
   <212> DNA
   <213> human
<400> 96
   gagcaccgga atcacataca ctgtcccatt gagtgggctg ctgaggaacg 50
<210> 97
   <211> 50
   <212> DNA
   <213> human
<400> 97
   gagtatcgga attatatata ttgttttatt gagtgggttg ttgaggaacg 50
<210> 98
   <211> 50
   <212> DNA
   <213> human
<400> 98
   gagtattgga attatatata ttgttttatt gagtgggttg ttgaggaatg 50
<210> 99
   <211> 50
   <212> DNA
   <213> human
<400> 99
   cgttttttag tagtttattt aatgggatag tgtatgtgat ttcggtgttt 50
<210> 100
   <211> 50
   <212> DNA
   <213> human
<400> 100
   tgttttttag tagtttattt aatgggatag tgtatgtgat tttggtgttt 50
<210> 101
   <211> 3001
   <212> DNA
   <213> human
<400> 101
   acgcgtccct cagatcatat atctgtcctc actctacaca tgacactgta caggagagga      60
   agtccactag gggaatttgt tcccttgatt atggagtctg agaagttgaa cataggctgt     120
   cattaaggta gtgtcctgga gataccaata tcctggctga gttagaatca gcttcaggag     180
   aaagagagga aactgccttg tctctgccct ttatcttcag tctgagctac cagctgattg     240
   gatagtgtcc acccacactg agggctgctg ttgcccactc agctcaccaa cttacatgac     300
   tctccggaaa caccttcaca gactcaccca gaaatcatgc tttaccaatt ctccatgtat     360
   tatttaatcc agtcaagtca acacctataa ttaaccataa taccatagta atataatgat     420
   atatatttca gtttttaaaa aactgactat atgtcagttc atagtttgag tagcccaaag     480
   aagaagttaa tttaagccat aagaaaaata aacccaacat ttttcattat ttattttatc     540
   tcctagtgat cctagactgg tttaattgct gtcttttttt ttttaatctg gtcggttggt     600
   ttgttctggg gttgtttttg ttttaaagag ccggcttctc ggtccctctc tctaaaaata     660
   tgtcatagca acctctgacc atcttctcat gttttgttac ccaatgtagg ttttctactc     720
   ctctcccata gtcattcagt tgttatttga aagagaaata gggacaagca tgtttcattc     780
   tagaccactt aagattaaaa cccgagtttc atgtcgacat ccagggagta ggtttttgaa     840
   cagatttgca ttatgctatg ggcaggaacc aggagtagaa gaggttgtga gtcatctagt     900
   cagcagtgtc tgctaaaaag cctaaagact ctctttgtgt ttgaattttt catttttgtt     960
   acagaggaaa tagtttgttc cgctttaatg agcactgtca agaagaaaat atagctatca    1020
   catatcacca catgttcacg gattttcacc aacccagacc ccaaaatgac atgctgctct    1080
   ttcttcttca gaaacatgaa ttctggaatc ttgcaagtct tccagagggc actcacctgt    1140
   cccatctgca tgaactactt cctagaccca gtcaccatag actgtgggca cagcttttgc    1200
   cggccctgtt tgtacctcaa ctggcaagac acggcagttc ttgctcagtg ctctgaatgc    1260
   aagaagacaa cgcggcagag aaacctcaac actgacattt gtttgaagaa catggctttc    1320
   attgccagaa aagccagcct ccggcaattc cttagctctg aggagcaaat atgtgggatg    1380
   cacagagaga caaagaagat gttctgtgaa gtggacaaga gcctgctctg tttgccgtgc    1440
   tccaactctc aggagcaccg gaatcacata cactgtccca ttgagtgggc tgctgaggaa    1500
   cgccgggtaa gtgatgcctc tgaagatcta tttctataca ggacacatga aattcttgta    1560
   agtctatttc cttcgagatt ggatgatgcc atctctgtgt ccccttaagc atgtctgtta    1620
   tgagcttcct tgacttcaca cctctcagat ttgacaaaca tgaggagaaa caaagcaaac    1680
   tctcttttct gtgggttaat ttgtctctca ttttgggtcc ttcgtatatc agagtgtgaa    1740
   tgatacttta ttgtcctcac ttgtgcttca attcgtggct gttttgcagg aggagctcct    1800
   aaaaaaaatg cagtctttat gggaaaaagc ttgtgaaaat ctcagaaatc tgaacatgga    1860
   aaccacaaga accagatgct ggaaggttag taccgtatga ctctaccttc tccgggaact    1920
   tatagtgaac aaatgggtga ctcttaaaat aggaacttga tctcaaccca taatgtttct    1980
   ggaattcaat aaacaaggaa aaaacacttg agaaaaaaac accctaattt tttatataag    2040
   ttagtgtgac tctttggtag gatatctaat cagaccacag atgttaccca agcatgctca    2100
   tctgtttcca tacaaaccta tagcaatacc ccagcaaata caagaaactg ggacacttca    2160
   cactcttccc aatgggctgc ctggataaat tctggacaca agagttattt gtcagtcatg    2220
   gaaatttcag gtgacccttc taaggctgag tcagtatgaa tttgaatcct ggtgggcaag    2280
   tagattgaaa tgtgagctaa atttcaggca gaaggagcaa gagcacaacc ttagagaaag    2340
   catgaaatga aatatcagca ctaattaata ttgaataagc cataacacat aatgggaaaa    2400
   gtggcgagaa atgtacactt gtgtcttgat gaagacataa atatgtgaga aatatgggaa    2460
   ctagtattta atataaggag aagtctaagg acttgagaag aattctagaa atgattttct    2520
   ctttgtggat gtttatattg agggtatgat atctgatatt cattcattaa tttattctaa    2580
   tattcattca ttgaaaggta ttttatgagt acctagccta tgtcaaatat caacttagaa    2640
   aattaaggtg taaagaagaa agaaaacaca caaatcttgc catggaaatg agaaaagcaa    2700
   atggtcacca tgcagatatg tgaagtacat gatgtgttag agtgtagtag tgtctttgga    2760
   gaataatcaa gcaggaaagt agaaaaggag cacagaggcc agggactggg gatgagggtt    2820
   tgagttttaa atagggtggt cagaaaaaag gctcatggaa aaattcacac tgaaacaaaa    2880
   tcttgatcag gaggaaatgt gtatatcctc ctgtttgcct tcctcttcct cataaatata    2940
   tgaatatata tgtgtatctg gatgttggta tatatatata tatatatgga tacatttgag    3000
   g                                                                    3001
<210> 102
   <211> 3001
   <212> DNA
   <213> human
<400> 102
   acgcgttttt tagattatat atttgttttt attttatata tgatattgta taggagagga 60
   agtttattag gggaatttgt ttttttgatt atggagtttg agaagttgaa tataggttgt 120
   tattaaggta gtgttttgga gatattaata ttttggttga gttagaatta gttttaggag 180
   aaagagagga aattgttttg tttttgtttt ttatttttag tttgagttat tagttgattg 240
   gatagtgttt atttatattg agggttgttg ttgtttattt agtttattaa tttatatgat 300
   ttttcggaaa tatttttata gatttattta gaaattatgt tttattaatt ttttatgtat 360
   tatttaattt agttaagtta atatttataa ttaattataa tattatagta atataatgat 420
   atatatttta gtttttaaaa aattgattat atgttagttt atagtttgag tagtttaaag 480
   aagaagttaa tttaagttat aagaaaaata aatttaatat tttttattat ttattttatt 540
   ttttagtgat tttagattgg tttaattgtt gttttttttt ttttaatttg gtcggttggt 600
   ttgttttggg gttgtttttg ttttaaagag tcggtttttc ggtttttttt tttaaaaata 660
   tgttatagta atttttgatt atttttttat gttttgttat ttaatgtagg ttttttattt 720
   tttttttata gttatttagt tgttatttga aagagaaata gggataagta tgttttattt 780
   tagattattt aagattaaaa ttcgagtttt atgtcgatat ttagggagta ggtttttgaa 840
   tagatttgta ttatgttatg ggtaggaatt aggagtagaa gaggttgtga gttatttagt 900
   tagtagtgtt tgttaaaaag tttaaagatt ttttttgtgt ttgaattttt tatttttgtt 960
   atagaggaaa tagtttgttt cgttttaatg agtattgtta agaagaaaat atagttatta 1020
   tatattatta tatgtttacg gatttttatt aatttagatt ttaaaatgat atgttgtttt 1080
   ttttttttta gaaatatgaa ttttggaatt ttgtaagttt tttagagggt atttatttgt 1140
   tttatttgta tgaattattt tttagattta gttattatag attgtgggta tagtttttgt 1200
   cggttttgtt tgtattttaa ttggtaagat acggtagttt ttgtttagtg ttttgaatgt 1260
   aagaagataa cgcggtagag aaattttaat attgatattt gtttgaagaa tatggttttt 1320
   attgttagaa aagttagttt tcggtaattt tttagttttg aggagtaaat atgtgggatg 1380
   tatagagaga taaagaagat gttttgtgaa gtggataaga gtttgttttg tttgtcgtgt 1440
   tttaattttt aggagtatcg gaattatata tattgtttta ttgagtgggt tgttgaggaa 1500
   cgtcgggtaa gtgatgtttt tgaagattta tttttatata ggatatatga aatttttgta 1560
   agtttatttt tttcgagatt ggatgatgtt atttttgtgt ttttttaagt atgtttgtta 1620
   tgagtttttt tgattttata ttttttagat ttgataaata tgaggagaaa taaagtaaat 1680
   tttttttttt gtgggttaat ttgtttttta ttttgggttt ttcgtatatt agagtgtgaa 1740
   tgatatttta ttgtttttat ttgtgtttta attcgtggtt gttttgtagg aggagttttt 1800
   aaaaaaaatg tagtttttat gggaaaaagt ttgtgaaaat tttagaaatt tgaatatgga 1860
   aattataaga attagatgtt ggaaggttag tatcgtatga ttttattttt ttcgggaatt 1920
   tatagtgaat aaatgggtga tttttaaaat aggaatttga ttttaattta taatgttttt 1980
   ggaatttaat aaataaggaa aaaatatttg agaaaaaaat attttaattt tttatataag 2040
   ttagtgtgat tttttggtag gatatttaat tagattatag atgttattta agtatgttta 2100
   tttgttttta tataaattta tagtaatatt ttagtaaata taagaaattg ggatatttta 2160
   tatttttttt aatgggttgt ttggataaat tttggatata agagttattt gttagttatg 2220
   gaaattttag gtgatttttt taaggttgag ttagtatgaa tttgaatttt ggtgggtaag 2280
   tagattgaaa tgtgagttaa attttaggta gaaggagtaa gagtataatt ttagagaaag 2340
   tatgaaatga aatattagta ttaattaata ttgaataagt tataatatat aatgggaaaa 2400
   gtggcgagaa atgtatattt gtgttttgat gaagatataa atatgtgaga aatatgggaa 2460
   ttagtattta atataaggag aagtttaagg atttgagaag aattttagaa atgatttttt 2520
   ttttgtggat gtttatattg agggtatgat atttgatatt tatttattaa tttattttaa 2580
   tatttattta ttgaaaggta ttttatgagt atttagttta tgttaaatat taatttagaa 2640
   aattaaggtg taaagaagaa agaaaatata taaattttgt tatggaaatg agaaaagtaa 2700
   atggttatta tgtagatatg tgaagtatat gatgtgttag agtgtagtag tgtttttgga 2760
   gaataattaa gtaggaaagt agaaaaggag tatagaggtt agggattggg gatgagggtt 2820
   tgagttttaa atagggtggt tagaaaaaag gtttatggaa aaatttatat tgaaataaaa 2880
   ttttgattag gaggaaatgt gtatattttt ttgtttgttt tttttttttt tataaatata 2940
   tgaatatata tgtgtatttg gatgttggta tatatatata tatatatgga tatatttgag 3000
   g 3001
<210> 103
   <211> 3001
   <212> DNA
   <213> human
<400> 103
   atgtgttttt tagattatat atttgttttt attttatata tgatattgta taggagagga 60
   agtttattag gggaatttgt ttttttgatt atggagtttg agaagttgaa tataggttgt 120
   tattaaggta gtgttttgga gatattaata ttttggttga gttagaatta gttttaggag 180
   aaagagagga aattgttttg tttttgtttt ttatttttag tttgagttat tagttgattg 240
   gatagtgttt atttatattg agggttgttg ttgtttattt agtttattaa tttatatgat 300
   tttttggaaa tatttttata gatttattta gaaattatgt tttattaatt ttttatgtat 360
   tatttaattt agttaagtta atatttataa ttaattataa tattatagta atataatgat 420
   atatatttta gtttttaaaa aattgattat atgttagttt atagtttgag tagtttaaag 480
   aagaagttaa tttaagttat aagaaaaata aatttaatat tttttattat ttattttatt 540
   ttttagtgat tttagattgg tttaattgtt gttttttttt ttttaatttg gttggttggt 600
   ttgttttggg gttgtttttg ttttaaagag ttggtttttt ggtttttttt tttaaaaata 660
   tgttatagta atttttgatt atttttttat gttttgttat ttaatgtagg ttttttattt 720
   tttttttata gttatttagt tgttatttga aagagaaata gggataagta tgttttattt 780
   tagattattt aagattaaaa tttgagtttt atgttgatat ttagggagta ggtttttgaa 840
   tagatttgta ttatgttatg ggtaggaatt aggagtagaa gaggttgtga gttatttagt 900
   tagtagtgtt tgttaaaaag tttaaagatt ttttttgtgt ttgaattttt tatttttgtt 960
   atagaggaaa tagtttgttt tgttttaatg agtattgtta agaagaaaat atagttatta 1020
   tatattatta tatgtttatg gatttttatt aatttagatt ttaaaatgat atgttgtttt 1080
   ttttttttta gaaatatgaa ttttggaatt ttgtaagttt tttagagggt atttatttgt 1140
   tttatttgta tgaattattt tttagattta gttattatag attgtgggta tagtttttgt 1200
   tggttttgtt tgtattttaa ttggtaagat atggtagttt ttgtttagtg ttttgaatgt 1260
   aagaagataa tgtggtagag aaattttaat attgatattt gtttgaagaa tatggttttt 1320
   attgttagaa aagttagttt ttggtaattt tttagttttg aggagtaaat atgtgggatg 1380
   tatagagaga taaagaagat gttttgtgaa gtggataaga gtttgttttg tttgttgtgt 1440
   tttaattttt aggagtattg gaattatata tattgtttta ttgagtgggt tgttgaggaa 1500
   tgttgggtaa gtgatgtttt tgaagattta tttttatata ggatatatga aatttttgta 1560
   agtttatttt ttttgagatt ggatgatgtt atttttgtgt ttttttaagt atgtttgtta 1620
   tgagtttttt tgattttata ttttttagat ttgataaata tgaggagaaa taaagtaaat 1680
   tttttttttt gtgggttaat ttgtttttta ttttgggttt tttgtatatt agagtgtgaa 1740
   tgatatttta ttgtttttat ttgtgtttta atttgtggtt gttttgtagg aggagttttt 1800
   aaaaaaaatg tagtttttat gggaaaaagt ttgtgaaaat tttagaaatt tgaatatgga 1860
   aattataaga attagatgtt ggaaggttag tattgtatga ttttattttt tttgggaatt 1920
   tatagtgaat aaatgggtga tttttaaaat aggaatttga ttttaattta taatgttttt 1980
   ggaatttaat aaataaggaa aaaatatttg agaaaaaaat attttaattt tttatataag 2040
   ttagtgtgat tttttggtag gatatttaat tagattatag atgttattta agtatgttta 2100
   tttgttttta tataaattta tagtaatatt ttagtaaata taagaaattg ggatatttta 2160
   tatttttttt aatgggttgt ttggataaat tttggatata agagttattt gttagttatg 2220
   gaaattttag gtgatttttt taaggttgag ttagtatgaa tttgaatttt ggtgggtaag 2280
   tagattgaaa tgtgagttaa attttaggta gaaggagtaa gagtataatt ttagagaaag 2340
   tatgaaatga aatattagta ttaattaata ttgaataagt tataatatat aatgggaaaa 2400
   gtggtgagaa atgtatattt gtgttttgat gaagatataa atatgtgaga aatatgggaa 2460
   ttagtattta atataaggag aagtttaagg atttgagaag aattttagaa atgatttttt 2520
   ttttgtggat gtttatattg agggtatgat atttgatatt tatttattaa tttattttaa 2580
   tatttattta ttgaaaggta ttttatgagt atttagttta tgttaaatat taatttagaa 2640
   aattaaggtg taaagaagaa agaaaatata taaattttgt tatggaaatg agaaaagtaa 2700
   atggttatta tgtagatatg tgaagtatat gatgtgttag agtgtagtag tgtttttgga 2760
   gaataattaa gtaggaaagt agaaaaggag tatagaggtt agggattggg gatgagggtt 2820
   tgagttttaa atagggtggt tagaaaaaag gtttatggaa aaatttatat tgaaataaaa 2880
   ttttgattag gaggaaatgt gtatattttt ttgtttgttt tttttttttt tataaatata 2940
   tgaatatata tgtgtatttg gatgttggta tatatatata tatatatgga tatatttgag 3000
   g 3001
<210> 104
   <211> 3001
   <212> DNA
   <213> human
<400> 104
   ttttaaatgt atttatatat atatatatat atattaatat ttagatatat atatatattt 60
   atatatttat gaggaagagg aaggtaaata ggaggatata tatatttttt tttgattaag 120
   attttgtttt agtgtgaatt tttttatgag tttttttttt gattatttta tttaaaattt 180
   aaatttttat ttttagtttt tggtttttgt gttttttttt tatttttttg tttgattatt 240
   ttttaaagat attattatat tttaatatat tatgtatttt atatatttgt atggtgatta 300
   tttgtttttt ttatttttat ggtaagattt gtgtgttttt tttttttttt atattttaat 360
   tttttaagtt gatatttgat ataggttagg tatttataaa atatttttta atgaatgaat 420
   attagaataa attaatgaat gaatattaga tattatattt ttaatataaa tatttataaa 480
   gagaaaatta tttttagaat tttttttaag tttttagatt tttttttata ttaaatatta 540
   gtttttatat tttttatata tttatgtttt tattaagata taagtgtata tttttcgtta 600
   ttttttttat tatgtgttat ggtttattta atattaatta gtgttgatat tttattttat 660
   gtttttttta aggttgtgtt tttgtttttt ttgtttgaaa tttagtttat attttaattt 720
   atttgtttat taggatttaa atttatattg atttagtttt agaagggtta tttgaaattt 780
   ttatgattga taaataattt ttgtgtttag aatttattta ggtagtttat tgggaagagt 840
   gtgaagtgtt ttagtttttt gtatttgttg gggtattgtt ataggtttgt atggaaatag 900
   atgagtatgt ttgggtaata tttgtggttt gattagatat tttattaaag agttatatta 960
   atttatataa aaaattaggg tgtttttttt ttaagtgttt tttttttgtt tattgaattt 1020
   tagaaatatt atgggttgag attaagtttt tattttaaga gttatttatt tgtttattat 1080
   aagttttcgg agaaggtaga gttatacggt attaattttt tagtatttgg tttttgtggt 1140
   ttttatgttt agatttttga gatttttata agtttttttt tataaagatt gtattttttt 1200
   taggagtttt ttttgtaaaa tagttacgaa ttgaagtata agtgaggata ataaagtatt 1260
   atttatattt tgatatacga aggatttaaa atgagagata aattaattta tagaaaagag 1320
   agtttgtttt gttttttttt atgtttgtta aatttgagag gtgtgaagtt aaggaagttt 1380
   ataatagata tgtttaaggg gatatagaga tggtattatt taatttcgaa ggaaatagat 1440
   ttataagaat tttatgtgtt ttgtatagaa atagattttt agaggtatta tttattcggc 1500
   gttttttagt agtttattta atgggatagt gtatgtgatt tcggtgtttt tgagagttgg 1560
   agtacggtaa atagagtagg tttttgttta ttttatagaa tatttttttt gtttttttgt 1620
   gtattttata tatttgtttt ttagagttaa ggaattgtcg gaggttggtt tttttggtaa 1680
   tgaaagttat gttttttaaa taaatgttag tgttgaggtt tttttgtcgc gttgtttttt 1740
   tgtatttaga gtattgagta agaattgtcg tgttttgtta gttgaggtat aaatagggtc 1800
   ggtaaaagtt gtgtttatag tttatggtga ttgggtttag gaagtagttt atgtagatgg 1860
   gataggtgag tgttttttgg aagatttgta agattttaga atttatgttt ttgaagaaga 1920
   aagagtagta tgttattttg gggtttgggt tggtgaaaat tcgtgaatat gtggtgatat 1980
   gtgatagtta tatttttttt ttgatagtgt ttattaaagc ggaataaatt attttttttg 2040
   taataaaaat gaaaaattta aatataaaga gagtttttag gttttttagt agatattgtt 2100
   gattagatga tttataattt tttttatttt tggtttttgt ttatagtata atgtaaattt 2160
   gtttaaaaat ttattttttg gatgtcgata tgaaattcgg gttttaattt taagtggttt 2220
   agaatgaaat atgtttgttt ttattttttt tttaaataat aattgaatga ttatgggaga 2280
   ggagtagaaa atttatattg ggtaataaaa tatgagaaga tggttagagg ttgttatgat 2340
   atatttttag agagagggat cgagaagtcg gttttttaaa ataaaaataa ttttagaata 2400
   aattaatcga ttagattaaa aaaaaaaaga tagtaattaa attagtttag gattattagg 2460
   agataaaata aataatgaaa aatgttgggt ttattttttt tatggtttaa attaattttt 2520
   tttttgggtt atttaaatta tgaattgata tatagttagt tttttaaaaa ttgaaatata 2580
   tattattata ttattatggt attatggtta attataggtg ttgatttgat tggattaaat 2640
   aatatatgga gaattggtaa agtatgattt ttgggtgagt ttgtgaaggt gttttcggag 2700
   agttatgtaa gttggtgagt tgagtgggta atagtagttt ttagtgtggg tggatattat 2760
   ttaattagtt ggtagtttag attgaagata aagggtagag ataaggtagt tttttttttt 2820
   ttttttgaag ttgattttaa tttagttagg atattggtat ttttaggata ttattttaat 2880
   gatagtttat gtttaatttt ttagatttta taattaaggg aataaatttt tttagtggat 2940
   tttttttttt gtatagtgtt atgtgtagag tgaggataga tatatgattt gagggacgcg 3000
   t                                                                 3001
<210> 105
   <211> 3001
   <212> DNA
   <213> human
<400> 105
   ttttaaatgt atttatatat atatatatat atattaatat ttagatatat atatatattt 60
   atatatttat gaggaagagg aaggtaaata ggaggatata tatatttttt tttgattaag 120
   attttgtttt agtgtgaatt tttttatgag tttttttttt gattatttta tttaaaattt 180
   aaatttttat ttttagtttt tggtttttgt gttttttttt tatttttttg tttgattatt 240
   ttttaaagat attattatat tttaatatat tatgtatttt atatatttgt atggtgatta 300
   tttgtttttt ttatttttat ggtaagattt gtgtgttttt tttttttttt atattttaat 360
   tttttaagtt gatatttgat ataggttagg tatttataaa atatttttta atgaatgaat 420
   attagaataa attaatgaat gaatattaga tattatattt ttaatataaa tatttataaa 480
   gagaaaatta tttttagaat tttttttaag tttttagatt tttttttata ttaaatatta 540
   gtttttatat tttttatata tttatgtttt tattaagata taagtgtata ttttttgtta 600
   ttttttttat tatgtgttat ggtttattta atattaatta gtgttgatat tttattttat 660
   gtttttttta aggttgtgtt tttgtttttt ttgtttgaaa tttagtttat attttaattt 720
   atttgtttat taggatttaa atttatattg atttagtttt agaagggtta tttgaaattt 780
   ttatgattga taaataattt ttgtgtttag aatttattta ggtagtttat tgggaagagt 840
   gtgaagtgtt ttagtttttt gtatttgttg gggtattgtt ataggtttgt atggaaatag 900
   atgagtatgt ttgggtaata tttgtggttt gattagatat tttattaaag agttatatta 960
   atttatataa aaaattaggg tgtttttttt ttaagtgttt tttttttgtt tattgaattt 1020
   tagaaatatt atgggttgag attaagtttt tattttaaga gttatttatt tgtttattat 1080
   aagtttttgg agaaggtaga gttatatggt attaattttt tagtatttgg tttttgtggt 1140
   ttttatgttt agatttttga gatttttata agtttttttt tataaagatt gtattttttt 1200
   taggagtttt ttttgtaaaa tagttatgaa ttgaagtata agtgaggata ataaagtatt 1260
   atttatattt tgatatatga aggatttaaa atgagagata aattaattta tagaaaagag 1320
   agtttgtttt gttttttttt atgtttgtta aatttgagag gtgtgaagtt aaggaagttt 1380
   ataatagata tgtttaaggg gatatagaga tggtattatt taattttgaa ggaaatagat 1440
   ttataagaat tttatgtgtt ttgtatagaa atagattttt agaggtatta tttatttggt 1500
   gttttttagt agtttattta atgggatagt gtatgtgatt ttggtgtttt tgagagttgg 1560
   agtatggtaa atagagtagg tttttgttta ttttatagaa tatttttttt gtttttttgt 1620
   gtattttata tatttgtttt ttagagttaa ggaattgttg gaggttggtt tttttggtaa 1680
   tgaaagttat gttttttaaa taaatgttag tgttgaggtt tttttgttgt gttgtttttt 1740
   tgtatttaga gtattgagta agaattgttg tgttttgtta gttgaggtat aaatagggtt 1800
   ggtaaaagtt gtgtttatag tttatggtga ttgggtttag gaagtagttt atgtagatgg 1860
   gataggtgag tgttttttgg aagatttgta agattttaga atttatgttt ttgaagaaga 1920
   aagagtagta tgttattttg gggtttgggt tggtgaaaat ttgtgaatat gtggtgatat 1980
   gtgatagtta tatttttttt ttgatagtgt ttattaaagt ggaataaatt attttttttg 2040
   taataaaaat gaaaaattta aatataaaga gagtttttag gttttttagt agatattgtt 2100
   gattagatga tttataattt tttttatttt tggtttttgt ttatagtata atgtaaattt 2160
   gtttaaaaat ttattttttg gatgttgata tgaaatttgg gttttaattt taagtggttt 2220
   agaatgaaat atgtttgttt ttattttttt tttaaataat aattgaatga ttatgggaga 2280
   ggagtagaaa atttatattg ggtaataaaa tatgagaaga tggttagagg ttgttatgat 2340
   atatttttag agagagggat tgagaagttg gttttttaaa ataaaaataa ttttagaata 2400
   aattaattga ttagattaaa aaaaaaaaga tagtaattaa attagtttag gattattagg 2460
   agataaaata aataatgaaa aatgttgggt ttattttttt tatggtttaa attaattttt 2520
   tttttgggtt atttaaatta tgaattgata tatagttagt tttttaaaaa ttgaaatata 2580
   tattattata ttattatggt attatggtta attataggtg ttgatttgat tggattaaat 2640
   aatatatgga gaattggtaa agtatgattt ttgggtgagt ttgtgaaggt gtttttggag 2700
   agttatgtaa gttggtgagt tgagtgggta atagtagttt ttagtgtggg tggatattat 2760
   ttaattagtt ggtagtttag attgaagata aagggtagag ataaggtagt tttttttttt 2820
   ttttttgaag ttgattttaa tttagttagg atattggtat ttttaggata ttattttaat 2880
   gatagtttat gtttaatttt ttagatttta taattaaggg aataaatttt tttagtggat 2940
   tttttttttt gtatagtgtt atgtgtagag tgaggataga tatatgattt gagggatgtg 3000
   t 3001
<210> 106
   <211> 50
   <212> DNA
   <213> human
<400> 106
   ccttccatat tctgtttctc cctgaccacg ggtgcatgta gtccttgtcg 50
<210> 107
   <211> 50
   <212> DNA
   <213> human
<400> 107
   ttttttatat tttgtttttt tttgattacg ggtgtatgta gtttttgtcg 50
<210> 108
   <211> 50
   <212> DNA
   <213> human
<400> 108
   ttttttatat tttgtttttt tttgattatg ggtgtatgta gtttttgttg 50
<210> 109
   <211> 50
   <212> DNA
   <213> human
<400> 109
   cgataaggat tatatgtatt cgtggttagg gagaaataga atatggaagg 50
<210> 110
   <211> 50
   <212> DNA
   <213> human
<400> 110
   tgataaggat tatatgtatt tgtggttagg gagaaataga atatggaagg 50
<210> 111
   <211> 3001
   <212> DNA
   <213> human
<400> 111
   atctaatcaa taatacaaaa gattcttgaa tcttggtata ggcagctatc tcttgaaaat    60
   attaacagca catcggaggg ctcttgttga agaaagatta tatcagaaac aaaatataca   120
   gttgcaaaga atactacatt gttttttcct cagaaaattc ttttacttag aatacttacc  180
   tgtgaagtaa aatgttgccc aagcttcacg tagaagatca gaatagtgtt tactacaagc   240
   acagtgaatt ctgcaattga tactatgaat catacccacc tgaacccctc caaatccctt   300
   gggagctaaa tatcgctcac attcaagagc aatatcaacc catcgccatt caaacagatg   360
   aacaatagat gttcgtcctt gttgtgtatt tgaggaatac tgagcccagc agaacccaat   420
   ggtgaaaagc aaccaaaaga gcttcatttt gctttgaagt tgtcagtgtc ctttccagaa   480
   actatttata ttcctgtaag aagcacattt tacaatgtaa atagtagcat gaataaatac   540
   ttgagggcaa actgtttatt cataatctga aaaggattat caataataat cctaactggt   600
   gaagaacatc aaaaagtctc tcatggaaat catctcaatg acctcttaga cattaatgtt   660
   tcttttcttt tctttttttt ttttttttgc atatggtagc acttttattt ttccttacac  720
   aatgacatgt tgctgtggcc taatgttctc acataacagc agaaaaccaa aatttgttgt   780
   caactcttaa aagatcgaga attgcatact aaaaaacttt acataaatta aaaggatgaa   840
   tacatttacc ggtataaatg cgaaccgctt ccaactcaag gcaagtaaca gcccacggtg   900
   ctctggtaga taacgtaagc taagaaagga aactgggtcc tatggcttgg actttccaac   960
   cctgacagac cgacaagacg gaaacaactg gttcaggagc ccttcccagc ctctagataa  1020
   atctcagaac actcagccct gacacattaa taccctgcac ggatcagaga ctgctgacca  1080
   cacagactca ccaagccaga cttgtcttcc acaagcacgt ttttagccat gaagtgacca  1140
   agccacgtgt actaaacact gacatcaaag gtatgtacag ataccaaggg gaagagttaa  1200
   cttgaatgcc aggccaaaat cagcaacaag ttctacaatc cagtgctgat attgagaaga  1260
   gagaaagacc ctgtcctctt gttttatatt gttttctatt cagtaaaaac aacaaggaag   1320
   caaaatcaaa ggcacaaaac caggcctggg cctggttggc ctaaacccag tagttacaaa  1380
   tcaacttatg atttagaagc cgatgttatt catagattcc agacattgta tagaagaaca  1440
   ttgtgaaact cccttccata ttctgtttct ccctgaccac gggtgcatgt agtccttgtc  1500
   gcatatccct ggcttgttca aatcaatcac gaccctttca tgtgacatct ttagtgttgt  1560
   gtgcccttaa aagggacaga aattgtgcac ttggggagct cggattttga gacagtagct  1620
   atccgatgct cccaggtgaa taacaccctt ccttctacaa ctcggtgtct gagagttttg  1680
   tctgccgctt gtcctgctac atttcttggt tccctgacct ggaagcaaga taactgatgg  1740
   acggccgagg cagctcctta ggcagattag gcatgccttg tggagtaagg ccggggcatc  1800
   cctcaggctg ccagggactc tggccagcct gagggatggg atccaaagag cgctcccagg  1860
   taggaaatgg ccccggttga acgcctcgcc agagcagtga tccccgcgga ggattaacac  1920
   agtggctgaa caccaggaag gaactggcac ttggagtcca gacatctgaa acttagtaag  1980
   acaagtcttt tcaacttgcc ccactccacc tgagcggaag cttggcctga tcgctcatgg  2040
   tgtgcctgca cccatatcaa tttccatata gagattccta gttacagcta gttttagatc  2100
   ctctacagtg gtaaaaggac aggcagcagc agtagtagta gcaaagggac atttatttca  2160
   ggaaagttct cgctccaccg gccaagagaa gccagcacct aaagttctgt ttgacccaga  2220
   gctcgaggac tcaaggcagg agatggcacc aacagtgccc tcaacccctt atccagtggg  2280
   gaggccccct tctcctgagc ccacagcccc tagaccaccc agagtagaca agaacaaaag  2340
   tgaaactgcg ggaaaatcca cttccctggc agcccgctta acggcccaag acgggaattc  2400
   aaatgccctg agagagcagc gatatactag gacagatgag gtcagacata cggtagaaag  2460
   gcatgctttt gtgtatcacc cttttacctc tgctgaacta ctcaactgga aaaataacac  2520
   tccatcttac actgaaaagc ctcaagccct aattgacttg cttcaaagaa ttatccagac  2580
   tcagaatcct acttaggctg attgccacca gctactcatg tacctcttta aaacacataa  2640
   aaggcaacag gtgctgcagg caacagttaa atggctggag gagcacatcc cagccaatta  2700
   tcaaagtccc caagaataca taagaattca gctgccagga acggaccctc aataggatcc  2760
   aaatgaagga ccagatatgg aaaggctaag agggtaccga gaggcattaa ttgaaaggtt  2820
   gaaaaaaggg gctcaaaagg gtaccaatgt aaataaagtt tctgaagtca tccaaggaaa  2880
   ggaggaaagc ccagcccagt tctatcaaag accgtgtgag gcctattgca tgtacactcc  2940
   tttcgatctg gagagtcctg aaaatcagcc gttgattaat acggccttag ttattcagag  3000
   t                                                                   3001
<210> 112
   <211> 3001
   <212> DNA
   <213> human
<400> 112
   atttaattaa taatataaaa gatttttgaa ttttggtata ggtagttatt ttttgaaaat     60
   attaatagta tatcggaggg tttttgttga agaaagatta tattagaaat aaaatatata    120
   gttgtaaaga atattatatt gttttttttt tagaaaattt ttttatttag aatatttatt    180
   tgtgaagtaa aatgttgttt aagttttacg tagaagatta gaatagtgtt tattataagt    240
   atagtgaatt ttgtaattga tattatgaat tatatttatt tgaatttttt taaatttttt    300
   gggagttaaa tatcgtttat atttaagagt aatattaatt tatcgttatt taaatagatg    360
   aataatagat gttcgttttt gttgtgtatt tgaggaatat tgagtttagt agaatttaat    420
   ggtgaaaagt aattaaaaga gttttatttt gttttgaagt tgttagtgtt ttttttagaa    480
   attatttata tttttgtaag aagtatattt tataatgtaa atagtagtat gaataaatat    540
   ttgagggtaa attgtttatt tataatttga aaaggattat taataataat tttaattggt    600
   gaagaatatt aaaaagtttt ttatggaaat tattttaatg attttttaga tattaatgtt    660
   tttttttttt tttttttttt ttttttttgt atatggtagt atttttattt ttttttatat    720
   aatgatatgt tgttgtggtt taatgttttt atataatagt agaaaattaa aatttgttgt    780
   taatttttaa aagatcgaga attgtatatt aaaaaatttt atataaatta aaaggatgaa    840
   tatatttatc ggtataaatg cgaatcgttt ttaatttaag gtaagtaata gtttacggtg    900
   ttttggtaga taacgtaagt taagaaagga aattgggttt tatggtttgg attttttaat    960
   tttgatagat cgataagacg gaaataattg gtttaggagt tttttttagt ttttagataa   1020
   attttagaat atttagtttt gatatattaa tattttgtac ggattagaga ttgttgatta   1080
   tatagattta ttaagttaga tttgtttttt ataagtacgt ttttagttat gaagtgatta   1140
   agttacgtgt attaaatatt gatattaaag gtatgtatag atattaaggg gaagagttaa   1200
   tttgaatgtt aggttaaaat tagtaataag ttttataatt tagtgttgat attgagaaga   1260
   gagaaagatt ttgttttttt gttttatatt gttttttatt tagtaaaaat aataaggaag   1320
   taaaattaaa ggtataaaat taggtttggg tttggttggt ttaaatttag tagttataaa   1380
   ttaatttatg atttagaagt cgatgttatt tatagatttt agatattgta tagaagaata   1440
   ttgtgaaatt tttttttata ttttgttttt ttttgattac gggtgtatgt agtttttgtc   1500
   gtatattttt ggtttgttta aattaattac gattttttta tgtgatattt ttagtgttgt   1560
   gtgtttttaa aagggataga aattgtgtat ttggggagtt cggattttga gatagtagtt   1620
   attcgatgtt tttaggtgaa taatattttt ttttttataa ttcggtgttt gagagttttg   1680
   tttgtcgttt gttttgttat attttttggt tttttgattt ggaagtaaga taattgatgg   1740
   acggtcgagg tagtttttta ggtagattag gtatgttttg tggagtaagg tcggggtatt   1800
   ttttaggttg ttagggattt tggttagttt gagggatggg atttaaagag cgtttttagg   1860
   taggaaatgg tttcggttga acgtttcgtt agagtagtga ttttcgcgga ggattaatat   1920
   agtggttgaa tattaggaag gaattggtat ttggagttta gatatttgaa atttagtaag   1980
   ataagttttt ttaatttgtt ttattttatt tgagcggaag tttggtttga tcgtttatgg   2040
   tgtgtttgta tttatattaa tttttatata gagattttta gttatagtta gttttagatt   2100
   ttttatagtg gtaaaaggat aggtagtagt agtagtagta gtaaagggat atttatttta   2160
   ggaaagtttt cgttttatcg gttaagagaa gttagtattt aaagttttgt ttgatttaga   2220
   gttcgaggat ttaaggtagg agatggtatt aatagtgttt ttaatttttt atttagtggg   2280
   gaggtttttt ttttttgagt ttatagtttt tagattattt agagtagata agaataaaag   2340
   tgaaattgcg ggaaaattta tttttttggt agttcgttta acggtttaag acgggaattt   2400
   aaatgttttg agagagtagc gatatattag gatagatgag gttagatata cggtagaaag   2460
   gtatgttttt gtgtattatt tttttatttt tgttgaatta tttaattgga aaaataatat   2520
   tttattttat attgaaaagt tttaagtttt aattgatttg ttttaaagaa ttatttagat   2580
   ttagaatttt atttaggttg attgttatta gttatttatg tattttttta aaatatataa   2640
   aaggtaatag gtgttgtagg taatagttaa atggttggag gagtatattt tagttaatta   2700
   ttaaagtttt taagaatata taagaattta gttgttagga acggattttt aataggattt   2760
   aaatgaagga ttagatatgg aaaggttaag agggtatcga gaggtattaa ttgaaaggtt   2820
   gaaaaaaggg gtttaaaagg gtattaatgt aaataaagtt tttgaagtta tttaaggaaa   2880
   ggaggaaagt ttagtttagt tttattaaag atcgtgtgag gtttattgta tgtatatttt   2940
   tttcgatttg gagagttttg aaaattagtc gttgattaat acggttttag ttatttagag   3000
   t                                                                   3001
<210> 113
   <211> 3001
   <212> DNA
   <213> human
<400> 113
   atttaattaa taatataaaa gatttttgaa ttttggtata ggtagttatt ttttgaaaat     60
   attaatagta tattggaggg tttttgttga agaaagatta tattagaaat aaaatatata    120
   gttgtaaaga atattatatt gttttttttt tagaaaattt ttttatttag aatatttatt    180
   tgtgaagtaa aatgttgttt aagttttatg tagaagatta gaatagtgtt tattataagt    240
   atagtgaatt ttgtaattga tattatgaat tatatttatt tgaatttttt taaatttttt    300
   gggagttaaa tattgtttat atttaagagt aatattaatt tattgttatt taaatagatg 360
   aataatagat gtttgttttt gttgtgtatt tgaggaatat tgagtttagt agaatttaat 420
   ggtgaaaagt aattaaaaga gttttatttt gttttgaagt tgttagtgtt ttttttagaa 480
   attatttata tttttgtaag aagtatattt tataatgtaa atagtagtat gaataaatat 540
   ttgagggtaa attgtttatt tataatttga aaaggattat taataataat tttaattggt 600
   gaagaatatt aaaaagtttt ttatggaaat tattttaatg attttttaga tattaatgtt 660
   tttttttttt tttttttttt ttttttttgt atatggtagt atttttattt ttttttatat 720
   aatgatatgt tgttgtggtt taatgttttt atataatagt agaaaattaa aatttgttgt 780
   taatttttaa aagattgaga attgtatatt aaaaaatttt atataaatta aaaggatgaa 840
   tatatttatt ggtataaatg tgaattgttt ttaatttaag gtaagtaata gtttatggtg 900
   ttttggtaga taatgtaagt taagaaagga aattgggttt tatggtttgg attttttaat 960
   tttgatagat tgataagatg gaaataattg gtttaggagt tttttttagt ttttagataa 1020
   attttagaat atttagtttt gatatattaa tattttgtat ggattagaga ttgttgatta 1080
   tatagattta ttaagttaga tttgtttttt ataagtatgt ttttagttat gaagtgatta 1140
   agttatgtgt attaaatatt gatattaaag gtatgtatag atattaaggg gaagagttaa 1200
   tttgaatgtt aggttaaaat tagtaataag ttttataatt tagtgttgat attgagaaga 1260
   gagaaagatt ttgttttttt gttttatatt gttttttatt tagtaaaaat aataaggaag 1320
   taaaattaaa ggtataaaat taggtttggg tttggttggt ttaaatttag tagttataaa 1380
   ttaatttatg atttagaagt tgatgttatt tatagatttt agatattgta tagaagaata 1440
   ttgtgaaatt tttttttata ttttgttttt ttttgattat gggtgtatgt agtttttgtt 1500
   gtatattttt ggtttgttta aattaattat gattttttta tgtgatattt ttagtgttgt 1560
   gtgtttttaa aagggataga aattgtgtat ttggggagtt tggattttga gatagtagtt 1620
   atttgatgtt tttaggtgaa taatattttt ttttttataa tttggtgttt gagagttttg 1680
   tttgttgttt gttttgttat attttttggt tttttgattt ggaagtaaga taattgatgg 1740
   atggttgagg tagtttttta ggtagattag gtatgttttg tggagtaagg ttggggtatt 1800
   ttttaggttg ttagggattt tggttagttt gagggatggg atttaaagag tgtttttagg 1860
   taggaaatgg ttttggttga atgttttgtt agagtagtga tttttgtgga ggattaatat 1920
   agtggttgaa tattaggaag gaattggtat ttggagttta gatatttgaa atttagtaag 1980
   ataagttttt ttaatttgtt ttattttatt tgagtggaag tttggtttga ttgtttatgg 2040
   tgtgtttgta tttatattaa tttttatata gagattttta gttatagtta gttttagatt 2100
   ttttatagtg gtaaaaggat aggtagtagt agtagtagta gtaaagggat atttatttta 2160
   ggaaagtttt tgttttattg gttaagagaa gttagtattt aaagttttgt ttgatttaga 2220
   gtttgaggat ttaaggtagg agatggtatt aatagtgttt ttaatttttt atttagtggg 2280
   gaggtttttt ttttttgagt ttatagtttt tagattattt agagtagata agaataaaag 2340
   tgaaattgtg ggaaaattta tttttttggt agtttgttta atggtttaag atgggaattt 2400
   aaatgttttg agagagtagt gatatattag gatagatgag gttagatata tggtagaaag 2460
   gtatgttttt gtgtattatt tttttatttt tgttgaatta tttaattgga aaaataatat 2520
   tttattttat attgaaaagt tttaagtttt aattgatttg ttttaaagaa ttatttagat 2580
   ttagaatttt atttaggttg attgttatta gttatttatg tattttttta aaatatataa 2640
   aaggtaatag gtgttgtagg taatagttaa atggttggag gagtatattt tagttaatta 2700
   ttaaagtttt taagaatata taagaattta gttgttagga atggattttt aataggattt 2760
   aaatgaagga ttagatatgg aaaggttaag agggtattga gaggtattaa ttgaaaggtt 2820
   gaaaaaaggg gtttaaaagg gtattaatgt aaataaagtt tttgaagtta tttaaggaaa 2880
   ggaggaaagt ttagtttagt tttattaaag attgtgtgag gtttattgta tgtatatttt 2940
   ttttgatttg gagagttttg aaaattagtt gttgattaat atggttttag ttatttagag 3000
   t 3001

<210> 114
   <211> 3001
   <212> DNA
   <213> human
<400> 114
   attttgaata attaaggtcg tattaattaa cggttgattt ttaggatttt ttagatcgaa     60
   aggagtgtat atgtaatagg ttttatacgg tttttgatag aattgggttg ggtttttttt    120
   ttttttttgg atgattttag aaattttatt tatattggta tttttttgag tttttttttt    180
   taatttttta attaatgttt ttcggtattt ttttagtttt tttatatttg gttttttatt    240
   tggattttat tgagggttcg tttttggtag ttgaattttt atgtattttt ggggattttg    300
   ataattggtt gggatgtgtt tttttagtta tttaattgtt gtttgtagta tttgttgttt    360
   tttatgtgtt ttaaagaggt atatgagtag ttggtggtaa ttagtttaag taggattttg    420
   agtttggata attttttgaa gtaagttaat tagggtttga ggttttttag tgtaagatgg    480
   agtgttattt ttttagttga gtagtttagt agaggtaaaa gggtgatata taaaagtatg    540
   tttttttatc gtatgtttga ttttatttgt tttagtatat cgttgttttt ttagggtatt    600
   tgaattttcg ttttgggtcg ttaagcgggt tgttagggaa gtggattttt tcgtagtttt    660
   atttttgttt ttgtttattt tgggtggttt aggggttgtg ggtttaggag aagggggttt    720
   ttttattgga taaggggttg agggtattgt tggtgttatt ttttgttttg agttttcgag    780
   ttttgggtta aatagaattt taggtgttgg ttttttttgg tcggtggagc gagaattttt    840
   ttgaaataaa tgtttttttg ttattattat tgttgttgtt tgttttttta ttattgtaga    900
   ggatttaaaa ttagttgtaa ttaggaattt ttatatggaa attgatatgg gtgtaggtat    960
   attatgagcg attaggttaa gttttcgttt aggtggagtg gggtaagttg aaaagatttg   1020
   ttttattaag ttttagatgt ttggatttta agtgttagtt tttttttggt gtttagttat   1080
   tgtgttaatt tttcgcgggg attattgttt tggcgaggcg tttaatcggg gttatttttt   1140
   atttgggagc gttttttgga ttttattttt taggttggtt agagtttttg gtagtttgag   1200
   ggatgtttcg gttttatttt ataaggtatg tttaatttgt ttaaggagtt gtttcggtcg   1260
   tttattagtt attttgtttt taggttaggg aattaagaaa tgtagtagga taagcggtag   1320
   ataaaatttt tagatatcga gttgtagaag gaagggtgtt atttatttgg gagtatcgga   1380
   tagttattgt tttaaaattc gagtttttta agtgtataat ttttgttttt tttaagggta   1440
   tataatatta aagatgttat atgaaagggt cgtgattgat ttgaataagt tagggatatg   1500
   cgataaggat tatatgtatt cgtggttagg gagaaataga atatggaagg gagttttata   1560
   atgttttttt atataatgtt tggaatttat gaataatatc ggtttttaaa ttataagttg   1620
   atttgtaatt attgggttta ggttaattag gtttaggttt ggttttgtgt ttttgatttt   1680
   gtttttttgt tgtttttatt gaatagaaaa taatataaaa taagaggata gggttttttt   1740
   ttttttttaa tattagtatt ggattgtaga atttgttgtt gattttggtt tggtatttaa   1800
   gttaattttt ttttttggta tttgtatata tttttgatgt tagtgtttag tatacgtggt   1860
   ttggttattt tatggttaaa aacgtgtttg tggaagataa gtttggtttg gtgagtttgt   1920
   gtggttagta gtttttgatt cgtgtagggt attaatgtgt tagggttgag tgttttgaga   1980
   tttatttaga ggttgggaag ggtttttgaa ttagttgttt tcgttttgtc ggtttgttag   2040
   ggttggaaag tttaagttat aggatttagt tttttttttt agtttacgtt atttattaga   2100
   gtatcgtggg ttgttatttg ttttgagttg gaagcggttc gtatttatat cggtaaatgt   2160
   atttattttt ttaatttatg taaagttttt tagtatgtaa ttttcgattt tttaagagtt   2220
   gataataaat tttggttttt tgttgttatg tgagaatatt aggttatagt aatatgttat   2280
   tgtgtaagga aaaataaaag tgttattata tgtaaaaaaa aaaaaaaaag aaaagaaaag   2340
   aaatattaat gtttaagagg ttattgagat gatttttatg agagattttt tgatgttttt   2400
   tattagttag gattattatt gataattttt tttagattat gaataaatag tttgttttta   2460
   agtatttatt tatgttatta tttatattgt aaaatgtgtt ttttatagga atataaatag   2520
   tttttggaaa ggatattgat aattttaaag taaaatgaag tttttttggt tgttttttat   2580
   tattgggttt tgttgggttt agtatttttt aaatatataa taaggacgaa tatttattgt   2640
   ttatttgttt gaatggcgat gggttgatat tgtttttgaa tgtgagcgat atttagtttt   2700
   taagggattt ggaggggttt aggtgggtat gatttatagt attaattgta gaatttattg   2760
   tgtttgtagt aaatattatt ttgatttttt acgtgaagtt tgggtaatat tttattttat   2820
   aggtaagtat tttaagtaaa agaatttttt gaggaaaaaa taatgtagta ttttttgtaa   2880
   ttgtatattt tgtttttgat ataatttttt tttaataaga gtttttcgat gtgttgttaa   2940
   tatttttaag agatagttgt ttatattaag atttaagaat tttttgtatt attgattaga   3000
   t                                                                  3001
<210> 115
   <211> 3001
   <212> DNA
   <213> human
<400> 115
   attttgaata attaaggttg tattaattaa tggttgattt ttaggatttt ttagattgaa     60
   aggagtgtat atgtaatagg ttttatatgg tttttgatag aattgggttg ggtttttttt    120
   ttttttttgg atgattttag aaattttatt tatattggta tttttttgag tttttttttt       180
   taatttttta attaatgttt tttggtattt ttttagtttt tttatatttg gttttttatt   240
   tggattttat tgagggtttg tttttggtag ttgaattttt atgtattttt ggggattttg    300
   ataattggtt gggatgtgtt tttttagtta tttaattgtt gtttgtagta tttgttgttt    360
   tttatgtgtt ttaaagaggt atatgagtag ttggtggtaa ttagtttaag taggattttg    420
   agtttggata attttttgaa gtaagttaat tagggtttga ggttttttag tgtaagatgg    480
   agtgttattt ttttagttga gtagtttagt agaggtaaaa gggtgatata taaaagtatg    540
   tttttttatt gtatgtttga ttttatttgt tttagtatat tgttgttttt ttagggtatt   600
   tgaatttttg ttttgggttg ttaagtgggt tgttagggaa gtggattttt ttgtagtttt     660
   atttttgttt ttgtttattt tgggtggttt aggggttgtg ggtttaggag aagggggttt    720
   ttttattgga taaggggttg agggtattgt tggtgttatt ttttgttttg agtttttgag    780
   ttttgggtta aatagaattt taggtgttgg ttttttttgg ttggtggagt gagaattttt    840
   ttgaaataaa tgtttttttg ttattattat tgttgttgtt tgttttttta ttattgtaga     900
   ggatttaaaa ttagttgtaa ttaggaattt ttatatggaa attgatatgg gtgtaggtat    960
   attatgagtg attaggttaa gtttttgttt aggtggagtg gggtaagttg aaaagatttg   1020
   ttttattaag ttttagatgt ttggatttta agtgttagtt tttttttggt gtttagttat  1080
   tgtgttaatt ttttgtgggg attattgttt tggtgaggtg tttaattggg gttatttttt   1140
   atttgggagt gttttttgga ttttattttt taggttggtt agagtttttg gtagtttgag   1200
   ggatgttttg gttttatttt ataaggtatg tttaatttgt ttaaggagtt gttttggttg   1260
   tttattagtt attttgtttt taggttaggg aattaagaaa tgtagtagga taagtggtag   1320
   ataaaatttt tagatattga gttgtagaag gaagggtgtt atttatttgg gagtattgga   1380
   tagttattgt tttaaaattt gagtttttta agtgtataat ttttgttttt tttaagggta   1440
   tataatatta aagatgttat atgaaagggt tgtgattgat ttgaataagt tagggatatg   1500
   tgataaggat tatatgtatt tgtggttagg gagaaataga atatggaagg gagttttata   1560
   atgttttttt atataatgtt tggaatttat gaataatatt ggtttttaaa ttataagttg   1620
   atttgtaatt attgggttta ggttaattag gtttaggttt ggttttgtgt ttttgatttt   1680
   gtttttttgt tgtttttatt gaatagaaaa taatataaaa taagaggata gggttttttt   1740
   ttttttttaa tattagtatt ggattgtaga atttgttgtt gattttggtt tggtatttaa   1800
   gttaattttt ttttttggta tttgtatata tttttgatgt tagtgtttag tatatgtggt   1860
   ttggttattt tatggttaaa aatgtgtttg tggaagataa gtttggtttg gtgagtttgt   1920
   gtggttagta gtttttgatt tgtgtagggt attaatgtgt tagggttgag tgttttgaga   1980
   tttatttaga ggttgggaag ggtttttgaa ttagttgttt ttgttttgtt ggtttgttag   2040
   ggttggaaag tttaagttat aggatttagt tttttttttt agtttatgtt atttattaga     2100
   gtattgtggg ttgttatttg ttttgagttg gaagtggttt gtatttatat tggtaaatgt   2160
   atttattttt ttaatttatg taaagttttt tagtatgtaa tttttgattt tttaagagtt   2220
   gataataaat tttggttttt tgttgttatg tgagaatatt aggttatagt aatatgttat   2280
   tgtgtaagga aaaataaaag tgttattata tgtaaaaaaa aaaaaaaaag aaaagaaaag   2340
   aaatattaat gtttaagagg ttattgagat gatttttatg agagattttt tgatgttttt   2400
   tattagttag gattattatt gataattttt tttagattat gaataaatag tttgttttta   2460
   agtatttatt tatgttatta tttatattgt aaaatgtgtt ttttatagga atataaatag   2520
   tttttggaaa ggatattgat aattttaaag taaaatgaag tttttttggt tgttttttat  2580
   tattgggttt tgttgggttt agtatttttt aaatatataa taaggatgaa tatttattgt   2640
   ttatttgttt gaatggtgat gggttgatat tgtttttgaa tgtgagtgat atttagtttt   2700
   taagggattt ggaggggttt aggtgggtat gatttatagt attaattgta gaatttattg   2760
   tgtttgtagt aaatattatt ttgatttttt atgtgaagtt tgggtaatat tttattttat   2820
   aggtaagtat tttaagtaaa agaatttttt gaggaaaaaa taatgtagta ttttttgtaa  2880
   ttgtatattt tgtttttgat ataatttttt tttaataaga gttttttgat gtgttgttaa  2940
   tatttttaag agatagttgt ttatattaag atttaagaat tttttgtatt attgattaga   3000
   t                                                                   3001
<210> 116
   <211> 50
   <212> DNA
   <213> human
<400> 116
   gaaactaaaa ctggacatgg ccctacacag gtaagcacat atcagcatcg 50
<210> 117
   <211> 50
   <212> DNA
   <213> human
<400> 117
   gaaattaaaa ttggatatgg ttttatatag gtaagtatat attagtatcg 50
<210> 118
   <211> 50
   <212> DNA
   <213> human
<400> 118
   gaaattaaaa ttggatatgg ttttatatag gtaagtatat attagtattg 50
<210> 119
   <211> 50
   <212> DNA
   <213> human
<400> 119
   cgatgttgat atgtgtttat ttgtgtaggg ttatgtttag ttttagtttt 50
<210> 120
   <211> 50
   <212> DNA
   <213> human
<400> 120
   tgatgttgat atgtgtttat ttgtgtaggg ttatgtttag ttttagtttt 50
<210> 121
   <211> 3001
   <212> DNA
   <213> human
<400> 121
   attatctgaa aactaatctt ccttttccta gctagtctct tgtctcttgt tattgggttt 60
   gtgttcacat tatctatttt gaagattttc aaatcttttt gttttctccc catataatca 120
   tggtaatgga acataaaatg cctaagacat gaatgaaaat tcacatgtcc ttgtttgctt 180
   ccttgctact ttatcacaaa tatttccagg acagcacatg tttttaataa cttgtacgtt 240
   cttataatta taatatagat cctttgtaaa catatgaact gcaaaaatag aagactgtaa 300
   aagtcaccca tattttacca ttcagatata ttctggttaa tgtttgctgt atactatttc 360
   taacttttta agtcataggt aaactttctt aaaaattaaa atgtgtcaga ttacacaaac 420
   tattttgtaa accgcttttt cattttcagc ctctagaaga gtgcctggca catcctaagt 480
   tttcaataaa tatttgtcaa ataaatgact gaacttattt ccataccatt aaatgtttat 540
   ctgtggtgtc atttttaacc attgcacaga attctattgt gtgaaatatg ataatttatc 600
   taacaaggcc ctgaagagct gcagaggaat aaggccttgt gatggacaca gaggtgctcc 660
   atgcagtccc ccttgaagtg ggaggttgcc ctgctgctgg gagtacttgc aacctccagc 720
   ctccagttat cagccccttt caggacttgc ctcagctaca gaaagctgcc ttcccagggt 780
   aatccacatt gaagggcaga tcatagagga gcccagtgtc agagctactc tgacaggcca 840
   tcttcctcca gagtttcctg aggagggctg cttaagactg tcccatctat attgcagctc 900
   aacttcttcc tctgcccagt cctgtttctc ccctctccct gccatgggtg ttaatctcag 960
   ggcactgtct aataaatata ttgcacacta aattttgtct caaagtctgc tcctgaacaa 1020
   cctaacctgc aacagagatg atgctgacct ttgaggaact cccaagtcta ttggtaaaag 1080
   cactcagtaa attctaacat tactgcagtt accgattcct aactcaggtt aaacaagcga 1140
   tggcgcatgt ataccatgat aggttatgca gccattaaaa taatgttctg ggtgccatgt 1200
   ataacacatg atggctccag aagtcaggga agaaggaaaa aagaagggaa gggaagagaa 1260
   gagaaaggga gggaagaaga ggggagggaa gaattggaca tcagataatc acttgagccc 1320
   tctctctccg tgaatttttc ccaatctgtt tctttccaca tctcttgctc cttatcagtc 1380
   tttgggccac ctacagtatg agcattactt gggcaattac tgtctctctg aatttggcat 1440
   atgcaaatta tagaaactaa aactggacat ggccctacac aggtaagcac atatcagcat 1500
   cgctgtgagc ggtcaggact aacaagcctt caacacacaa aagcaggagt aaggatcaaa 1560
   ttacacaaat caaatcaaat caaatcaaaa taaaatccct gagtttgtat tccagtaaag 1620
   cccaaaccta ggagggatcc aggaaagcga tgagtgtggc tgtcaaggtg cttccatctg 1680
   tcagagtcac acattccaaa gcagcagaaa tcaactgaca gcctccggtt actcccccgc 1740
   ctcagcgagg cagggcaggc agcggaaagc tcagcccatg tgaggtgcct cctgccaatc 1800
   acagactacc cttccctggt cctggaggtt caaagaattg caggagggta gaaaagcacc 1860
   tgggtcgggt gcagactgcg gagcgggccc taccgtgtgc gcagaaagag gaggcgctgt 1920
   gagtagaccg ctttccctct gcagggaatc tcccgggctc aagggctggg ttgctctcct 1980
   gggagggaag ctagcagact gggagggaaa ggggaagaag ttagattctt gattcataaa 2040
   gtctgtcatt tttttctttg taatttaggg tattgtgact gtcctgttct cttttacttt 2100
   gaaccgttgg aattgctttc ttaataccct tgatataagt cttgttctca ttaaacttct 2160
   ggagatatta cgcagccatc ctgctttcaa cctcagatat ccccacagaa agaacaagaa 2220
   aatccaagca ccccgagaaa ccctatatct cctgattaaa tcgcacatag acagtcacct 2280
   ttgtagaccc ccaatttcct tgtggagtca gaaaagcaat agaaagttta acgaaatagc 2340
   tcgtgttatt tcttaataat ctgttcaggc tagcagagag aaagtttctt ggtcttcttg 2400
   tcagaaatca tttggtcctg ccaacctgta atgaaatgtg tgaagcaatt tactcccagt 2460
   tattctctgc aaacacttct gcttaataaa tgtgtcagtt gaagtaagtc aacaaagtag 2520
   ctttatctac gcaatgttat ttttacataa tttgttttta ctccttacca attctttgct 2580
   ccaccatgat ctgcaggaca taaagatatt tttcattctg atcacatttt cacacaaaat 2640
   acaaatttaa gtttcaaata atgctagtat tattttatgt ttgcatttgt ttataaacct 2700
   aaaactccac ttctaactag ttttaaagat ttttaagcca tttaagaatt cgtagccagt 2760
   cacagtggca catgcctgca gttccagcta ttcgggaagc tgaggccacg tgatcaggag 2820
   ttctagtcca ggctgggcaa catagtgaga tcctgtctct aaaaaaaata aaaataaatt 2880
   tttaaagaag ttttgttaac ttgaatgtct tcttttaatg aaagacaacg tttcttgaac 2940
   atttcttgcc tgagttgcta agcacaacag gaaagaactc attaggtttt atagatatac 3000
   a 3001
<210> 122
   <211> 3001
   <212> DNA
   <213> human
<400> 122
   attatttgaa aattaatttt ttttttttta gttagttttt tgttttttgt tattgggttt      60
   gtgtttatat tatttatttt gaagattttt aaattttttt gttttttttt tatataatta     120
   tggtaatgga atataaaatg tttaagatat gaatgaaaat ttatatgttt ttgtttgttt     180
   ttttgttatt ttattataaa tatttttagg atagtatatg tttttaataa tttgtacgtt     240
   tttataatta taatatagat tttttgtaaa tatatgaatt gtaaaaatag aagattgtaa     300
   aagttattta tattttatta tttagatata ttttggttaa tgtttgttgt atattatttt     360
   taatttttta agttataggt aaattttttt aaaaattaaa atgtgttaga ttatataaat     420
   tattttgtaa atcgtttttt tatttttagt ttttagaaga gtgtttggta tattttaagt     480
   ttttaataaa tatttgttaa ataaatgatt gaatttattt ttatattatt aaatgtttat     540
   ttgtggtgtt atttttaatt attgtataga attttattgt gtgaaatatg ataatttatt     600
   taataaggtt ttgaagagtt gtagaggaat aaggttttgt gatggatata gaggtgtttt     660
   atgtagtttt ttttgaagtg ggaggttgtt ttgttgttgg gagtatttgt aatttttagt     720
   ttttagttat tagttttttt taggatttgt tttagttata gaaagttgtt tttttagggt     780
   aatttatatt gaagggtaga ttatagagga gtttagtgtt agagttattt tgataggtta     840
   ttttttttta gagttttttg aggagggttg tttaagattg ttttatttat attgtagttt     900
   aatttttttt tttgtttagt tttgtttttt tttttttttt gttatgggtg ttaattttag     960
   ggtattgttt aataaatata ttgtatatta aattttgttt taaagtttgt ttttgaataa    1020
   tttaatttgt aatagagatg atgttgattt ttgaggaatt tttaagttta ttggtaaaag    1080
   tatttagtaa attttaatat tattgtagtt atcgattttt aatttaggtt aaataagcga    1140
   tggcgtatgt atattatgat aggttatgta gttattaaaa taatgttttg ggtgttatgt    1200
   ataatatatg atggttttag aagttaggga agaaggaaaa aagaagggaa gggaagagaa    1260
   gagaaaggga gggaagaaga ggggagggaa gaattggata ttagataatt atttgagttt    1320
   ttttttttcg tgaatttttt ttaatttgtt tttttttata tttttgtttt tttattagtt    1380
   tttgggttat ttatagtatg agtattattt gggtaattat tgtttttttg aatttggtat     1440
   atgtaaatta tagaaattaa aattggatat ggttttatat aggtaagtat atattagtat    1500
   cgttgtgagc ggttaggatt aataagtttt taatatataa aagtaggagt aaggattaaa    1560
   ttatataaat taaattaaat taaattaaaa taaaattttt gagtttgtat tttagtaaag    1620
   tttaaattta ggagggattt aggaaagcga tgagtgtggt tgttaaggtg tttttatttg    1680
   ttagagttat atattttaaa gtagtagaaa ttaattgata gttttcggtt attttttcgt    1740
   tttagcgagg tagggtaggt agcggaaagt ttagtttatg tgaggtgttt tttgttaatt    1800
   atagattatt tttttttggt tttggaggtt taaagaattg taggagggta gaaaagtatt    1860
   tgggtcgggt gtagattgcg gagcgggttt tatcgtgtgc gtagaaagag gaggcgttgt    1920
   gagtagatcg tttttttttt gtagggaatt tttcgggttt aagggttggg ttgttttttt     1980
   gggagggaag ttagtagatt gggagggaaa ggggaagaag ttagattttt gatttataaa    2040
   gtttgttatt tttttttttg taatttaggg tattgtgatt gttttgtttt tttttatttt    2100
   gaatcgttgg aattgttttt ttaatatttt tgatataagt tttgttttta ttaaattttt    2160
   ggagatatta cgtagttatt ttgtttttaa ttttagatat ttttatagaa agaataagaa    2220
   aatttaagta tttcgagaaa ttttatattt tttgattaaa tcgtatatag atagttattt    2280
   ttgtagattt ttaatttttt tgtggagtta gaaaagtaat agaaagttta acgaaatagt    2340
   tcgtgttatt ttttaataat ttgtttaggt tagtagagag aaagtttttt ggtttttttg     2400
   ttagaaatta tttggttttg ttaatttgta atgaaatgtg tgaagtaatt tatttttagt    2460
   tattttttgt aaatattttt gtttaataaa tgtgttagtt gaagtaagtt aataaagtag     2520
   ttttatttac gtaatgttat ttttatataa tttgttttta ttttttatta attttttgtt    2580
   ttattatgat ttgtaggata taaagatatt ttttattttg attatatttt tatataaaat    2640
   ataaatttaa gttttaaata atgttagtat tattttatgt ttgtatttgt ttataaattt    2700
   aaaattttat ttttaattag ttttaaagat ttttaagtta tttaagaatt cgtagttagt    2760
   tatagtggta tatgtttgta gttttagtta ttcgggaagt tgaggttacg tgattaggag    2820
   ttttagttta ggttgggtaa tatagtgaga ttttgttttt aaaaaaaata aaaataaatt    2880
   tttaaagaag ttttgttaat ttgaatgttt ttttttaatg aaagataacg ttttttgaat   2940
   attttttgtt tgagttgtta agtataatag gaaagaattt attaggtttt atagatatat    3000
   a                                                                    3001
<210> 123
   <211> 3001
   <212> DNA
   <213> human
<400> 123
   attatttgaa aattaatttt ttttttttta gttagttttt tgttttttgt tattgggttt       60
   gtgtttatat tatttatttt gaagattttt aaattttttt gttttttttt tatataatta      120
   tggtaatgga atataaaatg tttaagatat gaatgaaaat ttatatgttt ttgtttgttt      180
   ttttgttatt ttattataaa tatttttagg atagtatatg tttttaataa tttgtatgtt      240
   tttataatta taatatagat tttttgtaaa tatatgaatt gtaaaaatag aagattgtaa      300
   aagttattta tattttatta tttagatata ttttggttaa tgtttgttgt atattatttt      360
   taatttttta agttataggt aaattttttt aaaaattaaa atgtgttaga ttatataaat      420
   tattttgtaa attgtttttt tatttttagt ttttagaaga gtgtttggta tattttaagt      480
   ttttaataaa tatttgttaa ataaatgatt gaatttattt ttatattatt aaatgtttat      540
   ttgtggtgtt atttttaatt attgtataga attttattgt gtgaaatatg ataatttatt      600
   taataaggtt ttgaagagtt gtagaggaat aaggttttgt gatggatata gaggtgtttt      660
   atgtagtttt ttttgaagtg ggaggttgtt ttgttgttgg gagtatttgt aatttttagt      720
   ttttagttat tagttttttt taggatttgt tttagttata gaaagttgtt tttttagggt      780
   aatttatatt gaagggtaga ttatagagga gtttagtgtt agagttattt tgataggtta      840
   ttttttttta gagttttttg aggagggttg tttaagattg ttttatttat attgtagttt      900
   aatttttttt tttgtttagt tttgtttttt tttttttttt gttatgggtg ttaattttag      960
   ggtattgttt aataaatata ttgtatatta aattttgttt taaagtttgt ttttgaataa     1020
   tttaatttgt aatagagatg atgttgattt ttgaggaatt tttaagttta ttggtaaaag     1080
   tatttagtaa attttaatat tattgtagtt attgattttt aatttaggtt aaataagtga     1140
   tggtgtatgt atattatgat aggttatgta gttattaaaa taatgttttg ggtgttatgt     1200
   ataatatatg atggttttag aagttaggga agaaggaaaa aagaagggaa gggaagagaa     1260
   gagaaaggga gggaagaaga ggggagggaa gaattggata ttagataatt atttgagttt     1320
   tttttttttg tgaatttttt ttaatttgtt tttttttata ttttttgttt tttattagtt     1380
   tttgggttat ttatagtatg agtattattt gggtaattat tgtttttttg aatttggtat     1440
   atgtaaatta tagaaattaa aattggatat ggttttatat aggtaagtat atattagtat     1500
   tgttgtgagt ggttaggatt aataagtttt taatatataa aagtaggagt aaggattaaa     1560
   ttatataaat taaattaaat taaattaaaa taaaattttt gagtttgtat tttagtaaag     1620
   tttaaattta ggagggattt aggaaagtga tgagtgtggt tgttaaggtg tttttatttg     1680
   ttagagttat atattttaaa gtagtagaaa ttaattgata gtttttggtt atttttttgt     1740
   tttagtgagg tagggtaggt agtggaaagt ttagtttatg tgaggtgttt tttgttaatt     1800
   atagattatt tttttttggt tttggaggtt taaagaattg taggagggta gaaaagtatt     1860
   tgggttgggt gtagattgtg gagtgggttt tattgtgtgt gtagaaagag gaggtgttgt     1920
   gagtagattg tttttttttt gtagggaatt ttttgggttt aagggttggg ttgttttttt     1980
   gggagggaag ttagtagatt gggagggaaa ggggaagaag ttagattttt gatttataaa     2040
   gtttgttatt tttttttttg taatttaggg tattgtgatt gttttgtttt tttttatttt     2100
   gaattgttgg aattgttttt ttaatatttt tgatataagt tttgttttta ttaaattttt     2160
   ggagatatta tgtagttatt ttgtttttaa ttttagatat ttttatagaa agaataagaa     2220
   aatttaagta ttttgagaaa ttttatattt tttgattaaa ttgtatatag atagttattt     2280
   ttgtagattt ttaatttttt tgtggagtta gaaaagtaat agaaagttta atgaaatagt     2340
   ttgtgttatt ttttaataat ttgtttaggt tagtagagag aaagtttttt ggtttttttg     2400
   ttagaaatta tttggttttg ttaatttgta atgaaatgtg tgaagtaatt tatttttagt     2460
   tattttttgt aaatattttt gtttaataaa tgtgttagtt gaagtaagtt aataaagtag     2520
   ttttatttat gtaatgttat ttttatataa tttgttttta ttttttatta attttttgtt     2580
   ttattatgat ttgtaggata taaagatatt ttttattttg attatatttt tatataaaat     2640
   ataaatttaa gttttaaata atgttagtat tattttatgt ttgtatttgt ttataaattt     2700
   aaaattttat ttttaattag ttttaaagat ttttaagtta tttaagaatt tgtagttagt     2760
   tatagtggta tatgtttgta gttttagtta tttgggaagt tgaggttatg tgattaggag     2820
   ttttagttta ggttgggtaa tatagtgaga ttttgttttt aaaaaaaata aaaataaatt     2880
   tttaaagaag ttttgttaat ttgaatgttt ttttttaatg aaagataatg ttttttgaat     2940
   attttttgtt tgagttgtta agtataatag gaaagaattt attaggtttt atagatatat     3000
   a                                                                     3001
<210> 124
   <211> 3001
   <212> DNA
   <213> human
<400> 124
<210> 125
   <211> 3001
   <212> DNA
   <213> human
<400> 125
   tgtatattta taaaatttaa tgagtttttt tttgttgtgt ttagtaattt aggtaagaaa     60
   tgtttaagaa atgttgtttt ttattaaaag aagatattta agttaataaa atttttttaa    120
   aaatttattt ttattttttt tagagatagg attttattat gttgtttagt ttggattaga    180
   atttttgatt atgtggtttt agttttttga atagttggaa ttgtaggtat gtgttattgt    240
   gattggttat gaatttttaa atggtttaaa aatttttaaa attagttaga agtggagttt    300
   taggtttata aataaatgta aatataaaat aatattagta ttatttgaaa tttaaatttg    360
   tattttgtgt gaaaatgtga ttagaatgaa aaatattttt atgttttgta gattatggtg    420
   gagtaaagaa ttggtaagga gtaaaaataa attatgtaaa aataatattg tgtagataaa    480
   gttattttgt tgatttattt taattgatat atttattaag tagaagtgtt tgtagagaat    540
   aattgggagt aaattgtttt atatatttta ttataggttg gtaggattaa atgatttttg    600
   ataagaagat taagaaattt tttttttgtt agtttgaata gattattaag aaataatatg    660
   agttattttg ttaaattttt tattgttttt ttgattttat aaggaaattg ggggtttata    720
   aaggtgattg tttatgtgtg atttaattag gagatatagg gttttttggg gtgtttggat    780
   ttttttgttt tttttgtggg gatatttgag gttgaaagta ggatggttgt gtaatatttt    840
   tagaagttta atgagaataa gatttatatt aagggtatta agaaagtaat tttaatggtt    900
   taaagtaaaa gagaatagga tagttataat attttaaatt ataaagaaaa aaatgataga    960
   ttttatgaat taagaattta attttttttt tttttttttt tagtttgtta gttttttttt   1020
   taggagagta atttagtttt tgagtttggg agattttttg tagagggaaa gtggtttatt   1080
   tatagtgttt tttttttttg tgtatatggt agggtttgtt ttgtagtttg tatttgattt   1140
   aggtgttttt ttattttttt gtaatttttt gaatttttag gattagggaa gggtagtttg   1200
   tgattggtag gaggtatttt atatgggttg agttttttgt tgtttgtttt gttttgttga   1260
   ggtgggggag taattggagg ttgttagttg atttttgttg ttttggaatg tgtgattttg   1320
   atagatggaa gtattttgat agttatattt attgtttttt tggatttttt ttaggtttgg   1380
   gttttattgg aatataaatt tagggatttt attttgattt gatttgattt gatttgtgta   1440
   atttgatttt tatttttgtt tttgtgtgtt gaaggtttgt tagttttgat tgtttatagt   1500
   gatgttgata tgtgtttatt tgtgtagggt tatgtttagt tttagttttt ataatttgta   1560
   tatgttaaat ttagagagat agtaattgtt taagtaatgt ttatattgta ggtggtttaa   1620
   agattgataa ggagtaagag atgtggaaag aaatagattg ggaaaaattt atggagagag   1680
   agggtttaag tgattatttg atgtttaatt tttttttttt tttttttttt tttttttttt   1740
   tttttttttt tttttttttt tttttttttt ttttttgatt tttggagtta ttatgtgtta   1800
   tatatggtat ttagaatatt attttaatgg ttgtataatt tattatggta tatatgtgtt   1860
   attgtttgtt taatttgagt taggaattgg taattgtagt aatgttagaa tttattgagt   1920
   gtttttatta atagatttgg gagtttttta aaggttagta ttatttttgt tgtaggttag   1980
   gttgtttagg agtagatttt gagataaaat ttagtgtgta atatatttat tagatagtgt   2040
   tttgagatta atatttatgg tagggagagg ggagaaatag gattgggtag aggaagaagt   2100
   tgagttgtaa tatagatggg atagttttaa gtagtttttt ttaggaaatt ttggaggaag   2160
   atggtttgtt agagtagttt tgatattggg tttttttatg atttgttttt taatgtggat   2220
   tattttggga aggtagtttt ttgtagttga ggtaagtttt gaaaggggtt gataattgga   2280
   ggttggaggt tgtaagtatt tttagtagta gggtaatttt ttattttaag ggggattgta   2340
   tggagtattt ttgtgtttat tataaggttt tatttttttg tagtttttta gggttttgtt   2400
   agataaatta ttatatttta tataatagaa ttttgtgtaa tggttaaaaa tgatattata   2460
   gataaatatt taatggtatg gaaataagtt tagttattta tttgataaat atttattgaa   2520
   aatttaggat gtgttaggta tttttttaga ggttgaaaat gaaaaagtgg tttataaaat   2580
   agtttgtgta atttgatata ttttaatttt taagaaagtt tatttatgat ttaaaaagtt   2640
   agaaatagta tatagtaaat attaattaga atatatttga atggtaaaat atgggtgatt   2700
   tttatagttt tttatttttg tagtttatat gtttataaag gatttatatt ataattataa   2760
   gaatgtataa gttattaaaa atatgtgttg ttttggaaat atttgtgata aagtagtaag   2820
   gaagtaaata aggatatgtg aatttttatt tatgttttag gtattttatg ttttattatt   2880
   atgattatat ggggagaaaa taaaaagatt tgaaaatttt taaaatagat aatgtgaata   2940
   taaatttaat aataagagat aagagattag ttaggaaaag gaagattagt ttttagataa   3000
   t                                                                   3001
<210> 126
   <211> 50
   <212> DNA
   <213> human
<400> 126
   cgcccagcca ggaggcaaag ctgtttcaat ggtgtagtgg acaagttacc 50
<210> 127
   <211> 50
   <212> DNA
   <213> human
<400> 127
   cgtttagtta ggaggtaaag ttgttttaat ggtgtagtgg ataagttatt 50
<210> 128
   <211> 50
   <212> DNA
   <213> human
<400> 128
   tgtttagtta ggaggtaaag ttgttttaat ggtgtagtgg ataagttatt 50
<210> 129
   <211> 50
   <212> DNA
   <213> human
<400> 129
   ggtaatttgt ttattatatt attgaaatag ttttgttttt tggttgggcg 50
<210> 130
   <211> 50
   <212> DNA
   <213> human
<400> 130
   ggtaatttgt ttattatatt attgaaatag ttttgttttt tggttgggtg 50
<210> 131
   <211> 3001
   <212> DNA
   <213> human
<400> 131
   cagaggggac gtggtgccgg cagggagggg aactgcccaa ggagacggga gtagatgtgg 60
   cgctggcccg gagcccgccc aacaggagta aaacgcttcg gacccacaca gacccgctcc 120
   agcgccaccg gctccggccc gggcctgagc ccgagtaacc cactcctccg ccccattcgg 180
   ccgccatctt cgggccgagc gtcgttacca tggagtgcgt gacgcctctg cgcccgcgcc 240
   ggccccgccc ctcgccgctg ggggtagacg gagggcccag ccggcggaaa ggcgctttcc 300
   gattggacag cgtcacccgc tagaagttcg ccgcagaaga tgagggcggc gatcacgtcc 360
   atcgcgtctc gtggcgcgaa gccggccaat aaatgctgtt tggaggccca cgtgagtggg 420
   gctgtgggag gaagaaaggc tagcgggcgt tggccgtatg tgggtgtctt gaggcagttt 480
   ttcagttctt tcatttacca aagtgacatg cacctactag gtgccaggtg tttagacgta 540
   catacaaccc tctgcaaaat ctttcagtgt agtcctctgt atgaaaaggt gagtggaata 600
   ctttcattta cttatcctga gtcaccaact ttaggcccat tacttaacta acgccatttc 660
   ctataaaaat ctatgataca ccgtttactg ttttactcaa ctccatcttt tagtttccag 720
   ccaagaattg ccactgcacc tgagataagg gggatcctgg ccattaagga aaccttgcct 780
   tcgaaactga gccgtgagga actatacaaa atgggaaatt gggacaaatc ccagtggctc     840
   atgacactaa gaagtaaaat tacgaactca ctgagctgga agtcattcaa cgggaattga     900
   ataggtaagc tactatactg ggaaatgaag gagtaggaaa gaaggaaaac ctcaagttac     960
   aaactatggg tggagattca gctacgagga tggggtggtg ttttaagtat taccatttta    1020
   caagtaaata ctcactaaat gtgtgtatca tcccacaact agtggaaggt gggattccaa    1080
   accacatttg taacttagga aacgtgccct ttccactaca ataccacaaa taaattgcta    1140
   ttgtgaataa agaatggaac cagtcatcct aagctcctgg ctgtaacaat agtgttatgc    1200
   tgaggaaagg ctgtttcttt tttttttgag acggagtttc actcgtcgcc caggctggag    1260
   tgcagtgaca caatctcggc tcaatgcaac ctctgcctcc cgggatcaag ctattcccct    1320
   gcctcagcct cccgagtagc cgggattaca ggcacccgcc accacgcccg gccaattttt    1380
   atgtttttag tagagacgta gtttcaccac gttggccagg ctggtctcaa cctcatgacc    1440
   tcaggtgatc cgcccgcctc cgcctcccaa agtgttggga ttacaggcgt gagccaccgc    1500
   gcccagccag gaggcaaagc tgtttcaatg gtgtagtgga caagttacca gccacgcaag    1560
   ccctttcctg taaaccacag gactcctcct ttggcaacgg tgacttgagt acttttgact    1620
   taatttccat atgggaggag aaaaacataa aaacatacat ttattattta caaatgctgc    1680
   atcatctcag taaggaagag catgtatttg cttttgagtc tcatttgcca ttgcacatgt    1740
   aagttactcg agttaaagtc tcaaccgttt gttttccccc gctttgctca gatgcctacc    1800
   acttaccgtg tgtctgttcc actcagaata ccatccactt ctccatatgc ctaacccaga    1860
   cctgaaatac ccatctgtct tcatggatat gtccacaggt taacatctta cagcttcctc    1920
   aaattcaact ttagctcctc ccacaaaacc atctcctgat tccccaactt tgtaaatgca    1980
   ccattatcca tccagccccc aaagccagaa acatgttaga attctccatt tgagactgaa    2040
   aagcccacct aattttccct tgtttatgct gggatgccat tgggatttga aataatctga    2100
   gctggtggct catatctgta atcccagtac tttaggagaa caaggcaggg ggatcacttg    2160
   aggccaggaa tcgagaccag cctgggaccc cgaaaaaaca ttttaattgg ctggacgtgc    2220
   ctggcatgca ccagattctc tggaggttgt gggcagggga ttgtctgagc ctagaagttc    2280
   aaaggtgcag tgagctatga tcacaccact gcactacagg ctgggcaaca gaaaccctgt    2340
   ctcaaatgag taaattttaa aatttcctgt gaatcaagtt cttcagtgac gttgtattac    2400
   actcaagaac aatcttaagc ctaagatcct cagttcagta caggagacct ctcatgtggc    2460
   attttagttt tctggacttt ccagtcagcc acttgtgttc ctaaaatctt cctgccattt    2520
   cctctttgag atgccgccct atttcttcct ctacacttta cggttttatg ttacgcagcc    2580
   agtataccta catgcctcta tccctgaata ccccgagtac tactgatcac atctacttaa    2640
   ctggcacaat gaacacagta ggtactttta gcttatgttg gccttttgga gtgggtgtcc    2700
   aagatcagca cttcctcgcg ctgactgcgt aattcccaag ttcaatttca ttagcattct    2760
   aatatacaac ttccaacttc cggccttgaa gtactgctca cttctggtga atacagcatc    2820
   agtgcacttt gtttgcctta aaaccacaat cctgggaggc tgagacagaa tggcgtgaac    2880
   ccgggaggcg gagcgtgcag tgagccaaga ttgcgccact gcactccagc ctagacgaca    2940
   gagcaagact gtctcaaaaa aaaaaaaaaa aaaaccacca caatcctaat aaatgagcct    3000
   g                                                                    3001
<210> 132
   <211> 3001
   <212> DNA
   <213> human
<400> 132
<210> 133
   <211> 3001
   <212> DNA
   <213> human
<400> 133
   tagaggggat gtggtgttgg tagggagggg aattgtttaa ggagatggga gtagatgtgg 60
   tgttggtttg gagtttgttt aataggagta aaatgttttg gatttatata gatttgtttt 120
   agtgttattg gttttggttt gggtttgagt ttgagtaatt tatttttttg ttttatttgg 180
   ttgttatttt tgggttgagt gttgttatta tggagtgtgt gatgtttttg tgtttgtgtt 240
   ggttttgttt tttgttgttg ggggtagatg gagggtttag ttggtggaaa ggtgtttttt 300
   gattggatag tgttatttgt tagaagtttg ttgtagaaga tgagggtggt gattatgttt 360
   attgtgtttt gtggtgtgaa gttggttaat aaatgttgtt tggaggttta tgtgagtggg 420
   gttgtgggag gaagaaaggt tagtgggtgt tggttgtatg tgggtgtttt gaggtagttt 480
   tttagttttt ttatttatta aagtgatatg tatttattag gtgttaggtg tttagatgta 540
   tatataattt tttgtaaaat tttttagtgt agttttttgt atgaaaaggt gagtggaata 600
   tttttattta tttattttga gttattaatt ttaggtttat tatttaatta atgttatttt 660
   ttataaaaat ttatgatata ttgtttattg ttttatttaa ttttattttt tagtttttag 720
   ttaagaattg ttattgtatt tgagataagg gggattttgg ttattaagga aattttgttt 780
   ttgaaattga gttgtgagga attatataaa atgggaaatt gggataaatt ttagtggttt 840
   atgatattaa gaagtaaaat tatgaattta ttgagttgga agttatttaa tgggaattga 900
   ataggtaagt tattatattg ggaaatgaag gagtaggaaa gaaggaaaat tttaagttat 960
   aaattatggg tggagattta gttatgagga tggggtggtg ttttaagtat tattatttta 1020
   taagtaaata tttattaaat gtgtgtatta ttttataatt agtggaaggt gggattttaa 1080
   attatatttg taatttagga aatgtgtttt ttttattata atattataaa taaattgtta 1140
   ttgtgaataa agaatggaat tagttatttt aagtttttgg ttgtaataat agtgttatgt 1200
   tgaggaaagg ttgttttttt tttttttgag atggagtttt atttgttgtt taggttggag 1260
   tgtagtgata taattttggt ttaatgtaat ttttgttttt tgggattaag ttattttttt 1320
   gttttagttt tttgagtagt tgggattata ggtatttgtt attatgtttg gttaattttt 1380
   atgtttttag tagagatgta gttttattat gttggttagg ttggttttaa ttttatgatt 1440
   ttaggtgatt tgtttgtttt tgttttttaa agtgttggga ttataggtgt gagttattgt 1500
   gtttagttag gaggtaaagt tgttttaatg gtgtagtgga taagttatta gttatgtaag 1560
   tttttttttg taaattatag gatttttttt ttggtaatgg tgatttgagt atttttgatt 1620
   taatttttat atgggaggag aaaaatataa aaatatatat ttattattta taaatgttgt 1680
   attattttag taaggaagag tatgtatttg tttttgagtt ttatttgtta ttgtatatgt 1740
   aagttatttg agttaaagtt ttaattgttt gttttttttt gttttgttta gatgtttatt 1800
   atttattgtg tgtttgtttt atttagaata ttatttattt ttttatatgt ttaatttaga 1860
   tttgaaatat ttatttgttt ttatggatat gtttataggt taatatttta tagttttttt 1920
   aaatttaatt ttagtttttt ttataaaatt attttttgat tttttaattt tgtaaatgta 1980
   ttattattta tttagttttt aaagttagaa atatgttaga attttttatt tgagattgaa 2040
   aagtttattt aatttttttt tgtttatgtt gggatgttat tgggatttga aataatttga 2100
   gttggtggtt tatatttgta attttagtat tttaggagaa taaggtaggg ggattatttg 2160
   aggttaggaa ttgagattag tttgggattt tgaaaaaata ttttaattgg ttggatgtgt 2220
   ttggtatgta ttagattttt tggaggttgt gggtagggga ttgtttgagt ttagaagttt 2280
   aaaggtgtag tgagttatga ttatattatt gtattatagg ttgggtaata gaaattttgt 2340
   tttaaatgag taaattttaa aattttttgt gaattaagtt ttttagtgat gttgtattat 2400
   atttaagaat aattttaagt ttaagatttt tagtttagta taggagattt tttatgtggt 2460
   attttagttt tttggatttt ttagttagtt atttgtgttt ttaaaatttt tttgttattt 2520
   tttttttgag atgttgtttt attttttttt ttatatttta tggttttatg ttatgtagtt 2580
   agtatattta tatgttttta tttttgaata ttttgagtat tattgattat atttatttaa 2640
   ttggtataat gaatatagta ggtattttta gtttatgttg gttttttgga gtgggtgttt 2700
   aagattagta tttttttgtg ttgattgtgt aatttttaag tttaatttta ttagtatttt 2760
   aatatataat ttttaatttt tggttttgaa gtattgttta tttttggtga atatagtatt 2820
   agtgtatttt gtttgtttta aaattataat tttgggaggt tgagatagaa tggtgtgaat 2880
   ttgggaggtg gagtgtgtag tgagttaaga ttgtgttatt gtattttagt ttagatgata 2940
   gagtaagatt gttttaaaaa aaaaaaaaaa aaaattatta taattttaat aaatgagttt 3000
   g 3001

<210> 134
   <211> 3001
   <212> DNA
   <213> human
<400> 134
   taggtttatt tattaggatt gtggtggttt tttttttttt ttttttgaga tagttttgtt 60
   ttgtcgttta ggttggagtg tagtggcgta attttggttt attgtacgtt tcgtttttcg 120
   ggtttacgtt attttgtttt agttttttag gattgtggtt ttaaggtaaa taaagtgtat 180
   tgatgttgta tttattagaa gtgagtagta ttttaaggtc ggaagttgga agttgtatat 240
   tagaatgtta atgaaattga atttgggaat tacgtagtta gcgcgaggaa gtgttgattt 300
   tggatattta ttttaaaagg ttaatataag ttaaaagtat ttattgtgtt tattgtgtta 360
   gttaagtaga tgtgattagt agtattcggg gtatttaggg atagaggtat gtaggtatat 420
   tggttgcgta atataaaatc gtaaagtgta gaggaagaaa tagggcggta ttttaaagag 480
   gaaatggtag gaagatttta ggaatataag tggttgattg gaaagtttag aaaattaaaa 540
   tgttatatga gaggtttttt gtattgaatt gaggatttta ggtttaagat tgtttttgag 600
   tgtaatataa cgttattgaa gaatttgatt tataggaaat tttaaaattt atttatttga 660
   gatagggttt ttgttgttta gtttgtagtg tagtggtgtg attatagttt attgtatttt 720
   tgaattttta ggtttagata attttttgtt tataattttt agagaatttg gtgtatgtta 780
   ggtacgttta gttaattaaa atgttttttc ggggttttag gttggtttcg atttttggtt 840
   ttaagtgatt tttttgtttt gtttttttaa agtattggga ttatagatat gagttattag 900
   tttagattat tttaaatttt aatggtattt tagtataaat aagggaaaat taggtgggtt 960
   ttttagtttt aaatggagaa ttttaatatg tttttggttt tgggggttgg atggataatg 1020
   gtgtatttat aaagttgggg aattaggaga tggttttgtg ggaggagtta aagttgaatt 1080
   tgaggaagtt gtaagatgtt aatttgtgga tatatttatg aagatagatg ggtattttag 1140
   gtttgggtta ggtatatgga gaagtggatg gtattttgag tggaatagat atacggtaag 1200
   tggtaggtat ttgagtaaag cgggggaaaa taaacggttg agattttaat tcgagtaatt 1260
   tatatgtgta atggtaaatg agatttaaaa gtaaatatat gttttttttt attgagatga 1320
   tgtagtattt gtaaataata aatgtatgtt tttatgtttt ttttttttta tatggaaatt 1380
   aagttaaaag tatttaagtt atcgttgtta aaggaggagt tttgtggttt ataggaaagg 1440
   gtttgcgtgg ttggtaattt gtttattata ttattgaaat agttttgttt tttggttggg 1500
   cgcggtggtt tacgtttgta attttaatat tttgggaggc ggaggcgggc ggattatttg 1560
   aggttatgag gttgagatta gtttggttaa cgtggtgaaa ttacgttttt attaaaaata 1620
   taaaaattgg tcgggcgtgg tggcgggtgt ttgtaatttc ggttattcgg gaggttgagg 1680
   taggggaata gtttgatttc gggaggtaga ggttgtattg agtcgagatt gtgttattgt 1740
   attttagttt gggcgacgag tgaaatttcg ttttaaaaaa aaaagaaata gttttttttt 1800
   agtataatat tattgttata gttaggagtt taggatgatt ggttttattt tttatttata 1860
   atagtaattt atttgtggta ttgtagtgga aagggtacgt tttttaagtt ataaatgtgg 1920
   tttggaattt tattttttat tagttgtggg atgatatata tatttagtga gtatttattt 1980
   gtaaaatggt aatatttaaa atattatttt attttcgtag ttgaattttt atttatagtt 2040
   tgtaatttga ggtttttttt tttttttatt tttttatttt ttagtatagt agtttattta 2100
   tttaattttc gttgaatgat ttttagttta gtgagttcgt aattttattt tttagtgtta 2160
   tgagttattg ggatttgttt taatttttta ttttgtatag ttttttacgg tttagtttcg 2220
   aaggtaaggt ttttttaatg gttaggattt tttttatttt aggtgtagtg gtaatttttg 2280
   gttggaaatt aaaagatgga gttgagtaaa atagtaaacg gtgtattata gatttttata 2340
   ggaaatggcg ttagttaagt aatgggttta aagttggtga tttaggataa gtaaatgaaa 2400
   gtattttatt tattttttta tatagaggat tatattgaaa gattttgtag agggttgtat 2460
   gtacgtttaa atatttggta tttagtaggt gtatgttatt ttggtaaatg aaagaattga 2520
   aaaattgttt taagatattt atatacggtt aacgttcgtt agtttttttt ttttttatag 2580
   ttttatttac gtgggttttt aaatagtatt tattggtcgg tttcgcgtta cgagacgcga 2640
   tggacgtgat cgtcgttttt attttttgcg gcgaattttt agcgggtgac gttgtttaat 2700
   cggaaagcgt tttttcgtcg gttgggtttt tcgtttattt ttagcggcga ggggcggggt 2760
   cggcgcgggc gtagaggcgt tacgtatttt atggtaacga cgttcggttc gaagatggcg 2820
   gtcgaatggg gcggaggagt gggttattcg ggtttaggtt cgggtcggag tcggtggcgt 2880
   tggagcgggt ttgtgtgggt tcgaagcgtt ttatttttgt tgggcgggtt tcgggttagc 2940
   gttatattta ttttcgtttt tttgggtagt tttttttttt gtcggtatta cgtttttttt 3000
   g 3001
<210> 135
   <211> 3001
   <212> DNA
   <213> human
<400> 135
   taggtttatt tattaggatt gtggtggttt tttttttttt ttttttgaga tagttttgtt 60
   ttgttgttta ggttggagtg tagtggtgta attttggttt attgtatgtt ttgttttttg 120
   ggtttatgtt attttgtttt agttttttag gattgtggtt ttaaggtaaa taaagtgtat 180
   tgatgttgta tttattagaa gtgagtagta ttttaaggtt ggaagttgga agttgtatat 240
   tagaatgtta atgaaattga atttgggaat tatgtagtta gtgtgaggaa gtgttgattt 300
   tggatattta ttttaaaagg ttaatataag ttaaaagtat ttattgtgtt tattgtgtta 360
   gttaagtaga tgtgattagt agtatttggg gtatttaggg atagaggtat gtaggtatat 420
   tggttgtgta atataaaatt gtaaagtgta gaggaagaaa tagggtggta ttttaaagag 480
   gaaatggtag gaagatttta ggaatataag tggttgattg gaaagtttag aaaattaaaa 540
   tgttatatga gaggtttttt gtattgaatt gaggatttta ggtttaagat tgtttttgag 600
   tgtaatataa tgttattgaa gaatttgatt tataggaaat tttaaaattt atttatttga 660
   gatagggttt ttgttgttta gtttgtagtg tagtggtgtg attatagttt attgtatttt 720
   tgaattttta ggtttagata attttttgtt tataattttt agagaatttg gtgtatgtta 780
   ggtatgttta gttaattaaa atgttttttt ggggttttag gttggttttg atttttggtt 840
   ttaagtgatt tttttgtttt gtttttttaa agtattggga ttatagatat gagttattag 900
   tttagattat tttaaatttt aatggtattt tagtataaat aagggaaaat taggtgggtt 960
   ttttagtttt aaatggagaa ttttaatatg tttttggttt tgggggttgg atggataatg 1020
   gtgtatttat aaagttgggg aattaggaga tggttttgtg ggaggagtta aagttgaatt 1080
   tgaggaagtt gtaagatgtt aatttgtgga tatatttatg aagatagatg ggtattttag 1140
   gtttgggtta ggtatatgga gaagtggatg gtattttgag tggaatagat atatggtaag 1200
   tggtaggtat ttgagtaaag tgggggaaaa taaatggttg agattttaat ttgagtaatt 1260
   tatatgtgta atggtaaatg agatttaaaa gtaaatatat gttttttttt attgagatga 1320
   tgtagtattt gtaaataata aatgtatgtt tttatgtttt ttttttttta tatggaaatt 1380
   aagttaaaag tatttaagtt attgttgtta aaggaggagt tttgtggttt ataggaaagg 1440
   gtttgtgtgg ttggtaattt gtttattata ttattgaaat agttttgttt tttggttggg 1500
   tgtggtggtt tatgtttgta attttaatat tttgggaggt ggaggtgggt ggattatttg 1560
   aggttatgag gttgagatta gtttggttaa tgtggtgaaa ttatgttttt attaaaaata 1620
   taaaaattgg ttgggtgtgg tggtgggtgt ttgtaatttt ggttatttgg gaggttgagg 1680
   taggggaata gtttgatttt gggaggtaga ggttgtattg agttgagatt gtgttattgt 1740
   attttagttt gggtgatgag tgaaattttg ttttaaaaaa aaaagaaata gttttttttt 1800
   agtataatat tattgttata gttaggagtt taggatgatt ggttttattt tttatttata 1860
   atagtaattt atttgtggta ttgtagtgga aagggtatgt tttttaagtt ataaatgtgg 1920
   tttggaattt tattttttat tagttgtggg atgatatata tatttagtga gtatttattt 1980
   gtaaaatggt aatatttaaa atattatttt atttttgtag ttgaattttt atttatagtt 2040
   tgtaatttga ggtttttttt tttttttatt tttttatttt ttagtatagt agtttattta 2100
   tttaattttt gttgaatgat ttttagttta gtgagtttgt aattttattt tttagtgtta 2160
   tgagttattg ggatttgttt taatttttta ttttgtatag ttttttatgg tttagttttg 2220
   aaggtaaggt ttttttaatg gttaggattt tttttatttt aggtgtagtg gtaatttttg 2280
   gttggaaatt aaaagatgga gttgagtaaa atagtaaatg gtgtattata gatttttata 2340
   ggaaatggtg ttagttaagt aatgggttta aagttggtga tttaggataa gtaaatgaaa 2400
   gtattttatt tattttttta tatagaggat tatattgaaa gattttgtag agggttgtat 2460
   gtatgtttaa atatttggta tttagtaggt gtatgttatt ttggtaaatg aaagaattga 2520
   aaaattgttt taagatattt atatatggtt aatgtttgtt agtttttttt ttttttatag 2580
   ttttatttat gtgggttttt aaatagtatt tattggttgg ttttgtgtta tgagatgtga 2640
   tggatgtgat tgttgttttt attttttgtg gtgaattttt agtgggtgat gttgtttaat 2700
   tggaaagtgt ttttttgttg gttgggtttt ttgtttattt ttagtggtga ggggtggggt 2760
   tggtgtgggt gtagaggtgt tatgtatttt atggtaatga tgtttggttt gaagatggtg 2820
   gttgaatggg gtggaggagt gggttatttg ggtttaggtt tgggttggag ttggtggtgt 2880
   tggagtgggt ttgtgtgggt ttgaagtgtt ttatttttgt tgggtgggtt ttgggttagt 2940
   gttatattta tttttgtttt tttgggtagt tttttttttt gttggtatta tgtttttttt 3000
   g 3001
<210> 136
   <211> 50
   <212> DNA
   <213> human
<400> 136
   gactttgaca gcttggaaat ccagggccag tacagtgaca tcaacaaccg 50
<210> 137
   <211> 50
   <212> DNA
   <213> human
<400> 137
   gattttgata gtttggaaat ttagggttag tatagtgata ttaataatcg 50
<210> 138
   <211> 50
   <212> DNA
   <213> human
<400> 138
   gattttgata gtttggaaat ttagggttag tatagtgata ttaataattg 50
<210> 139
   <211> 50
   <212> DNA
   <213> human
<400> 139
   cggttgttga tgttattgta ttggttttgg atttttaagt tgttaaagtt 50
<210> 140
   <211> 50
   <212> DNA
   <213> human
<400> 140
   tggttgttga tgttattgta ttggttttgg atttttaagt tgttaaagtt 50
<210> 141
   <211> 3001
   <212> DNA
   <213> human
<400> 141
   cccgccttgg cctcctaaaa gtgctgggat tacaggtgtg agccaatgcc cccagccaac     60
   ttaatgtctt tttttttttt tttttttttt ttgagacaga gtctcattct gccgcccagg    120
   ctggagtgca atggcgagat ctcggctcac tgcaagctcc gcctcctggg ttcacgccat    180
   tctcctgcct cagcctcccg agtagctggg actacaggca cccaccacca cacccagcta    240
   attttttgta ttttttagta gagacggggt ttcaccgtgt tagccaggga tggtctcgat    300
   ctcctgacct tgtgatccgc ccgccttggc ctcccaaagt gctgggatta caggcgtgag    360
   acaccgcgcc cagccaactt aatatctttt gtgcagcttc ctcatctatg aaatggggac    420
   aacagtgggc cctcacccca cagggagtta tggtgacgtg atgcatgtca atggcaggga    480
   tatgtcacag ccgacattta ttgagtactt actaacattc tctgtggcaa accaaaggaa    540
   acaaagcgga gagaagcata ctgtgtcctc ctttgaactc ctgggcactt gctaaacagt    600
   gtgtcagtct tcctggcgta tcataacttt ttcttaactc aagagcagaa tctaggtctt    660
   tttttttttc tactagtctc gctctaaggt gcgcccaggt tcaagcaata atcctgcttc    720
   agcctcctga gtagctggga ttacaggcgc ctgccactac gcccagctaa ttttttgtat    780
   ttttagtaga gatagggttt caccatgttg gtcaggctgg tcttgaactt ctgacctcag    840
   gtgatccacc cacctcggcc tcccaaagtg ctgtgattac aggcgtgagc caccgcgccc    900
   aacaaatctg ggtctttttc tctcaacttg cctacagggt atagtaggcc atcttgggtt    960
   gatcatgggc tcaaaacagt ttgtagaagg aaagaatgcc actttctagt cattattgcc   1020
   tgtggggccc catatgtgct gcctgcatta ccctgggctg cacactgctg tctgaagcac   1080
   tgaagcccat cacccctttc ttcaccccgc aaacccgtcc ctctctggcc cgaagtcgtt   1140
   ggagcctttc ctgaccaccg cattgctctg agctgctgct gccttcctgc ttcactttct   1200
   gtgcttctga tgtattcccg tccccaggct tcccaggtgg cttcccctct tcccctgggg   1260
   gctgatttcc actgagcagc caaccgcagc ctctggccac aaggagagcg gagcacaggt   1320
   agggcaagaa gacagaaggc cccggtggtg agtggagggc ttggtggtcc ctggacaccc   1380
   ctcactggcc acattctcct tgcaggagca ggaagccgcc taccaccatg agcagcacgc   1440
   tgtcacccac agactttgac agcttggaaa tccagggcca gtacagtgac atcaacaacc   1500
   gctgggacct tcctgactcg gactgggaca atgacagcag ctcggcccgc ctctttgaga   1560
   ggtctcgcat taaggctctg gcaggtgagg tcagaggagg gtcgaggtgg ggggatgctg   1620
   gaggaggctt tgtcagctcg tcatgaagag ccctttaact ttatgggaag actgactttt   1680
   ctttctaagt gggagaccca gagcactcta tcaattccct ggtccccaca tccagcctca    1740
   agaataggct ccaggccatt cagaactttc cccagctctt ccccccaagc atagagacgc    1800
   ttcactggcc cctggactgg ctgagctgac taaaacagct tccctctctg gcctcagccc    1860
   ccgggtcatg tcccttcatc cttcggcact ctcgctcttg ccactccttt ctccgcctac    1920
   ctctccctta cctgtcatgc ccttgttgtg tcttctttga tttccactgt catttcccac    1980
   acctgttccc agtctattat tcccctctgc tccctctcca cctgttcctt ccacctgcca    2040
   cctgttactc ccattatctc atctgcctgt tccctgaagc aggttccctc cacccgctgc    2100
   tgctggtttc tgcagccgcc cattctccct ggtgtgctct ctgcctgcct tggctcctac    2160
   tcttccctgc agcccagggc ctgggctctc cgctctgagc ggaaactcgt ccaacggcct    2220
   ccctgtcttc cgggggagga gttgtcatgg caacaagagt gcggctgcca cctgacacca    2280
   ccagtggctg gcatggggct gactcaccca cacaccccaa ggccacctac cccgtctggc    2340
   ttcctcctcc atcccactct tgtcattgcc attgacctgc tggctgccca gcccagccca    2400
   acccaaccca gtctgagggg tcccagctac tctcaccgag aggtggtgct ccgagtgggg    2460
   gtgggggttg gccactagaa gaagagagac tccaaggggg aggttcccaa acctggccac    2520
   atgttttaca ggatcaccca gggatgtttt ttacaaatac aggtttccaa gcccctccct    2580
   agacacaata agttagaatc ttggtccaga actcaggatt tttcaaaagc tccccaagtg    2640
   attcttttga gccagctcag caccagtctg aggaccccct tgtggcagcc atagcttccc    2700
   aggacccctt ctcattttct cctgggcctc tctgtccttc tggttgggtc ttcaggccgg    2760
   ctgtgaaaca ctcctaccag tgtgtctata catcagagaa gacagaaggt gtgggttatg    2820
   gagaatgtca tgtttgggga aaatgttcct ggcactctgg ttttggattc atgtagcctc    2880
   tagaactgtc atgatgtcta tgtgtgtaca acatgtgcca cccaccatgt ttgcacacat    2940
   gacaaatgcc atccaggact gtcaccatgc ctgtgtacat aggacacatg cctcccagga    3000
   c                                                                    3001
<210> 142
   <211> 3001
   <212> DNA
   <213> human
<400> 142
   ttcgttttgg ttttttaaaa gtgttgggat tataggtgtg agttaatgtt tttagttaat 60
   ttaatgtttt tttttttttt tttttttttt ttgagataga gttttatttt gtcgtttagg 120
   ttggagtgta atggcgagat ttcggtttat tgtaagtttc gttttttggg tttacgttat 180
   ttttttgttt tagtttttcg agtagttggg attataggta tttattatta tatttagtta 240
   attttttgta ttttttagta gagacggggt tttatcgtgt tagttaggga tggtttcgat 300
   tttttgattt tgtgattcgt tcgttttggt tttttaaagt gttgggatta taggcgtgag 360
   atatcgcgtt tagttaattt aatatttttt gtgtagtttt tttatttatg aaatggggat 420
   aatagtgggt ttttatttta tagggagtta tggtgacgtg atgtatgtta atggtaggga 480
   tatgttatag tcgatattta ttgagtattt attaatattt tttgtggtaa attaaaggaa 540
   ataaagcgga gagaagtata ttgtgttttt ttttgaattt ttgggtattt gttaaatagt 600
   gtgttagttt ttttggcgta ttataatttt tttttaattt aagagtagaa tttaggtttt 660
   tttttttttt tattagtttc gttttaaggt gcgtttaggt ttaagtaata attttgtttt 720
   agttttttga gtagttggga ttataggcgt ttgttattac gtttagttaa ttttttgtat 780
   ttttagtaga gatagggttt tattatgttg gttaggttgg ttttgaattt ttgattttag 840
   gtgatttatt tatttcggtt ttttaaagtg ttgtgattat aggcgtgagt tatcgcgttt 900
   aataaatttg ggtttttttt ttttaatttg tttatagggt atagtaggtt attttgggtt 960
   gattatgggt ttaaaatagt ttgtagaagg aaagaatgtt attttttagt tattattgtt 1020
   tgtggggttt tatatgtgtt gtttgtatta ttttgggttg tatattgttg tttgaagtat 1080
   tgaagtttat tatttttttt tttatttcgt aaattcgttt ttttttggtt cgaagtcgtt 1140
   ggagtttttt ttgattatcg tattgttttg agttgttgtt gtttttttgt tttatttttt 1200
   gtgtttttga tgtattttcg tttttaggtt ttttaggtgg tttttttttt ttttttgggg 1260
   gttgattttt attgagtagt taatcgtagt ttttggttat aaggagagcg gagtataggt 1320
   agggtaagaa gatagaaggt ttcggtggtg agtggagggt ttggtggttt ttggatattt 1380
   tttattggtt atattttttt tgtaggagta ggaagtcgtt tattattatg agtagtacgt 1440
   tgttatttat agattttgat agtttggaaa tttagggtta gtatagtgat attaataatc 1500
   gttgggattt ttttgattcg gattgggata atgatagtag ttcggttcgt ttttttgaga 1560
   ggtttcgtat taaggttttg gtaggtgagg ttagaggagg gtcgaggtgg ggggatgttg 1620
   gaggaggttt tgttagttcg ttatgaagag ttttttaatt ttatgggaag attgattttt 1680
   ttttttaagt gggagattta gagtatttta ttaatttttt ggtttttata tttagtttta 1740
   agaataggtt ttaggttatt tagaattttt tttagttttt ttttttaagt atagagacgt 1800
   tttattggtt tttggattgg ttgagttgat taaaatagtt tttttttttg gttttagttt 1860
   tcgggttatg tttttttatt tttcggtatt ttcgtttttg ttattttttt tttcgtttat 1920
   ttttttttta tttgttatgt ttttgttgtg ttttttttga tttttattgt tattttttat 1980
   atttgttttt agtttattat ttttttttgt ttttttttta tttgtttttt ttatttgtta 2040
   tttgttattt ttattatttt atttgtttgt tttttgaagt aggttttttt tattcgttgt 2100
   tgttggtttt tgtagtcgtt tatttttttt ggtgtgtttt ttgtttgttt tggtttttat 2160
   ttttttttgt agtttagggt ttgggttttt cgttttgagc ggaaattcgt ttaacggttt 2220
   ttttgttttt cgggggagga gttgttatgg taataagagt gcggttgtta tttgatatta 2280
   ttagtggttg gtatggggtt gatttattta tatattttaa ggttatttat ttcgtttggt 2340
   tttttttttt attttatttt tgttattgtt attgatttgt tggttgttta gtttagttta 2400
   atttaattta gtttgagggg ttttagttat ttttatcgag aggtggtgtt tcgagtgggg 2460
   gtgggggttg gttattagaa gaagagagat tttaaggggg aggtttttaa atttggttat 2520
   atgttttata ggattattta gggatgtttt ttataaatat aggtttttaa gttttttttt 2580
   agatataata agttagaatt ttggtttaga atttaggatt ttttaaaagt tttttaagtg 2640
   atttttttga gttagtttag tattagtttg aggatttttt tgtggtagtt atagtttttt 2700
   aggatttttt tttatttttt tttgggtttt tttgtttttt tggttgggtt tttaggtcgg 2760
   ttgtgaaata tttttattag tgtgtttata tattagagaa gatagaaggt gtgggttatg 2820
   gagaatgtta tgtttgggga aaatgttttt ggtattttgg ttttggattt atgtagtttt 2880
   tagaattgtt atgatgttta tgtgtgtata atatgtgtta tttattatgt ttgtatatat 2940
   gataaatgtt atttaggatt gttattatgt ttgtgtatat aggatatatg ttttttagga 3000
   t 3001
<210> 143
   <211> 3001
   <212> DNA
   <213> human
<400> 143
   tttgttttgg ttttttaaaa gtgttgggat tataggtgtg agttaatgtt tttagttaat 60
   ttaatgtttt tttttttttt tttttttttt ttgagataga gttttatttt gttgtttagg 120
   ttggagtgta atggtgagat tttggtttat tgtaagtttt gttttttggg tttatgttat 180
   ttttttgttt tagttttttg agtagttggg attataggta tttattatta tatttagtta 240
   attttttgta ttttttagta gagatggggt tttattgtgt tagttaggga tggttttgat 300
   tttttgattt tgtgatttgt ttgttttggt tttttaaagt gttgggatta taggtgtgag 360
   atattgtgtt tagttaattt aatatttttt gtgtagtttt tttatttatg aaatggggat 420
   aatagtgggt ttttatttta tagggagtta tggtgatgtg atgtatgtta atggtaggga 480
   tatgttatag ttgatattta ttgagtattt attaatattt tttgtggtaa attaaaggaa 540
   ataaagtgga gagaagtata ttgtgttttt ttttgaattt ttgggtattt gttaaatagt 600
   gtgttagttt ttttggtgta ttataatttt tttttaattt aagagtagaa tttaggtttt 660
   tttttttttt tattagtttt gttttaaggt gtgtttaggt ttaagtaata attttgtttt 720
   agttttttga gtagttggga ttataggtgt ttgttattat gtttagttaa ttttttgtat 780
   ttttagtaga gatagggttt tattatgttg gttaggttgg ttttgaattt ttgattttag 840
   gtgatttatt tattttggtt ttttaaagtg ttgtgattat aggtgtgagt tattgtgttt 900
   aataaatttg ggtttttttt ttttaatttg tttatagggt atagtaggtt attttgggtt 960
   gattatgggt ttaaaatagt ttgtagaagg aaagaatgtt attttttagt tattattgtt 1020
   tgtggggttt tatatgtgtt gtttgtatta ttttgggttg tatattgttg tttgaagtat 1080
   tgaagtttat tatttttttt tttattttgt aaatttgttt ttttttggtt tgaagttgtt 1140
   ggagtttttt ttgattattg tattgttttg agttgttgtt gtttttttgt tttatttttt 1200
   gtgtttttga tgtatttttg tttttaggtt ttttaggtgg tttttttttt ttttttgggg 1260
   gttgattttt attgagtagt taattgtagt ttttggttat aaggagagtg gagtataggt 1320
   agggtaagaa gatagaaggt tttggtggtg agtggagggt ttggtggttt ttggatattt 1380
   tttattggtt atattttttt tgtaggagta ggaagttgtt tattattatg agtagtatgt 1440
   tgttatttat agattttgat agtttggaaa tttagggtta gtatagtgat attaataatt 1500
   gttgggattt ttttgatttg gattgggata atgatagtag tttggtttgt ttttttgaga 1560
   ggttttgtat taaggttttg gtaggtgagg ttagaggagg gttgaggtgg ggggatgttg 1620
   gaggaggttt tgttagtttg ttatgaagag ttttttaatt ttatgggaag attgattttt 1680
   ttttttaagt gggagattta gagtatttta ttaatttttt ggtttttata tttagtttta 1740
   agaataggtt ttaggttatt tagaattttt tttagttttt ttttttaagt atagagatgt 1800
   tttattggtt tttggattgg ttgagttgat taaaatagtt tttttttttg gttttagttt 1860
   ttgggttatg tttttttatt ttttggtatt tttgtttttg ttattttttt ttttgtttat 1920
   ttttttttta tttgttatgt ttttgttgtg ttttttttga tttttattgt tattttttat 1980
   atttgttttt agtttattat ttttttttgt ttttttttta tttgtttttt ttatttgtta 2040
   tttgttattt ttattatttt atttgtttgt tttttgaagt aggttttttt tatttgttgt 2100
   tgttggtttt tgtagttgtt tatttttttt ggtgtgtttt ttgtttgttt tggtttttat 2160
   ttttttttgt agtttagggt ttgggttttt tgttttgagt ggaaatttgt ttaatggttt 2220
   ttttgttttt tgggggagga gttgttatgg taataagagt gtggttgtta tttgatatta 2280
   ttagtggttg gtatggggtt gatttattta tatattttaa ggttatttat tttgtttggt 2340
   tttttttttt attttatttt tgttattgtt attgatttgt tggttgttta gtttagttta 2400
   atttaattta gtttgagggg ttttagttat ttttattgag aggtggtgtt ttgagtgggg 2460
   gtgggggttg gttattagaa gaagagagat tttaaggggg aggtttttaa atttggttat 2520
   atgttttata ggattattta gggatgtttt ttataaatat aggtttttaa gttttttttt 2580
   agatataata agttagaatt ttggtttaga atttaggatt ttttaaaagt tttttaagtg 2640
   atttttttga gttagtttag tattagtttg aggatttttt tgtggtagtt atagtttttt 2700
   aggatttttt tttatttttt tttgggtttt tttgtttttt tggttgggtt tttaggttgg 2760
   ttgtgaaata tttttattag tgtgtttata tattagagaa gatagaaggt gtgggttatg 2820
   gagaatgtta tgtttgggga aaatgttttt ggtattttgg ttttggattt atgtagtttt 2880
   tagaattgtt atgatgttta tgtgtgtata atatgtgtta tttattatgt ttgtatatat 2940
   gataaatgtt atttaggatt gttattatgt ttgtgtatat aggatatatg ttttttagga 3000
   t 3001
<210> 144
   <211> 3001
   <212> DNA
   <213> human
<400> 144
   gttttgggag gtatgtgttt tatgtatata ggtatggtga tagttttgga tggtatttgt 60
   tatgtgtgta aatatggtgg gtggtatatg ttgtatatat atagatatta tgatagtttt 120
   agaggttata tgaatttaaa attagagtgt taggaatatt ttttttaaat atgatatttt 180
   ttataattta tattttttgt tttttttgat gtatagatat attggtagga gtgttttata 240
   gtcggtttga agatttaatt agaaggatag agaggtttag gagaaaatga gaaggggttt 300
   tgggaagtta tggttgttat aagggggttt ttagattggt gttgagttgg tttaaaagaa 360
   ttatttgggg agtttttgaa aaattttgag ttttggatta agattttaat ttattgtgtt 420
   tagggagggg tttggaaatt tgtatttgta aaaaatattt ttgggtgatt ttgtaaaata 480
   tgtggttagg tttgggaatt ttttttttgg agtttttttt tttttagtgg ttaattttta 540
   tttttattcg gagtattatt tttcggtgag agtagttggg attttttaga ttgggttggg 600
   ttgggttggg ttgggtagtt agtaggttaa tggtaatgat aagagtggga tggaggagga 660
   agttagacgg ggtaggtggt tttggggtgt gtgggtgagt tagttttatg ttagttattg 720
   gtggtgttag gtggtagtcg tatttttgtt gttatgataa tttttttttc ggaagatagg 780
   gaggtcgttg gacgagtttt cgtttagagc ggagagttta ggttttgggt tgtagggaag 840
   agtaggagtt aaggtaggta gagagtatat tagggagaat gggcggttgt agaaattagt 900
   agtagcgggt ggagggaatt tgttttaggg aataggtaga tgagataatg ggagtaatag 960
   gtggtaggtg gaaggaatag gtggagaggg agtagagggg aataatagat tgggaatagg 1020
   tgtgggaaat gatagtggaa attaaagaag atataataag ggtatgatag gtaagggaga 1080
   ggtaggcgga gaaaggagtg gtaagagcga gagtgtcgaa ggatgaaggg atatgattcg 1140
   ggggttgagg ttagagaggg aagttgtttt agttagttta gttagtttag gggttagtga 1200
   agcgttttta tgtttggggg gaagagttgg ggaaagtttt gaatggtttg gagtttattt 1260
   ttgaggttgg atgtggggat tagggaattg atagagtgtt ttgggttttt tatttagaaa 1320
   gaaaagttag tttttttata aagttaaagg gttttttatg acgagttgat aaagtttttt 1380
   ttagtatttt tttatttcga tttttttttg attttatttg ttagagtttt aatgcgagat 1440
   tttttaaaga ggcgggtcga gttgttgtta ttgttttagt tcgagttagg aaggttttag 1500
   cggttgttga tgttattgta ttggttttgg atttttaagt tgttaaagtt tgtgggtgat 1560
   agcgtgttgt ttatggtggt aggcggtttt ttgtttttgt aaggagaatg tggttagtga 1620
   ggggtgttta gggattatta agttttttat ttattatcgg ggttttttgt ttttttgttt 1680
   tatttgtgtt tcgttttttt tgtggttaga ggttgcggtt ggttgtttag tggaaattag 1740
   tttttagggg aagaggggaa gttatttggg aagtttgggg acgggaatat attagaagta 1800
   tagaaagtga agtaggaagg tagtagtagt ttagagtaat gcggtggtta ggaaaggttt 1860
   taacgatttc gggttagaga gggacgggtt tgcggggtga agaaaggggt gatgggtttt 1920
   agtgttttag atagtagtgt gtagtttagg gtaatgtagg tagtatatat ggggttttat 1980
   aggtaataat gattagaaag tggtattttt tttttttata aattgttttg agtttatgat 2040
   taatttaaga tggtttatta tattttgtag gtaagttgag agaaaaagat ttagatttgt 2100
   tgggcgcggt ggtttacgtt tgtaattata gtattttggg aggtcgaggt gggtggatta 2160
   tttgaggtta gaagtttaag attagtttga ttaatatggt gaaattttat ttttattaaa 2220
   aatataaaaa attagttggg cgtagtggta ggcgtttgta attttagtta tttaggaggt 2280
   tgaagtagga ttattgtttg aatttgggcg tattttagag cgagattagt agaaaaaaaa 2340
   aaagatttag attttgtttt tgagttaaga aaaagttatg atacgttagg aagattgata 2400
   tattgtttag taagtgttta ggagtttaaa ggaggatata gtatgttttt tttcgttttg 2460
   ttttttttgg tttgttatag agaatgttag taagtattta ataaatgtcg gttgtgatat 2520
   atttttgtta ttgatatgta ttacgttatt ataatttttt gtggggtgag ggtttattgt 2580
   tgtttttatt ttatagatga ggaagttgta taaaagatat taagttggtt gggcgcggtg 2640
   ttttacgttt gtaattttag tattttggga ggttaaggcg ggcggattat aaggttagga 2700
   gatcgagatt atttttggtt aatacggtga aatttcgttt ttattaaaaa atataaaaaa 2760
   ttagttgggt gtggtggtgg gtgtttgtag ttttagttat tcgggaggtt gaggtaggag 2820
   aatggcgtga atttaggagg cggagtttgt agtgagtcga gatttcgtta ttgtatttta 2880
   gtttgggcgg tagaatgaga ttttgtttta aaaaaaaaaa aaaaaaaaaa aaagatatta 2940
   agttggttgg gggtattggt ttatatttgt aattttagta tttttaggag gttaaggcgg 3000
   g 3001
<210> 145
   <211> 3001
   <212> DNA
   <213> human
<400> 145
   gttttgggag gtatgtgttt tatgtatata ggtatggtga tagttttgga tggtatttgt 60
   tatgtgtgta aatatggtgg gtggtatatg ttgtatatat atagatatta tgatagtttt 120
   agaggttata tgaatttaaa attagagtgt taggaatatt ttttttaaat atgatatttt 180
   ttataattta tattttttgt tttttttgat gtatagatat attggtagga gtgttttata 240
   gttggtttga agatttaatt agaaggatag agaggtttag gagaaaatga gaaggggttt 300
   tgggaagtta tggttgttat aagggggttt ttagattggt gttgagttgg tttaaaagaa 360
   ttatttgggg agtttttgaa aaattttgag ttttggatta agattttaat ttattgtgtt 420
   tagggagggg tttggaaatt tgtatttgta aaaaatattt ttgggtgatt ttgtaaaata 480
   tgtggttagg tttgggaatt ttttttttgg agtttttttt tttttagtgg ttaattttta 540
   tttttatttg gagtattatt ttttggtgag agtagttggg attttttaga ttgggttggg 600
   ttgggttggg ttgggtagtt agtaggttaa tggtaatgat aagagtggga tggaggagga 660
   agttagatgg ggtaggtggt tttggggtgt gtgggtgagt tagttttatg ttagttattg 720
   gtggtgttag gtggtagttg tatttttgtt gttatgataa tttttttttt ggaagatagg 780
   gaggttgttg gatgagtttt tgtttagagt ggagagttta ggttttgggt tgtagggaag 840
   agtaggagtt aaggtaggta gagagtatat tagggagaat gggtggttgt agaaattagt 900
   agtagtgggt ggagggaatt tgttttaggg aataggtaga tgagataatg ggagtaatag 960
   gtggtaggtg gaaggaatag gtggagaggg agtagagggg aataatagat tgggaatagg 1020
   tgtgggaaat gatagtggaa attaaagaag atataataag ggtatgatag gtaagggaga 1080
   ggtaggtgga gaaaggagtg gtaagagtga gagtgttgaa ggatgaaggg atatgatttg 1140
   ggggttgagg ttagagaggg aagttgtttt agttagttta gttagtttag gggttagtga 1200
   agtgttttta tgtttggggg gaagagttgg ggaaagtttt gaatggtttg gagtttattt 1260
   ttgaggttgg atgtggggat tagggaattg atagagtgtt ttgggttttt tatttagaaa 1320
   gaaaagttag tttttttata aagttaaagg gttttttatg atgagttgat aaagtttttt 1380
   ttagtatttt tttattttga tttttttttg attttatttg ttagagtttt aatgtgagat 1440
   tttttaaaga ggtgggttga gttgttgtta ttgttttagt ttgagttagg aaggttttag 1500
   tggttgttga tgttattgta ttggttttgg atttttaagt tgttaaagtt tgtgggtgat 1560
   agtgtgttgt ttatggtggt aggtggtttt ttgtttttgt aaggagaatg tggttagtga 1620
   ggggtgttta gggattatta agttttttat ttattattgg ggttttttgt ttttttgttt 1680
   tatttgtgtt ttgttttttt tgtggttaga ggttgtggtt ggttgtttag tggaaattag 1740
   tttttagggg aagaggggaa gttatttggg aagtttgggg atgggaatat attagaagta 1800
   tagaaagtga agtaggaagg tagtagtagt ttagagtaat gtggtggtta ggaaaggttt 1860
   taatgatttt gggttagaga gggatgggtt tgtggggtga agaaaggggt gatgggtttt 1920
   agtgttttag atagtagtgt gtagtttagg gtaatgtagg tagtatatat ggggttttat 1980
   aggtaataat gattagaaag tggtattttt tttttttata aattgttttg agtttatgat 2040
   taatttaaga tggtttatta tattttgtag gtaagttgag agaaaaagat ttagatttgt 2100
   tgggtgtggt ggtttatgtt tgtaattata gtattttggg aggttgaggt gggtggatta 2160
   tttgaggtta gaagtttaag attagtttga ttaatatggt gaaattttat ttttattaaa 2220
   aatataaaaa attagttggg tgtagtggta ggtgtttgta attttagtta tttaggaggt 2280
   tgaagtagga ttattgtttg aatttgggtg tattttagag tgagattagt agaaaaaaaa 2340
   aaagatttag attttgtttt tgagttaaga aaaagttatg atatgttagg aagattgata 2400
   tattgtttag taagtgttta ggagtttaaa ggaggatata gtatgttttt ttttgttttg 2460
   ttttttttgg tttgttatag agaatgttag taagtattta ataaatgttg gttgtgatat 2520
   atttttgtta ttgatatgta ttatgttatt ataatttttt gtggggtgag ggtttattgt 2580
   tgtttttatt ttatagatga ggaagttgta taaaagatat taagttggtt gggtgtggtg 2640
   ttttatgttt gtaattttag tattttggga ggttaaggtg ggtggattat aaggttagga 2700
   gattgagatt atttttggtt aatatggtga aattttgttt ttattaaaaa atataaaaaa 2760
   ttagttgggt gtggtggtgg gtgtttgtag ttttagttat ttgggaggtt gaggtaggag 2820
   aatggtgtga atttaggagg tggagtttgt agtgagttga gattttgtta ttgtatttta 2880
   gtttgggtgg tagaatgaga ttttgtttta aaaaaaaaaa aaaaaaaaaa aaagatatta 2940
   agttggttgg gggtattggt ttatatttgt aattttagta tttttaggag gttaaggtgg 3000
   g 3001
<210> 146
   <211> 50
   <212> DNA
   <213> human
<400> 146
   tgtctttcgg gtagtctcct tgggcgtgat ctgtactatt ctcagactcg 50
<210> 147
   <211> 50
   <212> DNA
   <213> human
<400> 147
   tgtttttcgg gtagtttttt tgggcgtgat ttgtattatt tttagattcg 50
<210> 148
   <211> 50
   <212> DNA
   <213> human
<400> 148
   tgttttttgg gtagtttttt tgggtgtgat ttgtattatt tttagatttg 50
<210> 149
   <211> 50
   <212> DNA
   <213> human
<400> 149
   cgagtttgag aatagtatag attacgttta aggagattat tcgaaagata 50
<210> 150
   <211> 50
   <212> DNA
   <213> human
<400> 150
   tgagtttgag aatagtatag attatgttta aggagattat ttgaaagata 50
<210> 151
   <211> 3001
   <212> DNA
   <213> human
<400> 151
   aaaatcacat tcagagatga tattccactg ctaggatata aatcagaact ccattacaat      60
   accacatacg attgtattga tgccatttgt ccactattta aaaaaataat ttaggtgttt     120
   gcaggaatta cttctaagct ctttaccaat ctgctttcca ctcacctaag tggtaggatg     180
   gccacgattg ttattaaaac tccctcaaat tctactctaa aaatagttga agtcatgttt     240
   attatggtgt gcaaagtggg tcattgtgat gcagaggcag atggcagggc tggccaatga     300
   ttctctttac aatggatgct gatcagttcc tggtcaagtg acctaagaca tgattgtgtc     360
   aatcaagaaa aacagccatg acaacaataa tgacaagccc tgctgggcta ggctgctgac     420
   tggaaggtac agctacggtg acacaaactt tcttcctcag acatctctaa agagatggtt     480
   ctttcttggt aattgccatg tgttcattta aaaagaaaag gccttgcaac tttctaggca     540
   catacacact ttcctatgaa aattaaattg ttcacaaaat cattatgccc actttcagaa     600
   aataagagac aaataactgc tgaattggct ttgggaggct tatgtaacct atatttacaa     660
   gtgttttact ctgaagcaag tagctgagga acttagtaaa catctgcaag aataccccaa     720
   aattattttt tccactgcta tgttagtcta gctatcacag aactgcagaa tcaatctgct     780
   atccacaaga gccccaaaca ccatcacatc aaaagaactg cagatctcaa aaacataata     840
   attcagaacc gtagtcaaga aaagcattaa ataaaaatcc accaccacca acctttgctt     900
   gggcttctgg tgctgtcacc ttgacgacct tattgcggtt tatcatttgc ttcacccatt     960
   tttctacttg gacgttgaat ttcatgaaaa ccacatagca aaaataagct gttaagagaa    1020
   gcaagctttc ccaccacatg atgacattat ccaggaaaaa tatgatcagc atgatcaagt    1080
   caacaatgta gaaagacaca tctcgaaaga gcggccacca tgtcaggttt aagatttctc    1140
   tagaaaacag agcacacatg ccaataacaa agaggatgtt gaatactgct gaacctacaa    1200
   ttgtgcctat gccaacgttg ctgtgagcga taaatacccc tatgagagat gtgaaaagtt    1260
   ctggggctga ccctcctgca gccatgaagg tggctccagc cacatcatca gagatgccca    1320
   gtttttcagt gatgacagtc aaagaaggaa caaagaactc atcacagaca atggctaagg    1380
   ctatgaacat gtagatcatt ccaatgacat gcagaatgat cgcacctttt cttctctcct    1440
   caagggaaaa gatgtctttc gggtagtctc cttgggcgtg atctgtacta ttctcagact    1500
   cgccttcctt agaaagaggt ggctgtggag tataatccag aatcttgtca tttaaatcta    1560
   agagagttct ctgatggtaa ccctgtgcta ccctagggcc acttacaaca ctggcctctc    1620
   ctgtgctctg tgtatctgtc tcagaaaagg cactgattga aaatgagaca gtgctaatgg    1680
   ctaccagacc catgaaaagg cctaagactc gaattaactt cagttttttc ttgacactat     1740
   aatgtcttct gcagccagac agtgactcat ccaaacacca tttctctagg gaagtgatgg    1800
   tggtgctttg ttgcagatcc atcctgggtc ttctggtgga tatggtgatc ttccaacttt    1860
   agactcaacc agatggttct ttcatacttt tccttacttt atagttaacg atgcttgtgg    1920
   ggtactttca caatcaggga ttgttatgct tcacaggaaa ctttcatcat ttatgcttaa    1980
   ataaaaataa aaacgagaat aagtaaatca taaaatcacg tctttttaat aaagatcttt    2040
   gcagatatga taaagtcctg tcctgcagct gtgcgatctt gggtaagtta cttaacttct    2100
   ctgtgcctca gtttcctcat ttaaaatggg gacaattacg gtacctacca cccaggattg    2160
   ttttcaagat gagtaaaaac atatgtagca cttagtatgg gatgtggcac ttatgaagat    2220
   gtatgtataa acccaaataa atattttggt aaatccaatg ttaggtgcag agaaggcaga    2280
   tgtaagaata aaaattttac aggtgaaaat ctatcagatt atctcacctt aaatcatgta    2340
   ataaccttcc agtgctataa aggtacctag acttctttca gaactgtcta ggtacttggc    2400
   aactaacaat tattcattgt ttacttttgg tccaattaca taattctcag ttgtaggcaa    2460
   ctttttctga tttaacctat catttggaaa gtgagtgatt ctttaattta gtgatctttt    2520
   ttctgcacac ataatgatat cagatcttat ttttcataaa aagtctctcc tttatcagat    2580
   gcgggaaagt agtcctttac aaatttaagt ttacaaaaaa aaattctggt gacatgcaca    2640
   agctgccttc cctaaacatc tagacattgt tttcagctaa atattttttt taaaaaaaca    2700
   acctttagtc atatttaaaa taggcagctt agtaagtcaa agaactccca atgcagtgaa    2760
   ataacaagta ggctatttcg agagaccaca tgtgtatttc ataaacaata cgcaagttgt    2820
   agcaatacct ctgtccacaa aatctgccca aaatagcccc accactgact ttaataggtt    2880
   agatatccaa agcagagttt tctttctcta tcccacggag gagatgggta aaatagccag    2940
   acttgacagc tgatattctg aagagttctc tttttagttt ttctttaaaa agactattct    3000
   t                                                                     3001
<210> 152
   <211> 3001
   <212> DNA
   <213> human
<400> 152
   aaaattatat ttagagatga tattttattg ttaggatata aattagaatt ttattataat 60
   attatatacg attgtattga tgttatttgt ttattattta aaaaaataat ttaggtgttt 120
   gtaggaatta tttttaagtt ttttattaat ttgtttttta tttatttaag tggtaggatg 180
   gttacgattg ttattaaaat ttttttaaat tttattttaa aaatagttga agttatgttt 240
   attatggtgt gtaaagtggg ttattgtgat gtagaggtag atggtagggt tggttaatga 300
   ttttttttat aatggatgtt gattagtttt tggttaagtg atttaagata tgattgtgtt 360
   aattaagaaa aatagttatg ataataataa tgataagttt tgttgggtta ggttgttgat 420
   tggaaggtat agttacggtg atataaattt ttttttttag atatttttaa agagatggtt 480
   tttttttggt aattgttatg tgtttattta aaaagaaaag gttttgtaat tttttaggta 540
   tatatatatt tttttatgaa aattaaattg tttataaaat tattatgttt atttttagaa 600
   aataagagat aaataattgt tgaattggtt ttgggaggtt tatgtaattt atatttataa 660
   gtgttttatt ttgaagtaag tagttgagga atttagtaaa tatttgtaag aatattttaa 720
   aattattttt tttattgtta tgttagttta gttattatag aattgtagaa ttaatttgtt 780
   atttataaga gttttaaata ttattatatt aaaagaattg tagattttaa aaatataata 840
   atttagaatc gtagttaaga aaagtattaa ataaaaattt attattatta atttttgttt 900
   gggtttttgg tgttgttatt ttgacgattt tattgcggtt tattatttgt tttatttatt 960
   tttttatttg gacgttgaat tttatgaaaa ttatatagta aaaataagtt gttaagagaa 1020
   gtaagttttt ttattatatg atgatattat ttaggaaaaa tatgattagt atgattaagt 1080
   taataatgta gaaagatata tttcgaaaga gcggttatta tgttaggttt aagatttttt 1140
   tagaaaatag agtatatatg ttaataataa agaggatgtt gaatattgtt gaatttataa 1200
   ttgtgtttat gttaacgttg ttgtgagcga taaatatttt tatgagagat gtgaaaagtt 1260
   ttggggttga tttttttgta gttatgaagg tggttttagt tatattatta gagatgttta 1320
   gttttttagt gatgatagtt aaagaaggaa taaagaattt attatagata atggttaagg 1380
   ttatgaatat gtagattatt ttaatgatat gtagaatgat cgtatttttt tttttttttt 1440
   taagggaaaa gatgtttttc gggtagtttt tttgggcgtg atttgtatta tttttagatt 1500
   cgtttttttt agaaagaggt ggttgtggag tataatttag aattttgtta tttaaattta 1560
   agagagtttt ttgatggtaa ttttgtgtta ttttagggtt atttataata ttggtttttt 1620
   ttgtgttttg tgtatttgtt ttagaaaagg tattgattga aaatgagata gtgttaatgg 1680
   ttattagatt tatgaaaagg tttaagattc gaattaattt tagttttttt ttgatattat 1740
   aatgtttttt gtagttagat agtgatttat ttaaatatta tttttttagg gaagtgatgg 1800
   tggtgttttg ttgtagattt attttgggtt ttttggtgga tatggtgatt ttttaatttt 1860
   agatttaatt agatggtttt tttatatttt tttttatttt atagttaacg atgtttgtgg 1920
   ggtattttta taattaggga ttgttatgtt ttataggaaa tttttattat ttatgtttaa 1980
   ataaaaataa aaacgagaat aagtaaatta taaaattacg tttttttaat aaagattttt 2040
   gtagatatga taaagttttg ttttgtagtt gtgcgatttt gggtaagtta tttaattttt 2100
   ttgtgtttta gtttttttat ttaaaatggg gataattacg gtatttatta tttaggattg 2160
   tttttaagat gagtaaaaat atatgtagta tttagtatgg gatgtggtat ttatgaagat 2220
   gtatgtataa atttaaataa atattttggt aaatttaatg ttaggtgtag agaaggtaga 2280
   tgtaagaata aaaattttat aggtgaaaat ttattagatt attttatttt aaattatgta 2340
   ataatttttt agtgttataa aggtatttag atttttttta gaattgttta ggtatttggt 2400
   aattaataat tatttattgt ttatttttgg tttaattata taatttttag ttgtaggtaa 2460
   ttttttttga tttaatttat tatttggaaa gtgagtgatt ttttaattta gtgatttttt 2520
   ttttgtatat ataatgatat tagattttat tttttataaa aagttttttt tttattagat 2580
   gcgggaaagt agttttttat aaatttaagt ttataaaaaa aaattttggt gatatgtata 2640
   agttgttttt tttaaatatt tagatattgt ttttagttaa atattttttt taaaaaaata 2700
   atttttagtt atatttaaaa taggtagttt agtaagttaa agaattttta atgtagtgaa 2760
   ataataagta ggttatttcg agagattata tgtgtatttt ataaataata cgtaagttgt 2820
   agtaatattt ttgtttataa aatttgttta aaatagtttt attattgatt ttaataggtt 2880
   agatatttaa agtagagttt ttttttttta ttttacggag gagatgggta aaatagttag 2940
   atttgatagt tgatattttg aagagttttt tttttagttt ttttttaaaa agattatttt 3000
   t 3001
<210> 153
   <211> 3001
   <212> DNA
   <213> human
<400> 153
   aaaattatat ttagagatga tattttattg ttaggatata aattagaatt ttattataat 60
   attatatatg attgtattga tgttatttgt ttattattta aaaaaataat ttaggtgttt 120
   gtaggaatta tttttaagtt ttttattaat ttgtttttta tttatttaag tggtaggatg 180
   gttatgattg ttattaaaat ttttttaaat tttattttaa aaatagttga agttatgttt 240
   attatggtgt gtaaagtggg ttattgtgat gtagaggtag atggtagggt tggttaatga 300
   ttttttttat aatggatgtt gattagtttt tggttaagtg atttaagata tgattgtgtt 360
   aattaagaaa aatagttatg ataataataa tgataagttt tgttgggtta ggttgttgat 420
   tggaaggtat agttatggtg atataaattt ttttttttag atatttttaa agagatggtt 480
   tttttttggt aattgttatg tgtttattta aaaagaaaag gttttgtaat tttttaggta 540
   tatatatatt tttttatgaa aattaaattg tttataaaat tattatgttt atttttagaa 600
   aataagagat aaataattgt tgaattggtt ttgggaggtt tatgtaattt atatttataa 660
   gtgttttatt ttgaagtaag tagttgagga atttagtaaa tatttgtaag aatattttaa 720
   aattattttt tttattgtta tgttagttta gttattatag aattgtagaa ttaatttgtt 780
   atttataaga gttttaaata ttattatatt aaaagaattg tagattttaa aaatataata 840
   atttagaatt gtagttaaga aaagtattaa ataaaaattt attattatta atttttgttt 900
   gggtttttgg tgttgttatt ttgatgattt tattgtggtt tattatttgt tttatttatt 960
   tttttatttg gatgttgaat tttatgaaaa ttatatagta aaaataagtt gttaagagaa 1020
   gtaagttttt ttattatatg atgatattat ttaggaaaaa tatgattagt atgattaagt 1080
   taataatgta gaaagatata ttttgaaaga gtggttatta tgttaggttt aagatttttt 1140
   tagaaaatag agtatatatg ttaataataa agaggatgtt gaatattgtt gaatttataa 1200
   ttgtgtttat gttaatgttg ttgtgagtga taaatatttt tatgagagat gtgaaaagtt 1260
   ttggggttga tttttttgta gttatgaagg tggttttagt tatattatta gagatgttta 1320
   gttttttagt gatgatagtt aaagaaggaa taaagaattt attatagata atggttaagg 1380
   ttatgaatat gtagattatt ttaatgatat gtagaatgat tgtatttttt tttttttttt 1440
   taagggaaaa gatgtttttt gggtagtttt tttgggtgtg atttgtatta tttttagatt 1500
   tgtttttttt agaaagaggt ggttgtggag tataatttag aattttgtta tttaaattta 1560
   agagagtttt ttgatggtaa ttttgtgtta ttttagggtt atttataata ttggtttttt 1620
   ttgtgttttg tgtatttgtt ttagaaaagg tattgattga aaatgagata gtgttaatgg 1680
   ttattagatt tatgaaaagg tttaagattt gaattaattt tagttttttt ttgatattat 1740
   aatgtttttt gtagttagat agtgatttat ttaaatatta tttttttagg gaagtgatgg 1800
   tggtgttttg ttgtagattt attttgggtt ttttggtgga tatggtgatt ttttaatttt 1860
   agatttaatt agatggtttt tttatatttt tttttatttt atagttaatg atgtttgtgg 1920
   ggtattttta taattaggga ttgttatgtt ttataggaaa tttttattat ttatgtttaa 1980
   ataaaaataa aaatgagaat aagtaaatta taaaattatg tttttttaat aaagattttt 2040
   gtagatatga taaagttttg ttttgtagtt gtgtgatttt gggtaagtta tttaattttt 2100
   ttgtgtttta gtttttttat ttaaaatggg gataattatg gtatttatta tttaggattg 2160
   tttttaagat gagtaaaaat atatgtagta tttagtatgg gatgtggtat ttatgaagat 2220
   gtatgtataa atttaaataa atattttggt aaatttaatg ttaggtgtag agaaggtaga 2280
   tgtaagaata aaaattttat aggtgaaaat ttattagatt attttatttt aaattatgta 2340
   ataatttttt agtgttataa aggtatttag atttttttta gaattgttta ggtatttggt 2400
   aattaataat tatttattgt ttatttttgg tttaattata taatttttag ttgtaggtaa 2460
   ttttttttga tttaatttat tatttggaaa gtgagtgatt ttttaattta gtgatttttt 2520
   ttttgtatat ataatgatat tagattttat tttttataaa aagttttttt tttattagat 2580
   gtgggaaagt agttttttat aaatttaagt ttataaaaaa aaattttggt gatatgtata 2640
   agttgttttt tttaaatatt tagatattgt ttttagttaa atattttttt taaaaaaata 2700
   atttttagtt atatttaaaa taggtagttt agtaagttaa agaattttta atgtagtgaa 2760
   ataataagta ggttattttg agagattata tgtgtatttt ataaataata tgtaagttgt 2820
   agtaatattt ttgtttataa aatttgttta aaatagtttt attattgatt ttaataggtt 2880
   agatatttaa agtagagttt ttttttttta ttttatggag gagatgggta aaatagttag 2940
   atttgatagt tgatattttg aagagttttt tttttagttt ttttttaaaa agattatttt 3000
   t 3001
<210> 154
   <211> 3001
   <212> DNA
   <213> human
<400> 154
   aagaatagtt tttttaaaga aaaattaaaa agagaatttt ttagaatatt agttgttaag 60
   tttggttatt ttatttattt ttttcgtggg atagagaaag aaaattttgt tttggatatt 120
   taatttatta aagttagtgg tggggttatt ttgggtagat tttgtggata gaggtattgt 180
   tataatttgc gtattgttta tgaaatatat atgtggtttt tcgaaatagt ttatttgtta 240
   ttttattgta ttgggagttt tttgatttat taagttgttt attttaaata tgattaaagg 300
   ttgttttttt aaaaaaaata tttagttgaa aataatgttt agatgtttag ggaaggtagt 360
   ttgtgtatgt tattagaatt tttttttgta aatttaaatt tgtaaaggat tattttttcg 420
   tatttgataa aggagagatt ttttatgaaa aataagattt gatattatta tgtgtgtaga 480
   aaaaagatta ttaaattaaa gaattattta ttttttaaat gataggttaa attagaaaaa 540
   gttgtttata attgagaatt atgtaattgg attaaaagta aataatgaat aattgttagt 600
   tgttaagtat ttagatagtt ttgaaagaag tttaggtatt tttatagtat tggaaggtta 660
   ttatatgatt taaggtgaga taatttgata gatttttatt tgtaaaattt ttatttttat 720
   atttgttttt tttgtattta atattggatt tattaaaata tttatttggg tttatatata 780
   tatttttata agtgttatat tttatattaa gtgttatata tgtttttatt tattttgaaa 840
   ataattttgg gtggtaggta tcgtaattgt ttttatttta aatgaggaaa ttgaggtata 900
   gagaagttaa gtaatttatt taagatcgta tagttgtagg ataggatttt attatatttg 960
   taaagatttt tattaaaaag acgtgatttt atgatttatt tattttcgtt tttattttta 1020
   tttaagtata aatgatgaaa gttttttgtg aagtataata atttttgatt gtgaaagtat 1080
   tttataagta tcgttaatta taaagtaagg aaaagtatga aagaattatt tggttgagtt 1140
   taaagttgga agattattat atttattaga agatttagga tggatttgta ataaagtatt 1200
   attattattt ttttagagaa atggtgtttg gatgagttat tgtttggttg tagaagatat 1260
   tatagtgtta agaaaaaatt gaagttaatt cgagttttag gtttttttat gggtttggta 1320
   gttattagta ttgttttatt tttaattagt gttttttttg agatagatat atagagtata 1380
   ggagaggtta gtgttgtaag tggttttagg gtagtatagg gttattatta gagaattttt 1440
   ttagatttaa atgataagat tttggattat attttatagt tatttttttt taaggaaggc 1500
   gagtttgaga atagtataga ttacgtttaa ggagattatt cgaaagatat tttttttttt 1560
   gaggagagaa gaaaaggtgc gattattttg tatgttattg gaatgattta tatgtttata 1620
   gttttagtta ttgtttgtga tgagtttttt gttttttttt tgattgttat tattgaaaaa 1680
   ttgggtattt ttgatgatgt ggttggagtt atttttatgg ttgtaggagg gttagtttta 1740
   gaatttttta tattttttat aggggtattt atcgtttata gtaacgttgg tataggtata 1800
   attgtaggtt tagtagtatt taatattttt tttgttattg gtatgtgtgt tttgtttttt 1860
   agagaaattt taaatttgat atggtggtcg ttttttcgag atgtgttttt ttatattgtt 1920
   gatttgatta tgttgattat attttttttg gataatgtta ttatgtggtg ggaaagtttg 1980
   ttttttttaa tagtttattt ttgttatgtg gtttttatga aatttaacgt ttaagtagaa 2040
   aaatgggtga agtaaatgat aaatcgtaat aaggtcgtta aggtgatagt attagaagtt 2100
   taagtaaagg ttggtggtgg tggattttta tttaatgttt tttttgatta cggttttgaa 2160
   ttattatgtt tttgagattt gtagtttttt tgatgtgatg gtgtttgggg tttttgtgga 2220
   tagtagattg attttgtagt tttgtgatag ttagattaat atagtagtgg aaaaaataat 2280
   tttggggtat ttttgtagat gtttattaag ttttttagtt atttgtttta gagtaaaata 2340
   tttgtaaata taggttatat aagtttttta aagttaattt agtagttatt tgttttttat 2400
   tttttgaaag tgggtataat gattttgtga ataatttaat ttttatagga aagtgtgtat 2460
   gtgtttagaa agttgtaagg tttttttttt ttaaatgaat atatggtaat tattaagaaa 2520
   gaattatttt tttagagatg tttgaggaag aaagtttgtg ttatcgtagt tgtatttttt 2580
   agttagtagt ttagtttagt agggtttgtt attattgttg ttatggttgt tttttttgat 2640
   tgatataatt atgttttagg ttatttgatt aggaattgat tagtatttat tgtaaagaga 2700
   attattggtt agttttgtta tttgtttttg tattataatg atttattttg tatattataa 2760
   taaatatgat tttaattatt tttagagtag aatttgaggg agttttaata ataatcgtgg 2820
   ttattttatt atttaggtga gtggaaagta gattggtaaa gagtttagaa gtaatttttg 2880
   taaatattta aattattttt ttaaatagtg gataaatggt attaatataa tcgtatgtgg 2940
   tattgtaatg gagttttgat ttatatttta gtagtggaat attatttttg aatgtgattt 3000
   t 3001

<210> 155
   <211> 3001
   <212> DNA
   <213> human
<400> 155
   aagaatagtt tttttaaaga aaaattaaaa agagaatttt ttagaatatt agttgttaag 60
   tttggttatt ttatttattt tttttgtggg atagagaaag aaaattttgt tttggatatt 120
   taatttatta aagttagtgg tggggttatt ttgggtagat tttgtggata gaggtattgt 180
   tataatttgt gtattgttta tgaaatatat atgtggtttt ttgaaatagt ttatttgtta 240
   ttttattgta ttgggagttt tttgatttat taagttgttt attttaaata tgattaaagg 300
   ttgttttttt aaaaaaaata tttagttgaa aataatgttt agatgtttag ggaaggtagt 360
   ttgtgtatgt tattagaatt tttttttgta aatttaaatt tgtaaaggat tatttttttg 420
   tatttgataa aggagagatt ttttatgaaa aataagattt gatattatta tgtgtgtaga 480
   aaaaagatta ttaaattaaa gaattattta ttttttaaat gataggttaa attagaaaaa 540
   gttgtttata attgagaatt atgtaattgg attaaaagta aataatgaat aattgttagt 600
   tgttaagtat ttagatagtt ttgaaagaag tttaggtatt tttatagtat tggaaggtta 660
   ttatatgatt taaggtgaga taatttgata gatttttatt tgtaaaattt ttatttttat 720
   atttgttttt tttgtattta atattggatt tattaaaata tttatttggg tttatatata 780
   tatttttata agtgttatat tttatattaa gtgttatata tgtttttatt tattttgaaa 840
   ataattttgg gtggtaggta ttgtaattgt ttttatttta aatgaggaaa ttgaggtata 900
   gagaagttaa gtaatttatt taagattgta tagttgtagg ataggatttt attatatttg 960
   taaagatttt tattaaaaag atgtgatttt atgatttatt tatttttgtt tttattttta 1020
   tttaagtata aatgatgaaa gttttttgtg aagtataata atttttgatt gtgaaagtat 1080
   tttataagta ttgttaatta taaagtaagg aaaagtatga aagaattatt tggttgagtt 1140
   taaagttgga agattattat atttattaga agatttagga tggatttgta ataaagtatt 1200
   attattattt ttttagagaa atggtgtttg gatgagttat tgtttggttg tagaagatat 1260
   tatagtgtta agaaaaaatt gaagttaatt tgagttttag gtttttttat gggtttggta 1320
   gttattagta ttgttttatt tttaattagt gttttttttg agatagatat atagagtata 1380
   ggagaggtta gtgttgtaag tggttttagg gtagtatagg gttattatta gagaattttt 1440
   ttagatttaa atgataagat tttggattat attttatagt tatttttttt taaggaaggt 1500
   gagtttgaga atagtataga ttatgtttaa ggagattatt tgaaagatat tttttttttt 1560
   gaggagagaa gaaaaggtgt gattattttg tatgttattg gaatgattta tatgtttata 1620
   gttttagtta ttgtttgtga tgagtttttt gttttttttt tgattgttat tattgaaaaa 1680
   ttgggtattt ttgatgatgt ggttggagtt atttttatgg ttgtaggagg gttagtttta 1740
   gaatttttta tattttttat aggggtattt attgtttata gtaatgttgg tataggtata 1800
   attgtaggtt tagtagtatt taatattttt tttgttattg gtatgtgtgt tttgtttttt 1860
   agagaaattt taaatttgat atggtggttg tttttttgag atgtgttttt ttatattgtt 1920
   gatttgatta tgttgattat attttttttg gataatgtta ttatgtggtg ggaaagtttg 1980
   ttttttttaa tagtttattt ttgttatgtg gtttttatga aatttaatgt ttaagtagaa 2040
   aaatgggtga agtaaatgat aaattgtaat aaggttgtta aggtgatagt attagaagtt 2100
   taagtaaagg ttggtggtgg tggattttta tttaatgttt tttttgatta tggttttgaa 2160
   ttattatgtt tttgagattt gtagtttttt tgatgtgatg gtgtttgggg tttttgtgga 2220
   tagtagattg attttgtagt tttgtgatag ttagattaat atagtagtgg aaaaaataat 2280
   tttggggtat ttttgtagat gtttattaag ttttttagtt atttgtttta gagtaaaata 2340
   tttgtaaata taggttatat aagtttttta aagttaattt agtagttatt tgttttttat 2400
   tttttgaaag tgggtataat gattttgtga ataatttaat ttttatagga aagtgtgtat 2460
   gtgtttagaa agttgtaagg tttttttttt ttaaatgaat atatggtaat tattaagaaa 2520
   gaattatttt tttagagatg tttgaggaag aaagtttgtg ttattgtagt tgtatttttt 2580
   agttagtagt ttagtttagt agggtttgtt attattgttg ttatggttgt tttttttgat 2640
   tgatataatt atgttttagg ttatttgatt aggaattgat tagtatttat tgtaaagaga 2700
   attattggtt agttttgtta tttgtttttg tattataatg atttattttg tatattataa 2760
   taaatatgat tttaattatt tttagagtag aatttgaggg agttttaata ataattgtgg 2820
   ttattttatt atttaggtga gtggaaagta gattggtaaa gagtttagaa gtaatttttg 2880
   taaatattta aattattttt ttaaatagtg gataaatggt attaatataa ttgtatgtgg 2940
   tattgtaatg gagttttgat ttatatttta gtagtggaat attatttttg aatgtgattt 3000
   t 3001
<210> 156
   <211> 50
   <212> DNA
   <213> human
<400> 156
   ttcctccatt tctggtttct tcttggtttc tgccactgtt tttctttacg 50
<210> 157
   <211> 50
   <212> DNA
   <213> human
<400> 157
   tttttttatt tttggttttt ttttggtttt tgttattgtt tttttttacg 50
<210> 158
   <211> 50
   <212> DNA
   <213> human
<400> 158
   tttttttatt tttggttttt ttttggtttt tgttattgtt tttttttatg 50
<210> 159
   <211> 50
   <212> DNA
   <213> human
<400> 159
   cgtaaagaaa aatagtggta gaaattaaga agaaattaga aatggaggaa 50
<210> 160
   <211> 50
   <212> DNA
   <213> human
<400> 160
   tgtaaagaaa aatagtggta gaaattaaga agaaattaga aatggaggaa 50
<210> 161
   <211> 3001
   <212> DNA
   <213> human
<400> 161
   gaatcacttg aggtcaggag tttgagacca gcctggccaa catggagaaa cctcgtctct      60
   actaaaaata caaaaattag ctgggcgtgg tggcgggcgc ctgtaatccc agctacttgg     120
   gaggctgagg caggagaata gcttgaaccc aggaggcaga ggctgcagtg agctgagatt     180
   gcgccactgc actccagcct gagtggaaga gtgagactct gtctcacaaa aaacaaaagc     240
   tttggaatgt tttaatagag tggtatgaac ttggtgttta cagattaaga gcggtcattt     300
   cttggttctt tacagtatgc caggtgctgt gttatgcaaa ccctccctag atgtccatgc     360
   ttaattcagc tctttgagta ttccatttac atttacaagt agaaaaatgg agtcagggag     420
   aagttaggta atttgccttt ggggtcatat aactaggaac tggtagagcc aggattcaaa     480
   cccaggttac ccattctcct atacatcaga gaccagtgtg taagaactgt aggatttgaa     540
   ttcatattgg aaatttcctc ccctttatgg atctttaatc tttctttctt tggctccttc     600
   acataatttg gataagcaca agtttcacct attttggcca gtgccttaag ttactccacc     660
   aacgtgcacc tcttcccagg agaagactgg gtcacattga aaagtagccc actgcaggca     720
   tgacattgtt atgaaatttt atgtttcatg taaaagatta aaagttactt tccactgtgc     780
   gcaactgaca caccagaaaa aggtatcttt ttttaatgcc tacccctatc cacacaccgt     840
   acctcaggtt gagacacagg ctatagaact ttccagtcat gtcataatca tatatggagt     900
   tcagttaatt cactatgttg ttgttgtggg ggtcaggttg ggaggaggtc accgacacag     960
   aacaagcagg caatagcgag gggttgaact gaggagtaac acagatccga acagtgtaag    1020
   ggagaagatt tgatgactgg agatggggag cttagttcct ccatttctgg tttcttcttg    1080
   gtttctgcca ctgtttttct ttacgctcct gtcctttaat tgtgggagtg tcttcagtcc    1140
   ttttcccact tactcacccc caccgcccca attgtgcgtt tgcccattgt cttggtttca    1200
   gctctcattt ctgtctgagt ggcttctaaa ctgcctgtgg ctttaatgag ctccagttca    1260
   gtgaatccac ctgagcccta atttgctgta gcaaatttgc agttgctcag tggctatatc    1320
   ctggatattt gaatgtctca gagtgagtac gtttgatttt aaccgctctt cctccaaagc    1380
   cagctgtaag catttttaag tggtacacaa attgatagga agagaacatt cccagatcca    1440
   ctgtagatga ggctcaggtt cctattttat cagccaaggt tgctgaactg ccctggaagc    1500
   gggcagccac atttgctgga aaagagacaa tatactgagg tgtagttttc tctggcccct    1560
   tggtgccctg tgaggcacac agctgtctgt gataatgtca gagattacat cacagtgcca    1620
   atggaactaa caggaaccac ctgtgcactt ccttttacag gaatgttcat aaacagcagg    1680
   ttctcctagg ctttgaacaa aatttagctt ctccaaggcc aaaagcctca ttgtgaagag    1740
   aaagaaaggt gggtgttctt cttaacgatg actctgcttg tagccttcca gtgtcctgca    1800
   acgctggttt ccacagctgt cttccttgct taattggtgt acactgccct cggcatgcaa    1860
   ctgaaaacgt tagggccata cgtcctaatg gttaagaatg tggaccgtaa aactagacat    1920
   gttggcttct aaattccagc cctgcttatt gctggtttta tgaatttaga caagttattt    1980
   attctctcca aacctccatt ttcttaactg aaacaggcta attagagtac ctaccacaaa    2040
   agttggtagt gagaattaaa tccgataaca cattagaagt atttagtgca gtgcctggca    2100
   cataccaaag gtcagtaaat gctaattgct attattaatt aacagtattg ttggtaaata    2160
   ccctaaaaaa gctgttttta ttttttctgt ctctgctatc tggtcaataa cctgatattg    2220
   tcatctcttc ctactcatag aatctcatgt tgtcatttgt ctctctgagt tactgctgct    2280
   accttctttg agcagatact cattaccact tcattgtgtg tacagaatgt tggtacagaa    2340
   gtcatcttcc tttttagaaa tgaccattag gcctggtgca gtggctcacg cctgtaatcc    2400
   cagcactttg ggaggccgag gtgggtggat cacttgagat caggagtttg agaccagcct    2460
   ggccaatgtg gcaaaactcc atctctacta aaaatacaaa aaatacaaaa atttgccagg    2520
   tgtagtggcg tgcgctggta atcccagcta cttgggggtc tgaggcagga gaattgcttg    2580
   aacctgggag gcagaggttg cagtgagcag agattgcacc actgcattcc agccccggtg    2640
   acagagtgag accctgtctc aaaaaaaaga aagaaaaaaa aagaagaaat gaccatttat    2700
   tctttggatc tactctacca cactgagtct aaattactca gtttgccttt caaagcctgt    2760
   catattgagg cctgtatatg gtattttcca ttactcttgt ttttctttta cataccctcc    2820
   actctagtca gacacaggaa tgtgtacaag gcatgcattt ttctttctct ttttgtctaa    2880
   attggcaccc tgtattgctt tgccaggcac atgcccattt gtctctcatg ggacagtttt    2940
   gaccctgtta tgtggaggtc gcatgtgacc tttgtctggc ctgcgctttc ccagggtctg    3000
   a                                                                    3001
<210> 162
   <211> 3001
   <212> DNA
   <213> human
<400> 162
   gaattatttg aggttaggag tttgagatta gtttggttaa tatggagaaa tttcgttttt     60
   attaaaaata taaaaattag ttgggcgtgg tggcgggcgt ttgtaatttt agttatttgg    120
   gaggttgagg taggagaata gtttgaattt aggaggtaga ggttgtagtg agttgagatt    180
   gcgttattgt attttagttt gagtggaaga gtgagatttt gttttataaa aaataaaagt    240
   tttggaatgt tttaatagag tggtatgaat ttggtgttta tagattaaga gcggttattt    300
   tttggttttt tatagtatgt taggtgttgt gttatgtaaa ttttttttag atgtttatgt    360
   ttaatttagt tttttgagta ttttatttat atttataagt agaaaaatgg agttagggag    420
   aagttaggta atttgttttt ggggttatat aattaggaat tggtagagtt aggatttaaa    480
   tttaggttat ttattttttt atatattaga gattagtgtg taagaattgt aggatttgaa    540
   tttatattgg aaattttttt ttttttatgg atttttaatt tttttttttt tggttttttt    600
   atataatttg gataagtata agttttattt attttggtta gtgttttaag ttattttatt    660
   aacgtgtatt tttttttagg agaagattgg gttatattga aaagtagttt attgtaggta    720
   tgatattgtt atgaaatttt atgttttatg taaaagatta aaagttattt tttattgtgc    780
   gtaattgata tattagaaaa aggtattttt ttttaatgtt tatttttatt tatatatcgt    840
   attttaggtt gagatatagg ttatagaatt ttttagttat gttataatta tatatggagt    900
   ttagttaatt tattatgttg ttgttgtggg ggttaggttg ggaggaggtt atcgatatag    960
   aataagtagg taatagcgag gggttgaatt gaggagtaat atagattcga atagtgtaag   1020
   ggagaagatt tgatgattgg agatggggag tttagttttt ttatttttgg tttttttttg   1080
   gtttttgtta ttgttttttt ttacgttttt gttttttaat tgtgggagtg tttttagttt   1140
   tttttttatt tatttatttt tatcgtttta attgtgcgtt tgtttattgt tttggtttta   1200
   gtttttattt ttgtttgagt ggtttttaaa ttgtttgtgg ttttaatgag ttttagttta   1260
   gtgaatttat ttgagtttta atttgttgta gtaaatttgt agttgtttag tggttatatt   1320
   ttggatattt gaatgtttta gagtgagtac gtttgatttt aatcgttttt tttttaaagt   1380
   tagttgtaag tatttttaag tggtatataa attgatagga agagaatatt tttagattta   1440
   ttgtagatga ggtttaggtt tttattttat tagttaaggt tgttgaattg ttttggaagc   1500
   gggtagttat atttgttgga aaagagataa tatattgagg tgtagttttt tttggttttt   1560
   tggtgttttg tgaggtatat agttgtttgt gataatgtta gagattatat tatagtgtta   1620
   atggaattaa taggaattat ttgtgtattt ttttttatag gaatgtttat aaatagtagg   1680
   tttttttagg ttttgaataa aatttagttt ttttaaggtt aaaagtttta ttgtgaagag   1740
   aaagaaaggt gggtgttttt tttaacgatg attttgtttg tagtttttta gtgttttgta   1800
   acgttggttt ttatagttgt tttttttgtt taattggtgt atattgtttt cggtatgtaa   1860
   ttgaaaacgt tagggttata cgttttaatg gttaagaatg tggatcgtaa aattagatat   1920
   gttggttttt aaattttagt tttgtttatt gttggtttta tgaatttaga taagttattt   1980
   atttttttta aatttttatt tttttaattg aaataggtta attagagtat ttattataaa   2040
   agttggtagt gagaattaaa ttcgataata tattagaagt atttagtgta gtgtttggta   2100
   tatattaaag gttagtaaat gttaattgtt attattaatt aatagtattg ttggtaaata   2160
   ttttaaaaaa gttgttttta ttttttttgt ttttgttatt tggttaataa tttgatattg   2220
   ttattttttt ttatttatag aattttatgt tgttatttgt ttttttgagt tattgttgtt   2280
   attttttttg agtagatatt tattattatt ttattgtgtg tatagaatgt tggtatagaa   2340
   gttatttttt tttttagaaa tgattattag gtttggtgta gtggtttacg tttgtaattt   2400
   tagtattttg ggaggtcgag gtgggtggat tatttgagat taggagtttg agattagttt   2460
   ggttaatgtg gtaaaatttt atttttatta aaaatataaa aaatataaaa atttgttagg   2520
   tgtagtggcg tgcgttggta attttagtta tttgggggtt tgaggtagga gaattgtttg   2580
   aatttgggag gtagaggttg tagtgagtag agattgtatt attgtatttt agtttcggtg   2640
   atagagtgag attttgtttt aaaaaaaaga aagaaaaaaa aagaagaaat gattatttat   2700
   tttttggatt tattttatta tattgagttt aaattattta gtttgttttt taaagtttgt   2760
   tatattgagg tttgtatatg gtatttttta ttatttttgt ttttttttta tatatttttt   2820
   attttagtta gatataggaa tgtgtataag gtatgtattt tttttttttt ttttgtttaa   2880
   attggtattt tgtattgttt tgttaggtat atgtttattt gttttttatg ggatagtttt   2940
   gattttgtta tgtggaggtc gtatgtgatt tttgtttggt ttgcgttttt ttagggtttg   3000
   a                                                                   3001
<210> 163
   <211> 3001
   <212> DNA
   <213> human
<400> 163
   gaattatttg aggttaggag tttgagatta gtttggttaa tatggagaaa ttttgttttt      60
   attaaaaata taaaaattag ttgggtgtgg tggtgggtgt ttgtaatttt agttatttgg     120
   gaggttgagg taggagaata gtttgaattt aggaggtaga ggttgtagtg agttgagatt     180
   gtgttattgt attttagttt gagtggaaga gtgagatttt gttttataaa aaataaaagt     240
   tttggaatgt tttaatagag tggtatgaat ttggtgttta tagattaaga gtggttattt     300
   tttggttttt tatagtatgt taggtgttgt gttatgtaaa ttttttttag atgtttatgt      360
   ttaatttagt tttttgagta ttttatttat atttataagt agaaaaatgg agttagggag     420
   aagttaggta atttgttttt ggggttatat aattaggaat tggtagagtt aggatttaaa     480
   tttaggttat ttattttttt atatattaga gattagtgtg taagaattgt aggatttgaa     540
   tttatattgg aaattttttt ttttttatgg atttttaatt tttttttttt tggttttttt     600
   atataatttg gataagtata agttttattt attttggtta gtgttttaag ttattttatt     660
   aatgtgtatt tttttttagg agaagattgg gttatattga aaagtagttt attgtaggta     720
   tgatattgtt atgaaatttt atgttttatg taaaagatta aaagttattt tttattgtgt     780
   gtaattgata tattagaaaa aggtattttt ttttaatgtt tatttttatt tatatattgt     840
   attttaggtt gagatatagg ttatagaatt ttttagttat gttataatta tatatggagt     900
   ttagttaatt tattatgttg ttgttgtggg ggttaggttg ggaggaggtt attgatatag     960
   aataagtagg taatagtgag gggttgaatt gaggagtaat atagatttga atagtgtaag    1020
   ggagaagatt tgatgattgg agatggggag tttagttttt ttatttttgg tttttttttg    1080
   gtttttgtta ttgttttttt ttatgttttt gttttttaat tgtgggagtg tttttagttt    1140
   tttttttatt tatttatttt tattgtttta attgtgtgtt tgtttattgt tttggtttta    1200
   gtttttattt ttgtttgagt ggtttttaaa ttgtttgtgg ttttaatgag ttttagttta    1260
   gtgaatttat ttgagtttta atttgttgta gtaaatttgt agttgtttag tggttatatt    1320
   ttggatattt gaatgtttta gagtgagtat gtttgatttt aattgttttt tttttaaagt    1380
   tagttgtaag tatttttaag tggtatataa attgatagga agagaatatt tttagattta    1440
   ttgtagatga ggtttaggtt tttattttat tagttaaggt tgttgaattg ttttggaagt    1500
   gggtagttat atttgttgga aaagagataa tatattgagg tgtagttttt tttggttttt    1560
   tggtgttttg tgaggtatat agttgtttgt gataatgtta gagattatat tatagtgtta    1620
   atggaattaa taggaattat ttgtgtattt ttttttatag gaatgtttat aaatagtagg    1680
   tttttttagg ttttgaataa aatttagttt ttttaaggtt aaaagtttta ttgtgaagag    1740
   aaagaaaggt gggtgttttt tttaatgatg attttgtttg tagtttttta gtgttttgta    1800
   atgttggttt ttatagttgt tttttttgtt taattggtgt atattgtttt tggtatgtaa    1860
   ttgaaaatgt tagggttata tgttttaatg gttaagaatg tggattgtaa aattagatat    1920
   gttggttttt aaattttagt tttgtttatt gttggtttta tgaatttaga taagttattt    1980
   atttttttta aatttttatt tttttaattg aaataggtta attagagtat ttattataaa    2040
   agttggtagt gagaattaaa tttgataata tattagaagt atttagtgta gtgtttggta    2100
   tatattaaag gttagtaaat gttaattgtt attattaatt aatagtattg ttggtaaata    2160
   ttttaaaaaa gttgttttta ttttttttgt ttttgttatt tggttaataa tttgatattg    2220
   ttattttttt ttatttatag aattttatgt tgttatttgt ttttttgagt tattgttgtt    2280
   attttttttg agtagatatt tattattatt ttattgtgtg tatagaatgt tggtatagaa    2340
   gttatttttt tttttagaaa tgattattag gtttggtgta gtggtttatg tttgtaattt    2400
   tagtattttg ggaggttgag gtgggtggat tatttgagat taggagtttg agattagttt    2460
   ggttaatgtg gtaaaatttt atttttatta aaaatataaa aaatataaaa atttgttagg    2520
   tgtagtggtg tgtgttggta attttagtta tttgggggtt tgaggtagga gaattgtttg    2580
   aatttgggag gtagaggttg tagtgagtag agattgtatt attgtatttt agttttggtg    2640
   atagagtgag attttgtttt aaaaaaaaga aagaaaaaaa aagaagaaat gattatttat    2700
   tttttggatt tattttatta tattgagttt aaattattta gtttgttttt taaagtttgt    2760
   tatattgagg tttgtatatg gtatttttta ttatttttgt ttttttttta tatatttttt   2820
   attttagtta gatataggaa tgtgtataag gtatgtattt tttttttttt ttttgtttaa    2880
   attggtattt tgtattgttt tgttaggtat atgtttattt gttttttatg ggatagtttt   2940
   gattttgtta tgtggaggtt gtatgtgatt tttgtttggt ttgtgttttt ttagggtttg    3000
   a                                                                    3001
<210> 164
   <211> 3001
   <212> DNA
   <213> human
<400> 164
   ttagattttg ggaaagcgta ggttagataa aggttatatg cgatttttat ataatagggt      60
   taaaattgtt ttatgagaga taaatgggta tgtgtttggt aaagtaatat agggtgttaa     120
   tttagataaa aagagaaaga aaaatgtatg ttttgtatat atttttgtgt ttgattagag     180
   tggagggtat gtaaaagaaa aataagagta atggaaaata ttatatatag gttttaatat     240
   gataggtttt gaaaggtaaa ttgagtaatt tagatttagt gtggtagagt agatttaaag     300
   aataaatggt tatttttttt tttttttttt tttttttttt tgagataggg ttttattttg     360
   ttatcggggt tggaatgtag tggtgtaatt tttgtttatt gtaatttttg ttttttaggt     420
   ttaagtaatt tttttgtttt agatttttaa gtagttggga ttattagcgt acgttattat     480
   atttggtaaa tttttgtatt ttttgtattt ttagtagaga tggagttttg ttatattggt     540
   taggttggtt ttaaattttt gattttaagt gatttattta tttcggtttt ttaaagtgtt     600
   gggattatag gcgtgagtta ttgtattagg tttaatggtt atttttaaaa aggaagatga     660
   tttttgtatt aatattttgt atatataatg aagtggtaat gagtatttgt ttaaagaagg     720
   tagtagtagt aatttagaga gataaatgat aatatgagat tttatgagta ggaagagatg     780
   ataatattag gttattgatt agatagtaga gatagaaaaa ataaaaatag tttttttagg     840
   gtatttatta ataatattgt taattaataa tagtaattag tatttattga tttttggtat     900
   gtgttaggta ttgtattaaa tatttttaat gtgttatcgg atttaatttt tattattaat     960
   ttttgtggta ggtattttaa ttagtttgtt ttagttaaga aaatggaggt ttggagagaa    1020
   taaataattt gtttaaattt ataaaattag taataagtag ggttggaatt tagaagttaa    1080
   tatgtttagt tttacggttt atatttttaa ttattaggac gtatggtttt aacgttttta    1140
   gttgtatgtc gagggtagtg tatattaatt aagtaaggaa gatagttgtg gaaattagcg    1200
   ttgtaggata ttggaaggtt ataagtagag ttatcgttaa gaagaatatt tatttttttt    1260
   tttttttata atgaggtttt tggttttgga gaagttaaat tttgtttaaa gtttaggaga    1320
   atttgttgtt tatgaatatt tttgtaaaag gaagtgtata ggtggttttt gttagtttta    1380
   ttggtattgt gatgtaattt ttgatattat tatagatagt tgtgtgtttt atagggtatt    1440
   aaggggttag agaaaattat attttagtat attgtttttt ttttagtaaa tgtggttgtt    1500
   cgtttttagg gtagtttagt aattttggtt gataaaatag gaatttgagt tttatttata    1560
   gtggatttgg gaatgttttt tttttattaa tttgtgtatt atttaaaaat gtttatagtt    1620
   ggttttggag gaagagcggt taaaattaaa cgtatttatt ttgagatatt taaatattta    1680
   ggatatagtt attgagtaat tgtaaatttg ttatagtaaa ttagggttta ggtggattta    1740
   ttgaattgga gtttattaaa gttataggta gtttagaagt tatttagata gaaatgagag    1800
   ttgaaattaa gataatgggt aaacgtataa ttggggcggt gggggtgagt aagtgggaaa    1860
   aggattgaag atatttttat aattaaagga taggagcgta aagaaaaata gtggtagaaa    1920
   ttaagaagaa attagaaatg gaggaattaa gtttttattt tttagttatt aaattttttt    1980
   ttttatattg ttcggatttg tgttattttt tagtttaatt tttcgttatt gtttgtttgt    2040
   tttgtgtcgg tgattttttt ttaatttgat ttttataata ataatatagt gaattaattg    2100
   aattttatat atgattatga tatgattgga aagttttata gtttgtgttt taatttgagg    2160
   tacggtgtgt ggataggggt aggtattaaa aaaagatatt tttttttggt gtgttagttg    2220
   cgtatagtgg aaagtaattt ttaatttttt atatgaaata taaaatttta taataatgtt    2280
   atgtttgtag tgggttattt tttaatgtga tttagttttt ttttgggaag aggtgtacgt    2340
   tggtggagta atttaaggta ttggttaaaa taggtgaaat ttgtgtttat ttaaattatg    2400
   tgaaggagtt aaagaaagaa agattaaaga tttataaagg ggaggaaatt tttaatatga    2460
   atttaaattt tatagttttt atatattggt ttttgatgta taggagaatg ggtaatttgg    2520
   gtttgaattt tggttttatt agtttttagt tatatgattt taaaggtaaa ttatttaatt    2580
   tttttttgat tttatttttt tatttgtaaa tgtaaatgga atatttaaag agttgaatta    2640
   agtatggata tttagggagg gtttgtataa tatagtattt ggtatattgt aaagaattaa    2700
   gaaatgatcg tttttaattt gtaaatatta agtttatatt attttattaa aatattttaa    2760
   agtttttgtt ttttgtgaga tagagtttta tttttttatt taggttggag tgtagtggcg    2820
   taattttagt ttattgtagt ttttgttttt tgggtttaag ttattttttt gttttagttt    2880
   tttaagtagt tgggattata ggcgttcgtt attacgttta gttaattttt gtatttttag    2940
   tagagacgag gtttttttat gttggttagg ttggttttaa atttttgatt ttaagtgatt    3000
   t                                                                    3001
<210> 165
   <211> 3001
   <212> DNA
   <213> human
<400> 165
   ttagattttg ggaaagtgta ggttagataa aggttatatg tgatttttat ataatagggt      60
   taaaattgtt ttatgagaga taaatgggta tgtgtttggt aaagtaatat agggtgttaa     120
   tttagataaa aagagaaaga aaaatgtatg ttttgtatat atttttgtgt ttgattagag     180
   tggagggtat gtaaaagaaa aataagagta atggaaaata ttatatatag gttttaatat     240
   gataggtttt gaaaggtaaa ttgagtaatt tagatttagt gtggtagagt agatttaaag     300
   aataaatggt tatttttttt tttttttttt tttttttttt tgagataggg ttttattttg     360
   ttattggggt tggaatgtag tggtgtaatt tttgtttatt gtaatttttg ttttttaggt     420
   ttaagtaatt tttttgtttt agatttttaa gtagttggga ttattagtgt atgttattat     480
   atttggtaaa tttttgtatt ttttgtattt ttagtagaga tggagttttg ttatattggt     540
   taggttggtt ttaaattttt gattttaagt gatttattta ttttggtttt ttaaagtgtt     600
   gggattatag gtgtgagtta ttgtattagg tttaatggtt atttttaaaa aggaagatga     660
   tttttgtatt aatattttgt atatataatg aagtggtaat gagtatttgt ttaaagaagg     720
   tagtagtagt aatttagaga gataaatgat aatatgagat tttatgagta ggaagagatg     780
   ataatattag gttattgatt agatagtaga gatagaaaaa ataaaaatag tttttttagg     840
   gtatttatta ataatattgt taattaataa tagtaattag tatttattga tttttggtat     900
   gtgttaggta ttgtattaaa tatttttaat gtgttattgg atttaatttt tattattaat     960
   ttttgtggta ggtattttaa ttagtttgtt ttagttaaga aaatggaggt ttggagagaa    1020
   taaataattt gtttaaattt ataaaattag taataagtag ggttggaatt tagaagttaa    1080
   tatgtttagt tttatggttt atatttttaa ttattaggat gtatggtttt aatgttttta    1140
   gttgtatgtt gagggtagtg tatattaatt aagtaaggaa gatagttgtg gaaattagtg    1200
   ttgtaggata ttggaaggtt ataagtagag ttattgttaa gaagaatatt tatttttttt   1260
   tttttttata atgaggtttt tggttttgga gaagttaaat tttgtttaaa gtttaggaga   1320
   atttgttgtt tatgaatatt tttgtaaaag gaagtgtata ggtggttttt gttagtttta    1380
   ttggtattgt gatgtaattt ttgatattat tatagatagt tgtgtgtttt atagggtatt    1440
   aaggggttag agaaaattat attttagtat attgtttttt ttttagtaaa tgtggttgtt    1500
   tgtttttagg gtagtttagt aattttggtt gataaaatag gaatttgagt tttatttata    1560
   gtggatttgg gaatgttttt tttttattaa tttgtgtatt atttaaaaat gtttatagtt    1620
   ggttttggag gaagagtggt taaaattaaa tgtatttatt ttgagatatt taaatattta    1680
   ggatatagtt attgagtaat tgtaaatttg ttatagtaaa ttagggttta ggtggattta    1740
   ttgaattgga gtttattaaa gttataggta gtttagaagt tatttagata gaaatgagag    1800
   ttgaaattaa gataatgggt aaatgtataa ttggggtggt gggggtgagt aagtgggaaa    1860
   aggattgaag atatttttat aattaaagga taggagtgta aagaaaaata gtggtagaaa    1920
   ttaagaagaa attagaaatg gaggaattaa gttttttatt tttagttatt aaattttttt   1980
   ttttatattg tttggatttg tgttattttt tagtttaatt ttttgttatt gtttgtttgt    2040
   tttgtgttgg tgattttttt ttaatttgat ttttataata ataatatagt gaattaattg    2100
   aattttatat atgattatga tatgattgga aagttttata gtttgtgttt taatttgagg    2160
   tatggtgtgt ggataggggt aggtattaaa aaaagatatt tttttttggt gtgttagttg   2220
   tgtatagtgg aaagtaattt ttaatttttt atatgaaata taaaatttta taataatgtt    2280
   atgtttgtag tgggttattt tttaatgtga tttagttttt ttttgggaag aggtgtatgt    2340
   tggtggagta atttaaggta ttggttaaaa taggtgaaat ttgtgtttat ttaaattatg    2400
   tgaaggagtt aaagaaagaa agattaaaga tttataaagg ggaggaaatt tttaatatga    2460
   atttaaattt tatagttttt atatattggt ttttgatgta taggagaatg ggtaatttgg    2520
   gtttgaattt tggttttatt agtttttagt tatatgattt taaaggtaaa ttatttaatt    2580
   tttttttgat tttatttttt tatttgtaaa tgtaaatgga atatttaaag agttgaatta    2640
   agtatggata tttagggagg gtttgtataa tatagtattt ggtatattgt aaagaattaa    2700
   gaaatgattg tttttaattt gtaaatatta agtttatatt attttattaa aatattttaa    2760
   agtttttgtt ttttgtgaga tagagtttta tttttttatt taggttggag tgtagtggtg    2820
   taattttagt ttattgtagt ttttgttttt tgggtttaag ttattttttt gttttagttt    2880
   tttaagtagt tgggattata ggtgtttgtt attatgttta gttaattttt gtatttttag    2940
   tagagatgag gtttttttat gttggttagg ttggttttaa atttttgatt ttaagtgatt    3000
   t                                                                    3001
<210> 166
   <211> 50
   <212> DNA
   <213> human
<400> 166
   cgccaagggc cgttcagctc aatatgcctg aggttcatac ttggtttctg 50
<210> 167
   <211> 50
   <212> DNA
   <213> human
<400> 167
   cgttaagggt cgtttagttt aatatgtttg aggtttatat ttggtttttg 50
<210> 168
   <211> 50
   <212> DNA
   <213> human
<400> 168
   tgttaagggt tgtttagttt aatatgtttg aggtttatat ttggtttttg 50
<210> 169
   <211> 50
   <212> DNA
   <213> human
<400> 169
   tagaaattaa gtatgaattt taggtatatt gagttgaacg gtttttggcg 50
<210> 170
   <211> 50
   <212> DNA
   <213> human
<400> 170
   tagaaattaa gtatgaattt taggtatatt gagttgaatg gtttttggtg 50
<210> 171
   <211> 3001
   <212> DNA
   <213> human
<400> 171
   tttccaaaac tattctaata cttaaagcaa aaacctctaa tacttaaagc aaaaaccttt 60
   cagcacaaat gcataattta aatctaagat tcaatttata ttcatgctgc cacttccact 120
   ttaaacattt tgaaaccttt actaagatct gacttaagaa aattctattt catagtcacg 180
   tatgttttaa ataaggcttt tatttaactc ttctgctggt tctgtaatct agcaaatttg 240
   gcagtaattt ttaaagctaa ctcatttagg agtgcaaata ctcatttgga aggtcattac   300
   ttaataaaac aactacttgt ctctgcaagg attgccgtag ctacttgcca ggctgttctg   360
   tcaaagttga gaccactgcg attctctttg gtaaataatc aaggaagaga gagagtagag   420
   agtgaaagag agaaagcgag agacctaagt gacccaacgc acacttacct tcaggcttag   480
   gaggtattta caatcaggaa ctgaaatatc acagttgtcc tgtctgaaag gaatctgtcg   540
   tgcattagag aaaacagatg ggttaagtgt gacaatgtcg cactgagcag gagggtgcca   600
   gggaaacaaa attaggtggg gctggctgta tcttgcttgt gtaacagagc gcccaggaac   660
   ctgactgacc tgcttctcag ctgtaagcat taagatatca cctcattcag gcctttaaca   720
   acaacaacaa aagcccaacc ctccagtttt cataggttga tcgttttagc tttgggacaa   780
   ggtattactc tcgtcagtgg tagcaaaaca cgggtcatca attttttttt tcaccctaag   840
   tgaaaaaaag gacaaagatt ttggcaaagt catctggtat agcaggaatt tctgacatgt   900
   gctgggcccc ctgctccttg caggtgagga atccaaacaa gtcatgccct gcacttgttg   960
   ctctatgcca gccgcacatt ctctgaaatc ataaacaatt tttctgtact ctagtagatt  1020
   gccctatttg tgtacttaaa gcctaagtag agaaaatgta gtggggtcag tagtgaggcg  1080
   tctattctga ggtccactga aattgttttt ttaagaaact cctacttaac acaaagtgaa  1140
   ttagtagcat atattgggat ccctgagaac gctgtgaggt gttacatgaa aaataatcat  1200
   catgactgag attcggtttc ttgctgactt tcaggagttt ggagaggaac tggggcctga  1260
   gaatggccat gtgatcctca cttatcctag gagccttctc gattaagtag tttccaccct  1320
   gtgttctctc tggtttactc tcactttaga agctggagaa tttcagaagt gcccaggtaa  1380
   tgcttgatta aatactagag ttgaataaca acttggcata cttcctacac ttcctttcag  1440
   agcttatgaa agggagaaga gtgaaactgg gatcttccgc acatcagcgt ttggggatgg  1500
   cgccaagggc cgttcagctc aatatgcctg aggttcatac ttggtttctg ctgcacaagg  1560
   agataggttt tcaactgctg agcgagaaac gaaactttag ctaaaatgca ggagctagta  1620
   agcttcaaat ggaatgcaaa gaatctgccc agttaattgg cccgaggaaa gaacaaggta  1680
   attctatagt tttaaatttt ctgaagccca aaggcccctt cagagtttga ttagaaatta  1740
   aaccaacccc gaaaaaatat tgtctcaaaa gatatttaag ctgtctctac tgtggtacga  1800
   agaaaagcag gttaaattta atcaaacagt tagttactga acagtggctt aatagaattc  1860
   aatggcttat tttcagatta gccacttttt aggtaaaaat ccaactaaat atattttctg  1920
   tttgtagtta gagaactgaa catgattggg caggactggg tgcacctttt ccctaggcct  1980
   tttctgcaat atgtgtggga atatcattat ccccttctgc cttaaccata gtaattatgg  2040
   cttataccag gcataaacat tgcaatgcac ttttgctata gctctccgtt ttcactgcca  2100
   taaaagtccc gctaatttct catgttgctt tttcctgaag accctttctg tgggcccaga  2160
   atcctattca tcattgccct ccttacaatc ataatcaatt gattagacct caccctagag  2220
   ataaacctta cctaccctca ctgtcttgag cacatctgga aataaactta taaaatgtaa  2280
   actgtacgta aatttaatgc ctcagttgtt acttagctca cttagttgtt atcagaaacc  2340
   tttgtttact catggagttg gagttaaaaa gtaatttcta gccaaacaaa caagtttcac  2400
   tgagatacag tcctaggttc taggtaagac caatcccact ttagacctcc tttctccaga  2460
   ggaactccac aatgagagat aattacctgc aatttcatca gcaaacccag ctgagatgac  2520
   acactgtgca gtgcttgtat aatttttttt ggtacttacc cactatggtg cctttcatct  2580
   ggcactcacc aattgcttta cactcattaa ttacgtttca caatacatac tgggaggaag  2640
   aaaaacctct tcctagtctg caatgataga accactcaca agtggagttt ccagaacacg  2700
   aagacacatg aatatcacta atggatctga atacagaagc agacatagct tgggaccaca  2760
   cttcaaacta attgaaaaca ttgttttgaa tccaaaagag tatctttttg tacaaatcct  2820
   atagcatcaa atcttgatgc tgatgtcaca tgtagggaat ttgcattgat cagattcact  2880
   gatagcatat ttagtctgat acaggctaca agtggaattc atgtttgttt ccccataggg  2940
   acataggtac tttaagcaaa taagaaatat ttgaataata gtaaaataaa ttacattatg  3000
   a                                                                  3001
<210> 172
   <211> 3001
   <212> DNA
   <213> human
<400> 172
   tttttaaaat tattttaata tttaaagtaa aaatttttaa tatttaaagt aaaaattttt 60
   tagtataaat gtataattta aatttaagat ttaatttata tttatgttgt tatttttatt 120
   ttaaatattt tgaaattttt attaagattt gatttaagaa aattttattt tatagttacg 180
   tatgttttaa ataaggtttt tatttaattt ttttgttggt tttgtaattt agtaaatttg 240
   gtagtaattt ttaaagttaa tttatttagg agtgtaaata tttatttgga aggttattat 300
   ttaataaaat aattatttgt ttttgtaagg attgtcgtag ttatttgtta ggttgttttg 360
   ttaaagttga gattattgcg attttttttg gtaaataatt aaggaagaga gagagtagag 420
   agtgaaagag agaaagcgag agatttaagt gatttaacgt atatttattt ttaggtttag 480
   gaggtattta taattaggaa ttgaaatatt atagttgttt tgtttgaaag gaatttgtcg 540
   tgtattagag aaaatagatg ggttaagtgt gataatgtcg tattgagtag gagggtgtta 600
   gggaaataaa attaggtggg gttggttgta ttttgtttgt gtaatagagc gtttaggaat 660
   ttgattgatt tgttttttag ttgtaagtat taagatatta ttttatttag gtttttaata 720
   ataataataa aagtttaatt ttttagtttt tataggttga tcgttttagt tttgggataa 780
   ggtattattt tcgttagtgg tagtaaaata cgggttatta attttttttt ttattttaag 840
   tgaaaaaaag gataaagatt ttggtaaagt tatttggtat agtaggaatt tttgatatgt 900
   gttgggtttt ttgttttttg taggtgagga atttaaataa gttatgtttt gtatttgttg 960
   ttttatgtta gtcgtatatt ttttgaaatt ataaataatt tttttgtatt ttagtagatt 1020
   gttttatttg tgtatttaaa gtttaagtag agaaaatgta gtggggttag tagtgaggcg 1080
   tttattttga ggtttattga aattgttttt ttaagaaatt tttatttaat ataaagtgaa 1140
   ttagtagtat atattgggat ttttgagaac gttgtgaggt gttatatgaa aaataattat 1200
   tatgattgag attcggtttt ttgttgattt ttaggagttt ggagaggaat tggggtttga 1260
   gaatggttat gtgattttta tttattttag gagttttttc gattaagtag tttttatttt 1320
   gtgttttttt tggtttattt ttattttaga agttggagaa ttttagaagt gtttaggtaa 1380
   tgtttgatta aatattagag ttgaataata atttggtata ttttttatat ttttttttag 1440
   agtttatgaa agggagaaga gtgaaattgg gatttttcgt atattagcgt ttggggatgg 1500
   cgttaagggt cgtttagttt aatatgtttg aggtttatat ttggtttttg ttgtataagg 1560
   agataggttt ttaattgttg agcgagaaac gaaattttag ttaaaatgta ggagttagta 1620
   agttttaaat ggaatgtaaa gaatttgttt agttaattgg ttcgaggaaa gaataaggta 1680
   attttatagt tttaaatttt ttgaagttta aaggtttttt tagagtttga ttagaaatta 1740
   aattaatttc gaaaaaatat tgttttaaaa gatatttaag ttgtttttat tgtggtacga 1800
   agaaaagtag gttaaattta attaaatagt tagttattga atagtggttt aatagaattt 1860
   aatggtttat ttttagatta gttatttttt aggtaaaaat ttaattaaat atattttttg 1920
   tttgtagtta gagaattgaa tatgattggg taggattggg tgtatttttt ttttaggttt 1980
   tttttgtaat atgtgtggga atattattat ttttttttgt tttaattata gtaattatgg 2040
   tttatattag gtataaatat tgtaatgtat ttttgttata gtttttcgtt tttattgtta 2100
   taaaagtttc gttaattttt tatgttgttt ttttttgaag attttttttg tgggtttaga 2160
   attttattta ttattgtttt ttttataatt ataattaatt gattagattt tattttagag 2220
   ataaatttta tttattttta ttgttttgag tatatttgga aataaattta taaaatgtaa 2280
   attgtacgta aatttaatgt tttagttgtt atttagttta tttagttgtt attagaaatt 2340
   tttgtttatt tatggagttg gagttaaaaa gtaattttta gttaaataaa taagttttat 2400
   tgagatatag ttttaggttt taggtaagat taattttatt ttagattttt tttttttaga 2460
   ggaattttat aatgagagat aattatttgt aattttatta gtaaatttag ttgagatgat 2520
   atattgtgta gtgtttgtat aatttttttt ggtatttatt tattatggtg ttttttattt 2580
   ggtatttatt aattgtttta tatttattaa ttacgtttta taatatatat tgggaggaag 2640
   aaaaattttt ttttagtttg taatgataga attatttata agtggagttt ttagaatacg 2700
   aagatatatg aatattatta atggatttga atatagaagt agatatagtt tgggattata 2760
   ttttaaatta attgaaaata ttgttttgaa tttaaaagag tatttttttg tataaatttt 2820
   atagtattaa attttgatgt tgatgttata tgtagggaat ttgtattgat tagatttatt 2880
   gatagtatat ttagtttgat ataggttata agtggaattt atgtttgttt ttttataggg 2940
   atataggtat tttaagtaaa taagaaatat ttgaataata gtaaaataaa ttatattatg 3000
   a 3001
<210> 173
   <211> 3001
   <212> DNA
   <213> human
<400> 173
   tttttaaaat tattttaata tttaaagtaa aaatttttaa tatttaaagt aaaaattttt 60
   tagtataaat gtataattta aatttaagat ttaatttata tttatgttgt tatttttatt 120
   ttaaatattt tgaaattttt attaagattt gatttaagaa aattttattt tatagttatg 180
   tatgttttaa ataaggtttt tatttaattt ttttgttggt tttgtaattt agtaaatttg 240
   gtagtaattt ttaaagttaa tttatttagg agtgtaaata tttatttgga aggttattat 300
   ttaataaaat aattatttgt ttttgtaagg attgttgtag ttatttgtta ggttgttttg 360
   ttaaagttga gattattgtg attttttttg gtaaataatt aaggaagaga gagagtagag 420
   agtgaaagag agaaagtgag agatttaagt gatttaatgt atatttattt ttaggtttag 480
   gaggtattta taattaggaa ttgaaatatt atagttgttt tgtttgaaag gaatttgttg 540
   tgtattagag aaaatagatg ggttaagtgt gataatgttg tattgagtag gagggtgtta 600
   gggaaataaa attaggtggg gttggttgta ttttgtttgt gtaatagagt gtttaggaat 660
   ttgattgatt tgttttttag ttgtaagtat taagatatta ttttatttag gtttttaata 720
   ataataataa aagtttaatt ttttagtttt tataggttga ttgttttagt tttgggataa 780
   ggtattattt ttgttagtgg tagtaaaata tgggttatta attttttttt ttattttaag 840
   tgaaaaaaag gataaagatt ttggtaaagt tatttggtat agtaggaatt tttgatatgt 900
   gttgggtttt ttgttttttg taggtgagga atttaaataa gttatgtttt gtatttgttg 960
   ttttatgtta gttgtatatt ttttgaaatt ataaataatt tttttgtatt ttagtagatt 1020
   gttttatttg tgtatttaaa gtttaagtag agaaaatgta gtggggttag tagtgaggtg 1080
   tttattttga ggtttattga aattgttttt ttaagaaatt tttatttaat ataaagtgaa 1140
   ttagtagtat atattgggat ttttgagaat gttgtgaggt gttatatgaa aaataattat 1200
   tatgattgag atttggtttt ttgttgattt ttaggagttt ggagaggaat tggggtttga 1260
   gaatggttat gtgattttta tttattttag gagttttttt gattaagtag tttttatttt 1320
   gtgttttttt tggtttattt ttattttaga agttggagaa ttttagaagt gtttaggtaa 1380
   tgtttgatta aatattagag ttgaataata atttggtata ttttttatat ttttttttag 1440
   agtttatgaa agggagaaga gtgaaattgg gattttttgt atattagtgt ttggggatgg 1500
   tgttaagggt tgtttagttt aatatgtttg aggtttatat ttggtttttg ttgtataagg 1560
   agataggttt ttaattgttg agtgagaaat gaaattttag ttaaaatgta ggagttagta 1620
   agttttaaat ggaatgtaaa gaatttgttt agttaattgg tttgaggaaa gaataaggta 1680
   attttatagt tttaaatttt ttgaagttta aaggtttttt tagagtttga ttagaaatta 1740
   aattaatttt gaaaaaatat tgttttaaaa gatatttaag ttgtttttat tgtggtatga 1800
   agaaaagtag gttaaattta attaaatagt tagttattga atagtggttt aatagaattt 1860
   aatggtttat ttttagatta gttatttttt aggtaaaaat ttaattaaat atattttttg 1920
   tttgtagtta gagaattgaa tatgattggg taggattggg tgtatttttt ttttaggttt 1980
   tttttgtaat atgtgtggga atattattat ttttttttgt tttaattata gtaattatgg 2040
   tttatattag gtataaatat tgtaatgtat ttttgttata gttttttgtt tttattgtta 2100
   taaaagtttt gttaattttt tatgttgttt ttttttgaag attttttttg tgggtttaga 2160
   attttattta ttattgtttt ttttataatt ataattaatt gattagattt tattttagag 2220
   ataaatttta tttattttta ttgttttgag tatatttgga aataaattta taaaatgtaa 2280
   attgtatgta aatttaatgt tttagttgtt atttagttta tttagttgtt attagaaatt 2340
   tttgtttatt tatggagttg gagttaaaaa gtaattttta gttaaataaa taagttttat 2400
   tgagatatag ttttaggttt taggtaagat taattttatt ttagattttt tttttttaga 2460
   ggaattttat aatgagagat aattatttgt aattttatta gtaaatttag ttgagatgat 2520
   atattgtgta gtgtttgtat aatttttttt ggtatttatt tattatggtg ttttttattt 2580
   ggtatttatt aattgtttta tatttattaa ttatgtttta taatatatat tgggaggaag 2640
   aaaaattttt ttttagtttg taatgataga attatttata agtggagttt ttagaatatg 2700
   aagatatatg aatattatta atggatttga atatagaagt agatatagtt tgggattata 2760
   ttttaaatta attgaaaata ttgttttgaa tttaaaagag tatttttttg tataaatttt 2820
   atagtattaa attttgatgt tgatgttata tgtagggaat ttgtattgat tagatttatt 2880
   gatagtatat ttagtttgat ataggttata agtggaattt atgtttgttt ttttataggg 2940
   atataggtat tttaagtaaa taagaaatat ttgaataata gtaaaataaa ttatattatg 3000
   a 3001
<210> 174
   <211> 3001
   <212> DNA
   <213> human
<400> 174
   ttataatgta atttatttta ttattattta aatatttttt atttgtttaa agtatttatg 60
   tttttatggg gaaataaata tgaattttat ttgtagtttg tattagatta aatatgttat 120
   tagtgaattt gattaatgta aattttttat atgtgatatt agtattaaga tttgatgtta 180
   taggatttgt ataaaaagat atttttttgg atttaaaata atgtttttaa ttagtttgaa 240
   gtgtggtttt aagttatgtt tgtttttgta tttagattta ttagtgatat ttatgtgttt 300
   tcgtgttttg gaaattttat ttgtgagtgg ttttattatt gtagattagg aagaggtttt 360
   tttttttttt agtatgtatt gtgaaacgta attaatgagt gtaaagtaat tggtgagtgt 420
   tagatgaaag gtattatagt gggtaagtat taaaaaaaat tatataagta ttgtatagtg 480
   tgttatttta gttgggtttg ttgatgaaat tgtaggtaat tattttttat tgtggagttt 540
   ttttggagaa aggaggttta aagtgggatt ggttttattt agaatttagg attgtatttt 600
   agtgaaattt gtttgtttgg ttagaaatta ttttttaatt ttaattttat gagtaaataa 660
   aggtttttga taataattaa gtgagttaag taataattga ggtattaaat ttacgtatag 720
   tttatatttt ataagtttat ttttagatgt gtttaagata gtgagggtag gtaaggttta 780
   tttttagggt gaggtttaat taattgatta tgattgtaag gagggtaatg atgaatagga 840
   ttttgggttt atagaaaggg tttttaggaa aaagtaatat gagaaattag cgggattttt 900
   atggtagtga aaacggagag ttatagtaaa agtgtattgt aatgtttatg tttggtataa 960
   gttataatta ttatggttaa ggtagaaggg gataatgata tttttatata tattgtagaa 1020
   aaggtttagg gaaaaggtgt atttagtttt gtttaattat gtttagtttt ttaattataa 1080
   atagaaaata tatttagttg gatttttatt taaaaagtgg ttaatttgaa aataagttat 1140
   tgaattttat taagttattg tttagtaatt aattgtttga ttaaatttaa tttgtttttt 1200
   ttcgtattat agtagagata gtttaaatat tttttgagat aatatttttt cggggttggt 1260
   ttaattttta attaaatttt gaagggtttt ttgggtttta gaaaatttaa aattatagaa 1320
   ttattttgtt tttttttcgg gttaattaat tgggtagatt ttttgtattt tatttgaagt 1380
   ttattagttt ttgtatttta gttaaagttt cgtttttcgt ttagtagttg aaaatttatt 1440
   tttttgtgta gtagaaatta agtatgaatt ttaggtatat tgagttgaac ggtttttggc 1500
   gttattttta aacgttgatg tgcggaagat tttagtttta tttttttttt ttttataagt 1560
   tttgaaagga agtgtaggaa gtatgttaag ttgttattta attttagtat ttaattaagt 1620
   attatttggg tatttttgaa attttttagt ttttaaagtg agagtaaatt agagagaata 1680
   tagggtggaa attatttaat cgagaaggtt tttaggataa gtgaggatta tatggttatt 1740
   tttaggtttt agtttttttt taaatttttg aaagttagta agaaatcgaa ttttagttat 1800
   gatgattatt ttttatgtaa tattttatag cgtttttagg gattttaata tatgttatta 1860
   atttattttg tgttaagtag gagtttttta aaaaaataat tttagtggat tttagaatag 1920
   acgttttatt attgatttta ttatattttt tttatttagg ttttaagtat ataaataggg 1980
   taatttatta gagtatagaa aaattgttta tgattttaga gaatgtgcgg ttggtataga 2040
   gtaataagtg tagggtatga tttgtttgga ttttttattt gtaaggagta gggggtttag 2100
   tatatgttag aaatttttgt tatattagat gattttgtta aaatttttgt tttttttttt 2160
   atttagggtg aaaaaaaaaa ttgatgattc gtgttttgtt attattgacg agagtaatat 2220
   tttgttttaa agttaaaacg attaatttat gaaaattgga gggttgggtt tttgttgttg 2280
   ttgttaaagg tttgaatgag gtgatatttt aatgtttata gttgagaagt aggttagtta 2340
   ggtttttggg cgttttgtta tataagtaag atatagttag ttttatttaa ttttgttttt 2400
   ttggtatttt tttgtttagt gcgatattgt tatatttaat ttatttgttt tttttaatgt 2460
   acgatagatt ttttttagat aggataattg tgatatttta gtttttgatt gtaaatattt 2520
   tttaagtttg aaggtaagtg tgcgttgggt tatttaggtt tttcgttttt ttttttttat 2580
   tttttatttt tttttttttt tgattattta ttaaagagaa tcgtagtggt tttaattttg 2640
   atagaatagt ttggtaagta gttacggtaa tttttgtaga gataagtagt tgttttatta 2700
   agtaatgatt ttttaaatga gtatttgtat ttttaaatga gttagtttta aaaattattg 2760
   ttaaatttgt tagattatag aattagtaga agagttaaat aaaagtttta tttaaaatat 2820
   acgtgattat gaaatagaat ttttttaagt tagattttag taaaggtttt aaaatgttta 2880
   aagtggaagt ggtagtatga atataaattg aattttagat ttaaattatg tatttgtgtt 2940
   gaaaggtttt tgttttaagt attagaggtt tttgttttaa gtattagaat agttttggaa 3000
   a 3001

<210> 175
   <211> 3001
   <212> DNA
   <213> human
<400> 175
   ttataatgta atttatttta ttattattta aatatttttt atttgtttaa agtatttatg 60
   tttttatggg gaaataaata tgaattttat ttgtagtttg tattagatta aatatgttat 120
   tagtgaattt gattaatgta aattttttat atgtgatatt agtattaaga tttgatgtta 180
   taggatttgt ataaaaagat atttttttgg atttaaaata atgtttttaa ttagtttgaa 240
   gtgtggtttt aagttatgtt tgtttttgta tttagattta ttagtgatat ttatgtgttt 300
   ttgtgttttg gaaattttat ttgtgagtgg ttttattatt gtagattagg aagaggtttt 360
   tttttttttt agtatgtatt gtgaaatgta attaatgagt gtaaagtaat tggtgagtgt 420
   tagatgaaag gtattatagt gggtaagtat taaaaaaaat tatataagta ttgtatagtg 480
   tgttatttta gttgggtttg ttgatgaaat tgtaggtaat tattttttat tgtggagttt 540
   ttttggagaa aggaggttta aagtgggatt ggttttattt agaatttagg attgtatttt 600
   agtgaaattt gtttgtttgg ttagaaatta ttttttaatt ttaattttat gagtaaataa 660
   aggtttttga taataattaa gtgagttaag taataattga ggtattaaat ttatgtatag 720
   tttatatttt ataagtttat ttttagatgt gtttaagata gtgagggtag gtaaggttta 780
   tttttagggt gaggtttaat taattgatta tgattgtaag gagggtaatg atgaatagga 840
   ttttgggttt atagaaaggg tttttaggaa aaagtaatat gagaaattag tgggattttt 900
   atggtagtga aaatggagag ttatagtaaa agtgtattgt aatgtttatg tttggtataa 960
   gttataatta ttatggttaa ggtagaaggg gataatgata tttttatata tattgtagaa 1020
   aaggtttagg gaaaaggtgt atttagtttt gtttaattat gtttagtttt ttaattataa 1080
   atagaaaata tatttagttg gatttttatt taaaaagtgg ttaatttgaa aataagttat 1140
   tgaattttat taagttattg tttagtaatt aattgtttga ttaaatttaa tttgtttttt 1200
   tttgtattat agtagagata gtttaaatat tttttgagat aatatttttt tggggttggt 1260
   ttaattttta attaaatttt gaaggggttt ttgggtttta gaaaatttaa aattatagaa 1320
   ttattttgtt ttttttttgg gttaattaat tgggtagatt ttttgtattt tatttgaagt 1380
   ttattagttt ttgtatttta gttaaagttt tgttttttgt ttagtagttg aaaatttatt 1440
   tttttgtgta gtagaaatta agtatgaatt ttaggtatat tgagttgaat ggtttttggt 1500
   gttattttta aatgttgatg tgtggaagat tttagtttta tttttttttt ttttataagt 1560
   tttgaaagga agtgtaggaa gtatgttaag ttgttattta attttagtat ttaattaagt 1620
   attatttggg tatttttgaa attttttagt ttttaaagtg agagtaaatt agagagaata 1680
   tagggtggaa attatttaat tgagaaggtt tttaggataa gtgaggatta tatggttatt 1740
   tttaggtttt agtttttttt taaatttttg aaagttagta agaaattgaa ttttagttat 1800
   gatgattatt ttttatgtaa tattttatag tgtttttagg gattttaata tatgttatta 1860
   atttattttg tgttaagtag gagtttttta aaaaaataat tttagtggat tttagaatag 1920
   atgttttatt attgatttta ttatattttt tttatttagg ttttaagtat ataaataggg 1980
   taatttatta gagtatagaa aaattgttta tgattttaga gaatgtgtgg ttggtataga 2040
   gtaataagtg tagggtatga tttgtttgga ttttttattt gtaaggagta gggggtttag 2100
   tatatgttag aaatttttgt tatattagat gattttgtta aaatttttgt tttttttttt 2160
   atttagggtg aaaaaaaaaa ttgatgattt gtgttttgtt attattgatg agagtaatat 2220
   tttgttttaa agttaaaatg attaatttat gaaaattgga gggttgggtt tttgttgttg 2280
   ttgttaaagg tttgaatgag gtgatatttt aatgtttata gttgagaagt aggttagtta 2340
   ggtttttggg tgttttgtta tataagtaag atatagttag ttttatttaa ttttgttttt 2400
   ttggtatttt tttgtttagt gtgatattgt tatatttaat ttatttgttt tttttaatgt 2460
   atgatagatt ttttttagat aggataattg tgatatttta gtttttgatt gtaaatattt 2520
   tttaagtttg aaggtaagtg tgtgttgggt tatttaggtt ttttgttttt ttttttttat 2580
   tttttatttt tttttttttt tgattattta ttaaagagaa ttgtagtggt tttaattttg 2640
   atagaatagt ttggtaagta gttatggtaa tttttgtaga gataagtagt tgttttatta 2700
   agtaatgatt ttttaaatga gtatttgtat ttttaaatga gttagtttta aaaattattg 2760
   ttaaatttgt tagattatag aattagtaga agagttaaat aaaagtttta tttaaaatat 2820
   atgtgattat gaaatagaat ttttttaagt tagattttag taaaggtttt aaaatgttta 2880
   aagtggaagt ggtagtatga atataaattg aattttagat ttaaattatg tatttgtgtt 2940
   gaaaggtttt tgttttaagt attagaggtt tttgttttaa gtattagaat agttttggaa 3000
   a 3001
<210> 176
   <211> 50
   <212> DNA
   <213> human
<400> 176
   cgccagtccc tggcgggacc tatagatgct atggttcctt caatgactct 50
<210> 177
   <211> 50
   <212> DNA
   <213> human
<400> 177
   cgttagtttt tggcgggatt tatagatgtt atggtttttt taatgatttt 50
<210> 178
   <211> 50
   <212> DNA
   <213> human
<400> 178
   tgttagtttt tggtgggatt tatagatgtt atggtttttt taatgatttt 50
<210> 179
   <211> 50
   <212> DNA
   <213> human
<400> 179
   agagttattg aaggaattat agtatttata ggtttcgtta gggattggcg 50
<210> 180
   <211> 50
   <212> DNA
   <213> human
<400> 180
   agagttattg aaggaattat agtatttata ggttttgtta gggattggtg 50
<210> 181
   <211> 3001
   <212> DNA
   <213> human
<400> 181
   tgtctctgct ctcaggcttg ttcacaaaac cctccatctc agcgcaccca agctccctgg      60
   tgcatgcagg agccagggtg agcctgcgct gtcactcaga actggccttt gatgaattta     120
   tcttatacaa agaggggcac atacagcatt cccagcagct tgaccagggg atggaggctg     180
   ggatccatta cgtcgaggct gtcttttcca tgggtcctgt aacgcctgcc catgcaggag     240
   cctacagatg ctgtggttgt ttcagtcact cccgctatga gtggtcggct cccagtgacc     300
   ccctggacat tgtgatcaca ggtgagtgtg gctggaccat ccgtggtctg ttggtgccat     360
   gggagcttca tgtaatgcgg ttgttaatca aacctcagta gaggaagaga aataaaggaa     420
   gagagggact gtgaaaaagt gctcagcaaa ggggtttaac atgtcttact gcttaaagag     480
   gcaggagggg tcacttctac cctccccaac aagtagtcac gtctaactcc ttgggcttgg     540
   gggtacagcc agttgttccc tgcagtggga tcctgtgagg ttccttctga tcctcccgag     600
   attcttatat ttttcaacaa acacaattga aaaatagtaa gactcataac cccgtagccc     660
   catgtcagag aactcaaggc cagcactatg ttttctgaag ggtctcaaag cacagagtaa     720
   ttcccatttt ccaggagaac cccacatctc atatggcttc taggggcggt aaaccacagg     780
   gactcagtca aaagcagaat caaagaaatg cagaggaagt aaaaagacac agacataggc     840
   tgactcagga ccctagagat gaacatgaga ttgacagggg caccagccga cattcggaat     900
   ctgctgccaa gataagaaca gcgaggctgg gcgctgtggc ttacgcctgt aatcccagca     960
   ctttgggagg ctgacgtggg cggatcacaa ggtcaggagt tcgagaccag cctgaccaac    1020
   atggtgaaac cctgtctcta ctaaaaatac aaaaattagc tgggcatggt ggtccgtgcc    1080
   tctaatccca gctactcagg aggctaaggc aggagaatca cttgaacccg ggatgctgag    1140
   gttgcagtga gccgagatcg tgctactgca ctccagccta ggagacaaga gtgaaaatct    1200
   gtctcaaagg aaaaaaaaaa aaaaaaaaaa agagggagag agattacttt gttcatacac    1260
   tcaggcgtgt gttctaaaat cagcctcttt ttctcctagg aaaatacaaa aagccttctc    1320
   tctccaccca ggtggacccc atgatgaggc tgggagagaa gttgaccctc ttctgcagct    1380
   ctgaaatctc atttgaccag taccatctgt tcagacacgg ggttgctcat ggacagtggc    1440
   tcagtggagg gcagagacac agggaagcat tccaggccaa cttttctgtg ggccgtgcaa    1500
   cgccagtccc tggcgggacc tatagatgct atggttcctt caatgactct ccctataagc    1560
   ccccagtgac ccgctgcaac tttacaccac aggtgaggag cccatgcctg ctgcatgctc    1620
   tggtgcccac tggatcacag agccacacgt gaggggcgtc ccgctgggca cacaggggta    1680
   ttaggtactc tggataaaat gaaacagtga caaacacaca caggaaaaaa gaagctgagc    1740
   atgatggggc tgtcagggtg tagggtggta agacggggca gctccacacc ctctgccacc    1800
   ttctgtatga agaaagaggt caggacaaac acagggggag gtgaggccag atgtagtttg    1860
   tcgaggtcag aggttgcccc tcaccctttt ccatttctcc aaagcccctc ctgacccctc    1920
   aacacagaga gatctccctg ctggggaacg tggagattta tcatcccgat gggagacaat    1980
   gtctgttgac atcacccacc atgcttctcc ttatcacctt cccatcccag ggaaggaatt    2040
   gtcccccaga gattccaagg aagagacccc agggccccca ttagtcttta ggtggatgac    2100
   agagtagggg gtgtgcaggg accaagcctc caaagaaaat ggtgctaatg ctcgggaggc    2160
   tgaggcagga gaatcgcttg aacccaggag gtggaggttg cagcgagaca agatggcacc    2220
   actgcactcc agcctgggcg acagagcaag actccatctc aaaaaaaaaa aaaaaagaaa    2280
   gaaagaaaga aagaaagaaa aagaaagaaa gaaagaaaga aaagaaaatg gtcctacttt    2340
   aatggggcaa tcaatgacgc ttgctcaccc cttctctgcc tttttttttc ctaggaaaca    2400
   ctaagagtac tcctctgtca ttcacagaat ccacccctga atctggtaag caaataactc    2460
   ttatcctagt gtctgagtcc ctggggagac agaaggcccc agtgtgagtg aaagctgtgc    2520
   cacctcccag ctccatgacc ctgggctagg cagcccctcc caggtcccca cattccccat    2580
   ccacatctga gactgtggtc agtgcgggaa tctgtaaggc ctttcagcct cagacgcttt    2640
   gggactgagg cctcatccac aggggaggaa gaggtcagag tcacctgacc cttgctgaaa    2700
   agcaatgctt ctcattcctc cagggaggtc tgtgaacatg aaaatgctgg aagatgggaa    2760
   ggatttaaaa gaccatattc gatattcact gtctgtcttt ttttgttgtt tttttttttg    2820
   agacagagtc tcactctgtc acccaggctg gagtgcggtg gcacaatctc agctcactgc    2880
   aacctctgcc tcctgggttc aagcgatttt cctgcctcag cctcccgagt agctgggatt    2940
   acaggtgccc accaccacac ccggctaatt tttgtatttt tagtagagtc agggtttcgc    3000
   c                                                                    3001
<210> 182
   <211> 3001
   <212> DNA
   <213> human
<400> 182
   tgtttttgtt tttaggtttg tttataaaat tttttatttt agcgtattta agttttttgg 60
   tgtatgtagg agttagggtg agtttgcgtt gttatttaga attggttttt gatgaattta 120
   ttttatataa agaggggtat atatagtatt tttagtagtt tgattagggg atggaggttg 180
   ggatttatta cgtcgaggtt gtttttttta tgggttttgt aacgtttgtt tatgtaggag 240
   tttatagatg ttgtggttgt tttagttatt ttcgttatga gtggtcggtt tttagtgatt 300
   ttttggatat tgtgattata ggtgagtgtg gttggattat tcgtggtttg ttggtgttat 360
   gggagtttta tgtaatgcgg ttgttaatta aattttagta gaggaagaga aataaaggaa 420
   gagagggatt gtgaaaaagt gtttagtaaa ggggtttaat atgttttatt gtttaaagag 480
   gtaggagggg ttatttttat tttttttaat aagtagttac gtttaatttt ttgggtttgg 540
   gggtatagtt agttgttttt tgtagtggga ttttgtgagg ttttttttga ttttttcgag 600
   atttttatat tttttaataa atataattga aaaatagtaa gatttataat ttcgtagttt 660
   tatgttagag aatttaaggt tagtattatg ttttttgaag ggttttaaag tatagagtaa 720
   tttttatttt ttaggagaat tttatatttt atatggtttt taggggcggt aaattatagg 780
   gatttagtta aaagtagaat taaagaaatg tagaggaagt aaaaagatat agatataggt 840
   tgatttagga ttttagagat gaatatgaga ttgatagggg tattagtcga tattcggaat 900
   ttgttgttaa gataagaata gcgaggttgg gcgttgtggt ttacgtttgt aattttagta 960
   ttttgggagg ttgacgtggg cggattataa ggttaggagt tcgagattag tttgattaat 1020
   atggtgaaat tttgttttta ttaaaaatat aaaaattagt tgggtatggt ggttcgtgtt 1080
   tttaatttta gttatttagg aggttaaggt aggagaatta tttgaattcg ggatgttgag 1140
   gttgtagtga gtcgagatcg tgttattgta ttttagttta ggagataaga gtgaaaattt 1200
   gttttaaagg aaaaaaaaaa aaaaaaaaaa agagggagag agattatttt gtttatatat 1260
   ttaggcgtgt gttttaaaat tagttttttt tttttttagg aaaatataaa aagttttttt 1320
   tttttattta ggtggatttt atgatgaggt tgggagagaa gttgattttt ttttgtagtt 1380
   ttgaaatttt atttgattag tattatttgt ttagatacgg ggttgtttat ggatagtggt 1440
   ttagtggagg gtagagatat agggaagtat tttaggttaa tttttttgtg ggtcgtgtaa 1500
   cgttagtttt tggcgggatt tatagatgtt atggtttttt taatgatttt ttttataagt 1560
   ttttagtgat tcgttgtaat tttatattat aggtgaggag tttatgtttg ttgtatgttt 1620
   tggtgtttat tggattatag agttatacgt gaggggcgtt tcgttgggta tataggggta 1680
   ttaggtattt tggataaaat gaaatagtga taaatatata taggaaaaaa gaagttgagt 1740
   atgatggggt tgttagggtg tagggtggta agacggggta gttttatatt ttttgttatt 1800
   ttttgtatga agaaagaggt taggataaat atagggggag gtgaggttag atgtagtttg 1860
   tcgaggttag aggttgtttt ttattttttt ttattttttt aaagtttttt ttgatttttt 1920
   aatatagaga gatttttttg ttggggaacg tggagattta ttatttcgat gggagataat 1980
   gtttgttgat attatttatt atgttttttt ttattatttt tttattttag ggaaggaatt 2040
   gttttttaga gattttaagg aagagatttt agggttttta ttagttttta ggtggatgat 2100
   agagtagggg gtgtgtaggg attaagtttt taaagaaaat ggtgttaatg ttcgggaggt 2160
   tgaggtagga gaatcgtttg aatttaggag gtggaggttg tagcgagata agatggtatt 2220
   attgtatttt agtttgggcg atagagtaag attttatttt aaaaaaaaaa aaaaaagaaa 2280
   gaaagaaaga aagaaagaaa aagaaagaaa gaaagaaaga aaagaaaatg gttttatttt 2340
   aatggggtaa ttaatgacgt ttgtttattt tttttttgtt tttttttttt ttaggaaata 2400
   ttaagagtat ttttttgtta tttatagaat ttatttttga atttggtaag taaataattt 2460
   ttattttagt gtttgagttt ttggggagat agaaggtttt agtgtgagtg aaagttgtgt 2520
   tattttttag ttttatgatt ttgggttagg tagttttttt taggttttta tattttttat 2580
   ttatatttga gattgtggtt agtgcgggaa tttgtaaggt tttttagttt tagacgtttt 2640
   gggattgagg ttttatttat aggggaggaa gaggttagag ttatttgatt tttgttgaaa 2700
   agtaatgttt tttatttttt tagggaggtt tgtgaatatg aaaatgttgg aagatgggaa 2760
   ggatttaaaa gattatattc gatatttatt gtttgttttt ttttgttgtt tttttttttg 2820
   agatagagtt ttattttgtt atttaggttg gagtgcggtg gtataatttt agtttattgt 2880
   aatttttgtt ttttgggttt aagcgatttt tttgttttag tttttcgagt agttgggatt 2940
   ataggtgttt attattatat tcggttaatt tttgtatttt tagtagagtt agggtttcgt 3000
   t 3001
<210> 183
   <211> 3001
   <212> DNA
   <213> human
<400> 183
   tgtttttgtt tttaggtttg tttataaaat tttttatttt agtgtattta agttttttgg 60
   tgtatgtagg agttagggtg agtttgtgtt gttatttaga attggttttt gatgaattta 120
   ttttatataa agaggggtat atatagtatt tttagtagtt tgattagggg atggaggttg 180
   ggatttatta tgttgaggtt gtttttttta tgggttttgt aatgtttgtt tatgtaggag 240
   tttatagatg ttgtggttgt tttagttatt tttgttatga gtggttggtt tttagtgatt 300
   ttttggatat tgtgattata ggtgagtgtg gttggattat ttgtggtttg ttggtgttat 360
   gggagtttta tgtaatgtgg ttgttaatta aattttagta gaggaagaga aataaaggaa 420
   gagagggatt gtgaaaaagt gtttagtaaa ggggtttaat atgttttatt gtttaaagag 480
   gtaggagggg ttatttttat tttttttaat aagtagttat gtttaatttt ttgggtttgg 540
   gggtatagtt agttgttttt tgtagtggga ttttgtgagg ttttttttga tttttttgag 600
   atttttatat tttttaataa atataattga aaaatagtaa gatttataat tttgtagttt 660
   tatgttagag aatttaaggt tagtattatg ttttttgaag ggttttaaag tatagagtaa 720
   tttttatttt ttaggagaat tttatatttt atatggtttt taggggtggt aaattatagg 780
   gatttagtta aaagtagaat taaagaaatg tagaggaagt aaaaagatat agatataggt 840
   tgatttagga ttttagagat gaatatgaga ttgatagggg tattagttga tatttggaat 900
   ttgttgttaa gataagaata gtgaggttgg gtgttgtggt ttatgtttgt aattttagta 960
   ttttgggagg ttgatgtggg tggattataa ggttaggagt ttgagattag tttgattaat 1020
   atggtgaaat tttgttttta ttaaaaatat aaaaattagt tgggtatggt ggtttgtgtt 1080
   tttaatttta gttatttagg aggttaaggt aggagaatta tttgaatttg ggatgttgag 1140
   gttgtagtga gttgagattg tgttattgta ttttagttta ggagataaga gtgaaaattt 1200
   gttttaaagg aaaaaaaaaa aaaaaaaaaa agagggagag agattatttt gtttatatat 1260
   ttaggtgtgt gttttaaaat tagttttttt tttttttagg aaaatataaa aagttttttt 1320
   tttttattta ggtggatttt atgatgaggt tgggagagaa gttgattttt ttttgtagtt 1380
   ttgaaatttt atttgattag tattatttgt ttagatatgg ggttgtttat ggatagtggt 1440
   ttagtggagg gtagagatat agggaagtat tttaggttaa tttttttgtg ggttgtgtaa 1500
   tgttagtttt tggtgggatt tatagatgtt atggtttttt taatgatttt ttttataagt 1560
   ttttagtgat ttgttgtaat tttatattat aggtgaggag tttatgtttg ttgtatgttt 1620
   tggtgtttat tggattatag agttatatgt gaggggtgtt ttgttgggta tataggggta 1680
   ttaggtattt tggataaaat gaaatagtga taaatatata taggaaaaaa gaagttgagt 1740
   atgatggggt tgttagggtg tagggtggta agatggggta gttttatatt ttttgttatt 1800
   ttttgtatga agaaagaggt taggataaat atagggggag gtgaggttag atgtagtttg 1860
   ttgaggttag aggttgtttt ttattttttt ttattttttt aaagtttttt ttgatttttt 1920
   aatatagaga gatttttttg ttggggaatg tggagattta ttattttgat gggagataat 1980
   gtttgttgat attatttatt atgttttttt ttattatttt tttattttag ggaaggaatt 2040
   gttttttaga gattttaagg aagagatttt agggttttta ttagttttta ggtggatgat 2100
   agagtagggg gtgtgtaggg attaagtttt taaagaaaat ggtgttaatg tttgggaggt 2160
   tgaggtagga gaattgtttg aatttaggag gtggaggttg tagtgagata agatggtatt 2220
   attgtatttt agtttgggtg atagagtaag attttatttt aaaaaaaaaa aaaaaagaaa 2280
   gaaagaaaga aagaaagaaa aagaaagaaa gaaagaaaga aaagaaaatg gttttatttt 2340
   aatggggtaa ttaatgatgt ttgtttattt tttttttgtt tttttttttt ttaggaaata 2400
   ttaagagtat ttttttgtta tttatagaat ttatttttga atttggtaag taaataattt 2460
   ttattttagt gtttgagttt ttggggagat agaaggtttt agtgtgagtg aaagttgtgt 2520
   tattttttag ttttatgatt ttgggttagg tagttttttt taggttttta tattttttat 2580
   ttatatttga gattgtggtt agtgtgggaa tttgtaaggt tttttagttt tagatgtttt 2640
   gggattgagg ttttatttat aggggaggaa gaggttagag ttatttgatt tttgttgaaa 2700
   agtaatgttt tttatttttt tagggaggtt tgtgaatatg aaaatgttgg aagatgggaa 2760
   ggatttaaaa gattatattt gatatttatt gtttgttttt ttttgttgtt tttttttttg 2820
   agatagagtt ttattttgtt atttaggttg gagtgtggtg gtataatttt agtttattgt 2880
   aatttttgtt ttttgggttt aagtgatttt tttgttttag ttttttgagt agttgggatt 2940
   ataggtgttt attattatat ttggttaatt tttgtatttt tagtagagtt agggttttgt 3000
   t 3001
<210> 184
   <211> 3001
   <212> DNA
   <213> human
<400> 184
   ggcgaaattt tgattttatt aaaaatataa aaattagtcg ggtgtggtgg tgggtatttg 60
   taattttagt tattcgggag gttgaggtag gaaaatcgtt tgaatttagg aggtagaggt 120
   tgtagtgagt tgagattgtg ttatcgtatt ttagtttggg tgatagagtg agattttgtt 180
   ttaaaaaaaa aaataataaa aaaagataga tagtgaatat cgaatatggt tttttaaatt 240
   ttttttattt tttagtattt ttatgtttat agattttttt ggaggaatga gaagtattgt 300
   tttttagtaa gggttaggtg attttgattt tttttttttt tgtggatgag gttttagttt 360
   taaagcgttt gaggttgaaa ggttttatag attttcgtat tgattatagt tttagatgtg 420
   gatggggaat gtggggattt gggaggggtt gtttagttta gggttatgga gttgggaggt 480
   ggtatagttt ttatttatat tggggttttt tgttttttta gggatttaga tattaggata 540
   agagttattt gtttattaga tttaggggtg gattttgtga atgatagagg agtattttta 600
   gtgtttttta ggaaaaaaaa aggtagagaa ggggtgagta agcgttattg attgttttat 660
   taaagtagga ttattttttt tttttttttt tttttttttt tttttttttt tttttttttt 720
   tttttttttt tttttttttt tgagatggag ttttgttttg tcgtttaggt tggagtgtag 780
   tggtgttatt ttgtttcgtt gtaattttta ttttttgggt ttaagcgatt tttttgtttt 840
   agtttttcga gtattagtat tatttttttt ggaggtttgg tttttgtata ttttttattt 900
   tgttatttat ttaaagatta atgggggttt tggggttttt tttttggaat ttttggggga 960
   taattttttt tttgggatgg gaaggtgata aggagaagta tggtgggtga tgttaataga 1020
   tattgttttt tatcgggatg ataaattttt acgtttttta gtagggagat ttttttgtgt 1080
   tgaggggtta ggaggggttt tggagaaatg gaaaagggtg aggggtaatt tttgatttcg 1140
   ataaattata tttggtttta tttttttttg tgtttgtttt gatttttttt tttatataga 1200
   aggtggtaga gggtgtggag ttgtttcgtt ttattatttt atattttgat agttttatta 1260
   tgtttagttt tttttttttt gtgtgtgttt gttattgttt tattttattt agagtattta 1320
   atatttttgt gtgtttagcg ggacgttttt tacgtgtggt tttgtgattt agtgggtatt 1380
   agagtatgta gtaggtatgg gttttttatt tgtggtgtaa agttgtagcg ggttattggg 1440
   ggtttatagg gagagttatt gaaggaatta tagtatttat aggtttcgtt agggattggc 1500
   gttgtacggt ttatagaaaa gttggtttgg aatgtttttt tgtgtttttg ttttttattg 1560
   agttattgtt tatgagtaat ttcgtgtttg aatagatggt attggttaaa tgagatttta 1620
   gagttgtaga agagggttaa tttttttttt agttttatta tggggtttat ttgggtggag 1680
   agagaaggtt ttttgtattt ttttaggaga aaaagaggtt gattttagaa tatacgtttg 1740
   agtgtatgaa taaagtaatt tttttttttt tttttttttt tttttttttt ttttttgaga 1800
   tagattttta tttttgtttt ttaggttgga gtgtagtagt acgatttcgg tttattgtaa 1860
   ttttagtatt tcgggtttaa gtgatttttt tgttttagtt ttttgagtag ttgggattag 1920
   aggtacggat tattatgttt agttaatttt tgtattttta gtagagatag ggttttatta 1980
   tgttggttag gttggtttcg aatttttgat tttgtgattc gtttacgtta gttttttaaa 2040
   gtgttgggat tataggcgta agttatagcg tttagtttcg ttgtttttat tttggtagta 2100
   gatttcgaat gtcggttggt gtttttgtta attttatgtt tatttttagg gttttgagtt 2160
   agtttatgtt tgtgtttttt tatttttttt gtattttttt gattttgttt ttgattgagt 2220
   ttttgtggtt tatcgttttt agaagttata tgagatgtgg ggtttttttg gaaaatggga 2280
   attattttgt gttttgagat tttttagaaa atatagtgtt ggttttgagt tttttgatat 2340
   ggggttacgg ggttatgagt tttattattt tttaattgtg tttgttgaaa aatataagaa 2400
   tttcgggagg attagaagga attttatagg attttattgt agggaataat tggttgtatt 2460
   tttaagttta aggagttaga cgtgattatt tgttggggag ggtagaagtg attttttttg 2520
   tttttttaag tagtaagata tgttaaattt ttttgttgag tattttttta tagttttttt 2580
   ttttttttat tttttttttt ttattgaggt ttgattaata atcgtattat atgaagtttt 2640
   tatggtatta atagattacg gatggtttag ttatatttat ttgtgattat aatgtttagg 2700
   gggttattgg gagtcgatta tttatagcgg gagtgattga aataattata gtatttgtag 2760
   gtttttgtat gggtaggcgt tataggattt atggaaaaga tagtttcgac gtaatggatt 2820
   ttagttttta ttttttggtt aagttgttgg gaatgttgta tgtgtttttt tttgtataag 2880
   ataaatttat taaaggttag ttttgagtga tagcgtaggt ttattttggt ttttgtatgt 2940
   attagggagt ttgggtgcgt tgagatggag ggttttgtga ataagtttga gagtagagat 3000
   a 3001
<210> 185
   <211> 3001
   <212> DNA
   <213> human
<400> 185
   ggtgaaattt tgattttatt aaaaatataa aaattagttg ggtgtggtgg tgggtatttg 60
   taattttagt tatttgggag gttgaggtag gaaaattgtt tgaatttagg aggtagaggt 120
   tgtagtgagt tgagattgtg ttattgtatt ttagtttggg tgatagagtg agattttgtt 180
   ttaaaaaaaa aaataataaa aaaagataga tagtgaatat tgaatatggt tttttaaatt 240
   ttttttattt tttagtattt ttatgtttat agattttttt ggaggaatga gaagtattgt 300
   tttttagtaa gggttaggtg attttgattt tttttttttt tgtggatgag gttttagttt 360
   taaagtgttt gaggttgaaa ggttttatag atttttgtat tgattatagt tttagatgtg 420
   gatggggaat gtggggattt gggaggggtt gtttagttta gggttatgga gttgggaggt 480
   ggtatagttt ttatttatat tggggttttt tgttttttta gggatttaga tattaggata 540
   agagttattt gtttattaga tttaggggtg gattttgtga atgatagagg agtattttta 600
   gtgtttttta ggaaaaaaaa aggtagagaa ggggtgagta agtgttattg attgttttat 660
   taaagtagga ttattttttt tttttttttt tttttttttt tttttttttt tttttttttt 720
   tttttttttt tttttttttt tgagatggag ttttgttttg ttgtttaggt tggagtgtag 780
   tggtgttatt ttgttttgtt gtaattttta ttttttgggt ttaagtgatt tttttgtttt 840
   agttttttga gtattagtat tatttttttt ggaggtttgg tttttgtata ttttttattt 900
   tgttatttat ttaaagatta atgggggttt tggggttttt tttttggaat ttttggggga 960
   taattttttt tttgggatgg gaaggtgata aggagaagta tggtgggtga tgttaataga 1020
   tattgttttt tattgggatg ataaattttt atgtttttta gtagggagat ttttttgtgt 1080
   tgaggggtta ggaggggttt tggagaaatg gaaaagggtg aggggtaatt tttgattttg 1140
   ataaattata tttggtttta tttttttttg tgtttgtttt gatttttttt tttatataga 1200
   aggtggtaga gggtgtggag ttgttttgtt ttattatttt atattttgat agttttatta 1260
   tgtttagttt tttttttttt gtgtgtgttt gttattgttt tattttattt agagtattta 1320
   atatttttgt gtgtttagtg ggatgttttt tatgtgtggt tttgtgattt agtgggtatt 1380
   agagtatgta gtaggtatgg gttttttatt tgtggtgtaa agttgtagtg ggttattggg 1440
   ggtttatagg gagagttatt gaaggaatta tagtatttat aggttttgtt agggattggt 1500
   gttgtatggt ttatagaaaa gttggtttgg aatgtttttt tgtgtttttg ttttttattg 1560
   agttattgtt tatgagtaat tttgtgtttg aatagatggt attggttaaa tgagatttta 1620
   gagttgtaga agagggttaa tttttttttt agttttatta tggggtttat ttgggtggag 1680
   agagaaggtt ttttgtattt ttttaggaga aaaagaggtt gattttagaa tatatgtttg 1740
   agtgtatgaa taaagtaatt tttttttttt tttttttttt tttttttttt ttttttgaga 1800
   tagattttta tttttgtttt ttaggttgga gtgtagtagt atgattttgg tttattgtaa 1860
   ttttagtatt ttgggtttaa gtgatttttt tgttttagtt ttttgagtag ttgggattag 1920
   aggtatggat tattatgttt agttaatttt tgtattttta gtagagatag ggttttatta 1980
   tgttggttag gttggttttg aatttttgat tttgtgattt gtttatgtta gttttttaaa 2040
   gtgttgggat tataggtgta agttatagtg tttagttttg ttgtttttat tttggtagta 2100
   gattttgaat gttggttggt gtttttgtta attttatgtt tatttttagg gttttgagtt 2160
   agtttatgtt tgtgtttttt tatttttttt gtattttttt gattttgttt ttgattgagt 2220
   ttttgtggtt tattgttttt agaagttata tgagatgtgg ggtttttttg gaaaatggga 2280
   attattttgt gttttgagat tttttagaaa atatagtgtt ggttttgagt tttttgatat 2340
   ggggttatgg ggttatgagt tttattattt tttaattgtg tttgttgaaa aatataagaa 2400
   ttttgggagg attagaagga attttatagg attttattgt agggaataat tggttgtatt 2460
   tttaagttta aggagttaga tgtgattatt tgttggggag ggtagaagtg attttttttg 2520
   tttttttaag tagtaagata tgttaaattt ttttgttgag tattttttta tagttttttt 2580
   ttttttttat tttttttttt ttattgaggt ttgattaata attgtattat atgaagtttt 2640
   tatggtatta atagattatg gatggtttag ttatatttat ttgtgattat aatgtttagg 2700
   gggttattgg gagttgatta tttatagtgg gagtgattga aataattata gtatttgtag 2760
   gtttttgtat gggtaggtgt tataggattt atggaaaaga tagttttgat gtaatggatt 2820
   ttagttttta ttttttggtt aagttgttgg gaatgttgta tgtgtttttt tttgtataag 2880
   ataaatttat taaaggttag ttttgagtga tagtgtaggt ttattttggt ttttgtatgt 2940
   attagggagt ttgggtgtgt tgagatggag ggttttgtga ataagtttga gagtagagat 3000
   a 3001
<210> 186
   <211> 50
   <212> DNA
   <213> human
<400> 186
   cgatcatgtc agatgtatgg taagtagcgt atctcatgtg gatagaaaaa 50
<210> 187
   <211> 50
   <212> DNA
   <213> human
<400> 187
   cgattatgtt agatgtatgg taagtagcgt attttatgtg gatagaaaaa 50
<210> 188
   <211> 50
   <212> DNA
   <213> human
<400> 188
   tgattatgtt agatgtatgg taagtagtgt attttatgtg gatagaaaaa 50
<210> 189
   <211> 50
   <212> DNA
   <213> human
<400> 189
   tttttttatt tatatgagat acgttattta ttatatattt gatatgatcg 50
<210> 190
   <211> 50
   <212> DNA
   <213> human
<400> 190
   tttttttatt tatatgagat atgttattta ttatatattt gatatgattg 50
<210> 191
   <211> 3001
   <212> DNA
   <213> human
<400> 191
   gaggtctggg accaaatata ttttgttatt gaattcctgg catctagccc aatgcctaag     60
   cgatagcaag gtttgattta ttttatttta ttttatttat ttttttgagg cagagtcttg    120
   ctctattgcc caggctggag tacagtggca tgaccttggc tcagtgcaac ctccgcctcc    180
   caggttcaag cgattctcct gcttcagcct cctgagtagc tgggattata ggcgcacagc    240
   accacacctg gctaattttg gtatttttag tatagacggg gtttcactat gttggccagg    300
   ctgtgttcaa tctcctgacc tcatcatccg cccgcctcag cctcccaaag tgttgggatt    360
   acaggcatga gccactgcgc ccagccaata gcaagatgtt agcaaatatt actgaatgaa    420
   tgaattatgg aaggagagaa ttgtttcatt atcttttttt ttttgaaaca gagtttcact    480
   cttttgccca ggctggagtg aagtggcgtg atctcagctc actgcaatct ctgccccctg   540
   ggttcaggtg attctcctgc ctcagcctcc tgagtagctg ggattacagg cgcgtgccac    600
   cacaccggct agttttccta tttttagtag agacgggatt tcaccatgtt ggccaggctg    660
   atctcgacct cctgacctca ggtgatccac ccacctcagc ctcccaaagt gctaggatta    720
   caggcatgag ccaccacgtc cagcctcatt atctatttta acttaataat actttcctat    780
   ttttcctctg tatctttctt atctggaagt cttccatatc ttagaaagct ttaacctact    840
   aagtatttta accttattga accttgaaaa acattagttt ttcaggataa accaagtaac    900
   ctgttcatgt cagctatacc tgtattgttt cccagatcct aagcttgcta gagaagcagt    960
   ttatattcac ttttcttcta agttagcaaa aattccatga aaacatgatg ttccagatgt   1020
   ggcttaatgg atgtaattat gtgttattgc tggtattgtt tctgtgtctg cctaatcttt   1080
   actgctaccc tgatatgagc cagttcctga gggtttgact gatttgtatt ttctttacct   1140
   atactttgtc ttgcagtgaa agccctggat aatatatcat gaggatctac tttattttgc   1200
   ctattgatgg ttattttggt gatagggtca aggaacagaa ttttaaagtg aaaagacaat   1260
   tccattgact attatatatt gacgccttgt gtgtgcgatg accaacatgc tttagaaaga   1320
   gtctctatct gcaaggggtg tacctttcca ggaaggggaa cacaaccatc acaaaactca   1380
   tttaggcaca gtcccaggtg attaatatta ataatagtgt tgtgaggatt caggaaaggg   1440
   aaaaataact acaacttggg atgggtaggg aacacttcct gaggaattga gactccttta   1500
   cgatcatgtc agatgtatgg taagtagcgt atctcatgtg gatagaaaaa tgaatacaat   1560
   tgtgcaatta gaatgaattg tacaaatatg gagacctatt tgtatactag aataacaggc   1620
   agctttgtgg gggagagtaa aagagaatag gaagaagtaa actgctgaaa ttgtgagggg   1680
   tatttagtgt tttttcaaaa attaatatat ttggaaatgt cctattttaa gcatgggcta   1740
   taaatattag ggctggaaga tccttcataa ttttgctgac atacaaaaga acctgctgta   1800
   tttttgagct tgtgtctata tattaccacc atgggtgcta ttaacctcag tgttttctgt   1860
   atatttcaga cattagtctt taaccatggg ggactggaac ttattgggtg gcatcctaga   1920
   ggaagttcac tcccactcaa ccatagtggg gaaaatctgg ctgaccatcc tcttcatctt   1980
   ccgaatgctg gtacttcgtg tggctgctga ggatgtctgg gatgatgaac agtcagcatt   2040
   tgcctgcaac acccggcagc caggttgcaa caatatctgt tatgatgatg cattccctat   2100
   ctctttgatc aggttctggg ttttacagat catctttgtg tcttctcctt ctttggtcta   2160
   tatgggccat gcactttata ggctcagggc ctttgagaaa gacaggcaga ggaaaaagtc   2220
   acaccttaga gcccagatgg agaatccaga tcttgacttg gaggagcagc aaagaataga   2280
   tagggaactg aggaggttag aggagcagaa gaggatccat aaagtccctc tgaaaggatg   2340
   tctgctgcgt acttatgtct tacacatctt gaccagatct gtgctggaag taggattcat   2400
   gataggccaa tatattctct atgggtttca aatgcacccc ctttacaaat gcactcaacc   2460
   tccttgcccc aatgcggtgg attgctttgt atccaggccc actgagaaga caattttcat   2520
   gctttttatg cacagcattg cagccatttc cttgttactc aatatactgg aaatatttca   2580
   tctaggcatc agaaaaatta tgaggacact ttataagaaa tccagcagtg agggcattga   2640
   ggatgaaaca ggccctccat tccatttgaa gaaatattct gtggcccagc agtgtatgat   2700
   ttgctcttca ttgcctgaaa gaatctctcc acttcaagct aacaatcaac agcaagtcat   2760
   tcgagttaat gtgccaaagt ctaaaaccat gtggcaaatc ccacagccaa ggcaacttga   2820
   agtagaccct tccaatggga aaaaggactg gtctgagaag gatcagcata gcggacagct   2880
   ccatgttcac agcccgtgtc cctgggctgg cagtgctgga aatcagcacc tgggacagca   2940
   atcagaccat tcctcatttg gcctgcagaa tacaatgtct cagtcctggc taggtacaac   3000
   t                                                                   3001
<210> 192
   <211> 3001
   <212> DNA
   <213> human
<400> 192
   gaggtttggg attaaatata ttttgttatt gaatttttgg tatttagttt aatgtttaag      60
   cgatagtaag gtttgattta ttttatttta ttttatttat ttttttgagg tagagttttg     120
   ttttattgtt taggttggag tatagtggta tgattttggt ttagtgtaat tttcgttttt     180
   taggtttaag cgattttttt gttttagttt tttgagtagt tgggattata ggcgtatagt     240
   attatatttg gttaattttg gtatttttag tatagacggg gttttattat gttggttagg     300
   ttgtgtttaa ttttttgatt ttattattcg ttcgttttag ttttttaaag tgttgggatt     360
   ataggtatga gttattgcgt ttagttaata gtaagatgtt agtaaatatt attgaatgaa     420
   tgaattatgg aaggagagaa ttgttttatt attttttttt ttttgaaata gagttttatt     480
   tttttgttta ggttggagtg aagtggcgtg attttagttt attgtaattt ttgttttttg     540
   ggtttaggtg atttttttgt tttagttttt tgagtagttg ggattatagg cgcgtgttat     600
   tatatcggtt agttttttta tttttagtag agacgggatt ttattatgtt ggttaggttg     660
   atttcgattt tttgatttta ggtgatttat ttattttagt tttttaaagt gttaggatta     720
   taggtatgag ttattacgtt tagttttatt atttatttta atttaataat atttttttat     780
   tttttttttg tatttttttt atttggaagt tttttatatt ttagaaagtt ttaatttatt     840
   aagtatttta attttattga attttgaaaa atattagttt tttaggataa attaagtaat     900
   ttgtttatgt tagttatatt tgtattgttt tttagatttt aagtttgtta gagaagtagt     960
   ttatatttat ttttttttta agttagtaaa aattttatga aaatatgatg ttttagatgt    1020
   ggtttaatgg atgtaattat gtgttattgt tggtattgtt tttgtgtttg tttaattttt    1080
   attgttattt tgatatgagt tagtttttga gggtttgatt gatttgtatt ttttttattt   1140
   atattttgtt ttgtagtgaa agttttggat aatatattat gaggatttat tttattttgt    1200
   ttattgatgg ttattttggt gatagggtta aggaatagaa ttttaaagtg aaaagataat    1260
   tttattgatt attatatatt gacgttttgt gtgtgcgatg attaatatgt tttagaaaga    1320
   gtttttattt gtaaggggtg tattttttta ggaaggggaa tataattatt ataaaattta   1380
   tttaggtata gttttaggtg attaatatta ataatagtgt tgtgaggatt taggaaaggg    1440
   aaaaataatt ataatttggg atgggtaggg aatatttttt gaggaattga gattttttta   1500
   cgattatgtt agatgtatgg taagtagcgt attttatgtg gatagaaaaa tgaatataat    1560
   tgtgtaatta gaatgaattg tataaatatg gagatttatt tgtatattag aataataggt    1620
   agttttgtgg gggagagtaa aagagaatag gaagaagtaa attgttgaaa ttgtgagggg    1680
   tatttagtgt ttttttaaaa attaatatat ttggaaatgt tttattttaa gtatgggtta    1740
   taaatattag ggttggaaga ttttttataa ttttgttgat atataaaaga atttgttgta   1800
   tttttgagtt tgtgtttata tattattatt atgggtgtta ttaattttag tgttttttgt   1860
   atattttaga tattagtttt taattatggg ggattggaat ttattgggtg gtattttaga    1920
   ggaagtttat ttttatttaa ttatagtggg gaaaatttgg ttgattattt tttttatttt    1980
   tcgaatgttg gtatttcgtg tggttgttga ggatgtttgg gatgatgaat agttagtatt    2040
   tgtttgtaat attcggtagt taggttgtaa taatatttgt tatgatgatg tattttttat   2100
   ttttttgatt aggttttggg ttttatagat tatttttgtg tttttttttt ttttggttta    2160
   tatgggttat gtattttata ggtttagggt ttttgagaaa gataggtaga ggaaaaagtt    2220
   atattttaga gtttagatgg agaatttaga ttttgatttg gaggagtagt aaagaataga    2280
   tagggaattg aggaggttag aggagtagaa gaggatttat aaagtttttt tgaaaggatg    2340
   tttgttgcgt atttatgttt tatatatttt gattagattt gtgttggaag taggatttat    2400
   gataggttaa tatatttttt atgggtttta aatgtatttt ttttataaat gtatttaatt    2460
   tttttgtttt aatgcggtgg attgttttgt atttaggttt attgagaaga taatttttat    2520
   gttttttatg tatagtattg tagttatttt tttgttattt aatatattgg aaatatttta   2580
   tttaggtatt agaaaaatta tgaggatatt ttataagaaa tttagtagtg agggtattga    2640
   ggatgaaata ggttttttat tttatttgaa gaaatatttt gtggtttagt agtgtatgat    2700
   ttgtttttta ttgtttgaaa gaattttttt attttaagtt aataattaat agtaagttat    2760
   tcgagttaat gtgttaaagt ttaaaattat gtggtaaatt ttatagttaa ggtaatttga    2820
   agtagatttt tttaatggga aaaaggattg gtttgagaag gattagtata gcggatagtt    2880
   ttatgtttat agttcgtgtt tttgggttgg tagtgttgga aattagtatt tgggatagta    2940
   attagattat tttttatttg gtttgtagaa tataatgttt tagttttggt taggtataat    3000
   t                                                                    3001
<210> 193
   <211> 3001
   <212> DNA
   <213> human
<400> 193
   gaggtttggg attaaatata ttttgttatt gaatttttgg tatttagttt aatgtttaag 60
   tgatagtaag gtttgattta ttttatttta ttttatttat ttttttgagg tagagttttg 120
   ttttattgtt taggttggag tatagtggta tgattttggt ttagtgtaat ttttgttttt 180
   taggtttaag tgattttttt gttttagttt tttgagtagt tgggattata ggtgtatagt 240
   attatatttg gttaattttg gtatttttag tatagatggg gttttattat gttggttagg 300
   ttgtgtttaa ttttttgatt ttattatttg tttgttttag ttttttaaag tgttgggatt 360
   ataggtatga gttattgtgt ttagttaata gtaagatgtt agtaaatatt attgaatgaa 420
   tgaattatgg aaggagagaa ttgttttatt attttttttt ttttgaaata gagttttatt 480
   tttttgttta ggttggagtg aagtggtgtg attttagttt attgtaattt ttgttttttg 540
   ggtttaggtg atttttttgt tttagttttt tgagtagttg ggattatagg tgtgtgttat 600
   tatattggtt agttttttta tttttagtag agatgggatt ttattatgtt ggttaggttg 660
   attttgattt tttgatttta ggtgatttat ttattttagt tttttaaagt gttaggatta 720
   taggtatgag ttattatgtt tagttttatt atttatttta atttaataat atttttttat 780
   tttttttttg tatttttttt atttggaagt tttttatatt ttagaaagtt ttaatttatt 840
   aagtatttta attttattga attttgaaaa atattagttt tttaggataa attaagtaat 900
   ttgtttatgt tagttatatt tgtattgttt tttagatttt aagtttgtta gagaagtagt 960
   ttatatttat ttttttttta agttagtaaa aattttatga aaatatgatg ttttagatgt 1020
   ggtttaatgg atgtaattat gtgttattgt tggtattgtt tttgtgtttg tttaattttt 1080
   attgttattt tgatatgagt tagtttttga gggtttgatt gatttgtatt ttttttattt 1140
   atattttgtt ttgtagtgaa agttttggat aatatattat gaggatttat tttattttgt 1200
   ttattgatgg ttattttggt gatagggtta aggaatagaa ttttaaagtg aaaagataat 1260
   tttattgatt attatatatt gatgttttgt gtgtgtgatg attaatatgt tttagaaaga 1320
   gtttttattt gtaaggggtg tattttttta ggaaggggaa tataattatt ataaaattta 1380
   tttaggtata gttttaggtg attaatatta ataatagtgt tgtgaggatt taggaaaggg 1440
   aaaaataatt ataatttggg atgggtaggg aatatttttt gaggaattga gattttttta 1500
   tgattatgtt agatgtatgg taagtagtgt attttatgtg gatagaaaaa tgaatataat 1560
   tgtgtaatta gaatgaattg tataaatatg gagatttatt tgtatattag aataataggt 1620
   agttttgtgg gggagagtaa aagagaatag gaagaagtaa attgttgaaa ttgtgagggg 1680
   tatttagtgt ttttttaaaa attaatatat ttggaaatgt tttattttaa gtatgggtta 1740
   taaatattag ggttggaaga ttttttataa ttttgttgat atataaaaga atttgttgta 1800
   tttttgagtt tgtgtttata tattattatt atgggtgtta ttaattttag tgttttttgt 1860
   atattttaga tattagtttt taattatggg ggattggaat ttattgggtg gtattttaga 1920
   ggaagtttat ttttatttaa ttatagtggg gaaaatttgg ttgattattt tttttatttt 1980
   ttgaatgttg gtattttgtg tggttgttga ggatgtttgg gatgatgaat agttagtatt 2040
   tgtttgtaat atttggtagt taggttgtaa taatatttgt tatgatgatg tattttttat 2100
   ttttttgatt aggttttggg ttttatagat tatttttgtg tttttttttt ttttggttta 2160
   tatgggttat gtattttata ggtttagggt ttttgagaaa gataggtaga ggaaaaagtt 2220
   atattttaga gtttagatgg agaatttaga ttttgatttg gaggagtagt aaagaataga 2280
   tagggaattg aggaggttag aggagtagaa gaggatttat aaagtttttt tgaaaggatg 2340
   tttgttgtgt atttatgttt tatatatttt gattagattt gtgttggaag taggatttat 2400
   gataggttaa tatatttttt atgggtttta aatgtatttt ttttataaat gtatttaatt 2460
   tttttgtttt aatgtggtgg attgttttgt atttaggttt attgagaaga taatttttat 2520
   gttttttatg tatagtattg tagttatttt tttgttattt aatatattgg aaatatttta 2580
   tttaggtatt agaaaaatta tgaggatatt ttataagaaa tttagtagtg agggtattga 2640
   ggatgaaata ggttttttat tttatttgaa gaaatatttt gtggtttagt agtgtatgat 2700
   ttgtttttta ttgtttgaaa gaattttttt attttaagtt aataattaat agtaagttat 2760
   ttgagttaat gtgttaaagt ttaaaattat gtggtaaatt ttatagttaa ggtaatttga 2820
   agtagatttt tttaatggga aaaaggattg gtttgagaag gattagtata gtggatagtt 2880
   ttatgtttat agtttgtgtt tttgggttgg tagtgttgga aattagtatt tgggatagta 2940
   attagattat tttttatttg gtttgtagaa tataatgttt tagttttggt taggtataat 3000
   t 3001
<210> 194
   <211> 3001
   <212> DNA
   <213> human
<400> 194
   agttgtattt agttaggatt gagatattgt attttgtagg ttaaatgagg aatggtttga      60
   ttgttgtttt aggtgttgat ttttagtatt gttagtttag ggatacgggt tgtgaatatg     120
   gagttgttcg ttatgttgat tttttttaga ttagtttttt tttttattgg aagggtttat     180
   tttaagttgt tttggttgtg ggatttgtta tatggtttta gattttggta tattaattcg     240
   aatgatttgt tgttgattgt tagtttgaag tggagagatt tttttaggta atgaagagta     300
   aattatatat tgttgggtta tagaatattt ttttaaatgg aatggagggt ttgttttatt     360
   tttaatgttt ttattgttgg attttttata aagtgttttt ataatttttt tgatgtttag     420
   atgaaatatt tttagtatat tgagtaataa ggaaatggtt gtaatgttgt gtataaaaag     480
   tatgaaaatt gtttttttag tgggtttgga tataaagtaa tttatcgtat tggggtaagg     540
   aggttgagtg tatttgtaaa gggggtgtat ttgaaattta tagagaatat attggtttat     600
   tatgaatttt atttttagta tagatttggt taagatgtgt aagatataag tacgtagtag     660
   atattttttt agagggattt tatggatttt tttttgtttt tttaattttt ttagtttttt     720
   atttattttt tgttgttttt ttaagttaag atttggattt tttatttggg ttttaaggtg     780
   tgattttttt ttttgtttgt tttttttaaa ggttttgagt ttataaagtg tatggtttat     840
   atagattaaa gaaggagaag atataaagat gatttgtaaa atttagaatt tgattaaaga     900
   gatagggaat gtattattat aatagatatt gttgtaattt ggttgtcggg tgttgtaggt     960
   aaatgttgat tgtttattat tttagatatt tttagtagtt atacgaagta ttagtattcg    1020
   gaagatgaag aggatggtta gttagatttt ttttattatg gttgagtggg agtgaatttt    1080
   ttttaggatg ttatttaata agttttagtt ttttatggtt aaagattaat gtttgaaata    1140
   tatagaaaat attgaggtta atagtattta tggtggtaat atatagatat aagtttaaaa    1200
   atatagtagg tttttttgta tgttagtaaa attatgaagg attttttagt tttaatattt    1260
   atagtttatg tttaaaatag gatattttta aatatattaa tttttgaaaa aatattaaat    1320
   attttttata attttagtag tttatttttt tttatttttt tttatttttt tttataaagt    1380
   tgtttgttat tttagtatat aaataggttt ttatatttgt ataatttatt ttaattgtat    1440
   aattgtattt atttttttat ttatatgaga tacgttattt attatatatt tgatatgatc    1500
   gtaaaggagt tttaattttt taggaagtgt tttttattta ttttaagttg tagttatttt    1560
   tttttttttt gaatttttat aatattatta ttaatattaa ttatttggga ttgtgtttaa    1620
   atgagttttg tgatggttgt gttttttttt ttggaaaggt atattttttg tagatagaga    1680
   ttttttttaa agtatgttgg ttatcgtata tataaggcgt taatatataa tagttaatgg    1740
   aattgttttt ttattttaaa attttgtttt ttgattttat tattaaaata attattaata    1800
   ggtaaaataa agtagatttt tatgatatat tatttagggt ttttattgta agataaagta    1860
   taggtaaaga aaatataaat tagttaaatt tttaggaatt ggtttatatt agggtagtag    1920
   taaagattag gtagatatag aaataatatt agtaataata tataattata tttattaagt    1980
   tatatttgga atattatgtt tttatggaat ttttgttaat ttagaagaaa agtgaatata    2040
   aattgttttt ttagtaagtt taggatttgg gaaataatat aggtatagtt gatatgaata    2100
   ggttatttgg tttattttga aaaattaatg ttttttaagg tttaataagg ttaaaatatt    2160
   tagtaggtta aagtttttta agatatggaa gatttttaga taagaaagat atagaggaaa    2220
   aataggaaag tattattaag ttaaaataga taatgaggtt ggacgtggtg gtttatgttt    2280
   gtaattttag tattttggga ggttgaggtg ggtggattat ttgaggttag gaggtcgaga    2340
   ttagtttggt taatatggtg aaatttcgtt tttattaaaa ataggaaaat tagtcggtgt    2400
   ggtggtacgc gtttgtaatt ttagttattt aggaggttga ggtaggagaa ttatttgaat    2460
   ttagggggta gagattgtag tgagttgaga ttacgttatt ttattttagt ttgggtaaaa    2520
   gagtgaaatt ttgttttaaa aaaaaaaaga taatgaaata attttttttt tttataattt    2580
   atttatttag taatatttgt taatattttg ttattggttg ggcgtagtgg tttatgtttg    2640
   taattttaat attttgggag gttgaggcgg gcggatgatg aggttaggag attgaatata    2700
   gtttggttaa tatagtgaaa tttcgtttat attaaaaata ttaaaattag ttaggtgtgg    2760
   tgttgtgcgt ttataatttt agttatttag gaggttgaag taggagaatc gtttgaattt    2820
   gggaggcgga ggttgtattg agttaaggtt atgttattgt attttagttt gggtaataga    2880
   gtaagatttt gttttaaaaa aataaataaa ataaaataaa ataaattaaa ttttgttatc    2940
   gtttaggtat tgggttagat gttaggaatt taataataaa atatatttgg ttttagattt    3000
   t                                                                     3001
<210> 195
   <211> 3001
   <212> DNA
   <213> human
<400> 195
   agttgtattt agttaggatt gagatattgt attttgtagg ttaaatgagg aatggtttga 60
   ttgttgtttt aggtgttgat ttttagtatt gttagtttag ggatatgggt tgtgaatatg 120
   gagttgtttg ttatgttgat tttttttaga ttagtttttt tttttattgg aagggtttat 180
   tttaagttgt tttggttgtg ggatttgtta tatggtttta gattttggta tattaatttg 240
   aatgatttgt tgttgattgt tagtttgaag tggagagatt tttttaggta atgaagagta 300
   aattatatat tgttgggtta tagaatattt ttttaaatgg aatggagggt ttgttttatt 360
   tttaatgttt ttattgttgg attttttata aagtgttttt ataatttttt tgatgtttag 420
   atgaaatatt tttagtatat tgagtaataa ggaaatggtt gtaatgttgt gtataaaaag 480
   tatgaaaatt gtttttttag tgggtttgga tataaagtaa tttattgtat tggggtaagg 540
   aggttgagtg tatttgtaaa gggggtgtat ttgaaattta tagagaatat attggtttat 600
   tatgaatttt atttttagta tagatttggt taagatgtgt aagatataag tatgtagtag 660
   atattttttt agagggattt tatggatttt tttttgtttt tttaattttt ttagtttttt 720
   atttattttt tgttgttttt ttaagttaag atttggattt tttatttggg ttttaaggtg 780
   tgattttttt ttttgtttgt tttttttaaa ggttttgagt ttataaagtg tatggtttat 840
   atagattaaa gaaggagaag atataaagat gatttgtaaa atttagaatt tgattaaaga 900
   gatagggaat gtattattat aatagatatt gttgtaattt ggttgttggg tgttgtaggt 960
   aaatgttgat tgtttattat tttagatatt tttagtagtt atatgaagta ttagtatttg 1020
   gaagatgaag aggatggtta gttagatttt ttttattatg gttgagtggg agtgaatttt 1080
   ttttaggatg ttatttaata agttttagtt ttttatggtt aaagattaat gtttgaaata 1140
   tatagaaaat attgaggtta atagtattta tggtggtaat atatagatat aagtttaaaa 1200
   atatagtagg tttttttgta tgttagtaaa attatgaagg attttttagt tttaatattt 1260
   atagtttatg tttaaaatag gatattttta aatatattaa tttttgaaaa aatattaaat 1320
   attttttata attttagtag tttatttttt tttatttttt tttatttttt tttataaagt 1380
   tgtttgttat tttagtatat aaataggttt ttatatttgt ataatttatt ttaattgtat 1440
   aattgtattt atttttttat ttatatgaga tatgttattt attatatatt tgatatgatt 1500
   gtaaaggagt tttaattttt taggaagtgt tttttattta ttttaagttg tagttatttt 1560
   tttttttttt gaatttttat aatattatta ttaatattaa ttatttggga ttgtgtttaa 1620
   atgagttttg tgatggttgt gttttttttt ttggaaaggt atattttttg tagatagaga 1680
   ttttttttaa agtatgttgg ttattgtata tataaggtgt taatatataa tagttaatgg 1740
   aattgttttt ttattttaaa attttgtttt ttgattttat tattaaaata attattaata 1800
   ggtaaaataa agtagatttt tatgatatat tatttagggt ttttattgta agataaagta 1860
   taggtaaaga aaatataaat tagttaaatt tttaggaatt ggtttatatt agggtagtag 1920
   taaagattag gtagatatag aaataatatt agtaataata tataattata tttattaagt 1980
   tatatttgga atattatgtt tttatggaat ttttgttaat ttagaagaaa agtgaatata 2040
   aattgttttt ttagtaagtt taggatttgg gaaataatat aggtatagtt gatatgaata 2100
   ggttatttgg tttattttga aaaattaatg ttttttaagg tttaataagg ttaaaatatt 2160
   tagtaggtta aagtttttta agatatggaa gatttttaga taagaaagat atagaggaaa 2220
   aataggaaag tattattaag ttaaaataga taatgaggtt ggatgtggtg gtttatgttt 2280
   gtaattttag tattttggga ggttgaggtg ggtggattat ttgaggttag gaggttgaga 2340
   ttagtttggt taatatggtg aaattttgtt tttattaaaa ataggaaaat tagttggtgt 2400
   ggtggtatgt gtttgtaatt ttagttattt aggaggttga ggtaggagaa ttatttgaat 2460
   ttagggggta gagattgtag tgagttgaga ttatgttatt ttattttagt ttgggtaaaa 2520
   gagtgaaatt ttgttttaaa aaaaaaaaga taatgaaata attttttttt tttataattt 2580
   atttatttag taatatttgt taatattttg ttattggttg ggtgtagtgg tttatgtttg 2640
   taattttaat attttgggag gttgaggtgg gtggatgatg aggttaggag attgaatata 2700
   gtttggttaa tatagtgaaa ttttgtttat attaaaaata ttaaaattag ttaggtgtgg 2760
   tgttgtgtgt ttataatttt agttatttag gaggttgaag taggagaatt gtttgaattt 2820
   gggaggtgga ggttgtattg agttaaggtt atgttattgt attttagttt gggtaataga 2880
   gtaagatttt gttttaaaaa aataaataaa ataaaataaa ataaattaaa ttttgttatt 2940
   gtttaggtat tgggttagat gttaggaatt taataataaa atatatttgg ttttagattt 3000
   t 3001

<210> 196
   <211> 50
   <212> DNA
   <213> human
<400> 196
   atgagctgga gcgccggaac cggctgttgg agaagcaact gcagcaagcg 50
<210> 197
   <211> 50
   <212> DNA
   <213> human
<400> 197
   atgagttgga gcgtcggaat cggttgttgg agaagtaatt gtagtaagcg 50
<210> 198
   <211> 50
   <212> DNA
   <213> human
<400> 198
   atgagttgga gtgttggaat tggttgttgg agaagtaatt gtagtaagtg 50
<210> 199
   <211> 50
   <212> DNA
   <213> human
<400> 199
   cgtttgttgt agttgttttt ttaatagtcg gtttcggcgt tttagtttat 50
<210> 200
   <211> 50
   <212> DNA
   <213> human
<400> 200
   tgtttgttgt agttgttttt ttaatagttg gttttggtgt tttagtttat 50
<210> 201
   <211> 3001
   <212> DNA
   <213> human
<400> 201
   taaggaccat catcagcccc ttggaagggc caagggggtt gagaagcagc agttgccaga      60
   gcagcctttt gagaaagctg ccttccagaa acagaatgat acccccaagg ggcctcagcc     120
   tcccactgtg tctcccatcc gttccagccg ccccccacca gcaaagagga agaaatctca     180
   gtccaggggc aacagccagc tgctgctatc ttagatggtt gaaaactaga cgggtggctc     240
   actgccattg tcaccaggtt ggaactcttg cctctgtgag gatgccttct ctactgtgca     300
   tacccatgaa atttaataca cattttaaaa cctctggcca ctgagtattt ttgagggaac     360
   tgggtgctgc ctttgccgct ttaagggtaa agatgggagg tgggactggg atcttctgag     420
   gagagggaat ggttgggtgc tactgagaaa taaggtcagt tttaggctcc tgcatccttg     480
   ggttttccca ggaagggaaa gccctggagt cagagctagg ggtgattgaa ctccggggag     540
   aggagggcaa tagaagtgta ggggaaggcg gggcacacta gctcatgcct gtaatcccag     600
   cactttctgg gaggccaagg cgggtggatc acctgaggtc aggtgttcga gaccagcctg     660
   gctaacatgg cgaaacctcg tctctactaa aaatacaaaa aatcagccgg gtgtggtggc     720
   gggtgcctgt aatcccagct actcgggaga ctgaagcagg agaatcgctt gaacctggga     780
   ggctgaggtt gcagtgagcc aagatcgcgc cactgcactc cagcccgggc gacagtgaga     840
   ctcggtctca aaaaaaaaaa aagtcgtgta ggggagatgg gcttgccacc agaacccatg     900
   gggtactgga ggtcctgggc tttacgccac cccggctgga gtccaggcgg tggctcccct     960
   catcacaggg ctcggcctca gacccggaaa ggtccagggt ccggcctcca attggagcct    1020
   gtcattggct tcccctcggc ccgccccctg acctctgccc cgctgccgag ccgcctgatt    1080
   ggtcggctct gtcccgccgg cgccgcattg gctcttctgg ctcgccttcc atccctgctc    1140
   ctgcaccggt ctgcatctcc cagcagaagg cgcagccatg aatccgttat tcggccccaa    1200
   cctcttcctc ctgcagcagg agcagcaggg cctggccggg ccactggggg actcactggg    1260
   aggcgaccac ttcgccgggg gaggagactt gcccccggcg cctctctcgc cggccggccc    1320
   tgctgcctac tcgccgcccg ggccgggccc ggccccgcct gccgccatgg ccctccgcaa    1380
   tgacctgggc tccaacatca acgtgctcaa gaccctgaac ctccggttcc gctgcttcct    1440
   ggccaaggtg catgagctgg agcgccggaa ccggctgttg gagaagcaac tgcagcaagc    1500
   gctggaggag ggtaagcagg gccggcgggg cctgggtcgt cgcgaccagg cagtgcagac    1560
   cggcttcgtc agccccatcc ggcccctggg gctgcagctg ggcgcccggc cggccgctgt    1620
   ctgcagccct tcggcgcgcg tgctgggctc gcccgcgcgc tccccggccg gccccctcgc    1680
   gccctccgcg gccagcctct cgtcgtcctc cacctccacc tccaccacct attcctcgtc    1740
   ggcccgcttc atgcccggca ccatctggtc gttctcgcac gcccgccggc tcgggccggg    1800
   actggagccc actctggtgc aagggcctgg cttgtcgtgg gtgcacccgg atggggtggg    1860
   cgtccagatc gacaccatca cgcccgagat ccgcgctctc tacaacgtgc tggccaaagt    1920
   gaagcgggag cgggacgagt acaagcggag gtagggattg ggaaaggggg acaggtctct    1980
   ctgtgtcata ccactcacaa cgggccgcga gttggtgtgg gttccgaaga atcaggggtg    2040
   aaaagaatga gagtaaagtt tgtgctgtgt aatgaggagc ttggaagcaa ttggatgcag    2100
   ggagactaga atccgcagag aatgcagaag ccctgctcgt gacaggaaga aggacgctgg    2160
   gagcggaaca cggggggatt cctacttttc cggcttgtct ctgccatcca ggcttaactg    2220
   cgggttttgt gcggtgtgaa agggactggg gctctgccct accggtctca gccctggggc    2280
   aggttaggga tataaacttc aaaagtagga ggcaaggata tagcgtaaat ttgggagact    2340
   aaattgagtg aaactggaag gggctggtga gggaggggct gtaccttgca acgagtgatt    2400
   ctttcacacc ccaaggtttc ccaaagaata ttgatggaaa ccaggtacct gctgtggttt    2460
   cataggaaaa gctccgggac ctcaagccat tgcttgagta ggtcaacaaa ctgggaaaag    2520
   aaagagtgaa gggaattcac tagggtgagg gtggagagca gggctgacag gggaatcttg    2580
   gcagtataaa gcgccagtgg ggagggagag tggagagcca gcaggggact gagcagggag    2640
   aagctgggat atgggccctc cctaggaccg cgaggccgcg gggcgggccc ggtgcattct    2700
   gtggatgaag atttggctgt ctgagaggag cccggcccca aggggcaagg gaaagggatg    2760
   tgatgccagg agtgctggct gtataccaag tggattcctt agatggctga taaccaagcc    2820
   gataggacag gagtgagagc agaggtagtg tcttgcagac tccaacccaa gccttgagga    2880
   gggagcgggt gactgacagg ctttggtttg gcggcctccc ctctcatctc agctattatg    2940
   accccttgtg ggcgggcctg ggaactacct atgcagccca gtgtggcgga gattcagcac    3000
   t                                                                    3001
<210> 202
   <211> 3001
   <212> DNA
   <213> human
<400> 202
   taaggattat tattagtttt ttggaagggt taagggggtt gagaagtagt agttgttaga 60
   gtagtttttt gagaaagttg ttttttagaa atagaatgat atttttaagg ggttttagtt 120
   ttttattgtg ttttttattc gttttagtcg ttttttatta gtaaagagga agaaatttta 180
   gtttaggggt aatagttagt tgttgttatt ttagatggtt gaaaattaga cgggtggttt 240
   attgttattg ttattaggtt ggaatttttg tttttgtgag gatgtttttt ttattgtgta 300
   tatttatgaa atttaatata tattttaaaa tttttggtta ttgagtattt ttgagggaat 360
   tgggtgttgt ttttgtcgtt ttaagggtaa agatgggagg tgggattggg attttttgag 420
   gagagggaat ggttgggtgt tattgagaaa taaggttagt tttaggtttt tgtatttttg 480
   ggttttttta ggaagggaaa gttttggagt tagagttagg ggtgattgaa tttcggggag 540
   aggagggtaa tagaagtgta ggggaaggcg gggtatatta gtttatgttt gtaattttag 600
   tattttttgg gaggttaagg cgggtggatt atttgaggtt aggtgttcga gattagtttg 660
   gttaatatgg cgaaatttcg tttttattaa aaatataaaa aattagtcgg gtgtggtggc 720
   gggtgtttgt aattttagtt attcgggaga ttgaagtagg agaatcgttt gaatttggga 780
   ggttgaggtt gtagtgagtt aagatcgcgt tattgtattt tagttcgggc gatagtgaga 840
   ttcggtttta aaaaaaaaaa aagtcgtgta ggggagatgg gtttgttatt agaatttatg 900
   gggtattgga ggttttgggt tttacgttat ttcggttgga gtttaggcgg tggttttttt 960
   tattataggg ttcggtttta gattcggaaa ggtttagggt tcggttttta attggagttt 1020
   gttattggtt ttttttcggt tcgttttttg atttttgttt cgttgtcgag tcgtttgatt 1080
   ggtcggtttt gtttcgtcgg cgtcgtattg gtttttttgg ttcgtttttt atttttgttt 1140
   ttgtatcggt ttgtattttt tagtagaagg cgtagttatg aattcgttat tcggttttaa 1200
   tttttttttt ttgtagtagg agtagtaggg tttggtcggg ttattggggg atttattggg 1260
   aggcgattat ttcgtcgggg gaggagattt gttttcggcg tttttttcgt cggtcggttt 1320
   tgttgtttat tcgtcgttcg ggtcgggttc ggtttcgttt gtcgttatgg tttttcgtaa 1380
   tgatttgggt tttaatatta acgtgtttaa gattttgaat tttcggtttc gttgtttttt 1440
   ggttaaggtg tatgagttgg agcgtcggaa tcggttgttg gagaagtaat tgtagtaagc 1500
   gttggaggag ggtaagtagg gtcggcgggg tttgggtcgt cgcgattagg tagtgtagat 1560
   cggtttcgtt agttttattc ggtttttggg gttgtagttg ggcgttcggt cggtcgttgt 1620
   ttgtagtttt tcggcgcgcg tgttgggttc gttcgcgcgt ttttcggtcg gttttttcgc 1680
   gtttttcgcg gttagttttt cgtcgttttt tatttttatt tttattattt atttttcgtc 1740
   ggttcgtttt atgttcggta ttatttggtc gttttcgtac gttcgtcggt tcgggtcggg 1800
   attggagttt attttggtgt aagggtttgg tttgtcgtgg gtgtattcgg atggggtggg 1860
   cgtttagatc gatattatta cgttcgagat tcgcgttttt tataacgtgt tggttaaagt 1920
   gaagcgggag cgggacgagt ataagcggag gtagggattg ggaaaggggg ataggttttt 1980
   ttgtgttata ttatttataa cgggtcgcga gttggtgtgg gtttcgaaga attaggggtg 2040
   aaaagaatga gagtaaagtt tgtgttgtgt aatgaggagt ttggaagtaa ttggatgtag 2100
   ggagattaga attcgtagag aatgtagaag ttttgttcgt gataggaaga aggacgttgg 2160
   gagcggaata cggggggatt tttatttttt cggtttgttt ttgttattta ggtttaattg 2220
   cgggttttgt gcggtgtgaa agggattggg gttttgtttt atcggtttta gttttggggt 2280
   aggttaggga tataaatttt aaaagtagga ggtaaggata tagcgtaaat ttgggagatt 2340
   aaattgagtg aaattggaag gggttggtga gggaggggtt gtattttgta acgagtgatt 2400
   tttttatatt ttaaggtttt ttaaagaata ttgatggaaa ttaggtattt gttgtggttt 2460
   tataggaaaa gtttcgggat tttaagttat tgtttgagta ggttaataaa ttgggaaaag 2520
   aaagagtgaa gggaatttat tagggtgagg gtggagagta gggttgatag gggaattttg 2580
   gtagtataaa gcgttagtgg ggagggagag tggagagtta gtaggggatt gagtagggag 2640
   aagttgggat atgggttttt tttaggatcg cgaggtcgcg gggcgggttc ggtgtatttt 2700
   gtggatgaag atttggttgt ttgagaggag ttcggtttta aggggtaagg gaaagggatg 2760
   tgatgttagg agtgttggtt gtatattaag tggatttttt agatggttga taattaagtc 2820
   gataggatag gagtgagagt agaggtagtg ttttgtagat tttaatttaa gttttgagga 2880
   gggagcgggt gattgatagg ttttggtttg gcggtttttt tttttatttt agttattatg 2940
   attttttgtg ggcgggtttg ggaattattt atgtagttta gtgtggcgga gatttagtat 3000
   t 3001
<210> 203
   <211> 3001
   <212> DNA
   <213> human
<400> 203
   taaggattat tattagtttt ttggaagggt taagggggtt gagaagtagt agttgttaga 60
   gtagtttttt gagaaagttg ttttttagaa atagaatgat atttttaagg ggttttagtt 120
   ttttattgtg ttttttattt gttttagttg ttttttatta gtaaagagga agaaatttta     180
   gtttaggggt aatagttagt tgttgttatt ttagatggtt gaaaattaga tgggtggttt     240
   attgttattg ttattaggtt ggaatttttg tttttgtgag gatgtttttt ttattgtgta     300
   tatttatgaa atttaatata tattttaaaa tttttggtta ttgagtattt ttgagggaat     360
   tgggtgttgt ttttgttgtt ttaagggtaa agatgggagg tgggattggg attttttgag     420
   gagagggaat ggttgggtgt tattgagaaa taaggttagt tttaggtttt tgtatttttg     480
   ggttttttta ggaagggaaa gttttggagt tagagttagg ggtgattgaa ttttggggag     540
   aggagggtaa tagaagtgta ggggaaggtg gggtatatta gtttatgttt gtaattttag     600
   tattttttgg gaggttaagg tgggtggatt atttgaggtt aggtgtttga gattagtttg     660
   gttaatatgg tgaaattttg tttttattaa aaatataaaa aattagttgg gtgtggtggt     720
   gggtgtttgt aattttagtt atttgggaga ttgaagtagg agaattgttt gaatttggga     780
   ggttgaggtt gtagtgagtt aagattgtgt tattgtattt tagtttgggt gatagtgaga     840
   tttggtttta aaaaaaaaaa aagttgtgta ggggagatgg gtttgttatt agaatttatg     900
   gggtattgga ggttttgggt tttatgttat tttggttgga gtttaggtgg tggttttttt    960
   tattataggg tttggtttta gatttggaaa ggtttagggt ttggttttta attggagttt    1020
   gttattggtt tttttttggt ttgttttttg atttttgttt tgttgttgag ttgtttgatt      1080
   ggttggtttt gttttgttgg tgttgtattg gtttttttgg tttgtttttt atttttgttt    1140
   ttgtattggt ttgtattttt tagtagaagg tgtagttatg aatttgttat ttggttttaa    1200
   tttttttttt ttgtagtagg agtagtaggg tttggttggg ttattggggg atttattggg    1260
   aggtgattat tttgttgggg gaggagattt gtttttggtg ttttttttgt tggttggttt    1320
   tgttgtttat ttgttgtttg ggttgggttt ggttttgttt gttgttatgg ttttttgtaa   1380
   tgatttgggt tttaatatta atgtgtttaa gattttgaat ttttggtttt gttgtttttt    1440
   ggttaaggtg tatgagttgg agtgttggaa ttggttgttg gagaagtaat tgtagtaagt    1500
   gttggaggag ggtaagtagg gttggtgggg tttgggttgt tgtgattagg tagtgtagat    1560
   tggttttgtt agttttattt ggtttttggg gttgtagttg ggtgtttggt tggttgttgt    1620
   ttgtagtttt ttggtgtgtg tgttgggttt gtttgtgtgt tttttggttg gtttttttgt   1680
   gttttttgtg gttagttttt tgttgttttt tatttttatt tttattattt attttttgtt    1740
   ggtttgtttt atgtttggta ttatttggtt gtttttgtat gtttgttggt ttgggttggg    1800
   attggagttt attttggtgt aagggtttgg tttgttgtgg gtgtatttgg atggggtggg    1860
   tgtttagatt gatattatta tgtttgagat ttgtgttttt tataatgtgt tggttaaagt    1920
   gaagtgggag tgggatgagt ataagtggag gtagggattg ggaaaggggg ataggttttt    1980
   ttgtgttata ttatttataa tgggttgtga gttggtgtgg gttttgaaga attaggggtg    2040
   aaaagaatga gagtaaagtt tgtgttgtgt aatgaggagt ttggaagtaa ttggatgtag    2100
   ggagattaga atttgtagag aatgtagaag ttttgtttgt gataggaaga aggatgttgg    2160
   gagtggaata tggggggatt tttatttttt tggtttgttt ttgttattta ggtttaattg    2220
   tgggttttgt gtggtgtgaa agggattggg gttttgtttt attggtttta gttttggggt    2280
   aggttaggga tataaatttt aaaagtagga ggtaaggata tagtgtaaat ttgggagatt    2340
   aaattgagtg aaattggaag gggttggtga gggaggggtt gtattttgta atgagtgatt    2400
   tttttatatt ttaaggtttt ttaaagaata ttgatggaaa ttaggtattt gttgtggttt    2460
   tataggaaaa gttttgggat tttaagttat tgtttgagta ggttaataaa ttgggaaaag    2520
   aaagagtgaa gggaatttat tagggtgagg gtggagagta gggttgatag gggaattttg    2580
   gtagtataaa gtgttagtgg ggagggagag tggagagtta gtaggggatt gagtagggag    2640
   aagttgggat atgggttttt tttaggattg tgaggttgtg gggtgggttt ggtgtatttt    2700
   gtggatgaag atttggttgt ttgagaggag tttggtttta aggggtaagg gaaagggatg     2760
   tgatgttagg agtgttggtt gtatattaag tggatttttt agatggttga taattaagtt    2820
   gataggatag gagtgagagt agaggtagtg ttttgtagat tttaatttaa gttttgagga    2880
   gggagtgggt gattgatagg ttttggtttg gtggtttttt tttttatttt agttattatg    2940
   attttttgtg ggtgggtttg ggaattattt atgtagttta gtgtggtgga gatttagtat     3000
   t                                                                    3001
<210> 204
   <211> 3001
   <212> DNA
   <213> human
<400> 204
   agtgttgaat tttcgttata ttgggttgta taggtagttt ttaggttcgt ttataagggg      60
   ttataatagt tgagatgaga ggggaggtcg ttaaattaaa gtttgttagt tattcgtttt     120
   ttttttaagg tttgggttgg agtttgtaag atattatttt tgtttttatt tttgttttat     180
   cggtttggtt attagttatt taaggaattt atttggtata tagttagtat ttttggtatt     240
   atattttttt tttttgtttt ttggggtcgg gtttttttta gatagttaaa tttttattta     300
   tagaatgtat cgggttcgtt tcgcggtttc gcggttttag ggagggttta tattttagtt     360
   tttttttgtt tagttttttg ttggtttttt attttttttt tttattggcg ttttatattg     420
   ttaagatttt tttgttagtt ttgtttttta tttttatttt agtgaatttt ttttattttt     480
   ttttttttta gtttgttgat ttatttaagt aatggtttga ggtttcggag ttttttttat     540
   gaaattatag taggtatttg gtttttatta atattttttg ggaaattttg gggtgtgaaa     600
   gaattattcg ttgtaaggta tagttttttt tttattagtt ttttttagtt ttatttaatt      660
   tagtttttta aatttacgtt atatttttgt tttttatttt tgaagtttat atttttaatt     720
   tgttttaggg ttgagatcgg tagggtagag ttttagtttt ttttatatcg tataaaattc     780
   gtagttaagt ttggatggta gagataagtc ggaaaagtag gaattttttc gtgtttcgtt     840
   tttagcgttt ttttttttgt tacgagtagg gtttttgtat tttttgcgga ttttagtttt     900
   tttgtattta attgttttta agttttttat tatatagtat aaattttatt tttatttttt      960
   ttatttttga tttttcggaa tttatattaa ttcgcggttc gttgtgagtg gtatgatata    1020
   gagagatttg tttttttttt ttaattttta ttttcgtttg tattcgtttc gttttcgttt    1080
   tattttggtt agtacgttgt agagagcgcg gatttcgggc gtgatggtgt cgatttggac    1140
   gtttatttta ttcgggtgta tttacgataa gttaggtttt tgtattagag tgggttttag    1200
   tttcggttcg agtcggcggg cgtgcgagaa cgattagatg gtgtcgggta tgaagcgggt    1260
   cgacgaggaa taggtggtgg aggtggaggt ggaggacgac gagaggttgg tcgcggaggg    1320
   cgcgaggggg tcggtcgggg agcgcgcggg cgagtttagt acgcgcgtcg aagggttgta    1380
   gatagcggtc ggtcgggcgt ttagttgtag ttttaggggt cggatggggt tgacgaagtc    1440
   ggtttgtatt gtttggtcgc gacgatttag gtttcgtcgg ttttgtttat ttttttttag     1500
   cgtttgttgt agttgttttt ttaatagtcg gtttcggcgt tttagtttat gtattttggt    1560
   taggaagtag cggaatcgga ggtttagggt tttgagtacg ttgatgttgg agtttaggtt    1620
   attgcggagg gttatggcgg taggcggggt cgggttcggt tcgggcggcg agtaggtagt    1680
   agggtcggtc ggcgagagag gcgtcggggg taagtttttt ttttcggcga agtggtcgtt    1740
   ttttagtgag ttttttagtg gttcggttag gttttgttgt ttttgttgta ggaggaagag    1800
   gttggggtcg aataacggat ttatggttgc gttttttgtt gggagatgta gatcggtgta   1860
   ggagtaggga tggaaggcga gttagaagag ttaatgcggc gtcggcggga tagagtcgat    1920
   taattaggcg gttcggtagc ggggtagagg ttagggggcg ggtcgagggg aagttaatga    1980
   taggttttaa ttggaggtcg gattttggat tttttcgggt ttgaggtcga gttttgtgat    2040
   gaggggagtt atcgtttgga ttttagtcgg ggtggcgtaa agtttaggat ttttagtatt    2100
   ttatgggttt tggtggtaag tttatttttt ttatacgatt tttttttttt ttgagatcga    2160
   gttttattgt cgttcgggtt ggagtgtagt ggcgcgattt tggtttattg taattttagt    2220
   tttttaggtt taagcgattt ttttgtttta gtttttcgag tagttgggat tataggtatt    2280
   cgttattata ttcggttgat tttttgtatt tttagtagag acgaggtttc gttatgttag    2340
   ttaggttggt ttcgaatatt tgattttagg tgatttattc gttttggttt tttagaaagt    2400
   gttgggatta taggtatgag ttagtgtgtt tcgttttttt ttatattttt attgtttttt    2460
   ttttttcgga gtttaattat ttttagtttt gattttaggg tttttttttt ttgggaaaat     2520
   ttaaggatgt aggagtttaa aattgatttt attttttagt agtatttaat tatttttttt    2580
   ttttagaaga ttttagtttt attttttatt tttattttta aagcggtaaa ggtagtattt   2640
   agttttttta aaaatattta gtggttagag gttttaaaat gtgtattaaa ttttatgggt     2700
   atgtatagta gagaaggtat ttttatagag gtaagagttt taatttggtg ataatggtag    2760
   tgagttattc gtttagtttt taattattta agatagtagt agttggttgt tgtttttgga    2820
   ttgagatttt tttttttttg ttggtggggg gcggttggaa cggatgggag atatagtggg    2880
   aggttgaggt tttttggggg tattattttg tttttggaag gtagtttttt taaaaggttg   2940
   ttttggtaat tgttgttttt taattttttt ggttttttta aggggttgat gatggttttt     3000
   a                                                                    3001
<210> 205
   <211> 3001
   <212> DNA
   <213> human
<400> 205
   agtgttgaat ttttgttata ttgggttgta taggtagttt ttaggtttgt ttataagggg      60
   ttataatagt tgagatgaga ggggaggttg ttaaattaaa gtttgttagt tatttgtttt     120
   ttttttaagg tttgggttgg agtttgtaag atattatttt tgtttttatt tttgttttat     180
   tggtttggtt attagttatt taaggaattt atttggtata tagttagtat ttttggtatt      240
   atattttttt tttttgtttt ttggggttgg gtttttttta gatagttaaa tttttattta      300
   tagaatgtat tgggtttgtt ttgtggtttt gtggttttag ggagggttta tattttagtt     360
   tttttttgtt tagttttttg ttggtttttt attttttttt tttattggtg ttttatattg     420
   ttaagatttt tttgttagtt ttgtttttta tttttatttt agtgaatttt ttttattttt     480
   ttttttttta gtttgttgat ttatttaagt aatggtttga ggttttggag ttttttttat     540
   gaaattatag taggtatttg gtttttatta atattttttg ggaaattttg gggtgtgaaa     600
   gaattatttg ttgtaaggta tagttttttt tttattagtt ttttttagtt ttatttaatt     660
   tagtttttta aatttatgtt atatttttgt tttttatttt tgaagtttat atttttaatt     720
   tgttttaggg ttgagattgg tagggtagag ttttagtttt ttttatattg tataaaattt     780
   gtagttaagt ttggatggta gagataagtt ggaaaagtag gaattttttt gtgttttgtt     840
   tttagtgttt ttttttttgt tatgagtagg gtttttgtat tttttgtgga ttttagtttt     900
   tttgtattta attgttttta agttttttat tatatagtat aaattttatt tttatttttt     960
   ttatttttga ttttttggaa tttatattaa tttgtggttt gttgtgagtg gtatgatata    1020
   gagagatttg tttttttttt ttaattttta tttttgtttg tatttgtttt gtttttgttt    1080
   tattttggtt agtatgttgt agagagtgtg gattttgggt gtgatggtgt tgatttggat    1140
   gtttatttta tttgggtgta tttatgataa gttaggtttt tgtattagag tgggttttag    1200
   ttttggtttg agttggtggg tgtgtgagaa tgattagatg gtgttgggta tgaagtgggt    1260
   tgatgaggaa taggtggtgg aggtggaggt ggaggatgat gagaggttgg ttgtggaggg    1320
   tgtgaggggg ttggttgggg agtgtgtggg tgagtttagt atgtgtgttg aagggttgta    1380
   gatagtggtt ggttgggtgt ttagttgtag ttttaggggt tggatggggt tgatgaagtt    1440
   ggtttgtatt gtttggttgt gatgatttag gttttgttgg ttttgtttat ttttttttag    1500
   tgtttgttgt agttgttttt ttaatagttg gttttggtgt tttagtttat gtattttggt    1560
   taggaagtag tggaattgga ggtttagggt tttgagtatg ttgatgttgg agtttaggtt    1620
   attgtggagg gttatggtgg taggtggggt tgggtttggt ttgggtggtg agtaggtagt    1680
   agggttggtt ggtgagagag gtgttggggg taagtttttt tttttggtga agtggttgtt    1740
   ttttagtgag ttttttagtg gtttggttag gttttgttgt ttttgttgta ggaggaagag    1800
   gttggggttg aataatggat ttatggttgt gttttttgtt gggagatgta gattggtgta    1860
   ggagtaggga tggaaggtga gttagaagag ttaatgtggt gttggtggga tagagttgat    1920
   taattaggtg gtttggtagt ggggtagagg ttagggggtg ggttgagggg aagttaatga    1980
   taggttttaa ttggaggttg gattttggat ttttttgggt ttgaggttga gttttgtgat    2040
   gaggggagtt attgtttgga ttttagttgg ggtggtgtaa agtttaggat ttttagtatt    2100
   ttatgggttt tggtggtaag tttatttttt ttatatgatt tttttttttt ttgagattga    2160
   gttttattgt tgtttgggtt ggagtgtagt ggtgtgattt tggtttattg taattttagt    2220
   tttttaggtt taagtgattt ttttgtttta gttttttgag tagttgggat tataggtatt    2280
   tgttattata tttggttgat tttttgtatt tttagtagag atgaggtttt gttatgttag    2340
   ttaggttggt tttgaatatt tgattttagg tgatttattt gttttggttt tttagaaagt    2400
   gttgggatta taggtatgag ttagtgtgtt ttgttttttt ttatattttt attgtttttt    2460
   tttttttgga gtttaattat ttttagtttt gattttaggg tttttttttt ttgggaaaat    2520
   ttaaggatgt aggagtttaa aattgatttt attttttagt agtatttaat tatttttttt    2580
   ttttagaaga ttttagtttt attttttatt tttattttta aagtggtaaa ggtagtattt    2640
   agttttttta aaaatattta gtggttagag gttttaaaat gtgtattaaa ttttatgggt    2700
   atgtatagta gagaaggtat ttttatagag gtaagagttt taatttggtg ataatggtag    2760
   tgagttattt gtttagtttt taattattta agatagtagt agttggttgt tgtttttgga    2820
   ttgagatttt tttttttttg ttggtggggg gtggttggaa tggatgggag atatagtggg    2880
   aggttgaggt tttttggggg tattattttg tttttggaag gtagtttttt taaaaggttg    2940
   ttttggtaat tgttgttttt taattttttt ggttttttta aggggttgat gatggttttt    3000
   a                                                                    3001
<210> 206
   <211> 3194
   <212> DNA
   <213> human
<400> 206
   aggctgcagt gagctgtgat gacgctactg cactccagcc cggatgacag agcaagtccc      60
   tgtctcaata ttttaagtca agggtctgta gtcatgtgtt caacgccact tcttgccatc     120
   actttgcaat tattgaaatg ggcatactga gctcagaaag caaatctgat gctttcacgg     180
   gagacagagc cacatttgtg ttctgggtgg gatcactagt gcaggagaac actacatctt     240
   cttctgaagg caaaatgctt gtaggttttg cctctttact ttgcatttac ttctaaaatt     300
   gcacttgttc acctaccagt gtttacgaaa tcctgtattt gggatgcttt ttctataata     360
   aaatattatc atttatggga aaaaaaaaaa aagaaagcct gctgatctgt gatctggtaa     420
   actccagcag ggtggagatc cctgaaggtc gcccctagac ccagcggtca tttcctagaa     480
   cattcggcca atcctcaccc agtgctgaat ctccgccaca ctgggctgca taggtagttc     540
   ccaggcccgc ccacaagggg tcataatagc tgagatgaga ggggaggccg ccaaaccaaa     600
   gcctgtcagt cacccgctcc ctcctcaagg cttgggttgg agtctgcaag acactacctc     660
   tgctctcact cctgtcctat cggcttggtt atcagccatc taaggaatcc acttggtata     720
   cagccagcac tcctggcatc acatcccttt cccttgcccc ttggggccgg gctcctctca     780
   gacagccaaa tcttcatcca cagaatgcac cgggcccgcc ccgcggcctc gcggtcctag     840
   ggagggccca tatcccagct tctccctgct cagtcccctg ctggctctcc actctccctc     900
   cccactggcg ctttatactg ccaagattcc cctgtcagcc ctgctctcca ccctcaccct     960
   agtgaattcc cttcactctt tcttttccca gtttgttgac ctactcaagc aatggcttga    1020
   ggtcccggag cttttcctat gaaaccacag caggtacctg gtttccatca atattctttg    1080
   ggaaaccttg gggtgtgaaa gaatcactcg ttgcaaggta cagcccctcc ctcaccagcc    1140
   ccttccagtt tcactcaatt tagtctccca aatttacgct atatccttgc ctcctacttt    1200
   tgaagtttat atccctaacc tgccccaggg ctgagaccgg tagggcagag ccccagtccc    1260
   tttcacaccg cacaaaaccc gcagttaagc ctggatggca gagacaagcc ggaaaagtag    1320
   gaatcccccc gtgttccgct cccagcgtcc ttcttcctgt cacgagcagg gcttctgcat    1380
   tctctgcgga ttctagtctc cctgcatcca attgcttcca agctcctcat tacacagcac    1440
   aaactttact ctcattcttt tcacccctga ttcttcggaa cccacaccaa ctcgcggccc    1500
   gttgtgagtg gtatgacaca gagagacctg tccccctttc ccaatcccta cctccgcttg    1560
   tactcgtccc gctcccgctt cactttggcc agcacgttgt agagagcgcg gatctcgggc    1620
   gtgatggtgt cgatctggac gcccacccca tccgggtgca cccacgacaa gccaggccct    1680
   tgcaccagag tgggctccag tcccggcccg agccggcggg cgtgcgagaa cgaccagatg    1740
   gtgccgggca tgaagcgggc cgacgaggaa taggtggtgg aggtggaggt ggaggacgac    1800
   gagaggctgg ccgcggaggg cgcgaggggg ccggccgggg agcgcgcggg cgagcccagc    1860
   acgcgcgccg aagggctgca gacagcggcc ggccgggcgc ccagctgcag ccccaggggc    1920
   cggatggggc tgacgaagcc ggtctgcact gcctggtcgc gacgacccag gccccgccgg    1980
   ccctgcttac cctcctccag cgcttgctgc agttgcttct ccaacagccg gttccggcgc    2040
   tccagctcat gcaccttggc caggaagcag cggaaccgga ggttcagggt cttgagcacg    2100
   ttgatgttgg agcccaggtc attgcggagg gccatggcgg caggcggggc cgggcccggc    2160
   ccgggcggcg agtaggcagc agggccggcc ggcgagagag gcgccggggg caagtctcct    2220
   cccccggcga agtggtcgcc tcccagtgag tcccccagtg gcccggccag gccctgctgc    2280
   tcctgctgca ggaggaagag gttggggccg aataacggat tcatggctgc gccttctgct    2340
   gggagatgca gaccggtgca ggagcaggga tggaaggcga gccagaagag ccaatgcggc    2400
   gccggcggga cagagccgac caatcaggcg gctcggcagc ggggcagagg tcagggggcg     2460
   ggccgagggg aagccaatga caggctccaa ttggaggccg gaccctggac ctttccgggt    2520
   ctgaggccga gccctgtgat gaggggagcc accgcctgga ctccagccgg ggtggcgtaa    2580
   agcccaggac ctccagtacc ccatgggttc tggtggcaag cccatctccc ctacacgact    2640
   tttttttttt ttgagaccga gtctcactgt cgcccgggct ggagtgcagt ggcgcgatct    2700
   tggctcactg caacctcagc ctcccaggtt caagcgattc tcctgcttca gtctcccgag    2760
   tagctgggat tacaggcacc cgccaccaca cccggctgat tttttgtatt tttagtagag    2820
   acgaggtttc gccatgttag ccaggctggt ctcgaacacc tgacctcagg tgatccaccc    2880
   gccttggcct cccagaaagt gctgggatta caggcatgag ctagtgtgcc ccgccttccc    2940
   ctacacttct attgccctcc tctccccgga gttcaatcac ccctagctct gactccaggg    3000
   ctttcccttc ctgggaaaac ccaaggatgc aggagcctaa aactgacctt atttctcagt    3060
   agcacccaac cattccctct cctcagaaga tcccagtccc acctcccatc tttaccctta    3120
   aagcggcaaa ggcagcaccc agttccctca aaaatactca gtggccagag gttttaaaat    3180
   gtgtattaaa tttc                                                     3194
<210> 207
   <211> 3194
   <212> DNA
   <213> human
<400> 207
   aggttgtagt gagttgtgat gacgttattg tattttagtt cggatgatag agtaagtttt      60
   tgttttaata ttttaagtta agggtttgta gttatgtgtt taacgttatt ttttgttatt     120
   attttgtaat tattgaaatg ggtatattga gtttagaaag taaatttgat gtttttacgg     180
   gagatagagt tatatttgtg ttttgggtgg gattattagt gtaggagaat attatatttt     240
   tttttgaagg taaaatgttt gtaggttttg tttttttatt ttgtatttat ttttaaaatt     300
   gtatttgttt atttattagt gtttacgaaa ttttgtattt gggatgtttt ttttataata     360
   aaatattatt atttatggga aaaaaaaaaa aagaaagttt gttgatttgt gatttggtaa     420
   attttagtag ggtggagatt tttgaaggtc gtttttagat ttagcggtta ttttttagaa      480
   tattcggtta atttttattt agtgttgaat tttcgttata ttgggttgta taggtagttt     540
   ttaggttcgt ttataagggg ttataatagt tgagatgaga ggggaggtcg ttaaattaaa     600
   gtttgttagt tattcgtttt ttttttaagg tttgggttgg agtttgtaag atattatttt     660
   tgtttttatt tttgttttat cggtttggtt attagttatt taaggaattt atttggtata     720
   tagttagtat ttttggtatt atattttttt tttttgtttt ttggggtcgg gtttttttta     780
   gatagttaaa tttttattta tagaatgtat cgggttcgtt tcgcggtttc gcggttttag      840
   ggagggttta tattttagtt tttttttgtt tagttttttg ttggtttttt attttttttt     900
   tttattggcg ttttatattg ttaagatttt tttgttagtt ttgtttttta tttttatttt     960
   agtgaatttt ttttattttt ttttttttta gtttgttgat ttatttaagt aatggtttga    1020
   ggtttcggag ttttttttat gaaattatag taggtatttg gtttttatta atattttttg    1080
   ggaaattttg gggtgtgaaa gaattattcg ttgtaaggta tagttttttt tttattagtt    1140
   ttttttagtt ttatttaatt tagtttttta aatttacgtt atatttttgt tttttatttt     1200
   tgaagtttat atttttaatt tgttttaggg ttgagatcgg tagggtagag ttttagtttt    1260
   ttttatatcg tataaaattc gtagttaagt ttggatggta gagataagtc ggaaaagtag    1320
   gaattttttc gtgtttcgtt tttagcgttt ttttttttgt tacgagtagg gtttttgtat    1380
   tttttgcgga ttttagtttt tttgtattta attgttttta agttttttat tatatagtat     1440
   aaattttatt tttatttttt ttatttttga tttttcggaa tttatattaa ttcgcggttc    1500
   gttgtgagtg gtatgatata gagagatttg tttttttttt ttaattttta ttttcgtttg    1560
   tattcgtttc gttttcgttt tattttggtt agtacgttgt agagagcgcg gatttcgggc    1620
   gtgatggtgt cgatttggac gtttatttta ttcgggtgta tttacgataa gttaggtttt    1680
   tgtattagag tgggttttag tttcggttcg agtcggcggg cgtgcgagaa cgattagatg    1740
   gtgtcgggta tgaagcgggt cgacgaggaa taggtggtgg aggtggaggt ggaggacgac    1800
   gagaggttgg tcgcggaggg cgcgaggggg tcggtcgggg agcgcgcggg cgagtttagt    1860
   acgcgcgtcg aagggttgta gatagcggtc ggtcgggcgt ttagttgtag ttttaggggt    1920
   cggatggggt tgacgaagtc ggtttgtatt gtttggtcgc gacgatttag gtttcgtcgg    1980
   ttttgtttat ttttttttag cgtttgttgt agttgttttt ttaatagtcg gtttcggcgt    2040
   tttagtttat gtattttggt taggaagtag cggaatcgga ggtttagggt tttgagtacg    2100
   ttgatgttgg agtttaggtt attgcggagg gttatggcgg taggcggggt cgggttcggt    2160
   tcgggcggcg agtaggtagt agggtcggtc ggcgagagag gcgtcggggg taagtttttt    2220
   ttttcggcga agtggtcgtt ttttagtgag ttttttagtg gttcggttag gttttgttgt    2280
   ttttgttgta ggaggaagag gttggggtcg aataacggat ttatggttgc gttttttgtt    2340
   gggagatgta gatcggtgta ggagtaggga tggaaggcga gttagaagag ttaatgcggc    2400
   gtcggcggga tagagtcgat taattaggcg gttcggtagc ggggtagagg ttagggggcg    2460
   ggtcgagggg aagttaatga taggttttaa ttggaggtcg gattttggat tttttcgggt    2520
   ttgaggtcga gttttgtgat gaggggagtt atcgtttgga ttttagtcgg ggtggcgtaa    2580
   agtttaggat ttttagtatt ttatgggttt tggtggtaag tttatttttt ttatacgatt    2640
   tttttttttt ttgagatcga gttttattgt cgttcgggtt ggagtgtagt ggcgcgattt    2700
   tggtttattg taattttagt tttttaggtt taagcgattt ttttgtttta gtttttcgag    2760
   tagttgggat tataggtatt cgttattata ttcggttgat tttttgtatt tttagtagag    2820
   acgaggtttc gttatgttag ttaggttggt ttcgaatatt tgattttagg tgatttattc    2880
   gttttggttt tttagaaagt gttgggatta taggtatgag ttagtgtgtt tcgttttttt    2940
   ttatattttt attgtttttt ttttttcgga gtttaattat ttttagtttt gattttaggg    3000
   tttttttttt ttgggaaaat ttaaggatgt aggagtttaa aattgatttt attttttagt    3060
   agtatttaat tatttttttt ttttagaaga ttttagtttt atttttattt tttattttta    3120
   aagcggtaaa ggtagtattt agttttttta aaaatattta gtggttagag gttttaaaat    3180
   gtgtattaaa tttt                                                       3194
<210> 208
   <211> 3194
   <212> DNA
   <213> human
<400> 208
   aggttgtagt gagttgtgat gatgttattg tattttagtt tggatgatag agtaagtttt 60
   tgttttaata ttttaagtta agggtttgta gttatgtgtt taatgttatt ttttgttatt 120
   attttgtaat tattgaaatg ggtatattga gtttagaaag taaatttgat gtttttatgg 180
   gagatagagt tatatttgtg ttttgggtgg gattattagt gtaggagaat attatatttt 240
   tttttgaagg taaaatgttt gtaggttttg tttttttatt ttgtatttat ttttaaaatt 300
   gtatttgttt atttattagt gtttatgaaa ttttgtattt gggatgtttt ttttataata 360
   aaatattatt atttatggga aaaaaaaaaa aagaaagttt gttgatttgt gatttggtaa 420
   attttagtag ggtggagatt tttgaaggtt gtttttagat ttagtggtta ttttttagaa 480
   tatttggtta atttttattt agtgttgaat ttttgttata ttgggttgta taggtagttt 540
   ttaggtttgt ttataagggg ttataatagt tgagatgaga ggggaggttg ttaaattaaa 600
   gtttgttagt tatttgtttt ttttttaagg tttgggttgg agtttgtaag atattatttt 660
   tgtttttatt tttgttttat tggtttggtt attagttatt taaggaattt atttggtata 720
   tagttagtat ttttggtatt atattttttt tttttgtttt ttggggttgg gtttttttta 780
   gatagttaaa tttttattta tagaatgtat tgggtttgtt ttgtggtttt gtggttttag 840
   ggagggttta tattttagtt tttttttgtt tagttttttg ttggtttttt attttttttt 900
   tttattggtg ttttatattg ttaagatttt tttgttagtt ttgtttttta tttttatttt 960
   agtgaatttt ttttattttt ttttttttta gtttgttgat ttatttaagt aatggtttga 1020
   ggttttggag ttttttttat gaaattatag taggtatttg gtttttatta atattttttg 1080
   ggaaattttg gggtgtgaaa gaattatttg ttgtaaggta tagttttttt tttattagtt 1140
   ttttttagtt ttatttaatt tagtttttta aatttatgtt atatttttgt tttttatttt 1200
   tgaagtttat atttttaatt tgttttaggg ttgagattgg tagggtagag ttttagtttt 1260
   ttttatattg tataaaattt gtagttaagt ttggatggta gagataagtt ggaaaagtag 1320
   gaattttttt gtgttttgtt tttagtgttt ttttttttgt tatgagtagg gtttttgtat 1380
   tttttgtgga ttttagtttt tttgtattta attgttttta agttttttat tatatagtat 1440
   aaattttatt tttatttttt ttatttttga ttttttggaa tttatattaa tttgtggttt 1500
   gttgtgagtg gtatgatata gagagatttg tttttttttt ttaattttta tttttgtttg 1560
   tatttgtttt gtttttgttt tattttggtt agtatgttgt agagagtgtg gattttgggt 1620
   gtgatggtgt tgatttggat gtttatttta tttgggtgta tttatgataa gttaggtttt 1680
   tgtattagag tgggttttag ttttggtttg agttggtggg tgtgtgagaa tgattagatg 1740
   gtgttgggta tgaagtgggt tgatgaggaa taggtggtgg aggtggaggt ggaggatgat 1800
   gagaggttgg ttgtggaggg tgtgaggggg ttggttgggg agtgtgtggg tgagtttagt 1860
   atgtgtgttg aagggttgta gatagtggtt ggttgggtgt ttagttgtag ttttaggggt 1920
   tggatggggt tgatgaagtt ggtttgtatt gtttggttgt gatgatttag gttttgttgg 1980
   ttttgtttat ttttttttag tgtttgttgt agttgttttt ttaatagttg gttttggtgt 2040
   tttagtttat gtattttggt taggaagtag tggaattgga ggtttagggt tttgagtatg 2100
   ttgatgttgg agtttaggtt attgtggagg gttatggtgg taggtggggt tgggtttggt 2160
   ttgggtggtg agtaggtagt agggttggtt ggtgagagag gtgttggggg taagtttttt 2220
   tttttggtga agtggttgtt ttttagtgag ttttttagtg gtttggttag gttttgttgt 2280
   ttttgttgta ggaggaagag gttggggttg aataatggat ttatggttgt gttttttgtt 2340
   gggagatgta gattggtgta ggagtaggga tggaaggtga gttagaagag ttaatgtggt 2400
   gttggtggga tagagttgat taattaggtg gtttggtagt ggggtagagg ttagggggtg 2460
   ggttgagggg aagttaatga taggttttaa ttggaggttg gattttggat ttttttgggt 2520
   ttgaggttga gttttgtgat gaggggagtt attgtttgga ttttagttgg ggtggtgtaa 2580
   agtttaggat ttttagtatt ttatgggttt tggtggtaag tttatttttt ttatatgatt 2640
   tttttttttt ttgagattga gttttattgt tgtttgggtt ggagtgtagt ggtgtgattt 2700
   tggtttattg taattttagt tttttaggtt taagtgattt ttttgtttta gttttttgag 2760
   tagttgggat tataggtatt tgttattata tttggttgat tttttgtatt tttagtagag 2820
   atgaggtttt gttatgttag ttaggttggt tttgaatatt tgattttagg tgatttattt 2880
   gttttggttt tttagaaagt gttgggatta taggtatgag ttagtgtgtt ttgttttttt 2940
   ttatattttt attgtttttt tttttttgga gtttaattat ttttagtttt gattttaggg 3000
   tttttttttt ttgggaaaat ttaaggatgt aggagtttaa aattgatttt attttttagt 3060
   agtatttaat tatttttttt ttttagaaga ttttagtttt attttttatt tttattttta 3120
   aagtggtaaa ggtagtattt agttttttta aaaatattta gtggttagag gttttaaaat 3180
   gtgtattaaa tttt 3194
<210> 209
   <211> 3194
   <212> DNA
   <213> human
<400> 209
   gaaatttaat atatatttta aaatttttgg ttattgagta tttttgaggg aattgggtgt      60
   tgtttttgtc gttttaaggg taaagatggg aggtgggatt gggatttttt gaggagaggg     120
   aatggttggg tgttattgag aaataaggtt agttttaggt ttttgtattt ttgggttttt     180
   ttaggaaggg aaagttttgg agttagagtt aggggtgatt gaatttcggg gagaggaggg     240
   taatagaagt gtaggggaag gcggggtata ttagtttatg tttgtaattt tagtattttt     300
   tgggaggtta aggcgggtgg attatttgag gttaggtgtt cgagattagt ttggttaata     360
   tggcgaaatt tcgtttttat taaaaatata aaaaattagt cgggtgtggt ggcgggtgtt     420
   tgtaatttta gttattcggg agattgaagt aggagaatcg tttgaatttg ggaggttgag     480
   gttgtagtga gttaagatcg cgttattgta ttttagttcg ggcgatagtg agattcggtt     540
   ttaaaaaaaa aaaaagtcgt gtaggggaga tgggtttgtt attagaattt atggggtatt     600
   ggaggttttg ggttttacgt tatttcggtt ggagtttagg cggtggtttt ttttattata     660
   gggttcggtt ttagattcgg aaaggtttag ggttcggttt ttaattggag tttgttattg     720
   gttttttttc ggttcgtttt ttgatttttg tttcgttgtc gagtcgtttg attggtcggt     780
   tttgtttcgt cggcgtcgta ttggtttttt tggttcgttt tttatttttg tttttgtatc     840
   ggtttgtatt ttttagtaga aggcgtagtt atgaattcgt tattcggttt taattttttt     900
   tttttgtagt aggagtagta gggtttggtc gggttattgg gggatttatt gggaggcgat     960
   tatttcgtcg ggggaggaga tttgttttcg gcgttttttt cgtcggtcgg ttttgttgtt    1020
   tattcgtcgt tcgggtcggg ttcggtttcg tttgtcgtta tggtttttcg taatgatttg    1080
   ggttttaata ttaacgtgtt taagattttg aattttcggt ttcgttgttt tttggttaag    1140
   gtgtatgagt tggagcgtcg gaatcggttg ttggagaagt aattgtagta agcgttggag    1200
   gagggtaagt agggtcggcg gggtttgggt cgtcgcgatt aggtagtgta gatcggtttc    1260
   gttagtttta ttcggttttt ggggttgtag ttgggcgttc ggtcggtcgt tgtttgtagt    1320
   ttttcggcgc gcgtgttggg ttcgttcgcg cgtttttcgg tcggtttttt cgcgtttttc    1380
   gcggttagtt tttcgtcgtt ttttattttt atttttatta tttatttttc gtcggttcgt    1440
   tttatgttcg gtattatttg gtcgttttcg tacgttcgtc ggttcgggtc gggattggag    1500
   tttattttgg tgtaagggtt tggtttgtcg tgggtgtatt cggatggggt gggcgtttag    1560
   atcgatatta ttacgttcga gattcgcgtt ttttataacg tgttggttaa agtgaagcgg    1620
   gagcgggacg agtataagcg gaggtaggga ttgggaaagg gggataggtt tttttgtgtt    1680
   atattattta taacgggtcg cgagttggtg tgggtttcga agaattaggg gtgaaaagaa    1740
   tgagagtaaa gtttgtgttg tgtaatgagg agtttggaag taattggatg tagggagatt    1800
   agaattcgta gagaatgtag aagttttgtt cgtgatagga agaaggacgt tgggagcgga    1860
   atacgggggg atttttattt tttcggtttg tttttgttat ttaggtttaa ttgcgggttt    1920
   tgtgcggtgt gaaagggatt ggggttttgt tttatcggtt ttagttttgg ggtaggttag    1980
   ggatataaat tttaaaagta ggaggtaagg atatagcgta aatttgggag attaaattga    2040
   gtgaaattgg aaggggttgg tgagggaggg gttgtatttt gtaacgagtg atttttttat    2100
   attttaaggt tttttaaaga atattgatgg aaattaggta tttgttgtgg ttttatagga    2160
   aaagtttcgg gattttaagt tattgtttga gtaggttaat aaattgggaa aagaaagagt    2220
   gaagggaatt tattagggtg agggtggaga gtagggttga taggggaatt ttggtagtat    2280
   aaagcgttag tggggaggga gagtggagag ttagtagggg attgagtagg gagaagttgg    2340
   gatatgggtt ttttttagga tcgcgaggtc gcggggcggg ttcggtgtat tttgtggatg    2400
   aagatttggt tgtttgagag gagttcggtt ttaaggggta agggaaaggg atgtgatgtt    2460
   aggagtgttg gttgtatatt aagtggattt tttagatggt tgataattaa gtcgatagga    2520
   taggagtgag agtagaggta gtgttttgta gattttaatt taagttttga ggagggagcg    2580
   ggtgattgat aggttttggt ttggcggttt ttttttttat tttagttatt atgatttttt    2640
   gtgggcgggt ttgggaatta tttatgtagt ttagtgtggc ggagatttag tattgggtga    2700
   ggattggtcg aatgttttag gaaatgatcg ttgggtttag ggcgattttt tagggatttt    2760
   tattttgttg gagtttatta gattatagat tagtaggttt tttttttttt ttttttttat    2820
   aaatgataat attttattat agaaaaagta ttttaaatat aggatttcgt aaatattggt    2880
   aggtgaataa gtgtaatttt agaagtaaat gtaaagtaaa gaggtaaaat ttataagtat    2940
   tttgttttta gaagaagatg tagtgttttt ttgtattagt gattttattt agaatataaa    3000
   tgtggttttg tttttcgtga aagtattaga tttgtttttt gagtttagta tgtttatttt    3060
   aataattgta aagtgatggt aagaagtggc gttgaatata tgattataga tttttgattt    3120
   aaaatattga gatagggatt tgttttgtta ttcgggttgg agtgtagtag cgttattata    3180
   gtttattgta gttt                                                      3194
<210> 210
   <211> 3194
   <212> DNA
   <213> human
<400> 210
   gaaatttaat atatatttta aaatttttgg ttattgagta tttttgaggg aattgggtgt      60
   tgtttttgtt gttttaaggg taaagatggg aggtgggatt gggatttttt gaggagaggg     120
   aatggttggg tgttattgag aaataaggtt agttttaggt ttttgtattt ttgggttttt     180
   ttaggaaggg aaagttttgg agttagagtt aggggtgatt gaattttggg gagaggaggg     240
   taatagaagt gtaggggaag gtggggtata ttagtttatg tttgtaattt tagtattttt     300
   tgggaggtta aggtgggtgg attatttgag gttaggtgtt tgagattagt ttggttaata     360
   tggtgaaatt ttgtttttat taaaaatata aaaaattagt tgggtgtggt ggtgggtgtt     420
   tgtaatttta gttatttggg agattgaagt aggagaattg tttgaatttg ggaggttgag     480
   gttgtagtga gttaagattg tgttattgta ttttagtttg ggtgatagtg agatttggtt     540
   ttaaaaaaaa aaaaagttgt gtaggggaga tgggtttgtt attagaattt atggggtatt     600
   ggaggttttg ggttttatgt tattttggtt ggagtttagg tggtggtttt ttttattata     660
   gggtttggtt ttagatttgg aaaggtttag ggtttggttt ttaattggag tttgttattg     720
   gttttttttt ggtttgtttt ttgatttttg ttttgttgtt gagttgtttg attggttggt      780
   tttgttttgt tggtgttgta ttggtttttt tggtttgttt tttatttttg tttttgtatt     840
   ggtttgtatt ttttagtaga aggtgtagtt atgaatttgt tatttggttt taattttttt     900
   tttttgtagt aggagtagta gggtttggtt gggttattgg gggatttatt gggaggtgat     960
   tattttgttg ggggaggaga tttgtttttg gtgttttttt tgttggttgg ttttgttgtt    1020
   tatttgttgt ttgggttggg tttggttttg tttgttgtta tggttttttg taatgatttg   1080
   ggttttaata ttaatgtgtt taagattttg aatttttggt tttgttgttt tttggttaag   1140
   gtgtatgagt tggagtgttg gaattggttg ttggagaagt aattgtagta agtgttggag    1200
   gagggtaagt agggttggtg gggtttgggt tgttgtgatt aggtagtgta gattggtttt    1260
   gttagtttta tttggttttt ggggttgtag ttgggtgttt ggttggttgt tgtttgtagt    1320
   tttttggtgt gtgtgttggg tttgtttgtg tgttttttgg ttggtttttt tgtgtttttt    1380
   gtggttagtt ttttgttgtt ttttattttt atttttatta tttatttttt gttggtttgt    1440
   tttatgtttg gtattatttg gttgtttttg tatgtttgtt ggtttgggtt gggattggag    1500
   tttattttgg tgtaagggtt tggtttgttg tgggtgtatt tggatggggt gggtgtttag    1560
   attgatatta ttatgtttga gatttgtgtt ttttataatg tgttggttaa agtgaagtgg    1620
   gagtgggatg agtataagtg gaggtaggga ttgggaaagg gggataggtt tttttgtgtt    1680
   atattattta taatgggttg tgagttggtg tgggttttga agaattaggg gtgaaaagaa    1740
   tgagagtaaa gtttgtgttg tgtaatgagg agtttggaag taattggatg tagggagatt    1800
   agaatttgta gagaatgtag aagttttgtt tgtgatagga agaaggatgt tgggagtgga    1860
   atatgggggg atttttattt ttttggtttg tttttgttat ttaggtttaa ttgtgggttt    1920
   tgtgtggtgt gaaagggatt ggggttttgt tttattggtt ttagttttgg ggtaggttag    1980
   ggatataaat tttaaaagta ggaggtaagg atatagtgta aatttgggag attaaattga    2040
   gtgaaattgg aaggggttgg tgagggaggg gttgtatttt gtaatgagtg atttttttat     2100
   attttaaggt tttttaaaga atattgatgg aaattaggta tttgttgtgg ttttatagga    2160
   aaagttttgg gattttaagt tattgtttga gtaggttaat aaattgggaa aagaaagagt    2220
   gaagggaatt tattagggtg agggtggaga gtagggttga taggggaatt ttggtagtat    2280
   aaagtgttag tggggaggga gagtggagag ttagtagggg attgagtagg gagaagttgg    2340
   gatatgggtt ttttttagga ttgtgaggtt gtggggtggg tttggtgtat tttgtggatg    2400
   aagatttggt tgtttgagag gagtttggtt ttaaggggta agggaaaggg atgtgatgtt    2460
   aggagtgttg gttgtatatt aagtggattt tttagatggt tgataattaa gttgatagga    2520
   taggagtgag agtagaggta gtgttttgta gattttaatt taagttttga ggagggagtg    2580
   ggtgattgat aggttttggt ttggtggttt ttttttttat tttagttatt atgatttttt     2640
   gtgggtgggt ttgggaatta tttatgtagt ttagtgtggt ggagatttag tattgggtga    2700
   ggattggttg aatgttttag gaaatgattg ttgggtttag gggtgatttt tagggatttt    2760
   tattttgttg gagtttatta gattatagat tagtaggttt tttttttttt ttttttttat    2820
   aaatgataat attttattat agaaaaagta ttttaaatat aggattttgt aaatattggt    2880
   aggtgaataa gtgtaatttt agaagtaaat gtaaagtaaa gaggtaaaat ttataagtat    2940
   tttgttttta gaagaagatg tagtgttttt ttgtattagt gattttattt agaatataaa    3000
   tgtggttttg ttttttgtga aagtattaga tttgtttttt gagtttagta tgtttatttt     3060
   aataattgta aagtgatggt aagaagtggt gttgaatata tgattataga tttttgattt    3120
   aaaatattga gatagggatt tgttttgtta tttgggttgg agtgtagtag tgttattata    3180
   gtttattgta gttt                                                      3194
<210> 211
   <211> 50
   <212> DNA
   <213> human
<400> 211
   cgacaggtgg gtcctggctt ggaactgttc tgcttagtgt ttgaggccac 50
<210> 212
   <211> 50
   <212> DNA
   <213> human
<400> 212
   cgataggtgg gttttggttt ggaattgttt tgtttagtgt ttgaggttat 50
<210> 213
   <211> 50
   <212> DNA
   <213> human
<400> 213
   tgataggtgg gttttggttt ggaattgttt tgtttagtgt ttgaggttat 50
<210> 214
   <211> 50
   <212> DNA
   <213> human
<400> 214
   gtggttttaa atattaagta gaatagtttt aagttaggat ttatttgtcg 50
<210> 215
   <211> 50
   <212> DNA
   <213> human
<400> 215
   gtggttttaa atattaagta gaatagtttt aagttaggat ttatttgttg 50
<210> 216
   <211> 3001
   <212> DNA
   <213> human
<400> 216
   ctcacagggt ccttggccac agtaaagagg aggtctcctt cgcggttata cttaatctgc 60
   gtaatggacc gctcatggcc ctgcagtagg atcggcttct gtggggacaa gacagtaacg 120
   tcagtgctcg gaattgggcc ccagcctcag cccaggaccc ccactgcaat acccctaact 180
   ctgaaactca ccatcccggc tgtgacgcga ggaaggccgc aacgtgagta agaccggaaa 240
   aggtttcgag gtcacttccg gattatgggt ctccgcccct tcgttcccgg aagtagaaga 300
   cagcggcgtt gccatggcgg cgtctctggg taggtagccg gccccgccct tccatggatt 360
   tcccgaccct cgcctcgaat tcatttcctg ctccacatcc accctctaac cccgacccct 420
   gctacaaccc cagaggccgg actcctggat tcatctccct agcttccctg ggcctgcctt 480
   agccgccagt cgcagccgag gcgaagagag cgaaggaggg aagtgggggc ggctagctgg 540
   ggctagaagg ccaggagagg gcggggtggg cggccgtttg gggtgggggt cagggtgact 600
   cactcgtctg cattcagggc aggtgttggc tctggtgctg gtggccgctc tgtggggtgg 660
   cacgcagccg ctgctgaagc gggcctccgc cggcctgcag cgggttcatg agccgacctg 720
   ggcccagcag ttgctacagg agatgaagac cctcttcttg aatactgagg tgcgtctctg 780
   tgaaaggccc ctctctcggt ggagttgccg ccaagaattt ggtctttgga gtcaaacagg 840
   tctgggtcag ccccaccact tactcactag aaattctcgg aaaagtcagt tgtccttgag 900
   cctcagtttc atcatctgta aatggcgatt ataataataa ccatctgata ggtcagttgc 960
   aggaattagg tgagatacgt agagcactta gcattaggcc tagaacatag taagtgctca 1020
   atctatggct gccattattc acattttatt gtgattcctt cagttacata aggcaacatt 1080
   tgtgagcctt tccagaggga gggcttgggg ctcaaattgg ccctgcaggg gaggagttca 1140
   gaggaggaag gctggagctg gaggacttgg ggaaggcttc ctggaggggc tgacattggc 1200
   ttcaaggcct tgcaggatgg gaactagcag aggtgaggga gagggtagtt tgagcaaagg 1260
   cacagacttc agtctgtttc cctctttgcc ctggaaggat cctccttaga gggattagtg 1320
   ttctctgggt ccttataacc ctgcctccct ccaaggaaag acattcgcta ggagctcaaa 1380
   gaaggcagtg ctgctcagtc aggccatcac actgactgca ggtttctcct ctttccagta 1440
   cctgatgccc tttctcctca accagtgtgg atcccttctc tattacctca ccttggcatc 1500
   gacaggtggg tcctggcttg gaactgttct gcttagtgtt tgaggccacc tggaagagtg 1560
   attggggggg gcggggtgga gcttgaaaca cctcattccc atcagtgaga gagggctgac 1620
   tagctctagt tccctcttac tatcctcttc tctgaacaag aggggcacag tgtgctggtt 1680
   atgagtatgt tgctaggcac tggagttggt ttgaagcctg gcagtcatgt gtgaccagcg 1740
   gaggaagaag gaaggttcat tttgctgtgc caagtgctgg ctttcacatt cttgcttcca 1800
   ctgtgtgcta cctacctgac catgagcaag tcacccattt gagccttggt tttatatagt 1860
   tggcttcata gcattgtgag gattaaattt aatgaatggg aaacatctag gagagtgcct 1920
   ggcacacagt cagagctcca tatacataaa tttcctttct cttgacattt gtgactgccc 1980
   tctactacgt cagatactgc actagtctta cacattattt aattccaatc atgactctgt 2040
   aaagcagata tgattatctt agaaaatgaa gttgcaaaac tttgtgcaat ttcacctctc 2100
   aagtcccaca ggatgtcagt gcagaaggat ttgaagccat atattaaaag tttatgctcg 2160
   actgggcatg gtgactcacg cctgtaatcc cagcactttg ggaggccaag gagggcagat 2220
   catttgaggt caggagttcg agaccagtct ggccaacatg ctgaaacccc gtctctacta 2280
   aaaataacaa aattagttgg gtgtggtggt gtgtgcctgt agtcccagct acttgggagg 2340
   ctgaggcgcg agaatggctt gaacctggga ggcggaggtt gcagtgagct gagatcatgc 2400
   cactgcactc cagtccctgg gcaacagagc gagactctgt ctcaaaaaca gaaagaaaga 2460
   aataatatgc acttatttaa agagtttgaa aacctctggc ctcatgcatt cactgctgtg 2520
   gaagaatgct gaacccttga ccagctgact acaggtagcc attcaccaag ttttaatcaa 2580
   attgcagttt taggttcttt ttatctcatt accctttctg cctgtatcct ttaatatcca 2640
   tcctttctaa taaccagaac agtaataact atttatgaag catccacttc agccagggac 2700
   tatccatgca ttatcacagt tcatcttacg acaacccaac aaaagaaatg gtatactgtg 2760
   acccctattt tacatgcggg gaaacaggtt gagtgtttga atatcctaac caagattgca 2820
   agggaaactc agatctgcct cactccaatg ccctcacttt ccactgcacc gtgctgcatc 2880
   cttcttccat ctactgttgc aaaccagacc ttagcagcca tgacactcct tgtaaggtat 2940
   gtaaacttaa gaaggcagct ctgggcaggg cacagtggct caggcctata attccagcac 3000
   t 3001

<210> 217
   <211> 3001
   <212> DNA
   <213> human
<400> 217
   tttatagggt ttttggttat agtaaagagg aggttttttt cgcggttata tttaatttgc      60
   gtaatggatc gtttatggtt ttgtagtagg atcggttttt gtggggataa gatagtaacg     120
   ttagtgttcg gaattgggtt ttagttttag tttaggattt ttattgtaat atttttaatt     180
   ttgaaattta ttatttcggt tgtgacgcga ggaaggtcgt aacgtgagta agatcggaaa     240
   aggtttcgag gttattttcg gattatgggt tttcgttttt tcgttttcgg aagtagaaga     300
   tagcggcgtt gttatggcgg cgtttttggg taggtagtcg gtttcgtttt tttatggatt     360
   tttcgatttt cgtttcgaat ttattttttg ttttatattt attttttaat ttcgattttt     420
   gttataattt tagaggtcgg atttttggat ttattttttt agtttttttg ggtttgtttt     480
   agtcgttagt cgtagtcgag gcgaagagag cgaaggaggg aagtgggggc ggttagttgg     540
   ggttagaagg ttaggagagg gcggggtggg cggtcgtttg gggtgggggt tagggtgatt     600
   tattcgtttg tatttagggt aggtgttggt tttggtgttg gtggtcgttt tgtggggtgg     660
   tacgtagtcg ttgttgaagc gggttttcgt cggtttgtag cgggtttatg agtcgatttg     720
   ggtttagtag ttgttatagg agatgaagat tttttttttg aatattgagg tgcgtttttg     780
   tgaaaggttt ttttttcggt ggagttgtcg ttaagaattt ggtttttgga gttaaatagg      840
   tttgggttag ttttattatt tatttattag aaattttcgg aaaagttagt tgtttttgag     900
   ttttagtttt attatttgta aatggcgatt ataataataa ttatttgata ggttagttgt     960
   aggaattagg tgagatacgt agagtattta gtattaggtt tagaatatag taagtgttta    1020
   atttatggtt gttattattt atattttatt gtgatttttt tagttatata aggtaatatt    1080
   tgtgagtttt tttagaggga gggtttgggg tttaaattgg ttttgtaggg gaggagttta    1140
   gaggaggaag gttggagttg gaggatttgg ggaaggtttt ttggaggggt tgatattggt    1200
   tttaaggttt tgtaggatgg gaattagtag aggtgaggga gagggtagtt tgagtaaagg    1260
   tatagatttt agtttgtttt tttttttgtt ttggaaggat tttttttaga gggattagtg     1320
   ttttttgggt ttttataatt ttgttttttt ttaaggaaag atattcgtta ggagtttaaa  1380
   gaaggtagtg ttgtttagtt aggttattat attgattgta ggtttttttt ttttttagta     1440
   tttgatgttt ttttttttta attagtgtgg attttttttt tattatttta ttttggtatc    1500
   gataggtggg ttttggtttg gaattgtttt gtttagtgtt tgaggttatt tggaagagtg    1560
   attggggggg gcggggtgga gtttgaaata ttttattttt attagtgaga gagggttgat    1620
   tagttttagt ttttttttat tatttttttt tttgaataag aggggtatag tgtgttggtt   1680
   atgagtatgt tgttaggtat tggagttggt ttgaagtttg gtagttatgt gtgattagcg    1740
   gaggaagaag gaaggtttat tttgttgtgt taagtgttgg tttttatatt tttgttttta    1800
   ttgtgtgtta tttatttgat tatgagtaag ttatttattt gagttttggt tttatatagt    1860
   tggttttata gtattgtgag gattaaattt aatgaatggg aaatatttag gagagtgttt   1920
   ggtatatagt tagagtttta tatatataaa tttttttttt tttgatattt gtgattgttt   1980
   tttattacgt tagatattgt attagtttta tatattattt aattttaatt atgattttgt    2040
   aaagtagata tgattatttt agaaaatgaa gttgtaaaat tttgtgtaat tttatttttt    2100
   aagttttata ggatgttagt gtagaaggat ttgaagttat atattaaaag tttatgttcg    2160
   attgggtatg gtgatttacg tttgtaattt tagtattttg ggaggttaag gagggtagat   2220
   tatttgaggt taggagttcg agattagttt ggttaatatg ttgaaatttc gtttttatta    2280
   aaaataataa aattagttgg gtgtggtggt gtgtgtttgt agttttagtt atttgggagg    2340
   ttgaggcgcg agaatggttt gaatttggga ggcggaggtt gtagtgagtt gagattatgt    2400
   tattgtattt tagtttttgg gtaatagagc gagattttgt tttaaaaata gaaagaaaga    2460
   aataatatgt atttatttaa agagtttgaa aatttttggt tttatgtatt tattgttgtg    2520
   gaagaatgtt gaatttttga ttagttgatt ataggtagtt atttattaag ttttaattaa    2580
   attgtagttt taggtttttt ttattttatt attttttttg tttgtatttt ttaatattta   2640
   ttttttttaa taattagaat agtaataatt atttatgaag tatttatttt agttagggat   2700
   tatttatgta ttattatagt ttattttacg ataatttaat aaaagaaatg gtatattgtg    2760
   atttttattt tatatgcggg gaaataggtt gagtgtttga atattttaat taagattgta   2820
   agggaaattt agatttgttt tattttaatg tttttatttt ttattgtatc gtgttgtatt    2880
   ttttttttat ttattgttgt aaattagatt ttagtagtta tgatattttt tgtaaggtat    2940
   gtaaatttaa gaaggtagtt ttgggtaggg tatagtggtt taggtttata attttagtat     3000
   t                                                                    3001
<210> 218
   <211> 3001
   <212> DNA
   <213> human
<400> 218
   tttatagggt ttttggttat agtaaagagg aggttttttt tgtggttata tttaatttgt 60
   gtaatggatt gtttatggtt ttgtagtagg attggttttt gtggggataa gatagtaatg 120
   ttagtgtttg gaattgggtt ttagttttag tttaggattt ttattgtaat atttttaatt 180
   ttgaaattta ttattttggt tgtgatgtga ggaaggttgt aatgtgagta agattggaaa 240
   aggttttgag gttatttttg gattatgggt ttttgttttt ttgtttttgg aagtagaaga 300
   tagtggtgtt gttatggtgg tgtttttggg taggtagttg gttttgtttt tttatggatt 360
   ttttgatttt tgttttgaat ttattttttg ttttatattt attttttaat tttgattttt 420
   gttataattt tagaggttgg atttttggat ttattttttt agtttttttg ggtttgtttt 480
   agttgttagt tgtagttgag gtgaagagag tgaaggaggg aagtgggggt ggttagttgg 540
   ggttagaagg ttaggagagg gtggggtggg tggttgtttg gggtgggggt tagggtgatt 600
   tatttgtttg tatttagggt aggtgttggt tttggtgttg gtggttgttt tgtggggtgg 660
   tatgtagttg ttgttgaagt gggtttttgt tggtttgtag tgggtttatg agttgatttg 720
   ggtttagtag ttgttatagg agatgaagat tttttttttg aatattgagg tgtgtttttg 780
   tgaaaggttt tttttttggt ggagttgttg ttaagaattt ggtttttgga gttaaatagg 840
   tttgggttag ttttattatt tatttattag aaatttttgg aaaagttagt tgtttttgag 900
   ttttagtttt attatttgta aatggtgatt ataataataa ttatttgata ggttagttgt 960
   aggaattagg tgagatatgt agagtattta gtattaggtt tagaatatag taagtgttta 1020
   atttatggtt gttattattt atattttatt gtgatttttt tagttatata aggtaatatt 1080
   tgtgagtttt tttagaggga gggtttgggg tttaaattgg ttttgtaggg gaggagttta 1140
   gaggaggaag gttggagttg gaggatttgg ggaaggtttt ttggaggggt tgatattggt 1200
   tttaaggttt tgtaggatgg gaattagtag aggtgaggga gagggtagtt tgagtaaagg 1260
   tatagatttt agtttgtttt tttttttgtt ttggaaggat tttttttaga gggattagtg 1320
   ttttttgggt ttttataatt ttgttttttt ttaaggaaag atatttgtta ggagtttaaa 1380
   gaaggtagtg ttgtttagtt aggttattat attgattgta ggtttttttt ttttttagta 1440
   tttgatgttt ttttttttta attagtgtgg attttttttt tattatttta ttttggtatt 1500
   gataggtggg ttttggtttg gaattgtttt gtttagtgtt tgaggttatt tggaagagtg 1560
   attggggggg gtggggtgga gtttgaaata ttttattttt attagtgaga gagggttgat 1620
   tagttttagt ttttttttat tatttttttt tttgaataag aggggtatag tgtgttggtt 1680
   atgagtatgt tgttaggtat tggagttggt ttgaagtttg gtagttatgt gtgattagtg 1740
   gaggaagaag gaaggtttat tttgttgtgt taagtgttgg tttttatatt tttgttttta 1800
   ttgtgtgtta tttatttgat tatgagtaag ttatttattt gagttttggt tttatatagt 1860
   tggttttata gtattgtgag gattaaattt aatgaatggg aaatatttag gagagtgttt 1920
   ggtatatagt tagagtttta tatatataaa tttttttttt tttgatattt gtgattgttt 1980
   tttattatgt tagatattgt attagtttta tatattattt aattttaatt atgattttgt 2040
   aaagtagata tgattatttt agaaaatgaa gttgtaaaat tttgtgtaat tttatttttt 2100
   aagttttata ggatgttagt gtagaaggat ttgaagttat atattaaaag tttatgtttg 2160
   attgggtatg gtgatttatg tttgtaattt tagtattttg ggaggttaag gagggtagat 2220
   tatttgaggt taggagtttg agattagttt ggttaatatg ttgaaatttt gtttttatta 2280
   aaaataataa aattagttgg gtgtggtggt gtgtgtttgt agttttagtt atttgggagg 2340
   ttgaggtgtg agaatggttt gaatttggga ggtggaggtt gtagtgagtt gagattatgt 2400
   tattgtattt tagtttttgg gtaatagagt gagattttgt tttaaaaata gaaagaaaga 2460
   aataatatgt atttatttaa agagtttgaa aatttttggt tttatgtatt tattgttgtg 2520
   gaagaatgtt gaatttttga ttagttgatt ataggtagtt atttattaag ttttaattaa 2580
   attgtagttt taggtttttt ttattttatt attttttttg tttgtatttt ttaatattta 2640
   ttttttttaa taattagaat agtaataatt atttatgaag tatttatttt agttagggat 2700
   tatttatgta ttattatagt ttattttatg ataatttaat aaaagaaatg gtatattgtg 2760
   atttttattt tatatgtggg gaaataggtt gagtgtttga atattttaat taagattgta 2820
   agggaaattt agatttgttt tattttaatg tttttatttt ttattgtatt gtgttgtatt 2880
   ttttttttat ttattgttgt aaattagatt ttagtagtta tgatattttt tgtaaggtat 2940
   gtaaatttaa gaaggtagtt ttgggtaggg tatagtggtt taggtttata attttagtat 3000
   t 3001
<210> 219
   <211> 3001
   <212> DNA
   <213> human
<400> 219
   agtgttggaa ttataggttt gagttattgt gttttgttta gagttgtttt tttaagttta 60
   tatattttat aaggagtgtt atggttgtta aggtttggtt tgtaatagta gatggaagaa 120
   ggatgtagta cggtgtagtg gaaagtgagg gtattggagt gaggtagatt tgagtttttt 180
   ttgtaatttt ggttaggata tttaaatatt taatttgttt tttcgtatgt aaaatagggg 240
   ttatagtata ttattttttt tgttgggttg tcgtaagatg aattgtgata atgtatggat 300
   agtttttggt tgaagtggat gttttataaa tagttattat tgttttggtt attagaaagg 360
   atggatatta aaggatatag gtagaaaggg taatgagata aaaagaattt aaaattgtaa 420
   tttgattaaa atttggtgaa tggttatttg tagttagttg gttaagggtt tagtattttt 480
   ttatagtagt gaatgtatga ggttagaggt ttttaaattt tttaaataag tgtatattat 540
   tttttttttt ttgtttttga gatagagttt cgttttgttg tttagggatt ggagtgtagt 600
   ggtatgattt tagtttattg taattttcgt tttttaggtt taagttattt tcgcgtttta 660
   gttttttaag tagttgggat tataggtata tattattata tttaattaat tttgttattt 720
   ttagtagaga cggggtttta gtatgttggt tagattggtt tcgaattttt gattttaaat 780
   gatttgtttt ttttggtttt ttaaagtgtt gggattatag gcgtgagtta ttatgtttag 840
   tcgagtataa atttttaata tatggtttta aatttttttg tattgatatt ttgtgggatt 900
   tgagaggtga aattgtataa agttttgtaa ttttattttt taagataatt atatttgttt 960
   tatagagtta tgattggaat taaataatgt gtaagattag tgtagtattt gacgtagtag 1020
   agggtagtta taaatgttaa gagaaaggaa atttatgtat atggagtttt gattgtgtgt 1080
   taggtatttt tttagatgtt ttttatttat taaatttaat ttttataatg ttatgaagtt 1140
   aattatataa aattaaggtt taaatgggtg atttgtttat ggttaggtag gtagtatata 1200
   gtggaagtaa gaatgtgaaa gttagtattt ggtatagtaa aatgaatttt tttttttttt 1260
   tcgttggtta tatatgattg ttaggtttta aattaatttt agtgtttagt aatatattta 1320
   taattagtat attgtgtttt ttttgtttag agaagaggat agtaagaggg aattagagtt 1380
   agttagtttt tttttattga tgggaatgag gtgttttaag ttttatttcg ttttttttaa 1440
   ttattttttt aggtggtttt aaatattaag tagaatagtt ttaagttagg atttatttgt 1500
   cgatgttaag gtgaggtaat agagaaggga tttatattgg ttgaggagaa agggtattag 1560
   gtattggaaa gaggagaaat ttgtagttag tgtgatggtt tgattgagta gtattgtttt 1620
   ttttgagttt ttagcgaatg tttttttttg gagggaggta gggttataag gatttagaga 1680
   atattaattt ttttaaggag gattttttta gggtaaagag ggaaatagat tgaagtttgt 1740
   gtttttgttt aaattatttt tttttttatt tttgttagtt tttattttgt aaggttttga 1800
   agttaatgtt agttttttta ggaagttttt tttaagtttt ttagttttag tttttttttt 1860
   ttgaattttt tttttgtagg gttaatttga gttttaagtt ttttttttgg aaaggtttat 1920
   aaatgttgtt ttatgtaatt gaaggaatta taataaaatg tgaataatgg tagttataga 1980
   ttgagtattt attatgtttt aggtttaatg ttaagtgttt tacgtatttt atttaatttt 2040
   tgtaattgat ttattagatg gttattatta taatcgttat ttatagatga tgaaattgag 2100
   gtttaaggat aattgatttt ttcgagaatt tttagtgagt aagtggtggg gttgatttag 2160
   atttgtttga ttttaaagat taaatttttg gcggtaattt tatcgagaga ggggtttttt 2220
   atagagacgt attttagtat ttaagaagag ggtttttatt ttttgtagta attgttgggt 2280
   ttaggtcggt ttatgaattc gttgtaggtc ggcggaggtt cgttttagta gcggttgcgt 2340
   gttattttat agagcggtta ttagtattag agttaatatt tgttttgaat gtagacgagt 2400
   gagttatttt gattttattt ttaaacggtc gtttatttcg tttttttttg gttttttagt 2460
   tttagttagt cgtttttatt tttttttttc gtttttttcg tttcggttgc gattggcggt 2520
   taaggtaggt ttagggaagt tagggagatg aatttaggag ttcggttttt ggggttgtag 2580
   taggggtcgg ggttagaggg tggatgtgga gtaggaaatg aattcgaggc gagggtcggg 2640
   aaatttatgg aagggcgggg tcggttattt atttagagac gtcgttatgg taacgtcgtt 2700
   gttttttatt ttcgggaacg aaggggcgga gatttataat tcggaagtga tttcgaaatt 2760
   tttttcggtt ttatttacgt tgcggttttt ttcgcgttat agtcgggatg gtgagtttta 2820
   gagttagggg tattgtagtg ggggttttgg gttgaggttg gggtttaatt tcgagtattg 2880
   acgttattgt tttgttttta tagaagtcga ttttattgta gggttatgag cggtttatta 2940
   cgtagattaa gtataatcgc gaaggagatt ttttttttat tgtggttaag gattttgtga 3000
   g 3001
<210> 220
   <211> 3001
   <212> DNA
   <213> human
<400> 220
   agtgttggaa ttataggttt gagttattgt gttttgttta gagttgtttt tttaagttta     60
   tatattttat aaggagtgtt atggttgtta aggtttggtt tgtaatagta gatggaagaa    120
   ggatgtagta tggtgtagtg gaaagtgagg gtattggagt gaggtagatt tgagtttttt    180
   ttgtaatttt ggttaggata tttaaatatt taatttgttt ttttgtatgt aaaatagggg    240
   ttatagtata ttattttttt tgttgggttg ttgtaagatg aattgtgata atgtatggat    300
   agtttttggt tgaagtggat gttttataaa tagttattat tgttttggtt attagaaagg    360
   atggatatta aaggatatag gtagaaaggg taatgagata aaaagaattt aaaattgtaa    420
   tttgattaaa atttggtgaa tggttatttg tagttagttg gttaagggtt tagtattttt    480
   ttatagtagt gaatgtatga ggttagaggt ttttaaattt tttaaataag tgtatattat    540
   tttttttttt ttgtttttga gatagagttt tgttttgttg tttagggatt ggagtgtagt    600
   ggtatgattt tagtttattg taatttttgt tttttaggtt taagttattt ttgtgtttta    660
   gttttttaag tagttgggat tataggtata tattattata tttaattaat tttgttattt    720
   ttagtagaga tggggtttta gtatgttggt tagattggtt ttgaattttt gattttaaat    780
   gatttgtttt ttttggtttt ttaaagtgtt gggattatag gtgtgagtta ttatgtttag    840
   ttgagtataa atttttaata tatggtttta aatttttttg tattgatatt ttgtgggatt    900
   tgagaggtga aattgtataa agttttgtaa ttttattttt taagataatt atatttgttt    960
   tatagagtta tgattggaat taaataatgt gtaagattag tgtagtattt gatgtagtag   1020
   agggtagtta taaatgttaa gagaaaggaa atttatgtat atggagtttt gattgtgtgt   1080
   taggtatttt tttagatgtt ttttatttat taaatttaat ttttataatg ttatgaagtt   1140
   aattatataa aattaaggtt taaatgggtg atttgtttat ggttaggtag gtagtatata   1200
   gtggaagtaa gaatgtgaaa gttagtattt ggtatagtaa aatgaatttt tttttttttt   1260
   ttgttggtta tatatgattg ttaggtttta aattaatttt agtgtttagt aatatattta   1320
   taattagtat attgtgtttt ttttgtttag agaagaggat agtaagaggg aattagagtt   1380
   agttagtttt tttttattga tgggaatgag gtgttttaag ttttattttg ttttttttaa   1440
   ttattttttt aggtggtttt aaatattaag tagaatagtt ttaagttagg atttatttgt   1500
   tgatgttaag gtgaggtaat agagaaggga tttatattgg ttgaggagaa agggtattag   1560
   gtattggaaa gaggagaaat ttgtagttag tgtgatggtt tgattgagta gtattgtttt   1620
   ttttgagttt ttagtgaatg tttttttttg gagggaggta gggttataag gatttagaga   1680
   atattaattt ttttaaggag gattttttta gggtaaagag ggaaatagat tgaagtttgt   1740
   gtttttgttt aaattatttt tttttttatt tttgttagtt tttattttgt aaggttttga   1800
   agttaatgtt agttttttta ggaagttttt tttaagtttt ttagttttag tttttttttt   1860
   ttgaattttt tttttgtagg gttaatttga gttttaagtt ttttttttgg aaaggtttat   1920
   aaatgttgtt ttatgtaatt gaaggaatta taataaaatg tgaataatgg tagttataga   1980
   ttgagtattt attatgtttt aggtttaatg ttaagtgttt tatgtatttt atttaatttt   2040
   tgtaattgat ttattagatg gttattatta taattgttat ttatagatga tgaaattgag   2100
   gtttaaggat aattgatttt tttgagaatt tttagtgagt aagtggtggg gttgatttag   2160
   atttgtttga ttttaaagat taaatttttg gtggtaattt tattgagaga ggggtttttt   2220
   atagagatgt attttagtat ttaagaagag ggtttttatt ttttgtagta attgttgggt   2280
   ttaggttggt ttatgaattt gttgtaggtt ggtggaggtt tgttttagta gtggttgtgt   2340
   gttattttat agagtggtta ttagtattag agttaatatt tgttttgaat gtagatgagt   2400
   gagttatttt gatttttatt ttaaatggtt gtttattttg tttttttttg gttttttagt   2460
   tttagttagt tgtttttatt tttttttttt gttttttttg ttttggttgt gattggtggt   2520
   taaggtaggt ttagggaagt tagggagatg aatttaggag tttggttttt ggggttgtag   2580
   taggggttgg ggttagaggg tggatgtgga gtaggaaatg aatttgaggt gagggttggg   2640
   aaatttatgg aagggtgggg ttggttattt atttagagat gttgttatgg taatgttgtt   2700
   gttttttatt tttgggaatg aaggggtgga gatttataat ttggaagtga ttttgaaatt   2760
   ttttttggtt ttatttatgt tgtggttttt tttgtgttat agttgggatg gtgagtttta   2820
   gagttagggg tattgtagtg gggttttggg gttgaggttg gggtttaatt ttgagtattg   2880
   atgttattgt tttgttttta tagaagttga ttttattgta gggttatgag tggtttatta   2940
   tgtagattaa gtataattgt gaaggagatt ttttttttat tgtggttaag gattttgtga   3000
   g                                                                  3001
<210> 221
   <211> 50
   <212> DNA
   <213> human
<400> 221
   cgctaggagc tcaaagaagg cagtgctgct cagtcaggcc atcacactga 50
<210> 222
   <211> 50
   <212> DNA
   <213> human
<400> 222
   cgttaggagt ttaaagaagg tagtgttgtt tagttaggtt attatattga 50
<210> 223
   <211> 50
   <212> DNA
   <213> human
<400> 223
   tgttaggagt ttaaagaagg tagtgttgtt tagttaggtt attatattga 50
<210> 224
   <211> 50
   <212> DNA
   <213> human
<400> 224
   ttagtgtgat ggtttgattg agtagtattg ttttttttga gtttttagcg 50
<210> 225
   <211> 50
   <212> DNA
   <213> human
<400> 225
   ttagtgtgat ggtttgattg agtagtattg ttttttttga gtttttagtg 50
<210> 226
   <211> 50
   <212> DNA
   <213> human
<400> 226
   cgctcccgcc cctacgaccc aacctggaag gccccagacc ctgaaggcgg 50
<210> 227
   <211> 50
   <212> DNA
   <213> human
<400> 227
   cgttttcgtt tttacgattt aatttggaag gttttagatt ttgaaggcgg 50
<210> 228
   <211> 50
   <212> DNA
   <213> human
<400> 228
   tgtttttgtt tttatgattt aatttggaag gttttagatt ttgaaggtgg 50
<210> 229
   <211> 50
   <212> DNA
   <213> human
<400> 229
   tcgtttttag ggtttggggt tttttaggtt gggtcgtagg ggcgggagcg 50
<210> 230
   <211> 50
   <212> DNA
   <213> human
<400> 230
   ttgtttttag ggtttggggt tttttaggtt gggttgtagg ggtgggagtg 50
<210> 231
   <211> 3001
   <212> DNA
   <213> human
<400> 231
   gacacaccta caggccctca gcaacagcca gcgagcttgg gcgcctgcct ctggctgctg 60
   gaagcgctgg gaagaaatgt tcagccaaga gccaggcatg ggggggtggg accctgaagc 120
   tggcgctcac cggcagtgac attctctcat gccagagcta attgaccatc cagccgggaa 180
   gagtggtgtc actggtgggt tagagtggca gtccctcttt ttgctggact gtgaccagac 240
   tgaccaactg ggtgttggct gagcggaggc ttagcagtta gatgcccgca gggactggcc 300
   aggtggaggg caggcgatag atgagctgac tcagtggggc tgcacaggag cacctcctcc 360
   cggggctcag ttcctccgca ctgagtgaga ggggacttgc ctcaaggctg gaggctcaaa 420
   actctttctg ggaggtcacg ggcttcacct ccacccagtc cccgcccccg gttttcagac 480
   ggtatgccag agcatcgggg aggcgggcag ggaggcttag aagagctgga cgaccaagca 540
   tggctcggga cgtcctgcct cccccgcccc tcgccctgca ggcgccgccg cgcgccccca 600
   ccccaagagc cccaggcctc cgcccgcggg ttccgccgcc gctctcccta cctctcatcg 660
   ctcttcccgg cagacccagg cgtccgaccc gggagcgggg cctcttcggc ggcggggcga 720
   ggggcggggg aggcggaggg agccaggagc cgggagccgg gagcgggagg cgccggcggg 780
   gctcgggtgt cgggaggcgg ctcccgagag cagctgtcgg gggcctgttc cggggagagg 840
   agccagggct ggggcggctc gggcccaggc gccgcctccc cctcgcctcg gctccctccc 900
   ttcctcctcc cgctcctcgc tcccctccct cccctcggcc ttctcgccgc gcgttcgggc 960
   tcgcagccct cccttccccc tcccggaccc agtagtccca gtggctccca cgcccggcgc 1020
   cgagctccag gccccgcgcc cgccacgccc accccgcgcg ctagcgcccg gcccggcctc 1080
   ccccaggtaa cccgcccgcc aagcccgcct gggcgctccc aggccgccca gccgcccgcg 1140
   ccgctgcctc cggccggctc cgcgcccagc cccctccacc cactcgcagc ccacccccgc 1200
   ttccccaaac gagaacctcc aaagctggcg ccccaggggg ctggctgcag tcccgctcag 1260
   ggcccacgag gggaagacgc ccctcctact tctgggatcg gttcctggtg ggagaggcaa 1320
   aggggagcag ccccagggca cagaggaaga agcaggttcc cacaggctcg gcccagccca 1380
   gctgcgggag ttagcccaga gaagggagca gagctcaggt ctcagggctc acctgtccag 1440
   ccaggcccca gactgctctt ctccctccac caacctttgc tcctctctcg cagcctgtgc 1500
   gctcccgccc ctacgaccca acctggaagg ccccagaccc tgaaggcggg cccactctct 1560
   tctccttctc ctctcagttc ctctctctga agggcgttct aagggacccg ggggtccaga 1620
   cgcccaggcc ctgattcccc tgggctccca acggagtgga gtgggagggg gactcaggag 1680
   tcctcagggc aagcctccct caggcccagc cagctctggc cgtagtgggc cagagggtct 1740
   gatcactggc cagaagaggc caaaaatgag gccaggacaa agttgagtgg tcgggaggag 1800
   gaagaagagg agcaggagaa ggatggaggc acactgaaaa tcacacacag tcaaaaggaa 1860
   ggggaccaag agacccaggg aagatgaaac agtgagactg agtaggcaaa gcaactgaga 1920
   gagctactgg gcagaggatg gagcgggaca gaacagcttg tggtgggagg tgctttttcc 1980
   ccactcagca ctacctcagc ccctcacctc tctccacacc cccacacctg ctcaccacct 2040
   agacaatgtc acggtcacag gaagttctcg ctccactgca catctccgcc ccaggaacct 2100
   cacagtagat aaaaacatac cctgcttgca gtcccagaca acaatcccaa gaaagaaaca 2160
   cgaagtccca accagacaag tacacaaaag cactaccagg aagaagctgg cccttgggaa 2220
   aggccgaggc acacatgggc actagttcag aagcccaagg acgttccaac ggctgaggga 2280
   cctgctaaca gacagcccca gggtcactcg aagagcagct tcttcccgcc ttcctgtccc 2340
   accaagcccc cagggattca ttcccccaga acacctaccc ctccagagaa cagcagctgg 2400
   ggaatctagg cccaggagac gctggactgg gggtggggcc agcctggggg tggggccaag 2460
   gcttcccaag tcacctcacc tggcagccca cagcccctcc ttccaggagc tgtgctctgg 2520
   gaaagcccca acaggcctgc caggcaagct acagcccgtc ataccctggt gggacattcc 2580
   acaccccagg cccacgctga ccccagctaa gtggcatggc agtgccagac agaggggtcc 2640
   actggggaga gcagaaggag ctagagagtc ctgaaacctt tgtcccctct tctcactgcc 2700
   tcaggagaaa ttcctagtgg tgatgcccac ctcgccccag ctctaggctg gaggagtggg 2760
   agggatgcaa ggctggggcc cagtggagtc aagtgacaac ggaaagggag ctggagccag 2820
   ctggaatgcg ggactcggag ccgagattcc caggggcctg agagggaaat cccagccatc 2880
   ctggggccca gagagcagca ccaaagacca agagggcctg attacccatc cgtggtcccc 2940
   agagcccatt ccacatctcc tgcatcactc cgaaccccag aggccccctg tgtccctgag 3000
   a 3001
<210> 232
   <211> 3001
   <212> DNA
   <213> human
<400> 232
   gatatattta taggttttta gtaatagtta gcgagtttgg gcgtttgttt ttggttgttg 60
   gaagcgttgg gaagaaatgt ttagttaaga gttaggtatg ggggggtggg attttgaagt 120
   tggcgtttat cggtagtgat atttttttat gttagagtta attgattatt tagtcgggaa 180
   gagtggtgtt attggtgggt tagagtggta gttttttttt ttgttggatt gtgattagat 240
   tgattaattg ggtgttggtt gagcggaggt ttagtagtta gatgttcgta gggattggtt 300
   aggtggaggg taggcgatag atgagttgat ttagtggggt tgtataggag tatttttttt 360
   cggggtttag ttttttcgta ttgagtgaga ggggatttgt tttaaggttg gaggtttaaa 420
   attttttttg ggaggttacg ggttttattt ttatttagtt ttcgttttcg gtttttagac 480
   ggtatgttag agtatcgggg aggcgggtag ggaggtttag aagagttgga cgattaagta 540
   tggttcggga cgttttgttt ttttcgtttt tcgttttgta ggcgtcgtcg cgcgttttta 600
   ttttaagagt tttaggtttt cgttcgcggg tttcgtcgtc gtttttttta ttttttatcg 660
   tttttttcgg tagatttagg cgttcgattc gggagcgggg ttttttcggc ggcggggcga 720
   ggggcggggg aggcggaggg agttaggagt cgggagtcgg gagcgggagg cgtcggcggg 780
   gttcgggtgt cgggaggcgg ttttcgagag tagttgtcgg gggtttgttt cggggagagg 840
   agttagggtt ggggcggttc gggtttaggc gtcgtttttt tttcgtttcg gttttttttt 900
   tttttttttt cgtttttcgt tttttttttt tttttcggtt ttttcgtcgc gcgttcgggt 960
   tcgtagtttt tttttttttt tttcggattt agtagtttta gtggttttta cgttcggcgt 1020
   cgagttttag gtttcgcgtt cgttacgttt atttcgcgcg ttagcgttcg gttcggtttt 1080
   ttttaggtaa ttcgttcgtt aagttcgttt gggcgttttt aggtcgttta gtcgttcgcg 1140
   tcgttgtttt cggtcggttt cgcgtttagt tttttttatt tattcgtagt ttattttcgt 1200
   ttttttaaac gagaattttt aaagttggcg ttttaggggg ttggttgtag tttcgtttag 1260
   ggtttacgag gggaagacgt tttttttatt tttgggatcg gtttttggtg ggagaggtaa 1320
   aggggagtag ttttagggta tagaggaaga agtaggtttt tataggttcg gtttagttta 1380
   gttgcgggag ttagtttaga gaagggagta gagtttaggt tttagggttt atttgtttag 1440
   ttaggtttta gattgttttt ttttttttat taatttttgt ttttttttcg tagtttgtgc 1500
   gttttcgttt ttacgattta atttggaagg ttttagattt tgaaggcggg tttatttttt 1560
   tttttttttt tttttagttt ttttttttga agggcgtttt aagggattcg ggggtttaga 1620
   cgtttaggtt ttgatttttt tgggttttta acggagtgga gtgggagggg gatttaggag 1680
   tttttagggt aagttttttt taggtttagt tagttttggt cgtagtgggt tagagggttt 1740
   gattattggt tagaagaggt taaaaatgag gttaggataa agttgagtgg tcgggaggag 1800
   gaagaagagg agtaggagaa ggatggaggt atattgaaaa ttatatatag ttaaaaggaa 1860
   ggggattaag agatttaggg aagatgaaat agtgagattg agtaggtaaa gtaattgaga 1920
   gagttattgg gtagaggatg gagcgggata gaatagtttg tggtgggagg tgtttttttt 1980
   ttatttagta ttattttagt tttttatttt tttttatatt tttatatttg tttattattt 2040
   agataatgtt acggttatag gaagttttcg ttttattgta tattttcgtt ttaggaattt 2100
   tatagtagat aaaaatatat tttgtttgta gttttagata ataattttaa gaaagaaata 2160
   cgaagtttta attagataag tatataaaag tattattagg aagaagttgg tttttgggaa 2220
   aggtcgaggt atatatgggt attagtttag aagtttaagg acgttttaac ggttgaggga 2280
   tttgttaata gatagtttta gggttattcg aagagtagtt ttttttcgtt tttttgtttt 2340
   attaagtttt tagggattta tttttttaga atatttattt ttttagagaa tagtagttgg 2400
   ggaatttagg tttaggagac gttggattgg gggtggggtt agtttggggg tggggttaag 2460
   gttttttaag ttattttatt tggtagttta tagttttttt ttttaggagt tgtgttttgg 2520
   gaaagtttta ataggtttgt taggtaagtt atagttcgtt atattttggt gggatatttt 2580
   atattttagg tttacgttga ttttagttaa gtggtatggt agtgttagat agaggggttt 2640
   attggggaga gtagaaggag ttagagagtt ttgaaatttt tgtttttttt ttttattgtt 2700
   ttaggagaaa tttttagtgg tgatgtttat ttcgttttag ttttaggttg gaggagtggg 2760
   agggatgtaa ggttggggtt tagtggagtt aagtgataac ggaaagggag ttggagttag 2820
   ttggaatgcg ggattcggag tcgagatttt taggggtttg agagggaaat tttagttatt 2880
   ttggggttta gagagtagta ttaaagatta agagggtttg attatttatt cgtggttttt 2940
   agagtttatt ttatattttt tgtattattt cgaattttag aggttttttg tgtttttgag 3000
   a 3001
<210> 233
   <211> 3001
   <212> DNA
   <213> human
<400> 233
   gatatattta taggttttta gtaatagtta gtgagtttgg gtgtttgttt ttggttgttg      60
   gaagtgttgg gaagaaatgt ttagttaaga gttaggtatg ggggggtggg attttgaagt     120
   tggtgtttat tggtagtgat atttttttat gttagagtta attgattatt tagttgggaa     180
   gagtggtgtt attggtgggt tagagtggta gttttttttt ttgttggatt gtgattagat     240
   tgattaattg ggtgttggtt gagtggaggt ttagtagtta gatgtttgta gggattggtt     300
   aggtggaggg taggtgatag atgagttgat ttagtggggt tgtataggag tatttttttt     360
   tggggtttag tttttttgta ttgagtgaga ggggatttgt tttaaggttg gaggtttaaa     420
   attttttttg ggaggttatg ggttttattt ttatttagtt tttgtttttg gtttttagat      480
   ggtatgttag agtattgggg aggtgggtag ggaggtttag aagagttgga tgattaagta     540
   tggtttggga tgttttgttt tttttgtttt ttgttttgta ggtgttgttg tgtgttttta     600
   ttttaagagt tttaggtttt tgtttgtggg ttttgttgtt gtttttttta ttttttattg       660
   ttttttttgg tagatttagg tgtttgattt gggagtgggg tttttttggt ggtggggtga     720
   ggggtggggg aggtggaggg agttaggagt tgggagttgg gagtgggagg tgttggtggg     780
   gtttgggtgt tgggaggtgg tttttgagag tagttgttgg gggtttgttt tggggagagg     840
   agttagggtt ggggtggttt gggtttaggt gttgtttttt ttttgttttg gttttttttt     900
   tttttttttt tgttttttgt tttttttttt ttttttggtt tttttgttgt gtgtttgggt      960
   ttgtagtttt tttttttttt ttttggattt agtagtttta gtggttttta tgtttggtgt    1020
   tgagttttag gttttgtgtt tgttatgttt attttgtgtg ttagtgtttg gtttggtttt    1080
   ttttaggtaa tttgtttgtt aagtttgttt gggtgttttt aggttgttta gttgtttgtg    1140
   ttgttgtttt tggttggttt tgtgtttagt tttttttatt tatttgtagt ttatttttgt    1200
   ttttttaaat gagaattttt aaagttggtg ttttaggggg ttggttgtag ttttgtttag     1260
   ggtttatgag gggaagatgt tttttttatt tttgggattg gtttttggtg ggagaggtaa    1320
   aggggagtag ttttagggta tagaggaaga agtaggtttt tataggtttg gtttagttta    1380
   gttgtgggag ttagtttaga gaagggagta gagtttaggt tttagggttt atttgtttag    1440
   ttaggtttta gattgttttt ttttttttat taatttttgt tttttttttg tagtttgtgt    1500
   gtttttgttt ttatgattta atttggaagg ttttagattt tgaaggtggg tttatttttt    1560
   tttttttttt tttttagttt ttttttttga agggtgtttt aagggatttg ggggtttaga    1620
   tgtttaggtt ttgatttttt tgggttttta atggagtgga gtgggagggg gatttaggag    1680
   tttttagggt aagttttttt taggtttagt tagttttggt tgtagtgggt tagagggttt    1740
   gattattggt tagaagaggt taaaaatgag gttaggataa agttgagtgg ttgggaggag    1800
   gaagaagagg agtaggagaa ggatggaggt atattgaaaa ttatatatag ttaaaaggaa    1860
   ggggattaag agatttaggg aagatgaaat agtgagattg agtaggtaaa gtaattgaga    1920
   gagttattgg gtagaggatg gagtgggata gaatagtttg tggtgggagg tgtttttttt    1980
   ttatttagta ttattttagt tttttatttt tttttatatt tttatatttg tttattattt    2040
   agataatgtt atggttatag gaagtttttg ttttattgta tatttttgtt ttaggaattt    2100
   tatagtagat aaaaatatat tttgtttgta gttttagata ataattttaa gaaagaaata    2160
   tgaagtttta attagataag tatataaaag tattattagg aagaagttgg tttttgggaa    2220
   aggttgaggt atatatgggt attagtttag aagtttaagg atgttttaat ggttgaggga    2280
   tttgttaata gatagtttta gggttatttg aagagtagtt tttttttgtt tttttgtttt    2340
   attaagtttt tagggattta tttttttaga atatttattt ttttagagaa tagtagttgg    2400
   ggaatttagg tttaggagat gttggattgg gggtggggtt agtttggggg tggggttaag    2460
   gttttttaag ttattttatt tggtagttta tagttttttt ttttaggagt tgtgttttgg     2520
   gaaagtttta ataggtttgt taggtaagtt atagtttgtt atattttggt gggatatttt    2580
   atattttagg tttatgttga ttttagttaa gtggtatggt agtgttagat agaggggttt    2640
   attggggaga gtagaaggag ttagagagtt ttgaaatttt tgtttttttt ttttattgtt    2700
   ttaggagaaa tttttagtgg tgatgtttat tttgttttag ttttaggttg gaggagtggg    2760
   agggatgtaa ggttggggtt tagtggagtt aagtgataat ggaaagggag ttggagttag    2820
   ttggaatgtg ggatttggag ttgagatttt taggggtttg agagggaaat tttagttatt    2880
   ttggggttta gagagtagta ttaaagatta agagggtttg attatttatt tgtggttttt    2940
   agagtttatt ttatattttt tgtattattt tgaattttag aggttttttg tgtttttgag   3000
   a                                                                    3001
<210> 234
   <211> 3001
   <212> DNA
   <213> human
<400> 234
   ttttagggat atagggggtt tttggggttc ggagtgatgt aggagatgtg gaatgggttt 60
   tggggattac ggatgggtaa ttaggttttt ttggtttttg gtgttgtttt ttgggtttta 120
   ggatggttgg gatttttttt ttaggttttt gggaatttcg gtttcgagtt tcgtatttta 180
   gttggtttta gttttttttt cgttgttatt tgattttatt gggttttagt tttgtatttt 240
   ttttattttt ttagtttaga gttggggcga ggtgggtatt attattagga attttttttg 300
   aggtagtgag aagaggggat aaaggtttta ggatttttta gttttttttg ttttttttag 360
   tggatttttt tgtttggtat tgttatgtta tttagttggg gttagcgtgg gtttggggtg 420
   tggaatgttt tattagggta tgacgggttg tagtttgttt ggtaggtttg ttggggtttt 480
   tttagagtat agtttttgga aggaggggtt gtgggttgtt aggtgaggtg atttgggaag 540
   ttttggtttt atttttaggt tggttttatt tttagtttag cgttttttgg gtttagattt 600
   tttagttgtt gttttttgga ggggtaggtg ttttggggga atgaattttt gggggtttgg 660
   tgggatagga aggcgggaag aagttgtttt tcgagtgatt ttggggttgt ttgttagtag 720
   gttttttagt cgttggaacg tttttgggtt tttgaattag tgtttatgtg tgtttcggtt 780
   ttttttaagg gttagttttt ttttggtagt gtttttgtgt atttgtttgg ttgggatttc 840
   gtgttttttt tttgggattg ttgtttggga ttgtaagtag ggtatgtttt tatttattgt 900
   gaggtttttg gggcggagat gtgtagtgga gcgagaattt tttgtgatcg tgatattgtt 960
   taggtggtga gtaggtgtgg gggtgtggag agaggtgagg ggttgaggta gtgttgagtg 1020
   gggaaaaagt attttttatt ataagttgtt ttgtttcgtt ttattttttg tttagtagtt 1080
   tttttagttg ttttgtttat ttagttttat tgttttattt tttttgggtt ttttggtttt 1140
   tttttttttg attgtgtgtg atttttagtg tgtttttatt ttttttttgt tttttttttt 1200
   ttttttttcg attatttaat tttgttttgg ttttattttt ggtttttttt ggttagtgat 1260
   tagatttttt ggtttattac ggttagagtt ggttgggttt gagggaggtt tgttttgagg 1320
   atttttgagt ttttttttta ttttatttcg ttgggagttt aggggaatta gggtttgggc 1380
   gtttggattt tcgggttttt tagaacgttt tttagagaga ggaattgaga ggagaaggag 1440
   aagagagtgg gttcgttttt agggtttggg gttttttagg ttgggtcgta ggggcgggag 1500
   cgtataggtt gcgagagagg agtaaaggtt ggtggaggga gaagagtagt ttggggtttg 1560
   gttggatagg tgagttttga gatttgagtt ttgttttttt ttttgggtta attttcgtag 1620
   ttgggttggg tcgagtttgt gggaatttgt tttttttttt gtgttttggg gttgtttttt 1680
   tttgtttttt ttattaggaa tcgattttag aagtaggagg ggcgtttttt tttcgtgggt 1740
   tttgagcggg attgtagtta gttttttggg gcgttagttt tggaggtttt cgtttgggga 1800
   agcgggggtg ggttgcgagt gggtggaggg ggttgggcgc ggagtcggtc ggaggtagcg 1860
   gcgcgggcgg ttgggcggtt tgggagcgtt taggcgggtt tggcgggcgg gttatttggg 1920
   ggaggtcggg tcgggcgtta gcgcgcgggg tgggcgtggc gggcgcgggg tttggagttc 1980
   ggcgtcgggc gtgggagtta ttgggattat tgggttcggg agggggaagg gagggttgcg 2040
   agttcgaacg cgcggcgaga aggtcgaggg gagggagggg agcgaggagc gggaggagga 2100
   agggagggag tcgaggcgag ggggaggcgg cgtttgggtt cgagtcgttt tagttttggt 2160
   tttttttttc ggaataggtt ttcgatagtt gttttcggga gtcgtttttc gatattcgag 2220
   tttcgtcggc gtttttcgtt ttcggttttc ggtttttggt ttttttcgtt tttttcgttt 2280
   ttcgtttcgt cgtcgaagag gtttcgtttt cgggtcggac gtttgggttt gtcgggaaga 2340
   gcgatgagag gtagggagag cggcggcgga attcgcgggc ggaggtttgg ggtttttggg 2400
   gtgggggcgc gcggcggcgt ttgtagggcg aggggcgggg gaggtaggac gtttcgagtt 2460
   atgtttggtc gtttagtttt tttaagtttt tttgttcgtt ttttcgatgt tttggtatat 2520
   cgtttgaaaa tcgggggcgg ggattgggtg gaggtgaagt tcgtgatttt ttagaaagag 2580
   ttttgagttt ttagttttga ggtaagtttt tttttattta gtgcggagga attgagtttc 2640
   gggaggaggt gtttttgtgt agttttattg agttagttta tttatcgttt gttttttatt 2700
   tggttagttt ttgcgggtat ttaattgtta agttttcgtt tagttaatat ttagttggtt 2760
   agtttggtta tagtttagta aaaagaggga ttgttatttt aatttattag tgatattatt 2820
   tttttcggtt ggatggttaa ttagttttgg tatgagagaa tgttattgtc ggtgagcgtt 2880
   agttttaggg ttttattttt ttatgtttgg tttttggttg aatatttttt tttagcgttt 2940
   ttagtagtta gaggtaggcg tttaagttcg ttggttgttg ttgagggttt gtaggtgtgt 3000
   t 3001
<210> 235
   <211> 3001
   <212> DNA
   <213> human
<400> 235
   ttttagggat atagggggtt tttggggttt ggagtgatgt aggagatgtg gaatgggttt 60
   tggggattat ggatgggtaa ttaggttttt ttggtttttg gtgttgtttt ttgggtttta 120
   ggatggttgg gatttttttt ttaggttttt gggaattttg gttttgagtt ttgtatttta 180
   gttggtttta gttttttttt tgttgttatt tgattttatt gggttttagt tttgtatttt 240
   ttttattttt ttagtttaga gttggggtga ggtgggtatt attattagga attttttttg 300
   aggtagtgag aagaggggat aaaggtttta ggatttttta gttttttttg ttttttttag 360
   tggatttttt tgtttggtat tgttatgtta tttagttggg gttagtgtgg gtttggggtg 420
   tggaatgttt tattagggta tgatgggttg tagtttgttt ggtaggtttg ttggggtttt 480
   tttagagtat agtttttgga aggaggggtt gtgggttgtt aggtgaggtg atttgggaag 540
   ttttggtttt atttttaggt tggttttatt tttagtttag tgttttttgg gtttagattt 600
   tttagttgtt gttttttgga ggggtaggtg ttttggggga atgaattttt gggggtttgg 660
   tgggatagga aggtgggaag aagttgtttt ttgagtgatt ttggggttgt ttgttagtag 720
   gttttttagt tgttggaatg tttttgggtt tttgaattag tgtttatgtg tgttttggtt 780
   ttttttaagg gttagttttt ttttggtagt gtttttgtgt atttgtttgg ttgggatttt 840
   gtgttttttt tttgggattg ttgtttggga ttgtaagtag ggtatgtttt tatttattgt 900
   gaggtttttg gggtggagat gtgtagtgga gtgagaattt tttgtgattg tgatattgtt 960
   taggtggtga gtaggtgtgg gggtgtggag agaggtgagg ggttgaggta gtgttgagtg 1020
   gggaaaaagt attttttatt ataagttgtt ttgttttgtt ttattttttg tttagtagtt 1080
   tttttagttg ttttgtttat ttagttttat tgttttattt tttttgggtt ttttggtttt 1140
   tttttttttg attgtgtgtg atttttagtg tgtttttatt ttttttttgt tttttttttt 1200
   tttttttttg attatttaat tttgttttgg ttttattttt ggtttttttt ggttagtgat 1260
   tagatttttt ggtttattat ggttagagtt ggttgggttt gagggaggtt tgttttgagg 1320
   atttttgagt ttttttttta ttttattttg ttgggagttt aggggaatta gggtttgggt 1380
   gtttggattt ttgggttttt tagaatgttt tttagagaga ggaattgaga ggagaaggag 1440
   aagagagtgg gtttgttttt agggtttggg gttttttagg ttgggttgta ggggtgggag 1500
   tgtataggtt gtgagagagg agtaaaggtt ggtggaggga gaagagtagt ttggggtttg 1560
   gttggatagg tgagttttga gatttgagtt ttgttttttt ttttgggtta atttttgtag 1620
   ttgggttggg ttgagtttgt gggaatttgt tttttttttt gtgttttggg gttgtttttt 1680
   tttgtttttt ttattaggaa ttgattttag aagtaggagg ggtgtttttt ttttgtgggt 1740
   tttgagtggg attgtagtta gttttttggg gtgttagttt tggaggtttt tgtttgggga 1800
   agtgggggtg ggttgtgagt gggtggaggg ggttgggtgt ggagttggtt ggaggtagtg 1860
   gtgtgggtgg ttgggtggtt tgggagtgtt taggtgggtt tggtgggtgg gttatttggg 1920
   ggaggttggg ttgggtgtta gtgtgtgggg tgggtgtggt gggtgtgggg tttggagttt 1980
   ggtgttgggt gtgggagtta ttgggattat tgggtttggg agggggaagg gagggttgtg 2040
   agtttgaatg tgtggtgaga aggttgaggg gagggagggg agtgaggagt gggaggagga 2100
   agggagggag ttgaggtgag ggggaggtgg tgtttgggtt tgagttgttt tagttttggt 2160
   tttttttttt ggaataggtt tttgatagtt gtttttggga gttgtttttt gatatttgag 2220
   ttttgttggt gttttttgtt tttggttttt ggtttttggt tttttttgtt ttttttgttt 2280
   tttgttttgt tgttgaagag gttttgtttt tgggttggat gtttgggttt gttgggaaga 2340
   gtgatgagag gtagggagag tggtggtgga atttgtgggt ggaggtttgg ggtttttggg 2400
   gtgggggtgt gtggtggtgt ttgtagggtg aggggtgggg gaggtaggat gttttgagtt 2460
   atgtttggtt gtttagtttt tttaagtttt tttgtttgtt tttttgatgt tttggtatat 2520
   tgtttgaaaa ttgggggtgg ggattgggtg gaggtgaagt ttgtgatttt ttagaaagag 2580
   ttttgagttt ttagttttga ggtaagtttt tttttattta gtgtggagga attgagtttt 2640
   gggaggaggt gtttttgtgt agttttattg agttagttta tttattgttt gttttttatt 2700
   tggttagttt ttgtgggtat ttaattgtta agtttttgtt tagttaatat ttagttggtt 2760
   agtttggtta tagtttagta aaaagaggga ttgttatttt aatttattag tgatattatt 2820
   ttttttggtt ggatggttaa ttagttttgg tatgagagaa tgttattgtt ggtgagtgtt 2880
   agttttaggg ttttattttt ttatgtttgg tttttggttg aatatttttt tttagtgttt 2940
   ttagtagtta gaggtaggtg tttaagtttg ttggttgttg ttgagggttt gtaggtgtgt 3000
   t 3001
<210> 236
   <211> 50
   <212> DNA
   <213> human
<400> 236
   cgcatctgtc tttgtccaca gtggcatgca tgggcagcct caccaatgtg 50
<210> 237
   <211> 50
   <212> DNA
   <213> human
<400> 237
   cgtatttgtt tttgtttata gtggtatgta tgggtagttt tattaatgtg 50
<210> 238
   <211> 50
   <212> DNA
   <213> human
<400> 238
   tgtatttgtt tttgtttata gtggtatgta tgggtagttt tattaatgtg 50
<210> 239
   <211> 50
   <212> DNA
   <213> human
<400> 239
   tatattggtg aggttgttta tgtatgttat tgtggataaa gatagatgcg 50
<210> 240
   <211> 50
   <212> DNA
   <213> human
<400> 240
   tatattggtg aggttgttta tgtatgttat tgtggataaa gatagatgtg 50
<210> 241
   <211> 3001
   <212> DNA
   <213> human
<400> 241
   caacagcacc ccatttttcc atacctcctt gctcagcgca ccgacacccc tctggcgcca      60
   cttctgcagg gcgggaaggg gctgggggtc tcacacatcc cctgcgccct gggacccctg     120
   ggacgggcct tggcatgcga gcgtggtggg tagggcgaga caggcacagc gcggggctta     180
   gggctaggcg ggacgggcgg agttggggct cacgagacgg ggcgtgcggg gcaccgggcg     240
   gccggcgggg agtcgcaggc ttccccagag ggggcgcgag ccgggccgcc ggggaactca     300
   ccataggtgc tgtggccgaa agccagcagg agcaagagga cgcgcaggag ggcttcggtc     360
   gcggtcatct ctgggcggcc ggggtcgcgg tcccgggagc ggtgcgggcg cgggtccggc     420
   tcctggcgcc gcactgggct tctggttgcg gacgcgaaag gggcgcgggg cgcgcagcga     480
   ggggaaagcc agggctgcac cgggctgcgc gctgcgctct ccaccgccgc tgccggggag     540
   ctgccgccgc cgccgcgcgc gccgcccttt tcgtaccgcc tcccccctcg cggcccccgc     600
   cccccgcccc cgcctccgtg cgcacggggc cggcgccggc cagtccctgt gaccccctcc     660
   cctcgcaggg ccgcccccga ccacggcggc gtcccagaag cccgctcgcg cacacgggtg     720
   cactcttggc tagacgggca caggtatccc gcgagcgttt ggtacacgtt ccctcacact     780
   gtacagcacg cggccgccgc gcacatacag gcctgcctag gacaagtcgc aggaatcgaa     840
   cctgggccgc agacctcccc ggagagcaca aatccgcacg caggaacacc gaaacactta     900
   tcgctgaagt cccgggcaca cgtgcaatgg ttagataccc aggaaaacac aaaggcgcac     960
   tatgaagatg tgaactaagc acataattac acacgcacac agaaaaaccc ccaggaaaag    1020
   cccgacgccc cctgtacccc tggccatgaa gacgcagaaa aatgcagttg ccccgtggca    1080
   acccacaccc cgcaacggaa acgcacacgg caactcaaac acactgtagc ccagacgccg    1140
   tatagacaca gtcctaggtg gcagagggtc aagcggacct gcggttaccc aaccatgggc    1200
   atcctccccc atgtgccgac cccctgcaca cagacgctta cgcagagcca cctgcacacc    1260
   caaacacgca aacacacgta taaacagacg gacacccgtg cccgcattca gtcacaccca    1320
   cccccgcaca cacagcatct cagtcagggc cttagccgcg ctgaaacaca aatgcaccga    1380
   ctcttacgca cacatgtacg cacagccatg cacctgatac ccacaagcac acatacccag    1440
   ctgactcgtg gtcacacaca ctgacaaaaa catatacgca gtccccacac acacacgatc    1500
   gcatctgtct ttgtccacag tggcatgcat gggcagcctc accaatgtgc tctcacatac    1560
   acacaatagc actccaggca ctcggcacgt attccagact cactgggaga ccttctctga    1620
   ggggtcgccc gctggagagg tggaggtaga ggatgctgat tgccagagac cctcttcctc    1680
   tgcagagctc ccagggagag ggagtgctcc tcagacaccc ctcctcccca ttgcccccgg    1740
   cttaggctta gagccctgct ggagaggaaa gctctgcacc cctgcaccca gtctggatgg    1800
   gggaggctca gaaagatagg gagggagagg ggtaggaggg gattaaagat atttcacctc    1860
   ctgggggaag ggtcccaggc gtcccaatct ttgggtgggt cgtttaggat acttaatttg    1920
   catatattaa tttgcataca tatcctagat gcagggagtg ggtgcccacc cagctcgcgg    1980
   cctccccttc ttcacagtgc tgcagggggg tgtgatctca ccactgtgaa cctggaggct    2040
   ggatgctttg acctcctggc cctctagtcc agccctggca ggtctgagca cagatttccc    2100
   atcttagcca tagatgtggg aaggcaggcc catgggcatc cccaggccca ccacccctaa    2160
   gtcagtgctc cagtaaggca ctcgggatgg gtacagggat ggacagagac cagagctgct    2220
   ctgggctggc tccaaggagg ctggagggaa actcccagcc cctggtggaa gatatccctt    2280
   ccctgatgtt atctgcagaa tgcccagata ggtaggggct gctgggaacg gctttgggga    2340
   caggctgtag ggagcagttg gggtgcccac tgtgtgcctg ccacatgccc ttgcattcct    2400
   gtgaccctac tttcttccgt ggactctttc ctcaagtcaa gaaaccaact tcagtgatgg    2460
   aaggtccttt gcccaaggcc cttggcctag aacgagagtg tctggctgac aggcccaacc    2520
   ctgggtgcca atgtccactg catggtgatc gccaaggtct tcctgaccag ggttcctctc    2580
   ttccctccct tcgaggcccc tgacttcagc ctccaaaatt atcccaacag caggcagacc    2640
   tggaagagct gagttctcag ccagaagcca aggaggggtg gaggatctca gggctgggta    2700
   gccctggggc aagggcctcc caggctcttg ccaaagtccc gcagagagga gactccctgg    2760
   actgcatgga gcacttaggg ggccccagtg tccccaccca cacacatacc tagactcctc    2820
   ccttccccat ccaggttctt cccccttccc acccccgccc ccagccccac cacagccctc    2880
   cccagtttag gaaagggagg gaggccagag gagggtattc tcgcccaagg gagtctgtca    2940
   aaatgtcaca ggcttacgct gaaagggcac tgccttggct tggggggcac ctgctctcta    3000
   a                                                                     3001

<210> 242
   <211> 3001
   <212> DNA
   <213> human
<400> 242
   taatagtatt ttattttttt atattttttt gtttagcgta tcgatatttt tttggcgtta 60
   tttttgtagg gcgggaaggg gttgggggtt ttatatattt tttgcgtttt gggatttttg 120
   ggacgggttt tggtatgcga gcgtggtggg tagggcgaga taggtatagc gcggggttta 180
   gggttaggcg ggacgggcgg agttggggtt tacgagacgg ggcgtgcggg gtatcgggcg 240
   gtcggcgggg agtcgtaggt ttttttagag ggggcgcgag tcgggtcgtc ggggaattta 300
   ttataggtgt tgtggtcgaa agttagtagg agtaagagga cgcgtaggag ggtttcggtc 360
   gcggttattt ttgggcggtc ggggtcgcgg tttcgggagc ggtgcgggcg cgggttcggt 420
   ttttggcgtc gtattgggtt tttggttgcg gacgcgaaag ggggcgcggg cgcgtagcga 480
   ggggaaagtt agggttgtat cgggttgcgc gttgcgtttt ttatcgtcgt tgtcggggag 540
   ttgtcgtcgt cgtcgcgcgc gtcgtttttt tcgtatcgtt ttttttttcg cggttttcgt 600
   ttttcgtttt cgttttcgtg cgtacggggt cggcgtcggt tagtttttgt gatttttttt 660
   tttcgtaggg tcgttttcga ttacggcggc gttttagaag ttcgttcgcg tatacgggtg 720
   tatttttggt tagacgggta taggtatttc gcgagcgttt ggtatacgtt tttttatatt 780
   gtatagtacg cggtcgtcgc gtatatatag gtttgtttag gataagtcgt aggaatcgaa 840
   tttgggtcgt agattttttc ggagagtata aattcgtacg taggaatatc gaaatattta 900
   tcgttgaagt ttcgggtata cgtgtaatgg ttagatattt aggaaaatat aaaggcgtat 960
   tatgaagatg tgaattaagt atataattat atacgtatat agaaaaattt ttaggaaaag 1020
   ttcgacgttt tttgtatttt tggttatgaa gacgtagaaa aatgtagttg tttcgtggta 1080
   atttatattt cgtaacggaa acgtatacgg taatttaaat atattgtagt ttagacgtcg 1140
   tatagatata gttttaggtg gtagagggtt aagcggattt gcggttattt aattatgggt 1200
   attttttttt atgtgtcgat tttttgtata tagacgttta cgtagagtta tttgtatatt 1260
   taaatacgta aatatacgta taaatagacg gatattcgtg ttcgtattta gttatattta 1320
   ttttcgtata tatagtattt tagttagggt tttagtcgcg ttgaaatata aatgtatcga 1380
   tttttacgta tatatgtacg tatagttatg tatttgatat ttataagtat atatatttag 1440
   ttgattcgtg gttatatata ttgataaaaa tatatacgta gtttttatat atatacgatc 1500
   gtatttgttt ttgtttatag tggtatgtat gggtagtttt attaatgtgt ttttatatat 1560
   atataatagt attttaggta ttcggtacgt attttagatt tattgggaga ttttttttga 1620
   ggggtcgttc gttggagagg tggaggtaga ggatgttgat tgttagagat tttttttttt 1680
   tgtagagttt ttagggagag ggagtgtttt ttagatattt ttttttttta ttgttttcgg 1740
   tttaggttta gagttttgtt ggagaggaaa gttttgtatt tttgtattta gtttggatgg 1800
   gggaggttta gaaagatagg gagggagagg ggtaggaggg gattaaagat attttatttt 1860
   ttgggggaag ggttttaggc gttttaattt ttgggtgggt cgtttaggat atttaatttg 1920
   tatatattaa tttgtatata tattttagat gtagggagtg ggtgtttatt tagttcgcgg 1980
   tttttttttt tttatagtgt tgtagggggg tgtgatttta ttattgtgaa tttggaggtt 2040
   ggatgttttg attttttggt tttttagttt agttttggta ggtttgagta tagatttttt 2100
   attttagtta tagatgtggg aaggtaggtt tatgggtatt tttaggttta ttatttttaa 2160
   gttagtgttt tagtaaggta ttcgggatgg gtatagggat ggatagagat tagagttgtt 2220
   ttgggttggt tttaaggagg ttggagggaa atttttagtt tttggtggaa gatatttttt 2280
   ttttgatgtt atttgtagaa tgtttagata ggtaggggtt gttgggaacg gttttgggga 2340
   taggttgtag ggagtagttg gggtgtttat tgtgtgtttg ttatatgttt ttgtattttt 2400
   gtgattttat tttttttcgt ggattttttt tttaagttaa gaaattaatt ttagtgatgg 2460
   aaggtttttt gtttaaggtt tttggtttag aacgagagtg tttggttgat aggtttaatt 2520
   ttgggtgtta atgtttattg tatggtgatc gttaaggttt ttttgattag ggtttttttt 2580
   tttttttttt tcgaggtttt tgattttagt ttttaaaatt attttaatag taggtagatt 2640
   tggaagagtt gagtttttag ttagaagtta aggaggggtg gaggatttta gggttgggta 2700
   gttttggggt aagggttttt taggtttttg ttaaagtttc gtagagagga gattttttgg 2760
   attgtatgga gtatttaggg ggttttagtg tttttattta tatatatatt tagatttttt 2820
   ttttttttat ttaggttttt tttttttttt attttcgttt ttagttttat tatagttttt 2880
   tttagtttag gaaagggagg gaggttagag gagggtattt tcgtttaagg gagtttgtta 2940
   aaatgttata ggtttacgtt gaaagggtat tgttttggtt tggggggtat ttgtttttta 3000
   a 3001
<210> 243
   <211> 3001
   <212> DNA
   <213> human
<400> 243
   taatagtatt ttattttttt atattttttt gtttagtgta ttgatatttt tttggtgtta 60
   tttttgtagg gtgggaaggg gttgggggtt ttatatattt tttgtgtttt gggatttttg 120
   ggatgggttt tggtatgtga gtgtggtggg tagggtgaga taggtatagt gtggggttta 180
   gggttaggtg ggatgggtgg agttggggtt tatgagatgg ggtgtgtggg gtattgggtg 240
   gttggtgggg agttgtaggt ttttttagag ggggtgtgag ttgggttgtt ggggaattta 300
   ttataggtgt tgtggttgaa agttagtagg agtaagagga tgtgtaggag ggttttggtt 360
   gtggttattt ttgggtggtt ggggttgtgg ttttgggagt ggtgtgggtg tgggtttggt 420
   ttttggtgtt gtattgggtt tttggttgtg gatgtgaaag ggggtgtggg tgtgtagtga 480
   ggggaaagtt agggttgtat tgggttgtgt gttgtgtttt ttattgttgt tgttggggag 540
   ttgttgttgt tgttgtgtgt gttgtttttt ttgtattgtt tttttttttg tggtttttgt 600
   tttttgtttt tgtttttgtg tgtatggggt tggtgttggt tagtttttgt gatttttttt 660
   ttttgtaggg ttgtttttga ttatggtggt gttttagaag tttgtttgtg tatatgggtg 720
   tatttttggt tagatgggta taggtatttt gtgagtgttt ggtatatgtt tttttatatt 780
   gtatagtatg tggttgttgt gtatatatag gtttgtttag gataagttgt aggaattgaa 840
   tttgggttgt agattttttt ggagagtata aatttgtatg taggaatatt gaaatattta 900
   ttgttgaagt tttgggtata tgtgtaatgg ttagatattt aggaaaatat aaaggtgtat 960
   tatgaagatg tgaattaagt atataattat atatgtatat agaaaaattt ttaggaaaag 1020
   tttgatgttt tttgtatttt tggttatgaa gatgtagaaa aatgtagttg ttttgtggta 1080
   atttatattt tgtaatggaa atgtatatgg taatttaaat atattgtagt ttagatgttg 1140
   tatagatata gttttaggtg gtagagggtt aagtggattt gtggttattt aattatgggt 1200
   attttttttt atgtgttgat tttttgtata tagatgttta tgtagagtta tttgtatatt 1260
   taaatatgta aatatatgta taaatagatg gatatttgtg tttgtattta gttatattta 1320
   tttttgtata tatagtattt tagttagggt tttagttgtg ttgaaatata aatgtattga 1380
   tttttatgta tatatgtatg tatagttatg tatttgatat ttataagtat atatatttag 1440
   ttgatttgtg gttatatata ttgataaaaa tatatatgta gtttttatat atatatgatt 1500
   gtatttgttt ttgtttatag tggtatgtat gggtagtttt attaatgtgt ttttatatat 1560
   atataatagt attttaggta tttggtatgt attttagatt tattgggaga ttttttttga 1620
   ggggttgttt gttggagagg tggaggtaga ggatgttgat tgttagagat tttttttttt 1680
   tgtagagttt ttagggagag ggagtgtttt ttagatattt ttttttttta ttgtttttgg 1740
   tttaggttta gagttttgtt ggagaggaaa gttttgtatt tttgtattta gtttggatgg 1800
   gggaggttta gaaagatagg gagggagagg ggtaggaggg gattaaagat attttatttt 1860
   ttgggggaag ggttttaggt gttttaattt ttgggtgggt tgtttaggat atttaatttg 1920
   tatatattaa tttgtatata tattttagat gtagggagtg ggtgtttatt tagtttgtgg 1980
   tttttttttt tttatagtgt tgtagggggg tgtgatttta ttattgtgaa tttggaggtt 2040
   ggatgttttg attttttggt tttttagttt agttttggta ggtttgagta tagatttttt 2100
   attttagtta tagatgtggg aaggtaggtt tatgggtatt tttaggttta ttatttttaa 2160
   gttagtgttt tagtaaggta tttgggatgg gtatagggat ggatagagat tagagttgtt 2220
   ttgggttggt tttaaggagg ttggagggaa atttttagtt tttggtggaa gatatttttt 2280
   ttttgatgtt atttgtagaa tgtttagata ggtaggggtt gttgggaatg gttttgggga 2340
   taggttgtag ggagtagttg gggtgtttat tgtgtgtttg ttatatgttt ttgtattttt 2400
   gtgattttat ttttttttgt ggattttttt tttaagttaa gaaattaatt ttagtgatgg 2460
   aaggtttttt gtttaaggtt tttggtttag aatgagagtg tttggttgat aggtttaatt 2520
   ttgggtgtta atgtttattg tatggtgatt gttaaggttt ttttgattag ggtttttttt 2580
   tttttttttt ttgaggtttt tgattttagt ttttaaaatt attttaatag taggtagatt 2640
   tggaagagtt gagtttttag ttagaagtta aggaggggtg gaggatttta gggttgggta 2700
   gttttggggt aagggttttt taggtttttg ttaaagtttt gtagagagga gattttttgg 2760
   attgtatgga gtatttaggg ggttttagtg tttttattta tatatatatt tagatttttt 2820
   ttttttttat ttaggttttt tttttttttt atttttgttt ttagttttat tatagttttt 2880
   tttagtttag gaaagggagg gaggttagag gagggtattt ttgtttaagg gagtttgtta 2940
   aaatgttata ggtttatgtt gaaagggtat tgttttggtt tggggggtat ttgtttttta 3000
   a 3001
<210> 244
   <211> 3001
   <212> DNA
   <213> human
<400> 244
   ttagagagta ggtgtttttt aagttaaggt agtgtttttt tagcgtaagt ttgtgatatt 60
   ttgatagatt tttttgggcg agaatatttt tttttggttt tttttttttt tttaaattgg 120
   ggagggttgt ggtggggttg ggggcggggg tgggaagggg gaagaatttg gatggggaag 180
   ggaggagttt aggtatgtgt gtgggtgggg atattggggt tttttaagtg ttttatgtag 240
   tttagggagt tttttttttg cgggattttg gtaagagttt gggaggtttt tgttttaggg 300
   ttatttagtt ttgagatttt ttattttttt ttggtttttg gttgagaatt tagttttttt 360
   aggtttgttt gttgttggga taattttgga ggttgaagtt aggggtttcg aagggaggga 420
   agagaggaat tttggttagg aagattttgg cgattattat gtagtggata ttggtattta 480
   gggttgggtt tgttagttag atattttcgt tttaggttaa gggttttggg taaaggattt 540
   tttattattg aagttggttt tttgatttga ggaaagagtt tacggaagaa agtagggtta 600
   taggaatgta agggtatgtg gtaggtatat agtgggtatt ttaattgttt tttatagttt 660
   gtttttaaag tcgtttttag tagtttttat ttatttgggt attttgtaga taatattagg 720
   gaagggatat tttttattag gggttgggag ttttttttta gtttttttgg agttagttta 780
   gagtagtttt ggtttttgtt tatttttgta tttatttcga gtgttttatt ggagtattga 840
   tttaggggtg gtgggtttgg ggatgtttat gggtttgttt ttttatattt atggttaaga 900
   tgggaaattt gtgtttagat ttgttagggt tggattagag ggttaggagg ttaaagtatt 960
   tagtttttag gtttatagtg gtgagattat atttttttgt agtattgtga agaaggggag 1020
   gtcgcgagtt gggtgggtat ttattttttg tatttaggat atgtatgtaa attaatatat 1080
   gtaaattaag tattttaaac gatttattta aagattggga cgtttgggat ttttttttta 1140
   ggaggtgaaa tatttttaat ttttttttat tttttttttt ttttattttt ttgagttttt 1200
   tttatttaga ttgggtgtag gggtgtagag tttttttttt tagtagggtt ttaagtttaa 1260
   gtcgggggta atggggagga ggggtgtttg aggagtattt tttttttttg ggagttttgt 1320
   agaggaagag ggtttttggt aattagtatt ttttattttt atttttttag cgggcgattt 1380
   tttagagaag gttttttagt gagtttggaa tacgtgtcga gtgtttggag tgttattgtg 1440
   tgtatgtgag agtatattgg tgaggttgtt tatgtatgtt attgtggata aagatagatg 1500
   cgatcgtgtg tgtgtgggga ttgcgtatat gtttttgtta gtgtgtgtga ttacgagtta 1560
   gttgggtatg tgtgtttgtg ggtattaggt gtatggttgt gcgtatatgt gtgcgtaaga 1620
   gtcggtgtat ttgtgtttta gcgcggttaa ggttttgatt gagatgttgt gtgtgcgggg 1680
   gtgggtgtga ttgaatgcgg gtacgggtgt tcgtttgttt atacgtgtgt ttgcgtgttt 1740
   gggtgtgtag gtggttttgc gtaagcgttt gtgtgtaggg ggtcggtata tgggggagga 1800
   tgtttatggt tgggtaatcg taggttcgtt tgattttttg ttatttagga ttgtgtttat 1860
   acggcgtttg ggttatagtg tgtttgagtt gtcgtgtgcg ttttcgttgc ggggtgtggg 1920
   ttgttacggg gtaattgtat ttttttgcgt ttttatggtt aggggtatag ggggcgtcgg 1980
   gttttttttg ggggtttttt tgtgtgcgtg tgtaattatg tgtttagttt atatttttat 2040
   agtgcgtttt tgtgtttttt tgggtattta attattgtac gtgtgttcgg gattttagcg 2100
   ataagtgttt cggtgttttt gcgtgcggat ttgtgttttt cggggaggtt tgcggtttag 2160
   gttcgatttt tgcgatttgt tttaggtagg tttgtatgtg cgcggcggtc gcgtgttgta 2220
   tagtgtgagg gaacgtgtat taaacgttcg cgggatattt gtgttcgttt agttaagagt 2280
   gtattcgtgt gcgcgagcgg gtttttggga cgtcgtcgtg gtcgggggcg gttttgcgag 2340
   gggagggggt tatagggatt ggtcggcgtc ggtttcgtgc gtacggaggc gggggcgggg 2400
   ggcgggggtc gcgagggggg aggcggtacg aaaagggcgg cgcgcgcggc ggcggcggta 2460
   gtttttcggt agcggcggtg gagagcgtag cgcgtagttc ggtgtagttt tggttttttt 2520
   ttcgttgcgc gttcgcgttt tttttcgcgt tcgtaattag aagtttagtg cggcgttagg 2580
   agtcggattc gcgttcgtat cgttttcggg atcgcgattt cggtcgttta gagatgatcg 2640
   cgatcgaagt ttttttgcgc gtttttttgt ttttgttggt tttcggttat agtatttatg 2700
   gtgagttttt cggcggttcg gttcgcgttt tttttgggga agtttgcgat ttttcgtcgg 2760
   tcgttcggtg tttcgtacgt ttcgtttcgt gagttttaat ttcgttcgtt tcgtttagtt 2820
   ttaagtttcg cgttgtgttt gtttcgtttt atttattacg ttcgtatgtt aaggttcgtt 2880
   ttaggggttt tagggcgtag gggatgtgtg agatttttag ttttttttcg ttttgtagaa 2940
   gtggcgttag aggggtgtcg gtgcgttgag taaggaggta tggaaaaatg gggtgttgtt 3000
   g 3001
<210> 245
   <211> 3001
   <212> DNA
   <213> human
<400> 245
   ttagagagta ggtgtttttt aagttaaggt agtgtttttt tagtgtaagt ttgtgatatt      60
   ttgatagatt tttttgggtg agaatatttt tttttggttt tttttttttt tttaaattgg     120
   ggagggttgt ggtggggttg ggggtggggg tgggaagggg gaagaatttg gatggggaag     180
   ggaggagttt aggtatgtgt gtgggtgggg atattggggt tttttaagtg ttttatgtag     240
   tttagggagt tttttttttg tgggattttg gtaagagttt gggaggtttt tgttttaggg     300
   ttatttagtt ttgagatttt ttattttttt ttggtttttg gttgagaatt tagttttttt      360
   aggtttgttt gttgttggga taattttgga ggttgaagtt aggggttttg aagggaggga     420
   agagaggaat tttggttagg aagattttgg tgattattat gtagtggata ttggtattta    480
   gggttgggtt tgttagttag atatttttgt tttaggttaa gggttttggg taaaggattt     540
   tttattattg aagttggttt tttgatttga ggaaagagtt tatggaagaa agtagggtta     600
   taggaatgta agggtatgtg gtaggtatat agtgggtatt ttaattgttt tttatagttt     660
   gtttttaaag ttgtttttag tagtttttat ttatttgggt attttgtaga taatattagg     720
   gaagggatat tttttattag gggttgggag ttttttttta gtttttttgg agttagttta    780
   gagtagtttt ggtttttgtt tatttttgta tttattttga gtgttttatt ggagtattga     840
   tttaggggtg gtgggtttgg ggatgtttat gggtttgttt ttttatattt atggttaaga     900
   tgggaaattt gtgtttagat ttgttagggt tggattagag ggttaggagg ttaaagtatt     960
   tagtttttag gtttatagtg gtgagattat atttttttgt agtattgtga agaaggggag    1020
   gttgtgagtt gggtgggtat ttattttttg tatttaggat atgtatgtaa attaatatat    1080
   gtaaattaag tattttaaat gatttattta aagattggga tgtttgggat ttttttttta    1140
   ggaggtgaaa tatttttaat ttttttttat tttttttttt ttttattttt ttgagttttt    1200
   tttatttaga ttgggtgtag gggtgtagag tttttttttt tagtagggtt ttaagtttaa    1260
   gttgggggta atggggagga ggggtgtttg aggagtattt tttttttttg ggagttttgt    1320
   agaggaagag ggtttttggt aattagtatt ttttattttt atttttttag tgggtgattt    1380
   tttagagaag gttttttagt gagtttggaa tatgtgttga gtgtttggag tgttattgtg   1440
   tgtatgtgag agtatattgg tgaggttgtt tatgtatgtt attgtggata aagatagatg    1500
   tgattgtgtg tgtgtgggga ttgtgtatat gtttttgtta gtgtgtgtga ttatgagtta    1560
   gttgggtatg tgtgtttgtg ggtattaggt gtatggttgt gtgtatatgt gtgtgtaaga    1620
   gttggtgtat ttgtgtttta gtgtggttaa ggttttgatt gagatgttgt gtgtgtgggg    1680
   gtgggtgtga ttgaatgtgg gtatgggtgt ttgtttgttt atatgtgtgt ttgtgtgttt    1740
   gggtgtgtag gtggttttgt gtaagtgttt gtgtgtaggg ggttggtata tgggggagga    1800
   tgtttatggt tgggtaattg taggtttgtt tgattttttg ttatttagga ttgtgtttat    1860
   atggtgtttg ggttatagtg tgtttgagtt gttgtgtgtg tttttgttgt ggggtgtggg    1920
   ttgttatggg gtaattgtat ttttttgtgt ttttatggtt aggggtatag ggggtgttgg   1980
   gttttttttg ggggtttttt tgtgtgtgtg tgtaattatg tgtttagttt atatttttat   2040
   agtgtgtttt tgtgtttttt tgggtattta attattgtat gtgtgtttgg gattttagtg    2100
   ataagtgttt tggtgttttt gtgtgtggat ttgtgttttt tggggaggtt tgtggtttag    2160
   gtttgatttt tgtgatttgt tttaggtagg tttgtatgtg tgtggtggtt gtgtgttgta    2220
   tagtgtgagg gaatgtgtat taaatgtttg tgggatattt gtgtttgttt agttaagagt    2280
   gtatttgtgt gtgtgagtgg gtttttggga tgttgttgtg gttgggggtg gttttgtgag    2340
   gggagggggt tatagggatt ggttggtgtt ggttttgtgt gtatggaggt gggggtgggg   2400
   ggtgggggtt gtgagggggg aggtggtatg aaaagggtgg tgtgtgtggt ggtggtggta    2460
   gttttttggt agtggtggtg gagagtgtag tgtgtagttt ggtgtagttt tggttttttt   2520
   tttgttgtgt gtttgtgttt ttttttgtgt ttgtaattag aagtttagtg tggtgttagg    2580
   agttggattt gtgtttgtat tgtttttggg attgtgattt tggttgttta gagatgattg    2640
   tgattgaagt ttttttgtgt gtttttttgt ttttgttggt ttttggttat agtatttatg   2700
   gtgagttttt tggtggtttg gtttgtgttt tttttgggga agtttgtgat tttttgttgg    2760
   ttgtttggtg ttttgtatgt tttgttttgt gagttttaat tttgtttgtt ttgtttagtt    2820
   ttaagttttg tgttgtgttt gttttgtttt atttattatg tttgtatgtt aaggtttgtt    2880
   ttaggggttt tagggtgtag gggatgtgtg agatttttag tttttttttg ttttgtagaa    2940
   gtggtgttag aggggtgttg gtgtgttgag taaggaggta tggaaaaatg gggtgttgtt    3000
   g                                                                    3001
<210> 246
   <211> 50
   <212> DNA
   <213> human
<400> 246
   tggtcttgag tcttcagggt gctccaggac cccgtagatc tgcaggtacg 50
<210> 247
   <211> 50
   <212> DNA
   <213> human
<400> 247
   tggttttgag tttttagggt gttttaggat ttcgtagatt tgtaggtacg 50
<210> 248
   <211> 50
   <212> DNA
   <213> human
<400> 248
   tggttttgag tttttagggt gttttaggat tttgtagatt tgtaggtatg 50
<210> 249
   <211> 50
   <212> DNA
   <213> human
<400> 249
   cgtatttgta gatttacggg gttttggagt attttgaaga tttaagatta 50
<210> 250
   <211> 50
   <212> DNA
   <213> human
<400> 250
   tgtatttgta gatttatggg gttttggagt attttgaaga tttaagatta 50
<210> 251
   <211> 3001
   <212> DNA
   <213> human
<400> 251
   caatgctgtc tgatccatct agcaccaggt agatgttcat ggagcctgaa gggtccagga      60
   cgatcttccg cttctgttgt tcccctgggt gccaggagag tggctcaggc tccagcatta     120
   acagttctgt cccttctcca ttttccccca gttccctgcc ctgcctccct tctctgtctt      180
   caaacctggg ccgtgcccat cctcagcatc gactccttct atggtctctg tcagggaaga     240
   caggaaagct tcggccacct cttgaggggt gtcgtacatg aaggagtctg ggagagtcag     300
   aaatgaggtc aaatgtctgg gagtgtcagg gatacagtga ccaaagagac gggggatcat     360
   gggtttccag ggtataaaag gctcagaagt gagatagttg tacagggagg tttaaacaaa     420
   gtgaggaaag agcagggttg aggtggggag agaagacagt aggatggaag accaggatct     480
   gacctggggg tacaggtcaa aggtcacctt ggcaggaagg ctccgtcccg ctccaagagc     540
   caccttcctg acacgttcgc cgctgggagc cacgcagggt aagcccccgg ctgcagtggt     600
   aggtgacgct gtcttcaagg cggtactggc tgcccacctt ccttgtgcca atggggatgc     660
   ccgggttgga gcagtacccc gctgcagagg tatgagacat cgaggtaagc actgaagcct     720
   gaggccccgt gagcaaggta gagagcaaga gttacagtgt ccggagccga gtgcccactc     780
   ctcgggctgg gcgccgtcag ggagacagca atgtaggggg aggggatgct tctcacctcc     840
   gttgtcacag atcgctgtct gcccactcca tcggccattc acttggcagg tgcgattggc     900
   agagccccgg agagtgtaac cgtcatagca gtggaaagag atctcatcac tcacattgta     960
   gtagggagac cggggccagt attccccgtt ctcgaagtcg tgtggtcttg gacagtggat    1020
   tgctttgaga aggggggaca agtagaagtc atcaagaggg aaaggctgcc ttaggtgtat   1080
   ccctcctggt ctcggagacc atgtcactga gaaacagcgc attcccagtc ccgcagaagc    1140
   agcatcttac ctactcctca acccatatgg atttccactg cttctccctc cccatttctg    1200
   agtgttctct tgacttccag ggctgcctgg aagcccaggg taaatgctta gtaagggtta    1260
   actccgcttt ttcttgcccc ctttccgcct gccaccctaa aactgctcct actcccggtc    1320
   agcccacctt gtcaccctgc ctagtctcat cctagtcctg accttgctgc cgcctgccct    1380
   gtttctgcct taggccactg cccacactca ttgccctcaa acctctgcac tctgccttcc    1440
   tgacagtctt ttggtcttga gtcttcaggg tgctccagga ccccgtagat ctgcaggtac    1500
   gtgtctgcac agggtacggg tagaagccag aaggacacac gtactccagt gcctggccct    1560
   cttggagaag tcggaaggag ccgcctttga tctctacccc ctccagagag caggatccct    1620
   ggggccgggc caaagaccat ggagtggtgg tcacacctga agagaaaggc tgatgaagcc    1680
   tggccccaaa aggccaagga gggatgctgg agacagcagg aagggaaggt taccctcgct    1740
   tacctccaga caagaggccc aagataaagg gcatcaggca gagttggggg ctgagattgc    1800
   tccccatggc gttggaaggc aggagagaag ctgggcctgg ggcaggatgg tgtgtcctgg    1860
   cttgctttgc ttgtctgctt ggctcagtgt ccaagctgaa actccagacc tagacctggt    1920
   cacattccct tcccctgctc cccaccagcc cccagccttt tatacaatct gtgttctggc    1980
   acctgcggct cgccccgcct gtcctaccca catcactttc ccggaacatc caagcgggag    2040
   ggccccgctg agctgccagt caaggaaaca gaaactgcag aagtcccacc ctttgctgcc    2100
   aaaggtccag gactctcccc ttcagtacct cctctccggc cttagctcct ccccagtaag    2160
   cccaaaccac ccacttaggg accagaaata aggatccagc tcactcccct gttgattgtg    2220
   tgttatggtg cagagtccag ccactgtttg tccagtgggg tctctgacct gccttcctgt    2280
   agctcttgga gtcattctgg cctccccctc ccccaaggcc agccctacct ggcctccaga    2340
   tagaggcact gagagatgtg gaaaccattg atttttatta atttcataac tgggaaattc    2400
   catgtgaaag tgaaacaagc atgagtcaag tcaaccaggg aaggaatctg gggacaggcc    2460
   aaggagcggg aggtggggca gcgaggcagt cctgctggta ggagccctga ggatttccca    2520
   gcttgtgtgc gctgcctctg gcatcctaga gacccggatt tactcagcta ggagagagga    2580
   tggatcacag ggtctaaggg tggccattca gaggtagaag atggaggggc ggcagattct    2640
   ggcagggcag cagagggctc agtggccatg gctagagggg taaaaaattc aggacatccc    2700
   ccaggtgctg cctcagccag ggctgcatgc ggaagagatt gatgtgaaag tctcgtggcg    2760
   gcgggacctt gctacgaggg gcccttttgc gggagttttt gtcagcagag ccaaggcagg   2820
   ggttgtaaag accccagctc accagaccca cctggagaaa aggagagaac tggctgggag    2880
   gctgccacag ccccaactgt aagccccaac ctccctagat ccctcaaagg cctccctcat    2940
   ccccccaaca aggctgagat cctgtaaccc ctgggtcctg caagcttcta ccttctcacc     3000
   t                                                                    3001
<210> 252
   <211> 3001
   <212> DNA
   <213> human
<400> 252
   taatgttgtt tgatttattt agtattaggt agatgtttat ggagtttgaa gggtttagga 60
   cgatttttcg tttttgttgt ttttttgggt gttaggagag tggtttaggt tttagtatta 120
   atagttttgt ttttttttta ttttttttta gttttttgtt ttgttttttt tttttgtttt 180
   taaatttggg tcgtgtttat ttttagtatc gatttttttt atggtttttg ttagggaaga 240
   taggaaagtt tcggttattt tttgaggggt gtcgtatatg aaggagtttg ggagagttag 300
   aaatgaggtt aaatgtttgg gagtgttagg gatatagtga ttaaagagac gggggattat 360
   gggtttttag ggtataaaag gtttagaagt gagatagttg tatagggagg tttaaataaa 420
   gtgaggaaag agtagggttg aggtggggag agaagatagt aggatggaag attaggattt 480
   gatttggggg tataggttaa aggttatttt ggtaggaagg tttcgtttcg ttttaagagt 540
   tatttttttg atacgttcgt cgttgggagt tacgtagggt aagttttcgg ttgtagtggt 600
   aggtgacgtt gtttttaagg cggtattggt tgtttatttt ttttgtgtta atggggatgt 660
   tcgggttgga gtagtatttc gttgtagagg tatgagatat cgaggtaagt attgaagttt 720
   gaggtttcgt gagtaaggta gagagtaaga gttatagtgt tcggagtcga gtgtttattt 780
   ttcgggttgg gcgtcgttag ggagatagta atgtaggggg aggggatgtt ttttattttc 840
   gttgttatag atcgttgttt gtttatttta tcggttattt atttggtagg tgcgattggt 900
   agagtttcgg agagtgtaat cgttatagta gtggaaagag attttattat ttatattgta 960
   gtagggagat cggggttagt atttttcgtt ttcgaagtcg tgtggttttg gatagtggat 1020
   tgttttgaga aggggggata agtagaagtt attaagaggg aaaggttgtt ttaggtgtat 1080
   tttttttggt ttcggagatt atgttattga gaaatagcgt atttttagtt tcgtagaagt 1140
   agtattttat ttatttttta atttatatgg atttttattg tttttttttt tttatttttg 1200
   agtgtttttt tgatttttag ggttgtttgg aagtttaggg taaatgttta gtaagggtta 1260
   atttcgtttt tttttgtttt tttttcgttt gttattttaa aattgttttt attttcggtt 1320
   agtttatttt gttattttgt ttagttttat tttagttttg attttgttgt cgtttgtttt 1380
   gtttttgttt taggttattg tttatattta ttgtttttaa atttttgtat tttgtttttt 1440
   tgatagtttt ttggttttga gtttttaggg tgttttagga tttcgtagat ttgtaggtac 1500
   gtgtttgtat agggtacggg tagaagttag aaggatatac gtattttagt gtttggtttt 1560
   tttggagaag tcggaaggag tcgtttttga tttttatttt ttttagagag taggattttt 1620
   ggggtcgggt taaagattat ggagtggtgg ttatatttga agagaaaggt tgatgaagtt 1680
   tggttttaaa aggttaagga gggatgttgg agatagtagg aagggaaggt tattttcgtt 1740
   tatttttaga taagaggttt aagataaagg gtattaggta gagttggggg ttgagattgt 1800
   tttttatggc gttggaaggt aggagagaag ttgggtttgg ggtaggatgg tgtgttttgg 1860
   tttgttttgt ttgtttgttt ggtttagtgt ttaagttgaa attttagatt tagatttggt 1920
   tatatttttt ttttttgttt tttattagtt tttagttttt tatataattt gtgttttggt 1980
   atttgcggtt cgtttcgttt gttttattta tattattttt tcggaatatt taagcgggag 2040
   ggtttcgttg agttgttagt taaggaaata gaaattgtag aagttttatt ttttgttgtt 2100
   aaaggtttag gatttttttt tttagtattt ttttttcggt tttagttttt ttttagtaag 2160
   tttaaattat ttatttaggg attagaaata aggatttagt ttattttttt gttgattgtg 2220
   tgttatggtg tagagtttag ttattgtttg tttagtgggg tttttgattt gtttttttgt 2280
   agtttttgga gttattttgg tttttttttt ttttaaggtt agttttattt ggtttttaga 2340
   tagaggtatt gagagatgtg gaaattattg atttttatta attttataat tgggaaattt 2400
   tatgtgaaag tgaaataagt atgagttaag ttaattaggg aaggaatttg gggataggtt 2460
   aaggagcggg aggtggggta gcgaggtagt tttgttggta ggagttttga ggatttttta 2520
   gtttgtgtgc gttgtttttg gtattttaga gattcggatt tatttagtta ggagagagga 2580
   tggattatag ggtttaaggg tggttattta gaggtagaag atggaggggc ggtagatttt 2640
   ggtagggtag tagagggttt agtggttatg gttagagggg taaaaaattt aggatatttt 2700
   ttaggtgttg ttttagttag ggttgtatgc ggaagagatt gatgtgaaag tttcgtggcg 2760
   gcgggatttt gttacgaggg gtttttttgc gggagttttt gttagtagag ttaaggtagg 2820
   ggttgtaaag attttagttt attagattta tttggagaaa aggagagaat tggttgggag 2880
   gttgttatag ttttaattgt aagttttaat ttttttagat tttttaaagg ttttttttat 2940
   ttttttaata aggttgagat tttgtaattt ttgggttttg taagttttta tttttttatt 3000
   t 3001
<210> 253
   <211> 3001
   <212> DNA
   <213> human
<400> 253
   taatgttgtt tgatttattt agtattaggt agatgtttat ggagtttgaa gggtttagga    60
   tgattttttg tttttgttgt ttttttgggt gttaggagag tggtttaggt tttagtatta   120
   atagttttgt ttttttttta ttttttttta gttttttgtt ttgttttttt tttttgtttt    180
   taaatttggg ttgtgtttat ttttagtatt gatttttttt atggtttttg ttagggaaga    240
   taggaaagtt ttggttattt tttgaggggt gttgtatatg aaggagtttg ggagagttag   300
   aaatgaggtt aaatgtttgg gagtgttagg gatatagtga ttaaagagat gggggattat   360
   gggtttttag ggtataaaag gtttagaagt gagatagttg tatagggagg tttaaataaa   420
   gtgaggaaag agtagggttg aggtggggag agaagatagt aggatggaag attaggattt   480
   gatttggggg tataggttaa aggttatttt ggtaggaagg ttttgttttg ttttaagagt   540
   tatttttttg atatgtttgt tgttgggagt tatgtagggt aagtttttgg ttgtagtggt  600
   aggtgatgtt gtttttaagg tggtattggt tgtttatttt ttttgtgtta atggggatgt   660
   ttgggttgga gtagtatttt gttgtagagg tatgagatat tgaggtaagt attgaagttt   720
   gaggttttgt gagtaaggta gagagtaaga gttatagtgt ttggagttga gtgtttattt   780
   tttgggttgg gtgttgttag ggagatagta atgtaggggg aggggatgtt ttttattttt   840
   gttgttatag attgttgttt gtttatttta ttggttattt atttggtagg tgtgattggt   900
   agagttttgg agagtgtaat tgttatagta gtggaaagag attttattat ttatattgta   960
   gtagggagat tggggttagt attttttgtt tttgaagttg tgtggttttg gatagtggat  1020
   tgttttgaga aggggggata agtagaagtt attaagaggg aaaggttgtt ttaggtgtat   1080
   tttttttggt tttggagatt atgttattga gaaatagtgt atttttagtt ttgtagaagt   1140
   agtattttat ttatttttta atttatatgg atttttattg tttttttttt tttatttttg   1200
   agtgtttttt tgatttttag ggttgtttgg aagtttaggg taaatgttta gtaagggtta  1260
   attttgtttt tttttgtttt ttttttgttt gttattttaa aattgttttt atttttggtt  1320
   agtttatttt gttattttgt ttagttttat tttagttttg attttgttgt tgtttgtttt  1380
   gtttttgttt taggttattg tttatattta ttgtttttaa atttttgtat tttgtttttt  1440
   tgatagtttt ttggttttga gtttttaggg tgttttagga ttttgtagat ttgtaggtat  1500
   gtgtttgtat agggtatggg tagaagttag aaggatatat gtattttagt gtttggtttt  1560
   tttggagaag ttggaaggag ttgtttttga tttttatttt ttttagagag taggattttt  1620
   ggggttgggt taaagattat ggagtggtgg ttatatttga agagaaaggt tgatgaagtt  1680
   tggttttaaa aggttaagga gggatgttgg agatagtagg aagggaaggt tatttttgtt  1740
   tatttttaga taagaggttt aagataaagg gtattaggta gagttggggg ttgagattgt  1800
   tttttatggt gttggaaggt aggagagaag ttgggtttgg ggtaggatgg tgtgttttgg  1860
   tttgttttgt ttgtttgttt ggtttagtgt ttaagttgaa attttagatt tagatttggt  1920
   tatatttttt ttttttgttt tttattagtt tttagttttt tatataattt gtgttttggt  1980
   atttgtggtt tgttttgttt gttttattta tattattttt ttggaatatt taagtgggag  2040
   ggttttgttg agttgttagt taaggaaata gaaattgtag aagttttatt ttttgttgtt  2100
   aaaggtttag gatttttttt tttagtattt tttttttggt tttagttttt ttttagtaag  2160
   tttaaattat ttatttaggg attagaaata aggatttagt ttattttttt gttgattgtg 2220
   tgttatggtg tagagtttag ttattgtttg tttagtgggg tttttgattt gtttttttgt  2280
   agtttttgga gttattttgg tttttttttt ttttaaggtt agttttattt ggtttttaga   2340
   tagaggtatt gagagatgtg gaaattattg atttttatta attttataat tgggaaattt  2400
   tatgtgaaag tgaaataagt atgagttaag ttaattaggg aaggaatttg gggataggtt  2460
   aaggagtggg aggtggggta gtgaggtagt tttgttggta ggagttttga ggatttttta  2520
   gtttgtgtgt gttgtttttg gtattttaga gatttggatt tatttagtta ggagagagga   2580
   tggattatag ggtttaaggg tggttattta gaggtagaag atggaggggt ggtagatttt  2640
   ggtagggtag tagagggttt agtggttatg gttagagggg taaaaaattt aggatatttt  2700
   ttaggtgttg ttttagttag ggttgtatgt ggaagagatt gatgtgaaag ttttgtggtg  2760
   gtgggatttt gttatgaggg gtttttttgt gggagttttt gttagtagag ttaaggtagg   2820
   ggttgtaaag attttagttt attagattta tttggagaaa aggagagaat tggttgggag   2880
   gttgttatag ttttaattgt aagttttaat ttttttagat tttttaaagg ttttttttat   2940
   ttttttaata aggttgagat tttgtaattt ttgggttttg taagttttta tttttttatt    3000
   t                                                                  3001
<210> 254
   <211> 3001
   <212> DNA
   <213> human
<400> 254
   aggtgagaag gtagaagttt gtaggattta ggggttatag gattttagtt ttgttggggg    60
   gatgagggag gtttttgagg gatttaggga ggttggggtt tatagttggg gttgtggtag  120
   ttttttagtt agtttttttt ttttttttag gtgggtttgg tgagttgggg tttttataat   180
   ttttgttttg gttttgttga taaaaatttt cgtaaaaggg tttttcgtag taaggtttcg    240
   tcgttacgag atttttatat taattttttt cgtatgtagt tttggttgag gtagtatttg     300
   ggggatgttt tgaatttttt atttttttag ttatggttat tgagtttttt gttgttttgt     360
   tagaatttgt cgttttttta ttttttattt ttgaatggtt atttttagat tttgtgattt    420
   attttttttt ttagttgagt aaattcgggt ttttaggatg ttagaggtag cgtatataag     480
   ttgggaaatt tttagggttt ttattagtag gattgtttcg ttgttttatt tttcgttttt     540
   tggtttgttt ttagattttt tttttggttg atttgattta tgtttgtttt atttttatat     600
   ggaatttttt agttatgaaa ttaataaaaa ttaatggttt ttatattttt tagtgttttt     660
   atttggaggt taggtagggt tggttttggg ggagggggag gttagaatga ttttaagagt     720
   tataggaagg taggttagag attttattgg ataaatagtg gttggatttt gtattataat     780
   atataattaa taggggagtg agttggattt ttatttttgg tttttaagtg ggtggtttgg     840
   gtttattggg gaggagttaa ggtcggagag gaggtattga aggggagagt tttggatttt     900
   tggtagtaaa gggtgggatt tttgtagttt ttgttttttt gattggtagt ttagcggggt     960
   tttttcgttt ggatgtttcg ggaaagtgat gtgggtagga taggcggggc gagtcgtagg    1020
   tgttagaata tagattgtat aaaaggttgg gggttggtgg ggagtagggg aagggaatgt    1080
   gattaggttt aggtttggag ttttagtttg gatattgagt taagtagata agtaaagtaa    1140
   gttaggatat attattttgt tttaggttta gttttttttt tgttttttaa cgttatgggg   1200
   agtaatttta gtttttaatt ttgtttgatg ttttttattt tgggtttttt gtttggaggt   1260
   aagcgagggt aatttttttt ttttgttgtt tttagtattt ttttttggtt ttttggggtt  1320
   aggttttatt agtttttttt tttaggtgtg attattattt tatggttttt ggttcggttt   1380
   tagggatttt gttttttgga gggggtagag attaaaggcg gtttttttcg atttttttaa  1440
   gagggttagg tattggagta cgtgtgtttt tttggttttt attcgtattt tgtgtagata    1500
   cgtatttgta gatttacggg gttttggagt attttgaaga tttaagatta aaagattgtt    1560
   aggaaggtag agtgtagagg tttgagggta atgagtgtgg gtagtggttt aaggtagaaa    1620
   tagggtaggc ggtagtaagg ttaggattag gatgagatta ggtagggtga taaggtgggt    1680
   tgatcgggag taggagtagt tttagggtgg taggcggaaa gggggtaaga aaaagcggag    1740
   ttaattttta ttaagtattt attttgggtt tttaggtagt tttggaagtt aagagaatat    1800
   ttagaaatgg ggagggagaa gtagtggaaa tttatatggg ttgaggagta ggtaagatgt    1860
   tgtttttgcg ggattgggaa tgcgttgttt tttagtgata tggttttcga gattaggagg    1920
   gatatattta aggtagtttt ttttttttga tgatttttat ttgttttttt tttttaaagt    1980
   aatttattgt ttaagattat acgatttcga gaacggggaa tattggtttc ggttttttta    2040
   ttataatgtg agtgatgaga ttttttttta ttgttatgac ggttatattt ttcggggttt   2100
   tgttaatcgt atttgttaag tgaatggtcg atggagtggg tagatagcga tttgtgataa    2160
   cggaggtgag aagtattttt tttttttata ttgttgtttt tttgacggcg tttagttcga   2220
   ggagtgggta ttcggtttcg gatattgtaa tttttgtttt ttattttgtt tacggggttt    2280
   taggttttag tgtttatttc gatgttttat atttttgtag cggggtattg ttttaattcg    2340
   ggtattttta ttggtataag gaaggtgggt agttagtatc gttttgaaga tagcgttatt    2400
   tattattgta gtcgggggtt tattttgcgt ggtttttagc ggcgaacgtg ttaggaaggt    2460
   ggtttttgga gcgggacgga gtttttttgt taaggtgatt tttgatttgt atttttaggt    2520
   tagattttgg ttttttattt tattgttttt tttttttatt ttaattttgt ttttttttta  2580
   ttttgtttaa atttttttgt ataattattt tatttttgag ttttttatat tttggaaatt   2640
   tatgattttt cgtttttttg gttattgtat ttttgatatt tttagatatt tgattttatt    2700
   tttgattttt ttagattttt ttatgtacga tattttttaa gaggtggtcg aagttttttt    2760
   gttttttttg atagagatta tagaaggagt cgatgttgag gatgggtacg gtttaggttt    2820
   gaagatagag aagggaggta gggtagggaa ttgggggaaa atggagaagg gatagaattg    2880
   ttaatgttgg agtttgagtt atttttttgg tatttagggg aataatagaa gcggaagatc    2940
   gttttggatt ttttaggttt tatgaatatt tatttggtgt tagatggatt agatagtatt    3000
   g                                                                    3001
<210> 255
   <211> 3001
   <212> DNA
   <213> human
<400> 255
   aggtgagaag gtagaagttt gtaggattta ggggttatag gattttagtt ttgttggggg     60
   gatgagggag gtttttgagg gatttaggga ggttggggtt tatagttggg gttgtggtag    120
   ttttttagtt agtttttttt ttttttttag gtgggtttgg tgagttgggg tttttataat     180
   ttttgttttg gttttgttga taaaaatttt tgtaaaaggg ttttttgtag taaggttttg    240
   ttgttatgag atttttatat taattttttt tgtatgtagt tttggttgag gtagtatttg    300
   ggggatgttt tgaatttttt atttttttag ttatggttat tgagtttttt gttgttttgt     360
   tagaatttgt tgttttttta ttttttattt ttgaatggtt atttttagat tttgtgattt    420
   attttttttt ttagttgagt aaatttgggt ttttaggatg ttagaggtag tgtatataag    480
   ttgggaaatt tttagggttt ttattagtag gattgttttg ttgttttatt ttttgttttt    540
   tggtttgttt ttagattttt tttttggttg atttgattta tgtttgtttt atttttatat    600
   ggaatttttt agttatgaaa ttaataaaaa ttaatggttt ttatattttt tagtgttttt    660
   atttggaggt taggtagggt tggttttggg ggagggggag gttagaatga ttttaagagt    720
   tataggaagg taggttagag attttattgg ataaatagtg gttggatttt gtattataat    780
   atataattaa taggggagtg agttggattt ttatttttgg tttttaagtg ggtggtttgg    840
   gtttattggg gaggagttaa ggttggagag gaggtattga aggggagagt tttggatttt    900
   tggtagtaaa gggtgggatt tttgtagttt ttgttttttt gattggtagt ttagtggggt   960
   ttttttgttt ggatgttttg ggaaagtgat gtgggtagga taggtggggt gagttgtagg  1020
   tgttagaata tagattgtat aaaaggttgg gggttggtgg ggagtagggg aagggaatgt  1080
   gattaggttt aggtttggag ttttagtttg gatattgagt taagtagata agtaaagtaa  1140
   gttaggatat attattttgt tttaggttta gttttttttt tgttttttaa tgttatgggg  1200
   agtaatttta gtttttaatt ttgtttgatg ttttttattt tgggtttttt gtttggaggt  1260
   aagtgagggt aatttttttt ttttgttgtt tttagtattt ttttttggtt ttttggggtt  1320
   aggttttatt agtttttttt tttaggtgtg attattattt tatggttttt ggtttggttt  1380
   tagggatttt gttttttgga gggggtagag attaaaggtg gttttttttg atttttttaa  1440
   gagggttagg tattggagta tgtgtgtttt tttggttttt atttgtattt tgtgtagata  1500
   tgtatttgta gatttatggg gttttggagt attttgaaga tttaagatta aaagattgtt  1560
   aggaaggtag agtgtagagg tttgagggta atgagtgtgg gtagtggttt aaggtagaaa  1620
   tagggtaggt ggtagtaagg ttaggattag gatgagatta ggtagggtga taaggtgggt  1680
   tgattgggag taggagtagt tttagggtgg taggtggaaa gggggtaaga aaaagtggag  1740
   ttaattttta ttaagtattt attttgggtt tttaggtagt tttggaagtt aagagaatat  1800
   ttagaaatgg ggagggagaa gtagtggaaa tttatatggg ttgaggagta ggtaagatgt  1860
   tgtttttgtg ggattgggaa tgtgttgttt tttagtgata tggtttttga gattaggagg  1920
   gatatattta aggtagtttt ttttttttga tgatttttat ttgttttttt tttttaaagt  1980
   aatttattgt ttaagattat atgattttga gaatggggaa tattggtttt ggttttttta  2040
   ttataatgtg agtgatgaga ttttttttta ttgttatgat ggttatattt tttggggttt  2100
   tgttaattgt atttgttaag tgaatggttg atggagtggg tagatagtga tttgtgataa  2160
   tggaggtgag aagtattttt tttttttata ttgttgtttt tttgatggtg tttagtttga  2220
   ggagtgggta tttggttttg gatattgtaa tttttgtttt ttattttgtt tatggggttt  2280
   taggttttag tgtttatttt gatgttttat atttttgtag tggggtattg ttttaatttg  2340
   ggtattttta ttggtataag gaaggtgggt agttagtatt gttttgaaga tagtgttatt  2400
   tattattgta gttgggggtt tattttgtgt ggtttttagt ggtgaatgtg ttaggaaggt  2460
   ggtttttgga gtgggatgga gtttttttgt taaggtgatt tttgatttgt atttttaggt  2520
   tagattttgg ttttttattt tattgttttt tttttttatt ttaattttgt ttttttttta  2580
   ttttgtttaa atttttttgt ataattattt tatttttgag ttttttatat tttggaaatt  2640
   tatgattttt tgtttttttg gttattgtat ttttgatatt tttagatatt tgattttatt  2700
   tttgattttt ttagattttt ttatgtatga tattttttaa gaggtggttg aagttttttt  2760
   gttttttttg atagagatta tagaaggagt tgatgttgag gatgggtatg gtttaggttt  2820
   gaagatagag aagggaggta gggtagggaa ttgggggaaa atggagaagg gatagaattg  2880
   ttaatgttgg agtttgagtt atttttttgg tatttagggg aataatagaa gtggaagatt  2940
   gttttggatt ttttaggttt tatgaatatt tatttggtgt tagatggatt agatagtatt  3000
   g                                                                  3001
<210> 256
   <211> 50
   <212> DNA
   <213> human
<400> 256
   agaacagatg ttggtggtga gccctgaccc aggctcctgt gcacacaccg 50
<210> 257
   <211> 50
   <212> DNA
   <213> human
<400> 257
   agaatagatg ttggtggtga gttttgattt aggtttttgt gtatatatcg 50
<210> 258
   <211> 50
   <212> DNA
   <213> human
<400> 258
   agaatagatg ttggtggtga gttttgattt aggtttttgt gtatatattg 50
<210> 259
   <211> 50
   <212> DNA
   <213> human
<400> 259
   cggtgtgtgt ataggagttt gggttagggt ttattattaa tatttgtttt 50
<210> 260
   <211> 50
   <212> DNA
   <213> human
<400> 260
   tggtgtgtgt ataggagttt gggttagggt ttattattaa tatttgtttt 50
<210> 261
   <211> 3001
   <212> DNA
   <213> human
<400> 261
   catgacctgc gcctccctca tgtgggaccc caggtgtcca gcagtgctga cggcaggacc 60
   ctctgtgctt ggacaaggtc aagtctcatt gccacaaagg ccttgtgcag gaggacgcag 120
   ccctccccac gctcagggca gaggtccggc ttctgacggt gatgacactc atccttggac 180
   ggctgctcac tacggactcc aatgggctca cggcatctct gtggctacac ccaggggaga 240
   tgccacctgc ctcccacaca caggccaggg aatcacaggc ccagctgtgg ccctggcagg 300
   actggacatt gtccggccga gtgccaaaac ccacagtgat gcctgttctt accctgtcct 360
   ctgggaggcc gggccccctg agctcctctg ctgtctgagc aggaggcatc cggggcatca 420
   ctgggctccc cgcctctccg agggcactca accagagaag ccgcctgggc accagtgctc 480
   caaggacgtc tgccaggccg gctgctctcc accccagcca ccgaccccca gcaggctctt 540
   tggggcagga gtgcggagct ggacactggt accctccgtc tctgaggcct gggctgtctg 600
   tatgtccatc tgactgactg cccacctatc caggagcaac ggtgatggcc acagcagtag 660
   cagtggcagt ggcagtggca tagcagtggc agtggcatag cagtggcagt gacagtagca 720
   gtagcagtgg cagtgggagt ggcatagcag tggcagtggc agtagcagtg gcagtgggag 780
   tggcagtggc agtggcagcg gcagtggcat tagcagtggc agtggcagtg gtagcagtag 840
   cagtggcagt ggtgacagtg gcagtggaag tagcagcggc agcggcagtg gcagtggcat 900
   tagcagtggc agtggcagtg gtagcagtag cagtggcagt ggtgacagtg gcagtggaag 960
   cagcggcagc ggcagtggca gtggcattag cagtggcagt ggcagtggct gctgttgatc 1020
   acatactgtt ctgtgtcaag caccacacca ggctttttcc cacttgcatg ggcactgaaa 1080
   ccatcctgca gctttgctga ggaagccaag ctcagagtgg tgccctctgc accttgaccc 1140
   gcttctcccc aagggattcc cctgcccctg tccacctcct ggctgcccct gtggaacacc 1200
   cactgcatag ctaggttggg cacatgcatg tggccggggc ccacctggcc ttggaggaag 1260
   tgctgattgt ctcattctac aggtttggac actgaggctt gaagcagatc agagatgagc 1320
   aggtggagca agatttggac ctggacatgg cctcagagtc tcctcccacc ctctgtggta 1380
   atgggcaaag cctctcccat ggagacttcg gccaggtcgc ttgtctggcc gtgcctcagt 1440
   ttctgtgact gtagaacaga tgttggtggt gagccctgac ccaggctcct gtgcacacac 1500
   cgccgcaggg cccccgggac agctgcacac gcatgttgtg tgtgcacatg cactcgtgta 1560
   ccccacacgc gcgtgcgccc gtgcactcgt gtgtacacat aaacagagga gccagccgag 1620
   gtgtccctgc agctcaggca cagcagccct gcaacctgac tccctcccgg aggtggaggt 1680
   gggccgggct gctctcctgc cctgccggct ggctccctga ggcctggccc gtgggggagg 1740
   gggttgagca aggcccgcct ttgtggctgc ttccccacag acacagcggc agaggagtcc 1800
   cctgctcagt gcccagctgc gggtgctgca cccctgcacc accctcaccc ctgtggctgc 1860
   gcccgcttat cctcttgtga acctggatgg tggggcggga caggcccagg gtggagggcg 1920
   aggccaccag cctgtccctg atgccccaag cccctcagca gaccatggac acttcctggg 1980
   ccctggtgcc agactaggcc tggcctcttt gcacccagct cctgccagga acgcccactg 2040
   agctctctcc gttgctaggt ccctggacca ggagcagcct tgtgccaccc cttagggaag 2100
   acgtccagtc ccacccaccc ccgcggctct cctcgcagga gtccaagctg ggcctccagc 2160
   ctccctggtg agcaccccag ccagggatcc cagctcaccc ggctacttgg gtggcctggc 2220
   cccggggtcc cgggtactca catggctcct ggccctgcct caacctctgt cgacctctgc 2280
   gcggagtccc ccacgctctt cacagggaga gagaagtgag cgggctgtga acctgcacag 2340
   gcaacaagac aggattttgg ccaaacccgc ttgacaggtt ttcgattctc ccagccagga 2400
   gcagaaagga ggcagagact gccctgagcc gccaggcggg ttctgccatc tcgcagaaag 2460
   tggccggaca ggttgggtct gaagccacat agagttggga ttgtttcctc ataatcactg 2520
   tgaggctggg ctgcaggagc caagctggtt agcactgaga atccagtttg gtgcctgtga 2580
   cacggagggc ccgggcgggg aagggtgggg ggcttgcagg ggtgcctggg tcgggggctg 2640
   caggtgggag tgagcaaatg tgctgtctcg ccacctcggg acaggtggta gggcctctga 2700
   cacagagtgg tccttcctgc tgttcacagc tcagctcacc gtgggctgac cataggtagg 2760
   tgacagtccc ctttctggcc tcggtttccc tgtctgtaaa ttggggataa taagctcaag 2820
   agctgtccct gccagaaccc agtggacaga aatccagcag ctgctatggg aagcgacccg 2880
   cccagcctgc cccgccctgc ctggggaagc aggccttctg cttgaggggg actgggcgca 2940
   tgctgggcct ctggtctgcg cagtctgccc ggggtctgtg agtgcttgtg agtgggcttc 3000
   t 3001
<210> 262
   <211> 3001
   <212> DNA
   <213> human
<400> 262
   tatgatttgc gtttttttta tgtgggattt taggtgttta gtagtgttga cggtaggatt     60
   ttttgtgttt ggataaggtt aagttttatt gttataaagg ttttgtgtag gaggacgtag    120
   ttttttttac gtttagggta gaggttcggt ttttgacggt gatgatattt atttttggac    180
   ggttgtttat tacggatttt aatgggttta cggtattttt gtggttatat ttaggggaga    240
   tgttatttgt tttttatata taggttaggg aattataggt ttagttgtgg ttttggtagg    300
   attggatatt gttcggtcga gtgttaaaat ttatagtgat gtttgttttt attttgtttt    360
   ttgggaggtc gggttttttg agtttttttg ttgtttgagt aggaggtatt cggggtatta    420
   ttgggttttt cgttttttcg agggtattta attagagaag tcgtttgggt attagtgttt    480
   taaggacgtt tgttaggtcg gttgtttttt attttagtta tcgattttta gtaggttttt    540
   tggggtagga gtgcggagtt ggatattggt atttttcgtt tttgaggttt gggttgtttg    600
   tatgtttatt tgattgattg tttatttatt taggagtaac ggtgatggtt atagtagtag    660
   tagtggtagt ggtagtggta tagtagtggt agtggtatag tagtggtagt gatagtagta    720
   gtagtagtgg tagtgggagt ggtatagtag tggtagtggt agtagtagtg gtagtgggag    780
   tggtagtggt agtggtagcg gtagtggtat tagtagtggt agtggtagtg gtagtagtag    840
   tagtggtagt ggtgatagtg gtagtggaag tagtagcggt agcggtagtg gtagtggtat    900
   tagtagtggt agtggtagtg gtagtagtag tagtggtagt ggtgatagtg gtagtggaag    960
   tagcggtagc ggtagtggta gtggtattag tagtggtagt ggtagtggtt gttgttgatt   1020
   atatattgtt ttgtgttaag tattatatta ggtttttttt tatttgtatg ggtattgaaa   1080
   ttattttgta gttttgttga ggaagttaag tttagagtgg tgttttttgt attttgattc   1140
   gttttttttt aagggatttt tttgtttttg tttatttttt ggttgttttt gtggaatatt   1200
   tattgtatag ttaggttggg tatatgtatg tggtcggggt ttatttggtt ttggaggaag   1260
   tgttgattgt tttattttat aggtttggat attgaggttt gaagtagatt agagatgagt   1320
   aggtggagta agatttggat ttggatatgg ttttagagtt tttttttatt ttttgtggta   1380
   atgggtaaag ttttttttat ggagatttcg gttaggtcgt ttgtttggtc gtgttttagt   1440
   ttttgtgatt gtagaataga tgttggtggt gagttttgat ttaggttttt gtgtatatat   1500
   cgtcgtaggg ttttcgggat agttgtatac gtatgttgtg tgtgtatatg tattcgtgta   1560
   ttttatacgc gcgtgcgttc gtgtattcgt gtgtatatat aaatagagga gttagtcgag   1620
   gtgtttttgt agtttaggta tagtagtttt gtaatttgat tttttttcgg aggtggaggt   1680
   gggtcgggtt gtttttttgt tttgtcggtt ggttttttga ggtttggttc gtgggggagg   1740
   gggttgagta aggttcgttt ttgtggttgt ttttttatag atatagcggt agaggagttt   1800
   tttgtttagt gtttagttgc gggtgttgta tttttgtatt atttttattt ttgtggttgc   1860
   gttcgtttat ttttttgtga atttggatgg tggggcggga taggtttagg gtggagggcg   1920
   aggttattag tttgtttttg atgttttaag ttttttagta gattatggat attttttggg   1980
   ttttggtgtt agattaggtt tggttttttt gtatttagtt tttgttagga acgtttattg   2040
   agtttttttc gttgttaggt ttttggatta ggagtagttt tgtgttattt tttagggaag   2100
   acgtttagtt ttatttattt tcgcggtttt tttcgtagga gtttaagttg ggtttttagt   2160
   ttttttggtg agtattttag ttagggattt tagtttattc ggttatttgg gtggtttggt   2220
   ttcggggttt cgggtattta tatggttttt ggttttgttt taatttttgt cgatttttgc   2280
   gcggagtttt ttacgttttt tatagggaga gagaagtgag cgggttgtga atttgtatag   2340
   gtaataagat aggattttgg ttaaattcgt ttgataggtt ttcgattttt ttagttagga   2400
   gtagaaagga ggtagagatt gttttgagtc gttaggcggg ttttgttatt tcgtagaaag   2460
   tggtcggata ggttgggttt gaagttatat agagttggga ttgttttttt ataattattg   2520
   tgaggttggg ttgtaggagt taagttggtt agtattgaga atttagtttg gtgtttgtga   2580
   tacggagggt tcgggcgggg aagggtgggg ggtttgtagg ggtgtttggg tcgggggttg   2640
   taggtgggag tgagtaaatg tgttgtttcg ttatttcggg ataggtggta gggtttttga   2700
   tatagagtgg ttttttttgt tgtttatagt ttagtttatc gtgggttgat tataggtagg   2760
   tgatagtttt ttttttggtt tcggtttttt tgtttgtaaa ttggggataa taagtttaag   2820
   agttgttttt gttagaattt agtggataga aatttagtag ttgttatggg aagcgattcg   2880
   tttagtttgt ttcgttttgt ttggggaagt aggttttttg tttgaggggg attgggcgta   2940
   tgttgggttt ttggtttgcg tagtttgttc ggggtttgtg agtgtttgtg agtgggtttt   3000
   t                                                                     3001
<210> 263
   <211> 3001
   <212> DNA
   <213> human
<400> 263
   tatgatttgt gtttttttta tgtgggattt taggtgttta gtagtgttga tggtaggatt     60
   ttttgtgttt ggataaggtt aagttttatt gttataaagg ttttgtgtag gaggatgtag    120
   ttttttttat gtttagggta gaggtttggt ttttgatggt gatgatattt atttttggat    180
   ggttgtttat tatggatttt aatgggttta tggtattttt gtggttatat ttaggggaga    240
   tgttatttgt tttttatata taggttaggg aattataggt ttagttgtgg ttttggtagg    300
   attggatatt gtttggttga gtgttaaaat ttatagtgat gtttgttttt attttgtttt    360
   ttgggaggtt gggttttttg agtttttttg ttgtttgagt aggaggtatt tggggtatta    420
   ttgggttttt tgtttttttg agggtattta attagagaag ttgtttgggt attagtgttt    480
   taaggatgtt tgttaggttg gttgtttttt attttagtta ttgattttta gtaggttttt    540
   tggggtagga gtgtggagtt ggatattggt attttttgtt tttgaggttt gggttgtttg    600
   tatgtttatt tgattgattg tttatttatt taggagtaat ggtgatggtt atagtagtag    660
   tagtggtagt ggtagtggta tagtagtggt agtggtatag tagtggtagt gatagtagta    720
   gtagtagtgg tagtgggagt ggtatagtag tggtagtggt agtagtagtg gtagtgggag    780
   tggtagtggt agtggtagtg gtagtggtat tagtagtggt agtggtagtg gtagtagtag    840
   tagtggtagt ggtgatagtg gtagtggaag tagtagtggt agtggtagtg gtagtggtat    900
   tagtagtggt agtggtagtg gtagtagtag tagtggtagt ggtgatagtg gtagtggaag    960
   tagtggtagt ggtagtggta gtggtattag tagtggtagt ggtagtggtt gttgttgatt   1020
   atatattgtt ttgtgttaag tattatatta ggtttttttt tatttgtatg ggtattgaaa   1080
   ttattttgta gttttgttga ggaagttaag tttagagtgg tgttttttgt attttgattt   1140
   gttttttttt aagggatttt tttgtttttg tttatttttt ggttgttttt gtggaatatt   1200
   tattgtatag ttaggttggg tatatgtatg tggttggggt ttatttggtt ttggaggaag   1260
   tgttgattgt tttattttat aggtttggat attgaggttt gaagtagatt agagatgagt   1320
   aggtggagta agatttggat ttggatatgg ttttagagtt tttttttatt ttttgtggta   1380
   atgggtaaag ttttttttat ggagattttg gttaggttgt ttgtttggtt gtgttttagt   1440
   ttttgtgatt gtagaataga tgttggtggt gagttttgat ttaggttttt gtgtatatat   1500
   tgttgtaggg tttttgggat agttgtatat gtatgttgtg tgtgtatatg tatttgtgta   1560
   ttttatatgt gtgtgtgttt gtgtatttgt gtgtatatat aaatagagga gttagttgag   1620
   gtgtttttgt agtttaggta tagtagtttt gtaatttgat ttttttttgg aggtggaggt   1680
   gggttgggtt gtttttttgt tttgttggtt ggttttttga ggtttggttt gtgggggagg   1740
   gggttgagta aggtttgttt ttgtggttgt ttttttatag atatagtggt agaggagttt   1800
   tttgtttagt gtttagttgt gggtgttgta tttttgtatt atttttattt ttgtggttgt   1860
   gtttgtttat ttttttgtga atttggatgg tggggtggga taggtttagg gtggagggtg   1920
   aggttattag tttgtttttg atgttttaag ttttttagta gattatggat attttttggg   1980
   ttttggtgtt agattaggtt tggttttttt gtatttagtt tttgttagga atgtttattg   2040
   agtttttttt gttgttaggt ttttggatta ggagtagttt tgtgttattt tttagggaag   2100
   atgtttagtt ttatttattt ttgtggtttt ttttgtagga gtttaagttg ggtttttagt   2160
   ttttttggtg agtattttag ttagggattt tagtttattt ggttatttgg gtggtttggt   2220
   tttggggttt tgggtattta tatggttttt ggttttgttt taatttttgt tgatttttgt   2280
   gtggagtttt ttatgttttt tatagggaga gagaagtgag tgggttgtga atttgtatag   2340
   gtaataagat aggattttgg ttaaatttgt ttgataggtt tttgattttt ttagttagga   2400
   gtagaaagga ggtagagatt gttttgagtt gttaggtggg ttttgttatt ttgtagaaag   2460
   tggttggata ggttgggttt gaagttatat agagttggga ttgttttttt ataattattg   2520
   tgaggttggg ttgtaggagt taagttggtt agtattgaga atttagtttg gtgtttgtga   2580
   tatggagggt ttgggtgggg aagggtgggg ggtttgtagg ggtgtttggg ttgggggttg   2640
   taggtgggag tgagtaaatg tgttgttttg ttattttggg ataggtggta gggtttttga   2700
   tatagagtgg ttttttttgt tgtttatagt ttagtttatt gtgggttgat tataggtagg   2760
   tgatagtttt ttttttggtt ttggtttttt tgtttgtaaa ttggggataa taagtttaag   2820
   agttgttttt gttagaattt agtggataga aatttagtag ttgttatggg aagtgatttg   2880
   tttagtttgt tttgttttgt ttggggaagt aggttttttg tttgaggggg attgggtgta   2940
   tgttgggttt ttggtttgtg tagtttgttt ggggtttgtg agtgtttgtg agtgggtttt   3000
   t                                                                   3001
<210> 264
   <211> 3001
   <212> DNA
   <213> human
<400> 264
   agaagtttat ttataagtat ttatagattt cgggtagatt gcgtagatta gaggtttagt 60
   atgcgtttag ttttttttaa gtagaaggtt tgttttttta ggtagggcgg ggtaggttgg 120
   gcgggtcgtt ttttatagta gttgttggat ttttgtttat tgggttttgg tagggatagt 180
   ttttgagttt attattttta atttatagat agggaaatcg aggttagaaa ggggattgtt 240
   atttatttat ggttagttta cggtgagttg agttgtgaat agtaggaagg attattttgt 300
   gttagaggtt ttattatttg tttcgaggtg gcgagatagt atatttgttt atttttattt 360
   gtagttttcg atttaggtat ttttgtaagt tttttatttt ttttcgttcg ggtttttcgt 420
   gttataggta ttaaattgga tttttagtgt taattagttt ggtttttgta gtttagtttt 480
   atagtgatta tgaggaaata attttaattt tatgtggttt tagatttaat ttgttcggtt 540
   attttttgcg agatggtaga attcgtttgg cggtttaggg tagtttttgt tttttttttg 600
   tttttggttg ggagaatcga aaatttgtta agcgggtttg gttaaaattt tgttttgttg 660
   tttgtgtagg tttatagttc gtttattttt ttttttttgt gaagagcgtg ggggatttcg 720
   cgtagaggtc gatagaggtt gaggtagggt taggagttat gtgagtattc gggatttcgg 780
   ggttaggtta tttaagtagt cgggtgagtt gggatttttg gttggggtgt ttattaggga 840
   ggttggaggt ttagtttgga tttttgcgag gagagtcgcg ggggtgggtg ggattggacg 900
   ttttttttaa ggggtggtat aaggttgttt ttggtttagg gatttagtaa cggagagagt 960
   ttagtgggcg tttttggtag gagttgggtg taaagaggtt aggtttagtt tggtattagg 1020
   gtttaggaag tgtttatggt ttgttgaggg gtttggggta ttagggatag gttggtggtt 1080
   tcgtttttta ttttgggttt gtttcgtttt attatttagg tttataagag gataagcggg 1140
   cgtagttata ggggtgaggg tggtgtaggg gtgtagtatt cgtagttggg tattgagtag 1200
   gggatttttt tgtcgttgtg tttgtgggga agtagttata aaggcgggtt ttgtttaatt 1260
   ttttttttta cgggttaggt tttagggagt tagtcggtag ggtaggagag tagttcggtt 1320
   tatttttatt ttcgggaggg agttaggttg tagggttgtt gtgtttgagt tgtagggata 1380
   tttcggttgg tttttttgtt tatgtgtata tacgagtgta cgggcgtacg cgcgtgtggg 1440
   gtatacgagt gtatgtgtat atataatatg cgtgtgtagt tgtttcgggg gttttgcggc 1500
   ggtgtgtgta taggagtttg ggttagggtt tattattaat atttgtttta tagttataga 1560
   aattgaggta cggttagata agcgatttgg tcgaagtttt tatgggagag gttttgttta 1620
   ttattataga gggtgggagg agattttgag gttatgttta ggtttaaatt ttgttttatt 1680
   tgtttatttt tgatttgttt taagttttag tgtttaaatt tgtagaatga gataattagt 1740
   atttttttta aggttaggtg ggtttcggtt atatgtatgt gtttaattta gttatgtagt 1800
   gggtgtttta taggggtagt taggaggtgg ataggggtag gggaattttt tggggagaag 1860
   cgggttaagg tgtagagggt attattttga gtttggtttt tttagtaaag ttgtaggatg 1920
   gttttagtgt ttatgtaagt gggaaaaagt ttggtgtggt gtttgatata gaatagtatg 1980
   tgattaatag tagttattgt tattgttatt gttaatgtta ttgttattgt cgttgtcgtt 2040
   gtttttattg ttattgttat tattgttatt gttattgtta ttattgttat tgttattgtt 2100
   aatgttattg ttattgtcgt tgtcgttgtt atttttattg ttattgttat tattgttatt 2160
   gttattgtta ttattgttat tgttattgtt aatgttattg tcgttgttat tgttattgtt 2220
   atttttattg ttattgttat tgttattgtt attgttatgt tatttttatt gttattgtta 2280
   ttgttattgt tattgttatt gttatgttat tgttattgtt atgttattgt tattgttatt 2340
   gttattgttg tggttattat cgttgttttt ggataggtgg gtagttagtt agatggatat 2400
   atagatagtt taggttttag agacggaggg tattagtgtt tagtttcgta tttttgtttt 2460
   aaagagtttg ttgggggtcg gtggttgggg tggagagtag tcggtttggt agacgttttt 2520
   ggagtattgg tgtttaggcg gtttttttgg ttgagtgttt tcggagaggc ggggagttta 2580
   gtgatgtttc ggatgttttt tgtttagata gtagaggagt ttagggggtt cggtttttta 2640
   gaggataggg taagaatagg tattattgtg ggttttggta ttcggtcgga taatgtttag 2700
   ttttgttagg gttatagttg ggtttgtgat tttttggttt gtgtgtggga ggtaggtggt 2760
   attttttttg ggtgtagtta tagagatgtc gtgagtttat tggagttcgt agtgagtagt 2820
   cgtttaagga tgagtgttat tatcgttaga agtcggattt ttgttttgag cgtggggagg 2880
   gttgcgtttt tttgtataag gtttttgtgg taatgagatt tgattttgtt taagtataga 2940
   gggttttgtc gttagtattg ttggatattt ggggttttat atgagggagg cgtaggttat 3000
   g 3001
<210> 265
   <211> 3001
   <212> DNA
   <213> human
<400> 265
   agaagtttat ttataagtat ttatagattt tgggtagatt gtgtagatta gaggtttagt 60
   atgtgtttag ttttttttaa gtagaaggtt tgttttttta ggtagggtgg ggtaggttgg 120
   gtgggttgtt ttttatagta gttgttggat ttttgtttat tgggttttgg tagggatagt 180
   ttttgagttt attattttta atttatagat agggaaattg aggttagaaa ggggattgtt 240
   atttatttat ggttagttta tggtgagttg agttgtgaat agtaggaagg attattttgt 300
   gttagaggtt ttattatttg ttttgaggtg gtgagatagt atatttgttt atttttattt 360
   gtagtttttg atttaggtat ttttgtaagt tttttatttt tttttgtttg ggttttttgt 420
   gttataggta ttaaattgga tttttagtgt taattagttt ggtttttgta gtttagtttt 480
   atagtgatta tgaggaaata attttaattt tatgtggttt tagatttaat ttgtttggtt 540
   attttttgtg agatggtaga atttgtttgg tggtttaggg tagtttttgt tttttttttg 600
   tttttggttg ggagaattga aaatttgtta agtgggtttg gttaaaattt tgttttgttg 660
   tttgtgtagg tttatagttt gtttattttt ttttttttgt gaagagtgtg ggggattttg 720
   tgtagaggtt gatagaggtt gaggtagggt taggagttat gtgagtattt gggattttgg 780
   ggttaggtta tttaagtagt tgggtgagtt gggatttttg gttggggtgt ttattaggga 840
   ggttggaggt ttagtttgga tttttgtgag gagagttgtg ggggtgggtg ggattggatg 900
   ttttttttaa ggggtggtat aaggttgttt ttggtttagg gatttagtaa tggagagagt 960
   ttagtgggtg tttttggtag gagttgggtg taaagaggtt aggtttagtt tggtattagg 1020
   gtttaggaag tgtttatggt ttgttgaggg gtttggggta ttagggatag gttggtggtt 1080
   ttgtttttta ttttgggttt gttttgtttt attatttagg tttataagag gataagtggg 1140
   tgtagttata ggggtgaggg tggtgtaggg gtgtagtatt tgtagttggg tattgagtag 1200
   gggatttttt tgttgttgtg tttgtgggga agtagttata aaggtgggtt ttgtttaatt 1260
   ttttttttta tgggttaggt tttagggagt tagttggtag ggtaggagag tagtttggtt 1320
   tatttttatt tttgggaggg agttaggttg tagggttgtt gtgtttgagt tgtagggata 1380
   ttttggttgg tttttttgtt tatgtgtata tatgagtgta tgggtgtatg tgtgtgtggg 1440
   gtatatgagt gtatgtgtat atataatatg tgtgtgtagt tgttttgggg gttttgtggt 1500
   ggtgtgtgta taggagtttg ggttagggtt tattattaat atttgtttta tagttataga 1560
   aattgaggta tggttagata agtgatttgg ttgaagtttt tatgggagag gttttgttta 1620
   ttattataga gggtgggagg agattttgag gttatgttta ggtttaaatt ttgttttatt 1680
   tgtttatttt tgatttgttt taagttttag tgtttaaatt tgtagaatga gataattagt 1740
   atttttttta aggttaggtg ggttttggtt atatgtatgt gtttaattta gttatgtagt 1800
   gggtgtttta taggggtagt taggaggtgg ataggggtag gggaattttt tggggagaag 1860
   tgggttaagg tgtagagggt attattttga gtttggtttt tttagtaaag ttgtaggatg 1920
   gttttagtgt ttatgtaagt gggaaaaagt ttggtgtggt gtttgatata gaatagtatg 1980
   tgattaatag tagttattgt tattgttatt gttaatgtta ttgttattgt tgttgttgtt 2040
   gtttttattg ttattgttat tattgttatt gttattgtta ttattgttat tgttattgtt 2100
   aatgttattg ttattgttgt tgttgttgtt atttttattg ttattgttat tattgttatt 2160
   gttattgtta ttattgttat tgttattgtt aatgttattg ttgttgttat tgttattgtt 2220
   atttttattg ttattgttat tgttattgtt attgttatgt tatttttatt gttattgtta 2280
   ttgttattgt tattgttatt gttatgttat tgttattgtt atgttattgt tattgttatt 2340
   gttattgttg tggttattat tgttgttttt ggataggtgg gtagttagtt agatggatat 2400
   atagatagtt taggttttag agatggaggg tattagtgtt tagttttgta tttttgtttt 2460
   aaagagtttg ttgggggttg gtggttgggg tggagagtag ttggtttggt agatgttttt 2520
   ggagtattgg tgtttaggtg gtttttttgg ttgagtgttt ttggagaggt ggggagttta 2580
   gtgatgtttt ggatgttttt tgtttagata gtagaggagt ttagggggtt tggtttttta 2640
   gaggataggg taagaatagg tattattgtg ggttttggta tttggttgga taatgtttag 2700
   ttttgttagg gttatagttg ggtttgtgat tttttggttt gtgtgtggga ggtaggtggt 2760
   attttttttg ggtgtagtta tagagatgtt gtgagtttat tggagtttgt agtgagtagt 2820
   tgtttaagga tgagtgttat tattgttaga agttggattt ttgttttgag tgtggggagg 2880
   gttgtgtttt tttgtataag gtttttgtgg taatgagatt tgattttgtt taagtataga 2940
   gggttttgtt gttagtattg ttggatattt ggggttttat atgagggagg tgtaggttat 3000
   g 3001
<210> 266
   <211> 50
   <212> DNA
   <213> human
<400> 266
   agaaggccgt cttcatgcgg ttgtgctggg agcacaccag gcggatggcg 50
<210> 267
   <211> 50
   <212> DNA
   <213> human
<400> 267
   agaaggtcgt ttttatgcgg ttgtgttggg agtatattag gcggatggcg 50
<210> 268
   <211> 50
   <212> DNA
   <213> human
<400> 268
   agaaggttgt ttttatgtgg ttgtgttggg agtatattag gtggatggtg 50
<210> 269
   <211> 50
   <212> DNA
   <213> human
<400> 269
   cgttattcgt ttggtgtgtt tttagtataa tcgtatgaag acggtttttt 50
<210> 270
   <211> 50
   <212> DNA
   <213> human
<400> 270
   tgttatttgt ttggtgtgtt tttagtataa ttgtatgaag atggtttttt 50
<210> 271
   <211> 3001
   <212> DNA
   <213> human
<400> 271
   aaggaaatgg tccctacctg ggacattata cagacgttaa tcaacgaaat gacctggctt 60

<210> 272
   <211> 3001
   <212> DNA
   <213> human
<400> 272
   aaggaaatgg tttttatttg ggatattata tagacgttaa ttaacgaaat gatttggttt     60
   tttttttttt tttttttttt aggataggac gttttatttt tttttagttc gtaacgtatt    120
   tttggatatg atggaatata cgaaggggtt tttatttata gtatgattaa tttttagtat    180
   ttaaaatcgt atatatatta atatgtgtat atatatattt atatttatat ttataaagat    240
   tttgtatttt ttgaaggtat attaatttat tatttttatt atatttattt tagtgtgtta    300
   tagttattgg tttatatgtt tgtttttgtt attttatttt atttgttttt ataatatagt    360
   aaattatttg gtttttaata aagggtttat gaattattta tgaatttatt ttgttaggtg    420
   tttagttttg tgtttggttt gaagtaggtg tttttaaggt gtggtatggt tgagtgaata    480
   taaataaaag gtatataaaa gtatttagtt ttgttttttt ttttttttta cgtttattat    540
   ttgtttcggt ttttagattt aatattggta aagagtatcg atgaaattta aaagtttagg    600
   atatatttaa gtacgtaaag gttaggtatt tgggtaggtt aatagtgtag tattttagtt    660
   tgggtttttt gggggaagag ttttataagg agttgttttt tagtttgatt tatttttttt    720
   gttttgtaag gtttttgttt ttagtttttg ggtttatttg tagttttaat ttttacgatt    780
   tttttttttg ttttttttat aataaatttt ttttttaaat aaagattgat agtattattt    840
   ttatagtatt tgtacggttt cgtttttttt tatttagttt tattttttta ttagattttt    900
   ttgttgaggt ggacgaggtt ttatattttg ttggtatttt gttttagaaa agggatgtag    960
   ttgagtatat ttttgttttt ttgtaagtat ttttttttat ttgtaggagg gagaagggaa   1020
   tgggagggaa ggagaagaga gggtagggga gggtaatgtt gttttaataa ttatattttg   1080
   ggtgagtttg gggtgttagg atggttttat tgggtaggga cgttagttta gtaagttgga   1140
   ggttttttat tttgttagta gtgagtttta tttgggatag gggtgttagt ttttattttt   1200
   aggtttgagg ttttgtttgt ttagtgagta ttttagtatt ttggattatt tttttaggcg   1260
   taggtattag ggaaggggta gagggattaa tcgatttgta gggattgagg gtgtagatgg   1320
   ttattgcggg gaagacgagt ttgttcgagt tgaggttgat gttgaggttg acggggtagt   1380
   tgaagtattt ttcgaaaagt aggtcgaatt gttagtatat tatgttaaag gtgtagagtt   1440
   atagtattgt ttagaaggtc gtttttatgc ggttgtgttg ggagtatatt aggcggatgg   1500
   cgtcgtggat ggtggtgttg ttgtagaaga attcgaagag ttttcggtag gagcggtgga   1560
   attcgattag ggtttttttt ttcgtcgtgg gttgttgggg cgtcgtaggt tcggggttta   1620
   gtttttgttt tttacgtttg ttttttttta tgagttttgg agtggattgt ggagggttag   1680
   agttttgttt ttttagtttg ttttttttta tgagatttgg tatgggttgt agaggtttag   1740
   ggttttgttt ttttagtttg ttttttttta tgagtttcgg agtggattgg ggagagtaag   1800
   ggttagggtt aaggttgagt tttgggtttt gagtgttttt ttttattatt tttggaattc   1860
   gagtgaggtt gtttttggtt atgtttatgt tttattttgc gtttatattt ttttattttt   1920
   tttttttttt tttatttttt ttggagttag tagatttgcg ggagttgggg ttaaaagtgt   1980
   cggagttggg tttttttaga acggtttttt ttttgttttt ttgatagggt tatttttcga   2040
   gttttgtttt agtatttttt tttttgtttt tgtttttttt ttttggtttt ttttttttag   2100
   gatttgtgtt ttagtttttg ttttttattt tttttttatt tgttttttgt ttttttttgg   2160
   gttttttttg tttttttttt tttggtttgt tttttttttt taattttgtt tttttttttt   2220
   tttttttttt gttttgtttt tttttatttt aggtttttag ttttagtttt tggttttgat   2280
   ttcgagttgt gttttgattt tgtttttgtt ttaggattgt agggttttag gaggtttggg   2340
   ttgtttttag ttttttattt ttaggttgcg gttggattgg gattggtttt tttttagttg   2400
   aatttggtag ttaaattttt tttttttttt atttgatagg tgtagcggtt tggttgggga   2460
   gttcgttcgt tggtcggtta gggatggaag cgataggaat tttattagta ttttaattaa   2520
   aggtgagtag ggcgggggga ggggttgagg aggagttaga gtcgggagtt tttcgaagga   2580
   aggagggttt tcgagggtag gtgaattggg agtattggat ttgagaaggc ggattttggg   2640
   taggggtttt agacgtagat aggtaaggag gttgggggat aggatggtag gtgaggggta   2700
   aagatttgaa taagtagaag gattttgagt ttatagttag gagggtaata taaggagaag   2760
   gggttagtta ggttgagagg gtttgggtgg ggaatcggga ggataggagg gtagaaagag   2820
   ggagataata gagagggata gcgaaggata gagagatagg gatggaggag gggtattgag   2880
   tgagtagagg taggtggggg gtagtggtgg gggtaaatag aaggagttag tattaaaggg   2940
   agtttgtttt taggaaggag agtaaggggg gagttttaag tgtgttttta ggagttagag   3000
   g                                                                   3001
<210> 273
   <211> 3001
   <212> DNA
   <213> human
<400> 273
<210> 274
   <211> 3001
   <212> DNA
   <213> human
<400> 274
   tttttagttt ttggaagtat atttgggatt ttttttttgt tttttttttt ggagatagat 60
   tttttttggt gttggttttt tttatttgtt tttattattg ttttttattt gtttttattt 120
   atttagtgtt ttttttttat ttttattttt ttgtttttcg ttgttttttt ttattgtttt 180
   tttttttttg tttttttgtt ttttcggttt tttatttagg tttttttagt ttggttggtt 240
   tttttttttt gtgttgtttt tttagttgtg ggtttaggat ttttttattt gtttagattt 300
   ttgtttttta tttgttattt tgttttttag tttttttgtt tgtttgcgtt taaagttttt 360
   gtttagagtt cgttttttta ggtttagtat ttttagttta tttgttttcg ggagtttttt 420
   ttttttcgga aaattttcgg ttttgatttt tttttagttt tttttttcgt tttgtttatt 480
   tttaattgag atgttaatga gatttttgtc gtttttattt ttggtcggtt agcgggcggg 540
   ttttttagtt aggtcgttgt atttgttagg tgagggggag gagaggtttg gttgttagat 600
   ttaattggaa aggaattagt tttagtttag tcgtaatttg ggagtgggaa gttggaggta 660
   gtttagattt tttggagttt tgtagttttg gggtagagat agaattagga tatagttcga 720
   ggttagggtt agaggttgga gttgagggtt tagagtgaga gggggtaagg taaggggggg 780
   agaggaagag aggtaggatt agagagagga ggtaggttag aaagaggaga gtaggagaga 840
   tttaaagaga aatagaaggt agatagagag ggagtgagag gtaggagttg agatatagat 900
   tttggaggaa gaagattaaa ggaagggggt agagatagaa agggaggtgt taggataaaa 960
   ttcgaaaggt ggttttatta gggaagtaga ggagaggtcg ttttagggaa gtttagtttc 1020
   ggtatttttg gttttaattt tcgtaggttt gttggtttta ggaaaggtgg aggagggagg 1080
   gaggagtggg agaatgtggg cgtagggtgg gatatgggta tggttagggg tagttttatt 1140
   cgggttttag gggtgatggg agagggtatt tagggtttag agtttagttt tgattttgat 1200
   ttttgttttt tttaatttat ttcggggttt atgaagggga ataagttgga ggagtaggat 1260
   tttagatttt tgtagtttat attaggtttt atggagggga ataagttgga ggagtaggat 1320
   tttagttttt tatagtttat tttagggttt atgaagggga ataagcgtga ggagtagggg 1380
   ttgggtttcg aatttgcggc gttttagtag tttacggcgg aggaggaggt tttgatcgag 1440
   ttttatcgtt tttatcgaga gtttttcgag tttttttgta ataatattat tatttacggc 1500
   gttattcgtt tggtgtgttt ttagtataat cgtatgaaga cggttttttg ggtagtgttg 1560
   tggttttgta tttttggtat gatgtattgg taattcggtt tgtttttcgg agagtatttt 1620
   agttatttcg ttagttttaa tattaatttt aattcggata agttcgtttt tttcgtagtg 1680
   attatttgta tttttaattt ttataggtcg gttagttttt ttgttttttt tttggtgttt 1740
   gcgtttggaa gggtggttta gggtgttgag gtgtttattg ggtaggtaga gttttagatt 1800
   tgagggtggg aattgatatt tttgttttag gtagagttta ttgttggtaa aatgaggagt 1860
   ttttagttta ttggattggc gtttttgttt agtggagtta ttttagtatt ttaggtttat 1920
   ttagagtgtg gttgttggag tagtattgtt tttttttgtt tttttttttt tttttttttt 1980
   attttttttt tttttttgta agtgggaggg agtgtttgta aaagggtagg ggtgtgttta 2040
   gttgtatttt ttttttggag taagatgtta gtaaagtata agatttcgtt tattttagta 2100
   ggagagtttg atggaggggt gaggttaggt gaggggaggc gagatcgtgt aggtgttgtg 2160
   gaggtggtgt tgttagtttt tgtttagaag gggaatttat tatggagaaa gtaaagaggg 2220
   ggatcgtgga ggttagggtt gtaagtgagt ttaaaggttg ggggtagggg ttttatagaa 2280
   taaggagaat gggttaggtt agagagtagt tttttgtgaa gttttttttt taaaagattt 2340
   aggttaaaat gttgtattgt tgatttgttt aggtgtttga tttttacgtg tttgggtgtg 2400
   ttttgaattt ttgggtttta tcggtgtttt ttgttaatgt tgggtttgga aatcggagta 2460
   ggtggtaagc gtggagaaga gaggagggta gggttgagtg tttttgtata ttttttattt 2520
   gtatttattt agttatgtta tattttggga atatttgttt taagttagat atagggttag 2580
   gtatttggta aaatggattt atgaataatt tataagtttt ttattgaagg ttaggtagtt 2640
   tgttgtgttg tggagataga taaaataaga tagtagagat aggtatataa gttagtaatt 2700
   gtggtatatt gggataagta tgataaaaat ggtgagttag tgtattttta aggagtgtaa 2760
   aatttttatg ggtgtgggtg tgagtgtgtg tgtgtatata ttggtatgtg tgcgattttg 2820
   gatgttgggg attggttatg ttgtgggtgg aggttttttc gtatatttta ttatgtttaa 2880
   aggtgcgtta cggattaaga aagggtggga cgttttgttt tggagagaga gagaaaaaaa 2940
   aaagttaggt tatttcgttg attaacgttt gtataatgtt ttaggtaggg attatttttt 3000
   t 3001
<210> 275
   <211> 3001
   <212> DNA
   <213> human
<400> 275
   tttttagttt ttggaagtat atttgggatt ttttttttgt tttttttttt ggagatagat 60
   tttttttggt gttggttttt tttatttgtt tttattattg ttttttattt gtttttattt 120
   atttagtgtt ttttttttat ttttattttt ttgttttttg ttgttttttt ttattgtttt 180
   tttttttttg tttttttgtt tttttggttt tttatttagg tttttttagt ttggttggtt 240
   tttttttttt gtgttgtttt tttagttgtg ggtttaggat ttttttattt gtttagattt 300
   ttgtttttta tttgttattt tgttttttag tttttttgtt tgtttgtgtt taaagttttt 360
   gtttagagtt tgttttttta ggtttagtat ttttagttta tttgtttttg ggagtttttt 420
   tttttttgga aaatttttgg ttttgatttt tttttagttt ttttttttgt tttgtttatt 480
   tttaattgag atgttaatga gatttttgtt gtttttattt ttggttggtt agtgggtggg 540
   ttttttagtt aggttgttgt atttgttagg tgagggggag gagaggtttg gttgttagat 600
   ttaattggaa aggaattagt tttagtttag ttgtaatttg ggagtgggaa gttggaggta 660
   gtttagattt tttggagttt tgtagttttg gggtagagat agaattagga tatagtttga 720
   ggttagggtt agaggttgga gttgagggtt tagagtgaga gggggtaagg taaggggggg 780
   agaggaagag aggtaggatt agagagagga ggtaggttag aaagaggaga gtaggagaga 840
   tttaaagaga aatagaaggt agatagagag ggagtgagag gtaggagttg agatatagat 900
   tttggaggaa gaagattaaa ggaagggggt agagatagaa agggaggtgt taggataaaa 960
   tttgaaaggt ggttttatta gggaagtaga ggagaggttg ttttagggaa gtttagtttt 1020
   ggtatttttg gttttaattt ttgtaggttt gttggtttta ggaaaggtgg aggagggagg 1080
   gaggagtggg agaatgtggg tgtagggtgg gatatgggta tggttagggg tagttttatt 1140
   tgggttttag gggtgatggg agagggtatt tagggtttag agtttagttt tgattttgat 1200
   ttttgttttt tttaatttat tttgggtttt atgaagggga ataagttgga ggagtaggat 1260
   tttagatttt tgtagtttat attaggtttt atggagggga ataagttgga ggagtaggat 1320
   tttagttttt tatagtttat tttagggttt atgaagggga ataagtgtga ggagtagggg 1380
   ttgggttttg aatttgtggt gttttagtag tttatggtgg aggaggaggt tttgattgag 1440
   ttttattgtt tttattgaga gttttttgag tttttttgta ataatattat tatttatggt 1500
   gttatttgtt tggtgtgttt ttagtataat tgtatgaaga tggttttttg ggtagtgttg 1560
   tggttttgta tttttggtat gatgtattgg taatttggtt tgttttttgg agagtatttt 1620
   agttattttg ttagttttaa tattaatttt aatttggata agtttgtttt ttttgtagtg 1680
   attatttgta tttttaattt ttataggttg gttagttttt ttgttttttt tttggtgttt 1740
   gtgtttggaa gggtggttta gggtgttgag gtgtttattg ggtaggtaga gttttagatt 1800
   tgagggtggg aattgatatt tttgttttag gtagagttta ttgttggtaa aatgaggagt 1860
   ttttagttta ttggattggt gtttttgttt agtggagtta ttttagtatt ttaggtttat 1920
   ttagagtgtg gttgttggag tagtattgtt tttttttgtt tttttttttt tttttttttt 1980
   attttttttt tttttttgta agtgggaggg agtgtttgta aaagggtagg ggtgtgttta 2040
   gttgtatttt ttttttggag taagatgtta gtaaagtata agattttgtt tattttagta 2100
   ggagagtttg atggaggggt gaggttaggt gaggggaggt gagattgtgt aggtgttgtg 2160
   gaggtggtgt tgttagtttt tgtttagaag gggaatttat tatggagaaa gtaaagaggg 2220
   ggattgtgga ggttagggtt gtaagtgagt ttaaaggttg ggggtagggg ttttatagaa 2280
   taaggagaat gggttaggtt agagagtagt tttttgtgaa gttttttttt taaaagattt 2340
   aggttaaaat gttgtattgt tgatttgttt aggtgtttga tttttatgtg tttgggtgtg 2400
   ttttgaattt ttgggtttta ttggtgtttt ttgttaatgt tgggtttgga aattggagta 2460
   ggtggtaagt gtggagaaga gaggagggta gggttgagtg tttttgtata ttttttattt 2520
   gtatttattt agttatgtta tattttggga atatttgttt taagttagat atagggttag 2580
   gtatttggta aaatggattt atgaataatt tataagtttt ttattgaagg ttaggtagtt 2640
   tgttgtgttg tggagataga taaaataaga tagtagagat aggtatataa gttagtaatt 2700
   gtggtatatt gggataagta tgataaaaat ggtgagttag tgtattttta aggagtgtaa 2760
   aatttttatg ggtgtgggtg tgagtgtgtg tgtgtatata ttggtatgtg tgtgattttg 2820
   gatgttgggg attggttatg ttgtgggtgg aggttttttt gtatatttta ttatgtttaa 2880
   aggtgtgtta tggattaaga aagggtggga tgttttgttt tggagagaga gagaaaaaaa 2940
   aaagttaggt tattttgttg attaatgttt gtataatgtt ttaggtaggg attatttttt 3000
   t 3001
<210> 276
   <211> 50
   <212> DNA
   <213> human
<400> 276
   tcttggtgcc ggtggctggc ggtgctcagg aagcagtacc tgttctcacg 50
<210> 277
   <211> 50
   <212> DNA
   <213> human
<400> 277
   ttttggtgtc ggtggttggc ggtgtttagg aagtagtatt tgtttttacg 50
<210> 278
   <211> 50
   <212> DNA
   <213> human
<400> 278
   ttttggtgtt ggtggttggt ggtgtttagg aagtagtatt tgtttttatg 50
<210> 279
   <211> 50
   <212> DNA
   <213> human
<400> 279
   cgtgagaata ggtattgttt tttgagtatc gttagttatc ggtattaaga 50
<210> 280
   <211> 50
   <212> DNA
   <213> human
<400> 280
   tgtgagaata ggtattgttt tttgagtatt gttagttatt ggtattaaga 50
<210> 281
   <211> 3001
   <212> DNA
   <213> human
<400> 281
   tcggctggag cattgtgaag ggcatgctgt aattacaata ttgtgatgtg gcgttgttgc 60
   ataactcaag gacacaccat tgtcgcctac ccactgtgat gtcgcaagaa cgagatttgt 120
   gagttttact ccaattgagc ttttccagtg attgactgcc actccctccc agcctttcgg 180
   ttctttaaaa tgaaagataa acctcaggac aaccaagacc tggggaaaag acagtctttg 240
   ggaatgtaat ttggaagaat ggatccagaa aaacaaccag gggctcttaa ctctagtttc 300
   ggcaggagat cccgagttac agacctgagc tcgtaaacaa acgctcacca ctttacagtc 360
   gccgcctcat cctgaaactc cggtcagaaa tgctggaagg cctttgctaa tgcgcagtga 420
   aaattagggg cacagatgtg gacggactgt gcttggtcag taattgttct cctccaaatc 480
   aagtttatga gaaatttcat ataaggaaaa aaaccctttt gtatctgtgt ctatatgtac 540
   agaagacctt aacaaccaaa cagaaactta acccctttgg tttggttttg ttttcgaaag 600
   cttatgccca attttaaact tcatgtaagc ttatgtactt attttaaact ttaaaactat 660
   taaaagttaa actagatagt aaattcggca ctgtctaatg tgcatacacc acaaacatat 720
   aaccctaaat tcagctgtgt ctaatgtgta tacaccacac atatatataa ccctaaattc 780
   agcctgatgt acttatacca cacatatata accctaaatt cagcctaatg tacatatacc 840
   acacacacat atatataacc ctaaattcag cctaatgtac atacaccaca catatatata 900
   accctaaatt cagccctaat gtacatacac cacacatata taaccctaaa ttcagcctaa 960
   tgtacataca ccacacatat atataaccct aaattcagcc ctaatgtaca tacaccacac     1020
   atataaccct aaattcaccc taatgtacat acaccacaca tatatatatg acccttacct     1080
   cttgttagca tttattgtag tttgtttttg aggttaggag cagttaagca ataatatgaa     1140
   tttgtgttgg tttaactttt atgaatttta gggcagaaca aggagttaat ttccagataa     1200
   gcttgttcat gttcttagaa taaagtgtca gcctcattag aagttttgta atcgtggtct     1260
   gcatcccagc catgcacctg ctgggtcctg agtaatcctc ggagcccaga gcaaggcaga     1320
   gcagacacgg agcaaacgct gcgttcagct gggtatttta gatacgtctg agaagagttt     1380
   taaaataaaa cttgaatttg aaaagaaata gtgagtactt accagaggtg ggcagtgctg     1440
   ggatgtggcc ggtcttggtg ccggtggctg gcggtgctca ggaagcagta cctgttctca     1500
   cgcttgctgg gtgaaactgg agcctgctgt tccagtggga gggatttaag tgtaggagtt     1560
   gagccctcgg ggcgtagaga tgcacctgat attcctcctg cccgcagcta cgattcatgt     1620
   gtttgcaccc accgagctgc tgcgggcagc ctccccagca ctggacccag caagcaagtt     1680
   atctcgccga ctcccagcgc tgtctccaga ctcaactgtc tgtgagtgcg gcgtcggtgc     1740
   tgcctggacg gccatatgtc agagggcttt cgatgtgtcc gcagttagaa tagagattac     1800
   tatattcatc agaatcacac ccgatgaata tttggcgtca tgataaagac atgtgagact     1860
   ttcctttaga acttgagcat gtgtacgcag gaggcaaaca cttggattga ttctcaagcg     1920
   cgcataccct gggaggcaca gtattcatgg cgtccttgcc atgaggtcca gtgaggcctt     1980
   tgaggatctg tggaccttcc accctctacc ctgctcctga gagtctctgt ccctggtcct     2040
   cctcaggagc tgagccggca gggagggagc ttatcccaga gcctggctgc agggacctgc     2100
   cggagcttgc ccgacaccag gcaccgcaag agctgcggag cagggtgtgt gggtgcgggg     2160
   gagggggcca gctgtgcact cccgcaaacc accctccccc cactttaaat tgcagaggca     2220
   cttaacttgg gggccagttt ttacctccac acccccccac caaaactgct tgcttgctta     2280
   atcggaggcc gcaccccagt tgtagcgaag gctggcatgt ggtgtcggtt tgaggactgg     2340
   gaaaggaggt gggatgagct ggtgtcaggc ccttggatga gcctgaggct gcagggcaga     2400
   ggctgcaggg ctgtggcata gggacgagct ggccaggaca gacaaactgg tttttttttt     2460
   tttttttttt tttttttttg agacagagtt tcactgttgc tgcccaggct ggagtgcaat     2520
   ggcatgatct tggctcactg cgtttctcct gcctcagcct cccgagtagc tgggactaca     2580
   ggcgcctgcc accacgcccg gctaattttt tgtattttta gtagagatgg ggtttcaccg     2640
   tgttagccag gatggtcttg atctcctgac cttgtgatcc acccacgtcg gcctcccaaa     2700
   gtgcttggat tacaggtgtg agccactgca cccggtctgt ttttttgttt ttgttatttt     2760
   tttccttaag acttaagatt ttacatggct ttgcgtttac ttttatattt aattcctttt     2820
   ttttttcttt tgagacaggg tcttgccctg tcacccaagc tggagtgcag tggtgcgatc     2880
   acggttcact gtagcctcaa cctcctggac tcgagtgatc ctcccgcctc agtctcccca     2940
   gtagctggga ctacaggcac ggtcactatg cctggagaat tttttgtaga gacagggttt     3000
   t                                                                     3001
<210> 282
   <211> 3001
   <212> DNA
   <213> human
<400> 282
   tcggttggag tattgtgaag ggtatgttgt aattataata ttgtgatgtg gcgttgttgt     60
   ataatttaag gatatattat tgtcgtttat ttattgtgat gtcgtaagaa cgagatttgt    120
   gagttttatt ttaattgagt ttttttagtg attgattgtt attttttttt agtttttcgg    180
   ttttttaaaa tgaaagataa attttaggat aattaagatt tggggaaaag atagtttttg    240
   ggaatgtaat ttggaagaat ggatttagaa aaataattag gggtttttaa ttttagtttc    300
   ggtaggagat ttcgagttat agatttgagt tcgtaaataa acgtttatta ttttatagtc    360
   gtcgttttat tttgaaattt cggttagaaa tgttggaagg tttttgttaa tgcgtagtga    420
   aaattagggg tatagatgtg gacggattgt gtttggttag taattgtttt tttttaaatt    480
   aagtttatga gaaattttat ataaggaaaa aaattttttt gtatttgtgt ttatatgtat    540
   agaagatttt aataattaaa tagaaattta atttttttgg tttggttttg ttttcgaaag    600
   tttatgttta attttaaatt ttatgtaagt ttatgtattt attttaaatt ttaaaattat    660
   taaaagttaa attagatagt aaattcggta ttgtttaatg tgtatatatt ataaatatat    720
   aattttaaat ttagttgtgt ttaatgtgta tatattatat atatatataa ttttaaattt    780
   agtttgatgt atttatatta tatatatata attttaaatt tagtttaatg tatatatatt    840
   atatatatat atatataatt ttaaatttag tttaatgtat atatattata tatatatata    900
   attttaaatt tagttttaat gtatatatat tatatatata taattttaaa tttagtttaa    960
   tgtatatata ttatatatat atataatttt aaatttagtt ttaatgtata tatattatat   1020
   atataatttt aaatttattt taatgtatat atattatata tatatatatg atttttattt   1080
   tttgttagta tttattgtag tttgtttttg aggttaggag tagttaagta ataatatgaa   1140
   tttgtgttgg tttaattttt atgaatttta gggtagaata aggagttaat ttttagataa   1200
   gtttgtttat gtttttagaa taaagtgtta gttttattag aagttttgta atcgtggttt   1260
   gtattttagt tatgtatttg ttgggttttg agtaattttc ggagtttaga gtaaggtaga   1320
   gtagatacgg agtaaacgtt gcgtttagtt gggtatttta gatacgtttg agaagagttt   1380
   taaaataaaa tttgaatttg aaaagaaata gtgagtattt attagaggtg ggtagtgttg   1440
   ggatgtggtc ggttttggtg tcggtggttg gcggtgttta ggaagtagta tttgttttta   1500
   cgtttgttgg gtgaaattgg agtttgttgt tttagtggga gggatttaag tgtaggagtt   1560
   gagttttcgg ggcgtagaga tgtatttgat attttttttg ttcgtagtta cgatttatgt   1620
   gtttgtattt atcgagttgt tgcgggtagt ttttttagta ttggatttag taagtaagtt   1680
   atttcgtcga tttttagcgt tgtttttaga tttaattgtt tgtgagtgcg gcgtcggtgt   1740
   tgtttggacg gttatatgtt agagggtttt cgatgtgttc gtagttagaa tagagattat   1800
   tatatttatt agaattatat tcgatgaata tttggcgtta tgataaagat atgtgagatt   1860
   tttttttaga atttgagtat gtgtacgtag gaggtaaata tttggattga tttttaagcg   1920
   cgtatatttt gggaggtata gtatttatgg cgtttttgtt atgaggttta gtgaggtttt   1980
   tgaggatttg tggatttttt atttttattt ttgtttttga gagtttttgt ttttggtttt   2040
   ttttaggagt tgagtcggta gggagggagt ttattttaga gtttggttgt agggatttgt   2100
   cggagtttgt tcgatattag gtatcgtaag agttgcggag tagggtgtgt gggtgcgggg   2160
   gagggggtta gttgtgtatt ttcgtaaatt attttttttt tattttaaat tgtagaggta   2220
   tttaatttgg gggttagttt ttatttttat atttttttat taaaattgtt tgtttgttta   2280
   atcggaggtc gtattttagt tgtagcgaag gttggtatgt ggtgtcggtt tgaggattgg   2340
   gaaaggaggt gggatgagtt ggtgttaggt ttttggatga gtttgaggtt gtagggtaga   2400
   ggttgtaggg ttgtggtata gggacgagtt ggttaggata gataaattgg tttttttttt   2460
   tttttttttt tttttttttg agatagagtt ttattgttgt tgtttaggtt ggagtgtaat   2520
   ggtatgattt tggtttattg cgtttttttt gttttagttt ttcgagtagt tgggattata   2580
   ggcgtttgtt attacgttcg gttaattttt tgtattttta gtagagatgg ggttttatcg   2640
   tgttagttag gatggttttg attttttgat tttgtgattt atttacgtcg gttttttaaa   2700
   gtgtttggat tataggtgtg agttattgta ttcggtttgt ttttttgttt ttgttatttt   2760
   tttttttaag atttaagatt ttatatggtt ttgcgtttat ttttatattt aatttttttt   2820
   tttttttttt tgagataggg ttttgttttg ttatttaagt tggagtgtag tggtgcgatt   2880
   acggtttatt gtagttttaa ttttttggat tcgagtgatt ttttcgtttt agttttttta   2940
   gtagttggga ttataggtac ggttattatg tttggagaat tttttgtaga gatagggttt   3000
   t                                                                   3001
<210> 283
   <211> 3001
   <212> DNA
   <213> human
<400> 283
   ttggttggag tattgtgaag ggtatgttgt aattataata ttgtgatgtg gtgttgttgt 60
   ataatttaag gatatattat tgttgtttat ttattgtgat gttgtaagaa tgagatttgt 120
   gagttttatt ttaattgagt ttttttagtg attgattgtt attttttttt agttttttgg 180
   ttttttaaaa tgaaagataa attttaggat aattaagatt tggggaaaag atagtttttg 240
   ggaatgtaat ttggaagaat ggatttagaa aaataattag gggtttttaa ttttagtttt 300
   ggtaggagat tttgagttat agatttgagt ttgtaaataa atgtttatta ttttatagtt 360
   gttgttttat tttgaaattt tggttagaaa tgttggaagg tttttgttaa tgtgtagtga 420
   aaattagggg tatagatgtg gatggattgt gtttggttag taattgtttt tttttaaatt 480
   aagtttatga gaaattttat ataaggaaaa aaattttttt gtatttgtgt ttatatgtat 540
   agaagatttt aataattaaa tagaaattta atttttttgg tttggttttg tttttgaaag 600
   tttatgttta attttaaatt ttatgtaagt ttatgtattt attttaaatt ttaaaattat 660
   taaaagttaa attagatagt aaatttggta ttgtttaatg tgtatatatt ataaatatat 720
   aattttaaat ttagttgtgt ttaatgtgta tatattatat atatatataa ttttaaattt 780
   agtttgatgt atttatatta tatatatata attttaaatt tagtttaatg tatatatatt 840
   atatatatat atatataatt ttaaatttag tttaatgtat atatattata tatatatata 900
   attttaaatt tagttttaat gtatatatat tatatatata taattttaaa tttagtttaa 960
   tgtatatata ttatatatat atataatttt aaatttagtt ttaatgtata tatattatat 1020
   atataatttt aaatttattt taatgtatat atattatata tatatatatg atttttattt 1080
   tttgttagta tttattgtag tttgtttttg aggttaggag tagttaagta ataatatgaa 1140
   tttgtgttgg tttaattttt atgaatttta gggtagaata aggagttaat ttttagataa 1200
   gtttgtttat gtttttagaa taaagtgtta gttttattag aagttttgta attgtggttt 1260
   gtattttagt tatgtatttg ttgggttttg agtaattttt ggagtttaga gtaaggtaga 1320
   gtagatatgg agtaaatgtt gtgtttagtt gggtatttta gatatgtttg agaagagttt 1380
   taaaataaaa tttgaatttg aaaagaaata gtgagtattt attagaggtg ggtagtgttg 1440
   ggatgtggtt ggttttggtg ttggtggttg gtggtgttta ggaagtagta tttgttttta 1500
   tgtttgttgg gtgaaattgg agtttgttgt tttagtggga gggatttaag tgtaggagtt 1560
   gagtttttgg ggtgtagaga tgtatttgat attttttttg tttgtagtta tgatttatgt 1620
   gtttgtattt attgagttgt tgtgggtagt ttttttagta ttggatttag taagtaagtt 1680
   attttgttga tttttagtgt tgtttttaga tttaattgtt tgtgagtgtg gtgttggtgt 1740
   tgtttggatg gttatatgtt agagggtttt tgatgtgttt gtagttagaa tagagattat 1800
   tatatttatt agaattatat ttgatgaata tttggtgtta tgataaagat atgtgagatt 1860
   tttttttaga atttgagtat gtgtatgtag gaggtaaata tttggattga tttttaagtg 1920
   tgtatatttt gggaggtata gtatttatgg tgtttttgtt atgaggttta gtgaggtttt 1980
   tgaggatttg tggatttttt atttttattt ttgtttttga gagtttttgt ttttggtttt 2040
   ttttaggagt tgagttggta gggagggagt ttattttaga gtttggttgt agggatttgt 2100
   tggagtttgt ttgatattag gtattgtaag agttgtggag tagggtgtgt gggtgtgggg 2160
   gagggggtta gttgtgtatt tttgtaaatt attttttttt tattttaaat tgtagaggta 2220
   tttaatttgg gggttagttt ttatttttat atttttttat taaaattgtt tgtttgttta 2280
   attggaggtt gtattttagt tgtagtgaag gttggtatgt ggtgttggtt tgaggattgg 2340
   gaaaggaggt gggatgagtt ggtgttaggt ttttggatga gtttgaggtt gtagggtaga 2400
   ggttgtaggg ttgtggtata gggatgagtt ggttaggata gataaattgg tttttttttt 2460
   tttttttttt tttttttttg agatagagtt ttattgttgt tgtttaggtt ggagtgtaat 2520
   ggtatgattt tggtttattg tgtttttttt gttttagttt tttgagtagt tgggattata 2580
   ggtgtttgtt attatgtttg gttaattttt tgtattttta gtagagatgg ggttttattg 2640
   tgttagttag gatggttttg attttttgat tttgtgattt atttatgttg gttttttaaa 2700
   gtgtttggat tataggtgtg agttattgta tttggtttgt ttttttgttt ttgttatttt 2760
   tttttttaag atttaagatt ttatatggtt ttgtgtttat ttttatattt aatttttttt 2820
   tttttttttt tgagataggg ttttgttttg ttatttaagt tggagtgtag tggtgtgatt 2880
   atggtttatt gtagttttaa ttttttggat ttgagtgatt tttttgtttt agttttttta 2940
   gtagttggga ttataggtat ggttattatg tttggagaat tttttgtaga gatagggttt 3000
   t 3001
<210> 284
   <211> 3001
   <212> DNA
   <213> human
<400> 284
   aaaattttgt ttttataaaa aattttttag gtatagtgat cgtgtttgta gttttagtta 60
   ttggggagat tgaggcggga ggattattcg agtttaggag gttgaggtta tagtgaatcg 120
   tgatcgtatt attgtatttt agtttgggtg atagggtaag attttgtttt aaaagaaaaa 180
   aaaaaggaat taaatataaa agtaaacgta aagttatgta aaattttaag ttttaaggaa 240
   aaaaataata aaaataaaaa aatagatcgg gtgtagtggt ttatatttgt aatttaagta 300
   ttttgggagg tcgacgtggg tggattataa ggttaggaga ttaagattat tttggttaat 360
   acggtgaaat tttattttta ttaaaaatat aaaaaattag tcgggcgtgg tggtaggcgt 420
   ttgtagtttt agttattcgg gaggttgagg taggagaaac gtagtgagtt aagattatgt 480
   tattgtattt tagtttgggt agtaatagtg aaattttgtt ttaaaaaaaa aaaaaaaaaa 540
   aaaaaaaaaa attagtttgt ttgttttggt tagttcgttt ttatgttata gttttgtagt 600
   ttttgttttg tagttttagg tttatttaag ggtttgatat tagtttattt tatttttttt 660
   tttagttttt aaatcgatat tatatgttag ttttcgttat aattggggtg cggttttcga 720
   ttaagtaagt aagtagtttt ggtggggggg tgtggaggta aaaattggtt tttaagttaa 780
   gtgtttttgt aatttaaagt ggggggaggg tggtttgcgg gagtgtatag ttggtttttt 840
   ttttcgtatt tatatatttt gtttcgtagt ttttgcggtg tttggtgtcg ggtaagtttc 900
   ggtaggtttt tgtagttagg ttttgggata agtttttttt ttgtcggttt agtttttgag 960
   gaggattagg gatagagatt tttaggagta gggtagaggg tggaaggttt atagattttt 1020
   aaaggtttta ttggatttta tggtaaggac gttatgaata ttgtgttttt tagggtatgc 1080
   gcgtttgaga attaatttaa gtgtttgttt tttgcgtata tatgtttaag ttttaaagga 1140
   aagttttata tgtttttatt atgacgttaa atatttatcg ggtgtgattt tgatgaatat 1200
   agtaattttt attttaattg cggatatatc gaaagttttt tgatatatgg tcgtttaggt 1260
   agtatcgacg tcgtatttat agatagttga gtttggagat agcgttggga gtcggcgaga 1320
   taatttgttt gttgggttta gtgttgggga ggttgttcgt agtagttcgg tgggtgtaaa 1380
   tatatgaatc gtagttgcgg gtaggaggaa tattaggtgt atttttacgt ttcgagggtt 1440
   taatttttat atttaaattt tttttattgg aatagtaggt tttagtttta tttagtaagc 1500
   gtgagaatag gtattgtttt ttgagtatcg ttagttatcg gtattaagat cggttatatt 1560
   ttagtattgt ttatttttgg taagtattta ttattttttt ttaaatttaa gttttatttt 1620
   aaaatttttt ttagacgtat ttaaaatatt tagttgaacg tagcgtttgt ttcgtgtttg 1680
   ttttgttttg ttttgggttt cgaggattat ttaggattta gtaggtgtat ggttgggatg 1740
   tagattacga ttataaaatt tttaatgagg ttgatatttt attttaagaa tatgaataag 1800
   tttatttgga aattaatttt ttgttttgtt ttaaaattta taaaagttaa attaatataa 1860
   atttatatta ttgtttaatt gtttttaatt ttaaaaataa attataataa atgttaataa 1920
   gaggtaaggg ttatatatat atgtgtggtg tatgtatatt agggtgaatt tagggttata 1980
   tgtgtggtgt atgtatatta gggttgaatt tagggttata tatatgtgtg gtgtatgtat 2040
   attaggttga atttagggtt atatatgtgt ggtgtatgta tattagggtt gaatttaggg 2100
   ttatatatat gtgtggtgta tgtatattag gttgaattta gggttatata tatgtgtgtg 2160
   tggtatatgt atattaggtt gaatttaggg ttatatatgt gtggtataag tatattaggt 2220
   tgaatttagg gttatatata tgtgtggtgt atatatatta gatatagttg aatttagggt 2280
   tatatgtttg tggtgtatgt atattagata gtgtcgaatt tattatttag tttaattttt 2340
   aatagtttta aagtttaaaa taagtatata agtttatatg aagtttaaaa ttgggtataa 2400
   gttttcgaaa ataaaattaa attaaagggg ttaagttttt gtttggttgt taaggttttt 2460
   tgtatatata gatatagata taaaagggtt ttttttttta tatgaaattt tttataaatt 2520
   tgatttggag gagaataatt attgattaag tatagttcgt ttatatttgt gtttttaatt 2580
   tttattgcgt attagtaaag gttttttagt atttttgatc ggagttttag gatgaggcgg 2640
   cgattgtaaa gtggtgagcg tttgtttacg agtttaggtt tgtaattcgg gattttttgt 2700
   cgaaattaga gttaagagtt tttggttgtt tttttggatt tattttttta aattatattt 2760
   ttaaagattg tttttttttt aggttttggt tgttttgagg tttatttttt attttaaaga 2820
   atcgaaaggt tgggagggag tggtagttaa ttattggaaa agtttaattg gagtaaaatt 2880
   tataaatttc gtttttgcga tattatagtg ggtaggcgat aatggtgtgt ttttgagtta 2940
   tgtaataacg ttatattata atattgtaat tatagtatgt tttttataat gttttagtcg 3000
   a 3001
<210> 285
   <211> 3001
   <212> DNA
   <213> human
<400> 285
   aaaattttgt ttttataaaa aattttttag gtatagtgat tgtgtttgta gttttagtta 60
   ttggggagat tgaggtggga ggattatttg agtttaggag gttgaggtta tagtgaattg 120
   tgattgtatt attgtatttt agtttgggtg atagggtaag attttgtttt aaaagaaaaa 180
   aaaaaggaat taaatataaa agtaaatgta aagttatgta aaattttaag ttttaaggaa 240
   aaaaataata aaaataaaaa aatagattgg gtgtagtggt ttatatttgt aatttaagta 300
   ttttgggagg ttgatgtggg tggattataa ggttaggaga ttaagattat tttggttaat 360
   atggtgaaat tttattttta ttaaaaatat aaaaaattag ttgggtgtgg tggtaggtgt 420
   ttgtagtttt agttatttgg gaggttgagg taggagaaat gtagtgagtt aagattatgt 480
   tattgtattt tagtttgggt agtaatagtg aaattttgtt ttaaaaaaaa aaaaaaaaaa 540
   aaaaaaaaaa attagtttgt ttgttttggt tagtttgttt ttatgttata gttttgtagt 600
   ttttgttttg tagttttagg tttatttaag ggtttgatat tagtttattt tatttttttt 660
   tttagttttt aaattgatat tatatgttag tttttgttat aattggggtg tggtttttga 720
   ttaagtaagt aagtagtttt ggtggggggg tgtggaggta aaaattggtt tttaagttaa 780
   gtgtttttgt aatttaaagt ggggggaggg tggtttgtgg gagtgtatag ttggtttttt 840
   tttttgtatt tatatatttt gttttgtagt ttttgtggtg tttggtgttg ggtaagtttt 900
   ggtaggtttt tgtagttagg ttttgggata agtttttttt ttgttggttt agtttttgag 960
   gaggattagg gatagagatt tttaggagta gggtagaggg tggaaggttt atagattttt 1020
   aaaggtttta ttggatttta tggtaaggat gttatgaata ttgtgttttt tagggtatgt 1080
   gtgtttgaga attaatttaa gtgtttgttt tttgtgtata tatgtttaag ttttaaagga 1140
   aagttttata tgtttttatt atgatgttaa atatttattg ggtgtgattt tgatgaatat 1200
   agtaattttt attttaattg tggatatatt gaaagttttt tgatatatgg ttgtttaggt 1260
   agtattgatg ttgtatttat agatagttga gtttggagat agtgttggga gttggtgaga 1320
   taatttgttt gttgggttta gtgttgggga ggttgtttgt agtagtttgg tgggtgtaaa 1380
   tatatgaatt gtagttgtgg gtaggaggaa tattaggtgt atttttatgt tttgagggtt 1440
   taatttttat atttaaattt tttttattgg aatagtaggt tttagtttta tttagtaagt 1500
   gtgagaatag gtattgtttt ttgagtattg ttagttattg gtattaagat tggttatatt 1560
   ttagtattgt ttatttttgg taagtattta ttattttttt ttaaatttaa gttttatttt 1620
   aaaatttttt ttagatgtat ttaaaatatt tagttgaatg tagtgtttgt tttgtgtttg 1680
   ttttgttttg ttttgggttt tgaggattat ttaggattta gtaggtgtat ggttgggatg 1740
   tagattatga ttataaaatt tttaatgagg ttgatatttt attttaagaa tatgaataag 1800
   tttatttgga aattaatttt ttgttttgtt ttaaaattta taaaagttaa attaatataa 1860
   atttatatta ttgtttaatt gtttttaatt ttaaaaataa attataataa atgttaataa 1920
   gaggtaaggg ttatatatat atgtgtggtg tatgtatatt agggtgaatt tagggttata 1980
   tgtgtggtgt atgtatatta gggttgaatt tagggttata tatatgtgtg gtgtatgtat 2040
   attaggttga atttagggtt atatatgtgt ggtgtatgta tattagggtt gaatttaggg 2100
   ttatatatat gtgtggtgta tgtatattag gttgaattta gggttatata tatgtgtgtg 2160
   tggtatatgt atattaggtt gaatttaggg ttatatatgt gtggtataag tatattaggt 2220
   tgaatttagg gttatatata tgtgtggtgt atatatatta gatatagttg aatttagggt 2280
   tatatgtttg tggtgtatgt atattagata gtgttgaatt tattatttag tttaattttt 2340
   aatagtttta aagtttaaaa taagtatata agtttatatg aagtttaaaa ttgggtataa 2400
   gtttttgaaa ataaaattaa attaaagggg ttaagttttt gtttggttgt taaggttttt 2460
   tgtatatata gatatagata taaaagggtt ttttttttta tatgaaattt tttataaatt 2520
   tgatttggag gagaataatt attgattaag tatagtttgt ttatatttgt gtttttaatt 2580
   tttattgtgt attagtaaag gttttttagt atttttgatt ggagttttag gatgaggtgg 2640
   tgattgtaaa gtggtgagtg tttgtttatg agtttaggtt tgtaatttgg gattttttgt 2700
   tgaaattaga gttaagagtt tttggttgtt tttttggatt tattttttta aattatattt 2760
   ttaaagattg tttttttttt aggttttggt tgttttgagg tttatttttt attttaaaga 2820
   attgaaaggt tgggagggag tggtagttaa ttattggaaa agtttaattg gagtaaaatt 2880
   tataaatttt gtttttgtga tattatagtg ggtaggtgat aatggtgtgt ttttgagtta 2940
   tgtaataatg ttatattata atattgtaat tatagtatgt tttttataat gttttagttg 3000
   a 3001
<210> 286
   <211> 50
   <212> DNA
   <213> human
<400> 286
   cgcctaatgt gtgccctact tatgtgggaa gtgttgatga agtaaatcat 50
<210> 287
   <211> 50
   <212> DNA
   <213> human
<400> 287
   cgtttaatgt gtgttttatt tatgtgggaa gtgttgatga agtaaattat 50
<210> 288
   <211> 50
   <212> DNA
   <213> human
<400> 288
   tgtttaatgt gtgttttatt tatgtgggaa gtgttgatga agtaaattat 50
<210> 289
   <211> 50
   <212> DNA
   <213> human
<400> 289
   atgatttatt ttattaatat tttttatata agtagggtat atattaggcg 50
<210> 290
   <211> 50
   <212> DNA
   <213> human
<400> 290
   atgatttatt ttattaatat tttttatata agtagggtat atattaggtg 50
<210> 291
   <211> 3001
   <212> DNA
   <213> human
<400> 291
   gagaaaacac aagagccaaa ctcaccccag ccccacagac catgtcttcc ctactccatg     60
   tctggatctc ctgttcctct ctgatcgtgc attctctgga ttatctcccc agtgccccca    120
   actcataaat gtcagtgcct tcgagaaata agtcctgcac tctctgttta actcaatacc    180
   ggcttctaca atggcccaca aatcactatc ttcagcccag accttcccca gctccaggtt    240
   cccaaacagc ttcctagatg cctgattctc ttctggaaat ccctccctca gacccctcag    300
   acactcctct cctcattaag ctgatggtgg cctccatcaa cctgaatctg ccgctccatc    360
   ccgttcccca tatcggggac gcctcagccc ttcagttgag acactagcag gtcctgtcat    420
   tttctcattc tttccttctc ccagaacgca cagaccttag tgtcttcaag tcctagagac    480
   acttcttctc agtaatacat cctctcttcg caattcccac agctatcagg cccaatttcc    540
   tcaccccctc ctataaactc ccgccttctg gcctccacga atttactcag gctgtgtttc    600
   aggcttaaca ggtcactgtc accaagacaa acaactgaga ttcctccctc ccgcttacgc    660
   ccaccgcaga gatttccaag gggtgaaaga cccggcgcgt cacctctgca gctccctccc    720
   ttccacggtt ttcaagaaat ccttctcctc cggagatggc cccgcgccgt ctcccgagtc    780
   ggccagccgc ctccgtgcgg acgtgcgagt gaagccgaat gaaattgggg cagtggaagc    840
   agccgtcagc ggcaaaacac cctctttgga gtcagccatc ttgctccttt tcccaggccg    900
   gcgcgctgct tgctgggaaa tttagtttgg gttcagatcg ctaggggccg gccaaagtgc    960
   gtaaaaacta ccttcccatc aaccactgtg gcaccggcgg gagggggcgg taaggggccg   1020
   tgggaagtaa ttgtcgctct ttcaaacgtg gtgggagcgc gtcgcgccaa gcgcccaggg   1080
   aaatgtagtt ttttttttgt ttgttttgtt ttgtttttta agatggagtc tcgctctgtt   1140
   tcacaggctg gagtgcagtg gcgtgatctc ggctcactgc aacctccgcc tcccaggttc   1200
   aagcgattct cctgcctcag cctcccgagt agctgagact aaaggcatgc gccaccacgc   1260
   ctggctaatt tttgtatttt tagtagagac ggggtttcac cgtgttagcc aggatggtct   1320
   cgatttcctg acctcgtgat tctcctgcct cggcctccca cagtgctggg attacaggtg   1380
   tgagccacca cgcctggctg gaaatgtagt ttttagcggg gtaggcatgg tggaaggtgg   1440
   attcattctt gccagacatt aatcttcctt cgtgtaggcg tttgacaagt atttatggag   1500
   cgcctaatgt gtgccctact tatgtgggaa gtgttgatga agtaaatcat ttagaaataa   1560
   tcagctgggt gtggtggcgc gcacctgtag ttccatttac tccgcaggct gaggcaggag   1620
   gatggcctga ggcctgggaa ttcgaggctg cacttagctc ttatcgcacc actgcattcc   1680
   agcctgggtg acagagcgag accccatctc taaaaaagaa aaaggtcggg ggtggggggg   1740
   atggagcgtc agttgtgtac aataataaat aatagacata aaataaataa tagacataaa   1800
   aaaccccgtc tctactgaaa atacaaaaat tagaggggcg tggtggctca tgcctgtaat   1860
   cccagcattt tgggaggccg aggctggtgg atcacctgag gtcaggactt tgagaccagc   1920
   ctgactcata tggtgaaacc ctgtctctac taaaaataca aaaattagcc gggcgtggtg   1980
   gtggcgcctg tatccccagc tacttgggag gctgagacag gaggattgct tgaatccggg   2040
   agacagagtt tgcagtgagc cgagatcctg ccactgcact ccagcctggg cgacagagcg   2100
   agactccgtc ttaaaaaaga aaaaagaaag aaagaaagaa atagtgccta ttggccaggc   2160
   tcatacccgt aatcccagca ctttaggagg ccgaggcggg cggatcactt gaggccagta   2220
   gttcgagacc agcctggcca acatggtgaa accccgtctc tactgaaaat acaaaaatta   2280
   gcggggtgtg gtggcgcatg cctgtaatcc cagctactcg ggaggttgag gcaggagaat   2340
   cgcttgaacc cgggaggcgg aggttacagt gagccgagat ctcaacactc cggcctgggc   2400
   tatagagtga cactcagtct caaaaacaaa agaaaaacaa aaaaaaaaaa aaaaaaagaa   2460
   aaagaatagt gcctactgat gtcagatttt aatactcaac aaggcactag attcaaatat   2520
   caataaaata ggctgggcac agtggctcat gcctgtaatc ccagcacttt gggagaccta   2580
   ggcgggcgga ccacctgagg tcaagagttt gagaccagcc tggccaacat ggcaaaaccc   2640
   aatctccact aaaaatacaa aaattagccg ggtgtggtgg cacatgcttg taatcccagc   2700
   tactcgggag gtggaggcag gagaatcgct tgaacctagt aggtggaagt tgcagtgagc   2760
   caagattgcc ccactgcact ccagcctggg caacagagcg agactctgtc tcaaaaaaaa   2820
   aaaaaagaaa aagaaaagaa aaagaaaaaa aagaaacttg tagtctagta gggaatacag   2880
   acatttaaat acatatttta aaaatatagc aacatgtgcc gagcttagtg gctcacgcct   2940
   ataatcccag cactttggga ggcctaggca ggaggattgt ttgagctcag gactttgaga   3000
   c                                                                    3001

<210> 292
   <211> 3001
   <212> DNA
   <213> human
<400> 292
   gagaaaatat aagagttaaa tttattttag ttttatagat tatgtttttt ttattttatg     60
   tttggatttt ttgttttttt ttgatcgtgt attttttgga ttattttttt agtgttttta    120
   atttataaat gttagtgttt tcgagaaata agttttgtat tttttgttta atttaatatc    180
   ggtttttata atggtttata aattattatt tttagtttag atttttttta gttttaggtt   240
   tttaaatagt tttttagatg tttgattttt ttttggaaat ttttttttta gattttttag    300
   atattttttt ttttattaag ttgatggtgg tttttattaa tttgaatttg tcgttttatt    360
   tcgtttttta tatcggggac gttttagttt tttagttgag atattagtag gttttgttat    420
   tttttatttt tttttttttt ttagaacgta tagattttag tgtttttaag ttttagagat    480
   attttttttt agtaatatat ttttttttcg taatttttat agttattagg tttaattttt    540
   ttattttttt ttataaattt tcgttttttg gtttttacga atttatttag gttgtgtttt   600
   aggtttaata ggttattgtt attaagataa ataattgaga tttttttttt tcgtttacgt    660
   ttatcgtaga gatttttaag gggtgaaaga ttcggcgcgt tatttttgta gttttttttt    720
   ttttacggtt tttaagaaat tttttttttt cggagatggt ttcgcgtcgt ttttcgagtc    780
   ggttagtcgt tttcgtgcgg acgtgcgagt gaagtcgaat gaaattgggg tagtggaagt    840
   agtcgttagc ggtaaaatat tttttttgga gttagttatt ttgttttttt ttttaggtcg    900
   gcgcgttgtt tgttgggaaa tttagtttgg gtttagatcg ttaggggtcg gttaaagtgc    960
   gtaaaaatta tttttttatt aattattgtg gtatcggcgg gagggggcgg taaggggtcg   1020
   tgggaagtaa ttgtcgtttt tttaaacgtg gtgggagcgc gtcgcgttaa gcgtttaggg   1080
   aaatgtagtt ttttttttgt ttgttttgtt ttgtttttta agatggagtt tcgttttgtt  1140
   ttataggttg gagtgtagtg gcgtgatttc ggtttattgt aattttcgtt ttttaggttt   1200
   aagcgatttt tttgttttag tttttcgagt agttgagatt aaaggtatgc gttattacgt   1260
   ttggttaatt tttgtatttt tagtagagac ggggttttat cgtgttagtt aggatggttt   1320
   cgattttttg atttcgtgat ttttttgttt cggtttttta tagtgttggg attataggtg  1380
   tgagttatta cgtttggttg gaaatgtagt ttttagcggg gtaggtatgg tggaaggtgg   1440
   atttattttt gttagatatt aatttttttt cgtgtaggcg tttgataagt atttatggag   1500
   cgtttaatgt gtgttttatt tatgtgggaa gtgttgatga agtaaattat ttagaaataa   1560
   ttagttgggt gtggtggcgc gtatttgtag ttttatttat ttcgtaggtt gaggtaggag   1620
   gatggtttga ggtttgggaa ttcgaggttg tatttagttt ttatcgtatt attgtatttt   1680
   agtttgggtg atagagcgag attttatttt taaaaaagaa aaaggtcggg ggtggggggg   1740
   atggagcgtt agttgtgtat aataataaat aatagatata aaataaataa tagatataaa   1800
   aaatttcgtt tttattgaaa atataaaaat tagaggggcg tggtggttta tgtttgtaat   1860
   tttagtattt tgggaggtcg aggttggtgg attatttgag gttaggattt tgagattagt   1920
   ttgatttata tggtgaaatt ttgtttttat taaaaatata aaaattagtc gggcgtggtg   1980
   gtggcgtttg tatttttagt tatttgggag gttgagatag gaggattgtt tgaattcggg   2040
   agatagagtt tgtagtgagt cgagattttg ttattgtatt ttagtttggg cgatagagcg   2100
   agatttcgtt ttaaaaaaga aaaaagaaag aaagaaagaa atagtgttta ttggttaggt   2160
   ttatattcgt aattttagta ttttaggagg tcgaggcggg cggattattt gaggttagta   2220
   gttcgagatt agtttggtta atatggtgaa atttcgtttt tattgaaaat ataaaaatta   2280
   gcggggtgtg gtggcgtatg tttgtaattt tagttattcg ggaggttgag gtaggagaat   2340
   cgtttgaatt cgggaggcgg aggttatagt gagtcgagat tttaatattt cggtttgggt   2400
   tatagagtga tatttagttt taaaaataaa agaaaaataa aaaaaaaaaa aaaaaaagaa   2460
   aaagaatagt gtttattgat gttagatttt aatatttaat aaggtattag atttaaatat   2520
   taataaaata ggttgggtat agtggtttat gtttgtaatt ttagtatttt gggagattta   2580
   ggcgggcgga ttatttgagg ttaagagttt gagattagtt tggttaatat ggtaaaattt   2640
   aatttttatt aaaaatataa aaattagtcg ggtgtggtgg tatatgtttg taattttagt   2700
   tattcgggag gtggaggtag gagaatcgtt tgaatttagt aggtggaagt tgtagtgagt   2760
   taagattgtt ttattgtatt ttagtttggg taatagagcg agattttgtt ttaaaaaaaa   2820
   aaaaaagaaa aagaaaagaa aaagaaaaaa aagaaatttg tagtttagta gggaatatag   2880
   atatttaaat atatatttta aaaatatagt aatatgtgtc gagtttagtg gtttacgttt   2940
   ataattttag tattttggga ggtttaggta ggaggattgt ttgagtttag gattttgaga   3000
   t                                                                   3001
<210> 293
   <211> 3001
   <212> DNA
   <213> human
<400> 293
   gagaaaatat aagagttaaa tttattttag ttttatagat tatgtttttt ttattttatg 60
   tttggatttt ttgttttttt ttgattgtgt attttttgga ttattttttt agtgttttta 120
   atttataaat gttagtgttt ttgagaaata agttttgtat tttttgttta atttaatatt 180
   ggtttttata atggtttata aattattatt tttagtttag atttttttta gttttaggtt 240
   tttaaatagt tttttagatg tttgattttt ttttggaaat ttttttttta gattttttag 300
   atattttttt ttttattaag ttgatggtgg tttttattaa tttgaatttg ttgttttatt 360
   ttgtttttta tattggggat gttttagttt tttagttgag atattagtag gttttgttat 420
   tttttatttt tttttttttt ttagaatgta tagattttag tgtttttaag ttttagagat 480
   attttttttt agtaatatat tttttttttg taatttttat agttattagg tttaattttt 540
   ttattttttt ttataaattt ttgttttttg gtttttatga atttatttag gttgtgtttt 600
   aggtttaata ggttattgtt attaagataa ataattgaga tttttttttt ttgtttatgt 660
   ttattgtaga gatttttaag gggtgaaaga tttggtgtgt tatttttgta gttttttttt 720
   ttttatggtt tttaagaaat tttttttttt tggagatggt tttgtgttgt tttttgagtt 780
   ggttagttgt ttttgtgtgg atgtgtgagt gaagttgaat gaaattgggg tagtggaagt 840
   agttgttagt ggtaaaatat tttttttgga gttagttatt ttgttttttt ttttaggttg 900
   gtgtgttgtt tgttgggaaa tttagtttgg gtttagattg ttaggggttg gttaaagtgt 960
   gtaaaaatta tttttttatt aattattgtg gtattggtgg gagggggtgg taaggggttg 1020
   tgggaagtaa ttgttgtttt tttaaatgtg gtgggagtgt gttgtgttaa gtgtttaggg 1080
   aaatgtagtt ttttttttgt ttgttttgtt ttgtttttta agatggagtt ttgttttgtt 1140
   ttataggttg gagtgtagtg gtgtgatttt ggtttattgt aatttttgtt ttttaggttt 1200
   aagtgatttt tttgttttag ttttttgagt agttgagatt aaaggtatgt gttattatgt 1260
   ttggttaatt tttgtatttt tagtagagat ggggttttat tgtgttagtt aggatggttt 1320
   tgattttttg attttgtgat ttttttgttt tggtttttta tagtgttggg attataggtg 1380
   tgagttatta tgtttggttg gaaatgtagt ttttagtggg gtaggtatgg tggaaggtgg 1440
   atttattttt gttagatatt aatttttttt tgtgtaggtg tttgataagt atttatggag 1500
   tgtttaatgt gtgttttatt tatgtgggaa gtgttgatga agtaaattat ttagaaataa 1560
   ttagttgggt gtggtggtgt gtatttgtag ttttatttat tttgtaggtt gaggtaggag 1620
   gatggtttga ggtttgggaa tttgaggttg tatttagttt ttattgtatt attgtatttt 1680
   agtttgggtg atagagtgag attttatttt taaaaaagaa aaaggttggg ggtggggggg 1740
   atggagtgtt agttgtgtat aataataaat aatagatata aaataaataa tagatataaa 1800
   aaattttgtt tttattgaaa atataaaaat tagaggggtg tggtggttta tgtttgtaat 1860
   tttagtattt tgggaggttg aggttggtgg attatttgag gttaggattt tgagattagt 1920
   ttgatttata tggtgaaatt ttgtttttat taaaaatata aaaattagtt gggtgtggtg 1980
   gtggtgtttg tatttttagt tatttgggag gttgagatag gaggattgtt tgaatttggg 2040
   agatagagtt tgtagtgagt tgagattttg ttattgtatt ttagtttggg tgatagagtg 2100
   agattttgtt ttaaaaaaga aaaaagaaag aaagaaagaa atagtgttta ttggttaggt 2160
   ttatatttgt aattttagta ttttaggagg ttgaggtggg tggattattt gaggttagta 2220
   gtttgagatt agtttggtta atatggtgaa attttgtttt tattgaaaat ataaaaatta 2280
   gtggggtgtg gtggtgtatg tttgtaattt tagttatttg ggaggttgag gtaggagaat 2340
   tgtttgaatt tgggaggtgg aggttatagt gagttgagat tttaatattt tggtttgggt 2400
   tatagagtga tatttagttt taaaaataaa agaaaaataa aaaaaaaaaa aaaaaaagaa 2460
   aaagaatagt gtttattgat gttagatttt aatatttaat aaggtattag atttaaatat 2520
   taataaaata ggttgggtat agtggtttat gtttgtaatt ttagtatttt gggagattta 2580
   ggtgggtgga ttatttgagg ttaagagttt gagattagtt tggttaatat ggtaaaattt 2640
   aatttttatt aaaaatataa aaattagttg ggtgtggtgg tatatgtttg taattttagt 2700
   tatttgggag gtggaggtag gagaattgtt tgaatttagt aggtggaagt tgtagtgagt 2760
   taagattgtt ttattgtatt ttagtttggg taatagagtg agattttgtt ttaaaaaaaa 2820
   aaaaaagaaa aagaaaagaa aaagaaaaaa aagaaatttg tagtttagta gggaatatag 2880
   atatttaaat atatatttta aaaatatagt aatatgtgtt gagtttagtg gtttatgttt 2940
   ataattttag tattttggga ggtttaggta ggaggattgt ttgagtttag gattttgaga 3000
   t 3001
<210> 294
   <211> 3001
   <212> DNA
   <213> human
<400> 294
   gttttaaagt tttgagttta aataattttt ttgtttaggt tttttaaagt gttgggatta      60
   taggcgtgag ttattaagtt cggtatatgt tgttatattt ttaaaatatg tatttaaatg     120
   tttgtatttt ttattagatt ataagttttt tttttttttt tttttttttt tttttttttt     180
   tttttttttg agatagagtt tcgttttgtt gtttaggttg gagtgtagtg gggtaatttt     240
   ggtttattgt aatttttatt tattaggttt aagcgatttt tttgttttta tttttcgagt     300
   agttgggatt ataagtatgt gttattatat tcggttaatt tttgtatttt tagtggagat     360
   tgggttttgt tatgttggtt aggttggttt taaatttttg attttaggtg gttcgttcgt      420
   ttaggttttt taaagtgttg ggattatagg tatgagttat tgtgtttagt ttattttatt     480
   gatatttgaa tttagtgttt tgttgagtat taaaatttga tattagtagg tattattttt     540
   tttttttttt tttttttttt tttgtttttt ttttgttttt gagattgagt gttattttat     600
   agtttaggtc ggagtgttga gatttcggtt tattgtaatt ttcgtttttc gggtttaagc     660
   gatttttttg ttttaatttt tcgagtagtt gggattatag gtatgcgtta ttatatttcg     720
   ttaatttttg tatttttagt agagacgggg ttttattatg ttggttaggt tggtttcgaa     780
   ttattggttt taagtgattc gttcgtttcg gttttttaaa gtgttgggat tacgggtatg    840
   agtttggtta ataggtatta tttttttttt tttttttttt ttttttttta agacggagtt      900
   tcgttttgtc gtttaggttg gagtgtagtg gtaggatttc ggtttattgt aaattttgtt     960
   tttcggattt aagtaatttt tttgttttag ttttttaagt agttggggat ataggcgtta    1020
   ttattacgtt cggttaattt ttgtattttt agtagagata gggttttatt atatgagtta    1080
   ggttggtttt aaagttttga ttttaggtga tttattagtt tcggtttttt aaaatgttgg    1140
   gattataggt atgagttatt acgttttttt aatttttgta tttttagtag agacggggtt    1200
   ttttatgttt attatttatt ttatgtttat tatttattat tgtatataat tgacgtttta    1260
   ttttttttat tttcgatttt tttttttttt agagatgggg tttcgttttg ttatttaggt   1320
   tggaatgtag tggtgcgata agagttaagt gtagtttcga atttttaggt tttaggttat    1380
   ttttttgttt tagtttgcgg agtaaatgga attataggtg cgcgttatta tatttagttg    1440
   attattttta aatgatttat tttattaata ttttttatat aagtagggta tatattaggc    1500
   gttttataaa tatttgttaa acgtttatac gaaggaagat taatgtttgg taagaatgaa    1560
   tttatttttt attatgttta tttcgttaaa aattatattt ttagttaggc gtggtggttt    1620
   atatttgtaa ttttagtatt gtgggaggtc gaggtaggag aattacgagg ttaggaaatc    1680
   gagattattt tggttaatac ggtgaaattt cgtttttatt aaaaatataa aaattagtta    1740
   ggcgtggtgg cgtatgtttt tagttttagt tattcgggag gttgaggtag gagaatcgtt    1800
   tgaatttggg aggcggaggt tgtagtgagt cgagattacg ttattgtatt ttagtttgtg    1860
   aaatagagcg agattttatt ttaaaaaata aaataaaata aataaaaaaa aaattatatt   1920
   tttttgggcg tttggcgcga cgcgttttta ttacgtttga aagagcgata attatttttt    1980
   acggtttttt atcgtttttt ttcgtcggtg ttatagtggt tgatgggaag gtagttttta    2040
   cgtattttgg tcggttttta gcgatttgaa tttaaattaa attttttagt aagtagcgcg     2100
   tcggtttggg aaaaggagta agatggttga ttttaaagag ggtgttttgt cgttgacggt    2160
   tgtttttatt gttttaattt tattcggttt tattcgtacg ttcgtacgga ggcggttggt    2220
   cgattcggga gacggcgcgg ggttattttc ggaggagaag gattttttga aaatcgtgga     2280
   agggagggag ttgtagaggt gacgcgtcgg gttttttatt ttttggaaat ttttgcggtg   2340
   ggcgtaagcg ggagggagga attttagttg tttgttttgg tgatagtgat ttgttaagtt    2400
   tgaaatatag tttgagtaaa ttcgtggagg ttagaaggcg ggagtttata ggagggggtg   2460
   aggaaattgg gtttgatagt tgtgggaatt gcgaagagag gatgtattat tgagaagaag    2520
   tgtttttagg atttgaagat attaaggttt gtgcgttttg ggagaaggaa agaatgagaa    2580
   aatgatagga tttgttagtg ttttaattga agggttgagg cgttttcgat atggggaacg    2640
   ggatggagcg gtagatttag gttgatggag gttattatta gtttaatgag gagaggagtg    2700
   tttgaggggt ttgagggagg gatttttaga agagaattag gtatttagga agttgtttgg    2760
   gaatttggag ttggggaagg tttgggttga agatagtgat ttgtgggtta ttgtagaagt    2820
   cggtattgag ttaaatagag agtgtaggat ttatttttcg aaggtattga tatttatgag    2880
   ttgggggtat tggggagata atttagagaa tgtacgatta gagaggaata ggagatttag    2940
   atatggagta gggaagatat ggtttgtggg gttggggtga gtttggtttt tgtgtttttt    3000
   t                                                                   3001
<210> 295
   <211> 3001
   <212> DNA
   <213> human
<400> 295
   gttttaaagt tttgagttta aataattttt ttgtttaggt tttttaaagt gttgggatta      60
   taggtgtgag ttattaagtt tggtatatgt tgttatattt ttaaaatatg tatttaaatg     120
   tttgtatttt ttattagatt ataagttttt tttttttttt tttttttttt tttttttttt     180
   tttttttttg agatagagtt ttgttttgtt gtttaggttg gagtgtagtg gggtaatttt     240
   ggtttattgt aatttttatt tattaggttt aagtgatttt tttgttttta ttttttgagt    300
   agttgggatt ataagtatgt gttattatat ttggttaatt tttgtatttt tagtggagat     360
   tgggttttgt tatgttggtt aggttggttt taaatttttg attttaggtg gtttgtttgt      420
   ttaggttttt taaagtgttg ggattatagg tatgagttat tgtgtttagt ttattttatt     480
   gatatttgaa tttagtgttt tgttgagtat taaaatttga tattagtagg tattattttt     540
   tttttttttt tttttttttt tttgtttttt ttttgttttt gagattgagt gttattttat     600
   agtttaggtt ggagtgttga gattttggtt tattgtaatt tttgtttttt gggtttaagt     660
   gatttttttg ttttaatttt ttgagtagtt gggattatag gtatgtgtta ttatattttg     720
   ttaatttttg tatttttagt agagatgggg ttttattatg ttggttaggt tggttttgaa     780
   ttattggttt taagtgattt gtttgttttg gttttttaaa gtgttgggat tatgggtatg    840
   agtttggtta ataggtatta tttttttttt tttttttttt ttttttttta agatggagtt     900
   ttgttttgtt gtttaggttg gagtgtagtg gtaggatttt ggtttattgt aaattttgtt     960
   ttttggattt aagtaatttt tttgttttag ttttttaagt agttggggat ataggtgtta    1020
   ttattatgtt tggttaattt ttgtattttt agtagagata gggttttatt atatgagtta    1080
   ggttggtttt aaagttttga ttttaggtga tttattagtt ttggtttttt aaaatgttgg     1140
   gattataggt atgagttatt atgttttttt aatttttgta tttttagtag agatggggtt     1200
   ttttatgttt attatttatt ttatgtttat tatttattat tgtatataat tgatgtttta     1260
   ttttttttat ttttgatttt tttttttttt agagatgggg ttttgttttg ttatttaggt     1320
   tggaatgtag tggtgtgata agagttaagt gtagttttga atttttaggt tttaggttat     1380
   ttttttgttt tagtttgtgg agtaaatgga attataggtg tgtgttatta tatttagttg    1440
   attattttta aatgatttat tttattaata ttttttatat aagtagggta tatattaggt   1500
   gttttataaa tatttgttaa atgtttatat gaaggaagat taatgtttgg taagaatgaa     1560
   tttatttttt attatgttta ttttgttaaa aattatattt ttagttaggt gtggtggttt     1620
   atatttgtaa ttttagtatt gtgggaggtt gaggtaggag aattatgagg ttaggaaatt     1680
   gagattattt tggttaatat ggtgaaattt tgtttttatt aaaaatataa aaattagtta     1740
   ggtgtggtgg tgtatgtttt tagttttagt tatttgggag gttgaggtag gagaattgtt     1800
   tgaatttggg aggtggaggt tgtagtgagt tgagattatg ttattgtatt ttagtttgtg     1860
   aaatagagtg agattttatt ttaaaaaata aaataaaata aataaaaaaa aaattatatt   1920
   tttttgggtg tttggtgtga tgtgttttta ttatgtttga aagagtgata attatttttt     1980
   atggtttttt attgtttttt tttgttggtg ttatagtggt tgatgggaag gtagttttta     2040
   tgtattttgg ttggttttta gtgatttgaa tttaaattaa attttttagt aagtagtgtg   2100
   ttggtttggg aaaaggagta agatggttga ttttaaagag ggtgttttgt tgttgatggt     2160
   tgtttttatt gttttaattt tatttggttt tatttgtatg tttgtatgga ggtggttggt     2220
   tgatttggga gatggtgtgg ggttattttt ggaggagaag gattttttga aaattgtgga   2280
   agggagggag ttgtagaggt gatgtgttgg gttttttatt ttttggaaat ttttgtggtg   2340
   ggtgtaagtg ggagggagga attttagttg tttgttttgg tgatagtgat ttgttaagtt     2400
   tgaaatatag tttgagtaaa tttgtggagg ttagaaggtg ggagtttata ggagggggtg   2460
   aggaaattgg gtttgatagt tgtgggaatt gtgaagagag gatgtattat tgagaagaag     2520
   tgtttttagg atttgaagat attaaggttt gtgtgttttg ggagaaggaa agaatgagaa     2580
   aatgatagga tttgttagtg ttttaattga agggttgagg tgtttttgat atggggaatg     2640
   ggatggagtg gtagatttag gttgatggag gttattatta gtttaatgag gagaggagtg     2700
   tttgaggggt ttgagggagg gatttttaga agagaattag gtatttagga agttgtttgg     2760
   gaatttggag ttggggaagg tttgggttga agatagtgat ttgtgggtta ttgtagaagt     2820
   tggtattgag ttaaatagag agtgtaggat ttattttttg aaggtattga tatttatgag     2880
   ttgggggtat tggggagata atttagagaa tgtatgatta gagaggaata ggagatttag     2940
   atatggagta gggaagatat ggtttgtggg gttggggtga gtttggtttt tgtgtttttt     3000
   t                                                                     3001
<210> 296
   <211> 50
   <212> DNA
   <213> human
<400> 296
   cggcctacga gcctcgccag acccatctcc cagggcctca gcgggataaa 50
<210> 297
   <211> 50
   <212> DNA
   <213> human
<400> 297
   cggtttacga gtttcgttag atttattttt tagggtttta gcgggataaa 50
<210> 298
   <211> 50
   <212> DNA
   <213> human
<400> 298
   tggtttatga gttttgttag atttattttt tagggtttta gtgggataaa 50
<210> 299
   <211> 50
   <212> DNA
   <213> human
<400> 299
   tttatttcgt tgaggttttg ggagatgggt ttggcgaggt tcgtaggtcg 50
<210> 300
   <211> 50
   <212> DNA
   <213> human
<400> 300
   tttattttgt tgaggttttg ggagatgggt ttggtgaggt ttgtaggttg 50
<210> 301
   <211> 3001
   <212> DNA
   <213> human
<400> 301
<210> 302
   <211> 3001
   <212> DNA
   <213> human
<400> 302
   ttgttagaga agttagggat ttggagtttt gaaaattata gagatattta aaattataat      60
   tttagagaaa gatttaaaat gatgaagtat taattatttt tgtttatagt ttttttttgt     120
   aaatgtggag gaaatatgta gggatatata tttttttttt ttttttttta taagatgatt      180
   tatttttttt aaattgagaa atgagaaaga ttgggtggaa ggaggttgtg gattgatatg    240
   aaagagaaaa tttttttaat ttagaaaaat agtttatgtg tttttatata cgtgtatata     300
   tgtgtatata tatatatata tatatatata tatatatatt taattttttt gaatatttta     360
   gttttttttg tatttttttt gttttttttt tattggtttt ttagtttttt gttttagaat    420
   aattttatta ggtttgtaga gttattattg aaaagggtat ggttgttttt tagattgtaa     480
   ttttgagttt agaggtttgg ttaaagattg gaggagtatt taggttttga tttttaggtt     540
   tgttagtgtt ttaggatatt tgtttattta tatttattta taattttacg tgtatttcgg     600
   tttttcgatt gagagaatta tgaggaagga gggggtagtt tttgttatta ttaaggtaaa     660
   gaggtgttag gtagttttgt tcggttttag ggtttattaa ttttgttatt atttttatta     720
   gtggattttt ttttaattat atttaggttt ttatattgtt gaggttattg atttttatag     780
   attaggttag aggttagttt agttttgttt ttttttaagg tttttttata tagagatgaa    840
   gtttaggttt ttaatgtttt ttttttgaat ttttttttta agtttaaaat ttttaaggag   900
   taagaaagag ggtaggtggt tgcgtgtgtg ttttagagga gagaggcgag gagttgttgt     960
   ggaatgggtt atgtgataat tttgttagtt tgataatgta agggtgtgaa cgttatttgt    1020
   gtagaataga gggaatggtt ttaggtgttg tttaggattt ttttttggaa gaggttatag     1080
   ttgtggtgag aaggtaatag tattttgttt atagtttcga aagtaggttt tatttaaaga    1140
   attaggtata aaatagaagt tttttttttt tttttataaa agaaaaggtt gttttttggt   1200
   gagtaaatta ggggagatag ggggtaagga gggtatttta tagagattat tttttttatt    1260
   tttggtggtt agtgatttag gaggatttag agaattttat ttttaggttt ttaggttttt    1320
   gttttttatt tttttaatat ttttaggttt gttgagttag ggtttagttg tttttttttt   1380
   tttttcgttg gaaatagaag tttttagcga atgtttttaa tttttgagtt tatttagtta    1440
   tgtttttttt gtaaaatttt ttttttttcg tatttttttg tatttagtta gttaattcgc   1500
   ggtttacgag tttcgttaga tttatttttt agggttttag cgggataaaa tcggtcggtg    1560
   atgtcgggtg gatgggaata aattttagaa ggaggagaga tatcgggttt agggtatttt    1620
   cgcgggcgga tttaattagt gagggtttgt agtcggtcgg ttaggtatgt tatttagggg    1680
   ttagttggaa gttggttagt agtgaggacg ggggatggaa gaaaggagag ggttttagga    1740
   tgtttggtag tttttttttt tttaagttaa acgggataag attgggatag cggagggagt    1800
   gggtacggag gttggagttt ggagtttttt tagcgtattt ttaatttttg attttttcgg   1860
   gaagtttgtt agttaggtta ggttgaggtt ggttttattt cgtttaaaaa tttttttatt   1920
   tttggtagac gatgtaggtg taaaggatga ggtttgggga gggggtttga ggatgtaggt    1980
   atcgaatttt atcgtatttt atgttttttt tttgggtttg tgtagggtag cggtaggcgc   2040
   ggttcggatt tacgggagga agtttcgagt tttcggcggg ttgcgagcga tttttcggcg    2100
   atgttttata attttattag atttggtaag aacgcggtgt ggttaggatt ttcgagtttt     2160
   cgcgtcgttg agcgttcgtg cgtgcgttcg ggtggttgga tttcgagagc gttagtacgg    2220
   gattcgcggg atttattcgc gtggggattt agttggggcg tttggggttt ggtttttcgt    2280
   tggagtcgtt tcgttgtttt cgggtattgg ggttttattc ggttagttga gtcgtttttg    2340
   ttcggtttcg agggattata tggatcggtg aggggatttt tgtggagtta gagggggttt    2400
   tttttgagtt ttatttttag ttttgtatgg cgtattagtg tggatatttt tcgtgttttg    2460
   tttttgggtt gtatcggtgt tggatttgtc gttttttcgt tttcgatttt taagtttttt   2520
   ttttcgggtg cgcgtagttt tttttagtga tttttttggt tggattgtga gtttttcgta   2580
   ttttttaagt taacggtaat ggagtcggtg gtttcgggtt cgggagtcgg tagcgtattt    2640
   aggtgttttt cgattttttt tggtttagta tcgggtaggt cgggcgtcgc gcgcggggcg    2700
   ttcggttggt gaggattaag tcgttcgcgg agttaaaatt ttttttttat ttgcgttcgg   2760
   aagtagggtt tttaaatata ttcgagtttt taggtcgcga tgtttgtttt tttttttttc    2820
   ggttagggtt gggtagggtt tttgttggga aggagtttta gggttagggt tggtgggcgt    2880
   agtcgtttcg gcgcgtagcg agtgcggata gcggtggagg cgtagcgcgg ttcgtagtga    2940
   tggatggagt tcggtcggtc gtagagaagg cgatttatgg ggttagggcg tatcgttatc    3000
   g                                                                    3001
<210> 303
   <211> 3001
   <212> DNA
   <213> human
<400> 303
   ttgttagaga agttagggat ttggagtttt gaaaattata gagatattta aaattataat 60
   tttagagaaa gatttaaaat gatgaagtat taattatttt tgtttatagt ttttttttgt 120
   aaatgtggag gaaatatgta gggatatata tttttttttt ttttttttta taagatgatt 180
   tatttttttt aaattgagaa atgagaaaga ttgggtggaa ggaggttgtg gattgatatg 240
   aaagagaaaa tttttttaat ttagaaaaat agtttatgtg tttttatata tgtgtatata 300
   tgtgtatata tatatatata tatatatata tatatatatt taattttttt gaatatttta 360
   gttttttttg tatttttttt gttttttttt tattggtttt ttagtttttt gttttagaat 420
   aattttatta ggtttgtaga gttattattg aaaagggtat ggttgttttt tagattgtaa 480
   ttttgagttt agaggtttgg ttaaagattg gaggagtatt taggttttga tttttaggtt 540
   tgttagtgtt ttaggatatt tgtttattta tatttattta taattttatg tgtattttgg 600
   ttttttgatt gagagaatta tgaggaagga gggggtagtt tttgttatta ttaaggtaaa 660
   gaggtgttag gtagttttgt ttggttttag ggtttattaa ttttgttatt atttttatta 720
   gtggattttt ttttaattat atttaggttt ttatattgtt gaggttattg atttttatag 780
   attaggttag aggttagttt agttttgttt ttttttaagg tttttttata tagagatgaa 840
   gtttaggttt ttaatgtttt ttttttgaat ttttttttta agtttaaaat ttttaaggag 900
   taagaaagag ggtaggtggt tgtgtgtgtg ttttagagga gagaggtgag gagttgttgt 960
   ggaatgggtt atgtgataat tttgttagtt tgataatgta agggtgtgaa tgttatttgt 1020
   gtagaataga gggaatggtt ttaggtgttg tttaggattt ttttttggaa gaggttatag 1080
   ttgtggtgag aaggtaatag tattttgttt atagttttga aagtaggttt tatttaaaga 1140
   attaggtata aaatagaagt tttttttttt tttttataaa agaaaaggtt gttttttggt 1200
   gagtaaatta ggggagatag ggggtaagga gggtatttta tagagattat tttttttatt 1260
   tttggtggtt agtgatttag gaggatttag agaattttat ttttaggttt ttaggttttt 1320
   gttttttatt tttttaatat ttttaggttt gttgagttag ggtttagttg tttttttttt 1380
   ttttttgttg gaaatagaag tttttagtga atgtttttaa tttttgagtt tatttagtta 1440
   tgtttttttt gtaaaatttt tttttttttg tatttttttg tatttagtta gttaatttgt 1500
   ggtttatgag ttttgttaga tttatttttt agggttttag tgggataaaa ttggttggtg 1560
   atgttgggtg gatgggaata aattttagaa ggaggagaga tattgggttt agggtatttt 1620
   tgtgggtgga tttaattagt gagggtttgt agttggttgg ttaggtatgt tatttagggg 1680
   ttagttggaa gttggttagt agtgaggatg ggggatggaa gaaaggagag ggttttagga 1740
   tgtttggtag tttttttttt tttaagttaa atgggataag attgggatag tggagggagt 1800
   gggtatggag gttggagttt ggagtttttt tagtgtattt ttaatttttg atttttttgg 1860
   gaagtttgtt agttaggtta ggttgaggtt ggttttattt tgtttaaaaa tttttttatt 1920
   tttggtagat gatgtaggtg taaaggatga ggtttgggga gggggtttga ggatgtaggt 1980
   attgaatttt attgtatttt atgttttttt tttgggtttg tgtagggtag tggtaggtgt 2040
   ggtttggatt tatgggagga agttttgagt ttttggtggg ttgtgagtga ttttttggtg 2100
   atgttttata attttattag atttggtaag aatgtggtgt ggttaggatt tttgagtttt 2160
   tgtgttgttg agtgtttgtg tgtgtgtttg ggtggttgga ttttgagagt gttagtatgg 2220
   gatttgtggg atttatttgt gtggggattt agttggggtg tttggggttt ggttttttgt 2280
   tggagttgtt ttgttgtttt tgggtattgg ggttttattt ggttagttga gttgtttttg 2340
   tttggttttg agggattata tggattggtg aggggatttt tgtggagtta gagggggttt 2400
   tttttgagtt ttatttttag ttttgtatgg tgtattagtg tggatatttt ttgtgttttg 2460
   tttttgggtt gtattggtgt tggatttgtt gtttttttgt ttttgatttt taagtttttt 2520
   tttttgggtg tgtgtagttt tttttagtga tttttttggt tggattgtga gttttttgta 2580
   ttttttaagt taatggtaat ggagttggtg gttttgggtt tgggagttgg tagtgtattt 2640
   aggtgttttt tgattttttt tggtttagta ttgggtaggt tgggtgttgt gtgtggggtg 2700
   tttggttggt gaggattaag ttgtttgtgg agttaaaatt ttttttttat ttgtgtttgg 2760
   aagtagggtt tttaaatata tttgagtttt taggttgtga tgtttgtttt tttttttttt 2820
   ggttagggtt gggtagggtt tttgttggga aggagtttta gggttagggt tggtgggtgt 2880
   agttgttttg gtgtgtagtg agtgtggata gtggtggagg tgtagtgtgg tttgtagtga 2940
   tggatggagt ttggttggtt gtagagaagg tgatttatgg ggttagggtg tattgttatt 3000
   g 3001
<210> 304
   <211> 3001
   <212> DNA
   <213> human
<400> 304
   cgatagcgat gcgttttggt tttataaatc gttttttttg cggtcggtcg ggttttattt 60
   attattgcgg gtcgcgttgc gtttttatcg ttgttcgtat tcgttgcgcg tcgggacggt 120
   tgcgtttatt agttttgatt ttggagtttt tttttagtaa agattttgtt tagttttggt 180
   cgggaagggg gaaaataggt atcgcggttt gggagttcga gtgtgtttga aggttttgtt 240
   ttcgggcgta ggtggggagg gggttttgat ttcgcgagcg gtttagtttt tattagtcgg 300
   acgtttcgcg cgcggcgttc ggtttgttcg gtgttgggtt aggaggagtc ggagagtatt 360
   tgagtgcgtt gtcggttttc gggttcggga ttatcggttt tattgtcgtt ggtttgagag 420
   atgcggggag tttatagttt agttagggga gttattggga aggattgcgc gtattcgagg 480
   aagaggattt agaggtcggg gacggggaaa cggtaagttt agtatcggtg tagtttaggg 540
   atagagtacg aagaatgttt atattaatgc gttatgtaag gttgggaatg gagtttaaag 600
   gaaatttttt ttaattttat agagattttt ttatcgattt atgtggtttt tcgaaatcgg 660
   gtaagggcgg tttagttggt cgggtggaat tttagtgttc gagaatagcg aggcggtttt 720
   agcggagggt taggttttag gcgttttagt tgggttttta cgcgggtggg tttcgcgaat 780
   ttcgtgttga cgttttcgag atttaattat tcgagcgtac gtacggacgt ttagcggcgc 840
   gggggttcgg ggattttgat tatatcgcgt ttttattaga tttgatggag ttgtgaggta 900
   tcgtcgggga gtcgttcgta gttcgtcgag ggttcggggt tttttttcgt aggttcgggt 960
   cgcgtttgtc gttgttttgt ataaatttag gagaagggta tgagatgcga tgagattcga 1020
   tgtttgtatt tttaggtttt ttttttaggt tttatttttt atatttgtat cgtttgttag 1080
   gagtaggagg atttttaggc gaagtagggt taattttagt ttagtttagt taataggttt 1140
   ttcgggagga ttaaggattg ggagtgcgtt gaagaagttt tagattttaa ttttcgtgtt 1200
   tatttttttc gttgttttag ttttatttcg tttaatttgg gagggaaaag gttgttaggt 1260
   attttgggat tttttttttt tttttatttt tcgtttttat tgttagttag tttttagtta 1320
   atttttgggt gatatatttg gtcggtcggt tgtaggtttt tattggttgg gttcgttcgc 1380
   gagggtgttt tgggttcggt gttttttttt tttttgaagt ttgtttttat ttattcggta 1440
   ttatcgatcg gttttatttc gttgaggttt tgggagatgg gtttggcgag gttcgtaggt 1500
   cgcggattgg ttggttgggt gtaggggggt gcgggaaggg gaggattttg taagggggat 1560
   atggttgagt ggatttagga gttagaaata ttcgttggga gtttttgttt ttagcggagg 1620
   gaagagggag gtagttgagt tttaatttag taggtttgga aatattggag gagtggggaa 1680
   tagaggttta ggagtttgag gatgaggttt tttgggtttt tttgggttat tgattattag 1740
   aagtgagagg gatggttttt gtaggatgtt ttttttgttt tttgtttttt ttggtttgtt 1800
   tattaggaag tagttttttt ttttgtaaaa ggaaggaggg aatttttgtt ttatgtttga 1860
   ttttttaggt agagtttgtt ttcggggttg taggtaagat gttattattt ttttattata 1920
   gttatgattt tttttaggga agaattttga gtagtatttg aagttatttt ttttgtttta 1980
   tatagatagc gtttatattt ttgtattgtt aggttaataa aattgttata taatttattt 2040
   tatagtaatt tttcgttttt tttttttgga atatatacgt agttatttgt tttttttttt 2100
   gttttttgag gattttgaat ttggaagaag agtttagggg aagggtattg agggtttaga 2160
   ttttattttt gtgtggaaag gttttagaaa ggaataggat tgagttgatt tttggtttgg 2220
   tttgtaggaa ttaatagttt tagtaatatg ggggtttggg tgtggttaag aaggggttta 2280
   ttggtgggaa tgatggtagg gttggtgagt tttgggatcg agtagagttg tttggtattt 2340
   ttttatttta gtgatggtaa aagttgtttt tttttttttt atagtttttt tagtcgggga 2400
   atcggggtat acgtaagatt gtgagtgggt atgagtgagt aaatgttttg gaatattggt 2460
   aggtttgggg gttaaggttt ggatgttttt ttaatttttg gttagatttt tggatttagg 2520
   attatagttt agaggatagt tatgtttttt ttagtgatgg ttttgtaggt ttggtggggt 2580
   tgttttggga taggaggtta agaagttagt gggagggggg taaggaaggt gtaggggaaa 2640
   ttgggatatt tagggggatt ggatgtgtgt gtgtgtgtgt gtgtgtgtgt gtgtatgtat 2700
   atatgtatac gtgtgtgaaa atatatgaat tatttttttg aattaaaaaa gttttttttt 2760
   ttatgttaat ttataatttt tttttattta gtttttttta ttttttaatt taggagaaat 2820
   gaattatttt gtgaaaaaag agagaagaag atgtgtattt ttatatgttt tttttatatt 2880
   tgtaaagagg ggttgtaaat aaaaatagtt ggtgttttat tattttaaat ttttttttaa 2940
   aattgtaatt ttaaatattt ttgtggtttt taaagtttta gatttttaat ttttttggta 3000
   g 3001
<210> 305
   <211> 3001
   <212> DNA
   <213> human
<400> 305
   tgatagtgat gtgttttggt tttataaatt gttttttttg tggttggttg ggttttattt 60
   attattgtgg gttgtgttgt gtttttattg ttgtttgtat ttgttgtgtg ttgggatggt 120
   tgtgtttatt agttttgatt ttggagtttt tttttagtaa agattttgtt tagttttggt 180
   tgggaagggg gaaaataggt attgtggttt gggagtttga gtgtgtttga aggttttgtt 240
   tttgggtgta ggtggggagg gggttttgat tttgtgagtg gtttagtttt tattagttgg 300
   atgttttgtg tgtggtgttt ggtttgtttg gtgttgggtt aggaggagtt ggagagtatt 360
   tgagtgtgtt gttggttttt gggtttggga ttattggttt tattgttgtt ggtttgagag 420
   atgtggggag tttatagttt agttagggga gttattggga aggattgtgt gtatttgagg 480
   aagaggattt agaggttggg gatggggaaa tggtaagttt agtattggtg tagtttaggg 540
   atagagtatg aagaatgttt atattaatgt gttatgtaag gttgggaatg gagtttaaag 600
   gaaatttttt ttaattttat agagattttt ttattgattt atgtggtttt ttgaaattgg 660
   gtaagggtgg tttagttggt tgggtggaat tttagtgttt gagaatagtg aggtggtttt 720
   agtggagggt taggttttag gtgttttagt tgggttttta tgtgggtggg ttttgtgaat 780
   tttgtgttga tgtttttgag atttaattat ttgagtgtat gtatggatgt ttagtggtgt 840
   gggggtttgg ggattttgat tatattgtgt ttttattaga tttgatggag ttgtgaggta 900
   ttgttgggga gttgtttgta gtttgttgag ggtttggggt ttttttttgt aggtttgggt 960
   tgtgtttgtt gttgttttgt ataaatttag gagaagggta tgagatgtga tgagatttga 1020
   tgtttgtatt tttaggtttt ttttttaggt tttatttttt atatttgtat tgtttgttag 1080
   gagtaggagg atttttaggt gaagtagggt taattttagt ttagtttagt taataggttt 1140
   tttgggagga ttaaggattg ggagtgtgtt gaagaagttt tagattttaa tttttgtgtt 1200
   tatttttttt gttgttttag ttttattttg tttaatttgg gagggaaaag gttgttaggt 1260
   attttgggat tttttttttt tttttatttt ttgtttttat tgttagttag tttttagtta 1320
   atttttgggt gatatatttg gttggttggt tgtaggtttt tattggttgg gtttgtttgt 1380
   gagggtgttt tgggtttggt gttttttttt tttttgaagt ttgtttttat ttatttggta 1440
   ttattgattg gttttatttt gttgaggttt tgggagatgg gtttggtgag gtttgtaggt 1500
   tgtggattgg ttggttgggt gtaggggggt gtgggaaggg gaggattttg taagggggat 1560
   atggttgagt ggatttagga gttagaaata tttgttggga gtttttgttt ttagtggagg 1620
   gaagagggag gtagttgagt tttaatttag taggtttgga aatattggag gagtggggaa 1680
   tagaggttta ggagtttgag gatgaggttt tttgggtttt tttgggttat tgattattag 1740
   aagtgagagg gatggttttt gtaggatgtt ttttttgttt tttgtttttt ttggtttgtt 1800
   tattaggaag tagttttttt ttttgtaaaa ggaaggaggg aatttttgtt ttatgtttga 1860
   ttttttaggt agagtttgtt tttggggttg taggtaagat gttattattt ttttattata 1920
   gttatgattt tttttaggga agaattttga gtagtatttg aagttatttt ttttgtttta 1980
   tatagatagt gtttatattt ttgtattgtt aggttaataa aattgttata taatttattt 2040
   tatagtaatt ttttgttttt tttttttgga atatatatgt agttatttgt tttttttttt 2100
   gttttttgag gattttgaat ttggaagaag agtttagggg aagggtattg agggtttaga 2160
   ttttattttt gtgtggaaag gttttagaaa ggaataggat tgagttgatt tttggtttgg 2220
   tttgtaggaa ttaatagttt tagtaatatg ggggtttggg tgtggttaag aaggggttta 2280
   ttggtgggaa tgatggtagg gttggtgagt tttgggattg agtagagttg tttggtattt 2340
   ttttatttta gtgatggtaa aagttgtttt tttttttttt atagtttttt tagttgggga 2400
   attggggtat atgtaagatt gtgagtgggt atgagtgagt aaatgttttg gaatattggt 2460
   aggtttgggg gttaaggttt ggatgttttt ttaatttttg gttagatttt tggatttagg 2520
   attatagttt agaggatagt tatgtttttt ttagtgatgg ttttgtaggt ttggtggggt 2580
   tgttttggga taggaggtta agaagttagt gggagggggg taaggaaggt gtaggggaaa 2640
   ttgggatatt tagggggatt ggatgtgtgt gtgtgtgtgt gtgtgtgtgt gtgtatgtat 2700
   atatgtatat gtgtgtgaaa atatatgaat tatttttttg aattaaaaaa gttttttttt 2760
   ttatgttaat ttataatttt tttttattta gtttttttta ttttttaatt taggagaaat 2820
   gaattatttt gtgaaaaaag agagaagaag atgtgtattt ttatatgttt tttttatatt 2880
   tgtaaagagg ggttgtaaat aaaaatagtt ggtgttttat tattttaaat ttttttttaa 2940
   aattgtaatt ttaaatattt ttgtggtttt taaagtttta gatttttaat ttttttggta 3000
   g 3001
<210> 306
   <211> 50
   <212> DNA
   <213> human
<400> 306
   ctaagcaggt ctcatggtcc actcaaggtg tttcatgctt ctcagttacg 50
<210> 307
   <211> 50
   <212> DNA
   <213> human
<400> 307
   ttaagtaggt tttatggttt atttaaggtg ttttatgttt tttagttacg 50
<210> 308
   <211> 50
   <212> DNA
   <213> human
<400> 308
   ttaagtaggt tttatggttt atttaaggtg ttttatgttt tttagttatg 50
<210> 309
   <211> 50
   <212> DNA
   <213> human
<400> 309
   cgtaattgag aagtatgaaa tattttgagt ggattatgag atttgtttag 50
<210> 310
   <211> 50
   <212> DNA
   <213> human
<400> 310
   tgtaattgag aagtatgaaa tattttgagt ggattatgag atttgtttag 50
<210> 311
   <211> 3001
   <212> DNA
   <213> human
<400> 311
   cagaactttg tgccagcgat cccgcaccaa aacttccagc agcccgctcc gctgcgcccg 60
   gccgcctccc gcgccagccg gcccagccgc cgccgccgcc gccgctaccg ccatctttcc 120
   ggcattctta aaatgccatg tgattggaaa aggggggaaa agtggggaag ggtggccggg 180
   gggaggacgc ggggcccggg ggagcgagga gagtgcgtcc cgcggggagg tggcggcacg 240
   cgggactccg ctccgggagt tcgcagacgc actcggcggg agttggcagc tgcactcagg 300
   ctggttcccc ctcgcctgat cctgaccggg gtggagcaac ctaaaagaag aaggagagac 360
   cgagacaggg gaggggaaaa aagccagcag ccagttgcaa aaccccaaag caaggggagc 420
   cgcggagcag ctcagcgctg gagagagtcc gtgggaggag gcacgctggc ggtgaagggc 480
   ggggacccac aacccagcgg ccgctcacct gttccagccg ccgcagcctc ggcggtcaca 540
   cgccacccgc ccgcccagac ctcccctcct ctgtccccag agcgcacgac gccgcgggga 600
   cagcttcccg agcgccttcc ccgtcccgct tctctccagc ccaccgcagc cccctttctt 660
   cccagctccc ccactccgtg ggcgcccaca ctgcaccttc gccgggtgcg tgcgtcccgc 720
   ctcggtgccc tcactcggtc ctcctgagcc gacttccctt tcatacccag cccgtagaat 780
   tgtccctggg tcttaacaac tcatttgtaa ctgatccagg tctcctccct ctgcttcctc 840
   aaacccaggc ttcgctgcct ctgcggagtt cttacctgtc tctcctttcc acccgggttc 900
   cctggaggaa gctaaactca gaccaaggcc ctgggctccc caggagttaa aagggaatac 960
   gctgtcccaa gattctagaa tgaagagtca acgtagcccg agtggcttaa acctcctgtc 1020
   cttaaatgca agaaatgttt tctatcgagc cctggacagg tgtctctgct ggcctggggt 1080
   tttcaacagg tcatgcctgc ctcagacccc agggacaaat gttcttccag ctctaactca 1140
   ttctatgctt taagcttttg acctatcttt gttttcccag tgccacacca aatgctgcct 1200
   ggggatctct ctttcttcct gagttcccat ataagaagcc ccccatttaa gaattcagtt 1260
   ggaatgggtt gtatttcaaa agttgctttg caagttagtt atttggattt caagttgcat 1320
   tttaccaggg taacaatatt ataatgattg tttaccttcc cagagcaatc cagaaatgcc 1380
   cacataaccc atgtcacacc tgaaccaccc tgagttcttc tatccttgaa cctctcaagc 1440
   tttcccctaa ctctaagcag gtctcatggt ccactcaagg tgtttcatgc ttctcagtta 1500
   cgtccccttc ccactgctgt ctaccctctc tccaaacaca acacaaaaca aacccacaac 1560
   agttctgtta attcctgaag taaacccaac ccagcaaggt gttgagagga aataaatctt 1620
   attgaacaca ttcccattga tgtgtatcat cgttgaaagt tctctaagtc ttttagaatg 1680
   actcacactt ttaatcaagg aggcttaaag gctcagatga ctccctaaag agcggagtcc 1740
   cagccaggtc aaggaatgaa atgaaaacat ttggacattt tcttgagaag gcggagtcac 1800
   tgcaggtgag agggtgccct gggttaatgc ttctgcttgt agctctccct tttcgtatat 1860
   ttttaactca cccattttcc aaaaactctt tactctttca tgcaaagatg gccagagcag 1920
   ggatggcagt ctcaggggct ctccagccct ttgtgatgtg cagaatcaga caaggagcta 1980
   atatagatgg taccaccatc cactctcagg atggaaaagc agctgaggct ggccccttgc 2040
   tttagagact gaggactcac gtgcatcatt cttcagtgaa accagagcct gagtatctgc 2100
   gttgaaatcc agatacacta aggttgagat aggcaccaga gtgccagtgt gaagatttac 2160
   aaatcaaaac agatccactc ctggaacaga aatcctgcat caacatcatg cctgcatctt 2220
   gcctctgtgg acaagcaatg tgggaggccc aggcacaacc acagattatg tatcgatttg 2280
   ccataagaaa ttgtaggctc tgggatgaga atttctcaga atctctgtga taagcacatt 2340
   ttttttctat gtaagcaaac ctacatggga aggagtatgt tgctttttga aagttgcatc 2400
   ggaatgagag gagacaatta ttcagcctga ggcatgtatg ttagccagag atggctttgc 2460
   cactggaatc ataattacag cccctattct aaatgcttta cacatattaa cttaatcctc 2520
   ccagcaattc ttgaggatat ttactattac cgtcattccc atttcacagg tgaagaaggg 2580
   gactaagtta ataagttgcc cctgtcatgt aattagtaaa tggcagagtc aggatttaaa 2640
   gccagctgtg agagtctaga ctctaaactg ttgctcttac actcttgaaa atcctcaaag 2700
   cctgttagct acagcccctg tgttcatctg cacactgaag ccagcctttc cactgggagg 2760
   gagacactga ggaggaaggc ccttccccta gaccatatgg agaagggtgg aaccagctga 2820
   tgcctctggc ctttccttct ttccaaggtt ggagaacacc ctgccctccc caagtcgaca 2880
   catttttttt ctatagacat gttaacaagt tgaaactggt gacttatggt cttattatac 2940
   ttcgcctgtc tttttttttt tttttttaaa aacttatggc taggcgcagt ggctcacgcc 3000
   t 3001
<210> 312
   <211> 3001
   <212> DNA
   <213> human
<400> 312
   tagaattttg tgttagcgat ttcgtattaa aatttttagt agttcgtttc gttgcgttcg 60
   gtcgtttttc gcgttagtcg gtttagtcgt cgtcgtcgtc gtcgttatcg ttattttttc 120
   ggtattttta aaatgttatg tgattggaaa aggggggaaa agtggggaag ggtggtcggg 180
   gggaggacgc ggggttcggg ggagcgagga gagtgcgttt cgcggggagg tggcggtacg     240
   cgggatttcg tttcgggagt tcgtagacgt attcggcggg agttggtagt tgtatttagg     300
   ttggtttttt ttcgtttgat tttgatcggg gtggagtaat ttaaaagaag aaggagagat     360
   cgagataggg gaggggaaaa aagttagtag ttagttgtaa aattttaaag taaggggagt     420
   cgcggagtag tttagcgttg gagagagttc gtgggaggag gtacgttggc ggtgaagggc     480
   ggggatttat aatttagcgg tcgtttattt gttttagtcg tcgtagtttc ggcggttata     540
   cgttattcgt tcgtttagat tttttttttt ttgtttttag agcgtacgac gtcgcgggga     600
   tagtttttcg agcgtttttt tcgtttcgtt tttttttagt ttatcgtagt tttttttttt     660
   tttagttttt ttatttcgtg ggcgtttata ttgtattttc gtcgggtgcg tgcgtttcgt     720
   ttcggtgttt ttattcggtt tttttgagtc gatttttttt ttatatttag ttcgtagaat     780
   tgtttttggg ttttaataat ttatttgtaa ttgatttagg tttttttttt ttgttttttt     840
   aaatttaggt ttcgttgttt ttgcggagtt tttatttgtt tttttttttt attcgggttt     900
   tttggaggaa gttaaattta gattaaggtt ttgggttttt taggagttaa aagggaatac     960
   gttgttttaa gattttagaa tgaagagtta acgtagttcg agtggtttaa attttttgtt   1020
   tttaaatgta agaaatgttt tttatcgagt tttggatagg tgtttttgtt ggtttggggt    1080
   ttttaatagg ttatgtttgt tttagatttt agggataaat gtttttttag ttttaattta    1140
   ttttatgttt taagtttttg atttattttt gtttttttag tgttatatta aatgttgttt   1200
   ggggattttt tttttttttt gagtttttat ataagaagtt ttttatttaa gaatttagtt    1260
   ggaatgggtt gtattttaaa agttgttttg taagttagtt atttggattt taagttgtat    1320
   tttattaggg taataatatt ataatgattg tttatttttt tagagtaatt tagaaatgtt    1380
   tatataattt atgttatatt tgaattattt tgagtttttt tatttttgaa ttttttaagt   1440
   ttttttttaa ttttaagtag gttttatggt ttatttaagg tgttttatgt tttttagtta   1500
   cgtttttttt ttattgttgt ttattttttt tttaaatata atataaaata aatttataat    1560
   agttttgtta atttttgaag taaatttaat ttagtaaggt gttgagagga aataaatttt    1620
   attgaatata tttttattga tgtgtattat cgttgaaagt tttttaagtt ttttagaatg    1680
   atttatattt ttaattaagg aggtttaaag gtttagatga ttttttaaag agcggagttt   1740
   tagttaggtt aaggaatgaa atgaaaatat ttggatattt ttttgagaag gcggagttat    1800
   tgtaggtgag agggtgtttt gggttaatgt ttttgtttgt agtttttttt tttcgtatat    1860
   ttttaattta tttatttttt aaaaattttt tattttttta tgtaaagatg gttagagtag   1920
   ggatggtagt tttaggggtt ttttagtttt ttgtgatgtg tagaattaga taaggagtta    1980
   atatagatgg tattattatt tatttttagg atggaaaagt agttgaggtt ggttttttgt   2040
   tttagagatt gaggatttac gtgtattatt ttttagtgaa attagagttt gagtatttgc    2100
   gttgaaattt agatatatta aggttgagat aggtattaga gtgttagtgt gaagatttat    2160
   aaattaaaat agatttattt ttggaataga aattttgtat taatattatg tttgtatttt    2220
   gtttttgtgg ataagtaatg tgggaggttt aggtataatt atagattatg tatcgatttg    2280
   ttataagaaa ttgtaggttt tgggatgaga attttttaga atttttgtga taagtatatt    2340
   ttttttttat gtaagtaaat ttatatggga aggagtatgt tgttttttga aagttgtatc    2400
   ggaatgagag gagataatta tttagtttga ggtatgtatg ttagttagag atggttttgt    2460
   tattggaatt ataattatag tttttatttt aaatgtttta tatatattaa tttaattttt    2520
   ttagtaattt ttgaggatat ttattattat cgttattttt attttatagg tgaagaaggg    2580
   gattaagtta ataagttgtt tttgttatgt aattagtaaa tggtagagtt aggatttaaa    2640
   gttagttgtg agagtttaga ttttaaattg ttgtttttat atttttgaaa atttttaaag    2700
   tttgttagtt atagtttttg tgtttatttg tatattgaag ttagtttttt tattgggagg   2760
   gagatattga ggaggaaggt ttttttttta gattatatgg agaagggtgg aattagttga    2820
   tgtttttggt tttttttttt ttttaaggtt ggagaatatt ttgttttttt taagtcgata    2880
   tatttttttt ttatagatat gttaataagt tgaaattggt gatttatggt tttattatat    2940
   ttcgtttgtt tttttttttt tttttttaaa aatttatggt taggcgtagt ggtttacgtt    3000
   t                                                                     3001
<210> 313
   <211> 3001
   <212> DNA
   <213> human
<400> 313
   tagaattttg tgttagtgat tttgtattaa aatttttagt agtttgtttt gttgtgtttg 60
   gttgtttttt gtgttagttg gtttagttgt tgttgttgtt gttgttattg ttattttttt 120
   ggtattttta aaatgttatg tgattggaaa aggggggaaa agtggggaag ggtggttggg 180
   gggaggatgt ggggtttggg ggagtgagga gagtgtgttt tgtggggagg tggtggtatg 240
   tgggattttg ttttgggagt ttgtagatgt atttggtggg agttggtagt tgtatttagg 300
   ttggtttttt tttgtttgat tttgattggg gtggagtaat ttaaaagaag aaggagagat 360
   tgagataggg gaggggaaaa aagttagtag ttagttgtaa aattttaaag taaggggagt 420
   tgtggagtag tttagtgttg gagagagttt gtgggaggag gtatgttggt ggtgaagggt 480
   ggggatttat aatttagtgg ttgtttattt gttttagttg ttgtagtttt ggtggttata 540
   tgttatttgt ttgtttagat tttttttttt ttgtttttag agtgtatgat gttgtgggga 600
   tagttttttg agtgtttttt ttgttttgtt tttttttagt ttattgtagt tttttttttt 660
   tttagttttt ttattttgtg ggtgtttata ttgtattttt gttgggtgtg tgtgttttgt 720
   tttggtgttt ttatttggtt tttttgagtt gatttttttt ttatatttag tttgtagaat 780
   tgtttttggg ttttaataat ttatttgtaa ttgatttagg tttttttttt ttgttttttt 840
   aaatttaggt tttgttgttt ttgtggagtt tttatttgtt tttttttttt atttgggttt 900
   tttggaggaa gttaaattta gattaaggtt ttgggttttt taggagttaa aagggaatat 960
   gttgttttaa gattttagaa tgaagagtta atgtagtttg agtggtttaa attttttgtt 1020
   tttaaatgta agaaatgttt tttattgagt tttggatagg tgtttttgtt ggtttggggt 1080
   ttttaatagg ttatgtttgt tttagatttt agggataaat gtttttttag ttttaattta 1140
   ttttatgttt taagtttttg atttattttt gtttttttag tgttatatta aatgttgttt 1200
   ggggattttt tttttttttt gagtttttat ataagaagtt ttttatttaa gaatttagtt 1260
   ggaatgggtt gtattttaaa agttgttttg taagttagtt atttggattt taagttgtat 1320
   tttattaggg taataatatt ataatgattg tttatttttt tagagtaatt tagaaatgtt 1380
   tatataattt atgttatatt tgaattattt tgagtttttt tatttttgaa ttttttaagt 1440
   ttttttttaa ttttaagtag gttttatggt ttatttaagg tgttttatgt tttttagtta 1500
   tgtttttttt ttattgttgt ttattttttt tttaaatata atataaaata aatttataat 1560
   agttttgtta atttttgaag taaatttaat ttagtaaggt gttgagagga aataaatttt 1620
   attgaatata tttttattga tgtgtattat tgttgaaagt tttttaagtt ttttagaatg 1680
   atttatattt ttaattaagg aggtttaaag gtttagatga ttttttaaag agtggagttt 1740
   tagttaggtt aaggaatgaa atgaaaatat ttggatattt ttttgagaag gtggagttat 1800
   tgtaggtgag agggtgtttt gggttaatgt ttttgtttgt agtttttttt ttttgtatat 1860
   ttttaattta tttatttttt aaaaattttt tattttttta tgtaaagatg gttagagtag 1920
   ggatggtagt tttaggggtt ttttagtttt ttgtgatgtg tagaattaga taaggagtta 1980
   atatagatgg tattattatt tatttttagg atggaaaagt agttgaggtt ggttttttgt 2040
   tttagagatt gaggatttat gtgtattatt ttttagtgaa attagagttt gagtatttgt 2100
   gttgaaattt agatatatta aggttgagat aggtattaga gtgttagtgt gaagatttat 2160
   aaattaaaat agatttattt ttggaataga aattttgtat taatattatg tttgtatttt 2220
   gtttttgtgg ataagtaatg tgggaggttt aggtataatt atagattatg tattgatttg 2280
   ttataagaaa ttgtaggttt tgggatgaga attttttaga atttttgtga taagtatatt 2340
   ttttttttat gtaagtaaat ttatatggga aggagtatgt tgttttttga aagttgtatt 2400
   ggaatgagag gagataatta tttagtttga ggtatgtatg ttagttagag atggttttgt 2460
   tattggaatt ataattatag tttttatttt aaatgtttta tatatattaa tttaattttt 2520
   ttagtaattt ttgaggatat ttattattat tgttattttt attttatagg tgaagaaggg 2580
   gattaagtta ataagttgtt tttgttatgt aattagtaaa tggtagagtt aggatttaaa 2640
   gttagttgtg agagtttaga ttttaaattg ttgtttttat atttttgaaa atttttaaag 2700
   tttgttagtt atagtttttg tgtttatttg tatattgaag ttagtttttt tattgggagg 2760
   gagatattga ggaggaaggt ttttttttta gattatatgg agaagggtgg aattagttga 2820
   tgtttttggt tttttttttt ttttaaggtt ggagaatatt ttgttttttt taagttgata 2880
   tatttttttt ttatagatat gttaataagt tgaaattggt gatttatggt tttattatat 2940
   tttgtttgtt tttttttttt tttttttaaa aatttatggt taggtgtagt ggtttatgtt 3000
   t 3001
<210> 314
   <211> 3001
   <212> DNA
   <213> human
<400> 314
   aggcgtgagt tattgcgttt agttataagt ttttaaaaaa aaaaaaaaaa agataggcga 60
   agtataataa gattataagt tattagtttt aatttgttaa tatgtttata gaaaaaaaat 120
   gtgtcgattt ggggagggta gggtgttttt taattttgga aagaaggaaa ggttagaggt 180
   attagttggt tttatttttt tttatatggt ttaggggaag ggtttttttt tttagtgttt 240
   tttttttagt ggaaaggttg gttttagtgt gtagatgaat ataggggttg tagttaatag 300
   gttttgagga tttttaagag tgtaagagta atagtttaga gtttagattt ttatagttgg 360
   ttttaaattt tgattttgtt atttattaat tatatgatag gggtaattta ttaatttagt 420
   tttttttttt atttgtgaaa tgggaatgac ggtaatagta aatattttta agaattgttg 480
   ggaggattaa gttaatatgt gtaaagtatt tagaataggg gttgtaatta tgattttagt 540
   ggtaaagtta tttttggtta atatatatgt tttaggttga ataattgttt ttttttattt 600
   cgatgtaatt tttaaaaagt aatatatttt tttttatgta ggtttgttta tatagaaaaa 660
   aaatgtgttt attatagaga ttttgagaaa tttttatttt agagtttata attttttatg 720
   gtaaatcgat atataatttg tggttgtgtt tgggtttttt atattgtttg tttatagagg 780
   taagatgtag gtatgatgtt gatgtaggat ttttgtttta ggagtggatt tgttttgatt 840
   tgtaaatttt tatattggta ttttggtgtt tattttaatt ttagtgtatt tggattttaa 900
   cgtagatatt taggttttgg ttttattgaa gaatgatgta cgtgagtttt tagtttttaa 960
   agtaaggggt tagttttagt tgttttttta ttttgagagt ggatggtggt attatttata 1020
   ttagtttttt gtttgatttt gtatattata aagggttgga gagtttttga gattgttatt 1080
   tttgttttgg ttatttttgt atgaaagagt aaagagtttt tggaaaatgg gtgagttaaa 1140
   aatatacgaa aagggagagt tataagtaga agtattaatt tagggtattt ttttatttgt 1200
   agtgatttcg tttttttaag aaaatgttta aatgttttta ttttattttt tgatttggtt 1260
   gggatttcgt tttttaggga gttatttgag tttttaagtt tttttgatta aaagtgtgag 1320
   ttattttaaa agatttagag aatttttaac gatgatatat attaatggga atgtgtttaa 1380
   taagatttat ttttttttaa tattttgttg ggttgggttt attttaggaa ttaatagaat 1440
   tgttgtgggt ttgttttgtg ttgtgtttgg agagagggta gatagtagtg ggaaggggac 1500
   gtaattgaga agtatgaaat attttgagtg gattatgaga tttgtttaga gttaggggaa 1560
   agtttgagag gtttaaggat agaagaattt agggtggttt aggtgtgata tgggttatgt 1620
   gggtattttt ggattgtttt gggaaggtaa ataattatta taatattgtt attttggtaa 1680
   aatgtaattt gaaatttaaa taattaattt gtaaagtaat ttttgaaata taatttattt 1740
   taattgaatt tttaaatggg gggtttttta tatgggaatt taggaagaaa gagagatttt 1800
   taggtagtat ttggtgtggt attgggaaaa taaagatagg ttaaaagttt aaagtataga 1860
   atgagttaga gttggaagaa tatttgtttt tggggtttga ggtaggtatg atttgttgaa 1920
   aattttaggt tagtagagat atttgtttag ggttcgatag aaaatatttt ttgtatttaa 1980
   ggataggagg tttaagttat tcgggttacg ttgatttttt attttagaat tttgggatag 2040
   cgtatttttt tttaattttt ggggagttta gggttttggt ttgagtttag ttttttttag 2100
   ggaattcggg tggaaaggag agataggtaa gaatttcgta gaggtagcga agtttgggtt 2160
   tgaggaagta gagggaggag atttggatta gttataaatg agttgttaag atttagggat 2220
   aattttacgg gttgggtatg aaagggaagt cggtttagga ggatcgagtg agggtatcga 2280
   ggcgggacgt acgtattcgg cgaaggtgta gtgtgggcgt ttacggagtg ggggagttgg 2340
   gaagaaaggg ggttgcggtg ggttggagag aagcgggacg gggaaggcgt tcgggaagtt 2400
   gttttcgcgg cgtcgtgcgt tttggggata gaggagggga ggtttgggcg ggcgggtggc 2460
   gtgtgatcgt cgaggttgcg gcggttggaa taggtgagcg gtcgttgggt tgtgggtttt 2520
   cgttttttat cgttagcgtg ttttttttta cggatttttt ttagcgttga gttgtttcgc 2580
   ggtttttttt gttttggggt tttgtaattg gttgttggtt tttttttttt tttttgtttc 2640
   ggtttttttt ttttttttta ggttgtttta tttcggttag gattaggcga gggggaatta 2700
   gtttgagtgt agttgttaat tttcgtcgag tgcgtttgcg aattttcgga gcggagtttc 2760
   gcgtgtcgtt attttttcgc gggacgtatt tttttcgttt tttcgggttt cgcgtttttt 2820
   tttcggttat ttttttttat tttttttttt ttttttaatt atatggtatt ttaagaatgt 2880
   cggaaagatg gcggtagcgg cggcggcggc ggcggttggg tcggttggcg cgggaggcgg 2940
   tcgggcgtag cggagcgggt tgttggaagt tttggtgcgg gatcgttggt ataaagtttt 3000
   g 3001
<210> 315
   <211> 3001
   <212> DNA
   <213> human
<400> 315
   aggtgtgagt tattgtgttt agttataagt ttttaaaaaa aaaaaaaaaa agataggtga      60
   agtataataa gattataagt tattagtttt aatttgttaa tatgtttata gaaaaaaaat     120
   gtgttgattt ggggagggta gggtgttttt taattttgga aagaaggaaa ggttagaggt     180
   attagttggt tttatttttt tttatatggt ttaggggaag ggtttttttt tttagtgttt     240
   tttttttagt ggaaaggttg gttttagtgt gtagatgaat ataggggttg tagttaatag    300
   gttttgagga tttttaagag tgtaagagta atagtttaga gtttagattt ttatagttgg     360
   ttttaaattt tgattttgtt atttattaat tatatgatag gggtaattta ttaatttagt     420
   tttttttttt atttgtgaaa tgggaatgat ggtaatagta aatattttta agaattgttg     480
   ggaggattaa gttaatatgt gtaaagtatt tagaataggg gttgtaatta tgattttagt     540
   ggtaaagtta tttttggtta atatatatgt tttaggttga ataattgttt ttttttattt    600
   tgatgtaatt tttaaaaagt aatatatttt tttttatgta ggtttgttta tatagaaaaa     660
   aaatgtgttt attatagaga ttttgagaaa tttttatttt agagtttata attttttatg    720
   gtaaattgat atataatttg tggttgtgtt tgggtttttt atattgtttg tttatagagg     780
   taagatgtag gtatgatgtt gatgtaggat ttttgtttta ggagtggatt tgttttgatt     840
   tgtaaatttt tatattggta ttttggtgtt tattttaatt ttagtgtatt tggattttaa     900
   tgtagatatt taggttttgg ttttattgaa gaatgatgta tgtgagtttt tagtttttaa     960
   agtaaggggt tagttttagt tgttttttta ttttgagagt ggatggtggt attatttata    1020
   ttagtttttt gtttgatttt gtatattata aagggttgga gagtttttga gattgttatt    1080
   tttgttttgg ttatttttgt atgaaagagt aaagagtttt tggaaaatgg gtgagttaaa    1140
   aatatatgaa aagggagagt tataagtaga agtattaatt tagggtattt ttttatttgt    1200
   agtgattttg tttttttaag aaaatgttta aatgttttta ttttattttt tgatttggtt   1260
   gggattttgt tttttaggga gttatttgag tttttaagtt tttttgatta aaagtgtgag    1320
   ttattttaaa agatttagag aatttttaat gatgatatat attaatggga atgtgtttaa    1380
   taagatttat ttttttttaa tattttgttg ggttgggttt attttaggaa ttaatagaat    1440
   tgttgtgggt ttgttttgtg ttgtgtttgg agagagggta gatagtagtg ggaaggggat    1500
   gtaattgaga agtatgaaat attttgagtg gattatgaga tttgtttaga gttaggggaa    1560
   agtttgagag gtttaaggat agaagaattt agggtggttt aggtgtgata tgggttatgt    1620
   gggtattttt ggattgtttt gggaaggtaa ataattatta taatattgtt attttggtaa    1680
   aatgtaattt gaaatttaaa taattaattt gtaaagtaat ttttgaaata taatttattt    1740
   taattgaatt tttaaatggg gggtttttta tatgggaatt taggaagaaa gagagatttt    1800
   taggtagtat ttggtgtggt attgggaaaa taaagatagg ttaaaagttt aaagtataga    1860
   atgagttaga gttggaagaa tatttgtttt tggggtttga ggtaggtatg atttgttgaa    1920
   aattttaggt tagtagagat atttgtttag ggtttgatag aaaatatttt ttgtatttaa    1980
   ggataggagg tttaagttat ttgggttatg ttgatttttt attttagaat tttgggatag    2040
   tgtatttttt tttaattttt ggggagttta gggttttggt ttgagtttag ttttttttag   2100
   ggaatttggg tggaaaggag agataggtaa gaattttgta gaggtagtga agtttgggtt    2160
   tgaggaagta gagggaggag atttggatta gttataaatg agttgttaag atttagggat    2220
   aattttatgg gttgggtatg aaagggaagt tggtttagga ggattgagtg agggtattga    2280
   ggtgggatgt atgtatttgg tgaaggtgta gtgtgggtgt ttatggagtg ggggagttgg    2340
   gaagaaaggg ggttgtggtg ggttggagag aagtgggatg gggaaggtgt ttgggaagtt    2400
   gtttttgtgg tgttgtgtgt tttggggata gaggagggga ggtttgggtg ggtgggtggt    2460
   gtgtgattgt tgaggttgtg gtggttggaa taggtgagtg gttgttgggt tgtgggtttt    2520
   tgttttttat tgttagtgtg ttttttttta tggatttttt ttagtgttga gttgttttgt    2580
   ggtttttttt gttttggggt tttgtaattg gttgttggtt tttttttttt tttttgtttt    2640
   ggtttttttt ttttttttta ggttgtttta ttttggttag gattaggtga gggggaatta    2700
   gtttgagtgt agttgttaat ttttgttgag tgtgtttgtg aatttttgga gtggagtttt    2760
   gtgtgttgtt atttttttgt gggatgtatt ttttttgttt ttttgggttt tgtgtttttt  2820
   ttttggttat ttttttttat tttttttttt ttttttaatt atatggtatt ttaagaatgt    2880
   tggaaagatg gtggtagtgg tggtggtggt ggtggttggg ttggttggtg tgggaggtgg    2940
   ttgggtgtag tggagtgggt tgttggaagt tttggtgtgg gattgttggt ataaagtttt    3000
   g                                                                    3001
<210> 316
   <211> 50
   <212> DNA
   <213> human
<400> 316
   cggttgattg tcttgtcata ccttggaggc aacatgagtt ccctggttca 50
<210> 317
   <211> 50
   <212> DNA
   <213> human
<400> 317
   cggttgattg ttttgttata ttttggaggt aatatgagtt ttttggttta 50
<210> 318
   <211> 50
   <212> DNA
   <213> human
<400> 318
   tggttgattg ttttgttata ttttggaggt aatatgagtt ttttggttta 50
<210> 319
   <211>. 50
   <212> DNA
   <213> human
<400> 319
   tgaattaggg aatttatgtt gtttttaagg tatgataaga taattaatcg 50
<210> 320
   <211> 50
   <212> DNA
   <213> human
<400> 320
   tgaattaggg aatttatgtt gtttttaagg tatgataaga taattaattg 50
<210> 321
   <211> 3001
   <212> DNA
   <213> human
<400> 321
   ttttgttgtt gttttgtttt ttacgtattt ccaagtctat ccttcatggt caaaatcaaa 60
   ttatttgtac ttcaaagtca agttaaaaat ttttataatt ctgtaagtat tttgagacct 120
   cagaagcagt gtttaacaga tttttctgac tcaacacttt gtattatact accataacaa 180
   taaatgcaac aattgttctt tacttataaa atttaatcag tgatgatgcc atatatagta 240
   atgttattta attgcaagtt aacatataac taaatgtttt gctgactgta cttggtcagt 300
   ccactaatgt ttaatgagca tttccaggaa gccaaggagt tcgataaatg ccagaagtat 360
   tgaaacaagt agttaactaa ttttatcagc ttagtttagt atgtgaacag ggtttatgga 420
   attacactaa gctcttttct gtcaaaaata tcctcagagc tggtatttac tgacatttgt 480
   ttttatttcg ctaaagcttc tgctttacag taaaaaaaaa attagccggc atatttcatc 540
   acaccactct atttactcag tcaagagaca cagcataccg gtatctatca aatcctgaaa 600
   acaagaagca gatgttctaa aattcttatt caaaggaaca ctctataagt cagtccattt 660
   ttaataatat acatattttc tttacttaat gtgatcatca gtctgttcat taatttatta 720
   cttttttaat actctgggcc agtaattgtt taatattttg ggtgcaaaat ggtaagccaa 780
   atcagacatg atccatgcac ataggaagtc aactgttcaa tggaagaaat agtaatcaaa 840
   taaaatataa cctgcaacca tgaaatatga tattactaaa ttctgaaata tatttctcag 900
   agtttatagt tcaaaaaagt ttgagcttgc tttctttatc ttattgccat ttatttattt 960
   ttaaacaata aaataacttt ataattacat ctaattattt ttcttttttc atcattatag 1020
   cattgtaggg tattagaaat tctcataaaa ggaaaacaac acagaagatg gaatttacag 1080
   atagaaacta cacgttggtc actgagttta ttctattagg ttttccaact cgccctgaac 1140
   tgcagattgt cctgttcctc atgtttctga cattgtatgc tataattctg atagggaaca 1200
   ttggattgat gctgttgatc aggattgatc ctcaccttca aacccccatg tattttttcc 1260
   ttagcaacct atcatttgta gacctttgct atttctcaga cattgttccc aaaatgctgg 1320
   tcaatttcct ctcggagaac aaatctattt cctattatgg gtgtgccctg cagttttatt 1380
   ttttctgtac ttttgcagat acagaatcct tcatcctggc cgccatggcc tatgatcgct 1440
   atgtcgccat ctgtaaccct ttattgtaca cagttgtgat gtctaggggc atctgtatgc 1500
   ggttgattgt cttgtcatac cttggaggca acatgagttc cctggttcac acatcctttg 1560
   cctttattct gaaatattgt gacaaaaatg ttattaatca ttttttctgt gacctccctc 1620
   ccctgcttaa actatcctgc actgacacaa caattaatga gtggctcctc tccacatacg 1680
   gcagctcagt ggaaatcatt tgttttatca tcatcatcat ctcctacttt ttcattcttc 1740
   tctcagtctt aaagatccgc tctttcagtg ggaggaagaa gaccttttct acatgcgcct 1800
   ctcacctgac ttcagtgacg atctaccaag ggactctcct ctttatttac tcacggccca 1860
   gctacctgta ttctccaaac actgataaaa ttatctcagt gttctacacc attttcattc 1920
   cagtgctgaa tccgttgatt tatagtttga gaaataaaga tgtaaaggat gcagctgaga 1980
   aagttctaag atcaaaggta gattcttcat gagataaagc atctcaagat acatctaatt 2040
   tctcaatcac atactgtaat tgggaactta caacaacctt acctgtacac acacacaacc 2100
   tctcacacta gggattttat caagcacttt gtttaatgca gccaaatgaa tttcatagtc 2160
   ctaaaatatt tttaaaatca tttaaaagtt tcaaagttcc tgctaaatgc tagttatact 2220
   acttttcagt tattactagc ttgagattta tgctaaaatc aatttcatca agccatctta 2280
   agggatatag taaatatttc agaaaagtat gcatgcatgt acatgtgcgt atctatctgt 2340
   agaatatctc agttataagt gtagattact ggataactgg ataagaaaat aagtcagatt 2400
   gctttgattt atatgccaga tctatacttg gctagtgagt ggcatattga atgagttgct 2460
   taatttttct atgcctcatt tatctgtaaa atgggatgaa tattgcacat atgctattga 2520
   ttgtttgatg gtgaacagag ttaatacagt taagagctga gaatagtgcc tggacattat 2580
   atacacttac atgtttgtta taagatatgt ttttataata tctcacacta attaaaatgc 2640
   atgtaataaa ataagatgat agaggctaga atgttagaaa agcaatcata aaaagccaaa 2700
   taatattttt taaaatgcag gtgataacat gttgataact ttctacatcc aatgtatcat 2760
   aaagcaattt ggaaacattt tcttagtggc ttgagcaata taggttaatt gcaatgcaaa 2820
   atatttgttt tactatctct tgtttttaaa tacaccaatg tgttactgaa tgtgaggaac 2880
   gttctttgct ggaatctcag aaaaagaata ctatctctaa tttattacac tatcgtaatt 2940
   gtatcctcat agggagatca atccagtact gtggtagaaa accatgcatg aaatgtgtgt 3000
   t 3001
<210> 322
   <211> 3001
   <212> DNA
   <213> human
<400> 322
   ttttgttgtt gttttgtttt ttacgtattt ttaagtttat tttttatggt taaaattaaa     60
   ttatttgtat tttaaagtta agttaaaaat ttttataatt ttgtaagtat tttgagattt    120
   tagaagtagt gtttaataga tttttttgat ttaatatttt gtattatatt attataataa    180
   taaatgtaat aattgttttt tatttataaa atttaattag tgatgatgtt atatatagta    240
   atgttattta attgtaagtt aatatataat taaatgtttt gttgattgta tttggttagt    300
   ttattaatgt ttaatgagta tttttaggaa gttaaggagt tcgataaatg ttagaagtat    360
   tgaaataagt agttaattaa ttttattagt ttagtttagt atgtgaatag ggtttatgga    420
   attatattaa gttttttttt gttaaaaata tttttagagt tggtatttat tgatatttgt    480
   ttttatttcg ttaaagtttt tgttttatag taaaaaaaaa attagtcggt atattttatt    540
   atattatttt atttatttag ttaagagata tagtatatcg gtatttatta aattttgaaa    600
   ataagaagta gatgttttaa aatttttatt taaaggaata ttttataagt tagtttattt    660
   ttaataatat atatattttt tttatttaat gtgattatta gtttgtttat taatttatta    720
   tttttttaat attttgggtt agtaattgtt taatattttg ggtgtaaaat ggtaagttaa     780
   attagatatg atttatgtat ataggaagtt aattgtttaa tggaagaaat agtaattaaa    840
   taaaatataa tttgtaatta tgaaatatga tattattaaa ttttgaaata tattttttag    900
   agtttatagt ttaaaaaagt ttgagtttgt tttttttatt ttattgttat ttatttattt    960
   ttaaataata aaataatttt ataattatat ttaattattt tttttttttt attattatag   1020
   tattgtaggg tattagaaat ttttataaaa ggaaaataat atagaagatg gaatttatag   1080
   atagaaatta tacgttggtt attgagttta ttttattagg ttttttaatt cgttttgaat   1140
   tgtagattgt tttgtttttt atgtttttga tattgtatgt tataattttg atagggaata   1200
   ttggattgat gttgttgatt aggattgatt tttattttta aatttttatg tatttttttt  1260
   ttagtaattt attatttgta gatttttgtt attttttaga tattgttttt aaaatgttgg   1320
   ttaatttttt ttcggagaat aaatttattt tttattatgg gtgtgttttg tagttttatt   1380
   ttttttgtat ttttgtagat atagaatttt ttattttggt cgttatggtt tatgatcgtt    1440
   atgtcgttat ttgtaatttt ttattgtata tagttgtgat gtttaggggt atttgtatgc   1500
   ggttgattgt tttgttatat tttggaggta atatgagttt tttggtttat atattttttg   1560
   tttttatttt gaaatattgt gataaaaatg ttattaatta ttttttttgt gatttttttt   1620
   ttttgtttaa attattttgt attgatataa taattaatga gtggtttttt tttatatacg   1680
   gtagtttagt ggaaattatt tgttttatta ttattattat tttttatttt tttatttttt   1740
   ttttagtttt aaagattcgt ttttttagtg ggaggaagaa gatttttttt atatgcgttt  1800
   tttatttgat tttagtgacg atttattaag ggattttttt ttttatttat ttacggttta   1860
   gttatttgta ttttttaaat attgataaaa ttattttagt gttttatatt attttatttt  1920
   tagtgttgaa ttcgttgatt tatagtttga gaaataaaga tgtaaaggat gtagttgaga   1980
   aagttttaag attaaaggta gattttttat gagataaagt attttaagat atatttaatt  2040
   ttttaattat atattgtaat tgggaattta taataatttt atttgtatat atatataatt   2100
   ttttatatta gggattttat taagtatttt gtttaatgta gttaaatgaa ttttatagtt   2160
   ttaaaatatt tttaaaatta tttaaaagtt ttaaagtttt tgttaaatgt tagttatatt  2220
   attttttagt tattattagt ttgagattta tgttaaaatt aattttatta agttatttta   2280
   agggatatag taaatatttt agaaaagtat gtatgtatgt atatgtgcgt atttatttgt   2340
   agaatatttt agttataagt gtagattatt ggataattgg ataagaaaat aagttagatt   2400
   gttttgattt atatgttaga tttatatttg gttagtgagt ggtatattga atgagttgtt   2460
   taattttttt atgttttatt tatttgtaaa atgggatgaa tattgtatat atgttattga   2520
   ttgtttgatg gtgaatagag ttaatatagt taagagttga gaatagtgtt tggatattat   2580
   atatatttat atgtttgtta taagatatgt ttttataata ttttatatta attaaaatgt   2640
   atgtaataaa ataagatgat agaggttaga atgttagaaa agtaattata aaaagttaaa   2700
   taatattttt taaaatgtag gtgataatat gttgataatt ttttatattt aatgtattat   2760
   aaagtaattt ggaaatattt ttttagtggt ttgagtaata taggttaatt gtaatgtaaa   2820
   atatttgttt tattattttt tgtttttaaa tatattaatg tgttattgaa tgtgaggaac   2880
   gttttttgtt ggaattttag aaaaagaata ttatttttaa tttattatat tatcgtaatt  2940
   gtatttttat agggagatta atttagtatt gtggtagaaa attatgtatg aaatgtgtgt   3000
   t                                                                   3001
<210> 323
   <211> 3001
   <212> DNA
   <213> human
<400> 323
   ttttgttgtt gttttgtttt ttatgtattt ttaagtttat tttttatggt taaaattaaa      60
   ttatttgtat tttaaagtta agttaaaaat ttttataatt ttgtaagtat tttgagattt     120
   tagaagtagt gtttaataga tttttttgat ttaatatttt gtattatatt attataataa     180
   taaatgtaat aattgttttt tatttataaa atttaattag tgatgatgtt atatatagta     240
   atgttattta attgtaagtt aatatataat taaatgtttt gttgattgta tttggttagt     300
   ttattaatgt ttaatgagta tttttaggaa gttaaggagt ttgataaatg ttagaagtat     360
   tgaaataagt agttaattaa ttttattagt ttagtttagt atgtgaatag ggtttatgga     420
   attatattaa gttttttttt gttaaaaata tttttagagt tggtatttat tgatatttgt     480
   ttttattttg ttaaagtttt tgttttatag taaaaaaaaa attagttggt atattttatt     540
   atattatttt atttatttag ttaagagata tagtatattg gtatttatta aattttgaaa     600
   ataagaagta gatgttttaa aatttttatt taaaggaata ttttataagt tagtttattt     660
   ttaataatat atatattttt tttatttaat gtgattatta gtttgtttat taatttatta     720
   tttttttaat attttgggtt agtaattgtt taatattttg ggtgtaaaat ggtaagttaa     780
   attagatatg atttatgtat ataggaagtt aattgtttaa tggaagaaat agtaattaaa     840
   taaaatataa tttgtaatta tgaaatatga tattattaaa ttttgaaata tattttttag    900
   agtttatagt ttaaaaaagt ttgagtttgt tttttttatt ttattgttat ttatttattt   960
   ttaaataata aaataatttt ataattatat ttaattattt tttttttttt attattatag    1020
   tattgtaggg tattagaaat ttttataaaa ggaaaataat atagaagatg gaatttatag    1080
   atagaaatta tatgttggtt attgagttta ttttattagg ttttttaatt tgttttgaat    1140
   tgtagattgt tttgtttttt atgtttttga tattgtatgt tataattttg atagggaata    1200
   ttggattgat gttgttgatt aggattgatt tttattttta aatttttatg tatttttttt  1260
   ttagtaattt attatttgta gatttttgtt attttttaga tattgttttt aaaatgttgg    1320
   ttaatttttt tttggagaat aaatttattt tttattatgg gtgtgttttg tagttttatt    1380
   ttttttgtat ttttgtagat atagaatttt ttattttggt tgttatggtt tatgattgtt    1440
   atgttgttat ttgtaatttt ttattgtata tagttgtgat gtttaggggt atttgtatgt    1500
   ggttgattgt tttgttatat tttggaggta atatgagttt tttggtttat atattttttg   1560
   tttttatttt gaaatattgt gataaaaatg ttattaatta ttttttttgt gatttttttt    1620
   ttttgtttaa attattttgt attgatataa taattaatga gtggtttttt tttatatatg    1680
   gtagtttagt ggaaattatt tgttttatta ttattattat tttttatttt tttatttttt    1740
   ttttagtttt aaagatttgt ttttttagtg ggaggaagaa gatttttttt atatgtgttt    1800
   tttatttgat tttagtgatg atttattaag ggattttttt ttttatttat ttatggttta    1860
   gttatttgta ttttttaaat attgataaaa ttattttagt gttttatatt atttttattt    1920
   tagtgttgaa tttgttgatt tatagtttga gaaataaaga tgtaaaggat gtagttgaga    1980
   aagttttaag attaaaggta gattttttat gagataaagt attttaagat atatttaatt   2040
   ttttaattat atattgtaat tgggaattta taataatttt atttgtatat atatataatt    2100
   ttttatatta gggattttat taagtatttt gtttaatgta gttaaatgaa ttttatagtt    2160
   ttaaaatatt tttaaaatta tttaaaagtt ttaaagtttt tgttaaatgt tagttatatt    2220
   attttttagt tattattagt ttgagattta tgttaaaatt aattttatta agttatttta   2280
   agggatatag taaatatttt agaaaagtat gtatgtatgt atatgtgtgt atttatttgt    2340
   agaatatttt agttataagt gtagattatt ggataattgg ataagaaaat aagttagatt    2400
   gttttgattt atatgttaga tttatatttg gttagtgagt ggtatattga atgagttgtt    2460
   taattttttt atgttttatt tatttgtaaa atgggatgaa tattgtatat atgttattga    2520
   ttgtttgatg gtgaatagag ttaatatagt taagagttga gaatagtgtt tggatattat    2580
   atatatttat atgtttgtta taagatatgt ttttataata ttttatatta attaaaatgt    2640
   atgtaataaa ataagatgat agaggttaga atgttagaaa agtaattata aaaagttaaa    2700
   taatattttt taaaatgtag gtgataatat gttgataatt ttttatattt aatgtattat    2760
   aaagtaattt ggaaatattt ttttagtggt ttgagtaata taggttaatt gtaatgtaaa    2820
   atatttgttt tattattttt tgtttttaaa tatattaatg tgttattgaa tgtgaggaat    2880
   gttttttgtt ggaattttag aaaaagaata ttatttttaa tttattatat tattgtaatt   2940
   gtatttttat agggagatta atttagtatt gtggtagaaa attatgtatg aaatgtgtgt    3000
   t                                                                    3001
<210> 324
   <211> 3001
   <212> DNA
   <213> human
<400> 324
   aatatatatt ttatgtatgg ttttttatta tagtattgga ttgatttttt tatgaggata 60
   taattacgat agtgtaataa attagagata gtattttttt tttgagattt tagtaaagaa 120
   cgttttttat atttagtaat atattggtgt atttaaaaat aagagatagt aaaataaata 180
   ttttgtattg taattaattt atattgttta agttattaag aaaatgtttt taaattgttt 240
   tatgatatat tggatgtaga aagttattaa tatgttatta tttgtatttt aaaaaatatt 300
   atttggtttt ttatgattgt ttttttaata ttttagtttt tattatttta ttttattata 360
   tgtattttaa ttagtgtgag atattataaa aatatatttt ataataaata tgtaagtgta 420
   tataatgttt aggtattatt tttagttttt aattgtatta attttgttta ttattaaata 480
   attaatagta tatgtgtaat atttatttta ttttatagat aaatgaggta tagaaaaatt 540
   aagtaattta tttaatatgt tatttattag ttaagtatag atttggtata taaattaaag 600
   taatttgatt tattttttta tttagttatt tagtaattta tatttataat tgagatattt 660
   tatagataga tacgtatatg tatatgtatg tatatttttt tgaaatattt attatatttt 720
   ttaagatggt ttgatgaaat tgattttagt ataaatttta agttagtaat aattgaaaag 780
   tagtataatt agtatttagt aggaattttg aaatttttaa atgattttaa aaatatttta 840
   ggattatgaa atttatttgg ttgtattaaa taaagtgttt gataaaattt ttagtgtgag 900
   aggttgtgtg tgtgtatagg taaggttgtt gtaagttttt aattatagta tgtgattgag 960
   aaattagatg tattttgaga tgttttattt tatgaagaat ttatttttga ttttagaatt 1020
   tttttagttg tattttttat atttttattt tttaaattat aaattaacgg atttagtatt 1080
   ggaatgaaaa tggtgtagaa tattgagata attttattag tgtttggaga atataggtag 1140
   ttgggtcgtg agtaaataaa gaggagagtt ttttggtaga tcgttattga agttaggtga 1200
   gaggcgtatg tagaaaaggt tttttttttt ttattgaaag agcggatttt taagattgag 1260
   agaagaatga aaaagtagga gatgatgatg atgataaaat aaatgatttt tattgagttg 1320
   tcgtatgtgg agaggagtta tttattaatt gttgtgttag tgtaggatag tttaagtagg 1380
   ggagggaggt tatagaaaaa atgattaata atatttttgt tataatattt tagaataaag 1440
   gtaaaggatg tgtgaattag ggaatttatg ttgtttttaa ggtatgataa gataattaat 1500
   cgtatataga tgtttttaga tattataatt gtgtataata aagggttata gatggcgata 1560
   tagcgattat aggttatggc ggttaggatg aaggattttg tatttgtaaa agtatagaaa 1620
   aaataaaatt gtagggtata tttataatag gaaatagatt tgtttttcga gaggaaattg 1680
   attagtattt tgggaataat gtttgagaaa tagtaaaggt ttataaatga taggttgtta 1740
   aggaaaaaat atatgggggt ttgaaggtga ggattaattt tgattaatag tattaattta 1800
   atgtttttta ttagaattat agtatataat gttagaaata tgaggaatag gataatttgt 1860
   agtttagggc gagttggaaa atttaataga ataaatttag tgattaacgt gtagttttta 1920
   tttgtaaatt ttattttttg tgttgttttt tttttatgag aatttttaat attttataat 1980
   gttataatga tgaaaaaaga aaaataatta gatgtaatta taaagttatt ttattgttta 2040
   aaaataaata aatggtaata agataaagaa agtaagttta aatttttttg aattataaat 2100
   tttgagaaat atattttaga atttagtaat attatatttt atggttgtag gttatatttt 2160
   atttgattat tatttttttt attgaatagt tgatttttta tgtgtatgga ttatgtttga 2220
   tttggtttat tattttgtat ttaaaatatt aaataattat tggtttagag tattaaaaaa 2280
   gtaataaatt aatgaataga ttgatgatta tattaagtaa agaaaatatg tatattatta 2340
   aaaatggatt gatttataga gtgttttttt gaataagaat tttagaatat ttgttttttg 2400
   tttttaggat ttgatagata tcggtatgtt gtgttttttg attgagtaaa tagagtggtg 2460
   tgatgaaata tgtcggttaa tttttttttt attgtaaagt agaagtttta gcgaaataaa 2520
   aataaatgtt agtaaatatt agttttgagg atatttttga tagaaaagag tttagtgtaa 2580
   ttttataaat tttgtttata tattaaatta agttgataaa attagttaat tatttgtttt 2640
   aatatttttg gtatttatcg aattttttgg ttttttggaa atgtttatta aatattagtg 2700
   gattgattaa gtatagttag taaaatattt agttatatgt taatttgtaa ttaaataata 2760
   ttattatata tggtattatt attgattaaa ttttataagt aaagaataat tgttgtattt 2820
   attgttatgg tagtataata taaagtgttg agttagaaaa atttgttaaa tattgttttt 2880
   gaggttttaa aatatttata gaattataaa aatttttaat ttgattttga agtataaata 2940
   atttgatttt gattatgaag gatagatttg gaaatacgta aaaaataaaa taataataaa 3000
   a 3001
<210> 325
   <211> 3001
   <212> DNA
   <213> human
<400> 325
   aatatatatt ttatgtatgg ttttttatta tagtattgga ttgatttttt tatgaggata 60
   taattatgat agtgtaataa attagagata gtattttttt tttgagattt tagtaaagaa 120
   tgttttttat atttagtaat atattggtgt atttaaaaat aagagatagt aaaataaata 180
   ttttgtattg taattaattt atattgttta agttattaag aaaatgtttt taaattgttt 240
   tatgatatat tggatgtaga aagttattaa tatgttatta tttgtatttt aaaaaatatt 300
   atttggtttt ttatgattgt ttttttaata ttttagtttt tattatttta ttttattata 360
   tgtattttaa ttagtgtgag atattataaa aatatatttt ataataaata tgtaagtgta 420
   tataatgttt aggtattatt tttagttttt aattgtatta attttgttta ttattaaata 480
   attaatagta tatgtgtaat atttatttta ttttatagat aaatgaggta tagaaaaatt 540
   aagtaattta tttaatatgt tatttattag ttaagtatag atttggtata taaattaaag 600
   taatttgatt tattttttta tttagttatt tagtaattta tatttataat tgagatattt 660
   tatagataga tatgtatatg tatatgtatg tatatttttt tgaaatattt attatatttt 720
   ttaagatggt ttgatgaaat tgattttagt ataaatttta agttagtaat aattgaaaag 780
   tagtataatt agtatttagt aggaattttg aaatttttaa atgattttaa aaatatttta 840
   ggattatgaa atttatttgg ttgtattaaa taaagtgttt gataaaattt ttagtgtgag 900
   aggttgtgtg tgtgtatagg taaggttgtt gtaagttttt aattatagta tgtgattgag 960
   aaattagatg tattttgaga tgttttattt tatgaagaat ttatttttga ttttagaatt 1020
   tttttagttg tattttttat atttttattt tttaaattat aaattaatgg atttagtatt 1080
   ggaatgaaaa tggtgtagaa tattgagata attttattag tgtttggaga atataggtag 1140
   ttgggttgtg agtaaataaa gaggagagtt ttttggtaga ttgttattga agttaggtga 1200
   gaggtgtatg tagaaaaggt tttttttttt ttattgaaag agtggatttt taagattgag 1260
   agaagaatga aaaagtagga gatgatgatg atgataaaat aaatgatttt tattgagttg 1320
   ttgtatgtgg agaggagtta tttattaatt gttgtgttag tgtaggatag tttaagtagg 1380
   ggagggaggt tatagaaaaa atgattaata atatttttgt tataatattt tagaataaag 1440
   gtaaaggatg tgtgaattag ggaatttatg ttgtttttaa ggtatgataa gataattaat 1500
   tgtatataga tgtttttaga tattataatt gtgtataata aagggttata gatggtgata 1560
   tagtgattat aggttatggt ggttaggatg aaggattttg tatttgtaaa agtatagaaa 1620
   aaataaaatt gtagggtata tttataatag gaaatagatt tgttttttga gaggaaattg 1680
   attagtattt tgggaataat gtttgagaaa tagtaaaggt ttataaatga taggttgtta 1740
   aggaaaaaat atatgggggt ttgaaggtga ggattaattt tgattaatag tattaattta 1800
   atgtttttta ttagaattat agtatataat gttagaaata tgaggaatag gataatttgt 1860
   agtttagggt gagttggaaa atttaataga ataaatttag tgattaatgt gtagttttta 1920
   tttgtaaatt ttattttttg tgttgttttt tttttatgag aatttttaat attttataat 1980
   gttataatga tgaaaaaaga aaaataatta gatgtaatta taaagttatt ttattgttta 2040
   aaaataaata aatggtaata agataaagaa agtaagttta aatttttttg aattataaat 2100
   tttgagaaat atattttaga atttagtaat attatatttt atggttgtag gttatatttt 2160
   atttgattat tatttttttt attgaatagt tgatttttta tgtgtatgga ttatgtttga 2220
   tttggtttat tattttgtat ttaaaatatt aaataattat tggtttagag tattaaaaaa 2280
   gtaataaatt aatgaataga ttgatgatta tattaagtaa agaaaatatg tatattatta 2340
   aaaatggatt gatttataga gtgttttttt gaataagaat tttagaatat ttgttttttg 2400
   tttttaggat ttgatagata ttggtatgtt gtgttttttg attgagtaaa tagagtggtg 2460
   tgatgaaata tgttggttaa tttttttttt attgtaaagt agaagtttta gtgaaataaa 2520
   aataaatgtt agtaaatatt agttttgagg atatttttga tagaaaagag tttagtgtaa 2580
   ttttataaat tttgtttata tattaaatta agttgataaa attagttaat tatttgtttt 2640
   aatatttttg gtatttattg aattttttgg ttttttggaa atgtttatta aatattagtg 2700
   gattgattaa gtatagttag taaaatattt agttatatgt taatttgtaa ttaaataata 2760
   ttattatata tggtattatt attgattaaa ttttataagt aaagaataat tgttgtattt 2820
   attgttatgg tagtataata taaagtgttg agttagaaaa atttgttaaa tattgttttt 2880
   gaggttttaa aatatttata gaattataaa aatttttaat ttgattttga agtataaata 2940
   atttgatttt gattatgaag gatagatttg gaaatatgta aaaaataaaa taataataaa 3000
   a 3001
<210> 326
   <211> 50
   <212> DNA
   <213> human
<400> 326
   tgagcaaggc ggagtgtggc ctctgggctt ccttgtgcag cagcattccg 50
<210> 327
   <211> 50
   <212> DNA
   <213> human
<400> 327
   tgagtaaggc ggagtgtggt ttttgggttt ttttgtgtag tagtatttcg 50
<210> 328
   <211> 50
   <212> DNA
   <213> human
<400> 328
   tgagtaaggt ggagtgtggt ttttgggttt ttttgtgtag tagtattttg 50
<210> 329
   <211> 50
   <212> DNA
   <213> human
<400> 329
   cggaatgttg ttgtataagg aagtttagag gttatatttc gttttgttta 50
<210> 330
   <211> 50
   <212> DNA
   <213> human
<400> 330
   tggaatgttg ttgtataagg aagtttagag gttatatttt gttttgttta 50
<210> 331
   <211> 3001
   <212> DNA
   <213> human
<400> 331
   gccctctaag gcctccagca tccagacaac ggtagattta caactgtcca ctagttgggt      60
   caggtcagcc aaatcaacta tctgaaggga caagaagggt ttgagagtga ggagtcatgc     120
   tgggtccaag atgacctcca ccgcactcca cacccaaagc caagcaaccc caaagctgtt     180
   tgagggcagt ggagcaaccc caaagctgtt tgagggcagt ggagcaaccc caaaccctat     240
   gagcctagtc caggtacctt gattcccaga cagtacctga cagccttggg caaagtcata     300
   aaccaccaca gtgtagaggt gaagtccctg aaagtacatc tgaagaaaag actgctgttg     360
   cttggaggag cccccataca cctgaggaag gaagcggaag gttggtatga ggagtagaga     420
   taaagagggc tccaagctga agactgaccc ggagtcctgg agagctgaca gagcccaggg     480
   agcacgagga aggacatcca gaagggccac tgccctctca ggatagacta ggctgtttgc     540
   tggctcgcta ggcactgtcc agagagcagc ttaacaagga gggagccagg acctccagat     600
   cttgccaaat accctaggct ggtagtgagt ccacagacca cgtccccacc ctacctccag     660
   gtccttaact cacaccatca tcccgcagct gctgcagaag agagcagtac cctccaagaa     720
   aagcaaagag gctcagaatg gcatcaagtc tatagcgcct cttggtgcct cgttccaggc     780
   ctgaaaggaa gaactggcag ctctcataca atgcccgaag tgggggtgat ggtgcctcca     840
   tactcttgct caggacagct gatgccttga tcagaagctt ggccactgcc tgaggtcctt     900
   cctccccaac ctgcagcagc ttaaccccca gctgacatag ctgaaggcta ggggctagat     960
   tggtgttcct taggtaactg tgagccttct ccactgcgct gatggctttg tcatggtggc    1020
   ggctccagca aaagcgacgg cagtgttcca aggtgagctc caagaggcag agggccctct    1080
   ggggagaaag aagcccagaa gagctccctc tctcacccac caaggctctg atcacgtgcc    1140
   tggagagcag gtcatctagg aaatcagcat ctgcttcatt tagaccatgg ccactggcat    1200
   caaatagctg atgggcagct accgctcgac aggctgttgg agaagcaaag tgaggaacct    1260
   cacaaggggt actttcatcc tccaagagta ctaggaagct caaggccttc agccgagctg    1320
   caaatgcagc tcgccgttcc aacagggctt ctgccccctt ccaaagcaga gaaaagctgc 1380
   cccgagccac ggcctcatag tcctggggag cagcctcgcg agagcactgc accaagcagg 1440
   catggagggg ctgagcaagg cggagtgtgg cctctgggct tccttgtgca gcagcattcc 1500
   gcagtaagac aaagagaatt cgttccaggt agaggggagg acgctgtggg gtagacacta 1560
   agtagccatc acaggccagc tctgccagct ccagcaggct ccccagatgc ctagggcaag 1620
   ctagcttagc agtcagctgc tggttgcaag ccctcaggat ggcatcacaa gcttgtctcc 1680
   tctcagcatc agatcggctg ctgggaaaac cagctggagg gttggacagg aactcctagg 1740
   agtagagaaa gatatgaatg gggctagcaa aggcaggaaa gaggtagaga aagtaactag 1800
   aaatagggtt actcagagag ggtcgcctac ttatccatct cttcaaaagg caaacacaaa 1860
   acagctcagt ttggaaagcc aggtgtatcc cgagccaggc agcaccccca ccttcaaggc 1920
   gggcagcaac tcttcagcct ccttctggct gcttagcaga gtcccaaagt tgactctttt 1980
   gaagctcctc atgacaccgg agagctaggg aaaaggagac caattagaga agattgttaa 2040
   tgaagctaac tggaggaagg gaagagggct gaaggtcgtg gaaggaggca aagaatcaca 2100
   tcagaaattt catcggggag aaaacccacg ccaggacgcc gtgctcccgt tataaaagga 2160
   cgattccgct cttccttcct ccgggcagat ccccgaccgc actaccacac cactcgccgc 2220
   gctaccccag aggcctcggc ccagcccttc agcggcccgg ggcagtccca aggcctcccg 2280
   acactccctc caggagccac ccacgcctct cctcgcgtca gcgctgggga tccccttcac 2340
   ttcagcacgt acccacgctc acgagtccct tccggcctta ccgccccaga gctcgccctc 2400
   tttcctaggt acaggactta accgcctgac cagtactccg cgaggatcca aaatgtaacc 2460
   ttcgttttcc gcgccagacc ctcctttcaa atatccgcgg ctgccaatca gcgggcgcgg 2520
   cgcaacgccg ggcgcgcggc acgctgggag ttgtagttta cccgccgcgc tgcgcgctcc 2580
   cgcggcgccc cggagggtct tgctgctctg cgcgttaggg cctcttgggg gtgcgggttt 2640
   cccccttttt tccagtcaga gctcagtttc tcagctacct gtcgcttagt cgctatcggt 2700
   aatgccaccc cttttcctcc tttatcttca gagctcaggc aagagtttaa gtttcaagag 2760
   aagagaaata acaaaaagaa cttaggaact cacgcggttt gggttactag aaaaaaaggg 2820
   cttagacttc tacgaatttt ttttttgata gggtcttgct ctgacacaca ggctggagtg 2880
   cagtggcgtg atcacagctc actgcagctt caaactcccg ggcttaagtg atcctcccac 2940
   ctcagcctcc caaagtgctg actacgggcg tgcaccgcca cgcccaactt cctacgaaat 3000
   t 3001
<210> 332
   <211> 3001
   <212> DNA
   <213> human
<400> 332
   gttttttaag gtttttagta tttagataac ggtagattta taattgttta ttagttgggt 60
   taggttagtt aaattaatta tttgaaggga taagaagggt ttgagagtga ggagttatgt 120
   tgggtttaag atgattttta tcgtatttta tatttaaagt taagtaattt taaagttgtt 180
   tgagggtagt ggagtaattt taaagttgtt tgagggtagt ggagtaattt taaattttat 240
   gagtttagtt taggtatttt gatttttaga tagtatttga tagttttggg taaagttata 300
   aattattata gtgtagaggt gaagtttttg aaagtatatt tgaagaaaag attgttgttg 360
   tttggaggag tttttatata tttgaggaag gaagcggaag gttggtatga ggagtagaga 420
   taaagagggt tttaagttga agattgattc ggagttttgg agagttgata gagtttaggg 480
   agtacgagga aggatattta gaagggttat tgttttttta ggatagatta ggttgtttgt 540
   tggttcgtta ggtattgttt agagagtagt ttaataagga gggagttagg atttttagat 600
   tttgttaaat attttaggtt ggtagtgagt ttatagatta cgtttttatt ttatttttag 660
   gtttttaatt tatattatta tttcgtagtt gttgtagaag agagtagtat tttttaagaa 720
   aagtaaagag gtttagaatg gtattaagtt tatagcgttt tttggtgttt cgttttaggt 780
   ttgaaaggaa gaattggtag tttttatata atgttcgaag tgggggtgat ggtgttttta 840
   tatttttgtt taggatagtt gatgttttga ttagaagttt ggttattgtt tgaggttttt 900
   tttttttaat ttgtagtagt ttaattttta gttgatatag ttgaaggtta ggggttagat 960
   tggtgttttt taggtaattg tgagtttttt ttattgcgtt gatggttttg ttatggtggc 1020
   ggttttagta aaagcgacgg tagtgtttta aggtgagttt taagaggtag agggtttttt 1080
   ggggagaaag aagtttagaa gagttttttt ttttatttat taaggttttg attacgtgtt 1140
   tggagagtag gttatttagg aaattagtat ttgttttatt tagattatgg ttattggtat 1200
   taaatagttg atgggtagtt atcgttcgat aggttgttgg agaagtaaag tgaggaattt 1260
   tataaggggt atttttattt tttaagagta ttaggaagtt taaggttttt agtcgagttg 1320
   taaatgtagt tcgtcgtttt aatagggttt ttgttttttt ttaaagtaga gaaaagttgt 1380
   ttcgagttac ggttttatag ttttggggag tagtttcgcg agagtattgt attaagtagg 1440
   tatggagggg ttgagtaagg cggagtgtgg tttttgggtt tttttgtgta gtagtatttc 1500
   gtagtaagat aaagagaatt cgttttaggt agaggggagg acgttgtggg gtagatatta 1560
   agtagttatt ataggttagt tttgttagtt ttagtaggtt ttttagatgt ttagggtaag 1620
   ttagtttagt agttagttgt tggttgtaag tttttaggat ggtattataa gtttgttttt 1680
   ttttagtatt agatcggttg ttgggaaaat tagttggagg gttggatagg aatttttagg 1740
   agtagagaaa gatatgaatg gggttagtaa aggtaggaaa gaggtagaga aagtaattag 1800
   aaatagggtt atttagagag ggtcgtttat ttatttattt ttttaaaagg taaatataaa 1860
   atagtttagt ttggaaagtt aggtgtattt cgagttaggt agtattttta tttttaaggc 1920
   gggtagtaat tttttagttt ttttttggtt gtttagtaga gttttaaagt tgattttttt 1980
   gaagtttttt atgatatcgg agagttaggg aaaaggagat taattagaga agattgttaa 2040
   tgaagttaat tggaggaagg gaagagggtt gaaggtcgtg gaaggaggta aagaattata 2100
   ttagaaattt tatcggggag aaaatttacg ttaggacgtc gtgttttcgt tataaaagga 2160
   cgatttcgtt tttttttttt tcgggtagat tttcgatcgt attattatat tattcgtcgc 2220
   gttattttag aggtttcggt ttagtttttt agcggttcgg ggtagtttta aggtttttcg 2280
   atattttttt taggagttat ttacgttttt tttcgcgtta gcgttgggga ttttttttat 2340
   tttagtacgt atttacgttt acgagttttt ttcggtttta tcgttttaga gttcgttttt 2400
   ttttttaggt ataggattta atcgtttgat tagtatttcg cgaggattta aaatgtaatt 2460
   ttcgtttttc gcgttagatt ttttttttaa atattcgcgg ttgttaatta gcgggcgcgg 2520
   cgtaacgtcg ggcgcgcggt acgttgggag ttgtagttta ttcgtcgcgt tgcgcgtttt 2580
   cgcggcgttt cggagggttt tgttgttttg cgcgttaggg ttttttgggg gtgcgggttt 2640
   tttttttttt tttagttaga gtttagtttt ttagttattt gtcgtttagt cgttatcggt 2700
   aatgttattt tttttttttt tttattttta gagtttaggt aagagtttaa gttttaagag 2760
   aagagaaata ataaaaagaa tttaggaatt tacgcggttt gggttattag aaaaaaaggg 2820
   tttagatttt tacgaatttt ttttttgata gggttttgtt ttgatatata ggttggagtg 2880
   tagtggcgtg attatagttt attgtagttt taaattttcg ggtttaagtg atttttttat 2940
   tttagttttt taaagtgttg attacgggcg tgtatcgtta cgtttaattt tttacgaaat 3000
   t 3001

<210> 333
   <211> 3001
   <212> DNA
   <213> human
<400> 333
   gttttttaag gtttttagta tttagataat ggtagattta taattgttta ttagttgggt      60
   taggttagtt aaattaatta tttgaaggga taagaagggt ttgagagtga ggagttatgt    120
   tgggtttaag atgattttta ttgtatttta tatttaaagt taagtaattt taaagttgtt    180
   tgagggtagt ggagtaattt taaagttgtt tgagggtagt ggagtaattt taaattttat    240
   gagtttagtt taggtatttt gatttttaga tagtatttga tagttttggg taaagttata    300
   aattattata gtgtagaggt gaagtttttg aaagtatatt tgaagaaaag attgttgttg    360
   tttggaggag tttttatata tttgaggaag gaagtggaag gttggtatga ggagtagaga    420
   taaagagggt tttaagttga agattgattt ggagttttgg agagttgata gagtttaggg    480
   agtatgagga aggatattta gaagggttat tgttttttta ggatagatta ggttgtttgt    540
   tggtttgtta ggtattgttt agagagtagt ttaataagga gggagttagg atttttagat    600
   tttgttaaat attttaggtt ggtagtgagt ttatagatta tgtttttatt ttatttttag    660
   gtttttaatt tatattatta ttttgtagtt gttgtagaag agagtagtat tttttaagaa    720
   aagtaaagag gtttagaatg gtattaagtt tatagtgttt tttggtgttt tgttttaggt    780
   ttgaaaggaa gaattggtag tttttatata atgtttgaag tgggggtgat ggtgttttta    840
   tatttttgtt taggatagtt gatgttttga ttagaagttt ggttattgtt tgaggttttt    900
   tttttttaat ttgtagtagt ttaattttta gttgatatag ttgaaggtta ggggttagat    960
   tggtgttttt taggtaattg tgagtttttt ttattgtgtt gatggttttg ttatggtggt   1020
   ggttttagta aaagtgatgg tagtgtttta aggtgagttt taagaggtag agggtttttt   1080
   ggggagaaag aagtttagaa gagttttttt ttttatttat taaggttttg attatgtgtt   1140
   tggagagtag gttatttagg aaattagtat ttgttttatt tagattatgg ttattggtat   1200
   taaatagttg atgggtagtt attgtttgat aggttgttgg agaagtaaag tgaggaattt   1260
   tataaggggt atttttattt tttaagagta ttaggaagtt taaggttttt agttgagttg   1320
   taaatgtagt ttgttgtttt aatagggttt ttgttttttt ttaaagtaga gaaaagttgt   1380
   tttgagttat ggttttatag ttttggggag tagttttgtg agagtattgt attaagtagg   1440
   tatggagggg ttgagtaagg tggagtgtgg tttttgggtt tttttgtgta gtagtatttt   1500
   gtagtaagat aaagagaatt tgttttaggt agaggggagg atgttgtggg gtagatatta   1560
   agtagttatt ataggttagt tttgttagtt ttagtaggtt ttttagatgt ttagggtaag   1620
   ttagtttagt agttagttgt tggttgtaag tttttaggat ggtattataa gtttgttttt   1680
   ttttagtatt agattggttg ttgggaaaat tagttggagg gttggatagg aatttttagg   1740
   agtagagaaa gatatgaatg gggttagtaa aggtaggaaa gaggtagaga aagtaattag   1800
   aaatagggtt atttagagag ggttgtttat ttatttattt ttttaaaagg taaatataaa   1860
   atagtttagt ttggaaagtt aggtgtattt tgagttaggt agtattttta tttttaaggt   1920
   gggtagtaat tttttagttt ttttttggtt gtttagtaga gttttaaagt tgattttttt   1980
   gaagtttttt atgatattgg agagttaggg aaaaggagat taattagaga agattgttaa   2040
   tgaagttaat tggaggaagg gaagagggtt gaaggttgtg gaaggaggta aagaattata   2100
   ttagaaattt tattggggag aaaatttatg ttaggatgtt gtgtttttgt tataaaagga   2160
   tgattttgtt tttttttttt ttgggtagat ttttgattgt attattatat tatttgttgt   2220
   gttattttag aggttttggt ttagtttttt agtggtttgg ggtagtttta aggttttttg   2280
   atattttttt taggagttat ttatgttttt ttttgtgtta gtgttgggga ttttttttat   2340
   tttagtatgt atttatgttt atgagttttt tttggtttta ttgttttaga gtttgttttt   2400
   ttttttaggt ataggattta attgtttgat tagtattttg tgaggattta aaatgtaatt   2460
   tttgtttttt gtgttagatt ttttttttaa atatttgtgg ttgttaatta gtgggtgtgg   2520
   tgtaatgttg ggtgtgtggt atgttgggag ttgtagttta tttgttgtgt tgtgtgtttt   2580
   tgtggtgttt tggagggttt tgttgttttg tgtgttaggg ttttttgggg gtgtgggttt   2640
   tttttttttt tttagttaga gtttagtttt ttagttattt gttgtttagt tgttattggt   2700
   aatgttattt tttttttttt tttattttta gagtttaggt aagagtttaa gttttaagag   2760
   aagagaaata ataaaaagaa tttaggaatt tatgtggttt gggttattag aaaaaaaggg   2820
   tttagatttt tatgaatttt ttttttgata gggttttgtt ttgatatata ggttggagtg   2880
   tagtggtgtg attatagttt attgtagttt taaatttttg ggtttaagtg atttttttat   2940
   tttagttttt taaagtgttg attatgggtg tgtattgtta tgtttaattt tttatgaaat   3000
   t                                                                   3001
<210> 334
   <211> 3001
   <212> DNA
   <213> human
<400> 334
   aatttcgtag gaagttgggc gtggcggtgt acgttcgtag ttagtatttt gggaggttga 60
   ggtgggagga ttatttaagt tcgggagttt gaagttgtag tgagttgtga ttacgttatt 120
   gtattttagt ttgtgtgtta gagtaagatt ttattaaaaa aaaaattcgt agaagtttaa 180
   gttttttttt tttagtaatt taaatcgcgt gagtttttaa gttttttttg ttattttttt 240
   ttttttgaaa tttaaatttt tgtttgagtt ttgaagataa aggaggaaaa ggggtggtat 300
   tatcgatagc gattaagcga taggtagttg agaaattgag ttttgattgg aaaaaagggg 360
   gaaattcgta tttttaagag gttttaacgc gtagagtagt aagatttttc ggggcgtcgc 420
   gggagcgcgt agcgcggcgg gtaaattata atttttagcg tgtcgcgcgt tcggcgttgc 480
   gtcgcgttcg ttgattggta gtcgcggata tttgaaagga gggtttggcg cggaaaacga 540
   aggttatatt ttggattttc gcggagtatt ggttaggcgg ttaagttttg tatttaggaa 600
   agagggcgag ttttggggcg gtaaggtcgg aagggattcg tgagcgtggg tacgtgttga 660
   agtgaagggg atttttagcg ttgacgcgag gagaggcgtg ggtggttttt ggagggagtg 720
   tcgggaggtt ttgggattgt ttcgggtcgt tgaagggttg ggtcgaggtt tttggggtag 780
   cgcggcgagt ggtgtggtag tgcggtcggg gatttgttcg gaggaaggaa gagcggaatc 840
   gtttttttat aacgggagta cggcgttttg gcgtgggttt ttttttcgat gaaatttttg 900
   atgtgatttt ttgttttttt ttacgatttt tagttttttt tttttttttt agttagtttt 960
   attaataatt ttttttaatt ggtttttttt tttttagttt ttcggtgtta tgaggagttt 1020
   taaaagagtt aattttggga ttttgttaag tagttagaag gaggttgaag agttgttgtt 1080
   cgttttgaag gtgggggtgt tgtttggttc gggatatatt tggtttttta aattgagttg 1140
   ttttgtgttt gttttttgaa gagatggata agtaggcgat tttttttgag taattttatt 1200
   tttagttatt ttttttattt tttttttgtt tttgttagtt ttatttatat ttttttttat 1260
   ttttaggagt ttttgtttaa ttttttagtt ggttttttta gtagtcgatt tgatgttgag 1320
   aggagataag tttgtgatgt tattttgagg gtttgtaatt agtagttgat tgttaagtta 1380
   gtttgtttta ggtatttggg gagtttgttg gagttggtag agttggtttg tgatggttat 1440
   ttagtgttta ttttatagcg tttttttttt tatttggaac gaattttttt tgttttattg 1500
   cggaatgttg ttgtataagg aagtttagag gttatatttc gttttgttta gtttttttat 1560
   gtttgtttgg tgtagtgttt tcgcgaggtt gttttttagg attatgaggt cgtggttcgg 1620
   ggtagttttt ttttgttttg gaagggggta gaagttttgt tggaacggcg agttgtattt 1680
   gtagttcggt tgaaggtttt gagtttttta gtatttttgg aggatgaaag tattttttgt 1740
   gaggtttttt atttgttttt tttaatagtt tgtcgagcgg tagttgttta ttagttattt 1800
   gatgttagtg gttatggttt aaatgaagta gatgttgatt ttttagatga tttgtttttt 1860
   aggtacgtga ttagagtttt ggtgggtgag agagggagtt tttttgggtt tttttttttt 1920
   tagagggttt tttgtttttt ggagtttatt ttggaatatt gtcgtcgttt ttgttggagt 1980
   cgttattatg ataaagttat tagcgtagtg gagaaggttt atagttattt aaggaatatt 2040
   aatttagttt ttagttttta gttatgttag ttgggggtta agttgttgta ggttggggag 2100
   gaaggatttt aggtagtggt taagtttttg attaaggtat tagttgtttt gagtaagagt 2160
   atggaggtat tattattttt atttcgggta ttgtatgaga gttgttagtt ttttttttta 2220
   ggtttggaac gaggtattaa gaggcgttat agatttgatg ttattttgag tttttttgtt 2280
   ttttttggag ggtattgttt ttttttgtag tagttgcggg atgatggtgt gagttaagga 2340
   tttggaggta gggtggggac gtggtttgtg gatttattat tagtttaggg tatttggtaa 2400
   gatttggagg ttttggtttt ttttttgtta agttgttttt tggatagtgt ttagcgagtt 2460
   agtaaatagt ttagtttatt ttgagagggt agtggttttt ttggatgttt tttttcgtgt 2520
   tttttgggtt ttgttagttt tttaggattt cgggttagtt tttagtttgg agtttttttt 2580
   atttttattt tttatattaa tttttcgttt ttttttttag gtgtatgggg gtttttttaa 2640
   gtaatagtag tttttttttt agatgtattt ttagggattt tatttttata ttgtggtggt 2700
   ttatgatttt gtttaaggtt gttaggtatt gtttgggaat taaggtattt ggattaggtt 2760
   tatagggttt ggggttgttt tattgttttt aaatagtttt ggggttgttt tattgttttt 2820
   aaatagtttt ggggttgttt ggttttgggt gtggagtgcg gtggaggtta ttttggattt 2880
   agtatgattt tttattttta aatttttttt gtttttttag atagttgatt tggttgattt 2940
   gatttaatta gtggatagtt gtaaatttat cgttgtttgg atgttggagg ttttagaggg 3000
   t 3001
<210> 335
   <211> 3001
   <212> DNA
   <213> human
<400> 335
   aattttgtag gaagttgggt gtggtggtgt atgtttgtag ttagtatttt gggaggttga 60
   ggtgggagga ttatttaagt ttgggagttt gaagttgtag tgagttgtga ttatgttatt 120
   gtattttagt ttgtgtgtta gagtaagatt ttattaaaaa aaaaatttgt agaagtttaa 180
   gttttttttt tttagtaatt taaattgtgt gagtttttaa gttttttttg ttattttttt 240
   ttttttgaaa tttaaatttt tgtttgagtt ttgaagataa aggaggaaaa ggggtggtat 300
   tattgatagt gattaagtga taggtagttg agaaattgag ttttgattgg aaaaaagggg 360
   gaaatttgta tttttaagag gttttaatgt gtagagtagt aagatttttt ggggtgttgt 420
   gggagtgtgt agtgtggtgg gtaaattata atttttagtg tgttgtgtgt ttggtgttgt 480
   gttgtgtttg ttgattggta gttgtggata tttgaaagga gggtttggtg tggaaaatga 540
   aggttatatt ttggattttt gtggagtatt ggttaggtgg ttaagttttg tatttaggaa 600
   agagggtgag ttttggggtg gtaaggttgg aagggatttg tgagtgtggg tatgtgttga 660
   agtgaagggg atttttagtg ttgatgtgag gagaggtgtg ggtggttttt ggagggagtg 720
   ttgggaggtt ttgggattgt tttgggttgt tgaagggttg ggttgaggtt tttggggtag 780
   tgtggtgagt ggtgtggtag tgtggttggg gatttgtttg gaggaaggaa gagtggaatt 840
   gtttttttat aatgggagta tggtgttttg gtgtgggttt tttttttgat gaaatttttg 900
   atgtgatttt ttgttttttt ttatgatttt tagttttttt tttttttttt agttagtttt 960
   attaataatt ttttttaatt ggtttttttt tttttagttt tttggtgtta tgaggagttt 1020
   taaaagagtt aattttggga ttttgttaag tagttagaag gaggttgaag agttgttgtt 1080
   tgttttgaag gtgggggtgt tgtttggttt gggatatatt tggtttttta aattgagttg 1140
   ttttgtgttt gttttttgaa gagatggata agtaggtgat tttttttgag taattttatt 1200
   tttagttatt ttttttattt tttttttgtt tttgttagtt ttatttatat ttttttttat 1260
   ttttaggagt ttttgtttaa ttttttagtt ggttttttta gtagttgatt tgatgttgag 1320
   aggagataag tttgtgatgt tattttgagg gtttgtaatt agtagttgat tgttaagtta 1380
   gtttgtttta ggtatttggg gagtttgttg gagttggtag agttggtttg tgatggttat 1440
   ttagtgttta ttttatagtg tttttttttt tatttggaat gaattttttt tgttttattg 1500
   tggaatgttg ttgtataagg aagtttagag gttatatttt gttttgttta gtttttttat 1560
   gtttgtttgg tgtagtgttt ttgtgaggtt gttttttagg attatgaggt tgtggtttgg 1620
   ggtagttttt ttttgttttg gaagggggta gaagttttgt tggaatggtg agttgtattt 1680
   gtagtttggt tgaaggtttt gagtttttta gtatttttgg aggatgaaag tattttttgt 1740
   gaggtttttt attttgtttt tttaatagtt tgttgagtgg tagttgttta ttagttattt 1800
   gatgttagtg gttatggttt aaatgaagta gatgttgatt ttttagatga tttgtttttt 1860
   aggtatgtga ttagagtttt ggtgggtgag agagggagtt tttttgggtt tttttttttt 1920
   tagagggttt tttgtttttt ggagtttatt ttggaatatt gttgttgttt ttgttggagt 1980
   tgttattatg ataaagttat tagtgtagtg gagaaggttt atagttattt aaggaatatt 2040
   aatttagttt ttagttttta gttatgttag ttgggggtta agttgttgta ggttggggag 2100
   gaaggatttt aggtagtggt taagtttttg attaaggtat tagttgtttt gagtaagagt 2160
   atggaggtat tattattttt attttgggta ttgtatgaga gttgttagtt ttttttttta 2220
   ggtttggaat gaggtattaa gaggtgttat agatttgatg ttattttgag tttttttgtt 2280
   ttttttggag ggtattgttt ttttttgtag tagttgtggg atgatggtgt gagttaagga 2340
   tttggaggta gggtggggat gtggtttgtg gatttattat tagtttaggg tatttggtaa 2400
   gatttggagg ttttggtttt ttttttgtta agttgttttt tggatagtgt ttagtgagtt 2460
   agtaaatagt ttagtttatt ttgagagggt agtggttttt ttggatgttt ttttttgtgt 2520
   tttttgggtt ttgttagttt tttaggattt tgggttagtt tttagtttgg agtttttttt 2580
   atttttattt tttatattaa ttttttgttt ttttttttag gtgtatgggg gtttttttaa 2640
   gtaatagtag tttttttttt agatgtattt ttagggattt tatttttata ttgtggtggt 2700
   ttatgatttt gtttaaggtt gttaggtatt gtttgggaat taaggtattt ggattaggtt 2760
   tatagggttt ggggttgttt tattgttttt aaatagtttt ggggttgttt tattgttttt 2820
   aaatagtttt ggggttgttt ggttttgggt gtggagtgtg gtggaggtta ttttggattt 2880
   agtatgattt tttattttta aatttttttt gtttttttag atagttgatt tggttgattt 2940
   gatttaatta gtggatagtt gtaaatttat tgttgtttgg atgttggagg ttttagaggg 3000
   t 3001
<210> 336
   <211> 50
   <212> DNA
   <213> human
<400> 336
   aacccctgcg atgatttcca gggtggacga gtctggcttt gagagggtcg 50
<210> 337
   <211> 50
   <212> DNA
   <213> human
<400> 337
   aatttttgcg atgattttta gggtggacga gtttggtttt gagagggtcg 50
<210> 338
   <211> 50
   <212> DNA
   <213> human
<400> 338
   aatttttgtg atgattttta gggtggatga gtttggtttt gagagggttg 50
<210> 339
   <211> 50
   <212> DNA
   <213> human
<400> 339
   cgattttttt aaagttagat tcgtttattt tggaaattat cgtaggggtt 50
<210> 340
   <211> 50
   <212> DNA
   <213> human
<400> 340
   tgattttttt aaagttagat ttgtttattt tggaaattat tgtaggggtt 50
<210> 341
   <211> 3001
   <212> DNA
   <213> human
<400> 341
   aaactcttaa aacgattagc agcaaaggtc tcatgtgctt ttttttaatt atcatttttt    60
   aatcgaggta taatttatgc atttaaaaaa tcagatcaat tgccccccgg gctctgggat   120
   ggaaggcggt gtgcgcctgc gaaggtcaga ggcccagcgg cgccagggct cctcggcgga   180
   gagccgcccg ggtccggcgc tttgctttcg ccttctgccc caccgcgggc gaagccccag   240
   ggccccgggg agactccagg ccggctgggc cggggtgcgc ccgccggctg tccgcgctcg   300
   ggcagggtgg ggcgcagcgg gcgtctgtcc tgctagcggg gatcgggcgg ctggacccaa   360
   gagcagggca cgtgcgcagc gacgcggggc ggccccgcca ggagccccga cccgggcgcg   420
   gcgcagggcc cggcggagag cgcgcattcc cgagcgccgc gagccggccg aagtcgggcg   480
   agcgcgccgt gcggcggctg ggagtgggct gcatgtgccg cgcggggagc ccgcccgact   540
   tcggggaggc agcggggcgc cgtggccccg cggctcagct ccctgccggg tcgggtgtcc   600
   cagccctgcg acttccgggg cccggcggct cctcggggag ctcgtccaca ggcagcctcc   660
   ggggcgagag agcgcgcggc gcgggcgccg ggacggtccg cggcacgcgg gtggagccgc   720
   cggcgctcgg gctcccgcag gggcggcggg cggggcgggg tgaggcgggc cggccgccct   780
   cccctctcct ccctcccgca gcccgccctc ccttcctccg gtcccgcagc caattaggcg   840
   gccccctcgg gcgggaggcg tggggcgctg cataaagcgg cggggagctg ccaccccggg   900
   agcgggctgc gcagtgtccg ggccgagccg gtgcgccgca gactagggcg cctcggccca   960
   gggagcgcgg aggagccatg gccaccgcta acggggccgt ggaaaacggg cagccggaca  1020
   ggaagccgcc ggccctgccg cgccccatcc gcaacctgga ggtcaagttc accaaggtga  1080
   ggcgggcgcc cctcccaccc gacggccgcg ggcccctgcg ctgggcagcc agactcgggg  1140
   cggcgggggc gagggagcag cgggccgggg gtccgccggg cgccgccgag acccgagcct  1200
   ccctgcccgg gcgcggctct ccagaaaccg cacctttcgc gggacgtctc gggcgggatc  1260
   gcaggcggcg gggctcggcg ctgtgagcct cgaagccggg aaaccgaggc cgtccagcgc  1320
   ccgcgcgggg ccgggccgcc cgcatccctg cgaggccccg acgtgtccct gcgctgcccg  1380
   ctttgatcgc gagtggaggt gcgcgccgcg ttgactcgga gcgcgctggt tttggggagc  1440
   tccctcccca aaaacccctg cgatgatttc cagggtggac gagtctggct ttgagagggt  1500
   cggagggtcc gagagcgcca agagagagtc cgggaggcag atggcggagc tgcagttgga  1560
   agactgggga agcgggtcct ccgagcaaag cgcagcgggc gccttccgtg cttagtttat  1620
   acccggctca gcagcagaac gaggaggagt aggaaggtgc tcgcttactg acgaaacctc  1680
   cccggagccc agaggtcccg gtgtttacac acaaggcgcg cgctccggaa ggtcgcagac  1740
   ctgggccttt gggaagcggt gggtcgcgag gccgcagaag cgcacggttc tctgcctcct  1800
   cgctctgtgg gctgggaggc tacagcgttg gcagaacggc gagattacct gcccggggca  1860
   gaaaggaata aaagaagatg gtgaggcctc cgaggcctct gagctggggc tgaggagggc  1920
   cgagagaccc gggtccggga gcgcgacccg atggaggaca cccccaggct gtctggcgtg  1980
   cgcgtctgtc gtgggccggt gctggggcac tttagaggca caggccaggc cgagagccat  2040
   tgccttaact ttccccacct ttggcttctc ttgatttttg cttttaccca tagcccccaa  2100
   ctccagctga caccgcactt agagaatttc ctcatccgtt ttactttaag aagtagcaat  2160
   tatctttctc aaagaatttt aatcaagcgg agaaagttgc agggcactat gtgtggcctt  2220
   cagatttggg tctcttggca cttaattccc aatgtcggct gagcctctga tcacaagaca  2280
   tttaaattaa ctgttttcag aaggctctgt aatcaacaaa gacacgtcgc tgtaaactaa  2340
   acgctggaaa ttattcttaa tgtagtttta tttgtaatat cagcttcaga gtgaaaattc  2400
   caacacgaat tctgggaaga gtgaaaatgg tacccgaatt ttgtaagacc ctaaccccag  2460
   gtgcttggct gctgctactg atggttaagt ggggaggttg tttgttttct tttggttgta  2520
   caggatttca aaatactgta tatagaaacc agacagtaaa tgtattatgg tccttttact  2580
   ttttatgttt ttgagtccta tcagcaatct gaccacatac aaaaagttta cctctttctc  2640
   tatgattttt aggacacttt taaagtgtag atgtagccct tatggagacc ttagacaatt  2700
   cacagagcaa tggttgcctg ttgtcttggt gggtgcgcaa cctttgccag ttaaagggaa  2760
   ccagtgcagg aatgaggctg agggaagctg gtggatcggc ctggagtgct gggatcttga  2820
   gtggtctccc aggcgataaa ggccccaact gcctgacacc ggctgctggc tcctcacccg  2880
   ttgggggact gtgctgctgc ctgctctgtc ctggctcctg gcccatctct gcagagacct  2940
   ccccatagca gttgtggacc caggcagctt gtgtggctga gggtggccaa ccagtgccct  3000
   g                                                                  3001
<210> 342
   <211> 3001
   <212> DNA
   <213> human
<400> 342
   aaatttttaa aacgattagt agtaaaggtt ttatgtgttt ttttttaatt attatttttt 60
   aatcgaggta taatttatgt atttaaaaaa ttagattaat tgtttttcgg gttttgggat 120
   ggaaggcggt gtgcgtttgc gaaggttaga ggtttagcgg cgttagggtt tttcggcgga 180
   gagtcgttcg ggttcggcgt tttgttttcg ttttttgttt tatcgcgggc gaagttttag 240
   ggtttcgggg agattttagg tcggttgggt cggggtgcgt tcgtcggttg ttcgcgttcg 300
   ggtagggtgg ggcgtagcgg gcgtttgttt tgttagcggg gatcgggcgg ttggatttaa 360
   gagtagggta cgtgcgtagc gacgcggggc ggtttcgtta ggagtttcga ttcgggcgcg 420
   gcgtagggtt cggcggagag cgcgtatttt cgagcgtcgc gagtcggtcg aagtcgggcg 480
   agcgcgtcgt gcggcggttg ggagtgggtt gtatgtgtcg cgcggggagt tcgttcgatt 540
   tcggggaggt agcggggcgt cgtggtttcg cggtttagtt ttttgtcggg tcgggtgttt 600
   tagttttgcg attttcgggg ttcggcggtt tttcggggag ttcgtttata ggtagttttc 660
   ggggcgagag agcgcgcggc gcgggcgtcg ggacggttcg cggtacgcgg gtggagtcgt 720
   cggcgttcgg gttttcgtag gggcggcggg cggggcgggg tgaggcgggt cggtcgtttt 780
   tttttttttt ttttttcgta gttcgttttt ttttttttcg gtttcgtagt taattaggcg 840
   gttttttcgg gcgggaggcg tggggcgttg tataaagcgg cggggagttg ttatttcggg 900
   agcgggttgc gtagtgttcg ggtcgagtcg gtgcgtcgta gattagggcg tttcgggtta 960
   gggagcgcgg aggagttatg gttatcgtta acggggtcgt ggaaaacggg tagtcggata 1020
   ggaagtcgtc ggttttgtcg cgttttattc gtaatttgga ggttaagttt attaaggtga 1080
   ggcgggcgtt ttttttattc gacggtcgcg ggtttttgcg ttgggtagtt agattcgggg 1140
   cggcgggggc gagggagtag cgggtcgggg gttcgtcggg cgtcgtcgag attcgagttt 1200
   ttttgttcgg gcgcggtttt ttagaaatcg tatttttcgc gggacgtttc gggcgggatc 1260
   gtaggcggcg gggttcggcg ttgtgagttt cgaagtcggg aaatcgaggt cgtttagcgt 1320
   tcgcgcgggg tcgggtcgtt cgtatttttg cgaggtttcg acgtgttttt gcgttgttcg 1380
   ttttgatcgc gagtggaggt gcgcgtcgcg ttgattcgga gcgcgttggt tttggggagt 1440
   ttttttttta aaaatttttg cgatgatttt tagggtggac gagtttggtt ttgagagggt 1500
   cggagggttc gagagcgtta agagagagtt cgggaggtag atggcggagt tgtagttgga 1560
   agattgggga agcgggtttt tcgagtaaag cgtagcgggc gtttttcgtg tttagtttat 1620
   attcggttta gtagtagaac gaggaggagt aggaaggtgt tcgtttattg acgaaatttt 1680
   ttcggagttt agaggtttcg gtgtttatat ataaggcgcg cgtttcggaa ggtcgtagat 1740
   ttgggttttt gggaagcggt gggtcgcgag gtcgtagaag cgtacggttt tttgtttttt 1800
   cgttttgtgg gttgggaggt tatagcgttg gtagaacggc gagattattt gttcggggta 1860
   gaaaggaata aaagaagatg gtgaggtttt cgaggttttt gagttggggt tgaggagggt 1920
   cgagagattc gggttcggga gcgcgattcg atggaggata tttttaggtt gtttggcgtg 1980
   cgcgtttgtc gtgggtcggt gttggggtat tttagaggta taggttaggt cgagagttat 2040
   tgttttaatt ttttttattt ttggtttttt ttgatttttg tttttattta tagtttttaa 2100
   ttttagttga tatcgtattt agagaatttt tttattcgtt ttattttaag aagtagtaat 2160
   tatttttttt aaagaatttt aattaagcgg agaaagttgt agggtattat gtgtggtttt 2220
   tagatttggg ttttttggta tttaattttt aatgtcggtt gagtttttga ttataagata 2280
   tttaaattaa ttgtttttag aaggttttgt aattaataaa gatacgtcgt tgtaaattaa 2340
   acgttggaaa ttatttttaa tgtagtttta tttgtaatat tagttttaga gtgaaaattt 2400
   taatacgaat tttgggaaga gtgaaaatgg tattcgaatt ttgtaagatt ttaattttag 2460
   gtgtttggtt gttgttattg atggttaagt ggggaggttg tttgtttttt tttggttgta 2520
   taggatttta aaatattgta tatagaaatt agatagtaaa tgtattatgg tttttttatt 2580
   ttttatgttt ttgagtttta ttagtaattt gattatatat aaaaagttta tttttttttt 2640
   tatgattttt aggatatttt taaagtgtag atgtagtttt tatggagatt ttagataatt 2700
   tatagagtaa tggttgtttg ttgttttggt gggtgcgtaa tttttgttag ttaaagggaa 2760
   ttagtgtagg aatgaggttg agggaagttg gtggatcggt ttggagtgtt gggattttga 2820
   gtggtttttt aggcgataaa ggttttaatt gtttgatatc ggttgttggt tttttattcg 2880
   ttgggggatt gtgttgttgt ttgttttgtt ttggtttttg gtttattttt gtagagattt 2940
   ttttatagta gttgtggatt taggtagttt gtgtggttga gggtggttaa ttagtgtttt 3000
   g 3001
<210> 343
   <211> 3001
   <212> DNA
   <213> human
<400> 343
   aaatttttaa aatgattagt agtaaaggtt ttatgtgttt ttttttaatt attatttttt      60
   aattgaggta taatttatgt atttaaaaaa ttagattaat tgttttttgg gttttgggat     120
   ggaaggtggt gtgtgtttgt gaaggttaga ggtttagtgg tgttagggtt ttttggtgga     180
   gagttgtttg ggtttggtgt tttgtttttg ttttttgttt tattgtgggt gaagttttag    240
   ggttttgggg agattttagg ttggttgggt tggggtgtgt ttgttggttg tttgtgtttg     300
   ggtagggtgg ggtgtagtgg gtgtttgttt tgttagtggg gattgggtgg ttggatttaa     360
   gagtagggta tgtgtgtagt gatgtggggt ggttttgtta ggagttttga tttgggtgtg     420
   gtgtagggtt tggtggagag tgtgtatttt tgagtgttgt gagttggttg aagttgggtg     480
   agtgtgttgt gtggtggttg ggagtgggtt gtatgtgttg tgtggggagt ttgtttgatt     540
   ttggggaggt agtggggtgt tgtggttttg tggtttagtt ttttgttggg ttgggtgttt     600
   tagttttgtg atttttgggg tttggtggtt ttttggggag tttgtttata ggtagttttt     660
   ggggtgagag agtgtgtggt gtgggtgttg ggatggtttg tggtatgtgg gtggagttgt     720
   tggtgtttgg gtttttgtag gggtggtggg tggggtgggg tgaggtgggt tggttgtttt     780
   tttttttttt tttttttgta gtttgttttt tttttttttg gttttgtagt taattaggtg      840
   gtttttttgg gtgggaggtg tggggtgttg tataaagtgg tggggagttg ttattttggg     900
   agtgggttgt gtagtgtttg ggttgagttg gtgtgttgta gattagggtg ttttgggtta     960
   gggagtgtgg aggagttatg gttattgtta atggggttgt ggaaaatggg tagttggata    1020
   ggaagttgtt ggttttgttg tgttttattt gtaatttgga ggttaagttt attaaggtga    1080
   ggtgggtgtt ttttttattt gatggttgtg ggtttttgtg ttgggtagtt agatttgggg    1140
   tggtgggggt gagggagtag tgggttgggg gtttgttggg tgttgttgag atttgagttt    1200
   ttttgtttgg gtgtggtttt ttagaaattg tattttttgt gggatgtttt gggtgggatt   1260
   gtaggtggtg gggtttggtg ttgtgagttt tgaagttggg aaattgaggt tgtttagtgt    1320
   ttgtgtgggg ttgggttgtt tgtatttttg tgaggttttg atgtgttttt gtgttgtttg   1380
   ttttgattgt gagtggaggt gtgtgttgtg ttgatttgga gtgtgttggt tttggggagt    1440
   ttttttttta aaaatttttg tgatgatttt tagggtggat gagtttggtt ttgagagggt   1500
   tggagggttt gagagtgtta agagagagtt tgggaggtag atggtggagt tgtagttgga    1560
   agattgggga agtgggtttt ttgagtaaag tgtagtgggt gttttttgtg tttagtttat   1620
   atttggttta gtagtagaat gaggaggagt aggaaggtgt ttgtttattg atgaaatttt    1680
   tttggagttt agaggttttg gtgtttatat ataaggtgtg tgttttggaa ggttgtagat    1740
   ttgggttttt gggaagtggt gggttgtgag gttgtagaag tgtatggttt tttgtttttt    1800
   tgttttgtgg gttgggaggt tatagtgttg gtagaatggt gagattattt gtttggggta    1860
   gaaaggaata aaagaagatg gtgaggtttt tgaggttttt gagttggggt tgaggagggt    1920
   tgagagattt gggtttggga gtgtgatttg atggaggata tttttaggtt gtttggtgtg    1980
   tgtgtttgtt gtgggttggt gttggggtat tttagaggta taggttaggt tgagagttat    2040
   tgttttaatt ttttttattt ttggtttttt ttgatttttg tttttattta tagtttttaa  2100
   ttttagttga tattgtattt agagaatttt tttatttgtt ttattttaag aagtagtaat    2160
   tatttttttt aaagaatttt aattaagtgg agaaagttgt agggtattat gtgtggtttt    2220
   tagatttggg ttttttggta tttaattttt aatgttggtt gagtttttga ttataagata    2280
   tttaaattaa ttgtttttag aaggttttgt aattaataaa gatatgttgt tgtaaattaa    2340
   atgttggaaa ttatttttaa tgtagtttta tttgtaatat tagttttaga gtgaaaattt    2400
   taatatgaat tttgggaaga gtgaaaatgg tatttgaatt ttgtaagatt ttaattttag    2460
   gtgtttggtt gttgttattg atggttaagt ggggaggttg tttgtttttt tttggttgta    2520
   taggatttta aaatattgta tatagaaatt agatagtaaa tgtattatgg tttttttatt    2580
   ttttatgttt ttgagtttta ttagtaattt gattatatat aaaaagttta tttttttttt    2640
   tatgattttt aggatatttt taaagtgtag atgtagtttt tatggagatt ttagataatt    2700
   tatagagtaa tggttgtttg ttgttttggt gggtgtgtaa tttttgttag ttaaagggaa    2760
   ttagtgtagg aatgaggttg agggaagttg gtggattggt ttggagtgtt gggattttga    2820
   gtggtttttt aggtgataaa ggttttaatt gtttgatatt ggttgttggt tttttatttg    2880
   ttgggggatt gtgttgttgt ttgttttgtt ttggtttttg gtttattttt gtagagattt    2940
   ttttatagta gttgtggatt taggtagttt gtgtggttga gggtggttaa ttagtgtttt    3000
   g                                                                    3001
<210> 344
   <211> 3001
   <212> DNA
   <213> human
<400> 344
   tagggtattg gttggttatt tttagttata taagttgttt gggtttataa ttgttatggg      60
   gaggtttttg tagagatggg ttaggagtta ggatagagta ggtagtagta tagtttttta     120
   acgggtgagg agttagtagt cggtgttagg tagttggggt ttttatcgtt tgggagatta    180
   tttaagattt tagtatttta ggtcgattta ttagtttttt ttagttttat ttttgtattg    240
   gtttttttta attggtaaag gttgcgtatt tattaagata ataggtaatt attgttttgt    300
   gaattgttta aggtttttat aagggttata tttatatttt aaaagtgttt taaaaattat    360
   agagaaagag gtaaattttt tgtatgtggt tagattgttg ataggattta aaaatataaa    420
   aagtaaaagg attataatat atttattgtt tggtttttat atatagtatt ttgaaatttt    480
   gtataattaa aagaaaataa ataatttttt tatttaatta ttagtagtag tagttaagta    540
   tttggggtta gggttttata aaattcgggt attattttta ttttttttag aattcgtgtt     600
   ggaattttta ttttgaagtt gatattataa ataaaattat attaagaata atttttagcg    660
   tttagtttat agcgacgtgt ttttgttgat tatagagttt tttgaaaata gttaatttaa    720
   atgttttgtg attagaggtt tagtcgatat tgggaattaa gtgttaagag atttaaattt    780
   gaaggttata tatagtgttt tgtaattttt ttcgtttgat taaaattttt tgagaaagat    840
   aattgttatt ttttaaagta aaacggatga ggaaattttt taagtgcggt gttagttgga    900
   gttgggggtt atgggtaaaa gtaaaaatta agagaagtta aaggtgggga aagttaaggt    960
   aatggttttc ggtttggttt gtgtttttaa agtgttttag tatcggttta cgatagacgc    1020
   gtacgttaga tagtttgggg gtgtttttta tcgggtcgcg ttttcggatt cgggtttttc    1080
   ggtttttttt agttttagtt tagaggtttc ggaggtttta ttattttttt ttattttttt     1140
   ttgtttcggg taggtaattt cgtcgttttg ttaacgttgt agttttttag tttatagagc    1200
   gaggaggtag agaatcgtgc gtttttgcgg tttcgcgatt tatcgttttt taaaggttta    1260
   ggtttgcgat ttttcggagc gcgcgttttg tgtgtaaata tcgggatttt tgggtttcgg    1320
   ggaggtttcg ttagtaagcg agtatttttt tatttttttt cgttttgttg ttgagtcggg    1380
   tataaattaa gtacggaagg cgttcgttgc gttttgttcg gaggattcgt ttttttagtt     1440
   ttttaattgt agtttcgtta tttgtttttc ggattttttt ttggcgtttt cggatttttc    1500
   gattttttta aagttagatt cgtttatttt ggaaattatc gtaggggttt ttggggaggg    1560
   agttttttaa aattagcgcg tttcgagtta acgcggcgcg tatttttatt cgcgattaaa    1620
   gcgggtagcg tagggatacg tcggggtttc gtagggatgc gggcggttcg gtttcgcgcg    1680
   ggcgttggac ggtttcggtt tttcggtttc gaggtttata gcgtcgagtt tcgtcgtttg    1740
   cgatttcgtt cgagacgttt cgcgaaaggt gcggtttttg gagagtcgcg ttcgggtagg    1800
   gaggttcggg tttcggcggc gttcggcgga ttttcggttc gttgtttttt cgttttcgtc    1860
   gtttcgagtt tggttgttta gcgtaggggt tcgcggtcgt cgggtgggag gggcgttcgt    1920
   tttattttgg tgaatttgat ttttaggttg cggatggggc gcggtagggt cggcggtttt    1980
   ttgttcggtt gttcgttttt tacggtttcg ttagcggtgg ttatggtttt ttcgcgtttt    2040
   ttggttcgag gcgttttagt ttgcggcgta tcggttcggt tcggatattg cgtagttcgt    2100
   tttcggggtg gtagtttttc gtcgttttat gtagcgtttt acgtttttcg ttcgaggggg    2160
   tcgtttaatt ggttgcggga tcggaggaag ggagggcggg ttgcgggagg gaggagaggg    2220
   gagggcggtc ggttcgtttt atttcgtttc gttcgtcgtt tttgcgggag ttcgagcgtc    2280
   ggcggtttta ttcgcgtgtc gcggatcgtt tcggcgttcg cgtcgcgcgt tttttcgttt    2340
   cggaggttgt ttgtggacga gtttttcgag gagtcgtcgg gtttcggaag tcgtagggtt    2400
   gggatattcg attcggtagg gagttgagtc gcggggttac ggcgtttcgt tgttttttcg   2460
   aagtcgggcg ggtttttcgc gcggtatatg tagtttattt ttagtcgtcg tacggcgcgt    2520
   tcgttcgatt tcggtcggtt cgcggcgttc gggaatgcgc gtttttcgtc gggttttgcg    2580
   tcgcgttcgg gtcggggttt ttggcggggt cgtttcgcgt cgttgcgtac gtgttttgtt    2640
   tttgggttta gtcgttcgat tttcgttagt aggatagacg ttcgttgcgt tttattttgt    2700
   tcgagcgcgg atagtcggcg ggcgtatttc ggtttagtcg gtttggagtt ttttcggggt    2760
   tttggggttt cgttcgcggt ggggtagaag gcgaaagtaa agcgtcggat tcgggcggtt    2820
   tttcgtcgag gagttttggc gtcgttgggt ttttgatttt cgtaggcgta tatcgttttt    2880
   tattttagag ttcggggggt aattgatttg attttttaaa tgtataaatt atatttcgat   2940
   taaaaaatga taattaaaaa aaagtatatg agatttttgt tgttaatcgt tttaagagtt    3000
   t                                                                    3001
<210> 345
   <211> 3001
   <212> DNA
   <213> human
<400> 345
   tagggtattg gttggttatt tttagttata taagttgttt gggtttataa ttgttatggg 60
   gaggtttttg tagagatggg ttaggagtta ggatagagta ggtagtagta tagtttttta 120
   atgggtgagg agttagtagt tggtgttagg tagttggggt ttttattgtt tgggagatta 180
   tttaagattt tagtatttta ggttgattta ttagtttttt ttagttttat ttttgtattg 240
   gtttttttta attggtaaag gttgtgtatt tattaagata ataggtaatt attgttttgt 300
   gaattgttta aggtttttat aagggttata tttatatttt aaaagtgttt taaaaattat 360
   agagaaagag gtaaattttt tgtatgtggt tagattgttg ataggattta aaaatataaa 420
   aagtaaaagg attataatat atttattgtt tggtttttat atatagtatt ttgaaatttt 480
   gtataattaa aagaaaataa ataatttttt tatttaatta ttagtagtag tagttaagta 540
   tttggggtta gggttttata aaatttgggt attattttta ttttttttag aatttgtgtt 600
   ggaattttta ttttgaagtt gatattataa ataaaattat attaagaata atttttagtg 660
   tttagtttat agtgatgtgt ttttgttgat tatagagttt tttgaaaata gttaatttaa 720
   atgttttgtg attagaggtt tagttgatat tgggaattaa gtgttaagag atttaaattt 780
   gaaggttata tatagtgttt tgtaattttt tttgtttgat taaaattttt tgagaaagat 840
   aattgttatt ttttaaagta aaatggatga ggaaattttt taagtgtggt gttagttgga 900
   gttgggggtt atgggtaaaa gtaaaaatta agagaagtta aaggtgggga aagttaaggt 960
   aatggttttt ggtttggttt gtgtttttaa agtgttttag tattggttta tgatagatgt 1020
   gtatgttaga tagtttgggg gtgtttttta ttgggttgtg tttttggatt tgggtttttt 1080
   ggtttttttt agttttagtt tagaggtttt ggaggtttta ttattttttt ttattttttt 1140
   ttgttttggg taggtaattt tgttgttttg ttaatgttgt agttttttag tttatagagt 1200
   gaggaggtag agaattgtgt gtttttgtgg ttttgtgatt tattgttttt taaaggttta 1260
   ggtttgtgat tttttggagt gtgtgttttg tgtgtaaata ttgggatttt tgggttttgg 1320
   ggaggttttg ttagtaagtg agtatttttt tatttttttt tgttttgttg ttgagttggg 1380
   tataaattaa gtatggaagg tgtttgttgt gttttgtttg gaggatttgt ttttttagtt 1440
   ttttaattgt agttttgtta tttgtttttt ggattttttt ttggtgtttt tggatttttt 1500
   gattttttta aagttagatt tgtttatttt ggaaattatt gtaggggttt ttggggaggg 1560
   agttttttaa aattagtgtg ttttgagtta atgtggtgtg tatttttatt tgtgattaaa 1620
   gtgggtagtg tagggatatg ttggggtttt gtagggatgt gggtggtttg gttttgtgtg 1680
   ggtgttggat ggttttggtt ttttggtttt gaggtttata gtgttgagtt ttgttgtttg 1740
   tgattttgtt tgagatgttt tgtgaaaggt gtggtttttg gagagttgtg tttgggtagg 1800
   gaggtttggg ttttggtggt gtttggtgga tttttggttt gttgtttttt tgtttttgtt 1860
   gttttgagtt tggttgttta gtgtaggggt ttgtggttgt tgggtgggag gggtgtttgt 1920
   tttattttgg tgaatttgat ttttaggttg tggatggggt gtggtagggt tggtggtttt 1980
   ttgtttggtt gtttgttttt tatggttttg ttagtggtgg ttatggtttt tttgtgtttt 2040
   ttggtttgag gtgttttagt ttgtggtgta ttggtttggt ttggatattg tgtagtttgt 2100
   ttttggggtg gtagtttttt gttgttttat gtagtgtttt atgttttttg tttgaggggg 2160
   ttgtttaatt ggttgtggga ttggaggaag ggagggtggg ttgtgggagg gaggagaggg 2220
   gagggtggtt ggtttgtttt attttgtttt gtttgttgtt tttgtgggag tttgagtgtt 2280
   ggtggtttta tttgtgtgtt gtggattgtt ttggtgtttg tgttgtgtgt ttttttgttt 2340
   tggaggttgt ttgtggatga gttttttgag gagttgttgg gttttggaag ttgtagggtt 2400
   gggatatttg atttggtagg gagttgagtt gtggggttat ggtgttttgt tgtttttttg 2460
   aagttgggtg ggttttttgt gtggtatatg tagtttattt ttagttgttg tatggtgtgt 2520
   ttgtttgatt ttggttggtt tgtggtgttt gggaatgtgt gttttttgtt gggttttgtg 2580
   ttgtgtttgg gttggggttt ttggtggggt tgttttgtgt tgttgtgtat gtgttttgtt 2640
   tttgggttta gttgtttgat ttttgttagt aggatagatg tttgttgtgt tttattttgt 2700
   ttgagtgtgg atagttggtg ggtgtatttt ggtttagttg gtttggagtt tttttggggt 2760
   tttggggttt tgtttgtggt ggggtagaag gtgaaagtaa agtgttggat ttgggtggtt 2820
   ttttgttgag gagttttggt gttgttgggt ttttgatttt tgtaggtgta tattgttttt 2880
   tattttagag tttggggggt aattgatttg attttttaaa tgtataaatt atattttgat 2940
   taaaaaatga taattaaaaa aaagtatatg agatttttgt tgttaattgt tttaagagtt 3000
   t 3001
<210> 346
   <211> 50
   <212> DNA
   <213> human
<400> 346
   tgtgggctgg gaggctacag cgttggcaga acggcgagat tacctgcccg 50
<210> 347
   <211> 50
   <212> DNA
   <213> human
<400> 347
   tgtgggttgg gaggttatag cgttggtaga acggcgagat tatttgttcg 50
<210> 348
   <211> 50
   <212> DNA
   <213> human
<400> 348
   tgtgggttgg gaggttatag tgttggtaga atggtgagat tatttgtttg 50
<210> 349
   <211> 50
   <212> DNA
   <213> human
<400> 349
   cgggtaggta atttcgtcgt tttgttaacg ttgtagtttt ttagtttata 50
<210> 350
   <211> 50
   <212> DNA
   <213> human
<400> 350
   tgggtaggta attttgttgt tttgttaatg ttgtagtttt ttagtttata 50
<210> 351
   <211> 3001
   <212> DNA
   <213> human
<400> 351
   gacccaagag cagggcacgt gcgcagcgac gcggggcggc cccgccagga gccccgaccc     60
   gggcgcggcg cagggcccgg cggagagcgc gcattcccga gcgccgcgag ccggccgaag    120
   tcgggcgagc gcgccgtgcg gcggctggga gtgggctgca tgtgccgcgc ggggagcccg    180
   cccgacttcg gggaggcagc ggggcgccgt ggccccgcgg ctcagctccc tgccgggtcg    240
   ggtgtcccag ccctgcgact tccggggccc ggcggctcct cggggagctc gtccacaggc    300
   agcctccggg gcgagagagc gcgcggcgcg ggcgccggga cggtccgcgg cacgcgggtg    360
   gagccgccgg cgctcgggct cccgcagggg cggcgggcgg ggcggggtga ggcgggccgg    420
   ccgccctccc ctctcctccc tcccgcagcc cgccctccct tcctccggtc ccgcagccaa    480
   ttaggcggcc ccctcgggcg ggaggcgtgg ggcgctgcat aaagcggcgg ggagctgcca    540
   ccccgggagc gggctgcgca gtgtccgggc cgagccggtg cgccgcagac tagggcgcct    600
   cgggccaggg agcgcggagg agccatggcc accgctaacg gggccgtgga aaacgggcag    660
   ccggacagga agccgccggc cctgccgcgc cccatccgca acctggaggt caagttcacc    720
   aaggtgaggc gggcgcccct cccacccgac ggccgcgggc ccctgcgctg ggcagccaga    780
   ctcggggcgg cgggggcgag ggagcagcgg gccgggggtc cgccgggcgc cgccgagacc    840
   cgagcctccc tgcccgggcg cggctctcca gaaaccgcac ctttcgcggg acgtctcggg    900
   cgggatcgca ggcggcgggg ctcggcgctg tgagcctcga agccgggaaa ccgaggccgt    960
   ccagcgcccg cgcggggccg ggccgcccgc atccctgcga ggccccgacg tgtccctgcg   1020
   ctgcccgctt tgatcgcgag tggaggtgcg cgccgcgttg actcggagcg cgctggtttt   1080
   ggggagctcc ctccccaaaa acccctgcga tgatttccag ggtggacgag tctggctttg   1140
   agagggtcgg agggtccgag agcgccaaga gagagtccgg gaggcagatg gcggagctgc   1200
   agttggaaga ctggggaagc gggtcctccg agcaaagcgc agcgggcgcc ttccgtgctt   1260
   agtttatacc cggctcagca gcagaacgag gaggagtagg aaggtgctcg cttactgacg   1320
   aaacctcccc ggagcccaga ggtcccggtg tttacacaca aggcgcgcgc tccggaaggt   1380
   cgcagacctg ggcctttggg aagcggtggg tcgcgaggcc gcagaagcgc acggttctct   1440
   gcctcctcgc tctgtgggct gggaggctac agcgttggca gaacggcgag attacctgcc   1500
   cggggcagaa aggaataaaa gaagatggtg aggcctccga ggcctctgag ctggggctga   1560
   ggagggccga gagacccggg tccgggagcg cgacccgatg gaggacaccc ccaggctgtc   1620
   tggcgtgcgc gtctgtcgtg ggccggtgct ggggcacttt agaggcacag gccaggccga   1680
   gagccattgc cttaactttc cccacctttg gcttctcttg atttttgctt ttacccatag   1740
   cccccaactc cagctgacac cgcacttaga gaatttcctc atccgtttta ctttaagaag   1800
   tagcaattat ctttctcaaa gaattttaat caagcggaga aagttgcagg gcactatgtg   1860
   tggccttcag atttgggtct cttggcactt aattcccaat gtcggctgag cctctgatca   1920
   caagacattt aaattaactg ttttcagaag gctctgtaat caacaaagac acgtcgctgt   1980
   aaactaaacg ctggaaatta ttcttaatgt agttttattt gtaatatcag cttcagagtg   2040
   aaaattccaa cacgaattct gggaagagtg aaaatggtac ccgaattttg taagacccta   2100
   accccaggtg cttggctgct gctactgatg gttaagtggg gaggttgttt gttttctttt   2160
   ggttgtacag gatttcaaaa tactgtatat agaaaccaga cagtaaatgt attatggtcc   2220
   ttttactttt tatgtttttg agtcctatca gcaatctgac cacatacaaa aagtttacct   2280
   ctttctctat gatttttagg acacttttaa agtgtagatg tagcccttat ggagacctta   2340
   gacaattcac agagcaatgg ttgcctgttg tcttggtggg tgcgcaacct ttgccagtta   2400
   aagggaacca gtgcaggaat gaggctgagg gaagctggtg gatcggcctg gagtgctggg   2460
   atcttgagtg gtctcccagg cgataaaggc cccaactgcc tgacaccggc tgctggctcc   2520
   tcacccgttg ggggactgtg ctgctgcctg ctctgtcctg gctcctggcc catctctgca   2580
   gagacctccc catagcagtt gtggacccag gcagcttgtg tggctgaggg tggccaacca   2640
   gtgccctggc acagtcctca gggagactgg actggagagg cggccgagag tgaggccacc   2700
   aagagtgcct gccgagggaa ggagcgggat aaactgaagt gcaggactga ggcccagagt   2760
   gggcgagtgg cccggcaggt gggtggcctg gcaggagagc agacggtgga gcatgggagc   2820
   cagcagtggt ctgctatttt ccaaaattga ctcatggtgc ttctcgataa tggggcacac   2880
   accagagttg gtccatctga gccctagccc cggcagtgcc atttcccagg gtggtccaac   2940
   tttcaggatg actttgtcaa agccccttca tttgtcctca catgttaaca tttcattctc   3000
   t                                                                   3001
<210> 352
   <211> 3001
   <212> DNA
   <213> human
<400> 352
   gatttaagag tagggtacgt gcgtagcgac gcggggcggt ttcgttagga gtttcgattc 60
   gggcgcggcg tagggttcgg cggagagcgc gtattttcga gcgtcgcgag tcggtcgaag 120
   tcgggcgagc gcgtcgtgcg gcggttggga gtgggttgta tgtgtcgcgc ggggagttcg 180
   ttcgatttcg gggaggtagc ggggcgtcgt ggtttcgcgg tttagttttt tgtcgggtcg 240
   ggtgttttag ttttgcgatt ttcggggttc ggcggttttt cggggagttc gtttataggt 300
   agttttcggg gcgagagagc gcgcggcgcg ggcgtcggga cggttcgcgg tacgcgggtg 360
   gagtcgtcgg cgttcgggtt ttcgtagggg cggcgggcgg ggcggggtga ggcgggtcgg 420
   tcgttttttt tttttttttt tttcgtagtt cgtttttttt tttttcggtt tcgtagttaa 480
   ttaggcggtt ttttcgggcg ggaggcgtgg ggcgttgtat aaagcggcgg ggagttgtta 540
   tttcgggagc gggttgcgta gtgttcgggt cgagtcggtg cgtcgtagat tagggcgttt 600
   cgggttaggg agcgcggagg agttatggtt atcgttaacg gggtcgtgga aaacgggtag 660
   tcggatagga agtcgtcggt tttgtcgcgt tttattcgta atttggaggt taagtttatt 720
   aaggtgaggc gggcgttttt tttattcgac ggtcgcgggt ttttgcgttg ggtagttaga 780
   ttcggggcgg cgggggcgag ggagtagcgg gtcgggggtt cgtcgggcgt cgtcgagatt 840
   cgagtttttt tgttcgggcg cggtttttta gaaatcgtat ttttcgcggg acgtttcggg 900
   cgggatcgta ggcggcgggg ttcggcgttg tgagtttcga agtcgggaaa tcgaggtcgt 960
   ttagcgttcg cgcggggtcg ggtcgttcgt atttttgcga ggtttcgacg tgtttttgcg 1020
   ttgttcgttt tgatcgcgag tggaggtgcg cgtcgcgttg attcggagcg cgttggtttt 1080
   ggggagtttt ttttttaaaa atttttgcga tgatttttag ggtggacgag tttggttttg 1140
   agagggtcgg agggttcgag agcgttaaga gagagttcgg gaggtagatg gcggagttgt 1200
   agttggaaga ttggggaagc gggtttttcg agtaaagcgt agcgggcgtt tttcgtgttt 1260
   agtttatatt cggtttagta gtagaacgag gaggagtagg aaggtgttcg tttattgacg 1320
   aaattttttc ggagtttaga ggtttcggtg tttatatata aggcgcgcgt ttcggaaggt 1380
   cgtagatttg ggtttttggg aagcggtggg tcgcgaggtc gtagaagcgt acggtttttt 1440
   gttttttcgt tttgtgggtt gggaggttat agcgttggta gaacggcgag attatttgtt 1500
   cggggtagaa aggaataaaa gaagatggtg aggttttcga ggtttttgag ttggggttga 1560
   ggagggtcga gagattcggg ttcgggagcg cgattcgatg gaggatattt ttaggttgtt 1620
   tggcgtgcgc gtttgtcgtg ggtcggtgtt ggggtatttt agaggtatag gttaggtcga 1680
   gagttattgt tttaattttt tttatttttg gttttttttg atttttgttt ttatttatag 1740
   tttttaattt tagttgatat cgtatttaga gaattttttt attcgtttta ttttaagaag 1800
   tagtaattat tttttttaaa gaattttaat taagcggaga aagttgtagg gtattatgtg 1860
   tggtttttag atttgggttt tttggtattt aatttttaat gtcggttgag tttttgatta 1920
   taagatattt aaattaattg tttttagaag gttttgtaat taataaagat acgtcgttgt 1980
   aaattaaacg ttggaaatta tttttaatgt agttttattt gtaatattag ttttagagtg 2040
   aaaattttaa tacgaatttt gggaagagtg aaaatggtat tcgaattttg taagatttta 2100
   attttaggtg tttggttgtt gttattgatg gttaagtggg gaggttgttt gttttttttt 2160
   ggttgtatag gattttaaaa tattgtatat agaaattaga tagtaaatgt attatggttt 2220
   ttttattttt tatgtttttg agttttatta gtaatttgat tatatataaa aagtttattt 2280
   ttttttttat gatttttagg atatttttaa agtgtagatg tagtttttat ggagatttta 2340
   gataatttat agagtaatgg ttgtttgttg ttttggtggg tgcgtaattt ttgttagtta 2400
   aagggaatta gtgtaggaat gaggttgagg gaagttggtg gatcggtttg gagtgttggg 2460
   attttgagtg gttttttagg cgataaaggt tttaattgtt tgatatcggt tgttggtttt 2520
   ttattcgttg ggggattgtg ttgttgtttg ttttgttttg gtttttggtt tatttttgta 2580
   gagatttttt tatagtagtt gtggatttag gtagtttgtg tggttgaggg tggttaatta 2640
   gtgttttggt atagttttta gggagattgg attggagagg cggtcgagag tgaggttatt 2700
   aagagtgttt gtcgagggaa ggagcgggat aaattgaagt gtaggattga ggtttagagt 2760
   gggcgagtgg ttcggtaggt gggtggtttg gtaggagagt agacggtgga gtatgggagt 2820
   tagtagtggt ttgttatttt ttaaaattga tttatggtgt ttttcgataa tggggtatat 2880
   attagagttg gtttatttga gttttagttt cggtagtgtt attttttagg gtggtttaat 2940
   ttttaggatg attttgttaa agttttttta tttgttttta tatgttaata ttttattttt 3000
   t 3001
<210> 353
   <211> 3001
   <212> DNA
   <213> human
<400> 353
   gatttaagag tagggtatgt gtgtagtgat gtggggtggt tttgttagga gttttgattt 60
   gggtgtggtg tagggtttgg tggagagtgt gtatttttga gtgttgtgag ttggttgaag 120
   ttgggtgagt gtgttgtgtg gtggttggga gtgggttgta tgtgttgtgt ggggagtttg 180
   tttgattttg gggaggtagt ggggtgttgt ggttttgtgg tttagttttt tgttgggttg 240
   ggtgttttag ttttgtgatt tttggggttt ggtggttttt tggggagttt gtttataggt 300
   agtttttggg gtgagagagt gtgtggtgtg ggtgttggga tggtttgtgg tatgtgggtg 360
   gagttgttgg tgtttgggtt tttgtagggg tggtgggtgg ggtggggtga ggtgggttgg 420
   ttgttttttt tttttttttt ttttgtagtt tgtttttttt ttttttggtt ttgtagttaa 480
   ttaggtggtt tttttgggtg ggaggtgtgg ggtgttgtat aaagtggtgg ggagttgtta 540
   ttttgggagt gggttgtgta gtgtttgggt tgagttggtg tgttgtagat tagggtgttt 600
   tgggttaggg agtgtggagg agttatggtt attgttaatg gggttgtgga aaatgggtag 660
   ttggatagga agttgttggt tttgttgtgt tttatttgta atttggaggt taagtttatt 720
   aaggtgaggt gggtgttttt tttatttgat ggttgtgggt ttttgtgttg ggtagttaga 780
   tttggggtgg tgggggtgag ggagtagtgg gttgggggtt tgttgggtgt tgttgagatt 840
   tgagtttttt tgtttgggtg tggtttttta gaaattgtat tttttgtggg atgttttggg 900
   tgggattgta ggtggtgggg tttggtgttg tgagttttga agttgggaaa ttgaggttgt 960
   ttagtgtttg tgtggggttg ggttgtttgt atttttgtga ggttttgatg tgtttttgtg 1020
   ttgtttgttt tgattgtgag tggaggtgtg tgttgtgttg atttggagtg tgttggtttt 1080
   ggggagtttt ttttttaaaa atttttgtga tgatttttag ggtggatgag tttggttttg 1140
   agagggttgg agggtttgag agtgttaaga gagagtttgg gaggtagatg gtggagttgt 1200
   agttggaaga ttggggaagt gggttttttg agtaaagtgt agtgggtgtt ttttgtgttt 1260
   agtttatatt tggtttagta gtagaatgag gaggagtagg aaggtgtttg tttattgatg 1320
   aaattttttt ggagtttaga ggttttggtg tttatatata aggtgtgtgt tttggaaggt 1380
   tgtagatttg ggtttttggg aagtggtggg ttgtgaggtt gtagaagtgt atggtttttt 1440
   gtttttttgt tttgtgggtt gggaggttat agtgttggta gaatggtgag attatttgtt 1500
   tggggtagaa aggaataaaa gaagatggtg aggtttttga ggtttttgag ttggggttga 1560
   ggagggttga gagatttggg tttgggagtg tgatttgatg gaggatattt ttaggttgtt 1620
   tggtgtgtgt gtttgttgtg ggttggtgtt ggggtatttt agaggtatag gttaggttga 1680
   gagttattgt tttaattttt tttatttttg gttttttttg atttttgttt ttatttatag 1740
   tttttaattt tagttgatat tgtatttaga gaattttttt atttgtttta ttttaagaag 1800
   tagtaattat tttttttaaa gaattttaat taagtggaga aagttgtagg gtattatgtg 1860
   tggtttttag atttgggttt tttggtattt aatttttaat gttggttgag tttttgatta 1920
   taagatattt aaattaattg tttttagaag gttttgtaat taataaagat atgttgttgt 1980
   aaattaaatg ttggaaatta tttttaatgt agttttattt gtaatattag ttttagagtg 2040
   aaaattttaa tatgaatttt gggaagagtg aaaatggtat ttgaattttg taagatttta 2100
   attttaggtg tttggttgtt gttattgatg gttaagtggg gaggttgttt gttttttttt 2160
   ggttgtatag gattttaaaa tattgtatat agaaattaga tagtaaatgt attatggttt 2220
   ttttattttt tatgtttttg agttttatta gtaatttgat tatatataaa aagtttattt 2280
   ttttttttat gatttttagg atatttttaa agtgtagatg tagtttttat ggagatttta 2340
   gataatttat agagtaatgg ttgtttgttg ttttggtggg tgtgtaattt ttgttagtta 2400
   aagggaatta gtgtaggaat gaggttgagg gaagttggtg gattggtttg gagtgttggg 2460
   attttgagtg gttttttagg tgataaaggt tttaattgtt tgatattggt tgttggtttt 2520
   ttatttgttg ggggattgtg ttgttgtttg ttttgttttg gtttttggtt tatttttgta 2580
   gagatttttt tatagtagtt gtggatttag gtagtttgtg tggttgaggg tggttaatta 2640
   gtgttttggt atagttttta gggagattgg attggagagg tggttgagag tgaggttatt 2700
   aagagtgttt gttgagggaa ggagtgggat aaattgaagt gtaggattga ggtttagagt 2760
   gggtgagtgg tttggtaggt gggtggtttg gtaggagagt agatggtgga gtatgggagt 2820
   tagtagtggt ttgttatttt ttaaaattga tttatggtgt tttttgataa tggggtatat 2880
   attagagttg gtttatttga gttttagttt tggtagtgtt attttttagg gtggtttaat 2940
   ttttaggatg attttgttaa agttttttta tttgttttta tatgttaata ttttattttt 3000
   t 3001

<210> 354
   <211> 3001
   <212> DNA
   <213> human
<400> 354
   agagaatgaa atgttaatat gtgaggataa atgaaggggt tttgataaag ttattttgaa 60
   agttggatta ttttgggaaa tggtattgtc ggggttaggg tttagatgga ttaattttgg 120
   tgtgtgtttt attatcgaga agtattatga gttaattttg gaaaatagta gattattgtt 180
   ggtttttatg ttttatcgtt tgtttttttg ttaggttatt tatttgtcgg gttattcgtt 240
   tattttgggt tttagttttg tattttagtt tatttcgttt tttttttcgg taggtatttt 300
   tggtggtttt attttcggtc gtttttttag tttagttttt ttgaggattg tgttagggta 360
   ttggttggtt atttttagtt atataagttg tttgggttta taattgttat ggggaggttt 420
   ttgtagagat gggttaggag ttaggataga gtaggtagta gtatagtttt ttaacgggtg 480
   aggagttagt agtcggtgtt aggtagttgg ggtttttatc gtttgggaga ttatttaaga 540
   ttttagtatt ttaggtcgat ttattagttt tttttagttt tatttttgta ttggtttttt 600
   ttaattggta aaggttgcgt atttattaag ataataggta attattgttt tgtgaattgt 660
   ttaaggtttt tataagggtt atatttatat tttaaaagtg ttttaaaaat tatagagaaa 720
   gaggtaaatt ttttgtatgt ggttagattg ttgataggat ttaaaaatat aaaaagtaaa 780
   aggattataa tatatttatt gtttggtttt tatatatagt attttgaaat tttgtataat 840
   taaaagaaaa taaataattt ttttatttaa ttattagtag tagtagttaa gtatttgggg 900
   ttagggtttt ataaaattcg ggtattattt ttattttttt tagaattcgt gttggaattt 960
   ttattttgaa gttgatatta taaataaaat tatattaaga ataattttta gcgtttagtt 1020
   tatagcgacg tgtttttgtt gattatagag ttttttgaaa atagttaatt taaatgtttt 1080
   gtgattagag gtttagtcga tattgggaat taagtgttaa gagatttaaa tttgaaggtt 1140
   atatatagtg ttttgtaatt tttttcgttt gattaaaatt ttttgagaaa gataattgtt 1200
   attttttaaa gtaaaacgga tgaggaaatt ttttaagtgc ggtgttagtt ggagttgggg 1260
   gttatgggta aaagtaaaaa ttaagagaag ttaaaggtgg ggaaagttaa ggtaatggtt 1320
   ttcggtttgg tttgtgtttt taaagtgttt tagtatcggt ttacgataga cgcgtacgtt 1380
   agatagtttg ggggtgtttt ttatcgggtc gcgttttcgg attcgggttt ttcggttttt 1440
   tttagtttta gtttagaggt ttcggaggtt ttattatttt tttttatttt tttttgtttc 1500
   gggtaggtaa tttcgtcgtt ttgttaacgt tgtagttttt tagtttatag agcgaggagg 1560
   tagagaatcg tgcgtttttg cggtttcgcg atttatcgtt ttttaaaggt ttaggtttgc 1620
   gatttttcgg agcgcgcgtt ttgtgtgtaa atatcgggat ttttgggttt cggggaggtt 1680
   tcgttagtaa gcgagtattt ttttattttt tttcgttttg ttgttgagtc gggtataaat 1740
   taagtacgga aggcgttcgt tgcgttttgt tcggaggatt cgttttttta gttttttaat 1800
   tgtagtttcg ttatttgttt ttcggatttt tttttggcgt tttcggattt ttcgattttt 1860
   ttaaagttag attcgtttat tttggaaatt atcgtagggg tttttgggga gggagttttt 1920
   taaaattagc gcgtttcgag ttaacgcggc gcgtattttt attcgcgatt aaagcgggta 1980
   gcgtagggat acgtcggggt ttcgtaggga tgcgggcggt tcggtttcgc gcgggcgttg 2040
   gacggtttcg gtttttcggt ttcgaggttt atagcgtcga gtttcgtcgt ttgcgatttc 2100
   gttcgagacg tttcgcgaaa ggtgcggttt ttggagagtc gcgttcgggt agggaggttc 2160
   gggtttcggc ggcgttcggc ggattttcgg ttcgttgttt tttcgttttc gtcgtttcga 2220
   gtttggttgt ttagcgtagg ggttcgcggt cgtcgggtgg gaggggcgtt cgttttattt 2280
   tggtgaattt gatttttagg ttgcggatgg ggcgcggtag ggtcggcggt tttttgttcg 2340
   gttgttcgtt ttttacggtt tcgttagcgg tggttatggt tttttcgcgt tttttggttc 2400
   gaggcgtttt agtttgcggc gtatcggttc ggttcggata ttgcgtagtt cgttttcggg 2460
   gtggtagttt ttcgtcgttt tatgtagcgt tttacgtttt tcgttcgagg gggtcgttta 2520
   attggttgcg ggatcggagg aagggagggc gggttgcggg agggaggaga ggggagggcg 2580
   gtcggttcgt tttatttcgt ttcgttcgtc gtttttgcgg gagttcgagc gtcggcggtt 2640
   ttattcgcgt gtcgcggatc gtttcggcgt tcgcgtcgcg cgttttttcg tttcggaggt 2700
   tgtttgtgga cgagtttttc gaggagtcgt cgggtttcgg aagtcgtagg gttgggatat 2760
   tcgattcggt agggagttga gtcgcggggt tacggcgttt cgttgttttt tcgaagtcgg 2820
   gcgggttttt cgcgcggtat atgtagttta tttttagtcg tcgtacggcg cgttcgttcg 2880
   atttcggtcg gttcgcggcg ttcgggaatg cgcgtttttc gtcgggtttt gcgtcgcgtt 2940
   cgggtcgggg tttttggcgg ggtcgtttcg cgtcgttgcg tacgtgtttt gtttttgggt 3000
   t 3001
<210> 355
   <211> 3001
   <212> DNA
   <213> human
<400> 355
   agagaatgaa atgttaatat gtgaggataa atgaaggggt tttgataaag ttattttgaa      60
   agttggatta ttttgggaaa tggtattgtt ggggttaggg tttagatgga ttaattttgg     120
   tgtgtgtttt attattgaga agtattatga gttaattttg gaaaatagta gattattgtt     180
   ggtttttatg ttttattgtt tgtttttttg ttaggttatt tatttgttgg gttatttgtt     240
   tattttgggt tttagttttg tattttagtt tattttgttt ttttttttgg taggtatttt      300
   tggtggtttt atttttggtt gtttttttag tttagttttt ttgaggattg tgttagggta    360
   ttggttggtt atttttagtt atataagttg tttgggttta taattgttat ggggaggttt     420
   ttgtagagat gggttaggag ttaggataga gtaggtagta gtatagtttt ttaatgggtg     480
   aggagttagt agttggtgtt aggtagttgg ggtttttatt gtttgggaga ttatttaaga     540
   ttttagtatt ttaggttgat ttattagttt tttttagttt tatttttgta ttggtttttt     600
   ttaattggta aaggttgtgt atttattaag ataataggta attattgttt tgtgaattgt     660
   ttaaggtttt tataagggtt atatttatat tttaaaagtg ttttaaaaat tatagagaaa     720
   gaggtaaatt ttttgtatgt ggttagattg ttgataggat ttaaaaatat aaaaagtaaa     780
   aggattataa tatatttatt gtttggtttt tatatatagt attttgaaat tttgtataat     840
   taaaagaaaa taaataattt ttttatttaa ttattagtag tagtagttaa gtatttgggg     900
   ttagggtttt ataaaatttg ggtattattt ttattttttt tagaatttgt gttggaattt     960
   ttattttgaa gttgatatta taaataaaat tatattaaga ataattttta gtgtttagtt    1020
   tatagtgatg tgtttttgtt gattatagag ttttttgaaa atagttaatt taaatgtttt    1080
   gtgattagag gtttagttga tattgggaat taagtgttaa gagatttaaa tttgaaggtt    1140
   atatatagtg ttttgtaatt ttttttgttt gattaaaatt ttttgagaaa gataattgtt     1200
   attttttaaa gtaaaatgga tgaggaaatt ttttaagtgt ggtgttagtt ggagttgggg     1260
   gttatgggta aaagtaaaaa ttaagagaag ttaaaggtgg ggaaagttaa ggtaatggtt    1320
   tttggtttgg tttgtgtttt taaagtgttt tagtattggt ttatgataga tgtgtatgtt    1380
   agatagtttg gggtgttttt ttattgggtt gtgtttttgg atttgggttt tttggttttt    1440
   tttagtttta gtttagaggt tttggaggtt ttattatttt tttttatttt tttttgtttt    1500
   gggtaggtaa ttttgttgtt ttgttaatgt tgtagttttt tagtttatag agtgaggagg    1560
   tagagaattg tgtgtttttg tggttttgtg atttattgtt ttttaaaggt ttaggtttgt    1620
   gattttttgg agtgtgtgtt ttgtgtgtaa atattgggat ttttgggttt tggggaggtt    1680
   ttgttagtaa gtgagtattt ttttattttt ttttgttttg ttgttgagtt gggtataaat    1740
   taagtatgga aggtgtttgt tgtgttttgt ttggaggatt tgttttttta gttttttaat   1800
   tgtagttttg ttatttgttt tttggatttt tttttggtgt ttttggattt tttgattttt    1860
   ttaaagttag atttgtttat tttggaaatt attgtagggg tttttgggga gggagttttt    1920
   taaaattagt gtgttttgag ttaatgtggt gtgtattttt atttgtgatt aaagtgggta    1980
   gtgtagggat atgttggggt tttgtaggga tgtgggtggt ttggttttgt gtgggtgttg    2040
   gatggttttg gttttttggt tttgaggttt atagtgttga gttttgttgt ttgtgatttt   2100
   gtttgagatg ttttgtgaaa ggtgtggttt ttggagagtt gtgtttgggt agggaggttt    2160
   gggttttggt ggtgtttggt ggatttttgg tttgttgttt ttttgttttt gttgttttga    2220
   gtttggttgt ttagtgtagg ggtttgtggt tgttgggtgg gaggggtgtt tgttttattt    2280
   tggtgaattt gatttttagg ttgtggatgg ggtgtggtag ggttggtggt tttttgtttg    2340
   gttgtttgtt ttttatggtt ttgttagtgg tggttatggt ttttttgtgt tttttggttt     2400
   gaggtgtttt agtttgtggt gtattggttt ggtttggata ttgtgtagtt tgtttttggg    2460
   gtggtagttt tttgttgttt tatgtagtgt tttatgtttt ttgtttgagg gggttgttta    2520
   attggttgtg ggattggagg aagggagggt gggttgtggg agggaggaga ggggagggtg    2580
   gttggtttgt tttattttgt tttgtttgtt gtttttgtgg gagtttgagt gttggtggtt    2640
   ttatttgtgt gttgtggatt gttttggtgt ttgtgttgtg tgtttttttg ttttggaggt   2700
   tgtttgtgga tgagtttttt gaggagttgt tgggttttgg aagttgtagg gttgggatat    2760
   ttgatttggt agggagttga gttgtggggt tatggtgttt tgttgttttt ttgaagttgg    2820
   gtgggttttt tgtgtggtat atgtagttta tttttagttg ttgtatggtg tgtttgtttg    2880
   attttggttg gtttgtggtg tttgggaatg tgtgtttttt gttgggtttt gtgttgtgtt    2940
   tgggttgggg tttttggtgg ggttgttttg tgttgttgtg tatgtgtttt gtttttgggt    3000
   t                                                                    3001
<210> 356
   <211> 50
   <212> DNA
   <213> human
<400> 356
   tgcctgtaca attgtttctc aagcagttgt gtccctgtgg ctttggtgcg 50
<210> 357
   <211> 50
   <212> DNA
   <213> human
<400> 357
   tgtttgtata attgtttttt aagtagttgt gtttttgtgg ttttggtgcg 50
<210> 358
   <211> 50
   <212> DNA
   <213> human
<400> 358
   tgtttgtata attgtttttt aagtagttgt gtttttgtgg ttttggtgtg 50
<210> 359
   <211> 50
   <212> DNA
   <213> human
<400> 359
   cgtattaaag ttatagggat ataattgttt gagaaataat tgtataggta 50
<210> 360
   <211> 50
   <212> DNA
   <213> human
<400> 360
   tgtattaaag ttatagggat ataattgttt gagaaataat tgtataggta 50
<210> 361
   <211> 3001
   <212> DNA
   <213> human
<400> 361
<210> 362
   <211> 3001
   <212> DNA
   <213> human
<400> 362
   tttttttttt ttttttttta tatatatata tatatatata ttttttttta tgttttttta     60
   ttttagttgt ttttggggag tttggattgg atttgtgttt acggtttgta gtttttttcg    120
   gtttgtttgt tggttatttt ttgaaggaac ggggtttagt tttgtttatt ttttggttta    180
   aggattaagt atgtttttat ttatttttta tttttaagta tttattttat ttcgatttaa    240
   ttgtttgatt tataattaaa aagaatagtt ttttgatttt tataggtgtg gtgaggtttg    300
   gtgagagggg agttttttag ttagtgattt attacgtttt aggaattatg ttagttttta    360
   ggatttagag atgattaaaa ttaattacgt attttgagaa atttatagtg gaaggaaaat    420
   tagtaagtag aaaaatgtta tataattagg taaatgttgg gataaattta ggaaggaacg    480
   atgtggatat attggagtag gttttaaagt agtattattt cggaggagga ttttgaagat    540
   ttaagagtta attgggttaa gaaatatata tattaatatt tttgataggt ttgtagaata    600
   tagggagttt ataaaaatag tatttgggta atggaagtag gatatattat tttatgttta    660
   tagtattatt ttttgtgtgt agtgtattgt gtgatgattt atagagatta aataagtcga    720
   ggttttcgtt tttagttatt tgggagagac gtataaggta aattatttaa atagaaggta    780
   aattgtttag ggttgtaatt aatatataat atgcgtcgag agggaaaggt taatgttaat    840
   ttcggtttta agtataaaga ttattgggag ttaggggtgt atttttatta gggtatagta    900
   tgggtgggtg gagagagtaa aaattagaat aaattaaggt ttagtagttg gaagttagtt    960
   ttagggagaa aaggggcgga gattgattgg gttagaatag ggattgggat agaaaaagag   1020
   ggtggggtta gtgggtttta tttattcgtt tcggtttttt ttattatttt ttattttatt   1080
   tattttttat agtttgtgga gataggtgag cgacgaattt ttgagatagg tgtgggtgcg   1140
   agggtcggga gggttatggg attgggatcg aggtgtgagg agggaatttg taattttttg   1200
   ttatatagag cgttggtaat ttttgatagg ttgtttttgg ggtatgggtt gtttcgggtt   1260
   gttgttgtta taaggaaaga aaagagtttt tttgtttatc gttttttagt tattgggtta   1320
   ttttttggta ggaaatttgt aaattgagtt taataagtta ggattagtag agggtagagg   1380
   agggttttgg tagatgtggg gtttagaaga ggataggagt tattagggtt ttcggttatt   1440
   gtgttgggtt tttgtttgta taattgtttt ttaagtagtt gtgtttttgt ggttttggtg   1500
   cgtttgtgtg tatttttttt ttttatttgg agtatgggtt aatatcggag gaaaggaaaa   1560
   gatagagtta gatagggtaa gtggggtttt tttttttttt ttttttaacg gggttggatt   1620
   gagggttttt ggttggggag gtgggggtgg agatttagta ggagtaataa tagaggaagg   1680
   gtagggtttg ttttattatt tgaattttag agatgttagt gtgtattgaa gttttaggta   1740
   gggcgtgttt aggatcggat tttggatggt ggtaagggat aaagttggaa gggagattat   1800
   agggaaggag aaaggaaaga aggtagagtt atgatatagt taatttatag aatggttggg   1860
   agttaaagtt tttgggttgg ttttttggtt attttatata ttttaagata tagagagaat   1920
   ttcggaattg gaggggattt tatagatgat ttatttgaga ttgaagaatt tagaaattaa   1980
   ataaagaaat attatatggt tattttggta tttgatatag ttatgatatt aattgttagt   2040
   gtagatataa tagttgtgtg tttttttgta ggagtttggg gaggggttat ggtgttaatg   2100
   tatttattgg ggagattgga gaagtcgttt ttttttttgt gttgcgtttt ttttttattg   2160
   gggttggttt tgttgggtat aaagacggat attattttcg ttgtttattt tttttttata   2220
   ttgggtggtt tttttttgtt tgtttatttt ttggttcggt ttttggaatg ggggtttcgg   2280
   ttttagtttt aattaatgta gattgagagt ttagggtttt taggtaatgt acggtgagtt   2340
   aagggtgggt atcgtaagag gaatatattt tgggtttatt tttttttttt ttttgtagtt   2400
   tttgaggtag agagtttttg gttttttgtt attggtatta gcgggaggag gggttagagg   2460
   ttaatagagg attttttgtt tatagtagat aaaaaaaagg gaaaaagggt tttttttatg   2520
   gaagttatta atggttttat tttttttttt ttagggataa tgaagttttt gaagtgttag   2580
   tttatgaaga ggtcgtggtg ggattagaat tttagtgtcg tttttaagag ttggattaat   2640
   tattttttta tagtattgtt gtgatatttt tagtatttga ggaggaataa tttagttatt   2700
   tagaggggtt taggagagtt aaattggaat agaggcgaat ggttttagag gggtttatgg   2760
   tttaggaagg aagttttgga agagttttaa ttaattttcg gtttcgggga ttacgggttg   2820
   tgtttattgt ttttgatttg tagagtttgg cggtagtttt tatattagag tttttgattt   2880
   tattttttgt ttatgatgtt tgttttggtt attttgatga tgatagtgtt ttttatgagg   2940
   ataattgggt atttttttaa atgatttttt taagattttt ttttttttta tattagattt   3000
   t                                                                  3001
<210> 363
   <211> 3001
   <212> DNA
   <213> human
<400> 363
   tttttttttt ttttttttta tatatatata tatatatata ttttttttta tgttttttta     60
   ttttagttgt ttttggggag tttggattgg atttgtgttt atggtttgta gttttttttg    120
   gtttgtttgt tggttatttt ttgaaggaat ggggtttagt tttgtttatt ttttggttta    180
   aggattaagt atgtttttat ttatttttta tttttaagta tttattttat tttgatttaa    240
   ttgtttgatt tataattaaa aagaatagtt ttttgatttt tataggtgtg gtgaggtttg    300
   gtgagagggg agttttttag ttagtgattt attatgtttt aggaattatg ttagttttta    360
   ggatttagag atgattaaaa ttaattatgt attttgagaa atttatagtg gaaggaaaat    420
   tagtaagtag aaaaatgtta tataattagg taaatgttgg gataaattta ggaaggaatg    480
   atgtggatat attggagtag gttttaaagt agtattattt tggaggagga ttttgaagat    540
   ttaagagtta attgggttaa gaaatatata tattaatatt tttgataggt ttgtagaata    600
   tagggagttt ataaaaatag tatttgggta atggaagtag gatatattat tttatgttta    660
   tagtattatt ttttgtgtgt agtgtattgt gtgatgattt atagagatta aataagttga    720
   ggtttttgtt tttagttatt tgggagagat gtataaggta aattatttaa atagaaggta    780
   aattgtttag ggttgtaatt aatatataat atgtgttgag agggaaaggt taatgttaat    840
   tttggtttta agtataaaga ttattgggag ttaggggtgt atttttatta gggtatagta    900
   tgggtgggtg gagagagtaa aaattagaat aaattaaggt ttagtagttg gaagttagtt    960
   ttagggagaa aaggggtgga gattgattgg gttagaatag ggattgggat agaaaaagag   1020
   ggtggggtta gtgggtttta tttatttgtt ttggtttttt ttattatttt ttattttatt   1080
   tattttttat agtttgtgga gataggtgag tgatgaattt ttgagatagg tgtgggtgtg   1140
   agggttggga gggttatggg attgggattg aggtgtgagg agggaatttg taattttttg   1200
   ttatatagag tgttggtaat ttttgatagg ttgtttttgg ggtatgggtt gttttgggtt   1260
   gttgttgtta taaggaaaga aaagagtttt tttgtttatt gttttttagt tattgggtta   1320
   ttttttggta ggaaatttgt aaattgagtt taataagtta ggattagtag agggtagagg   1380
   agggttttgg tagatgtggg gtttagaaga ggataggagt tattagggtt tttggttatt   1440
   gtgttgggtt tttgtttgta taattgtttt ttaagtagtt gtgtttttgt ggttttggtg   1500
   tgtttgtgtg tatttttttt ttttatttgg agtatgggtt aatattggag gaaaggaaaa   1560
   gatagagtta gatagggtaa gtggggtttt tttttttttt ttttttaatg gggttggatt   1620
   gagggttttt ggttggggag gtgggggtgg agatttagta ggagtaataa tagaggaagg   1680
   gtagggtttg ttttattatt tgaattttag agatgttagt gtgtattgaa gttttaggta   1740
   gggtgtgttt aggattggat tttggatggt ggtaagggat aaagttggaa gggagattat   1800
   agggaaggag aaaggaaaga aggtagagtt atgatatagt taatttatag aatggttggg   1860
   agttaaagtt tttgggttgg ttttttggtt attttatata ttttaagata tagagagaat   1920
   tttggaattg gaggggattt tatagatgat ttatttgaga ttgaagaatt tagaaattaa   1980
   ataaagaaat attatatggt tattttggta tttgatatag ttatgatatt aattgttagt   2040
   gtagatataa tagttgtgtg tttttttgta ggagtttggg gaggggttat ggtgttaatg   2100
   tatttattgg ggagattgga gaagttgttt ttttttttgt gttgtgtttt ttttttattg   2160
   gggttggttt tgttgggtat aaagatggat attatttttg ttgtttattt tttttttata   2220
   ttgggtggtt tttttttgtt tgtttatttt ttggtttggt ttttggaatg ggggttttgg   2280
   ttttagtttt aattaatgta gattgagagt ttagggtttt taggtaatgt atggtgagtt   2340
   aagggtgggt attgtaagag gaatatattt tgggtttatt tttttttttt ttttgtagtt   2400
   tttgaggtag agagtttttg gttttttgtt attggtatta gtgggaggag gggttagagg   2460
   ttaatagagg attttttgtt tatagtagat aaaaaaaagg gaaaaagggt tttttttatg   2520
   gaagttatta atggttttat tttttttttt ttagggataa tgaagttttt gaagtgttag   2580
   tttatgaaga ggttgtggtg ggattagaat tttagtgttg tttttaagag ttggattaat   2640
   tattttttta tagtattgtt gtgatatttt tagtatttga ggaggaataa tttagttatt   2700
   tagaggggtt taggagagtt aaattggaat agaggtgaat ggttttagag gggtttatgg   2760
   tttaggaagg aagttttgga agagttttaa ttaatttttg gttttgggga ttatgggttg   2820
   tgtttattgt ttttgatttg tagagtttgg tggtagtttt tatattagag tttttgattt   2880
   tattttttgt ttatgatgtt tgttttggtt attttgatga tgatagtgtt ttttatgagg   2940
   ataattgggt atttttttaa atgatttttt taagattttt ttttttttta tattagattt   3000
   t                                                                   3001
<210> 364
   <211> 3001
   <212> DNA
   <213> human
<400> 364
   gaggtttggt gtggagagaa gagaaatttt gggagagtta tttaaggggg tgtttagttg 60
   tttttataaa aaatattatt attattaggg tgattaaagt agatattata ggtagggggt 120
   ggagttagag gttttaatgt ggggattgtc gttaagtttt gtagattagg agtagtggat 180
   atagttcgtg gttttcgaag tcgaaggttg attggagttt ttttagggtt ttttttttgg 240
   gttatggatt tttttgaggt tattcgtttt tgttttagtt tggttttttt ggattttttt 300
   ggatggttag gttgtttttt tttaggtgtt gggggtatta taatagtgtt gtaggggggt 360
   ggttggttta atttttgggg gcggtattgg gattttagtt ttattacggt ttttttatag 420
   attggtattt taaaggtttt attgtttttg agggaagaga aataaggtta ttggtggttt 480
   ttatgaaaga agtttttttt tttttttttt tgtttgttgt gggtaaaaga ttttttgttg 540
   gtttttgatt ttttttttcg ttgatgttaa tgataggaaa ttagagattt tttgttttag 600
   agattatagg gagagaggga ggatgggttt aaggtgtatt ttttttacga tgtttatttt 660
   taatttatcg tgtattgttt gagggttttg ggtttttagt ttgtattgat tggagttggg 720
   atcgaagttt ttattttaga aatcggatta ggagataggt aaataagaag aagttattta 780
   atgtgagaaa gaaataagta acgggggtga tgttcgtttt tatgtttagt aaagttagtt 840
   ttagtaggaa ggaggcgtag tataggagga gaagcggttt ttttagtttt tttagtaagt 900
   gtattggtat tatggttttt ttttaggttt ttgtaaaaag atatatagtt attgtgttta 960
   tattagtaat tggtgttatg gttgtgttag atattaaagt aattatatga tatttttttg 1020
   tttggttttt aggtttttta attttaaata aattatttgt agagtttttt ttagtttcga 1080
   aatttttttt atgttttgga gtgtgtggaa tagttaggaa attagtttag gagttttgat 1140
   ttttagttat tttgtaagtt gattgtgtta tggttttgtt tttttttttt tttttttttt 1200
   tgtagttttt tttttagttt tgttttttat tattatttag gattcggttt tgggtacgtt 1260
   ttgtttgggg ttttagtgta tattggtatt tttggaattt aggtgatggg gtaggttttg 1320
   tttttttttt gttattgttt ttattggatt tttattttta tttttttagt tagaggtttt 1380
   taatttagtt tcgttagaga gaagagggga gggagtttta tttattttgt ttgattttgt 1440
   tttttttttt ttttcggtgt tagtttatgt tttaggtgga gggagaaagt gtatataggc 1500
   gtattaaagt tatagggata taattgtttg agaaataatt gtataggtaa aggtttagta 1560
   taatggtcgg aggttttgat aatttttgtt tttttttaga ttttatattt gttagggttt 1620
   ttttttattt tttgttgatt ttaatttgtt aaatttagtt tgtaaatttt ttgttaggag 1680
   gtagtttagt ggttgggagg cggtgggtag gggaattttt tttttttttt gtaatagtaa 1740
   taattcgagg tagtttatat tttagaaata gtttgttaga agttgttagc gttttgtgta 1800
   gtaaggaatt gtagattttt tttttatatt tcggttttaa ttttatgatt ttttcgattt 1860
   tcgtatttat atttgtttta gaagttcgtc gtttatttgt ttttatagat tgtagaaagt 1920
   agatggggtg gggggtggtg ggaagggtcg gagcgggtag gtggagttta ttggttttat 1980
   tttttttttt tgttttaatt tttgttttgg tttagttagt tttcgttttt ttttttttta 2040
   aggttgattt ttaattgttg aattttgatt tgttttggtt tttatttttt ttatttattt 2100
   atgttgtatt ttaatgagag tgtatttttg atttttaatg atttttgtat ttaggatcgg 2160
   agttagtatt aatttttttt tttcggcgta tattgtatgt taattatagt tttgggtagt 2220
   ttgtttttta tttgggtaat ttattttgtg cgtttttttt aagtgattgg aagcggagat 2280
   ttcggtttat ttaatttttg tagattatta tatagtgtat tgtatataga aagtaatgtt 2340
   gtgggtatgg aatggtgtgt tttgttttta ttatttaagt gttattttta tggatttttt 2400
   gtattttgta gatttgttag aagtgttagt gtgtatattt tttgatttag ttaatttttg 2460
   agtttttaag attttttttc gaggtgatgt tgttttggag tttgttttag tgtatttata 2520
   tcgttttttt ttaaatttgt tttagtattt atttggttat gtagtatttt tttgtttatt 2580
   gatttttttt ttattgtgag ttttttaagg tgcgtgatta gttttaatta tttttgaatt 2640
   ttggaaatta atatggtttt tgaaacgtgg taggttatta attagaaggt ttttttttta 2700
   ttaggtttta ttatatttgt gaagattaaa gagttgtttt ttttagttgt gggttaggta 2760
   gttaagtcgg agtagggtag gtgtttggag gtagagagtg agtagaagta tatttggttt 2820
   ttgagttaga gggtgaataa aattgggttt cgttttttta gagggtgatt agtagataga 2880
   tcgagggaag ttgtagatcg tgggtataaa tttaatttag gttttttagg gatagttggg 2940
   gtggggaagt ataggagagg gtgtgtgtgt gtgtgtgtgt gtgagagaga gagagagaga 3000
   g 3001
<210> 365
   <211> 3001
   <212> DNA
   <213> human
<400> 365
   gaggtttggt gtggagagaa gagaaatttt gggagagtta tttaaggggg tgtttagttg     60
   tttttataaa aaatattatt attattaggg tgattaaagt agatattata ggtagggggt    120
   ggagttagag gttttaatgt ggggattgtt gttaagtttt gtagattagg agtagtggat    180
   atagtttgtg gtttttgaag ttgaaggttg attggagttt ttttagggtt ttttttttgg     240
   gttatggatt tttttgaggt tatttgtttt tgttttagtt tggttttttt ggattttttt    300
   ggatggttag gttgtttttt tttaggtgtt gggggtatta taatagtgtt gtaggggggt     360
   ggttggttta atttttgggg gtggtattgg gattttagtt ttattatggt ttttttatag     420
   attggtattt taaaggtttt attgtttttg agggaagaga aataaggtta ttggtggttt     480
   ttatgaaaga agtttttttt tttttttttt tgtttgttgt gggtaaaaga ttttttgttg    540
   gtttttgatt tttttttttg ttgatgttaa tgataggaaa ttagagattt tttgttttag     600
   agattatagg gagagaggga ggatgggttt aaggtgtatt ttttttatga tgtttatttt     660
   taatttattg tgtattgttt gagggttttg ggtttttagt ttgtattgat tggagttggg    720
   attgaagttt ttattttaga aattggatta ggagataggt aaataagaag aagttattta    780
   atgtgagaaa gaaataagta atgggggtga tgtttgtttt tatgtttagt aaagttagtt    840
   ttagtaggaa ggaggtgtag tataggagga gaagtggttt ttttagtttt tttagtaagt    900
   gtattggtat tatggttttt ttttaggttt ttgtaaaaag atatatagtt attgtgttta    960
   tattagtaat tggtgttatg gttgtgttag atattaaagt aattatatga tatttttttg   1020
   tttggttttt aggtttttta attttaaata aattatttgt agagtttttt ttagttttga   1080
   aatttttttt atgttttgga gtgtgtggaa tagttaggaa attagtttag gagttttgat   1140
   ttttagttat tttgtaagtt gattgtgtta tggttttgtt tttttttttt tttttttttt   1200
   tgtagttttt tttttagttt tgttttttat tattatttag gatttggttt tgggtatgtt   1260
   ttgtttgggg ttttagtgta tattggtatt tttggaattt aggtgatggg gtaggttttg   1320
   tttttttttt gttattgttt ttattggatt tttattttta tttttttagt tagaggtttt      1380
   taatttagtt ttgttagaga gaagagggga gggagtttta tttattttgt ttgattttgt     1440
   tttttttttt tttttggtgt tagtttatgt tttaggtgga gggagaaagt gtatataggt    1500
   gtattaaagt tatagggata taattgtttg agaaataatt gtataggtaa aggtttagta    1560
   taatggttgg aggttttgat aatttttgtt tttttttaga ttttatattt gttagggttt    1620
   ttttttattt tttgttgatt ttaatttgtt aaatttagtt tgtaaatttt ttgttaggag    1680
   gtagtttagt ggttgggagg tggtgggtag gggaattttt tttttttttt gtaatagtaa    1740
   taatttgagg tagtttatat tttagaaata gtttgttaga agttgttagt gttttgtgta    1800
   gtaaggaatt gtagattttt tttttatatt ttggttttaa ttttatgatt tttttgattt    1860
   ttgtatttat atttgtttta gaagtttgtt gtttatttgt ttttatagat tgtagaaagt    1920
   agatggggtg gggggtggtg ggaagggttg gagtgggtag gtggagttta ttggttttat    1980
   tttttttttt tgttttaatt tttgttttgg tttagttagt ttttgttttt ttttttttta    2040
   aggttgattt ttaattgttg aattttgatt tgttttggtt tttatttttt ttatttattt    2100
   atgttgtatt ttaatgagag tgtatttttg atttttaatg atttttgtat ttaggattgg    2160
   agttagtatt aatttttttt ttttggtgta tattgtatgt taattatagt tttgggtagt    2220
   ttgtttttta tttgggtaat ttattttgtg tgtttttttt aagtgattgg aagtggagat   2280
   tttggtttat ttaatttttg tagattatta tatagtgtat tgtatataga aagtaatgtt     2340
   gtgggtatgg aatggtgtgt tttgttttta ttatttaagt gttattttta tggatttttt    2400
   gtattttgta gatttgttag aagtgttagt gtgtatattt tttgatttag ttaatttttg     2460
   agtttttaag attttttttt gaggtgatgt tgttttggag tttgttttag tgtatttata    2520
   ttgttttttt ttaaatttgt tttagtattt atttggttat gtagtatttt tttgtttatt    2580
   gatttttttt ttattgtgag ttttttaagg tgtgtgatta gttttaatta tttttgaatt   2640
   ttggaaatta atatggtttt tgaaatgtgg taggttatta attagaaggt ttttttttta   2700
   ttaggtttta ttatatttgt gaagattaaa gagttgtttt ttttagttgt gggttaggta    2760
   gttaagttgg agtagggtag gtgtttggag gtagagagtg agtagaagta tatttggttt    2820
   ttgagttaga gggtgaataa aattgggttt tgttttttta gagggtgatt agtagataga    2880
   ttgagggaag ttgtagattg tgggtataaa tttaatttag gttttttagg gatagttggg   2940
   gtggggaagt ataggagagg gtgtgtgtgt gtgtgtgtgt gtgagagaga gagagagaga   3000
   g                                                                   3001
<210> 366
   <211> 50
   <212> DNA
   <213> human
<400> 366
   ggggctgggg gttggcgggg agcgtcctca gggacaagtc tcccagcacg 50
<210> 367
   <211> 50
   <212> DNA
   <213> human
<400> 367
   ggggttgggg gttggcgggg agcgttttta gggataagtt ttttagtacg 50
<210> 368
   <211> 50
   <212> DNA
   <213> human
<400> 368
   ggggttgggg gttggtgggg agtgttttta gggataagtt ttttagtatg 50
<210> 369
   <211> 50
   <212> DNA
   <213> human
<400> 369
   cgtgttggga gatttgtttt tgaggacgtt tttcgttaat ttttagtttt 50
<210> 370
   <211> 50
   <212> DNA
   <213> human
<400> 370
   tgtgttggga gatttgtttt tgaggatgtt ttttgttaat ttttagtttt 50
<210> 371
   <211> 3001
   <212> DNA
   <213> human
<400> 371
   gaaggggaaa gggtggtgag aggggcggcc tgcaagtctg tgcaggcccc tttgcagctg 60
   ctaagggggc ccagacctca tcctgcagga caggcgtgaa gctgggaggg ccactgtggg 120
   tttgcccggt aagtcagtcc ctccagctgc tgtaggaggg gtggacaggg gagcccaaat 180
   gaaggcaggg agaccccagg gcagggcaca gaagtaatgc tggtgagagg agatggggtt 240
   gcagccggga cagaggaagc aggctggaca agagtgggtg gtttaaaaca ttgggaggta 300
   aaagcaacag gatgggagag gggggtggga cgaggaggga gcagaactgc ctggttttca 360
   acaccttgct aagtccagga ggcagttgga gagagggctg ggtgggggca ggcagttcct 420
   agggtgccac gacaccagca gcacctaaga tctccactca tgctcactca gtccacacga 480
   cacgagggcc cagcacacat caggcaccca gaacaagaac aggtggctgc cctccaagag 540
   ctgatttgca tgggagtgtg ggagcggggg ataattaata ataaatataa taaggaaatc 600
   atatagaaag tggaaatggg aagtgttggg ggggcagaat gctgcagata tcctaaggag 660
   atgtgccaga gccagcccaa tgcccatcct tcccttggct acaggcggca cttgtgactt 720
   gaatctgagt agaactgggc cgagggggtg gcatgtcacc cacaattagg ttctgttcca 780
   caggactcca tctgggggtg gggctctccc acggaccctg gggaagcggc caccctgtga     840
   accgcccacc ctcccacagt agggactggc aggcagcccc cgggagctga gagcagctcc     900
   tgccggccct cagtcctaca ggcaccagga catgacttct gccagcaatc gcatgaggtg     960
   ggaagtgatc cttccccagt ccagccccca gatgagaaca cagcggctga ccagagctga    1020
   ggacccagct aagcctggcc ttctggccca cagaagcctt gagataatag gcggatgtcg    1080
   ttttaggaca cccaatttgc ggtacgtagg gaactaacac gcggggctct ggcgggcctg    1140
   gcggctcagt gcgatctggg caggcaggaa gtggagtgag cacagccagc ggctgggagc    1200
   acatcccgga ggggctgcct gcggcgggag gagcggcagg ggccacgtgg tgggggacgg    1260
   ctcgccaggg cgcgcacgcg gtggcggcgg cggtggaggc gttgcccgcc tagctagggg    1320
   cccgcggcct ggagaggcgt cggttctcct tccgcgctga aggcggagac agcgacttcc    1380
   cgtggaggca agccgtgggc gccgcggagg cgggggctgg gggtcggcgg aggcgggagc    1440
   tgggggtcgg cgggggctgg gggttggcgg ggagcgtcct cagggacaag tctcccagca    1500
   cgcggccgcg acctccccgt tgggggtctg gagccaggag cccacgcgcg tcggatccgg    1560
   agagtctggt gggcgcagcc tacgtgacag ggtcggctcg ggaccgagct gctggggact    1620
   gggccttgcg gggcggggcc gctgggggcg gggcatgagg gtgccggttc ggggcggggc    1680
   tttttggggg cgggacatgg ggcggagcct gggggcggag catgtgggga ggtggctggg    1740
   gaggaacttt cgggggcggg acacgagggc ggagcctgag ggggcggggc atgaggggaa    1800
   gccggctcgg gaaggagctt tcggaggcgg ggcccgctcg gggagggacc tgatttcggg    1860
   ggagccttcg ggagggcgcc tgccatagac gactgtaaag gggcggggtc agctcgggga    1920
   ggaccctgcg ggcgtggcgg cagttatggg tgaagcctga gggggcgggg cctgatcggg    1980
   gcggggtctt cgggggcgga gtcggctggg gatggagccg ctcgggccga gccctccacc    2040
   gcccaccatc accgagtccg gcccaggctc ctagcgcggc cgccggaagg ggcgggtcag    2100
   cgggcgggca tttcctgccg gtgcctaggg ccgacgggga cgctggcact ggtggggagc    2160
   tgttgggggg tcccgcatct agggctctcc tttcctgcct ccggcccccg cgaggggcgc    2220
   gcgcctctcc ccgccctcca gcagtcctgg tagggcccgg gcgcgtccgc gctctgcagc    2280
   gggaggtcct cgtcgcctct cgtctccagc caggtactca ctgggctcct gcctgccgcc    2340
   tccggcccag gacgcgcagc gtcggtatgg gccccgccgc ctccccgcac cccggcgctc    2400
   cagggatgtg gcaagacccg gctttgagca cctcccgggc gccggccccg aggccgacct    2460
   agctctgcac gtgggccgcg gggctctgcc gttgaaagga gagcctctaa ctaggagcac    2520
   gcacgggacc ctggcgggcc cctccgcctg gctcagatgc ctggcatctc aggccttggc    2580
   acagctagca ggcccctggc aggccgtgga cccagcaggc tgttttagtg cagattgccc    2640
   ctgaatctca agcccactaa tgttccagtc tctccatgag acccagatcc ctgaaagtag    2700
   tggagggagg cgccagggtg agtgaggttt tctaggcagc ccttaccgcc cacggatacc    2760
   cagagcttca tattacaagt gaagggtcct ccgcccagag aggcgacgga gctacgctgg    2820
   agttgcacga cagaaccaga accccagtct ttaggggtgt ggcaagcgtg ggttgcgctg    2880
   tttgtaacta gctaatcact cctgtctaca ggcctgtgag gtgaggccaa gaaaatcgct    2940
   atggtccaat ccgtttttgt tttttgcgtg tggggttttt tgttttggtt tggtttggtt    3000
   t                                                                    3001
<210> 372
   <211> 3001
   <212> DNA
   <213> human
<400> 372
   gaaggggaaa gggtggtgag aggggcggtt tgtaagtttg tgtaggtttt tttgtagttg     60
   ttaagggggt ttagatttta ttttgtagga taggcgtgaa gttgggaggg ttattgtggg    120
   tttgttcggt aagttagttt ttttagttgt tgtaggaggg gtggataggg gagtttaaat    180
   gaaggtaggg agattttagg gtagggtata gaagtaatgt tggtgagagg agatggggtt    240
   gtagtcggga tagaggaagt aggttggata agagtgggtg gtttaaaata ttgggaggta    300
   aaagtaatag gatgggagag gggggtggga cgaggaggga gtagaattgt ttggttttta    360
   atattttgtt aagtttagga ggtagttgga gagagggttg ggtgggggta ggtagttttt    420
   agggtgttac gatattagta gtatttaaga tttttattta tgtttattta gtttatacga    480
   tacgagggtt tagtatatat taggtattta gaataagaat aggtggttgt tttttaagag    540
   ttgatttgta tgggagtgtg ggagcggggg ataattaata ataaatataa taaggaaatt    600
   atatagaaag tggaaatggg aagtgttggg ggggtagaat gttgtagata ttttaaggag    660
   atgtgttaga gttagtttaa tgtttatttt ttttttggtt ataggcggta tttgtgattt    720
   gaatttgagt agaattgggt cgagggggtg gtatgttatt tataattagg ttttgtttta    780
   taggatttta tttgggggtg gggttttttt acggattttg gggaagcggt tattttgtga    840
   atcgtttatt tttttatagt agggattggt aggtagtttt cgggagttga gagtagtttt    900
   tgtcggtttt tagttttata ggtattagga tatgattttt gttagtaatc gtatgaggtg    960
   ggaagtgatt tttttttagt ttagttttta gatgagaata tagcggttga ttagagttga   1020
   ggatttagtt aagtttggtt ttttggttta tagaagtttt gagataatag gcggatgtcg   1080
   ttttaggata tttaatttgc ggtacgtagg gaattaatac gcggggtttt ggcgggtttg   1140
   gcggtttagt gcgatttggg taggtaggaa gtggagtgag tatagttagc ggttgggagt   1200
   atatttcgga ggggttgttt gcggcgggag gagcggtagg ggttacgtgg tgggggacgg   1260
   ttcgttaggg cgcgtacgcg gtggcggcgg cggtggaggc gttgttcgtt tagttagggg   1320
   ttcgcggttt ggagaggcgt cggttttttt ttcgcgttga aggcggagat agcgattttt   1380
   cgtggaggta agtcgtgggc gtcgcggagg cgggggttgg gggtcggcgg aggcgggagt   1440
   tgggggtcgg cgggggttgg gggttggcgg ggagcgtttt tagggataag ttttttagta   1500
   cgcggtcgcg attttttcgt tgggggtttg gagttaggag tttacgcgcg tcggattcgg   1560
   agagtttggt gggcgtagtt tacgtgatag ggtcggttcg ggatcgagtt gttggggatt   1620
   gggttttgcg gggcggggtc gttgggggcg gggtatgagg gtgtcggttc ggggcggggt   1680
   tttttggggg cgggatatgg ggcggagttt gggggcggag tatgtgggga ggtggttggg   1740
   gaggaatttt cgggggcggg atacgagggc ggagtttgag ggggcggggt atgaggggaa   1800
   gtcggttcgg gaaggagttt tcggaggcgg ggttcgttcg gggagggatt tgatttcggg   1860
   ggagttttcg ggagggcgtt tgttatagac gattgtaaag gggcggggtt agttcgggga   1920
   ggattttgcg ggcgtggcgg tagttatggg tgaagtttga gggggcgggg tttgatcggg   1980
   gcggggtttt cgggggcgga gtcggttggg gatggagtcg ttcgggtcga gttttttatc   2040
   gtttattatt atcgagttcg gtttaggttt ttagcgcggt cgtcggaagg ggcgggttag   2100
   cgggcgggta ttttttgtcg gtgtttaggg tcgacgggga cgttggtatt ggtggggagt   2160
   tgttgggggg tttcgtattt agggtttttt ttttttgttt tcggttttcg cgaggggcgc   2220
   gcgttttttt tcgtttttta gtagttttgg tagggttcgg gcgcgttcgc gttttgtagc   2280
   gggaggtttt cgtcgttttt cgtttttagt taggtattta ttgggttttt gtttgtcgtt   2340
   ttcggtttag gacgcgtagc gtcggtatgg gtttcgtcgt tttttcgtat ttcggcgttt   2400
   tagggatgtg gtaagattcg gttttgagta tttttcgggc gtcggtttcg aggtcgattt   2460
   agttttgtac gtgggtcgcg gggttttgtc gttgaaagga gagtttttaa ttaggagtac   2520
   gtacgggatt ttggcgggtt ttttcgtttg gtttagatgt ttggtatttt aggttttggt   2580
   atagttagta ggtttttggt aggtcgtgga tttagtaggt tgttttagtg tagattgttt   2640
   ttgaatttta agtttattaa tgttttagtt tttttatgag atttagattt ttgaaagtag   2700
   tggagggagg cgttagggtg agtgaggttt tttaggtagt ttttatcgtt tacggatatt   2760
   tagagtttta tattataagt gaagggtttt tcgtttagag aggcgacgga gttacgttgg   2820
   agttgtacga tagaattaga attttagttt ttaggggtgt ggtaagcgtg ggttgcgttg   2880
   tttgtaatta gttaattatt tttgtttata ggtttgtgag gtgaggttaa gaaaatcgtt   2940
   atggtttaat tcgtttttgt tttttgcgtg tggggttttt tgttttggtt tggtttggtt   3000
   t                                                                   3001
<210> 373
   <211> 3001
   <212> DNA
   <213> human
<400> 373
   gaaggggaaa gggtggtgag aggggtggtt tgtaagtttg tgtaggtttt tttgtagttg      60
   ttaagggggt ttagatttta ttttgtagga taggtgtgaa gttgggaggg ttattgtggg     120
   tttgtttggt aagttagttt ttttagttgt tgtaggaggg gtggataggg gagtttaaat     180
   gaaggtaggg agattttagg gtagggtata gaagtaatgt tggtgagagg agatggggtt     240
   gtagttggga tagaggaagt aggttggata agagtgggtg gtttaaaata ttgggaggta     300
   aaagtaatag gatgggagag gggggtggga tgaggaggga gtagaattgt ttggttttta     360
   atattttgtt aagtttagga ggtagttgga gagagggttg ggtgggggta ggtagttttt     420
   agggtgttat gatattagta gtatttaaga tttttattta tgtttattta gtttatatga     480
   tatgagggtt tagtatatat taggtattta gaataagaat aggtggttgt tttttaagag     540
   ttgatttgta tgggagtgtg ggagtggggg ataattaata ataaatataa taaggaaatt     600
   atatagaaag tggaaatggg aagtgttggg ggggtagaat gttgtagata ttttaaggag     660
   atgtgttaga gttagtttaa tgtttatttt ttttttggtt ataggtggta tttgtgattt      720
   gaatttgagt agaattgggt tgagggggtg gtatgttatt tataattagg ttttgtttta     780
   taggatttta tttgggggtg gggttttttt atggattttg gggaagtggt tattttgtga     840
   attgtttatt tttttatagt agggattggt aggtagtttt tgggagttga gagtagtttt     900
   tgttggtttt tagttttata ggtattagga tatgattttt gttagtaatt gtatgaggtg     960
   ggaagtgatt tttttttagt ttagttttta gatgagaata tagtggttga ttagagttga     1020
   ggatttagtt aagtttggtt ttttggttta tagaagtttt gagataatag gtggatgttg    1080
   ttttaggata tttaatttgt ggtatgtagg gaattaatat gtggggtttt ggtgggtttg    1140
   gtggtttagt gtgatttggg taggtaggaa gtggagtgag tatagttagt ggttgggagt    1200
   atattttgga ggggttgttt gtggtgggag gagtggtagg ggttatgtgg tgggggatgg    1260
   tttgttaggg tgtgtatgtg gtggtggtgg tggtggaggt gttgtttgtt tagttagggg    1320
   tttgtggttt ggagaggtgt tggttttttt tttgtgttga aggtggagat agtgattttt    1380
   tgtggaggta agttgtgggt gttgtggagg tgggggttgg gggttggtgg aggtgggagt    1440
   tgggggttgg tgggggttgg gggttggtgg ggagtgtttt tagggataag ttttttagta   1500
   tgtggttgtg atttttttgt tgggggtttg gagttaggag tttatgtgtg ttggatttgg    1560
   agagtttggt gggtgtagtt tatgtgatag ggttggtttg ggattgagtt gttggggatt    1620
   gggttttgtg gggtggggtt gttgggggtg gggtatgagg gtgttggttt ggggtggggt    1680
   tttttggggg tgggatatgg ggtggagttt gggggtggag tatgtgggga ggtggttggg    1740
   gaggaatttt tgggggtggg atatgagggt ggagtttgag ggggtggggt atgaggggaa     1800
   gttggtttgg gaaggagttt ttggaggtgg ggtttgtttg gggagggatt tgattttggg    1860
   ggagtttttg ggagggtgtt tgttatagat gattgtaaag gggtggggtt agtttgggga    1920
   ggattttgtg ggtgtggtgg tagttatggg tgaagtttga gggggtgggg tttgattggg    1980
   gtggggtttt tgggggtgga gttggttggg gatggagttg tttgggttga gttttttatt   2040
   gtttattatt attgagtttg gtttaggttt ttagtgtggt tgttggaagg ggtgggttag    2100
   tgggtgggta ttttttgttg gtgtttaggg ttgatgggga tgttggtatt ggtggggagt    2160
   tgttgggggg ttttgtattt agggtttttt ttttttgttt ttggtttttg tgaggggtgt   2220
   gtgttttttt ttgtttttta gtagttttgg tagggtttgg gtgtgtttgt gttttgtagt    2280
   gggaggtttt tgttgttttt tgtttttagt taggtattta ttgggttttt gtttgttgtt    2340
   tttggtttag gatgtgtagt gttggtatgg gttttgttgt ttttttgtat tttggtgttt   2400
   tagggatgtg gtaagatttg gttttgagta ttttttgggt gttggttttg aggttgattt    2460
   agttttgtat gtgggttgtg gggttttgtt gttgaaagga gagtttttaa ttaggagtat    2520
   gtatgggatt ttggtgggtt tttttgtttg gtttagatgt ttggtatttt aggttttggt    2580
   atagttagta ggtttttggt aggttgtgga tttagtaggt tgttttagtg tagattgttt    2640
   ttgaatttta agtttattaa tgttttagtt tttttatgag atttagattt ttgaaagtag    2700
   tggagggagg tgttagggtg agtgaggttt tttaggtagt ttttattgtt tatggatatt    2760
   tagagtttta tattataagt gaagggtttt ttgtttagag aggtgatgga gttatgttgg    2820
   agttgtatga tagaattaga attttagttt ttaggggtgt ggtaagtgtg ggttgtgttg    2880
   tttgtaatta gttaattatt tttgtttata ggtttgtgag gtgaggttaa gaaaattgtt    2940
   atggtttaat ttgtttttgt tttttgtgtg tggggttttt tgttttggtt tggtttggtt    3000
   t                                                                    3001

<210> 374
   <211> 3001
   <212> DNA
   <213> human
<400> 374
   aaattaaatt aaattaaaat aaaaaatttt atacgtaaaa aataaaaacg gattggatta     60
   tagcgatttt tttggtttta ttttataggt ttgtagatag gagtgattag ttagttataa    120
   atagcgtaat ttacgtttgt tatattttta aagattgggg ttttggtttt gtcgtgtaat    180
   tttagcgtag tttcgtcgtt tttttgggcg gaggattttt tatttgtaat atgaagtttt    240
   gggtattcgt gggcggtaag ggttgtttag aaaattttat ttattttggc gttttttttt    300
   attattttta gggatttggg ttttatggag agattggaat attagtgggt ttgagattta    360
   ggggtaattt gtattaaaat agtttgttgg gtttacggtt tgttaggggt ttgttagttg    420
   tgttaaggtt tgagatgtta ggtatttgag ttaggcggag gggttcgtta gggtttcgtg    480
   cgtgttttta gttagaggtt ttttttttaa cggtagagtt tcgcggttta cgtgtagagt    540
   taggtcggtt tcggggtcgg cgttcgggag gtgtttaaag tcgggttttg ttatattttt    600
   ggagcgtcgg ggtgcgggga ggcggcgggg tttatatcga cgttgcgcgt tttgggtcgg    660
   aggcggtagg taggagttta gtgagtattt ggttggagac gagaggcgac gaggattttt    720
   cgttgtagag cgcggacgcg ttcgggtttt attaggattg ttggagggcg gggagaggcg    780
   cgcgtttttc gcgggggtcg gaggtaggaa aggagagttt tagatgcggg attttttaat    840
   agttttttat tagtgttagc gttttcgtcg gttttaggta tcggtaggaa atgttcgttc    900
   gttgattcgt ttttttcggc ggtcgcgtta ggagtttggg tcggattcgg tgatggtggg    960
   cggtggaggg ttcggttcga gcggttttat ttttagtcga tttcgttttc gaagatttcg   1020
   tttcgattag gtttcgtttt tttaggtttt atttataatt gtcgttacgt tcgtagggtt   1080
   tttttcgagt tgatttcgtt tttttatagt cgtttatggt aggcgttttt tcgaaggttt   1140
   tttcgaaatt aggttttttt tcgagcgggt ttcgttttcg aaagtttttt ttcgagtcgg   1200
   ttttttttta tgtttcgttt ttttaggttt cgttttcgtg tttcgttttc gaaagttttt   1260
   ttttagttat ttttttatat gtttcgtttt taggtttcgt tttatgtttc gtttttaaaa   1320
   agtttcgttt cgaatcggta tttttatgtt tcgtttttag cggtttcgtt tcgtaaggtt   1380
   tagtttttag tagttcggtt tcgagtcgat tttgttacgt aggttgcgtt tattagattt   1440
   ttcggattcg acgcgcgtgg gtttttggtt ttagattttt aacggggagg tcgcggtcgc   1500
   gtgttgggag atttgttttt gaggacgttt ttcgttaatt tttagttttc gtcgattttt   1560
   agttttcgtt ttcgtcgatt tttagttttc gttttcgcgg cgtttacggt ttgtttttac   1620
   gggaagtcgt tgttttcgtt tttagcgcgg aaggagaatc gacgtttttt taggtcgcgg   1680
   gtttttagtt aggcgggtaa cgtttttatc gtcgtcgtta tcgcgtgcgc gttttggcga   1740
   gtcgtttttt attacgtggt ttttgtcgtt tttttcgtcg taggtagttt tttcgggatg   1800
   tgtttttagt cgttggttgt gtttatttta ttttttgttt gtttagatcg tattgagtcg   1860
   ttaggttcgt tagagtttcg cgtgttagtt ttttacgtat cgtaaattgg gtgttttaaa   1920
   acgatattcg tttattattt taaggttttt gtgggttaga aggttaggtt tagttgggtt   1980
   tttagttttg gttagtcgtt gtgtttttat ttgggggttg gattggggaa ggattatttt   2040
   ttattttatg cgattgttgg tagaagttat gttttggtgt ttgtaggatt gagggtcggt   2100
   aggagttgtt tttagttttc gggggttgtt tgttagtttt tattgtggga gggtgggcgg   2160
   tttatagggt ggtcgttttt ttagggttcg tgggagagtt ttatttttag atggagtttt   2220
   gtggaataga atttaattgt gggtgatatg ttattttttc ggtttagttt tatttagatt   2280
   taagttataa gtgtcgtttg tagttaaggg aaggatgggt attgggttgg ttttggtata   2340
   tttttttagg atatttgtag tattttgttt ttttaatatt ttttattttt attttttata   2400
   tgattttttt attatattta ttattaatta tttttcgttt ttatattttt atgtaaatta   2460
   gtttttggag ggtagttatt tgtttttgtt ttgggtgttt gatgtgtgtt gggttttcgt   2520
   gtcgtgtgga ttgagtgagt atgagtggag attttaggtg ttgttggtgt cgtggtattt    2580
   taggaattgt ttgtttttat ttagtttttt ttttaattgt tttttggatt tagtaaggtg   2640
   ttgaaaatta ggtagttttg tttttttttc gttttatttt ttttttttat tttgttgttt    2700
   ttatttttta atgttttaaa ttatttattt ttgtttagtt tgtttttttt gtttcggttg    2760
   taattttatt tttttttatt agtattattt ttgtgttttg ttttggggtt tttttgtttt   2820
   tatttgggtt tttttgttta ttttttttat agtagttgga gggattgatt tatcgggtaa   2880
   atttatagtg gtttttttag ttttacgttt gttttgtagg atgaggtttg ggttttttta    2940
   gtagttgtaa aggggtttgt atagatttgt aggtcgtttt ttttattatt tttttttttt   3000
   t                                                                   3001
<210> 375
   <211> 3001
   <212> DNA
   <213> human
<400> 375
   aaattaaatt aaattaaaat aaaaaatttt atatgtaaaa aataaaaatg gattggatta      60
   tagtgatttt tttggtttta ttttataggt ttgtagatag gagtgattag ttagttataa     120
   atagtgtaat ttatgtttgt tatattttta aagattgggg ttttggtttt gttgtgtaat     180
   tttagtgtag ttttgttgtt tttttgggtg gaggattttt tatttgtaat atgaagtttt     240
   gggtatttgt gggtggtaag ggttgtttag aaaattttat ttattttggt gttttttttt     300
   attattttta gggatttggg ttttatggag agattggaat attagtgggt ttgagattta     360
   ggggtaattt gtattaaaat agtttgttgg gtttatggtt tgttaggggt ttgttagttg     420
   tgttaaggtt tgagatgtta ggtatttgag ttaggtggag ggtttgttta gggttttgtg     480
   tgtgttttta gttagaggtt ttttttttaa tggtagagtt ttgtggttta tgtgtagagt     540
   taggttggtt ttggggttgg tgtttgggag gtgtttaaag ttgggttttg ttatattttt     600
   ggagtgttgg ggtgtgggga ggtggtgggg tttatattga tgttgtgtgt tttgggttgg     660
   aggtggtagg taggagttta gtgagtattt ggttggagat gagaggtgat gaggattttt     720
   tgttgtagag tgtggatgtg tttgggtttt attaggattg ttggagggtg gggagaggtg     780
   tgtgtttttt gtgggggttg gaggtaggaa aggagagttt tagatgtggg attttttaat      840
   agttttttat tagtgttagt gtttttgttg gttttaggta ttggtaggaa atgtttgttt      900
   gttgatttgt tttttttggt ggttgtgtta ggagtttggg ttggatttgg tgatggtggg     960
   tggtggaggg tttggtttga gtggttttat ttttagttga ttttgttttt gaagattttg    1020
   ttttgattag gttttgtttt tttaggtttt atttataatt gttgttatgt ttgtagggtt    1080
   ttttttgagt tgattttgtt tttttatagt tgtttatggt aggtgttttt ttgaaggttt    1140
   ttttgaaatt aggttttttt ttgagtgggt tttgtttttg aaagtttttt tttgagttgg    1200
   ttttttttta tgttttgttt ttttaggttt tgtttttgtg ttttgttttt gaaagttttt    1260
   ttttagttat ttttttatat gttttgtttt taggttttgt tttatgtttt gtttttaaaa    1320
   agttttgttt tgaattggta tttttatgtt ttgtttttag tggttttgtt ttgtaaggtt    1380
   tagtttttag tagtttggtt ttgagttgat tttgttatgt aggttgtgtt tattagattt    1440
   tttggatttg atgtgtgtgg gtttttggtt ttagattttt aatggggagg ttgtggttgt    1500
   gtgttgggag atttgttttt gaggatgttt tttgttaatt tttagttttt gttgattttt    1560
   agtttttgtt tttgttgatt tttagttttt gtttttgtgg tgtttatggt ttgtttttat    1620
   gggaagttgt tgtttttgtt tttagtgtgg aaggagaatt gatgtttttt taggttgtgg    1680
   gtttttagtt aggtgggtaa tgtttttatt gttgttgtta ttgtgtgtgt gttttggtga    1740
   gttgtttttt attatgtggt ttttgttgtt ttttttgttg taggtagttt ttttgggatg   1800
   tgtttttagt tgttggttgt gtttatttta ttttttgttt gtttagattg tattgagttg   1860
   ttaggtttgt tagagttttg tgtgttagtt ttttatgtat tgtaaattgg gtgttttaaa    1920
   atgatatttg tttattattt taaggttttt gtgggttaga aggttaggtt tagttgggtt    1980
   tttagttttg gttagttgtt gtgtttttat ttgggggttg gattggggaa ggattatttt    2040
   ttattttatg tgattgttgg tagaagttat gttttggtgt ttgtaggatt gagggttggt    2100
   aggagttgtt tttagttttt gggggttgtt tgttagtttt tattgtggga gggtgggtgg    2160
   tttatagggt ggttgttttt ttagggtttg tgggagagtt ttatttttag atggagtttt    2220
   gtggaataga atttaattgt gggtgatatg ttattttttt ggtttagttt tatttagatt    2280
   taagttataa gtgttgtttg tagttaaggg aaggatgggt attgggttgg ttttggtata    2340
   tttttttagg atatttgtag tattttgttt ttttaatatt ttttattttt attttttata   2400
   tgattttttt attatattta ttattaatta ttttttgttt ttatattttt atgtaaatta   2460
   gtttttggag ggtagttatt tgtttttgtt ttgggtgttt gatgtgtgtt gggtttttgt    2520
   gttgtgtgga ttgagtgagt atgagtggag attttaggtg ttgttggtgt tgtggtattt    2580
   taggaattgt ttgtttttat ttagtttttt ttttaattgt tttttggatt tagtaaggtg    2640
   ttgaaaatta ggtagttttg tttttttttt gttttatttt ttttttttat tttgttgttt    2700
   ttatttttta atgttttaaa ttatttattt ttgtttagtt tgtttttttt gttttggttg   2760
   taattttatt tttttttatt agtattattt ttgtgttttg ttttggggtt tttttgtttt   2820
   tatttgggtt tttttgttta ttttttttat agtagttgga gggattgatt tattgggtaa    2880
   atttatagtg gtttttttag ttttatgttt gttttgtagg atgaggtttg ggttttttta   2940
   gtagttgtaa aggggtttgt atagatttgt aggttgtttt ttttattatt tttttttttt    3000
   t                                                                    3001
<210> 376
   <211> 50
   <212> DNA
   <213> human
<400> 376
   tgcagacgtc cccacagagg gcagtgccga ggacagtgtg tgtgcagacg 50
<210> 377
   <211> 50
   <212> DNA
   <213> human
<400> 377
   tgtagacgtt tttatagagg gtagtgtcga ggatagtgtg tgtgtagacg 50
<210> 378
   <211> 50
   <212> DNA
   <213> human
<400> 378
   tgtagatgtt tttatagagg gtagtgttga ggatagtgtg tgtgtagatg 50
<210> 379
   <211> 50
   <212> DNA
   <213> human
<400> 379
   cgtttgtata tatattgttt tcggtattgt tttttgtggg gacgtttgta 50
<210> 380
   <211> 50
   <212> DNA
   <213> human
<400> 380
   tgtttgtata tatattgttt ttggtattgt tttttgtggg gatgtttgta 50
<210> 381
   <211> 3001
   <212> DNA
   <213> human
<400> 381
   ttaaaggtta aaggtggcct tttgttaaag agggagacac agagagagat ttatctttag      60
   ggaattggct tgtgtgaccg tggaagccgg caggtccaag gtctgcaggg tggaccctcg     120
   gctggagacc cggggaagag ccagcactgc cagtctagtc tgaagccgtc ggacgcagag     180
   ttccttcctg gtgcagggtg ttatctctgt tctagtcagg ccttcaacgg atcggaggag     240
   acccacccac gctatgcagg ctcatcttct ttcctcacag cccacccatc caacgccttc     300
   cagaggcctc tagagtaatg tcccatcaga cccgggcacc gtggcccagc ccagtggagg     360
   aatacagcta accaccacac acgctcaact ctgaaaatcc tcagggattc caaatgttct     420
   ttactaccaa ttttctcaag tgtttcggct gtgtgaaatt tagcagaatt ggtcattgaa     480
   tcattggaaa caaaaaataa aacctattga gtgactgttt gtgaaaattt acaaaagcac     540
   agtcaggcct ccagagcgag tgctcctaac ccgagctggg ccaaagacac tgtgtgcaga     600
   cgtcccggag ggcagtgccg aggacactgt gagtgcagac gtccccacgg agggcagtgc     660
   cgaggacatt ctgtgagtgc agacgtcccc acggagggca gtgccgagga cactgtgagt     720
   gcagacgtcc ccacggaggg cagtgccgag gacactgtga gtgcagacgt ccccacggag     780
   ggcagtgccg aggacattct gtgagtgcag acgtccccac ggagggcagt gccgaggaca     840
   ctgtgagtgc agacgtcccc atggagggca gtgccgagga cattctgtga gtgcagacgt     900
   ccccacggag ggcagtgccg aggacactgt gagtgcagac gtccccacgg agggcagtgc     960
   cgaggacact gtgagtgcag acgtccccac ggagggcagt gccgaggaca ttccgtgagt    1020
   gcagacgtcc ccacggaggg cagtgccgag gacattctgt gagtgcagac gtccccacgg    1080
   agggcagtgc cgaggacact gtgagtgcag acgtccccac ggagggcagt gccgaggaca    1140
   ttctgtgagt gcagacgtcc ccatttacgg agggcagtgc cgaggacact gtgagtgcag    1200
   acgtccccac ggagggcagt gccgaggaca gtgtgtgtgc agacgtcccc acggagggca    1260
   gtgccgagga cactgtgagt gcagacgtcc ccatggacgg gcagtgccga ggacactgtg    1320
   agtgcagacg tccccacgga gggcagtgcc gaggacagtg tgtgtgcaga cgtccccacg    1380
   gagggcagtg ccgaggacac tgtgagtgca gacgtcccca tggacgggca gtgccgagga    1440
   cattctgtga gtgcagacgt ccccacagag ggcagtgccg aggacagtgt gtgtgcagac    1500
   gtccccattt acagagggca gtgccgagga cactgtgagt gcagacgtcc ccatttacgg    1560
   agggcagtgc cgaggacatt ctgtgagtgc agacgtcccc atggacgggc agtgccgagg    1620
   acagtgtgtg tgcagacgtc cccacggagg gcagtgccga ggacactgtg agtgcagacg    1680
   tccccatgga cgggcagtgc tgaagtcctg atgagcgagt ctgaggggaa cggtgccact    1740
   gcggcagcac cccctcgccc caggccctca gatgtgtgag ggctgggaaa tcccctctct    1800
   ctctctggaa gtgcacaggg tcccctcccc taaggagtca ccaggatgcc tcaaagggtt    1860
   ctcttcccct ctcacccagc tccagccttg ggagtcctct cacgctggga ccctccgttg    1920
   ccggggcctg ggacgtgtct tccgccctct tgctgactgt ctcccatgct gtctgccacc    1980
   tccgccttcc caggcccttc tcaaatgtta gaacttcctg cctgatcctt tgatcactca    2040
   ctgtgtttcg cattcttttt tctctcagga agagctctgt atgaatccag tctcacattg    2100
   ctataaagaa ataccccaga ctgggtaatt tatagagaaa agaggtttag ttggctcaca    2160
   gttttgcaga cttcacagga agcatggcag cttctgcttc tggggaggcc tcgggaagct    2220
   tccaatcatg gtggaaggca aagcaggaac aggcgtctca cagggtcaga gcaggagcaa    2280
   gagcgaggtg ggagctgcca cacactttta aatgaccaga tctcatgaga acgcactcac    2340
   tgtctcgagg acagtgccaa gggatggtgc tagctcattc atgagaatcc accctgagat    2400
   ccaagcacct cccgccaggc cccacctcca acgctgggga ctaagagtca acaggagatt    2460
   ttgtgcggac acggagccaa gccttatcaa gctcccacac ttaatacttg gataattatt    2520
   ctcagcaatc tgttcttgtt ccatgaatgc atagtcccat catctcttgg agggtagtca    2580
   ttatattttt tggaaattat ccgtattctc tgattatttg tttcctctgg gcttttttga   2640
   tttctttatt ttggttttta tcttgactgc tacctgtctg tactcgttta aaaaggaagg    2700
   ctttgagtgt tgctctacca agtaacccca taccaccctc caacagaaaa caaccgaaag   2760
   tctggaaaaa gatgtaagaa aacatccttg aagatgcgag agggctgata gtaaggaagg    2820
   ggctgctgag tcagaatatg ctgtagaaca gggccccggt ggggagtgct cactcaggga    2880
   caagcctccc ctggggctgt ttcccaacgg tagagccgca gaggctgagc attggtgagg    2940
   ggcttggggc cgagcccaga tccacatgac cccaggctgg tccgccccgt gaacacagct    3000
   c                                                                    3001
<210> 382
   <211> 3001
   <212> DNA
   <213> human
<400> 382
   ttaaaggtta aaggtggttt tttgttaaag agggagatat agagagagat ttatttttag     60
   ggaattggtt tgtgtgatcg tggaagtcgg taggtttaag gtttgtaggg tggattttcg    120
   gttggagatt cggggaagag ttagtattgt tagtttagtt tgaagtcgtc ggacgtagag    180
   tttttttttg gtgtagggtg ttatttttgt tttagttagg tttttaacgg atcggaggag    240
   atttatttac gttatgtagg tttatttttt ttttttatag tttatttatt taacgttttt    300
   tagaggtttt tagagtaatg ttttattaga ttcgggtatc gtggtttagt ttagtggagg    360
   aatatagtta attattatat acgtttaatt ttgaaaattt ttagggattt taaatgtttt    420
   ttattattaa tttttttaag tgtttcggtt gtgtgaaatt tagtagaatt ggttattgaa    480
   ttattggaaa taaaaaataa aatttattga gtgattgttt gtgaaaattt ataaaagtat    540
   agttaggttt ttagagcgag tgtttttaat tcgagttggg ttaaagatat tgtgtgtaga    600
   cgtttcggag ggtagtgtcg aggatattgt gagtgtagac gtttttacgg agggtagtgt    660
   cgaggatatt ttgtgagtgt agacgttttt acggagggta gtgtcgagga tattgtgagt    720
   gtagacgttt ttacggaggg tagtgtcgag gatattgtga gtgtagacgt ttttacggag    780
   ggtagtgtcg aggatatttt gtgagtgtag acgtttttac ggagggtagt gtcgaggata    840
   ttgtgagtgt agacgttttt atggagggta gtgtcgagga tattttgtga gtgtagacgt    900
   ttttacggag ggtagtgtcg aggatattgt gagtgtagac gtttttacgg agggtagtgt    960
   cgaggatatt gtgagtgtag acgtttttac ggagggtagt gtcgaggata tttcgtgagt   1020
   gtagacgttt ttacggaggg tagtgtcgag gatattttgt gagtgtagac gtttttacgg   1080
   agggtagtgt cgaggatatt gtgagtgtag acgtttttac ggagggtagt gtcgaggata   1140
   ttttgtgagt gtagacgttt ttatttacgg agggtagtgt cgaggatatt gtgagtgtag   1200
   acgtttttac ggagggtagt gtcgaggata gtgtgtgtgt agacgttttt acggagggta   1260
   gtgtcgagga tattgtgagt gtagacgttt ttatggacgg gtagtgtcga ggatattgtg   1320
   agtgtagacg tttttacgga gggtagtgtc gaggatagtg tgtgtgtaga cgtttttacg   1380
   gagggtagtg tcgaggatat tgtgagtgta gacgttttta tggacgggta gtgtcgagga   1440
   tattttgtga gtgtagacgt ttttatagag ggtagtgtcg aggatagtgt gtgtgtagac   1500
   gtttttattt atagagggta gtgtcgagga tattgtgagt gtagacgttt ttatttacgg   1560
   agggtagtgt cgaggatatt ttgtgagtgt agacgttttt atggacgggt agtgtcgagg   1620
   atagtgtgtg tgtagacgtt tttacggagg gtagtgtcga ggatattgtg agtgtagacg   1680
   tttttatgga cgggtagtgt tgaagttttg atgagcgagt ttgaggggaa cggtgttatt   1740
   gcggtagtat tttttcgttt taggttttta gatgtgtgag ggttgggaaa tttttttttt   1800
   ttttttggaa gtgtataggg tttttttttt taaggagtta ttaggatgtt ttaaagggtt   1860
   tttttttttt tttatttagt tttagttttg ggagtttttt tacgttggga tttttcgttg   1920
   tcggggtttg ggacgtgttt ttcgtttttt tgttgattgt tttttatgtt gtttgttatt   1980
   ttcgtttttt taggtttttt ttaaatgtta gaattttttg tttgattttt tgattattta   2040
   ttgtgtttcg tatttttttt ttttttagga agagttttgt atgaatttag ttttatattg   2100
   ttataaagaa atattttaga ttgggtaatt tatagagaaa agaggtttag ttggtttata   2160
   gttttgtaga ttttatagga agtatggtag tttttgtttt tggggaggtt tcgggaagtt   2220
   tttaattatg gtggaaggta aagtaggaat aggcgtttta tagggttaga gtaggagtaa   2280
   gagcgaggtg ggagttgtta tatattttta aatgattaga ttttatgaga acgtatttat   2340
   tgtttcgagg atagtgttaa gggatggtgt tagtttattt atgagaattt attttgagat   2400
   ttaagtattt ttcgttaggt tttattttta acgttgggga ttaagagtta ataggagatt   2460
   ttgtgcggat acggagttaa gttttattaa gtttttatat ttaatatttg gataattatt   2520
   tttagtaatt tgtttttgtt ttatgaatgt atagttttat tattttttgg agggtagtta   2580
   ttatattttt tggaaattat tcgtattttt tgattatttg ttttttttgg gtttttttga   2640
   tttttttatt ttggttttta ttttgattgt tatttgtttg tattcgttta aaaaggaagg   2700
   ttttgagtgt tgttttatta agtaatttta tattattttt taatagaaaa taatcgaaag   2760
   tttggaaaaa gatgtaagaa aatatttttg aagatgcgag agggttgata gtaaggaagg   2820
   ggttgttgag ttagaatatg ttgtagaata gggtttcggt ggggagtgtt tatttaggga   2880
   taagtttttt ttggggttgt tttttaacgg tagagtcgta gaggttgagt attggtgagg   2940
   ggtttggggt cgagtttaga tttatatgat tttaggttgg ttcgtttcgt gaatatagtt   3000
   t                                                                   3001
<210> 383
   <211> 3001
   <212> DNA
   <213> human
<400> 383
   ttaaaggtta aaggtggttt tttgttaaag agggagatat agagagagat ttatttttag      60
   ggaattggtt tgtgtgattg tggaagttgg taggtttaag gtttgtaggg tggatttttg     120
   gttggagatt tggggaagag ttagtattgt tagtttagtt tgaagttgtt ggatgtagag     180
   tttttttttg gtgtagggtg ttatttttgt tttagttagg tttttaatgg attggaggag     240
   atttatttat gttatgtagg tttatttttt ttttttatag tttatttatt taatgttttt     300
   tagaggtttt tagagtaatg ttttattaga tttgggtatt gtggtttagt ttagtggagg     360
   aatatagtta attattatat atgtttaatt ttgaaaattt ttagggattt taaatgtttt     420
   ttattattaa tttttttaag tgttttggtt gtgtgaaatt tagtagaatt ggttattgaa     480
   ttattggaaa taaaaaataa aatttattga gtgattgttt gtgaaaattt ataaaagtat     540
   agttaggttt ttagagtgag tgtttttaat ttgagttggg ttaaagatat tgtgtgtaga     600
   tgttttggag ggtagtgttg aggatattgt gagtgtagat gtttttatgg agggtagtgt     660
   tgaggatatt ttgtgagtgt agatgttttt atggagggta gtgttgagga tattgtgagt     720
   gtagatgttt ttatggaggg tagtgttgag gatattgtga gtgtagatgt ttttatggag     780
   ggtagtgttg aggatatttt gtgagtgtag atgtttttat ggagggtagt gttgaggata     840
   ttgtgagtgt agatgttttt atggagggta gtgttgagga tattttgtga gtgtagatgt     900
   ttttatggag ggtagtgttg aggatattgt gagtgtagat gtttttatgg agggtagtgt     960
   tgaggatatt gtgagtgtag atgtttttat ggagggtagt gttgaggata ttttgtgagt    1020
   gtagatgttt ttatggaggg tagtgttgag gatattttgt gagtgtagat gtttttatgg    1080
   agggtagtgt tgaggatatt gtgagtgtag atgtttttat ggagggtagt gttgaggata    1140
   ttttgtgagt gtagatgttt ttatttatgg agggtagtgt tgaggatatt gtgagtgtag    1200
   atgtttttat ggagggtagt gttgaggata gtgtgtgtgt agatgttttt atggagggta    1260
   gtgttgagga tattgtgagt gtagatgttt ttatggatgg gtagtgttga ggatattgtg    1320
   agtgtagatg tttttatgga gggtagtgtt gaggatagtg tgtgtgtaga tgtttttatg    1380
   gagggtagtg ttgaggatat tgtgagtgta gatgttttta tggatgggta gtgttgagga    1440
   tattttgtga gtgtagatgt ttttatagag ggtagtgttg aggatagtgt gtgtgtagat    1500
   gtttttattt atagagggta gtgttgagga tattgtgagt gtagatgttt ttatttatgg    1560
   agggtagtgt tgaggatatt ttgtgagtgt agatgttttt atggatgggt agtgttgagg    1620
   atagtgtgtg tgtagatgtt tttatggagg gtagtgttga ggatattgtg agtgtagatg    1680
   tttttatgga tgggtagtgt tgaagttttg atgagtgagt ttgaggggaa tggtgttatt    1740
   gtggtagtat ttttttgttt taggttttta gatgtgtgag ggttgggaaa tttttttttt    1800
   ttttttggaa gtgtataggg tttttttttt taaggagtta ttaggatgtt ttaaagggtt    1860
   tttttttttt tttatttagt tttagttttg ggagtttttt tatgttggga ttttttgttg    1920
   ttggggtttg ggatgtgttt tttgtttttt tgttgattgt tttttatgtt gtttgttatt    1980
   tttgtttttt taggtttttt ttaaatgtta gaattttttg tttgattttt tgattattta    2040
   ttgtgttttg tatttttttt ttttttagga agagttttgt atgaatttag ttttatattg    2100
   ttataaagaa atattttaga ttgggtaatt tatagagaaa agaggtttag ttggtttata    2160
   gttttgtaga ttttatagga agtatggtag tttttgtttt tggggaggtt ttgggaagtt    2220
   tttaattatg gtggaaggta aagtaggaat aggtgtttta tagggttaga gtaggagtaa    2280
   gagtgaggtg ggagttgtta tatattttta aatgattaga ttttatgaga atgtatttat    2340
   tgttttgagg atagtgttaa gggatggtgt tagtttattt atgagaattt attttgagat    2400
   ttaagtattt tttgttaggt tttattttta atgttgggga ttaagagtta ataggagatt    2460
   ttgtgtggat atggagttaa gttttattaa gtttttatat ttaatatttg gataattatt    2520
   tttagtaatt tgtttttgtt ttatgaatgt atagttttat tattttttgg agggtagtta    2580
   ttatattttt tggaaattat ttgtattttt tgattatttg ttttttttgg gtttttttga    2640
   tttttttatt ttggttttta ttttgattgt tatttgtttg tatttgttta aaaaggaagg    2700
   ttttgagtgt tgttttatta agtaatttta tattattttt taatagaaaa taattgaaag    2760
   tttggaaaaa gatgtaagaa aatatttttg aagatgtgag agggttgata gtaaggaagg    2820
   ggttgttgag ttagaatatg ttgtagaata gggttttggt ggggagtgtt tatttaggga    2880
   taagtttttt ttggggttgt tttttaatgg tagagttgta gaggttgagt attggtgagg    2940
   ggtttggggt tgagtttaga tttatatgat tttaggttgg tttgttttgt gaatatagtt    3000
   t                                                                    3001
<210> 384
   <211> 3001
   <212> DNA
   <213> human
<400> 384
   gagttgtgtt tacggggcgg attagtttgg ggttatgtgg atttgggttc ggttttaagt     60
   tttttattaa tgtttagttt ttgcggtttt atcgttggga aatagtttta ggggaggttt    120
   gtttttgagt gagtattttt tatcggggtt ttgttttata gtatattttg atttagtagt    180
   ttttttttta ttattagttt tttcgtattt ttaaggatgt ttttttatat ttttttttag   240
   attttcggtt gttttttgtt ggagggtggt atggggttat ttggtagagt aatatttaaa   300
   gttttttttt ttaaacgagt atagataggt agtagttaag ataaaaatta aaataaagaa    360
   attaaaaaag tttagaggaa ataaataatt agagaatacg gataattttt aaaaaatata    420
   atgattattt tttaagagat gatgggatta tgtatttatg gaataagaat agattgttga    480
   gaataattat ttaagtatta agtgtgggag tttgataagg tttggtttcg tgttcgtata    540
   aaattttttg ttgattttta gtttttagcg ttggaggtgg ggtttggcgg gaggtgtttg    600
   gattttaggg tggattttta tgaatgagtt agtattattt tttggtattg ttttcgagat    660
   agtgagtgcg tttttatgag atttggttat ttaaaagtgt gtggtagttt ttatttcgtt    720
   tttgtttttg ttttgatttt gtgagacgtt tgtttttgtt ttgtttttta ttatgattgg    780
   aagtttttcg aggttttttt agaagtagaa gttgttatgt tttttgtgaa gtttgtaaaa    840
   ttgtgagtta attaaatttt ttttttttat aaattattta gtttggggta tttttttata   900
   gtaatgtgag attggattta tatagagttt tttttgagag aaaaaagaat gcgaaatata    960
   gtgagtgatt aaaggattag gtaggaagtt ttaatatttg agaagggttt gggaaggcgg   1020
   aggtggtaga tagtatggga gatagttagt aagagggcgg aagatacgtt ttaggtttcg   1080
   gtaacggagg gttttagcgt gagaggattt ttaaggttgg agttgggtga gaggggaaga   1140
   gaattttttg aggtattttg gtgatttttt aggggagggg attttgtgta tttttagaga  1200
   gagagagggg attttttagt ttttatatat ttgagggttt ggggcgaggg ggtgttgtcg   1260
   tagtggtatc gtttttttta gattcgttta ttaggatttt agtattgttc gtttatgggg   1320
   acgtttgtat ttatagtgtt ttcggtattg tttttcgtgg ggacgtttgt atatatattg   1380
   ttttcggtat tgttcgttta tggggacgtt tgtatttata gaatgttttc ggtattgttt   1440
   ttcgtaaatg gggacgtttg tatttatagt gttttcggta ttgttttttg taaatgggga  1500
   cgtttgtata tatattgttt tcggtattgt tttttgtggg gacgtttgta tttatagaat   1560
   gttttcggta ttgttcgttt atggggacgt ttgtatttat agtgttttcg gtattgtttt   1620
   tcgtggggac gtttgtatat atattgtttt cggtattgtt tttcgtgggg acgtttgtat   1680
   ttatagtgtt ttcggtattg ttcgtttatg gggacgtttg tatttatagt gttttcggta    1740
   ttgtttttcg tggggacgtt tgtatatata ttgttttcgg tattgttttt cgtggggacg   1800
   tttgtattta tagtgttttc ggtattgttt ttcgtaaatg gggacgtttg tatttataga   1860
   atgttttcgg tattgttttt cgtggggacg tttgtattta tagtgttttc ggtattgttt   1920
   ttcgtgggga cgtttgtatt tatagaatgt tttcggtatt gtttttcgtg gggacgtttg   1980
   tatttacgga atgttttcgg tattgttttt cgtggggacg tttgtattta tagtgttttc   2040
   ggtattgttt ttcgtgggga cgtttgtatt tatagtgttt tcggtattgt ttttcgtggg   2100
   gacgtttgta tttatagaat gttttcggta ttgtttttta tggggacgtt tgtatttata   2160
   gtgttttcgg tattgttttt cgtggggacg tttgtattta tagaatgttt tcggtattgt   2220
   ttttcgtggg gacgtttgta tttatagtgt tttcggtatt gtttttcgtg gggacgtttg   2280
   tatttatagt gttttcggta ttgtttttcg tggggacgtt tgtatttata gaatgttttc   2340
   ggtattgttt ttcgtgggga cgtttgtatt tatagtgttt tcggtattgt ttttcgggac   2400
   gtttgtatat agtgtttttg gtttagttcg ggttaggagt attcgttttg gaggtttgat   2460
   tgtgtttttg taaattttta taaatagtta tttaataggt tttatttttt gtttttaatg   2520
   atttaatgat taattttgtt aaattttata tagtcgaaat atttgagaaa attggtagta   2580
   aagaatattt ggaatttttg aggattttta gagttgagcg tgtgtggtgg ttagttgtat  2640
   ttttttattg ggttgggtta cggtgttcgg gtttgatggg atattatttt agaggttttt   2700
   ggaaggcgtt ggatgggtgg gttgtgagga aagaagatga gtttgtatag cgtgggtggg   2760
   tttttttcga ttcgttgaag gtttgattag aatagagata atattttgta ttaggaagga  2820
   attttgcgtt cgacggtttt agattagatt ggtagtgttg gttttttttc gggtttttag   2880
   tcgagggttt attttgtaga ttttggattt gtcggttttt acggttatat aagttaattt   2940
   tttaaagata aatttttttt tgtgtttttt tttttaataa aaggttattt ttaattttta  3000
   a                                                                   3001
<210> 385
   <211> 3001
   <212> DNA
   <213> human
<400> 385
   gagttgtgtt tatggggtgg attagtttgg ggttatgtgg atttgggttt ggttttaagt     60
   tttttattaa tgtttagttt ttgtggtttt attgttggga aatagtttta ggggaggttt    120
   gtttttgagt gagtattttt tattggggtt ttgttttata gtatattttg atttagtagt    180
   ttttttttta ttattagttt ttttgtattt ttaaggatgt ttttttatat ttttttttag 240
   atttttggtt gttttttgtt ggagggtggt atggggttat ttggtagagt aatatttaaa 300
   gttttttttt ttaaatgagt atagataggt agtagttaag ataaaaatta aaataaagaa 360
   attaaaaaag tttagaggaa ataaataatt agagaatatg gataattttt aaaaaatata 420
   atgattattt tttaagagat gatgggatta tgtatttatg gaataagaat agattgttga 480
   gaataattat ttaagtatta agtgtgggag tttgataagg tttggttttg tgtttgtata 540
   aaattttttg ttgattttta gtttttagtg ttggaggtgg ggtttggtgg gaggtgtttg 600
   gattttaggg tggattttta tgaatgagtt agtattattt tttggtattg tttttgagat 660
   agtgagtgtg tttttatgag atttggttat ttaaaagtgt gtggtagttt ttattttgtt 720
   tttgtttttg ttttgatttt gtgagatgtt tgtttttgtt ttgtttttta ttatgattgg 780
   aagttttttg aggttttttt agaagtagaa gttgttatgt tttttgtgaa gtttgtaaaa 840
   ttgtgagtta attaaatttt ttttttttat aaattattta gtttggggta tttttttata 900
   gtaatgtgag attggattta tatagagttt tttttgagag aaaaaagaat gtgaaatata 960
   gtgagtgatt aaaggattag gtaggaagtt ttaatatttg agaagggttt gggaaggtgg 1020
   aggtggtaga tagtatggga gatagttagt aagagggtgg aagatatgtt ttaggttttg 1080
   gtaatggagg gttttagtgt gagaggattt ttaaggttgg agttgggtga gaggggaaga 1140
   gaattttttg aggtattttg gtgatttttt aggggagggg attttgtgta tttttagaga 1200
   gagagagggg attttttagt ttttatatat ttgagggttt ggggtgaggg ggtgttgttg 1260
   tagtggtatt gtttttttta gatttgttta ttaggatttt agtattgttt gtttatgggg 1320
   atgtttgtat ttatagtgtt tttggtattg ttttttgtgg ggatgtttgt atatatattg 1380
   tttttggtat tgtttgttta tggggatgtt tgtatttata gaatgttttt ggtattgttt 1440
   tttgtaaatg gggatgtttg tatttatagt gtttttggta ttgttttttg taaatgggga 1500
   tgtttgtata tatattgttt ttggtattgt tttttgtggg gatgtttgta tttatagaat 1560
   gtttttggta ttgtttgttt atggggatgt ttgtatttat agtgtttttg gtattgtttt 1620
   ttgtggggat gtttgtatat atattgtttt tggtattgtt ttttgtgggg atgtttgtat 1680
   ttatagtgtt tttggtattg tttgtttatg gggatgtttg tatttatagt gtttttggta 1740
   ttgttttttg tggggatgtt tgtatatata ttgtttttgg tattgttttt tgtggggatg 1800
   tttgtattta tagtgttttt ggtattgttt tttgtaaatg gggatgtttg tatttataga 1860
   atgtttttgg tattgttttt tgtggggatg tttgtattta tagtgttttt ggtattgttt 1920
   tttgtgggga tgtttgtatt tatagaatgt ttttggtatt gttttttgtg gggatgtttg 1980
   tatttatgga atgtttttgg tattgttttt tgtggggatg tttgtattta tagtgttttt 2040
   ggtattgttt tttgtgggga tgtttgtatt tatagtgttt ttggtattgt tttttgtggg 2100
   gatgtttgta tttatagaat gtttttggta ttgtttttta tggggatgtt tgtatttata 2160
   gtgtttttgg tattgttttt tgtggggatg tttgtattta tagaatgttt ttggtattgt 2220
   tttttgtggg gatgtttgta tttatagtgt ttttggtatt gttttttgtg gggatgtttg 2280
   tatttatagt gtttttggta ttgttttttg tggggatgtt tgtatttata gaatgttttt 2340
   ggtattgttt tttgtgggga tgtttgtatt tatagtgttt ttggtattgt tttttgggat 2400
   gtttgtatat agtgtttttg gtttagtttg ggttaggagt atttgttttg gaggtttgat 2460
   tgtgtttttg taaattttta taaatagtta tttaataggt tttatttttt gtttttaatg 2520
   atttaatgat taattttgtt aaattttata tagttgaaat atttgagaaa attggtagta 2580
   aagaatattt ggaatttttg aggattttta gagttgagtg tgtgtggtgg ttagttgtat 2640
   ttttttattg ggttgggtta tggtgtttgg gtttgatggg atattatttt agaggttttt 2700
   ggaaggtgtt ggatgggtgg gttgtgagga aagaagatga gtttgtatag tgtgggtggg 2760
   ttttttttga tttgttgaag gtttgattag aatagagata atattttgta ttaggaagga 2820
   attttgtgtt tgatggtttt agattagatt ggtagtgttg gttttttttt gggtttttag 2880
   ttgagggttt attttgtaga ttttggattt gttggttttt atggttatat aagttaattt 2940
   tttaaagata aatttttttt tgtgtttttt tttttaataa aaggttattt ttaattttta 3000
   a 3001
<210> 386
   <211> 50
   <212> DNA
   <213> human
<400> 386
   tgcacaaggc agggtcactc cagatggcca tgggagctgt gcttacctcg 50
<210> 387
   <211> 50
   <212> DNA
   <213> human
<400> 387
   tgtataaggt agggttattt tagatggtta tgggagttgt gtttatttcg 50
<210> 388
   <211> 50
   <212> DNA
   <213> human
<400> 388
   tgtataaggt agggttattt tagatggtta tgggagttgt gtttattttg 50
<210> 389
   <211> 50
   <212> DNA
   <213> human
<400> 389
   cgaggtaagt atagttttta tggttatttg gagtgatttt gttttgtgta 50
<210> 390
   <211> 50
   <212> DNA
   <213> human
<400> 390
   tgaggtaagt atagttttta tggttatttg gagtgatttt gttttgtgta 50
<210> 391
   <211> 3001
   <212> DNA
   <213> human
<400> 391
   cctgggaccc cttccctttc ccttccccct gggactccct tcctccccct gggacccctt        60
   cctttccccc tgggactccc tccctccccc tgggacccct tcccttcccc ctgggactcc       120
   ctccctcccc ctgggacccc ttcccttccc cctgggattc cctccctccc cctgggaccc       180
   cttcccttcc ccctgggact ccctccctcc ccctgggacc ccttcccttc cccctgggac       240
   tccctccctc cccctgggac cccttccctt ccccctggga ctccctccct ccccctggga       300
   cccctccctt cccccctgga ttccctccct ccccctggga ccccttcccc ttccccctgg       360
   gactccctcc cctccccctg ggaccccttc ccttccccct gggactccct ccctccccct       420
   gggacccctt cccttccccc gggactccct cccttccccc gggactccct cctcccccag       480
   gactccctcc tctccttagg actccctccc tctccctggg acactctccc tacccctggg       540
   actcccttct ccccctagga ctccctccct tctcctggga ctccctcctc ccccaggact       600
   ccctcctcac cctggggcca ccccttcccc tgggactccc ccctcccccg ccactccctc       660
   ctctccctag gactccctcc ctccccctgg gactccctcc ctccccctgg ggttccctcc       720
   tccccgccag ggctccctct tcctcctggg gctccccctt tcccgggtct gccccttggc       780
   ctcacctctt ctctcccgtc ccccagctcc acatccagag gccggagatg gtagcatcca       840
   ctgtgtgagc caacgggggc ctcccaccct catctagctc tgttcaggag agctgcaaac       900
   acagagccca ccacaagcct ccggggcggg tcaagaggag accgaagtca ggccctgaag       960
   ccggtcctgc tctgagctga cagacttggc cagtcccctg cctgtgctcc tgctggggaa      1020
   ggctgggggc tgtaagcctc tccatccggg agcttccaga ctcccagaag cctcggcacc      1080
   cctgtctcct cctgggtggc tccccactct ggaatttccc taccaataaa agcaaatctg      1140
   aaagctcaga atgagaagtg agaggtttcc ttctcctcct tctgcagttg gggtcaggtt       1200
   aggggtcaga gccctcctgg gatgggattt ctcccaggac tggctgctga atggggcatg      1260
   gaacgaatgc tctgggcccc ttcctgtgtc tttgcaggga gccccctccc cggctccagt      1320
   caggacaggt ctaggcctgc ctctgctgag gtccctgtgg cagaaaagcc taatgtcagg      1380
   ttctggggac aggagaggac ctcagattca accccagttg gccagggagt gagcacttgg      1440
   ctctgctgag cttgcacaag gcagggtcac tccagatggc catgggagct gtgcttacct      1500
   cggaacccac agaccaaacc caagaactcc ctgcccaggc atggccatgt cttcagggca      1560
   tactcatagg acaataagaa gggttatttt atttatttat ttttgccgag acagagtctt      1620
   gctctgttgc ccaggctgga gtacggaggc acgatcttgg ctctctgcaa cctccgcctc      1680
   ccaggttcaa gcgattctcc tgcctcagcc tcctgagtag ctgggattac aggcgcccgc      1740
   caccaagtct ggctgatttt tgtattttta gtagagatgg tgtttcatca tgttggccag      1800
   gttggtctca aactcctcat ctcaagtgat cttcccgcgt cagcctccca aagtgctggg      1860
   attacaggtg tgagccacca tacccggcca ataagaaggg ttagaaaaga gacaaagggg      1920
   gtgtccctgc taggggttag ggtcaaatgc aggtggtacc ctcctccttt cccagctcag      1980
   gcccagtccc tcttgcccat ctcccaccac cctcggtcgg gggatctcat cagttccccc      2040
   ctggctccta caggccctct gggctgcccc tctgttgggg tgataaagct gataaccctc      2100
   atgcctatgg gctgccgggc cagggagagg caccccctcc ctctgccctt tggcctgaaa      2160
   caggccctgc gccccaagtc atgagggaag cttccagact gactcaggct caggctggcc      2220
   cctgatcccc agtgtctcag ggaccaaagg tctctggcac ccatttattt atcagtttcc      2280
   ttctctgagg ctcatttcgc cagctcctct gggggtgaca ggcaagtgag acgtgctcag      2340
   agctccgatg ccaaggccag ggaccatggc gctgtgtctg ctgaccttgg tcctctcgct      2400
   cttgccccca caagctgctg cagaacaggg tgagtgaccc ccacgccccg ctgcccaaga      2460
   ggcccccatc ccaggggcca gcctgctctg tctgcccatc cccgagggag agctgcctcc      2520
   aggacaccaa taggggtgtg ccctctgcct ggagctcagc cctgtgccta ccccctggac      2580
   ttctcttctc tccccagcga gctttggggg aagagccagg gcattggagc ctcttccagt      2640
   tgaggtctct gagccttagt ttccccttcc gagcctcagt ctcccctccg gaaagtaaga      2700
   ataataacac ctgcttttca gggcagctgc taggattgaa aagagctccc cccccgaggc      2760
   atgatccctg gcacaagact ggtgttcaac ccatattaga gtctatcctt acccagtgaa      2820
   tgttcccgcc aagtccctgt cacctcccac accagcacac cctgctgtgt gccccaggaa      2880
   tgtttccggg acatagccac atgggcacaa aatggcgttt caggagcccc agattctaat      2940
   cctgccatga accagctgtg caccctgggg ccgctcagtt catctcctgc ctcagtctcc      3000
   c                                                                      3001
<210> 392
   <211> 3001
   <212> DNA
   <213> human
<400> 392
   tttgggattt tttttttttt tttttttttt gggatttttt tttttttttt gggatttttt     60
   tttttttttt tgggattttt tttttttttt tgggattttt tttttttttt ttgggatttt    120
   tttttttttt ttgggatttt tttttttttt tttgggattt tttttttttt tttgggattt    180
   tttttttttt ttttgggatt tttttttttt ttttgggatt tttttttttt tttttgggat    240
   tttttttttt tttttgggat tttttttttt ttttttggga tttttttttt ttttttggga    300
   tttttttttt tttttttgga tttttttttt ttttttggga tttttttttt ttttttttgg    360
   gatttttttt tttttttttg ggattttttt tttttttttt gggatttttt tttttttttt    420
   gggatttttt tttttttttc gggatttttt tttttttttc gggatttttt ttttttttag    480
   gatttttttt tttttttagg attttttttt tttttttggg atattttttt tatttttggg    540
   attttttttt ttttttagga tttttttttt ttttttggga tttttttttt ttttaggatt    600
   ttttttttat tttggggtta tttttttttt tgggattttt ttttttttcg ttattttttt    660
   ttttttttag gatttttttt ttttttttgg gatttttttt ttttttttgg ggtttttttt    720
   ttttcgttag ggtttttttt tttttttggg gttttttttt tttcgggttt gttttttggt    780
   tttatttttt ttttttcgtt ttttagtttt atatttagag gtcggagatg gtagtattta    840
   ttgtgtgagt taacgggggt tttttatttt tatttagttt tgtttaggag agttgtaaat    900
   atagagttta ttataagttt tcggggcggg ttaagaggag atcgaagtta ggttttgaag    960
   tcggttttgt tttgagttga tagatttggt tagttttttg tttgtgtttt tgttggggaa   1020
   ggttgggggt tgtaagtttt tttattcggg agtttttaga tttttagaag tttcggtatt   1080
   tttgtttttt tttgggtggt tttttatttt ggaatttttt tattaataaa agtaaatttg   1140
   aaagtttaga atgagaagtg agaggttttt tttttttttt tttgtagttg gggttaggtt   1200
   aggggttaga gtttttttgg gatgggattt tttttaggat tggttgttga atggggtatg   1260
   gaacgaatgt tttgggtttt tttttgtgtt tttgtaggga gttttttttt cggttttagt   1320
   taggataggt ttaggtttgt ttttgttgag gtttttgtgg tagaaaagtt taatgttagg   1380
   ttttggggat aggagaggat tttagattta attttagttg gttagggagt gagtatttgg   1440
   ttttgttgag tttgtataag gtagggttat tttagatggt tatgggagtt gtgtttattt   1500
   cggaatttat agattaaatt taagaatttt ttgtttaggt atggttatgt ttttagggta   1560
   tatttatagg ataataagaa gggttatttt atttatttat ttttgtcgag atagagtttt   1620
   gttttgttgt ttaggttgga gtacggaggt acgattttgg ttttttgtaa ttttcgtttt   1680
   ttaggtttaa gcgatttttt tgttttagtt ttttgagtag ttgggattat aggcgttcgt   1740
   tattaagttt ggttgatttt tgtattttta gtagagatgg tgttttatta tgttggttag   1800
   gttggtttta aattttttat tttaagtgat tttttcgcgt tagtttttta aagtgttggg   1860
   attataggtg tgagttatta tattcggtta ataagaaggg ttagaaaaga gataaagggg   1920
   gtgtttttgt taggggttag ggttaaatgt aggtggtatt tttttttttt tttagtttag   1980
   gtttagtttt ttttgtttat tttttattat tttcggtcgg gggattttat tagttttttt   2040
   ttggttttta taggtttttt gggttgtttt tttgttgggg tgataaagtt gataattttt   2100
   atgtttatgg gttgtcgggt tagggagagg tatttttttt ttttgttttt tggtttgaaa   2160
   taggttttgc gttttaagtt atgagggaag tttttagatt gatttaggtt taggttggtt   2220
   tttgattttt agtgttttag ggattaaagg tttttggtat ttatttattt attagttttt   2280
   ttttttgagg tttatttcgt tagttttttt gggggtgata ggtaagtgag acgtgtttag   2340
   agtttcgatg ttaaggttag ggattatggc gttgtgtttg ttgattttgg ttttttcgtt   2400
   tttgttttta taagttgttg tagaataggg tgagtgattt ttacgtttcg ttgtttaaga   2460
   ggtttttatt ttaggggtta gtttgttttg tttgtttatt ttcgagggag agttgttttt   2520
   aggatattaa taggggtgtg ttttttgttt ggagtttagt tttgtgttta ttttttggat   2580
   tttttttttt tttttagcga gttttggggg aagagttagg gtattggagt tttttttagt   2640
   tgaggttttt gagttttagt tttttttttc gagttttagt ttttttttcg gaaagtaaga   2700
   ataataatat ttgtttttta gggtagttgt taggattgaa aagagttttt ttttcgaggt   2760
   atgatttttg gtataagatt ggtgtttaat ttatattaga gtttattttt atttagtgaa   2820
   tgttttcgtt aagtttttgt tattttttat attagtatat tttgttgtgt gttttaggaa   2880
   tgttttcggg atatagttat atgggtataa aatggcgttt taggagtttt agattttaat   2940
   tttgttatga attagttgtg tattttgggg tcgtttagtt tattttttgt tttagttttt   3000
   t                                                                   3001
<210> 393
   <211> 3001
   <212> DNA
   <213> human
<400> 393
   tttgggattt tttttttttt tttttttttt gggatttttt tttttttttt gggatttttt      60
   tttttttttt tgggattttt tttttttttt tgggattttt tttttttttt ttgggatttt     120
   tttttttttt ttgggatttt tttttttttt tttgggattt tttttttttt tttgggattt     180
   tttttttttt ttttgggatt tttttttttt ttttgggatt tttttttttt tttttgggat     240
   tttttttttt tttttgggat tttttttttt ttttttggga tttttttttt ttttttggga     300
   tttttttttt tttttttgga tttttttttt ttttttggga tttttttttt ttttttttgg     360
   gatttttttt tttttttttg ggattttttt tttttttttt gggatttttt tttttttttt     420
   gggatttttt tttttttttt gggatttttt tttttttttt gggatttttt ttttttttag     480
   gatttttttt tttttttagg attttttttt tttttttggg atattttttt tatttttggg     540
   attttttttt ttttttagga tttttttttt ttttttggga tttttttttt ttttaggatt     600
   ttttttttat tttggggtta tttttttttt tgggattttt tttttttttg ttattttttt     660
   ttttttttag gatttttttt ttttttttgg gatttttttt ttttttttgg ggtttttttt     720
   tttttgttag ggtttttttt tttttttggg gttttttttt ttttgggttt gttttttggt     780
   tttatttttt tttttttgtt ttttagtttt atatttagag gttggagatg gtagtattta     840
   ttgtgtgagt taatgggggt tttttatttt tatttagttt tgtttaggag agttgtaaat     900
   atagagttta ttataagttt ttggggtggg ttaagaggag attgaagtta ggttttgaag     960
   ttggttttgt tttgagttga tagatttggt tagttttttg tttgtgtttt tgttggggaa    1020
   ggttgggggt tgtaagtttt tttatttggg agtttttaga tttttagaag ttttggtatt    1080
   tttgtttttt tttgggtggt tttttatttt ggaatttttt tattaataaa agtaaatttg    1140
   aaagtttaga atgagaagtg agaggttttt tttttttttt tttgtagttg gggttaggtt    1200
   aggggttaga gtttttttgg gatgggattt tttttaggat tggttgttga atggggtatg    1260
   gaatgaatgt tttgggtttt tttttgtgtt tttgtaggga gttttttttt tggttttagt    1320
   taggataggt ttaggtttgt ttttgttgag gtttttgtgg tagaaaagtt taatgttagg    1380
   ttttggggat aggagaggat tttagattta attttagttg gttagggagt gagtatttgg    1440
   ttttgttgag tttgtataag gtagggttat tttagatggt tatgggagtt gtgtttattt    1500
   tggaatttat agattaaatt taagaatttt ttgtttaggt atggttatgt ttttagggta    1560
   tatttatagg ataataagaa gggttatttt atttatttat ttttgttgag atagagtttt    1620
   gttttgttgt ttaggttgga gtatggaggt atgattttgg ttttttgtaa tttttgtttt    1680
   ttaggtttaa gtgatttttt tgttttagtt ttttgagtag ttgggattat aggtgtttgt    1740
   tattaagttt ggttgatttt tgtattttta gtagagatgg tgttttatta tgttggttag    1800
   gttggtttta aattttttat tttaagtgat ttttttgtgt tagtttttta aagtgttggg    1860
   attataggtg tgagttatta tatttggtta ataagaaggg ttagaaaaga gataaagggg    1920
   gtgtttttgt taggggttag ggttaaatgt aggtggtatt tttttttttt tttagtttag    1980
   gtttagtttt ttttgtttat tttttattat ttttggttgg gggattttat tagttttttt    2040
   ttggttttta taggtttttt gggttgtttt tttgttgggg tgataaagtt gataattttt    2100
   atgtttatgg gttgttgggt tagggagagg tatttttttt ttttgttttt tggtttgaaa    2160
   taggttttgt gttttaagtt atgagggaag tttttagatt gatttaggtt taggttggtt    2220
   tttgattttt agtgttttag ggattaaagg tttttggtat ttatttattt attagttttt    2280
   ttttttgagg tttattttgt tagttttttt gggggtgata ggtaagtgag atgtgtttag    2340
   agttttgatg ttaaggttag ggattatggt gttgtgtttg ttgattttgg tttttttgtt    2400
   tttgttttta taagttgttg tagaataggg tgagtgattt ttatgttttg ttgtttaaga    2460
   ggttttattt ttaggggtta gtttgttttg tttgtttatt tttgagggag agttgttttt    2520
   aggatattaa taggggtgtg ttttttgttt ggagtttagt tttgtgttta ttttttggat    2580
   tttttttttt tttttagtga gttttggggg aagagttagg gtattggagt tttttttagt    2640
   tgaggttttt gagttttagt tttttttttt gagttttagt tttttttttg gaaagtaaga    2700
   ataataatat ttgtttttta gggtagttgt taggattgaa aagagttttt tttttgaggt    2760
   atgatttttg gtataagatt ggtgtttaat ttatattaga gtttattttt atttagtgaa    2820
   tgtttttgtt aagtttttgt tattttttat attagtatat tttgttgtgt gttttaggaa    2880
   tgtttttggg atatagttat atgggtataa aatggtgttt taggagtttt agattttaat    2940
   tttgttatga attagttgtg tattttgggg ttgtttagtt tattttttgt tttagttttt    3000
   t                                                                   3001
<210> 394
   <211> 3001
   <212> DNA
   <213> human
<400> 394
   gggagattga ggtaggagat gaattgagcg gttttagggt gtatagttgg tttatggtag     60
   gattagaatt tggggttttt gaaacgttat tttgtgttta tgtggttatg tttcggaaat    120
   atttttgggg tatatagtag ggtgtgttgg tgtgggaggt gatagggatt tggcgggaat    180
   atttattggg taaggataga ttttaatatg ggttgaatat tagttttgtg ttagggatta    240
   tgtttcgggg ggggagtttt ttttaatttt agtagttgtt ttgaaaagta ggtgttatta    300
   tttttatttt tcggagggga gattgaggtt cggaagggga aattaaggtt tagagatttt    360
   aattggaaga ggttttaatg ttttggtttt ttttttaaag ttcgttgggg agagaagaga    420
   agtttagggg gtaggtatag ggttgagttt taggtagagg gtatattttt attggtgttt    480
   tggaggtagt tttttttcgg ggatgggtag atagagtagg ttggtttttg ggatgggggt    540
   tttttgggta gcggggcgtg ggggttattt attttgtttt gtagtagttt gtgggggtaa    600
   gagcgagagg attaaggtta gtagatatag cgttatggtt tttggttttg gtatcggagt    660
   tttgagtacg ttttatttgt ttgttatttt tagaggagtt ggcgaaatga gttttagaga    720
   aggaaattga taaataaatg ggtgttagag atttttggtt tttgagatat tggggattag    780
   gggttagttt gagtttgagt tagtttggaa gtttttttta tgatttgggg cgtagggttt    840
   gttttaggtt aaagggtaga gggagggggt gttttttttt ggttcggtag tttataggta    900
   tgagggttat tagttttatt attttaatag aggggtagtt tagagggttt gtaggagtta    960
   ggggggaatt gatgagattt ttcgatcgag ggtggtggga gatgggtaag agggattggg   1020
   tttgagttgg gaaaggagga gggtattatt tgtatttgat tttaattttt agtagggata   1080
   ttttttttgt ttttttttta atttttttta ttggtcgggt atggtggttt atatttgtaa   1140
   ttttagtatt ttgggaggtt gacgcgggaa gattatttga gatgaggagt ttgagattaa   1200
   tttggttaat atgatgaaat attattttta ttaaaaatat aaaaattagt tagatttggt   1260
   ggcgggcgtt tgtaatttta gttatttagg aggttgaggt aggagaatcg tttgaatttg   1320
   ggaggcggag gttgtagaga gttaagatcg tgttttcgta ttttagtttg ggtaatagag   1380
   taagattttg tttcggtaaa aataaataaa taaaataatt ttttttattg ttttatgagt    1440
   atgttttgaa gatatggtta tgtttgggta gggagttttt gggtttggtt tgtgggtttc   1500
   gaggtaagta tagtttttat ggttatttgg agtgattttg ttttgtgtaa gtttagtaga   1560
   gttaagtgtt tattttttgg ttaattgggg ttgaatttga ggtttttttt tgtttttaga   1620
   atttgatatt aggttttttt gttataggga ttttagtaga ggtaggttta gatttgtttt   1680
   gattggagtc ggggaggggg ttttttgtaa agatatagga aggggtttag agtattcgtt    1740
   ttatgtttta tttagtagtt agttttggga gaaattttat tttaggaggg ttttgatttt   1800
   taatttgatt ttaattgtag aaggaggaga aggaaatttt ttatttttta ttttgagttt    1860
   ttagatttgt ttttattggt agggaaattt tagagtgggg agttatttag gaggagatag   1920
   gggtgtcgag gtttttggga gtttggaagt tttcggatgg agaggtttat agtttttagt   1980
   tttttttagt aggagtatag gtaggggatt ggttaagttt gttagtttag agtaggatcg  2040
   gttttagggt ttgatttcgg tttttttttg attcgtttcg gaggtttgtg gtgggttttg   2100
   tgtttgtagt ttttttgaat agagttagat gagggtggga ggttttcgtt ggtttatata   2160
   gtggatgtta ttattttcgg tttttggatg tggagttggg ggacgggaga gaagaggtga   2220
   ggttaagggg tagattcggg aaagggggag ttttaggagg aagagggagt tttggcgggg   2280
   aggagggaat tttaggggga gggagggagt tttaggggga gggagggagt tttagggaga   2340
   ggagggagtg gcgggggagg ggggagtttt aggggaaggg gtggttttag ggtgaggagg   2400
   gagttttggg ggaggaggga gttttaggag aagggaggga gttttagggg gagaagggag   2460
   ttttaggggt agggagagtg ttttagggag agggagggag ttttaaggag aggagggagt   2520
   tttgggggag gagggagttt cgggggaagg gagggagttt cgggggaagg gaaggggttt   2580
   tagggggagg gagggagttt tagggggaag ggaaggggtt ttagggggag gggagggagt   2640
   tttaggggga aggggaaggg gttttagggg gagggaggga atttaggggg gaagggaggg   2700
   gttttagggg gagggaggga gttttagggg gaagggaagg ggttttaggg ggagggaggg   2760
   agttttaggg ggaagggaag gggttttagg gggagggagg gagttttagg gggaagggaa   2820
   ggggttttag ggggagggag ggaattttag ggggaaggga aggggtttta gggggaggga   2880
   gggagtttta gggggaaggg aaggggtttt agggggaggg agggagtttt agggggaaag   2940
   gaaggggttt tagggggagg aagggagttt tagggggaag ggaaagggaa ggggttttag   3000
   g                                                                   3001
<210> 395
   <211> 3001
   <212> DNA
   <213> human
<400> 395
   gggagattga ggtaggagat gaattgagtg gttttagggt gtatagttgg tttatggtag     60
   gattagaatt tggggttttt gaaatgttat tttgtgttta tgtggttatg ttttggaaat    120
   atttttgggg tatatagtag ggtgtgttgg tgtgggaggt gatagggatt tggtgggaat    180
   atttattggg taaggataga ttttaatatg ggttgaatat tagttttgtg ttagggatta    240
   tgttttgggg ggggagtttt ttttaatttt agtagttgtt ttgaaaagta ggtgttatta    300
   tttttatttt ttggagggga gattgaggtt tggaagggga aattaaggtt tagagatttt    360
   aattggaaga ggttttaatg ttttggtttt ttttttaaag tttgttgggg agagaagaga    420
   agtttagggg gtaggtatag ggttgagttt taggtagagg gtatattttt attggtgttt    480
   tggaggtagt ttttttttgg ggatgggtag atagagtagg ttggtttttg ggatgggggt    540
   tttttgggta gtggggtgtg ggggttattt attttgtttt gtagtagttt gtgggggtaa    600
   gagtgagagg attaaggtta gtagatatag tgttatggtt tttggttttg gtattggagt    660
   tttgagtatg ttttatttgt ttgttatttt tagaggagtt ggtgaaatga gttttagaga    720
   aggaaattga taaataaatg ggtgttagag atttttggtt tttgagatat tggggattag    780
   gggttagttt gagtttgagt tagtttggaa gtttttttta tgatttgggg tgtagggttt    840
   gttttaggtt aaagggtaga gggagggggt gttttttttt ggtttggtag tttataggta    900
   tgagggttat tagttttatt attttaatag aggggtagtt tagagggttt gtaggagtta    960
   ggggggaatt gatgagattt tttgattgag ggtggtggga gatgggtaag agggattggg   1020
   tttgagttgg gaaaggagga gggtattatt tgtatttgat tttaattttt agtagggata   1080
   ttttttttgt ttttttttta atttttttta ttggttgggt atggtggttt atatttgtaa   1140
   ttttagtatt ttgggaggtt gatgtgggaa gattatttga gatgaggagt ttgagattaa   1200
   tttggttaat atgatgaaat attattttta ttaaaaatat aaaaattagt tagatttggt   1260
   ggtgggtgtt tgtaatttta gttatttagg aggttgaggt aggagaattg tttgaatttg   1320
   ggaggtggag gttgtagaga gttaagattg tgtttttgta ttttagtttg ggtaatagag   1380
   taagattttg ttttggtaaa aataaataaa taaaataatt ttttttattg ttttatgagt   1440
   atgttttgaa gatatggtta tgtttgggta gggagttttt gggtttggtt tgtgggtttt   1500
   gaggtaagta tagtttttat ggttatttgg agtgattttg ttttgtgtaa gtttagtaga   1560
   gttaagtgtt tattttttgg ttaattgggg ttgaatttga ggtttttttt tgtttttaga   1620
   atttgatatt aggttttttt gttataggga ttttagtaga ggtaggttta gatttgtttt   1680
   gattggagtt ggggaggggg ttttttgtaa agatatagga aggggtttag agtatttgtt   1740
   ttatgtttta tttagtagtt agttttggga gaaattttat tttaggaggg ttttgatttt   1800
   taatttgatt ttaattgtag aaggaggaga aggaaatttt ttatttttta ttttgagttt   1860
   ttagatttgt ttttattggt agggaaattt tagagtgggg agttatttag gaggagatag   1920
   gggtgttgag gtttttggga gtttggaagt ttttggatgg agaggtttat agtttttagt   1980
   tttttttagt aggagtatag gtaggggatt ggttaagttt gttagtttag agtaggattg   2040
   gttttagggt ttgattttgg tttttttttg atttgttttg gaggtttgtg gtgggttttg   2100
   tgtttgtagt ttttttgaat agagttagat gagggtggga ggtttttgtt ggtttatata   2160
   gtggatgtta ttatttttgg tttttggatg tggagttggg ggatgggaga gaagaggtga   2220
   ggttaagggg tagatttggg aaagggggag ttttaggagg aagagggagt tttggtgggg   2280
   aggagggaat tttaggggga gggagggagt tttaggggga gggagggagt tttagggaga   2340
   ggagggagtg gtgggggagg ggggagtttt aggggaaggg gtggttttag ggtgaggagg   2400
   gagttttggg ggaggaggga gttttaggag aagggaggga gttttagggg gagaagggag   2460
   ttttaggggt agggagagtg ttttagggag agggagggag ttttaaggag aggagggagt   2520
   tttgggggag gagggagttt tgggggaagg gagggagttt tgggggaagg gaaggggttt   2580
   tagggggagg gagggagttt tagggggaag ggaaggggtt ttagggggag gggagggagt   2640
   tttaggggga aggggaaggg gttttagggg gagggaggga atttaggggg gaagggaggg   2700
   gttttagggg gagggaggga gttttagggg gaagggaagg ggttttaggg ggagggaggg   2760
   agttttaggg ggaagggaag gggttttagg gggagggagg gagttttagg gggaagggaa   2820
   ggggttttag ggggagggag ggaattttag ggggaaggga aggggtttta gggggaggga   2880
   gggagtttta gggggaaggg aaggggtttt agggggaggg agggagtttt agggggaaag   2940
   gaaggggttt tagggggagg aagggagttt tagggggaag ggaaagggaa ggggttttag   3000
   g                                                                   3001

<210> 396
   <211> 50
   <212> DNA
   <213> human
<400> 396
   agaagtttat tgcatacatt ccctctgtct tccatcgact tatctcaacg 50
<210> 397
   <211> 50
   <212> DNA
   <213> human
<400> 397
   agaagtttat tgtatatatt ttttttgttt tttatcgatt tattttaacg 50
<210> 398
   <211> 50
   <212> DNA
   <213> human
<400> 398
   agaagtttat tgtatatatt ttttttgttt tttattgatt tattttaatg 50
<210> 399
   <211> 50
   <212> DNA
   <213> human
<400> 399
   cgttgagata agtcgatgga agatagaggg aatgtatgta ataaattttt 50
<210> 400
   <211> 50
   <212> DNA
   <213> human
<400> 400
   tgttgagata agttgatgga agatagaggg aatgtatgta ataaattttt 50
<210> 401
   <211> 3001
   <212> DNA
   <213> human
<400> 401
   ctttcagtta gcataattta ttctaaaagg aacagagaaa ccccacagtt ataaaggtac    60
   aagtaagaca catccagaga cataaagcaa agaattatgg aatgataggt attcttaaac   120
   ctcttagatg ctaactgctc ttgatcttgg cttgatccag ggtgacctcg tttgtttgtt   180
   attgcagtca ggtttgggtg atcttcttat gtcccctcat gttaggttgt cagggaagga   240
   tttcaataaa atccagagaa tccatatcct ccatagcatg atggacggca gctgcggtat   300
   ccatagcctc cgaagccaga gccgtatccg tagcctccgt agccaccgcc atagcccagt   360
   ctgcggaagc tgccacatcc acagccatag ccatagccca ggtcatcgaa gcctccgtag   420
   ccgtagccca ggcctccata gtagttgccg tagtagttca tggtgtcagg agtggtgagt   480
   tggtatgctg ctcaaggcaa gatcctgagt gtgaacacca gcatgtgtag gaggcttata   540
   taccctggtc agagcgtgtg atgagtcatc tgtataccct cctttgctat agttatttgt   600
   attacgtttg atgttacaac tcatttatct attgtcttct gatgtaggga gatgccctca   660
   ttctcattaa aatatttcag cttacttaat gtcttattca ggacacgatt gaacttactg   720
   ctttgctttt atgaaaagaa agggcttact gttcaaaata tgcacattcc aaatgctcaa   780
   cagaattgcc tattaccagg tgtattacat catctacact ttatggtatt ccttttataa   840
   tgaataattt cctttgtccg tcatcttcca aactgataac ttcatattca actaaattta   900
   cagctgtttt ttcaatttac agatgttaaa tctatagttt taaaaagcaa acttatttat   960
   caaggtgagg tcttcagaaa gtgcctatat gcacaataca ggaaaacatt tgaaatcaaa  1020
   atgattttaa tgccctgctg tgtacaatcg ccaaaggact caaaaaaaga gagtgtatgc  1080
   tgatttttag cattttattc acaataactt ttatactatt tttgactttg cacaccagct  1140
   tcagaacctt ttggttgcaa aatacataag aattaatttc agaaattgag ggcacaaaat  1200
   ccatctccta cgcagactta tcagagtagc actttagttt tccattcaag tcctaaaact  1260
   ctcctttata attggtttta ttcaaagtcc tggtaaaatt gcagattgca tctgcctctg  1320
   ttttgcacat tctagtcaat tactttcttt ctttatcatt tttcagtagt gtatatatga  1380
   cctctatttc ctttctcatt tcagctttta ttcttcttcc cattttctta atgtctatag  1440
   aaacagccag gtagaagttt attgcataca ttccctctgt cttccatcga cttatctcaa  1500
   cgcttttttt tttctatctc ctctttgtat tcactgtaac atctttcctg atttttttgt  1560
   ttgtttgttt tttgagacgg agtctcactc tgtcgcccag tctggagtgc agtggcacca  1620
   tctcggctta ctacaagctc cgcctcccgg gttcacgcca ttctcctgcc tcaacctccc  1680
   cagtagctgg gactacaggc gcccgccacc acgcctggct attttttttg gatttttagt  1740
   acagacgggg tttcaccatg ttagccagaa tggtcttgat ctcctgatct cgtgatctgc  1800
   ccgcctcggc cccgcaaagt gctaggatta caggcgtgat tctgatgttc ttctatgtct  1860
   atcctgtact catttattct gaggtaattt ctaaccactg cctactgatc tgttaatagt  1920
   tcattaaaat atcattttta aagctttcta ttttttgtac ttactaaaca tcctcaactc  1980
   cccaccccag caaaaatact tagaaataat gaataagacc tactatttga tagcacaata  2040
   gagtgactat agtcaataac aacttaattg tacattttaa aatgtcttga gtaaaattag  2100
   actgtttgtt aactcaacag ataaatactt gaggggatgg gttttttaaa atgtactcca  2160
   actcctatgg ctttctgaaa tttaaaaggt atcctaaaat agaaattgtc ttcaaagttt  2220
   tcaaaataaa ggcaccatgg tgaaatggca cagaatagag aaacatatgt aaagaaattt  2280
   gccctgggct tcatagagtt cctcagctga ctttgttgaa ttagtgaaac agatacaaca  2340
   ttgagaccac tcttgattaa tctccatgca gcacctgtct cttggcttcg acaactaaaa  2400
   attcatttaa tttacatgac tttgaacatg ctggtgagtt tttgtgagat ttataagtat  2460
   tttatcaaac ataaatttcc taaaaggaat gcttcagttc ttctaagcaa cagactaggg  2520
   aattaaaaat tgtttattga agaaattcta agtaaattga cagtaactgc cacagcaacc  2580
   acaatctgtg cacaaccctg actacgcctt agtgagctac ctttttaaga cagaaaaata  2640
   gctcaataag tctttccatt tatagagtag gatttaatcc aatatttgct acatttcttt  2700
   tatccaaata ttggttcatt cttatttctc atctgaactt gaaacagtca ataagctgag  2760
   ttctcagtga gtccttttga agccataaaa tcacaaataa ctcaaaatca caaataacta  2820
   tattaaggat accacagttt gtccatataa atcagcctga taatgtaaga gtgtttaata  2880
   gctaattttt gtttgggatg cataaacaat gcatacatct gagatataaa accaaacaag  2940
   atataaatgg aaaacctcaa tagccaaata agaattagaa tgttttaaag aaatagacta  3000
   t                                                                  3001
<210> 402
   <211> 3001
   <212> DNA
   <213> human
<400> 402
   tttttagtta gtataattta ttttaaaagg aatagagaaa ttttatagtt ataaaggtat      60
   aagtaagata tatttagaga tataaagtaa agaattatgg aatgataggt atttttaaat     120
   tttttagatg ttaattgttt ttgattttgg tttgatttag ggtgatttcg tttgtttgtt     180
   attgtagtta ggtttgggtg atttttttat gtttttttat gttaggttgt tagggaagga     240
   ttttaataaa atttagagaa tttatatttt ttatagtatg atggacggta gttgcggtat     300
   ttatagtttt cgaagttaga gtcgtattcg tagttttcgt agttatcgtt atagtttagt     360
   ttgcggaagt tgttatattt atagttatag ttatagttta ggttatcgaa gttttcgtag     420
   tcgtagttta ggtttttata gtagttgtcg tagtagttta tggtgttagg agtggtgagt     480
   tggtatgttg tttaaggtaa gattttgagt gtgaatatta gtatgtgtag gaggtttata     540
   tattttggtt agagcgtgtg atgagttatt tgtatatttt tttttgttat agttatttgt     600
   attacgtttg atgttataat ttatttattt attgtttttt gatgtaggga gatgttttta     660
   tttttattaa aatattttag tttatttaat gttttattta ggatacgatt gaatttattg     720
   ttttgttttt atgaaaagaa agggtttatt gtttaaaata tgtatatttt aaatgtttaa     780
   tagaattgtt tattattagg tgtattatat tatttatatt ttatggtatt ttttttataa     840
   tgaataattt tttttgttcg ttatttttta aattgataat tttatattta attaaattta     900
   tagttgtttt tttaatttat agatgttaaa tttatagttt taaaaagtaa atttatttat     960
   taaggtgagg tttttagaaa gtgtttatat gtataatata ggaaaatatt tgaaattaaa    1020
   atgattttaa tgttttgttg tgtataatcg ttaaaggatt taaaaaaaga gagtgtatgt    1080
   tgatttttag tattttattt ataataattt ttatattatt tttgattttg tatattagtt    1140
   ttagaatttt ttggttgtaa aatatataag aattaatttt agaaattgag ggtataaaat    1200
   ttatttttta cgtagattta ttagagtagt attttagttt tttatttaag ttttaaaatt    1260
   tttttttata attggtttta tttaaagttt tggtaaaatt gtagattgta tttgtttttg    1320
   ttttgtatat tttagttaat tatttttttt ttttattatt ttttagtagt gtatatatga    1380
   tttttatttt tttttttatt ttagttttta tttttttttt tattttttta atgtttatag    1440
   aaatagttag gtagaagttt attgtatata ttttttttgt tttttatcga tttattttaa    1500
   cgtttttttt tttttatttt ttttttgtat ttattgtaat attttttttg atttttttgt    1560
   ttgtttgttt tttgagacgg agtttatttt tgtcgtttag tttggagtgt agtggtatta    1620
   tttcggttta ttataagttt cgtttttcgg gtttacgtta tttttttgtt ttaatttttt     1680
   tagtagttgg gattataggc gttcgttatt acgtttggtt attttttttg gatttttagt    1740
   atagacgggg ttttattatg ttagttagaa tggttttgat tttttgattt cgtgatttgt    1800
   tcgtttcggt ttcgtaaagt gttaggatta taggcgtgat tttgatgttt ttttatgttt    1860
   attttgtatt tatttatttt gaggtaattt ttaattattg tttattgatt tgttaatagt    1920
   ttattaaaat attattttta aagtttttta ttttttgtat ttattaaata tttttaattt    1980
   tttattttag taaaaatatt tagaaataat gaataagatt tattatttga tagtataata    2040
   gagtgattat agttaataat aatttaattg tatattttaa aatgttttga gtaaaattag    2100
   attgtttgtt aatttaatag ataaatattt gaggggatgg gttttttaaa atgtatttta    2160
   atttttatgg ttttttgaaa tttaaaaggt attttaaaat agaaattgtt tttaaagttt    2220
   ttaaaataaa ggtattatgg tgaaatggta tagaatagag aaatatatgt aaagaaattt    2280
   gttttgggtt ttatagagtt ttttagttga ttttgttgaa ttagtgaaat agatataata    2340
   ttgagattat ttttgattaa tttttatgta gtatttgttt tttggtttcg ataattaaaa    2400
   atttatttaa tttatatgat tttgaatatg ttggtgagtt tttgtgagat ttataagtat    2460
   tttattaaat ataaattttt taaaaggaat gttttagttt ttttaagtaa tagattaggg    2520
   aattaaaaat tgtttattga agaaatttta agtaaattga tagtaattgt tatagtaatt    2580
   ataatttgtg tataattttg attacgtttt agtgagttat ttttttaaga tagaaaaata     2640
   gtttaataag tttttttatt tatagagtag gatttaattt aatatttgtt atattttttt    2700
   tatttaaata ttggtttatt tttatttttt atttgaattt gaaatagtta ataagttgag     2760
   tttttagtga gtttttttga agttataaaa ttataaataa tttaaaatta taaataatta    2820
   tattaaggat attatagttt gtttatataa attagtttga taatgtaaga gtgtttaata    2880
   gttaattttt gtttgggatg tataaataat gtatatattt gagatataaa attaaataag    2940
   atataaatgg aaaattttaa tagttaaata agaattagaa tgttttaaag aaatagatta    3000
   t                                                                    3001
<210> 403
   <211> 3001
   <212> DNA
   <213> human
<400> 403
   tttttagtta gtataattta ttttaaaagg aatagagaaa ttttatagtt ataaaggtat     60
   aagtaagata tatttagaga tataaagtaa agaattatgg aatgataggt atttttaaat    120
   tttttagatg ttaattgttt ttgattttgg tttgatttag ggtgattttg tttgtttgtt    180
   attgtagtta ggtttgggtg atttttttat gtttttttat gttaggttgt tagggaagga    240
   ttttaataaa atttagagaa tttatatttt ttatagtatg atggatggta gttgtggtat    300
   ttatagtttt tgaagttaga gttgtatttg tagtttttgt agttattgtt atagtttagt    360
   ttgtggaagt tgttatattt atagttatag ttatagttta ggttattgaa gtttttgtag    420
   ttgtagttta ggtttttata gtagttgttg tagtagttta tggtgttagg agtggtgagt    480
   tggtatgttg tttaaggtaa gattttgagt gtgaatatta gtatgtgtag gaggtttata    540
   tattttggtt agagtgtgtg atgagttatt tgtatatttt tttttgttat agttatttgt    600
   attatgtttg atgttataat ttatttattt attgtttttt gatgtaggga gatgttttta    660
   tttttattaa aatattttag tttatttaat gttttattta ggatatgatt gaatttattg    720
   ttttgttttt atgaaaagaa agggtttatt gtttaaaata tgtatatttt aaatgtttaa    780
   tagaattgtt tattattagg tgtattatat tatttatatt ttatggtatt ttttttataa    840
   tgaataattt tttttgtttg ttatttttta aattgataat tttatattta attaaattta    900
   tagttgtttt tttaatttat agatgttaaa tttatagttt taaaaagtaa atttatttat    960
   taaggtgagg tttttagaaa gtgtttatat gtataatata ggaaaatatt tgaaattaaa   1020
   atgattttaa tgttttgttg tgtataattg ttaaaggatt taaaaaaaga gagtgtatgt   1080
   tgatttttag tattttattt ataataattt ttatattatt tttgattttg tatattagtt   1140
   ttagaatttt ttggttgtaa aatatataag aattaatttt agaaattgag ggtataaaat   1200
   ttatttttta tgtagattta ttagagtagt attttagttt tttatttaag ttttaaaatt   1260
   tttttttata attggtttta tttaaagttt tggtaaaatt gtagattgta tttgtttttg   1320
   ttttgtatat tttagttaat tatttttttt ttttattatt ttttagtagt gtatatatga   1380
   tttttatttt tttttttatt ttagttttta tttttttttt tattttttta atgtttatag   1440
   aaatagttag gtagaagttt attgtatata ttttttttgt tttttattga tttattttaa   1500
   tgtttttttt tttttatttt ttttttgtat ttattgtaat attttttttg atttttttgt   1560
   ttgtttgttt tttgagatgg agttttattt tgttgtttag tttggagtgt agtggtatta   1620
   ttttggttta ttataagttt tgttttttgg gtttatgtta tttttttgtt ttaatttttt   1680
   tagtagttgg gattataggt gtttgttatt atgtttggtt attttttttg gatttttagt   1740
   atagatgggg ttttattatg ttagttagaa tggttttgat tttttgattt tgtgatttgt   1800
   ttgttttggt tttgtaaagt gttaggatta taggtgtgat tttgatgttt ttttatgttt   1860
   attttgtatt tatttatttt gaggtaattt ttaattattg tttattgatt tgttaatagt   1920
   ttattaaaat attattttta aagtttttta ttttttgtat ttattaaata tttttaattt   1980
   tttattttag taaaaatatt tagaaataat gaataagatt tattatttga tagtataata   2040
   gagtgattat agttaataat aatttaattg tatattttaa aatgttttga gtaaaattag   2100
   attgtttgtt aatttaatag ataaatattt gaggggatgg gttttttaaa atgtatttta   2160
   atttttatgg ttttttgaaa tttaaaaggt attttaaaat agaaattgtt tttaaagttt   2220
   ttaaaataaa ggtattatgg tgaaatggta tagaatagag aaatatatgt aaagaaattt   2280
   gttttgggtt ttatagagtt ttttagttga ttttgttgaa ttagtgaaat agatataata   2340
   ttgagattat ttttgattaa tttttatgta gtatttgttt tttggttttg ataattaaaa   2400
   atttatttaa tttatatgat tttgaatatg ttggtgagtt tttgtgagat ttataagtat   2460
   tttattaaat ataaattttt taaaaggaat gttttagttt ttttaagtaa tagattaggg   2520
   aattaaaaat tgtttattga agaaatttta agtaaattga tagtaattgt tatagtaatt   2580
   ataatttgtg tataattttg attatgtttt agtgagttat ttttttaaga tagaaaaata   2640
   gtttaataag tttttttatt tatagagtag gatttaattt aatatttgtt atattttttt   2700
   tatttaaata ttggtttatt tttatttttt atttgaattt gaaatagtta ataagttgag   2760
   tttttagtga gtttttttga agttataaaa ttataaataa tttaaaatta taaataatta   2820
   tattaaggat attatagttt gtttatataa attagtttga taatgtaaga gtgtttaata   2880
   gttaattttt gtttgggatg tataaataat gtatatattt gagatataaa attaaataag   2940
   atataaatgg aaaattttaa tagttaaata agaattagaa tgttttaaag aaatagatta   3000
   t                                                                   3001
<210> 404
   <211> 3001
   <212> DNA
   <213> human
<400> 404
   atagtttatt tttttaaaat attttaattt ttatttggtt attgaggttt tttatttata     60
   ttttgtttgg ttttatattt tagatgtatg tattgtttat gtattttaaa taaaaattag    120
   ttattaaata tttttatatt attaggttga tttatatgga taaattgtgg tatttttaat    180
   atagttattt gtgattttga gttatttgtg attttatggt tttaaaagga tttattgaga    240
   atttagttta ttgattgttt taagtttaga tgagaaataa gaatgaatta atatttggat    300
   aaaagaaatg tagtaaatat tggattaaat tttattttat aaatggaaag atttattgag    360
   ttattttttt gttttaaaaa ggtagtttat taaggcgtag ttagggttgt gtatagattg    420
   tggttgttgt ggtagttatt gttaatttat ttagaatttt tttaataaat aatttttaat    480
   tttttagttt gttgtttaga agaattgaag tatttttttt aggaaattta tgtttgataa    540
   aatatttata aattttataa aaatttatta gtatgtttaa agttatgtaa attaaatgaa    600
   tttttagttg tcgaagttaa gagataggtg ttgtatggag attaattaag agtggtttta    660
   atgttgtatt tgttttatta atttaataaa gttagttgag gaattttatg aagtttaggg    720
   taaatttttt tatatatgtt tttttatttt gtgttatttt attatggtgt ttttattttg    780
   aaaattttga agataatttt tattttagga tattttttaa attttagaaa gttataggag    840
   ttggagtata ttttaaaaaa tttatttttt taagtattta tttgttgagt taataaatag    900
   tttaatttta tttaagatat tttaaaatgt ataattaagt tgttattgat tatagttatt    960
   ttattgtgtt attaaatagt aggttttatt tattattttt aagtattttt gttggggtgg   1020
   ggagttgagg atgtttagta agtataaaaa atagaaagtt ttaaaaatga tattttaatg   1080
   aattattaat agattagtag gtagtggtta gaaattattt tagaataaat gagtatagga   1140
   tagatataga agaatattag aattacgttt gtaattttag tattttgcgg ggtcgaggcg   1200
   ggtagattac gagattagga gattaagatt attttggtta atatggtgaa atttcgtttg   1260
   tattaaaaat ttaaaaaaaa tagttaggcg tggtggcggg cgtttgtagt tttagttatt   1320
   ggggaggttg aggtaggaga atggcgtgaa ttcgggaggc ggagtttgta gtaagtcgag   1380
   atggtgttat tgtattttag attgggcgat agagtgagat ttcgttttaa aaaataaata   1440
   aataaaaaaa ttaggaaaga tgttatagtg aatataaaga ggagatagaa aaaaaaaagc   1500
   gttgagataa gtcgatggaa gatagaggga atgtatgtaa taaattttta tttggttgtt   1560
   tttatagata ttaagaaaat gggaagaaga ataaaagttg aaatgagaaa ggaaatagag   1620
   gttatatata tattattgaa aaatgataaa gaaagaaagt aattgattag aatgtgtaaa   1680
   atagaggtag atgtaatttg taattttatt aggattttga ataaaattaa ttataaagga   1740
   gagttttagg atttgaatgg aaaattaaag tgttattttg ataagtttgc gtaggagatg   1800
   gattttgtgt ttttaatttt tgaaattaat ttttatgtat tttgtaatta aaaggttttg   1860
   aagttggtgt gtaaagttaa aaatagtata aaagttattg tgaataaaat gttaaaaatt   1920
   agtatatatt tttttttttt gagttttttg gcgattgtat atagtagggt attaaaatta   1980
   ttttgatttt aaatgttttt ttgtattgtg tatataggta ttttttgaag attttatttt   2040
   gataaataag tttgtttttt aaaattatag atttaatatt tgtaaattga aaaaatagtt   2100
   gtaaatttag ttgaatatga agttattagt ttggaagatg acggataaag gaaattattt   2160
   attataaaag gaatattata aagtgtagat gatgtaatat atttggtaat aggtaatttt   2220
   gttgagtatt tggaatgtgt atattttgaa tagtaagttt ttttttttta taaaagtaaa   2280
   gtagtaagtt taatcgtgtt ttgaataaga tattaagtaa gttgaaatat tttaatgaga   2340
   atgagggtat ttttttatat tagaagataa tagataaatg agttgtaata ttaaacgtaa   2400
   tataaataat tatagtaaag gagggtatat agatgattta ttatacgttt tgattagggt   2460
   atataagttt tttatatatg ttggtgttta tatttaggat tttgttttga gtagtatatt   2520
   aatttattat ttttgatatt atgaattatt acggtaatta ttatggaggt ttgggttacg   2580
   gttacggagg tttcgatgat ttgggttatg gttatggttg tggatgtggt agttttcgta   2640
   gattgggtta tggcggtggt tacggaggtt acggatacgg ttttggtttc ggaggttatg   2700
   gatatcgtag ttgtcgttta ttatgttatg gaggatatgg attttttgga ttttattgaa   2760
   attttttttt gataatttaa tatgagggga tataagaaga ttatttaaat ttgattgtaa   2820
   taataaataa acgaggttat tttggattaa gttaagatta agagtagtta gtatttaaga   2880
   ggtttaagaa tatttattat tttataattt tttgttttat gtttttggat gtgttttatt   2940
   tgtattttta taattgtggg gtttttttgt tttttttaga ataaattatg ttaattgaaa   3000
   g                                                                   3001
<210> 405
   <211> 3001
   <212> DNA
   <213> human
<400> 405
   atagtttatt tttttaaaat attttaattt ttatttggtt attgaggttt tttatttata      60
   ttttgtttgg ttttatattt tagatgtatg tattgtttat gtattttaaa taaaaattag     120
   ttattaaata tttttatatt attaggttga tttatatgga taaattgtgg tatttttaat     180
   atagttattt gtgattttga gttatttgtg attttatggt tttaaaagga tttattgaga     240
   atttagttta ttgattgttt taagtttaga tgagaaataa gaatgaatta atatttggat     300
   aaaagaaatg tagtaaatat tggattaaat tttattttat aaatggaaag atttattgag     360
   ttattttttt gttttaaaaa ggtagtttat taaggtgtag ttagggttgt gtatagattg     420
   tggttgttgt ggtagttatt gttaatttat ttagaatttt tttaataaat aatttttaat     480
   tttttagttt gttgtttaga agaattgaag tatttttttt aggaaattta tgtttgataa     540
   aatatttata aattttataa aaatttatta gtatgtttaa agttatgtaa attaaatgaa     600
   tttttagttg ttgaagttaa gagataggtg ttgtatggag attaattaag agtggtttta     660
   atgttgtatt tgttttatta atttaataaa gttagttgag gaattttatg aagtttaggg     720
   taaatttttt tatatatgtt tttttatttt gtgttatttt attatggtgt ttttattttg     780
   aaaattttga agataatttt tattttagga tattttttaa attttagaaa gttataggag     840
   ttggagtata ttttaaaaaa tttatttttt taagtattta tttgttgagt taataaatag     900
   tttaatttta tttaagatat tttaaaatgt ataattaagt tgttattgat tatagttatt     960
   ttattgtgtt attaaatagt aggttttatt tattattttt aagtattttt gttggggtgg    1020
   ggagttgagg atgtttagta agtataaaaa atagaaagtt ttaaaaatga tattttaatg    1080
   aattattaat agattagtag gtagtggtta gaaattattt tagaataaat gagtatagga    1140
   tagatataga agaatattag aattatgttt gtaattttag tattttgtgg ggttgaggtg    1200
   ggtagattat gagattagga gattaagatt attttggtta atatggtgaa attttgtttg    1260
   tattaaaaat ttaaaaaaaa tagttaggtg tggtggtggg tgtttgtagt tttagttatt    1320
   ggggaggttg aggtaggaga atggtgtgaa tttgggaggt ggagtttgta gtaagttgag    1380
   atggtgttat tgtattttag attgggtgat agagtgagat tttgttttaa aaaataaata    1440
   aataaaaaaa ttaggaaaga tgttatagtg aatataaaga ggagatagaa aaaaaaaagt    1500
   gttgagataa gttgatggaa gatagaggga atgtatgtaa taaattttta tttggttgtt    1560
   tttatagata ttaagaaaat gggaagaaga ataaaagttg aaatgagaaa ggaaatagag    1620
   gttatatata tattattgaa aaatgataaa gaaagaaagt aattgattag aatgtgtaaa    1680
   atagaggtag atgtaatttg taattttatt aggattttga ataaaattaa ttataaagga    1740
   gagttttagg atttgaatgg aaaattaaag tgttattttg ataagtttgt gtaggagatg    1800
   gattttgtgt ttttaatttt tgaaattaat ttttatgtat tttgtaatta aaaggttttg    1860
   aagttggtgt gtaaagttaa aaatagtata aaagttattg tgaataaaat gttaaaaatt    1920
   agtatatatt tttttttttt gagttttttg gtgattgtat atagtagggt attaaaatta    1980
   ttttgatttt aaatgttttt ttgtattgtg tatataggta ttttttgaag attttatttt     2040
   gataaataag tttgtttttt aaaattatag atttaatatt tgtaaattga aaaaatagtt    2100
   gtaaatttag ttgaatatga agttattagt ttggaagatg atggataaag gaaattattt    2160
   attataaaag gaatattata aagtgtagat gatgtaatat atttggtaat aggtaatttt    2220
   gttgagtatt tggaatgtgt atattttgaa tagtaagttt ttttttttta taaaagtaaa    2280
   gtagtaagtt taattgtgtt ttgaataaga tattaagtaa gttgaaatat tttaatgaga    2340
   atgagggtat ttttttatat tagaagataa tagataaatg agttgtaata ttaaatgtaa    2400
   tataaataat tatagtaaag gagggtatat agatgattta ttatatgttt tgattagggt    2460
   atataagttt tttatatatg ttggtgttta tatttaggat tttgttttga gtagtatatt    2520
   aatttattat ttttgatatt atgaattatt atggtaatta ttatggaggt ttgggttatg    2580
   gttatggagg ttttgatgat ttgggttatg gttatggttg tggatgtggt agtttttgta    2640
   gattgggtta tggtggtggt tatggaggtt atggatatgg ttttggtttt ggaggttatg    2700
   gatattgtag ttgttgttta ttatgttatg gaggatatgg attttttgga ttttattgaa    2760
   attttttttt gataatttaa tatgagggga tataagaaga ttatttaaat ttgattgtaa    2820
   taataaataa atgaggttat tttggattaa gttaagatta agagtagtta gtatttaaga    2880
   ggtttaagaa tatttattat tttataattt tttgttttat gtttttggat gtgttttatt    2940
   tgtattttta taattgtggg gtttttttgt tttttttaga ataaattatg ttaattgaaa    3000
   g                                                                      3001
<210> 406
   <211> 50
   <212> DNA
   <213> human
<400> 406
   cgctttgcat tcagccaggc tccaggttgg gcacaaaccc tatagcagag 50
<210> 407
   <211> 50
   <212> DNA
   <213> human
<400> 407
   cgttttgtat ttagttaggt tttaggttgg gtataaattt tatagtagag 50
<210> 408
   <211> 50
   <212> DNA
   <213> human
<400> 408
   tgttttgtat ttagttaggt tttaggttgg gtataaattt tatagtagag 50
<210> 409
   <211> 50
   <212> DNA
   <213> human
<400> 409
   ttttgttata gggtttgtgt ttaatttgga gtttggttga atgtaaagcg 50
<210> 410
   <211> 50
   <212> DNA
   <213> human
<400> 410
   ttttgttata gggtttgtgt ttaatttgga gtttggttga atgtaaagtg 50
<210> 411
   <211> 3001
   <212> DNA
   <213> human
<400> 411
   cttttcacct ccaacattct ctgtgtccac tctaggttcc cagctggtcc acaggtatac 60
   aaactgactt tcacagactg cttggtccct gtagcagcca gcgcacactg tagacactca 120
   tgacataaag ttcctgtcta gttgatttaa ggaaacctga aaaaatacct tacctctggc 180
   tctaggccct aaacagatgg cttttggatc atccatgcca cctacccctc ccctgaatat 240
   ttattttctt cattccagcc ccagccgtga gccaaccagg actctgtaaa cactgagtct     300
   gcacacctgg gcttagtcaa cagtcatcaa acaaaggcac tgccaaatca cctcacaagc     360
   ccaaacttgc acctactctg tctgtcacag ggaagcagcc acctgggagg tgtgaatggt     420
   gtgctcccct tctcccaaca cagaatgggc atgagggatt gggctgtata gaggatgtag     480
   ccggaacggc cccagcctac aaatgggttg caagtttaat tagacttaag ggatatgaga     540
   aaacagcatg agaagcagcc agcagttgca agaatcttcc agaagcagct agacatttct     600
   cagtcccttg gcttcttcga taatcatcat taaaatatga tgcactggcc gggcatggtg     660
   gctcacacct gtaatcccag cactttggga ggccgaggca ggctaatcac ctgaggtcag     720
   gagttcaaga tcggcctgga caacatggtg aaaccccatc tctactaaaa atacaaaaat     780
   tagccaggcg tggtggcagg tgcctgtaat cccagctact caggaggctg aggcaggaga     840
   attgcttgag cccaggaggc agaggttgca gggaggcaga ggttgcaggg agccaagatc     900
   acgccattgc actccagcct ggaagacaag agcgagactc cacctcagaa aaaaaaaaac     960
   aaaaaacaaa aaacaaaaaa aaccatgatg cattcagtgc ttgaacctgg gaggcggagg    1020
   ttgcagtgac ctgagatcac accactcact ccagcctggg caacagagcg agacctccat    1080
   ctcaaaaaga gaaacgatga tacattcagg tagtgcttga acactgcttg ctggtggcaa    1140
   acatatgact gactcgtcct gcaaggtctg tgtatcccta acagcagagt tgtggggatt    1200
   agcagaccca cagcccggac acgggttgaa ttgagattca tgctaccttt gtgtatggtg    1260
   aacctgtgtg catgtgcaaa ataactaaag aggaaggatg aacaggaaag gcaaggatga    1320
   aaacccagct tccacagggg aaacatctgt gaattgtagc caggatctgc tgatactagt    1380
   catttgagaa tcccaagata gattatcctc tcactctgac cacactgact gtccacaatg    1440
   attgtgcttt acgtgtgcac agcactcaac agcttaagac gttgcgggaa agtccccgaa    1500
   cgctttgcat tcagccaggc tccaggttgg gcacaaaccc tatagcagag agctggccca    1560
   ggtgccatgc catcctcaca acaatcctat gaagtaggga ctgttatttt gcagaggagg    1620
   ggattgaagt acagaggagt taaatacctt gccccaagtc tcgcagccag gagtaaccag    1680
   gtcaggattc taccccagag cccacactcc aacccctcac catgttgtca ccccacttac    1740
   aataatgaat ctctgcacct ctgcatggga caaagaggga aactgagtct tccaggaggg    1800
   ctgggcactt gaggccatcc tggcaatggt cccacctcca gccatcctgg aggaggagcg    1860
   aagccactgc gttgagtgcg tgggtgcagt gggccagcca gggaacaggg aggtaaaaaa    1920
   ccaagggggc acctcagctc tgagctgggc aggccaggcc ccacctcatg cttcctctgc    1980
   cacagcagtc ccaggccagg agggcacctt gccaggctcc actgccaccc agaacatggc    2040
   ctccctgtcc tggtctgggt tctcctcgag ttgtatttcc atggcagcca caatgggcag    2100
   cagccagggt gagtcaggcc aggcaagagg aagtggccac acttgaaaat tattaacatg    2160
   cagggctgag cttgagtcct ttgttcttga gcaaagactg tagcatttca ctttcaggaa    2220
   ggagctagga gggtggggca ggctcagaat tgcacaacca aagcacaggc tcaaacacac    2280
   acaggtccca ggtggaagtc agcttagaca ggccacctgc agaaaagtgc aggccaaccc    2340
   acagccccat acagcaggtc cacagagaaa atacaggcca actcatctcc actgtttttt    2400
   aaatcagagg ctgggcacag tatatgaaaa cctatataat cccagtgctt tgggtggctg    2460
   agatgagagg accacttgag gccaggagct tgagaccagc ttgagcaaca tagagaccct    2520
   ttctctacac aaaaggaaaa attagctagg catggtggtg cacacctgta gtcccggcta    2580
   ttcaggaggg tgagatggga ggattacttg agcccaagcg ttcaaggctg cagtgagcta    2640
   tgatggctcc actgcactcc agcctgggtg acagagagag agaccgtgtt tcaaaaaaaa    2700
   aaaaaatcag agagggtttc caacagggat aaaatcaggg tttttgtggt caaaagtgtt    2760
   ccaagtcagg gagaccaagc aaaggaggca caaggccggc cacagaggga aggatgggac    2820
   tctgggcagc cagaccccag tgggagccct gcgtctggga gagatgaaag acaccccctc    2880
   accccagctg gcagggggca gccccagctc taggaagact gccttcctgc acccccttgc      2940
   caaaagatgg catctctcct ccaaccatgc agctgtcctg gcccccctac cccaccgcgt    3000
   c                                                                    3001
<210> 412
   <211> 3001
   <212> DNA
   <213> human
<400> 412
   ttttttattt ttaatatttt ttgtgtttat tttaggtttt tagttggttt ataggtatat 60
   aaattgattt ttatagattg tttggttttt gtagtagtta gcgtatattg tagatattta 120
   tgatataaag tttttgttta gttgatttaa ggaaatttga aaaaatattt tatttttggt 180
   tttaggtttt aaatagatgg tttttggatt atttatgtta tttatttttt ttttgaatat 240
   ttattttttt tattttagtt ttagtcgtga gttaattagg attttgtaaa tattgagttt 300
   gtatatttgg gtttagttaa tagttattaa ataaaggtat tgttaaatta ttttataagt 360
   ttaaatttgt atttattttg tttgttatag ggaagtagtt atttgggagg tgtgaatggt 420
   gtgttttttt ttttttaata tagaatgggt atgagggatt gggttgtata gaggatgtag 480
   tcggaacggt tttagtttat aaatgggttg taagtttaat tagatttaag ggatatgaga 540
   aaatagtatg agaagtagtt agtagttgta agaatttttt agaagtagtt agatattttt 600
   tagttttttg gttttttcga taattattat taaaatatga tgtattggtc gggtatggtg 660
   gtttatattt gtaattttag tattttggga ggtcgaggta ggttaattat ttgaggttag 720
   gagtttaaga tcggtttgga taatatggtg aaattttatt tttattaaaa atataaaaat 780
   tagttaggcg tggtggtagg tgtttgtaat tttagttatt taggaggttg aggtaggaga 840
   attgtttgag tttaggaggt agaggttgta gggaggtaga ggttgtaggg agttaagatt 900
   acgttattgt attttagttt ggaagataag agcgagattt tattttagaa aaaaaaaaat 960
   aaaaaataaa aaataaaaaa aattatgatg tatttagtgt ttgaatttgg gaggcggagg 1020
   ttgtagtgat ttgagattat attatttatt ttagtttggg taatagagcg agatttttat 1080
   tttaaaaaga gaaacgatga tatatttagg tagtgtttga atattgtttg ttggtggtaa 1140
   atatatgatt gattcgtttt gtaaggtttg tgtattttta atagtagagt tgtggggatt 1200
   agtagattta tagttcggat acgggttgaa ttgagattta tgttattttt gtgtatggtg 1260
   aatttgtgtg tatgtgtaaa ataattaaag aggaaggatg aataggaaag gtaaggatga 1320
   aaatttagtt tttatagggg aaatatttgt gaattgtagt taggatttgt tgatattagt 1380
   tatttgagaa ttttaagata gattattttt ttattttgat tatattgatt gtttataatg 1440
   attgtgtttt acgtgtgtat agtatttaat agtttaagac gttgcgggaa agttttcgaa 1500
   cgttttgtat ttagttaggt tttaggttgg gtataaattt tatagtagag agttggttta 1560
   ggtgttatgt tatttttata ataattttat gaagtaggga ttgttatttt gtagaggagg 1620
   ggattgaagt atagaggagt taaatatttt gttttaagtt tcgtagttag gagtaattag 1680
   gttaggattt tattttagag tttatatttt aattttttat tatgttgtta ttttatttat 1740
   aataatgaat ttttgtattt ttgtatggga taaagaggga aattgagttt tttaggaggg 1800
   ttgggtattt gaggttattt tggtaatggt tttattttta gttattttgg aggaggagcg 1860
   aagttattgc gttgagtgcg tgggtgtagt gggttagtta gggaataggg aggtaaaaaa 1920
   ttaagggggt attttagttt tgagttgggt aggttaggtt ttattttatg ttttttttgt 1980
   tatagtagtt ttaggttagg agggtatttt gttaggtttt attgttattt agaatatggt 2040
   ttttttgttt tggtttgggt ttttttcgag ttgtattttt atggtagtta taatgggtag 2100
   tagttagggt gagttaggtt aggtaagagg aagtggttat atttgaaaat tattaatatg 2160
   tagggttgag tttgagtttt ttgtttttga gtaaagattg tagtatttta tttttaggaa 2220
   ggagttagga gggtggggta ggtttagaat tgtataatta aagtataggt ttaaatatat 2280
   ataggtttta ggtggaagtt agtttagata ggttatttgt agaaaagtgt aggttaattt 2340
   atagttttat atagtaggtt tatagagaaa atataggtta atttattttt attgtttttt 2400
   aaattagagg ttgggtatag tatatgaaaa tttatataat tttagtgttt tgggtggttg 2460
   agatgagagg attatttgag gttaggagtt tgagattagt ttgagtaata tagagatttt 2520
   ttttttatat aaaaggaaaa attagttagg tatggtggtg tatatttgta gtttcggtta 2580
   tttaggaggg tgagatggga ggattatttg agtttaagcg tttaaggttg tagtgagtta 2640
   tgatggtttt attgtatttt agtttgggtg atagagagag agatcgtgtt ttaaaaaaaa 2700
   aaaaaattag agagggtttt taatagggat aaaattaggg tttttgtggt taaaagtgtt 2760
   ttaagttagg gagattaagt aaaggaggta taaggtcggt tatagaggga aggatgggat 2820
   tttgggtagt tagattttag tgggagtttt gcgtttggga gagatgaaag atattttttt 2880
   attttagttg gtagggggta gttttagttt taggaagatt gtttttttgt atttttttgt 2940
   taaaagatgg tatttttttt ttaattatgt agttgttttg gtttttttat tttatcgcgt 3000
   t 3001
<210> 413
   <211> 3001
   <212> DNA
   <213> human
<400> 413
   ttttttattt ttaatatttt ttgtgtttat tttaggtttt tagttggttt ataggtatat 60
   aaattgattt ttatagattg tttggttttt gtagtagtta gtgtatattg tagatattta 120
   tgatataaag tttttgttta gttgatttaa ggaaatttga aaaaatattt tatttttggt 180
   tttaggtttt aaatagatgg tttttggatt atttatgtta tttatttttt ttttgaatat 240
   ttattttttt tattttagtt ttagttgtga gttaattagg attttgtaaa tattgagttt 300
   gtatatttgg gtttagttaa tagttattaa ataaaggtat tgttaaatta ttttataagt 360
   ttaaatttgt atttattttg tttgttatag ggaagtagtt atttgggagg tgtgaatggt 420
   gtgttttttt ttttttaata tagaatgggt atgagggatt gggttgtata gaggatgtag 480
   ttggaatggt tttagtttat aaatgggttg taagtttaat tagatttaag ggatatgaga 540
   aaatagtatg agaagtagtt agtagttgta agaatttttt agaagtagtt agatattttt 600
   tagttttttg gtttttttga taattattat taaaatatga tgtattggtt gggtatggtg 660
   gtttatattt gtaattttag tattttggga ggttgaggta ggttaattat ttgaggttag 720
   gagtttaaga ttggtttgga taatatggtg aaattttatt tttattaaaa atataaaaat 780
   tagttaggtg tggtggtagg tgtttgtaat tttagttatt taggaggttg aggtaggaga 840
   attgtttgag tttaggaggt agaggttgta gggaggtaga ggttgtaggg agttaagatt 900
   atgttattgt attttagttt ggaagataag agtgagattt tattttagaa aaaaaaaaat 960
   aaaaaataaa aaataaaaaa aattatgatg tatttagtgt ttgaatttgg gaggtggagg 1020
   ttgtagtgat ttgagattat attatttatt ttagtttggg taatagagtg agatttttat 1080
   tttaaaaaga gaaatgatga tatatttagg tagtgtttga atattgtttg ttggtggtaa 1140
   atatatgatt gatttgtttt gtaaggtttg tgtattttta atagtagagt tgtggggatt 1200
   agtagattta tagtttggat atgggttgaa ttgagattta tgttattttt gtgtatggtg 1260
   aatttgtgtg tatgtgtaaa ataattaaag aggaaggatg aataggaaag gtaaggatga 1320
   aaatttagtt tttatagggg aaatatttgt gaattgtagt taggatttgt tgatattagt 1380
   tatttgagaa ttttaagata gattattttt ttattttgat tatattgatt gtttataatg 1440
   attgtgtttt atgtgtgtat agtatttaat agtttaagat gttgtgggaa agtttttgaa 1500
   tgttttgtat ttagttaggt tttaggttgg gtataaattt tatagtagag agttggttta 1560
   ggtgttatgt tatttttata ataattttat gaagtaggga ttgttatttt gtagaggagg 1620
   ggattgaagt atagaggagt taaatatttt gttttaagtt ttgtagttag gagtaattag 1680
   gttaggattt tattttagag tttatatttt aattttttat tatgttgtta ttttatttat 1740
   aataatgaat ttttgtattt ttgtatggga taaagaggga aattgagttt tttaggaggg 1800
   ttgggtattt gaggttattt tggtaatggt tttattttta gttattttgg aggaggagtg 1860
   aagttattgt gttgagtgtg tgggtgtagt gggttagtta gggaataggg aggtaaaaaa 1920
   ttaagggggt atttagtttt tgagttgggt aggttaggtt ttattttatg ttttttttgt 1980
   tatagtagtt ttaggttagg agggtatttt gttaggtttt attgttattt agaatatggt 2040
   ttttttgttt tggtttgggt tttttttgag ttgtattttt atggtagtta taatgggtag 2100
   tagttagggt gagttaggtt aggtaagagg aagtggttat atttgaaaat tattaatatg 2160
   tagggttgag tttgagtttt ttgtttttga gtaaagattg tagtatttta tttttaggaa 2220
   ggagttagga gggtggggta ggtttagaat tgtataatta aagtataggt ttaaatatat 2280
   ataggtttta ggtggaagtt agtttagata ggttatttgt agaaaagtgt aggttaattt 2340
   atagttttat atagtaggtt tatagagaaa atataggtta atttattttt attgtttttt 2400
   aaattagagg ttgggtatag tatatgaaaa tttatataat tttagtgttt tgggtggttg 2460
   agatgagagg attatttgag gttaggagtt tgagattagt ttgagtaata tagagatttt 2520
   ttttttatat aaaaggaaaa attagttagg tatggtggtg tatatttgta gttttggtta 2580
   tttaggaggg tgagatggga ggattatttg agtttaagtg tttaaggttg tagtgagtta 2640
   tgatggtttt attgtatttt agtttgggtg atagagagag agattgtgtt ttaaaaaaaa 2700
   aaaaaattag agagggtttt taatagggat aaaattaggg tttttgtggt taaaagtgtt 2760
   ttaagttagg gagattaagt aaaggaggta taaggttggt tatagaggga aggatgggat 2820
   tttgggtagt tagattttag tgggagtttt gtgtttggga gagatgaaag atattttttt 2880
   attttagttg gtagggggta gttttagttt taggaagatt gtttttttgt atttttttgt 2940
   taaaagatgg tatttttttt ttaattatgt agttgttttg gtttttttat tttattgtgt 3000
   t 3001
<210> 414
   <211> 3001
   <212> DNA
   <213> human
<400> 414
<210> 415
   <211> 3001
   <212> DNA
   <213> human
<400> 415
   gatgtggtgg ggtagggggg ttaggatagt tgtatggttg gaggagagat gttatttttt 60
   ggtaaggggg tgtaggaagg tagttttttt agagttgggg ttgttttttg ttagttgggg 120
   tgagggggtg ttttttattt tttttagatg tagggttttt attggggttt ggttgtttag 180
   agttttattt ttttttttgt ggttggtttt gtgttttttt tgtttggttt ttttgatttg 240
   gaatattttt gattataaaa attttgattt tatttttgtt ggaaattttt tttgattttt 300
   tttttttttg aaatatggtt tttttttttg ttatttaggt tggagtgtag tggagttatt 360
   atagtttatt gtagttttga atgtttgggt ttaagtaatt tttttatttt atttttttga 420
   atagttggga ttataggtgt gtattattat gtttagttaa tttttttttt tgtgtagaga 480
   aagggttttt atgttgttta agttggtttt aagtttttgg ttttaagtgg tttttttatt 540
   ttagttattt aaagtattgg gattatatag gtttttatat attgtgttta gtttttgatt 600
   taaaaaatag tggagatgag ttggtttgta ttttttttgt ggatttgttg tatggggttg 660
   tgggttggtt tgtatttttt tgtaggtggt ttgtttaagt tgatttttat ttgggatttg 720
   tgtgtgtttg agtttgtgtt ttggttgtgt aattttgagt ttgttttatt tttttagttt 780
   ttttttgaaa gtgaaatgtt atagtttttg tttaagaata aaggatttaa gtttagtttt 840
   gtatgttaat aatttttaag tgtggttatt tttttttgtt tggtttgatt tattttggtt 900
   gttgtttatt gtggttgtta tggaaatata atttgaggag aatttagatt aggataggga 960
   ggttatgttt tgggtggtag tggagtttgg taaggtgttt ttttggtttg ggattgttgt 1020
   ggtagaggaa gtatgaggtg gggtttggtt tgtttagttt agagttgagg tgtttttttg 1080
   gttttttatt tttttgtttt ttggttggtt tattgtattt atgtatttaa tgtagtggtt 1140
   ttgttttttt tttaggatgg ttggaggtgg gattattgtt aggatggttt taagtgttta 1200
   gtttttttgg aagatttagt tttttttttt gttttatgta gaggtgtaga gatttattat 1260
   tgtaagtggg gtgataatat ggtgaggggt tggagtgtgg gttttggggt agaattttga 1320
   tttggttatt tttggttgtg agatttgggg taaggtattt aatttttttg tattttaatt 1380
   tttttttttg taaaataata gtttttattt tataggattg ttgtgaggat ggtatggtat 1440
   ttgggttagt tttttgttat agggtttgtg tttaatttgg agtttggttg aatgtaaagt 1500
   gtttggggat ttttttgtaa tgttttaagt tgttgagtgt tgtgtatatg taaagtataa 1560
   ttattgtgga tagttagtgt ggttagagtg agaggataat ttattttggg atttttaaat 1620
   gattagtatt agtagatttt ggttataatt tatagatgtt ttttttgtgg aagttgggtt 1680
   tttatttttg ttttttttgt ttattttttt tttttagtta ttttgtatat gtatataggt 1740
   ttattatata taaaggtagt atgaatttta atttaatttg tgtttgggtt gtgggtttgt 1800
   taatttttat aattttgttg ttagggatat atagattttg taggatgagt tagttatatg 1860
   tttgttatta gtaagtagtg tttaagtatt atttgaatgt attattgttt ttttttttga 1920
   gatggaggtt ttgttttgtt gtttaggttg gagtgagtgg tgtgatttta ggttattgta 1980
   atttttgttt tttaggttta agtattgaat gtattatggt tttttttgtt ttttgttttt 2040
   tgtttttttt tttttgaggt ggagttttgt ttttgttttt taggttggag tgtaatggtg 2100
   tgattttggt tttttgtaat ttttgttttt ttgtaatttt tgttttttgg gtttaagtaa 2160
   tttttttgtt ttagtttttt gagtagttgg gattataggt atttgttatt atgtttggtt 2220
   aatttttgta tttttagtag agatggggtt ttattatgtt gtttaggttg attttgaatt 2280
   tttgatttta ggtgattagt ttgttttggt tttttaaagt gttgggatta taggtgtgag 2340
   ttattatgtt tggttagtgt attatatttt aatgatgatt attgaagaag ttaagggatt 2400
   gagaaatgtt tagttgtttt tggaagattt ttgtaattgt tggttgtttt ttatgttgtt 2460
   tttttatatt ttttaagttt aattaaattt gtaatttatt tgtaggttgg ggttgttttg 2520
   gttatatttt ttatatagtt taatttttta tgtttatttt gtgttgggag aaggggagta 2580
   tattatttat attttttagg tggttgtttt tttgtgatag atagagtagg tgtaagtttg 2640
   ggtttgtgag gtgatttggt agtgtttttg tttgatgatt gttgattaag tttaggtgtg 2700
   tagatttagt gtttatagag ttttggttgg tttatggttg gggttggaat gaagaaaata 2760
   aatatttagg ggaggggtag gtggtatgga tgatttaaaa gttatttgtt tagggtttag 2820
   agttagaggt aaggtatttt tttaggtttt tttaaattaa ttagatagga attttatgtt 2880
   atgagtgttt atagtgtgtg ttggttgtta tagggattaa gtagtttgtg aaagttagtt 2940
   tgtatatttg tggattagtt gggaatttag agtggatata gagaatgttg gaggtgaaaa 3000
   g 3001
<210> 416
   <211> 50
   <212> DNA
   <213> human
<400> 416
   cgcctgcacg gccagctttg tccagcgagc caccccctgc ctgggcagcc 50
<210> 417
   <211> 50
   <212> DNA
   <213> human
<400> 417
   cgtttgtacg gttagttttg tttagcgagt tattttttgt ttgggtagtt 50
<210> 418
   <211> 50
   <212> DNA
   <213> human
<400> 418
   tgtttgtatg gttagttttg tttagtgagt tattttttgt ttgggtagtt 50
<210> 419
   <211> 50
   <212> DNA
   <213> human
<400> 419
   ggttgtttag gtagggggtg gttcgttgga taaagttggt cgtgtaggcg 50
<210> 420
   <211> 50
   <212> DNA
   <213> human
<400> 420
   ggttgtttag gtagggggtg gtttgttgga taaagttggt tgtgtaggtg 50
<210> 421
   <211> 3001
   <212> DNA
   <213> human
<400> 421
   ctctgggcca agctgggtac tcacggccac cgcgagggtc accgatgcca gctccacctt 60
   gcccaggtgc ccgcagaaca cagtgctcac gatgtagatc ataaaagtca gcacctggaa 120
   caggaactgg aggacagcgg ccaggtcaga ctcggggcca caggtgccca cacagccgag 180
   ccctgcaaga caggcctctc cagctttgag ggccccgcac cagtgccccg agaggtcacc 240
   tacgcctggg gaagccaggg ctggaggtcg gatgacctag cccagctccc attctgcgtt 300
   ctgcagctgc agtcacgcct ggcctccacg tccctaccca gaagggtcag tgcctggcgc 360
   agcgcagtgc ctggcgccct gagcttgacc caagtgtctg atactgctat tgcagttcct 420
   attataacaa taatttggtc aggccgcaca cccccaaaaa aggaggggaa ttaaatacac 480
   caaaatctta gtaatggcta tttccaagta ctaggatttg agttaaattt ctttcttctt 540
   tatatctttc atttttttct aaaagtttta caggaagtgc aattccatta ctcctttttg 600
   gttttgtttt ttttgttttt tttttttttt ttgagacgga gtttcgccct tgttgcccag 660
   gctggagtgc agtggcgcga tctcggctca ctgcaaccta cccgtcccgg gttcaagcga 720
   ttctctgcct ctctctccca agtagctgga attacaggcg cccgccacca tgcctggtta 780
   atttttgtat ttttagtaga gtcggggggt tctccatgtt ggccgggctg gtctccaact 840
   cctgacctca ggtgatcctc cctgctcggc ctcccaaagt gctgagataa caggcgtgag 900
   gcagcacgcc cagcctattc ctcttcatga tcagaaaaaa taaatgtatt taaaacaatt 960
   tcccctttgg aaatgaaatg ggtagccggc cccttcttcc ccctgcgaag gtgctgcagg 1020
   cttcatgcat ctctgggtcc catttgtcct ggagccatct tccaactcag gcttttgtgg 1080
   gtttccagct gaggcacacc caccccttct gtgtgggggc tgcctgctgc tgagcaaagt 1140
   caggccccac tgccactggg gattgtccag agacagcttt caggagccat cctgctgtcc 1200
   ccagactctg gacctggcag tcaacatggg cagtttctgg ggaaaatgtg agcacatttc 1260
   tggatcctgc ctgcaaggca gacaccctca gcctctcagc ttcctccact ccctacctgc 1320
   cccccagctc ctcctcctta ccaggggtcc agaaagggca aagagagtcc acatctcagt 1380
   cccaaagcct ctgggaacca gcctgctgag ggcagggcag cagcccccat ggtccagggc 1440
   cactgtgtcc tggaggctgt ccattcctgg ccggggcact ggctaccctg cacgcctgag 1500
   cgcctgcacg gccagctttg tccagcgagc caccccctgc ctgggcagcc cgtgtcggta 1560
   cagcctggct gatgagtaag gggcaggagg aggggctacc atggcaactt gtgggatgag 1620
   ccctgcctcc tagggcactg agtccctgct gccaagccta ctgcctcagc cactagggag 1680
   gacaggttcc tgggagtcac ccagaccgct gcctgttgtg tctggtctcc cagagggatt 1740
   ggcagtaagg cctcagcagg agaaggtcca gggctgcctg cagtggctgt gggaggtgtg 1800
   ctgaggccag gacagggcag cctggctggg gcagctgagc cactgggcgt cctgtacccg 1860
   ggcgtgatgg tgcccacagt gactcccgtt tttcagaaga attggtggcc tgcccgaagt 1920
   cacagagcag gtggtggcag agcctgaaca gccctcagcc ctgcttttcc ctgggagggg 1980
   tggcttccca agagacaggg gcctggcaaa ccccatggca gtgcggtccc tagcctttca 2040
   tgctggaggg gcctgggcct gggcctgggg atagggatgg gagctaggac acctcggcgg 2100
   agtcccaagc cccccactgc ccgtcacccc tttcagcagc atatctgaga gccaggggtg 2160
   gcagtgcttg ggaaagcagc tcactatgca cgccagtcac tgtgcccagc aaggctgtga 2220
   ctcgacgtcc cacagggtcc tctgtcacct tcccaaattg ccctctctgc ttttccttgc 2280
   agggatggcc tgtgactccc ccctcaacct gcctcaagac cagcagggca gtgggaacca 2340
   agagaggcat ctaaggattg caccggtcac ctagcccagt cctggatcaa gcttttgggt 2400
   gtggttcagc ccaggagaag gaggagtgag cgaggggggc aggaagacag caggcaggct 2460
   ggccagtggc tgtgggaggg ctgaagacat cccaaaggcc ccacttggtt cccatgtcca 2520
   cccctgcagc ggaggagtgg ccggcaggat tccgagatgc agcccccaga gccgcgagga 2580
   ggaaggagca gaccctgaag gagaggaggc aggacaaata caaaaaaaaa aaaagggttt 2640
   gctacaccca tgggcggccc caaacacagg cacagcccca ccccagtcaa cacgcagcct 2700
   tcttttttga cacagaattc tgaaacgctc cactggatcc tcccaggatc acctgggagg 2760
   tcacattcct gcccttgttc tgatggttct tatcacagca ctctccaacc tgtgcaccaa 2820
   atagtacctt taccacctgc ctagaggaaa gaagacattt tatcaccatc gtgattgtga 2880
   tgatgacacc aaacaggagg aagtacaggt gaaagctaca gccctgcagg ccaggctatt 2940
   gctagtatgt ctgacttccc tcctccaaca ccttctccac gtccttctca cctttggcca 3000
   g 3001

<210> 422
   <211> 3001
   <212> DNA
   <213> human
<400> 422
   ttttgggtta agttgggtat ttacggttat cgcgagggtt atcgatgtta gttttatttt      60
   gtttaggtgt tcgtagaata tagtgtttac gatgtagatt ataaaagtta gtatttggaa     120
   taggaattgg aggatagcgg ttaggttaga ttcggggtta taggtgttta tatagtcgag     180
   ttttgtaaga taggtttttt tagttttgag ggtttcgtat tagtgtttcg agaggttatt     240
   tacgtttggg gaagttaggg ttggaggtcg gatgatttag tttagttttt attttgcgtt     300
   ttgtagttgt agttacgttt ggtttttacg tttttattta gaagggttag tgtttggcgt     360
   agcgtagtgt ttggcgtttt gagtttgatt taagtgtttg atattgttat tgtagttttt     420
   attataataa taatttggtt aggtcgtata tttttaaaaa aggaggggaa ttaaatatat     480
   taaaatttta gtaatggtta tttttaagta ttaggatttg agttaaattt tttttttttt     540
   tatatttttt attttttttt aaaagtttta taggaagtgt aattttatta tttttttttg     600
   gttttgtttt ttttgttttt tttttttttt ttgagacgga gtttcgtttt tgttgtttag     660
   gttggagtgt agtggcgcga tttcggttta ttgtaattta ttcgtttcgg gtttaagcga     720
   ttttttgttt ttttttttta agtagttgga attataggcg ttcgttatta tgtttggtta     780
   atttttgtat ttttagtaga gtcggggggt tttttatgtt ggtcgggttg gtttttaatt     840
   tttgatttta ggtgattttt tttgttcggt tttttaaagt gttgagataa taggcgtgag     900
   gtagtacgtt tagtttattt ttttttatga ttagaaaaaa taaatgtatt taaaataatt     960
   ttttttttgg aaatgaaatg ggtagtcggt tttttttttt ttttgcgaag gtgttgtagg    1020
   ttttatgtat ttttgggttt tatttgtttt ggagttattt tttaatttag gtttttgtgg    1080
   gtttttagtt gaggtatatt tatttttttt gtgtgggggt tgtttgttgt tgagtaaagt    1140
   taggttttat tgttattggg gattgtttag agatagtttt taggagttat tttgttgttt    1200
   ttagattttg gatttggtag ttaatatggg tagtttttgg ggaaaatgtg agtatatttt    1260
   tggattttgt ttgtaaggta gatattttta gttttttagt tttttttatt ttttatttgt    1320
   tttttagttt ttttttttta ttaggggttt agaaagggta aagagagttt atattttagt    1380
   tttaaagttt ttgggaatta gtttgttgag ggtagggtag tagtttttat ggtttagggt    1440
   tattgtgttt tggaggttgt ttatttttgg tcggggtatt ggttattttg tacgtttgag    1500
   cgtttgtacg gttagttttg tttagcgagt tattttttgt ttgggtagtt cgtgtcggta    1560
   tagtttggtt gatgagtaag gggtaggagg aggggttatt atggtaattt gtgggatgag    1620
   ttttgttttt tagggtattg agtttttgtt gttaagttta ttgttttagt tattagggag    1680
   gataggtttt tgggagttat ttagatcgtt gtttgttgtg tttggttttt tagagggatt    1740
   ggtagtaagg ttttagtagg agaaggttta gggttgtttg tagtggttgt gggaggtgtg    1800
   ttgaggttag gatagggtag tttggttggg gtagttgagt tattgggcgt tttgtattcg    1860
   ggcgtgatgg tgtttatagt gattttcgtt ttttagaaga attggtggtt tgttcgaagt    1920
   tatagagtag gtggtggtag agtttgaata gtttttagtt ttgttttttt ttgggagggg    1980
   tggtttttta agagataggg gtttggtaaa ttttatggta gtgcggtttt tagtttttta    2040
   tgttggaggg gtttgggttt gggtttgggg atagggatgg gagttaggat atttcggcgg    2100
   agttttaagt tttttattgt tcgttatttt ttttagtagt atatttgaga gttaggggtg    2160
   gtagtgtttg ggaaagtagt ttattatgta cgttagttat tgtgtttagt aaggttgtga    2220
   ttcgacgttt tatagggttt tttgttattt ttttaaattg ttttttttgt ttttttttgt    2280
   agggatggtt tgtgattttt tttttaattt gttttaagat tagtagggta gtgggaatta    2340
   agagaggtat ttaaggattg tatcggttat ttagtttagt tttggattaa gtttttgggt    2400
   gtggtttagt ttaggagaag gaggagtgag cgaggggggt aggaagatag taggtaggtt    2460
   ggttagtggt tgtgggaggg ttgaagatat tttaaaggtt ttatttggtt tttatgttta    2520
   tttttgtagc ggaggagtgg tcggtaggat ttcgagatgt agtttttaga gtcgcgagga    2580
   ggaaggagta gattttgaag gagaggaggt aggataaata taaaaaaaaa aaaagggttt    2640
   gttatattta tgggcggttt taaatatagg tatagtttta ttttagttaa tacgtagttt    2700
   ttttttttga tatagaattt tgaaacgttt tattggattt ttttaggatt atttgggagg    2760
   ttatattttt gtttttgttt tgatggtttt tattatagta ttttttaatt tgtgtattaa    2820
   atagtatttt tattatttgt ttagaggaaa gaagatattt tattattatc gtgattgtga    2880
   tgatgatatt aaataggagg aagtataggt gaaagttata gttttgtagg ttaggttatt    2940
   gttagtatgt ttgatttttt ttttttaata ttttttttac gtttttttta tttttggtta    3000
   g                                                                    3001
<210> 423
   <211> 3001
   <212> DNA
   <213> human
<400> 423
   ttttgggtta agttgggtat ttatggttat tgtgagggtt attgatgtta gttttatttt 60
   gtttaggtgt ttgtagaata tagtgtttat gatgtagatt ataaaagtta gtatttggaa 120
   taggaattgg aggatagtgg ttaggttaga tttggggtta taggtgttta tatagttgag 180
   ttttgtaaga taggtttttt tagttttgag ggttttgtat tagtgttttg agaggttatt 240
   tatgtttggg gaagttaggg ttggaggttg gatgatttag tttagttttt attttgtgtt 300
   ttgtagttgt agttatgttt ggtttttatg tttttattta gaagggttag tgtttggtgt 360
   agtgtagtgt ttggtgtttt gagtttgatt taagtgtttg atattgttat tgtagttttt 420
   attataataa taatttggtt aggttgtata tttttaaaaa aggaggggaa ttaaatatat 480
   taaaatttta gtaatggtta tttttaagta ttaggatttg agttaaattt tttttttttt 540
   tatatttttt attttttttt aaaagtttta taggaagtgt aattttatta tttttttttg 600
   gttttgtttt ttttgttttt tttttttttt ttgagatgga gttttgtttt tgttgtttag 660
   gttggagtgt agtggtgtga ttttggttta ttgtaattta tttgttttgg gtttaagtga 720
   ttttttgttt ttttttttta agtagttgga attataggtg tttgttatta tgtttggtta 780
   atttttgtat ttttagtaga gttggggggt tttttatgtt ggttgggttg gtttttaatt 840
   tttgatttta ggtgattttt tttgtttggt tttttaaagt gttgagataa taggtgtgag 900
   gtagtatgtt tagtttattt ttttttatga ttagaaaaaa taaatgtatt taaaataatt 960
   ttttttttgg aaatgaaatg ggtagttggt tttttttttt ttttgtgaag gtgttgtagg 1020
   ttttatgtat ttttgggttt tatttgtttt ggagttattt tttaatttag gtttttgtgg 1080
   gtttttagtt gaggtatatt tatttttttt gtgtgggggt tgtttgttgt tgagtaaagt 1140
   taggttttat tgttattggg gattgtttag agatagtttt taggagttat tttgttgttt 1200
   ttagattttg gatttggtag ttaatatggg tagtttttgg ggaaaatgtg agtatatttt 1260
   tggattttgt ttgtaaggta gatattttta gttttttagt tttttttatt ttttatttgt 1320
   tttttagttt ttttttttta ttaggggttt agaaagggta aagagagttt atattttagt 1380
   tttaaagttt ttgggaatta gtttgttgag ggtagggtag tagtttttat ggtttagggt 1440
   tattgtgttt tggaggttgt ttatttttgg ttggggtatt ggttattttg tatgtttgag 1500
   tgtttgtatg gttagttttg tttagtgagt tattttttgt ttgggtagtt tgtgttggta 1560
   tagtttggtt gatgagtaag gggtaggagg aggggttatt atggtaattt gtgggatgag 1620
   ttttgttttt tagggtattg agtttttgtt gttaagttta ttgttttagt tattagggag 1680
   gataggtttt tgggagttat ttagattgtt gtttgttgtg tttggttttt tagagggatt 1740
   ggtagtaagg ttttagtagg agaaggttta gggttgtttg tagtggttgt gggaggtgtg 1800
   ttgaggttag gatagggtag tttggttggg gtagttgagt tattgggtgt tttgtatttg 1860
   ggtgtgatgg tgtttatagt gatttttgtt ttttagaaga attggtggtt tgtttgaagt 1920
   tatagagtag gtggtggtag agtttgaata gtttttagtt ttgttttttt ttgggagggg 1980
   tggtttttta agagataggg gtttggtaaa ttttatggta gtgtggtttt tagtttttta 2040
   tgttggaggg gtttgggttt gggtttgggg atagggatgg gagttaggat attttggtgg 2100
   agttttaagt tttttattgt ttgttatttt ttttagtagt atatttgaga gttaggggtg 2160
   gtagtgtttg ggaaagtagt ttattatgta tgttagttat tgtgtttagt aaggttgtga 2220
   tttgatgttt tatagggttt tttgttattt ttttaaattg ttttttttgt ttttttttgt 2280
   agggatggtt tgtgattttt tttttaattt gttttaagat tagtagggta gtgggaatta 2340
   agagaggtat ttaaggattg tattggttat ttagtttagt tttggattaa gtttttgggt 2400
   gtggtttagt ttaggagaag gaggagtgag tgaggggggt aggaagatag taggtaggtt 2460
   ggttagtggt tgtgggaggg ttgaagatat tttaaaggtt ttatttggtt tttatgttta 2520
   tttttgtagt ggaggagtgg ttggtaggat tttgagatgt agtttttaga gttgtgagga 2580
   ggaaggagta gattttgaag gagaggaggt aggataaata taaaaaaaaa aaaagggttt 2640
   gttatattta tgggtggttt taaatatagg tatagtttta ttttagttaa tatgtagttt 2700
   ttttttttga tatagaattt tgaaatgttt tattggattt ttttaggatt atttgggagg 2760
   ttatattttt gtttttgttt tgatggtttt tattatagta ttttttaatt tgtgtattaa 2820
   atagtatttt tattatttgt ttagaggaaa gaagatattt tattattatt gtgattgtga 2880
   tgatgatatt aaataggagg aagtataggt gaaagttata gttttgtagg ttaggttatt 2940
   gttagtatgt ttgatttttt ttttttaata ttttttttat gtttttttta tttttggtta 3000
   g 3001
<210> 424
   <211> 3001
   <212> DNA
   <213> human
<400> 424
   ttggttaaag gtgagaagga cgtggagaag gtgttggagg agggaagtta gatatattag 60
   taatagtttg gtttgtaggg ttgtagtttt tatttgtatt tttttttgtt tggtgttatt 120
   attataatta cgatggtgat aaaatgtttt ttttttttta ggtaggtggt aaaggtatta 180
   tttggtgtat aggttggaga gtgttgtgat aagaattatt agaataaggg taggaatgtg 240
   attttttagg tgattttggg aggatttagt ggagcgtttt agaattttgt gttaaaaaag 300
   aaggttgcgt gttgattggg gtggggttgt gtttgtgttt ggggtcgttt atgggtgtag 360
   taaatttttt tttttttttt gtatttgttt tgtttttttt tttttagggt ttgttttttt 420
   tttttcgcgg ttttgggggt tgtatttcgg aattttgtcg gttatttttt cgttgtaggg 480
   gtggatatgg gaattaagtg gggtttttgg gatgttttta gtttttttat agttattggt 540
   tagtttgttt gttgtttttt tgtttttttc gtttattttt tttttttttg ggttgaatta 600
   tatttaaaag tttgatttag gattgggtta ggtgatcggt gtaattttta gatgtttttt 660
   ttggttttta ttgttttgtt ggttttgagg taggttgagg ggggagttat aggttatttt 720
   tgtaaggaaa agtagagagg gtaatttggg aaggtgatag aggattttgt gggacgtcga 780
   gttatagttt tgttgggtat agtgattggc gtgtatagtg agttgttttt ttaagtattg 840
   ttatttttgg tttttagata tgttgttgaa aggggtgacg ggtagtgggg ggtttgggat 900
   ttcgtcgagg tgttttagtt tttattttta tttttaggtt taggtttagg tttttttagt 960
   atgaaaggtt agggatcgta ttgttatggg gtttgttagg tttttgtttt ttgggaagtt 1020
   atttttttta gggaaaagta gggttgaggg ttgtttaggt tttgttatta tttgttttgt 1080
   gatttcgggt aggttattaa tttttttgaa aaacgggagt tattgtgggt attattacgt 1140
   tcgggtatag gacgtttagt ggtttagttg ttttagttag gttgttttgt tttggtttta 1200
   gtatattttt tatagttatt gtaggtagtt ttggattttt ttttgttgag gttttattgt 1260
   taattttttt gggagattag atataatagg tagcggtttg ggtgattttt aggaatttgt 1320
   tttttttagt ggttgaggta gtaggtttgg tagtagggat ttagtgtttt aggaggtagg 1380
   gtttatttta taagttgtta tggtagtttt tttttttgtt ttttatttat tagttaggtt 1440
   gtatcgatac gggttgttta ggtagggggt ggttcgttgg ataaagttgg tcgtgtaggc 1500
   gtttaggcgt gtagggtagt tagtgtttcg gttaggaatg gatagttttt aggatatagt 1560
   ggttttggat tatgggggtt gttgttttgt ttttagtagg ttggttttta gaggttttgg 1620
   gattgagatg tggatttttt ttgttttttt tggatttttg gtaaggagga ggagttgggg 1680
   ggtaggtagg gagtggagga agttgagagg ttgagggtgt ttgttttgta ggtaggattt 1740
   agaaatgtgt ttatattttt tttagaaatt gtttatgttg attgttaggt ttagagtttg 1800
   gggatagtag gatggttttt gaaagttgtt tttggataat ttttagtggt agtggggttt 1860
   gattttgttt agtagtaggt agtttttata tagaaggggt gggtgtgttt tagttggaaa 1920
   tttataaaag tttgagttgg aagatggttt taggataaat gggatttaga gatgtatgaa 1980
   gtttgtagta ttttcgtagg gggaagaagg ggtcggttat ttattttatt tttaaagggg 2040
   aaattgtttt aaatatattt attttttttg attatgaaga ggaataggtt gggcgtgttg 2100
   ttttacgttt gttattttag tattttggga ggtcgagtag ggaggattat ttgaggttag 2160
   gagttggaga ttagttcggt taatatggag aatttttcga ttttattaaa aatataaaaa 2220
   ttaattaggt atggtggcgg gcgtttgtaa ttttagttat ttgggagaga gaggtagaga 2280
   atcgtttgaa ttcgggacgg gtaggttgta gtgagtcgag atcgcgttat tgtattttag 2340
   tttgggtaat aagggcgaaa tttcgtttta aaaaaaaaaa aaaaaataaa aaaaataaaa 2400
   ttaaaaagga gtaatggaat tgtatttttt gtaaaatttt tagaaaaaaa tgaaagatat 2460
   aaagaagaaa gaaatttaat ttaaatttta gtatttggaa atagttatta ttaagatttt 2520
   ggtgtattta attttttttt ttttttgggg gtgtgcggtt tgattaaatt attgttataa 2580
   taggaattgt aatagtagta ttagatattt gggttaagtt tagggcgtta ggtattgcgt 2640
   tgcgttaggt attgattttt ttgggtaggg acgtggaggt taggcgtgat tgtagttgta 2700
   gaacgtagaa tgggagttgg gttaggttat tcgattttta gttttggttt ttttaggcgt 2760
   aggtgatttt tcggggtatt ggtgcggggt ttttaaagtt ggagaggttt gttttgtagg 2820
   gttcggttgt gtgggtattt gtggtttcga gtttgatttg gtcgttgttt tttagttttt 2880
   gttttaggtg ttgattttta tgatttatat cgtgagtatt gtgttttgcg ggtatttggg 2940
   taaggtggag ttggtatcgg tgattttcgc ggtggtcgtg agtatttagt ttggtttaga 3000
   g 3001
<210> 425
   <211> 3001
   <212> DNA
   <213> human
<400> 425
   ttggttaaag gtgagaagga tgtggagaag gtgttggagg agggaagtta gatatattag      60
   taatagtttg gtttgtaggg ttgtagtttt tatttgtatt tttttttgtt tggtgttatt     120
   attataatta tgatggtgat aaaatgtttt ttttttttta ggtaggtggt aaaggtatta     180
   tttggtgtat aggttggaga gtgttgtgat aagaattatt agaataaggg taggaatgtg     240
   attttttagg tgattttggg aggatttagt ggagtgtttt agaattttgt gttaaaaaag     300
   aaggttgtgt gttgattggg gtggggttgt gtttgtgttt ggggttgttt atgggtgtag     360
   taaatttttt tttttttttt gtatttgttt tgtttttttt tttttagggt ttgttttttt     420
   ttttttgtgg ttttgggggt tgtattttgg aattttgttg gttatttttt tgttgtaggg     480
   gtggatatgg gaattaagtg gggtttttgg gatgttttta gtttttttat agttattggt     540
   tagtttgttt gttgtttttt tgtttttttt gtttattttt tttttttttg ggttgaatta     600
   tatttaaaag tttgatttag gattgggtta ggtgattggt gtaattttta gatgtttttt     660
   ttggttttta ttgttttgtt ggttttgagg taggttgagg ggggagttat aggttatttt     720
   tgtaaggaaa agtagagagg gtaatttggg aaggtgatag aggattttgt gggatgttga     780
   gttatagttt tgttgggtat agtgattggt gtgtatagtg agttgttttt ttaagtattg     840
   ttatttttgg tttttagata tgttgttgaa aggggtgatg ggtagtgggg ggtttgggat     900
   tttgttgagg tgttttagtt tttattttta tttttaggtt taggtttagg tttttttagt     960
   atgaaaggtt agggattgta ttgttatggg gtttgttagg tttttgtttt ttgggaagtt    1020
   atttttttta gggaaaagta gggttgaggg ttgtttaggt tttgttatta tttgttttgt    1080
   gattttgggt aggttattaa tttttttgaa aaatgggagt tattgtgggt attattatgt    1140
   ttgggtatag gatgtttagt ggtttagttg ttttagttag gttgttttgt tttggtttta    1200
   gtatattttt tatagttatt gtaggtagtt ttggattttt ttttgttgag gttttattgt    1260
   taattttttt gggagattag atataatagg tagtggtttg ggtgattttt aggaatttgt    1320
   tttttttagt ggttgaggta gtaggtttgg tagtagggat ttagtgtttt aggaggtagg    1380
   gtttatttta taagttgtta tggtagtttt tttttttgtt ttttatttat tagttaggtt    1440
   gtattgatat gggttgttta ggtagggggt ggtttgttgg ataaagttgg ttgtgtaggt    1500
   gtttaggtgt gtagggtagt tagtgttttg gttaggaatg gatagttttt aggatatagt    1560
   ggttttggat tatgggggtt gttgttttgt ttttagtagg ttggttttta gaggttttgg    1620
   gattgagatg tggatttttt ttgttttttt tggatttttg gtaaggagga ggagttgggg    1680
   ggtaggtagg gagtggagga agttgagagg ttgagggtgt ttgttttgta ggtaggattt    1740
   agaaatgtgt ttatattttt tttagaaatt gtttatgttg attgttaggt ttagagtttg    1800
   gggatagtag gatggttttt gaaagttgtt tttggataat ttttagtggt agtggggttt    1860
   gattttgttt agtagtaggt agtttttata tagaaggggt gggtgtgttt tagttggaaa    1920
   tttataaaag tttgagttgg aagatggttt taggataaat gggatttaga gatgtatgaa    1980
   gtttgtagta tttttgtagg gggaagaagg ggttggttat ttattttatt tttaaagggg    2040
   aaattgtttt aaatatattt attttttttg attatgaaga ggaataggtt gggtgtgttg    2100
   ttttatgttt gttattttag tattttggga ggttgagtag ggaggattat ttgaggttag    2160
   gagttggaga ttagtttggt taatatggag aattttttga ttttattaaa aatataaaaa    2220
   ttaattaggt atggtggtgg gtgtttgtaa ttttagttat ttgggagaga gaggtagaga    2280
   attgtttgaa tttgggatgg gtaggttgta gtgagttgag attgtgttat tgtattttag    2340
   tttgggtaat aagggtgaaa ttttgtttta aaaaaaaaaa aaaaaataaa aaaaataaaa    2400
   ttaaaaagga gtaatggaat tgtatttttt gtaaaatttt tagaaaaaaa tgaaagatat    2460
   aaagaagaaa gaaatttaat ttaaatttta gtatttggaa atagttatta ttaagatttt    2520
   ggtgtattta attttttttt ttttttgggg gtgtgtggtt tgattaaatt attgttataa    2580
   taggaattgt aatagtagta ttagatattt gggttaagtt tagggtgtta ggtattgtgt    2640
   tgtgttaggt attgattttt ttgggtaggg atgtggaggt taggtgtgat tgtagttgta    2700
   gaatgtagaa tgggagttgg gttaggttat ttgattttta gttttggttt ttttaggtgt    2760
   aggtgatttt ttggggtatt ggtgtggggt ttttaaagtt ggagaggttt gttttgtagg    2820
   gtttggttgt gtgggtattt gtggttttga gtttgatttg gttgttgttt tttagttttt    2880
   gttttaggtg ttgattttta tgatttatat tgtgagtatt gtgttttgtg ggtatttggg    2940
   taaggtggag ttggtattgg tgatttttgt ggtggttgtg agtatttagt ttggtttaga    3000
   g                                                                    3001
<210> 426
   <211> 50
   <212> DNA
   <213> human
<400> 426
   cgcccggctt taaaaaatgg ttttgtaatg taagtggagg ataataccct 50
<210> 427
   <211> 50
   <212> DNA
   <213> human
<400> 427
   cgttcggttt taaaaaatgg ttttgtaatg taagtggagg ataatatttt 50
<210> 428
   <211> 50
   <212> DNA
   <213> human
<400> 428
   tgtttggttt taaaaaatgg ttttgtaatg taagtggagg ataatatttt 50
<210> 429
   <211> 50
   <212> DNA
   <213> human
<400> 429
   agggtattat tttttattta tattataaaa ttatttttta aagtcgggcg 50
<210> 430
   <211> 50
   <212> DNA
   <213> human
<400> 430
   agggtattat tttttattta tattataaaa ttatttttta aagttgggtg 50
<210> 431
   <211> 3001
   <212> DNA
   <213> human
<400> 431
   gttcaaaaat cacttttata tttaactgaa atttgcttac ttataatcac atctaacctt 60
   caaagaaaac acattaacca actgtactgg gtaatgttac tgggtgatcc cacgttttac 120
   aaatgagaag atatattctg gtaagttgaa tacttagcac ccaggggtac acagcttgga 180
   caggaccagg tccaaagact gttaagagtc ttctgactcc aaactcagtg ctccctccag 240
   tgccacaagc aaactccata aaggtatcct gtgctgaata gagactgtag agtggtacaa 300
   agtaagacag acattatatt aagtcttagc tttgtgactt cgaatgactt acctaatcta 360
   gctaaatttc agttttacca tgtgtaaatc gggaagagta atagaacaaa ccttgaaggg 420
   tcccaatggt gattaaatga ggtgatgtac ataacatgca tcactcataa taagtgctct 480
   ttaaatatta gtcactatta ttagccatct ctgattagat ttgacaatag gaacattagg 540
   aaagatatag tacattcagg attttgttag aaagagatga agaaattccc ttccttcctg 600
   ccctaggtca tctaggagtt gtcatggttc attgttgaca aattaatttt cccaaatttt 660
   tcactttgct cagaaagtct acatcgaagc acccaagact gtacaatcta gtccatcttt 720
   ttccacttaa ctcatactgt gctctccctt tctcaaagca aactgtttgc tattccttga 780
   atacactctg agttttctgc ctttgcctac tcagctggcc catggcccct aatgtttctt 840
   ctcatctcca ctgggtcaaa tcctacctgt accttatggt tctgttaaaa gcagtgcttc 900
   cataaagtac tcctagcaaa tgcacggcct ctctcacgga ttataagaac acagtttatt 960
   ttataaagca tgtagctatt ctctcccttg aaatacgatt attattatta agaatttata 1020
   gcggggatat aattttgtat gatgattctt ctggttaatc caaccaagat tgattttata 1080
   tctattacgt aagacagtag ccagacatag ccgggatatg aaaataaagt ctctgccttc 1140
   aacaagttcc agtattcttt tctttcctcc cctcccctcc cctcccttcc cctccccttc 1200
   cttccctttc ccttcccttc ctttctttct tgagggagtc tcactctgtc accaggctcc 1260
   agtgcagtgg cgctatcttg gctgactgca acctccgcct ccccggttca agcgattctc 1320
   ctgcctcagc ctcctgagta gctgggacta caggagcccg ccaccacgcc cagctaattt 1380
   ttgtattttt agtagagatg gggtttcacc atgttggcca ggatggtctc gatttctcga 1440
   cttcgtgatc cgcctgtctg ggcctcccaa agtgctggga ttacaggcgt gagccaccac 1500
   gcccggcttt aaaaaatggt tttgtaatgt aagtggagga taatacccta catgtttatt 1560
   aataacaata atattcttta ggaaaaaggg cgcggtggtg atttacactg atgacaagca 1620
   ttcccgacta tggaaaaaaa gcgcagcttt ttctgctctg cttttattca gtagagtatt 1680
   gtagagattg tatagaattt cagagttgaa taaaagttcc tcataattat aggagtggag 1740
   agaggagagt ctctttcttc ctttcatttt tatatttaag caagagctgg acattttcca 1800
   agaaagtttt tttttttaag gcgcctctca aaaggggccg gatttccttc tcctggaggc 1860
   agatgttgcc tctctctctc gctcggattg gttcagtgca ctctagaaac actgctgtgg 1920
   tggagaaact ggaccccagg tctggagcga attccagcct gcagggctga taagcgaggc 1980
   attagtgaga ttgagagaga ctttaccccg ccgtggtggt tggagggcgc gcagtagagc 2040
   agcagcacag gcgcgggtcc cgggaggccg gctctgctcg cgccgagatg tggaatctcc 2100
   ttcacgaaac cgactcggct gtggccaccg cgcgccgccc gcgctggctg tgcgctgggg 2160
   cgctggtgct ggcgggtggc ttctttctcc tcggcttcct cttcggtagg ggggcgcctc 2220
   gcggagcaaa cctcggagtc ttccccgtgg tgccgcggtg ctgggactcg cgggtcagct 2280
   gccgagtggg atcctgttgc tggtcttccc caggggcggc gattagggtc ggggtaatgt 2340
   ggggtgagca cccctcgagt taggaggagg gtagctggga acggtgcagg gctgagttct 2400
   cgacaagctg ctggtaggac agtcactcag gttgagggta gaactgagag aacctgaaac 2460
   tgggcgtagg aaggttccaa gtgctggagc cctgcaagac agaggaagtt ttttttttgc 2520
   ttttgttttg ttttgttttg ttttgtttgt ttgtttgttt ttttacctct ctgtgcattc 2580
   tttcttcctt ggaagtaaca gaggcaagct tgggaactgt gtgaaccagg tcagcaatct 2640
   ggacaggtct ttaccagcgg gtcttttgct gtttttcctg ggtactgatt tgcagacttg 2700
   atccaacttt ctaagaaaag cagaaccaca caggcaagct cagactcttt tattaaattc 2760
   cagttttgac tttgccactt cttagtggcc ttgaacaagt taccgagtcc ctctcagcgt 2820
   tagttaccct attttatgat gaggataata ttatctgcaa attattggta atagtaaata 2880
   atatagcatg taaatctcct agcacagtac tgggattttc gccactttat ttcttctttt 2940
   accaagatac tcctcattgg actttaatac acaggactag tctaaggtat caccaggtag 3000
   t 3001
<210> 432
   <211> 3001
   <212> DNA
   <213> human
<400> 432
   gtttaaaaat tatttttata tttaattgaa atttgtttat ttataattat atttaatttt 60
   taaagaaaat atattaatta attgtattgg gtaatgttat tgggtgattt tacgttttat 120
   aaatgagaag atatattttg gtaagttgaa tatttagtat ttaggggtat atagtttgga 180
   taggattagg tttaaagatt gttaagagtt ttttgatttt aaatttagtg ttttttttag 240
   tgttataagt aaattttata aaggtatttt gtgttgaata gagattgtag agtggtataa 300
   agtaagatag atattatatt aagttttagt tttgtgattt cgaatgattt atttaattta 360
   gttaaatttt agttttatta tgtgtaaatc gggaagagta atagaataaa ttttgaaggg 420
   ttttaatggt gattaaatga ggtgatgtat ataatatgta ttatttataa taagtgtttt 480
   ttaaatatta gttattatta ttagttattt ttgattagat ttgataatag gaatattagg 540
   aaagatatag tatatttagg attttgttag aaagagatga agaaattttt tttttttttg 600
   ttttaggtta tttaggagtt gttatggttt attgttgata aattaatttt tttaaatttt 660
   ttattttgtt tagaaagttt atatcgaagt atttaagatt gtataattta gtttattttt 720
   ttttatttaa tttatattgt gttttttttt ttttaaagta aattgtttgt tattttttga 780
   atatattttg agttttttgt ttttgtttat ttagttggtt tatggttttt aatgtttttt 840
   tttattttta ttgggttaaa ttttatttgt attttatggt tttgttaaaa gtagtgtttt 900
   tataaagtat ttttagtaaa tgtacggttt tttttacgga ttataagaat atagtttatt 960
   ttataaagta tgtagttatt tttttttttg aaatacgatt attattatta agaatttata 1020
   gcggggatat aattttgtat gatgattttt ttggttaatt taattaagat tgattttata 1080
   tttattacgt aagatagtag ttagatatag tcgggatatg aaaataaagt ttttgttttt 1140
   aataagtttt agtatttttt tttttttttt tttttttttt tttttttttt tttttttttt 1200
   tttttttttt tttttttttt tttttttttt tgagggagtt ttattttgtt attaggtttt 1260
   agtgtagtgg cgttattttg gttgattgta attttcgttt tttcggttta agcgattttt 1320
   ttgttttagt tttttgagta gttgggatta taggagttcg ttattacgtt tagttaattt 1380
   ttgtattttt agtagagatg gggttttatt atgttggtta ggatggtttc gatttttcga 1440
   tttcgtgatt cgtttgtttg ggttttttaa agtgttggga ttataggcgt gagttattac 1500
   gttcggtttt aaaaaatggt tttgtaatgt aagtggagga taatatttta tatgtttatt 1560
   aataataata atatttttta ggaaaaaggg cgcggtggtg atttatattg atgataagta 1620
   ttttcgatta tggaaaaaaa gcgtagtttt ttttgttttg tttttattta gtagagtatt 1680
   gtagagattg tatagaattt tagagttgaa taaaagtttt ttataattat aggagtggag 1740
   agaggagagt tttttttttt tttttatttt tatatttaag taagagttgg atatttttta 1800
   agaaagtttt tttttttaag gcgtttttta aaaggggtcg gatttttttt ttttggaggt 1860
   agatgttgtt tttttttttc gttcggattg gtttagtgta ttttagaaat attgttgtgg 1920
   tggagaaatt ggattttagg tttggagcga attttagttt gtagggttga taagcgaggt 1980
   attagtgaga ttgagagaga ttttatttcg tcgtggtggt tggagggcgc gtagtagagt 2040
   agtagtatag gcgcgggttt cgggaggtcg gttttgttcg cgtcgagatg tggaattttt 2100
   tttacgaaat cgattcggtt gtggttatcg cgcgtcgttc gcgttggttg tgcgttgggg 2160
   cgttggtgtt ggcgggtggt tttttttttt tcggtttttt tttcggtagg ggggcgtttc 2220
   gcggagtaaa tttcggagtt tttttcgtgg tgtcgcggtg ttgggattcg cgggttagtt 2280
   gtcgagtggg attttgttgt tggttttttt taggggcggc gattagggtc ggggtaatgt 2340
   ggggtgagta tttttcgagt taggaggagg gtagttggga acggtgtagg gttgagtttt 2400
   cgataagttg ttggtaggat agttatttag gttgagggta gaattgagag aatttgaaat 2460
   tgggcgtagg aaggttttaa gtgttggagt tttgtaagat agaggaagtt ttttttttgt 2520
   ttttgttttg ttttgttttg ttttgtttgt ttgtttgttt ttttattttt ttgtgtattt 2580
   tttttttttt ggaagtaata gaggtaagtt tgggaattgt gtgaattagg ttagtaattt 2640
   ggataggttt ttattagcgg gttttttgtt gttttttttg ggtattgatt tgtagatttg 2700
   atttaatttt ttaagaaaag tagaattata taggtaagtt tagatttttt tattaaattt 2760
   tagttttgat tttgttattt tttagtggtt ttgaataagt tatcgagttt tttttagcgt 2820
   tagttatttt attttatgat gaggataata ttatttgtaa attattggta atagtaaata 2880
   atatagtatg taaatttttt agtatagtat tgggattttc gttattttat tttttttttt 2940
   attaagatat tttttattgg attttaatat ataggattag tttaaggtat tattaggtag 3000
   t 3001
<210> 433
   <211> 3001
   <212> DNA
   <213> human
<400> 433
   gtttaaaaat tatttttata tttaattgaa atttgtttat ttataattat atttaatttt      60
   taaagaaaat atattaatta attgtattgg gtaatgttat tgggtgattt tatgttttat     120
   aaatgagaag atatattttg gtaagttgaa tatttagtat ttaggggtat atagtttgga     180
   taggattagg tttaaagatt gttaagagtt ttttgatttt aaatttagtg ttttttttag     240
   tgttataagt aaattttata aaggtatttt gtgttgaata gagattgtag agtggtataa     300
   agtaagatag atattatatt aagttttagt tttgtgattt tgaatgattt atttaattta     360
   gttaaatttt agttttatta tgtgtaaatt gggaagagta atagaataaa ttttgaaggg     420
   ttttaatggt gattaaatga ggtgatgtat ataatatgta ttatttataa taagtgtttt     480
   ttaaatatta gttattatta ttagttattt ttgattagat ttgataatag gaatattagg     540
   aaagatatag tatatttagg attttgttag aaagagatga agaaattttt tttttttttg     600
   ttttaggtta tttaggagtt gttatggttt attgttgata aattaatttt tttaaatttt     660
   ttattttgtt tagaaagttt atattgaagt atttaagatt gtataattta gtttattttt     720
   ttttatttaa tttatattgt gttttttttt ttttaaagta aattgtttgt tattttttga      780
   atatattttg agttttttgt ttttgtttat ttagttggtt tatggttttt aatgtttttt    840
   tttattttta ttgggttaaa ttttatttgt attttatggt tttgttaaaa gtagtgtttt     900
   tataaagtat ttttagtaaa tgtatggttt tttttatgga ttataagaat atagtttatt     960
   ttataaagta tgtagttatt tttttttttg aaatatgatt attattatta agaatttata    1020
   gtggggatat aattttgtat gatgattttt ttggttaatt taattaagat tgattttata    1080
   tttattatgt aagatagtag ttagatatag ttgggatatg aaaataaagt ttttgttttt    1140
   aataagtttt agtatttttt tttttttttt tttttttttt tttttttttt tttttttttt    1200
   tttttttttt tttttttttt tttttttttt tgagggagtt ttattttgtt attaggtttt    1260
   agtgtagtgg tgttattttg gttgattgta atttttgttt ttttggttta agtgattttt    1320
   ttgttttagt tttttgagta gttgggatta taggagtttg ttattatgtt tagttaattt    1380
   ttgtattttt agtagagatg ggttttattt atgttggtta ggatggtttt gattttttga     1440
   ttttgtgatt tgtttgtttg ggttttttaa agtgttggga ttataggtgt gagttattat    1500
   gtttggtttt aaaaaatggt tttgtaatgt aagtggagga taatatttta tatgtttatt    1560
   aataataata atatttttta ggaaaaaggg tgtggtggtg atttatattg atgataagta   1620
   tttttgatta tggaaaaaaa gtgtagtttt ttttgttttg tttttattta gtagagtatt    1680
   gtagagattg tatagaattt tagagttgaa taaaagtttt ttataattat aggagtggag    1740
   agaggagagt tttttttttt tttttatttt tatatttaag taagagttgg atatttttta    1800
   agaaagtttt tttttttaag gtgtttttta aaaggggttg gatttttttt ttttggaggt   1860
   agatgttgtt tttttttttt gtttggattg gtttagtgta ttttagaaat attgttgtgg    1920
   tggagaaatt ggattttagg tttggagtga attttagttt gtagggttga taagtgaggt    1980
   attagtgaga ttgagagaga ttttattttg ttgtggtggt tggagggtgt gtagtagagt     2040
   agtagtatag gtgtgggttt tgggaggttg gttttgtttg tgttgagatg tggaattttt    2100
   tttatgaaat tgatttggtt gtggttattg tgtgttgttt gtgttggttg tgtgttgggg    2160
   tgttggtgtt ggtgggtggt tttttttttt ttggtttttt ttttggtagg ggggtgtttt    2220
   gtggagtaaa ttttggagtt ttttttgtgg tgttgtggtg ttgggatttg tgggttagtt     2280
   gttgagtggg attttgttgt tggttttttt taggggtggt gattagggtt ggggtaatgt    2340
   ggggtgagta ttttttgagt taggaggagg gtagttggga atggtgtagg gttgagtttt   2400
   tgataagttg ttggtaggat agttatttag gttgagggta gaattgagag aatttgaaat    2460
   tgggtgtagg aaggttttaa gtgttggagt tttgtaagat agaggaagtt ttttttttgt    2520
   ttttgttttg ttttgttttg ttttgtttgt ttgtttgttt ttttattttt ttgtgtattt    2580
   tttttttttt ggaagtaata gaggtaagtt tgggaattgt gtgaattagg ttagtaattt    2640
   ggataggttt ttattagtgg gttttttgtt gttttttttg ggtattgatt tgtagatttg     2700
   atttaatttt ttaagaaaag tagaattata taggtaagtt tagatttttt tattaaattt    2760
   tagttttgat tttgttattt tttagtggtt ttgaataagt tattgagttt tttttagtgt    2820
   tagttatttt attttatgat gaggataata ttatttgtaa attattggta atagtaaata    2880
   atatagtatg taaatttttt agtatagtat tgggattttt gttattttat tttttttttt    2940
   attaagatat tttttattgg attttaatat ataggattag tttaaggtat tattaggtag    3000
   t                                                                    3001
<210> 434
   <211> 3001
   <212> DNA
   <213> human
<400> 434
   attatttggt gatattttag attagttttg tgtattaaag tttaatgagg agtattttgg 60
   taaaagaaga aataaagtgg cgaaaatttt agtattgtgt taggagattt atatgttata 120
   ttatttatta ttattaataa tttgtagata atattatttt tattataaaa tagggtaatt 180
   aacgttgaga gggattcggt aatttgttta aggttattaa gaagtggtaa agttaaaatt 240
   ggaatttaat aaaagagttt gagtttgttt gtgtggtttt gtttttttta gaaagttgga 300
   ttaagtttgt aaattagtat ttaggaaaaa tagtaaaaga ttcgttggta aagatttgtt 360
   tagattgttg atttggttta tatagttttt aagtttgttt ttgttatttt taaggaagaa 420
   agaatgtata gagaggtaaa aaaataaata aataaataaa ataaaataaa ataaaataaa 480
   agtaaaaaaa aaattttttt tgttttgtag ggttttagta tttggaattt ttttacgttt 540
   agttttaggt ttttttagtt ttatttttaa tttgagtgat tgttttatta gtagtttgtc 600
   gagaatttag ttttgtatcg tttttagtta tttttttttt aattcgaggg gtgtttattt 660
   tatattattt cgattttaat cgtcgttttt ggggaagatt agtaatagga ttttattcgg 720
   tagttgattc gcgagtttta gtatcgcggt attacgggga agatttcgag gtttgtttcg 780
   cgaggcgttt ttttatcgaa gaggaagtcg aggagaaaga agttattcgt tagtattagc 840
   gttttagcgt atagttagcg cgggcggcgc gcggtggtta tagtcgagtc ggtttcgtga 900
   aggagatttt atatttcggc gcgagtagag tcggtttttc gggattcgcg tttgtgttgt 960
   tgttttattg cgcgtttttt aattattacg gcggggtaaa gtttttttta attttattaa 1020
   tgtttcgttt attagttttg taggttggaa ttcgttttag atttggggtt tagttttttt 1080
   attatagtag tgtttttaga gtgtattgaa ttaattcgag cgagagagag aggtaatatt 1140
   tgtttttagg agaaggaaat tcggtttttt ttgagaggcg ttttaaaaaa aaaaattttt 1200
   ttggaaaatg tttagttttt gtttaaatat aaaaatgaaa ggaagaaaga gatttttttt 1260
   tttttatttt tataattatg aggaattttt atttaatttt gaaattttat ataattttta 1320
   taatatttta ttgaataaaa gtagagtaga aaaagttgcg tttttttttt atagtcggga 1380
   atgtttgtta ttagtgtaaa ttattatcgc gttttttttt ttaaagaata ttattgttat 1440
   taataaatat gtagggtatt atttttattt tatattataa aattattttt taaagtcggg 1500
   cgtggtggtt tacgtttgta attttagtat tttgggaggt ttagataggc ggattacgaa 1560
   gtcgagaaat cgagattatt ttggttaata tggtgaaatt ttatttttat taaaaatata 1620
   aaaattagtt gggcgtggtg gcgggttttt gtagttttag ttatttagga ggttgaggta 1680
   ggagaatcgt ttgaatcggg gaggcggagg ttgtagttag ttaagatagc gttattgtat 1740
   tggagtttgg tgatagagtg agattttttt aagaaagaaa ggaagggaag ggaaagggaa 1800
   ggaaggggag gggaagggag gggaggggag gggaggaaag aaaagaatat tggaatttgt 1860
   tgaaggtaga gattttattt ttatatttcg gttatgtttg gttattgttt tacgtaatag 1920
   atataaaatt aattttggtt ggattaatta gaagaattat tatataaaat tatattttcg 1980
   ttataaattt ttaataataa taatcgtatt ttaagggaga gaatagttat atgttttata 2040
   aaataaattg tgtttttata attcgtgaga gaggtcgtgt atttgttagg agtattttat 2100
   ggaagtattg tttttaatag aattataagg tataggtagg atttgattta gtggagatga 2160
   gaagaaatat taggggttat gggttagttg agtaggtaaa ggtagaaaat ttagagtgta 2220
   tttaaggaat agtaaatagt ttgttttgag aaagggagag tatagtatga gttaagtgga 2280
   aaaagatgga ttagattgta tagttttggg tgtttcgatg tagatttttt gagtaaagtg 2340
   aaaaatttgg gaaaattaat ttgttaataa tgaattatga taatttttag atgatttagg 2400
   gtaggaagga agggaatttt tttatttttt tttaataaaa ttttgaatgt attatatttt 2460
   ttttaatgtt tttattgtta aatttaatta gagatggtta ataatagtga ttaatattta 2520
   aagagtattt attatgagtg atgtatgtta tgtatattat tttatttaat tattattggg 2580
   attttttaag gtttgtttta ttattttttt cgatttatat atggtaaaat tgaaatttag 2640
   ttagattagg taagttattc gaagttataa agttaagatt taatataatg tttgttttat 2700
   tttgtattat tttatagttt ttatttagta taggatattt ttatggagtt tgtttgtggt 2760
   attggaggga gtattgagtt tggagttaga agatttttaa tagtttttgg atttggtttt 2820
   gtttaagttg tgtatttttg ggtgttaagt atttaattta ttagaatata tttttttatt 2880
   tgtaaaacgt gggattattt agtaatatta tttagtatag ttggttaatg tgtttttttt 2940
   gaaggttaga tgtgattata agtaagtaaa ttttagttaa atataaaagt gatttttgaa 3000
   t 3001
<210> 435
   <211> 3001
   <212> DNA
   <213> human
<400> 435
   attatttggt gatattttag attagttttg tgtattaaag tttaatgagg agtattttgg        60
   taaaagaaga aataaagtgg tgaaaatttt agtattgtgt taggagattt atatgttata       120
   ttatttatta ttattaataa tttgtagata atattatttt tattataaaa tagggtaatt       180
   aatgttgaga gggatttggt aatttgttta aggttattaa gaagtggtaa agttaaaatt       240
   ggaatttaat aaaagagttt gagtttgttt gtgtggtttt gtttttttta gaaagttgga       300
   ttaagtttgt aaattagtat ttaggaaaaa tagtaaaaga tttgttggta aagatttgtt       360
   tagattgttg atttggttta tatagttttt aagtttgttt ttgttatttt taaggaagaa       420
   agaatgtata gagaggtaaa aaaataaata aataaataaa ataaaataaa ataaaataaa       480
   agtaaaaaaa aaattttttt tgttttgtag ggttttagta tttggaattt ttttatgttt       540
   agttttaggt ttttttagtt ttatttttaa tttgagtgat tgttttatta gtagtttgtt        600
   gagaatttag ttttgtattg tttttagtta tttttttttt aatttgaggg gtgtttattt       660
   tatattattt tgattttaat tgttgttttt ggggaagatt agtaatagga ttttatttgg       720
   tagttgattt gtgagtttta gtattgtggt attatgggga agattttgag gtttgttttg       780
   tgaggtgttt ttttattgaa gaggaagttg aggagaaaga agttatttgt tagtattagt       840
   gttttagtgt atagttagtg tgggtggtgt gtggtggtta tagttgagtt ggttttgtga       900
   aggagatttt atattttggt gtgagtagag ttggtttttt gggatttgtg tttgtgttgt       960
   tgttttattg tgtgtttttt aattattatg gtggggtaaa gtttttttta attttattaa      1020
   tgttttgttt attagttttg taggttggaa tttgttttag atttggggtt tagttttttt      1080
   attatagtag tgtttttaga gtgtattgaa ttaatttgag tgagagagag aggtaatatt      1140
   tgtttttagg agaaggaaat ttggtttttt ttgagaggtg ttttaaaaaa aaaaattttt      1200
   ttggaaaatg tttagttttt gtttaaatat aaaaatgaaa ggaagaaaga gatttttttt      1260
   tttttatttt tataattatg aggaattttt atttaatttt gaaattttat ataattttta      1320
   taatatttta ttgaataaaa gtagagtaga aaaagttgtg tttttttttt atagttggga      1380
   atgtttgtta ttagtgtaaa ttattattgt gttttttttt ttaaagaata ttattgttat      1440
   taataaatat gtagggtatt atttttattt tatattataa aattattttt taaagttggg      1500
   tgtggtggtt tatgtttgta attttagtat tttgggaggt ttagataggt ggattatgaa      1560
   gttgagaaat tgagattatt ttggttaata tggtgaaatt ttatttttat taaaaatata      1620
   aaaattagtt gggtgtggtg gtgggttttt gtagttttag ttatttagga ggttgaggta      1680
   ggagaattgt ttgaattggg gaggtggagg ttgtagttag ttaagatagt gttattgtat      1740
   tggagtttgg tgatagagtg agattttttt aagaaagaaa ggaagggaag ggaaagggaa      1800
   ggaaggggag gggaagggag gggaggggag gggaggaaag aaaagaatat tggaatttgt      1860
   tgaaggtaga gattttattt ttatattttg gttatgtttg gttattgttt tatgtaatag      1920
   atataaaatt aattttggtt ggattaatta gaagaattat tatataaaat tatatttttg      1980
   ttataaattt ttaataataa taattgtatt ttaagggaga gaatagttat atgttttata      2040
   aaataaattg tgtttttata atttgtgaga gaggttgtgt atttgttagg agtattttat      2100
   ggaagtattg tttttaatag aattataagg tataggtagg atttgattta gtggagatga      2160
   gaagaaatat taggggttat gggttagttg agtaggtaaa ggtagaaaat ttagagtgta      2220
   tttaaggaat agtaaatagt ttgttttgag aaagggagag tatagtatga gttaagtgga      2280
   aaaagatgga ttagattgta tagttttggg tgttttgatg tagatttttt gagtaaagtg      2340
   aaaaatttgg gaaaattaat ttgttaataa tgaattatga taatttttag atgatttagg      2400
   gtaggaagga agggaatttt tttatttttt tttaataaaa ttttgaatgt attatatttt      2460
   ttttaatgtt tttattgtta aatttaatta gagatggtta ataatagtga ttaatattta      2520
   aagagtattt attatgagtg atgtatgtta tgtatattat tttatttaat tattattggg      2580
   attttttaag gtttgtttta ttattttttt tgatttatat atggtaaaat tgaaatttag      2640
   ttagattagg taagttattt gaagttataa agttaagatt taatataatg tttgttttat      2700
   tttgtattat tttatagttt ttatttagta taggatattt ttatggagtt tgtttgtggt      2760
   attggaggga gtattgagtt tggagttaga agatttttaa tagtttttgg atttggtttt      2820
   gtttaagttg tgtatttttg ggtgttaagt atttaattta ttagaatata tttttttatt      2880
   tgtaaaatgt gggattattt agtaatatta tttagtatag ttggttaatg tgtttttttt      2940
   gaaggttaga tgtgattata agtaagtaaa ttttagttaa atataaaagt gatttttgaa      3000
   t                                                                      3001
<210> 436
   <211> 50
   <212> DNA
   <213> human
<400> 436
   cactgtcatg cctcagtgga gagtgtcggc cttcattgaa aacaacatcg 50
<210> 437
   <211> 50
   <212> DNA
   <213> human
<400> 437
   tattgttatg ttttagtgga gagtgtcggt ttttattgaa aataatatcg 50
<210> 438
   <211> 50
   <212> DNA
   <213> human
<400> 438
   tattgttatg ttttagtgga gagtgttggt ttttattgaa aataatattg 50
<210> 439
   <211> 50
   <212> DNA
   <213> human
<400> 439
   cgatgttgtt tttaatgaag gtcgatattt tttattgagg tatgatagtg 50
<210> 440
   <211> 50
   <212> DNA
   <213> human
<400> 440
   tgatgttgtt tttaatgaag gttgatattt tttattgagg tatgatagtg 50
<210> 441
   <211> 3001
   <212> DNA
   <213> human
<400> 441
   catatttgga tttaaggtag tccaatgaaa cagagagaaa gtaaaactgt aaagagaaaa      60
   tcatttttta tgatgttaag aagaaaagaa aatgaagtgg cagcttaaac tttggaaaga      120
   tcagacagag agcttaaggt gatctccaga agaaaattgc tagtcgaatt ttctcagata      180
   aacatttatg cttagtatag cactactgtt agagcatcat tattttgaga agaaagttca      240
   aattgtcttg cgtttagggg tttctatgtc ctactgcaga cataatatta aaaggtagga      300
   aaacatattt ggttaatacc taatataata cagtttggta aatatagttc ccttgtcttg      360
   taaatatctt attttgaaat atcagccatt tgtataggcc caagggtctt gtgtcatctt      420
   cagaattcta gttccttcag cccaggaatt caaagcagca agaataacac cacaaaataa      480
   gctcataagc ttattttaat gctatagtgc tggatacata agataatcat ctggtgattt      540
   ttattttggc aaaaaatgtt tttataagtg aagattatca gaaaagtgtc aatgagtatt      600
   ttttctggct taaagaactc caatatgttt tcagtgccaa ggagaaggac agccttgtgt      660
   atttaattac tttagcttat tttgtcctct ggcatgatct gttccctgat ttcaccacag      720
   tagagcttgc tttgaagttg tagaaaaatt aaatcaatca cttggatcac atgtgtccca      780
   aagaatgtgg cttcccaggt aaaagcaatc tttcaatccc ctactctcgt ttcactttgg      840
   catttcaaca gcgttgcaga attcttcggc agataattta ttttatccct tggtcccagc      900
   agctcttatt aagagtttca gaattgcctg tacaaggtgc acctttcaca ttacaaggga      960
   tcggctcatt tcttgatgct tatgcaacca cttataacta tgcctacagg taaacatact     1020
   gctgtcttct tggaagaccg agaagtataa agaatgttta tgtgcaggtc tagctgttcc     1080
   aagtacccaa gtgttggttt agacacacct ggattaccat ccacctttta ttggctgaga     1140
   aagttgccac acctgatttg taagtttacc tgttgcagcc aatagcaggg ccatctcagc     1200
   cagccagcac tggatactat ctggccagaa gtagcaaagc agctcttatt tgaaaaacca     1260
   ctgggttccg agttcattac tacaggaaaa actgttctct tctgtggcac agagaaccct     1320
   gcttcaaagc agaagtagca gttccggagt ccagctggct aaaactcatc ccagaggata     1380
   atggcaaccc atgccttaga aatcgctggg ctgtttcttg gtggtgttgg aatggtgggc     1440
   acagtggctg tcactgtcat gcctcagtgg agagtgtcgg ccttcattga aaacaacatc     1500
   gtggtttttg aaaacttctg ggaaggactg tggatgaatt gcgtgaggca ggctaacatc     1560
   aggatgcagt gcaaaatcta tgattccctg ctggctcttt ctccggacct acaggcagcc     1620
   agaggactga tgtgtgctgc ttccgtgatg tccttcttgg ctttcatgat ggccatcctt     1680
   ggcatgaaat gcaccaggtg cacgggggac aatgagaagg tgaaggctca cattctgctg     1740
   acggctggaa tcatcttcat catcacgggc atggtggtgc tcatccctgt gagctgggtt     1800
   gccaatgcca tcatcagaga tttctataac tcaatagtga atgttgccca aaaacgtgag     1860
   cttggagaag ctctctactt aggatggacc acggcactgg tgctgattgt tggaggagct     1920
   ctgttctgct gcgttttttg ttgcaacgaa aagagcagta gctacagata ctcgatacct     1980
   tcccatcgca caacccaaaa aagttatcac accggaaaga agtcaccgag cgtctactcc     2040
   agaagtcagt atgtgtagtt gtgtatgttt ttttaacttt actataaagc catgcaaatg     2100
   acaaaaatct atattacttt ctcaaaatgg accccaaaga aactttgatt tactgttctt     2160
   aactgcctaa tcttaattac aggaactgtg catcagctat ttatgattct ataagctatt     2220
   tcagcagaat gagatattaa acccaatgct ttgattgttc tagaaagtat agtaatttgt     2280
   tttctaaggt ggttcaagca tctactcttt ttatcattta cttcaaaatg acattgctaa     2340
   agactgcatt attttactac tgtaatttct ccacgacata gcattatgta catagatgag     2400
   tgtaacattt atatctcaca tagagacatg cttatatggt tttatttaaa atgaaatgcc     2460
   agtccattac actgaataaa tagaactcaa ctattgcttt tcagggaaat catggatagg     2520
   gttgaagaag gttactatta attgtttaaa aacagcttag ggattaatgt cctccattta     2580
   taatgaagat taaaatgaag gctttaatca gcattgtaaa ggaaattgaa tggctttctg     2640
   atatgctgtt ttttagccta ggagttagaa atcctaactt ctttatcctc ttctcccaga     2700
   ggcttttttt ttcttgtgta ttaaattaac atttttaaaa agcagatatt ttgtcaaggg     2760
   gctttgcatt caaactgctt ttccagggct atactcagaa gaaagataaa agtgtgatct     2820
   aagaaaaagt gatggtttta ggaaagtgaa aatatttttg tttttgtatt tgaagaagaa     2880
   tgatgcattt tgacaagaaa tcatatatgt atggatatat tttaataagt atttgagtac     2940
   agactttgag gtttcatcaa tataaataaa agagcagaaa aatatgtctt ggttttcatt     3000
   t                                                                     3001
<210> 442
   <211> 3001
   <212> DNA
   <213> human
<400> 442
   tatatttgga tttaaggtag tttaatgaaa tagagagaaa gtaaaattgt aaagagaaaa      60
   ttatttttta tgatgttaag aagaaaagaa aatgaagtgg tagtttaaat tttggaaaga      120
   ttagatagag agtttaaggt gatttttaga agaaaattgt tagtcgaatt tttttagata      180
   aatatttatg tttagtatag tattattgtt agagtattat tattttgaga agaaagttta      240
   aattgttttg cgtttagggg tttttatgtt ttattgtaga tataatatta aaaggtagga      300
   aaatatattt ggttaatatt taatataata tagtttggta aatatagttt ttttgttttg      360
   taaatatttt attttgaaat attagttatt tgtataggtt taagggtttt gtgttatttt      420
   tagaatttta gtttttttag tttaggaatt taaagtagta agaataatat tataaaataa      480
   gtttataagt ttattttaat gttatagtgt tggatatata agataattat ttggtgattt      540
   ttattttggt aaaaaatgtt tttataagtg aagattatta gaaaagtgtt aatgagtatt      600
   ttttttggtt taaagaattt taatatgttt ttagtgttaa ggagaaggat agttttgtgt      660
   atttaattat tttagtttat tttgtttttt ggtatgattt gttttttgat tttattatag      720
   tagagtttgt tttgaagttg tagaaaaatt aaattaatta tttggattat atgtgtttta      780
   aagaatgtgg ttttttaggt aaaagtaatt ttttaatttt ttattttcgt tttattttgg      840
   tattttaata gcgttgtaga atttttcggt agataattta ttttattttt tggttttagt      900
   agtttttatt aagagtttta gaattgtttg tataaggtgt attttttata ttataaggga      960
   tcggtttatt ttttgatgtt tatgtaatta tttataatta tgtttatagg taaatatatt      1020
   gttgtttttt tggaagatcg agaagtataa agaatgttta tgtgtaggtt tagttgtttt      1080
   aagtatttaa gtgttggttt agatatattt ggattattat ttatttttta ttggttgaga      1140
   aagttgttat atttgatttg taagtttatt tgttgtagtt aatagtaggg ttattttagt      1200
   tagttagtat tggatattat ttggttagaa gtagtaaagt agtttttatt tgaaaaatta      1260
   ttgggtttcg agtttattat tataggaaaa attgtttttt tttgtggtat agagaatttt      1320
   gttttaaagt agaagtagta gtttcggagt ttagttggtt aaaatttatt ttagaggata      1380
   atggtaattt atgttttaga aatcgttggg ttgttttttg gtggtgttgg aatggtgggt      1440
   atagtggttg ttattgttat gttttagtgg agagtgtcgg tttttattga aaataatatc      1500
   gtggtttttg aaaatttttg ggaaggattg tggatgaatt gcgtgaggta ggttaatatt      1560
   aggatgtagt gtaaaattta tgattttttg ttggtttttt tttcggattt ataggtagtt      1620
   agaggattga tgtgtgttgt tttcgtgatg ttttttttgg tttttatgat ggttattttt      1680
   ggtatgaaat gtattaggtg tacgggggat aatgagaagg tgaaggttta tattttgttg      1740
   acggttggaa ttatttttat tattacgggt atggtggtgt ttatttttgt gagttgggtt      1800
   gttaatgtta ttattagaga tttttataat ttaatagtga atgttgttta aaaacgtgag      1860
   tttggagaag ttttttattt aggatggatt acggtattgg tgttgattgt tggaggagtt      1920
   ttgttttgtt gcgttttttg ttgtaacgaa aagagtagta gttatagata ttcgatattt      1980
   ttttatcgta taatttaaaa aagttattat atcggaaaga agttatcgag cgtttatttt      2040
   agaagttagt atgtgtagtt gtgtatgttt ttttaatttt attataaagt tatgtaaatg      2100
   ataaaaattt atattatttt tttaaaatgg attttaaaga aattttgatt tattgttttt      2160
   aattgtttaa ttttaattat aggaattgtg tattagttat ttatgatttt ataagttatt      2220
   ttagtagaat gagatattaa atttaatgtt ttgattgttt tagaaagtat agtaatttgt      2280
   tttttaaggt ggtttaagta tttatttttt ttattattta ttttaaaatg atattgttaa      2340
   agattgtatt attttattat tgtaattttt ttacgatata gtattatgta tatagatgag      2400
   tgtaatattt atattttata tagagatatg tttatatggt tttatttaaa atgaaatgtt      2460
   agtttattat attgaataaa tagaatttaa ttattgtttt ttagggaaat tatggatagg      2520
   gttgaagaag gttattatta attgtttaaa aatagtttag ggattaatgt tttttattta      2580
   taatgaagat taaaatgaag gttttaatta gtattgtaaa ggaaattgaa tggttttttg      2640
   atatgttgtt ttttagttta ggagttagaa attttaattt ttttattttt tttttttaga      2700
   ggtttttttt tttttgtgta ttaaattaat atttttaaaa agtagatatt ttgttaaggg      2760
   gttttgtatt taaattgttt ttttagggtt atatttagaa gaaagataaa agtgtgattt      2820
   aagaaaaagt gatggtttta ggaaagtgaa aatatttttg tttttgtatt tgaagaagaa      2880
   tgatgtattt tgataagaaa ttatatatgt atggatatat tttaataagt atttgagtat      2940
   agattttgag gttttattaa tataaataaa agagtagaaa aatatgtttt ggtttttatt      3000
   t                                                                      3001
<210> 443
   <211> 3001
   <212> DNA
   <213> human
<400> 443
   tatatttgga tttaaggtag tttaatgaaa tagagagaaa gtaaaattgt aaagagaaaa      60
   ttatttttta tgatgttaag aagaaaagaa aatgaagtgg tagtttaaat tttggaaaga      120
   ttagatagag agtttaaggt gatttttaga agaaaattgt tagttgaatt tttttagata 180
   aatatttatg tttagtatag tattattgtt agagtattat tattttgaga agaaagttta 240
   aattgttttg tgtttagggg tttttatgtt ttattgtaga tataatatta aaaggtagga 300
   aaatatattt ggttaatatt taatataata tagtttggta aatatagttt ttttgttttg 360
   taaatatttt attttgaaat attagttatt tgtataggtt taagggtttt gtgttatttt 420
   tagaatttta gtttttttag tttaggaatt taaagtagta agaataatat tataaaataa 480
   gtttataagt ttattttaat gttatagtgt tggatatata agataattat ttggtgattt 540
   ttattttggt aaaaaatgtt tttataagtg aagattatta gaaaagtgtt aatgagtatt 600
   ttttttggtt taaagaattt taatatgttt ttagtgttaa ggagaaggat agttttgtgt 660
   atttaattat tttagtttat tttgtttttt ggtatgattt gttttttgat tttattatag 720
   tagagtttgt tttgaagttg tagaaaaatt aaattaatta tttggattat atgtgtttta 780
   aagaatgtgg ttttttaggt aaaagtaatt ttttaatttt ttatttttgt tttattttgg 840
   tattttaata gtgttgtaga attttttggt agataattta ttttattttt tggttttagt 900
   agtttttatt aagagtttta gaattgtttg tataaggtgt attttttata ttataaggga 960
   ttggtttatt ttttgatgtt tatgtaatta tttataatta tgtttatagg taaatatatt 1020
   gttgtttttt tggaagattg agaagtataa agaatgttta tgtgtaggtt tagttgtttt 1080
   aagtatttaa gtgttggttt agatatattt ggattattat ttatttttta ttggttgaga 1140
   aagttgttat atttgatttg taagtttatt tgttgtagtt aatagtaggg ttattttagt 1200
   tagttagtat tggatattat ttggttagaa gtagtaaagt agtttttatt tgaaaaatta 1260
   ttgggttttg agtttattat tataggaaaa attgtttttt tttgtggtat agagaatttt 1320
   gttttaaagt agaagtagta gttttggagt ttagttggtt aaaatttatt ttagaggata 1380
   atggtaattt atgttttaga aattgttggg ttgttttttg gtggtgttgg aatggtgggt 1440
   atagtggttg ttattgttat gttttagtgg agagtgttgg tttttattga aaataatatt 1500
   gtggtttttg aaaatttttg ggaaggattg tggatgaatt gtgtgaggta ggttaatatt 1560
   aggatgtagt gtaaaattta tgattttttg ttggtttttt ttttggattt ataggtagtt 1620
   agaggattga tgtgtgttgt ttttgtgatg ttttttttgg tttttatgat ggttattttt 1680
   ggtatgaaat gtattaggtg tatgggggat aatgagaagg tgaaggttta tattttgttg 1740
   atggttggaa ttatttttat tattatgggt atggtggtgt ttatttttgt gagttgggtt 1800
   gttaatgtta ttattagaga tttttataat ttaatagtga atgttgttta aaaatgtgag 1860
   tttggagaag ttttttattt aggatggatt atggtattgg tgttgattgt tggaggagtt 1920
   ttgttttgtt gtgttttttg ttgtaatgaa aagagtagta gttatagata tttgatattt 1980
   ttttattgta taatttaaaa aagttattat attggaaaga agttattgag tgtttatttt 2040
   agaagttagt atgtgtagtt gtgtatgttt ttttaatttt attataaagt tatgtaaatg 2100
   ataaaaattt atattatttt tttaaaatgg atttaaaaga aattttgatt tattgttttt 2160
   aattgtttaa ttttaattat aggaattgtg tattagttat ttatgatttt ataagttatt 2220
   ttagtagaat gagatattaa atttaatgtt ttgattgttt tagaaagtat agtaatttgt 2280
   tttttaaggt ggtttaagta tttatttttt ttattattta ttttaaaatg atattgttaa 2340
   agattgtatt attttattat tgtaattttt ttatgatata gtattatgta tatagatgag 2400
   tgtaatattt atattttata tagagatatg tttatatggt tttatttaaa atgaaatgtt 2460
   agtttattat attgaataaa tagaatttaa ttattgtttt ttagggaaat tatggatagg 2520
   gttgaagaag gttattatta attgtttaaa aatagtttag ggattaatgt tttttattta 2580
   taatgaagat taaaatgaag gttttaatta gtattgtaaa ggaaattgaa tggttttttg 2640
   atatgttgtt ttttagttta ggagttagaa attttaattt ttttattttt tttttttaga 2700
   ggtttttttt tttttgtgta ttaaattaat atttttaaaa agtagatatt ttgttaaggg 2760
   gttttgtatt taaattgttt ttttagggtt atatttagaa gaaagataaa agtgtgattt 2820
   aagaaaaagt gatggtttta ggaaagtgaa aatatttttg tttttgtatt tgaagaagaa 2880
   tgatgtattt tgataagaaa ttatatatgt atggatatat tttaataagt atttgagtat 2940
   agattttgag gttttattaa tataaataaa agagtagaaa aatatgtttt ggtttttatt 3000
   t 3001
<210> 444
   <211> 3001
   <212> DNA
   <213> human
<400> 444
   aaatgaaaat taagatatat ttttttgttt ttttatttat attgatgaaa ttttaaagtt      60
   tgtatttaaa tatttattaa aatatattta tatatatatg attttttgtt aaaatgtatt     120
   attttttttt aaatataaaa ataaaaatat ttttattttt ttaaaattat tatttttttt     180
   tagattatat ttttattttt tttttgagta tagttttgga aaagtagttt gaatgtaaag     240
   ttttttgata aaatatttgt tttttaaaaa tgttaattta atatataaga aaaaaaaagt     300
   ttttgggaga agaggataaa gaagttagga tttttaattt ttaggttaaa aaatagtata     360
   ttagaaagtt atttaatttt ttttataatg ttgattaaag tttttatttt aatttttatt     420
   ataaatggag gatattaatt tttaagttgt ttttaaataa ttaatagtaa ttttttttaa    480
   ttttatttat gatttttttg aaaagtaata gttgagtttt atttatttag tgtaatggat     540
   tggtatttta ttttaaataa aattatataa gtatgttttt atgtgagata taaatgttat     600
   atttatttat gtatataatg ttatgtcgtg gagaaattat agtagtaaaa taatgtagtt     660
   tttagtaatg ttattttgaa gtaaatgata aaaagagtag atgtttgaat tattttagaa     720
   aataaattat tatatttttt agaataatta aagtattggg tttaatattt tattttgttg     780
   aaatagttta tagaattata aatagttgat gtatagtttt tgtaattaag attaggtagt     840
   taagaatagt aaattaaagt ttttttgggg tttattttga gaaagtaata tagatttttg     900
   ttatttgtat ggttttatag taaagttaaa aaaatatata taattatata tattgatttt     960
   tggagtagac gttcggtgat tttttttcgg tgtgataatt tttttgggtt gtgcgatggg    1020
   aaggtatcga gtatttgtag ttattgtttt tttcgttgta ataaaaaacg tagtagaata    1080
   gagttttttt aataattagt attagtgtcg tggtttattt taagtagaga gtttttttaa   1140
   gtttacgttt ttgggtaata tttattattg agttatagaa atttttgatg atggtattgg    1200
   taatttagtt tatagggatg agtattatta tgttcgtgat gatgaagatg attttagtcg    1260
   ttagtagaat gtgagttttt atttttttat tgtttttcgt gtatttggtg tattttatgt    1320
   taaggatggt tattatgaaa gttaagaagg atattacgga agtagtatat attagttttt    1380
   tggttgtttg taggttcgga gaaagagtta gtagggaatt atagattttg tattgtattt   1440
   tgatgttagt ttgttttacg taatttattt atagtttttt ttagaagttt ttaaaaatta    1500
   cgatgttgtt tttaatgaag gtcgatattt tttattgagg tatgatagtg atagttattg    1560
   tgtttattat tttaatatta ttaagaaata gtttagcgat ttttaaggta tgggttgtta    1620
   ttattttttg ggatgagttt tagttagttg gatttcggaa ttgttatttt tgttttgaag    1680
   tagggttttt tgtgttatag aagagaatag ttttttttgt agtaatgaat tcggaattta    1740
   gtggtttttt aaataagagt tgttttgtta tttttggtta gatagtattt agtgttggtt    1800
   ggttgagatg gttttgttat tggttgtaat aggtaaattt ataaattagg tgtggtaatt    1860
   tttttagtta ataaaaggtg gatggtaatt taggtgtgtt taaattaata tttgggtatt    1920
   tggaatagtt agatttgtat ataaatattt tttatatttt tcggtttttt aagaagatag    1980
   tagtatgttt atttgtaggt atagttataa gtggttgtat aagtattaag aaatgagtcg    2040
   attttttgta atgtgaaagg tgtattttgt ataggtaatt ttgaaatttt taataagagt   2100
   tgttgggatt aagggataaa ataaattatt tgtcgaagaa ttttgtaacg ttgttgaaat    2160
   gttaaagtga aacgagagta ggggattgaa agattgtttt tatttgggaa gttatatttt    2220
   ttgggatata tgtgatttaa gtgattgatt taattttttt ataattttaa agtaagtttt    2280
   attgtggtga aattagggaa tagattatgt tagaggataa aataagttaa agtaattaaa    2340
   tatataaggt tgtttttttt tttggtattg aaaatatatt ggagtttttt aagttagaaa    2400
   aaatatttat tgatattttt ttgataattt ttatttataa aaatattttt tgttaaaata    2460
   aaaattatta gatgattatt ttatgtattt agtattatag tattaaaata agtttatgag    2520
   tttattttgt ggtgttattt ttgttgtttt gaatttttgg gttgaaggaa ttagaatttt    2580
   gaagatgata taagattttt gggtttatat aaatggttga tattttaaaa taagatattt    2640
   ataagataag ggaattatat ttattaaatt gtattatatt aggtattaat taaatatgtt    2700
   tttttatttt ttaatattat gtttgtagta ggatatagaa atttttaaac gtaagataat    2760
   ttgaattttt tttttaaaat aatgatgttt taatagtagt gttatattaa gtataaatgt    2820
   ttatttgaga aaattcgatt agtaattttt ttttggagat tattttaagt tttttgtttg    2880
   atttttttaa agtttaagtt gttattttat tttttttttt ttttaatatt ataaaaaatg    2940
   attttttttt tatagtttta tttttttttt gttttattgg attattttaa atttaaatat    3000
   g                                                                    3001
<210> 445
   <211> 3001
   <212> DNA
   <213> human
<400> 445
   aaatgaaaat taagatatat ttttttgttt ttttatttat attgatgaaa ttttaaagtt 60
   tgtatttaaa tatttattaa aatatattta tatatatatg attttttgtt aaaatgtatt 120
   attttttttt aaatataaaa ataaaaatat ttttattttt ttaaaattat tatttttttt 180
   tagattatat ttttattttt tttttgagta tagttttgga aaagtagttt gaatgtaaag 240
   ttttttgata aaatatttgt tttttaaaaa tgttaattta atatataaga aaaaaaaagt 300
   ttttgggaga agaggataaa gaagttagga tttttaattt ttaggttaaa aaatagtata 360
   ttagaaagtt atttaatttt ttttataatg ttgattaaag tttttatttt aatttttatt 420
   ataaatggag gatattaatt tttaagttgt ttttaaataa ttaatagtaa ttttttttaa 480
   ttttatttat gatttttttg aaaagtaata gttgagtttt atttatttag tgtaatggat 540
   tggtatttta ttttaaataa aattatataa gtatgttttt atgtgagata taaatgttat 600
   atttatttat gtatataatg ttatgttgtg gagaaattat agtagtaaaa taatgtagtt 660
   tttagtaatg ttattttgaa gtaaatgata aaaagagtag atgtttgaat tattttagaa 720
   aataaattat tatatttttt agaataatta aagtattggg tttaatattt tattttgttg 780
   aaatagttta tagaattata aatagttgat gtatagtttt tgtaattaag attaggtagt 840
   taagaatagt aaattaaagt ttttttgggg tttattttga gaaagtaata tagatttttg 900
   ttatttgtat ggttttatag taaagttaaa aaaatatata taattatata tattgatttt 960
   tggagtagat gtttggtgat ttttttttgg tgtgataatt tttttgggtt gtgtgatggg 1020
   aaggtattga gtatttgtag ttattgtttt ttttgttgta ataaaaaatg tagtagaata 1080
   gagttttttt aataattagt attagtgttg tggtttattt taagtagaga gtttttttaa 1140
   gtttatgttt ttgggtaata tttattattg agttatagaa atttttgatg atggtattgg 1200
   taatttagtt tatagggatg agtattatta tgtttgtgat gatgaagatg attttagttg 1260
   ttagtagaat gtgagttttt atttttttat tgttttttgt gtatttggtg tattttatgt 1320
   taaggatggt tattatgaaa gttaagaagg atattatgga agtagtatat attagttttt 1380
   tggttgtttg taggtttgga gaaagagtta gtagggaatt atagattttg tattgtattt 1440
   tgatgttagt ttgttttatg taatttattt atagtttttt ttagaagttt ttaaaaatta 1500
   tgatgttgtt tttaatgaag gttgatattt tttattgagg tatgatagtg atagttattg 1560
   tgtttattat tttaatatta ttaagaaata gtttagtgat ttttaaggta tgggttgtta 1620
   ttattttttg ggatgagttt tagttagttg gattttggaa ttgttatttt tgttttgaag 1680
   tagggttttt tgtgttatag aagagaatag ttttttttgt agtaatgaat ttggaattta 1740
   gtggtttttt aaataagagt tgttttgtta tttttggtta gatagtattt agtgttggtt 1800
   ggttgagatg gttttgttat tggttgtaat aggtaaattt ataaattagg tgtggtaatt 1860
   tttttagtta ataaaaggtg gatggtaatt taggtgtgtt taaattaata tttgggtatt 1920
   tggaatagtt agatttgtat ataaatattt tttatatttt ttggtttttt aagaagatag 1980
   tagtatgttt atttgtaggt atagttataa gtggttgtat aagtattaag aaatgagttg 2040
   attttttgta atgtgaaagg tgtattttgt ataggtaatt ttgaaatttt taataagagt 2100
   tgttgggatt aagggataaa ataaattatt tgttgaagaa ttttgtaatg ttgttgaaat 2160
   gttaaagtga aatgagagta ggggattgaa agattgtttt tatttgggaa gttatatttt 2220
   ttgggatata tgtgatttaa gtgattgatt taattttttt ataattttaa agtaagtttt 2280
   attgtggtga aattagggaa tagattatgt tagaggataa aataagttaa agtaattaaa 2340
   tatataaggt tgtttttttt tttggtattg aaaatatatt ggagtttttt aagttagaaa 2400
   aaatatttat tgatattttt ttgataattt ttatttataa aaatattttt tgttaaaata 2460
   aaaattatta gatgattatt ttatgtattt agtattatag tattaaaata agtttatgag 2520
   tttattttgt ggtgttattt ttgttgtttt gaatttttgg gttgaaggaa ttagaatttt 2580
   gaagatgata taagattttt gggtttatat aaatggttga tattttaaaa taagatattt 2640
   ataagataag ggaattatat ttattaaatt gtattatatt aggtattaat taaatatgtt 2700
   tttttatttt ttaatattat gtttgtagta ggatatagaa atttttaaat gtaagataat 2760
   ttgaattttt tttttaaaat aatgatgttt taatagtagt gttatattaa gtataaatgt 2820
   ttatttgaga aaatttgatt agtaattttt ttttggagat tattttaagt tttttgtttg 2880
   atttttttaa agtttaagtt gttattttat tttttttttt ttttaatatt ataaaaaatg 2940
   attttttttt tatagtttta tttttttttt gttttattgg attattttaa atttaaatat 3000
   g 3001
<210> 446
   <211> 50
   <212> DNA
   <213> human
<400> 446
   ttttaaagac ccgacaaact gggaaattga ccgagttctg tttctccccg 50
<210> 447
   <211> 50
   <212> DNA
   <213> human
<400> 447
   ttttaaagat tcgataaatt gggaaattga tcgagttttg ttttttttcg 50
<210> 448
   <211> 50
   <212> DNA
   <213> human
<400> 448
   ttttaaagat ttgataaatt gggaaattga ttgagttttg tttttttttg 50
<210> 449
   <211> 50
   <212> DNA
   <213> human
<400> 449
   cggggagaaa tagaattcgg ttaatttttt agtttgtcgg gtttttaaaa 50
<210> 450
   <211> 50
   <212> DNA
   <213> human
<400> 450
   tggggagaaa tagaatttgg ttaatttttt agtttgttgg gtttttaaaa 50
<210> 451
   <211> 3001
   <212> DNA
   <213> human
<400> 451
   ctcgtttcac ttacacgaaa gaaacagggg aagaggatta aagggaaaag tagtgccccc      60
   aaagctgaac tattttagaa aggaaagcaa atttaaaaat cacttttctg aggattaaaa     120
   aaggccggga aaccggctcg tcggaggcac aaaggtaagt tttctgggag atgaggggac     180
   gagcattagg aggagagaag agaacacaga actacaggtt taaaaggggt agagggaatt     240
   aaaactatat ttgtcagcca tgttgaagag gctccagtaa tttgtgaagc ttccctcttc     300
   cctcttggta tttgtgggtg acactacagg gtttgctccc cagtccctgg aagcccgaga     360
   aaaagcgaag gggagaagaa agggagagtt tcgcggcata agagaagcgc tctgacctcg     420
   ttctcgtccc tcagccttgc agcaatccag cagttgcagt cgggtctcct ttctaacaaa     480
   tcctaaaggg cagccatctt gcttccttct gcttctctac gaaagcagcc tagcctccgc     540
   atcaatattc ataaggcagt ttgacgtctg ttctcatcac tcagcggggc cggcgtctcg     600
   gggcggccgc gccgaggggg cggacgtgac ttgcatgcga gcgggacccg ggcgcgcggc     660
   gctcgcgagg aggcggcggc ggccgcccaa gctctcgccg ctgccactgc cgctaccgcc     720
   ggctctcctc ctccgcccgg gcctcctcct gctccgcccc ggcctcctcc cgctccgccc     780
   cggcctgcgc cgccgccctg gggccctcag ccccggctcc cagggaatta gccccccgca     840
   ccgggcgtcc agcacaaagg ccggcggcac ctggggccgt acagcggggc gcgcagtgga     900
   gacctgggct gccctcacgc cgtgcagtgg cgcgggggga gtggtgcgcc cggaccagcg     960
   cggagtaggg gccagggctg gcgagcgcgg ggaagggtgc agccctcggg aaccccgggg    1020
   acctcggcgg gtctccaccc ctgggagaag aagcagagca agcgcgaagg ttgggggcga    1080
   ggtgggaggt tgggatggca cgggtgcctg gacaaagacg tgacatctaa gatttccctt    1140
   tgaaactttt tgctggtctg cactccacca ccacttcgag gtcccgcgca cacgcccccg    1200
   cgcgcaccca cctcctccgg agtgtacccg cacgtgccct cgctctggcc cccgcctcca    1260
   cccactggca cctctccccg caccacacct ccgcttgcgc ccaccttacc ctcagaggcc    1320
   cgcgtctcta tctcccttta gatcccgttg cccgcgctct cctttccctt ttctgtcgct   1380
   ttaatctcat tcagcagaag cgctcccctg tttcaccgag ggcatgccct tagtgcttta    1440
   ggggcctgta tttttaaaga cccgacaaac tgggaaattg accgagttct gtttctcccc    1500
   gccccggccc accttctgtt tactttagcc acgtgggctg agtagaaggg ggcgtctcac    1560
   agattgaaat aggtttctca ctagtgaccc cctacttctc gctaggagga aataaacctg    1620
   cactttcaaa tgcttaagtt gcctctcgta agagccctta ctttgaagta gtttcccccc    1680
   gtacaattaa gtaatgaaga aaccgaccga tacagagcta attccgaatg acggatgctt    1740
   gattttccct gacattcgga gctctgtaac tatgctcttc ctttgaccgg agtttttctc   1800
   tattgccgag tagtgttact tcccccttct aatgctcctt tttctaatca tcaacttttt   1860
   atttgaagat gagatgaaga acaaagccca tatgagtcat agtaagtatc atattacgtc    1920
   agatagtcgt ttttgaaata ttcccttcat cttttacatg ttcttacatg actgacgttc     1980
   aaattttaag tgggtatttt ggtcacatct tttatgggat cactgcatgg tgatcacagt    2040
   acccttaagt caatactagt tatattgtgt aaccacactt tttttggtct gtaatttttt    2100
   ttaaaagcga agccctaatt ttacgaaatc ggaatatttc ctctttgaag agtgtgaaaa    2160
   agggaactcc ttgcaagtgt cgcattgttg ctaagaagaa tgtttagtgg gtccaatcaa    2220
   actttgttta ttccattttg aaacaaagtt gactcagatt tcagattttg cacaaactaa   2280
   ggaaatagta cattttccct taaacatttc aggtgggaat gacattccat gttttctagc    2340
   tgtacactaa agcgtcactt aggtcttgga aattgttaat gcactttaaa atctaagctg    2400
   tttgaaagtt ctttgtgatc tttatttctt tctttctttt ctttcctttt ctttctttct    2460
   tgagacagcc tctctctgtc acccaggctg gagtgcagtg gcgcgatctc ggctcactgc    2520
   aacctcggcc ttccgggttc aagcgattct tctgcctcag cctccggagt agctgggact    2580
   acaggcgtgc gccaccacgc ccagctattt tttgtatttt taatagagac ggggtttcac    2640
   catattggcc aggctggtct ggaactcctg acctcccaga gtgctgggat tacaggcgtg    2700
   agccaccgcg cccggcttgt gatgtatttt tcaagtccag gaaataaagt tctctcaaga    2760
   ccacaacata catttataca gtgcacagta ttgcagagtt gttgaaacat gggctttgga    2820
   gttttacaga gctgagtgac tcttaactct atcttttcca gtgtgacctt gtgcatttta    2880
   cttaactctc tcggtatcgg tttctttcaa tctgttcaac aacctgtaag acagcagagt    2940
   tgtggtgagt ttttaataag agcactaggt aagtgcccag gtagttacta ctactacagt    3000
   a                                                                    3001
<210> 452
   <211> 3001
   <212> DNA
   <213> human
<400> 452
   ttcgttttat ttatacgaaa gaaatagggg aagaggatta aagggaaaag tagtgttttt      60
   aaagttgaat tattttagaa aggaaagtaa atttaaaaat tatttttttg aggattaaaa     120
   aaggtcggga aatcggttcg tcggaggtat aaaggtaagt tttttgggag atgaggggac     180
   gagtattagg aggagagaag agaatataga attataggtt taaaaggggt agagggaatt     240
   aaaattatat ttgttagtta tgttgaagag gttttagtaa tttgtgaagt tttttttttt     300
   ttttttggta tttgtgggtg atattatagg gtttgttttt tagtttttgg aagttcgaga      360
   aaaagcgaag gggagaagaa agggagagtt tcgcggtata agagaagcgt tttgatttcg 420
   ttttcgtttt ttagttttgt agtaatttag tagttgtagt cgggtttttt ttttaataaa 480
   ttttaaaggg tagttatttt gttttttttt gtttttttac gaaagtagtt tagttttcgt 540
   attaatattt ataaggtagt ttgacgtttg tttttattat ttagcggggt cggcgtttcg 600
   gggcggtcgc gtcgaggggg cggacgtgat ttgtatgcga gcgggattcg ggcgcgcggc 660
   gttcgcgagg aggcggcggc ggtcgtttaa gttttcgtcg ttgttattgt cgttatcgtc 720
   ggtttttttt tttcgttcgg gttttttttt gtttcgtttc ggtttttttt cgtttcgttt 780
   cggtttgcgt cgtcgttttg gggtttttag tttcggtttt tagggaatta gtttttcgta 840
   tcgggcgttt agtataaagg tcggcggtat ttggggtcgt atagcggggc gcgtagtgga 900
   gatttgggtt gtttttacgt cgtgtagtgg cgcgggggga gtggtgcgtt cggattagcg 960
   cggagtaggg gttagggttg gcgagcgcgg ggaagggtgt agttttcggg aatttcgggg 1020
   atttcggcgg gtttttattt ttgggagaag aagtagagta agcgcgaagg ttgggggcga 1080
   ggtgggaggt tgggatggta cgggtgtttg gataaagacg tgatatttaa gatttttttt 1140
   tgaaattttt tgttggtttg tattttatta ttatttcgag gtttcgcgta tacgttttcg 1200
   cgcgtattta tttttttcgg agtgtattcg tacgtgtttt cgttttggtt ttcgttttta 1260
   tttattggta ttttttttcg tattatattt tcgtttgcgt ttattttatt tttagaggtt 1320
   cgcgttttta ttttttttta gatttcgttg ttcgcgtttt tttttttttt ttttgtcgtt 1380
   ttaattttat ttagtagaag cgtttttttg ttttatcgag ggtatgtttt tagtgtttta 1440
   ggggtttgta tttttaaaga ttcgataaat tgggaaattg atcgagtttt gttttttttc 1500
   gtttcggttt attttttgtt tattttagtt acgtgggttg agtagaaggg ggcgttttat 1560
   agattgaaat aggtttttta ttagtgattt tttatttttc gttaggagga aataaatttg 1620
   tatttttaaa tgtttaagtt gtttttcgta agagttttta ttttgaagta gttttttttc 1680
   gtataattaa gtaatgaaga aatcgatcga tatagagtta atttcgaatg acggatgttt 1740
   gatttttttt gatattcgga gttttgtaat tatgtttttt ttttgatcgg agtttttttt 1800
   tattgtcgag tagtgttatt tttttttttt aatgtttttt tttttaatta ttaatttttt 1860
   atttgaagat gagatgaaga ataaagttta tatgagttat agtaagtatt atattacgtt 1920
   agatagtcgt ttttgaaata ttttttttat tttttatatg tttttatatg attgacgttt 1980
   aaattttaag tgggtatttt ggttatattt tttatgggat tattgtatgg tgattatagt 2040
   atttttaagt taatattagt tatattgtgt aattatattt tttttggttt gtaatttttt 2100
   ttaaaagcga agttttaatt ttacgaaatc ggaatatttt ttttttgaag agtgtgaaaa 2160
   agggaatttt ttgtaagtgt cgtattgttg ttaagaagaa tgtttagtgg gtttaattaa 2220
   attttgttta ttttattttg aaataaagtt gatttagatt ttagattttg tataaattaa 2280
   ggaaatagta tatttttttt taaatatttt aggtgggaat gatattttat gttttttagt 2340
   tgtatattaa agcgttattt aggttttgga aattgttaat gtattttaaa atttaagttg 2400
   tttgaaagtt ttttgtgatt tttatttttt tttttttttt tttttttttt tttttttttt 2460
   tgagatagtt tttttttgtt atttaggttg gagtgtagtg gcgcgatttc ggtttattgt 2520
   aatttcggtt tttcgggttt aagcgatttt tttgttttag ttttcggagt agttgggatt 2580
   ataggcgtgc gttattacgt ttagttattt tttgtatttt taatagagac ggggttttat 2640
   tatattggtt aggttggttt ggaatttttg attttttaga gtgttgggat tataggcgtg 2700
   agttatcgcg ttcggtttgt gatgtatttt ttaagtttag gaaataaagt ttttttaaga 2760
   ttataatata tatttatata gtgtatagta ttgtagagtt gttgaaatat gggttttgga 2820
   gttttataga gttgagtgat ttttaatttt atttttttta gtgtgatttt gtgtatttta 2880
   tttaattttt tcggtatcgg ttttttttaa tttgtttaat aatttgtaag atagtagagt 2940
   tgtggtgagt ttttaataag agtattaggt aagtgtttag gtagttatta ttattatagt 3000
   a 3001
<210> 453
   <211> 3001
   <212> DNA
   <213> human
<400> 453
   tttgttttat ttatatgaaa gaaatagggg aagaggatta aagggaaaag tagtgttttt      60
   aaagttgaat tattttagaa aggaaagtaa atttaaaaat tatttttttg aggattaaaa      120
   aaggttggga aattggtttg ttggaggtat aaaggtaagt tttttgggag atgaggggat      180
   gagtattagg aggagagaag agaatataga attataggtt taaaaggggt agagggaatt      240
   aaaattatat ttgttagtta tgttgaagag gttttagtaa tttgtgaagt tttttttttt      300
   ttttttggta tttgtgggtg atattatagg gtttgttttt tagtttttgg aagtttgaga      360
   aaaagtgaag gggagaagaa agggagagtt ttgtggtata agagaagtgt tttgattttg      420
   tttttgtttt ttagttttgt agtaatttag tagttgtagt tgggtttttt ttttaataaa      480
   ttttaaaggg tagttatttt gttttttttt gtttttttat gaaagtagtt tagtttttgt      540
   attaatattt ataaggtagt ttgatgtttg tttttattat ttagtggggt tggtgttttg      600
   gggtggttgt gttgaggggg tggatgtgat ttgtatgtga gtgggatttg ggtgtgtggt      660
   gtttgtgagg aggtggtggt ggttgtttaa gtttttgttg ttgttattgt tgttattgtt      720
   ggtttttttt ttttgtttgg gttttttttt gttttgtttt ggtttttttt tgttttgttt      780
   tggtttgtgt tgttgttttg gggtttttag ttttggtttt tagggaatta gttttttgta      840
   ttgggtgttt agtataaagg ttggtggtat ttggggttgt atagtggggt gtgtagtgga    900
   gatttgggtt gtttttatgt tgtgtagtgg tgtgggggga gtggtgtgtt tggattagtg    960
   tggagtaggg gttagggttg gtgagtgtgg ggaagggtgt agtttttggg aattttgggg      1020
   attttggtgg gtttttattt ttgggagaag aagtagagta agtgtgaagg ttgggggtga      1080
   ggtgggaggt tgggatggta tgggtgtttg gataaagatg tgatatttaa gatttttttt      1140
   tgaaattttt tgttggtttg tattttatta ttattttgag gttttgtgta tatgtttttg      1200
   tgtgtattta ttttttttgg agtgtatttg tatgtgtttt tgttttggtt tttgttttta      1260
   tttattggta tttttttttg tattatattt ttgtttgtgt ttattttatt tttagaggtt      1320
   tgtgttttta ttttttttta gattttgttg tttgtgtttt tttttttttt ttttgttgtt      1380
   ttaattttat ttagtagaag tgtttttttg ttttattgag ggtatgtttt tagtgtttta      1440
   ggggtttgta tttttaaaga tttgataaat tgggaaattg attgagtttt gttttttttt      1500
   gttttggttt attttttgtt tattttagtt atgtgggttg agtagaaggg ggtgttttat      1560
   agattgaaat aggtttttta ttagtgattt tttatttttt gttaggagga aataaatttg      1620
   tatttttaaa tgtttaagtt gttttttgta agagttttta ttttgaagta gttttttttt      1680
   gtataattaa gtaatgaaga aattgattga tatagagtta attttgaatg atggatgttt      1740
   gatttttttt gatatttgga gttttgtaat tatgtttttt ttttgattgg agtttttttt      1800
   tattgttgag tagtgttatt tttttttttt aatgtttttt tttttaatta ttaatttttt      1860
   atttgaagat gagatgaaga ataaagttta tatgagttat agtaagtatt atattatgtt      1920
   agatagttgt ttttgaaata ttttttttat tttttatatg tttttatatg attgatgttt      1980
   aaattttaag tgggtatttt ggttatattt tttatgggat tattgtatgg tgattatagt      2040
   atttttaagt taatattagt tatattgtgt aattatattt tttttggttt gtaatttttt      2100
   ttaaaagtga agttttaatt ttatgaaatt ggaatatttt ttttttgaag agtgtgaaaa      2160
   agggaatttt ttgtaagtgt tgtattgttg ttaagaagaa tgtttagtgg gtttaattaa      2220
   attttgttta ttttattttg aaataaagtt gatttagatt ttagattttg tataaattaa      2280
   ggaaatagta tatttttttt taaatatttt aggtgggaat gatattttat gttttttagt      2340
   tgtatattaa agtgttattt aggttttgga aattgttaat gtattttaaa atttaagttg      2400
   tttgaaagtt ttttgtgatt tttatttttt tttttttttt tttttttttt tttttttttt      2460
   tgagatagtt tttttttgtt atttaggttg gagtgtagtg gtgtgatttt ggtttattgt      2520
   aattttggtt ttttgggttt aagtgatttt tttgttttag tttttggagt agttgggatt      2580
   ataggtgtgt gttattatgt ttagttattt tttgtatttt taatagagat ggggttttat      2640
   tatattggtt aggttggttt ggaatttttg attttttaga gtgttgggat tataggtgtg      2700
   agttattgtg tttggtttgt gatgtatttt ttaagtttag gaaataaagt ttttttaaga      2760
   ttataatata tatttatata gtgtatagta ttgtagagtt gttgaaatat gggttttgga   2820
   gttttataga gttgagtgat ttttaatttt atttttttta gtgtgatttt gtgtatttta   2880
   tttaattttt ttggtattgg ttttttttaa tttgtttaat aatttgtaag atagtagagt      2940
   tgtggtgagt ttttaataag agtattaggt aagtgtttag gtagttatta ttattatagt      3000
   a                                                                      3001
<210> 454
   <211> 3001
   <212> DNA
   <213> human
<400> 454
   tattgtagta gtagtaatta tttgggtatt tatttagtgt ttttattaaa aatttattat 60
   aattttgttg ttttataggt tgttgaatag attgaaagaa atcgatatcg agagagttaa 120
   gtaaaatgta taaggttata ttggaaaaga tagagttaag agttatttag ttttgtaaaa 180
   ttttaaagtt tatgttttaa taattttgta atattgtgta ttgtataaat gtatgttgtg 240
   gttttgagag aattttattt tttggatttg aaaaatatat tataagtcgg gcgcggtggt 300
   ttacgtttgt aattttagta ttttgggagg ttaggagttt tagattagtt tggttaatat 360
   ggtgaaattt cgtttttatt aaaaatataa aaaatagttg ggcgtggtgg cgtacgtttg 420
   tagttttagt tatttcggag gttgaggtag aagaatcgtt tgaattcgga aggtcgaggt 480
   tgtagtgagt cgagatcgcg ttattgtatt ttagtttggg tgatagagag aggttgtttt 540
   aagaaagaaa gaaaaggaaa gaaaagaaag aaagaaataa agattataaa gaatttttaa 600
   atagtttaga ttttaaagtg tattaataat ttttaagatt taagtgacgt tttagtgtat 660
   agttagaaaa tatggaatgt tatttttatt tgaaatgttt aagggaaaat gtattatttt 720
   tttagtttgt gtaaaatttg aaatttgagt taattttgtt ttaaaatgga ataaataaag 780
   tttgattgga tttattaaat atttttttta gtaataatgc gatatttgta aggagttttt 840
   ttttttatat tttttaaaga ggaaatattt cgatttcgta aaattagggt ttcgttttta 900
   aaaaaaatta tagattaaaa aaagtgtggt tatataatat aattagtatt gatttaaggg 960
   tattgtgatt attatgtagt gattttataa aagatgtgat taaaatattt atttaaaatt 1020
   tgaacgttag ttatgtaaga atatgtaaaa gatgaaggga atattttaaa aacgattatt 1080
   tgacgtaata tgatatttat tatgatttat atgggttttg ttttttattt tatttttaaa 1140
   taaaaagttg atgattagaa aaaggagtat tagaaggggg aagtaatatt attcggtaat 1200
   agagaaaaat ttcggttaaa ggaagagtat agttatagag tttcgaatgt tagggaaaat 1260
   taagtattcg ttattcggaa ttagttttgt atcggtcggt ttttttatta tttaattgta 1320
   cggggggaaa ttattttaaa gtaagggttt ttacgagagg taatttaagt atttgaaagt 1380
   gtaggtttat ttttttttag cgagaagtag ggggttatta gtgagaaatt tattttaatt 1440
   tgtgagacgt ttttttttat ttagtttacg tggttaaagt aaatagaagg tgggtcgggg 1500
   cggggagaaa tagaattcgg ttaatttttt agtttgtcgg gtttttaaaa atataggttt 1560
   ttaaagtatt aagggtatgt tttcggtgaa ataggggagc gtttttgttg aatgagatta 1620
   aagcgataga aaagggaaag gagagcgcgg gtaacgggat ttaaagggag atagagacgc 1680
   gggtttttga gggtaaggtg ggcgtaagcg gaggtgtggt gcggggagag gtgttagtgg 1740
   gtggaggcgg gggttagagc gagggtacgt gcgggtatat ttcggaggag gtgggtgcgc 1800
   gcgggggcgt gtgcgcggga tttcgaagtg gtggtggagt gtagattagt aaaaagtttt 1860
   aaagggaaat tttagatgtt acgtttttgt ttaggtattc gtgttatttt aattttttat 1920
   ttcgttttta attttcgcgt ttgttttgtt ttttttttta ggggtggaga ttcgtcgagg 1980
   ttttcggggt tttcgagggt tgtatttttt ttcgcgttcg ttagttttgg tttttatttc 2040
   gcgttggttc gggcgtatta tttttttcgc gttattgtac ggcgtgaggg tagtttaggt 2100
   ttttattgcg cgtttcgttg tacggtttta ggtgtcgtcg gtttttgtgt tggacgttcg 2160
   gtgcgggggg ttaatttttt gggagtcggg gttgagggtt ttagggcggc ggcgtaggtc 2220
   ggggcggagc gggaggaggt cggggcggag taggaggagg ttcgggcgga ggaggagagt 2280
   cggcggtagc ggtagtggta gcggcgagag tttgggcggt cgtcgtcgtt ttttcgcgag 2340
   cgtcgcgcgt tcgggtttcg ttcgtatgta agttacgttc gttttttcgg cgcggtcgtt 2400
   tcgagacgtc ggtttcgttg agtgatgaga atagacgtta aattgtttta tgaatattga 2460
   tgcggaggtt aggttgtttt cgtagagaag tagaaggaag taagatggtt gttttttagg 2520
   atttgttaga aaggagattc gattgtaatt gttggattgt tgtaaggttg agggacgaga 2580
   acgaggttag agcgtttttt ttatgtcgcg aaattttttt tttttttttt ttttcgtttt 2640
   ttttcgggtt tttagggatt ggggagtaaa ttttgtagtg ttatttataa atattaagag 2700
   ggaagaggga agttttataa attattggag tttttttaat atggttgata aatatagttt 2760
   taattttttt tatttttttt aaatttgtag ttttgtgttt tttttttttt ttttaatgtt 2820
   cgttttttta ttttttagaa aatttatttt tgtgttttcg acgagtcggt ttttcggttt 2880
   tttttaattt ttagaaaagt gatttttaaa tttgtttttt tttttaaaat agtttagttt 2940
   tgggggtatt attttttttt ttaatttttt ttttttgttt ttttcgtgta agtgaaacga 3000
   g 3001
<210> 455
   <211> 3001
   <212> DNA
   <213> human
<400> 455
   tattgtagta gtagtaatta tttgggtatt tatttagtgt ttttattaaa aatttattat      60
   aattttgttg ttttataggt tgttgaatag attgaaagaa attgatattg agagagttaa     120
   gtaaaatgta taaggttata ttggaaaaga tagagttaag agttatttag ttttgtaaaa     180
   ttttaaagtt tatgttttaa taattttgta atattgtgta ttgtataaat gtatgttgtg     240
   gttttgagag aattttattt tttggatttg aaaaatatat tataagttgg gtgtggtggt     300
   ttatgtttgt aattttagta ttttgggagg ttaggagttt tagattagtt tggttaatat     360
   ggtgaaattt tgtttttatt aaaaatataa aaaatagttg ggtgtggtgg tgtatgtttg     420
   tagttttagt tattttggag gttgaggtag aagaattgtt tgaatttgga aggttgaggt     480
   tgtagtgagt tgagattgtg ttattgtatt ttagtttggg tgatagagag aggttgtttt     540
   aagaaagaaa gaaaaggaaa gaaaagaaag aaagaaataa agattataaa gaatttttaa     600
   atagtttaga ttttaaagtg tattaataat ttttaagatt taagtgatgt tttagtgtat     660
   agttagaaaa tatggaatgt tatttttatt tgaaatgttt aagggaaaat gtattatttt     720
   tttagtttgt gtaaaatttg aaatttgagt taattttgtt ttaaaatgga ataaataaag     780
   tttgattgga tttattaaat atttttttta gtaataatgt gatatttgta aggagttttt     840
   ttttttatat tttttaaaga ggaaatattt tgattttgta aaattagggt tttgttttta     900
   aaaaaaatta tagattaaaa aaagtgtggt tatataatat aattagtatt gatttaaggg     960
   tattgtgatt attatgtagt gattttataa aagatgtgat taaaatattt atttaaaatt    1020
   tgaatgttag ttatgtaaga atatgtaaaa gatgaaggga atattttaaa aatgattatt    1080
   tgatgtaata tgatatttat tatgatttat atgggttttg ttttttattt tatttttaaa   1140
   taaaaagttg atgattagaa aaaggagtat tagaaggggg aagtaatatt atttggtaat    1200
   agagaaaaat tttggttaaa ggaagagtat agttatagag ttttgaatgt tagggaaaat    1260
   taagtatttg ttatttggaa ttagttttgt attggttggt ttttttatta tttaattgta   1320
   tggggggaaa ttattttaaa gtaagggttt ttatgagagg taatttaagt atttgaaagt   1380
   gtaggtttat ttttttttag tgagaagtag ggggttatta gtgagaaatt tattttaatt  1440
   tgtgagatgt ttttttttat ttagtttatg tggttaaagt aaatagaagg tgggttgggg  1500
   tggggagaaa tagaatttgg ttaatttttt agtttgttgg gtttttaaaa atataggttt   1560
   ttaaagtatt aagggtatgt ttttggtgaa ataggggagt gtttttgttg aatgagatta    1620
   aagtgataga aaagggaaag gagagtgtgg gtaatgggat ttaaagggag atagagatgt    1680
   gggtttttga gggtaaggtg ggtgtaagtg gaggtgtggt gtggggagag gtgttagtgg    1740
   gtggaggtgg gggttagagt gagggtatgt gtgggtatat tttggaggag gtgggtgtgt    1800
   gtgggggtgt gtgtgtggga ttttgaagtg gtggtggagt gtagattagt aaaaagtttt    1860
   aaagggaaat tttagatgtt atgtttttgt ttaggtattt gtgttatttt aattttttat    1920
   tttgttttta atttttgtgt ttgttttgtt ttttttttta ggggtggaga tttgttgagg    1980
   tttttggggt ttttgagggt tgtatttttt tttgtgtttg ttagttttgg tttttatttt    2040
   gtgttggttt gggtgtatta ttttttttgt gttattgtat ggtgtgaggg tagtttaggt   2100
   ttttattgtg tgttttgttg tatggtttta ggtgttgttg gtttttgtgt tggatgtttg    2160
   gtgtgggggg ttaatttttt gggagttggg gttgagggtt ttagggtggt ggtgtaggtt    2220
   ggggtggagt gggaggaggt tggggtggag taggaggagg tttgggtgga ggaggagagt    2280
   tggtggtagt ggtagtggta gtggtgagag tttgggtggt tgttgttgtt tttttgtgag    2340
   tgttgtgtgt ttgggttttg tttgtatgta agttatgttt gtttttttgg tgtggttgtt  2400
   ttgagatgtt ggttttgttg agtgatgaga atagatgtta aattgtttta tgaatattga    2460
   tgtggaggtt aggttgtttt tgtagagaag tagaaggaag taagatggtt gttttttagg   2520
   atttgttaga aaggagattt gattgtaatt gttggattgt tgtaaggttg agggatgaga    2580
   atgaggttag agtgtttttt ttatgttgtg aaattttttt tttttttttt tttttgtttt    2640
   tttttgggtt tttagggatt ggggagtaaa ttttgtagtg ttatttataa atattaagag    2700
   ggaagaggga agttttataa attattggag tttttttaat atggttgata aatatagttt   2760
   taattttttt tatttttttt aaatttgtag ttttgtgttt tttttttttt ttttaatgtt   2820
   tgttttttta ttttttagaa aatttatttt tgtgtttttg atgagttggt tttttggttt  2880
   tttttaattt ttagaaaagt gatttttaaa tttgtttttt tttttaaaat agtttagttt    2940
   tgggggtatt attttttttt ttaatttttt ttttttgttt tttttgtgta agtgaaatga   3000
   g                                                                   3001
<210> 456 -
   <211> 50
   <212> DNA
   <213> human
<400> 456
   cgtcccggca aggaaaaggg caagaaaagc aaagaactca aaatgttaag 50
<210> 457
   <211> 50
   <212> DNA
   <213> human
<400> 457
   cgtttcggta aggaaaaggg taagaaaagt aaagaattta aaatgttaag 50
<210> 458
   <211> 50
   <212> DNA
   <213> human
<400> 458
   tgttttggta aggaaaaggg taagaaaagt aaagaattta aaatgttaag 50
<210> 459
   <211> 50
   <212> DNA
   <213> human
<400> 459
   tttaatattt tgagtttttt gttttttttg tttttttttt tgtcgggacg 50
<210> 460
   <211> 50
   <212> DNA
   <213> human
<400> 460
   tttaatattt tgagtttttt gttttttttg tttttttttt tgttgggatg 50
<210> 461
   <211> 3001
   <212> DNA
   <213> human
<400> 461
   cgcagagagt cacttgcttc tcctttaact ttgggagcca gggtcgcagt ctagggcagg 60
   aggatcgtcc tgggagccaa ccaacaaggc cgtccaggtc ctggacttcc cggaatagag 120
   taacggctat gggatgggaa agaaataggg ggatacacgt tccgcctccc ttctcccaaa 180
   agcaaaagcg tgcatggcac ttgctggggc ttagcggccg cggggcgcat cgccggccgc 240
   cgccgaaaac ctgctgcgtc cccgcaggct ctgcctcccg accccggcgg ggaaggcgcc 300
   ggtgcagtga gtgcagccgg cgcacccggg gcggcttgag agcggagtcc ccagactcaa 360
   gggggcggac gggcgccctc cccgggacac ccgcgccgcg ccactgctct cgggtcttcc 420
   cccgcacgtg gaggcgccgc ggacgctgca tcccgactgc ggccggaggg ctgcggctgc 480
   ccacattgag gcgccctaca tcctgccgga ccccctggcg ctcctgcaag ccccggcatc 540
   cacctactcc cggagccaaa ctcggcttcg gcagccggga cccgggcagg gcggccgggg 600
   tccgcggagc tggtggaaag ttagtcgcgg ggcctcaaag tcccctcccc agggccggaa 660
   cccccacccc cgcgtcccct ggtgcccccg gcgtccgaga ctccgcgtcc tcgcggtccc 720
   cactcagcca cctacctgct cccgcggccg gcccagggat cggccggcca gtcgcgcggt 780
   cctgtcctcc ggagcccgag cctggccaga gcgccaggca ggagcagggg gccgcgagca 840
   gccgggagcc gggcggcggg cagcgggcac cgggagcgac tgagcgagcg agcgagcggg 900
   cggctggagc cgcggcggct gctgccgagc cagcccccac ccgcttcgtc cctcggcccc 960
   tccctctcct tccctcccag ccttcctcct cccggctcgc agctccctcc ctccgctctc 1020
   ccgcacaccc ccctcatttt tttcctcttt ctctctgttt ggtcctttca gatcctgctg 1080
   ttacgcgcgc gctcgttcgt tctttttcct gtcttcagtc tctctcttca gcttgccttt 1140
   ggattctcct ttttctccca ttcatccttt tccagcatct cacctccctc ctccccactt 1200
   tcgttctttt ggtcattttt cttgctccct cttttctcta ttttctattt tctcctcctg 1260
   ctaaccttct cgcgcgtcta ctcccctttc tcttccctac tccccctttc cctcctgggg 1320
   gagtggagcg gcctgcgcgc ctcctcctct atgctctcga tgggagatcc cggggatggg 1380
   agtgggggtc tgggggttaa atggagccag gggcaccccc aggccgtgct cctaacaggc 1440
   cgaaggtcga gagggagggg tagggccgga gacagggact caagacaggg gctcgagaaa 1500
   cgtcccggca aggaaaaggg caagaaaagc aaagaactca aaatgttaag acccaagtcg 1560
   ggctgagccg tgcctggtga gatgaggaaa ctgagaccca aagagaagag ggcaatgaac 1620
   ttgtcttgcc gaggtctact ccaggcaagg tcgagacccc caccgctcct cgcagcctag 1680
   gactgggctc ctgcgctgat gcgggaagtg cgagggcgcc tgaggcccgt cagctcgttc 1740
   ccgtcccttt gcctgcctct gcgcccaagt cccgggggcc atggggagga agccttcctc 1800
   ctctgctaca gcacctgggg ccgcctggct gccccacgac actcctagtg aggccaagcc 1860
   agacaggggt aaagacctgg ctcttaagag tggtgcaggg aacccccttt cctccacctc 1920
   ctaccccttc ccaggaaatg tgggctgact cctgcttaca cgggatagga gtggcggcag 1980
   ggtactttgc tcgtggggtt tgccctcttt tgtccttgaa ctaaaactgg agcggaaagg 2040
   cccagggttg gaaaggtggt tatacacacc tggggagatg aatttttgcg aataaagtag 2100
   gcctaatgcc tgagactggg agctgtgtgt ccgtccgtgt gtctgagcac ctagcccacc 2160
   cgggccattc tgggtctgat ggagcaagtc cctcccctag aagcttttcc tggcacttcc 2220
   tcgtttcagc ccagtggagt cgggcgggag gagagacgtg gggtctccgt tcccatggag 2280
   tccacgctgc ggcccgacac caccaggatt ctcatagttc cacccctaat ttctggggcc 2340
   agaaatttct gtggtacaca accagaagca gaaacagaag tacaaccaga agcagaacct 2400
   ctgctcagcc cctgggtaaa aatctacttt ggggtgctag caaaactctg cacaaatatt 2460
   ctattcattc ctcgctttgg gatatcaaat taagtcaata aagcccggcc tgggcctctg 2520
   tggttcacac cacaaagcca gaagaagcat ctgctcaagg ctgagacttg ggtggggcac 2580
   gtggagtgct caccctcaga gtctgcaact gagggtgagc gcctccttaa attctcccct 2640
   cagagcctcc ctgcctcacc ctagtcccga ctgcaggcaa atctgagggc tgctgttcac 2700
   cagctgtctg gccttggtcc atggccgccc ccatgctggc attatttccc cacctgacca 2760
   tgaagaggct gaattcgatg ttctctaaga ttttctccag ctctgaggtg ccacagtttg 2820
   tgggtatcat agatgccaaa tttgtatgcc tgctccacca cttactaact gtgacactgg 2880
   gcagtcactt aacctgtttc cctatcagta aaatgggaac gatgatagta ctgtgagctg 2940
   tgtgaggatt aaaccacata atgcaccgaa gcacaattcc tgacacagag tagcacttga 3000
   c 3001

<210> 462
   <211> 3001
   <212> DNA
   <213> human
<400> 462
   cgtagagagt tatttgtttt ttttttaatt ttgggagtta gggtcgtagt ttagggtagg      60
   aggatcgttt tgggagttaa ttaataaggt cgtttaggtt ttggattttt cggaatagag     120
   taacggttat gggatgggaa agaaataggg ggatatacgt ttcgtttttt tttttttaaa    180
   agtaaaagcg tgtatggtat ttgttggggt ttagcggtcg cggggcgtat cgtcggtcgt     240
   cgtcgaaaat ttgttgcgtt ttcgtaggtt ttgtttttcg atttcggcgg ggaaggcgtc     300
   ggtgtagtga gtgtagtcgg cgtattcggg gcggtttgag agcggagttt ttagatttaa     360
   gggggcggac gggcgttttt ttcgggatat tcgcgtcgcg ttattgtttt cgggtttttt     420
   ttcgtacgtg gaggcgtcgc ggacgttgta tttcgattgc ggtcggaggg ttgcggttgt     480
   ttatattgag gcgttttata ttttgtcgga ttttttggcg tttttgtaag tttcggtatt     540
   tatttatttt cggagttaaa ttcggtttcg gtagtcggga ttcgggtagg gcggtcgggg     600
   ttcgcggagt tggtggaaag ttagtcgcgg ggttttaaag tttttttttt agggtcggaa     660
   tttttatttt cgcgtttttt ggtgttttcg gcgttcgaga tttcgcgttt tcgcggtttt     720
   tatttagtta tttatttgtt ttcgcggtcg gtttagggat cggtcggtta gtcgcgcggt     780
   tttgtttttc ggagttcgag tttggttaga gcgttaggta ggagtagggg gtcgcgagta     840
   gtcgggagtc gggcggcggg tagcgggtat cgggagcgat tgagcgagcg agcgagcggg     900
   cggttggagt cgcggcggtt gttgtcgagt tagtttttat tcgtttcgtt tttcggtttt     960
   tttttttttt ttttttttag tttttttttt ttcggttcgt agtttttttt tttcgttttt    1020
   tcgtatattt tttttatttt tttttttttt ttttttgttt ggttttttta gattttgttg    1080
   ttacgcgcgc gttcgttcgt tttttttttt gtttttagtt ttttttttta gtttgttttt     1140
   ggattttttt ttttttttta tttatttttt tttagtattt tatttttttt ttttttattt   1200
   tcgttttttt ggttattttt tttgtttttt ttttttttta ttttttattt tttttttttg   1260
   ttaatttttt cgcgcgttta tttttttttt ttttttttat tttttttttt ttttttgggg    1320
   gagtggagcg gtttgcgcgt tttttttttt atgttttcga tgggagattt cggggatggg    1380
   agtgggggtt tgggggttaa atggagttag gggtattttt aggtcgtgtt tttaataggt    1440
   cgaaggtcga gagggagggg tagggtcgga gatagggatt taagataggg gttcgagaaa    1500
   cgtttcggta aggaaaaggg taagaaaagt aaagaattta aaatgttaag atttaagtcg    1560
   ggttgagtcg tgtttggtga gatgaggaaa ttgagattta aagagaagag ggtaatgaat    1620
   ttgttttgtc gaggtttatt ttaggtaagg tcgagatttt tatcgttttt cgtagtttag    1680
   gattgggttt ttgcgttgat gcgggaagtg cgagggcgtt tgaggttcgt tagttcgttt    1740
   tcgttttttt gtttgttttt gcgtttaagt ttcgggggtt atggggagga agtttttttt    1800
   ttttgttata gtatttgggg tcgtttggtt gttttacgat atttttagtg aggttaagtt    1860
   agataggggt aaagatttgg tttttaagag tggtgtaggg aatttttttt tttttatttt    1920
   ttattttttt ttaggaaatg tgggttgatt tttgtttata cgggatagga gtggcggtag    1980
   ggtattttgt tcgtggggtt tgtttttttt tgtttttgaa ttaaaattgg agcggaaagg    2040
   tttagggttg gaaaggtggt tatatatatt tggggagatg aatttttgcg aataaagtag    2100
   gtttaatgtt tgagattggg agttgtgtgt tcgttcgtgt gtttgagtat ttagtttatt    2160
   cgggttattt tgggtttgat ggagtaagtt ttttttttag aagttttttt tggtattttt   2220
   tcgttttagt ttagtggagt cgggcgggag gagagacgtg gggttttcgt ttttatggag    2280
   tttacgttgc ggttcgatat tattaggatt tttatagttt tatttttaat ttttggggtt    2340
   agaaattttt gtggtatata attagaagta gaaatagaag tataattaga agtagaattt    2400
   ttgtttagtt tttgggtaaa aatttatttt ggggtgttag taaaattttg tataaatatt   2460
   ttatttattt ttcgttttgg gatattaaat taagttaata aagttcggtt tgggtttttg    2520
   tggtttatat tataaagtta gaagaagtat ttgtttaagg ttgagatttg ggtggggtac    2580
   gtggagtgtt tatttttaga gtttgtaatt gagggtgagc gtttttttaa attttttttt   2640
   tagagttttt ttgttttatt ttagtttcga ttgtaggtaa atttgagggt tgttgtttat    2700
   tagttgtttg gttttggttt atggtcgttt ttatgttggt attatttttt tatttgatta    2760
   tgaagaggtt gaattcgatg ttttttaaga ttttttttag ttttgaggtg ttatagtttg  2820
   tgggtattat agatgttaaa tttgtatgtt tgttttatta tttattaatt gtgatattgg    2880
   gtagttattt aatttgtttt tttattagta aaatgggaac gatgatagta ttgtgagttg    2940
   tgtgaggatt aaattatata atgtatcgaa gtataatttt tgatatagag tagtatttga    3000
   t                                                                    3001
<210> 463
   <211> 3001
   <212> DNA
   <213> human
<400> 463
   tgtagagagt tatttgtttt ttttttaatt ttgggagtta gggttgtagt ttagggtagg      60
   aggattgttt tgggagttaa ttaataaggt tgtttaggtt ttggattttt tggaatagag     120
   taatggttat gggatgggaa agaaataggg ggatatatgt tttgtttttt tttttttaaa     180
   agtaaaagtg tgtatggtat ttgttggggt ttagtggttg tggggtgtat tgttggttgt     240
   tgttgaaaat ttgttgtgtt tttgtaggtt ttgttttttg attttggtgg ggaaggtgtt     300
   ggtgtagtga gtgtagttgg tgtatttggg gtggtttgag agtggagttt ttagatttaa     360
   gggggtggat gggtgttttt tttgggatat ttgtgttgtg ttattgtttt tgggtttttt     420
   tttgtatgtg gaggtgttgt ggatgttgta ttttgattgt ggttggaggg ttgtggttgt     480
   ttatattgag gtgttttata ttttgttgga ttttttggtg tttttgtaag ttttggtatt     540
   tatttatttt tggagttaaa tttggttttg gtagttggga tttgggtagg gtggttgggg     600
   tttgtggagt tggtggaaag ttagttgtgg ggttttaaag tttttttttt agggttggaa     660
   tttttatttt tgtgtttttt ggtgtttttg gtgtttgaga ttttgtgttt ttgtggtttt     720
   tatttagtta tttatttgtt tttgtggttg gtttagggat tggttggtta gttgtgtggt     780
   tttgtttttt ggagtttgag tttggttaga gtgttaggta ggagtagggg gttgtgagta     840
   gttgggagtt gggtggtggg tagtgggtat tgggagtgat tgagtgagtg agtgagtggg     900
   tggttggagt tgtggtggtt gttgttgagt tagtttttat ttgttttgtt ttttggtttt     960
   tttttttttt ttttttttag tttttttttt tttggtttgt agtttttttt ttttgttttt    1020
   ttgtatattt tttttatttt tttttttttt ttttttgttt ggttttttta gattttgttg    1080
   ttatgtgtgt gtttgtttgt tttttttttt gtttttagtt ttttttttta gtttgttttt    1140
   ggattttttt ttttttttta tttatttttt tttagtattt tatttttttt ttttttattt   1200
   ttgttttttt ggttattttt tttgtttttt ttttttttta ttttttattt tttttttttg   1260
   ttaatttttt tgtgtgttta tttttttttt ttttttttat tttttttttt ttttttgggg    1320
   gagtggagtg gtttgtgtgt tttttttttt atgtttttga tgggagattt tggggatggg    1380
   agtgggggtt tgggggttaa atggagttag gggtattttt aggttgtgtt tttaataggt    1440
   tgaaggttga gagggagggg tagggttgga gatagggatt taagataggg gtttgagaaa    1500
   tgttttggta aggaaaaggg taagaaaagt aaagaattta aaatgttaag atttaagttg    1560
   ggttgagttg tgtttggtga gatgaggaaa ttgagattta aagagaagag ggtaatgaat    1620
   ttgttttgtt gaggtttatt ttaggtaagg ttgagatttt tattgttttt tgtagtttag    1680
   gattgggttt ttgtgttgat gtgggaagtg tgagggtgtt tgaggtttgt tagtttgttt    1740
   ttgttttttt gtttgttttt gtgtttaagt tttgggggtt atggggagga agtttttttt   1800
   ttttgttata gtatttgggg ttgtttggtt gttttatgat atttttagtg aggttaagtt    1860
   agataggggt aaagatttgg tttttaagag tggtgtaggg aatttttttt tttttatttt    1920
   ttattttttt ttaggaaatg tgggttgatt tttgtttata tgggatagga gtggtggtag    1980
   ggtattttgt ttgtggggtt tgtttttttt tgtttttgaa ttaaaattgg agtggaaagg    2040
   tttagggttg gaaaggtggt tatatatatt tggggagatg aatttttgtg aataaagtag    2100
   gtttaatgtt tgagattggg agttgtgtgt ttgtttgtgt gtttgagtat ttagtttatt    2160
   tgggttattt tgggtttgat ggagtaagtt ttttttttag aagttttttt tggtattttt    2220
   ttgttttagt ttagtggagt tgggtgggag gagagatgtg gggtttttgt ttttatggag    2280
   tttatgttgt ggtttgatat tattaggatt tttatagttt tatttttaat ttttggggtt    2340
   agaaattttt gtggtatata attagaagta gaaatagaag tataattaga agtagaattt    2400
   ttgtttagtt tttgggtaaa aatttatttt ggggtgttag taaaattttg tataaatatt   2460
   ttatttattt tttgttttgg gatattaaat taagttaata aagtttggtt tgggtttttg    2520
   tggtttatat tataaagtta gaagaagtat ttgtttaagg ttgagatttg ggtggggtat    2580
   gtggagtgtt tatttttaga gtttgtaatt gagggtgagt gtttttttaa attttttttt    2640
   tagagttttt ttgttttatt ttagttttga ttgtaggtaa atttgagggt tgttgtttat    2700
   tagttgtttg gttttggttt atggttgttt ttatgttggt attatttttt tatttgatta    2760
   tgaagaggtt gaatttgatg ttttttaaga ttttttttag ttttgaggtg ttatagtttg    2820
   tgggtattat agatgttaaa tttgtatgtt tgttttatta tttattaatt gtgatattgg    2880
   gtagttattt aatttgtttt tttattagta aaatgggaat gatgatagta ttgtgagttg    2940
   tgtgaggatt aaattatata atgtattgaa gtataatttt tgatatagag tagtatttga    3000
   t                                                                   3001
<210> 464
   <211> 3001
   <212> DNA
   <213> human
<400> 464
   gttaagtgtt attttgtgtt aggaattgtg tttcggtgta ttatgtggtt taatttttat 60
   atagtttata gtattattat cgtttttatt ttattgatag ggaaataggt taagtgattg 120
   tttagtgtta tagttagtaa gtggtggagt aggtatataa atttggtatt tatgatattt 180
   ataaattgtg gtattttaga gttggagaaa attttagaga atatcgaatt tagttttttt 240
   atggttaggt ggggaaataa tgttagtatg ggggcggtta tggattaagg ttagatagtt 300
   ggtgaatagt agtttttaga tttgtttgta gtcgggatta gggtgaggta gggaggtttt 360
   gaggggagaa tttaaggagg cgtttatttt tagttgtaga ttttgagggt gagtatttta 420
   cgtgttttat ttaagtttta gttttgagta gatgtttttt ttggttttgt ggtgtgaatt 480
   atagaggttt aggtcgggtt ttattgattt aatttgatat tttaaagcga ggaatgaata 540
   gaatatttgt gtagagtttt gttagtattt taaagtagat ttttatttag gggttgagta 600
   gaggttttgt ttttggttgt atttttgttt ttgtttttgg ttgtgtatta tagaaatttt 660
   tggttttaga aattaggggt ggaattatga gaattttggt ggtgtcgggt cgtagcgtgg 720
   attttatggg aacggagatt ttacgttttt tttttcgttc gattttattg ggttgaaacg 780
   aggaagtgtt aggaaaagtt tttaggggag ggatttgttt tattagattt agaatggttc 840
   gggtgggtta ggtgtttaga tatacggacg gatatatagt ttttagtttt aggtattagg 900
   tttattttat tcgtaaaaat ttattttttt aggtgtgtat aattattttt ttaattttgg 960
   gttttttcgt tttagtttta gtttaaggat aaaagagggt aaattttacg agtaaagtat 1020
   tttgtcgtta tttttatttc gtgtaagtag gagttagttt atattttttg ggaaggggta 1080
   ggaggtggag gaaagggggt tttttgtatt atttttaaga gttaggtttt tatttttgtt 1140
   tggtttggtt ttattaggag tgtcgtgggg tagttaggcg gttttaggtg ttgtagtaga 1200
   ggaggaaggt ttttttttta tggttttcgg gatttgggcg tagaggtagg taaagggacg 1260
   ggaacgagtt gacgggtttt aggcgttttc gtatttttcg tattagcgta ggagtttagt 1320
   tttaggttgc gaggagcggt gggggtttcg attttgtttg gagtagattt cggtaagata 1380
   agtttattgt tttttttttt ttgggtttta gtttttttat tttattaggt acggtttagt 1440
   tcgatttggg ttttaatatt ttgagttttt tgtttttttt gttttttttt ttgtcgggac 1500
   gtttttcgag tttttgtttt gagtttttgt tttcggtttt attttttttt ttcgattttc 1560
   ggtttgttag gagtacggtt tgggggtgtt tttggtttta tttaattttt agatttttat 1620
   ttttattttc gggatttttt atcgagagta tagaggagga ggcgcgtagg tcgttttatt 1680
   tttttaggag ggaaaggggg agtagggaag agaaagggga gtagacgcgc gagaaggtta 1740
   gtaggaggag aaaatagaaa atagagaaaa gagggagtaa gaaaaatgat taaaagaacg 1800
   aaagtgggga ggagggaggt gagatgttgg aaaaggatga atgggagaaa aaggagaatt 1860
   taaaggtaag ttgaagagag agattgaaga taggaaaaag aacgaacgag cgcgcgcgta 1920
   atagtaggat ttgaaaggat taaatagaga gaaagaggaa aaaaatgagg ggggtgtgcg 1980
   ggagagcgga gggagggagt tgcgagtcgg gaggaggaag gttgggaggg aaggagaggg 2040
   aggggtcgag ggacgaagcg ggtgggggtt ggttcggtag tagtcgtcgc ggttttagtc 2100
   gttcgttcgt tcgttcgttt agtcgttttc ggtgttcgtt gttcgtcgtt cggttttcgg 2160
   ttgttcgcgg ttttttgttt ttgtttggcg ttttggttag gttcgggttt cggaggatag 2220
   gatcgcgcga ttggtcggtc gatttttggg tcggtcgcgg gagtaggtag gtggttgagt 2280
   ggggatcgcg aggacgcgga gtttcggacg tcgggggtat taggggacgc gggggtgggg 2340
   gtttcggttt tggggagggg attttgaggt ttcgcgatta attttttatt agtttcgcgg 2400
   atttcggtcg ttttgttcgg gtttcggttg tcgaagtcga gtttggtttc gggagtaggt 2460
   ggatgtcggg gtttgtagga gcgttagggg gttcggtagg atgtagggcg ttttaatgtg 2520
   ggtagtcgta gtttttcggt cgtagtcggg atgtagcgtt cgcggcgttt ttacgtgcgg 2580
   gggaagattc gagagtagtg gcgcggcgcg ggtgtttcgg ggagggcgtt cgttcgtttt 2640
   tttgagtttg gggatttcgt ttttaagtcg tttcgggtgc gtcggttgta tttattgtat 2700
   cggcgttttt ttcgtcgggg tcgggaggta gagtttgcgg ggacgtagta ggttttcggc 2760
   ggcggtcggc gatgcgtttc gcggtcgtta agttttagta agtgttatgt acgtttttgt 2820
   ttttgggaga agggaggcgg aacgtgtatt tttttatttt ttttttattt tatagtcgtt 2880
   attttatttc gggaagttta ggatttggac ggttttgttg gttggttttt aggacgattt 2940
   ttttgtttta gattgcgatt ttggttttta aagttaaagg agaagtaagt gattttttgc 3000
   g 3001
<210> 465
   <211> 3001
   <212> DNA
   <213> human
<400> 465
   gttaagtgtt attttgtgtt aggaattgtg ttttggtgta ttatgtggtt taatttttat 60
   atagtttata gtattattat tgtttttatt ttattgatag ggaaataggt taagtgattg 120
   tttagtgtta tagttagtaa gtggtggagt aggtatataa atttggtatt tatgatattt 180
   ataaattgtg gtattttaga gttggagaaa attttagaga atattgaatt tagttttttt 240
   atggttaggt ggggaaataa tgttagtatg ggggtggtta tggattaagg ttagatagtt 300
   ggtgaatagt agtttttaga tttgtttgta gttgggatta gggtgaggta gggaggtttt 360
   gaggggagaa tttaaggagg tgtttatttt tagttgtaga ttttgagggt gagtatttta 420
   tgtgttttat ttaagtttta gttttgagta gatgtttttt ttggttttgt ggtgtgaatt 480
   atagaggttt aggttgggtt ttattgattt aatttgatat tttaaagtga ggaatgaata 540
   gaatatttgt gtagagtttt gttagtattt taaagtagat ttttatttag gggttgagta 600
   gaggttttgt ttttggttgt atttttgttt ttgtttttgg ttgtgtatta tagaaatttt 660
   tggttttaga aattaggggt ggaattatga gaattttggt ggtgttgggt tgtagtgtgg 720
   attttatggg aatggagatt ttatgttttt ttttttgttt gattttattg ggttgaaatg 780
   aggaagtgtt aggaaaagtt tttaggggag ggatttgttt tattagattt agaatggttt 840
   gggtgggtta ggtgtttaga tatatggatg gatatatagt ttttagtttt aggtattagg 900
   tttattttat ttgtaaaaat ttattttttt aggtgtgtat aattattttt ttaattttgg 960
   gtttttttgt tttagtttta gtttaaggat aaaagagggt aaattttatg agtaaagtat 1020
   tttgttgtta tttttatttt gtgtaagtag gagttagttt atattttttg ggaaggggta 1080
   ggaggtggag gaaagggggt tttttgtatt atttttaaga gttaggtttt tatttttgtt 1140
   tggtttggtt ttattaggag tgttgtgggg tagttaggtg gttttaggtg ttgtagtaga 1200
   ggaggaaggt ttttttttta tggtttttgg gatttgggtg tagaggtagg taaagggatg 1260
   ggaatgagtt gatgggtttt aggtgttttt gtattttttg tattagtgta ggagtttagt 1320
   tttaggttgt gaggagtggt gggggttttg attttgtttg gagtagattt tggtaagata 1380
   agtttattgt tttttttttt ttgggtttta gtttttttat tttattaggt atggtttagt 1440
   ttgatttggg ttttaatatt ttgagttttt tgtttttttt gttttttttt ttgttgggat 1500
   gttttttgag tttttgtttt gagtttttgt ttttggtttt attttttttt tttgattttt 1560
   ggtttgttag gagtatggtt tgggggtgtt tttggtttta tttaattttt agatttttat 1620
   ttttattttt gggatttttt attgagagta tagaggagga ggtgtgtagg ttgttttatt 1680
   tttttaggag ggaaaggggg agtagggaag agaaagggga gtagatgtgt gagaaggtta 1740
   gtaggaggag aaaatagaaa atagagaaaa gagggagtaa gaaaaatgat taaaagaatg 1800
   aaagtgggga ggagggaggt gagatgttgg aaaaggatga atgggagaaa aaggagaatt 1860
   taaaggtaag ttgaagagag agattgaaga taggaaaaag aatgaatgag tgtgtgtgta 1920
   atagtaggat ttgaaaggat taaatagaga gaaagaggaa aaaaatgagg ggggtgtgtg 1980
   ggagagtgga gggagggagt tgtgagttgg gaggaggaag gttgggaggg aaggagaggg 2040
   aggggttgag ggatgaagtg ggtgggggtt ggtttggtag tagttgttgt ggttttagtt 2100
   gtttgtttgt ttgtttgttt agttgttttt ggtgtttgtt gtttgttgtt tggtttttgg 2160
   ttgtttgtgg ttttttgttt ttgtttggtg ttttggttag gtttgggttt tggaggatag 2220
   gattgtgtga ttggttggtt gatttttggg ttggttgtgg gagtaggtag gtggttgagt 2280
   ggggattgtg aggatgtgga gttttggatg ttgggggtat taggggatgt gggggtgggg 2340
   gttttggttt tggggagggg attttgaggt tttgtgatta attttttatt agttttgtgg 2400
   attttggttg ttttgtttgg gttttggttg ttgaagttga gtttggtttt gggagtaggt 2460
   ggatgttggg gtttgtagga gtgttagggg gtttggtagg atgtagggtg ttttaatgtg 2520
   ggtagttgta gttttttggt tgtagttggg atgtagtgtt tgtggtgttt ttatgtgtgg 2580
   gggaagattt gagagtagtg gtgtggtgtg ggtgttttgg ggagggtgtt tgtttgtttt 2640
   tttgagtttg gggattttgt ttttaagttg ttttgggtgt gttggttgta tttattgtat 2700
   tggtgttttt tttgttgggg ttgggaggta gagtttgtgg ggatgtagta ggtttttggt 2760
   ggtggttggt gatgtgtttt gtggttgtta agttttagta agtgttatgt atgtttttgt 2820
   ttttgggaga agggaggtgg aatgtgtatt tttttatttt ttttttattt tatagttgtt 2880
   attttatttt gggaagttta ggatttggat ggttttgttg gttggttttt aggatgattt 2940
   ttttgtttta gattgtgatt ttggttttta aagttaaagg agaagtaagt gattttttgt 3000
   g 3001
<210> 466
   <211> 50
   <212> DNA
   <213> human
<400> 466
   cggaaaccgc cccgtgaatt cccaccgagt cctcagatcc tgccgagcca 50
<210> 467
   <211> 50
   <212> DNA
   <213> human
<400> 467
   cggaaatcgt ttcgtgaatt tttatcgagt ttttagattt tgtcgagtta 50
<210> 468
   <211> 50
   <212> DNA
   <213> human
<400> 468
   tggaaattgt tttgtgaatt tttattgagt ttttagattt tgttgagtta 50
<210> 469
   <211> 50
   <212> DNA
   <213> human
<400> 469
   tggttcggta ggatttgagg attcggtggg aatttacggg gcggttttcg 50
<210> 470
   <211> 50
   <212> DNA
   <213> human
<400> 470
   tggtttggta ggatttgagg atttggtggg aatttatggg gtggtttttg 50
<210> 471
   <211> 3001
   <212> DNA
   <213> human
<400> 471
<210> 472
   <211> 3001
   <212> DNA
   <213> human
<400> 472
   tatatgcggt tgtttagtag tttttgatag ttaggataaa ggttttcgta gatagatggt      60
   agtatggagg atagatttgt tcgaggagtt tttaaagggt tttttggttt tagaatttga     120
   tagtagggta ggggtagggt taggtgagag ggaggtggag gttttgttgt gggagtttga     180
   ggtttaagtg gtagagattt ttttttttgt tgttttttaa gaggttaaag gaagaatttt     240
   ttttgttttt taagggtgga aggtagttag agttaggaga ggggtttttt gagatttttg    300
   ttaggtaggt attttgggaa gaagagaaga ggagagggtt ttgtttaagt aagttgggaa     360
   ggttttaggg tgaaagtatt attatggtgt tttacgggat aatttttttt ttatgggaag     420
   gggtggtaag gtaagtttag gattttggat ttttttttta atttgattta gtttttagtt      480
   ataggtattt ttattttttg gtagtaaagg ggagtaggtt tatatggatt attttattac     540
   ggcgtttatt tttttttttt tttatcgttg tggagatttt tttttattaa gagttagagt     600
   attacgattt tagattttgt tgaaggtgat taggtgattt taggtagttt atttagtttt     660
   ttcgagtttt agttttttta tttgtgtaat ggaggtttta gcgtttgttt tgaaatattg     720
   ttgtgaaggg ggaaattgtt cgtgttattt gaggattaat tattattttg gaattttttt     780
   taaggttatt aaggaatacg tattttttat tagatttttt agttgttggg ggtttttaga       840
   gtgaggttga ggttatggag aagggaatgg ggggttttta tggttagttg gatttgatta     900
   ttgttttttt atttagttat agtttttagg gttttgtgtt agtttagaag tttatttagg      960
   gatatttttg gttaatgttt tgttttattt gcgaggtaat tttttttagt gttttaatta    1020
   tagcgttttt ttttaaatat ttttaggaag gagggattat tatttgtgta gggggggtta   1080
   ggagtttttt gagagtttta tatggggagg aagtggtatt attttatttt tattgttttt    1140
   tttttttatt tttatttggt tagttttttt tagtgttttt tttgttttag tgtttttttt      1200
   tttagtgttt tttttgtttg tcgttttagg ggtttgattt ttgatttggt agggagggga    1260
   atggagttcg gggatagagg ttaatgagtt ttgagaatag tggatgtttt ttttgatttt    1320
   attatcgggt ttgtgttatg attttaataa ggatttcgag ggtatgtata ggttggggtt    1380
   tgttatggta gggagggggt cgtttatttt cggagattgt gtgtgtgtgt aggtgtgcgt    1440
   atataaggtt ttttttttaa gtttaggcgt ttcgtagttg gtattaaatt ttgtagggta     1500
   cggaaatcgt ttcgtgaatt tttatcgagt ttttagattt tgtcgagtta ggggaggcga    1560
   cgagggggcg tttaatttcg tgacgcgtgt gtttcgtttt gtcgcggggg ttagggatag    1620
   gggaatgcgt tttgtttttg gggatgtaag gatattatcg tttttttcgt ttagggtttt    1680
   tgatttgagg tttttatttt ttttttttga ggataggagc gttatttttt ggggggcgtt   1740
   agggtttggg ttttttttta gggatggagg aaacgcgttt attttttata gttttttttt      1800
   taaattttag gcgtttttat ttcgaggtgg gcggaaaatt ggatttttta tttatttagg   1860
   ggggtgggat taaagttttt tggtcgcggt ttggattaag tttaataggt tgggggttta    1920
   gtcggtttag tttaggtttt tttttatttt tttagttgtt gttaagatcg ggttgcgcga    1980
   gtttagttgc gtttattttt cgtttttgga gattcgggtt ttttacgcgt tcgtttttat    2040
   ttttcgtttt cgttcgttcg cgcggggttt tcgttgcgag tcgacgggag tttggatatt    2100
   tttgtttttt tttttttttt agggtcgtag ttttggcggg gcgtatttat ttaagggttt    2160
   gggtcggggg cgttttgggt tcggtatttt ggtgttcgcg gtcgtcgttg tagttttgtt    2220
   tagatgcggt cggcgttcgt cgtcgtcgta gttcggggag ggggacgtta tatgatcgcg    2280
   gagcgttttt ttttcggcgt cgttttttat ttattgcgga gtttaagtcg cgggcgcggg      2340
   agcggcgggg aggttttcgt tttgtcgttt cgtttcgtcg taggttttgg aggagggttt      2400
   tcggtgtttt tattttatag gtggggaaat tgaggattag agaggggtag ggattggttt    2460
   aaggttatat agttagatag tggtagaatt tgggcgatag tttagttgtt taggtaatag    2520
   tttatgtttt aaggggattg ggataatttt tggattaagt ggagacgtcg ggataggtgg    2580
   ggtttacggt ttttatgggt ttcgtttgtt tgtaggcgcg ggaggaaggc gtataggttt    2640
   cgttgggcga gaaagttgtt gtgttttggt tgacgttttt tttattcggg cgttcggcgc    2700
   gttttttggt agaggttacg cggttgattt tcgttagttt attcgcggtt tttggatttt    2760
   gtttttggat tgggtagttc gttttcgttt tagtttaatt cgggtttagg gcgggtgtgg    2820
   ggaggggagt tataggttac ggagaagtgg aagggaggta ttcggaagag agtgggatta    2880
   gaataagagt gaaaatttag gtagggaggg ggcggtagag attatttcgg tttgattttt    2940
   aaggtttagg gagagttaga gatttgttta aggtttatag taagtgagcg gtttgtttat    3000
   t                                                                    3001
<210> 473
   <211> 3001
   <212> DNA
   <213> human
<400> 473
   tatatgtggt tgtttagtag tttttgatag ttaggataaa ggtttttgta gatagatggt 60
   agtatggagg atagatttgt ttgaggagtt tttaaagggt tttttggttt tagaatttga 120
   tagtagggta ggggtagggt taggtgagag ggaggtggag gttttgttgt gggagtttga 180
   ggtttaagtg gtagagattt ttttttttgt tgttttttaa gaggttaaag gaagaatttt 240
   ttttgttttt taagggtgga aggtagttag agttaggaga ggggtttttt gagatttttg 300
   ttaggtaggt attttgggaa gaagagaaga ggagagggtt ttgtttaagt aagttgggaa 360
   ggttttaggg tgaaagtatt attatggtgt tttatgggat aatttttttt ttatgggaag 420
   gggtggtaag gtaagtttag gattttggat ttttttttta atttgattta gtttttagtt 480
   ataggtattt ttattttttg gtagtaaagg ggagtaggtt tatatggatt attttattat 540
   ggtgtttatt tttttttttt tttattgttg tggagatttt tttttattaa gagttagagt 600
   attatgattt tagattttgt tgaaggtgat taggtgattt taggtagttt atttagtttt 660
   tttgagtttt agttttttta tttgtgtaat ggaggtttta gtgtttgttt tgaaatattg 720
   ttgtgaaggg ggaaattgtt tgtgttattt gaggattaat tattattttg gaattttttt 780
   taaggttatt aaggaatatg tattttttat tagatttttt agttgttggg ggtttttaga 840
   gtgaggttga ggttatggag aagggaatgg ggggttttta tggttagttg gatttgatta 900
   ttgttttttt atttagttat agtttttagg gttttgtgtt agtttagaag tttatttagg 960
   gatatttttg gttaatgttt tgttttattt gtgaggtaat tttttttagt gttttaatta 1020
   tagtgttttt ttttaaatat ttttaggaag gagggattat tatttgtgta gggggggtta 1080
   ggagtttttt gagagtttta tatggggagg aagtggtatt attttatttt tattgttttt 1140
   tttttttatt tttatttggt tagttttttt tagtgttttt tttgttttag tgtttttttt 1200
   tttagtgttt tttttgtttg ttgttttagg ggtttgattt ttgatttggt agggagggga 1260
   atggagtttg gggatagagg ttaatgagtt ttgagaatag tggatgtttt ttttgatttt 1320
   attattgggt ttgtgttatg attttaataa ggattttgag ggtatgtata ggttggggtt 1380
   tgttatggta gggagggggt tgtttatttt tggagattgt gtgtgtgtgt aggtgtgtgt 1440
   atataaggtt ttttttttaa gtttaggtgt tttgtagttg gtattaaatt ttgtagggta 1500
   tggaaattgt tttgtgaatt tttattgagt ttttagattt tgttgagtta ggggaggtga 1560
   tgagggggtg tttaattttg tgatgtgtgt gttttgtttt gttgtggggg ttagggatag 1620
   gggaatgtgt tttgtttttg gggatgtaag gatattattg ttttttttgt ttagggtttt 1680
   tgatttgagg tttttatttt ttttttttga ggataggagt gttatttttt ggggggtgtt 1740
   agggtttggg ttttttttta gggatggagg aaatgtgttt attttttata gttttttttt 1800
   taaattttag gtgtttttat tttgaggtgg gtggaaaatt ggatttttta tttatttagg 1860
   ggggtgggat taaagttttt tggttgtggt ttggattaag tttaataggt tgggggttta 1920
   gttggtttag tttaggtttt tttttatttt tttagttgtt gttaagattg ggttgtgtga 1980
   gtttagttgt gtttattttt tgtttttgga gatttgggtt ttttatgtgt ttgtttttat 2040
   tttttgtttt tgtttgtttg tgtggggttt ttgttgtgag ttgatgggag tttggatatt 2100
   tttgtttttt tttttttttt agggttgtag ttttggtggg gtgtatttat ttaagggttt 2160
   gggttggggg tgttttgggt ttggtatttt ggtgtttgtg gttgttgttg tagttttgtt 2220
   tagatgtggt tggtgtttgt tgttgttgta gtttggggag ggggatgtta tatgattgtg 2280
   gagtgttttt tttttggtgt tgttttttat ttattgtgga gtttaagttg tgggtgtggg 2340
   agtggtgggg aggtttttgt tttgttgttt tgttttgttg taggttttgg aggagggttt 2400
   ttggtgtttt tattttatag gtggggaaat tgaggattag agaggggtag ggattggttt 2460
   aaggttatat agttagatag tggtagaatt tgggtgatag tttagttgtt taggtaatag 2520
   tttatgtttt aaggggattg ggataatttt tggattaagt ggagatgttg ggataggtgg 2580
   ggtttatggt ttttatgggt tttgtttgtt tgtaggtgtg ggaggaaggt gtataggttt 2640
   tgttgggtga gaaagttgtt gtgttttggt tgatgttttt tttatttggg tgtttggtgt 2700
   gttttttggt agaggttatg tggttgattt ttgttagttt atttgtggtt tttggatttt 2760
   gtttttggat tgggtagttt gtttttgttt tagtttaatt tgggtttagg gtgggtgtgg 2820
   ggaggggagt tataggttat ggagaagtgg aagggaggta tttggaagag agtgggatta 2880
   gaataagagt gaaaatttag gtagggaggg ggtggtagag attattttgg tttgattttt 2940
   aaggtttagg gagagttaga gatttgttta aggtttatag taagtgagtg gtttgtttat 3000
   t 3001
<210> 474
   <211> 3001
   <212> DNA
   <213> human
<400> 474
   agtgggtagg tcgtttattt gttgtggatt ttgggtaagt ttttaatttt ttttgggttt      60
   tggaagttag gtcgaggtga tttttgtcgt tttttttttg tttgggtttt tatttttgtt     120
   ttgattttat tttttttcgg gtgttttttt tttatttttt cgtagtttgt ggtttttttt     180
   tttatattcg ttttgggttc gggttaggtt ggggcggggg cgagttgttt agtttagaga     240
   taaagtttaa gagtcgcgaa tgggttggcg agggttaatc gcgtggtttt tgttaggaag     300
   cgcgtcgagc gttcgggtga ggagggcgtt agttagggta tagtagtttt ttcgtttagc     360
   gaagtttgtg cgtttttttt tcgcgtttgt agataagcgg agtttatggg ggtcgtgggt     420
   tttatttatt tcgacgtttt tatttgattt agaagttgtt ttagtttttt tggagtatga     480
   gttgttgttt ggatagttgg gttgtcgttt aggttttgtt attgtttagt tgtgtaattt     540
   tgagttagtt tttgtttttt tttggttttt agttttttta tttgtaaaat gggggtatcg    600
   agaatttttt tttaggattt gcggcggggc ggggcggtag ggcgggggtt ttttcgtcgt     660
   tttcgcgttc gcggtttggg tttcgtagtg agtggggagc ggcgtcgggg aaggagcgtt     720
   tcgcggttat gtgacgtttt ttttttcgaa ttgcggcggc ggcgagcgtc ggtcgtattt     780
   gagtagagtt gtagcggcgg tcgcgggtat tagagtgtcg agtttaggac gttttcggtt     840
   taggtttttg ggtgagtgcg tttcgttagg gttgcggttt tggaaagggg aaggggatag     900
   aggtgtttag attttcgtcg gttcgtagcg aagatttcgc gcgggcgggc ggggacggag     960
   ggtgggggcg aacgcgtggg ggattcgggt ttttaggggc gaggggtggg cgtagttggg    1020
   ttcgcgtagt tcggttttga tagtagttgg agaagtgggg ggaggtttgg attgagtcgg    1080
   ttgaattttt agtttgttgg atttggttta ggtcgcggtt agggggtttt ggttttattt    1140
   ttttgggtgg gtggggagtt tagtttttcg tttatttcga ggtgggggcg tttggggttt    1200
   agaggaggga ttgtgggaag tgggcgcgtt ttttttattt ttggggaggg gtttaagttt    1260
   tagcgttttt tagagggtgg cgtttttgtt tttagaggag ggaagtgggg gttttagatt    1320
   aaggattttg agcggggagg gcggtgatgt ttttgtattt ttagagatag gacgtatttt    1380
   tttgtttttg attttcgcgg tagagcgggg tatacgcgtt acgaagttgg gcgttttttc   1440
   gtcgtttttt ttggttcggt aggatttgag gattcggtgg gaatttacgg ggcggttttc    1500
   gtgttttgta gggtttggtg ttagttgcgg ggcgtttggg tttggggaag aaattttgtg    1560
   tacgtatatt tgtatatata tatagttttc ggaggtgagc ggtttttttt ttattataat    1620
   agattttagt ttgtgtatgt tttcggagtt tttgttgggg ttatagtata ggttcggtgg    1680
   tggggttagg aagaatattt attgttttta gggtttattg gtttttgttt tcgggtttta    1740
   tttttttttt tgttaagtta gaggttaggt ttttggggcg gtaggtagga aggatattga    1800
   gggaaggggt attgaggtag gaggggtatt gagggaagtt ggttaggtgg aagtaggaga    1860
   gaaaggtaat gggggtgaga tggtattatt ttttttttat atgaggtttt taggaggttt     1920
   ttggtttttt ttgtataggt agtggttttt tttttttgag ggtgtttggg ggaaaacgtt     1980
   atggttgggg tattggggaa ggttgtttcg tagatgaaat agaatattgg ttaaaggtgt    2040
   ttttgaatgg gtttttggat tggtataggg ttttgagggt tgtggttgag tggggaggta    2100
   gtgattaggt ttagttggtt atgggggttt tttatttttt tttttatgat tttagtttta     2160
   ttttgagagt ttttagtagt tggaaggttt ggtaaggggt gcgtgttttt tgatggtttt    2220
   gggaagggtt ttagggtaat gattaatttt taggtggtac gagtaatttt tttttttata   2280
   gtaatatttt agggtaggcg ttgaagtttt tattgtatag gtgaggaaat tgaggttcgg    2340
   agaggttaaa tgaattgttt gaggttattt agttattttt agtaaggttt agaatcgtgg    2400
   tattttgatt tttgatggga gaagattttt atagcggtgg agaggaggaa gggtgaacgt    2460
   cgtggtggag tggtttatgt ggatttgttt ttttttgttg ttaggaggtg agggtgtttg    2520
   tggttggaag ttgggttagg ttgaggagag ggtttagagt tttaggtttg ttttgttatt    2580
   tttttttatg gagggagggt tgtttcgtgg agtattataa tggtgttttt attttgggat   2640
   ttttttagtt tgtttgggta aagttttttt tttttttttt ttttttaagg tgtttgtttg    2700
   gtaggggttt taggaggttt tttttttggt tttaattatt ttttattttt aggagataag   2760
   aaggattttt tttttggttt tttggagggt agtagggaga gaggtttttg ttatttaggt    2820
   tttaggtttt tatagtaggg tttttatttt ttttttattt ggttttgttt ttgttttgtt    2880
   gttaaatttt ggggttaaga ggttttttga ggatttttcg ggtagatttg ttttttatgt  2940
   tgttatttgt ttacggaggt ttttgttttg gttgttagag gttgttgagt agtcgtatgt    3000
   g                                                                    3001
<210> 475
   <211> 3001
   <212> DNA
   <213> human
<400> 475
   agtgggtagg ttgtttattt gttgtggatt ttgggtaagt ttttaatttt ttttgggttt      60
   tggaagttag gttgaggtga tttttgttgt tttttttttg tttgggtttt tatttttgtt      120
   ttgattttat ttttttttgg gtgttttttt tttatttttt tgtagtttgt ggtttttttt      180
   tttatatttg ttttgggttt gggttaggtt ggggtggggg tgagttgttt agtttagaga      240
   taaagtttaa gagttgtgaa tgggttggtg agggttaatt gtgtggtttt tgttaggaag      300
   tgtgttgagt gtttgggtga ggagggtgtt agttagggta tagtagtttt tttgtttagt      360
   gaagtttgtg tgtttttttt ttgtgtttgt agataagtgg agtttatggg ggttgtgggt      420
   tttatttatt ttgatgtttt tatttgattt agaagttgtt ttagtttttt tggagtatga      480
   gttgttgttt ggatagttgg gttgttgttt aggttttgtt attgtttagt tgtgtaattt      540
   tgagttagtt tttgtttttt tttggttttt agttttttta tttgtaaaat gggggtattg      600
   agaatttttt tttaggattt gtggtggggt ggggtggtag ggtgggggtt tttttgttgt      660
   ttttgtgttt gtggtttggg ttttgtagtg agtggggagt ggtgttgggg aaggagtgtt      720
   ttgtggttat gtgatgtttt tttttttgaa ttgtggtggt ggtgagtgtt ggttgtattt      780
   gagtagagtt gtagtggtgg ttgtgggtat tagagtgttg agtttaggat gtttttggtt      840
   taggtttttg ggtgagtgtg ttttgttagg gttgtggttt tggaaagggg aaggggatag      900
   aggtgtttag atttttgttg gtttgtagtg aagattttgt gtgggtgggt ggggatggag      960
   ggtgggggtg aatgtgtggg ggatttgggt ttttaggggt gaggggtggg tgtagttggg      1020
   tttgtgtagt ttggttttga tagtagttgg agaagtgggg ggaggtttgg attgagttgg      1080
   ttgaattttt agtttgttgg atttggttta ggttgtggtt agggggtttt ggttttattt      1140
   ttttgggtgg gtggggagtt tagttttttg tttattttga ggtgggggtg tttggggttt      1200
   agaggaggga ttgtgggaag tgggtgtgtt ttttttattt ttggggaggg gtttaagttt      1260
   tagtgttttt tagagggtgg tgtttttgtt tttagaggag ggaagtgggg gttttagatt      1320
   aaggattttg agtggggagg gtggtgatgt ttttgtattt ttagagatag gatgtatttt      1380
   tttgtttttg atttttgtgg tagagtgggg tatatgtgtt atgaagttgg gtgttttttt      1440
   gttgtttttt ttggtttggt aggatttgag gatttggtgg gaatttatgg ggtggttttt      1500
   gtgttttgta gggtttggtg ttagttgtgg ggtgtttggg tttggggaag aaattttgtg      1560
   tatgtatatt tgtatatata tatagttttt ggaggtgagt ggtttttttt ttattataat      1620
   agattttagt ttgtgtatgt ttttggagtt tttgttgggg ttatagtata ggtttggtgg      1680
   tggggttagg aagaatattt attgttttta gggtttattg gtttttgttt ttgggtttta      1740
   tttttttttt tgttaagtta gaggttaggt ttttggggtg gtaggtagga aggatattga      1800
   gggaaggggt attgaggtag gaggggtatt gagggaagtt ggttaggtgg aagtaggaga      1860
   gaaaggtaat gggggtgaga tggtattatt ttttttttat atgaggtttt taggaggttt      1920
   ttggtttttt ttgtataggt agtggttttt tttttttgag ggtgtttggg ggaaaatgtt      1980
   atggttgggg tattggggaa ggttgttttg tagatgaaat agaatattgg ttaaaggtgt      2040
   ttttgaatgg gtttttggat tggtataggg ttttgagggt tgtggttgag tggggaggta      2100
   gtgattaggt ttagttggtt atgggggttt tttatttttt tttttatgat tttagtttta      2160
   ttttgagagt ttttagtagt tggaaggttt ggtaaggggt gtgtgttttt tgatggtttt      2220
   gggaagggtt ttagggtaat gattaatttt taggtggtat gagtaatttt tttttttata      2280
   gtaatatttt agggtaggtg ttgaagtttt tattgtatag gtgaggaaat tgaggtttgg      2340
   agaggttaaa tgaattgttt gaggttattt agttattttt agtaaggttt agaattgtgg      2400
   tattttgatt tttgatggga gaagattttt atagtggtgg agaggaggaa gggtgaatgt      2460
   tgtggtggag tggtttatgt ggatttgttt ttttttgttg ttaggaggtg agggtgtttg      2520
   tggttggaag ttgggttagg ttgaggagag ggtttagagt tttaggtttg ttttgttatt      2580
   tttttttatg gagggagggt tgttttgtgg agtattataa tggtgttttt attttgggat      2640
   ttttttagtt tgtttgggta aagttttttt tttttttttt ttttttaagg tgtttgtttg      2700
   gtaggggttt taggaggttt tttttttggt tttaattatt ttttattttt aggagataag      2760
   aaggattttt tttttggttt tttggagggt agtagggaga gaggtttttg ttatttaggt      2820
   tttaggtttt tatagtaggg tttttatttt ttttttattt ggttttgttt ttgttttgtt      2880
   gttaaatttt ggggttaaga ggttttttga ggattttttg ggtagatttg ttttttatgt      2940
   tgttatttgt ttatggaggt ttttgttttg gttgttagag gttgttgagt agttgtatgt      3000
   g                                                                     3001
<210> 476
   <211> 50
   <212> DNA
   <213> human
<400> 476
   acctcgggat ctatgaaaac tacattacct agaatgctct gcgttgaacg 50
<210> 477
   <211> 50
   <212> DNA
   <213> human
<400> 477
   atttcgggat ttatgaaaat tatattattt agaatgtttt gcgttgaacg 50
<210> 478
   <211> 50
   <212> DNA
   <213> human
<400> 478
   attttgggat ttatgaaaat tatattattt agaatgtttt gtgttgaatg 50
<210> 479
   <211> 50
   <212> DNA
   <213> human
<400> 479
   cgtttaacgt agagtatttt aggtaatgta gtttttatag atttcgaggt 50
<210> 480
   <211> 50
   <212> DNA
   <213> human
<400> 480
   tgtttaatgt agagtatttt aggtaatgta gtttttatag attttgaggt 50
<210> 481
   <211> 3001
   <212> DNA
   <213> human
<400> 481
   aatacaaaaa ttagctgggt gtcatggcgt gcgcctgtag tcccagctac ttgggaggct      60
   gaggcaagat aatcgcttga acccaggagg tggagaggtt gcagtgaaac aagactgcgt     120
   cattgcactc cagcctgggc aataagagtg aaactccatc tcaaaaaaaa agaaaaaaaa     180
   aaaagaagag atttctcacc tatataccct gagtgtctct gcagccagca aagcagatga     240
   aggcctgctg ggttggagcc ctaggccctc caatgctgaa atgccctgtg aagtctccat     300
   ccatccaggc actctagtaa cctgcttctt ctgcctggaa tttattctaa aaatacccac     360
   atggctcatt tctcacttcc ttcaagtgca tttcaccggc tttctgatta agaagtccca     420
   cctctcccct ttgtctctta tcaaagtagc atttctttca ttgttttttg agattgatgt     480
   tagacttcta gaaggaatat atctcagata tcaagtaaga attatgaaat ggataaaagc     540
   gttgctcagt gaattggaca cagttattga ctggacacag taattggaca caataattga     600
   ctatgcaggc aaaagatgtt ttctgttgga atcagacaga attctacctt tgtactactg     660
   tttcaaggtg atctaacatt tctcttctat cttctagaag cactatcatg ccactatctg     720
   aagtagtttt tcacctgtcc ctgaccaaaa gtgcacagac tgacttcaat gcttcggagt     780
   gcatacattt gaaatgtaat atgatagagg ctgggtacgg tggcttacgc ctgtaatccc     840
   agcactttga gaggccgagg cgggcagatc acctggggcc aggaatttgg gaccaccata     900
   gtgaaaccct gtctctactg aaaatacaaa aaattagcct ggcgtggtgg cgcgcgccta     960
   tagtcccagc tacttggaag gttgaggcac gagaatcgat taacccggga ggcggaggtt    1020
   gcagtgatcc cagatggcgc cgctgcactc cagcctgggg gacagaggga aactttgtct    1080
   taaaaaaaaa aaaaaaggaa taaacttata tggtaatgtt tattagcttc cctactagtg    1140
   gatatatttc gtgtctgaat ggctctatgg aatgatgtac atatttggat tggcagcctt    1200
   agggagcgtc tgataataat tttagcctct gggagcctgc tgaactttat gaagtttgaa    1260
   ataatattcc ccaaacactt gggtgatcag ttgcagatgg cacctctaat atgctgtctg    1320
   attacgaccc atttaagaaa gggcgaagtc cctccactgc cctaggactt ggataagcca    1380
   tttctcatcg cccagcgccc tggagcttcg cttttactcc aagaggttgg tgcaaagggt    1440
   ccccggcacc cacctcggga tctatgaaaa ctacattacc tagaatgctc tgcgttgaac    1500
   gccacgctac taagccagta agagctcaga aaaccgactt tccttgagag tcacaaaaag    1560
   aaaggacggg acttttgggg gggcctcttc gtggcggcca ttttagcttc tctgaggtgt    1620
   gttcaccgga tcagagatag cagagcgccg agttggggcc acgaaggcgt gaggggagtc    1680
   gtcgtccctc ctgcacgaaa gcgtctaagc cttggcgacg ccgccctggg ggacccacgt    1740
   caggcctggg atagggaccg ctgtccccgg gtccctacca atgtcgcccg tcgctcccgg    1800
   cccagctcta cccgcagagt ctgatggcag cggccactct gaggacgcca actcaggtga    1860
   gtgcggcgtc ttcccgtcct cacacacctt cccccaccca cgttctaaag ccatcagtga    1920
   ggggcgcctg ctcgagtccc cgctgcccag ggtcggggac actgaggcgt tcgtgggtgg    1980
   ggcccttttt ttgacactgc gtgtgacgag gtgtgggaga gcgtgacagg cggaggaacc    2040
   ggcgcgtgca aaggttgagg cgcgactgag ccaggagaat tcggaaagct gttttctgca    2100
   gggaacgaag aatatgaggc ggagttactc ctagggttgt actgtcattg tgaggatgct    2160
   agaagccacg gagagttctg aacaaaggag ggacgccatt tatgttagtt tttttttctt    2220
   tgagacggag tttcgctctt atcgcccagg ctggagtgca atgacgcgat ctcgggtcac    2280
   tgcaacctcc gactcccggg ttcaagcgat tctcctgcct cagcctcccc agcagctggg    2340
   attacaagcc tgcagctggg attacaacgt ccggctaatt ttttgtattt ttagtagaga    2400
   cgggatttca ccattttggc cagactggtc tggaactcct gacaagtgat cctcctgcct    2460
   cggcctccca aagtgctggg attacaggcg tgagccaccg tgcccgccct atgttagggt    2520
   ttaaaaactt ttccaaagac tgttcacagg aagagcagcg atggggcgat gaggacattg    2580
   agatagtggg aggctacatc tttttttcat tgccctacag ttggcaagag gtgacagcac    2640
   tgagaccaga aagtaaaaca gccagcctga gttcagggcc aggcagggct acagggaggc    2700
   ttgagctcat ctagcctcac cagggatgca ttcctctttc cctggttctc ccagttgtag    2760
   aagggcgtat ggaactcaca tagggaagtg gagagtgttc ggtccctcag tagcctctac    2820
   ccccatgttc ttgggattgt ggtttcctag tattcttaag tgctgttacc ccaatcctgg    2880
   gtctggtgcc ctggagaaat tccaagccca gggctcttaa gtccatgcca gaggttacag    2940
   gggttatact gaggtgttcc catttttgtc agccagtgga aacttgtgaa aggttatctc    3000
   t                                                                    3001

<210> 482
   <211> 3001
   <212> DNA
   <213> human
<400> 482
   aatataaaaa ttagttgggt gttatggcgt gcgtttgtag ttttagttat ttgggaggtt      60
   gaggtaagat aatcgtttga atttaggagg tggagaggtt gtagtgaaat aagattgcgt     120
   tattgtattt tagtttgggt aataagagtg aaattttatt ttaaaaaaaa agaaaaaaaa     180
   aaaagaagag atttttattt tatatatttt gagtgttttt gtagttagta aagtagatga     240
   aggtttgttg ggttggagtt ttaggttttt taatgttgaa atgttttgtg aagtttttat     300
   ttatttaggt attttagtaa tttgtttttt ttgtttggaa tttattttaa aaatatttat     360
   atggtttatt ttttattttt tttaagtgta ttttatcggt tttttgatta agaagtttta     420
   tttttttttt ttgtttttta ttaaagtagt atttttttta ttgttttttg agattgatgt    480
   tagattttta gaaggaatat attttagata ttaagtaaga attatgaaat ggataaaagc     540
   gttgtttagt gaattggata tagttattga ttggatatag taattggata taataattga     600
   ttatgtaggt aaaagatgtt ttttgttgga attagataga attttatttt tgtattattg     660
   ttttaaggtg atttaatatt ttttttttat tttttagaag tattattatg ttattatttg    720
   aagtagtttt ttatttgttt ttgattaaaa gtgtatagat tgattttaat gtttcggagt     780
   gtatatattt gaaatgtaat atgatagagg ttgggtacgg tggtttacgt ttgtaatttt     840
   agtattttga gaggtcgagg cgggtagatt atttggggtt aggaatttgg gattattata     900
   gtgaaatttt gtttttattg aaaatataaa aaattagttt ggcgtggtgg cgcgcgttta     960
   tagttttagt tatttggaag gttgaggtac gagaatcgat taattcggga ggcggaggtt    1020
   gtagtgattt tagatggcgt cgttgtattt tagtttgggg gatagaggga aattttgttt    1080
   taaaaaaaaa aaaaaaggaa taaatttata tggtaatgtt tattagtttt tttattagtg    1140
   gatatatttc gtgtttgaat ggttttatgg aatgatgtat atatttggat tggtagtttt    1200
   agggagcgtt tgataataat tttagttttt gggagtttgt tgaattttat gaagtttgaa    1260
   ataatatttt ttaaatattt gggtgattag ttgtagatgg tatttttaat atgttgtttg    1320
   attacgattt atttaagaaa gggcgaagtt tttttattgt tttaggattt ggataagtta    1380
   ttttttatcg tttagcgttt tggagtttcg tttttatttt aagaggttgg tgtaaagggt   1440
   tttcggtatt tatttcggga tttatgaaaa ttatattatt tagaatgttt tgcgttgaac    1500
   gttacgttat taagttagta agagtttaga aaatcgattt tttttgagag ttataaaaag    1560
   aaaggacggg atttttgggg gggttttttc gtggcggtta ttttagtttt tttgaggtgt   1620
   gtttatcgga ttagagatag tagagcgtcg agttggggtt acgaaggcgt gaggggagtc    1680
   gtcgtttttt ttgtacgaaa gcgtttaagt tttggcgacg tcgttttggg ggatttacgt    1740
   taggtttggg atagggatcg ttgttttcgg gtttttatta atgtcgttcg tcgttttcgg    1800
   tttagtttta ttcgtagagt ttgatggtag cggttatttt gaggacgtta atttaggtga    1860
   gtgcggcgtt ttttcgtttt tatatatttt tttttattta cgttttaaag ttattagtga    1920
   ggggcgtttg ttcgagtttt cgttgtttag ggtcggggat attgaggcgt tcgtgggtgg    1980
   ggtttttttt ttgatattgc gtgtgacgag gtgtgggaga gcgtgatagg cggaggaatc    2040
   ggcgcgtgta aaggttgagg cgcgattgag ttaggagaat tcggaaagtt gttttttgta   2100
   gggaacgaag aatatgaggc ggagttattt ttagggttgt attgttattg tgaggatgtt    2160
   agaagttacg gagagttttg aataaaggag ggacgttatt tatgttagtt tttttttttt    2220
   tgagacggag tttcgttttt atcgtttagg ttggagtgta atgacgcgat ttcgggttat    2280
   tgtaattttc gattttcggg tttaagcgat ttttttgttt tagttttttt agtagttggg    2340
   attataagtt tgtagttggg attataacgt tcggttaatt ttttgtattt ttagtagaga    2400
   cgggatttta ttattttggt tagattggtt tggaattttt gataagtgat ttttttgttt   2460
   cggtttttta aagtgttggg attataggcg tgagttatcg tgttcgtttt atgttagggt    2520
   ttaaaaattt ttttaaagat tgtttatagg aagagtagcg atggggcgat gaggatattg    2580
   agatagtggg aggttatatt ttttttttat tgttttatag ttggtaagag gtgatagtat    2640
   tgagattaga aagtaaaata gttagtttga gtttagggtt aggtagggtt atagggaggt    2700
   ttgagtttat ttagttttat tagggatgta tttttttttt tttggttttt ttagttgtag    2760
   aagggcgtat ggaatttata tagggaagtg gagagtgttc ggttttttag tagtttttat    2820
   ttttatgttt ttgggattgt ggttttttag tatttttaag tgttgttatt ttaattttgg    2880
   gtttggtgtt ttggagaaat tttaagttta gggtttttaa gtttatgtta gaggttatag    2940
   gggttatatt gaggtgtttt tatttttgtt agttagtgga aatttgtgaa aggttatttt    3000
   t                                                                    3001
<210> 483
   <211> 3001
   <212> DNA
   <213> human
<400> 483
   aatataaaaa ttagttgggt gttatggtgt gtgtttgtag ttttagttat ttgggaggtt      60
   gaggtaagat aattgtttga atttaggagg tggagaggtt gtagtgaaat aagattgtgt     120
   tattgtattt tagtttgggt aataagagtg aaattttatt ttaaaaaaaa agaaaaaaaa     180
   aaaagaagag atttttattt tatatatttt gagtgttttt gtagttagta aagtagatga     240
   aggtttgttg ggttggagtt ttaggttttt taatgttgaa atgttttgtg aagtttttat     300
   ttatttaggt attttagtaa tttgtttttt ttgtttggaa tttattttaa aaatatttat     360
   atggtttatt ttttattttt tttaagtgta ttttattggt tttttgatta agaagtttta     420
   tttttttttt ttgtttttta ttaaagtagt atttttttta ttgttttttg agattgatgt     480
   tagattttta gaaggaatat attttagata ttaagtaaga attatgaaat ggataaaagt     540
   gttgtttagt gaattggata tagttattga ttggatatag taattggata taataattga     600
   ttatgtaggt aaaagatgtt ttttgttgga attagataga attttatttt tgtattattg     660
   ttttaaggtg atttaatatt ttttttttat tttttagaag tattattatg ttattatttg     720
   aagtagtttt ttatttgttt ttgattaaaa gtgtatagat tgattttaat gttttggagt     780
   gtatatattt gaaatgtaat atgatagagg ttgggtatgg tggtttatgt ttgtaatttt     840
   agtattttga gaggttgagg tgggtagatt atttggggtt aggaatttgg gattattata     900
   gtgaaatttt gtttttattg aaaatataaa aaattagttt ggtgtggtgg tgtgtgttta     960
   tagttttagt tatttggaag gttgaggtat gagaattgat taatttggga ggtggaggtt    1020
   gtagtgattt tagatggtgt tgttgtattt tagtttgggg gatagaggga aattttgttt    1080
   taaaaaaaaa aaaaaaggaa taaatttata tggtaatgtt tattagtttt tttattagtg    1140
   gatatatttt gtgtttgaat ggttttatgg aatgatgtat atatttggat tggtagtttt    1200
   agggagtgtt tgataataat tttagttttt gggagtttgt tgaattttat gaagtttgaa    1260
   ataatatttt ttaaatattt gggtgattag ttgtagatgg tatttttaat atgttgtttg    1320
   attatgattt atttaagaaa gggtgaagtt tttttattgt tttaggattt ggataagtta    1380
   ttttttattg tttagtgttt tggagttttg tttttatttt aagaggttgg tgtaaagggt    1440
   ttttggtatt tattttggga tttatgaaaa ttatattatt tagaatgttt tgtgttgaat    1500
   gttatgttat taagttagta agagtttaga aaattgattt tttttgagag ttataaaaag    1560
   aaaggatggg atttttgggg gggttttttt gtggtggtta ttttagtttt tttgaggtgt    1620
   gtttattgga ttagagatag tagagtgttg agttggggtt atgaaggtgt gaggggagtt    1680
   gttgtttttt ttgtatgaaa gtgtttaagt tttggtgatg ttgttttggg ggatttatgt    1740
   taggtttggg atagggattg ttgtttttgg gtttttatta atgttgtttg ttgtttttgg    1800
   tttagtttta tttgtagagt ttgatggtag tggttatttt gaggatgtta atttaggtga    1860
   gtgtggtgtt tttttgtttt tatatatttt tttttattta tgttttaaag ttattagtga    1920
   ggggtgtttg tttgagtttt tgttgtttag ggttggggat attgaggtgt ttgtgggtgg    1980
   ggtttttttt ttgatattgt gtgtgatgag gtgtgggaga gtgtgatagg tggaggaatt    2040
   ggtgtgtgta aaggttgagg tgtgattgag ttaggagaat ttggaaagtt gttttttgta    2100
   gggaatgaag aatatgaggt ggagttattt ttagggttgt attgttattg tgaggatgtt    2160
   agaagttatg gagagttttg aataaaggag ggatgttatt tatgttagtt tttttttttt    2220
   tgagatggag ttttgttttt attgtttagg ttggagtgta atgatgtgat tttgggttat    2280
   tgtaattttt gatttttggg tttaagtgat ttttttgttt tagttttttt agtagttggg    2340
   attataagtt tgtagttggg attataatgt ttggttaatt ttttgtattt ttagtagaga    2400
   tgggatttta ttattttggt tagattggtt tggaattttt gataagtgat ttttttgttt    2460
   tggtttttta aagtgttggg attataggtg tgagttattg tgtttgtttt atgttagggt    2520
   ttaaaaattt ttttaaagat tgtttatagg aagagtagtg atggggtgat gaggatattg    2580
   agatagtggg aggttatatt ttttttttat tgttttatag ttggtaagag gtgatagtat    2640
   tgagattaga aagtaaaata gttagtttga gtttagggtt aggtagggtt atagggaggt    2700
   ttgagtttat ttagttttat tagggatgta tttttttttt tttggttttt ttagttgtag    2760
   aagggtgtat ggaatttata tagggaagtg gagagtgttt ggttttttag tagtttttat    2820
   ttttatgttt ttgggattgt ggttttttag tatttttaag tgttgttatt ttaattttgg    2880
   gtttggtgtt ttggagaaat tttaagttta gggtttttaa gtttatgtta gaggttatag    2940
   gggttatatt gaggtgtttt tatttttgtt agttagtgga aatttgtgaa aggttatttt    3000
   t                                                                    3001
<210> 484
   <211> 3001
   <212> DNA
   <213> human
<400> 484
   agagataatt ttttataagt ttttattggt tgataaaaat gggaatattt tagtataatt      60
   tttgtaattt ttggtatgga tttaagagtt ttgggtttgg aattttttta gggtattaga     120
   tttaggattg ggtaatagtt atttaagaat attaggaaat tataatttta agaatatggg      180
   ggtagaggtt attgagggat cgaatatttt ttattttttt atgtgagttt tatacgtttt     240
   tttataattg ggagaattag ggaaagagga atgtattttt ggtgaggtta gatgagttta     300
   agtttttttg tagttttgtt tggttttgaa tttaggttgg ttgttttatt ttttggtttt     360
   agtgttgtta ttttttgtta attgtagggt aatgaaaaaa agatgtagtt ttttattatt     420
   ttaatgtttt tatcgtttta tcgttgtttt ttttgtgaat agtttttgga aaagttttta     480
   aattttaata tagggcgggt acggtggttt acgtttgtaa ttttagtatt ttgggaggtc     540
   gaggtaggag gattatttgt taggagtttt agattagttt ggttaaaatg gtgaaatttc     600
   gtttttatta aaaatataaa aaattagtcg gacgttgtaa ttttagttgt aggtttgtaa     660
   ttttagttgt tggggaggtt gaggtaggag aatcgtttga attcgggagt cggaggttgt     720
   agtgattcga gatcgcgtta ttgtatttta gtttgggcga taagagcgaa atttcgtttt     780
   aaagaaaaaa aaattaatat aaatggcgtt ttttttttgt ttagaatttt tcgtggtttt     840
   tagtattttt ataatgatag tataatttta ggagtaattt cgttttatat ttttcgtttt     900
   ttgtagaaaa tagtttttcg aatttttttg gtttagtcgc gttttaattt ttgtacgcgt     960
   cggttttttc gtttgttacg tttttttata tttcgttata cgtagtgtta aaaaaagggt    1020
   tttatttacg aacgttttag tgttttcgat tttgggtagc ggggattcga gtaggcgttt    1080
   tttattgatg gttttagaac gtgggtgggg gaaggtgtgt gaggacggga agacgtcgta    1140
   tttatttgag ttggcgtttt tagagtggtc gttgttatta gattttgcgg gtagagttgg    1200
   gtcgggagcg acgggcgata ttggtaggga ttcggggata gcggttttta ttttaggttt    1260
   gacgtgggtt ttttagggcg gcgtcgttaa ggtttagacg ttttcgtgta ggagggacga    1320
   cgattttttt tacgttttcg tggttttaat tcggcgtttt gttatttttg attcggtgaa    1380
   tatattttag agaagttaaa atggtcgtta cgaagaggtt tttttaaaag tttcgttttt    1440
   tttttttgtg atttttaagg aaagtcggtt ttttgagttt ttattggttt agtagcgtgg    1500
   cgtttaacgt agagtatttt aggtaatgta gtttttatag atttcgaggt gggtgtcggg    1560
   gattttttgt attaattttt tggagtaaaa gcgaagtttt agggcgttgg gcgatgagaa     1620
   atggtttatt taagttttag ggtagtggag ggatttcgtt tttttttaaa tgggtcgtaa    1680
   ttagatagta tattagaggt gttatttgta attgattatt taagtgtttg gggaatatta    1740
   ttttaaattt tataaagttt agtaggtttt tagaggttaa aattattatt agacgttttt    1800
   taaggttgtt aatttaaata tgtatattat tttatagagt tatttagata cgaaatatat    1860
   ttattagtag ggaagttaat aaatattatt atataagttt attttttttt tttttttttt     1920
   aagataaagt ttttttttgt tttttaggtt ggagtgtagc ggcgttattt gggattattg    1980
   taattttcgt ttttcgggtt aatcgatttt cgtgttttaa ttttttaagt agttgggatt   2040
   ataggcgcgc gttattacgt taggttaatt ttttgtattt ttagtagaga tagggtttta    2100
   ttatggtggt tttaaatttt tggttttagg tgatttgttc gtttcggttt tttaaagtgt    2160
   tgggattata ggcgtaagtt atcgtattta gtttttatta tattatattt taaatgtatg    2220
   tatttcgaag tattgaagtt agtttgtgta tttttggtta gggataggtg aaaaattatt    2280
   ttagatagtg gtatgatagt gtttttagaa gatagaagag aaatgttaga ttattttgaa    2340
   atagtagtat aaaggtagaa ttttgtttga ttttaataga aaatattttt tgtttgtata    2400
   gttaattatt gtgtttaatt attgtgttta gttaataatt gtgtttaatt tattgagtaa    2460
   cgtttttatt tattttataa tttttatttg atatttgaga tatatttttt ttagaagttt    2520
   aatattaatt ttaaaaaata atgaaagaaa tgttattttg ataagagata aaggggagag    2580
   gtgggatttt ttaattagaa agtcggtgaa atgtatttga aggaagtgag aaatgagtta    2640
   tgtgggtatt tttagaataa attttaggta gaagaagtag gttattagag tgtttggatg     2700
   gatggagatt ttatagggta ttttagtatt ggagggttta gggttttaat ttagtaggtt    2760
   tttatttgtt ttgttggttg tagagatatt tagggtatat aggtgagaaa tttttttttt     2820
   tttttttttt tttttttttg agatggagtt ttatttttat tgtttaggtt ggagtgtaat    2880
   gacgtagttt tgttttattg taattttttt attttttggg tttaagcgat tattttgttt    2940
   tagtttttta agtagttggg attataggcg tacgttatga tatttagtta atttttgtat    3000
   t                                                                    3001
<210> 485
   <211> 3001
   <212> DNA
   <213> human
<400> 485
   agagataatt ttttataagt ttttattggt tgataaaaat gggaatattt tagtataatt      60
   tttgtaattt ttggtatgga tttaagagtt ttgggtttgg aattttttta gggtattaga     120
   tttaggattg gggtaatagt atttaagaat attaggaaat tataatttta agaatatggg     180
   ggtagaggtt attgagggat tgaatatttt ttattttttt atgtgagttt tatatgtttt     240
   tttataattg ggagaattag ggaaagagga atgtattttt ggtgaggtta gatgagttta     300
   agtttttttg tagttttgtt tggttttgaa tttaggttgg ttgttttatt ttttggtttt     360
   agtgttgtta ttttttgtta attgtagggt aatgaaaaaa agatgtagtt ttttattatt     420
   ttaatgtttt tattgtttta ttgttgtttt ttttgtgaat agtttttgga aaagttttta     480
   aattttaata tagggtgggt atggtggttt atgtttgtaa ttttagtatt ttgggaggtt     540
   gaggtaggag gattatttgt taggagtttt agattagttt ggttaaaatg gtgaaatttt     600
   gtttttatta aaaatataaa aaattagttg gatgttgtaa ttttagttgt aggtttgtaa     660
   ttttagttgt tggggaggtt gaggtaggag aattgtttga atttgggagt tggaggttgt     720
   agtgatttga gattgtgtta ttgtatttta gtttgggtga taagagtgaa attttgtttt     780
   aaagaaaaaa aaattaatat aaatggtgtt ttttttttgt ttagaatttt ttgtggtttt     840
   tagtattttt ataatgatag tataatttta ggagtaattt tgttttatat tttttgtttt     900
   ttgtagaaaa tagttttttg aatttttttg gtttagttgt gttttaattt ttgtatgtgt     960
   tggttttttt gtttgttatg tttttttata ttttgttata tgtagtgtta aaaaaagggt    1020
   tttatttatg aatgttttag tgtttttgat tttgggtagt ggggatttga gtaggtgttt    1080
   tttattgatg gttttagaat gtgggtgggg gaaggtgtgt gaggatggga agatgttgta    1140
   tttatttgag ttggtgtttt tagagtggtt gttgttatta gattttgtgg gtagagttgg    1200
   gttgggagtg atgggtgata ttggtaggga tttggggata gtggttttta ttttaggttt    1260
   gatgtgggtt ttttagggtg gtgttgttaa ggtttagatg tttttgtgta ggagggatga    1320
   tgattttttt tatgtttttg tggttttaat ttggtgtttt gttatttttg atttggtgaa    1380
   tatattttag agaagttaaa atggttgtta tgaagaggtt tttttaaaag ttttgttttt    1440
   tttttttgtg atttttaagg aaagttggtt ttttgagttt ttattggttt agtagtgtgg    1500
   tgtttaatgt agagtatttt aggtaatgta gtttttatag attttgaggt gggtgttggg    1560
   gattttttgt attaattttt tggagtaaaa gtgaagtttt agggtgttgg gtgatgagaa    1620
   atggtttatt taagttttag ggtagtggag ggattttgtt tttttttaaa tgggttgtaa    1680
   ttagatagta tattagaggt gttatttgta attgattatt taagtgtttg gggaatatta    1740
   ttttaaattt tataaagttt agtaggtttt tagaggttaa aattattatt agatgttttt    1800
   taaggttgtt aatttaaata tgtatattat tttatagagt tatttagata tgaaatatat    1860
   ttattagtag ggaagttaat aaatattatt atataagttt attttttttt tttttttttt    1920
   aagataaagt ttttttttgt tttttaggtt ggagtgtagt ggtgttattt gggattattg    1980
   taatttttgt tttttgggtt aattgatttt tgtgttttaa ttttttaagt agttgggatt    2040
   ataggtgtgt gttattatgt taggttaatt ttttgtattt ttagtagaga tagggtttta    2100
   ttatggtggt tttaaatttt tggttttagg tgatttgttt gttttggttt tttaaagtgt    2160
   tgggattata ggtgtaagtt attgtattta gtttttatta tattatattt taaatgtatg    2220
   tattttgaag tattgaagtt agtttgtgta tttttggtta gggataggtg aaaaattatt    2280
   ttagatagtg gtatgatagt gtttttagaa gatagaagag aaatgttaga ttattttgaa    2340
   atagtagtat aaaggtagaa ttttgtttga ttttaataga aaatattttt tgtttgtata    2400
   gttaattatt gtgtttaatt attgtgttta gttaataatt gtgtttaatt tattgagtaa    2460
   tgtttttatt tattttataa tttttatttg atatttgaga tatatttttt ttagaagttt    2520
   aatattaatt ttaaaaaata atgaaagaaa tgttattttg ataagagata aaggggagag    2580
   gtgggatttt ttaattagaa agttggtgaa atgtatttga aggaagtgag aaatgagtta    2640
   tgtgggtatt tttagaataa attttaggta gaagaagtag gttattagag tgtttggatg    2700
   gatggagatt ttatagggta ttttagtatt ggagggttta gggttttaat ttagtaggtt    2760
   tttatttgtt ttgttggttg tagagatatt tagggtatat aggtgagaaa tttttttttt    2820
   tttttttttt tttttttttg agatggagtt ttatttttat tgtttaggtt ggagtgtaat    2880
   gatgtagttt tgttttattg taattttttt atttttgggg tttaagtgat tattttgttt    2940
   tagtttttta agtagttggg attataggtg tatgttatga tatttagtta atttttgtat    3000
   t                                                                    3001
<210> 486
   <211> 2182
   <212> DNA
   <213> human
<400> 486
   tactttctgg tctcagtgct gtcacctctt gccaactgta gggcaatgaa aaaaagatgt      60
   agcctcccac tatctcaatg tcctcatcgc cccatcgctg ctcttcctgt gaacagtctt     120
   tggaaaagtt tttaaaccct aacatagggc gggcacggtg gctcacgcct gtaatcccag     180
   cactttggga ggccgaggca ggaggatcac ttgtcaggag ttccagacca gtctggccaa     240
   aatggtgaaa tcccgtctct actaaaaata caaaaaatta gccggacgtt gtaatcccag     300
   ctgcaggctt gtaatcccag ctgctgggga ggctgaggca ggagaatcgc ttgaacccgg     360
   gagtcggagg ttgcagtgac ccgagatcgc gtcattgcac tccagcctgg gcgataagag     420
   cgaaactccg tctcaaagaa aaaaaaacta acataaatgg cgtccctcct ttgttcagaa     480
   ctctccgtgg cttctagcat cctcacaatg acagtacaac cctaggagta actccgcctc     540
   atattcttcg ttccctgcag aaaacagctt tccgaattct cctggctcag tcgcgcctca     600
   acctttgcac gcgccggttc ctccgcctgt cacgctctcc cacacctcgt cacacgcagt     660
   gtcaaaaaaa gggccccacc cacgaacgcc tcagtgtccc cgaccctggg cagcggggac     720
   tcgagcaggc gcccctcact gatggcttta gaacgtgggt gggggaaggt gtgtgaggac     780
   gggaagacgc cgcactcacc tgagttggcg tcctcagagt ggccgctgcc atcagactct     840
   gcgggtagag ctgggccggg agcgacgggc gacattggta gggacccggg gacagcggtc     900
   cctatcccag gcctgacgtg ggtcccccag ggcggcgtcg ccaaggctta gacgctttcg     960
   tgcaggaggg acgacgactc ccctcacgcc ttcgtggccc caactcggcg ctctgctatc    1020
   tctgatccgg tgaacacacc tcagagaagc taaaatggcc gccacgaaga ggccccccca    1080
   aaagtcccgt cctttctttt tgtgactctc aaggaaagtc ggttttctga gctcttactg    1140
   gcttagtagc gtggcgttca acgcagagca ttctaggtaa tgtagttttc atagatcccg    1200
   aggtgggtgc cggggaccct ttgcaccaac ctcttggagt aaaagcgaag ctccagggcg    1260
   ctgggcgatg agaaatggct tatccaagtc ctagggcagt ggagggactt cgccctttct    1320
   taaatgggtc gtaatcagac agcatattag aggtgccatc tgcaactgat cacccaagtg    1380
   tttggggaat attatttcaa acttcataaa gttcagcagg ctcccagagg ctaaaattat    1440
   tatcagacgc tccctaaggc tgccaatcca aatatgtaca tcattccata gagccattca    1500
   gacacgaaat atatccacta gtagggaagc taataaacat taccatataa gtttattcct    1560
   tttttttttt ttttaagaca aagtttccct ctgtccccca ggctggagtg cagcggcgcc    1620
   atctgggatc actgcaacct ccgcctcccg ggttaatcga ttctcgtgcc tcaaccttcc    1680
   aagtagctgg gactataggc gcgcgccacc acgccaggct aattttttgt attttcagta    1740
   gagacagggt ttcactatgg tggtcccaaa ttcctggccc caggtgatct gcccgcctcg    1800
   gcctctcaaa gtgctgggat tacaggcgta agccaccgta cccagcctct atcatattac    1860
   atttcaaatg tatgcactcc gaagcattga agtcagtctg tgcacttttg gtcagggaca    1920
   ggtgaaaaac tacttcagat agtggcatga tagtgcttct agaagataga agagaaatgt    1980
   tagatcacct tgaaacagta gtacaaaggt agaattctgt ctgattccaa cagaaaacat    2040
   cttttgcctg catagtcaat tattgtgtcc aattactgtg tccagtcaat aactgtgtcc    2100
   aattcactga gcaacgcttt tatccatttc ataattctta cttgatatct gagatatatt    2160
   ccttctagaa gtctaacatc aa                                              2182
<210> 487
   <211> 2182
   <212> DNA
   <213> human
<400> 487
   tattttttgg ttttagtgtt gttatttttt gttaattgta gggtaatgaa aaaaagatgt      60
   agttttttat tattttaatg tttttatcgt tttatcgttg ttttttttgt gaatagtttt     120
   tggaaaagtt tttaaatttt aatatagggc gggtacggtg gtttacgttt gtaattttag    180
   tattttggga ggtcgaggta ggaggattat ttgttaggag ttttagatta gtttggttaa    240
   aatggtgaaa tttcgttttt attaaaaata taaaaaatta gtcggacgtt gtaattttag    300
   ttgtaggttt gtaattttag ttgttgggga ggttgaggta ggagaatcgt ttgaattcgg    360
   gagtcggagg ttgtagtgat tcgagatcgc gttattgtat tttagtttgg gcgataagag    420
   cgaaatttcg ttttaaagaa aaaaaaatta atataaatgg cgtttttttt ttgtttagaa    480
   tttttcgtgg tttttagtat ttttataatg atagtataat tttaggagta atttcgtttt    540
   atatttttcg ttttttgtag aaaatagttt ttcgaatttt tttggtttag tcgcgtttta    600
   atttttgtac gcgtcggttt tttcgtttgt tacgtttttt tatatttcgt tatacgtagt    660
   gttaaaaaaa gggttttatt tacgaacgtt ttagtgtttt cgattttggg tagcggggat    720
   tcgagtaggc gttttttatt gatggtttta gaacgtgggt gggggaaggt gtgtgaggac    780
   gggaagacgt cgtatttatt tgagttggcg tttttagagt ggtcgttgtt attagatttt    840
   gcgggtagag ttgggtcggg agcgacgggc gatattggta gggattcggg gatagcggtt    900
   tttattttag gtttgacgtg ggttttttag ggcggcgtcg ttaaggttta gacgttttcg    960
   tgtaggaggg acgacgattt tttttacgtt ttcgtggttt taattcggcg ttttgttatt   1020
   tttgattcgg tgaatatatt ttagagaagt taaaatggtc gttacgaaga ggttttttta   1080
   aaagtttcgt tttttttttt tgtgattttt aaggaaagtc ggttttttga gtttttattg  1140
   gtttagtagc gtggcgttta acgtagagta ttttaggtaa tgtagttttt atagatttcg   1200
   aggtgggtgt cggggatttt ttgtattaat tttttggagt aaaagcgaag ttttagggcg   1260
   ttgggcgatg agaaatggtt tatttaagtt ttagggtagt ggagggattt cgtttttttt   1320
   taaatgggtc gtaattagat agtatattag aggtgttatt tgtaattgat tatttaagtg   1380
   tttggggaat attattttaa attttataaa gtttagtagg tttttagagg ttaaaattat   1440
   tattagacgt tttttaaggt tgttaattta aatatgtata ttattttata gagttattta  1500
   gatacgaaat atatttatta gtagggaagt taataaatat tattatataa gtttattttt   1560
   tttttttttt ttttaagata aagttttttt ttgtttttta ggttggagtg tagcggcgtt   1620
   atttgggatt attgtaattt tcgtttttcg ggttaatcga ttttcgtgtt ttaatttttt   1680
   aagtagttgg gattataggc gcgcgttatt acgttaggtt aattttttgt atttttagta   1740
   gagatagggt tttattatgg tggttttaaa tttttggttt taggtgattt gttcgtttcg   1800
   gttttttaaa gtgttgggat tataggcgta agttatcgta tttagttttt attatattat  1860
   attttaaatg tatgtatttc gaagtattga agttagtttg tgtatttttg gttagggata   1920
   ggtgaaaaat tattttagat agtggtatga tagtgttttt agaagataga agagaaatgt   1980
   tagattattt tgaaatagta gtataaaggt agaattttgt ttgattttaa tagaaaatat   2040
   tttttgtttg tatagttaat tattgtgttt aattattgtg tttagttaat aattgtgttt   2100
   aatttattga gtaacgtttt tatttatttt ataattttta tttgatattt gagatatatt   2160
   ttttttagaa gtttaatatt aa                                             2182
<210> 488
   <211> 2182
   <212> DNA
   <213> human
<400> 488
   tattttttgg ttttagtgtt gttatttttt gttaattgta gggtaatgaa aaaaagatgt      60
   agttttttat tattttaatg tttttattgt tttattgttg ttttttttgt gaatagtttt     120
   tggaaaagtt tttaaatttt aatatagggt gggtatggtg gtttatgttt gtaattttag    180
   tattttggga ggttgaggta ggaggattat ttgttaggag ttttagatta gtttggttaa    240
   aatggtgaaa ttttgttttt attaaaaata taaaaaatta gttggatgtt gtaattttag    300
   ttgtaggttt gtaattttag ttgttgggga ggttgaggta ggagaattgt ttgaatttgg    360
   gagttggagg ttgtagtgat ttgagattgt gttattgtat tttagtttgg gtgataagag    420
   tgaaattttg ttttaaagaa aaaaaaatta atataaatgg tgtttttttt ttgtttagaa 480
   ttttttgtgg tttttagtat ttttataatg atagtataat tttaggagta attttgtttt 540
   atattttttg ttttttgtag aaaatagttt tttgaatttt tttggtttag ttgtgtttta 600
   atttttgtat gtgttggttt ttttgtttgt tatgtttttt tatattttgt tatatgtagt 660
   gttaaaaaaa gggttttatt tatgaatgtt ttagtgtttt tgattttggg tagtggggat 720
   ttgagtaggt gttttttatt gatggtttta gaatgtgggt gggggaaggt gtgtgaggat 780
   gggaagatgt tgtatttatt tgagttggtg tttttagagt ggttgttgtt attagatttt 840
   gtgggtagag ttgggttggg agtgatgggt gatattggta gggatttggg gatagtggtt 900
   tttattttag gtttgatgtg ggttttttag ggtggtgttg ttaaggttta gatgtttttg 960
   tgtaggaggg atgatgattt tttttatgtt tttgtggttt taatttggtg ttttgttatt 1020
   tttgatttgg tgaatatatt ttagagaagt taaaatggtt gttatgaaga ggttttttta 1080
   aaagttttgt tttttttttt tgtgattttt aaggaaagtt ggttttttga gtttttattg 1140
   gtttagtagt gtggtgttta atgtagagta ttttaggtaa tgtagttttt atagattttg 1200
   aggtgggtgt tggggatttt ttgtattaat tttttggagt aaaagtgaag ttttagggtg 1260
   ttgggtgatg agaaatggtt tatttaagtt ttagggtagt ggagggattt tgtttttttt 1320
   taaatgggtt gtaattagat agtatattag aggtgttatt tgtaattgat tatttaagtg 1380
   tttggggaat attattttaa attttataaa gtttagtagg tttttagagg ttaaaattat 1440
   tattagatgt tttttaaggt tgttaattta aatatgtata ttattttata gagttattta 1500
   gatatgaaat atatttatta gtagggaagt taataaatat tattatataa gtttattttt 1560
   tttttttttt ttttaagata aagttttttt ttgtttttta ggttggagtg tagtggtgtt 1620
   atttgggatt attgtaattt ttgttttttg ggttaattga tttttgtgtt ttaatttttt 1680
   aagtagttgg gattataggt gtgtgttatt atgttaggtt aattttttgt atttttagta 1740
   gagatagggt tttattatgg tggttttaaa tttttggttt taggtgattt gtttgttttg 1800
   gttttttaaa gtgttgggat tataggtgta agttattgta tttagttttt attatattat 1860
   attttaaatg tatgtatttt gaagtattga agttagtttg tgtatttttg gttagggata 1920
   ggtgaaaaat tattttagat agtggtatga tagtgttttt agaagataga agagaaatgt 1980
   tagattattt tgaaatagta gtataaaggt agaattttgt ttgattttaa tagaaaatat 2040
   tttttgtttg tatagttaat tattgtgttt aattattgtg tttagttaat aattgtgttt 2100
   aatttattga gtaatgtttt tatttatttt ataattttta tttgatattt gagatatatt 2160
   ttttttagaa gtttaatatt aa 2182
<210> 489
   <211> 2182
   <212> DNA
   <213> human
<400> 489
   ttgatgttag atttttagaa ggaatatatt ttagatatta agtaagaatt atgaaatgga      60
   taaaagcgtt gtttagtgaa ttggatatag ttattgattg gatatagtaa ttggatataa     120
   taattgatta tgtaggtaaa agatgttttt tgttggaatt agatagaatt ttatttttgt     180
   attattgttt taaggtgatt taatattttt tttttatttt ttagaagtat tattatgtta     240
   ttatttgaag tagtttttta tttgtttttg attaaaagtg tatagattga ttttaatgtt     300
   tcggagtgta tatatttgaa atgtaatatg atagaggttg ggtacggtgg tttacgtttg     360
   taattttagt attttgagag gtcgaggcgg gtagattatt tggggttagg aatttgggat     420
   tattatagtg aaattttgtt tttattgaaa atataaaaaa ttagtttggc gtggtggcgc     480
   gcgtttatag ttttagttat ttggaaggtt gaggtacgag aatcgattaa ttcgggaggc     540
   ggaggttgta gtgattttag atggcgtcgt tgtattttag tttgggggat agagggaaat     600
   tttgttttaa aaaaaaaaaa aaaggaataa atttatatgg taatgtttat tagttttttt     660
   attagtggat atatttcgtg tttgaatggt tttatggaat gatgtatata tttggattgg     720
   tagttttagg gagcgtttga taataatttt agtttttggg agtttgttga attttatgaa     780
   gtttgaaata atatttttta aatatttggg tgattagttg tagatggtat ttttaatatg      840
   ttgtttgatt acgatttatt taagaaaggg cgaagttttt ttattgtttt aggatttgga     900
   taagttattt tttatcgttt agcgttttgg agtttcgttt ttattttaag aggttggtgt     960
   aaagggtttt cggtatttat ttcgggattt atgaaaatta tattatttag aatgttttgc    1020
   gttgaacgtt acgttattaa gttagtaaga gtttagaaaa tcgatttttt ttgagagtta    1080
   taaaaagaaa ggacgggatt tttggggggg ttttttcgtg gcggttattt tagttttttt    1140
   gaggtgtgtt tatcggatta gagatagtag agcgtcgagt tggggttacg aaggcgtgag    1200
   gggagtcgtc gttttttttg tacgaaagcg tttaagtttt ggcgacgtcg ttttggggga    1260
   tttacgttag gtttgggata gggatcgttg ttttcgggtt tttattaatg tcgttcgtcg    1320
   ttttcggttt agttttattc gtagagtttg atggtagcgg ttattttgag gacgttaatt    1380
   taggtgagtg cggcgttttt tcgtttttat atattttttt ttatttacgt tttaaagtta    1440
   ttagtgaggg gcgtttgttc gagttttcgt tgtttagggt cggggatatt gaggcgttcg    1500
   tgggtggggt tttttttttg atattgcgtg tgacgaggtg tgggagagcg tgataggcgg     1560
   aggaatcggc gcgtgtaaag gttgaggcgc gattgagtta ggagaattcg gaaagttgtt    1620
   ttttgtaggg aacgaagaat atgaggcgga gttattttta gggttgtatt gttattgtga    1680
   ggatgttaga agttacggag agttttgaat aaaggaggga cgttatttat gttagttttt    1740
   ttttttttga gacggagttt cgtttttatc gtttaggttg gagtgtaatg acgcgatttc     1800
   gggttattgt aattttcgat tttcgggttt aagcgatttt tttgttttag tttttttagt    1860
   agttgggatt ataagtttgt agttgggatt ataacgttcg gttaattttt tgtattttta    1920
   gtagagacgg gattttatta ttttggttag attggtttgg aatttttgat aagtgatttt    1980
   tttgtttcgg ttttttaaag tgttgggatt ataggcgtga gttatcgtgt tcgttttatg    2040
   ttagggttta aaaatttttt taaagattgt ttataggaag agtagcgatg gggcgatgag    2100
   gatattgaga tagtgggagg ttatattttt tttttattgt tttatagttg gtaagaggtg    2160
   atagtattga gattagaaag ta                                             2182
<210> 490
   <211> 2182
   <212> DNA
   <213> human
<400> 490
   ttgatgttag atttttagaa ggaatatatt ttagatatta agtaagaatt atgaaatgga 60
   taaaagtgtt gtttagtgaa ttggatatag ttattgattg gatatagtaa ttggatataa 120
   taattgatta tgtaggtaaa agatgttttt tgttggaatt agatagaatt ttatttttgt 180
   attattgttt taaggtgatt taatattttt tttttatttt ttagaagtat tattatgtta 240
   ttatttgaag tagtttttta tttgtttttg attaaaagtg tatagattga ttttaatgtt 300
   ttggagtgta tatatttgaa atgtaatatg atagaggttg ggtatggtgg tttatgtttg 360
   taattttagt attttgagag gttgaggtgg gtagattatt tggggttagg aatttgggat 420
   tattatagtg aaattttgtt tttattgaaa atataaaaaa ttagtttggt gtggtggtgt 480
   gtgtttatag ttttagttat ttggaaggtt gaggtatgag aattgattaa tttgggaggt 540
   ggaggttgta gtgattttag atggtgttgt tgtattttag tttgggggat agagggaaat 600
   tttgttttaa aaaaaaaaaa aaaggaataa atttatatgg taatgtttat tagttttttt 660
   attagtggat atattttgtg tttgaatggt tttatggaat gatgtatata tttggattgg 720
   tagttttagg gagtgtttga taataatttt agtttttggg agtttgttga attttatgaa 780
   gtttgaaata atatttttta aatatttggg tgattagttg tagatggtat ttttaatatg 840
   ttgtttgatt atgatttatt taagaaaggg tgaagttttt ttattgtttt aggatttgga 900
   taagttattt tttattgttt agtgttttgg agttttgttt ttattttaag aggttggtgt 960
   aaagggtttt tggtatttat tttgggattt atgaaaatta tattatttag aatgttttgt 1020
   gttgaatgtt atgttattaa gttagtaaga gtttagaaaa ttgatttttt ttgagagtta 1080
   taaaaagaaa ggatgggatt tttggggggg tttttttgtg gtggttattt tagttttttt 1140
   gaggtgtgtt tattggatta gagatagtag agtgttgagt tggggttatg aaggtgtgag 1200
   gggagttgtt gttttttttg tatgaaagtg tttaagtttt ggtgatgttg ttttggggga 1260
   tttatgttag gtttgggata gggattgttg tttttgggtt tttattaatg ttgtttgttg 1320
   tttttggttt agttttattt gtagagtttg atggtagtgg ttattttgag gatgttaatt 1380
   taggtgagtg tggtgttttt ttgtttttat atattttttt ttatttatgt tttaaagtta 1440
   ttagtgaggg gtgtttgttt gagtttttgt tgtttagggt tggggatatt gaggtgtttg 1500
   tgggtggggt tttttttttg atattgtgtg tgatgaggtg tgggagagtg tgataggtgg 1560
   aggaattggt gtgtgtaaag gttgaggtgt gattgagtta ggagaatttg gaaagttgtt 1620
   ttttgtaggg aatgaagaat atgaggtgga gttattttta gggttgtatt gttattgtga 1680
   ggatgttaga agttatggag agttttgaat aaaggaggga tgttatttat gttagttttt 1740
   ttttttttga gatggagttt tgtttttatt gtttaggttg gagtgtaatg atgtgatttt 1800
   gggttattgt aatttttgat ttttgggttt aagtgatttt tttgttttag tttttttagt 1860
   agttgggatt ataagtttgt agttgggatt ataatgtttg gttaattttt tgtattttta 1920
   gtagagatgg gattttatta ttttggttag attggtttgg aatttttgat aagtgatttt 1980
   tttgttttgg ttttttaaag tgttgggatt ataggtgtga gttattgtgt ttgttttatg 2040
   ttagggttta aaaatttttt taaagattgt ttataggaag agtagtgatg gggtgatgag 2100
   gatattgaga tagtgggagg ttatattttt tttttattgt tttatagttg gtaagaggtg 2160
   atagtattga gattagaaag ta 2182
<210> 491
   <211> 50
   <212> DNA
   <213> human
<400> 491
   cgccttcgtg gccccaactc ggcgctctgc tatctctgat ccggtgaaca 50
<210> 492
   <211> 50
   <212> DNA
   <213> human
<400> 492
   cgttttcgtg gttttaattc ggcgttttgt tatttttgat tcggtgaata 50
<210> 493
   <211> 50
   <212> DNA
   <213> human
<400> 493
   tgtttttgtg gttttaattt ggtgttttgt tatttttgat ttggtgaata 50
<210> 494
   <211> 50
   <212> DNA
   <213> human
<400> 494
   tgtttatcgg attagagata gtagagcgtc gagttggggt tacgaaggcg 50
<210> 495
   <211> 50
   <212> DNA
   <213> human
<400> 495
   tgtttattgg attagagata gtagagtgtt gagttggggt tatgaaggtg 50
<210> 496
   <211> 3001
   <212> DNA
   <213> human
<400> 496
   aaatctctca ttacctccag aataggtacc caacttctca ggcagccact gcagtcctga    60
   ctccttcccc ctgctctttg ttcccagcta aaaggaaaca gatctatggt ttcctcaaaa   120
   atctcagctt agttttacta agcacttgcg cctcctgata ccagtgccag agataacctt   180
   tcacaagttt ccactggctg acaaaaatgg gaacacctca gtataacccc tgtaacctct   240
   ggcatggact taagagccct gggcttggaa tttctccagg gcaccagacc caggattggg   300
   gtaacagcac ttaagaatac taggaaacca caatcccaag aacatggggg tagaggctac   360
   tgagggaccg aacactctcc acttccctat gtgagttcca tacgcccttc tacaactggg   420
   agaaccaggg aaagaggaat gcatccctgg tgaggctaga tgagctcaag cctccctgta   480
   gccctgcctg gccctgaact caggctggct gttttacttt ctggtctcag tgctgtcacc   540
   tcttgccaac tgtagggcaa tgaaaaaaag atgtagcctc ccactatctc aatgtcctca   600
   tcgccccatc gctgctcttc ctgtgaacag tctttggaaa agtttttaaa ccctaacata   660
   gggcgggcac ggtggctcac gcctgtaatc ccagcacttt gggaggccga ggcaggagga   720
   tcacttgtca ggagttccag accagtctgg ccaaaatggt gaaatcccgt ctctactaaa   780
   aatacaaaaa attagccgga cgttgtaatc ccagctgcag gcttgtaatc ccagctgctg   840
   gggaggctga ggcaggagaa tcgcttgaac ccgggagtcg gaggttgcag tgacccgaga   900
   tcgcgtcatt gcactccagc ctgggcgata agagcgaaac tccgtctcaa agaaaaaaaa   960
   actaacataa atggcgtccc tcctttgttc agaactctcc gtggcttcta gcatcctcac  1020
   aatgacagta caaccctagg agtaactccg cctcatattc ttcgttccct gcagaaaaca  1080
   gctttccgaa ttctcctggc tcagtcgcgc ctcaaccttt gcacgcgccg gttcctccgc  1140
   ctgtcacgct ctcccacacc tcgtcacacg cagtgtcaaa aaaagggccc cacccacgaa  1200
   cgcctcagtg tccccgaccc tgggcagcgg ggactcgagc aggcgcccct cactgatggc  1260
   tttagaacgt gggtggggga aggtgtgtga ggacgggaag acgccgcact cacctgagtt  1320
   ggcgtcctca gagtggccgc tgccatcaga ctctgcgggt agagctgggc cgggagcgac  1380
   gggcgacatt ggtagggacc cggggacagc ggtccctatc ccaggcctga cgtgggtccc  1440
   ccagggcggc gtcgccaagg cttagacgct ttcgtgcagg agggacgacg actcccctca  1500
   cgccttcgtg gccccaactc ggcgctctgc tatctctgat ccggtgaaca cacctcagag  1560
   aagctaaaat ggccgccacg aagaggcccc cccaaaagtc ccgtcctttc tttttgtgac  1620
   tctcaaggaa agtcggtttt ctgagctctt actggcttag tagcgtggcg ttcaacgcag  1680
   agcattctag gtaatgtagt tttcatagat cccgaggtgg gtgccgggga ccctttgcac  1740
   caacctcttg gagtaaaagc gaagctccag ggcgctgggc gatgagaaat ggcttatcca  1800
   agtcctaggg cagtggaggg acttcgccct ttcttaaatg ggtcgtaatc agacagcata  1860
   ttagaggtgc catctgcaac tgatcaccca agtgtttggg gaatattatt tcaaacttca  1920
   taaagttcag caggctccca gaggctaaaa ttattatcag acgctcccta aggctgccaa  1980
   tccaaatatg tacatcattc catagagcca ttcagacacg aaatatatcc actagtaggg  2040
   aagctaataa acattaccat ataagtttat tccttttttt ttttttttaa gacaaagttt  2100
   ccctctgtcc cccaggctgg agtgcagcgg cgccatctgg gatcactgca acctccgcct  2160
   cccgggttaa tcgattctcg tgcctcaacc ttccaagtag ctgggactat aggcgcgcgc  2220
   caccacgcca ggctaatttt ttgtattttc agtagagaca gggtttcact atggtggtcc  2280
   caaattcctg gccccaggtg atctgcccgc ctcggcctct caaagtgctg ggattacagg  2340
   cgtaagccac cgtacccagc ctctatcata ttacatttca aatgtatgca ctccgaagca  2400
   ttgaagtcag tctgtgcact tttggtcagg gacaggtgaa aaactacttc agatagtggc  2460
   atgatagtgc ttctagaaga tagaagagaa atgttagatc accttgaaac agtagtacaa  2520
   aggtagaatt ctgtctgatt ccaacagaaa acatcttttg cctgcatagt caattattgt     2580
   gtccaattac tgtgtccagt caataactgt gtccaattca ctgagcaacg cttttatcca     2640
   tttcataatt cttacttgat atctgagata tattccttct agaagtctaa catcaatctc     2700
   aaaaaacaat gaaagaaatg ctactttgat aagagacaaa ggggagaggt gggacttctt     2760
   aatcagaaag ccggtgaaat gcacttgaag gaagtgagaa atgagccatg tgggtatttt     2820
   tagaataaat tccaggcaga agaagcaggt tactagagtg cctggatgga tggagacttc     2880
   acagggcatt tcagcattgg agggcctagg gctccaaccc agcaggcctt catctgcttt     2940
   gctggctgca gagacactca gggtatatag gtgagaaatc tcttcttttt tttttttctt     3000
   t                                                                     3001
<210> 497
   <211> 3001
   <212> DNA
   <213> human
<400> 497
   aaatttttta ttatttttag aataggtatt taatttttta ggtagttatt gtagttttga 60
   tttttttttt ttgttttttg tttttagtta aaaggaaata gatttatggt ttttttaaaa 120
   attttagttt agttttatta agtatttgcg ttttttgata ttagtgttag agataatttt 180
   ttataagttt ttattggttg ataaaaatgg gaatatttta gtataatttt tgtaattttt 240
   ggtatggatt taagagtttt gggtttggaa tttttttagg gtattagatt taggattggg 300
   gtaatagtat ttaagaatat taggaaatta taattttaag aatatggggg tagaggttat 360
   tgagggatcg aatatttttt atttttttat gtgagtttta tacgtttttt tataattggg 420
   agaattaggg aaagaggaat gtatttttgg tgaggttaga tgagtttaag tttttttgta 480
   gttttgtttg gttttgaatt taggttggtt gttttatttt ttggttttag tgttgttatt 540
   ttttgttaat tgtagggtaa tgaaaaaaag atgtagtttt ttattatttt aatgttttta 600
   tcgttttatc gttgtttttt ttgtgaatag tttttggaaa agtttttaaa ttttaatata 660
   gggcgggtac ggtggtttac gtttgtaatt ttagtatttt gggaggtcga ggtaggagga 720
   ttatttgtta ggagttttag attagtttgg ttaaaatggt gaaatttcgt ttttattaaa 780
   aatataaaaa attagtcgga cgttgtaatt ttagttgtag gtttgtaatt ttagttgttg 840
   gggaggttga ggtaggagaa tcgtttgaat tcgggagtcg gaggttgtag tgattcgaga 900
   tcgcgttatt gtattttagt ttgggcgata agagcgaaat ttcgttttaa agaaaaaaaa 960
   attaatataa atggcgtttt ttttttgttt agaatttttc gtggttttta gtatttttat 1020
   aatgatagta taattttagg agtaatttcg ttttatattt ttcgtttttt gtagaaaata 1080
   gtttttcgaa tttttttggt ttagtcgcgt tttaattttt gtacgcgtcg gttttttcgt 1140
   ttgttacgtt tttttatatt tcgttatacg tagtgttaaa aaaagggttt tatttacgaa 1200
   cgttttagtg ttttcgattt tgggtagcgg ggattcgagt aggcgttttt tattgatggt 1260
   tttagaacgt gggtggggga aggtgtgtga ggacgggaag acgtcgtatt tatttgagtt 1320
   ggcgttttta gagtggtcgt tgttattaga ttttgcgggt agagttgggt cgggagcgac 1380
   gggcgatatt ggtagggatt cggggatagc ggtttttatt ttaggtttga cgtgggtttt 1440
   ttagggcggc gtcgttaagg tttagacgtt ttcgtgtagg agggacgacg atttttttta 1500
   cgttttcgtg gttttaattc ggcgttttgt tatttttgat tcggtgaata tattttagag 1560
   aagttaaaat ggtcgttacg aagaggtttt tttaaaagtt tcgttttttt tttttgtgat 1620
   ttttaaggaa agtcggtttt ttgagttttt attggtttag tagcgtggcg tttaacgtag 1680
   agtattttag gtaatgtagt ttttatagat ttcgaggtgg gtgtcgggga ttttttgtat 1740
   taattttttg gagtaaaagc gaagttttag ggcgttgggc gatgagaaat ggtttattta 1800
   agttttaggg tagtggaggg atttcgtttt tttttaaatg ggtcgtaatt agatagtata 1860
   ttagaggtgt tatttgtaat tgattattta agtgtttggg gaatattatt ttaaatttta 1920
   taaagtttag taggttttta gaggttaaaa ttattattag acgtttttta aggttgttaa 1980
   tttaaatatg tatattattt tatagagtta tttagatacg aaatatattt attagtaggg 2040
   aagttaataa atattattat ataagtttat tttttttttt ttttttttaa gataaagttt 2100
   ttttttgttt tttaggttgg agtgtagcgg cgttatttgg gattattgta attttcgttt 2160
   ttcgggttaa tcgattttcg tgttttaatt ttttaagtag ttgggattat aggcgcgcgt 2220
   tattacgtta ggttaatttt ttgtattttt agtagagata gggttttatt atggtggttt 2280
   taaatttttg gttttaggtg atttgttcgt ttcggttttt taaagtgttg ggattatagg 2340
   cgtaagttat cgtatttagt ttttattata ttatatttta aatgtatgta tttcgaagta 2400
   ttgaagttag tttgtgtatt tttggttagg gataggtgaa aaattatttt agatagtggt 2460
   atgatagtgt ttttagaaga tagaagagaa atgttagatt attttgaaat agtagtataa 2520
   aggtagaatt ttgtttgatt ttaatagaaa atattttttg tttgtatagt taattattgt 2580
   gtttaattat tgtgtttagt taataattgt gtttaattta ttgagtaacg tttttattta 2640
   ttttataatt tttatttgat atttgagata tatttttttt agaagtttaa tattaatttt 2700
   aaaaaataat gaaagaaatg ttattttgat aagagataaa ggggagaggt gggatttttt 2760
   aattagaaag tcggtgaaat gtatttgaag gaagtgagaa atgagttatg tgggtatttt 2820
   tagaataaat tttaggtaga agaagtaggt tattagagtg tttggatgga tggagatttt 2880
   atagggtatt ttagtattgg agggtttagg gttttaattt agtaggtttt tatttgtttt 2940
   gttggttgta gagatattta gggtatatag gtgagaaatt tttttttttt tttttttttt 3000
   t 3001
<210> 498
   <211> 3001
   <212> DNA
   <213> human
<400> 498
   aaatttttta ttatttttag aataggtatt taatttttta ggtagttatt gtagttttga     60
   tttttttttt ttgttttttg tttttagtta aaaggaaata gatttatggt ttttttaaaa      120
   attttagttt agttttatta agtatttgtg ttttttgata ttagtgttag agataatttt     180
   ttataagttt ttattggttg ataaaaatgg gaatatttta gtataatttt tgtaattttt    240
   ggtatggatt taagagtttt gggtttggaa tttttttagg gtattagatt taggattggg     300
   gtaatagtat ttaagaatat taggaaatta taattttaag aatatggggg tagaggttat    360
   tgagggattg aatatttttt atttttttat gtgagtttta tatgtttttt tataattggg    420
   agaattaggg aaagaggaat gtatttttgg tgaggttaga tgagtttaag tttttttgta    480
   gttttgtttg gttttgaatt taggttggtt gttttatttt ttggttttag tgttgttatt    540
   ttttgttaat tgtagggtaa tgaaaaaaag atgtagtttt ttattatttt aatgttttta    600
   ttgttttatt gttgtttttt ttgtgaatag tttttggaaa agtttttaaa ttttaatata    660
   gggtgggtat ggtggtttat gtttgtaatt ttagtatttt gggaggttga ggtaggagga    720
   ttatttgtta ggagttttag attagtttgg ttaaaatggt gaaattttgt ttttattaaa    780
   aatataaaaa attagttgga tgttgtaatt ttagttgtag gtttgtaatt ttagttgttg    840
   gggaggttga ggtaggagaa ttgtttgaat ttgggagttg gaggttgtag tgatttgaga    900
   ttgtgttatt gtattttagt ttgggtgata agagtgaaat tttgttttaa agaaaaaaaa    960
   attaatataa atggtgtttt ttttttgttt agaatttttt gtggttttta gtatttttat     1020
   aatgatagta taattttagg agtaattttg ttttatattt tttgtttttt gtagaaaata     1080
   gttttttgaa tttttttggt ttagttgtgt tttaattttt gtatgtgttg gtttttttgt    1140
   ttgttatgtt tttttatatt ttgttatatg tagtgttaaa aaaagggttt tatttatgaa     1200
   tgttttagtg tttttgattt tgggtagtgg ggatttgagt aggtgttttt tattgatggt    1260
   tttagaatgt gggtggggga aggtgtgtga ggatgggaag atgttgtatt tatttgagtt    1320
   ggtgttttta gagtggttgt tgttattaga ttttgtgggt agagttgggt tgggagtgat     1380
   gggtgatatt ggtagggatt tggggatagt ggtttttatt ttaggtttga tgtgggtttt    1440
   ttagggtggt gttgttaagg tttagatgtt tttgtgtagg agggatgatg atttttttta     1500
   tgtttttgtg gttttaattt ggtgttttgt tatttttgat ttggtgaata tattttagag     1560
   aagttaaaat ggttgttatg aagaggtttt tttaaaagtt ttgttttttt tttttgtgat    1620
   ttttaaggaa agttggtttt ttgagttttt attggtttag tagtgtggtg tttaatgtag    1680
   agtattttag gtaatgtagt ttttatagat tttgaggtgg gtgttgggga ttttttgtat   1740
   taattttttg gagtaaaagt gaagttttag ggtgttgggt gatgagaaat ggtttattta     1800
   agttttaggg tagtggaggg attttgtttt tttttaaatg ggttgtaatt agatagtata    1860
   ttagaggtgt tatttgtaat tgattattta agtgtttggg gaatattatt ttaaatttta    1920
   taaagtttag taggttttta gaggttaaaa ttattattag atgtttttta aggttgttaa    1980
   tttaaatatg tatattattt tatagagtta tttagatatg aaatatattt attagtaggg    2040
   aagttaataa atattattat ataagtttat tttttttttt ttttttttaa gataaagttt     2100
   ttttttgttt tttaggttgg agtgtagtgg tgttatttgg gattattgta atttttgttt    2160
   tttgggttaa ttgatttttg tgttttaatt ttttaagtag ttgggattat aggtgtgtgt     2220
   tattatgtta ggttaatttt ttgtattttt agtagagata gggttttatt atggtggttt   2280
   taaatttttg gttttaggtg atttgtttgt tttggttttt taaagtgttg ggattatagg    2340
   tgtaagttat tgtatttagt ttttattata ttatatttta aatgtatgta ttttgaagta    2400
   ttgaagttag tttgtgtatt tttggttagg gataggtgaa aaattatttt agatagtggt    2460
   atgatagtgt ttttagaaga tagaagagaa atgttagatt attttgaaat agtagtataa    2520
   aggtagaatt ttgtttgatt ttaatagaaa atattttttg tttgtatagt taattattgt    2580
   gtttaattat tgtgtttagt taataattgt gtttaattta ttgagtaatg tttttattta    2640
   ttttataatt tttatttgat atttgagata tatttttttt agaagtttaa tattaatttt   2700
   aaaaaataat gaaagaaatg ttattttgat aagagataaa ggggagaggt gggatttttt    2760
   aattagaaag ttggtgaaat gtatttgaag gaagtgagaa atgagttatg tgggtatttt    2820
   tagaataaat tttaggtaga agaagtaggt tattagagtg tttggatgga tggagatttt    2880
   atagggtatt ttagtattgg agggtttagg gttttaattt agtaggtttt tatttgtttt    2940
   gttggttgta gagatattta gggtatatag gtgagaaatt tttttttttt tttttttttt    3000
   t                                                                    3001
<210> 499
   <211> 3001
   <212> DNA
   <213> human
<400> 499
   aaagaaaaaa aaaaaagaag agatttttta tttatatatt ttgagtgttt ttgtagttag     60
   taaagtagat gaaggtttgt tgggttggag ttttaggttt tttaatgttg aaatgttttg    120
   tgaagttttt atttatttag gtattttagt aatttgtttt ttttgtttgg aatttatttt    180
   aaaaatattt atatggttta ttttttattt tttttaagtg tattttatcg gttttttgat     240
   taagaagttt tatttttttt ttttgttttt tattaaagta gtattttttt tattgttttt    300
   tgagattgat gttagatttt tagaaggaat atattttaga tattaagtaa gaattatgaa    360
   atggataaaa gcgttgttta gtgaattgga tatagttatt gattggatat agtaattgga     420
   tataataatt gattatgtag gtaaaagatg ttttttgttg gaattagata gaattttatt     480
   tttgtattat tgttttaagg tgatttaata tttttttttt attttttaga agtattatta     540
   tgttattatt tgaagtagtt ttttatttgt ttttgattaa aagtgtatag attgatttta     600
   atgtttcgga gtgtatatat ttgaaatgta atatgataga ggttgggtac ggtggtttac     660
   gtttgtaatt ttagtatttt gagaggtcga ggcgggtaga ttatttgggg ttaggaattt     720
   gggattatta tagtgaaatt ttgtttttat tgaaaatata aaaaattagt ttggcgtggt     780
   ggcgcgcgtt tatagtttta gttatttgga aggttgaggt acgagaatcg attaattcgg     840
   gaggcggagg ttgtagtgat tttagatggc gtcgttgtat tttagtttgg gggatagagg     900
   gaaattttgt tttaaaaaaa aaaaaaaagg aataaattta tatggtaatg tttattagtt     960
   tttttattag tggatatatt tcgtgtttga atggttttat ggaatgatgt atatatttgg    1020
   attggtagtt ttagggagcg tttgataata attttagttt ttgggagttt gttgaatttt    1080
   atgaagtttg aaataatatt ttttaaatat ttgggtgatt agttgtagat ggtattttta    1140
   atatgttgtt tgattacgat ttatttaaga aagggcgaag tttttttatt gttttaggat    1200
   ttggataagt tattttttat cgtttagcgt tttggagttt cgtttttatt ttaagaggtt    1260
   ggtgtaaagg gttttcggta tttatttcgg gatttatgaa aattatatta tttagaatgt    1320
   tttgcgttga acgttacgtt attaagttag taagagttta gaaaatcgat tttttttgag    1380
   agttataaaa agaaaggacg ggatttttgg gggggttttt tcgtggcggt tattttagtt    1440
   tttttgaggt gtgtttatcg gattagagat agtagagcgt cgagttgggg ttacgaaggc    1500
   gtgaggggag tcgtcgtttt ttttgtacga aagcgtttaa gttttggcga cgtcgttttg    1560
   ggggatttac gttaggtttg ggatagggat cgttgttttc gggtttttat taatgtcgtt    1620
   cgtcgttttc ggtttagttt tattcgtaga gtttgatggt agcggttatt ttgaggacgt    1680
   taatttaggt gagtgcggcg ttttttcgtt tttatatatt tttttttatt tacgttttaa    1740
   agttattagt gaggggcgtt tgttcgagtt ttcgttgttt agggtcgggg atattgaggc    1800
   gttcgtgggt ggggtttttt ttttgatatt gcgtgtgacg aggtgtggga gagcgtgata    1860
   ggcggaggaa tcggcgcgtg taaaggttga ggcgcgattg agttaggaga attcggaaag    1920
   ttgttttttg tagggaacga agaatatgag gcggagttat ttttagggtt gtattgttat    1980
   tgtgaggatg ttagaagtta cggagagttt tgaataaagg agggacgtta tttatgttag    2040
   tttttttttt tttgagacgg agtttcgttt ttatcgttta ggttggagtg taatgacgcg    2100
   atttcgggtt attgtaattt tcgattttcg ggtttaagcg atttttttgt tttagttttt    2160
   ttagtagttg ggattataag tttgtagttg ggattataac gttcggttaa ttttttgtat    2220
   ttttagtaga gacgggattt tattattttg gttagattgg tttggaattt ttgataagtg    2280
   atttttttgt ttcggttttt taaagtgttg ggattatagg cgtgagttat cgtgttcgtt    2340
   ttatgttagg gtttaaaaat ttttttaaag attgtttata ggaagagtag cgatggggcg    2400
   atgaggatat tgagatagtg ggaggttata tttttttttt attgttttat agttggtaag    2460
   aggtgatagt attgagatta gaaagtaaaa tagttagttt gagtttaggg ttaggtaggg    2520
   ttatagggag gtttgagttt atttagtttt attagggatg tatttttttt tttttggttt    2580
   ttttagttgt agaagggcgt atggaattta tatagggaag tggagagtgt tcggtttttt    2640
   agtagttttt atttttatgt ttttgggatt gtggtttttt agtattttta agtgttgtta    2700
   ttttaatttt gggtttggtg ttttggagaa attttaagtt tagggttttt aagtttatgt    2760
   tagaggttat aggggttata ttgaggtgtt tttatttttg ttagttagtg gaaatttgtg    2820
   aaaggttatt tttggtattg gtattaggag gcgtaagtgt ttagtaaaat taagttgaga    2880
   tttttgagga aattatagat ttgttttttt ttagttggga ataaagagta gggggaagga    2940
   gttaggattg tagtggttgt ttgagaagtt gggtatttat tttggaggta atgagagatt    3000
   t                                                                    3001

<210> 500
   <211> 3001
   <212> DNA
   <213> human
<400> 500
   aaagaaaaaa aaaaaagaag agatttttta tttatatatt ttgagtgttt ttgtagttag      60
   taaagtagat gaaggtttgt tgggttggag ttttaggttt tttaatgttg aaatgttttg     120
   tgaagttttt atttatttag gtattttagt aatttgtttt ttttgtttgg aatttatttt     180
   aaaaatattt atatggttta tttttatttt tttttaagtg tattttattg gttttttgat     240
   taagaagttt tatttttttt ttttgttttt tattaaagta gtattttttt tattgttttt     300
   tgagattgat gttagatttt tagaaggaat atattttaga tattaagtaa gaattatgaa     360
   atggataaaa gtgttgttta gtgaattgga tatagttatt gattggatat agtaattgga     420
   tataataatt gattatgtag gtaaaagatg ttttttgttg gaattagata gaattttatt     480
   tttgtattat tgttttaagg tgatttaata tttttttttt attttttaga agtattatta     540
   tgttattatt tgaagtagtt ttttatttgt ttttgattaa aagtgtatag attgatttta     600
   atgttttgga gtgtatatat ttgaaatgta atatgataga ggttgggtat ggtggtttat     660
   gtttgtaatt ttagtatttt gagaggttga ggtgggtaga ttatttgggg ttaggaattt     720
   gggattatta tagtgaaatt ttgtttttat tgaaaatata aaaaattagt ttggtgtggt     780
   ggtgtgtgtt tatagtttta gttatttgga aggttgaggt atgagaattg attaatttgg     840
   gaggtggagg ttgtagtgat tttagatggt gttgttgtat tttagtttgg gggatagagg     900
   gaaattttgt tttaaaaaaa aaaaaaaagg aataaattta tatggtaatg tttattagtt     960
   tttttattag tggatatatt ttgtgtttga atggttttat ggaatgatgt atatatttgg    1020
   attggtagtt ttagggagtg tttgataata attttagttt ttgggagttt gttgaatttt    1080
   atgaagtttg aaataatatt ttttaaatat ttgggtgatt agttgtagat ggtattttta    1140
   atatgttgtt tgattatgat ttatttaaga aagggtgaag tttttttatt gttttaggat    1200
   ttggataagt tattttttat tgtttagtgt tttggagttt tgtttttatt ttaagaggtt    1260
   ggtgtaaagg gtttttggta tttattttgg gatttatgaa aattatatta tttagaatgt    1320
   tttgtgttga atgttatgtt attaagttag taagagttta gaaaattgat tttttttgag    1380
   agttataaaa agaaaggatg ggatttttgg gggggttttt ttgtggtggt tattttagtt    1440
   tttttgaggt gtgtttattg gattagagat agtagagtgt tgagttgggg ttatgaaggt    1500
   gtgaggggag ttgttgtttt ttttgtatga aagtgtttaa gttttggtga tgttgttttg    1560
   ggggatttat gttaggtttg ggatagggat tgttgttttt gggtttttat taatgttgtt    1620
   tgttgttttt ggtttagttt tatttgtaga gtttgatggt agtggttatt ttgaggatgt    1680
   taatttaggt gagtgtggtg tttttttgtt tttatatatt tttttttatt tatgttttaa    1740
   agttattagt gaggggtgtt tgtttgagtt tttgttgttt agggttgggg atattgaggt    1800
   gtttgtgggt ggggtttttt ttttgatatt gtgtgtgatg aggtgtggga gagtgtgata    1860
   ggtggaggaa ttggtgtgtg taaaggttga ggtgtgattg agttaggaga atttggaaag    1920
   ttgttttttg tagggaatga agaatatgag gtggagttat ttttagggtt gtattgttat    1980
   tgtgaggatg ttagaagtta tggagagttt tgaataaagg agggatgtta tttatgttag    2040
   tttttttttt tttgagatgg agttttgttt ttattgttta ggttggagtg taatgatgtg    2100
   attttgggtt attgtaattt ttgatttttg ggtttaagtg atttttttgt tttagttttt    2160
   ttagtagttg ggattataag tttgtagttg ggattataat gtttggttaa ttttttgtat    2220
   ttttagtaga gatgggattt tattattttg gttagattgg tttggaattt ttgataagtg    2280
   atttttttgt tttggttttt taaagtgttg ggattatagg tgtgagttat tgtgtttgtt    2340
   ttatgttagg gtttaaaaat ttttttaaag attgtttata ggaagagtag tgatggggtg    2400
   atgaggatat tgagatagtg ggaggttata tttttttttt attgttttat agttggtaag    2460
   aggtgatagt attgagatta gaaagtaaaa tagttagttt gagtttaggg ttaggtaggg    2520
   ttatagggag gtttgagttt atttagtttt attagggatg tatttttttt tttttggttt    2580
   ttttagttgt agaagggtgt atggaattta tatagggaag tggagagtgt ttggtttttt    2640
   agtagttttt atttttatgt ttttgggatt gtggtttttt agtattttta agtgttgtta    2700
   ttttaatttt gggtttggtg ttttggagaa attttaagtt tagggttttt aagtttatgt    2760
   tagaggttat aggggttata ttgaggtgtt tttatttttg ttagttagtg gaaatttgtg    2820
   aaaggttatt tttggtattg gtattaggag gtgtaagtgt ttagtaaaat taagttgaga    2880
   tttttgagga aattatagat ttgttttttt ttagttggga ataaagagta gggggaagga    2940
   gttaggattg tagtggttgt ttgagaagtt gggtatttat tttggaggta atgagagatt    3000
   t                                                                     3001
<210> 501
   <211> 50
   <212> DNA
   <213> human
<400> 501
   tagcgcctct ctgatcttcc tgcatggctc aggtggattt caattttacg 50
<210> 502
   <211> 50
   <212> DNA
   <213> human
<400> 502
   tagcgttttt ttgatttttt tgtatggttt aggtggattt taattttacg 50
<210> 503
   <211> 50
   <212> DNA
   <213> human
<400> 503
   tagtgttttt ttgatttttt tgtatggttt aggtggattt taattttatg 50
<210> 504
   <211> 50
   <212> DNA
   <213> human
<400> 504
   cgtaaaattg aaatttattt gagttatgta ggaagattag agaggcgtta 50
<210> 505
   <211> 50
   <212> DNA
   <213> human
<400> 505
   tgtaaaattg aaatttattt gagttatgta ggaagattag agaggtgtta 50
<210> 506
   <211> 3001
   <212> DNA
   <213> human
<400> 506
   aatgcagact acaatgaatg gaaggccagt gtttaatggt acttaagctt ttactgggtt      60
   attttgttga attttacatg aggcaagagc aacctgctgg ggccactgcc ttcagtatta     120
   aaagatacct atgttttgga ctttatacag gaaataattc catttcttag tatttttaaa     180
   attatggtaa aatacacata aaatttacta ggtgtatagt tcactggcag ttattaacac     240
   tgttgtacaa ccatcatcac tgtccatttt taaaaatgtt tcactatcta agcagaaact     300
   cttgtactca ttaaacaaca acctcccaac tccctcttcc cttagtccct ggtaacctct     360
   aatttcactt tctgtcccta tgcatttgcc aattctagat atctcatgta agtggaatca     420
   tacaattatt tgtccttttg tgtctggctt cactcggcat aatgttttca aggttcatcc     480
   atgttgtagc atgtatccga atcacattct ttttacaggc tgaataatat tccattccaa     540
   tatgtacata gcacatttgg tttatctctc catctgtaga tgaacatttg agttgtttcc     600
   actttttggt acttgtgaat aatgctgcta cagaacactg aagtacaagt atttgtttga     660
   gtccctgctt tcatctcttt tgggtatata cctagaagtg gaactgctag tacctatgct     720
   aagtcaatgt ttaacttgtt gaggagccac caaattattt ctattgccgc aacatcattt     780
   tacattctga tcagctatgc acaaggtttc taatttctac acatctttac taatacttat     840
   tttctgtttt tttgataaga gccattctaa tgggtatata aagcagaatc tcattaaatt     900
   agatttgctt tgtaaactat ttattaaaaa taagagacac gaagagaact tattattata     960
   tgcttcgctt accctgtata tcaagagctg caaacatcct gtgcgagtcc caaagtgaaa    1020
   ggtaaaatat ggttctatta aattttaaac atagtaacac gaatatttct ccaaataagg    1080
   taatcttgag ttttgggatt gcatttaaaa ttcctacagc aaatgctcag gatataaaca    1140
   tatccattag tcattacaga agtccacacg aggctgacac acgtagaata ccattattag    1200
   caaaactaaa ctcagtacag ttacaactgc ctcaaacttg accccaaagt attgaaagct    1260
   cctcagagag cgggccagca gcgctgcgca tgcgcgctcc cgggcgtgct cccgcgcgtg    1320
   cctgcgtatt tgcgtgcgcg gccccgcgcg gcaacgcagg ggcggaaccg catgactggc    1380
   agtggcatca gcgatggcgg ctgcgtcggg gtcggttctg cagcgctgta tcgtgtcgcc    1440
   ggcagggagg catagcgcct ctctgatctt cctgcatggc tcaggtggat ttcaatttta    1500
   cgtcctggtt ttctacagct cgggaaacat cctcccctcg gttaccccag tattgcccaa    1560
   gtggaggtgt cgccgggccc aaatagggcc cagtgggtgg ctcccccgtc gccagccccg    1620
   gctgtagatg cacgggcagc gccacctgcc cccagcctcc cccgttagtc ttcctctttc    1680
   tatcgggcgg tcactggtcc accaccctcg ctttggggcc ttgtgtcccg ccgctcagcc    1740
   agccctccat ccccacaaca cacctcccca ttcctcgccc caagtttaaa cagagcaagt    1800
   tagtaaggta agtcttctgc tagagggaaa ttatttagtg tttttcatta tcagctacta    1860
   gtgatttttc agtcgtaatt gcccattgct ttttccaggc tcccatacta atgtagacaa     1920
   ttgagattca cagttaactt agatactgtt tacagattat ctcttttctg taagccactg    1980
   tgccatggct ggagtgcttt ctctcacttt cctttttgct gcttctcttt ccttcctttc    2040
   cctttcccgc ctgtgtcatt aaacttccaa gatagactac aggggcagga cccccaggga    2100
   caggcatctt ccactacctg cccttagtaa cgtgtgctta atgctgtatc gttacttaac    2160
   actcatctgt aatcactttt gtctgtcttc cccactggat tgtgaacttc ccactggatt    2220
   atgagagtaa gggcccttgt gtcaaccttt gcacccccag tgcttggccc agggtaagct     2280
   ctcaggaaat gttttttaaa gaaatgagat gcagccggtg tcctcagggg gcctacctaa    2340
   agttaaaaac aaaaacaatc caattagttt gagtcagcag tgcatggaag atcacaagac    2400
   ggtgcatggt ttcctgccaa gtgaattgta agagaaaaaa atgtggttag gcattattaa    2460
   ggcatttgga agatgaaaag cttgtcagac ttttcgaaga aagtggtctt tgagccgaga    2520
   cgggtaggct taaatgggac ggtgattgga agggaattgg aaaggtactg ggcagagacc    2580
   tggaaaaggg caattgtggg aggtattctc tttattttga aaatgaggcc agacaggttc    2640
   agcagcttgg ccaaggtctc ttagctgtta ctgtgagtag tggcggattt gggattagaa    2700
   cccaaagctc tccgacctca aactcagggc tttggtgggg taaggtgaaa aagtttgaac    2760
   taggatatat tgttttggag atagggaaga agaggtccaa tagtgtttaa agggaaagac    2820
   ggcatatatt tagaaggtga cgtgtgtaag atgacagcgg tacttaataa ctaggagact    2880
   ggtagtacta tttagggctc caagagtgtt catttataag tacccagaaa ttccccaaca    2940
   ctgtaccctt tctcataatc aattctcatt atctttagga agaactccta gactatgttc    3000
   t                                                                    3001
<210> 507
   <211> 3001
   <212> DNA
   <213> human
<400> 507
   aatgtagatt ataatgaatg gaaggttagt gtttaatggt atttaagttt ttattgggtt 60
   attttgttga attttatatg aggtaagagt aatttgttgg ggttattgtt tttagtatta 120
   aaagatattt atgttttgga ttttatatag gaaataattt tattttttag tatttttaaa 180
   attatggtaa aatatatata aaatttatta ggtgtatagt ttattggtag ttattaatat 240
   tgttgtataa ttattattat tgtttatttt taaaaatgtt ttattattta agtagaaatt 300
   tttgtattta ttaaataata attttttaat tttttttttt tttagttttt ggtaattttt 360
   aattttattt tttgttttta tgtatttgtt aattttagat attttatgta agtggaatta 420
   tataattatt tgtttttttg tgtttggttt tattcggtat aatgttttta aggtttattt 480
   atgttgtagt atgtattcga attatatttt ttttataggt tgaataatat tttattttaa 540
   tatgtatata gtatatttgg tttatttttt tatttgtaga tgaatatttg agttgttttt 600
   attttttggt atttgtgaat aatgttgtta tagaatattg aagtataagt atttgtttga 660
   gtttttgttt ttattttttt tgggtatata tttagaagtg gaattgttag tatttatgtt 720
   aagttaatgt ttaatttgtt gaggagttat taaattattt ttattgtcgt aatattattt 780
   tatattttga ttagttatgt ataaggtttt taatttttat atatttttat taatatttat 840
   tttttgtttt tttgataaga gttattttaa tgggtatata aagtagaatt ttattaaatt 900
   agatttgttt tgtaaattat ttattaaaaa taagagatac gaagagaatt tattattata 960
   tgtttcgttt attttgtata ttaagagttg taaatatttt gtgcgagttt taaagtgaaa 1020
   ggtaaaatat ggttttatta aattttaaat atagtaatac gaatattttt ttaaataagg 1080
   taattttgag ttttgggatt gtatttaaaa tttttatagt aaatgtttag gatataaata 1140
   tatttattag ttattataga agtttatacg aggttgatat acgtagaata ttattattag 1200
   taaaattaaa tttagtatag ttataattgt tttaaatttg attttaaagt attgaaagtt 1260
   ttttagagag cgggttagta gcgttgcgta tgcgcgtttt cgggcgtgtt ttcgcgcgtg 1320
   tttgcgtatt tgcgtgcgcg gtttcgcgcg gtaacgtagg ggcggaatcg tatgattggt 1380
   agtggtatta gcgatggcgg ttgcgtcggg gtcggttttg tagcgttgta tcgtgtcgtc 1440
   ggtagggagg tatagcgttt ttttgatttt tttgtatggt ttaggtggat tttaatttta 1500
   cgttttggtt ttttatagtt cgggaaatat ttttttttcg gttattttag tattgtttaa 1560
   gtggaggtgt cgtcgggttt aaatagggtt tagtgggtgg ttttttcgtc gttagtttcg 1620
   gttgtagatg tacgggtagc gttatttgtt tttagttttt ttcgttagtt tttttttttt 1680
   tatcgggcgg ttattggttt attattttcg ttttggggtt ttgtgtttcg tcgtttagtt 1740
   agttttttat ttttataata tattttttta tttttcgttt taagtttaaa tagagtaagt 1800
   tagtaaggta agttttttgt tagagggaaa ttatttagtg ttttttatta ttagttatta 1860
   gtgatttttt agtcgtaatt gtttattgtt ttttttaggt ttttatatta atgtagataa 1920
   ttgagattta tagttaattt agatattgtt tatagattat tttttttttg taagttattg 1980
   tgttatggtt ggagtgtttt tttttatttt tttttttgtt gttttttttt tttttttttt 2040
   tttttttcgt ttgtgttatt aaatttttaa gatagattat aggggtagga tttttaggga 2100
   taggtatttt ttattatttg tttttagtaa cgtgtgttta atgttgtatc gttatttaat 2160
   atttatttgt aattattttt gtttgttttt tttattggat tgtgaatttt ttattggatt 2220
   atgagagtaa gggtttttgt gttaattttt gtatttttag tgtttggttt agggtaagtt 2280
   tttaggaaat gttttttaaa gaaatgagat gtagtcggtg tttttagggg gtttatttaa 2340
   agttaaaaat aaaaataatt taattagttt gagttagtag tgtatggaag attataagac 2400
   ggtgtatggt tttttgttaa gtgaattgta agagaaaaaa atgtggttag gtattattaa 2460
   ggtatttgga agatgaaaag tttgttagat ttttcgaaga aagtggtttt tgagtcgaga 2520
   cgggtaggtt taaatgggac ggtgattgga agggaattgg aaaggtattg ggtagagatt 2580
   tggaaaaggg taattgtggg aggtattttt tttattttga aaatgaggtt agataggttt 2640
   agtagtttgg ttaaggtttt ttagttgtta ttgtgagtag tggcggattt gggattagaa 2700
   tttaaagttt ttcgatttta aatttagggt tttggtgggg taaggtgaaa aagtttgaat 2760
   taggatatat tgttttggag atagggaaga agaggtttaa tagtgtttaa agggaaagac 2820
   ggtatatatt tagaaggtga cgtgtgtaag atgatagcgg tatttaataa ttaggagatt 2880
   ggtagtatta tttagggttt taagagtgtt tatttataag tatttagaaa ttttttaata 2940
   ttgtattttt ttttataatt aatttttatt atttttagga agaattttta gattatgttt 3000
   t 3001
<210> 508
   <211> 3001
   <212> DNA
   <213> human
<400> 508
   aatgtagatt ataatgaatg gaaggttagt gtttaatggt atttaagttt ttattgggtt      60
   attttgttga attttatatg aggtaagagt aatttgttgg ggttattgtt tttagtatta     120
   aaagatattt atgttttgga ttttatatag gaaataattt tattttttag tatttttaaa     180
   attatggtaa aatatatata aaatttatta ggtgtatagt ttattggtag ttattaatat     240
   tgttgtataa ttattattat tgtttatttt taaaaatgtt ttattattta agtagaaatt     300
   tttgtattta ttaaataata attttttaat tttttttttt tttagttttt ggtaattttt     360
   aattttattt tttgttttta tgtatttgtt aattttagat attttatgta agtggaatta     420
   tataattatt tgtttttttg tgtttggttt tatttggtat aatgttttta aggtttattt      480
   atgttgtagt atgtatttga attatatttt ttttataggt tgaataatat tttattttaa     540
   tatgtatata gtatatttgg tttatttttt tatttgtaga tgaatatttg agttgttttt      600
   attttttggt atttgtgaat aatgttgtta tagaatattg aagtataagt atttgtttga      660
   gtttttgttt ttattttttt tgggtatata tttagaagtg gaattgttag tatttatgtt     720
   aagttaatgt ttaatttgtt gaggagttat taaattattt ttattgttgt aatattattt      780
   tatattttga ttagttatgt ataaggtttt taatttttat atatttttat taatatttat      840
   tttttgtttt tttgataaga gttattttaa tgggtatata aagtagaatt ttattaaatt     900
   agatttgttt tgtaaattat ttattaaaaa taagagatat gaagagaatt tattattata     960
   tgttttgttt attttgtata ttaagagttg taaatatttt gtgtgagttt taaagtgaaa    1020
   ggtaaaatat ggttttatta aattttaaat atagtaatat gaatattttt ttaaataagg    1080
   taattttgag ttttgggatt gtatttaaaa tttttatagt aaatgtttag gatataaata    1140
   tatttattag ttattataga agtttatatg aggttgatat atgtagaata ttattattag    1200
   taaaattaaa tttagtatag ttataattgt tttaaatttg attttaaagt attgaaagtt    1260
   ttttagagag tgggttagta gtgttgtgta tgtgtgtttt tgggtgtgtt tttgtgtgtg    1320
   tttgtgtatt tgtgtgtgtg gttttgtgtg gtaatgtagg ggtggaattg tatgattggt    1380
   agtggtatta gtgatggtgg ttgtgttggg gttggttttg tagtgttgta ttgtgttgtt    1440
   ggtagggagg tatagtgttt ttttgatttt tttgtatggt ttaggtggat tttaatttta    1500
   tgttttggtt ttttatagtt tgggaaatat tttttttttg gttattttag tattgtttaa   1560
   gtggaggtgt tgttgggttt aaatagggtt tagtgggtgg tttttttgtt gttagttttg    1620
   gttgtagatg tatgggtagt gttatttgtt tttagttttt tttgttagtt tttttttttt    1680
   tattgggtgg ttattggttt attatttttg ttttggggtt ttgtgttttg ttgtttagtt   1740
   agttttttat ttttataata tattttttta ttttttgttt taagtttaaa tagagtaagt    1800
   tagtaaggta agttttttgt tagagggaaa ttatttagtg ttttttatta ttagttatta  1860
   gtgatttttt agttgtaatt gtttattgtt ttttttaggt ttttatatta atgtagataa    1920
   ttgagattta tagttaattt agatattgtt tatagattat tttttttttg taagttattg   1980
   tgttatggtt ggagtgtttt tttttatttt tttttttgtt gttttttttt tttttttttt     2040
   ttttttttgt ttgtgttatt aaatttttaa gatagattat aggggtagga tttttaggga    2100
   taggtatttt ttattatttg tttttagtaa tgtgtgttta atgttgtatt gttatttaat    2160
   atttatttgt aattattttt gtttgttttt tttattggat tgtgaatttt ttattggatt    2220
   atgagagtaa gggtttttgt gttaattttt gtatttttag tgtttggttt agggtaagtt    2280
   tttaggaaat gttttttaaa gaaatgagat gtagttggtg tttttagggg gtttatttaa   2340
   agttaaaaat aaaaataatt taattagttt gagttagtag tgtatggaag attataagat    2400
   ggtgtatggt tttttgttaa gtgaattgta agagaaaaaa atgtggttag gtattattaa    2460
   ggtatttgga agatgaaaag tttgttagat tttttgaaga aagtggtttt tgagttgaga    2520
   tgggtaggtt taaatgggat ggtgattgga agggaattgg aaaggtattg ggtagagatt    2580
   tggaaaaggg taattgtggg aggtattttt tttattttga aaatgaggtt agataggttt    2640
   agtagtttgg ttaaggtttt ttagttgtta ttgtgagtag tggtggattt gggattagaa    2700
   tttaaagttt tttgatttta aatttagggt tttggtgggg taaggtgaaa aagtttgaat    2760
   taggatatat tgttttggag atagggaaga agaggtttaa tagtgtttaa agggaaagat    2820
   ggtatatatt tagaaggtga tgtgtgtaag atgatagtgg tatttaataa ttaggagatt    2880
   ggtagtatta tttagggttt taagagtgtt tatttataag tatttagaaa ttttttaata    2940
   ttgtattttt ttttataatt aatttttatt atttttagga agaattttta gattatgttt    3000
   t                                                                    3001
<210> 509
   <211> 3001
   <212> DNA
   <213> human
<400> 509
   agaatatagt ttaggagttt tttttaaaga taatgagaat tgattatgag aaagggtata     60
   gtgttgggga atttttgggt atttataaat gaatattttt ggagttttaa atagtattat    120
   tagtttttta gttattaagt atcgttgtta ttttatatac gttatttttt aaatatatgt    180
   cgtttttttt tttaaatatt attggatttt ttttttttta tttttaaaat aatatatttt      240
   agtttaaatt tttttatttt attttattaa agttttgagt ttgaggtcgg agagttttgg    300
   gttttaattt taaattcgtt attatttata gtaatagtta agagattttg gttaagttgt    360
   tgaatttgtt tggttttatt tttaaaataa agagaatatt ttttataatt gttttttttt    420
   aggtttttgt ttagtatttt tttaattttt ttttaattat cgttttattt aagtttattc     480
   gtttcggttt aaagattatt tttttcgaaa agtttgataa gttttttatt ttttaaatgt     540
   tttaataatg tttaattata tttttttttt ttataattta tttggtagga aattatgtat    600
   cgttttgtga ttttttatgt attgttgatt taaattaatt ggattgtttt tgtttttaat    660
   tttaggtagg ttttttgagg atatcggttg tattttattt ttttaaaaaa tattttttga     720
   gagtttattt tgggttaagt attgggggtg taaaggttga tataagggtt tttattttta     780
   taatttagtg ggaagtttat aatttagtgg ggaagataga taaaagtgat tatagatgag    840
   tgttaagtaa cgatatagta ttaagtatac gttattaagg gtaggtagtg gaagatgttt    900
   gtttttgggg gttttgtttt tgtagtttat tttggaagtt taatgatata ggcgggaaag    960
   ggaaaggaag gaaagagaag tagtaaaaag gaaagtgaga gaaagtattt tagttatggt   1020
   atagtggttt atagaaaaga gataatttgt aaatagtatt taagttaatt gtgaatttta   1080
   attgtttata ttagtatggg agtttggaaa aagtaatggg taattacgat tgaaaaatta   1140
   ttagtagttg ataatgaaaa atattaaata attttttttt agtagaagat ttattttatt   1200
   aatttgtttt gtttaaattt ggggcgagga atggggaggt gtgttgtggg gatggagggt   1260
   tggttgagcg gcgggatata aggttttaaa gcgagggtgg tggattagtg atcgttcgat   1320
   agaaagagga agattaacgg gggaggttgg gggtaggtgg cgttgttcgt gtatttatag   1380
   tcggggttgg cgacggggga gttatttatt gggttttatt tgggttcggc gatattttta   1440
   tttgggtaat attggggtaa tcgaggggag gatgtttttc gagttgtaga aaattaggac   1500
   gtaaaattga aatttatttg agttatgtag gaagattaga gaggcgttat gtttttttgt   1560
   cggcgatacg atatagcgtt gtagaatcga tttcgacgta gtcgttatcg ttgatgttat   1620
   tgttagttat gcggtttcgt ttttgcgttg tcgcgcgggg tcgcgtacgt aaatacgtag   1680
   gtacgcgcgg gagtacgttc gggagcgcgt atgcgtagcg ttgttggttc gttttttgag    1740
   gagtttttaa tattttgggg ttaagtttga ggtagttgta attgtattga gtttagtttt   1800
   gttaataatg gtattttacg tgtgttagtt tcgtgtggat ttttgtaatg attaatggat   1860
   atgtttatat tttgagtatt tgttgtagga attttaaatg taattttaaa atttaagatt   1920
   attttatttg gagaaatatt cgtgttatta tgtttaaaat ttaatagaat tatattttat   1980
   tttttatttt gggattcgta taggatgttt gtagtttttg atatataggg taagcgaagt   2040
   atataataat aagttttttt cgtgtttttt atttttaata aatagtttat aaagtaaatt   2100
   taatttaatg agattttgtt ttatatattt attagaatgg tttttattaa aaaaatagaa   2160
   aataagtatt agtaaagatg tgtagaaatt agaaattttg tgtatagttg attagaatgt   2220
   aaaatgatgt tgcggtaata gaaataattt ggtggttttt taataagtta aatattgatt   2280
   tagtataggt attagtagtt ttatttttag gtatatattt aaaagagatg aaagtaggga   2340
   tttaaataaa tatttgtatt ttagtgtttt gtagtagtat tatttataag tattaaaaag   2400
   tggaaataat ttaaatgttt atttatagat ggagagataa attaaatgtg ttatgtatat   2460
   attggaatgg aatattattt agtttgtaaa aagaatgtga ttcggatata tgttataata   2520
   tggatgaatt ttgaaaatat tatgtcgagt gaagttagat ataaaaggat aaataattgt   2580
   atgattttat ttatatgaga tatttagaat tggtaaatgt atagggatag aaagtgaaat   2640
   tagaggttat tagggattaa gggaagaggg agttgggagg ttgttgttta atgagtataa   2700
   gagtttttgt ttagatagtg aaatattttt aaaaatggat agtgatgatg gttgtataat   2760
   agtgttaata attgttagtg aattatatat ttagtaaatt ttatgtgtat tttattataa   2820
   ttttaaaaat attaagaaat ggaattattt tttgtataaa gtttaaaata taggtatttt   2880
   ttaatattga aggtagtggt tttagtaggt tgtttttgtt ttatgtaaaa tttaataaaa   2940
   taatttagta aaagtttaag tattattaaa tattggtttt ttatttattg tagtttgtat   3000
   t                                                                   3001
<210> 510
   <211> 3001
   <212> DNA
   <213> human
<400> 510
   agaatatagt ttaggagttt tttttaaaga taatgagaat tgattatgag aaagggtata      60
   gtgttgggga atttttgggt atttataaat gaatattttt ggagttttaa atagtattat     120
   tagtttttta gttattaagt attgttgtta ttttatatat gttatttttt aaatatatgt     180
   tgtttttttt tttaaatatt attggatttt ttttttttta tttttaaaat aatatatttt     240
   agtttaaatt tttttatttt attttattaa agttttgagt ttgaggttgg agagttttgg     300
   gttttaattt taaatttgtt attatttata gtaatagtta agagattttg gttaagttgt     360
   tgaatttgtt tggttttatt tttaaaataa agagaatatt ttttataatt gttttttttt     420
   aggtttttgt ttagtatttt tttaattttt ttttaattat tgttttattt aagtttattt      480
   gttttggttt aaagattatt ttttttgaaa agtttgataa gttttttatt ttttaaatgt      540
   tttaataatg tttaattata tttttttttt ttataattta tttggtagga aattatgtat     600
   tgttttgtga ttttttatgt attgttgatt taaattaatt ggattgtttt tgtttttaat     660
   tttaggtagg ttttttgagg atattggttg tattttattt ttttaaaaaa tattttttga     720
   gagtttattt tgggttaagt attgggggtg taaaggttga tataagggtt tttattttta     780
   taatttagtg ggaagtttat aatttagtgg ggaagataga taaaagtgat tatagatgag     840
   tgttaagtaa tgatatagta ttaagtatat gttattaagg gtaggtagtg gaagatgttt     900
   gtttttgggg gttttgtttt tgtagtttat tttggaagtt taatgatata ggtgggaaag     960
   ggaaaggaag gaaagagaag tagtaaaaag gaaagtgaga gaaagtattt tagttatggt    1020
   atagtggttt atagaaaaga gataatttgt aaatagtatt taagttaatt gtgaatttta    1080
   attgtttata ttagtatggg agtttggaaa aagtaatggg taattatgat tgaaaaatta    1140
   ttagtagttg ataatgaaaa atattaaata attttttttt agtagaagat ttattttatt    1200
   aatttgtttt gtttaaattt ggggtgagga atggggaggt gtgttgtggg gatggagggt    1260
   tggttgagtg gtgggatata aggttttaaa gtgagggtgg tggattagtg attgtttgat    1320
   agaaagagga agattaatgg gggaggttgg gggtaggtgg tgttgtttgt gtatttatag    1380
   ttggggttgg tgatggggga gttatttatt gggttttatt tgggtttggt gatattttta    1440
   tttgggtaat attggggtaa ttgaggggag gatgtttttt gagttgtaga aaattaggat    1500
   gtaaaattga aatttatttg agttatgtag gaagattaga gaggtgttat gtttttttgt   1560
   tggtgatatg atatagtgtt gtagaattga ttttgatgta gttgttattg ttgatgttat    1620
   tgttagttat gtggttttgt ttttgtgttg ttgtgtgggg ttgtgtatgt aaatatgtag    1680
   gtatgtgtgg gagtatgttt gggagtgtgt atgtgtagtg ttgttggttt gttttttgag   1740
   gagtttttaa tattttgggg ttaagtttga ggtagttgta attgtattga gtttagtttt    1800
   gttaataatg gtattttatg tgtgttagtt ttgtgtggat ttttgtaatg attaatggat    1860
   atgtttatat tttgagtatt tgttgtagga attttaaatg taattttaaa atttaagatt    1920
   attttatttg gagaaatatt tgtgttatta tgtttaaaat ttaatagaat tatattttat    1980
   tttttatttt gggatttgta taggatgttt gtagtttttg atatataggg taagtgaagt    2040
   atataataat aagttttttt tgtgtttttt atttttaata aatagtttat aaagtaaatt    2100
   taatttaatg agattttgtt ttatatattt attagaatgg tttttattaa aaaaatagaa    2160
   aataagtatt agtaaagatg tgtagaaatt agaaattttg tgtatagttg attagaatgt    2220
   aaaatgatgt tgtggtaata gaaataattt ggtggttttt taataagtta aatattgatt    2280
   tagtataggt attagtagtt ttatttttag gtatatattt aaaagagatg aaagtaggga   2340
   tttaaataaa tatttgtatt ttagtgtttt gtagtagtat tatttataag tattaaaaag    2400
   tggaaataat ttaaatgttt atttatagat ggagagataa attaaatgtg ttatgtatat    2460
   attggaatgg aatattattt agtttgtaaa aagaatgtga tttggatata tgttataata    2520
   tggatgaatt ttgaaaatat tatgttgagt gaagttagat ataaaaggat aaataattgt    2580
   atgattttat ttatatgaga tatttagaat tggtaaatgt atagggatag aaagtgaaat    2640
   tagaggttat tagggattaa gggaagaggg agttgggagg ttgttgttta atgagtataa    2700
   gagtttttgt ttagatagtg aaatattttt aaaaatggat agtgatgatg gttgtataat    2760
   agtgttaata attgttagtg aattatatat ttagtaaatt ttatgtgtat tttattataa    2820
   ttttaaaaat attaagaaat ggaattattt tttgtataaa gtttaaaata taggtatttt    2880
   ttaatattga aggtagtggt tttagtaggt tgtttttgtt ttatgtaaaa tttaataaaa    2940
   taatttagta aaagtttaag tattattaaa tattggtttt ttatttattg tagtttgtat    3000
   t                                                                    3001
<210> 511
   <211> 50
   <212> DNA
   <213> human
<400> 511
   cagaggatcc ttgcaagaac agcgcccaga gagcattctt gacagatgcg 50
<210> 512
   <211> 50
   <212> DNA
   <213> human
<400> 512
   tagaggattt ttgtaagaat agcgtttaga gagtattttt gatagatgcg 50
<210> 513
   <211> 50
   <212> DNA
   <213> human
<400> 513
   tagaggattt ttgtaagaat agtgtttaga gagtattttt gatagatgtg 50
<210> 514
   <211> 50
   <212> DNA
   <213> human
<400> 514
   cgtatttgtt aagaatgttt tttgggcgtt gtttttgtaa ggattttttg 50
<210> 515
   <211> 50
   <212> DNA
   <213> human
<400> 515
   tgtatttgtt aagaatgttt tttgggtgtt gtttttgtaa ggattttttg 50
<210> 516
   <211> 3001
   <212> DNA
   <213> human
<400> 516
   tgaggtcagg agttcgagac cagcctgacc aacatggtaa aacgccatct ccactaaaaa 60
   tacaaaatta gccgtgtgtg gcggcacacg cctgtaatcc cagctacttg acaggctgag 120
   gcaggagaat cacttgaacc cgggaggcag aggttgcagt gggccaagat cacgccactg 180
   aactccagcc tgggcaacaa aagtgaaact ctgtctcaaa aaaataaaaa gaaagaagcc 240
   aggagtctca ggctttcagt gacttggata acttggggtg tagtctcctt cctccccttt 300
   ctaacgcctc ttgtttcctg gccactcctc ctcaacctcc ccaagttgga aaatcagctg 360
   ataaatcatg ggcagggggt gaataaacaa atcactcccc agttttaaca tactgatgct 420
   gaggtttggg cctaaagcaa attcatatgt ttgcatttat gtaagagaaa gtggttaaaa 480
   ctgcttgatt ttatataaac ttgaaaaaca ttactttttg cttttgacct ggacagctgt 540
   ggaagtcggt ctctccttgc aaggtaagga tatgagcttg gtcatattga tgaccatcaa 600
   cgaacatgat gttcgttacg ggcgctgttc ttattccaat gctagaagga cctgtgagca 660
   ttatataaca ttttcatgca cccttacata aacaattaga agctgtttac tcctcaagga 720
   ctcaattaga gattttttta atttttttat tttttttttt atttcaggcc tgagctgagg 780
   acactacagg agccaaatta gagaaggggc ttgcagtcct gccatattgc atattcatgg 840
   ctttcttgtc ccctcacccc cagtataatc ctttttgcca ggttaacaac tcactaattt 900
   caaagcaatt catttgccaa ctcaaaaaaa aaaaaaaaaa aaaccacaga tacaacttta 960
   aatgcttttg gattttaatc ctctggagta tttatatgtt ttcttgtctc atcccttcaa 1020
   aactaatctc agagttttga gaatctggga acttgggcaa aggagaaaac aagcacgcag 1080
   accaaggaat ctgaaatcgc agttcattac gtcagcataa actgacagta cctttttgtt 1140
   tgtattgacc cagctcaaat tataaaatca catgagtaat aaaacacaaa atacaggtgt 1200
   tatcattgaa aagtctaaaa tgtaattaaa cgcgtttttt ccccctcgcg gtggtatcgt 1260
   ttactctgta tctcaacgtt tctgtctccc taagcctctc ctttcatatc atgagcatac 1320
   atttttgcat catgctcaca cgttcattag ctaggactga ggagtgtgta cgattccaaa 1380
   gggccctcag cgttagctgt tagatgcaca aaccttcgct tcctttccac atctactgca 1440
   ctcgaggttc aacagaggat ccttgcaaga acagcgccca gagagcattc ttgacagatg 1500
   cgcgcttggc tccagcaacc cgctctgctg ggaagtttct tctaaccact aacacccacc 1560
   tccaatcccc caagctgtca cgacgcatgc tggctgggtc ccgtcttgac gggggaaggg 1620
   ttttacacac accctgctag gctgccccac atcacaacca agctcgcagg gcaaactcct 1680
   ccaagcctgg cggacaagct gtcccaggcg ctctggcgct tcctgaacac caaggtcccc 1740
   tccccgctca agggagctag cgctctgttc cgcagagaac cccggaactg caggtcgagg 1800
   ggatgcgggg ggagcggggg cgcaggaggg agccgagtgc tggaggcaaa cggggcgcag 1860
   gagcgggtgc gggaggcaaa cggggcgcag gagcgggtgc gggaggcaaa cggggcgcag 1920
   gagtgggtgc gggagcgagt gggggctgag ggaggggacg gggccccggg gggagccagg 1980
   cgcggaaggg ggcgcggggg aacagggacc aggaaccagc gggcgcagga aggggtgcgt 2040
   ccgcaggaac ccgcgggcgc acgggaggca ctagctacgc gatcagctcg ggactctcag 2100
   gagccgctca attgccaacg ggaggggggt gcggggagtt ggaggtgggg gtgcagacca 2160
   gacgggggcg tgcctttgcc cggattggct gcaggagcct gacgcgaggc cccgggggtt 2220
   ggcttgggga gtgggagcgg ggtggggtgg gtgctgggtg ccggagctgc gggcccggcg 2280
   cgctcagaaa catgctgaag tcccggcggc tcttccagca gcggcagcgg ctccagcagc 2340
   agcggcggcg gcggcggcgg cggcagcggc agcgacagcg ctcggctcct gcgggaaagg 2400
   cgcccggcgc ccatgcctcc ggccccgcgc cgcggctgcc ctgacccggc cgcgacctcc 2460
   ctctgcgcac cacgccgccc gggcttctgg ggtgttcccc aaccacggcc cagccctgcc 2520
   acaccccccg cccccggcct ccgcagctcg gcatgggcgc gggggtgctc gtcctgggcg 2580
   cctccgagcc cggtaacctg tcgtcggccg caccgctccc cgacggcgcg gccaccgcgg 2640
   cgcggctgct ggtgcccgcg tcgccgcccg cctcgttgct gcctcccgcc agcgaaagcc 2700
   ccgagccgct gtctcagcag tggacagcgg gcatgggtct gctgatggcg ctcatcgtgc 2760
   tgctcatcgt ggcgggcaat gtgctggtga tcgtggccat cgccaagacg ccgcggctgc 2820
   agacgctcac caacctcttc atcatgtccc tggccagcgc cgacctggtc atggggctgc 2880
   tggtggtgcc gttcggggcc accatcgtgg tgtggggccg ctgggagtac ggctccttct 2940
   tctgcgagct gtggacctca gtggacgtgc tgtgcgtgac ggccagcatc gagaccctgt 3000
   g 3001
<210> 517
   <211> 3001
   <212> DNA
   <213> human
<400> 517
   tgaggttagg agttcgagat tagtttgatt aatatggtaa aacgttattt ttattaaaaa 60
   tataaaatta gtcgtgtgtg gcggtatacg tttgtaattt tagttatttg ataggttgag 120
   gtaggagaat tatttgaatt cgggaggtag aggttgtagt gggttaagat tacgttattg 180
   aattttagtt tgggtaataa aagtgaaatt ttgttttaaa aaaataaaaa gaaagaagtt 240
   aggagtttta ggtttttagt gatttggata atttggggtg tagttttttt tttttttttt 300
   ttaacgtttt ttgttttttg gttatttttt tttaattttt ttaagttgga aaattagttg 360
   ataaattatg ggtagggggt gaataaataa attatttttt agttttaata tattgatgtt 420
   gaggtttggg tttaaagtaa atttatatgt ttgtatttat gtaagagaaa gtggttaaaa 480
   ttgtttgatt ttatataaat ttgaaaaata ttattttttg tttttgattt ggatagttgt 540
   ggaagtcggt ttttttttgt aaggtaagga tatgagtttg gttatattga tgattattaa 600
   cgaatatgat gttcgttacg ggcgttgttt ttattttaat gttagaagga tttgtgagta 660
   ttatataata tttttatgta tttttatata aataattaga agttgtttat tttttaagga 720
   tttaattaga gattttttta atttttttat tttttttttt attttaggtt tgagttgagg 780
   atattatagg agttaaatta gagaaggggt ttgtagtttt gttatattgt atatttatgg 840
   tttttttgtt tttttatttt tagtataatt ttttttgtta ggttaataat ttattaattt 900
   taaagtaatt tatttgttaa tttaaaaaaa aaaaaaaaaa aaattataga tataatttta 960
   aatgtttttg gattttaatt ttttggagta tttatatgtt tttttgtttt atttttttaa 1020
   aattaatttt agagttttga gaatttggga atttgggtaa aggagaaaat aagtacgtag 1080
   attaaggaat ttgaaatcgt agtttattac gttagtataa attgatagta tttttttgtt 1140
   tgtattgatt tagtttaaat tataaaatta tatgagtaat aaaatataaa atataggtgt 1200
   tattattgaa aagtttaaaa tgtaattaaa cgcgtttttt ttttttcgcg gtggtatcgt 1260
   ttattttgta ttttaacgtt tttgtttttt taagtttttt tttttatatt atgagtatat 1320
   atttttgtat tatgtttata cgtttattag ttaggattga ggagtgtgta cgattttaaa 1380
   gggtttttag cgttagttgt tagatgtata aattttcgtt ttttttttat atttattgta 1440
   ttcgaggttt aatagaggat ttttgtaaga atagcgttta gagagtattt ttgatagatg 1500
   cgcgtttggt tttagtaatt cgttttgttg ggaagttttt tttaattatt aatatttatt 1560
   tttaattttt taagttgtta cgacgtatgt tggttgggtt tcgttttgac gggggaaggg 1620
   ttttatatat attttgttag gttgttttat attataatta agttcgtagg gtaaattttt 1680
   ttaagtttgg cggataagtt gttttaggcg ttttggcgtt ttttgaatat taaggttttt 1740
   ttttcgttta agggagttag cgttttgttt cgtagagaat ttcggaattg taggtcgagg 1800
   ggatgcgggg ggagcggggg cgtaggaggg agtcgagtgt tggaggtaaa cggggcgtag 1860
   gagcgggtgc gggaggtaaa cggggcgtag gagcgggtgc gggaggtaaa cggggcgtag 1920
   gagtgggtgc gggagcgagt gggggttgag ggaggggacg gggtttcggg gggagttagg 1980
   cgcggaaggg ggcgcggggg aatagggatt aggaattagc gggcgtagga aggggtgcgt 2040
   tcgtaggaat tcgcgggcgt acgggaggta ttagttacgc gattagttcg ggatttttag 2100
   gagtcgttta attgttaacg ggaggggggt gcggggagtt ggaggtgggg gtgtagatta 2160
   gacgggggcg tgtttttgtt cggattggtt gtaggagttt gacgcgaggt ttcgggggtt 2220
   ggtttgggga gtgggagcgg ggtggggtgg gtgttgggtg tcggagttgc gggttcggcg 2280
   cgtttagaaa tatgttgaag tttcggcggt ttttttagta gcggtagcgg ttttagtagt 2340
   agcggcggcg gcggcggcgg cggtagcggt agcgatagcg ttcggttttt gcgggaaagg 2400
   cgttcggcgt ttatgttttc ggtttcgcgt cgcggttgtt ttgattcggt cgcgattttt 2460
   ttttgcgtat tacgtcgttc gggtttttgg ggtgtttttt aattacggtt tagttttgtt 2520
   atatttttcg ttttcggttt tcgtagttcg gtatgggcgc gggggtgttc gttttgggcg 2580
   ttttcgagtt cggtaatttg tcgtcggtcg tatcgttttt cgacggcgcg gttatcgcgg 2640
   cgcggttgtt ggtgttcgcg tcgtcgttcg tttcgttgtt gtttttcgtt agcgaaagtt 2700
   tcgagtcgtt gttttagtag tggatagcgg gtatgggttt gttgatggcg tttatcgtgt 2760
   tgtttatcgt ggcgggtaat gtgttggtga tcgtggttat cgttaagacg tcgcggttgt 2820
   agacgtttat taattttttt attatgtttt tggttagcgt cgatttggtt atggggttgt 2880
   tggtggtgtc gttcggggtt attatcgtgg tgtggggtcg ttgggagtac ggtttttttt 2940
   tttgcgagtt gtggatttta gtggacgtgt tgtgcgtgac ggttagtatc gagattttgt 3000
   g 3001
<210> 518
   <211> 3001
   <212> DNA
   <213> human
<400> 518
   tgaggttagg agtttgagat tagtttgatt aatatggtaa aatgttattt ttattaaaaa 60
   tataaaatta gttgtgtgtg gtggtatatg tttgtaattt tagttatttg ataggttgag 120
   gtaggagaat tatttgaatt tgggaggtag aggttgtagt gggttaagat tatgttattg 180
   aattttagtt tgggtaataa aagtgaaatt ttgttttaaa aaaataaaaa gaaagaagtt 240
   aggagtttta ggtttttagt gatttggata atttggggtg tagttttttt tttttttttt 300
   ttaatgtttt ttgttttttg gttatttttt tttaattttt ttaagttgga aaattagttg 360
   ataaattatg ggtagggggt gaataaataa attatttttt agttttaata tattgatgtt 420
   gaggtttggg tttaaagtaa atttatatgt ttgtatttat gtaagagaaa gtggttaaaa 480
   ttgtttgatt ttatataaat ttgaaaaata ttattttttg tttttgattt ggatagttgt 540
   ggaagttggt ttttttttgt aaggtaagga tatgagtttg gttatattga tgattattaa 600
   tgaatatgat gtttgttatg ggtgttgttt ttattttaat gttagaagga tttgtgagta 660
   ttatataata tttttatgta tttttatata aataattaga agttgtttat tttttaagga 720
   tttaattaga gattttttta atttttttat tttttttttt attttaggtt tgagttgagg 780
   atattatagg agttaaatta gagaaggggt ttgtagtttt gttatattgt atatttatgg 840
   tttttttgtt tttttatttt tagtataatt ttttttgtta ggttaataat ttattaattt 900
   taaagtaatt tatttgttaa tttaaaaaaa aaaaaaaaaa aaattataga tataatttta 960
   aatgtttttg gattttaatt ttttggagta tttatatgtt tttttgtttt atttttttaa 1020
   aattaatttt agagttttga gaatttggga atttgggtaa aggagaaaat aagtatgtag 1080
   attaaggaat ttgaaattgt agtttattat gttagtataa attgatagta tttttttgtt 1140
   tgtattgatt tagtttaaat tataaaatta tatgagtaat aaaatataaa atataggtgt 1200
   tattattgaa aagtttaaaa tgtaattaaa tgtgtttttt tttttttgtg gtggtattgt 1260
   ttattttgta ttttaatgtt tttgtttttt taagtttttt tttttatatt atgagtatat 1320
   atttttgtat tatgtttata tgtttattag ttaggattga ggagtgtgta tgattttaaa 1380
   gggtttttag tgttagttgt tagatgtata aatttttgtt ttttttttat atttattgta 1440
   tttgaggttt aatagaggat ttttgtaaga atagtgttta gagagtattt ttgatagatg 1500
   tgtgtttggt tttagtaatt tgttttgttg ggaagttttt tttaattatt aatatttatt 1560
   tttaattttt taagttgtta tgatgtatgt tggttgggtt ttgttttgat gggggaaggg 1620
   ttttatatat attttgttag gttgttttat attataatta agtttgtagg gtaaattttt 1680
   ttaagtttgg tggataagtt gttttaggtg ttttggtgtt ttttgaatat taaggttttt 1740
   tttttgttta agggagttag tgttttgttt tgtagagaat tttggaattg taggttgagg 1800
   ggatgtgggg ggagtggggg tgtaggaggg agttgagtgt tggaggtaaa tggggtgtag 1860
   gagtgggtgt gggaggtaaa tggggtgtag gagtgggtgt gggaggtaaa tggggtgtag 1920
   gagtgggtgt gggagtgagt gggggttgag ggaggggatg gggttttggg gggagttagg 1980
   tgtggaaggg ggtgtggggg aatagggatt aggaattagt gggtgtagga aggggtgtgt 2040
   ttgtaggaat ttgtgggtgt atgggaggta ttagttatgt gattagtttg ggatttttag 2100
   gagttgttta attgttaatg ggaggggggt gtggggagtt ggaggtgggg gtgtagatta 2160
   gatgggggtg tgtttttgtt tggattggtt gtaggagttt gatgtgaggt tttgggggtt 2220
   ggtttgggga gtgggagtgg ggtggggtgg gtgttgggtg ttggagttgt gggtttggtg 2280
   tgtttagaaa tatgttgaag ttttggtggt ttttttagta gtggtagtgg ttttagtagt 2340
   agtggtggtg gtggtggtgg tggtagtggt agtgatagtg tttggttttt gtgggaaagg 2400
   tgtttggtgt ttatgttttt ggttttgtgt tgtggttgtt ttgatttggt tgtgattttt 2460
   ttttgtgtat tatgttgttt gggtttttgg ggtgtttttt aattatggtt tagttttgtt 2520
   atattttttg tttttggttt ttgtagtttg gtatgggtgt gggggtgttt gttttgggtg 2580
   tttttgagtt tggtaatttg ttgttggttg tattgttttt tgatggtgtg gttattgtgg 2640
   tgtggttgtt ggtgtttgtg ttgttgtttg ttttgttgtt gttttttgtt agtgaaagtt 2700
   ttgagttgtt gttttagtag tggatagtgg gtatgggttt gttgatggtg tttattgtgt 2760
   tgtttattgt ggtgggtaat gtgttggtga ttgtggttat tgttaagatg ttgtggttgt 2820
   agatgtttat taattttttt attatgtttt tggttagtgt tgatttggtt atggggttgt 2880
   tggtggtgtt gtttggggtt attattgtgg tgtggggttg ttgggagtat ggtttttttt 2940
   tttgtgagtt gtggatttta gtggatgtgt tgtgtgtgat ggttagtatt gagattttgt 3000
   g 3001
<210> 519
   <211> 3001
   <212> DNA
   <213> human
<400> 519
   tatagggttt cgatgttggt cgttacgtat agtacgttta ttgaggttta tagttcgtag 60
   aagaaggagt cgtattttta gcggttttat attacgatgg tggtttcgaa cggtattatt 120
   agtagtttta tgattaggtc ggcgttggtt agggatatga tgaagaggtt ggtgagcgtt 180
   tgtagtcgcg gcgttttggc gatggttacg attattagta tattgttcgt tacgatgagt 240
   agtacgatga gcgttattag tagatttatg ttcgttgttt attgttgaga tagcggttcg 300
   gggttttcgt tggcgggagg tagtaacgag gcgggcggcg acgcgggtat tagtagtcgc 360
   gtcgcggtgg tcgcgtcgtc ggggagcggt gcggtcgacg ataggttatc gggttcggag 420
   gcgtttagga cgagtatttt cgcgtttatg tcgagttgcg gaggtcgggg gcggggggtg 480
   tggtagggtt gggtcgtggt tggggaatat tttagaagtt cgggcggcgt ggtgcgtaga 540
   gggaggtcgc ggtcgggtta gggtagtcgc ggcgcggggt cggaggtatg ggcgtcgggc 600
   gtttttttcg taggagtcga gcgttgtcgt tgtcgttgtc gtcgtcgtcg tcgtcgtcgt 660
   tgttgttgga gtcgttgtcg ttgttggaag agtcgtcggg attttagtat gtttttgagc 720
   gcgtcgggtt cgtagtttcg gtatttagta tttattttat ttcgttttta ttttttaagt 780
   taattttcgg ggtttcgcgt taggtttttg tagttaattc gggtaaaggt acgttttcgt 840
   ttggtttgta tttttatttt taatttttcg tatttttttt tcgttggtaa ttgagcggtt 900
   tttgagagtt tcgagttgat cgcgtagtta gtgtttttcg tgcgttcgcg ggtttttgcg 960
   gacgtatttt tttttgcgtt cgttggtttt tggtttttgt tttttcgcgt tttttttcgc 1020
   gtttggtttt tttcggggtt tcgttttttt ttttagtttt tattcgtttt cgtatttatt 1080
   tttgcgtttc gtttgttttt cgtattcgtt tttgcgtttc gtttgttttt cgtattcgtt 1140
   tttgcgtttc gtttgttttt agtattcggt ttttttttgc gttttcgttt ttttcgtatt 1200
   ttttcgattt gtagtttcgg ggttttttgc ggaatagagc gttagttttt ttgagcgggg 1260
   aggggatttt ggtgtttagg aagcgttaga gcgtttggga tagtttgttc gttaggtttg 1320
   gaggagtttg ttttgcgagt ttggttgtga tgtggggtag tttagtaggg tgtgtgtaaa 1380
   attttttttt cgttaagacg ggatttagtt agtatgcgtc gtgatagttt gggggattgg 1440
   aggtgggtgt tagtggttag aagaaatttt ttagtagagc gggttgttgg agttaagcgc 1500
   gtatttgtta agaatgtttt ttgggcgttg tttttgtaag gattttttgt tgaatttcga 1560
   gtgtagtaga tgtggaaagg aagcgaaggt ttgtgtattt aatagttaac gttgagggtt 1620
   ttttggaatc gtatatattt tttagtttta gttaatgaac gtgtgagtat gatgtaaaaa 1680
   tgtatgttta tgatatgaaa ggagaggttt agggagatag aaacgttgag atatagagta 1740
   aacgatatta tcgcgagggg gaaaaaacgc gtttaattat attttagatt ttttaatgat 1800
   aatatttgta ttttgtgttt tattatttat gtgattttat aatttgagtt gggttaatat 1860
   aaataaaaag gtattgttag tttatgttga cgtaatgaat tgcgatttta gattttttgg 1920
   tttgcgtgtt tgtttttttt tttgtttaag tttttagatt tttaaaattt tgagattagt 1980
   tttgaaggga tgagataaga aaatatataa atattttaga ggattaaaat ttaaaagtat 2040
   ttaaagttgt atttgtggtt tttttttttt ttttttttga gttggtaaat gaattgtttt 2100
   gaaattagtg agttgttaat ttggtaaaaa ggattatatt gggggtgagg ggataagaaa 2160
   gttatgaata tgtaatatgg taggattgta agtttttttt ttaatttggt ttttgtagtg 2220
   tttttagttt aggtttgaaa taaaaaaaaa aataaaaaaa ttaaaaaaat ttttaattga 2280
   gtttttgagg agtaaatagt ttttaattgt ttatgtaagg gtgtatgaaa atgttatata 2340
   atgtttatag gtttttttag tattggaata agaatagcgt tcgtaacgaa tattatgttc 2400
   gttgatggtt attaatatga ttaagtttat atttttattt tgtaaggaga gatcgatttt 2460
   tatagttgtt taggttaaaa gtaaaaagta atgtttttta agtttatata aaattaagta 2520
   gttttaatta ttttttttta tataaatgta aatatatgaa tttgttttag gtttaaattt 2580
   tagtattagt atgttaaaat tggggagtga tttgtttatt tattttttgt ttatgattta 2640
   ttagttgatt ttttaatttg gggaggttga ggaggagtgg ttaggaaata agaggcgtta 2700
   gaaaggggag gaaggagatt atattttaag ttatttaagt tattgaaagt ttgagatttt 2760
   tggttttttt tttttatttt ttttgagata gagttttatt tttgttgttt aggttggagt 2820
   ttagtggcgt gattttggtt tattgtaatt tttgtttttc gggtttaagt gatttttttg 2880
   ttttagtttg ttaagtagtt gggattatag gcgtgtgtcg ttatatacgg ttaattttgt 2940
   atttttagtg gagatggcgt tttattatgt tggttaggtt ggtttcgaat ttttgatttt 3000
   a 3001
<210> 520
   <211> 3001
   <212> DNA
   <213> human
<400> 520
   tatagggttt tgatgttggt tgttatgtat agtatgttta ttgaggttta tagtttgtag      60
   aagaaggagt tgtattttta gtggttttat attatgatgg tggttttgaa tggtattatt     120
   agtagtttta tgattaggtt ggtgttggtt agggatatga tgaagaggtt ggtgagtgtt     180
   tgtagttgtg gtgttttggt gatggttatg attattagta tattgtttgt tatgatgagt     240
   agtatgatga gtgttattag tagatttatg tttgttgttt attgttgaga tagtggtttg     300
   gggtttttgt tggtgggagg tagtaatgag gtgggtggtg atgtgggtat tagtagttgt     360
   gttgtggtgg ttgtgttgtt ggggagtggt gtggttgatg ataggttatt gggtttggag     420
   gtgtttagga tgagtatttt tgtgtttatg ttgagttgtg gaggttgggg gtggggggtg      480
   tggtagggtt gggttgtggt tggggaatat tttagaagtt tgggtggtgt ggtgtgtaga     540
   gggaggttgt ggttgggtta gggtagttgt ggtgtggggt tggaggtatg ggtgttgggt     600
   gttttttttg taggagttga gtgttgttgt tgttgttgtt gttgttgttg ttgttgttgt      660
   tgttgttgga gttgttgttg ttgttggaag agttgttggg attttagtat gtttttgagt     720
   gtgttgggtt tgtagttttg gtatttagta tttattttat tttgttttta ttttttaagt      780
   taatttttgg ggttttgtgt taggtttttg tagttaattt gggtaaaggt atgtttttgt     840
   ttggtttgta tttttatttt taattttttg tatttttttt ttgttggtaa ttgagtggtt      900
   tttgagagtt ttgagttgat tgtgtagtta gtgttttttg tgtgtttgtg ggtttttgtg      960
   gatgtatttt tttttgtgtt tgttggtttt tggtttttgt ttttttgtgt ttttttttgt      1020
   gtttggtttt ttttggggtt ttgttttttt ttttagtttt tatttgtttt tgtatttatt    1080
   tttgtgtttt gtttgttttt tgtatttgtt tttgtgtttt gtttgttttt tgtatttgtt   1140
   tttgtgtttt gtttgttttt agtatttggt ttttttttgt gtttttgttt tttttgtatt    1200
   tttttgattt gtagttttgg ggttttttgt ggaatagagt gttagttttt ttgagtgggg     1260
   aggggatttt ggtgtttagg aagtgttaga gtgtttggga tagtttgttt gttaggtttg   1320
   gaggagtttg ttttgtgagt ttggttgtga tgtggggtag tttagtaggg tgtgtgtaaa   1380
   attttttttt tgttaagatg ggatttagtt agtatgtgtt gtgatagttt gggggattgg   1440
   aggtgggtgt tagtggttag aagaaatttt ttagtagagt gggttgttgg agttaagtgt   1500
   gtatttgtta agaatgtttt ttgggtgttg tttttgtaag gattttttgt tgaattttga    1560
   gtgtagtaga tgtggaaagg aagtgaaggt ttgtgtattt aatagttaat gttgagggtt   1620
   ttttggaatt gtatatattt tttagtttta gttaatgaat gtgtgagtat gatgtaaaaa   1680
   tgtatgttta tgatatgaaa ggagaggttt agggagatag aaatgttgag atatagagta   1740
   aatgatatta ttgtgagggg gaaaaaatgt gtttaattat attttagatt ttttaatgat   1800
   aatatttgta ttttgtgttt tattatttat gtgattttat aatttgagtt gggttaatat   1860
   aaataaaaag gtattgttag tttatgttga tgtaatgaat tgtgatttta gattttttgg    1920
   tttgtgtgtt tgtttttttt tttgtttaag tttttagatt tttaaaattt tgagattagt   1980
   tttgaaggga tgagataaga aaatatataa atattttaga ggattaaaat ttaaaagtat   2040
   ttaaagttgt atttgtggtt tttttttttt ttttttttga gttggtaaat gaattgtttt   2100
   gaaattagtg agttgttaat ttggtaaaaa ggattatatt gggggtgagg ggataagaaa   2160
   gttatgaata tgtaatatgg taggattgta agtttttttt ttaatttggt ttttgtagtg    2220
   tttttagttt aggtttgaaa taaaaaaaaa aataaaaaaa ttaaaaaaat ttttaattga   2280
   gtttttgagg agtaaatagt ttttaattgt ttatgtaagg gtgtatgaaa atgttatata   2340
   atgtttatag gtttttttag tattggaata agaatagtgt ttgtaatgaa tattatgttt   2400
   gttgatggtt attaatatga ttaagtttat atttttattt tgtaaggaga gattgatttt   2460
   tatagttgtt taggttaaaa gtaaaaagta atgtttttta agtttatata aaattaagta   2520
   gttttaatta ttttttttta tataaatgta aatatatgaa tttgttttag gtttaaattt    2580
   tagtattagt atgttaaaat tggggagtga tttgtttatt tattttttgt ttatgattta   2640
   ttagttgatt ttttaatttg gggaggttga ggaggagtgg ttaggaaata agaggtgtta   2700
   gaaaggggag gaaggagatt atattttaag ttatttaagt tattgaaagt ttgagatttt   2760
   tggttttttt ttttttattt ttttgagata gagttttatt tttgttgttt aggttggagt   2820
   ttagtggtgt gattttggtt tattgtaatt tttgtttttt gggtttaagt gatttttttg   2880
   ttttagtttg ttaagtagtt gggattatag gtgtgtgttg ttatatatgg ttaattttgt   2940
   atttttagtg gagatggtgt tttattatgt tggttaggtt ggttttgaat ttttgatttt   3000
   a                                                                    3001

<210> 521
   <211> 50
   <212> DNA
   <213> human
<400> 521
   ctaagtggtt tgaagcccct tccgggatgc ctcttcttat acgaacaacg 50
<210> 522
   <211> 50
   <212> DNA
   <213> human
<400> 522
   ttaagtggtt tgaagttttt ttcgggatgt ttttttttat acgaataacg 50
<210> 523
   <211> 50
   <212> DNA
   <213> human
<400> 523
   ttaagtggtt tgaagttttt tttgggatgt ttttttttat atgaataatg 50
<210> 524
   <211> 50
   <212> DNA
   <213> human
<400> 524
   cgttgttcgt ataagaagag gtatttcgga aggggtttta aattatttag 50
<210> 525
   <211> 50
   <212> DNA
   <213> human
<400> 525
   tgttgtttgt ataagaagag gtattttgga aggggtttta aattatttag 50
<210> 526
   <211> 3001
   <212> DNA
   <213> human
<400> 526
   atgtatattt cttttctcag tcaataaaca cttcatgatg gtctatgata tacacactta 60
   tgcaattatg ttacctgttt attattactg aagatatagt gataaacaac atatagcgtt 120
   tatatacatg tgggataaat agacacatga cagttgcagt ggtccaagtt tagtacaatg 180
   gtgcaatggg agctatggga ggtacgtcca tcacagtcta gaaagggatc atcaacggag 240
   gcttctagtg gggagaaaca tggggagaga catgtaaact aaaatccgat ggaggtgtag 300
   agtagttggc ttggcaaatc ttaggaaaag gagaagcaaa gaagaggaga cagcagactc 360
   acacgtgcaa aaatgggaat ggagagagca gagagcgtgt ggggtttgca caaagaacag 420
   ttcagaacct cactgtcatg acagtatgaa gggaaagcgg gcagtactcc accacccacc 480
   aatgtagccc tcaaaatcag ctgagttgtt tgaactttac cctaacggca atatgacata 540
   atcaaaagat ttttaagtgg aggcgtgaaa aatcagaagt attttggggc tcacacttga 600
   gattggattg gggaagatac ggctaaaagt aagtgggagt aatgaggggg ctgtcccagt 660
   aattgggttc agatgacatt tgcttgattt tagggatgta cgagatattc gtggatcttg 720
   tgttgaagca cctaatacac gatgtccctt ctgaagacgg gtgcattaaa ctggggtgaa 780
   gcacaaaggc cactgagcag cctggcttga ctcctggctt tgccagtttg catttgcacg 840
   tgtccaagcc cttgagaggt gctgtgaaaa catagaggtg gatgagggaa gtgcttatac 900
   agcacatacc gtataaaaag agggcttcat acttgctagt tgttttaatt tttattatta 960
   cttttcctcg ctgcacatgg aaatgtcttt acctttgtct tattataagg tacatacgag 1020
   gagagttaca gaacatgggt gttagaagag atttgacata tctgttcatt ttcctaattt 1080
   tagtgatgag aaaataaagg gaaagaaagc aatttagatt tttgccagta ttacaattct 1140
   ttgttcacgg gcacaccacg ccagcctgaa actgttcatt ggtctggttc tcttaccacc 1200
   tcgtatatgt aggtttatag tttggttacg aacaccacct tgtcttaacc atagatagtt 1260
   cctgtgtttg ccgacagcag cgatttcttg tgtggaaacc cctaatttgg gaaacaatct 1320
   gttttaaaca ctttgtagca catcactcaa accgcacagt tttcgataat cttagaaaca 1380
   aaaactgggg ttgaggcgga cagttaaatt ctgcagacac cacaagcttg gattcgtttc 1440
   agcagtcggt cctaagtggt ttgaagcccc ttccgggatg cctcttctta tacgaacaac 1500
   gcagcggcgc caggcgccag ggtcctcact tctgcctcct ggatttcctc cgcctttctg 1560
   cttcaccgag ggccctgtga tccgattcca tcgaattaac cgctgtttac cgagggtctg 1620
   ctgcgcctaa ggaaggcacc gggcgacgag caaggcgggg acgcagtttg gggacgcagg 1680
   ggtccgggcc ccagtccaga gacaggaggt cggcgcttcg acttggcacc cgccgacgaa 1740
   ttcactccaa ggttcccact ggcggagctc ctgctacctc ccccctcccc taaatgcagc 1800
   tcccgctacc ttcccgcgcc caggggctgc tgcctgagcc cagctctgct cactggaggg 1860
   cggcggtgga gagggtgctg gcccagcgtc ggcagctggg ctcaggacca aggtgggcgc 1920
   ccgccggtcc tggtcccccc agcctcacgg tgcaggaccc agggcgcggc cactcgagtt 1980
   ccccggacgc tctccttcct gatgctcgct gtccctgccc tggcgtcccc agcccggggt 2040
   cgcagcctgt cctcctctct ctccccctcc gccgcagccc gtcgcctccg ggcagagttc 2100
   atgttcagct tgggctttcg ctcgggattt aggaagaggc cggcggggct ggagggtgcg 2160
   ggccggcgcg ggagggcgcg ggaggcgggg agcggcggcc tctgattggc cggggcgggg 2220
   gagtgagcgg agtttcccac aatgccccgg tgcggtgcag ccgcggatgg atgctgcggg 2280
   aaggggctgc catttgctgc ccctgccagc ggcgcgcgga cctgcccgcg ctcctgcagc 2340
   cgccgccgcc gccgccgcca gcccgcccgg cccctgcagc ggcgccgcct gcgctccctc 2400
   cgcggccgcc ggagcgggcg ccatgaaccc cagctcctcg gcgggagagg agaaaggggc 2460
   gacgggcggc agcagcagca gcggaagcgg cgccgggagc tgctgcctgg gcgccgaggg 2520
   cggcgcggac ccgcggggcg cagggtcagc cgcggcggcg ggggccgctg ccctggacga 2580
   gcccgcggcc gccggccaga aggagaagga cgaagcgctg gaggagaagc tgaggaactt 2640
   aactttccgg aagcaggtct cgtacaggta ggtgcgggga gccggcagcc gagacaggtg 2700
   cgccgcggcg ggagacccac tcttctaccc agcaaggggc gcttccgcgg agttgggcac 2760
   ctctctgttt ccacgcctct ccttccccct ttccccctct cctcccgggg atgtctgtct 2820
   tctccccatc ctatcattcc aggctggagg gcagcaacgc ctgctttccc ctgtggcagg 2880
   agagaagcac tgggtcccta aggtcagagc aacagaaatt ttgtctgtgt gtgtgtgtgt 2940
   gtgtgtgtgt gtgtgtgtgt gtgtgtgaga gagagagaga gagagagaga gagaaagaga 3000
   g 3001
<210> 527
   <211> 3001
   <212> DNA
   <213> human
<400> 527
<210> 528
   <211> 3001
   <212> DNA
   <213> human
<400> 528
   atgtatattt ttttttttag ttaataaata ttttatgatg gtttatgata tatatattta     60
   tgtaattatg ttatttgttt attattattg aagatatagt gataaataat atatagtgtt    120
   tatatatatg tgggataaat agatatatga tagttgtagt ggtttaagtt tagtataatg    180
   gtgtaatggg agttatggga ggtatgttta ttatagttta gaaagggatt attaatggag    240
   gtttttagtg gggagaaata tggggagaga tatgtaaatt aaaatttgat ggaggtgtag    300
   agtagttggt ttggtaaatt ttaggaaaag gagaagtaaa gaagaggaga tagtagattt    360
   atatgtgtaa aaatgggaat ggagagagta gagagtgtgt ggggtttgta taaagaatag    420
   tttagaattt tattgttatg atagtatgaa gggaaagtgg gtagtatttt attatttatt    480
   aatgtagttt ttaaaattag ttgagttgtt tgaattttat tttaatggta atatgatata    540
   attaaaagat ttttaagtgg aggtgtgaaa aattagaagt attttggggt ttatatttga    600
   gattggattg gggaagatat ggttaaaagt aagtgggagt aatgaggggg ttgttttagt    660
   aattgggttt agatgatatt tgtttgattt tagggatgta tgagatattt gtggattttg    720
   tgttgaagta tttaatatat gatgtttttt ttgaagatgg gtgtattaaa ttggggtgaa    780
   gtataaaggt tattgagtag tttggtttga tttttggttt tgttagtttg tatttgtatg    840
   tgtttaagtt tttgagaggt gttgtgaaaa tatagaggtg gatgagggaa gtgtttatat    900
   agtatatatt gtataaaaag agggttttat atttgttagt tgttttaatt tttattatta    960
   tttttttttg ttgtatatgg aaatgttttt atttttgttt tattataagg tatatatgag   1020
   gagagttata gaatatgggt gttagaagag atttgatata tttgtttatt tttttaattt   1080
   tagtgatgag aaaataaagg gaaagaaagt aatttagatt tttgttagta ttataatttt   1140
   ttgtttatgg gtatattatg ttagtttgaa attgtttatt ggtttggttt ttttattatt   1200
   ttgtatatgt aggtttatag tttggttatg aatattattt tgttttaatt atagatagtt   1260
   tttgtgtttg ttgatagtag tgattttttg tgtggaaatt tttaatttgg gaaataattt   1320
   gttttaaata ttttgtagta tattatttaa attgtatagt ttttgataat tttagaaata   1380
   aaaattgggg ttgaggtgga tagttaaatt ttgtagatat tataagtttg gatttgtttt   1440
   agtagttggt tttaagtggt ttgaagtttt ttttgggatg ttttttttta tatgaataat   1500
   gtagtggtgt taggtgttag ggtttttatt tttgtttttt ggattttttt tgtttttttg   1560
   ttttattgag ggttttgtga tttgatttta ttgaattaat tgttgtttat tgagggtttg   1620
   ttgtgtttaa ggaaggtatt gggtgatgag taaggtgggg atgtagtttg gggatgtagg   1680
   ggtttgggtt ttagtttaga gataggaggt tggtgttttg atttggtatt tgttgatgaa   1740
   tttattttaa ggtttttatt ggtggagttt ttgttatttt tttttttttt taaatgtagt   1800
   ttttgttatt tttttgtgtt taggggttgt tgtttgagtt tagttttgtt tattggaggg   1860
   tggtggtgga gagggtgttg gtttagtgtt ggtagttggg tttaggatta aggtgggtgt   1920
   ttgttggttt tggttttttt agttttatgg tgtaggattt agggtgtggt tatttgagtt   1980
   ttttggatgt tttttttttt gatgtttgtt gtttttgttt tggtgttttt agtttggggt   2040
   tgtagtttgt tttttttttt tttttttttt gttgtagttt gttgtttttg ggtagagttt   2100
   atgtttagtt tgggtttttg tttgggattt aggaagaggt tggtggggtt ggagggtgtg   2160
   ggttggtgtg ggagggtgtg ggaggtgggg agtggtggtt tttgattggt tggggtgggg   2220
   gagtgagtgg agttttttat aatgttttgg tgtggtgtag ttgtggatgg atgttgtggg   2280
   aaggggttgt tatttgttgt ttttgttagt ggtgtgtgga tttgtttgtg tttttgtagt   2340
   tgttgttgtt gttgttgtta gtttgtttgg tttttgtagt ggtgttgttt gtgttttttt   2400
   tgtggttgtt ggagtgggtg ttatgaattt tagttttttg gtgggagagg agaaaggggt   2460
   gatgggtggt agtagtagta gtggaagtgg tgttgggagt tgttgtttgg gtgttgaggg   2520
   tggtgtggat ttgtggggtg tagggttagt tgtggtggtg ggggttgttg ttttggatga   2580
   gtttgtggtt gttggttaga aggagaagga tgaagtgttg gaggagaagt tgaggaattt   2640
   aattttttgg aagtaggttt tgtataggta ggtgtgggga gttggtagtt gagataggtg   2700
   tgttgtggtg ggagatttat ttttttattt agtaaggggt gtttttgtgg agttgggtat   2760
   ttttttgttt ttatgttttt tttttttttt tttttttttt ttttttgggg atgtttgttt   2820
   tttttttatt ttattatttt aggttggagg gtagtaatgt ttgttttttt ttgtggtagg   2880
   agagaagtat tgggttttta aggttagagt aatagaaatt ttgtttgtgt gtgtgtgtgt   2940
   gtgtgtgtgt gtgtgtgtgt gtgtgtgaga gagagagaga gagagagaga gagaaagaga   3000
   g                                                                   3001
<210> 529
   <211> 3001
   <212> DNA
   <213> human
<400> 529
   tttttttttt tttttttttt tttttttttt tttttatata tatatatata tatatatata 60
   tatatatata tatatagata aaatttttgt tgttttgatt ttagggattt agtgtttttt 120
   ttttgttata ggggaaagta ggcgttgttg ttttttagtt tggaatgata ggatggggag 180
   aagatagata ttttcgggag gagaggggga aagggggaag gagaggcgtg gaaatagaga 240
   ggtgtttaat ttcgcggaag cgttttttgt tgggtagaag agtgggtttt tcgtcgcggc 300
   gtatttgttt cggttgtcgg tttttcgtat ttatttgtac gagatttgtt ttcggaaagt 360
   taagtttttt agtttttttt ttagcgtttc gttttttttt ttttggtcgg cggtcgcggg 420
   ttcgtttagg gtagcggttt tcgtcgtcgc ggttgatttt gcgtttcgcg ggttcgcgtc 480
   gttttcggcg tttaggtagt agttttcggc gtcgttttcg ttgttgttgt tgtcgttcgt 540
   cgtttttttt ttttttttcg tcgaggagtt ggggtttatg gcgttcgttt cggcggtcgc 600
   ggagggagcg taggcggcgt cgttgtaggg gtcgggcggg ttggcggcgg cggcggcggc 660
   ggttgtagga gcgcgggtag gttcgcgcgt cgttggtagg ggtagtaaat ggtagttttt 720
   tttcgtagta tttattcgcg gttgtatcgt atcggggtat tgtgggaaat ttcgtttatt 780
   ttttcgtttc ggttaattag aggtcgtcgt ttttcgtttt tcgcgttttt tcgcgtcggt 840
   tcgtattttt tagtttcgtc ggtttttttt taaatttcga gcgaaagttt aagttgaata 900
   tgaattttgt tcggaggcga cgggttgcgg cggaggggga gagagaggag gataggttgc 960
   gatttcgggt tggggacgtt agggtaggga tagcgagtat taggaaggag agcgttcggg 1020
   gaattcgagt ggtcgcgttt tgggttttgt atcgtgaggt tggggggatt aggatcggcg 1080
   ggcgtttatt ttggttttga gtttagttgt cgacgttggg ttagtatttt ttttatcgtc 1140
   gttttttagt gagtagagtt gggtttaggt agtagttttt gggcgcggga aggtagcggg 1200
   agttgtattt aggggagggg ggaggtagta ggagtttcgt tagtgggaat tttggagtga 1260
   attcgtcggc gggtgttaag tcgaagcgtc gattttttgt ttttggattg gggttcggat 1320
   ttttgcgttt ttaaattgcg ttttcgtttt gttcgtcgtt cggtgttttt tttaggcgta 1380
   gtagattttc ggtaaatagc ggttaattcg atggaatcgg attatagggt tttcggtgaa 1440
   gtagaaaggc ggaggaaatt taggaggtag aagtgaggat tttggcgttt ggcgtcgttg 1500
   cgttgttcgt ataagaagag gtatttcgga aggggtttta aattatttag gatcgattgt 1560
   tgaaacgaat ttaagtttgt ggtgtttgta gaatttaatt gttcgtttta attttagttt 1620
   ttgtttttaa gattatcgaa aattgtgcgg tttgagtgat gtgttataaa gtgtttaaaa 1680
   tagattgttt tttaaattag gggtttttat ataagaaatc gttgttgtcg gtaaatatag 1740
   gaattattta tggttaagat aaggtggtgt tcgtaattaa attataaatt tatatatacg 1800
   aggtggtaag agaattagat taatgaatag ttttaggttg gcgtggtgtg ttcgtgaata 1860
   aagaattgta atattggtaa aaatttaaat tgtttttttt ttttttattt ttttattatt 1920
   aaaattagga aaatgaatag atatgttaaa ttttttttaa tatttatgtt ttgtaatttt 1980
   tttcgtatgt attttataat aagataaagg taaagatatt tttatgtgta gcgaggaaaa 2040
   gtaataataa aaattaaaat aattagtaag tatgaagttt tttttttata cggtatgtgt 2100
   tgtataagta ttttttttat ttatttttat gtttttatag tattttttaa gggtttggat 2160
   acgtgtaaat gtaaattggt aaagttagga gttaagttag gttgtttagt ggtttttgtg 2220
   ttttatttta gtttaatgta ttcgttttta gaagggatat cgtgtattag gtgttttaat 2280
   ataagattta cgaatatttc gtatattttt aaaattaagt aaatgttatt tgaatttaat 2340
   tattgggata gtttttttat tatttttatt tatttttagt cgtatttttt ttaatttaat 2400
   tttaagtgtg agttttaaaa tatttttgat tttttacgtt tttatttaaa aattttttga 2460
   ttatgttata ttgtcgttag ggtaaagttt aaataattta gttgattttg agggttatat 2520
   tggtgggtgg tggagtattg ttcgtttttt ttttatattg ttatgatagt gaggttttga 2580
   attgtttttt gtgtaaattt tatacgtttt ttgttttttt tatttttatt tttgtacgtg 2640
   tgagtttgtt gttttttttt ttttgttttt ttttttttta agatttgtta agttaattat 2700
   tttatatttt tatcggattt tagtttatat gttttttttt atgttttttt ttattagaag 2760
   ttttcgttga tgattttttt ttagattgtg atggacgtat tttttatagt ttttattgta 2820
   ttattgtatt aaatttggat tattgtaatt gttatgtgtt tatttatttt atatgtatat 2880
   aaacgttata tgttgtttat tattatattt ttagtaataa taaataggta atataattgt 2940
   ataagtgtgt atattataga ttattatgaa gtgtttattg attgagaaaa gaaatatata 3000
   t 3001
<210> 530
   <211> 3001
   <212> DNA
   <213> human
<400> 530
   tttttttttt tttttttttt tttttttttt tttttatata tatatatata tatatatata     60
   tatatatata tatatagata aaatttttgt tgttttgatt ttagggattt agtgtttttt    120
   ttttgttata ggggaaagta ggtgttgttg ttttttagtt tggaatgata ggatggggag    180
   aagatagata tttttgggag gagaggggga aagggggaag gagaggtgtg gaaatagaga    240
   ggtgtttaat tttgtggaag tgttttttgt tgggtagaag agtgggtttt ttgttgtggt    300
   gtatttgttt tggttgttgg ttttttgtat ttatttgtat gagatttgtt tttggaaagt    360
   taagtttttt agtttttttt ttagtgtttt gttttttttt ttttggttgg tggttgtggg    420
   tttgtttagg gtagtggttt ttgttgttgt ggttgatttt gtgttttgtg ggtttgtgtt    480
   gtttttggtg tttaggtagt agtttttggt gttgtttttg ttgttgttgt tgttgtttgt    540
   tgtttttttt tttttttttg ttgaggagtt ggggtttatg gtgtttgttt tggtggttgt    600
   ggagggagtg taggtggtgt tgttgtaggg gttgggtggg ttggtggtgg tggtggtggt    660
   ggttgtagga gtgtgggtag gtttgtgtgt tgttggtagg ggtagtaaat ggtagttttt    720
   ttttgtagta tttatttgtg gttgtattgt attggggtat tgtgggaaat tttgtttatt    780
   tttttgtttt ggttaattag aggttgttgt tttttgtttt ttgtgttttt ttgtgttggt    840
   ttgtattttt tagttttgtt ggtttttttt taaattttga gtgaaagttt aagttgaata    900
   tgaattttgt ttggaggtga tgggttgtgg tggaggggga gagagaggag gataggttgt    960
   gattttgggt tggggatgtt agggtaggga tagtgagtat taggaaggag agtgtttggg   1020
   gaatttgagt ggttgtgttt tgggttttgt attgtgaggt tggggggatt aggattggtg   1080
   ggtgtttatt ttggttttga gtttagttgt tgatgttggg ttagtatttt ttttattgtt   1140
   gttttttagt gagtagagtt gggtttaggt agtagttttt gggtgtggga aggtagtggg   1200
   agttgtattt aggggagggg ggaggtagta ggagttttgt tagtgggaat tttggagtga   1260
   atttgttggt gggtgttaag ttgaagtgtt gattttttgt ttttggattg gggtttggat   1320
   ttttgtgttt ttaaattgtg tttttgtttt gtttgttgtt tggtgttttt tttaggtgta   1380
   gtagattttt ggtaaatagt ggttaatttg atggaattgg attatagggt ttttggtgaa   1440
   gtagaaaggt ggaggaaatt taggaggtag aagtgaggat tttggtgttt ggtgttgttg   1500
   tgttgtttgt ataagaagag gtattttgga aggggtttta aattatttag gattgattgt   1560
   tgaaatgaat ttaagtttgt ggtgtttgta gaatttaatt gtttgtttta attttagttt   1620
   ttgtttttaa gattattgaa aattgtgtgg tttgagtgat gtgttataaa gtgtttaaaa   1680
   tagattgttt tttaaattag gggtttttat ataagaaatt gttgttgttg gtaaatatag   1740
   gaattattta tggttaagat aaggtggtgt ttgtaattaa attataaatt tatatatatg   1800
   aggtggtaag agaattagat taatgaatag ttttaggttg gtgtggtgtg tttgtgaata   1860
   aagaattgta atattggtaa aaatttaaat tgtttttttt ttttttattt ttttattatt   1920
   aaaattagga aaatgaatag atatgttaaa ttttttttaa tatttatgtt ttgtaatttt   1980
   ttttgtatgt attttataat aagataaagg taaagatatt tttatgtgta gtgaggaaaa   2040
   gtaataataa aaattaaaat aattagtaag tatgaagttt tttttttata tggtatgtgt   2100
   tgtataagta ttttttttat ttatttttat gtttttatag tattttttaa gggtttggat   2160
   atgtgtaaat gtaaattggt aaagttagga gttaagttag gttgtttagt ggtttttgtg   2220
   ttttatttta gtttaatgta tttgttttta gaagggatat tgtgtattag gtgttttaat   2280
   ataagattta tgaatatttt gtatattttt aaaattaagt aaatgttatt tgaatttaat   2340
   tattgggata gtttttttat tatttttatt tatttttagt tgtatttttt ttaatttaat   2400
   tttaagtgtg agttttaaaa tatttttgat tttttatgtt tttatttaaa aattttttga   2460
   ttatgttata ttgttgttag ggtaaagttt aaataattta gttgattttg agggttatat   2520
   tggtgggtgg tggagtattg tttgtttttt ttttatattg ttatgatagt gaggttttga   2580
   attgtttttt gtgtaaattt tatatgtttt ttgttttttt tatttttatt tttgtatgtg   2640
   tgagtttgtt gttttttttt ttttgttttt ttttttttta agatttgtta agttaattat   2700
   tttatatttt tattggattt tagtttatat gttttttttt atgttttttt ttattagaag   2760
   tttttgttga tgattttttt ttagattgtg atggatgtat tttttatagt ttttattgta   2820
   ttattgtatt aaatttggat tattgtaatt gttatgtgtt tatttatttt atatgtatat   2880
   aaatgttata tgttgtttat tattatattt ttagtaataa taaataggta atataattgt   2940
   ataagtgtgt atattataga ttattatgaa gtgtttattg attgagaaaa gaaatatata   3000
   t                                                                   3001
<210> 531
   <211> 1571
   <212> DNA
   <213> human
<400> 531
<210> 532
   <211> 1571
   <212> DNA
   <213> human
<400> 532
   taggggaaag taggcgttgt tgttttttag tttggaatga taggatgggg agaagataga 60
   tattttcggg aggagagggg gaaaggggga aggagaggcg tggaaataga gaggtgttta 120
   atttcgcgga agcgtttttt gttgggtaga agagtgggtt tttcgtcgcg gcgtatttgt 180
   ttcggttgtc ggtttttcgt atttatttgt acgagatttg ttttcggaaa gttaagtttt 240
   ttagtttttt ttttagcgtt tcgttttttt ttttttggtc ggcggtcgcg ggttcgttta 300
   gggtagcggt tttcgtcgtc gcggttgatt ttgcgtttcg cgggttcgcg tcgttttcgg 360
   cgtttaggta gtagttttcg gcgtcgtttt cgttgttgtt gttgtcgttc gtcgtttttt 420
   tttttttttt cgtcgaggag ttggggttta tggcgttcgt ttcggcggtc gcggagggag 480
   cgtaggcggc gtcgttgtag gggtcgggcg ggttggcggc ggcggcggcg gcggttgtag 540
   gagcgcgggt aggttcgcgc gtcgttggta ggggtagtaa atggtagttt tttttcgtag 600
   tatttattcg cggttgtatc gtatcggggt attgtgggaa atttcgttta ttttttcgtt 660
   tcggttaatt agaggtcgtc gtttttcgtt tttcgcgttt tttcgcgtcg gttcgtattt 720
   tttagtttcg tcggtttttt tttaaatttc gagcgaaagt ttaagttgaa tatgaatttt 780
   gttcggaggc gacgggttgc ggcggagggg gagagagagg aggataggtt gcgatttcgg 840
   gttggggacg ttagggtagg gatagcgagt attaggaagg agagcgttcg gggaattcga 900
   gtggtcgcgt tttgggtttt gtatcgtgag gttgggggga ttaggatcgg cgggcgttta 960
   ttttggtttt gagtttagtt gtcgacgttg ggttagtatt ttttttatcg tcgtttttta 1020
   gtgagtagag ttgggtttag gtagtagttt ttgggcgcgg gaaggtagcg ggagttgtat 1080
   ttaggggagg ggggaggtag taggagtttc gttagtggga attttggagt gaattcgtcg 1140
   gcgggtgtta agtcgaagcg tcgatttttt gtttttggat tggggttcgg atttttgcgt 1200
   ttttaaattg cgttttcgtt ttgttcgtcg ttcggtgttt tttttaggcg tagtagattt 1260
   tcggtaaata gcggttaatt cgatggaatc ggattatagg gttttcggtg aagtagaaag 1320
   gcggaggaaa tttaggaggt agaagtgagg attttggcgt ttggcgtcgt tgcgttgttc 1380
   gtataagaag aggtatttcg gaaggggttt taaattattt aggatcgatt gttgaaacga 1440
   atttaagttt gtggtgtttg tagaatttaa ttgttcgttt taattttagt ttttgttttt 1500
   aagattatcg aaaattgtgc ggtttgagtg atgtgttata aagtgtttaa aatagattgt 1560
   tttttaaatt a 1571
<210> 533
   <211> 1571
   <212> DNA
   <213> human
<400> 533
   taggggaaag taggtgttgt tgttttttag tttggaatga taggatgggg agaagataga      60
   tatttttggg aggagagggg gaaaggggga aggagaggtg tggaaataga gaggtgttta     120
   attttgtgga agtgtttttt gttgggtaga agagtgggtt ttttgttgtg gtgtatttgt     180
   tttggttgtt ggttttttgt atttatttgt atgagatttg tttttggaaa gttaagtttt     240
   ttagtttttt ttttagtgtt ttgttttttt ttttttggtt ggtggttgtg ggtttgttta     300
   gggtagtggt ttttgttgtt gtggttgatt ttgtgttttg tgggtttgtg ttgtttttgg     360
   tgtttaggta gtagtttttg gtgttgtttt tgttgttgtt gttgttgttt gttgtttttt      420
   tttttttttt tgttgaggag ttggggttta tggtgtttgt tttggtggtt gtggagggag     480
   tgtaggtggt gttgttgtag gggttgggtg ggttggtggt ggtggtggtg gtggttgtag     540
   gagtgtgggt aggtttgtgt gttgttggta ggggtagtaa atggtagttt ttttttgtag     600
   tatttatttg tggttgtatt gtattggggt attgtgggaa attttgttta tttttttgtt     660
   ttggttaatt agaggttgtt gttttttgtt ttttgtgttt ttttgtgttg gtttgtattt    720
   tttagttttg ttggtttttt tttaaatttt gagtgaaagt ttaagttgaa tatgaatttt     780
   gtttggaggt gatgggttgt ggtggagggg gagagagagg aggataggtt gtgattttgg     840
   gttggggatg ttagggtagg gatagtgagt attaggaagg agagtgtttg gggaatttga     900
   gtggttgtgt tttgggtttt gtattgtgag gttgggggga ttaggattgg tgggtgttta     960
   ttttggtttt gagtttagtt gttgatgttg ggttagtatt ttttttattg ttgtttttta    1020
   gtgagtagag ttgggtttag gtagtagttt ttgggtgtgg gaaggtagtg ggagttgtat    1080
   ttaggggagg ggggaggtag taggagtttt gttagtggga attttggagt gaatttgttg    1140
   gtgggtgtta agttgaagtg ttgatttttt gtttttggat tggggtttgg atttttgtgt    1200
   ttttaaattg tgtttttgtt ttgtttgttg tttggtgttt tttttaggtg tagtagattt    1260
   ttggtaaata gtggttaatt tgatggaatt ggattatagg gtttttggtg aagtagaaag    1320
   gtggaggaaa tttaggaggt agaagtgagg attttggtgt ttggtgttgt tgtgttgttt    1380
   gtataagaag aggtattttg gaaggggttt taaattattt aggattgatt gttgaaatga    1440
   atttaagttt gtggtgtttg tagaatttaa ttgtttgttt taattttagt ttttgttttt    1500
   aagattattg aaaattgtgt ggtttgagtg atgtgttata aagtgtttaa aatagattgt    1560
   tttttaaatt a                                                         1571
<210> 534
   <211> 1571
   <212> DNA
   <213> human
<400> 534
   taatttggga aataatttgt tttaaatatt ttgtagtata ttatttaaat cgtatagttt      60
   tcgataattt tagaaataaa aattggggtt gaggcggata gttaaatttt gtagatatta     120
   taagtttgga ttcgttttag tagtcggttt taagtggttt gaagtttttt tcgggatgtt     180
   tttttttata cgaataacgt agcggcgtta ggcgttaggg tttttatttt tgttttttgg    240
   atttttttcg tttttttgtt ttatcgaggg ttttgtgatt cgattttatc gaattaatcg   300
   ttgtttatcg agggtttgtt gcgtttaagg aaggtatcgg gcgacgagta aggcggggac     360
   gtagtttggg gacgtagggg ttcgggtttt agtttagaga taggaggtcg gcgtttcgat     420
   ttggtattcg tcgacgaatt tattttaagg tttttattgg cggagttttt gttatttttt     480
   ttttttttta aatgtagttt tcgttatttt ttcgcgttta ggggttgttg tttgagttta     540
   gttttgttta ttggagggcg gcggtggaga gggtgttggt ttagcgtcgg tagttgggtt     600
   taggattaag gtgggcgttc gtcggttttg gtttttttag ttttacggtg taggatttag     660
   ggcgcggtta ttcgagtttt tcggacgttt ttttttttga tgttcgttgt ttttgttttg     720
   gcgtttttag ttcggggtcg tagtttgttt tttttttttt tttttttcgt cgtagttcgt     780
   cgttttcggg tagagtttat gtttagtttg ggttttcgtt cgggatttag gaagaggtcg     840
   gcggggttgg agggtgcggg tcggcgcggg agggcgcggg aggcggggag cggcggtttt     900
   tgattggtcg gggcggggga gtgagcggag ttttttataa tgtttcggtg cggtgtagtc    960
   gcggatggat gttgcgggaa ggggttgtta tttgttgttt ttgttagcgg cgcgcggatt    1020
   tgttcgcgtt tttgtagtcg tcgtcgtcgt cgtcgttagt tcgttcggtt tttgtagcgg    1080
   cgtcgtttgc gtttttttcg cggtcgtcgg agcgggcgtt atgaatttta gtttttcggc   1140
   gggagaggag aaaggggcga cgggcggtag tagtagtagc ggaagcggcg tcgggagttg    1200
   ttgtttgggc gtcgagggcg gcgcggattc gcggggcgta gggttagtcg cggcggcggg    1260
   ggtcgttgtt ttggacgagt tcgcggtcgt cggttagaag gagaaggacg aagcgttgga    1320
   ggagaagttg aggaatttaa tttttcggaa gtaggtttcg tataggtagg tgcggggagt    1380
   cggtagtcga gataggtgcg tcgcggcggg agatttattt ttttatttag taaggggcgt    1440
   tttcgcggag ttgggtattt ttttgttttt acgttttttt tttttttttt tttttttttt    1500
   tttcggggat gtttgttttt tttttatttt attattttag gttggagggt agtaacgttt    1560
   gttttttttt g                                                         1571
<210> 535
   <211> 1571
   <212> DNA
   <213> human
<400> 535
   taatttggga aataatttgt tttaaatatt ttgtagtata ttatttaaat tgtatagttt      60
   ttgataattt tagaaataaa aattggggtt gaggtggata gttaaatttt gtagatatta     120
   taagtttgga tttgttttag tagttggttt taagtggttt gaagtttttt ttgggatgtt     180
   tttttttata tgaataatgt agtggtgtta ggtgttaggg tttttatttt tgttttttgg    240
   attttttttg tttttttgtt ttattgaggg ttttgtgatt tgattttatt gaattaattg     300
   ttgtttattg agggtttgtt gtgtttaagg aaggtattgg gtgatgagta aggtggggat     360
   gtagtttggg gatgtagggg tttgggtttt agtttagaga taggaggttg gtgttttgat     420
   ttggtatttg ttgatgaatt tattttaagg tttttattgg tggagttttt gttatttttt     480
   ttttttttta aatgtagttt ttgttatttt tttgtgttta ggggttgttg tttgagttta     540
   gttttgttta ttggagggtg gtggtggaga gggtgttggt ttagtgttgg tagttgggtt     600
   taggattaag gtgggtgttt gttggttttg gtttttttag ttttatggtg taggatttag    660
   ggtgtggtta tttgagtttt ttggatgttt ttttttttga tgtttgttgt ttttgttttg      720
   gtgtttttag tttggggttg tagtttgttt tttttttttt ttttttttgt tgtagtttgt    780
   tgtttttggg tagagtttat gtttagtttg ggtttttgtt tgggatttag gaagaggttg     840
   gtggggttgg agggtgtggg ttggtgtggg agggtgtggg aggtggggag tggtggtttt     900
   tgattggttg gggtggggga gtgagtggag ttttttataa tgttttggtg tggtgtagtt      960
   gtggatggat gttgtgggaa ggggttgtta tttgttgttt ttgttagtgg tgtgtggatt    1020
   tgtttgtgtt tttgtagttg ttgttgttgt tgttgttagt ttgtttggtt tttgtagtgg    1080
   tgttgtttgt gttttttttg tggttgttgg agtgggtgtt atgaatttta gttttttggt   1140
   gggagaggag aaaggggtga tgggtggtag tagtagtagt ggaagtggtg ttgggagttg    1200
   ttgtttgggt gttgagggtg gtgtggattt gtggggtgta gggttagttg tggtggtggg    1260
   ggttgttgtt ttggatgagt ttgtggttgt tggttagaag gagaaggatg aagtgttgga    1320
   ggagaagttg aggaatttaa ttttttggaa gtaggttttg tataggtagg tgtggggagt     1380
   tggtagttga gataggtgtg ttgtggtggg agatttattt ttttatttag taaggggtgt    1440
   ttttgtggag ttgggtattt ttttgttttt atgttttttt tttttttttt tttttttttt   1500
   ttttggggat gtttgttttt tttttatttt attattttag gttggagggt agtaatgttt    1560
   gttttttttt g                                                         1571
<210> 536
   <211> 50
   <212> DNA
   <213> human
<400> 536
   gcatctacaa agactttcat cacagatgat ttcctctcag caagaaaacg 50
<210> 537
   <211> 50
   <212> DNA
   <213> human
<400> 537
   gtatttataa agatttttat tatagatgat ttttttttag taagaaaacg 50
<210> 538
   <211> 50
   <212> DNA
   <213> human
<400> 538
   gtatttataa agatttttat tatagatgat ttttttttag taagaaaatg 50
<210> 539
   <211> 50
   <212> DNA
   <213> human
<400> 539
   cgtttttttg ttgagaggaa attatttgtg atgaaagttt ttgtagatgt 50
<210> 540
   <211> 50
   <212> DNA
   <213> human
<400> 540
   tgtttttttg ttgagaggaa attatttgtg atgaaagttt ttgtagatgt 50
<210> 541
   <211> 3001
   <212> DNA
   <213> human
<400> 541
   aatgccttgt acttcacaac cctttgctcc ccagggagct ctgcaagtcc ccagaaatac 60

<210> 542
   <211> 3001
   <212> DNA
   <213> human
<400> 542
   aatgttttgt attttataat tttttgtttt ttagggagtt ttgtaagttt ttagaaatat 60
   ttgtttaatt acgggtattt ttggaagggt ttagttgttg attattagta ttatgtgatt 120
   tttagtgtta gtgatatttg gaggaattga gtaatggttg aggtggaatt ttatttggta 180
   tttaatgtaa ttttttgttt ttgttagagg cggtgaattt tatataaagt tgttaaattt 240
   ggtcgttttt ttattgagtt ttgatttttt ttatttaagg gtaaggtaat ggattgttgt 300
   gtatgattta cgcgtagtaa ttgtggtatt ggggtttagt ttagttgagt tgagtagagt 360
   aggtcggttt ggcggaagtg ggtttttttc gttttaattt ttggttttga tgagtagtta 420
   tataaattga gataggatat agatatattt tgtttgagtt agtagtaaga aatagtcgtt 480
   ttttattttt tttgtagttt tcgtatagta gggattttgt tataaattaa ttcgaagttt 540
   aatgtggaat ggtttgtaag taagggttgt atcgttgttt gttttttaag gtaagtgttg 600
   aaggtttgaa atttgagttt tggagaattg tttgggttgt tgggttgggt tgggtacggg 660
   ggcgggggtt aggtaagttt atttttttta gatatgagaa tgttttttag ttttattttt 720
   ttagtgattg aattggaata atgatagtgg tttttgtttt tttaatttag gatttgtcgt 780
   atatatatat ataaatatat gtttaagtta cgtgtttcgg gaattatagt tttatgtgta 840
   attatataaa tatgtttagt acggatttag tatttaggat gtgttagatt tcgtgtcggg 900
   atatttatac gtatttcgtg ttgttggatg agttattttt tgttattatt tttatttttt 960
   tattttgtag atgaggaaat tggagttaag agaggttgtt ttgtttatag gtagtgtaga 1020
   tgattttagt gtagattgag gggtttcgtt tttaagtatt attgatgttt gcgtattttt 1080
   aggttgacgt aagtataatt ataggtatat ggagtgtcgt gtatatacgt agatcgaagt 1140
   agtatttttt tacgttaagt tttaagttta ggcggggtga ggggtgaggt tttataattt 1200
   tttggatttt aaagatttat aaaagagttg gtgtttttaa tatttatatt attattatat 1260
   taataaggta ttattgttat atgataggtt tagggtattt tttttttgat agattttaag 1320
   aattagatcg cggtagggtg ggggtaagag ggatgtatat tacgtttttt ggtaaaatta 1380
   aatggtagtt tttattgtaa aagtttaacg gtagaggaag agggtagaga gaggtttagt 1440
   aatgttttta agtatttata aagattttta ttatagatga ttttttttta gtaagaaaac 1500
   gtgatggttt aaaagagaaa attagtagcg aggaattata aatttttaaa ggatttaggt 1560
   cgtaggattt tttcgtattt gtgtgttttt gcggcgttta gcggagcgag taagtttttg 1620
   ggtaaggttg gagttgtttg gagtttatcg gagtttggtc ggtcggattt tggttgggga 1680
   gtagtataga agtaatttgt gatgtgaatt tttatttaat tgttttttag ggagataatt 1740
   ttggttttat atattttata tgtttcgtat aaagaaatag aaagcgggcg atacgttttg 1800
   ggagggcggg ttggcggttt ttttagttcg gagcgcgtgg aggttgcgcg taggggttcg 1860
   ggacgcggga tagggatttc gcgtagttta ggcgtcgggg aatcggggtt ggagtcgggg 1920
   atttgagttt agcggacgtt tttttcgttt ggaggttcgg tacggatcgg gatattaggg 1980
   cgttcgtggt cgtggttcgg tttttggttg ttgcgggtcg cggtttttag tgggttttta 2040
   ggattttttt ttacggcggg tatttatcga ggttgagagg ttacgtcgtg ttgttggggt 2100
   ttttcgtcga ttatattttt gggcgttagt tcgggtagag atcgcgggga ttggttaggt 2160
   cgggttaggt cgggtcgggg gagggtcgcg ttttcgggtt tttgattttc ggcggcgaga 2220
   ggaagcggta tttgggttat atttttttcg cgcgggtcga ggggaggggt tttggttcgg 2280
   agataggtag attttattta tttttttttt gtagatttgg ttggtggggt ttttattttt 2340
   tttttttttt ttaaatatat attcgtttat ttttgaattt ttttttgttt taattttttt 2400
   gcgtaagaat tacgtttttt taatcgtttt tttttttatt tgttattatc ggatatagag 2460
   gttgtggttg agaatttttt ttggagtgta gggaaaattt tttaatgtga tatgatggtt 2520
   tggtttagta ttgtcgggga tggtgattaa atagggtaga tttgtgttga aattttggtt 2580
   ggttgcgcgg tcgttggata gttgggcggt tttttttagt tgcggggtat ttgtttttgg 2640
   agagagtttt tgttttgttg gtttttgggg gttttgtatt tatggatggg ttgttgagta 2700
   aagattattt taaaatagga agaattgttg gataagagtc gtgatttgtt tttattgttt 2760
   ttgtttgaag ttgttagata tagtgtagtt tgtgaattcg ttttattttt gttatgaaaa 2820
   tttttacgtg gatttcgata tatatttttg ttggttaaat gtagttaaat ggtattattt 2880
   ataaaaaatg tattatagta ttatttgttt tttttgttta gagataaggg tagggaattt 2940
   gaggtttttg ttttaaattt ttatttaggg gaatattttg tgtttttttt gtgtgatgtt 3000
   t 3001
<210> 543
   <211> 3001
   <212> DNA
   <213> human
<400> 543
   aatgttttgt attttataat tttttgtttt ttagggagtt ttgtaagttt ttagaaatat 60
   ttgtttaatt atgggtattt ttggaagggt ttagttgttg attattagta ttatgtgatt 120
   tttagtgtta gtgatatttg gaggaattga gtaatggttg aggtggaatt ttatttggta 180
   tttaatgtaa ttttttgttt ttgttagagg tggtgaattt tatataaagt tgttaaattt 240
   ggttgttttt ttattgagtt ttgatttttt ttatttaagg gtaaggtaat ggattgttgt 300
   gtatgattta tgtgtagtaa ttgtggtatt ggggtttagt ttagttgagt tgagtagagt 360
   aggttggttt ggtggaagtg ggtttttttt gttttaattt ttggttttga tgagtagtta 420
   tataaattga gataggatat agatatattt tgtttgagtt agtagtaaga aatagttgtt 480
   ttttattttt tttgtagttt ttgtatagta gggattttgt tataaattaa tttgaagttt 540
   aatgtggaat ggtttgtaag taagggttgt attgttgttt gttttttaag gtaagtgttg 600
   aaggtttgaa atttgagttt tggagaattg tttgggttgt tgggttgggt tgggtatggg 660
   ggtgggggtt aggtaagttt atttttttta gatatgagaa tgttttttag ttttattttt 720
   ttagtgattg aattggaata atgatagtgg tttttgtttt tttaatttag gatttgttgt 780
   atatatatat ataaatatat gtttaagtta tgtgttttgg gaattatagt tttatgtgta 840
   attatataaa tatgtttagt atggatttag tatttaggat gtgttagatt ttgtgttggg 900
   atatttatat gtattttgtg ttgttggatg agttattttt tgttattatt tttatttttt 960
   tattttgtag atgaggaaat tggagttaag agaggttgtt ttgtttatag gtagtgtaga 1020
   tgattttagt gtagattgag gggttttgtt tttaagtatt attgatgttt gtgtattttt 1080
   aggttgatgt aagtataatt ataggtatat ggagtgttgt gtatatatgt agattgaagt 1140
   agtatttttt tatgttaagt tttaagttta ggtggggtga ggggtgaggt tttataattt 1200
   tttggatttt aaagatttat aaaagagttg gtgtttttaa tatttatatt attattatat 1260
   taataaggta ttattgttat atgataggtt tagggtattt tttttttgat agattttaag 1320
   aattagattg tggtagggtg ggggtaagag ggatgtatat tatgtttttt ggtaaaatta 1380
   aatggtagtt tttattgtaa aagtttaatg gtagaggaag agggtagaga gaggtttagt 1440
   aatgttttta agtatttata aagattttta ttatagatga ttttttttta gtaagaaaat 1500
   gtgatggttt aaaagagaaa attagtagtg aggaattata aatttttaaa ggatttaggt 1560
   tgtaggattt ttttgtattt gtgtgttttt gtggtgttta gtggagtgag taagtttttg 1620
   ggtaaggttg gagttgtttg gagtttattg gagtttggtt ggttggattt tggttgggga 1680
   gtagtataga agtaatttgt gatgtgaatt tttatttaat tgttttttag ggagataatt 1740
   ttggttttat atattttata tgttttgtat aaagaaatag aaagtgggtg atatgttttg 1800
   ggagggtggg ttggtggttt ttttagtttg gagtgtgtgg aggttgtgtg taggggtttg 1860
   ggatgtggga tagggatttt gtgtagttta ggtgttgggg aattggggtt ggagttgggg 1920
   atttgagttt agtggatgtt ttttttgttt ggaggtttgg tatggattgg gatattaggg 1980
   tgtttgtggt tgtggtttgg tttttggttg ttgtgggttg tggtttttag tgggttttta 2040
   ggattttttt ttatggtggg tatttattga ggttgagagg ttatgttgtg ttgttggggt 2100
   tttttgttga ttatattttt gggtgttagt ttgggtagag attgtgggga ttggttaggt 2160
   tgggttaggt tgggttgggg gagggttgtg tttttgggtt tttgattttt ggtggtgaga 2220
   ggaagtggta tttgggttat attttttttg tgtgggttga ggggaggggt tttggtttgg 2280
   agataggtag attttattta tttttttttt gtagatttgg ttggtggggt ttttattttt 2340
   tttttttttt ttaaatatat atttgtttat ttttgaattt tttttgtttt taattttttt 2400
   gtgtaagaat tatgtttttt taattgtttt tttttttatt tgttattatt ggatatagag 2460
   gttgtggttg agaatttttt ttggagtgta gggaaaattt tttaatgtga tatgatggtt 2520
   tggtttagta ttgttgggga tggtgattaa atagggtaga tttgtgttga aattttggtt 2580
   ggttgtgtgg ttgttggata gttgggtggt tttttttagt tgtggggtat ttgtttttgg 2640
   agagagtttt tgttttgttg gtttttgggg gttttgtatt tatggatggg ttgttgagta 2700
   aagattattt taaaatagga agaattgttg gataagagtt gtgatttgtt tttattgttt 2760
   ttgtttgaag ttgttagata tagtgtagtt tgtgaatttg ttttattttt gttatgaaaa 2820
   tttttatgtg gattttgata tatatttttg ttggttaaat gtagttaaat ggtattattt 2880
   ataaaaaatg tattatagta ttatttgttt tttttgttta gagataaggg tagggaattt 2940
   gaggtttttg ttttaaattt ttatttaggg gaatattttg tgtttttttt gtgtgatgtt 3000
   t 3001
<210> 544
   <211> 3001
   <212> DNA
   <213> human
<400> 544
<210> 545
   <211> 3001
   <212> DNA
   <213> human
<400> 545
   agatattata taggagaagt atagggtgtt tttttgggtg gggatttaaa ataagggttt 60
   taagtttttt gtttttgttt ttgggtagaa ggggtagata atgttatgat atattttttg 120
   taggtggtgt tatttgattg tatttaatta ataaaggtat gtattgaaat ttatgtggag 180
   atttttatgg taaaagtaga gtgaatttat aaattgtatt gtgtttggta gttttagata 240
   aaagtaatgg gaataaatta tgatttttgt ttagtaattt tttttgtttt ggaatgattt 300
   ttgtttaata gtttatttat gagtataggg tttttaaagg ttaataagat agggattttt 360
   tttagggata aatgttttgt agttgagaga ggttgtttag ttatttagtg gttgtgtagt 420
   tagttaagat tttaatatag gtttgtttta tttggttatt atttttgata gtgttgggtt 480
   aggttattat gttatattgg ggagtttttt ttgtatttta gggggaattt ttaattataa 540
   tttttgtatt tggtagtggt agatggaaag agaaatggtt agaaaagtgt ggtttttgtg 600
   taggaaagtt ggagtaaaga agagtttaga agtgggtggg tgtgtgttta aaaaaaaaaa 660
   agggggtgga aattttatta gttaagtttg tagaaaaaaa ataaatgaag tttgtttatt 720
   tttgggttag agtttttttt tttggtttgt gtgggaggag tgtgatttag gtgttgtttt 780
   tttttgttgt tgagggttag gagtttggga gtgtgatttt tttttggttt ggtttggttt 840
   ggtttggtta gtttttgtgg tttttgtttg ggttgatgtt taggaatgtg gttgatgaga 900
   agttttaata gtatggtgtg gttttttagt tttggtgagt atttgttgtg gggaagggtt 960
   ttggggattt attggaggtt gtggtttgta gtagttaggg gttgagttat ggttatggat 1020
   gttttggtgt tttggtttgt gttgggtttt taggtggagg aggtgtttgt tgggtttaga 1080
   tttttgattt tagttttggt tttttggtgt ttgggttgtg tggagttttt gttttgtgtt 1140
   ttggattttt atgtgtagtt tttatgtgtt ttgagttgga gaagttgtta gtttgttttt 1200
   ttagggtgtg ttgtttgttt tttgtttttt tgtgtggggt atgtgaaatg tgtggggtta 1260
   gaattgtttt tttaaagaat agttaggtga gagtttatat tataagttgt ttttgtgttg 1320
   ttttttagtt agggtttgat tggttaggtt ttggtgaatt ttaggtaatt ttagttttgt 1380
   ttagggattt gtttgttttg ttgggtgttg taaaggtata tagatgtgag ggaattttgt 1440
   ggtttggatt ttttggggat ttgtaatttt ttgttgttgg tttttttttt taaattatta 1500
   tgtttttttg ttgagaggaa attatttgtg atgaaagttt ttgtagatgt ttggaaatat 1560
   tgttgggttt ttttttgttt tttttttttg ttgttggatt tttgtaataa aaattattat 1620
   ttgattttat taaaaaatgt aatgtatatt tttttatttt ttattttgtt gtggtttgat 1680
   ttttagagtt tgttagagag gagatatttt gagtttatta tgtgataatg atattttatt 1740
   gatatgataa tgatatgggt attggagata ttaatttttt tataagtttt tgaaatttag 1800
   ggggttgtga ggttttattt tttattttgt ttgagtttgg agtttggtgt ggaggagtgt 1860
   tgttttggtt tatgtgtgta tatggtattt tatgtgtttg tggttgtgtt tgtgttagtt 1920
   tgggagtgtg taggtattag tggtgtttgg gagtgaggtt ttttagtttg tattgggatt 1980
   atttgtattg tttataaata aggtagtttt ttttagtttt agttttttta tttgtaaagt 2040
   aggaaagtag gaataataat aggagataat ttatttagta gtatgaagtg tgtataaatg 2100
   ttttggtatg gagtttgata tattttaagt gttggatttg tgttgggtat gtttatataa 2160
   ttgtatatga gattgtggtt tttggagtat gtaatttgag tatatgttta tgtgtgtgta 2220
   tatggtaggt tttgggttaa agggatagga gttattgtta ttattttaat ttagttattg 2280
   aggaggtgag gttgagaggt atttttatgt ttgaagagga tgggtttgtt tgatttttgt 2340
   ttttgtgttt agtttagttt agtagtttaa gtagtttttt aggatttagg ttttagattt 2400
   ttaatatttg ttttgggaga taaatagtgg tgtagttttt gtttgtaagt tattttatat 2460
   tagattttgg attgatttat ggtaggattt ttgttgtgtg agaattgtag ggggggtggg 2520
   gggtggttat tttttgttgt taatttaggt aggatgtgtt tatattttgt tttagtttat 2580
   gtagttgttt attagagtta gaggttagag tggagaggat ttatttttgt taggttggtt 2640
   tgttttgttt agtttagttg ggttaggttt taatgttata gttattgtgt gtgagttatg 2700
   tatagtaatt tattgttttg tttttggatg gaaggggtta aggtttagtg agaagatggt 2760
   taggtttggt agttttatat agaatttatt gtttttggta gaagtagagg attgtattaa 2820
   gtgttaaatg aaattttatt ttagttattg tttaattttt ttagatgtta ttggtattgg 2880
   gagttatatg atgttggtgg ttagtagttg gattttttta agggtgtttg tggttgggta 2940
   agtatttttg gggatttgta gagttttttg gggagtaaag ggttgtgaag tataaggtat 3000
   t 3001
<210> 546
   <211> 50
   <212> DNA
   <213> human
<400> 546
   cattctcagc tacttctgcc tccttgaaag tttctcatga tgaaatttcg 50
<210> 547
   <211> 50
   <212> DNA
   <213> human
<400> 547
   tatttttagt tatttttgtt tttttgaaag ttttttatga tgaaatttcg 50
<210> 548
   <211> 50
   <212> DNA
   <213> human
<400> 548
   tatttttagt tatttttgtt tttttgaaag ttttttatga tgaaattttg 50
<210> 549
   <211> 50
   <212> DNA
   <213> human
<400> 549
   cgaaatttta ttatgagaaa tttttaagga ggtagaagta gttgagaatg 50
<210> 550
   <211> 50
   <212> DNA
   <213> human
<400> 550
   tgaaatttta ttatgagaaa tttttaagga ggtagaagta gttgagaatg 50
<210> 551
   <211> 3001
   <212> DNA
   <213> human
<400> 551
   aactctttaa acacctcctc tcttttatgt ttttccaatt ttaagtaaat cttaaagtcc      60
   taaatttctg aagtttcata aagatagctc actgcctttt ttacttccat tcctttttcc     120
   tacatcagaa ctttctagtg gcaaatacta catcctatat catgtgacag caaatcagat     180
   ttgggtagct ccaatattaa accaacacct gttctcacca cttctacttt agccttgtct     240
   gtgaacaaga gttttaattg tatcctaaca tttacaaatt tgagtacaaa aatggaaata     300
   gccagttatg aaacaatcat aaatgttctc taatgaaaac cacactctcc cacctgaatt     360
   atacacatag gtggttttac ccagagaaat ttaggactaa cttagttata ctgcaagatt     420
   gtattaaaaa taaatatttt aataagctta cattgattac cccgtatgta atatttttaa     480
   aaaattgaaa atgcaaatag ttttgacact tacctgtagg aggaccttcc tcccatcctt     540
   ctgctctctt aattcgatgt gcagtgaatc ctaagcagat attgactcca acggcaaaag     600
   tgaacaggga tattatctaa gagaataaag agaccaagtt gtcaaataca gctggagaag     660
   actaaatcaa attacacaaa ggtgattcaa atggcttcaa tatcaacacc caatcctacc     720
   acttccaaaa tgcataataa ggtgctatct taatttgcac aacaaaaagg aaaatgccaa     780
   tcagtcactg attctgaaga tcaagcatgt cccccgtcat tcctccgttc tcccacctga     840
   cacgactgga acgagctcct ccttgccatg tcgaggattc ttggaaagcc ttttgcagcg     900
   tgttctcttt gccttcacgg agtgcactgc tttgaggctc tgggtttagt ctattaacta     960
   taagcaatcc cacctgggag gttgacagac tcctctgatt tgtccagagc ttgattacat   1020
   cacagggtgt tcacaaatcg cagctctgag cctaagaggc ttaagctatc atccccctta    1080
   tccccgcccc cttacaggtc cctcccctaa cccacagaca ctttcatgtg gtatcctctg   1140
   gcaacaggag ggtttgttac attgccgtgg cccataacag tgcatgttca ttttcctttc   1200
   ctcacaaaaa catggtttgt caaaaagaaa ccaaatgcat ttcatccgat aaaaatgaaa   1260
   acagggctgt gcaagggctt tccaccagct ttaagctttt aaggtttaca atgcagtcat   1320
   tcaatggcgt caacctcacc actttatttt tttaagcaag gaaagtttta tcatttataa   1380
   acctaataac gttcctgtct ttcccatcta tttattttgc tggcagtttg agcgaccctg   1440
   tccctagtat cacattctca gctacttctg cctccttgaa agtttctcat gatgaaattt   1500
   cgcaaaattg taactaacat aaaagataac attattttcc ccatgctgtg gttcaagttt   1560
   agagaagtaa cttttatcaa aatactggaa atgccagcca cgcagcataa agtccttctg   1620
   ccctgggagg acggacaaaa taacttcagt ctctgcctct tcttcccgta aagactgtca   1680
   gcttgtttcg ggcggggcgg aggagggggc ggtttttgag aggcagatgg gtgggggaag   1740
   gggatggcat gggaagcttt attagcaatt gaatttagga aggaaatctc aatggcagca   1800
   agaattgaaa tgtcaagaat tgtttcccta gttatttctt ggttaaatgt agccaccaaa   1860
   tactgtgtcc tttgaggcta ttgtgggtac taaagtttct ttactaagca atttggcaac   1920
   cagcacactt tcaaaagaaa aaatcaaggg ctctaaatct gaaagttgtt taaatattac   1980
   atgccttcag gattcaaatg agggaaaaag atttgttttt ggcctcttcc tcagcaggtc   2040
   cagtgaccat cttttcttcc gcagcacatg tgttcgtgcg taaacaaata cgagacttac   2100
   catgcaacaa attagtaaca gagtagtggc tctgctcaaa taaaacatga tagcacaaaa   2160
   gcaaaaactt gtttataggg ggttgtacag taggcctggg tttcattaca gattcttaga   2220
   atctagtaat atctaattga aaaaaagaaa atacacatac tgccaaactg ttaattgcct   2280
   ttctaaaatc cttttagttt tgcactgttg caaagaggac aggcttatac atttttatat   2340
   gtaagcctct tgcttttgtc tgtatgccat actgaacaaa tttggagtag ggtatttagc   2400
   aaggcttata aaagatgtta actccatgaa agattttcct ataatttatg ttaaatttca   2460
   agatacaagc cagacatgat agaggaaaac aataaacccc aaagaaaaat atcaaggcaa   2520
   tcctgttatt tcattatatg ccacagtgct ctaaatgttc taaaaacaaa aacagcctct   2580
   tcttttgcct tgccagagtc taacaacttt tgactttgtc tgatatttga ttgtataaac   2640
   ctagctagaa gacaatgagg gttcccttgg aaaaaaagtt agggttcagg agctgaacat   2700
   catccattta tttttctctt atatgcagac tagctgggca gggggaaaag ctgtaatagc   2760
   caagttgatc tgcttgttac ccaatatcca aaagagttga cctcagccac tatttctttg   2820
   tttttttgtt tttgtttgtt tatttgttta ttttgagatg gagtttcact cttgttgccc   2880
   aggctggagt gcaatggctc gatctcggct cactgcagcc tccacctccc gggttcaagt   2940
   gattcctgtc tcagcctccc gagtagctgg gattacaggt gcatgccaac acgcctggct   3000
   a                                                                   3001
<210> 552
   <211> 3001
   <212> DNA
   <213> human
<400> 552
   aattttttaa atattttttt ttttttatgt ttttttaatt ttaagtaaat tttaaagttt 60
   taaatttttg aagttttata aagatagttt attgtttttt ttatttttat tttttttttt 120
   tatattagaa ttttttagtg gtaaatatta tattttatat tatgtgatag taaattagat 180
   ttgggtagtt ttaatattaa attaatattt gtttttatta tttttatttt agttttgttt 240
   gtgaataaga gttttaattg tattttaata tttataaatt tgagtataaa aatggaaata 300
   gttagttatg aaataattat aaatgttttt taatgaaaat tatatttttt tatttgaatt 360
   atatatatag gtggttttat ttagagaaat ttaggattaa tttagttata ttgtaagatt 420
   gtattaaaaa taaatatttt aataagttta tattgattat ttcgtatgta atatttttaa 480
   aaaattgaaa atgtaaatag ttttgatatt tatttgtagg aggatttttt ttttattttt 540
   ttgttttttt aattcgatgt gtagtgaatt ttaagtagat attgatttta acggtaaaag 600
   tgaataggga tattatttaa gagaataaag agattaagtt gttaaatata gttggagaag 660
   attaaattaa attatataaa ggtgatttaa atggttttaa tattaatatt taattttatt 720
   atttttaaaa tgtataataa ggtgttattt taatttgtat aataaaaagg aaaatgttaa 780
   ttagttattg attttgaaga ttaagtatgt ttttcgttat tttttcgttt ttttatttga 840
   tacgattgga acgagttttt ttttgttatg tcgaggattt ttggaaagtt ttttgtagcg 900
   tgtttttttt gtttttacgg agtgtattgt tttgaggttt tgggtttagt ttattaatta 960
   taagtaattt tatttgggag gttgatagat ttttttgatt tgtttagagt ttgattatat 1020
   tatagggtgt ttataaatcg tagttttgag tttaagaggt ttaagttatt atttttttta 1080
   ttttcgtttt tttataggtt ttttttttaa tttatagata tttttatgtg gtattttttg 1140
   gtaataggag ggtttgttat attgtcgtgg tttataatag tgtatgttta tttttttttt 1200
   tttataaaaa tatggtttgt taaaaagaaa ttaaatgtat tttattcgat aaaaatgaaa 1260
   atagggttgt gtaagggttt tttattagtt ttaagttttt aaggtttata atgtagttat 1320
   ttaatggcgt taattttatt attttatttt tttaagtaag gaaagtttta ttatttataa 1380
   atttaataac gtttttgttt tttttattta tttattttgt tggtagtttg agcgattttg 1440
   tttttagtat tatattttta gttatttttg tttttttgaa agttttttat gatgaaattt 1500
   cgtaaaattg taattaatat aaaagataat attatttttt ttatgttgtg gtttaagttt 1560
   agagaagtaa tttttattaa aatattggaa atgttagtta cgtagtataa agtttttttg 1620
   ttttgggagg acggataaaa taattttagt ttttgttttt ttttttcgta aagattgtta 1680
   gtttgtttcg ggcggggcgg aggagggggc ggtttttgag aggtagatgg gtgggggaag 1740
   gggatggtat gggaagtttt attagtaatt gaatttagga aggaaatttt aatggtagta 1800
   agaattgaaa tgttaagaat tgttttttta gttatttttt ggttaaatgt agttattaaa 1860
   tattgtgttt tttgaggtta ttgtgggtat taaagttttt ttattaagta atttggtaat 1920
   tagtatattt ttaaaagaaa aaattaaggg ttttaaattt gaaagttgtt taaatattat 1980
   atgtttttag gatttaaatg agggaaaaag atttgttttt ggtttttttt ttagtaggtt 2040
   tagtgattat tttttttttc gtagtatatg tgttcgtgcg taaataaata cgagatttat 2100
   tatgtaataa attagtaata gagtagtggt tttgtttaaa taaaatatga tagtataaaa 2160
   gtaaaaattt gtttataggg ggttgtatag taggtttggg ttttattata gatttttaga 2220
   atttagtaat atttaattga aaaaaagaaa atatatatat tgttaaattg ttaattgttt 2280
   ttttaaaatt tttttagttt tgtattgttg taaagaggat aggtttatat atttttatat 2340
   gtaagttttt tgtttttgtt tgtatgttat attgaataaa tttggagtag ggtatttagt 2400
   aaggtttata aaagatgtta attttatgaa agattttttt ataatttatg ttaaatttta 2460
   agatataagt tagatatgat agaggaaaat aataaatttt aaagaaaaat attaaggtaa 2520
   ttttgttatt ttattatatg ttatagtgtt ttaaatgttt taaaaataaa aatagttttt 2580
   ttttttgttt tgttagagtt taataatttt tgattttgtt tgatatttga ttgtataaat 2640
   ttagttagaa gataatgagg gtttttttgg aaaaaaagtt agggtttagg agttgaatat 2700
   tatttattta tttttttttt atatgtagat tagttgggta gggggaaaag ttgtaatagt 2760
   taagttgatt tgtttgttat ttaatattta aaagagttga ttttagttat tatttttttg 2820
   tttttttgtt tttgtttgtt tatttgttta ttttgagatg gagttttatt tttgttgttt 2880
   aggttggagt gtaatggttc gatttcggtt tattgtagtt tttatttttc gggtttaagt 2940
   gatttttgtt ttagtttttc gagtagttgg gattataggt gtatgttaat acgtttggtt 3000
   a 3001
<210> 553
   <211> 3001
   <212> DNA
   <213> human
<400> 553
   aattttttaa atattttttt ttttttatgt ttttttaatt ttaagtaaat tttaaagttt      60
   taaatttttg aagttttata aagatagttt attgtttttt ttatttttat tttttttttt    120
   tatattagaa ttttttagtg gtaaatatta tattttatat tatgtgatag taaattagat    180
   ttgggtagtt ttaatattaa attaatattt gtttttatta tttttatttt agttttgttt    240
   gtgaataaga gttttaattg tattttaata tttataaatt tgagtataaa aatggaaata    300
   gttagttatg aaataattat aaatgttttt taatgaaaat tatatttttt tatttgaatt    360
   atatatatag gtggttttat ttagagaaat ttaggattaa tttagttata ttgtaagatt    420
   gtattaaaaa taaatatttt aataagttta tattgattat tttgtatgta atatttttaa    480
   aaaattgaaa atgtaaatag ttttgatatt tatttgtagg aggatttttt ttttattttt    540
   ttgttttttt aatttgatgt gtagtgaatt ttaagtagat attgatttta atggtaaaag    600
   tgaataggga tattatttaa gagaataaag agattaagtt gttaaatata gttggagaag    660
   attaaattaa attatataaa ggtgatttaa atggttttaa tattaatatt taattttatt    720
   attttaaaaa tgtataataa ggtgttattt taatttgtat aataaaaagg aaaatgttaa    780
   ttagttattg attttgaaga ttaagtatgt tttttgttat ttttttgttt ttttatttga    840
   tatgattgga atgagttttt ttttgttatg ttgaggattt ttggaaagtt ttttgtagtg    900
   tgtttttttt gtttttatgg agtgtattgt tttgaggttt tgggtttagt ttattaatta    960
   taagtaattt tatttgggag gttgatagat ttttttgatt tgtttagagt ttgattatat   1020
   tatagggtgt ttataaattg tagttttgag tttaagaggt ttaagttatt atttttttta   1080
   tttttgtttt tttataggtt ttttttttaa tttatagata tttttatgtg gtattttttg   1140
   gtaataggag ggtttgttat attgttgtgg tttataatag tgtatgttta tttttttttt   1200
   tttataaaaa tatggtttgt taaaaagaaa ttaaatgtat tttatttgat aaaaatgaaa   1260
   atagggttgt gtaagggttt tttattagtt ttaagttttt aaggtttata atgtagttat   1320
   ttaatggtgt taattttatt attttatttt tttaagtaag gaaagtttta ttatttataa   1380
   atttaataat gtttttgttt tttttattta tttattttgt tggtagtttg agtgattttg   1440
   tttttagtat tatattttta gttatttttg tttttttgaa agttttttat gatgaaattt   1500
   tgtaaaattg taattaatat aaaagataat attatttttt ttatgttgtg gtttaagttt   1560
   agagaagtaa tttttattaa aatattggaa atgttagtta tgtagtataa agtttttttg   1620
   ttttgggagg atggataaaa taattttagt ttttgttttt tttttttgta aagattgtta   1680
   gtttgttttg ggtggggtgg aggagggggt ggtttttgag aggtagatgg gtgggggaag   1740
   gggatggtat gggaagtttt attagtaatt gaatttagga aggaaatttt aatggtagta   1800
   agaattgaaa tgttaagaat tgttttttta gttatttttt ggttaaatgt agttattaaa   1860
   tattgtgttt tttgaggtta ttgtgggtat taaagttttt ttattaagta atttggtaat   1920
   tagtatattt ttaaaagaaa aaattaaggg ttttaaattt gaaagttgtt taaatattat   1980
   atgtttttag gatttaaatg agggaaaaag atttgttttt ggtttttttt ttagtaggtt   2040
   tagtgattat tttttttttt gtagtatatg tgtttgtgtg taaataaata tgagatttat   2100
   tatgtaataa attagtaata gagtagtggt tttgtttaaa taaaatatga tagtataaaa   2160
   gtaaaaattt gtttataggg ggttgtatag taggtttggg ttttattata gatttttaga   2220
   atttagtaat atttaattga aaaaaagaaa atatatatat tgttaaattg ttaattgttt   2280
   ttttaaaatt tttttagttt tgtattgttg taaagaggat aggtttatat atttttatat   2340
   gtaagttttt tgtttttgtt tgtatgttat attgaataaa tttggagtag ggtatttagt   2400
   aaggtttata aaagatgtta attttatgaa agattttttt ataatttatg ttaaatttta   2460
   agatataagt tagatatgat agaggaaaat aataaatttt aaagaaaaat attaaggtaa   2520
   ttttgttatt ttattatatg ttatagtgtt ttaaatgttt taaaaataaa aatagttttt   2580
   ttttttgttt tgttagagtt taataatttt tgattttgtt tgatatttga ttgtataaat   2640
   ttagttagaa gataatgagg gtttttttgg aaaaaaagtt agggtttagg agttgaatat   2700
   tatttattta tttttttttt atatgtagat tagttgggta gggggaaaag ttgtaatagt   2760
   taagttgatt tgtttgttat ttaatattta aaagagttga ttttagttat tatttttttg   2820
   tttttttgtt tttgtttgtt tatttgttta ttttgagatg gagttttatt tttgttgttt   2880
   aggttggagt gtaatggttt gattttggtt tattgtagtt tttatttttt gggtttaagt   2940
   gatttttgtt ttagtttttt gagtagttgg gattataggt gtatgttaat atgtttggtt   3000
   a                                                                   3001
<210> 554
   <211> 3001
   <212> DNA
   <213> human
<400> 554
   tagttaggcg tgttggtatg tatttgtaat tttagttatt cgggaggttg agataggaat      60
   tatttgaatt cgggaggtgg aggttgtagt gagtcgagat cgagttattg tattttagtt     120
   tgggtaataa gagtgaaatt ttattttaaa ataaataaat aaataaataa aaataaaaaa     180
   ataaagaaat agtggttgag gttaattttt ttggatattg ggtaataagt agattaattt     240
   ggttattata gttttttttt ttgtttagtt agtttgtata taagagaaaa ataaatggat     300
   gatgtttagt ttttgaattt taattttttt tttaagggaa tttttattgt tttttagtta     360
   ggtttatata attaaatatt agataaagtt aaaagttgtt agattttggt aaggtaaaag     420
   aagaggttgt ttttgttttt agaatattta gagtattgtg gtatataatg aaataatagg     480
   attgttttga tatttttttt tggggtttat tgtttttttt tattatgttt ggtttgtatt     540
   ttgaaattta atataaatta taggaaaatt ttttatggag ttaatatttt ttataagttt     600
   tgttaaatat tttattttaa atttgtttag tatggtatat agataaaagt aagaggttta     660
   tatataaaaa tgtataagtt tgtttttttt gtaatagtgt aaaattaaaa ggattttaga     720
   aaggtaatta atagtttggt agtatgtgta tttttttttt tttaattaga tattattaga     780
   ttttaagaat ttgtaatgaa atttaggttt attgtataat tttttataaa taagtttttg     840
   tttttgtgtt attatgtttt atttgagtag agttattatt ttgttattaa tttgttgtat     900
   ggtaagtttc gtatttgttt acgtacgaat atatgtgttg cggaagaaaa gatggttatt     960
   ggatttgttg aggaagaggt taaaaataaa tttttttttt ttatttgaat tttgaaggta    1020
   tgtaatattt aaataatttt tagatttaga gtttttgatt tttttttttg aaagtgtgtt    1080
   ggttgttaaa ttgtttagta aagaaatttt agtatttata atagttttaa aggatatagt    1140
   atttggtggt tatatttaat taagaaataa ttagggaaat aatttttgat attttaattt    1200
   ttgttgttat tgagattttt tttttaaatt taattgttaa taaagttttt tatgttattt    1260
   ttttttttta tttatttgtt ttttaaaaat cgtttttttt ttcgtttcgt tcgaaataag    1320
   ttgatagttt ttacgggaag aagaggtaga gattgaagtt attttgttcg tttttttagg    1380
   gtagaaggat tttatgttgc gtggttggta tttttagtat tttgataaaa gttatttttt    1440
   taaatttgaa ttatagtatg gggaaaataa tgttattttt tatgttagtt ataattttgc    1500
   gaaattttat tatgagaaat ttttaaggag gtagaagtag ttgagaatgt gatattaggg    1560
   atagggtcgt ttaaattgtt agtaaaataa atagatggga aagataggaa cgttattagg    1620
   tttataaatg ataaaatttt ttttgtttaa aaaaataaag tggtgaggtt gacgttattg    1680
   aatgattgta ttgtaaattt taaaagttta aagttggtgg aaagtttttg tatagttttg    1740
   tttttatttt tatcggatga aatgtatttg gttttttttt gataaattat gtttttgtga    1800
   ggaaaggaaa atgaatatgt attgttatgg gttacggtaa tgtaataaat ttttttgttg    1860
   ttagaggata ttatatgaaa gtgtttgtgg gttaggggag ggatttgtaa gggggcgggg    1920
   ataaggggga tgatagttta agttttttag gtttagagtt gcgatttgtg aatattttgt    1980
   gatgtaatta agttttggat aaattagagg agtttgttaa ttttttaggt gggattgttt    2040
   atagttaata gattaaattt agagttttaa agtagtgtat ttcgtgaagg taaagagaat    2100
   acgttgtaaa aggtttttta agaattttcg atatggtaag gaggagttcg ttttagtcgt    2160
   gttaggtggg agaacggagg aatgacgggg gatatgtttg atttttagaa ttagtgattg    2220
   attggtattt ttttttttgt tgtgtaaatt aagatagtat tttattatgt attttggaag     2280
   tggtaggatt gggtgttgat attgaagtta tttgaattat ttttgtgtaa tttgatttag    2340
   ttttttttag ttgtatttga taatttggtt tttttatttt tttagataat atttttgttt     2400
   atttttgtcg ttggagttaa tatttgttta ggatttattg tatatcgaat taagagagta    2460
   gaaggatggg aggaaggttt ttttataggt aagtgttaaa attatttgta tttttaattt    2520
   tttaaaaata ttatatacgg ggtaattaat gtaagtttat taaaatattt atttttaata    2580
   taattttgta gtataattaa gttagtttta aatttttttg ggtaaaatta tttatgtgta    2640
   taatttaggt gggagagtgt ggtttttatt agagaatatt tatgattgtt ttataattgg    2700
   ttatttttat ttttgtattt aaatttgtaa atgttaggat ataattaaaa tttttgttta    2760
   tagataaggt taaagtagaa gtggtgagaa taggtgttgg tttaatattg gagttattta    2820
   aatttgattt gttgttatat gatataggat gtagtatttg ttattagaaa gttttgatgt    2880
   aggaaaaagg aatggaagta aaaaaggtag tgagttattt ttatgaaatt ttagaaattt    2940
   aggattttaa gatttattta aaattggaaa aatataaaag agaggaggtg tttaaagagt    3000
   t                                                                 3001
<210> 555
   <211> 3001
   <212> DNA
   <213> human
<400> 555
   tagttaggtg tgttggtatg tatttgtaat tttagttatt tgggaggttg agataggaat    60
   tatttgaatt tgggaggtgg aggttgtagt gagttgagat tgagttattg tattttagtt   120
   tgggtaataa gagtgaaatt ttattttaaa ataaataaat aaataaataa aaataaaaaa   180
   ataaagaaat agtggttgag gttaattttt ttggatattg ggtaataagt agattaattt   240
   ggttattata gttttttttt ttgtttagtt agtttgtata taagagaaaa ataaatggat   300
   gatgtttagt ttttgaattt taattttttt tttaagggaa tttttattgt tttttagtta   360
   ggtttatata attaaatatt agataaagtt aaaagttgtt agattttggt aaggtaaaag   420
   aagaggttgt ttttgttttt agaatattta gagtattgtg gtatataatg aaataatagg   480
   attgttttga tatttttttt tggggtttat tgtttttttt tattatgttt ggtttgtatt   540
   ttgaaattta atataaatta taggaaaatt ttttatggag ttaatatttt ttataagttt   600
   tgttaaatat tttattttaa atttgtttag tatggtatat agataaaagt aagaggttta   660
   tatataaaaa tgtataagtt tgtttttttt gtaatagtgt aaaattaaaa ggattttaga   720
   aaggtaatta atagtttggt agtatgtgta tttttttttt tttaattaga tattattaga   780
   ttttaagaat ttgtaatgaa atttaggttt attgtataat tttttataaa taagtttttg    840
   tttttgtgtt attatgtttt atttgagtag agttattatt ttgttattaa tttgttgtat   900
   ggtaagtttt gtatttgttt atgtatgaat atatgtgttg tggaagaaaa gatggttatt   960
   ggatttgttg aggaagaggt taaaaataaa tttttttttt ttatttgaat tttgaaggta  1020
   tgtaatattt aaataatttt tagatttaga gtttttgatt tttttttttg aaagtgtgtt  1080
   ggttgttaaa ttgtttagta aagaaatttt agtatttata atagttttaa aggatatagt  1140
   atttggtggt tatatttaat taagaaataa ttagggaaat aatttttgat attttaattt  1200
   ttgttgttat tgagattttt tttttaaatt taattgttaa taaagttttt tatgttattt  1260
   ttttttttta tttatttgtt ttttaaaaat tgtttttttt tttgttttgt ttgaaataag  1320
   ttgatagttt ttatgggaag aagaggtaga gattgaagtt attttgtttg tttttttagg  1380
   gtagaaggat tttatgttgt gtggttggta tttttagtat tttgataaaa gttatttttt  1440
   taaatttgaa ttatagtatg gggaaaataa tgttattttt tatgttagtt ataattttgt  1500
   gaaattttat tatgagaaat ttttaaggag gtagaagtag ttgagaatgt gatattaggg  1560
   atagggttgt ttaaattgtt agtaaaataa atagatggga aagataggaa tgttattagg  1620
   tttataaatg ataaaatttt ttttgtttaa aaaaataaag tggtgaggtt gatgttattg  1680
   aatgattgta ttgtaaattt taaaagttta aagttggtgg aaagtttttg  tatagttttg  1740
   tttttatttt tattggatga aatgtatttg gttttttttt gataaattat gtttttgtga  1800
   ggaaaggaaa atgaatatgt attgttatgg gttatggtaa tgtaataaat ttttttgttg 1860
   ttagaggata ttatatgaaa gtgtttgtgg gttaggggag ggatttgtaa gggggtgggg  1920
   ataaggggga tgatagttta agttttttag gtttagagtt gtgatttgtg aatattttgt  1980
   gatgtaatta agttttggat aaattagagg agtttgttaa ttttttaggt gggattgttt  2040
   atagttaata gattaaattt agagttttaa agtagtgtat tttgtgaagg taaagagaat  2100
   atgttgtaaa aggtttttta agaatttttg atatggtaag gaggagtttg ttttagttgt 2160
   gttaggtggg agaatggagg aatgatgggg gatatgtttg atttttagaa ttagtgattg  2220
   attggtattt ttttttttgt  tgtgtaaatt aagatagtat tttattatgt attttggaag  2280
   tggtaggatt gggtgttgat attgaagtta tttgaattat ttttgtgtaa tttgatttag  2340
   ttttttttag ttgtatttga taatttggtt tttttatttt tttagataat atttttgttt  2400
   atttttgttg ttggagttaa tatttgttta ggatttattg tatattgaat taagagagta  2460
   gaaggatggg aggaaggttt ttttataggt aagtgttaaa attatttgta tttttaattt  2520
   tttaaaaata ttatatatgg ggtaattaat gtaagtttat taaaatattt atttttaata  2580
   taattttgta gtataattaa gttagtttta aatttttttg ggtaaaatta tttatgtgta 2640
   taatttaggt gggagagtgt ggtttttatt agagaatatt tatgattgtt ttataattgg  2700
   ttatttttat  ttttgtattt aaatttgtaa atgttaggat ataattaaaa tttttgttta  2760
   tagataaggt taaagtagaa gtggtgagaa taggtgttgg tttaatattg gagttattta  2820
   aatttgattt gttgttatat gatataggat gtagtatttg ttattagaaa gttttgatgt  2880
   aggaaaaagg aatggaagta aaaaaggtag tgagttattt ttatgaaatt ttagaaattt  2940
   aggattttaa gatttattta aaattggaaa aatataaaag agaggaggtg tttaaagagt  3000
   t                                                                  3001
<210> 556
   <211> 50
   <212> DNA
   <213> human
<400> 556
   cggagattat aaatagtcat gatcccagcg agacccagag atgctgtaat 50
<210> 557
   <211> 50
   <212> DNA
   <213> human
<400> 557
   cggagattat aaatagttat gattttagcg agatttagag atgttgtaat 50
<210> 558
   <211> 50
   <212> DNA
   <213> human
<400> 558
   tggagattat aaatagttat gattttagtg agatttagag atgttgtaat 50
<210> 559
   <211> 50
   <212> DNA
   <213> human
<400> 559
   attatagtat ttttgggttt cgttgggatt atgattattt ataattttcg 50
<210> 560
   <211> 50
   <212> DNA
   <213> human
<400> 560
   attatagtat ttttgggttt tgttgggatt atgattattt ataatttttg 50
<210> 561
   <211> 3001
   <212> DNA
   <213> human
<400> 561
   tgagacggat tcttgctctg tcgcccaggc tggagtgcag tggcgctgtc ttggctcact     60
   gcaagctccg cctgccgcgt tcacgccatt ctcctgtctc agcctcccga gtagctggga    120
   ctgcaggcgc ccgccaccat gcccggctaa tttttttttt tttttttttt ttttttgtat    180
   ttttagtaga gaatggtttc accgtgttag ccaggatggt cttgatctcc tgacctcgtg    240
   atccgcccac ctcggcctcc caaagtgctg ggattacagg cgtgagccac cgcgcccggc    300
   ctttttttct attattcact gaagaatcac cagcttcttg gagagtacct gatacatagc    360
   gtgtattcgg caaatacctg cttaattgaa ttcggtattt attgcatttc tagatgctat    420
   aagcttgaga ctagcagaaa gtgatgcaga attcagaatg ggtgatatgg caaatgcctt    480
   attaaatata gggctcggcg cggtggctca cacttgtaat cccagcactt tgggaggcca    540
   aggcgggcag atcacaaggt caggagtttg agaccagcct ggccaatatg gtgaaacccc    600
   gtctttacta aaaatacaaa aattagcggg gcgtggtggc gggcgcctat agtcccagct    660
   actcgggagg ctgaggcagg agaattgctt gaacccggga ggcggaggtt gcagtgagcc    720
   gagatcgcac cactgcactc cagcctgggc gacagagaga gactccatat caaaaaaaaa    780
   aaaaaaaaaa aaaaaaggat atggattgta ttccataaac agttgaacct cttattccta    840
   ctgccctgat agttggtctg gctcctagaa attcagattt tcttccctcc aggtgagatt    900
   atgcgaagaa aggaacactg gcaaaattat ttccctgatt ttcttctctc aggctgtctt    960
   tccctcccct ttaagattgt gtactccgtc tcaaaaaaaa aagaaaaaaa aaagattgta   1020
   gacccagcct ataaattcat aggatcaaga attcttacta acactctttc taacaacctt   1080
   aaatctctga tagtagcacc cacttgactt cgttttgtat ggatagattt attctaatct   1140
   ctctgtagat tgttttaatc ccaagcagat gcgagagcat aaaatagtgt atttgccctc   1200
   cctccaggct tttctggtag tattttgtct gtgaaactaa gaggctctag ctgctattag   1260
   aagagggaag gagtaaggat gagcttttga aaaaaaaaaa aaaaaaaaaa cccagggtgg   1320
   agagagtgga aggatgtggt tttaagagag gggggaggga gggcgttatt gtctgtgggg   1380
   cgggggcggg ggtagttctg ataacacaga attccgagag atcacaagat tgcttcaggg   1440
   gggtggggtg gggtggggtg gggctggggg cttgtcgccc tttcaggctc caccctttgc   1500
   ggagattata aatagtcatg atcccagcga gacccagaga tgctgtaatg gtaagacttt   1560
   ggatccttcc tgaggacgtg gagaaaactt gctgctgaga aggacatttt gaaggttttg   1620
   ttggctgaaa aagctgtttc tggaatcacc cctagatctt tcttgaagac ttgaattaga   1680
   ttacagcgat ggggacacag aaggtaagaa tgatataatt actggttatt tttgcttgtt   1740
   tgggatacac tcagaaaggc aaatattaat ggtaatctcc ttgttctatt ttctcctaag   1800
   tcaccaagct atcttaaggc cttttcattt cgtctctagc ctgcactgct gccgcttcat   1860
   cagttctctt tgaatgtcat acagttctgt tcaccttatc ataggaaagt cacccggaga   1920
   agttgtgttt gcttctcagg tttattttag gtaaccatat gccactgagg acgagatggt   1980
   tgagtgttta gaggatcggg ctgcttggtt gtatggatta caagtgtggg tctcagagcc   2040
   agtgtgaaga cttctctgga agcattagac cttgaaaagg tctgtgtttg gaggcacagg   2100
   ccagggtctg gccatctgtt tgtcctataa catatggggt tcgtggatgc taaggagaaa   2160
   gaattccagt tgtccttcct attagggtta aaatcgtgag tgcaggacat actaaaatat   2220
   tgagtgttgt atatgcatta tttgcaggga gaaaggcttt tctttttttt ctgattgagt   2280
   tttactcttg ttgcccaggc tggagtgcaa tggagcgacc gcagctcacc acagcctccg   2340
   cctcccgggt tcaagcgatt ctcctgcctc agcctcctga gtagctgaga ttacaggcat   2400
   gaaccaccgg gcccggctaa ttttgtattt ttagtagaga cggggtttct ctatgttggt   2460
   caggctggtc tcgactcccg acctcaggcg atccgccggc ctcggcctcc caaggtgctg   2520
   agattacagg catgagccac tgcgcgcagc aagaaaggca ttttttacca agataaattt   2580
   aactaaaaat aaggggccag gtgtggtggg tcacacctat aaccccagca tttgggaaac   2640
   caaggcagga ggatcacatg aggcctggag tttgagacta gccttgggca atatagtgag   2700
   atcccatctc tacaaagcag aaagaaagaa aatgtaggaa aataagaaag ggatggaaaa   2760
   ttatgagact aaagaagaaa aggctctcaa agatagtgaa tgatgagagg agacagaaga   2820
   gatttttact aaacggaaaa agatttaaca gagcattgca ctggcagggc cctccggggt   2880
   accgatgctt agctgggact gagacatctg ggaggctgca ggcaaggtca cttctcctgc   2940
   tgaagctccc agcctgctgc ctaatctgcc cgacctgaaa ggaaaggagt ttaagatatg   3000
   a                                                                   3001

<210> 562
   <211> 3001
   <212> DNA
   <213> human
<400> 562
   tgagacggat ttttgttttg tcgtttaggt tggagtgtag tggcgttgtt ttggtttatt 60
   gtaagtttcg tttgtcgcgt ttacgttatt tttttgtttt agtttttcga gtagttggga 120
   ttgtaggcgt tcgttattat gttcggttaa tttttttttt tttttttttt ttttttgtat 180
   ttttagtaga gaatggtttt atcgtgttag ttaggatggt tttgattttt tgatttcgtg 240
   attcgtttat ttcggttttt taaagtgttg ggattatagg cgtgagttat cgcgttcggt 300
   tttttttttt attatttatt gaagaattat tagttttttg gagagtattt gatatatagc 360
   gtgtattcgg taaatatttg tttaattgaa ttcggtattt attgtatttt tagatgttat 420
   aagtttgaga ttagtagaaa gtgatgtaga atttagaatg ggtgatatgg taaatgtttt 480
   attaaatata gggttcggcg cggtggttta tatttgtaat tttagtattt tgggaggtta 540
   aggcgggtag attataaggt taggagtttg agattagttt ggttaatatg gtgaaatttc 600
   gtttttatta aaaatataaa aattagcggg gcgtggtggc gggcgtttat agttttagtt 660
   attcgggagg ttgaggtagg agaattgttt gaattcggga ggcggaggtt gtagtgagtc 720
   gagatcgtat tattgtattt tagtttgggc gatagagaga gattttatat taaaaaaaaa 780
   aaaaaaaaaa aaaaaaggat atggattgta ttttataaat agttgaattt tttattttta 840
   ttgttttgat agttggtttg gtttttagaa atttagattt tttttttttt aggtgagatt 900
   atgcgaagaa aggaatattg gtaaaattat ttttttgatt tttttttttt aggttgtttt 960
   tttttttttt ttaagattgt gtatttcgtt ttaaaaaaaa aagaaaaaaa aaagattgta 1020
   gatttagttt ataaatttat aggattaaga atttttatta atattttttt taataatttt 1080
   aaatttttga tagtagtatt tatttgattt cgttttgtat ggatagattt attttaattt 1140
   ttttgtagat tgttttaatt ttaagtagat gcgagagtat aaaatagtgt atttgttttt 1200
   tttttaggtt tttttggtag tattttgttt gtgaaattaa gaggttttag ttgttattag 1260
   aagagggaag gagtaaggat gagtttttga aaaaaaaaaa aaaaaaaaaa tttagggtgg 1320
   agagagtgga aggatgtggt tttaagagag gggggaggga gggcgttatt gtttgtgggg 1380
   cgggggcggg ggtagttttg ataatataga atttcgagag attataagat tgttttaggg 1440
   gggtggggtg gggtggggtg gggttggggg tttgtcgttt ttttaggttt tattttttgc 1500
   ggagattata aatagttatg attttagcga gatttagaga tgttgtaatg gtaagatttt 1560
   ggattttttt tgaggacgtg gagaaaattt gttgttgaga aggatatttt gaaggttttg 1620
   ttggttgaaa aagttgtttt tggaattatt tttagatttt ttttgaagat ttgaattaga 1680
   ttatagcgat ggggatatag aaggtaagaa tgatataatt attggttatt tttgtttgtt 1740
   tgggatatat ttagaaaggt aaatattaat ggtaattttt ttgttttatt tttttttaag 1800
   ttattaagtt attttaaggt ttttttattt cgtttttagt ttgtattgtt gtcgttttat 1860
   tagttttttt tgaatgttat atagttttgt ttattttatt ataggaaagt tattcggaga 1920
   agttgtgttt gttttttagg tttattttag gtaattatat gttattgagg acgagatggt 1980
   tgagtgttta gaggatcggg ttgtttggtt gtatggatta taagtgtggg ttttagagtt 2040
   agtgtgaaga tttttttgga agtattagat tttgaaaagg tttgtgtttg gaggtatagg 2100
   ttagggtttg gttatttgtt tgttttataa tatatggggt tcgtggatgt taaggagaaa 2160
   gaattttagt tgtttttttt attagggtta aaatcgtgag tgtaggatat attaaaatat 2220
   tgagtgttgt atatgtatta tttgtaggga gaaaggtttt tttttttttt ttgattgagt 2280
   tttatttttg ttgtttaggt tggagtgtaa tggagcgatc gtagtttatt atagttttcg 2340
   tttttcgggt ttaagcgatt tttttgtttt agttttttga gtagttgaga ttataggtat 2400
   gaattatcgg gttcggttaa ttttgtattt ttagtagaga cggggttttt ttatgttggt 2460
   taggttggtt tcgattttcg attttaggcg attcgtcggt ttcggttttt taaggtgttg 2520
   agattatagg tatgagttat tgcgcgtagt aagaaaggta ttttttatta agataaattt 2580
   aattaaaaat aaggggttag gtgtggtggg ttatatttat aattttagta tttgggaaat 2640
   taaggtagga ggattatatg aggtttggag tttgagatta gttttgggta atatagtgag 2700
   attttatttt tataaagtag aaagaaagaa aatgtaggaa aataagaaag ggatggaaaa 2760
   ttatgagatt aaagaagaaa aggtttttaa agatagtgaa tgatgagagg agatagaaga 2820
   gatttttatt aaacggaaaa agatttaata gagtattgta ttggtagggt ttttcggggt 2880
   atcgatgttt agttgggatt gagatatttg ggaggttgta ggtaaggtta ttttttttgt 2940
   tgaagttttt agtttgttgt ttaatttgtt cgatttgaaa ggaaaggagt ttaagatatg 3000
   a 3001
<210> 563
   <211> 3001
   <212> DNA
   <213> human
<400> 563
   tgagatggat ttttgttttg ttgtttaggt tggagtgtag tggtgttgtt ttggtttatt      60
   gtaagttttg tttgttgtgt ttatgttatt tttttgtttt agttttttga gtagttggga     120
   ttgtaggtgt ttgttattat gtttggttaa tttttttttt tttttttttt ttttttgtat     180
   ttttagtaga gaatggtttt attgtgttag ttaggatggt tttgattttt tgattttgtg     240
   atttgtttat tttggttttt taaagtgttg ggattatagg tgtgagttat tgtgtttggt     300
   tttttttttt attatttatt gaagaattat tagttttttg gagagtattt gatatatagt      360
   gtgtatttgg taaatatttg tttaattgaa tttggtattt attgtatttt tagatgttat     420
   aagtttgaga ttagtagaaa gtgatgtaga atttagaatg ggtgatatgg taaatgtttt     480
   attaaatata gggtttggtg tggtggttta tatttgtaat tttagtattt tgggaggtta     540
   aggtgggtag attataaggt taggagtttg agattagttt ggttaatatg gtgaaatttt     600
   gtttttatta aaaatataaa aattagtggg gtgtggtggt gggtgtttat agttttagtt     660
   atttgggagg ttgaggtagg agaattgttt gaatttggga ggtggaggtt gtagtgagtt     720
   gagattgtat tattgtattt tagtttgggt gatagagaga gattttatat taaaaaaaaa     780
   aaaaaaaaaa aaaaaaggat atggattgta ttttataaat agttgaattt tttattttta     840
   ttgttttgat agttggtttg gtttttagaa atttagattt tttttttttt aggtgagatt     900
   atgtgaagaa aggaatattg gtaaaattat ttttttgatt tttttttttt aggttgtttt     960
   tttttttttt ttaagattgt gtattttgtt ttaaaaaaaa aagaaaaaaa aaagattgta    1020
   gatttagttt ataaatttat aggattaaga atttttatta atattttttt taataatttt    1080
   aaatttttga tagtagtatt tatttgattt tgttttgtat ggatagattt attttaattt    1140
   ttttgtagat tgttttaatt ttaagtagat gtgagagtat aaaatagtgt atttgttttt    1200
   tttttaggtt tttttggtag tattttgttt gtgaaattaa gaggttttag ttgttattag    1260
   aagagggaag gagtaaggat gagtttttga aaaaaaaaaa aaaaaaaaaa tttagggtgg    1320
   agagagtgga aggatgtggt tttaagagag gggggaggga gggtgttatt gtttgtgggg    1380
   tgggggtggg ggtagttttg ataatataga attttgagag attataagat tgttttaggg    1440
   gggtggggtg gggtggggtg gggttggggg tttgttgttt ttttaggttt tattttttgt    1500
   ggagattata aatagttatg attttagtga gatttagaga tgttgtaatg gtaagatttt    1560
   ggattttttt tgaggatgtg gagaaaattt gttgttgaga aggatatttt gaaggttttg    1620
   ttggttgaaa aagttgtttt tggaattatt tttagatttt ttttgaagat ttgaattaga    1680
   ttatagtgat ggggatatag aaggtaagaa tgatataatt attggttatt tttgtttgtt    1740
   tgggatatat ttagaaaggt aaatattaat ggtaattttt ttgttttatt tttttttaag    1800
   ttattaagtt attttaaggt ttttttattt tgtttttagt ttgtattgtt gttgttttat    1860
   tagttttttt tgaatgttat atagttttgt ttattttatt ataggaaagt tatttggaga    1920
   agttgtgttt gttttttagg tttattttag gtaattatat gttattgagg atgagatggt   1980
   tgagtgttta gaggattggg ttgtttggtt gtatggatta taagtgtggg ttttagagtt    2040
   agtgtgaaga tttttttgga agtattagat tttgaaaagg tttgtgtttg gaggtatagg   2100
   ttagggtttg gttatttgtt tgttttataa tatatggggt ttgtggatgt taaggagaaa    2160
   gaattttagt tgtttttttt attagggtta aaattgtgag tgtaggatat attaaaatat    2220
   tgagtgttgt atatgtatta tttgtaggga gaaaggtttt tttttttttt ttgattgagt    2280
   tttatttttg ttgtttaggt tggagtgtaa tggagtgatt gtagtttatt atagtttttg   2340
   ttttttgggt ttaagtgatt tttttgtttt agttttttga gtagttgaga ttataggtat  2400
   gaattattgg gtttggttaa ttttgtattt ttagtagaga tggggttttt ttatgttggt    2460
   taggttggtt ttgatttttg attttaggtg atttgttggt tttggttttt taaggtgttg   2520
   agattatagg tatgagttat tgtgtgtagt aagaaaggta ttttttatta agataaattt   2580
   aattaaaaat aaggggttag gtgtggtggg ttatatttat aattttagta tttgggaaat    2640
   taaggtagga ggattatatg aggtttggag tttgagatta gttttgggta atatagtgag    2700
   attttatttt tataaagtag aaagaaagaa aatgtaggaa aataagaaag ggatggaaaa    2760
   ttatgagatt aaagaagaaa aggtttttaa agatagtgaa tgatgagagg agatagaaga    2820
   gatttttatt aaatggaaaa agatttaata gagtattgta ttggtagggt tttttggggt    2880
   attgatgttt agttgggatt gagatatttg ggaggttgta ggtaaggtta ttttttttgt   2940
   tgaagttttt agtttgttgt ttaatttgtt tgatttgaaa ggaaaggagt ttaagatatg    3000
   a                                                                    3001
<210> 564
   <211> 3001
   <212> DNA
   <213> human
<400> 564
   ttatatttta aatttttttt tttttaggtc gggtagatta ggtagtaggt tgggagtttt 60
   agtaggagaa gtgattttgt ttgtagtttt ttagatgttt tagttttagt taagtatcgg 120
   tatttcggag ggttttgtta gtgtaatgtt ttgttaaatt ttttttcgtt tagtaaaaat 180
   tttttttgtt tttttttatt atttattatt tttgagagtt tttttttttt tagttttata 240
   attttttatt ttttttttat ttttttatat tttttttttt tttgttttgt agagatggga 300
   ttttattata ttgtttaagg ttagttttaa attttaggtt ttatgtgatt tttttgtttt 360
   ggttttttaa atgttggggt tataggtgtg atttattata tttggttttt tatttttagt 420
   taaatttatt ttggtaaaaa atgttttttt tgttgcgcgt agtggtttat gtttgtaatt 480
   ttagtatttt gggaggtcga ggtcggcgga tcgtttgagg tcgggagtcg agattagttt 540
   gattaatata gagaaatttc gtttttatta aaaatataaa attagtcggg ttcggtggtt 600
   tatgtttgta attttagtta tttaggaggt tgaggtagga gaatcgtttg aattcgggag 660
   gcggaggttg tggtgagttg cggtcgtttt attgtatttt agtttgggta ataagagtaa 720
   aatttaatta gaaaaaaaag aaaagttttt ttttttgtaa ataatgtata tataatattt 780
   aatattttag tatgttttgt atttacgatt ttaattttaa taggaaggat aattggaatt 840
   tttttttttt agtatttacg aattttatat gttataggat aaatagatgg ttagattttg 900
   gtttgtgttt ttaaatatag atttttttaa ggtttaatgt ttttagagaa gtttttatat 960
   tggttttgag atttatattt gtaatttata taattaagta gttcgatttt ttaaatattt 1020
   aattatttcg tttttagtgg tatatggtta tttaaaataa atttgagaag taaatataat 1080
   tttttcgggt gattttttta tgataaggtg aatagaattg tatgatattt aaagagaatt 1140
   gatgaagcgg tagtagtgta ggttagagac gaaatgaaaa ggttttaaga tagtttggtg 1200
   atttaggaga aaatagaata aggagattat tattaatatt tgtttttttg agtgtatttt 1260
   aaataagtaa aaataattag taattatatt atttttattt tttgtgtttt tatcgttgta 1320
   atttaattta agtttttaag aaagatttag gggtgatttt agaaatagtt tttttagtta 1380
   ataaaatttt taaaatgttt tttttagtag taagtttttt ttacgttttt aggaaggatt 1440
   taaagtttta ttattatagt atttttgggt ttcgttggga ttatgattat ttataatttt 1500
   cgtaaagggt ggagtttgaa agggcgataa gtttttagtt ttattttatt ttattttatt 1560
   tttttgaagt aattttgtga tttttcggaa ttttgtgtta ttagaattat tttcgttttc 1620
   gttttataga taataacgtt tttttttttt ttttttttaa aattatattt ttttattttt 1680
   tttattttgg gttttttttt tttttttttt tttaaaagtt tatttttatt tttttttttt 1740
   tttaatagta gttagagttt tttagtttta tagataaaat attattagaa aagtttggag 1800
   ggagggtaaa tatattattt tatgttttcg tatttgtttg ggattaaaat aatttataga 1860
   gagattagaa taaatttatt tatataaaac gaagttaagt gggtgttatt attagagatt 1920
   taaggttgtt agaaagagtg ttagtaagaa tttttgattt tatgaattta taggttgggt 1980
   ttataatttt tttttttttt tttttttttg agacggagta tataatttta aaggggaggg 2040
   aaagatagtt tgagagaaga aaattaggga aataattttg ttagtgtttt ttttttcgta 2100
   taattttatt tggagggaag aaaatttgaa tttttaggag ttagattaat tattagggta 2160
   gtaggaataa gaggtttaat tgtttatgga atataattta tatttttttt tttttttttt 2220
   tttttttttt gatatggagt ttttttttgt cgtttaggtt ggagtgtagt ggtgcgattt 2280
   cggtttattg taattttcgt ttttcgggtt taagtaattt ttttgtttta gtttttcgag 2340
   tagttgggat tataggcgtt cgttattacg tttcgttaat ttttgtattt ttagtaaaga 2400
   cggggtttta ttatattggt taggttggtt ttaaattttt gattttgtga tttgttcgtt 2460
   ttggtttttt aaagtgttgg gattataagt gtgagttatc gcgtcgagtt ttatatttaa 2520
   taaggtattt gttatattat ttattttgaa ttttgtatta ttttttgtta gttttaagtt 2580
   tatagtattt agaaatgtaa taaatatcga atttaattaa gtaggtattt gtcgaatata 2640
   cgttatgtat taggtatttt ttaagaagtt ggtgattttt tagtgaataa tagaaaaaaa 2700
   ggtcgggcgc ggtggtttac gtttgtaatt ttagtatttt gggaggtcga ggtgggcgga 2760
   ttacgaggtt aggagattaa gattattttg gttaatacgg tgaaattatt ttttattaaa 2820
   aatataaaaa aaaaaaaaaa aaaaaaaaaa attagtcggg tatggtggcg ggcgtttgta 2880
   gttttagtta ttcgggaggt tgagatagga gaatggcgtg aacgcggtag gcggagtttg 2940
   tagtgagtta agatagcgtt attgtatttt agtttgggcg atagagtaag aattcgtttt 3000
   a 3001
<210> 565
   <211> 3001
   <212> DNA
   <213> human
<400> 565
   ttatatttta aatttttttt tttttaggtt gggtagatta ggtagtaggt tgggagtttt 60
   agtaggagaa gtgattttgt ttgtagtttt ttagatgttt tagttttagt taagtattgg 120
   tattttggag ggttttgtta gtgtaatgtt ttgttaaatt tttttttgtt tagtaaaaat 180
   tttttttgtt tttttttatt atttattatt tttgagagtt tttttttttt tagttttata 240
   attttttatt ttttttttat ttttttatat tttttttttt tttgttttgt agagatggga 300
   ttttattata ttgtttaagg ttagttttaa attttaggtt ttatgtgatt tttttgtttt 360
   ggttttttaa atgttggggt tataggtgtg atttattata tttggttttt tatttttagt 420
   taaatttatt ttggtaaaaa atgttttttt tgttgtgtgt agtggtttat gtttgtaatt 480
   ttagtatttt gggaggttga ggttggtgga ttgtttgagg ttgggagttg agattagttt 540
   gattaatata gagaaatttt gtttttatta aaaatataaa attagttggg tttggtggtt 600
   tatgtttgta attttagtta tttaggaggt tgaggtagga gaattgtttg aatttgggag 660
   gtggaggttg tggtgagttg tggttgtttt attgtatttt agtttgggta ataagagtaa 720
   aatttaatta gaaaaaaaag aaaagttttt ttttttgtaa ataatgtata tataatattt 780
   aatattttag tatgttttgt atttatgatt ttaattttaa taggaaggat aattggaatt 840
   tttttttttt agtatttatg aattttatat gttataggat aaatagatgg ttagattttg 900
   gtttgtgttt ttaaatatag atttttttaa ggtttaatgt ttttagagaa gtttttatat 960
   tggttttgag atttatattt gtaatttata taattaagta gtttgatttt ttaaatattt 1020
   aattattttg tttttagtgg tatatggtta tttaaaataa atttgagaag taaatataat 1080
   ttttttgggt gattttttta tgataaggtg aatagaattg tatgatattt aaagagaatt 1140
   gatgaagtgg tagtagtgta ggttagagat gaaatgaaaa ggttttaaga tagtttggtg 1200
   atttaggaga aaatagaata aggagattat tattaatatt tgtttttttg agtgtatttt 1260
   aaataagtaa aaataattag taattatatt atttttattt tttgtgtttt tattgttgta 1320
   atttaattta agtttttaag aaagatttag gggtgatttt agaaatagtt tttttagtta 1380
   ataaaatttt taaaatgttt tttttagtag taagtttttt ttatgttttt aggaaggatt 1440
   taaagtttta ttattatagt atttttgggt tttgttggga ttatgattat ttataatttt 1500
   tgtaaagggt ggagtttgaa agggtgataa gtttttagtt ttattttatt ttattttatt 1560
   tttttgaagt aattttgtga ttttttggaa ttttgtgtta ttagaattat ttttgttttt 1620
   gttttataga taataatgtt tttttttttt ttttttttaa aattatattt ttttattttt 1680
   tttattttgg gttttttttt tttttttttt tttaaaagtt tatttttatt tttttttttt 1740
   tttaatagta gttagagttt tttagtttta tagataaaat attattagaa aagtttggag 1800
   ggagggtaaa tatattattt tatgtttttg tatttgtttg ggattaaaat aatttataga 1860
   gagattagaa taaatttatt tatataaaat gaagttaagt gggtgttatt attagagatt 1920
   taaggttgtt agaaagagtg ttagtaagaa tttttgattt tatgaattta taggttgggt 1980
   ttataatttt tttttttttt tttttttttg agatggagta tataatttta aaggggaggg 2040
   aaagatagtt tgagagaaga aaattaggga aataattttg ttagtgtttt tttttttgta 2100
   taattttatt tggagggaag aaaatttgaa tttttaggag ttagattaat tattagggta 2160
   gtaggaataa gaggtttaat tgtttatgga atataattta tatttttttt tttttttttt 2220
   tttttttttt gatatggagt ttttttttgt tgtttaggtt ggagtgtagt ggtgtgattt 2280
   tggtttattg taatttttgt tttttgggtt taagtaattt ttttgtttta gttttttgag 2340
   tagttgggat tataggtgtt tgttattatg ttttgttaat ttttgtattt ttagtaaaga 2400
   tggggtttta ttatattggt taggttggtt ttaaattttt gattttgtga tttgtttgtt 2460
   ttggtttttt aaagtgttgg gattataagt gtgagttatt gtgttgagtt ttatatttaa 2520
   taaggtattt gttatattat ttattttgaa ttttgtatta ttttttgtta gttttaagtt 2580
   tatagtattt agaaatgtaa taaatattga atttaattaa gtaggtattt gttgaatata 2640
   tgttatgtat taggtatttt ttaagaagtt ggtgattttt tagtgaataa tagaaaaaaa 2700
   ggttgggtgt ggtggtttat gtttgtaatt ttagtatttt gggaggttga ggtgggtgga 2760
   ttatgaggtt aggagattaa gattattttg gttaatatgg tgaaattatt ttttattaaa 2820
   aatataaaaa aaaaaaaaaa aaaaaaaaaa attagttggg tatggtggtg ggtgtttgta 2880
   gttttagtta tttgggaggt tgagatagga gaatggtgtg aatgtggtag gtggagtttg 2940
   tagtgagtta agatagtgtt attgtatttt agtttgggtg atagagtaag aatttgtttt 3000
   a 3001
<210> 566
   <211> 50
   <212> DNA
   <213> human
<400> 566
   ctgaggtttc tagacgtgac ccagggcaga ctggtagcaa agcccccacg 50
<210> 567
   <211> 50
   <212> DNA
   <213> human
<400> 567
   ttgaggtttt tagacgtgat ttagggtaga ttggtagtaa agtttttacg 50
<210> 568
   <211> 50
   <212> DNA
   <213> human
<400> 568
   ttgaggtttt tagatgtgat ttagggtaga ttggtagtaa agtttttatg 50
<210> 569
   <211> 50
   <212> DNA
   <213> human
<400> 569
   cgtgggggtt ttgttattag tttgttttgg gttacgttta gaaattttag 50
<210> 570
   <211> 50
   <212> DNA
   <213> human
<400> 570
   tgtgggggtt ttgttattag tttgttttgg gttatgttta gaaattttag 50
<210> 571
   <211> 3001
   <212> DNA
   <213> human
<400> 571
   ttttcaggta tatacatgct atttttattc ttaaggagac tcttgaattg ttagcatata    60
   tacttagata ttttctatca aaatgcccac tgacaagcat ctatattcct ccccagatag   120
   gatggaacgg caatgtgctc cctggcccct cccaacccag tcctctaggc gttttgtttc   180
   acagcgttaa tattcttacc actatttttt aaatcaacag gttatgacat cataattttt   240
   acttatttct ttgcccaact ttgtatcatc tgctcttgta tttctcatgg atttcttcat   300
   gtctatttct gcaagacatc tttaaaacag agtttaggag ccgggagcca tggctcacgc   360
   cagtaatccc agcactttgg aaggcagagg tggatggatc acttgaagcc aggagttcaa   420
   gaccagcctg gtcaacatag tgaaaccctg tctctactaa gaatacaaaa attacccagg   480
   cgtggtggta catgtgcctg taatcccagc tacttgggag gctgaggcag gagaattgct   540
   tgaacccggg aggcagaggt tgcagtgagc tgagatcacg ccactgcact ccagcctggg   600
   tgacaaaagc aaaactctgt ctcaaaaaaa actgaaaaca taaaaataaa taaataaata   660
   aaacagagtt tagggtagta atgtgtctga gtccttgctc tgaagatgtc tgatttgggt   720
   tttttttcta ggtataatct ctttccctcc tcttcctcct cctcctcctc tcccttctct   780
   ctctctctga gcctgtagta ttttcccttt cgtgttctgt ggtatcctcc taatgtccct   840
   agttaacagg acctcatcca tccatccctg gggtgttcag tggcctcgca atccacgtcc   900
   ccgcccagat ccgcagcctt cctcctgtga cctgagtgtc tcctcctcgc tgtccctgtt   960
   ctgtcccctc ccgccctcac atgcaggtgg atgtcaatgc gtgcacgcac ccaggttttc  1020
   ttgcctgttc ccacctcttt atcctccttg gctggttcct ccgctccatt ctctggctca  1080
   ggaatttgct ccccagggtt tttcattccg ctctgtagca gaatgtgcta ctgttgcttt  1140
   ccagcatcaa acttttcact cccatggcct ctgtgttctt cttcctaatg ggtgcccttt  1200
   ctgcctggcg ctcctggagg ccgggcccct ctcccaagtg tgaatctgat ggaagcaggt  1260
   ccttctcgcc gggacctcat gtgagaaatg acatgtccca cctgtctcaa ggggccgctc  1320
   tctgacatca gagctgctgt agagcggaga ggggcagggg tgaagggcca cggtggtgca  1380
   acccaccact tcctccaagg aggagctgag aggaacagga agtgtcagga ctttacgacc  1440
   cgcgcctcca gctgaggttt ctagacgtga cccagggcag actggtagca aagcccccac  1500
   gcccagccag gagcaccgcc gaggactcca gcacaccgag ggtgagtgtg cagaggcccc  1560
   gctatgtcca cggtgatttt tggagggaag cactctcctg ccccagcctg gagtgaccct  1620
   ggtggccctg cttatagccc cggcccaggg gtgtctggca ggaaggggtc tccagaccgt  1680
   ctgctccagg gtcccctttg agacccggca aggggaagtg cctgccacca agagcagagc  1740
   cacatggtgt gctgggagcg cttgcggctg aggatggggc cggcatggga ggacaagggg  1800
   ctgtgaggcc ggcaaggcca ggaagaggct gggcccacaa gcccctaggg ggacggctgg  1860
   ggggtcctgg tgctccgggc tgggcaggag gcaggttccc ggggcctcct ctgtgaacct  1920
   gctgcgcctg cctgtgttag gccctcctgt ctgcatgctg ccccgcctgg ctacagcgat  1980
   tccatctcca gatcgactcg cagtgtgtgg tgtgtgatat ggtgtggtgt ggttcgtggt  2040
   gtgtagtgtc gtgtgtgtag tgtggcatgg tgtgcatgta tggtgcacgt gtgtggcata  2100
   gcgtagtgtg gtgtgtgtgt gtgcatgtat ggtgtgcgag tgtgcgcatg tgatgtgggg  2160
   agtctgttgt atgtacatgt atggtgcaca tgtgtaatgt agtgtggtgt ggtgtggcac  2220
   atgctccatg ctcctctggg accctcgagc ttccctgcct cctccgtggg gacccctggg  2280
   tgacgtcttg ctgtagggga gcgggcaccg ctgtgcgatt ctggtcgagt tggttcagct  2340
   ggtgaccctg gcctccccac aacaggctcc tggaggccat tcccaaggtc ctggtgggag  2400
   ggggattgaa gccacgcccc tgggtcccag gcccacctgg cccccttccc ccaacactgc  2460
   actcttgggg ccagccctcc ccatgcatcc tccacccctg gcaccacccc ttgcaccacc  2520
   gtccccacac ccaatccccc ctcccctgga tctgggagct gggattctgc cctgctgtcc  2580
   ctgtccatcc ggggcccctc tcctctgtca gttcctgggt ggggagaagg aggggtggca  2640
   tggctgcaat ggggaagggg ggggatggcc ggagagctca gcctgggagc gagctgtggg  2700
   ggcatagggg acactcgctg tcactcgcct tccccgtgac ggtggcgcaa cgcctgcggg  2760
   tttgggggct gaggccggat tctggaggct tcacagaaat gagctgctag aagctctggg  2820
   ctgtctgccg tcaccccagc gtttcaccat gagtgaagcc cagctcactg cagaatccat  2880
   gcccagctca ctgcagaatc cccgggggcc agcactttac caagagtggc ctccagactc  2940
   cacattttag gaaggaactc cttccccatc cagctcacag cagaatcccc agggtccctg  3000
   c                                                                  3001
<210> 572
   <211> 3001
   <212> DNA
   <213> human
<400> 572
   tttttaggta tatatatgtt atttttattt ttaaggagat ttttgaattg ttagtatata 60
   tatttagata ttttttatta aaatgtttat tgataagtat ttatattttt ttttagatag 120
   gatggaacgg taatgtgttt tttggttttt tttaatttag ttttttaggc gttttgtttt 180
   atagcgttaa tatttttatt attatttttt aaattaatag gttatgatat tataattttt 240
   atttattttt ttgtttaatt ttgtattatt tgtttttgta ttttttatgg atttttttat 300
   gtttattttt gtaagatatt tttaaaatag agtttaggag tcgggagtta tggtttacgt 360
   tagtaatttt agtattttgg aaggtagagg tggatggatt atttgaagtt aggagtttaa 420
   gattagtttg gttaatatag tgaaattttg tttttattaa gaatataaaa attatttagg 480
   cgtggtggta tatgtgtttg taattttagt tatttgggag gttgaggtag gagaattgtt 540
   tgaattcggg aggtagaggt tgtagtgagt tgagattacg ttattgtatt ttagtttggg 600
   tgataaaagt aaaattttgt tttaaaaaaa attgaaaata taaaaataaa taaataaata 660
   aaatagagtt tagggtagta atgtgtttga gtttttgttt tgaagatgtt tgatttgggt 720
   ttttttttta ggtataattt tttttttttt tttttttttt tttttttttt tttttttttt 780
   ttttttttga gtttgtagta tttttttttt cgtgttttgt ggtatttttt taatgttttt 840
   agttaatagg attttattta tttatttttg gggtgtttag tggtttcgta atttacgttt 900
   tcgtttagat tcgtagtttt ttttttgtga tttgagtgtt tttttttcgt tgtttttgtt 960
   ttgttttttt tcgtttttat atgtaggtgg atgttaatgc gtgtacgtat ttaggttttt 1020
   ttgtttgttt ttattttttt attttttttg gttggttttt tcgttttatt ttttggttta 1080
   ggaatttgtt ttttagggtt ttttatttcg ttttgtagta gaatgtgtta ttgttgtttt 1140
   ttagtattaa attttttatt tttatggttt ttgtgttttt ttttttaatg ggtgtttttt 1200
   ttgtttggcg tttttggagg tcgggttttt tttttaagtg tgaatttgat ggaagtaggt 1260
   ttttttcgtc gggattttat gtgagaaatg atatgtttta tttgttttaa ggggtcgttt 1320
   tttgatatta gagttgttgt agagcggaga ggggtagggg tgaagggtta cggtggtgta 1380
   atttattatt ttttttaagg aggagttgag aggaatagga agtgttagga ttttacgatt 1440
   cgcgttttta gttgaggttt ttagacgtga tttagggtag attggtagta aagtttttac 1500
   gtttagttag gagtatcgtc gaggatttta gtatatcgag ggtgagtgtg tagaggtttc 1560
   gttatgttta cggtgatttt tggagggaag tatttttttg ttttagtttg gagtgatttt 1620
   ggtggttttg tttatagttt cggtttaggg gtgtttggta ggaaggggtt tttagatcgt 1680
   ttgttttagg gttttttttg agattcggta aggggaagtg tttgttatta agagtagagt 1740
   tatatggtgt gttgggagcg tttgcggttg aggatggggt cggtatggga ggataagggg 1800
   ttgtgaggtc ggtaaggtta ggaagaggtt gggtttataa gtttttaggg ggacggttgg 1860
   ggggttttgg tgtttcgggt tgggtaggag gtaggttttc ggggtttttt ttgtgaattt 1920
   gttgcgtttg tttgtgttag gtttttttgt ttgtatgttg tttcgtttgg ttatagcgat 1980
   tttattttta gatcgattcg tagtgtgtgg tgtgtgatat ggtgtggtgt ggttcgtggt 2040
   gtgtagtgtc gtgtgtgtag tgtggtatgg tgtgtatgta tggtgtacgt gtgtggtata 2100
   gcgtagtgtg gtgtgtgtgt gtgtatgtat ggtgtgcgag tgtgcgtatg tgatgtgggg 2160
   agtttgttgt atgtatatgt atggtgtata tgtgtaatgt agtgtggtgt ggtgtggtat 2220
   atgttttatg tttttttggg attttcgagt ttttttgttt ttttcgtggg gatttttggg 2280
   tgacgttttg ttgtagggga gcgggtatcg ttgtgcgatt ttggtcgagt tggtttagtt 2340
   ggtgattttg gtttttttat aataggtttt tggaggttat ttttaaggtt ttggtgggag 2400
   ggggattgaa gttacgtttt tgggttttag gtttatttgg tttttttttt ttaatattgt 2460
   atttttgggg ttagtttttt ttatgtattt tttatttttg gtattatttt ttgtattatc 2520
   gtttttatat ttaatttttt tttttttgga tttgggagtt gggattttgt tttgttgttt 2580
   ttgtttattc ggggtttttt ttttttgtta gtttttgggt ggggagaagg aggggtggta 2640
   tggttgtaat ggggaagggg ggggatggtc ggagagttta gtttgggagc gagttgtggg 2700
   ggtatagggg atattcgttg ttattcgttt ttttcgtgac ggtggcgtaa cgtttgcggg 2760
   tttgggggtt gaggtcggat tttggaggtt ttatagaaat gagttgttag aagttttggg 2820
   ttgtttgtcg ttattttagc gttttattat gagtgaagtt tagtttattg tagaatttat 2880
   gtttagttta ttgtagaatt ttcgggggtt agtattttat taagagtggt ttttagattt 2940
   tatattttag gaaggaattt tttttttatt tagtttatag tagaattttt agggtttttg 3000
   t 3001
<210> 573
   <211> 3001
   <212> DNA
   <213> human
<400> 573
   tttttaggta tatatatgtt atttttattt ttaaggagat ttttgaattg ttagtatata 60
   tatttagata ttttttatta aaatgtttat tgataagtat ttatattttt ttttagatag 120
   gatggaatgg taatgtgttt tttggttttt tttaatttag ttttttaggt gttttgtttt 180
   atagtgttaa tatttttatt attatttttt aaattaatag gttatgatat tataattttt 240
   atttattttt ttgtttaatt ttgtattatt tgtttttgta ttttttatgg atttttttat 300
   gtttattttt gtaagatatt tttaaaatag agtttaggag ttgggagtta tggtttatgt 360
   tagtaatttt agtattttgg aaggtagagg tggatggatt atttgaagtt aggagtttaa 420
   gattagtttg gttaatatag tgaaattttg tttttattaa gaatataaaa attatttagg 480
   tgtggtggta tatgtgtttg taattttagt tatttgggag gttgaggtag gagaattgtt 540
   tgaatttggg aggtagaggt tgtagtgagt tgagattatg ttattgtatt ttagtttggg 600
   tgataaaagt aaaattttgt tttaaaaaaa attgaaaata taaaaataaa taaataaata 660
   aaatagagtt tagggtagta atgtgtttga gtttttgttt tgaagatgtt tgatttgggt 720
   ttttttttta ggtataattt tttttttttt tttttttttt tttttttttt tttttttttt 780
   ttttttttga gtttgtagta tttttttttt tgtgttttgt ggtatttttt taatgttttt 840
   agttaatagg attttattta tttatttttg gggtgtttag tggttttgta atttatgttt 900
   ttgtttagat ttgtagtttt ttttttgtga tttgagtgtt ttttttttgt tgtttttgtt 960
   ttgttttttt ttgtttttat atgtaggtgg atgttaatgt gtgtatgtat ttaggttttt 1020
   ttgtttgttt ttattttttt attttttttg gttggttttt ttgttttatt ttttggttta 1080
   ggaatttgtt ttttagggtt ttttattttg ttttgtagta gaatgtgtta ttgttgtttt 1140
   ttagtattaa attttttatt tttatggttt ttgtgttttt ttttttaatg ggtgtttttt 1200
   ttgtttggtg tttttggagg ttgggttttt tttttaagtg tgaatttgat ggaagtaggt 1260
   tttttttgtt gggattttat gtgagaaatg atatgtttta tttgttttaa ggggttgttt 1320
   tttgatatta gagttgttgt agagtggaga ggggtagggg tgaagggtta tggtggtgta 1380
   atttattatt ttttttaagg aggagttgag aggaatagga agtgttagga ttttatgatt 1440
   tgtgttttta gttgaggttt ttagatgtga tttagggtag attggtagta aagtttttat 1500
   gtttagttag gagtattgtt gaggatttta gtatattgag ggtgagtgtg tagaggtttt 1560
   gttatgttta tggtgatttt tggagggaag tatttttttg ttttagtttg gagtgatttt 1620
   ggtggttttg tttatagttt tggtttaggg gtgtttggta ggaaggggtt tttagattgt 1680
   ttgttttagg gttttttttg agatttggta aggggaagtg tttgttatta agagtagagt 1740
   tatatggtgt gttgggagtg tttgtggttg aggatggggt tggtatggga ggataagggg 1800
   ttgtgaggtt ggtaaggtta ggaagaggtt gggtttataa gtttttaggg ggatggttgg 1860
   ggggttttgg tgttttgggt tgggtaggag gtaggttttt ggggtttttt ttgtgaattt 1920
   gttgtgtttg tttgtgttag gtttttttgt ttgtatgttg ttttgtttgg ttatagtgat 1980
   tttattttta gattgatttg tagtgtgtgg tgtgtgatat ggtgtggtgt ggtttgtggt 2040
   gtgtagtgtt gtgtgtgtag tgtggtatgg tgtgtatgta tggtgtatgt gtgtggtata 2100
   gtgtagtgtg gtgtgtgtgt gtgtatgtat ggtgtgtgag tgtgtgtatg tgatgtgggg 2160
   agtttgttgt atgtatatgt atggtgtata tgtgtaatgt agtgtggtgt ggtgtggtat 2220
   atgttttatg tttttttggg atttttgagt ttttttgttt tttttgtggg gatttttggg 2280
   tgatgttttg ttgtagggga gtgggtattg ttgtgtgatt ttggttgagt tggtttagtt 2340
   ggtgattttg gtttttttat aataggtttt tggaggttat ttttaaggtt ttggtgggag 2400
   ggggattgaa gttatgtttt tgggttttag gtttatttgg tttttttttt ttaatattgt 2460
   atttttgggg ttagtttttt ttatgtattt tttatttttg gtattatttt ttgtattatt 2520
   gtttttatat ttaatttttt tttttttgga tttgggagtt gggattttgt tttgttgttt 2580
   ttgtttattt ggggtttttt ttttttgtta gtttttgggt ggggagaagg aggggtggta 2640
   tggttgtaat ggggaagggg ggggatggtt ggagagttta gtttgggagt gagttgtggg 2700
   ggtatagggg atatttgttg ttatttgttt tttttgtgat ggtggtgtaa tgtttgtggg 2760
   tttgggggtt gaggttggat tttggaggtt ttatagaaat gagttgttag aagttttggg 2820
   ttgtttgttg ttattttagt gttttattat gagtgaagtt tagtttattg tagaatttat 2880
   gtttagttta ttgtagaatt tttgggggtt agtattttat taagagtggt ttttagattt 2940
   tatattttag gaaggaattt tttttttatt tagtttatag tagaattttt agggtttttg 3000
   t 3001
<210> 574
   <211> 3001
   <212> DNA
   <213> human
<400> 574
   gtagggattt tggggatttt gttgtgagtt ggatggggaa ggagtttttt tttaaaatgt     60
   ggagtttgga ggttattttt ggtaaagtgt tggttttcgg ggattttgta gtgagttggg    120
   tatggatttt gtagtgagtt gggttttatt tatggtgaaa cgttggggtg acggtagata    180
   gtttagagtt tttagtagtt tatttttgtg aagtttttag aattcggttt tagtttttaa    240
   attcgtaggc gttgcgttat cgttacgggg aaggcgagtg atagcgagtg ttttttatgt    300
   ttttatagtt cgtttttagg ttgagttttt cggttatttt tttttttttt tattgtagtt    360
   atgttatttt tttttttttt tatttaggaa ttgatagagg agaggggttt cggatggata    420
   gggatagtag ggtagaattt tagtttttag atttagggga ggggggattg ggtgtgggga    480
   cggtggtgta aggggtggtg ttaggggtgg aggatgtatg ggagggttgg gttttaagag    540
   tgtagtgttg ggggaagggg gttaggtggg tttgggattt aggggcgtgg ttttaatttt    600
   ttttttatta ggattttggg aatggttttt aggagtttgt tgtggggagg ttagggttat     660
   tagttgaatt aattcgatta gaatcgtata gcggtgttcg tttttttata gtaagacgtt    720
   atttaggggt ttttacggag gaggtaggga agttcgaggg ttttagagga gtatggagta    780
   tgtgttatat tatattatat tatattatat atgtgtatta tatatgtata tataatagat    840
   tttttatatt atatgcgtat attcgtatat tatatatgta tatatatata ttatattacg    900
   ttatgttata tacgtgtatt atatatgtat attatgttat attatatata cgatattata    960
   tattacgaat tatattatat tatattatat attatatatt gcgagtcgat ttggagatgg   1020
   aatcgttgta gttaggcggg gtagtatgta gataggaggg tttaatatag gtaggcgtag   1080
   taggtttata gaggaggttt cgggaatttg ttttttgttt agttcggagt attaggattt   1140
   tttagtcgtt tttttagggg tttgtgggtt tagttttttt ttggttttgt cggttttata   1200
   gttttttgtt tttttatgtc ggttttattt ttagtcgtaa gcgtttttag tatattatgt    1260
   ggttttgttt ttggtggtag gtattttttt ttgtcgggtt ttaaagggga ttttggagta   1320
   gacggtttgg agattttttt ttgttagata tttttgggtc ggggttataa gtagggttat   1380
   tagggttatt ttaggttggg gtaggagagt gttttttttt aaaaattatc gtggatatag   1440
   cggggttttt gtatatttat tttcggtgtg ttggagtttt cggcggtgtt tttggttggg   1500
   cgtgggggtt ttgttattag tttgttttgg gttacgttta gaaattttag ttggaggcgc   1560
   gggtcgtaaa gttttgatat tttttgtttt ttttagtttt tttttggagg aagtggtggg   1620
   ttgtattatc gtggtttttt atttttgttt tttttcgttt tatagtagtt ttgatgttag   1680
   agagcggttt tttgagatag gtgggatatg ttatttttta tatgaggttt cggcgagaag   1740
   gatttgtttt tattagattt atatttggga gaggggttcg gtttttagga gcgttaggta   1800
   gaaagggtat ttattaggaa gaagaatata gaggttatgg gagtgaaaag tttgatgttg   1860
   gaaagtaata gtagtatatt ttgttataga gcggaatgaa aaattttggg gagtaaattt   1920
   ttgagttaga gaatggagcg gaggaattag ttaaggagga taaagaggtg ggaataggta   1980
   agaaaatttg ggtgcgtgta cgtattgata tttatttgta tgtgagggcg ggaggggata   2040
   gaatagggat agcgaggagg agatatttag gttataggag gaaggttgcg gatttgggcg   2100
   gggacgtgga ttgcgaggtt attgaatatt ttagggatgg atggatgagg ttttgttaat   2160
   tagggatatt aggaggatat tatagaatac gaaagggaaa atattatagg tttagagaga   2220
   gagagaaggg agaggaggag gaggaggaag aggagggaaa gagattatat ttagaaaaaa   2280
   aatttaaatt agatattttt agagtaagga tttagatata ttattatttt aaattttgtt   2340
   ttatttattt atttattttt atgtttttag ttttttttga gatagagttt tgtttttgtt   2400
   atttaggttg gagtgtagtg gcgtgatttt agtttattgt aatttttgtt tttcgggttt   2460
   aagtaatttt tttgttttag ttttttaagt agttgggatt ataggtatat gtattattac   2520
   gtttgggtaa tttttgtatt tttagtagag atagggtttt attatgttga ttaggttggt   2580
   tttgaatttt tggttttaag tgatttattt atttttgttt tttaaagtgt tgggattatt   2640
   ggcgtgagtt atggttttcg gtttttaaat tttgttttaa agatgttttg tagaaataga   2700
   tatgaagaaa tttatgagaa atataagagt agatgatata aagttgggta aagaaataag   2760
   taaaaattat gatgttataa tttgttgatt taaaaaatag tggtaagaat attaacgttg   2820
   tgaaataaaa cgtttagagg attgggttgg gaggggttag ggagtatatt gtcgttttat   2880
   tttatttggg gaggaatata gatgtttgtt agtgggtatt ttgatagaaa atatttaagt   2940
   atatatgtta ataatttaag agttttttta agaataaaaa tagtatgtat atatttgaaa   3000
   a                                                                   3001
<210> 575
   <211> 3001
   <212> DNA
   <213> human
<400> 575
   gtagggattt tggggatttt gttgtgagtt ggatggggaa ggagtttttt tttaaaatgt 60
   ggagtttgga ggttattttt ggtaaagtgt tggtttttgg ggattttgta gtgagttggg 120
   tatggatttt gtagtgagtt gggttttatt tatggtgaaa tgttggggtg atggtagata 180
   gtttagagtt tttagtagtt tatttttgtg aagtttttag aatttggttt tagtttttaa 240
   atttgtaggt gttgtgttat tgttatgggg aaggtgagtg atagtgagtg ttttttatgt 300
   ttttatagtt tgtttttagg ttgagttttt tggttatttt tttttttttt tattgtagtt 360
   atgttatttt tttttttttt tatttaggaa ttgatagagg agaggggttt tggatggata 420
   gggatagtag ggtagaattt tagtttttag atttagggga ggggggattg ggtgtgggga 480
   tggtggtgta aggggtggtg ttaggggtgg aggatgtatg gggagggttg gttttaagag 540
   tgtagtgttg ggggaagggg gttaggtggg tttgggattt aggggtgtgg ttttaatttt 600
   ttttttatta ggattttggg aatggttttt aggagtttgt tgtggggagg ttagggttat 660
   tagttgaatt aatttgatta gaattgtata gtggtgtttg tttttttata gtaagatgtt 720
   atttaggggt ttttatggag gaggtaggga agtttgaggg ttttagagga gtatggagta 780
   tgtgttatat tatattatat tatattatat atgtgtatta tatatgtata tataatagat 840
   tttttatatt atatgtgtat atttgtatat tatatatgta tatatatata ttatattatg 900
   ttatgttata tatgtgtatt atatatgtat attatgttat attatatata tgatattata 960
   tattatgaat tatattatat tatattatat attatatatt gtgagttgat ttggagatgg 1020
   aattgttgta gttaggtggg gtagtatgta gataggaggg tttaatatag gtaggtgtag 1080
   taggtttata gaggaggttt tgggaatttg ttttttgttt agtttggagt attaggattt 1140
   tttagttgtt tttttagggg tttgtgggtt tagttttttt ttggttttgt tggttttata 1200
   gttttttgtt tttttatgtt ggttttattt ttagttgtaa gtgtttttag tatattatgt 1260
   ggttttgttt ttggtggtag gtattttttt ttgttgggtt ttaaagggga ttttggagta 1320
   gatggtttgg agattttttt ttgttagata tttttgggtt ggggttataa gtagggttat 1380
   tagggttatt ttaggttggg gtaggagagt gttttttttt aaaaattatt gtggatatag 1440
   tggggttttt gtatatttat ttttggtgtg ttggagtttt tggtggtgtt tttggttggg 1500
   tgtgggggtt ttgttattag tttgttttgg gttatgttta gaaattttag ttggaggtgt 1560
   gggttgtaaa gtttgatatt tttttgtttt ttttagtttt tttttggagg aagtggtggg 1620
   ttgtattatt gtggtttttt atttttgttt ttttttgttt tatagtagtt ttgatgttag 1680
   agagtggttt tttgagatag gtgggatatg ttatttttta tatgaggttt tggtgagaag 1740
   gatttgtttt tattagattt atatttggga gaggggtttg gtttttagga gtgttaggta 1800
   gaaagggtat ttattaggaa gaagaatata gaggttatgg gagtgaaaag tttgatgttg 1860
   gaaagtaata gtagtatatt ttgttataga gtggaatgaa aaattttggg gagtaaattt 1920
   ttgagttaga gaatggagtg gaggaattag ttaaggagga taaagaggtg ggaataggta 1980
   agaaaatttg ggtgtgtgta tgtattgata tttatttgta tgtgagggtg ggaggggata 2040
   gaatagggat agtgaggagg agatatttag gttataggag gaaggttgtg gatttgggtg 2100
   gggatgtgga ttgtgaggtt attgaatatt ttagggatgg atggatgagg ttttgttaat 2160
   tagggatatt aggaggatat tatagaatat gaaagggaaa atattatagg tttagagaga 2220
   gagagaaggg agaggaggag gaggaggaag aggagggaaa gagattatat ttagaaaaaa 2280
   aatttaaatt agatattttt agagtaagga tttagatata ttattatttt aaattttgtt 2340
   ttatttattt atttattttt atgtttttag ttttttttga gatagagttt tgtttttgtt 2400
   atttaggttg gagtgtagtg gtgtgatttt agtttattgt aatttttgtt ttttgggttt 2460
   aagtaatttt tttgttttag ttttttaagt agttgggatt ataggtatat gtattattat 2520
   gtttgggtaa tttttgtatt tttagtagag atagggtttt attatgttga ttaggttggt 2580
   tttgaatttt tggttttaag tgatttattt atttttgttt tttaaagtgt tgggattatt 2640
   ggtgtgagtt atggtttttg gtttttaaat tttgttttaa agatgttttg tagaaataga 2700
   tatgaagaaa tttatgagaa atataagagt agatgatata aagttgggta aagaaataag 2760
   taaaaattat gatgttataa tttgttgatt taaaaaatag tggtaagaat attaatgttg 2820
   tgaaataaaa tgtttagagg attgggttgg gaggggttag ggagtatatt gttgttttat 2880
   tttatttggg gaggaatata gatgtttgtt agtgggtatt ttgatagaaa atatttaagt 2940
   atatatgtta ataatttaag agttttttta agaataaaaa tagtatgtat atatttgaaa 3000
   a 3001
<210> 576
   <211> 50
   <212> DNA
   <213> human
<400> 576
   cctaccggca ttgaaatact tatggataaa gttctcgcaa tggcttcacg 50
<210> 577
   <211> 50
   <212> DNA
   <213> human
<400> 577
   tttatcggta ttgaaatatt tatggataaa gttttcgtaa tggttttacg 50
<210> 578
   <211> 50
   <212> DNA
   <213> human
<400> 578
   tttattggta ttgaaatatt tatggataaa gtttttgtaa tggttttatg 50
<210> 579
   <211> 50
   <212> DNA
   <213> human
<400> 579
   cgtgaagtta ttgcgagaat tttatttata agtattttaa tgtcggtagg 50
<210> 580
   <211> 50
   <212> DNA
   <213> human
<400> 580
   tgtgaagtta ttgtgagaat tttatttata agtattttaa tgttggtagg 50
<210> 581
   <211> 3001
   <212> DNA
   <213> human
<400> 581
   cccaaaacac tacacttggt gaaagaagcc agacacggaa ggtcacatat tgtatgatcc      60
   catttataag aaatattcag aataggtaaa tccatagaga caaaaggagt tttgtagttg     120
   ccaggagttg gaggcaagaa gagtttaaca ggtatgctta atggggcctc ctatgcagtt     180
   gacgaaaatg ttttgggcta gataggagtg atggttgcat aacattgtac taaatgccac     240
   tgaattctac tctttaaaat ttttagtttt atgttatgtg gcgtgtaccc taaaaaaaaa     300
   aatagaggtg cagtgctcca gcacgggatg aggcagcgtg gacaggagca tctcccaacc     360
   tcagtgaagt ctgagccgcg tgcctgcaac aatcccactg tggcagagaa ccgcagagtt     420
   ccttccggtt tggcagcagt cattcgcaac ctcacagccc tctggaaccc cagcctgggg     480
   gtctcagaac gccgaggcgg ggactgggag ccgagtcgga ttccgagact atgggccagg     540
   gttggctgga ttcagttacc tggctgaggc ctggtgagca aaatatccca aacctcgcgt     600
   gatctggaag gggaagccgg ataaatacgg atctccagat gtgccagtct cgagtctatc     660
   gatatgaggt ccccctagag tttctattca tcattttaac cgcatttcat cgatcttgag     720
   acacggcttt tgatatttta tcacctcaag ataaatagtg ttagatgtct aatagcagcg     780
   tttttctaag agatacatga aacaacagtg tcagaaacga tgctgtcttc catgcgatga     840
   aattgttgta ataggtgctc aataaatgtt gacaataaat gagtgaatga atgaaaatta     900
   ttttattttt atttgagctt tggttctgcc atttgctagc agtgtgactc aagagaagcc     960
   agtaaccccc ctgagcttcc ctagttcaca aaatgcttgt catgaagtcg acagcttccg    1020
   gaggctgcga ggctcgcaag aaatgcccac atgaatgtgc gcttagggcg tgagtgctca    1080
   ctccagaaaa ctccaacaca gtgaaaaggc agaagcggtg tttttctttt ttacattttt    1140
   ataagaatat ataaaaaatg atataaatgg acatttacgg tagtggggga aggcatatat    1200
   ctacgttaaa aggcaggaca tttttaaaag ctctattttc taaatgaaaa ctacgaaagc    1260
   ggggtgggtt gtggcggggg cagttgtggc cctgtaggac cttcggtgac tgatgatcta    1320
   agtttcccga ggtttctcag agcctctctg gttctttcaa tcggggatgt ctgcagaggg    1380
   cagaaagaaa acaggcgtta gaaacctgag gtcaaagatg tgtggcacat cccgccctcc    1440
   tctcttgccg tccctaccgg cattgaaata cttatggata aagttctcgc aatggcttca    1500
   cgtgcatgta cccgccgcca ccgctctccc acacctccct ggtccagcag ctagtccact    1560
   gcccgcctgg ctgctccagg cgcgccgacc gctcaagcgc tccaggtcca cccggcggag    1620
   ggcagagaaa gcgcgaccgc gcggcccgca gggttgcaag aagaaaacga gtgttatata    1680
   atgagtctca gtggttgctc acaatgccag gcgcgaaggc gtgaagatgt ggcctttccc    1740
   ttcccgcatc cccaggcatc ttttgcacct ggtgcggagt gagccagcca gcttgcgata    1800
   accaaagggc gcctcaggct ctggcgctcc tcggcggaat cccgtagctt ccctacgcat    1860
   gcctgcttct acaaacccac aaatggtttc cgatcatttc tgaaacaaaa tggatgctca    1920
   tttattcatg tgctctggct tctgccttcc tctctaatct cgttgcgtat gggctccagc    1980
   tcgccgttcg gttctcccga ggcagcattt acacttgaga gtctcaagat tattttattc    2040
   ctgagggagc atttgcactt gaaagtctct ttttacgttt attcctgagg cagcatttgc    2100
   acttgagttt ctttctcccg tagcttgcat tagattctcc gaccactctt tagcttctcc    2160
   tcctattcac acttcatatt tacccattgc attggtttta taaactcgct ctctgaaaat    2220
   agattgttat cttccttaac gtctgtttcc caggtcgggc aagatagctt gggactgtaa    2280
   tcccagtact ttaggaggag gaggggggat gatcgcttga gcccagataa catggtgaga    2340
   ccttcgtctc tattaaacaa acaaacaaac ccaggcgtcg tggcgtgcac ctgtggtccc    2400
   agctagtcgg gaggctcagg tgggagaacc ccttgagcca gggagtttga ggctgcagtg    2460
   agctgtgatc gcgccactgc actccaggtt gggcaacaga tcgactctgt ctccaaatgt    2520
   aaaccccatg agggcaagac tcttgtttgg tctcattcac cttggcgtgc ccaccaccta    2580
   gaacagggct gatcacgcag tagaatctaa ccatataatt aattgtgctt gaagaggggg    2640
   tgttggggag taagagaagg aagggaggag ggaagaaatg aaagacttgt gtgtttggat    2700
   taaatatatt aggtttggtt aagagtcgtt cagtttattc atttgcttgt ggcccaattc    2760
   agtagtttta ctccctctcc cacttggctc ctcaggcttt ttgctcagcc ctggaaccgc    2820
   gctgtaattg gcagctcctt ctaaatcggg acccggatgc tagctgtaac tggagccgaa    2880
   gtctccttct tcacctcccg ggacctggat gctagctgta actggaggga attggcgggg    2940
   gggaggggag gaggggccga gtaaagaaga acttcgcgtc tttaacttcg aaggtgattt    3000
   t                                                                    3001
<210> 582
   <211> 3001
   <212> DNA
   <213> human
<400> 582
   tttaaaatat tatatttggt gaaagaagtt agatacggaa ggttatatat tgtatgattt      60
   tatttataag aaatatttag aataggtaaa tttatagaga taaaaggagt tttgtagttg     120
   ttaggagttg gaggtaagaa gagtttaata ggtatgttta atggggtttt ttatgtagtt     180
   gacgaaaatg ttttgggtta gataggagtg atggttgtat aatattgtat taaatgttat 240
   tgaattttat tttttaaaat ttttagtttt atgttatgtg gcgtgtattt taaaaaaaaa 300
   aatagaggtg tagtgtttta gtacgggatg aggtagcgtg gataggagta ttttttaatt 360
   ttagtgaagt ttgagtcgcg tgtttgtaat aattttattg tggtagagaa tcgtagagtt 420
   tttttcggtt tggtagtagt tattcgtaat tttatagttt tttggaattt tagtttgggg 480
   gttttagaac gtcgaggcgg ggattgggag tcgagtcgga tttcgagatt atgggttagg 540
   gttggttgga tttagttatt tggttgaggt ttggtgagta aaatatttta aatttcgcgt 600
   gatttggaag gggaagtcgg ataaatacgg atttttagat gtgttagttt cgagtttatc 660
   gatatgaggt ttttttagag tttttattta ttattttaat cgtattttat cgattttgag 720
   atacggtttt tgatatttta ttattttaag ataaatagtg ttagatgttt aatagtagcg 780
   tttttttaag agatatatga aataatagtg ttagaaacga tgttgttttt tatgcgatga 840
   aattgttgta ataggtgttt aataaatgtt gataataaat gagtgaatga atgaaaatta 900
   ttttattttt atttgagttt tggttttgtt atttgttagt agtgtgattt aagagaagtt 960
   agtaattttt ttgagttttt ttagtttata aaatgtttgt tatgaagtcg atagttttcg 1020
   gaggttgcga ggttcgtaag aaatgtttat atgaatgtgc gtttagggcg tgagtgttta 1080
   ttttagaaaa ttttaatata gtgaaaaggt agaagcggtg tttttttttt ttatattttt 1140
   ataagaatat ataaaaaatg atataaatgg atatttacgg tagtggggga aggtatatat 1200
   ttacgttaaa aggtaggata tttttaaaag ttttattttt taaatgaaaa ttacgaaagc 1260
   ggggtgggtt gtggcggggg tagttgtggt tttgtaggat tttcggtgat tgatgattta 1320
   agtttttcga ggttttttag agtttttttg gtttttttaa tcggggatgt ttgtagaggg 1380
   tagaaagaaa ataggcgtta gaaatttgag gttaaagatg tgtggtatat ttcgtttttt 1440
   ttttttgtcg tttttatcgg tattgaaata tttatggata aagttttcgt aatggtttta 1500
   cgtgtatgta ttcgtcgtta tcgttttttt atattttttt ggtttagtag ttagtttatt 1560
   gttcgtttgg ttgttttagg cgcgtcgatc gtttaagcgt tttaggttta ttcggcggag 1620
   ggtagagaaa gcgcgatcgc gcggttcgta gggttgtaag aagaaaacga gtgttatata 1680
   atgagtttta gtggttgttt ataatgttag gcgcgaaggc gtgaagatgt ggtttttttt 1740
   ttttcgtatt tttaggtatt ttttgtattt ggtgcggagt gagttagtta gtttgcgata 1800
   attaaagggc gttttaggtt ttggcgtttt tcggcggaat ttcgtagttt ttttacgtat 1860
   gtttgttttt ataaatttat aaatggtttt cgattatttt tgaaataaaa tggatgttta 1920
   tttatttatg tgttttggtt tttgtttttt tttttaattt cgttgcgtat gggttttagt 1980
   tcgtcgttcg gttttttcga ggtagtattt atatttgaga gttttaagat tattttattt 2040
   ttgagggagt atttgtattt gaaagttttt ttttacgttt atttttgagg tagtatttgt 2100
   atttgagttt ttttttttcg tagtttgtat tagatttttc gattattttt tagttttttt 2160
   ttttatttat attttatatt tatttattgt attggtttta taaattcgtt ttttgaaaat 2220
   agattgttat tttttttaac gtttgttttt taggtcgggt aagatagttt gggattgtaa 2280
   ttttagtatt ttaggaggag gaggggggat gatcgtttga gtttagataa tatggtgaga 2340
   ttttcgtttt tattaaataa ataaataaat ttaggcgtcg tggcgtgtat ttgtggtttt 2400
   agttagtcgg gaggtttagg tgggagaatt ttttgagtta gggagtttga ggttgtagtg 2460
   agttgtgatc gcgttattgt attttaggtt gggtaataga tcgattttgt ttttaaatgt 2520
   aaattttatg agggtaagat ttttgtttgg ttttatttat tttggcgtgt ttattattta 2580
   gaatagggtt gattacgtag tagaatttaa ttatataatt aattgtgttt gaagaggggg 2640
   tgttggggag taagagaagg aagggaggag ggaagaaatg aaagatttgt gtgtttggat 2700
   taaatatatt aggtttggtt aagagtcgtt tagtttattt atttgtttgt ggtttaattt 2760
   agtagtttta tttttttttt tatttggttt tttaggtttt ttgtttagtt ttggaatcgc 2820
   gttgtaattg gtagtttttt ttaaatcggg attcggatgt tagttgtaat tggagtcgaa 2880
   gttttttttt ttatttttcg ggatttggat gttagttgta attggaggga attggcgggg 2940
   gggaggggag gaggggtcga gtaaagaaga atttcgcgtt tttaatttcg aaggtgattt 3000
   t 3001
<210> 583
   <211> 3001
   <212> DNA
   <213> human
<400> 583
   tttaaaatat tatatttggt gaaagaagtt agatatggaa ggttatatat tgtatgattt     60
   tatttataag aaatatttag aataggtaaa tttatagaga taaaaggagt tttgtagttg    120
   ttaggagttg gaggtaagaa gagtttaata ggtatgttta atggggtttt ttatgtagtt    180
   gatgaaaatg ttttgggtta gataggagtg atggttgtat aatattgtat taaatgttat    240
   tgaattttat tttttaaaat ttttagtttt atgttatgtg gtgtgtattt taaaaaaaaa    300
   aatagaggtg tagtgtttta gtatgggatg aggtagtgtg gataggagta ttttttaatt    360
   ttagtgaagt ttgagttgtg tgtttgtaat aattttattg tggtagagaa ttgtagagtt    420
   ttttttggtt tggtagtagt tatttgtaat tttatagttt tttggaattt tagtttgggg    480
   gttttagaat gttgaggtgg ggattgggag ttgagttgga ttttgagatt atgggttagg    540
   gttggttgga tttagttatt tggttgaggt ttggtgagta aaatatttta aattttgtgt    600
   gatttggaag gggaagttgg ataaatatgg atttttagat gtgttagttt tgagtttatt    660
   gatatgaggt ttttttagag tttttattta ttattttaat tgtattttat tgattttgag    720
   atatggtttt tgatatttta ttattttaag ataaatagtg ttagatgttt aatagtagtg    780
   tttttttaag agatatatga aataatagtg ttagaaatga tgttgttttt tatgtgatga    840
   aattgttgta ataggtgttt aataaatgtt gataataaat gagtgaatga atgaaaatta    900
   ttttattttt atttgagttt tggttttgtt atttgttagt agtgtgattt aagagaagtt    960
   agtaattttt ttgagttttt ttagtttata aaatgtttgt tatgaagttg atagtttttg   1020
   gaggttgtga ggtttgtaag aaatgtttat atgaatgtgt gtttagggtg tgagtgttta   1080
   ttttagaaaa ttttaatata gtgaaaaggt agaagtggtg tttttttttt ttatattttt   1140
   ataagaatat ataaaaaatg atataaatgg atatttatgg tagtggggga aggtatatat   1200
   ttatgttaaa aggtaggata tttttaaaag ttttattttt taaatgaaaa ttatgaaagt   1260
   ggggtgggtt gtggtggggg tagttgtggt tttgtaggat ttttggtgat tgatgattta   1320
   agtttttgga ggttttttag agtttttttg gtttttttaa ttggggatgt ttgtagaggg   1380
   tagaaagaaa ataggtgtta gaaatttgag gttaaagatg tgtggtatat tttgtttttt   1440
   ttttttgttg tttttattgg tattgaaata tttatggata aagtttttgt aatggtttta   1500
   tgtgtatgta tttgttgtta ttgttttttt atattttttt ggtttagtag ttagtttatt   1560
   gtttgtttgg ttgttttagg tgtgttgatt gtttaagtgt tttaggttta tttggtggag   1620
   ggtagagaaa gtgtgattgt gtggtttgta gggttgtaag aagaaaatga gtgttatata   1680
   atgagtttta gtggttgttt ataatgttag gtgtgaaggt gtgaagatgt ggtttttttt   1740
   tttttgtatt tttaggtatt ttttgtattt ggtgtggagt gagttagtta gtttgtgata   1800
   attaaagggt gttttaggtt ttggtgtttt ttggtggaat tttgtagttt ttttatgtat   1860
   gtttgttttt ataaatttat aaatggtttt tgattatttt tgaaataaaa tggatgttta   1920
   tttatttatg tgttttggtt tttgtttttt tttttaattt tgttgtgtat gggttttagt   1980
   ttgttgtttg gtttttttga ggtagtattt atatttgaga gttttaagat tattttattt   2040
   ttgagggagt atttgtattt gaaagttttt ttttatgttt atttttgagg tagtatttgt   2100
   atttgagttt tttttttttg tagtttgtat tagatttttt gattattttt tagttttttt   2160
   ttttatttat attttatatt tatttattgt attggtttta taaatttgtt ttttgaaaat   2220
   agattgttat tttttttaat gtttgttttt taggttgggt aagatagttt gggattgtaa   2280
   ttttagtatt ttaggaggag gaggggggat gattgtttga gtttagataa tatggtgaga   2340
   tttttgtttt tattaaataa ataaataaat ttaggtgttg tggtgtgtat ttgtggtttt   2400
   agttagttgg gaggtttagg tgggagaatt ttttgagtta gggagtttga ggttgtagtg   2460
   agttgtgatt gtgttattgt attttaggtt gggtaataga ttgattttgt ttttaaatgt   2520
   aaattttatg agggtaagat ttttgtttgg ttttatttat tttggtgtgt ttattattta   2580
   gaatagggtt gattatgtag tagaatttaa ttatataatt aattgtgttt gaagaggggg   2640
   tgttggggag taagagaagg aagggaggag ggaagaaatg aaagatttgt gtgtttggat   2700
   taaatatatt aggtttggtt aagagttgtt tagtttattt atttgtttgt ggtttaattt   2760
   agtagtttta tttttttttt tatttggttt tttaggtttt ttgtttagtt ttggaattgt   2820
   gttgtaattg gtagtttttt ttaaattggg atttggatgt tagttgtaat tggagttgaa   2880
   gttttttttt ttattttttg ggatttggat gttagttgta attggaggga attggtgggg   2940
   gggaggggag gaggggttga gtaaagaaga attttgtgtt tttaattttg aaggtgattt   3000
   t                                                                   3001

<210> 584
   <211> 3001
   <212> DNA
   <213> human.
<400> 584
<210> 585
   <211> 3001
   <212> DNA
   <213> human
<400> 585
   aaaattattt ttgaagttaa agatgtgaag ttttttttta tttggttttt tttttttttt 60
   tttttgttaa ttttttttag ttatagttag tatttaggtt ttgggaggtg aagaaggaga 120
   ttttggtttt agttatagtt agtatttggg ttttgattta gaaggagttg ttaattatag 180
   tgtggtttta gggttgagta aaaagtttga ggagttaagt gggagaggga gtaaaattat 240
   tgaattgggt tataagtaaa tgaataaatt gaatgatttt taattaaatt taatatattt 300
   aatttaaata tataagtttt ttattttttt tttttttttt tttttttttt attttttaat 360
   attttttttt taagtataat taattatatg gttagatttt attgtgtgat tagttttgtt 420
   ttaggtggtg ggtatgttaa ggtgaatgag attaaataag agttttgttt ttatggggtt 480
   tatatttgga gatagagttg atttgttgtt taatttggag tgtagtggtg tgattatagt 540
   ttattgtagt tttaaatttt ttggtttaag gggttttttt atttgagttt tttgattagt 600
   tgggattata ggtgtatgtt atgatgtttg ggtttgtttg tttgtttaat agagatgaag 660
   gttttattat gttatttggg tttaagtgat tatttttttt ttttttttta aagtattggg 720
   attatagttt taagttattt tgtttgattt gggaaataga tgttaaggaa gataataatt 780
   tatttttaga gagtgagttt ataaaattaa tgtaatgggt aaatatgaag tgtgaatagg 840
   aggagaagtt aaagagtggt tggagaattt aatgtaagtt atgggagaaa gaaatttaag 900
   tgtaaatgtt gttttaggaa taaatgtaaa aagagatttt taagtgtaaa tgttttttta 960
   ggaataaaat aattttgaga tttttaagtg taaatgttgt tttgggagaa ttgaatggtg 1020
   agttggagtt tatatgtaat gagattagag aggaaggtag aagttagagt atatgaataa 1080
   atgagtattt attttgtttt agaaatgatt ggaaattatt tgtgggtttg tagaagtagg 1140
   tatgtgtagg gaagttatgg gattttgttg aggagtgtta gagtttgagg tgttttttgg 1200
   ttattgtaag ttggttggtt tattttgtat taggtgtaaa agatgtttgg ggatgtggga 1260
   agggaaaggt tatattttta tgtttttgtg tttggtattg tgagtaatta ttgagattta 1320
   ttatataata tttgtttttt ttttgtaatt ttgtgggttg tgtggttgtg ttttttttgt 1380
   tttttgttgg gtggatttgg agtgtttgag tggttggtgt gtttggagta gttaggtggg 1440
   tagtggatta gttgttggat tagggaggtg tgggagagtg gtggtggtgg gtatatgtat 1500
   gtgaagttat tgtgagaatt ttatttataa gtattttaat gttggtaggg atggtaagag 1560
   aggagggtgg gatgtgttat atatttttga ttttaggttt ttaatgtttg tttttttttt 1620
   gttttttgta gatatttttg attgaaagaa ttagagaggt tttgagaaat tttgggaaat 1680
   ttagattatt agttattgaa ggttttatag ggttataatt gtttttgtta taatttattt 1740
   tgtttttgta gtttttattt agaaaataga gtttttaaaa atgttttgtt ttttaatgta 1800
   gatatatgtt tttttttatt attgtaaatg tttatttata ttatttttta tatattttta 1860
   taaaaatgta aaaaagaaaa atattgtttt tgttttttta ttgtgttgga gttttttgga 1920
   gtgagtattt atgttttaag tgtatattta tgtgggtatt ttttgtgagt tttgtagttt 1980
   ttggaagttg ttgattttat gataagtatt ttgtgaatta gggaagttta ggggggttat 2040
   tggttttttt tgagttatat tgttagtaaa tggtagaatt aaagtttaaa taaaaataaa 2100
   ataattttta tttatttatt tatttattgt taatatttat tgagtattta ttataataat 2160
   tttattgtat ggaagatagt attgtttttg atattgttgt tttatgtatt ttttagaaaa 2220
   atgttgttat tagatattta atattattta ttttgaggtg ataaaatatt aaaagttgtg 2280
   ttttaagatt gatgaaatgt ggttaaaatg atgaatagaa attttagggg gattttatat 2340
   tgatagattt gagattggta tatttggaga tttgtattta tttggttttt ttttttagat 2400
   tatgtgaggt ttgggatatt ttgtttatta ggttttagtt aggtaattga atttagttaa 2460
   ttttggttta tagttttgga atttgatttg gtttttagtt tttgttttgg tgttttgaga 2520
   tttttaggtt ggggttttag agggttgtga ggttgtgaat gattgttgtt aaattggaag 2580
   gaattttgtg gttttttgtt atagtgggat tgttgtaggt atgtggttta gattttattg 2640
   aggttgggag atgtttttgt ttatgttgtt ttattttgtg ttggagtatt gtatttttat 2700
   tttttttttt agggtatatg ttatataata taaaattaaa aattttaaag agtagaattt 2760
   agtggtattt agtataatgt tatgtaatta ttatttttat ttagtttaaa atatttttgt 2820
   taattgtata ggaggtttta ttaagtatat ttgttaaatt ttttttgttt ttaatttttg 2880
   gtaattataa aatttttttt gtttttatgg atttatttat tttgaatatt ttttataaat 2940
   gggattatat aatatgtgat tttttgtgtt tggttttttt tattaagtgt agtgttttgg 3000
   g 3001
<210> 586
   <211> 50
   <212> DNA
   <213> human
<400> 586
   gcgtctgtaa ttgcttatta acagcgaata ttcaggcttc tccttatccg 50
<210> 587
   <211> 50
   <212> DNA
   <213> human
<400> 587
   gcgtttgtaa ttgtttatta atagcgaata tttaggtttt tttttattcg 50
<210> 588
   <211> 50
   <212> DNA
   <213> human
<400> 588
   gtgtttgtaa ttgtttatta atagtgaata tttaggtttt tttttatttg 50
<210> 589
   <211> 50
   <212> DNA
   <213> human
<400> 589
   cggataagga gaagtttgaa tattcgttgt taataagtaa ttatagacgt 50
<210> 590
   <211> 50
   <212> DNA
   <213> human
<400> 590
   tggataagga gaagtttgaa tatttgttgt taataagtaa ttatagatgt 50
<210> 591
   <211> 3001
   <212> DNA
   <213> human
<400> 591
   tgtagctgaa acagtgcttt tcattatttt aaagagaatt tgtaacatat acttacaaat 60
   gtaatgtaat acaatatgta atataatatt atgtacatat tttccatgtt ccaaaaatcc 120
   tgggtcctag agtagtttta tagaaactga atttttattc tctttgaatt tgtctttgct 180
   cctccatatg taaaatgtgt tgaagtttgg gaaaaataat agatttactg gggaagtggg 240
   tagatatcaa ccaaaatttc ctgtccccaa gaatcaatac aaattctaat tatatacatc 300
   gtgtgtatat ttaataatta ggtggtgctc atactgaact catgcgcttt caaaagcttg 360
   ctctggggct ggaataaaat caacgctttc tgatgtggct cgggggacag ctggcccgcg 420
   ttattcgcgc cagagtgggt cggaacgatg cctgttgcct gcgatggcac tttgccacgc      480
   gaagcgcttc cctggctgcg tcaaaagcat tttgaaggga cgccgaggcc cgcttcttgc      540
   tttgttaaag cgcaaaactg tcatgtacct ttggccaccc tagctccaaa taacgtgaga      600
   ttaggtctca cattagggag atttctcatc cctacttttg ccttagatat cttgcaactc      660
   aggtaaagga taatgtggag gatcctacga atagtaatca ggacccgcta gcccagggat      720
   agatacgtcg tccaagccgc ccaagctgca cggcctcttt ctttccttga gctcgcgtac      780
   ccctcacggg atcttcacgt tgacagtcat gaggtattca cagagctata ctacttgaac      840
   aatcaatgtt ctagacttcc ttgggtcaaa ataacactaa agcaggggag gaaggcatgg      900
   acaatgtagt gatcaaaaca gccgtgccaa acactaacta tactgatgac acaaaacgca      960
   ggaagcgcag ggcagaacgc gctcagtacc agctttctgg cgatctgctc ctggccactc     1020
   tgtcccagga cctacaaggc caccgcccca gcccactagt ctcaacttgc cccctcccct     1080
   gtggctgtgc cttgaccctg aaaatcttcg cgcactcctg ggtttcacca ccacacacac     1140
   gcgagcgcgc gagcgcgcga acacacacat caaaaaagaa aaaaaaaacc gtgataaatg     1200
   ccctggagag tctaagtgca ttaaggacct cgttctcgga gaccccagac atgccgatgt     1260
   tggtgctgct gtggttggaa aactgggttg gaaagcttcg cactgttcta cacttgcgtg     1320
   tgcgcactca gcaatccttt ggccatctca tctgttgtgg gcgaagagtt tcccgtgtga     1380
   tcgcgttcgg ttggggaagc agagtcccga catctcagcc ggaaaatgcg ctcccggagc     1440
   gattactggc ggcgtctgta attgcttatt aacagcgaat attcaggctt ctccttatcc     1500
   gcaacgaaac gtgccccccg cttccgtaat aatgaaacga taaaatatga cggccccgct     1560
   cttgaatcta tctgaggaaa cgcagcgaag aaacaagcag ctggagttta attcaatatc     1620
   aaactgatta tttcactaat tattctacct tctgtatttg ccgcagagca gaggcgcagg     1680
   gaatcctaac tgtggggctg ttagggaagc tttgtttctt ggtgtcaggc ctaactgaag     1740
   atggtggtga agggacggaa tccgggatca ggaaaactga gcagaaagga ttttaaattg     1800
   tggggaataa aagtgactgt ctccccgaaa gcagttgtgt tggcgtgagt atgaagttgg     1860
   tgttagctcg cttagtttca ctcttgtcct gtccccagta ccctgtccaa gcttctgatt     1920
   tagaagctga gagcttaggt cctccaggat ggcaatgtgc tctggccgtc cccggaatcc     1980
   caaaagctta gcgcgagttt cctctctaca aacctttccc actgggtaaa aggatgggaa     2040
   caaacaaaaa gtggttctgt gcgttgaagg ccagggagtt tgccttcgac aagaggaagg     2100
   tgtgctttga agttacagct tccgacgagg aaagagactt ccttgggcct gtctccaact     2160
   tgccccagtt tccccagcct ccgggacggg cgcttccctg caagctatca gcttgaagag     2220
   tctttctctc tctggcctct aaaatctgct ccagccatct cattcctcca gcggagggct     2280
   gagatgacta atgcgccaag ggtggacacc gctatggaga ccccaaactt tgtcccccta     2340
   aattccggcg aaattacaga caccaggatt gattatcaca gcgggacgca gtctcaaaaa     2400
   aaaaaaaaaa aaaaaaaaaa aaaaaaggaa aagaaagtca caggcgactt gatccaatta     2460
   gtaaatgcct aattgaatta gtgtcacctc caccagccaa taggagggtc tccgggctgt     2520
   gaggccggga agcccctccc tggccgcaaa tatataaagc agctagtgac agcccataag     2580
   tttccaagtg gtcaacttga ccgatgcttt ggcaattgaa aaagggcaga aaggcgcggg     2640
   ctagtgggtg gatggggaca aagatctaag tcaccttctt ccagcgtgtg agcctgggag     2700
   gagggtgggg gtcctgagga gcaagaggta cgaggaagga aaaggagagg gcttctgggt     2760
   tagtttccac ctcctgcttt ccaactcacg gcgctttcct tccggaaagg acgctggatt     2820
   cagggcgcgc cagtacgcgc agtagcggcc cgcgagtcgg caggtgggta gccccggcgc     2880
   gggaggaagg ggaagttacc ttcccctcgg aagagggcgc tggctccccc atcctgcctt     2940
   tataataagg ccaccggagg agaggaagca gccagctgcc gtctgcgctt tgcaaagcat     3000
   g                                                                     3001
<210> 592
   <211> 3001
   <212> DNA
   <213> human
<400> 592
   tgtagttgaa atagtgtttt ttattatttt aaagagaatt tgtaatatat atttataaat 60
   gtaatgtaat ataatatgta atataatatt atgtatatat tttttatgtt ttaaaaattt 120
   tgggttttag agtagtttta tagaaattga atttttattt tttttgaatt tgtttttgtt 180
   tttttatatg taaaatgtgt tgaagtttgg gaaaaataat agatttattg gggaagtggg 240
   tagatattaa ttaaaatttt ttgtttttaa gaattaatat aaattttaat tatatatatc 300
   gtgtgtatat ttaataatta ggtggtgttt atattgaatt tatgcgtttt taaaagtttg 360
   ttttggggtt ggaataaaat taacgttttt tgatgtggtt cgggggatag ttggttcgcg 420
   ttattcgcgt tagagtgggt cggaacgatg tttgttgttt gcgatggtat tttgttacgc 480
   gaagcgtttt tttggttgcg ttaaaagtat tttgaaggga cgtcgaggtt cgttttttgt 540
   tttgttaaag cgtaaaattg ttatgtattt ttggttattt tagttttaaa taacgtgaga 600
   ttaggtttta tattagggag atttttattt tttatttttg ttttagatat tttgtaattt 660
   aggtaaagga taatgtggag gattttacga atagtaatta ggattcgtta gtttagggat 720
   agatacgtcg tttaagtcgt ttaagttgta cggttttttt ttttttttga gttcgcgtat 780
   tttttacggg atttttacgt tgatagttat gaggtattta tagagttata ttatttgaat 840
   aattaatgtt ttagattttt ttgggttaaa ataatattaa agtaggggag gaaggtatgg 900
   ataatgtagt gattaaaata gtcgtgttaa atattaatta tattgatgat ataaaacgta 960
   ggaagcgtag ggtagaacgc gtttagtatt agttttttgg cgatttgttt ttggttattt 1020
   tgttttagga tttataaggt tatcgtttta gtttattagt tttaatttgt tttttttttt 1080
   gtggttgtgt tttgattttg aaaattttcg cgtatttttg ggttttatta ttatatatac 1140
   gcgagcgcgc gagcgcgcga atatatatat taaaaaagaa aaaaaaaatc gtgataaatg 1200
   ttttggagag tttaagtgta ttaaggattt cgttttcgga gattttagat atgtcgatgt 1260
   tggtgttgtt gtggttggaa aattgggttg gaaagtttcg tattgtttta tatttgcgtg 1320
   tgcgtattta gtaatttttt ggttatttta tttgttgtgg gcgaagagtt tttcgtgtga 1380
   tcgcgttcgg ttggggaagt agagtttcga tattttagtc ggaaaatgcg ttttcggagc 1440
   gattattggc ggcgtttgta attgtttatt aatagcgaat atttaggttt ttttttattc 1500
   gtaacgaaac gtgtttttcg ttttcgtaat aatgaaacga taaaatatga cggtttcgtt 1560
   tttgaattta tttgaggaaa cgtagcgaag aaataagtag ttggagttta atttaatatt 1620
   aaattgatta ttttattaat tattttattt tttgtatttg tcgtagagta gaggcgtagg 1680
   gaattttaat tgtggggttg ttagggaagt tttgtttttt ggtgttaggt ttaattgaag 1740
   atggtggtga agggacggaa ttcgggatta ggaaaattga gtagaaagga ttttaaattg 1800
   tggggaataa aagtgattgt tttttcgaaa gtagttgtgt tggcgtgagt atgaagttgg 1860
   tgttagttcg tttagtttta tttttgtttt gtttttagta ttttgtttaa gtttttgatt 1920
   tagaagttga gagtttaggt tttttaggat ggtaatgtgt tttggtcgtt ttcggaattt 1980
   taaaagttta gcgcgagttt tttttttata aatttttttt attgggtaaa aggatgggaa 2040
   taaataaaaa gtggttttgt gcgttgaagg ttagggagtt tgttttcgat aagaggaagg 2100
   tgtgttttga agttatagtt ttcgacgagg aaagagattt ttttgggttt gtttttaatt 2160
   tgttttagtt tttttagttt tcgggacggg cgtttttttg taagttatta gtttgaagag 2220
   tttttttttt tttggttttt aaaatttgtt ttagttattt tattttttta gcggagggtt 2280
   gagatgatta atgcgttaag ggtggatatc gttatggaga ttttaaattt tgttttttta 2340
   aatttcggcg aaattataga tattaggatt gattattata gcgggacgta gttttaaaaa 2400
   aaaaaaaaaa aaaaaaaaaa aaaaaaggaa aagaaagtta taggcgattt gatttaatta 2460
   gtaaatgttt aattgaatta gtgttatttt tattagttaa taggagggtt ttcgggttgt 2520
   gaggtcggga agtttttttt tggtcgtaaa tatataaagt agttagtgat agtttataag 2580
   tttttaagtg gttaatttga tcgatgtttt ggtaattgaa aaagggtaga aaggcgcggg 2640
   ttagtgggtg gatggggata aagatttaag ttattttttt ttagcgtgtg agtttgggag 2700
   gagggtgggg gttttgagga gtaagaggta cgaggaagga aaaggagagg gtttttgggt 2760
   tagtttttat tttttgtttt ttaatttacg gcgttttttt ttcggaaagg acgttggatt 2820
   tagggcgcgt tagtacgcgt agtagcggtt cgcgagtcgg taggtgggta gtttcggcgc 2880
   gggaggaagg ggaagttatt tttttttcgg aagagggcgt tggttttttt attttgtttt 2940
   tataataagg ttatcggagg agaggaagta gttagttgtc gtttgcgttt tgtaaagtat 3000
   g 3001
<210> 593
   <211> 3001
   <212> DNA
   <213> human
<400> 593
   tgtagttgaa atagtgtttt ttattatttt aaagagaatt tgtaatatat atttataaat     60
   gtaatgtaat ataatatgta atataatatt atgtatatat tttttatgtt ttaaaaattt    120
   tgggttttag agtagtttta tagaaattga atttttattt tttttgaatt tgtttttgtt    180
   tttttatatg taaaatgtgt tgaagtttgg gaaaaataat agatttattg gggaagtggg    240
   tagatattaa ttaaaatttt ttgtttttaa gaattaatat aaattttaat tatatatatt    300
   gtgtgtatat ttaataatta ggtggtgttt atattgaatt tatgtgtttt taaaagtttg    360
   ttttggggtt ggaataaaat taatgttttt tgatgtggtt tgggggatag ttggtttgtg    420
   ttatttgtgt tagagtgggt tggaatgatg tttgttgttt gtgatggtat tttgttatgt    480
   gaagtgtttt tttggttgtg ttaaaagtat tttgaaggga tgttgaggtt tgttttttgt    540
   tttgttaaag tgtaaaattg ttatgtattt ttggttattt tagttttaaa taatgtgaga    600
   ttaggtttta tattagggag attttttatt tttatttttg ttttagatat tttgtaattt    660
   aggtaaagga taatgtggag gattttatga atagtaatta ggatttgtta gtttagggat    720
   agatatgttg tttaagttgt ttaagttgta tggttttttt ttttttttga gtttgtgtat    780
   tttttatggg atttttatgt tgatagttat gaggtattta tagagttata ttatttgaat    840
   aattaatgtt ttagattttt ttgggttaaa ataatattaa agtaggggag gaaggtatgg    900
   ataatgtagt gattaaaata gttgtgttaa atattaatta tattgatgat ataaaatgta    960
   ggaagtgtag ggtagaatgt gtttagtatt agttttttgg tgatttgttt ttggttattt   1020
   tgttttagga tttataaggt tattgtttta gtttattagt tttaatttgt tttttttttt   1080
   gtggttgtgt tttgattttg aaaatttttg tgtatttttg ggttttatta ttatatatat   1140
   gtgagtgtgt gagtgtgtga atatatatat taaaaaagaa aaaaaaaatt gtgataaatg   1200
   ttttggagag tttaagtgta ttaaggattt tgtttttgga gattttagat atgttgatgt   1260
   tggtgttgtt gtggttggaa aattgggttg gaaagttttg tattgtttta tatttgtgtg   1320
   tgtgtattta gtaatttttt ggttatttta tttgttgtgg gtgaagagtt ttttgtgtga   1380
   ttgtgtttgg ttggggaagt agagttttga tattttagtt ggaaaatgtg tttttggagt   1440
   gattattggt ggtgtttgta attgtttatt aatagtgaat atttaggttt ttttttattt   1500
   gtaatgaaat gtgttttttg tttttgtaat aatgaaatga taaaatatga tggttttgtt   1560
   tttgaattta tttgaggaaa tgtagtgaag aaataagtag ttggagttta atttaatatt   1620
   aaattgatta ttttattaat tattttattt tttgtatttg ttgtagagta gaggtgtagg   1680
   gaattttaat tgtggggttg ttagggaagt tttgtttttt ggtgttaggt ttaattgaag   1740
   atggtggtga agggatggaa tttgggatta ggaaaattga gtagaaagga ttttaaattg   1800
   tggggaataa aagtgattgt ttttttgaaa gtagttgtgt tggtgtgagt atgaagttgg   1860
   tgttagtttg tttagtttta tttttgtttt gtttttagta ttttgtttaa gtttttgatt   1920
   tagaagttga gagtttaggt tttttaggat ggtaatgtgt tttggttgtt tttggaattt   1980
   taaaagttta gtgtgagttt tttttttata aatttttttt attgggtaaa aggatgggaa   2040
   taaataaaaa gtggttttgt gtgttgaagg ttagggagtt tgtttttgat aagaggaagg   2100
   tgtgttttga agttatagtt tttgatgagg aaagagattt ttttgggttt gtttttaatt   2160
   tgttttagtt tttttagttt ttgggatggg tgtttttttg taagttatta gtttgaagag   2220
   tttttttttt tttggttttt aaaatttgtt ttagttattt tattttttta gtggagggtt   2280
   gagatgatta atgtgttaag ggtggatatt gttatggaga ttttaaattt tgttttttta   2340
   aattttggtg aaattataga tattaggatt gattattata gtgggatgta gttttaaaaa   2400
   aaaaaaaaaa aaaaaaaaaa aaaaaaggaa aagaaagtta taggtgattt gatttaatta   2460
   gtaaatgttt aattgaatta gtgttatttt tattagttaa taggagggtt tttgggttgt   2520
   gaggttggga agtttttttt tggttgtaaa tatataaagt agttagtgat agtttataag   2580
   tttttaagtg gttaatttga ttgatgtttt ggtaattgaa aaagggtaga aaggtgtggg   2640
   ttagtgggtg gatggggata aagatttaag ttattttttt ttagtgtgtg agtttgggag   2700
   gagggtgggg gttttgagga gtaagaggta tgaggaagga aaaggagagg gtttttgggt   2760
   tagtttttat tttttgtttt ttaatttatg gtgttttttt tttggaaagg atgttggatt   2820
   tagggtgtgt tagtatgtgt agtagtggtt tgtgagttgg taggtgggta gttttggtgt   2880
   gggaggaagg ggaagttatt ttttttttgg aagagggtgt tggttttttt attttgtttt   2940
   tataataagg ttattggagg agaggaagta gttagttgtt gtttgtgttt tgtaaagtat   3000
   g                                                                    3001
<210> 594
   <211> 3001
   <212> DNA
   <213> human
<400> 594
   tatgttttgt aaagcgtaga cggtagttgg ttgttttttt tttttcggtg gttttattat      60
   aaaggtagga tgggggagtt agcgtttttt ttcgagggga aggtaatttt tttttttttt     120
   cgcgtcgggg ttatttattt gtcgattcgc gggtcgttat tgcgcgtatt ggcgcgtttt     180
   gaatttagcg tttttttcgg aaggaaagcg tcgtgagttg gaaagtagga ggtggaaatt    240
   aatttagaag tttttttttt tttttttttc gtattttttg ttttttagga tttttatttt   300
   ttttttaggt ttatacgttg gaagaaggtg atttagattt ttgtttttat ttatttatta    360
   gttcgcgttt ttttgttttt ttttaattgt taaagtatcg gttaagttga ttatttggaa     420
   atttatgggt tgttattagt tgttttatat atttgcggtt agggaggggt ttttcggttt     480
   tatagttcgg agattttttt attggttggt ggaggtgata ttaatttaat taggtattta     540
   ttaattggat taagtcgttt gtgatttttt tttttttttt tttttttttt tttttttttt     600
   ttttttgaga ttgcgtttcg ttgtgataat taattttggt gtttgtaatt tcgtcggaat    660
   ttagggggat aaagtttggg gtttttatag cggtgtttat ttttggcgta ttagttattt     720
   tagtttttcg ttggaggaat gagatggttg gagtagattt tagaggttag agagagaaag     780
   attttttaag ttgatagttt gtagggaagc gttcgtttcg gaggttgggg aaattggggt    840
   aagttggaga taggtttaag gaagtttttt ttttcgtcgg aagttgtaat tttaaagtat     900
   attttttttt tgtcgaaggt aaattttttg gtttttaacg tatagaatta ttttttgttt  960
   gtttttattt ttttatttag tgggaaaggt ttgtagagag gaaattcgcg ttaagttttt    1020
   gggatttcgg ggacggttag agtatattgt tattttggag gatttaagtt tttagttttt    1080
   aaattagaag tttggatagg gtattgggga taggataaga gtgaaattaa gcgagttaat    1140
   attaatttta tatttacgtt aatataattg ttttcgggga gatagttatt tttatttttt    1200
   ataatttaaa attttttttg tttagttttt ttgatttcgg atttcgtttt tttattatta   1260
   tttttagtta ggtttgatat taagaaataa agttttttta atagttttat agttaggatt    1320
   ttttgcgttt ttgttttgcg gtaaatatag aaggtagaat aattagtgaa ataattagtt    1380
   tgatattgaa ttaaatttta gttgtttgtt ttttcgttgc gtttttttag atagatttaa  1440
   gagcggggtc gttatatttt atcgttttat tattacggaa gcggggggta cgtttcgttg    1500
   cggataagga gaagtttgaa tattcgttgt taataagtaa ttatagacgt cgttagtaat    1560
   cgtttcggga gcgtattttt cggttgagat gtcgggattt tgttttttta atcgaacgcg    1620
   attatacggg aaatttttcg tttataatag atgagatggt taaaggattg ttgagtgcgt    1680
   atacgtaagt gtagaatagt gcgaagtttt ttaatttagt tttttaatta tagtagtatt    1740
   aatatcggta tgtttggggt tttcgagaac gaggttttta atgtatttag attttttagg   1800
   gtatttatta cggttttttt tttttttttt gatgtgtgtg ttcgcgcgtt cgcgcgttcg    1860
   cgtgtgtgtg gtggtgaaat ttaggagtgc gcgaagattt ttagggttaa ggtatagtta    1920
   taggggaggg ggtaagttga gattagtggg ttggggcggt ggttttgtag gttttgggat    1980
   agagtggtta ggagtagatc gttagaaagt tggtattgag cgcgttttgt tttgcgtttt    2040
   ttgcgttttg tgttattagt atagttagtg tttggtacgg ttgttttgat tattatattg    2100
   tttatgtttt ttttttttgt tttagtgtta ttttgattta aggaagttta gaatattgat   2160
   tgtttaagta gtatagtttt gtgaatattt tatgattgtt aacgtgaaga tttcgtgagg    2220
   ggtacgcgag tttaaggaaa gaaagaggtc gtgtagtttg ggcggtttgg acgacgtatt    2280
   tatttttggg ttagcgggtt ttgattatta ttcgtaggat tttttatatt attttttatt   2340
   tgagttgtaa gatatttaag gtaaaagtag ggatgagaaa tttttttaat gtgagattta    2400
   attttacgtt atttggagtt agggtggtta aaggtatatg atagttttgc gttttaataa    2460
   agtaagaagc gggtttcggc gtttttttaa aatgtttttg acgtagttag ggaagcgttt   2520
   cgcgtggtaa agtgttatcg taggtaatag gtatcgtttc gatttatttt ggcgcgaata    2580
   acgcgggtta gttgtttttc gagttatatt agaaagcgtt gattttattt tagttttaga    2640
   gtaagttttt gaaagcgtat gagtttagta tgagtattat ttaattatta aatatatata    2700
   cgatgtatat aattagaatt tgtattgatt tttggggata ggaaattttg gttgatattt    2760
   atttattttt ttagtaaatt tattattttt tttaaatttt aatatatttt atatatggag    2820
   gagtaaagat aaatttaaag agaataaaaa tttagttttt ataaaattat tttaggattt    2880
   aggatttttg gaatatggaa aatatgtata taatattata ttatatattg tattatatta   2940
   tatttgtaag tatatgttat aaattttttt taaaataatg aaaagtattg ttttagttat    3000
   a                                                                    3001
<210> 595
   <211> 3001
   <212> DNA
   <213> human
<400> 595
   tatgttttgt aaagtgtaga tggtagttgg ttgttttttt ttttttggtg gttttattat 60
   aaaggtagga tgggggagtt agtgtttttt tttgagggga aggtaatttt tttttttttt 120
   tgtgttgggg ttatttattt gttgatttgt gggttgttat tgtgtgtatt ggtgtgtttt 180
   gaatttagtg ttttttttgg aaggaaagtg ttgtgagttg gaaagtagga ggtggaaatt 240
   aatttagaag tttttttttt tttttttttt gtattttttg ttttttagga tttttatttt 300
   ttttttaggt ttatatgttg gaagaaggtg atttagattt ttgtttttat ttatttatta 360
   gtttgtgttt ttttgttttt ttttaattgt taaagtattg gttaagttga ttatttggaa 420
   atttatgggt tgttattagt tgttttatat atttgtggtt agggaggggt tttttggttt 480
   tatagtttgg agattttttt attggttggt ggaggtgata ttaatttaat taggtattta 540
   ttaattggat taagttgttt gtgatttttt tttttttttt tttttttttt tttttttttt 600
   ttttttgaga ttgtgttttg ttgtgataat taattttggt gtttgtaatt ttgttggaat 660
   ttagggggat aaagtttggg gtttttatag tggtgtttat ttttggtgta ttagttattt 720
   tagttttttg ttggaggaat gagatggttg gagtagattt tagaggttag agagagaaag 780
   attttttaag ttgatagttt gtagggaagt gtttgttttg gaggttgggg aaattggggt 840
   aagttggaga taggtttaag gaagtttttt tttttgttgg aagttgtaat tttaaagtat 900
   attttttttt tgttgaaggt aaattttttg gtttttaatg tatagaatta ttttttgttt 960
   gtttttattt ttttatttag tgggaaaggt ttgtagagag gaaatttgtg ttaagttttt 1020
   gggattttgg ggatggttag agtatattgt tattttggag gatttaagtt tttagttttt 1080
   aaattagaag tttggatagg gtattgggga taggataaga gtgaaattaa gtgagttaat 1140
   attaatttta tatttatgtt aatataattg tttttgggga gatagttatt tttatttttt 1200
   ataatttaaa attttttttg tttagttttt ttgattttgg attttgtttt tttattatta 1260
   tttttagtta ggtttgatat taagaaataa agttttttta atagttttat agttaggatt 1320
   ttttgtgttt ttgttttgtg gtaaatatag aaggtagaat aattagtgaa ataattagtt 1380
   tgatattgaa ttaaatttta gttgtttgtt tttttgttgt gtttttttag atagatttaa 1440
   gagtggggtt gttatatttt attgttttat tattatggaa gtggggggta tgttttgttg 1500
   tggataagga gaagtttgaa tatttgttgt taataagtaa ttatagatgt tgttagtaat 1560
   tgttttggga gtgtattttt tggttgagat gttgggattt tgttttttta attgaatgtg 1620
   attatatggg aaattttttg tttataatag atgagatggt taaaggattg ttgagtgtgt 1680
   atatgtaagt gtagaatagt gtgaagtttt ttaatttagt tttttaatta tagtagtatt 1740
   aatattggta tgtttggggt ttttgagaat gaggttttta atgtatttag attttttagg 1800
   gtatttatta tggttttttt tttttttttt gatgtgtgtg tttgtgtgtt tgtgtgtttg 1860
   tgtgtgtgtg gtggtgaaat ttaggagtgt gtgaagattt ttagggttaa ggtatagtta 1920
   taggggaggg ggtaagttga gattagtggg ttggggtggt ggttttgtag gttttgggat 1980
   agagtggtta ggagtagatt gttagaaagt tggtattgag tgtgttttgt tttgtgtttt 2040
   ttgtgttttg tgttattagt atagttagtg tttggtatgg ttgttttgat tattatattg 2100
   tttatgtttt ttttttttgt tttagtgtta ttttgattta aggaagttta gaatattgat 2160
   tgtttaagta gtatagtttt gtgaatattt tatgattgtt aatgtgaaga ttttgtgagg 2220
   ggtatgtgag tttaaggaaa gaaagaggtt gtgtagtttg ggtggtttgg atgatgtatt 2280
   tatttttggg ttagtgggtt ttgattatta tttgtaggat tttttatatt attttttatt 2340
   tgagttgtaa gatatttaag gtaaaagtag ggatgagaaa tttttttaat gtgagattta 2400
   attttatgtt atttggagtt agggtggtta aaggtatatg atagttttgt gttttaataa 2460
   agtaagaagt gggttttggt gtttttttaa aatgtttttg atgtagttag ggaagtgttt 2520
   tgtgtggtaa agtgttattg taggtaatag gtattgtttt gatttatttt ggtgtgaata 2580
   atgtgggtta gttgtttttt gagttatatt agaaagtgtt gattttattt tagttttaga 2640
   gtaagttttt gaaagtgtat gagtttagta tgagtattat ttaattatta aatatatata 2700
   tgatgtatat aattagaatt tgtattgatt tttggggata ggaaattttg gttgatattt 2760
   atttattttt ttagtaaatt tattattttt tttaaatttt aatatatttt atatatggag 2820
   gagtaaagat aaatttaaag agaataaaaa tttagttttt ataaaattat tttaggattt 2880
   aggatttttg gaatatggaa aatatgtata taatattata ttatatattg tattatatta 2940
   tatttgtaag tatatgttat aaattttttt taaaataatg aaaagtattg ttttagttat 3000
   a 3001
<210> 596
   <211> 50
   <212> DNA
   <213> human
<400> 596
   cggaccactg gggatcaccc ccagcagctc ctgaagcctc tggccgggct 50
<210> 597
   <211> 50
   <212> DNA
   <213> human
<400> 597
   cggattattg gggattattt ttagtagttt ttgaagtttt tggtcgggtt 50
<210> 598
   <211> 50
   <212> DNA
   <213> human
<400> 598
   tggattattg gggattattt ttagtagttt ttgaagtttt tggttgggtt 50
<210> 599
   <211> 50
   <212> DNA
   <213> human
<400> 599
   agttcggtta gaggttttag gagttgttgg gggtgatttt tagtggttcg 50
<210> 600
   <211> 50
   <212> DNA
   <213> human
<400> 600
   agtttggtta gaggttttag gagttgttgg gggtgatttt tagtggtttg 50
<210> 601
   <211> 3001
   <212> DNA
   <213> human
<400> 601
   tacattgtgc catatagtag catccatatc tatatatatc atgatgtgtt gtaatttata 60
   aactcatgca atgatagcct cccatgcaca taacagcaca cacatagaca cagtggcaaa 120
   caaacataga tgcaatgaca cagaaacata catacagata cacccaatgt gtttttgtta 180
   cgttttatta tgtgactcat agacacataa tagcacacac atatatacac acaatggccc 240
   cttcacatat acagaaatag ataatgtcta ttctatagat gcatactata cacacgcatg 300
   cgcataatgg catgcatata tactctgacc taaacagcaa cacatcatgc agtggcgtgc 360
   gtgttgacct acacactgct ttagggaagt gaagatgtcc actcctcccc atgcaccccc 420
   tacctccagt tccattggct cttgcctggg tggagcccac agtcaggggc tctagggttc 480
   agtatcaccg ctgctattct gtgggggtgc cacacgaggg cccccgacct tggctgaaac 540
   tggcttgcct aatgagtccc gggttcagag tggagagggg ctgcctctcg gggcttctcc 600
   caacacccct ggctctgggg actctgtcta gacccctgcc actgcatata gactgactcc 660
   tggctgaggg gattctcacg tccttgtccc ccaaattatt tagcaacaag atctaaggct 720
   agacagaaga tgaacttcca ggcaattcgg gacaaagcca acccaacagg ccatgtgggc 780
   ttccctggtg gcctgggaca gccaggttgc cccccaagtg caatctgcca ggaggaagca 840
   gaaggaattc tccgtctatt tctacctgtc tctttgcttg tcttcccggt ttctttctct 900
   tggtttctgt ctctctcttt gtttcatctc aagtcccatc ttttctagtt ctgcccttct 960
   ctgtggctgg atctctgtca taccctcccc cgttctgtct ctatcagtct cacccgatgt 1020
   agggaggacc atctgcaccc cactcgcctc tgtactccgg aaggtgtgag aatgtgtgaa 1080
   gatgagggtg ggaggccaca tgtgggagcc ctgggctgac atgccgtgag aaacctgctc 1140
   ttccttctgt ccccgaccct gtcagccggt tcagcccacc agtccagcgc catcaccatg 1200
   gaaaccagca gccgacttcc tattggtggg tgggagggga ggaggaggag gaggagggga 1260
   gggctggctc ttcccggctc acccccccac ccaattctga ggctcagccc tcctcggagc 1320
   ccaccatctc gaggctcctg gactgtaagg gacccctgca gtccccagag cagaggccag 1380
   caggggctga gactatgagg caagcagccc atcgacccag gcctccttag gaccagccca 1440
   gccacccagc aagcgaaaga gcagcagcag agagcagagc cggagataaa ggaccacagc 1500
   ggaccactgg ggatcacccc cagcagctcc tgaagcctct ggccgggctg aggggttgcg 1560
   acagacggcc agacccacag acactgacag acacacaacg acaccgatgc acacagctgc 1620
   acgacactga cacactcaga tacaaccagg gacagcatca catactgcca cagccctgca 1680
   atacagacac ccacacacac acacacaaac aactggcaac tctgaaatgg gcagacagtg 1740
   acacacagca ccacccccct caacacacag aagtgggggc acccatcttc ccaacacatg 1800
   agacagcatt gacctgcacg gacagacctg gagacacaca cacgcaccct cacccaggcg 1860
   cagctgtcgg tggcctctgc acatcctccc tacccccaca gggacaccct cgctcctcca 1920
   catactcgcg cacacacaca gctaccagca aacatagctc acatacccct ggctccagct 1980
   ccagcagccc tggcgccttc tgcagcctcc tttgtgggtg gggaagagct cagccaacct 2040
   tggatggagc gtcggtgaag caagagctca gaggggagag gggctggcct ggcatgaccc 2100
   tcgccagtcc ccccacctgg ctgggtgatg tcccccggcc cgggttctgg ggccccttgg 2160
   cagtaccatg gagcaactga caaccctccc acggcctggg gaccctggag ccatggagcc 2220
   atgggcactg cccacctggc atagctggac tccaggtcga gggggtgaac ctagcagtgc 2280
   agccccaagc atcgctgata ctcctccggc agctctgcag cttcaagaac tgaggtctga 2340
   ggagagttcc aagcccaaag gagacgggag ctccaggccc gtggggggaa ctgaccctga 2400
   aggagcagag gcttgtctgc ccagcctggg ccagcaagca tccagctctg gacccgcctg 2460
   ccagaggcca gaggatgagg aagtggaggc tttcctgaag gtaagggatc tgtggagggc 2520
   tggggtctgc aaggtggggg tctctcacca tgtcctccag cctggccagg aggtgaggcg 2580
   aggtatgcat ccccaatgcc cacctgtcct ctcttggcct gtctgtgggt ccatctctgt 2640
   ggctatctgt cctctcttca tcgagcagaa atctgagggg ctccatttct ttcgtcattt 2700
   gtctgtcctt ctgaccctct ttgcctgtct gtctagatct ggccctgctg ctgccccgtc 2760
   cctctctgtg cctcctcctg acagtgtgtc tgtctgtgtc tcttcttcca tgagtctgtc 2820
   tgtccttagg ggctggggga tacaggggga gtgtggaggt gcacagagaa tcagtgcaga 2880
   ggaagtagat tgaggggagg gggcttggtg ctgggctgag gcagggttgg ggtgggtgtg 2940
   agcaggctcc tgtagaagag ggaaggaaca ggactttggg ggagggcagg ggagtctgga 3000
   g 3001
<210> 602
   <211> 3001
   <212> DNA
   <213> human
<400> 602
   tatattgtgt tatatagtag tatttatatt tatatatatt atgatgtgtt gtaatttata 60
   aatttatgta atgatagttt tttatgtata taatagtata tatatagata tagtggtaaa 120
   taaatataga tgtaatgata tagaaatata tatatagata tatttaatgt gtttttgtta 180
   cgttttatta tgtgatttat agatatataa tagtatatat atatatatat ataatggttt 240
   ttttatatat atagaaatag ataatgttta ttttatagat gtatattata tatacgtatg 300
   cgtataatgg tatgtatata tattttgatt taaatagtaa tatattatgt agtggcgtgc 360
   gtgttgattt atatattgtt ttagggaagt gaagatgttt attttttttt atgtattttt 420
   tatttttagt tttattggtt tttgtttggg tggagtttat agttaggggt tttagggttt 480
   agtattatcg ttgttatttt gtgggggtgt tatacgaggg ttttcgattt tggttgaaat 540
   tggtttgttt aatgagtttc gggtttagag tggagagggg ttgtttttcg gggttttttt 600
   taatattttt ggttttgggg attttgttta gatttttgtt attgtatata gattgatttt 660
   tggttgaggg gatttttacg tttttgtttt ttaaattatt tagtaataag atttaaggtt 720
   agatagaaga tgaattttta ggtaattcgg gataaagtta atttaatagg ttatgtgggt 780
   ttttttggtg gtttgggata gttaggttgt tttttaagtg taatttgtta ggaggaagta 840
   gaaggaattt ttcgtttatt tttatttgtt tttttgtttg ttttttcggt tttttttttt 900
   tggtttttgt tttttttttt gttttatttt aagttttatt ttttttagtt ttgttttttt 960
   ttgtggttgg atttttgtta tatttttttt cgttttgttt ttattagttt tattcgatgt 1020
   agggaggatt atttgtattt tattcgtttt tgtatttcgg aaggtgtgag aatgtgtgaa 1080
   gatgagggtg ggaggttata tgtgggagtt ttgggttgat atgtcgtgag aaatttgttt 1140
   ttttttttgt tttcgatttt gttagtcggt ttagtttatt agtttagcgt tattattatg 1200
   gaaattagta gtcgattttt tattggtggg tgggagggga ggaggaggag gaggagggga 1260
   gggttggttt ttttcggttt atttttttat ttaattttga ggtttagttt ttttcggagt 1320
   ttattatttc gaggtttttg gattgtaagg gatttttgta gtttttagag tagaggttag 1380
   taggggttga gattatgagg taagtagttt atcgatttag gtttttttag gattagttta 1440
   gttatttagt aagcgaaaga gtagtagtag agagtagagt cggagataaa ggattatagc 1500
   ggattattgg ggattatttt tagtagtttt tgaagttttt ggtcgggttg aggggttgcg 1560
   atagacggtt agatttatag atattgatag atatataacg atatcgatgt atatagttgt 1620
   acgatattga tatatttaga tataattagg gatagtatta tatattgtta tagttttgta 1680
   atatagatat ttatatatat atatataaat aattggtaat tttgaaatgg gtagatagtg 1740
   atatatagta ttattttttt taatatatag aagtgggggt atttattttt ttaatatatg 1800
   agatagtatt gatttgtacg gatagatttg gagatatata tacgtatttt tatttaggcg 1860
   tagttgtcgg tggtttttgt atattttttt tatttttata gggatatttt cgttttttta 1920
   tatattcgcg tatatatata gttattagta aatatagttt atatattttt ggttttagtt 1980
   ttagtagttt tggcgttttt tgtagttttt tttgtgggtg gggaagagtt tagttaattt 2040
   tggatggagc gtcggtgaag taagagttta gaggggagag gggttggttt ggtatgattt 2100
   tcgttagttt ttttatttgg ttgggtgatg tttttcggtt cgggttttgg ggttttttgg 2160
   tagtattatg gagtaattga taattttttt acggtttggg gattttggag ttatggagtt 2220
   atgggtattg tttatttggt atagttggat tttaggtcga gggggtgaat ttagtagtgt 2280
   agttttaagt atcgttgata ttttttcggt agttttgtag ttttaagaat tgaggtttga 2340
   ggagagtttt aagtttaaag gagacgggag ttttaggttc gtggggggaa ttgattttga 2400
   aggagtagag gtttgtttgt ttagtttggg ttagtaagta tttagttttg gattcgtttg 2460
   ttagaggtta gaggatgagg aagtggaggt ttttttgaag gtaagggatt tgtggagggt 2520
   tggggtttgt aaggtggggg ttttttatta tgttttttag tttggttagg aggtgaggcg 2580
   aggtatgtat ttttaatgtt tatttgtttt tttttggttt gtttgtgggt ttatttttgt 2640
   ggttatttgt ttttttttta tcgagtagaa atttgagggg ttttattttt ttcgttattt 2700
   gtttgttttt ttgatttttt ttgtttgttt gtttagattt ggttttgttg ttgtttcgtt 2760
   tttttttgtg tttttttttg atagtgtgtt tgtttgtgtt ttttttttta tgagtttgtt 2820
   tgtttttagg ggttggggga tataggggga gtgtggaggt gtatagagaa ttagtgtaga 2880
   ggaagtagat tgaggggagg gggtttggtg ttgggttgag gtagggttgg ggtgggtgtg 2940
   agtaggtttt tgtagaagag ggaaggaata ggattttggg ggagggtagg ggagtttgga 3000
   g 3001
<210> 603
   <211> 3001
   <212> DNA
   <213> human
<400> 603
   tatattgtgt tatatagtag tatttatatt tatatatatt atgatgtgtt gtaatttata      60
   aatttatgta atgatagttt tttatgtata taatagtata tatatagata tagtggtaaa     120
   taaatataga tgtaatgata tagaaatata tatatagata tatttaatgt gtttttgtta     180
   tgttttatta tgtgatttat agatatataa tagtatatat atatatatat ataatggttt     240
   ttttatatat atagaaatag ataatgttta ttttatagat gtatattata tatatgtatg     300
   tgtataatgg tatgtatata tattttgatt taaatagtaa tatattatgt agtggtgtgt     360
   gtgttgattt atatattgtt ttagggaagt gaagatgttt attttttttt atgtattttt     420
   tatttttagt tttattggtt tttgtttggg tggagtttat agttaggggt tttagggttt     480
   agtattattg ttgttatttt gtgggggtgt tatatgaggg tttttgattt tggttgaaat     540
   tggtttgttt aatgagtttt gggtttagag tggagagggg ttgttttttg gggttttttt      600
   taatattttt ggttttgggg attttgttta gatttttgtt attgtatata gattgatttt     660
   tggttgaggg gatttttatg tttttgtttt ttaaattatt tagtaataag atttaaggtt     720
   agatagaaga tgaattttta ggtaatttgg gataaagtta atttaatagg ttatgtgggt     780
   ttttttggtg gtttgggata gttaggttgt tttttaagtg taatttgtta ggaggaagta     840
   gaaggaattt tttgtttatt tttatttgtt tttttgtttg tttttttggt tttttttttt      900
   tggtttttgt tttttttttt gttttatttt aagttttatt ttttttagtt ttgttttttt     960
   ttgtggttgg atttttgtta tatttttttt tgttttgttt ttattagttt tatttgatgt    1020
   agggaggatt atttgtattt tatttgtttt tgtattttgg aaggtgtgag aatgtgtgaa    1080
   gatgagggtg ggaggttata tgtgggagtt ttgggttgat atgttgtgag aaatttgttt    1140
   ttttttttgt ttttgatttt gttagttggt ttagtttatt agtttagtgt tattattatg    1200
   gaaattagta gttgattttt tattggtggg tgggagggga ggaggaggag gaggagggga    1260
   gggttggttt tttttggttt atttttttat ttaattttga ggtttagttt tttttggagt    1320
   ttattatttt gaggtttttg gattgtaagg gatttttgta gtttttagag tagaggttag    1380
   taggggttga gattatgagg taagtagttt attgatttag gtttttttag gattagttta    1440
   gttatttagt aagtgaaaga gtagtagtag agagtagagt tggagataaa ggattatagt    1500
   ggattattgg ggattatttt tagtagtttt tgaagttttt ggttgggttg aggggttgtg    1560
   atagatggtt agatttatag atattgatag atatataatg atattgatgt atatagttgt    1620
   atgatattga tatatttaga tataattagg gatagtatta tatattgtta tagttttgta    1680
   atatagatat ttatatatat atatataaat aattggtaat tttgaaatgg gtagatagtg    1740
   atatatagta ttattttttt taatatatag aagtgggggt atttattttt ttaatatatg    1800
   agatagtatt gatttgtatg gatagatttg gagatatata tatgtatttt tatttaggtg    1860
   tagttgttgg tggtttttgt atattttttt tatttttata gggatatttt tgttttttta    1920
   tatatttgtg tatatatata gttattagta aatatagttt atatattttt ggttttagtt    1980
   ttagtagttt tggtgttttt tgtagttttt tttgtgggtg gggaagagtt tagttaattt    2040
   tggatggagt gttggtgaag taagagttta gaggggagag gggttggttt ggtatgattt    2100
   ttgttagttt ttttatttgg ttgggtgatg ttttttggtt tgggttttgg ggttttttgg    2160
   tagtattatg gagtaattga taattttttt atggtttggg gattttggag ttatggagtt    2220
   atgggtattg tttatttggt atagttggat tttaggttga gggggtgaat ttagtagtgt    2280
   agttttaagt attgttgata tttttttggt agttttgtag ttttaagaat tgaggtttga    2340
   ggagagtttt aagtttaaag gagatgggag ttttaggttt gtggggggaa ttgattttga    2400
   aggagtagag gtttgtttgt ttagtttggg ttagtaagta tttagttttg gatttgtttg    2460
   ttagaggtta gaggatgagg aagtggaggt ttttttgaag gtaagggatt tgtggagggt    2520
   tggggtttgt aaggtggggg ttttttatta tgttttttag tttggttagg aggtgaggtg    2580
   aggtatgtat ttttaatgtt tatttgtttt tttttggttt gtttgtgggt ttatttttgt    2640
   ggttatttgt ttttttttta ttgagtagaa atttgagggg ttttattttt tttgttattt    2700
   gtttgttttt ttgatttttt ttgtttgttt gtttagattt ggttttgttg ttgttttgtt    2760
   tttttttgtg tttttttttg atagtgtgtt tgtttgtgtt ttttttttta tgagtttgtt    2820
   tgtttttagg ggttggggga tataggggga gtgtggaggt gtatagagaa ttagtgtaga    2880
   ggaagtagat tgaggggagg gggtttggtg ttgggttgag gtagggttgg ggtgggtgtg    2940
   agtaggtttt tgtagaagag ggaaggaata ggattttggg ggagggtagg ggagtttgga    3000
   g                                                                     3001
<210> 604
   <211> 3001
   <212> DNA
   <213> human
<400> 604
   ttttagattt ttttgttttt ttttaaagtt ttgttttttt ttttttttat aggagtttgt      60
   ttatatttat tttaattttg ttttagttta gtattaagtt tttttttttt aatttatttt     120
   ttttgtattg attttttgtg tatttttata ttttttttgt attttttagt ttttaaggat      180
   agatagattt atggaagaag agatatagat agatatattg ttaggaggag gtatagagag     240
   ggacggggta gtagtagggt tagatttaga tagataggta aagagggtta gaaggataga     300
   taaatgacga aagaaatgga gttttttaga tttttgttcg atgaagagag gatagatagt      360
   tatagagatg gatttataga taggttaaga gaggataggt gggtattggg gatgtatatt     420
   tcgttttatt ttttggttag gttggaggat atggtgagag atttttattt tgtagatttt     480
   agttttttat agatttttta tttttaggaa agtttttatt tttttatttt ttggtttttg        540
   gtaggcgggt ttagagttgg atgtttgttg gtttaggttg ggtagataag tttttgtttt     600
   tttagggtta gtttttttta cgggtttgga gttttcgttt tttttgggtt tggaattttt     660
   tttagatttt agtttttgaa gttgtagagt tgtcggagga gtattagcga tgtttggggt     720
   tgtattgtta ggtttatttt ttcgatttgg agtttagtta tgttaggtgg gtagtgttta     780
   tggttttatg gttttagggt ttttaggtcg tgggagggtt gttagttgtt ttatggtatt     840
   gttaaggggt tttagaattc gggtcggggg atattattta gttaggtggg gggattggcg     900
   agggttatgt taggttagtt tttttttttt ttgagttttt gttttatcga cgttttattt     960
   aaggttggtt gagttttttt ttatttataa aggaggttgt agaaggcgtt agggttgttg    1020
   gagttggagt taggggtatg tgagttatgt ttgttggtag ttgtgtgtgt gcgcgagtat    1080
   gtggaggagc gagggtgttt ttgtgggggt agggaggatg tgtagaggtt atcgatagtt    1140
   gcgtttgggt gagggtgcgt gtgtgtgttt ttaggtttgt tcgtgtaggt taatgttgtt    1200
   ttatgtgttg ggaagatggg tgtttttatt tttgtgtgtt gaggggggtg gtgttgtgtg   1260
   ttattgtttg tttattttag agttgttagt tgtttgtgtg tgtgtgtgtg ggtgtttgta    1320
   ttgtagggtt gtggtagtat gtgatgttgt ttttggttgt atttgagtgt gttagtgtcg    1380
   tgtagttgtg tgtatcggtg tcgttgtgtg tttgttagtg tttgtgggtt tggtcgtttg    1440
   tcgtaatttt ttagttcggt tagaggtttt aggagttgtt gggggtgatt tttagtggtt    1500
   cgttgtggtt ttttattttc ggttttgttt tttgttgttg ttttttcgtt tgttgggtgg    1560
   ttgggttggt tttaaggagg tttgggtcga tgggttgttt gttttatagt tttagttttt    1620
   gttggttttt gttttgggga ttgtaggggt tttttatagt ttaggagttt cgagatggtg    1680
   ggtttcgagg agggttgagt tttagaattg ggtggggggg tgagtcggga agagttagtt    1740
   tttttttttt tttttttttt tttttttttt atttattaat aggaagtcgg ttgttggttt    1800
   ttatggtgat ggcgttggat tggtgggttg aatcggttga tagggtcggg gatagaagga    1860
   agagtaggtt ttttacggta tgttagttta gggtttttat atgtggtttt ttatttttat    1920
   ttttatatat ttttatattt ttcggagtat agaggcgagt ggggtgtaga tggttttttt    1980
   tatatcgggt gagattgata gagatagaac gggggagggt atgatagaga tttagttata    2040
   gagaagggta gaattagaaa agatgggatt tgagatgaaa taaagagaga gatagaaatt    2100
   aagagaaaga aatcgggaag ataagtaaag agataggtag aaatagacgg agaatttttt    2160
   ttgttttttt ttggtagatt gtatttgggg ggtaatttgg ttgttttagg ttattaggga    2220
   agtttatatg gtttgttggg ttggttttgt ttcgaattgt ttggaagttt attttttgtt   2280
   tagttttaga ttttgttgtt aaataatttg ggggataagg acgtgagaat ttttttagtt    2340
   aggagttagt ttatatgtag tggtaggggt ttagatagag tttttagagt taggggtgtt    2400
   gggagaagtt tcgagaggta gttttttttt attttgaatt cgggatttat taggtaagtt     2460
   agttttagtt aaggtcgggg gttttcgtgt ggtattttta tagaatagta gcggtgatat    2520
   tgaattttag agtttttgat tgtgggtttt atttaggtaa gagttaatgg aattggaggt    2580
   agggggtgta tggggaggag tggatatttt tattttttta aagtagtgtg taggttaata     2640
   cgtacgttat tgtatgatgt gttgttgttt aggttagagt atatatgtat gttattatgc    2700
   gtatgcgtgt gtatagtatg tatttataga atagatatta tttatttttg tatatgtgaa    2760
   ggggttattg tgtgtatata tgtgtgtgtt attatgtgtt tatgagttat ataataaaac    2820
   gtaataaaaa tatattgggt gtatttgtat gtatgttttt gtgttattgt atttatgttt    2880
   gtttgttatt gtgtttatgt gtgtgttgtt atgtgtatgg gaggttatta ttgtatgagt    2940
   ttataaatta taatatatta tgatatatat agatatggat gttattatat ggtataatgt    3000
   a                                                                    3001
<210> 605
   <211> 3001
   <212> DNA
   <213> human
<400> 605
   ttttagattt ttttgttttt ttttaaagtt ttgttttttt ttttttttat aggagtttgt      60
   ttatatttat tttaattttg ttttagttta gtattaagtt tttttttttt aatttatttt     120
   ttttgtattg attttttgtg tatttttata ttttttttgt attttttagt ttttaaggat     180
   agatagattt atggaagaag agatatagat agatatattg ttaggaggag gtatagagag     240
   ggatggggta gtagtagggt tagatttaga tagataggta aagagggtta gaaggataga     300
   taaatgatga aagaaatgga gttttttaga tttttgtttg atgaagagag gatagatagt     360
   tatagagatg gatttataga taggttaaga gaggataggt gggtattggg gatgtatatt     420
   ttgttttatt ttttggttag gttggaggat atggtgagag atttttattt tgtagatttt     480
   agttttttat agatttttta tttttaggaa agtttttatt tttttatttt ttggtttttg     540
   gtaggtgggt ttagagttgg atgtttgttg gtttaggttg ggtagataag tttttgtttt     600
   tttagggtta gtttttttta tgggtttgga gtttttgttt tttttgggtt tggaattttt     660
   tttagatttt agtttttgaa gttgtagagt tgttggagga gtattagtga tgtttggggt     720
   tgtattgtta ggtttatttt tttgatttgg agtttagtta tgttaggtgg gtagtgttta     780
   tggttttatg gttttagggt ttttaggttg tgggagggtt gttagttgtt ttatggtatt     840
   gttaaggggt tttagaattt gggttggggg atattattta gttaggtggg gggattggtg     900
   agggttatgt taggttagtt tttttttttt ttgagttttt gttttattga tgttttattt     960
   aaggttggtt gagttttttt ttatttataa aggaggttgt agaaggtgtt agggttgttg    1020
   gagttggagt taggggtatg tgagttatgt ttgttggtag ttgtgtgtgt gtgtgagtat    1080
   gtggaggagt gagggtgttt ttgtgggggt agggaggatg tgtagaggtt attgatagtt    1140
   gtgtttgggt gagggtgtgt gtgtgtgttt ttaggtttgt ttgtgtaggt taatgttgtt    1200
   ttatgtgttg ggaagatggg tgtttttatt tttgtgtgtt gaggggggtg gtgttgtgtg    1260
   ttattgtttg tttattttag agttgttagt tgtttgtgtg tgtgtgtgtg ggtgtttgta    1320
   ttgtagggtt gtggtagtat gtgatgttgt ttttggttgt atttgagtgt gttagtgttg    1380
   tgtagttgtg tgtattggtg ttgttgtgtg tttgttagtg tttgtgggtt tggttgtttg    1440
   ttgtaatttt ttagtttggt tagaggtttt aggagttgtt gggggtgatt tttagtggtt    1500
   tgttgtggtt ttttattttt ggttttgttt tttgttgttg tttttttgtt tgttgggtgg    1560
   ttgggttggt tttaaggagg tttgggttga tgggttgttt gttttatagt tttagttttt    1620
   gttggttttt gttttgggga ttgtaggggt tttttatagt ttaggagttt tgagatggtg    1680
   ggttttgagg agggttgagt tttagaattg ggtggggggg tgagttggga agagttagtt    1740
   tttttttttt tttttttttt tttttttttt atttattaat aggaagttgg ttgttggttt    1800
   ttatggtgat ggtgttggat tggtgggttg aattggttga tagggttggg gatagaagga    1860
   agagtaggtt ttttatggta tgttagttta gggtttttat atgtggtttt ttatttttat    1920
   ttttatatat ttttatattt tttggagtat agaggtgagt ggggtgtaga tggttttttt    1980
   tatattgggt gagattgata gagatagaat gggggagggt atgatagaga tttagttata    2040
   gagaagggta gaattagaaa agatgggatt tgagatgaaa taaagagaga gatagaaatt    2100
   aagagaaaga aattgggaag ataagtaaag agataggtag aaatagatgg agaatttttt    2160
   ttgttttttt ttggtagatt gtatttgggg ggtaatttgg ttgttttagg ttattaggga    2220
   agtttatatg gtttgttggg ttggttttgt tttgaattgt ttggaagttt attttttgtt    2280
   tagttttaga ttttgttgtt aaataatttg ggggataagg atgtgagaat ttttttagtt    2340
   aggagttagt ttatatgtag tggtaggggt ttagatagag tttttagagt taggggtgtt    2400
   gggagaagtt ttgagaggta gttttttttt attttgaatt tgggatttat taggtaagtt    2460
   agttttagtt aaggttgggg gtttttgtgt ggtattttta tagaatagta gtggtgatat    2520
   tgaattttag agtttttgat tgtgggtttt atttaggtaa gagttaatgg aattggaggt    2580
   agggggtgta tggggaggag tggatatttt tattttttta aagtagtgtg taggttaata    2640
   tgtatgttat tgtatgatgt gttgttgttt aggttagagt atatatgtat gttattatgt    2700
   gtatgtgtgt gtatagtatg tatttataga atagatatta tttatttttg tatatgtgaa    2760
   ggggttattg tgtgtatata tgtgtgtgtt attatgtgtt tatgagttat ataataaaat    2820
   gtaataaaaa tatattgggt gtatttgtat gtatgttttt gtgttattgt atttatgttt    2880
   gtttgttatt gtgtttatgt gtgtgttgtt atgtgtatgg gaggttatta ttgtatgagt    2940
   ttataaatta taatatatta tgatatatat agatatggat gttattatat ggtataatgt    3000
   a                                                                    3001
<210> 606
   <211> 50
   <212> DNA
   <213> human
<400> 606
   cggcaccatg acccagggaa aggtaccggc atctccccag cctgcaccat 50
<210> 607
   <211> 50
   <212> DNA
   <213> human
<400> 607
   cggtattatg atttagggaa aggtatcggt atttttttag tttgtattat 50
<210> 608
   <211> 50
   <212> DNA
   <213> human
<400> 608
   tggtattatg atttagggaa aggtattggt atttttttag tttgtattat 50
<210> 609
   <211> 50
   <212> DNA
   <213> human
<400> 609
   atggtgtagg ttggggagat gtcggtattt ttttttgggt tatggtgtcg 50
<210> 610
   <211> 50
   <212> DNA
   <213> human
<400> 610
   atggtgtagg ttggggagat gttggtattt ttttttgggt tatggtgttg 50
<210> 611
   <211> 3001
   <212> DNA
   <213> human
<400> 611
   actccagcct gagcaacaga acaagatttt ctctgtaaaa aaaaaaaaaa aaaaaaaaaa 60
   aaagactttg ataatttgtc tgcctacctg ggccctgctt tctcagggac tttaggtggg 120
   tccctgggca gtgagaggga agcagaaggt aagtggccac ccccacctgt cccaggtctt 180
   agtttctcgt cccatcctag caggttcaga tacttctttt aggcagattt gtgggtgctc 240
   gctctggggc agcccaggac attctggtag gagactcctc agttaataat acctcacata 300
   cttccagcac ttcttggtct tttatgtgct tttacggcca taacctgtgc gtctgacagt 360
   caatctagga tggacagagc tctgttctaa gcctcttttt cgtaggagct gcagctcaga 420
   gaggttaagg gccttgccaa ggccacgcgg ccagtgggtg actaaattgg gacttgatcc 480
   cagctcttga gattgcaaac cccatagtga cactgcagca gctacagaaa acggggcctg 540
   atgatggctg ggtcccagtg acacgtctgg ggaagcgggg agaacgagct ctggcatggg 600
   aggaatggaa aaggggggaa gatggagacg gggtgatgga agctaagcgt ggaggcagga 660
   agagagctct ggcaggggtt gtagaggctt tgagacagaa ctagcggtgt ctccagaaag 720
   accccgtgaa atgtcccagc agggctgaaa gaaacagaag aggtgtcctc ccaccccatg 780
   tctctgtgaa ccaaggcagg gagggaccta ctgtgtgcat ttcacaggcg agaaaaagga 840
   gcaaggatag gtggctattg gcttgcccca ggcccacagg agcgcagggc ccaggggccc 900
   tgttagaagc gggcagcaca ggcgagactt ccagaccttc ccctctacca gggatgatga 960
   aatagatgat gagaggggag gtggagatgg agacagaaat cagggccgca tccaaagagg 1020
   gaggaggaaa aggcagaggc gcagctggag ctgcagagag gtgcagagac agtggagatg 1080
   aggccctgat gcccggctct ggtttcacgc gggcagcgtc agctggtcgg tgtcacggag 1140
   ttcccgcgtc ctccgcccgc tcagcccccg cggacccatc cactggggtt gctccgcagc 1200
   gcgactctct ctcatcccgg gggagggcta agggcggcgg tggggggcgg ggtggggggg 1260
   aagcggcggg agggggcggt gcgccgccgc cttaccccgg gctcgcgaat ccgcagcgtc 1320
   ctccgcagca tcttcccagg cccggccctc tcctcccccc gcgccgatgg tacgcgccgg 1380
   ctcgcctggc ccggctgcag tggatgttgc tagaacccac gcggaggaag gaagagacgc 1440
   aggcaggctg cggttaccca agcggccacc cgggcctcag ggaccccttc cccgagagac 1500
   ggcaccatga cccagggaaa ggtaccggca tctccccagc ctgcaccatc ctccatcctc 1560
   cggctcccgg ctccctgtca tcctcagcaa gccctctgga ccacgaaagc atcctcccct 1620
   ggccctagtt tgaaggtcct ccgtggtcct tgctgaagtc agaggaccga aaggccggtg 1680
   tcaaggaatg gaccgaggcc tcttcctgcc cgcaggcgcg cctctcctcc cctgtcaccc 1740
   tgccacccct gctttggcat tacagcatta caaggctcct cacagatacc caggcccccc 1800
   gtgaggagtc agcccgggcg tctggtgtga ctgtatgtgt gtgcatgggt atgtgtgtgc 1860
   cagggtgagc cctgaggggc agtgcctatc gggcaacccc ctctggccca accctccagt 1920
   cccaaaccca accactttat gctcatcttt cccaccctcc acggaaacaa cccccaccct 1980
   actccctgcc attgttccac atctgggcgt cttggctcca tccctgtgct tggggcttgc 2040
   tatgacaact ctgcatcaag atgctctaat tgttgttttg gttttggcca ggcatggatg 2100
   ggtccggatg aagcatctct tctccagttt taggcctgaa ttgtagggga ggggctgggg 2160
   cagagactcg gctggcctag taggcttatg taacatcaat agcctgtgga attatacaac 2220
   cactagggcc tggggctaag ggggagggta ctcgggccct ctgtttgggt tgctaaggat 2280
   tgggcagaga aaacagggga aggacctccc ctacactccc ccttcttctc tttgagagga 2340
   gcttcagagc caatgacatc cttccctgga ccctcagacc ctcctctccc tgtatgcaag 2400
   ctctttgggc ctcaaatgat gtcactgggt gataccacct cccgccattg gtcacacctc 2460
   acttgggagt gccgtatgtg tgtcgggggc ctgggcatta gggtccagga ggctctgggg 2520
   ggcggagtgg cagggtggag gctgaactcg atttaggtgg acatgatcct tcagatccgg 2580
   gtgatgaggg gggctgggga gtgggaggca gtgggaggcc tggggagtgt tcagttccgt 2640
   aaacatggag aactcacccc ttctgctgag tctccttcca cctccaccct gcctccacca 2700
   ggccctttag tggcgctgtt cccttcctgg tgtgaggggg acctctgcct cctctgcccc 2760
   ttgtccccag ctcacgcaca ggcttgctgg gggatggaga agaccgctcc agttcagtgg 2820
   tccttgggcc tggatggaac cagaagtctt ctggtgcgtg cctgtctcca ggctgcctca 2880
   gccagatgct ccctgcaaag aaggaagtcc gcagtcttcc tcagtggccc tttaggtcta 2940
   acacagtctt ctgggaacca gacctaaatc ccattttctg agagccgggc tttttgttgc 3000
   t 3001

<210> 612
   <211> 3001
   <212> DNA
   <213> human
<400> 612
   attttagttt gagtaataga ataagatttt ttttgtaaaa aaaaaaaaaa aaaaaaaaaa 60
   aaagattttg ataatttgtt tgtttatttg ggttttgttt ttttagggat tttaggtggg 120
   tttttgggta gtgagaggga agtagaaggt aagtggttat ttttatttgt tttaggtttt 180
   agtttttcgt tttattttag taggtttaga tatttttttt aggtagattt gtgggtgttc 240
   gttttggggt agtttaggat attttggtag gagatttttt agttaataat attttatata 300
   tttttagtat tttttggttt tttatgtgtt tttacggtta taatttgtgc gtttgatagt 360
   taatttagga tggatagagt tttgttttaa gttttttttt cgtaggagtt gtagtttaga 420
   gaggttaagg gttttgttaa ggttacgcgg ttagtgggtg attaaattgg gatttgattt 480
   tagtttttga gattgtaaat tttatagtga tattgtagta gttatagaaa acggggtttg 540
   atgatggttg ggttttagtg atacgtttgg ggaagcgggg agaacgagtt ttggtatggg 600
   aggaatggaa aaggggggaa gatggagacg gggtgatgga agttaagcgt ggaggtagga 660
   agagagtttt ggtaggggtt gtagaggttt tgagatagaa ttagcggtgt ttttagaaag 720
   atttcgtgaa atgttttagt agggttgaaa gaaatagaag aggtgttttt ttattttatg 780
   tttttgtgaa ttaaggtagg gagggattta ttgtgtgtat tttataggcg agaaaaagga 840
   gtaaggatag gtggttattg gtttgtttta ggtttatagg agcgtagggt ttaggggttt 900
   tgttagaagc gggtagtata ggcgagattt ttagattttt ttttttatta gggatgatga 960
   aatagatgat gagaggggag gtggagatgg agatagaaat tagggtcgta tttaaagagg 1020
   gaggaggaaa aggtagaggc gtagttggag ttgtagagag gtgtagagat agtggagatg 1080
   aggttttgat gttcggtttt ggttttacgc gggtagcgtt agttggtcgg tgttacggag 1140
   ttttcgcgtt tttcgttcgt ttagttttcg cggatttatt tattggggtt gtttcgtagc 1200
   gcgatttttt tttatttcgg gggagggtta agggcggcgg tggggggcgg ggtggggggg 1260
   aagcggcggg agggggcggt gcgtcgtcgt tttatttcgg gttcgcgaat tcgtagcgtt 1320
   tttcgtagta tttttttagg ttcggttttt tttttttttc gcgtcgatgg tacgcgtcgg 1380
   ttcgtttggt tcggttgtag tggatgttgt tagaatttac gcggaggaag gaagagacgt 1440
   aggtaggttg cggttattta agcggttatt cgggttttag ggattttttt ttcgagagac 1500
   ggtattatga tttagggaaa ggtatcggta tttttttagt ttgtattatt ttttattttt 1560
   cggttttcgg ttttttgtta tttttagtaa gttttttgga ttacgaaagt attttttttt 1620
   ggttttagtt tgaaggtttt tcgtggtttt tgttgaagtt agaggatcga aaggtcggtg 1680
   ttaaggaatg gatcgaggtt tttttttgtt cgtaggcgcg tttttttttt tttgttattt 1740
   tgttattttt gttttggtat tatagtatta taaggttttt tatagatatt taggtttttc 1800
   gtgaggagtt agttcgggcg tttggtgtga ttgtatgtgt gtgtatgggt atgtgtgtgt 1860
   tagggtgagt tttgaggggt agtgtttatc gggtaatttt ttttggttta attttttagt 1920
   tttaaattta attattttat gtttattttt tttatttttt acggaaataa tttttatttt 1980
   attttttgtt attgttttat atttgggcgt tttggtttta tttttgtgtt tggggtttgt 2040
   tatgataatt ttgtattaag atgttttaat tgttgttttg gttttggtta ggtatggatg 2100
   ggttcggatg aagtattttt tttttagttt taggtttgaa ttgtagggga ggggttgggg 2160
   tagagattcg gttggtttag taggtttatg taatattaat agtttgtgga attatataat 2220
   tattagggtt tggggttaag ggggagggta ttcgggtttt ttgtttgggt tgttaaggat 2280
   tgggtagaga aaatagggga aggatttttt ttatattttt tttttttttt tttgagagga 2340
   gttttagagt taatgatatt tttttttgga tttttagatt tttttttttt tgtatgtaag 2400
   ttttttgggt tttaaatgat gttattgggt gatattattt ttcgttattg gttatatttt 2460
   atttgggagt gtcgtatgtg tgtcgggggt ttgggtatta gggtttagga ggttttgggg 2520
   ggcggagtgg tagggtggag gttgaattcg atttaggtgg atatgatttt ttagattcgg 2580
   gtgatgaggg gggttgggga gtgggaggta gtgggaggtt tggggagtgt ttagtttcgt 2640
   aaatatggag aatttatttt ttttgttgag ttttttttta tttttatttt gtttttatta 2700
   ggttttttag tggcgttgtt ttttttttgg tgtgaggggg atttttgttt tttttgtttt 2760
   ttgtttttag tttacgtata ggtttgttgg gggatggaga agatcgtttt agtttagtgg 2820
   tttttgggtt tggatggaat tagaagtttt ttggtgcgtg tttgttttta ggttgtttta 2880
   gttagatgtt ttttgtaaag aaggaagttc gtagtttttt ttagtggttt tttaggttta 2940
   atatagtttt ttgggaatta gatttaaatt ttattttttg agagtcgggt tttttgttgt 3000
   t 3001
<210> 613
   <211> 3001
   <212> DNA
   <213> human
<400> 613
   attttagttt gagtaataga ataagatttt ttttgtaaaa aaaaaaaaaa aaaaaaaaaa 60
   aaagattttg ataatttgtt tgtttatttg ggttttgttt ttttagggat tttaggtggg 120
   tttttgggta gtgagaggga agtagaaggt aagtggttat ttttatttgt tttaggtttt 180
   agttttttgt tttattttag taggtttaga tatttttttt aggtagattt gtgggtgttt 240
   gttttggggt agtttaggat attttggtag gagatttttt agttaataat attttatata 300
   tttttagtat tttttggttt tttatgtgtt tttatggtta taatttgtgt gtttgatagt 360
   taatttagga tggatagagt tttgttttaa gttttttttt tgtaggagtt gtagtttaga 420
   gaggttaagg gttttgttaa ggttatgtgg ttagtgggtg attaaattgg gatttgattt 480
   tagtttttga gattgtaaat tttatagtga tattgtagta gttatagaaa atggggtttg 540
   atgatggttg ggttttagtg atatgtttgg ggaagtgggg agaatgagtt ttggtatggg 600
   aggaatggaa aaggggggaa gatggagatg gggtgatgga agttaagtgt ggaggtagga 660
   agagagtttt ggtaggggtt gtagaggttt tgagatagaa ttagtggtgt ttttagaaag 720
   attttgtgaa atgttttagt agggttgaaa gaaatagaag aggtgttttt ttattttatg 780
   tttttgtgaa ttaaggtagg gagggattta ttgtgtgtat tttataggtg agaaaaagga 840
   gtaaggatag gtggttattg gtttgtttta ggtttatagg agtgtagggt ttaggggttt 900
   tgttagaagt gggtagtata ggtgagattt ttagattttt ttttttatta gggatgatga 960
   aatagatgat gagaggggag gtggagatgg agatagaaat tagggttgta tttaaagagg 1020
   gaggaggaaa aggtagaggt gtagttggag ttgtagagag gtgtagagat agtggagatg 1080
   aggttttgat gtttggtttt ggttttatgt gggtagtgtt agttggttgg tgttatggag 1140
   tttttgtgtt ttttgtttgt ttagtttttg tggatttatt tattggggtt gttttgtagt 1200
   gtgatttttt tttattttgg gggagggtta agggtggtgg tggggggtgg ggtggggggg 1260
   aagtggtggg agggggtggt gtgttgttgt tttattttgg gtttgtgaat ttgtagtgtt 1320
   ttttgtagta tttttttagg tttggttttt tttttttttt gtgttgatgg tatgtgttgg 1380
   tttgtttggt ttggttgtag tggatgttgt tagaatttat gtggaggaag gaagagatgt 1440
   aggtaggttg tggttattta agtggttatt tgggttttag ggattttttt tttgagagat 1500
   ggtattatga tttagggaaa ggtattggta tttttttagt ttgtattatt ttttattttt 1560
   tggtttttgg ttttttgtta tttttagtaa gttttttgga ttatgaaagt attttttttt 1620
   ggttttagtt tgaaggtttt ttgtggtttt tgttgaagtt agaggattga aaggttggtg 1680
   ttaaggaatg gattgaggtt tttttttgtt tgtaggtgtg tttttttttt tttgttattt 1740
   tgttattttt gttttggtat tatagtatta taaggttttt tatagatatt taggtttttt 1800
   gtgaggagtt agtttgggtg tttggtgtga ttgtatgtgt gtgtatgggt atgtgtgtgt 1860
   tagggtgagt tttgaggggt agtgtttatt gggtaatttt ttttggttta attttttagt 1920
   tttaaattta attattttat gtttattttt tttatttttt atggaaataa tttttatttt 1980
   atttttgttt attgttttat atttgggtgt tttggtttta tttttgtgtt tggggtttgt 2040
   tatgataatt ttgtattaag atgttttaat tgttgttttg gttttggtta ggtatggatg 2100
   ggtttggatg aagtattttt tttttagttt taggtttgaa ttgtagggga ggggttgggg 2160
   tagagatttg gttggtttag taggtttatg taatattaat agtttgtgga attatataat 2220
   tattagggtt tggggttaag ggggagggta tttgggtttt ttgtttgggt tgttaaggat 2280
   tgggtagaga aaatagggga aggatttttt ttatattttt tttttttttt tttgagagga 2340
   gttttagagt taatgatatt tttttttgga tttttagatt tttttttttt tgtatgtaag 2400
   ttttttgggt tttaaatgat gttattgggt gatattattt tttgttattg gttatatttt 2460
   atttgggagt gttgtatgtg tgttgggggt ttgggtatta gggtttagga ggttttgggg 2520
   ggtggagtgg tagggtggag gttgaatttg atttaggtgg atatgatttt ttagatttgg 2580
   gtgatgaggg gggttgggga gtgggaggta gtgggaggtt tggggagtgt ttagttttgt 2640
   aaatatggag aatttatttt ttttgttgag ttttttttta tttttatttt gtttttatta 2700
   ggttttttag tggtgttgtt ttttttttgg tgtgaggggg atttttgttt tttttgtttt 2760
   ttgtttttag tttatgtata ggtttgttgg gggatggaga agattgtttt agtttagtgg 2820
   tttttgggtt tggatggaat tagaagtttt ttggtgtgtg tttgttttta ggttgtttta 2880
   gttagatgtt ttttgtaaag aaggaagttt gtagtttttt ttagtggttt tttaggttta 2940
   atatagtttt ttgggaatta gatttaaatt ttattttttg agagttgggt tttttgttgt 3000
   t 3001
<210> 614
   <211> 3001
   <212> DNA
   <213> human
<400> 614
   agtaataaaa agttcggttt ttagaaaatg ggatttaggt ttggttttta gaagattgtg 60
   ttagatttaa agggttattg aggaagattg cggatttttt tttttgtagg gagtatttgg 120
   ttgaggtagt ttggagatag gtacgtatta gaagattttt ggttttattt aggtttaagg 180
   attattgaat tggagcggtt tttttatttt tttagtaagt ttgtgcgtga gttggggata 240
   aggggtagag gaggtagagg ttttttttat attaggaagg gaatagcgtt attaaagggt 300
   ttggtggagg tagggtggag gtggaaggag atttagtaga aggggtgagt tttttatgtt 360
   tacggaattg aatatttttt aggtttttta ttgtttttta ttttttagtt ttttttatta 420
   ttcggatttg aaggattatg tttatttaaa tcgagtttag tttttatttt gttatttcgt 480
   tttttagagt tttttggatt ttaatgttta ggttttcgat atatatacgg tatttttaag 540
   tgaggtgtga ttaatggcgg gaggtggtat tatttagtga tattatttga ggtttaaaga 600
   gtttgtatat agggagagga gggtttgagg gtttagggaa ggatgttatt ggttttgaag 660
   ttttttttaa agagaagaag ggggagtgta ggggaggttt tttttttgtt ttttttgttt 720
   aatttttagt aatttaaata gagggttcga gtattttttt ttttagtttt aggttttagt 780
   ggttgtataa ttttataggt tattgatgtt atataagttt attaggttag tcgagttttt 840
   gttttagttt tttttttata atttaggttt aaaattggag aagagatgtt ttattcggat 900
   ttatttatgt ttggttaaaa ttaaaataat aattagagta ttttgatgta gagttgttat 960
   agtaagtttt aagtataggg atggagttaa gacgtttaga tgtggaataa tggtagggag 1020
   tagggtgggg gttgttttcg tggagggtgg gaaagatgag tataaagtgg ttgggtttgg 1080
   gattggaggg ttgggttaga gggggttgtt cgataggtat tgttttttag ggtttatttt 1140
   ggtatatata tatttatgta tatatatata gttatattag acgttcgggt tgatttttta 1200
   cggggggttt gggtatttgt gaggagtttt gtaatgttgt aatgttaaag taggggtggt 1260
   agggtgatag gggaggagag gcgcgtttgc gggtaggaag aggtttcggt ttattttttg 1320
   atatcggttt ttcggttttt tgattttagt aaggattacg gaggattttt aaattagggt 1380
   taggggagga tgttttcgtg gtttagaggg tttgttgagg atgataggga gtcgggagtc 1440
   ggaggatgga ggatggtgta ggttggggag atgtcggtat ttttttttgg gttatggtgt 1500
   cgtttttcgg ggaaggggtt tttgaggttc gggtggtcgt ttgggtaatc gtagtttgtt 1560
   tgcgtttttt ttttttttcg cgtgggtttt agtaatattt attgtagtcg ggttaggcga 1620
   gtcggcgcgt attatcggcg cggggggagg agagggtcgg gtttgggaag atgttgcgga 1680
   ggacgttgcg gattcgcgag ttcggggtaa ggcggcggcg tatcgttttt tttcgtcgtt 1740
   ttttttttat ttcgtttttt atcgtcgttt ttagtttttt ttcgggatga gagagagtcg 1800
   cgttgcggag taattttagt ggatgggttc gcgggggttg agcgggcgga ggacgcggga 1860
   atttcgtgat atcgattagt tgacgttgtt cgcgtgaaat tagagtcggg tattagggtt 1920
   ttatttttat tgtttttgta tttttttgta gttttagttg cgtttttgtt tttttttttt 1980
   ttttttttgg atgcggtttt gatttttgtt tttattttta tttttttttt tattatttat 2040
   tttattattt ttggtagagg ggaaggtttg gaagtttcgt ttgtgttgtt cgtttttaat 2100
   agggtttttg ggttttgcgt ttttgtgggt ttggggtaag ttaatagtta tttatttttg 2160
   tttttttttt tcgtttgtga aatgtatata gtaggttttt ttttgttttg gtttatagag 2220
   atatggggtg ggaggatatt ttttttgttt tttttagttt tgttgggata ttttacgggg 2280
   tttttttgga gatatcgtta gttttgtttt aaagttttta taatttttgt tagagttttt 2340
   tttttgtttt tacgtttagt ttttattatt tcgtttttat tttttttttt tttttatttt 2400
   ttttatgtta gagttcgttt ttttcgtttt tttagacgtg ttattgggat ttagttatta 2460
   ttaggtttcg ttttttgtag ttgttgtagt gttattatgg ggtttgtaat tttaagagtt 2520
   gggattaagt tttaatttag ttatttattg gtcgcgtggt tttggtaagg tttttaattt 2580
   ttttgagttg tagtttttac gaaaaagagg tttagaatag agttttgttt attttagatt 2640
   gattgttaga cgtataggtt atggtcgtaa aagtatataa aagattaaga agtgttggaa 2700
   gtatgtgagg tattattaat tgaggagttt tttattagaa tgttttgggt tgttttagag 2760
   cgagtattta taaatttgtt taaaagaagt atttgaattt gttaggatgg gacgagaaat 2820
   taagatttgg gataggtggg ggtggttatt tattttttgt ttttttttta ttgtttaggg 2880
   atttatttaa agtttttgag aaagtagggt ttaggtaggt agataaatta ttaaagtttt 2940
   tttttttttt tttttttttt tttttataga gaaaattttg ttttgttgtt taggttggag 3000
   t 3001
<210> 615
   <211> 3001
   <212> DNA
   <213> human
<400> 615
   agtaataaaa agtttggttt ttagaaaatg ggatttaggt ttggttttta gaagattgtg 60
   ttagatttaa agggttattg aggaagattg tggatttttt tttttgtagg gagtatttgg 120
   ttgaggtagt ttggagatag gtatgtatta gaagattttt ggttttattt aggtttaagg 180
   attattgaat tggagtggtt ttttttattt tttagtaagt ttgtgtgtga gttggggata 240
   aggggtagag gaggtagagg ttttttttat attaggaagg gaatagtgtt attaaagggt 300
   ttggtggagg tagggtggag gtggaaggag atttagtaga aggggtgagt tttttatgtt 360
   tatggaattg aatatttttt aggtttttta ttgtttttta ttttttagtt ttttttatta 420
   tttggatttg aaggattatg tttatttaaa ttgagtttag tttttatttt gttattttgt 480
   tttttagagt tttttggatt ttaatgttta ggtttttgat atatatatgg tatttttaag 540
   tgaggtgtga ttaatggtgg gaggtggtat tatttagtga tattatttga ggtttaaaga 600
   gtttgtatat agggagagga gggtttgagg gtttagggaa ggatgttatt ggttttgaag 660
   ttttttttaa agagaagaag ggggagtgta ggggaggttt tttttttgtt ttttttgttt 720
   aatttttagt aatttaaata gagggtttga gtattttttt ttttagtttt aggttttagt 780
   ggttgtataa ttttataggt tattgatgtt atataagttt attaggttag ttgagttttt 840
   gttttagttt tttttttata atttaggttt aaaattggag aagagatgtt ttatttggat 900
   ttatttatgt ttggttaaaa ttaaaataat aattagagta ttttgatgta gagttgttat 960
   agtaagtttt aagtataggg atggagttaa gatgtttaga tgtggaataa tggtagggag 1020
   tagggtgggg gttgtttttg tggagggtgg gaaagatgag tataaagtgg ttgggtttgg 1080
   gattggaggg ttgggttaga gggggttgtt tgataggtat tgttttttag ggtttatttt 1140
   ggtatatata tatttatgta tatatatata gttatattag atgtttgggt tgatttttta 1200
   tggggggttt gggtatttgt gaggagtttt gtaatgttgt aatgttaaag taggggtggt 1260
   agggtgatag gggaggagag gtgtgtttgt gggtaggaag aggttttggt ttattttttg 1320
   atattggttt tttggttttt tgattttagt aaggattatg gaggattttt aaattagggt 1380
   taggggagga tgtttttgtg gtttagaggg tttgttgagg atgataggga gttgggagtt 1440
   ggaggatgga ggatggtgta ggttggggag atgttggtat ttttttttgg gttatggtgt 1500
   tgttttttgg ggaaggggtt tttgaggttt gggtggttgt ttgggtaatt gtagtttgtt 1560
   tgtgtttttt tttttttttg tgtgggtttt agtaatattt attgtagttg ggttaggtga 1620
   gttggtgtgt attattggtg tggggggagg agagggttgg gtttgggaag atgttgtgga 1680
   ggatgttgtg gatttgtgag tttggggtaa ggtggtggtg tattgttttt ttttgttgtt 1740
   ttttttttat tttgtttttt attgttgttt ttagtttttt tttgggatga gagagagttg 1800
   tgttgtggag taattttagt ggatgggttt gtgggggttg agtgggtgga ggatgtggga 1860
   attttgtgat attgattagt tgatgttgtt tgtgtgaaat tagagttggg tattagggtt 1920
   ttatttttat tgtttttgta tttttttgta gttttagttg tgtttttgtt tttttttttt 1980
   ttttttttgg atgtggtttt gatttttgtt tttattttta tttttttttt tattatttat 2040
   tttattattt ttggtagagg ggaaggtttg gaagttttgt ttgtgttgtt tgtttttaat 2100
   agggtttttg ggttttgtgt ttttgtgggt ttggggtaag ttaatagtta tttatttttg 2160
   tttttttttt ttgtttgtga aatgtatata gtaggttttt ttttgttttg gtttatagag 2220
   atatggggtg ggaggatatt ttttttgttt tttttagttt tgttgggata ttttatgggg 2280
   tttttttgga gatattgtta gttttgtttt aaagttttta taatttttgt tagagttttt 2340
   tttttgtttt tatgtttagt ttttattatt ttgtttttat tttttttttt tttttatttt 2400
   ttttatgtta gagtttgttt tttttgtttt tttagatgtg ttattgggat ttagttatta 2460
   ttaggttttg ttttttgtag ttgttgtagt gttattatgg ggtttgtaat tttaagagtt 2520
   gggattaagt tttaatttag ttatttattg gttgtgtggt tttggtaagg tttttaattt 2580
   ttttgagttg tagtttttat gaaaaagagg tttagaatag agttttgttt attttagatt 2640
   gattgttaga tgtataggtt atggttgtaa aagtatataa aagattaaga agtgttggaa 2700
   gtatgtgagg tattattaat tgaggagttt tttattagaa tgttttgggt tgttttagag 2760
   tgagtattta taaatttgtt taaaagaagt atttgaattt gttaggatgg gatgagaaat 2820
   taagatttgg gataggtggg ggtggttatt tattttttgt ttttttttta ttgtttaggg 2880
   atttatttaa agtttttgag aaagtagggt ttaggtaggt agataaatta ttaaagtttt 2940
   tttttttttt tttttttttt tttttataga gaaaattttg ttttgttgtt taggttggag 3000
   t 3001
<210> 616
   <211> 50
   <212> DNA
   <213> human
<400> 616
   ttggagagcg ctcggggtac cacgaggctt tgcgcaggca agaagttgcg 50
<210> 617
   <211> 50
   <212> DNA
   <213> human
<400> 617
   ttggagagcg ttcggggtat tacgaggttt tgcgtaggta agaagttgcg 50
<210> 618
   <211> 50
   <212> DNA
   <213> human
<400> 618
   ttggagagtg tttggggtat tatgaggttt tgtgtaggta agaagttgtg 50
<210> 619
   <211> 50
   <212> DNA
   <213> human
<400> 619
   cgtaattttt tgtttgcgta aagtttcgtg gtatttcgag cgttttttaa 50
<210> 620
   <211> 50
   <212> DNA
   <213> human
<400> 620
   tgtaattttt tgtttgtgta aagttttgtg gtattttgag tgttttttaa 50
<210> 621
   <211> 3001
   <212> DNA
   <213> human
<400> 621
   ctgccttcca gctgcgccgc atcctgttct ccgtcgccgc ctggtggcga gctcccggga     60
   cccgcaggcg ctcgggtgag gcaggaggga aggcgcgggg aacaaaggtc cctgcagtgg    120
   aggggcgcct cgtctacctc cggatgcttt tgccacgact ccgtcttggc accagtcttg    180
   ctgccctgga atgcagtttc ccaaacgcag cgctcactct gactcctctc tgcttttatc    240
   cgtcaggtct cagttcttgc gggtgggagt cggctgaagg agctgcgtta tatggttgtg    300
   acatcaccct ccccggcccc cattttcagg tcagaacttt catgcctttg gaaacgaggg    360
   acccagaaga gcccttactg taggaggaat gtcgccgtta gacctctaag ctccgaagaa    420
   ccaaggggag acagaatcca gattgcttcc ttaaatccca agtagtattg ttttttccac     480
   cttagagtcc gcccccccgc ccccacttgt cccatatatt gatcggccac tttgccaccg    540
   tgttccaagg ttggagagcg tcacaatgcc ccaccgtccc caactctcaa gtggccgaca    600
   gtgggcagtc cagaagctgg gaccagactc atctgcccga gggcacagta ggagggacgg    660
   agggatctcc caagagctcc gagggcgagg tgagaaccaa ttgtggggag ggggacctta    720
   taacggggca gttaggaacg ggcagaggtg cccccacctc cagctttcaa ctcaggcgag    780
   gaaacttcga gaccttcccg tccacacctg attgacagaa ggaagctgtc gcggcccagg    840
   aggcgagtgt gggtgtgcag aggtggagac aaaacctaaa agcacgcaga gccggcgagg    900
   gcttgcggtc ctggacggta ggggtctgcg tctggtgcca ccttctccat caggctgctt    960
   gctgggtcca ccaagcctga cccctccagc caggagggga tcacggagtg tgtcccccgc   1020
   ccgggctgct gcctgccgga agggggctgg gaaaccggga tccccgcgcg ccacttgcct   1080
   ggcagcgtca gtttcaccca ggactggcta gcggttccct cgtggctctg cgcggaggtc   1140
   cgcctcctcc cttccctccc ccatcccgcg cgctgcctcc gcctccttct tctgcctcca   1200
   ccctcaaccc ccatccccgc ctgacgggag ctagccctca gtccgcccga gctgtggttg   1260
   tgggcgccgg acaagtccaa ggcgcctcct cccaatatgg acagccgcta caacagcact   1320
   gcgggcatcg gggacttgaa ccagctgagc gctgccatcc cggccacgcg ggtggaggtg   1380
   tccgtgtcct gcaggtgagg acgcagccct tcccctctct cctgccctct catctgtggg   1440
   gttggtagca cttggagagc gctcggggta ccacgaggct ttgcgcaggc aagaagttgc   1500
   gccatgagtg ggaaatatct cagtagtggg gctggagtta ccccgggtca ggtgacacag   1560
   gggagcggga attagaagcg gtgggctccg ggactgagac tttgtatttt ctttctggga   1620
   aacgctgtaa aagttggctg ggctgtgact cagctgtggg aacaccggcc gccgcccact   1680
   ttacaagccg gttttccgcc ccaaacccgg gctggatggt tgggtgatga ccggaatcga   1740
   aggggacagc actgccaaaa gctcttgtga ccgctgcacg cttaggcccg ggacttcctc   1800
   tgctaggcgt gtgcaaacct gctgttgcta gggttggctc caaccccggc agcttcctcc   1860
   ccacccactt cctggagcct tccagcttgc ttctccccga cccttccggg ctttttgcct   1920
   tttgcctgat aggtagagat ttcggccctc cttgcagacg aatctatgcc ttacctaaga   1980
   tctgggaagt cacgactttt gttttgattt gttttcgggc ccacatctga gtgcttgtaa   2040
   aaactgactc tcggccgggc gcagtggctc agtacagttt atacgcctgt attcccagca   2100
   ctttgggagg ccaaggcggg tggatcacga ggtcaggagt tcgagaccag cctggccaat   2160
   atggtgaaac tccgtctcta ctaaaaatgc aaaaattagc cggacgtggt ggcacgcgcc   2220
   tgtagtccca gctactcggg agactgagac aggagaatcg cttgaacccg ggatgtggat   2280
   gttgcaatga gctgagatct tgccattgca ctccagcctg ggcgacagcg caagactcca   2340
   tctcaaaaaa aaaaaaaaaa caaaaaaaaa ctgactttca accctgtcca gagactgtaa   2400
   attgcaaact cgttgtcctg acctttcggt tagttgccct gtaaataaag cagcatcacc   2460
   gctatatcat agatgatttt gagaaggaga tgagcgatgg tgctaaattg gtactggcca   2520
   ctaatttgcg aattaatatt gaccctggag cccactccct tgtttgcata attgggtctg   2580
   tctcactgga aaactggctc ctcatttatc catttttgcc ctaagattgt ttttatgccc   2640
   tcacatcctc cctttccttt ccttagcaaa cttgccttga agatatctaa caaatttggg   2700
   tgatcttgat gtaaataagt ttaaaattcc atcaatcttg actttgcatt ttcacaaact   2760
   gagcctaaag ccaaaggaat tcttttctgg gggtgaaaat tcgagcacca gcaaccgtag   2820
   tagaagtatt atttgagggt aggggacagc ctttatctag ccctgaaact atatcacctg   2880
   attgtttgcc gcctcttaag actgatctgg gactatggaa ataatcatgc tgataatgat   2940
   agctatcatt aagctcttgc ctcattgctg tcactgcttt aagtgctgcc tgagattttt   3000
   c                                                                   3001
<210> 622
   <211> 3001
   <212> DNA
   <213> human
<400> 622
   ttgtttttta gttgcgtcgt attttgtttt tcgtcgtcgt ttggtggcga gttttcggga      60
   ttcgtaggcg ttcgggtgag gtaggaggga aggcgcgggg aataaaggtt tttgtagtgg     120
   aggggcgttt cgtttatttt cggatgtttt tgttacgatt tcgttttggt attagttttg     180
   ttgttttgga atgtagtttt ttaaacgtag cgtttatttt gatttttttt tgtttttatt     240
   cgttaggttt tagtttttgc gggtgggagt cggttgaagg agttgcgtta tatggttgtg     300
   atattatttt tttcggtttt tatttttagg ttagaatttt tatgtttttg gaaacgaggg      360
   atttagaaga gtttttattg taggaggaat gtcgtcgtta gatttttaag tttcgaagaa     420
   ttaaggggag atagaattta gattgttttt ttaaatttta agtagtattg ttttttttat     480
   tttagagttc gttttttcgt ttttatttgt tttatatatt gatcggttat tttgttatcg      540
   tgttttaagg ttggagagcg ttataatgtt ttatcgtttt taatttttaa gtggtcgata     600
   gtgggtagtt tagaagttgg gattagattt atttgttcga gggtatagta ggagggacgg     660
   agggattttt taagagtttc gagggcgagg tgagaattaa ttgtggggag ggggatttta     720
   taacggggta gttaggaacg ggtagaggtg tttttatttt tagtttttaa tttaggcgag     780
   gaaatttcga gattttttcg tttatatttg attgatagaa ggaagttgtc gcggtttagg     840
   aggcgagtgt gggtgtgtag aggtggagat aaaatttaaa agtacgtaga gtcggcgagg     900
   gtttgcggtt ttggacggta ggggtttgcg tttggtgtta ttttttttat taggttgttt     960
   gttgggttta ttaagtttga tttttttagt taggagggga ttacggagtg tgtttttcgt   1020
   tcgggttgtt gtttgtcgga agggggttgg gaaatcggga ttttcgcgcg ttatttgttt    1080
   ggtagcgtta gttttattta ggattggtta gcggtttttt cgtggttttg cgcggaggtt    1140
   cgtttttttt tttttttttt ttatttcgcg cgttgttttc gttttttttt tttgttttta    1200
   tttttaattt ttattttcgt ttgacgggag ttagttttta gttcgttcga gttgtggttg    1260
   tgggcgtcgg ataagtttaa ggcgtttttt tttaatatgg atagtcgtta taatagtatt    1320
   gcgggtatcg gggatttgaa ttagttgagc gttgttattt cggttacgcg ggtggaggtg    1380
   ttcgtgtttt gtaggtgagg acgtagtttt tttttttttt tttgtttttt tatttgtggg    1440
   gttggtagta tttggagagc gttcggggta ttacgaggtt ttgcgtaggt aagaagttgc    1500
   gttatgagtg ggaaatattt tagtagtggg gttggagtta tttcgggtta ggtgatatag    1560
   gggagcggga attagaagcg gtgggtttcg ggattgagat tttgtatttt ttttttggga   1620
   aacgttgtaa aagttggttg ggttgtgatt tagttgtggg aatatcggtc gtcgtttatt    1680
   ttataagtcg gtttttcgtt ttaaattcgg gttggatggt tgggtgatga tcggaatcga    1740
   aggggatagt attgttaaaa gtttttgtga tcgttgtacg tttaggttcg ggattttttt    1800
   tgttaggcgt gtgtaaattt gttgttgtta gggttggttt taatttcggt agtttttttt    1860
   ttatttattt tttggagttt tttagtttgt tttttttcga ttttttcggg ttttttgttt  1920
   tttgtttgat aggtagagat ttcggttttt tttgtagacg aatttatgtt ttatttaaga    1980
   tttgggaagt tacgattttt gttttgattt gttttcgggt ttatatttga gtgtttgtaa    2040
   aaattgattt tcggtcgggc gtagtggttt agtatagttt atacgtttgt atttttagta    2100
   ttttgggagg ttaaggcggg tggattacga ggttaggagt tcgagattag tttggttaat    2160
   atggtgaaat ttcgttttta ttaaaaatgt aaaaattagt cggacgtggt ggtacgcgtt    2220
   tgtagtttta gttattcggg agattgagat aggagaatcg tttgaattcg ggatgtggat    2280
   gttgtaatga gttgagattt tgttattgta ttttagtttg ggcgatagcg taagatttta    2340
   ttttaaaaaa aaaaaaaaaa taaaaaaaaa ttgattttta attttgttta gagattgtaa    2400
   attgtaaatt cgttgttttg atttttcggt tagttgtttt gtaaataaag tagtattatc    2460
   gttatattat agatgatttt gagaaggaga tgagcgatgg tgttaaattg gtattggtta    2520
   ttaatttgcg aattaatatt gattttggag tttatttttt tgtttgtata attgggtttg    2580
   ttttattgga aaattggttt tttatttatt tatttttgtt ttaagattgt ttttatgttt    2640
   ttatattttt tttttttttt ttttagtaaa tttgttttga agatatttaa taaatttggg    2700
   tgattttgat gtaaataagt ttaaaatttt attaattttg attttgtatt tttataaatt    2760
   gagtttaaag ttaaaggaat ttttttttgg gggtgaaaat tcgagtatta gtaatcgtag    2820
   tagaagtatt atttgagggt aggggatagt ttttatttag ttttgaaatt atattatttg    2880
   attgtttgtc gttttttaag attgatttgg gattatggaa ataattatgt tgataatgat   2940
   agttattatt aagtttttgt tttattgttg ttattgtttt aagtgttgtt tgagattttt    3000
   t                                                                    3001
<210> 623
   <211> 3001
   <212> DNA
   <213> human
<400> 623
   ttgtttttta gttgtgttgt attttgtttt ttgttgttgt ttggtggtga gtttttggga      60
   tttgtaggtg tttgggtgag gtaggaggga aggtgtgggg aataaaggtt tttgtagtgg     120
   aggggtgttt tgtttatttt tggatgtttt tgttatgatt ttgttttggt attagttttg     180
   ttgttttgga atgtagtttt ttaaatgtag tgtttatttt gatttttttt tgtttttatt     240
   tgttaggttt tagtttttgt gggtgggagt tggttgaagg agttgtgtta tatggttgtg     300
   atattatttt ttttggtttt tatttttagg ttagaatttt tatgtttttg gaaatgaggg     360
   atttagaaga gtttttattg taggaggaat gttgttgtta gatttttaag ttttgaagaa     420
   ttaaggggag atagaattta gattgttttt ttaaatttta agtagtattg ttttttttat     480
   tttagagttt gtttttttgt ttttatttgt tttatatatt gattggttat tttgttattg     540
   tgttttaagg ttggagagtg ttataatgtt ttattgtttt taatttttaa gtggttgata     600
   gtgggtagtt tagaagttgg gattagattt atttgtttga gggtatagta ggagggatgg     660
   agggattttt taagagtttt gagggtgagg tgagaattaa ttgtggggag ggggatttta     720
   taatggggta gttaggaatg ggtagaggtg tttttatttt tagtttttaa tttaggtgag     780
   gaaattttga gatttttttg tttatatttg attgatagaa ggaagttgtt gtggtttagg     840
   aggtgagtgt gggtgtgtag aggtggagat aaaatttaaa agtatgtaga gttggtgagg     900
   gtttgtggtt ttggatggta ggggtttgtg tttggtgtta ttttttttat taggttgttt     960
   gttgggttta ttaagtttga tttttttagt taggagggga ttatggagtg tgttttttgt   1020
   ttgggttgtt gtttgttgga agggggttgg gaaattggga tttttgtgtg ttatttgttt   1080
   ggtagtgtta gttttattta ggattggtta gtggtttttt tgtggttttg tgtggaggtt   1140
   tgtttttttt tttttttttt ttattttgtg tgttgttttt gttttttttt tttgttttta   1200
   tttttaattt ttatttttgt ttgatgggag ttagttttta gtttgtttga gttgtggttg   1260
   tgggtgttgg ataagtttaa ggtgtttttt tttaatatgg atagttgtta taatagtatt   1320
   gtgggtattg gggatttgaa ttagttgagt gttgttattt tggttatgtg ggtggaggtg   1380
   tttgtgtttt gtaggtgagg atgtagtttt tttttttttt tttgtttttt tatttgtggg   1440
   gttggtagta tttggagagt gtttggggta ttatgaggtt ttgtgtaggt aagaagttgt   1500
   gttatgagtg ggaaatattt tagtagtggg gttggagtta ttttgggtta ggtgatatag   1560
   gggagtggga attagaagtg gtgggttttg ggattgagat tttgtatttt ttttttggga   1620
   aatgttgtaa aagttggttg ggttgtgatt tagttgtggg aatattggtt gttgtttatt   1680
   ttataagttg gttttttgtt ttaaatttgg gttggatggt tgggtgatga ttggaattga   1740
   aggggatagt attgttaaaa gtttttgtga ttgttgtatg tttaggtttg ggattttttt   1800
   tgttaggtgt gtgtaaattt gttgttgtta gggttggttt taattttggt agtttttttt   1860
   ttatttattt tttggagttt tttagtttgt ttttttttga tttttttggg ttttttgttt   1920
   tttgtttgat aggtagagat tttggttttt tttgtagatg aatttatgtt ttatttaaga   1980
   tttgggaagt tatgattttt gttttgattt gtttttgggt ttatatttga gtgtttgtaa   2040
   aaattgattt ttggttgggt gtagtggttt agtatagttt atatgtttgt atttttagta   2100
   ttttgggagg ttaaggtggg tggattatga ggttaggagt ttgagattag tttggttaat   2160
   atggtgaaat tttgttttta ttaaaaatgt aaaaattagt tggatgtggt ggtatgtgtt   2220
   tgtagtttta gttatttggg agattgagat aggagaattg tttgaatttg ggatgtggat   2280
   gttgtaatga gttgagattt tgttattgta ttttagtttg ggtgatagtg taagatttta   2340
   ttttaaaaaa aaaaaaaaaa taaaaaaaaa ttgattttta attttgttta gagattgtaa   2400
   attgtaaatt tgttgttttg attttttggt tagttgtttt gtaaataaag tagtattatt   2460
   gttatattat agatgatttt gagaaggaga tgagtgatgg tgttaaattg gtattggtta   2520
   ttaatttgtg aattaatatt gattttggag tttatttttt tgtttgtata attgggtttg   2580
   ttttattgga aaattggttt tttatttatt tatttttgtt ttaagattgt ttttatgttt   2640
   ttatattttt tttttttttt ttttagtaaa tttgttttga agatatttaa taaatttggg   2700
   tgattttgat gtaaataagt ttaaaatttt attaattttg attttgtatt tttataaatt   2760
   gagtttaaag ttaaaggaat ttttttttgg gggtgaaaat ttgagtatta gtaattgtag   2820
   tagaagtatt atttgagggt aggggatagt ttttatttag ttttgaaatt atattatttg   2880
   attgtttgtt gttttttaag attgatttgg gattatggaa ataattatgt tgataatgat   2940
   agttattatt aagtttttgt tttattgttg ttattgtttt aagtgttgtt tgagattttt   3000
   t                                                                     3001
<210> 624
   <211> 3001
   <212> DNA
   <213> human
<400> 624
   gaaaaatttt aggtagtatt taaagtagtg atagtaatga ggtaagagtt taatgatagt 60
   tattattatt agtatgatta tttttatagt tttagattag ttttaagagg cggtaaataa 120
   ttaggtgata tagttttagg gttagataaa ggttgttttt tatttttaaa taatattttt 180
   attacggttg ttggtgttcg aatttttatt tttagaaaag aatttttttg gttttaggtt 240
   tagtttgtga aaatgtaaag ttaagattga tggaatttta aatttattta tattaagatt 300
   atttaaattt gttagatatt tttaaggtaa gtttgttaag gaaaggaaag ggaggatgtg 360
   agggtataaa aataatttta gggtaaaaat ggataaatga ggagttagtt ttttagtgag 420
   atagatttaa ttatgtaaat aagggagtgg gttttagggt taatattaat tcgtaaatta 480
   gtggttagta ttaatttagt attatcgttt attttttttt taaaattatt tatgatatag 540
   cggtgatgtt gttttattta tagggtaatt aatcgaaagg ttaggataac gagtttgtaa 600
   tttatagttt ttggataggg ttgaaagtta gttttttttt gttttttttt tttttttgag 660
   atggagtttt gcgttgtcgt ttaggttgga gtgtaatggt aagattttag tttattgtaa 720
   tatttatatt tcgggtttaa gcgatttttt tgttttagtt tttcgagtag ttgggattat 780
   aggcgcgtgt tattacgttc ggttaatttt tgtattttta gtagagacgg agttttatta 840
   tattggttag gttggtttcg aatttttgat ttcgtgattt attcgttttg gttttttaaa 900
   gtgttgggaa tataggcgta taaattgtat tgagttattg cgttcggtcg agagttagtt 960
   tttataagta tttagatgtg ggttcgaaaa taaattaaaa taaaagtcgt gattttttag 1020
   attttaggta aggtatagat tcgtttgtaa ggagggtcga aatttttatt tattaggtaa 1080
   aaggtaaaaa gttcggaagg gtcggggaga agtaagttgg aaggttttag gaagtgggtg 1140
   gggaggaagt tgtcggggtt ggagttaatt ttagtaatag taggtttgta tacgtttagt 1200
   agaggaagtt tcgggtttaa gcgtgtagcg gttataagag tttttggtag tgttgttttt 1260
   ttcgatttcg gttattattt aattatttag ttcgggtttg gggcggaaaa tcggtttgta 1320
   aagtgggcgg cggtcggtgt ttttatagtt gagttatagt ttagttaatt tttatagcgt 1380
   tttttagaaa gaaaatataa agttttagtt tcggagttta tcgtttttaa ttttcgtttt 1440
   tttgtgttat ttgattcggg gtaattttag ttttattatt gagatatttt ttatttatgg 1500
   cgtaattttt tgtttgcgta aagtttcgtg gtatttcgag cgttttttaa gtgttattaa 1560
   ttttatagat gagagggtag gagagagggg aagggttgcg tttttatttg taggatacgg 1620
   atatttttat tcgcgtggtc gggatggtag cgtttagttg gtttaagttt tcgatgttcg 1680
   tagtgttgtt gtagcggttg tttatattgg gaggaggcgt tttggatttg ttcggcgttt 1740
   ataattatag ttcgggcgga ttgagggtta gttttcgtta ggcggggatg ggggttgagg 1800
   gtggaggtag aagaaggagg cggaggtagc gcgcgggatg ggggagggaa gggaggaggc 1860
   ggattttcgc gtagagttac gagggaatcg ttagttagtt ttgggtgaaa ttgacgttgt 1920
   taggtaagtg gcgcgcgggg atttcggttt tttagttttt tttcggtagg tagtagttcg 1980
   ggcgggggat atatttcgtg attttttttt ggttggaggg gttaggtttg gtggatttag 2040
   taagtagttt gatggagaag gtggtattag acgtagattt ttatcgttta ggatcgtaag 2100
   ttttcgtcgg ttttgcgtgt ttttaggttt tgtttttatt tttgtatatt tatattcgtt 2160
   ttttgggtcg cgatagtttt tttttgttaa ttaggtgtgg acgggaaggt ttcgaagttt 2220
   tttcgtttga gttgaaagtt ggaggtgggg gtatttttgt tcgtttttaa ttgtttcgtt 2280
   ataaggtttt tttttttata attggttttt atttcgtttt cggagttttt gggagatttt 2340
   ttcgtttttt ttattgtgtt ttcgggtaga tgagtttggt tttagttttt ggattgttta 2400
   ttgtcggtta tttgagagtt ggggacggtg gggtattgtg acgtttttta attttggaat 2460
   acggtggtaa agtggtcgat taatatatgg gataagtggg ggcggggggg cggattttaa 2520
   ggtggaaaaa ataatattat ttgggattta aggaagtaat ttggattttg tttttttttg 2580
   gtttttcgga gtttagaggt ttaacggcga tatttttttt atagtaaggg tttttttggg 2640
   tttttcgttt ttaaaggtat gaaagttttg atttgaaaat gggggtcggg gagggtgatg 2700
   ttataattat ataacgtagt ttttttagtc gatttttatt cgtaagaatt gagatttgac 2760
   ggataaaagt agagaggagt tagagtgagc gttgcgtttg ggaaattgta ttttagggta 2820
   gtaagattgg tgttaagacg gagtcgtggt aaaagtattc ggaggtagac gaggcgtttt 2880
   tttattgtag ggatttttgt ttttcgcgtt tttttttttg ttttattcga gcgtttgcgg 2940
   gtttcgggag ttcgttatta ggcggcgacg gagaatagga tgcggcgtag ttggaaggta 3000
   g 3001
<210> 625
   <211> 3001
   <212> DNA
   <213> human
<400> 625
   gaaaaatttt aggtagtatt taaagtagtg atagtaatga ggtaagagtt taatgatagt 60
   tattattatt agtatgatta tttttatagt tttagattag ttttaagagg tggtaaataa 120
   ttaggtgata tagttttagg gttagataaa ggttgttttt tatttttaaa taatattttt 180
   attatggttg ttggtgtttg aatttttatt tttagaaaag aatttttttg gttttaggtt 240
   tagtttgtga aaatgtaaag ttaagattga tggaatttta aatttattta tattaagatt 300
   atttaaattt gttagatatt tttaaggtaa gtttgttaag gaaaggaaag ggaggatgtg 360
   agggtataaa aataatttta gggtaaaaat ggataaatga ggagttagtt ttttagtgag 420
   atagatttaa ttatgtaaat aagggagtgg gttttagggt taatattaat ttgtaaatta 480
   gtggttagta ttaatttagt attattgttt attttttttt taaaattatt tatgatatag 540
   tggtgatgtt gttttattta tagggtaatt aattgaaagg ttaggataat gagtttgtaa 600
   tttatagttt ttggataggg ttgaaagtta gttttttttt gttttttttt tttttttgag 660
   atggagtttt gtgttgttgt ttaggttgga gtgtaatggt aagattttag tttattgtaa 720
   tatttatatt ttgggtttaa gtgatttttt tgttttagtt ttttgagtag ttgggattat 780
   aggtgtgtgt tattatgttt ggttaatttt tgtattttta gtagagatgg agttttatta 840
   tattggttag gttggttttg aatttttgat tttgtgattt atttgttttg gttttttaaa 900
   gtgttgggaa tataggtgta taaattgtat tgagttattg tgtttggttg agagttagtt 960
   tttataagta tttagatgtg ggtttgaaaa taaattaaaa taaaagttgt gattttttag 1020
   attttaggta aggtatagat ttgtttgtaa ggagggttga aatttttatt tattaggtaa 1080
   aaggtaaaaa gtttggaagg gttggggaga agtaagttgg aaggttttag gaagtgggtg 1140
   gggaggaagt tgttggggtt ggagttaatt ttagtaatag taggtttgta tatgtttagt 1200
   agaggaagtt ttgggtttaa gtgtgtagtg gttataagag tttttggtag tgttgttttt 1260
   tttgattttg gttattattt aattatttag tttgggtttg gggtggaaaa ttggtttgta 1320
   aagtgggtgg tggttggtgt ttttatagtt gagttatagt ttagttaatt tttatagtgt 1380
   tttttagaaa gaaaatataa agttttagtt ttggagttta ttgtttttaa tttttgtttt 1440
   tttgtgttat ttgatttggg gtaattttag ttttattatt gagatatttt ttatttatgg 1500
   tgtaattttt tgtttgtgta aagttttgtg gtattttgag tgttttttaa gtgttattaa 1560
   ttttatagat gagagggtag gagagagggg aagggttgtg tttttatttg taggatatgg 1620
   atatttttat ttgtgtggtt gggatggtag tgtttagttg gtttaagttt ttgatgtttg 1680
   tagtgttgtt gtagtggttg tttatattgg gaggaggtgt tttggatttg tttggtgttt 1740
   ataattatag tttgggtgga ttgagggtta gtttttgtta ggtggggatg gggttgaggg 1800
   gtggaggtag aagaaggagg tggaggtagt gtgtgggatg ggggagggaa gggaggaggt 1860
   ggatttttgt gtagagttat gagggaattg ttagttagtt ttgggtgaaa ttgatgttgt 1920
   taggtaagtg gtgtgtgggg attttggttt tttagttttt ttttggtagg tagtagtttg 1980
   ggtgggggat atattttgtg attttttttt ggttggaggg gttaggtttg gtggatttag 2040
   taagtagttt gatggagaag gtggtattag atgtagattt ttattgttta ggattgtaag 2100
   tttttgttgg ttttgtgtgt ttttaggttt tgtttttatt tttgtatatt tatatttgtt 2160
   ttttgggttg tgatagtttt tttttgttaa ttaggtgtgg atgggaaggt tttgaagttt 2220
   ttttgtttga gttgaaagtt ggaggtgggg gtatttttgt ttgtttttaa ttgttttgtt 2280
   ataaggtttt tttttttata attggttttt attttgtttt tggagttttt gggagatttt 2340
   tttgtttttt ttattgtgtt tttgggtaga tgagtttggt tttagttttt ggattgttta 2400
   ttgttggtta tttgagagtt ggggatggtg gggtattgtg atgtttttta attttggaat 2460
   atggtggtaa agtggttgat taatatatgg gataagtggg ggtggggggg tggattttaa 2520
   ggtggaaaaa ataatattat ttgggattta aggaagtaat ttggattttg tttttttttg 2580
   gtttttggga gtttagaggt ttaatggtga tatttttttt atagtaaggg tttttttggg 2640
   tttttgtttt ttaaaggtat gaaagttttg atttgaaaat gggggttggg gagggtgatg 2700
   ttataattat ataatgtagt ttttttagtt gatttttatt tgtaagaatt gagatttgat 2760
   ggataaaagt agagaggagt tagagtgagt gttgtgtttg ggaaattgta ttttagggta 2820
   gtaagattgg tgttaagatg gagttgtggt aaaagtattt ggaggtagat gaggtgtttt 2880
   tttattgtag ggatttttgt tttttgtgtt tttttttttg ttttatttga gtgtttgtgg 2940
   gttttgggag tttgttatta ggtggtgatg gagaatagga tgtggtgtag ttggaaggta 3000
   g 3001
<210> 626
   <211> 50
   <212> DNA
   <213> human
<400> 626
   cgcaaggggc agcggttctc ccaacccagt ctaatgcaca ttggcccagg 50
<210> 627
   <211> 50
   <212> DNA
   <213> human
<400> 627
   cgtaaggggt agcggttttt ttaatttagt ttaatgtata ttggtttagg 50
<210> 628
   <211> 50
   <212> DNA
   <213> human
<400> 628
   tgtaaggggt agtggttttt ttaatttagt ttaatgtata ttggtttagg 50
<210> 629
   <211> 50
   <212> DNA
   <213> human
<400> 629
   tttgggttaa tgtgtattag attgggttgg gagaatcgtt gttttttgcg 50
<210> 630
   <211> 50
   <212> DNA
   <213> human
<400> 630
   tttgggttaa tgtgtattag attgggttgg gagaattgtt gttttttgtg 50
<210> 631
   <211> 3001
   <212> DNA
   <213> human
<400> 631
   ggcgggcgcc gaggccagca ggtcggctgc catctgccgg cgctttgtct tgtagcgacg     60
   gttctggaac cagattttca cctgcgtctc ggtgagcttc agcgacgcgg ccaggtctgc    120
   gcgctcgggc ccggacaggt agcgctggtg gttaaagcgg cgctccagct cgaagacctg    180
   cgcgtgggag aaagcggccc gcgagcgctt cttgcgtggc ttgggcgccg ccggctcctc    240
   ctcctcctcc gcgacgcctg ccggcccgct gccgcccccg ccgccggccc cgctgcacag    300
   cgcggacacg tgtgcacctc tggggccaac accgtcgtcc tcggtccttg ggctgcggtc    360
   gcctgcggac cccggtggga acagaaacaa gagactgtca gcgccacaga cgaggtgagg    420
   ccgggcctca actgcagggg tcacgggagt ggggcggaaa tacactttga tcccactcaa    480
   gcggagcgga ggtctgggag gccctgggcc cgggagacca gtcttagact cttgccccac    540
   tgggtatccc atctaggcct cttctgggga gggcggcaga ctcagccgct gtgtcaacgc    600
   tgtgttgtcg agaccagctc cccaccctct ctgggcccca ggctcccctc agtaacttgg    660
   ggcactcgac ccgagcatcc gcgaaagccc tcccggctct cagcgttgag cattgggatt    720
   ctagactgca tttccgtctc tctgcttggg ttcacgcgcc tctccacact tagttcacac    780
   gcacacacgc gcgcgtcctc gcagcacaca cttgtctggt gcaggtaagg gaaggtggag    840
   gcggatcctg gggccaaagg tatttagaat ctttcaccct cagccgcctg ggattgctgt    900
   gagagacatg gaaacaggct gagccgaggc cttagatgag aggatggact ggagagtaaa    960
   gagggagggt tgcccctgca tcgagttttt ggaccctgat cccacaccag cttctcggtc   1020
   tcgtacccgc ccttccgaag aactccagca gaaaggtcca gcggtcccct gtgcttgagg   1080
   cctacagaag cttgtaccca actagggcag gcacccgggt cttccagacc acaggacagg   1140
   acaggccacg gctgaggagg cctctctcct gcctccagga tgaactaaag acccaatccg   1200
   ggatcttcgg cctagggctg ctctcccaga cctggggtct gagaaagcca aaccagccct   1260
   ttccccaaag ctctagttct gcagattctc agctctggcc cactcggagg tgttcttcac   1320
   cacctatcca cctactgtgg ggcccggccc tgggaccttg aactggcagg tctctggtcc   1380
   agagctaggt cactggctac ctgaggtctc tgaacccctc acttttccgc ttccctgatt   1440
   ttggggattt ggggacagac acggcagaaa gcactggcga cgaactcaaa aactcccgaa   1500
   cgcaaggggc agcggttctc ccaacccagt ctaatgcaca ttggcccagg atgtctcagg   1560
   cctcacccca ggacgtaggg ctctgaggag ctactccggt ctctcgcggg ctcagttccc   1620
   gaagtgatag agcagctcgc gccagagcgc agaacttcgg gatttggcca gcctccgagc   1680
   cccagggcgc agggtgctca agccgaccac cccactcggc gtggttgccc tccgcgtcca   1740
   tcccctcagc ccggccccca tccccgcgaa gccgcagcag acctgagacg ctggcggaca   1800
   tctcgctgtc gctccggccc gcggcttcct cctctaggtc tttggaagcg gccagctcac   1860
   agaccggctg gccgaggctc aaggatcccc ccgcaaggcc ggccccgctg gccccccgcg    1920
   cgtccgcgca gcgccgcctg ctctcgttct cctcgctgag cgcggagtcc gagtcccagc   1980
   cttccgggct ctccgcagtc cgccccgcag ctgttctggt accggcagga gacgccagca   2040
   gagagtcctc ggcgcccccc aacgcgcccg cgtccctctc cccaaagagc cgccaacagc   2100
   agacagcggg agccgcggcc accgatgccg ctgtgccccc gggcgccggg cgcccctctg   2160
   gcgcggccag cccgccgcgc tcctctttct tgttgaggat cgcctggatg gagaaggacg   2220
   tcaaggtgtt ggcgccgcgc acagccatct gcgccgcggg caggagcggc cggcggggcg   2280
   ggcagctggg gcgccgagca gctccgagcg ggacagagag cgccggcggc cgcagcgcga   2340
   gtgagctggg tgtgcgaggc cgccgccgcc cactgctgcg cggcccagca gctcccgccc   2400
   cactccgtcc caggatcagc gccgaccctc gcccccacct tagaggccca ccccgcccgg   2460
   agaccccctc cccccgaatc cagagccaga cgctctcctt tcgcagctca gctggattat  2520
   ctcatcgctt ctcgccctta ggggcgggct ggggtctgcc ccctcggggg acgtgaagga   2580
   ggattggcgg gggcccctcc gtggcagcag tcccctcccg agcgccgccg gggcgcacag   2640
   cccgagtcac tttttctttg cgcgtctgtc ccttcctcgc ctgcaggatt tcgctcctgg   2700
   cccgtcctcc ccttccctgc tgggcaggct cttccggagc ccacccccgg cggcggcggc   2760
   ggtagccgcg gccgcggccg ctggattccg agaggcgcgg ggaacttttt taccccctgc   2820
   cctggtttta tctgtttctt ttctctttcc ttcccttgcg ttccactgta ccacccccct   2880
   cccgcccggg tgtcattcct cactgctgag tgacagcctg aggccacacc cctccactgg   2940
   gctgtcccgg gcgctcccgg ccgtccattg gctggctcca gttgggcgac gttgacttta   3000
   a                                                                   3001
<210> 632
   <211> 3001
   <212> DNA
   <213> human
<400> 632
   ggcgggcgtc gaggttagta ggtcggttgt tatttgtcgg cgttttgttt tgtagcgacg      60
   gttttggaat tagattttta tttgcgtttc ggtgagtttt agcgacgcgg ttaggtttgc     120
   gcgttcgggt tcggataggt agcgttggtg gttaaagcgg cgttttagtt cgaagatttg     180
   cgcgtgggag aaagcggttc gcgagcgttt tttgcgtggt ttgggcgtcg tcggtttttt     240
   tttttttttc gcgacgtttg tcggttcgtt gtcgttttcg tcgtcggttt cgttgtatag      300
   cgcggatacg tgtgtatttt tggggttaat atcgtcgttt tcggtttttg ggttgcggtc      360
   gtttgcggat ttcggtggga atagaaataa gagattgtta gcgttataga cgaggtgagg     420
   tcgggtttta attgtagggg ttacgggagt ggggcggaaa tatattttga ttttatttaa     480
   gcggagcgga ggtttgggag gttttgggtt cgggagatta gttttagatt tttgttttat     540
   tgggtatttt atttaggttt tttttgggga gggcggtaga tttagtcgtt gtgttaacgt     600
   tgtgttgtcg agattagttt tttatttttt ttgggtttta ggtttttttt agtaatttgg    660
   ggtattcgat tcgagtattc gcgaaagttt tttcggtttt tagcgttgag tattgggatt    720
   ttagattgta ttttcgtttt tttgtttggg tttacgcgtt tttttatatt tagtttatac    780
   gtatatacgc gcgcgttttc gtagtatata tttgtttggt gtaggtaagg gaaggtggag    840
   gcggattttg gggttaaagg tatttagaat tttttatttt tagtcgtttg ggattgttgt    900
   gagagatatg gaaataggtt gagtcgaggt tttagatgag aggatggatt ggagagtaaa    960
   gagggagggt tgtttttgta tcgagttttt ggattttgat tttatattag tttttcggtt   1020
   tcgtattcgt tttttcgaag aattttagta gaaaggttta gcggtttttt gtgtttgagg   1080
   tttatagaag tttgtattta attagggtag gtattcgggt tttttagatt ataggatagg   1140
   ataggttacg gttgaggagg tttttttttt gtttttagga tgaattaaag atttaattcg    1200
   ggattttcgg tttagggttg tttttttaga tttggggttt gagaaagtta aattagtttt   1260
   ttttttaaag ttttagtttt gtagattttt agttttggtt tattcggagg tgttttttat   1320
   tatttattta tttattgtgg ggttcggttt tgggattttg aattggtagg tttttggttt   1380
   agagttaggt tattggttat ttgaggtttt tgaatttttt attttttcgt ttttttgatt    1440
   ttggggattt ggggatagat acggtagaaa gtattggcga cgaatttaaa aattttcgaa   1500
   cgtaaggggt agcggttttt ttaatttagt ttaatgtata ttggtttagg atgttttagg   1560
   ttttatttta ggacgtaggg ttttgaggag ttatttcggt ttttcgcggg tttagttttc   1620
   gaagtgatag agtagttcgc gttagagcgt agaatttcgg gatttggtta gttttcgagt   1680
   tttagggcgt agggtgttta agtcgattat tttattcggc gtggttgttt ttcgcgttta   1740
   tttttttagt tcggttttta ttttcgcgaa gtcgtagtag atttgagacg ttggcggata   1800
   tttcgttgtc gtttcggttc gcggtttttt tttttaggtt tttggaagcg gttagtttat   1860
   agatcggttg gtcgaggttt aaggattttt tcgtaaggtc ggtttcgttg gtttttcgcg   1920
   cgttcgcgta gcgtcgtttg ttttcgtttt tttcgttgag cgcggagttc gagttttagt   1980
   ttttcgggtt tttcgtagtt cgtttcgtag ttgttttggt atcggtagga gacgttagta   2040
   gagagttttc ggcgtttttt aacgcgttcg cgtttttttt tttaaagagt cgttaatagt   2100
   agatagcggg agtcgcggtt atcgatgtcg ttgtgttttc gggcgtcggg cgtttttttg   2160
   gcgcggttag ttcgtcgcgt tttttttttt tgttgaggat cgtttggatg gagaaggacg   2220
   ttaaggtgtt ggcgtcgcgt atagttattt gcgtcgcggg taggagcggt cggcggggcg   2280
   ggtagttggg gcgtcgagta gtttcgagcg ggatagagag cgtcggcggt cgtagcgcga   2340
   gtgagttggg tgtgcgaggt cgtcgtcgtt tattgttgcg cggtttagta gttttcgttt   2400
   tatttcgttt taggattagc gtcgattttc gtttttattt tagaggttta tttcgttcgg   2460
   agattttttt ttttcgaatt tagagttaga cgtttttttt tcgtagttta gttggattat   2520
   tttatcgttt ttcgttttta ggggcgggtt ggggtttgtt ttttcggggg acgtgaagga   2580
   ggattggcgg gggttttttc gtggtagtag ttttttttcg agcgtcgtcg gggcgtatag      2640
   ttcgagttat tttttttttg cgcgtttgtt tttttttcgt ttgtaggatt tcgtttttgg      2700
   ttcgtttttt ttttttttgt tgggtaggtt ttttcggagt ttattttcgg cggcggcggc     2760
   ggtagtcgcg gtcgcggtcg ttggatttcg agaggcgcgg ggaatttttt tattttttgt     2820
   tttggtttta tttgtttttt tttttttttt tttttttgcg ttttattgta ttattttttt   2880
   ttcgttcggg tgttattttt tattgttgag tgatagtttg aggttatatt tttttattgg   2940
   gttgtttcgg gcgttttcgg tcgtttattg gttggtttta gttgggcgac gttgatttta   3000
   a                                                                   3001

<210> 633
   <211> 3001
   <212> DNA
   <213> human
<400> 633
   ggtgggtgtt gaggttagta ggttggttgt tatttgttgg tgttttgttt tgtagtgatg      60
   gttttggaat tagattttta tttgtgtttt ggtgagtttt agtgatgtgg ttaggtttgt     120
   gtgtttgggt ttggataggt agtgttggtg gttaaagtgg tgttttagtt tgaagatttg     180
   tgtgtgggag aaagtggttt gtgagtgttt tttgtgtggt ttgggtgttg ttggtttttt     240
   tttttttttt gtgatgtttg ttggtttgtt gttgtttttg ttgttggttt tgttgtatag     300
   tgtggatatg tgtgtatttt tggggttaat attgttgttt ttggtttttg ggttgtggtt     360
   gtttgtggat tttggtggga atagaaataa gagattgtta gtgttataga tgaggtgagg     420
   ttgggtttta attgtagggg ttatgggagt ggggtggaaa tatattttga ttttatttaa     480
   gtggagtgga ggtttgggag gttttgggtt tgggagatta gttttagatt tttgttttat     540
   tgggtatttt atttaggttt tttttgggga gggtggtaga tttagttgtt gtgttaatgt     600
   tgtgttgttg agattagttt tttatttttt ttgggtttta ggtttttttt agtaatttgg     660
   ggtatttgat ttgagtattt gtgaaagttt ttttggtttt tagtgttgag tattgggatt     720
   ttagattgta tttttgtttt tttgtttggg tttatgtgtt tttttatatt tagtttatat     780
   gtatatatgt gtgtgttttt gtagtatata tttgtttggt gtaggtaagg gaaggtggag     840
   gtggattttg gggttaaagg tatttagaat tttttatttt tagttgtttg ggattgttgt     900
   gagagatatg gaaataggtt gagttgaggt tttagatgag aggatggatt ggagagtaaa     960
   gagggagggt tgtttttgta ttgagttttt ggattttgat tttatattag ttttttggtt    1020
   ttgtatttgt ttttttgaag aattttagta gaaaggttta gtggtttttt gtgtttgagg    1080
   tttatagaag tttgtattta attagggtag gtatttgggt tttttagatt ataggatagg    1140
   ataggttatg gttgaggagg tttttttttt gtttttagga tgaattaaag atttaatttg    1200
   ggatttttgg tttagggttg tttttttaga tttggggttt gagaaagtta aattagtttt    1260
   ttttttaaag ttttagtttt gtagattttt agttttggtt tatttggagg tgttttttat   1320
   tatttattta tttattgtgg ggtttggttt tgggattttg aattggtagg tttttggttt    1380
   agagttaggt tattggttat ttgaggtttt tgaatttttt atttttttgt ttttttgatt  1440
   ttggggattt ggggatagat atggtagaaa gtattggtga tgaatttaaa aatttttgaa    1500
   tgtaaggggt agtggttttt ttaatttagt ttaatgtata ttggtttagg atgttttagg    1560
   ttttatttta ggatgtaggg ttttgaggag ttattttggt tttttgtggg tttagttttt    1620
   gaagtgatag agtagtttgt gttagagtgt agaattttgg gatttggtta gtttttgagt    1680
   tttagggtgt agggtgttta agttgattat tttatttggt gtggttgttt tttgtgttta    1740
   tttttttagt ttggttttta tttttgtgaa gttgtagtag atttgagatg ttggtggata   1800
   ttttgttgtt gttttggttt gtggtttttt tttttaggtt tttggaagtg gttagtttat    1860
   agattggttg gttgaggttt aaggattttt ttgtaaggtt ggttttgttg gttttttgtg   1920
   tgtttgtgta gtgttgtttg tttttgtttt ttttgttgag tgtggagttt gagttttagt    1980
   tttttgggtt ttttgtagtt tgttttgtag ttgttttggt attggtagga gatgttagta    2040
   gagagttttt ggtgtttttt aatgtgtttg tgtttttttt tttaaagagt tgttaatagt    2100
   agatagtggg agttgtggtt attgatgttg ttgtgttttt gggtgttggg tgtttttttg    2160
   gtgtggttag tttgttgtgt tttttttttt tgttgaggat tgtttggatg gagaaggatg    2220
   ttaaggtgtt ggtgttgtgt atagttattt gtgttgtggg taggagtggt tggtggggtg    2280
   ggtagttggg gtgttgagta gttttgagtg ggatagagag tgttggtggt tgtagtgtga    2340
   gtgagttggg tgtgtgaggt tgttgttgtt tattgttgtg tggtttagta gtttttgttt    2400
   tattttgttt taggattagt gttgattttt gtttttattt tagaggttta ttttgtttgg    2460
   agattttttt tttttgaatt tagagttaga tgtttttttt ttgtagttta gttggattat    2520
   tttattgttt tttgttttta ggggtgggtt ggggtttgtt tttttggggg atgtgaagga    2580
   ggattggtgg gggttttttt gtggtagtag tttttttttg agtgttgttg gggtgtatag    2640
   tttgagttat tttttttttg tgtgtttgtt ttttttttgt ttgtaggatt ttgtttttgg    2700
   tttgtttttt ttttttttgt tgggtaggtt tttttggagt ttatttttgg tggtggtggt    2760
   ggtagttgtg gttgtggttg ttggattttg agaggtgtgg ggaatttttt tattttttgt   2820
   tttggtttta tttgtttttt tttttttttt tttttttgtg ttttattgta ttattttttt   2880
   tttgtttggg tgttattttt tattgttgag tgatagtttg aggttatatt tttttattgg    2940
   gttgttttgg gtgtttttgg ttgtttattg gttggtttta gttgggtgat gttgatttta    3000
   a                                                                    3001
<210> 634
   <211> 3001
   <212> DNA
   <213> human
<400> 634
   ttaaagttaa cgtcgtttaa ttggagttag ttaatggacg gtcgggagcg ttcgggatag 60
   tttagtggag gggtgtggtt ttaggttgtt atttagtagt gaggaatgat attcgggcgg 120
   gaggggggtg gtatagtgga acgtaaggga aggaaagaga aaagaaatag ataaaattag 180
   ggtagggggt aaaaaagttt ttcgcgtttt tcggaattta gcggtcgcgg tcgcggttat 240
   cgtcgtcgtc gtcgggggtg ggtttcggaa gagtttgttt agtagggaag gggaggacgg 300
   gttaggagcg aaattttgta ggcgaggaag ggatagacgc gtaaagaaaa agtgattcgg 360
   gttgtgcgtt tcggcggcgt tcgggagggg attgttgtta cggaggggtt ttcgttaatt 420
   tttttttacg tttttcgagg gggtagattt tagttcgttt ttaagggcga gaagcgatga 480
   gataatttag ttgagttgcg aaaggagagc gtttggtttt ggattcgggg ggagggggtt 540
   ttcgggcggg gtgggttttt aaggtggggg cgagggtcgg cgttgatttt gggacggagt 600
   ggggcgggag ttgttgggtc gcgtagtagt gggcggcggc ggtttcgtat atttagttta 660
   ttcgcgttgc ggtcgtcggc gttttttgtt tcgttcggag ttgttcggcg ttttagttgt 720
   tcgtttcgtc ggtcgttttt gttcgcggcg tagatggttg tgcgcggcgt taatattttg 780
   acgttttttt ttatttaggc gatttttaat aagaaagagg agcgcggcgg gttggtcgcg 840
   ttagaggggc gttcggcgtt cgggggtata gcggtatcgg tggtcgcggt tttcgttgtt 900
   tgttgttggc ggttttttgg ggagagggac gcgggcgcgt tggggggcgt cgaggatttt 960
   ttgttggcgt tttttgtcgg tattagaata gttgcggggc ggattgcgga gagttcggaa 1020
   ggttgggatt cggatttcgc gtttagcgag gagaacgaga gtaggcggcg ttgcgcggac 1080
   gcgcgggggg ttagcggggt cggttttgcg gggggatttt tgagtttcgg ttagtcggtt 1140
   tgtgagttgg tcgtttttaa agatttagag gaggaagtcg cgggtcggag cgatagcgag 1200
   atgttcgtta gcgttttagg tttgttgcgg tttcgcgggg atgggggtcg ggttgagggg 1260
   atggacgcgg agggtaatta cgtcgagtgg ggtggtcggt ttgagtattt tgcgttttgg 1320
   ggttcggagg ttggttaaat ttcgaagttt tgcgttttgg cgcgagttgt tttattattt 1380
   cgggaattga gttcgcgaga gatcggagta gttttttaga gttttacgtt ttggggtgag 1440
   gtttgagata ttttgggtta atgtgtatta gattgggttg ggagaatcgt tgttttttgc 1500
   gttcgggagt ttttgagttc gtcgttagtg ttttttgtcg tgtttgtttt taaattttta 1560
   aaattaggga agcggaaaag tgaggggttt agagatttta ggtagttagt gatttagttt 1620
   tggattagag atttgttagt ttaaggtttt agggtcgggt tttatagtag gtggataggt 1680
   ggtgaagaat attttcgagt gggttagagt tgagaatttg tagaattaga gttttgggga 1740
   aagggttggt ttggtttttt tagattttag gtttgggaga gtagttttag gtcgaagatt 1800
   tcggattggg tttttagttt attttggagg taggagagag gtttttttag tcgtggtttg 1860
   ttttgttttg tggtttggaa gattcgggtg tttgttttag ttgggtataa gtttttgtag 1920
   gttttaagta taggggatcg ttggattttt ttgttggagt ttttcggaag ggcgggtacg 1980
   agatcgagaa gttggtgtgg gattagggtt taaaaattcg atgtaggggt aatttttttt 2040
   ttttattttt tagtttattt ttttatttaa ggtttcggtt tagtttgttt ttatgttttt 2100
   tatagtaatt ttaggcggtt gagggtgaaa gattttaaat atttttggtt ttaggattcg 2160
   tttttatttt tttttatttg tattagataa gtgtgtgttg cgaggacgcg cgcgtgtgtg 2220
   cgtgtgaatt aagtgtggag aggcgcgtga atttaagtag agagacggaa atgtagttta 2280
   gaattttaat gtttaacgtt gagagtcggg agggttttcg cggatgttcg ggtcgagtgt 2340
   tttaagttat tgaggggagt ttggggttta gagagggtgg ggagttggtt tcgataatat 2400
   agcgttgata tagcggttga gtttgtcgtt ttttttagaa gaggtttaga tgggatattt 2460
   agtggggtaa gagtttaaga ttggtttttc gggtttaggg ttttttagat tttcgtttcg 2520
   tttgagtggg attaaagtgt attttcgttt tattttcgtg atttttgtag ttgaggttcg 2580
   gttttatttc gtttgtggcg ttgatagttt tttgtttttg tttttatcgg ggttcgtagg 2640
   cgatcgtagt ttaaggatcg aggacgacgg tgttggtttt agaggtgtat acgtgttcgc 2700
   gttgtgtagc ggggtcggcg gcgggggcgg tagcgggtcg gtaggcgtcg cggaggagga 2760
   ggaggagtcg gcggcgttta agttacgtaa gaagcgttcg cgggtcgttt ttttttacgc 2820
   gtaggttttc gagttggagc gtcgttttaa ttattagcgt tatttgttcg ggttcgagcg 2880
   cgtagatttg gtcgcgtcgt tgaagtttat cgagacgtag gtgaaaattt ggttttagaa 2940
   tcgtcgttat aagataaagc gtcggtagat ggtagtcgat ttgttggttt cggcgttcgt 3000
   t 3001
<210> 635
   <211> 3001
   <212> DNA
   <213> human
<400> 635
   ttaaagttaa tgttgtttaa ttggagttag ttaatggatg gttgggagtg tttgggatag 60
   tttagtggag gggtgtggtt ttaggttgtt atttagtagt gaggaatgat atttgggtgg 120
   gaggggggtg gtatagtgga atgtaaggga aggaaagaga aaagaaatag ataaaattag 180
   ggtagggggt aaaaaagttt tttgtgtttt ttggaattta gtggttgtgg ttgtggttat 240
   tgttgttgtt gttgggggtg ggttttggaa gagtttgttt agtagggaag gggaggatgg 300
   gttaggagtg aaattttgta ggtgaggaag ggatagatgt gtaaagaaaa agtgatttgg 360
   gttgtgtgtt ttggtggtgt ttgggagggg attgttgtta tggaggggtt tttgttaatt 420
   tttttttatg ttttttgagg gggtagattt tagtttgttt ttaagggtga gaagtgatga 480
   gataatttag ttgagttgtg aaaggagagt gtttggtttt ggatttgggg ggagggggtt 540
   tttgggtggg gtgggttttt aaggtggggg tgagggttgg tgttgatttt gggatggagt 600
   ggggtgggag ttgttgggtt gtgtagtagt gggtggtggt ggttttgtat atttagttta 660
   tttgtgttgt ggttgttggt gttttttgtt ttgtttggag ttgtttggtg ttttagttgt 720
   ttgttttgtt ggttgttttt gtttgtggtg tagatggttg tgtgtggtgt taatattttg 780
   atgttttttt ttatttaggt gatttttaat aagaaagagg agtgtggtgg gttggttgtg 840
   ttagaggggt gtttggtgtt tgggggtata gtggtattgg tggttgtggt ttttgttgtt 900
   tgttgttggt ggttttttgg ggagagggat gtgggtgtgt tggggggtgt tgaggatttt 960
   ttgttggtgt tttttgttgg tattagaata gttgtggggt ggattgtgga gagtttggaa 1020
   ggttgggatt tggattttgt gtttagtgag gagaatgaga gtaggtggtg ttgtgtggat 1080
   gtgtgggggg ttagtggggt tggttttgtg gggggatttt tgagttttgg ttagttggtt 1140
   tgtgagttgg ttgtttttaa agatttagag gaggaagttg tgggttggag tgatagtgag 1200
   atgtttgtta gtgttttagg tttgttgtgg ttttgtgggg atgggggttg ggttgagggg 1260
   atggatgtgg agggtaatta tgttgagtgg ggtggttggt ttgagtattt tgtgttttgg 1320
   ggtttggagg ttggttaaat tttgaagttt tgtgttttgg tgtgagttgt tttattattt 1380
   tgggaattga gtttgtgaga gattggagta gttttttaga gttttatgtt ttggggtgag 1440
   gtttgagata ttttgggtta atgtgtatta gattgggttg ggagaattgt tgttttttgt 1500
   gtttgggagt ttttgagttt gttgttagtg ttttttgttg tgtttgtttt taaattttta 1560
   aaattaggga agtggaaaag tgaggggttt agagatttta ggtagttagt gatttagttt 1620
   tggattagag atttgttagt ttaaggtttt agggttgggt tttatagtag gtggataggt 1680
   ggtgaagaat atttttgagt gggttagagt tgagaatttg tagaattaga gttttgggga 1740
   aagggttggt ttggtttttt tagattttag gtttgggaga gtagttttag gttgaagatt 1800
   ttggattggg tttttagttt attttggagg taggagagag gtttttttag ttgtggtttg 1860
   ttttgttttg tggtttggaa gatttgggtg tttgttttag ttgggtataa gtttttgtag 1920
   gttttaagta taggggattg ttggattttt ttgttggagt tttttggaag ggtgggtatg 1980
   agattgagaa gttggtgtgg gattagggtt taaaaatttg atgtaggggt aatttttttt 2040
   ttttattttt tagtttattt ttttatttaa ggttttggtt tagtttgttt ttatgttttt 2100
   tatagtaatt ttaggtggtt gagggtgaaa gattttaaat atttttggtt ttaggatttg 2160
   tttttatttt tttttatttg tattagataa gtgtgtgttg tgaggatgtg tgtgtgtgtg 2220
   tgtgtgaatt aagtgtggag aggtgtgtga atttaagtag agagatggaa atgtagttta 2280
   gaattttaat gtttaatgtt gagagttggg agggtttttg tggatgtttg ggttgagtgt 2340
   tttaagttat tgaggggagt ttggggttta gagagggtgg ggagttggtt ttgataatat 2400
   agtgttgata tagtggttga gtttgttgtt ttttttagaa gaggtttaga tgggatattt 2460
   agtggggtaa gagtttaaga ttggtttttt gggtttaggg ttttttagat ttttgttttg 2520
   tttgagtggg attaaagtgt atttttgttt tatttttgtg atttttgtag ttgaggtttg 2580
   gttttatttt gtttgtggtg ttgatagttt tttgtttttg tttttattgg ggtttgtagg 2640
   tgattgtagt ttaaggattg aggatgatgg tgttggtttt agaggtgtat atgtgtttgt 2700
   gttgtgtagt ggggttggtg gtgggggtgg tagtgggttg gtaggtgttg tggaggagga 2760
   ggaggagttg gtggtgttta agttatgtaa gaagtgtttg tgggttgttt ttttttatgt 2820
   gtaggttttt gagttggagt gttgttttaa ttattagtgt tatttgtttg ggtttgagtg 2880
   tgtagatttg gttgtgttgt tgaagtttat tgagatgtag gtgaaaattt ggttttagaa 2940
   ttgttgttat aagataaagt gttggtagat ggtagttgat ttgttggttt tggtgtttgt 3000
   t 3001
<210> 636
   <211> 120
   <212> DNA
   <213> human
<400> 636
   tttttagtag agacggggtt tcaccgtgtt gggcaggata gtctccatct cctgacctca 60
   tgatccaccc gcctcggcct tccaaagtgc ttgtattaca ggcttgagcc tccgcgcccg 120
<210> 637
   <211> 85
   <212> DNA
   <213> human
<400> 637
   cccctcaccg cggaaggcgt ggccggaatg ctcggcccac tccgtctttg gcttgtgggc 60
   tctgcctcgt gcaccgcgtg ctgca 85
<210> 638
   <211> 103
   <212> DNA
   <213> human
<400> 638
   cccggaccgg gacacggagg tgaagctggt gcgggagaca gacggggtga tggcagaggc 60
   ggtgctgaag ctacgactgc ccccgctccg gaaggacaca ctt 103
<210> 639
   <211> 18
   <212> DNA
   <213> human
<400> 639 18
   ttttttatcg cggaaggc 18
<210> 640
   <211> 18
   <212> DNA
   <213> human
<400> 640 18
   cgaacgcgaa aactcaaa 18
<210> 641
   <211> 30
   <212> DNA
   <213> human
<400> 641
   cactttaaaa aaccgaaacg aataaatcat 30
<210> 642
   <211> 18
   <212> DNA
   <213> human
<400> 642
   ttttttatcg cggaaggc 18
<210> 643
   <211> 18
   <212> DNA
   <213> human
<400> 643
   tacaacacgc gatacacg 18
<210> 644
   <211> 29
   <212> DNA
   <213> human
<400> 644
   aaaaacgaaa taaaccgaac attccgacc 29
<210> 645
   <211> 18
   <212> DNA
   <213> human
<400> 645
   ttcggatcgg gatacgga 18
<210> 646
   <211> 21
   <212> DNA
   <213> human
<400> 646
   aaatatatcc ttccgaaacg a 21
<210> 647
   <211> 22
   <212> DNA
   <213> human
<400> 647
   tcaccccgtc tatctcccgc ac 22
<210> 648
   <211> 25
   <212> DNA
   <213> human
<400> 648
   tggtgatgga ggaggtttag taagt 25
<210> 649
   <211> 27
   <212> DNA
   <213> human
<400> 649
   aaccaataaa acctactcct cccttaa 27
<210> 650
   <211> 30
   <212> DNA
   <213> human
<400> 650
   accaccaccc aacacacaat aacaaacaca 30

## Description

The present invention relates to genomic DNA sequences that exhibit altered CpG methylation patterns in disease states relative to normal. Particular embodiments provide methods, nucleic acids and kits useful for detecting ovarian cancer (carcinomas).

### Background

**DNA methylation.** The etiology of pathogenic states is known to involve modified methylation patterns of individual genes or of the genome. 5-methylcytosine, in the context of CpG dinucleotide sequences, is the most frequent covalently modified base in the DNA of eukaryotic cells, and plays a role in the regulation of transcription, genetic imprinting, and tumorigenesis. The identification and quantification of 5-methylcytosine sites in a specific specimen, or between or among a plurality of specimens, is thus of considerable interest, not only in research, but particularly for the molecular diagnoses of various diseases.

**Correlation of aberrant DNA methylation with cancer.** Aberrant DNA methylation within CpG "islands" is characterized by hyper- or hypomethylation of CpG dinucleotide sequences leading to abrogation or overexpression of a broad spectrum of genes, and is among the earliest and most common alterations found in, and correlated with human malignancies. Additionally, abnormal methylation has been shown to occur in CpG-rich regulatory elements in intronic and coding parts of genes for certain tumors. In ovarian carcinoma, for example, aberrant DNA methylation constitutes one of the most prominent alterations and inactivates tumor suppressor genes such as ARH1, RASSF1A and DNA mismatch repair genes such as BRCA1 (Hennessy BT et al. Ovarian cancer: homeobox genes, autocrine/paracrine growth, and kinase signaling. Int J Biochem Cell Biol. 2006;38(9):1450-6).

In contrast to the specific hypermethylation of tumor suppressor genes, an overall hypomethylation of DNA can be observed in tumor cells. This decrease in global methylation can be detected early, far before the development of frank tumor formation. A correlation between hypomethylation and increased gene expression has been determined for many oncogenes.

**Ovarian carcinoma.** Ovarian cancer is the fifth most common cancer in women and the leading cause of death from gynecological cancers. It is estimated that approximately 21,650 new cases will be diagnosed 2008 in the US, with 15,520 of the women dying from this disease, corresponding to 6 % of all cancer deaths. Worldwide there are more than 200,000 new cases of ovarian carcinoma each year. Studies in Europe and the US show that the average five-year survival rate is between 35 % and 45 %.

As for most cancers, incidence is highest for women older than 60. Younger women are affected more often when a predisposition for the disease is inherited, such as by mutations in the BRCAI/2 or HPNCC genes. By far the most common cancers of the ovaries constitute adenocarcinomas of epithelial origin with serous, clear cell, endometrioid or mucous histological subtypes.

Treatment of most epithelial ovarian carcinomas consists of surgical removal of as much cancerous tissue as possible (debulking), both ovaries (bilateral salpingo oophorectomy) and the uterus (hysterectomy), followed by a combination chemotherapy with platinum and another agent (typically a taxane).

**Diagnosis of ovarian carcinoma.** Survival of ovarian carcinoma patients is mainly influenced by the stage of the disease when initially diagnosed. More than 90 % of patients survive longer than 5 years, when the disease is recognized while still confined to the ovaries. With regional metastases, the 5-year survival is at 70 %; with distant metastasis the 5-year survival rate is as low as 25 %. Unfortunately, symptoms of ovarian carcinoma are very vague and the disease is typically diagnosed at an advanced stage.

Ovarian carcinoma fulfills all major WHO criteria for suitability of a disease for screening. With a lifetime risk of 2 % ovarian carcinoma is less prevalent than other major cancers but still constitutes a significant disease burden for the population. Most importantly it is a fatal disease with high mortality rate that can be effectively treated when diagnosed early. However, since there is no viable early detection test for ovarian carcinoma, the disease is usually diagnosed when patients already experience symptoms and consult their gynecologist. Even then the diagnostic options are very limited. Pelvic examination, i.e., feeling the uterus, vagina, ovaries, fallopian tubes, bladder and rectum to find any abnormality in their shape or size and transvaginal ultrasound (TVS), a procedure that uses highfrequency sound waves to detect lesions in the ovaries, are the most frequently used investigation methods. However, they are not capable of differentiating between (frequent) benign ovarian lesions and truly dangerous tissue resulting in frequent false positive test results. Computerized axial tomography (CAT) scan produces a series of detailed pictures of areas inside the body created by a computer linked to an X-ray machine. Similar to TVS it has problems in distinguishing between benign and cancerous lesions. Cancer Antigen 125 (CA-125) assay measures the level of the CA-125 protein, a sugar protein that may be released when cells are inflamed or damaged. It is a tumor marker that is often found in higher-than-normal amounts in the blood of women with ovarian carcinoma. However, it is not recommended for screening purposes as it produces an unacceptably high number of false positive events, due to its up-regulation in various benign conditions (Jacobs IJ et al. Progress and challenges in screening for early detection of ovarian carcinoma. Mol Cell Proteomics. 2004 Apr;3(4):355-66).

The concentration measurement of the CA125 protein in serum is widely used to monitor ovarian carcinoma patients for response to chemotherapy and recurrence of disease (Podczaski E et al. Use of CA 125 to monitor patients with ovarian epithelial carcinomas. Gynecol Oncol. 1989;33:193-7). About 80 % of ovarian carcinomas express high levels of CA-125. However, even as a monitoring test for response and recurrence CA-125 has several shortcomings. Firstly, it only detects about 80 % of ovarian carcinomas and cannot be used as a monitoring test for the remaining 20 % of patients. Secondly, the sensitivity of CA-125 for small lesions is very limited. Rubin and colleagues have reported that 50% of women with normal CA-125 levels after primary therapy had persistent disease documented at second-look surgery (Rubin SC et al. Serum CA 125 levels and surgical findings in patients undergoing secondary operations for epithelial ovarian carcinoma. Am J Obstet Gynecol. 1989 Mar;160(3):667-71). Thirdly, due to CA125 serum levels beeing elevated in a variety of benign conditions treatment options for patients with elevated CA125 level and no symptoms are ambiguous. The initiation of salvage therapy (chemotherapy, radiotherapy, or surgery) solely based on an increased CA 125 level is questionable, due to its high false positive rate and was recently shown to have no survival benefit (Rustin GJ et al. A randomized trial in ovarian carcinoma of early treatment of relapse based on CA125 level alone versus delayed treatment based on conventional clinical indicators (MRC OV05/EORTC 55955 trials). J Clin Oncol 27:18s, 2009 (suppl; abstr 1)).

**Development of medical tests.** Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (IN) result is where the test is negative but the condition is not present. In this context: Sensitivity=TP/(TP+FN); Specificity=TN/(FP+TN); and Predictive value=TP/(TP+FP).

Sensitivity is a measure of a test's ability to correctly detect the target disease in an individual being tested. A test having poor sensitivity produces a high rate of false negatives, i.e., individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, i.e., individuals who are falsely identified as having the disease. A drawback of false positives is that they force patients to undergo unnecessary medical procedures treatments with their attendant risks, emotional and financial stresses, and which could have adverse effects on the patient's health. A feature of many diseases making it difficult to develop diagnostic tests with high specificity is that disease mechanisms, particularly in cancer, often involve a plurality of genes and proteins. Additionally, certain proteins may be elevated for reasons unrelated to a disease state. In example of a test that has high specificity is a gene-based test that can detect a p53 mutation. Specificity is important when the cost or risk associated with further diagnostic procedures or further medical interventions are very high.

**Pronounced need in the art.** Currently available diagnostic procedures for early detection and monitoring of ovarian carcinoma are often not applicable in medical practice due to their lack in sensitivity and specificity. A sufficiently sensitive and specific diagnostic test would make these applications feasible and could dramatically improve ovarian carcinoma patient prognosis.

In view of the incidence and mortality of ovarian carcinoma and disadvantages associated with current ovarian carcinoma diagnosis methods there is a substantial need in the art for improved methods for the early detection and monitoring of ovarian carcinoma, to be used in addition to or as a substitute for currently available tests.

### Definitions

The term "methylation status" refers to whether a given cytosine residue in a CpG dinucleotide of a genomic nucleic acid is methylated or unmethylated. The term can also refer to a plurality of CpG dinuleotides within a given genomic nucleic acid.

The term "detection" of ovarian carcinoma refers to the discrimination of patients with ovarian carcinoma from women without the disease. A particular detection method is usually not correct in every case - its quality is determined by the true positive rate (sensitivity) and the false positive rate (1 - specificity). The term "detection" is meant to encompass both the diagnosis of the disease as well as monitoring the disease.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation status of one or more CpG dinucleotide sequences within a sequence of DNA.

The term "MethyLight" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers*) covering CpG positions between, or covered by the amplification primers enable methylation-specific selective amplification of a nucleic acid sample.

The term "HeavyMethyl™ MethyLight" assay, in the embodiment thereof implemented herein, refers to a HeavyMethyl™ MethyLight assay, which is a variation of the MethyLight assay, wherein the MethyLight assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridising at 68 °C in 5x SSC/5x Denhardt's solution/1.0 % SDS, and washing in 0.2x SSC/0.1 % SDS at room temperature, or involve the art-recognized equivalent thereof (e.g., conditions in which a hybridization is carried out at 60 °C in 2.5 x SSC buffer, followed by several washing steps at 37°C in a low buffer concentration, and remains stable). Moderately stringent conditions, as defined herein, involve including washing in 3x SSC at 42 °C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "methylation-specific restriction enzyme" or "methylation-sensitive restriction enzyme" shall be taken to mean an enzyme that selectively digests a nucleic acid dependant on the methylation status of its recognition site. Preferred are methylation-specific restriction enzymes, the recognition sequence of which comprises a CG dinucleotide.

Any reference to a gene by name shall be taken to include all transcript variants thereof and all promoter and regulatory elements thereof.

### Description of the invention

The present invention provides methods, nucleic acids and kits for detecting ovarian carcinoma in a subject. Said method comprises determining the methylation status of at least one target nucleic acid selected from the group consisting of the genes as referred to by name in Table 1 in a biological sample isolated from said subject. The target nucleic acid is also referred to as a "marker" herein.

Specifically, the invention refers to a method for detecting ovarian cancer in a subject using an ovarian tissue as the basis of a biological sample that is isolated from the subject and that contains genomic DNA. The method comprises at least the following steps:

Firstly, the methylation status of at least one CpG dinucleotide in a target sequence, i.e. in a gene or DNA portion according to SEQ ID NOs 1 to 5 is determined that is selected from the group consisting of the target sequences as referred to by gene name in Table 1, in a biological sample isolated from a subject. It is preferred that the target sequence is a target sequence as listed with SEQ ID NO 1 in table 1, which is a genomic sequence.

Secondly, from the determined methylation status of the target sequence, it is deduced whether the subject has ovarian cancer. This deduction takes place, depending on the target sequence used, on the basis of the methylation status, i.e. an upmethylation or a downmethylation that was determined for the target sequence, as will be explained in detail below.

When the methylation status of more than one CpG dinucleotide is determined, the at least one CpG dinucleotide can be located within one or within at least two target sequences selected form the group consisting of the genomic sequences as provided in Table 1. -

It is preferred that the methylation status of at least 2, 3, 4, 5, 6, 7, 8, 9, 10, 11, 12, 13, 14, 15, or 25 CpG dinucleotides are determined together for determining whether the subject has ovarian cancer or not. As pointed out above, the CpG dinucleotides can be located within one target sequence or several target sequences, depending on the number of CpG dinucleotides that are being determined.

The biological sample is preferably selected from the group consisting of ovarian tissue, peritoneal tissue, lymph node tissue, fallopian tube tissue, blood, serum, plasma, and peritoneal cavity fluid and combinations thereof. It is preferred that the biological sample stems from blood; serum; plasma or peritoneal cavity fluid, as they can be obtained using minimally invasive techniques.

Specifically, the method preferably comprises isolating genomic DNA from the biological sample that was obtained from the subject. The subject can be any animal, but is preferably a human.

Preferably, determining the methylation status comprises treating the genomic DNA or a fragment thereof with a chemical reagent or an enzyme containing solution, whereby the base pairing behavior of methylated cytosine bases and/or unmethylated cytosine bases of the nucleic acid are altered such that methylated cytosine bases become distinguishable from unmethylated cytosine bases. This allows for an analysis of the methylation status of the target sequence using common DNA analysis techniques, such as amplification techniques like PCR. The preferred chemical reagent is bisulfite.

Accordingly, the determination of the methylation status of the method preferably comprises amplifying the treated DNA or selected fragments thereof by means of methylation specific primers and/or blocking oligonucleotides. Most preferred, the presence or absence of the amplificates by means of a real-time detection probe is determined.

The invention also refers to the use of a nucleic acid for the detection of ovarian carcinoma in a subject using a biological sample isolated from the subject, wherein the nucleic acid is chosen from the group of nucleic acids with the SEQ ID NOs 1 to 5.

Further, the invention refers to the use of a nucleic acid for the detection of ovarian carcinoma, wherein the nucleic acid comprises at least 16 contiguous nucleotides of a sequence selected from the group consisting of the bisulfite sequences according to SEQ ID NOs 2 to 5 of Table 1, and sequences complementary thereto.

Further, the invention refers to a nucleic acid for detecting ovarian cancer in a subject that comprises at least 50, preferably 60, 70, or 80 contiguous nucleotides of a sequence selected from the group consisting of the bisulfite sequences according to SEQ ID NOs 2 to 5 as provided in Table 1, and sequences complementary thereto. The use of such nucleic acids for detecting ovarian cancer in a subject is also part of the invention.

In one aspect, the invention provides a method comprising the following steps: i) contacting genomic DNA isolated from a biological sample obtained from the subject with at least one reagent, or series of reagents that distinguishes between methylated and non-methylated CpG dinucleotides within at least one target region of the genomic DNA, wherein the nucleotide sequence of said target region comprises at least one CpG dinucleotide sequence from the group consisting of the genes according to SEQ ID NOs 1 to 5 as referred to by name in Table 1 and ii) detecting ovarian carcinoma. Preferably the target region comprises, or hybridizes under stringent conditions to a sequence of at least 16 contiguous nucleotides of a sequence selected from the group consisting of the genomic sequences according to SEQ ID NO 1 as provided in Table 1.

More specifically, the present invention provides a method for detecting ovarian carcinoma suitable for use in a diagnostic tool, comprising: obtaining a biological sample comprising genomic nucleic acid(s); contacting the nucleic acid(s), or a fragment thereof, with a reagent or a plurality of reagents sufficient for distinguishing between methylated and non methylated CpG dinucleotide sequences within a target sequence of the subject nucleic acid, wherein the target sequence comprises, or hybridizes under stringent conditions to, a sequence comprising at least 16 contiguous nucleotides of the genomic sequence according to SEQ ID NO 1 as provided in Table 1 said contiguous nucleotides comprising at least one CpG dinucleotide sequence; and determining, based at least in part on said distinguishing, the methylation status of at least one target CpG dinucleotide sequence, or an average, or a value reflecting an average methylation status of a plurality of target CpG dinucleotide sequences. The methylation status of a plurality of CpG dinucleotides can for example be determined by numerically averaging hybridization measurements from several contiguous nucleotide sequences comprising at least one CpG dinucleotide sequence, by measuring hybridization of a single contiguous nucleotide sequence comprising more than one CpG dinucleotide sequence, or by requiring concurrent hybridization of several contiguous nucleotide sequences comprising at least one CpG dinucleotide sequence (e.g. MSP MethyLight or HeavyMethyl^{™} MethyLight assays). It is particularly preferred that the methylation status of a plurality of CpG positions is determined, preferably at least 2, 3, 4, 5, 6, 7, 8, 9, 10, 11, 12, 13, 14, 15 or 25 CpG positions.

Further embodiments provide a method for the detection (i.e. diagnosis or monitoring of chemotherapy response) of ovarian carcinoma, comprising: obtaining a biological sample having subject genomic DNA; extracting the genomic DNA; contacting the genomic DNA, or a fragment thereof, comprising one or more sequences selected from the group consisting of the genomic sequence according to SEQ ID NO 1 as provided in Table 1 or a sequence that hybridizes under stringent conditions thereto, with one or more methylation-sensitive restriction enzymes, wherein the genomic DNA of the genomic sequence according to SEQ ID NO 1 as provided in Table 1 is either digested thereby to produce digestion fragments, or is not digested thereby; and determining, based on a presence or absence of, or on property of at least one such fragment, the methylation status of at least one CpG dinucleotide sequence of the genomic sequence according to SEQ ID NO 1 as provided in Table 1, or more preferably an average, or a value reflecting an average methylation status of a plurality of CpG dinucleotide sequences thereof. Preferably said plurality is at least 2, 5, 10, 15 or 25 CpG positions. Preferably, said enzymatically treated genomic DNA is amplified prior to said determining.

Additionally, genomic and chemically modified nucleic acid sequences, as well as oligonucleotides and/or PNA-oligomers for analysis of cytosine methylation patterns within the genomic sequences as provided in Table 1 are disclosed, in particular for a medical use, in particular for detecting ovarian cancer in a subject.

**The present invention provides** a method for detecting the presence of ovarian carcinoma in a subject comprising determining the methylation levels of at least one gene with a sequence according to SEQ ID NO 1 as referred to by name in Table 1 in a biological sample isolated from the subject. These markers may be used for the diagnosis of ovarian carcinoma.

The present invention provides for the use of a bisulfite technique, in combination with one or more methylation assays, for determination of the methylation status of CpG dinucleotide sequences within the genomic sequence according to SEQ ID NO 1 as provided in Table 1. Genomic CpG dinucleotides can be methylated or unmethylated (alternatively known as up- and down- methylated, respectively). However, the methods of the present invention are suitable for the analysis of biological samples of a heterogeneous nature e.g. a low concentration of tumor cells within a background of blood or peritoneal cavity fluid. Accordingly, when analyzing the methylation status of a CpG position within such a sample the person skilled in the art may use a quantitative assay for determining the level (e.g. percent, fraction, ratio, proportion or degree) of methylation at a particular CpG position as opposed to a methylation status. Accordingly, the term methylation status should also be taken to mean a value reflecting the degree of methylation at a CpG position. Unless specifically stated, the terms "hypermethylated" or "upmethylated" shall be taken to mean a methylation level above that of a specified cut-off point, wherein said cut-off may be a value representing the average or median methylation level for a given population, or is preferably an optimized cut-off level. The "cut-off" is also referred herein as a "threshold". In the context of the present invention the terms "methylated", "hypermethylated" or "upmethylated" shall be taken to include a methylation level of one hundred (100) % (or equivalents thereof) or at or above a selected cut-off methylation value for all CpG positions within and associated with (e.g. in promoter or regulatory regions) at least one gene selected from the group consisting of the genes as referred to by name in Table 1. Unless specifically stated the terms "hypomethylated" or "down-methylated" shall be taken to mean a methylation level below that of a specified cut-off point, wherein said cut-off may be a value representing the average or median methylation level for a given population, or is preferably an optimized cut-off level. The "cut-off" is also referred herein as a "threshold". In the context of the present invention the terms "unmethylated", "hypomethylated" or "downmethylated" shall be taken to include a methylation level of zero (0) % (or equivalents thereof) or at or below a selected cut-off methylation value for all CpG positions within and associated with (e.g. in promoter or regulatory regions) at least one gene selected from the group consisting of the genes as referred to by name in Table 1.

According to the present invention, determination of the methylation status of CpG dinucleotide sequences within the genomic sequence according to SEQ ID NO 1 as provided in Table 1 has utility in detection (diagnosis, and monitoring chemotherapy response) of ovarian carcinoma.

The detection of neoplastic ovarian cells within a biological sample has further utility in the monitoring of patients in order to detect recurrence or to monitor disease progression or regression during treatment. Furthermore, the detection of early stage ovarian carcinoma is directly linked with disease prognosis, and the disclosed method thereby improves patient prognosis (Jacobs IJ et al. Progress and challenges in screening for early detection of ovarian carcinoma. Mol Cell Proteomics. 2004 Apr;3(4):355-66).

In one embodiment the invention of said method comprises the following steps: i) contacting genomic DNA (isolated from ovarian tissue) obtained from the subject with at least one reagent, or series of reagents that distinguishes between methylated and non-methylated CpG dinucleotides within at least one gene according to SEQ ID NOs 1 to 5 referred to by name in Table 1 (including promoter and regulatory regions thereof) and ii) determining the presence or absence of ovarian neoplastic cells within said sample.

It is preferred that said one or more CpG dinucleotides of at least one gene selected from the group consisting of the genes according to SEQ ID NOs 1 to 5 as referred to by name in Table 1 are comprised of a respective genomic target sequence thereof as provided in the genomic sequence according to SEQ ID NO 1_in Table 1 and complements thereof.

In a preferred embodiment, said method comprises the following steps: In the *first step,* a sample of the tissue to be analyzed is obtained.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lyzed by enzymatic, chemical or mechanical means. The DNA solution may then be cleared of proteins and other contaminants and the genomic DNA is then recovered from the solution. The person skilled in the art may also make use of devices such as filter devices e.g. ultrafiltration, silica surfaces or membranes, magnetic particles, polystyrol particles, polystyrol surfaces, positively charged surfaces, and positively charged membranes, charged membranes, charged surfaces, charged switch membranes, charged switched surfaces.

Once the nucleic acids have been extracted, the genomic double stranded DNA is analyzed.

In the *second step* of the method, the genomic DNA sample is treated that methylated cytosines are differentiated form non-methylated cytosines. A number of suitable treatment methods are known in the art, including but not limited to methylation sensitive restriction enzyme digest and treatment of the sample with bisulfite reagents. Both of said methods may be utilized in the method of the present invention. However, the bisulfite treatment method is particularly preferred.

### Bisulfite analysis method

Methods of bisulfite treatment are known in the art. The bisulfite treated DNA is preferably purified prior to the subsequent method steps. This may be conducted by any means known in the art, such as but not limited to ultrafiltration. The purification is carried out according to the manufacturer's protocol. Suitable kits are commercially available, such as but not limited to the EpiTect Bisulfite Kit^{™} and EZ DNA Methylation Kit^{™}.

In the *third step* of the method, fragments of the treated DNA are amplified, using one or more pairs of primer oligonucleotides according to the present invention, and an amplification enzyme. Preferably, the amplification is carried out by means of a polymerase chain reaction (PCR), in one embodiment said amplificates are 80 to 1,000 base pairs in length. Preferably each of said pair of primer oligonucleotides includes at least two oligonucleotides whose sequences are each reverse complementary, identical, or hybridize under stringent or highly stringent conditions to an at least 16-base-pair long segment of the base sequences of a sequence selected from the group consisting of the bisulfite sequences according to SEQ ID NOs 2 to 5 as provided in Table 1 and sequences complementary thereto.

Alternatively, the methylation status of pre-selected CpG positions within at least one gene selected from the group consisting of the gene according to SEQ ID NOs 1 to 5 as referred to by name in Table 1 and preferably within a target nucleic acid sequences thereof according to the genomic sequence according to SEQ ID NO 1 in Table 1 may be detected by use of methylation-specific primer oligonucleotides. This technique has been described in United States Patent No. 6,265,171 (hereby incorporated by reference in its entirety). MSP primer pairs comprise at least one primer which hybridizes to a bisulfite treated CpG dinucleotide. Therefore, the sequence of said primers comprises at least one CpG dinucleotide. MSP primers specific for methylated DNA contain a "C" or "G" at the position of the C position in the CpG. MSP primers specific for non-methylated DNA contain a "T" or "A" at the position of the C position in the CpG. Preferably, therefore, the base sequence of said primers is required to comprise a sequence having a length of at least 9 nucleotides which hybridizes to a treated nucleic acid sequence according to one of the bisulfite sequences according to SEQ ID NOs 2 to 5 as provided in Table 1 and sequences complementary thereto, wherein the base sequence of said oligomers comprises at least one CpG dinucleotide.

A further preferred embodiment of the method comprises the use of *blocker* oligonucleotides (the HeavyMethyl™ assay). The use of such blocker oligonucleotides has been described by Yu et al., BioTechniques 23:714-720, 1997. Blocking probe oligonucleotides are hybridized to the bisulfite treated nucleic acid in tandem with the PCR primers. PCR amplification of the nucleic acid is terminated at the 5' position of the blocking probe, such that amplification of a nucleic acid is suppressed where the complementary sequence to the blocking probe is present. Disruption of polymerase-mediated amplification requires that blocker oligonucleotides are not elongated by the polymerase. This is achieved through the use of blockers that are 3'-deoxyoligonucleotides, or oligonucleotides derivatized at the 3' position with other than a "free" hydroxyl group. For example, 3'-O-acetyl oligonucleotides are representative of a preferred class of blocker molecule.

Said probes are preferably designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpG' at the position in question, as opposed to a 'CpG' if the suppression of amplification of methylated nucleic acids is desired.

It is further preferred that polymerase-mediated decomposition of the blocker oligonucleotides should be minimized. This may be achieved by use of a polymerase lacking 5'-3' exonuclease activity, or use of modified blocker oligonucleotides having, for example, thioate bridges at the 5'-terminii thereof that render the blocker molecule nuclease-resistant.

Preferably, the base sequence of said blocking oligonucleotides is required to comprise a sequence having a length of at least 9 nucleotides which hybridizes to a treated nucleic acid sequence according to one of the bisulfite sequences according to SEQ ID NOs 2 to 5 as provided in Table 1 and sequences complementary thereto, wherein the base sequence of said oligonucleotides comprises at least one CpG, TpG or CpA dinucleotide. More preferably the base sequence of said *blocking oligonucleotides* is required to comprise a sequence having a length of at least 9 nucleotides which hybridises to a treated nucleic acid sequence selected from the group consisting of the 0% methylated bisulfite sequences according to SEQ ID NOs 3 and 5 as provided in Table 1 and sequences complementary thereto.

The nucleic acid amplificates may be detectably labeled. Any suitable labels known in the art may be utilized, including but not limited to fluorescence labels, radionuclides, or detachable molecule fragments having a typical mass which can be detected in a mass spectrometer.

In the *fourth step* of the method, the amplificates obtained during the third step of the method are analyzed in order to ascertain the methylation status of said at least one or more CpG dinucleotides prior to the treatment.

In embodiments where the amplificates were obtained by means of MSP amplification, the presence or absence of an amplificate is in itself indicative of the methylation status of the CpG positions covered by the primer, according to the base sequences of said primer. The presence or absence of said amplificate may be detected by means of any suitable means known in the art, including the detection of suitable labels, or by means of a nucleic acid detection probe.

Amplificates obtained by means of both standard and methylation specific PCR may be further analyzed by means of methods such as, but not limited to, array technology and probe based technologies as well as by means of techniques such as sequencing and template directed extension.

In one embodiment of the method, the amplificates synthesized in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG, TpG or CpA dinucleotide. The hybridizing portion of the hybridizing nucleic acids is typically at least 9, 15, 20, 25, 30 or 35 nucleotides in length. However, longer molecules have inventive utility, and are thus within the scope of the present invention.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within the genomic sequence according to SEQ ID NO 1 as provided in Table 1, and the equivalent positions within the bisulfite sequences according to SEQ ID NOs 2 to 5 as provided in Table 1. Said oligonucleotides may also be peptide nucleic acids. The non-hybridized amplificates are then removed, and the hybridized amplificates are detected. It is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes (as detailed above) that are hybridized to the bisulfite treated DNA in tandem with the PCR amplification primers (wherein said primers may either be methylation-specific or standard).

A particularly preferred embodiment of this method is the use of fluorescence-based Real Time Quantitative PCR (Heid et al., Genome Res. 6:986-994, 1996; *also see* United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridize to a CpG-rich sequence located between the forward and reverse amplification primers. The TaqMan™ probe further comprises a fluorescent "reporter moiety" and a "quencher moiety" covalently bound to linker moieties (*e.g.* phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethyLightTM™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

In a further preferred embodiment of the method, the *fourth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sci USA 74:5463-5467, 1977).

In the most preferred embodiment of the method, the genomic nucleic acids are isolated and treated according to the first three steps of the method outlined above, namely:
a) Obtaining a biological sample from a subject comprising subject genomic DNA;
b) extracting or otherwise isolating said genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and
d) amplifying selected fragments of said treated DNA by means of methylation specific primers and/or blocking oligonucleotides, and
e) determining the presence or absence of said amplificates by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of the bisulfite sequences according to SEQ ID NOs 2 to 5 as provided in Table 1 and sequences complementary thereto, wherein the base sequence of said oligomers comprise at least one CpG dinucleotide.

Step e) of the method, namely the detection of the specific amplificates indicative of the methylation status of one or more CpG positions according to the genomic sequence according to SEQ ID NO 1 as provided in Table 1 is carried out by means of *real-time* detection methods as described above.

### Restriction Enzyme Analysis

Additional embodiments of the invention provide a method for the analysis of the methylation status of the at least one gene selected from the group consisting of the genes according to SEQ ID NO 1 to 5 as referred to by name in Table 1 (preferably the genomic sequence according to SEQ ID NO 1 as provided in Table 1, and complements thereof) without the need for bisulfite conversion. Methods are known in the art wherein a methylation sensitive restriction enzyme reagent, or a series of restriction enzyme reagents comprising methylation sensitive restriction enzyme reagents that distinguishes between methylated and non-methylated CpG dinucleotides within a target region are utilized in determining methylation.

In the *third step,* the DNA (or fragments thereof) is digested with one or more methylation sensitive restriction enzymes. The digestion is carried out such that hydrolysis of the DNA at the restriction site is informative of the methylation status of a specific CpG dinucleotide of at least one gene selected from the group consisting of the gene according to SEQ ID NOs 1 to 5 as referred to by name in Table 1, it is particularly preferred that a target region of said gene(s) selected from Table 1 according to SEQ ID NOs 1 to 5 is analysed.

Preferably, the methylation-specific restriction enzyme is selected from the group consisting of *Bsi E1, Hga I HinPl, Hpy99I, Ava I, Bce AI, Bsa HI, BisI, BstUI, BshI236I, AccII, BstFNI, McrBC, GlaI, MvnI, HpaII (HapII), HhaI, AciI, SmaI, HinP1I, HpyCH4IV, EagI* and mixtures of two or more of the above enzymes. Preferred is a mixture containing the restriction enzymes BstUI, HpaII, HpyCH4IV and HinP1I.

In the *fourth step,* which is optional but a preferred embodiment, the restriction fragments are amplified. This is preferably carried out using a polymerase chain reaction, and said amplificates may carry suitable detectable labels as discussed above, namely fluorophore labels, radionuclides and mass labels. Particularly preferred is amplification by means of an amplification enzyme and one or more primer pairs, each member of said pair comprising, in each case a contiguous sequence at least 16 nucleotides in length that is complementary to, or hybridizes under moderately stringent or stringent conditions to a sequence selected from the group consisting the genomic sequence according to SEQ ID NO 1 as provided in Table 1, and complements thereof. Preferably said contiguous sequence is at least 16, 20 or 25 nucleotides in length. In an alternative embodiment said primers may be complementary to any adaptors linked to the fragments.

In the *fifth step,* the amplificates are detected. The detection may be by any means standard in the art, for example, but not limited to, gel electrophoresis analysis, hybridization analysis, incorporation of detectable tags within the PCR products, DNA array analysis, MALDI or ESI analysis. Preferably said detection is carried out by hybridization to at least one nucleic acid or peptide nucleic acid comprising in each case a contiguous sequence at least 16 nucleotides in length that is complementary to, or hybridizes under moderately stringent or stringent conditions to a sequence selected from the group consisting of the genomic sequence according to SEQ ID NO 1 as provided in Table 1, and complements thereof. Preferably said contiguous sequence is at least 16, 20 or 25 nucleotides in length.

Subsequent to the determination of the methylation status or level of the genomic nucleic acids the presence, absence of ovarian carcinoma is deduced based upon the methylation status or level of at least one CpG dinucleotide sequence of the genomic sequences as provided in Table I according to SEQ ID NO 1, or an average, or a value reflecting an average methylation status of a plurality of CpG dinucleotide sequences of the genomic sequence according to SEQ ID NO I as provided in Table 1 wherein methylation is associated with the presence of ovarian carcinoma. Wherein said methylation is determined by quantitative means and for markers whose hypermethylation is associated with the presence of ovarian carcinoma, the cut-off point for determining said prognosis is preferably 0 % (i.e. wherein a sample displays any degree of methylation it is determined as having an ovarian carcinoma). For markers whose hypomethylation is associated with the presence of ovarian carcinoma, the cut-off point for determining said prognosis is preferably 100 % (i.e. wherein a sample displays any degree of unmethylated DNA it is determined as having an ovarian carcinoma).

Nonetheless, it is foreseen that the person skilled in the art may wish to adjust said cut-off value in order to provide an assay of a particularly preferred sensitivity or specificity. Accordingly said cut-off value may be increased (thus increasing the specificity) for genes whose hypermethylation is indicative of ovarian carcinoma. Said cut off value may be within a range selected form the group consisting of 0%-5%, 5%-10%, 10%-15%, 15%-20%, 20%-30% and 30%-50%. Particularly preferred are the cut-offs 0.01 %, 0.1 %, 1 % and 10 %. Said cut-off value may be decreased (thus increasing the specificity) for genes whose hypomethylation is indicative of ovarian carcinoma. Said cut off value may be within a range selected form the group consisting of 100%-95%, 95%-90%, 90%-85%, 85%-80%, 80%-70% and 70%-50%. Particularly preferred are the cut-offs 99.99%, 99.90%, 99% and 90 %.

Upon determination of the methylation of at least one gene selected from the group consisting of the gene according to SEQ ID NOs 1 to 5 as referred to by name in Table 1 the presence or absence of ovarian carcinoma is determined. Hypomethylation of the gene ODF2 indicates the presence of ovarian carcinoma.

### Nucleic acids

The disclosure provides treated nucleic acids, the bisulfite sequences according to SEQ ID NOs 2 to 5 as provided in Table 1, derived from the genomic sequences according to SEQ ID NO 1 as provided in Table 1. The sequences of the bisulfite sequences according to SEQ ID NOs 2 to 5 as provided in Table 1 are non-naturally occurring sequences and provide the sequences of various bisulfite modified nucleic acids of the genomic sequence according to SEQ ID NO 1 as provided in Table 1.

In a preferred embodiment of the invention, the invention provides a non-naturally occurring modified nucleic acid comprising a sequence of at least 16 contiguous nucleotide bases in length of a sequence selected from the group consisting of the bisulfite sequences as provided in table 1 according to SEQ ID NOs 2 to 5. In further preferred embodiments of the invention said nucleic acid is at least 50, 100, 150, 200, 250 or 500 base pairs in length of a segment of the nucleic acid sequence disclosed in the bisulfite sequences as provided in Table 1 according to SEQ ID NOs 2 to 5. Particularly preferred is a nucleic acid molecule that is identical or complementary to all or a portion of the bisulfite sequences as provided in Table 1 according to SEQ ID NOs 2 to 5 but not the genomic sequences as provided in Table 1 according to SEQ ID NO 1 or other naturally occurring DNA.

It is preferred that said sequence comprises at least one CpG, TpG or CpA dinucleotide and sequences complementary thereto. The sequences of the bisulfite sequences according to SEQ ID NOs 2 to 5 of table 1 provide non-naturally occurring modified versions of the nucleic acid according to the genomic sequence of SEQ ID NO 1 of table 1, wherein the modification of each genomic sequence results in the synthesis of a nucleic acid having a sequence that is unique and distinct from said genomic sequence as follows. For each sense strand genomic DNA-of the genomic sequences according to SEQ ID NO 1 as provided in Table 1, four converted versions are disclosed. A first version wherein "C" is converted to "T," but "CpG" remains "CpG" (*i.e*., corresponds to case where, for the genomic sequence, 100 % "C" residues of CpG dinucleotide sequences are methylated and are thus not converted); a second version of each genomic sequences is provided, wherein "C" is converted to "T" for all "C" residues, including those of "CpG" dinucleotide sequences (*i.e*., corresponds to case where, for the genomic sequences, 100 % of "C" residues of CpG dinucleotide sequences are unmethylated). A third chemically converted version discloses the complement of the disclosed genomic DNA sequence (i.e. *anti*sense strand), wherein "C" is converted to "T," but "CpG" remains "CpG" (*i.e*., corresponds to case where 100 % of "C" residues of CpG dinucleotide sequences are methylated and are thus not converted); a final bisulfite converted version of each sequence, discloses the complement of the disclosed genomic DNA sequence (i.e. *anti*sense strand), wherein "C" is converted to "T" for 100% of "C" residues, including those of "CpG" dinucleotide sequences (*i.e*., corresponds to case where, for the complement (*anti*sense strand) of each genomic sequence, all "C" residues of CpG dinucleotide sequences are *un*methylated)..

Alternatively, the invention further provides oligonucleotides or oligomers suitable for use in the methods of the invention for detecting the cytosine methylation status within genomic or treated (bisulfite modified) DNA, according to SEQ ID NOs: 1 to 5. Accordingly, said oligonucleotide or oligomer nucleic acids provide novel diagnostic means. Said oligonucleotide or oligomer comprise a nucleic acid sequence having a length of at least nine (9) nucleotides which is identical to, hybridizes, under moderately stringent or stringent conditions (as defined herein above), to a treated nucleic acid sequence according to the bisulfite sequences of SEQ ID NOs 2 to 5 as provided in Table 1 and/or sequences complementary thereto, or to a genomic sequence according to the genomic sequence of SEQ ID NO 1 as provided in Table 1 and/or sequences complementary thereto.

Particularly preferred is a nucleic acid molecule that hybridizes under moderately stringent and/or stringent hybridization conditions to all or a portion of the sequences the bisulfite sequences according to SEQ ID NOs 2 to 5 as provided in Table 1 but not the genomic sequence according to SEQ ID NO 1 as provided in Table 1 or other human genomic DNA.

The identical or hybridizing portion of the hybridizing nucleic acids is typically at least 9, 16, 20, 25, 30 or 35 nucleotides in length. However, longer molecules have inventive utility, and can also be used in the present invention.

Preferably, the hybridizing portion of the inventive hybridizing nucleic acids is at least 95 %, or at least 98 %, or 100 % identical to the sequence, or to a portion thereof of SEQ ID NOs: 1 to 5, or to the complements thereof.

In further embodiments, the present invention provides a set of at least two (2) oligonucleotides that are used as 'primer' oligonucleotides for amplifying DNA sequences of one of SEQ ID NOs: 1 to 5 and sequences complementary thereto, or segments thereof.

It is particularly preferred that the oligomers according to the invention are utilized for detecting, or for diagnosing ovarian carcinoma.

Genomic SEQ ID NO: 1 provides the target genomic nucleic acid of the gene ODF2.

### Kit

Moreover, an additional aspect of the present invention is the use of a kit in a method as described herein, wherein the kit comprises: a means for determining methylation of the gene according to SEQ ID NO2 1 to 5 as referred to by name in Table 1. The means for determining methylation of the gene according to SEQ ID NOs I to 5 as referred to by name in Table 1 comprise preferably a bisulfite-containing reagent; one or a plurality of oligonucleotides consisting whose sequences in each case are identical, are complementary, or hybridize under stringent or highly stringent conditions to a 9 or more preferably 18 base long segment of a sequence selected from the bisulfite sequences according to SEQ ID NOs 1 to 5 as provided in Table 1; and optionally instructions for carrying out and evaluating the described method of methylation analysis. In one embodiment the base sequence of said oligonucleotides comprises at least one CpG, CpA or TpG dinucleotide.

Said kit may further comprise standard reagents for performing a CpG position-specific methylation analysis, wherein said analysis comprises one or more of the following techniques: MS-SNuPE, MSP, MethyLight^{™}, HeavyMethyl, COBRA, and nucleic acid sequencing. However, a kit can also contain only part of the aforementioned components.

The kit may comprise additional bisulfite conversion reagents selected from the group consisting: DNA denaturation buffer; sulfonation buffer; DNA recovery reagents or kits (e.g., precipitation, ultrafiltration, affinity column); desulfonation buffer; and DNA recovery components.

In a further alternative embodiment, the kit may contain further elements packaged in separate containers, such as but not limited to a polymerase and a reaction buffer optimised for primer extension mediated by the polymerase, such as PCR. In another embodiment of the invention the kit further comprises means for obtaining a biological sample of the patient. Preferred is a kit, which further comprises a container suitable for containing the means for determining methylation of at least one gene according to SEQ ID NOs 1 to 5 as referred to by name in Table 1 in the biological sample of the patient, and most preferably further comprises instructions for use and interpretation of the kit results. In a preferred embodiment the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to an at least 9 or more preferably 18 base long segment of a sequence selected from the bisulfite sequences according to SEQ ID NOs 2 to 5 as provided in Table 1; and optionally (d) instructions for use and interpretation of the kit results. In an alternative preferred embodiment the kit comprises: (a) a bisulfite reagent; (b) a container suitable for containing the said bisulfite reagent and the biological sample of the patient; (c) at least one oligonucleotides and/or PNA-oligomer having a length of at least 9 or 18 nucleotides which is identical to or hybridises to a bisulfite treated nucleic acid sequence according to one of the bisulfite sequences according to SEQ ID NOs 1 to 5 as provided in Table 1 and sequences complementary thereto; and optionally (d) instructions for use and interpretation of the kit results.

In an alternative embodiment the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to an at least 9 or more preferably 18 base long segment of a sequence selected from the bisulfite sequences according to SEQ ID NOs 1 to 5 as provided in Table 1; (d) at least one oligonucleotides and/or PNA-oligomer having a length of at least 9 or 16 nucleotides which is identical to or hybridises to a pre-treated nucleic acid sequence according to one of the bisulfite sequences according to SEQ ID NOs 1 to 5 as provided in Table 1 and sequences complementary thereto; and optionally (e) instructions for use and interpretation of the kit results.

The kit may also contain other components such as buffers or solutions suitable for blocking, washing or coating, packaged in a separate container.

Moreover, an additional aspect of the present invention is an alternative use of a kit in a method as described herein, wherein the kit comprises a means for determining the CpG methylation status of at least one gene selected from the group consisting of the gene according to SEQ ID NOs 1 to 5 as referred to by name in Table 1, wherein said means comprise preferably at least one methylation specific restriction enzyme; one or a plurality of primer oligonucleotides (preferably one or a plurality of primer pairs) suitable for the amplification of a sequence comprising at least one CpG dinucleotide of a sequence according to SEQ ID NO 1 as provided in Table 1; and optionally instructions for carrying out and evaluating the described method of methylation analysis. In one embodiment the base sequence of said oligonucleotides are identical, are complementary, or hybridise under stringent or highly stringent conditions to an at least 18 base long segment of a sequence selected from the genomic sequences according to SEQ ID NO 1 as provided in Table 1.

Said kit may comprise one or a plurality of oligonucleotide probes for the analysis of the digest fragments, preferably said oligonucleotides are identical, are complementary, or hybridise under stringent or highly stringent conditions to an at least 16 base long segment of a sequence selected from the genomic sequence according to SEQ ID NO 1 as provided in Table 1, it is further preferred that the sequence thereof comprises at least one or more CpG dinucleotides.

The kit may comprise additional reagents selected from the group consisting: buffer (e.g. restriction enzyme, PCR, storage or washing buffers); DNA recovery reagents or kits (e.g., precipitation, ultrafiltration, affinity column) and DNA recovery components.

In a further alternative embodiment, the kit may contain, packaged in separate containers, a polymerase and a reaction buffer optimized for primer extension mediated by the polymerase, such as PCR. In another embodiment of the invention the kit further comprising means for obtaining a biological sample of the patient. In a preferred embodiment the kit comprises: (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridize under stringent or highly stringent conditions to an at least 9 base long segment of a sequence selected from the genomic sequence according to SEQ ID NO 1 as provided in Table 1; and optionally (d) instructions for use and interpretation of the kit results.

In an alternative preferred embodiment the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from the genomic sequence according to SEQ ID NO 1 as provided in Table 1; and optionally (d) instructions for use and interpretation of the kit results.

In an alternative embodiment the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of the sequence according to SEQ ID NO 1as provided in Table 1; (d) at least one set of oligonucleotides one or a plurality of nucleic acids or peptide nucleic acids which are identical, are complementary, or hybridize under stringent or highly stringent conditions to an at least 9 base long segment of the genomic sequence according to SEQ ID NO 1 as provided in Table 1 and optionally (e) instructions for use and interpretation of the kit results.

The kit may also contain other components such as buffers or solutions suitable for blocking, washing or coating, packaged in a separate container.

**TABLE 1: Target sequences according to the invention are shown that can be used as a marker to detect ovarian cancer. The genomic sequence of each target sequence can be used in the method of the invention to detect ovarian cancer. The corresponding bisulfite sequences i.e. the sequences obtainable from the respective target sequence after treatment with bisulfite (when the genomic target sequence is either 100 % or 0 % methylated) can be used in detecting ovarian cancer according to the invention. In the left column, target sequences are referred to by gene name.**

| | | Bisulfite Sequences | | | |
|---|---|---|---|---|---|
| Target Sequence | Genomic sequence | 100% methylated bisulfite converted sense strand | 0% methylated bisulfite converted sense strand | 100% methylated bisulfite converted antisense strand | 0% mthylated busulfite converted antisense strand |
| ODF2 | SEQ ID NO 1 | SEQ ID NO 2 | SEQ ID NO 3 | SEQ ID NO 4 | SEQ ID NO 5 |

### Figures

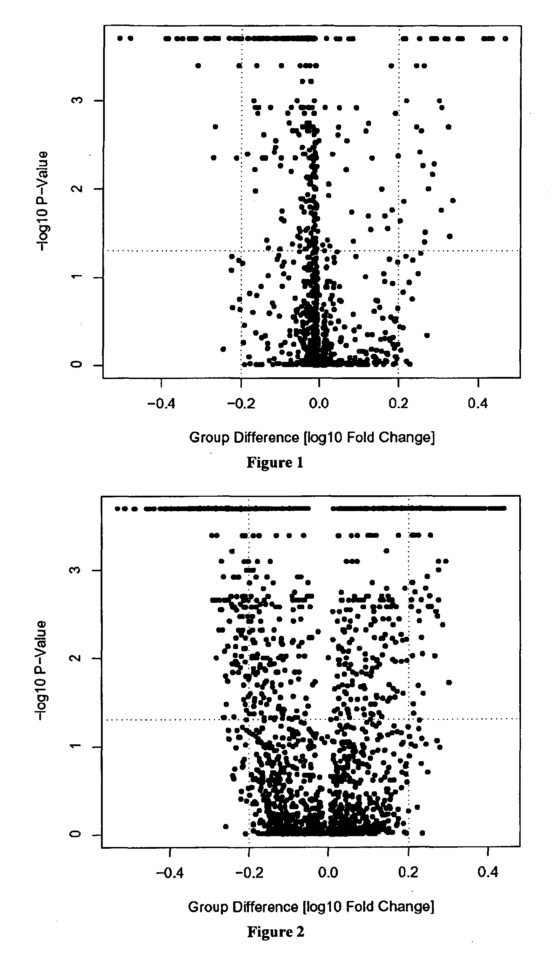

Figure 1 - Volcano plot of CpG dinucleotides with blood methylation <15% for the comparison between the group of normal and benign control tissues (ovarian cyst, normal ovary, normal colon, normal uterus, normal breast, normal lung) and the group of ovarian cancer cases. The horizontal axis shows the decadic logarithm of the group fold-changes. The vertical axis shows the negative decadic logarithm of p-values according to the Westfall-Young method. The horizontal dotted lines indicate the significance levels of p=0.05. The vertical dotted lines indicate fold-change of 0.6 and 1.6. Each individual point is one CpG dinucleotide. Points in the upper right corner fulfill the chosen selection criteria for hypermethylated ovarian cancer markers.
Figure 2 - Volcano plot of CpG dinucleotides with blood methylation >85% for the comparison between the group of normal and benign control tissues (ovarian cyst, normal ovary, normal colon, normal uterus, normal breast, normal lung) and the group of ovarian cancer cases. The horizontal axis shows the decadic logarithm of the group fold-changes. The vertical axis shows the negative decadic logarithm of p-values according to the Westfall-Young method. The horizontal dotted lines indicate the significance levels of p=0.05. The vertical dotted lines indicate fold-change of 0.6 and 1.6. Each individual point is one CpG dinucleotide. Points in the upper left corner fulfill the chosen selection criteria for hypomethylated ovarian cancer markers.

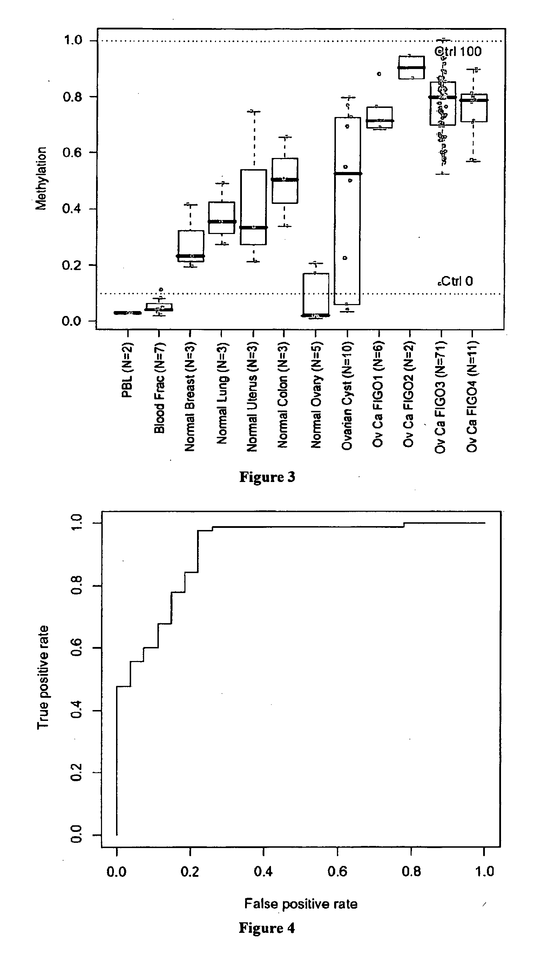

Figure 3 - Boxplot of DNA methylation distribution for SEQ ID NO 196 (gene HOM-TES-103). Horizontal axis shows the different tissue groups. Vertical axis shows DNA methylation as a number between 0 (0 %) and 1 (100 %). Open circles are individual sample measurements. Individual box plots, middle 50 % of the data; middle line, median; whiskers extend to the most extreme data point which is no more than 1.5 times the interquartile range from the box.
Figure 4 - Receiver operating characteristic (ROC) curve of SEQ ID NO 196 (gene HOM-TES-103) for the comparison between the group of normal and benign control tissues (ovarian cyst, normal ovary, normal colon, normal uterus, normal breast, normal lung) and the group of ovarian cancer cases.

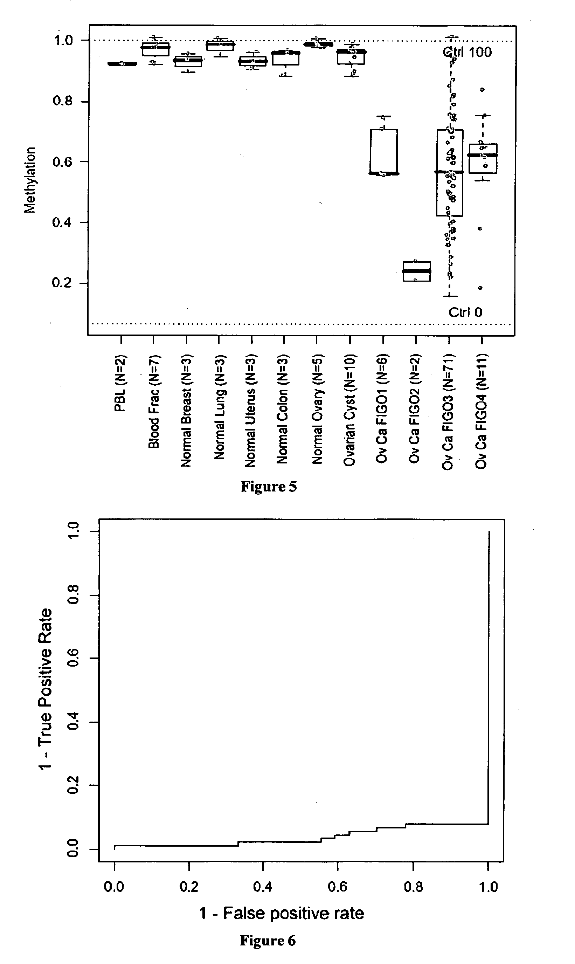

Figure 5 - Boxplot of DNA methylation distribution for SEQ ID NO 1 (gene ODF2). Horizontal axis shows the different tissue groups. Vertical axis shows DNA methylation as a number between 0 (0%) and 1 (100%). Open circles are individual sample measurements. Individual box plots, middle 50% of the data; middle line, median; whiskers extend to the most extreme data point which is no more than 1.5 times the interquartile range from the box.
Figure 6 - Receiver operating characteristic (ROC) curve of SEQ ID NO 1 (gene ODF2) for the comparison between the group of normal and benign control tissues (ovarian cyst, normal ovary, normal colon, normal uterus, normal breast, normal lung) and the group of ovarian cancer cases.

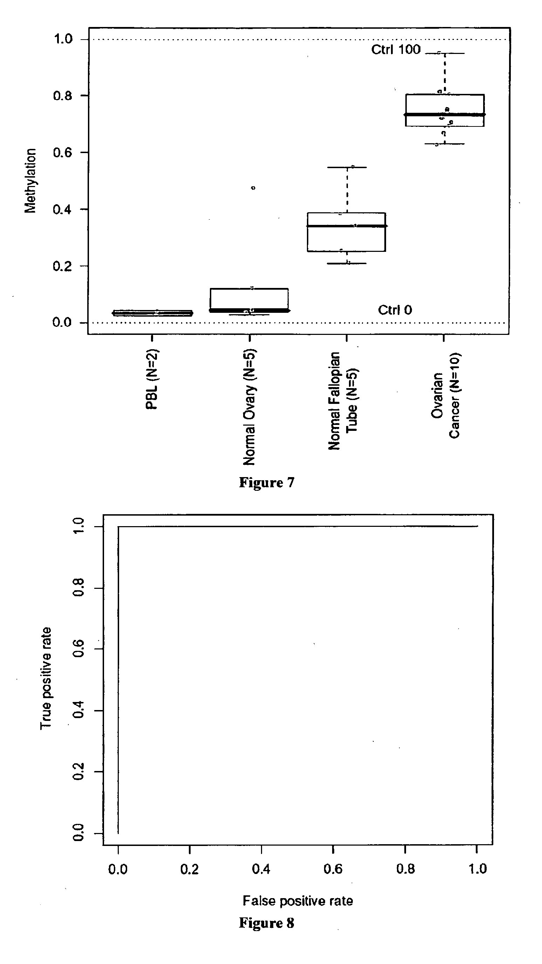

Figure 7 - Boxplot of DNA methylation distribution for SEQ ID NO 196 (gene HOM-TES-103). Horizontal axis shows the different tissue groups. Vertical axis shows DNA methylation as a number between 0 (0%) and 1 (100%). Open circles are individual sample measurements. Individual box plots, middle 50% of the data; middle line, median; whiskers extend to the most extreme data point which is no more than 1.5 times the interquartile range from the box.
Figure 8 - Receiver operating characteristic (ROC) curve of SEQ ID NO 196 (gene HOM-TES-103) for the comparison between the group of normal control tissues (normal ovary, normal fallopian tube) and the group of ovarian cancer cases.

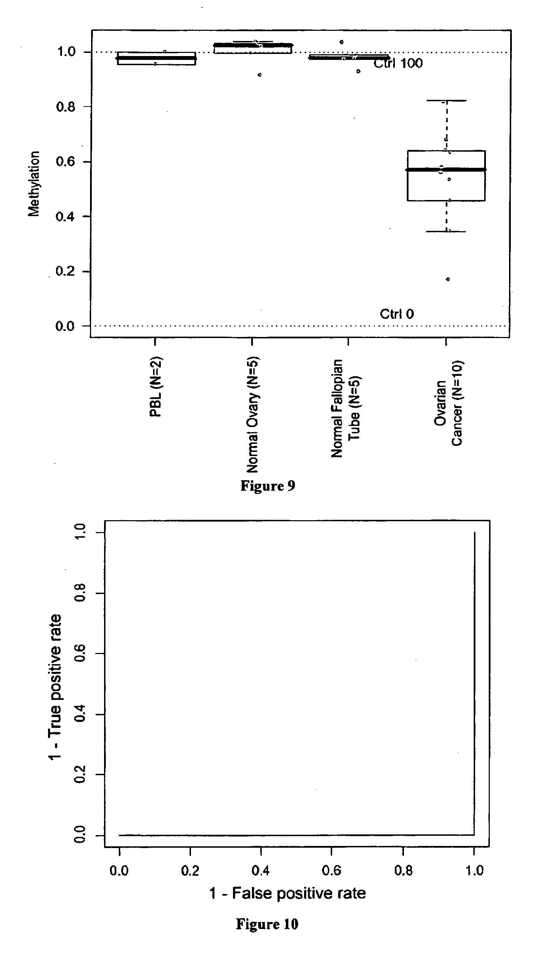

Figure 9 - Boxplot of DNA methylation distribution for SEQ ID NO 1 (gene ODF2). Horizontal axis shows the different tissue groups. Vertical axis shows DNA methylation as a number between 0 (0%) and 1 (100%). Open circles are individual sample measurements. Individual box plots, middle 50% of the data; middle line, median; whiskers extend to the most extreme data point which is no more than 1.5 times the interquartile range from the box.
Figure 10 - Receiver operating characteristic (ROC) curve of SEQ ID NO 1 (gene ODF2) for the comparison between the group normal control tissues (normal ovary, normal fallopian tube) and the group of ovarian cancer cases.

### Examples

### Example 1 - DNA methylation marker selection

The following experiments and analyses were performed in order to find target sequences that could be used to diagnose ovarian cancer based on the methylation status of the target sequence.

### 1. Samples

The sample set included fresh frozen tissue from 90 primary ovarian carcinoma, 10 ovarian cyst, 5 normal ovary, 3 normal colon, 3 normal uterus, 3 normal breast, and 3 normal lung samples. In addition the sample set included 2 peripheral blood samples (PBL) and one sample of each of the following blood cell types (blood fractions): CD 15+ granulocytes, CD 14+ monocytes, CD56+ NK cells, CD4+CD27+CD45RA+ naive T cells, CD4+CD27+CD45RA- memory T cells, CD8+CD27+CD45RA+ naive cytotoxic T cells, CD8+CD27+CD45RA- memory cytotoxic T cells. Tissue samples were received either as frozen tissue or extracted genomic DNA. Samples of the various blood cell types were generated by FACS sorting. DNA samples were extracted using the QIAamp DNA Mini Kit from Qiagen. In addition to the patient samples unmethylated human DNA (REPLI-g Mini Kit, Qiagen) and enzymatically methylated human DNA (CpGenome Universal Methylated DNA, Millipore) were included as calibration controls. All samples were quantitated using spectrophotometric or fluorometric techniques and agarose gels.

### 2. Microarray Experiments

Total genomic DNA of all samples was bisulfite treated converting unmethylated cytosines to uracil. Methylated cytosines remained conserved. Bisulfite treatment was performed using the EZ-96 DNA Methylation-Gold Kit from Zymo Research. The bisulfite treated DNA was whole genome amplified via random hexamer priming and Phi29 DNA polymerase, enzymatically fragmented and hybridized to Illumina Infinium II BeadChips. Target preparation and microarray hybridization, washing, and scanning was performed according to the manufacturer's protocol (Illumina, Inc., San Diego, Calif.). The methylation values for each CpG dinucleotide were calculated from the raw data using the BeadStudio software (Illumina, Inc., San Diego, Calif.).

### 3. Data Analysis

To identify methylation markers for the detection of ovarian cancer, the methylation levels between the group of normal and benign control tissues (ovarian cyst, normal ovary, normal colon, normal uterus, normal breast, normal lung) and the group of ovarian cancer cases were compared. The following filter criteria were applied to find markers that can particularly be detected in blood samples (see Lofton-Day C et al. DNA methylation biomarkers for blood-based colorectal cancer screening. Clin Chem. 2008 Feb;54(2):414-23). For CpG dinucleotide markers that are hypermethylated in ovarian cancer as compared to the control group, the methylation level in all blood samples (whole blood and fractions) had to be below 15 %. For markers that are hypomethylated in ovarian cancer as compared to the control group, the methylation level in all blood samples (whole blood and fractions) had to be above 85 %. For measured CpG dinucleotides that fulfilled these filter criteria the methylation measurements of the control group and ovarian cancer group were compared with a Student t-test. Results were corrected for multiple testing by using the Westfall-Young method (Westfall PH and Young SS Resampling-Based Multiple Testing: Examples and Methods for p-Value Adjustment. John Wiley & Sons, New York, 1993). This resulted in 38 CpG dinucleotides that showed significant hypermethylation in ovarian cancer with P<0.05 and a fold-change>1.6 (see Figure 01). 281 CpG dinucleotides showed significant hypomethylation in ovarian cancer with P<0.05 and a fold-change<0.6 (see Figure 02). These marker CpG dinucleotides were further ranked according to their area und the receiver operating characteristic curve (AUC). A receiver operating characteristic (ROC) curve is a plot of the true positive rate against the false positive rate for the different possible cutpoints of a diagnostic test. It shows the tradeoff between sensitivity and specificity depending on the selected cutpoint (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J. P. Egan. Signal Detection Theory and ROC Analysis, Academic Press, New York, 1975). Figures 03-06 show DNA methylation measurement values and receiver operating characteristic curves for the highest ranking hypermethylated CpG dinucleotide associated with gene HOM-TES-103 and the highest ranking hypomethylated CpG dinucleotide associated with gene ODF2. The highest ranking 22 hypermethylated CpG dinucleutides and 41 hypomethylated CpG dinucleotides are listed in Table 2.

**Table 2: Results of Example 1 - DNA methylation marker selection**

| **Target sequence** | **SEQ ID** | **Methylation in Cancer** | **AUC** |
|---|---|---|---|
| HOM-TES-103 | SEQ ID NO 196 | Hypermethylated | 0.92 |
| ALDH1A3 | SEQ ID NO 336 | Hypermethylated | 0.91 |
| ZFP41 | SEQ ID NO 366 | Hypermethylated | 0.91 |
| FLJ20032 | SEQ ID NO 446 | Hypermethylated | 0.90 |
| MEGF 11 | SEQ ID NO 456 | Hypermethylated | 0.89 |
| RAP1GA1 | SEQ ID NO 466 | Hypermethylated | 0.88 |
| ZNF154 | SEQ ID NO 476 | Hypermethylated | 0.87 |
| ADRB1 | SEQ ID NO 511 | Hypermethylated | 0.87 |
| LYPLAL1 | SEQ ID NO 501 | Hypermethylated | 0.87 |
| DGKI | SEQ ID NO 521 | Hypermethylated | 0.86 |
| ZNF154 | SEQ ID NO 491 | Hypermethylated | 0.85 |
| ALDH1A3 | SEQ ID NO 346 | Hypermethylated | 0.85 |
| GYPC | SEQ ID NO 536 | Hypermethylated | 0.85 |
| ENPP2 | SEQ ID NO 546 | Hypermethylated | 0.84 |
| SLC2A3 | SEQ ID NO 556 | Hypermethylated | 0.84 |
| ITGB2 | SEQ ID NO 566 | Hypermethylated | 0.83 |
| CDKN2A | SEQ ID NO 576 | Hypermethylated | 0.82 |
| BZRAP1 | SEQ ID NO 596 | Hypermethylated | 0.82 |
| EOMES | SEQ ID NO 586 | Hypermethylated | 0.82 |
| CPNE8 | SEQ ID NO 616 | Hypermethylated | 0.81 |
| FAIM2 | SEQ ID NO 606 | Hypermethylated | 0.81 |
| BAPX1 | SEQ ID NO 626 | Hypermethylated | 0.80 |
| CLDN16 | SEQ ID NO 11 | Hypomethylated | 0.96 |
| CTCFL | SEQ ID NO 21 | Hypomethylated | 0.96 |
| FLJ10379 | SEQ ID NO 31 | Hypomethylated | 0.96 |
| ODF2 | SEQ ID NO 1 | Hypomethylated | 0.96 |
| KRT5 | SEQ ID NO 41 | Hypomethylated | 0.95 |
| PCP4 | SEQ ID NO 66 | Hypomethylated | 0.95 |
| S100A1 | SEQ ID NO 51 | Hypomethylated | 0.95 |
| TREM2 | SEQ ID NO 76 | Hypomethylated | 0.95 |
| AMY1A | SEQ ID NO 106 | Hypomethylated | 0.94 |
| TMPRSS3 | SEQ ID NO 86 | Hypomethylated | 0.94 |
| TRIM51 | SEQ ID NO 96 | Hypomethylated | 0.94 |
| UPK1B | SEQ ID NO 116 | Hypomethylated | 0.94 |
| CX62 | SEQ ID NO 186 | Hypomethylated | 0.93 |
| FLJ00060 | SEQ ID NO 176 | Hypomethylated | 0.93 |
| LMLN | SEQ ID NO 126 | Hypomethylated | 0.93 |
| MPP7 | SEQ ID NO 156 | Hypomethylated | 0.93 |
| MUC15 | SEQ ID NO 166 | Hypomethylated | 0.93 |
| SLC24A2 | SEQ ID NO 146 | Hypomethylated | 0.93 |
| SPTBN2 | SEQ ID NO 136 | Hypomethylated | 0.93 |
| DDR1 | SEQ ID NO 226 | Hypomethylated | 0.92 |
| DLK1 | SEQ ID NO 236 | Hypomethylated | 0.92 |
| ESPL1 | SEQ ID NO 326 | Hypomethylated | 0.92 |
| FLJ13841 | SEQ ID NO 276 | Hypomethylated | 0.92 |
| GPKOW | SEQ ID NO 286 | Hypomethylated | 0.92 |
| ORSI1 | SEQ ID NO 316 | Hypomethylated | 0.92 |
| PAX8 | SEQ ID NO 296 | Hypomethylated | 0.92 |
| SCNN1A | SEQ ID NO 266 | Hypomethylated | 0.92 |
| SNTB1 | SEQ ID NO 306 | Hypomethylated | 0.92 |
| EIF3S2 | SEQ ID NO 211 | Hypomethylated | 0.92 |
| CFB | SEQ ID NO 246 | Hypomethylated | 0.92 |
| S100A1 | SEQ ID NO 56 | Hypomethylated | 0.92 |
| C20orf151 | SEQ ID NO 256 | Hypomethylated | 0.92 |
| CLDN8 | SEQ ID NO 436 | Hypomethylated | 0.91 |
| CUEDC1 | SEQ ID NO 406 | Hypomethylated | 0.91 |
| EIF3S2 | SEQ ID NO 221 | Hypomethylated | 0.91 |
| FLJ31196 | SEQ ID NO 416 | Hypomethylated | 0.91 |
| FLJ38379 | SEQ ID NO 376 | Hypomethylated | 0.91 |
| FOLH1 | SEQ ID NO 426 | Hypomethylated | 0.91 |
| KRTAP19-5 | SEQ ID NO 396 | Hypomethylated | 0.91 |
| MUC17 | SEQ ID NO 386 | Hypomethylated | 0.91 |
| FLJ90586 | SEQ ID NO 356 | Hypomethylated | 0.91 |

### Example 2 - DNA methylation marker validation

The following experiments and analyses were performed in order to validate the target sequences identified in Example 1.

### 1. Samples

The sample set included fresh frozen tissue from 10 primary ovarian carcinoma, 5 normal ovary, 5 normal fallopian tube samples. In addition the sample set included 2 peripheral blood samples (PBL). All samples were collected independently and from different sites than samples in Example 1. Tissue samples were received either as frozen tissue or extracted genomic DNA. DNA samples were extracted using the QIAamp DNA Mini Kit from Qiagen. In addition to the patient samples enzymatically methylated human DNA (CpGenome Universal Methylated DNA, Millipore) was included as calibration control. All samples were quantitated using spectrophotometric or fluorometric techniques and agarose gels.

### 2. Microarray Experiments

Total genomic DNA of all samples was bisulfite treated converting unmethylated cytosines to uracil. Methylated cytosines remained conserved. Bisulfite treatment was performed using the EZ-96 DNA Methylation-Gold Kit from Zymo Research. The bisulfite treated DNA was whole genome amplified via random hexamer priming and Phi29 DNA polymerase, enzymatically fragmented and hybridized to Illumina Infinium II BeadChips. Target preparation and microarray hybridization, washing, and scanning were performed according to the manufacturer's protocol (Illumina, Inc., San Diego, Calif.). The methylation values for each CpG dinucleotide were calculated from the raw data using the BeadStudio software (Illumina, Inc., San Diego, Calif.).

### 3. Data Analysis

To validate the methylation markers identified in Example 1, the methylation levels between the group of normal control tissues (normal ovary, normal fallopian tube) and the group of ovarian cancer cases on an independent samples set were compared. In addition, the methylation status in blood was verified.

For each methylation marker identified in Example 1, the area und the receiver operating characteristic curve (AUC) and the significance of the discrimination between control tissues and ovarian cancer cases using a Mann-Whitney-Wilcoxon test was computed. Results for the 22 hypermethylated CpG dinucleutides and 41 hypomethylated CpG dinucleotides identified in Example 1 are listed in Table 3.

Figures 7-10 show DNA methylation measurement values and receiver operating characteristic curves for the highest ranking hypermethylated CpG dinucleotide associated with gene HOM-TES-103 and the highest ranking hypomethylated CpG dinucleotide associated with gene ODF2.

**Table 3: Results of Example 2 - DNA methylation marker validation**

| **Gene Name** | **SEQ ID** | **Methylation in Cancer** | **AUC Validation** | **P Validation** |
|---|---|---|---|---|
| HOM-TES-103 | SEQ ID NO 196 | Hypermethylated | 1.0 | < 0.001 |
| ALDH1A3 | SEQ ID NO 336 | Hypermethylated | 1.0 | < 0.001 |
| ZFP41 | SEQ ID NO 366 | Hypermethylated | 0.98 | < 0.001 |
| FLJ20032 | SEQ ID NO 446 | Hypermethylated | 0.94 | < 0.001 |
| MEGF11 | SEQ ID NO 456 | Hypermethylated | 0.87 | 0.003 |
| RAP1GA1 | SEQ ID NO 466 | Hypermethylated | 0.90 | < 0.001 |
| ZNF154 | SEQ ID NO 476 | Hypermethylated | 0.98 | < 0.001 |
| ADRB1 | SEQ ID NO 511 | Hypermethylated | 0.71 | 0.1 |
| LYPLAL1 | SEQ ID NO 501 | Hypermethylated | 0.92 | < 0.001 |
| DGKI | SEQ ID NO 521 | Hypermethylated | 1.0 | < 0.001 |
| ZNF154 | SEQ ID NO 491 | Hypermethylated | 0.99 | < 0.001 |
| ALDH1A3 | SEQ ID NO 346 | Hypermethylated | 0.89 | 0.001 |
| GYPC | SEQ ID NO 536 | Hypermethylated | 0.83 | 0.007 |
| ENPP2 | SEQ ID NO 546 | Hypermethylated | 0.88 | 0.002 |
| SLC2A3 | SEQ ID NO 556 | Hypermethylated | 0.85 | 0.004 |
| ITGB2 | SEQ ID NO 566 | Hypermethylated | 0.63 | 0.3 |
| CDKN2A | SEQ ID NO 576 | Hypermethylated | 1.0 | < 0.001 |
| BZRAP1 | SEQ ID NO 596 | Hypermethylated | 0.95 | < 0.001 |
| EOMES | SEQ ID NO 586 | Hypermethylated | 1.0 | < 0.001 |
| CPNE8 | SEQ ID NO 616 | Hypermethylated | 0.73 | 0.07 |
| FAIM2 | SEQ ID NO 606 | Hypermethylated | 0.97 | < 0.001 |
| BAPX1 | SEQ ID NO 626 | Hypermethylated | 0.85 | 0.004 |
| CLDN16 | SEQ ID NO 11 | Hypomethylated | 0.95 | < 0.001 |
| CTCFL | SEQ ID NO 21 | Hypomethylated | 0.92 | < 0.001 |
| FLJ10379 | SEQ ID NO 31 | Hypomethylated | 0.78 | 0.02 |
| ODF2 | SEQ ID NO 1 | Hypomethylated | 1.0 | < 0.001 |
| KRT5 | SEQ ID NO 41 | Hypomethylated | 0.93 | < 0.001 |
| PCP4 | SEQ ID NO 66 | Hypomethylated | 0.90 | < 0.001 |
| S100A1 | SEQ ID NO 51 | Hypomethylated | 0.92 | < 0.001 |
| TREM2 | SEQ ID NO 76 | Hypomethylated | 0.88 | 0.002 |
| AMY1A | SEQ ID NO 106 | Hypomethylated | 0.90 | < 0.001 |
| TMPRSS3 | SEQ ID NO 86 | Hypomethylated | 0.96 | < 0.001 |
| TRIM51 | SEQ ID NO 96 | Hypomethylated | 0.95 | < 0.001 |
| UPK1B | SEQ ID NO 116 | Hypomethylated | 0.83 | 0.007 |
| CX62 | SEQ ID NO 186 | Hypomethylated | 0.79 | 0.02 |
| FLJ00060 | SEQ ID NO 176 | Hypomethylated | 0.87 | 0.002 |
| LMLN | SEQ ID NO 126 | Hypomethylated | 1.0 | < 0.001 |
| MPP7 | SEQ ID NO 156 | Hypomethylated | 0.96 | < 0.001 |
| MUC15 | SEQ ID NO 166 | Hypomethylated | 0.91 | < 0.001 |
| SLC24A2 | SEQ ID NO 146 | Hypomethylated | 0.98 | < 0.001 |
| SPTBN2 | SEQ ID NO 136 | Hypomethylated | 0.99 | < 0.001 |
| DDR1 | SEQ ID NO 226 | Hypomethylated | 0.97 | < 0.001 |
| DLK1 | SEQ ID NO 236 | Hypomethylated | 0.96 | < 0.001 |
| ESPL1 | SEQ ID NO 326 | Hypomethylated | 0.81 | 0.01 |
| FLJ13841 | SEQ ID NO 276 | Hypomethylated | 0.93 | < 0.001 |
| GPKOW | SEQ ID NO 286 | Hypomethylated | 0.90 | < 0.001 |
| OR5I1 | SEQ ID NO 316 | Hypomethylated | 0.88 | 0.002 |
| PAX8 | SEQ ID NO 296 | Hypomethylated | 0.90 | < 0.001 |
| SCNN1A | SEQ ID NO 266 | Hypomethylated | 0.94 | < 0.001 |
| SNTB1 | SEQ ID NO 306 | Hypomethylated | 0.90 | < 0.001 |
| EIF3S2 | SEQ ID NO 211 | Hypomethylated | 1.0 | < 0.001 |
| CFB | SEQ ID NO 246 | Hypomethylated | 0.96 | < 0.001 |
| S100A1 | SEQ ID NO 56 | Hypomethylated | 0.98 | < 0.001 |
| C20orf151 | SEQ ID NO 256 | Hypomethylated | 0.96 | < 0.001 |
| CLDN8 | SEQ ID NO 436 | Hypomethylated | 0.72 | 0.1 |
| CUEDC1 | SEQ ID NO 406 | Hypomethylated | 0.85 | 0.004 |
| EIF3S2 | SEQ ID NO 221 | Hypomethylated | 0.95 | < 0.001 |
| FLJ31196 | SEQ ID NO 416 | Hypomethylated | 0.82 | 0.009 |
| FLJ38379 | SEQ ID NO 376 | Hypomethylated | 0.95 | < 0.001 |
| FOLH1 | SEQ ID NO 426 | Hypomethylated | 0.80 | 0.02 |
| KRTAP19-5 | SEQ ID NO 396 | Hypomethylated | 0.89 | 0.001 |
| MUC17 | SEQ ID NO 386 | Hypomethylated | 0.94 | < 0.001 |
| FLJ90586 | SEQ ID NO 356 | Hypomethylated | 0.99 | < 0.001 |

### Example 3 - Marker Verification Using MSP-MethyLight Assays The following experiments and analyses can be performed in order to verify the target sequences found in Example 1 and validated in Example 2.

Determination of the methylation status within ovarian carcinoma using 3 MSP-MethyLight assays for the genes ODF2, CTCFL and KRT5, respectively (SEQ ID NO 636 for Gene ODF2, SEQ ID NO 637 for Gene CTCFL and SEQ ID NO 638 for Gene KRT5) as target sequences. DNA is extracted from 30 ovarian adenocarcinoma samples and 30 normal ovarian tissues using a Qiagen extraction kit. The DNA from each sample is treated using an EpiTect Bisulfite Kit from Qiagen. The treatment is such that all unmethylated cytosines within the sample are converted to thymidine. Conversely, 5-methylated cytosines within the sample remain unmodified.

The methylation status is determined with an MSP-MethyLight assay designed for the sequence fragments of interest (methylation assays) and a control fragment (control assay) from the beta actin gene (Eads et al., 2001). The methylation assays cover CpG sites in both the primers and the Taqman style probe, while the control assay does not. The control assay is used as a measure of total DNA concentration, and the methylation assays determine the methylation levels at that site.

### Methods:

For the gene ODF2, the SEQ ID NO 636 gene methylation assay is performed using the following primers and probes:
Forward Primer: TTTTTTATCGCGGAAGGC; (SEQ ID NO: 639)
Reverse Primer: CGAACGCGAAAACTCAAA; (SEQ ID NO: 640)
and Probe: CACTTTAAAAAACCGAAACGAATAAATCAT (SEQ ID NO: 641).

For the gene CTCFL, the SEQ ID NO 637 gene methylation assay is performed using the following primers and probes:
Forward Primer: TTTTTTATCGCGGAAGGC; (SEQ ID NO: 642)
Reverse Primer: TACAACACGCGATACACG; (SEQ ID NO: 643)
and Probe: AAAAACGAAATAAACCGAACATTCCGACC. (SEQ ID NO: 644)

For the Gene KRT5, the SEQ ID NO 638 gene methylation assay is performed using the following primers and probes:
Forward Primer: TTCGGATCGGGATACGGA; (SEQ ID NO: 645)
Reverse Primer: AAATATATCCTTCCGAAACGA; (SEQ ID NO: 646)
and Probe: TCACCCCGTCTATCTCCCGCAC. (SEQ ID NO: 647)

The corresponding control assay is performed using the following primers and probes:
Primer: TGGTGATGGAGGAGGTTTAGTAAGT; (SEQ ID NO: 648)
Primer: AACCAATAAAACCTACTCCTCCCTTAA; (SEQ ID NO: 649)
and Probe: ACCACCACCCAACACACAATAACAAACACA (SEQ ID NO: 650)

The reactions are run in triplicate on each DNA sample with the following assay conditions: Reaction solution: (900 nmol/l primers; 300 nmol/l probe; 3.5 mmol/l magnesium chloride; 1 unit of Taq polymerase; 200 µmol/l dNTPs; 7 µmol/l of DNA, in a final reaction volume of 20 µmol/l); Cycling conditions: (95 °C for 10 minutes; then 45 cycles of: 95 °C for 15 seconds; 60 °C for 1 minute).

## Claims

1. A method for detecting ovarian cancer in a subject using an ovarian tissue as a biological sample comprising genomic DNA isolated from the subject, comprising:
- Determining the methylation status of a CpG dinucleotide in a target sequence that is selected from the group consisting of the target sequences according to SEQ ID NOs 1 to 5 in a biological sample isolated from a subject, and
- Deducing from the determined methylation status of the target sequence whether the subject has ovarian cancer.

2. The method according to claims 1, further comprising isolating genomic DNA from the biological sample.

3. The method according to claims 1 or 2, wherein determining the methylation status comprises treating the genomic DNA or a fragment thereof with a chemical reagent or an enzyme containing solution, whereby the base pairing behavior of methylated cytosine bases and/or unmethylated cytosine bases of the nucleic acid are altered such that methylated cytosine bases become distinguishable from unmethylated cytosine bases.

4. The method according to claims 1 to 3, wherein determining the methylation status comprises amplifying the treated genomic DNA by means of methylation specific primers and/or blocking oligonucleotides.

5. The method according to claim 4, wherein determining the methylation status comprises determining the presence or absence of the amplified DNA by means of a real-time detection probe.

6. Use of a nucleic acid for the detection of ovarian carcinoma, wherein the nucleic acid comprises at least 16 contiguous nucleotides of a sequence selected from the group consisting of the bisulfite sequences of ODF2 with a sequence according to SEQ ID NOs 2
to 5, and sequences complementary thereto.

7. Use of a nucleic acid for detecting ovarian cancer in a subject, comprising at least 50 contiguous nucleotides of a sequence selected from the group consisting of the bisulfite sequences of ODF2 with a sequence according to SEQ ID NOs 2 to 5, and sequences complementary thereto.

8. Use of a kit in a method according to claims 1 to 5, wherein the kit comprises
(a) a bisulfite reagent for converting a nucleic acid, and
(c) a set of oligonucleotides containing two oligonucleotides whose sequences in each case are identical, are complementary, or hybridize under stringent or highly stringent conditions to a 9, or more preferably 18, base long segment of a sequence selected from the bisulfite sequences of ODF2 with a sequence according to SEQ ID NOs 1 to 5.

9. Use of a kit in a method according to claims 1 to 5, wherein the kit comprises
(a) a methylation sensitive restriction enzyme reagent, and
(b) at least one set of oligonucleotides containing one or a plurality of nucleic acids or peptide nucleic acids which are identical, are complementary, or hybridize under stringent or highly stringent conditions to an at least 9 base long segment of the genomic sequences of ODF2 with a sequence according to SEQ ID NO 1.

## Patentansprüche

1. Verfahren zur Detektion von Ovarialkrebs in einem Subjekt unter Verwendung eines Ovarialgewebes als biologische Probe, wobei die biologische Probe von dem Subjekt isolierte genomische DNA umfasst, aufweisend:
- Bestimmung des Methylierungsstatus eines CpG Dinucleotides in einer Zielsequenz die ausgebildet ist aus der Gruppe bestehend aus den Zielsequenzen gemäß SEQ ID Nr.1 bis 5 in einer von dem Subjekt isolierten biologischen Probe, und
- Folgern auf der Grundlage des bestimmten Methylierungsstatus der Zielsequenz, ob das Subjekt Ovarialkrebs hat.

2. Verfahren gemäß Anspruch 1, weiterhin umfassend die Isolierung genomischer DNA aus der biologischen Probe.

3. Verfahren gemäß den Ansprüchen 1 oder 2, wobei die Bestimmung des Methylierungsstatus die Behandlung der genomischen DNA oder eines Fragmentes davon mit einem chemischen Reagenz oder mit einer ein Enzym enthaltenden Lösung umfasst, wobei das Basenpaarungsverhalten von methylierten Cytosinbasen und/oder unmethylierten Cytosinbasen der Nukleinsäure derart verändert werden, dass methylierte Cytosinbasen von unmethylierten Cytosinbasen unterscheidbar werden.

4. Verfahren gemäß Anspruch 1 bis 3, wobei die Bestimmung des Methylierungsstatus die Amplifizierung der behandelten genomischen DNA mittels methylierungsspezifischer Primer und/oder Blockierungs-Oligonukleotide umfasst.

5. Verfahren gemäß Anspruch 4, wobei die Bestimmung des Methylierungsstatus die Bestimmung der Anwesenheit oder der Abwesenheit der amplifizierten DNA mittels einer Real-time Detektionssonde umfasst.

6. Verwendung einer Nukleinsäure zur Detektion von Ovarialkrebs, wobei die Nukleinsäure mindestens 16 aufeinander folgende Nukleotide einer Sequenz umfasst, die ausgewählt ist aus der Gruppe bestehend aus den Bisulfitsequenzen von ODF2 mit einer Sequenz gemäß SEQ ID Nr. 2 bis 5 und dazu komplementären Sequenzen.

7. Verwendung einer Nukleinsäure zur Detektion von Ovarialkrebs in einem Subjekt, umfassend mindestens 50 aufeinander folgende Nukleotide einer Sequenz, die ausgewählt ist aus der Gruppe bestehend aus den Bisulfitsequenzen von ODF2 mit einer Sequenz gemäß SEQ ID Nr. 2 bis 5 und dazu komplementären Sequenzen.

8. Verwendung eines Kits in einem Verfahren gemäß Anspruch 1 bis 5, wobei der Kit umfasst
(a) eine Bisulfitreagenz zur Konvertierung einer Nukleinsäure und
(c) ein Satz von Oligonukleotiden, enthaltend zwei Oligonukleotide, deren Sequenz jeweils identisch ist mit oder komplementär ist zu oder unter stringenten oder hochstringenten Bedingungen hybridisiert mit
einem 9, bevorzugt einem 18 Basenpaar langen Segment einer Sequenz, die ausgewählt ist aus den Bisulfitsequenzen von ODF2 mit einer Sequenz gemäß SEQ ID Nr.1 bis 5.

9. Verwendung eines Kits in einem Verfahren gemäß Anspruch 1 bis 5, wobei der Kit umfasst
(a) ein methylierungssensitives Restriktionsenzym Reagenz und
(b) mindestens einen Satz von Oligonukleotiden, enthaltend eine oder eine Mehrzahl von Nukleinsäuren oder Peptidnukleinsäuren, die identisch ist mit oder komplementär ist zu oder unter stringenten oder hochstringenten Bedingungen hybridisiert mit einem mindestens 9 Basen langen Segment der genomischen Sequenz von ODF2 mit einer Sequenz gemäß SEQ ID Nr.1.

## Revendications

1. Procédé pour détecter le cancer de l'ovaire chez un sujet en utilisant un tissu ovarien en tant qu'échantillon biologique comprenant de l'ADN génomique isolé à partir du sujet, comprenant :
- la détermination du statut de méthylation d'un dinucléotide CpG dans une séquence cible qui est choisi dans le groupe constitué des séquences cibles selon SEQ ID NO 1 à 5 dans un échantillon biologique isolé à partir d'un sujet, et
- la déduction à partir du statut de méthylation déterminé de la séquence cibler du fait que le sujet a un cancer de l'ovaire.

2. Procédé selon la revendication 1, comprenant en outre l'isolement d'ADN génomique à partir de l'échantillon biologique.

3. Procédé selon les revendications 1 ou 2, dans lequel la détermination du statut de méthylation comprend le traitement de l'ADN génomique ou un fragment de celui-ci avec un réactif chimique ou une solution contenant une enzyme, de sorte que le comportement d'appariement de bases de bases cytosines méthylées et/ou bases cytosines non méthylées de l'acide nucléique soit modifié de sorte que les bases cytosines méthylées puissent être distinguées des bases cytosines non méthylées.

4. Procédé selon les revendications 1 à 3, dans lequel la détermination du statut de méthylation comprend l'amplification de l'ADN génomique traité au moyen d'amorces et/ou d'oligonucléotides bloquants spécifiques pour la méthylation.

5. Procédé selon la revendication 4, dans lequel la détermination du statut de méthylation comprend la détermination de la présence ou l'absence de l'ADN amplifié au moyen d'une sonde de détection en temps réel.

6. Utilisation d'un acide nucléique pour la détection d'un carcinome ovarien, l'acide nucléique comprenant au moins 16 nucléotides contigus d'une séquence choisie dans le groupe constitué des séquences de bisulfite de ODF2 avec une séquence selon SEQ ID NO: 2 à 5, et des séquences complémentaires de celles-ci.

7. Utilisation d'un acide nucléique pour détecter un cancer de l'ovaire chez un sujet, comprenant au moins 50 nucléotides contigus d'une séquence choisie dans le groupe constitué des séquences de bisulfite de ODF2 avec une séquence selon SEQ ID NO: 2 à 5, et des séquences complémentaires de celles-ci.

8. Utilisation d'un kit dans un procédé selon les revendications 1 à 5, le kit comprenant
(a) un réactif à bisulfite pour convertir un acide nucléique, et
(c) un ensemble d'oligonucléotides contenant deux oligonucléotides dont les séquences dans chaque cas sont identiques, sont complémentaires, ou s'hybrident dans des conditions stringentes ou très stringentes à un segment de 9, ou plus préférablement 18, bases de longueur d'une séquence choisie parmi les séquences de bisulfite de ODF2 avec une séquence selon SEQ ID NO 1 à 5.

9. Utilisation d'un kit dans un procédé selon les revendications 1 à 5, le kit comprenant
(a) un réactif d'enzyme de restriction sensible à la méthylation, et
(b) au moins un ensemble d'oligonucléotides contenant un ou une pluralité d'acides nucléiques ou d'acides nucléiques peptidiques qui sont identiques, sont complémentaires, ou s'hybrident dans des conditions stringentes ou très stringentes à un segment d'au moins 9 bases de longueur des séquences génomiques de ODF2 avec une séquence selon SEQ ID NO 1.
